# EUROPEAN PATENT APPLICATION

(11) **EP 1 634 952 A2**
(43) Date of publication of application: **15.03.2006**
(21) Application number: 05019191.5
(22) Date of filing: 23.06.2000
(51) Int. Cl.: C12N 15/31, C12N 15/52, C12N 9/00, C07K 14/34

(54) **Corynebacterium glutamicum genes encoding proteins involved in membrane synthesis and membrane transport**

(30) Priority: 25.06.1999 US 141031 P; 08.07.1999 DE 19931454; 08.07.1999 DE 19931478; 08.07.1999 DE 19931563; 09.07.1999 DE 19932122; 09.07.1999 DE 19932124; 09.07.1999 DE 19932125; 09.07.1999 DE 19932128; 09.07.1999 DE 19932180; 09.07.1999 DE 19932182; 09.07.1999 DE 19932190; 09.07.1999 DE 19932191; 09.07.1999 DE 19932209; 09.07.1999 DE 19932212; 09.07.1999 DE 19932227; 09.07.1999 DE 19932228; 09.07.1999 DE 19932229; 09.07.1999 DE 19932230; 14.07.1999 DE 19932927; 14.07.1999 DE 19933005; 14.07.1999 DE 19933006; 27.08.1999 DE 19940764; 27.08.1999 DE 19940765; 27.08.1999 DE 19940766; 27.08.1999 DE 19940830; 27.08.1999 DE 19940831; 27.08.1999 DE 19940832; 27.08.1999 DE 19940833; 31.08.1999 DE 19941378; 31.08.1999 DE 19941379; 31.08.1999 DE 19941395; 03.09.1999 DE 19942077; 03.09.1999 DE 19942078; 03.09.1999 DE 19942079; 03.09.1999 DE 19942088
(62) Divisional of application: 00939001.4
(71) Applicant: BASF Aktiengesellschaft, 67056 Ludwigshafen (DE)
(72) Inventor: Pompejus, Markus, Dr., Seoul 140-210 (KR); Kröger, Burkhard, Dr., 67117 Limburgerhof (DE); Schröder, Hartwig, Dr., 69226 Nussloch (DE); Zelder, Oskar, Dr., 67346 Speyer (DE); Haberhauer, Gregor, 67117 Limburgerhof (DE)

(57) **Abstract**

Isolated nucleic acid molecules, designated MCT nucleic acid molecules, which encode novel MCT proteins from *Corynebacterium glutamicum* are described. The invention also provides antisense nucleic acid molecules, recombinant expression vectors containing MCT nucleic acid molecules, and host cells into which the expression vectors have been introduced. The invention still further provides isolated MCT proteins, mutated MCT proteins, fusion proteins, antigenic peptides and methods for the improvement of production of a desired compound from *C. glutamicum* based on genetic engineering of MCT genes in this organism.

## Description

### Related Applications

This application claims priority to prior filed U.S. Provisional Patent Application Serial No. 60/141031, filed June 25, 1999. This application also claims priority to German Patent Application No. 19931454.3, filed July 8, 1999, German Patent Application No. 19931478.0, filed July 8, 1999, German Patent Application No. 19931563.9, filed July 8, 1999, German Patent Application No. 19932122.1, filed July 9, 1999, German Patent Application No. 19932124.8, filed 99709, German Patent Application No. 19932125.6, filed July 9, 1999, German Patent Application No. 19932128.0, filed July 9, 1999, German Patent Application No. 19932180.9, filed July 9, 1999, German Patent Application No. 19932182.5, filed July 9, 1999, German Patent Application No. 19932190.6, filed July 9, 1999, German Patent Application No. 19932191.4, filed July 9, 1999, German Patent Application No. 19932209.0, filed July 9, 1999, German Patent Application No. 19932212.0, filed July 9, 1999, German Patent Application No. 19932227.9, filed July 9, 1999, German Patent Application No. 19932228.7, filed July 9, 1999, German Patent Application No. 19932229.5, filed 99070, German Patent Application No. 19932230.9, filed July 9, 1999, German Patent Application No. 19932927.3, filed July 14, 1999, German Patent Application No. 19933005.0, filed July 14, 1999, German Patent Application No. 19933006.9, filed July 14,1999, German Patent Application No. 19940764.9, filed August 27, 1999, German Patent Application No. 19940765.7, filed August 27, 1999, German Patent Application No. 19940766.5, filed August 27, 1999, German Patent Application No. 19940830.0, filed August 27, 1999, German Patent Application No. 19940831.9, filed August 27, 1999, German Patent Application No. 19940832.7, filed August 27, 1999, German Patent Application No. 19940833.5, filed August 27, 1999, German Patent Application No. 19941378.9 filed August 31, 1999, German Patent Application No. 19941379.7, filed August 31, 1999, German Patent Application No. 19941395.9, filed August 31, 1999, German Patent Application No. 19942077.7, filed September 3, 1999, German Patent Application No. 19942078.5, filed September 3, 1999, German Patent Application No. 19942079.3, filed September 3, 1999, and German Patent Application No. 19942088.2, filed September 3, 1999. The entire contents of all of the above referenced applications are hereby expressly incorporated herein by this reference.

### Background of the Invention

Certain products and by-products of naturally-occurring metabolic processes in cells have utility in a wide array of industries, including the food, feed, cosmetics, and pharmaceutical industries. These molecules, collectively termed 'fine chemicals', include organic acids, both proteinogenic and non-proteinogenic amino acids, nucleotides and nucleosides, lipids and fatty acids, diols, carbohydrates, aromatic compounds, vitamins and cofactors, and enzymes. Their production is most conveniently performed through the large-scale culture of bacteria developed to produce and secrete large quantities of one or more desired molecules. One particularly useful organism for this purpose is *Corynebacterium glutamicum,* a gram positive, nonpathogenic bacterium. Through strain selection, a number of mutant strains have been developed which produce an array of desirable compounds. However, selection of strains improved for the production of a particular molecule is a time-consuming and difficult process.

### Summary of the Invention

The invention provides novel bacterial nucleic acid molecules which have a variety of uses. These uses include the identification of microorganisms which can be used to produce fine chemicals, the modulation of fine chemical production in *C*. *glutamicum* or related bacteria, the typing or identification of *C*. *glutamicum* or related bacteria, as reference points for mapping the *C*. *glutamicum* genome, and as markers for transformation. These novel nucleic acid molecules encode proteins, referred to herein as membrane construction and membrane transport (MCT) proteins.

*C. glutamicum* is a gram positive, aerobic bacterium which is commonly used in industry for the large-scale production of a variety of fine chemicals, and also for the degradation of hydrocarbons (such as in petroleum spills) and for the oxidation of terpenoids. The MCT nucleic acid molecules of the invention, therefore, can be used to identify microorganisms which can be used to produce fine chemicals, e.g., by fermentation processes. Modulation of the expression of the MCT nucleic acids of the invention, or modification of the sequence of the MCT nucleic acid molecules of the invention, can be used to modulate the production of one or more fine chemicals from a microorganism (*e*.*g*., to improve the yield or production of one or more fine chemicals from a Corynebacterium or Brevibacterium species).

The MCT nucleic acids of the invention may also be used to identify an organism as being *Corynebacterium glutamicum* or a close relative thereof, or to identify the presence of *C*. *glutamicum* or a relative thereof in a mixed population of microorganisms. The invention provides the nucleic acid sequences of a number of *C*. *glutamicum* genes; by probing the extracted genomic DNA ofa culture of a unique or mixed population of microorganisms under stringent conditions with a probe spanning a region of a C. *glutamicum* gene which is unique to this organism, one can ascertain whether this organism is present. Although *Corynebacterium glutamicum* itself is nonpathogenic, it is related to species pathogenic in humans, such as *Corynebacterium diphtheriae* (the causative agent of diphtheria); the detection of such organisms is of significant clinical relevance.

The MCT nucleic acid molecules of the invention may also serve as reference points for mapping of the *C*. *glutamicum* genome, or of genomes of related organisms. Similarly, these molecules, or variants or portions thereof, may serve as markers for genetically engineered Corynebacterium or Brevibacterium species.

The MCT proteins encoded by the novel nucleic acid molecules of the invention are capable of, for example, performing a function involved in the metabolism (*e*.*g*., the biosynthesis or degradation) of compounds necessary for membrane biosynthesis, or of assisting in the transmembrane transport of one or more compounds either into or out of the cell. Given the availability of cloning vectors for use in *Corynebacterium glutamicum,* such as those disclosed in Sinskey *et al*., U.S. Patent No. 4,649,119, and techniques for genetic manipulation of *C*. *glutamicum* and the related *Brevibacterium* species (*e*.*g*., *lactofermentum)* (Yoshihama et al, *J. Bacteriol.* 162: 591-597 (1985); Katsumata *et al*., *J. Bacteriol.* 159: 306-311 (1984); and Santamaria *et al*., *J. Gen. Microbiol.* 130: 2237-2246 (1984)), the nucleic acid molecules of the invention may be utilized in the genetic engineering of this organism to make it a better or more efficient producer of one or more fine chemicals. This improved production or efficiency of production of a fine chemical may be due to a direct effect of manipulation of a gene of the invention, or it may be due to an indirect effect of such manipulation.

There are a number of mechanisms by which the alteration of an MCT protein of the invention may directly affect the yield, production, and/or efficiency of production of a fine chemical from a *C*. *glutamicum* strain incorporating such an altered protein. Those MCT proteins involved in the export of fine chemical molecules from the cell may be increased in number or activity such that greater quantities of these compounds are secreted to the extracellular medium, from which they are more readily recovered. Similarly, those MCT proteins involved in the import of nutrients necessary for the biosynthesis of one or more fine chemicals (*e*.g., phosphate, sulfate, nitrogen compounds, etc.) may be increased in number or activity such that these precursors, cofactors, or intermediate compounds are increased in concentration within the cell. Further, fatty acids and lipids themselves are desirable fine chemicals; by optimizing the activity or increasing the number of one or more MCT proteins of the invention which participate in the biosynthesis of these compounds, or by impairing the activity of one or more MCT proteins which are involved in the degradation of these compounds, it may be possible to increase the yield, production, and/or efficiency of production of fatty acid and lipid molecules from *C. glutamicum.*

The mutagenesis of one or more MCT genes of the invention may also result in MCT proteins having altered activities which indirectly impact the production of one or more desired fine chemicals from *C. glutamicum.* For example, MCT proteins of the invention involved in the export of waste products may be increased in number or activity such that the normal metabolic wastes of the cell (possibly increased in quantity due to the overproduction of the desired fine chemical) are efficiently exported before they are able to damage nucleotides and proteins within the cell (which would decrease the viability of the cell) or to interfere with fine chemical biosynthetic pathways (which would decrease the yield, production, or efficiency of production of the desired fine chemical). Further, the relatively large intracellular quantities of the desired fine chemical may in itself be toxic to the cell, so by increasing the activity or number of transporters able to export this compound from the cell, one may increase the viability of the cell in culture, in turn leading to a greater number of cells in the culture producing the desired fine chemical. The MCT proteins of the invention may also be manipulated such that the relative amounts of different lipid and fatty acid molecules are produced. This may have a profound effect on the lipid composition of the membrane of the cell. Since each type of lipid has different physical properties, an alteration in the lipid composition of a membrane may significantly alter membrane fluidity. Changes in membrane fluidity can impact the transport of molecules across the membrane, as well as the integrity of the cell, both of which have a profound effect on the production of fine chemicals from *C*. *glutamicum* in large-scale fermentative culture.

The invention provides novel nucleic acid molecules which encode proteins, referred to herein as MCT proteins, which are capable of, for example, participating in the metabolism of compounds necessary for the construction of cellular membranes in *C. glutamicum,* or in the transport of molecules across these membranes. Nucleic acid molecules encoding an MCT protein are referred to herein as MCT nucleic acid molecules. In a preferred embodiment, the MCT protein participates in the metabolism of compounds necessary for the construction of cellular membranes in *C*. *glutamicum,* or in the transport of molecules across these membranes. Examples of such proteins include those encoded by the genes set forth in Table 1.

Accordingly, one aspect of the invention pertains to isolated nucleic acid molecules (e.g., cDNAs, DNAs, or RNAs) comprising a nucleotide sequence encoding an MCT protein or biologically active portions thereof, as well as nucleic acid fragments suitable as primers or hybridization probes for the detection or amplification of MCT-encoding nucleic acid (e.g., DNA or mRNA). In particularly preferred embodiments, the isolated nucleic acid molecule comprises one of the nucleotide sequences set forth as the odd-numbered SEQ ID NOs in the Sequence Listing (*e*.*g*., SEQ ID NO:1, SEQ ID NO:3, SEQ ID NO:5, SEQ ID NO:7 .... ), forth as the odd-numbered SEQ ID NOs in the Sequence Listing (*e*.*g*., SEQ ID NO:1, SEQ ID NO:3, SEQ ID NO:5, SEQ ID NO:7....), or the coding region or a complement thereof of one of these nucleotide sequences. In other particularly preferred embodiments, the isolated nucleic acid molecule of the invention comprises a nucleotide sequence which hybridizes to or is at least about 50%, preferably at least about 60%, more preferably at least about 70%, 80% or 90%, and even more preferably at least about 95%, 96%, 97%, 98%, 99% or more homologous to a nucleotide sequence set forth as an odd-numbered SEQ ID NO in the Sequence Listing (*e*.*g*., SEQ ID NO:1, SEQ ID NO:3, SEQ ID NO:5, SEQ ID NO:7 .... ), or a portion thereof. In other preferred embodiments, the isolated nucleic acid molecule encodes one of the amino acid sequences set forth as an even-numbered SEQ ID NO in the Sequence Listing (*e*.*g*., SEQ ID NO:2, SEQ ID NO:4, SEQ ID NO:6, SEQ ID NO:8...). The preferred MCT proteins of the present invention also preferably possess at least one of the MCT activities described herein.

In another embodiment, the isolated nucleic acid molecule encodes a protein or portion thereof wherein the protein or portion thereof includes an amino acid sequence which is sufficiently homologous to an amino acid sequence of the invention (*e*.*g*., a sequence having an even-numbered SEQ ID NO: in the Sequence Listing), *e.g.,* sufficiently homologous to an amino acid sequence of the invention such that the protein or portion thereof maintains an MCT activity. Preferably; the protein or portion thereof encoded by the nucleic acid molecule maintains.the ability to participate in the metabolism of compounds necessary for the construction of cellular membranes in *C*. *glutamicum,* or in the transport of molecules across these membranes. In one embodiment, the protein encoded by the nucleic acid molecule is at least about 50%, preferably at least about 60%, and more preferably at least about 70%, 80%, or 90% and most preferably at least about 95%, 96%, 97%, 98%, or 99% or more homologous to an amino acid sequence of the invention (*e*.*g*., an entire amino acid sequence selected from those having an even-numbered SEQ ID NO in the Sequence Listing). In another preferred embodiment, the protein is a full length *C. glutamicum* protein which is substantially homologous to an entire amino acid sequence of the invention (encoded by an open reading frame shown in the corresponding odd-numbered SEQ ID NOs in the Sequence Listing (*e*.*g*., SEQ ID NO:1, SEQ ID NO:3, SEQ ID NO:5, SEQ ID NO:7....).

In another preferred embodiment, the isolated nucleic acid molecule is derived from *C. glutamicum* and encodes a protein (*e*.*g*., an MCT fusion protein) which includes a biologically active domain which is at least about 50% or more homologous to one of the amino acid sequences of the invention (e.g., a sequence of one of the even-numbered SEQ ID NOs in the Sequence Listing) and is able to participate in the metabolism of compounds necessary for the construction of cellular membranes in *C*. *glutamicum,* or in the transport of molecules across these membranes, or has one or more of the activities set forth in Table 1, and which also includes heterologous nucleic acid sequences encoding a heterologous polypeptide or regulatory regions.

In another embodiment, the isolated nucleic acid molecule is at least 15 nucleotides in length and hybridizes under stringent conditions to a nucleic acid molecule comprising a nucleotide sequence of the invention (*e*.*g*., a sequence of an odd-numbered SEQ ID NO in the Sequence Listing). Preferably, the isolated nucleic acid molecule corresponds to a naturally-occurring nucleic acid molecule. More preferably, the isolated nucleic acid encodes a naturally-occurring *C*. *glutamicum* MCT protein, or a biologically active portion thereof.

Another aspect of the invention pertains to vectors, e.g., recombinant expression vectors, containing the nucleic acid molecules of the invention, and host cells into which such vectors have been introduced. In one embodiment, such a host cell is used to produce an MCT protein by culturing the host cell in a suitable medium. The MCT protein can be then isolated from the medium or the host cell.

Yet another aspect of the invention pertains to a genetically altered microorganism in which an MCT gene has been introduced or altered. In one embodiment, the genome of the microorganism has been altered by introduction of a nucleic acid molecule of the invention encoding wild-type or mutated MCT sequence as a transgene. In another embodiment, an endogenous MCT gene within the genome of the microorganism has been altered, *e*.*g*., functionally disrupted, by homologous recombination with an altered MCT gene. In another embodiment, an endogenous or introduced MCT gene in a microorganism has been altered by one or more point mutations, deletions, or inversions, but still encodes a functional MCT protein. In still another embodiment, one or more of the regulatory regions (*e*.*g*., a promoter, repressor, or inducer) of an MCT gene in a microorganism has been altered (*e*.*g*., by deletion, truncation, inversion, or point mutation) such that the expression of the MCT gene is modulated. In a preferred embodiment, the microorganism belongs to the genus *Corynebacterium* or *Brevibacterium,* with *Corynebacterium glutamicum* being particularly preferred. In a preferred embodiment, the microorganism is also utilized for the production of a desired compound, such as an amino acid, with lysine being particularly preferred.

In another aspect, the invention provides a method of identifying the presence or activity of *Cornyebacterium diphtheriae* in a subject. This method includes detection of one or more of the nucleic acid or amino acid sequences of the invention (*e*.*g*., the sequences set forth in the Sequence Listing as SEQ ID NOs 1 through 676) in a subject, thereby detecting the presence or activity *of Corynebacterium diphtheriae* in the subject.

Still another aspect of the invention pertains to an isolated MCT protein or a portion, e.g., a biologically active portion, thereof. In a preferred embodiment, the isolated MCT protein or portion thereof can participate in the metabolism of compounds necessary for the construction of cellular membranes in *C*. *glutamicum,* or in the transport of molecules across these membranes. In another preferred embodiment, the isolated MCT protein or portion thereof is sufficiently homologous to an amino acid sequence of the invention (*e*.*g*., a sequence of an even-numbered SEQ ID NO: in the Sequence Listing) such that the protein or portion thereof maintains the ability to participate in the metabolism of compounds necessary for the construction of cellular membranes in *C*. *glutamicum,* or in the transport of molecules across these membranes.

The invention also provides an isolated preparation of an MCT protein. In preferred embodiments, the MCT protein comprises an amino acid sequence of the invention (e.g., a sequence of an even-numbered SEQ ID NO: of the Sequence Listing). In another preferred embodiment, the invention pertains to an isolated full length protein which is substantially homologous to an entire amino acid sequence of the invention *(e.g.,* a sequence of an even-numbered SEQ ID NO: of the Sequence Listing) (encoded by an open reading frame set forth in a corresponding odd-numbered SEQ ID NO: of the Sequence Listing A). In yet another embodiment, the protein is at least about 50%, preferably at least about 60%, and more preferably at least about 70%, 80%, or 90%, and most preferably at least about 95%, 96%, 97%, 98%, or 99% or more homologous to an entire amino acid sequence of the invention (*e*.*g*., a sequence of an even-numbered SEQ ID NO: of the Sequence Listing). In other embodiments, the isolated MCT protein comprises an amino acid sequence which is at least about 50% or more homologous to one of the amino acid sequences of the invention (*e*.*g*., a sequence of an even-numbered SEQ ID NO: of the Sequence Listing) and is able to participate in the metabolism of compounds necessary for the construction of cellular membranes in *C*. *glutamicum,* or in the transport of molecules across these membranes, or has one or more of the activities set forth in Table 1.

Alternatively, the isolated MCT protein can comprise an amino acid sequence which is encoded by a nucleotide sequence which hybridizes, *e*.*g*., hybridizes under stringent conditions, or is at least about 50%, preferably at least about 60%, more preferably at least about 70%, 80%, or 90%, and even more preferably at least about 95%, 96%, 97%, 98,%, or 99% or more homologous, to a nucleotide sequence of one of the even-numbered SEQ ID NOs set forth in the Sequence Listing. It is also preferred that the preferred forms of MCT proteins also have one or more of the MCT bioactivities described herein.

The MCT polypeptide, or a biologically active portion thereof, can be operatively linked to a non-MCT polypeptide to form a fusion protein. In preferred embodiments, this fusion protein has an activity which differs from that of the MCT protein alone. In other preferred embodiments, this fusion protein participate in the metabolism of compounds necessary for the construction of cellular membranes in *C*. *glutamicum,* or in the transport of molecules across these membranes. In particularly preferred embodiments, integration of this fusion protein into a host cell modulates production of a desired compound from the cell.

In another aspect, the invention provides methods for screening molecules which modulate the activity of an MCT protein, either by interacting with the protein itself or a substrate or binding partner of the MCT protein, or by modulating the transcription or translation of an MCT nucleic acid molecule of the invention.

Another aspect of the invention pertains to a method for producing a fine chemical. This method involves the culturing of a cell containing a vector directing the expression of an MCT nucleic acid molecule of the invention, such that a fine chemical is produced. In a preferred embodiment, this method further includes the step of obtaining a cell containing such a vector, in which a cell is transfected with a vector directing the expression of an MCT nucleic acid. In another preferred embodiment, this method further includes the step of recovering the fine chemical from the culture. In a particularly preferred embodiment, the cell is from the genus *Corynebacterium* or *Brevibacterium,* or is selected from those strains set forth in Table 3.

Another aspect of the invention pertains to methods for modulating production of a molecule from a microorganism. Such methods include contacting the cell with an agent which modulates MCT protein activity or MCT nucleic acid expression such that a cell associated activity is altered relative to this same activity in the absence of the agent. In a preferred embodiment, the cell is modulated for one or more *C. glutamicum* metabolic pathways for cell membrane components or is modulated for the transport of compounds across such membranes, such that the yields or rate of production of a desired fine chemical by this microorganism is improved. The agent which modulates MCT protein activity can be an agent which stimulates MCT protein activity or MCT nucleic acid expression. Examples of agents which stimulate MCT protein activity or MCT nucleic acid expression include small molecules, active MCT proteins, and nucleic acids encoding MCT proteins that have been introduced into the cell. Examples of agents which inhibit MCT activity or expression include small molecules and antisense MCT nucleic acid molecules.

Another aspect of the invention pertains to methods for modulating yields of a desired compound from a cell, involving the introduction of a wild-type or mutant MCT gene into a cell, either maintained on a separate plasmid or integrated into the genome of the host cell. If integrated into the genome, such integration can be random, or it can take place by homologous recombination such that the native gene is replaced by the introduced copy, causing the production of the desired compound from the cell to be modulated. In a preferred embodiment, said yields are increased. In another preferred embodiment, said chemical is a fine chemical. In a particularly preferred embodiment, said fine chemical is an amino acid. In especially preferred embodiments, said amino acid is L-lysine.

### Detailed Description of the Invention

The present invention provides MCT nucleic acid and protein molecules which are involved in the metabolism of cellular membrane components in *C*. glutamicum or in the transport of compounds across such membranes. The molecules of the invention may be utilized in the modulation of production of fine chemicals from microorganisms, such as *C. glutamicum,* either directly (*e*.*g*., where overexpression or optimization of a fatty acid biosynthesis protein has a direct impact on the yield, production, and/or efficiency of production of the fatty acid from modified *C*. *glutamicum*)*,* or may have an indirect impact which nonetheless results in an increase of yield, production, and/or efficiency of production of the desired compound (*e*.*g*., where modulation of the metabolism of cell membrane components results in alterations in the yield, production, and/or efficiency of production or the composition of the cell membrane, which in turn may impact the production of one or more fine chemicals). Aspects of the invention are further explicated below.

### 1. Fine Chemicals

The term 'fine chemical' is art-recognized and includes molecules produced by an organism which have applications in various industries, such as, but not limited to, the pharmaceutical, agriculture, and cosmetics industries. Such compounds include organic acids, such as tartaric acid, itaconic acid, and diaminopimelic acid, both proteinogenic and non-proteinogenic amino acids, purine and pyrimidine bases, nucleosides, and nucleotides (as described e.g. in Kuninaka, A. (1996) Nucleotides and related compounds, p. 561-612, in Biotechnology vol. 6, Rehm *et al*., eds. VCH: Weinheim, and references contained therein), lipids, both saturated and unsaturated fatty acids (*e*.*g*., arachidonic acid), diols (*e*.*g*., propane diol, and butane diol), carbohydrates (*e*.*g*., hyaluronic acid and trehalose), aromatic compounds (*e*.*g*., aromatic amines, vanillin, and indigo), vitamins and cofactors (as described in Ullmann's Encyclopedia of Industrial Chemistry, vol. A27, "Vitamins", p. 443-613 (1996) VCH: Weinheim and references therein; and Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press, (1995)), enzymes, polyketides (Cane *et al*. (1998) *Science* 282: 63-68), and all other chemicals described in Gutcho (1983) Chemicals by Fermentation, Noyes Data Corporation, ISBN: 0818805086 and references therein. The metabolism and uses of certain of these fine chemicals are further explicated below.

### A. Amino Acid Metabolism and Uses

Amino acids comprise the basic structural units of all proteins, and as such are essential for normal cellular functioning in all organisms. The term "amino acid" is art-recognized. The proteinogenic amino acids, of which there are 20 species, serve as structural units for proteins, in which they are linked by peptide bonds, while the nonproteinogenic amino acids (hundreds of which are known) are not normally found in proteins (see Ulmann's Encyclopedia of Industrial Chemistry, vol. A2, p. 57-97 VCH: Weinheim (1985)). Amino acids may be in the D- or L- optical configuration, though L-amino acids are generally the only type found in naturally-occurring proteins. Biosynthetic and degradative pathways of each of the 20 proteinogenic amino acids have been well characterized in both prokaryotic and eukaryotic cells (see, for example, Stryer, L. Biochemistry, 3^{rd} edition, pages 578-590 (1988)). The 'essential' amino acids (histidine, isoleucine, leucine, lysine, methionine, phenylalanine, threonine, tryptophan, and valine), so named because they are generally a nutritional requirement due to the complexity of their biosyntheses, are readily converted by simple biosynthetic pathways to the remaining I 'nonessential' amino acids (alanine, arginine, asparagine, aspartate, cysteine, glutamate, glutamine, glycine, proline, serine, and tyrosine). Higher animals do retain the ability to synthesize some of these amino acids, but the essential amino acids must be supplied from the diet in order for normal protein synthesis to occur.

Aside from their function in protein biosynthesis, these amino acids are interesting chemicals in their own right, and many have been found to have various applications in the food, feed, chemical, cosmetics, agriculture, and pharmaceutical industries. Lysine is an important amino acid in the nutrition not only of humans, but also of monogastric animals such as poultry and swine. Glutamate is most commonly used as a flavor additive (mono-sodium glutamate, MSG) and is widely used throughout the food industry, as are aspartate, phenylalanine, glycine, and cysteine. Glycine, L-methionine and tryptophan are all utilized in the pharmaceutical industry. Glutamine, valine, leucine, isoleucine, histidine, arginine, proline, serine and alanine are of use in both the pharmaceutical and cosmetics industries. Threonine, tryptophan, and D/ L-methionine are common feed additives. (Leuchtenberger, W. (1996) Amino aids - technical production and use, p. 466-502 in Rehm *et al*. (eds.) Biotechnology vol. 6, chapter 14a, VCH: Weinheim). Additionally, these amino acids have been found to be useful as precursors for the synthesis of synthetic amino acids and proteins, such as N-acetylcysteine, S-carboxymethyl-L-cysteine, (S)-5-hydroxytryptophan, and others described in Ulmann's Encyclopedia of Industrial Chemistry, vol. A2, p. 57-97, VCH: Weinheim, 1985.

The biosynthesis of these natural amino acids in organisms capable of producing them, such as bacteria, has been well characterized (for review of bacterial amino acid biosynthesis and regulation thereof, see Umbarger, H.E.(1978) *Ann*. *Rev. Biochem.* 47: 533-606). Glutamate is synthesized by the reductive amination of α-ketoglutarate, an intermediate in the citric acid cycle. Glutamine, proline, and arginine are each subsequently produced from glutamate. The biosynthesis of serine is a three-step process beginning with 3-phosphoglycerate (an intermediate in glycolysis), and resulting in this amino acid after oxidation, transamination, and hydrolysis steps. Both cysteine and glycine are produced from serine; the former by the condensation of homocysteine with serine, and the latter by the transferal of the side-chain β-carbon atom to tetrahydrofolate, in a reaction catalyzed by serine transhydroxymethylase. Phenylalanine, and tyrosine are synthesized from the glycolytic and pentose phosphate pathway precursors erythrose 4-phosphate and phosphoenolpyruvate in a 9-step biosynthetic pathway that differ only at the final two steps after synthesis of prephenate. Tryptophan is also produced from these two initial molecules, but its synthesis is an 11-step pathway. Tyrosine may also be synthesized from phenylalanine, in a reaction catalyzed by phenylalanine hydroxylase. Alanine, valine, and leucine are all biosynthetic products of pyruvate, the final product of glycolysis. Aspartate is formed from oxaloacetate, an intermediate of the citric acid cycle. Asparagine, methionine, threonine, and lysine are each produced by the conversion of aspartate. Isoleucine is formed from threonine. A complex 9-step pathway results in the production of histidine from 5-phosphoribosyl-1-pyrophosphate, an activated sugar.

Amino acids in excess of the protein synthesis needs of the cell cannot be stored, and are instead degraded to provide intermediates for the major metabolic pathways of the cell (for review see Stryer, L. Biochemistry 3^{rd} ed. Ch. 21 "Amino Acid Degradation and the Urea Cycle" p. 495-516 (1988)). Although the cell is able to convert unwanted amino acids into useful metabolic intermediates, amino acid production is costly in terms of energy, precursor molecules, and the enzymes necessary to synthesize them. Thus it is not surprising that amino acid biosynthesis is regulated by feedback inhibition, in which the presence of a particular amino acid serves to slow or entirely stop its own production (for overview of feedback mechanisms in amino acid biosynthetic pathways, see Stryer, L. Biochemistry, 3^{rd} ed. Ch. 24: "Biosynthesis of Amino Acids and Heme" p. 575-600 (1988)). Thus, the output of any particular amino acid is limited by the amount of that amino acid present in the cell.

### B. Vitamin, Cofactor, and Nutraceutical Metabolism and Uses

Vitamins, cofactors, and nutraceuticals comprise another group of molecules which the higher animals have lost the ability to synthesize and so must ingest, although they are readily synthesized by other organisms such as bacteria. These molecules are either bioactive substances themselves, or are precursors of biologically active substances which may serve as electron carriers or intermediates in a variety of metabolic pathways. Aside from their nutritive value, these compounds also have significant industrial value as coloring agents, antioxidants, and catalysts or other processing aids. (For an overview of the structure, activity, and industrial applications of these compounds, see, for example, Ullman's Encyclopedia of Industrial Chemistry, "Vitamins" vol. A27, p. 443-613, VCH: Weinheim, 1996.) The term "vitamin" is art-recognized, and includes nutrients which are required by an organism for normal functioning, but which that organism cannot synthesize by itself. The group of vitamins may encompass cofactors and nutraceutical compounds. The language "cofactor" includes nonproteinaceous compounds required for a normal enzymatic activity to occur. Such compounds may be organic or inorganic; the cofactor molecules of the invention are preferably organic. The term "nutraceutical" includes dietary supplements having health benefits in plants and animals, particularly humans. Examples of such molecules are vitamins, antioxidants, and also certain lipids (*e*.*g*., polyunsaturated fatty acids).

The biosynthesis of these molecules in organisms capable of producing them, such as bacteria, has been largely characterized (Ullman's Encyclopedia of Industrial Chemistry, "Vitamins" vol. A27, p. 443-613, VCH: Weinheim, 1996; Michal, G. (1999) Biochemical Pathways: An Atlas of Biochemistry and Molecular Biology, John Wiley & Sons; Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press: Champaign, IL X, 374 S).

Thiamin (vitamin B₁) is produced by the chemical coupling of pyrimidine and thiazole moieties. Riboflavin (vitamin B₂) is synthesized from guanosine-5'-triphosphate (GTP) and ribose-5'-phosphate. Riboflavin, in turn, is utilized for the synthesis of flavin mononucleotide (FMN) and flavin adenine dinucleotide (FAD). The family of compounds collectively termed 'vitamin B₆' (*e*.*g*., pyridoxine, pyridoxamine, pyridoxa-5'-phosphate, and the commercially used pyridoxin hydrochloride) are all derivatives of the common structural unit, 5-hydroxy-6-methylpyridine. Pantothenate (pantothenic acid, (R)-(+)-N-(2,4-dihydroxy-3,3-dimethyl-1-oxobutyl)-β-alanine) can be produced either by chemical synthesis or by fermentation. The final steps in pantothenate biosynthesis consist of the ATP-driven condensation of β-alanine and pantoic acid. The enzymes responsible for the biosynthesis steps for the conversion to pantoic acid, to β-alanine and for the condensation to panthotenic acid are known. The metabolically active form of pantothenate is Coenzyme A, for which the biosynthesis proceeds in 5 enzymatic steps. Pantothenate, pyridoxal-5'-phosphate, cysteine and ATP are the precursors of Coenzyme A. These enzymes not only catalyze the formation of panthothante, but also the production of (R)-pantoic acid, (R)-pantolacton, (R)-panthenol (provitamin B₅), pantetheine (and its derivatives) and coenzyme A.

Biotin biosynthesis from the precursor molecule pimeloyl-CoA in microorganisms has been studied in detail and several of the genes involved have been identified. Many of the corresponding proteins have been found to also be involved in Fe-cluster synthesis and are members of the nifS class of proteins. Lipoic acid is derived from octanoic acid, and serves as a coenzyme in energy metabolism, where it becomes part of the pyruvate dehydrogenase complex and the α-ketoglutarate dehydrogenase complex. The folates are a group of substances which are all derivatives of folic acid, which is turn is derived from L-glutamic acid, p-amino-benzoic acid and 6-methylpterin. The biosynthesis of folic acid and its derivatives, starting from the metabolism intermediates guanosine-5'-triphosphate (GTP), L-glutamic acid and p-amino-benzoic acid has been studied in detail in certain microorganisms.

Corrinoids (such as the cobalamines and particularly vitamin B₁₂) and porphyrines belong to a group of chemicals characterized by a tetrapyrole ring system. The biosynthesis of vitamin B₁₂ is sufficiently complex that it has not yet been completely characterized, but many of the enzymes and substrates involved are now known. Nicotinic acid (nicotinate), and nicotinamide are pyridine derivatives which are also termed 'niacin'. Niacin is the precursor of the important coenzymes NAD (nicotinamide adenine dinucleotide) and NADP (nicotinamide adenine dinucleotide phosphate) and their reduced forms.

The large-scale production of these compounds has largely relied on cell-free chemical syntheses, though some of these chemicals have also been produced by large-scale culture of microorganisms, such as riboflavin, Vitamin B₆, pantothenate, and biotin. Only Vitamin B₁₂ is produced solely by fermentation, due to the complexity of its synthesis. *In vitro* methodologies require significant inputs of materials and time, often at great cost.

### C. Purine, Pyrimidine, Nucleoside and Nucleotide Metabolism and Uses

Purine and pyrimidine metabolism genes and their corresponding proteins are important targets for the therapy of tumor diseases and viral infections. The language "purine" or "pyrimidine" includes the nitrogenous bases which are constituents of nucleic acids, co-enzymes, and nucleotides. The term "nucleotide" includes the basic structural units of nucleic acid molecules, which are comprised of a nitrogenous base, a pentose sugar (in the case of RNA, the sugar is ribose; in the case of DNA, the sugar is D-deoxyribose), and phosphoric acid. The language "nucleoside" includes molecules which serve as precursors to nucleotides, but which are lacking the phosphoric acid moiety that nucleotides possess. By inhibiting the biosynthesis of these molecules, or their mobilization to form nucleic acid molecules, it is possible to inhibit RNA and DNA synthesis; by inhibiting this activity in a fashion targeted to cancerous cells, the ability of tumor cells to divide and replicate may be inhibited. Additionally, there are nucleotides which do not form nucleic acid molecules, but rather serve as energy stores (*i*.*e*., AMP) or as coenzymes (*i*.*e*., FAD and NAD).

Several publications have described the use of these chemicals for these medical indications, by influencing purine and/or pyrimidine metabolism (e.g. Christopherson, R.I. and Lyons, S.D. (1990) "Potent inhibitors of *de novo* pyrimidine and purine biosynthesis as chemotherapeutic agents." *Med. Res. Reviews* 10: 505-548). Studies of enzymes involved in purine and pyrimidine metabolism have been focused on the development of new drugs which can be used, for example, as immunosuppressants or anti-proliferants (Smith, J.L., (1995) "Enzymes in nucleotide synthesis." *Curr. Opin*. *Struct. Biol.* 5: 752-757; (1995) *Biochem Soc. Transact.* 23: 877-902). However, purine and pyrimidine bases, nucleosides and nucleotides have other utilities: as intermediates in the biosynthesis of several fine chemicals (*e*.*g*., thiamine, S-adenosyl-methionine, folates, or riboflavin), as energy carriers for the cell *(e.g.,* ATP or GTP), and for chemicals themselves, commonly used as flavor enhancers (*e*.*g*., IMP or GMP) or for several medicinal applications (see, for example, Kuninaka, A. (1996) Nucleotides and Related Compounds in Biotechnology vol. 6, Rehm *et al*., eds. VCH: Weinheim, p. 561-612). Also, enzymes involved in purine, pyrimidine, nucleoside, or nucleotide metabolism are increasingly serving as targets against which chemicals for crop protection, including fungicides, herbicides and insecticides, are developed.

The metabolism of these compounds in bacteria has been characterized (for reviews see, for example, Zalkin, H. and Dixon, J.E. (1992) *"de novo* purine nucleotide biosynthesis", in: Progress in Nucleic Acid Research and Molecular Biology, vol. 42, Academic Press:, p. 259-287; and Michal, G. (1999) "Nucleotides and Nucleosides", Chapter 8 in: Biochemical Pathways: An Atlas of Biochemistry and Molecular Biology, Wiley: New York). Purine metabolism has been the subject of intensive research, and is essential to the normal functioning of the cell. Impaired purine metabolism in higher animals can cause severe disease, such as gout. Purine nucleotides are synthesized from ribose-5-phosphate, in a series of steps through the intermediate compound inosine-5'-phosphate (IMP), resulting in the production of guanosine-5'-monophosphate (GMP) or adenosine-5'-monophosphate (AMP), from which the triphosphate forms utilized as nucleotides are readily formed. These compounds are also utilized as energy stores, so their degradation provides energy for many different biochemical processes in the cell. Pyrimidine biosynthesis proceeds by the formation of uridine-5'-monophosphate (UMP) from ribose-5-phosphate. UMP, in turn, is converted to cytidine-5'-triphosphate (CTP). The deoxy- forms of all of these nucleotides are produced in a one step reduction reaction from the diphosphate ribose form of the nucleotide to the diphosphate deoxyribose form of the nucleotide. Upon phosphorylation, these molecules are able to participate in DNA synthesis.

### D. Trehalose Metabolism and Uses

Trehalose consists of two glucose molecules, bound in α, α-1,1 linkage. It is commonly used in the food industry as a sweetener, an additive for dried or frozen foods, and in beverages. However, it also has applications in the pharmaceutical, cosmetics and biotechnology industries (see, for example, Nishimoto *et al*., (1998) U.S. Patent No. 5,759,610; Singer, MA. and Lindquist, S. (1998) *Trends Biotech.* 16: 460-467; Paiva, C.L.A. and Panek, A.D. (1996) *Biotech. Ann*. *Rev.* 2: 293-314; and Shiosaka, M. (1997) J. Japan 172: 97-102). Trehalose is produced by enzymes from many microorganisms and is naturally released into the surrounding medium, from which it can be collected using methods known in the art.

### II. Membrane Biosynthesis and Transmembrane Transport

Cellular membranes serve a variety of functions in a cell. First and foremost, a membrane differentiates the contents of a cell from the surrounding environment, thus giving integrity to the cell. Membranes may also serve as barriers to the influx of hazardous or unwanted compounds, and also to the efflux of desired compounds. Cellular membranes are by nature impervious to the unfacilitated diffusion of hydrophilic compounds such as proteins, water molecules and ions due to their structure: a bilayer of lipid molecules in which the polar head groups face outwards (towards the exterior and interior of the cell, respectively) and the nonpolar tails face inwards at the center of the bilayer, forming a hydrophobic core (for a general review of membrane structure and function, see Gennis, R.B. (1989) Biomembranes, Molecular Structure and Function, Springer: Heidelberg). This barrier enables cells to maintain a relatively higher concentration of desired compounds and a relatively lower concentration of undesired compounds than are contained within the surrounding medium, since the diffusion of these compounds is effectively blocked by the membrane. However, the membrane also presents an effective barrier to the import of desired compounds and the export of waste molecules. To overcome this difficulty, cellular membranes incorporate many kinds of transporter proteins which are able to facilitate the transmembrane transport of different kinds of compounds. There are two general classes of these transport proteins: pores or channels and transporters. The former are integral membrane proteins, sometimes complexes of proteins, which form a regulated hole through the membrane. This regulation, or 'gating' is generally specific to the molecules to be transported by the pore or channel, rendering these transmembrane constructs selectively permeable to a specific class of substrates; for example, a potassium channel is constructed such that only ions having a like charge and size to that of potassium may pass through. Channel and pore proteins tend to have discrete hydrophobic and hydrophilic domains, such that the hydrophobic face of the protein may associate with the interior of the membrane while the hydrophilic face lines the interior of the channel, thus providing a sheltered hydrophilic environment through which the selected hydrophilic molecule may pass. Many such pores/channels are known in the art, including those for potassium, calcium, sodium, and chloride ions.

This pore and channel-mediated system of facilitated diffusion is limited to very small molecules, such as ions, because pores or channels large enough to permit the passage of whole proteins by facilitated diffusion would be unable to prevent the passage of smaller hydrophilic molecules as well. Transport of molecules by this process is sometimes termed 'facilitated diffusion' since the driving force of a concentration gradient is required for the transport to occur. Permeases also permit facilitated diffusion of larger molecules, such as glucose or other sugars, into the cell when the concentration of these molecules on one side of the membrane is greater than that on the other (also called 'uniport'). In contrast to pores or channels, these integral membrane proteins (often having between 6-14 membrane-spanning α-helices) do not form open channels through the membrane, but rather bind to the target molecule at the surface of the membrane and then undergo a conformational shift such that the target molecule is released on the opposite side of the membrane.

However, cells frequently require the import or export of molecules against the existing concentration gradient ('active transport'), a situation in which facilitated diffusion cannot occur. There are two general mechanisms used by cells for such membrane transport: symport or antiport, and energy-coupled transport such as that mediated by the ABC transporters. Symport and antiport systems couple the movement of two different molecules across the membrane (via pertneases having two separate binding sites for the two different molecules); in symport, both molecules are transported in the same direction, while in antiport, one molecule is imported while the other is exported. This is possible energetically because one of the two molecules moves in accordance with a concentration gradient, and this energetically favorable event is permitted only upon concomitant movement of a desired compound against the prevailing concentration gradient. Single molecules may be transported across the membrane against the concentration gradient in an energy-driven process, such as that utilized by the ABC transporters. In this system, the transport protein located in the membrane has an ATP-binding cassette; upon binding of the target molecule, the ATP is converted to ADP + Pi, and the resulting release of energy is used to drive the movement of the target molecule to the opposite face of the membrane, facilitated by the transporter. For more detailed descriptions of all of these transport systems, see: Bamberg, E. *et al*., (1993) "Charge transport of ion pumps on lipid bilayer membranes", *Q*. *Rev. Biophys.* 26: 1-25; Findlay, J.B.C. (1991) "Structure and function in membrane transport systems", *Curr. Opin. Struct. Biol.* 1:804-810; Higgins, C.F. (1992) "ABC transporters from microorganisms to man", *Ann. Rev. Cell Biol.* 8: 67-113; Gennis, R.B. (1989) "Pores, Channels and Transporters", in: Biomembranes, Molecular Structure and *Function*, Springer: Heidelberg, p. 270-322; and Nikaido, H. and Saier, H. (1992) "Transport proteins in bacteria: common themes in their design", *Science* 258: 936-942, and references contained within each of these references.

The synthesis of membranes is a well-characterized process involving a number of components, the most important of which are lipid molecules. Lipid synthesis may be divided into two parts: the synthesis of fatty acids and their attachment to sn-glycerol-3-phosphate, and the addition or modification of a polar head group. Typical lipids utilized in bacterial membranes include phospholipids, glycolipids, sphingolipids, and phosphoglycerides. Fatty acid synthesis begins with the conversion of acetyl CoA either to malonyl CoA by acetyl CoA carboxylase, or to acetyl-ACP by acetyltransacylase. Following a condensation reaction, these two product molecules together form acetoacetyl-ACP, which is converted by a series of condensation, reduction and dehydration reactions to yield a saturated fatty acid molecule having a desired chain length. The production of unsaturated fatty acids from such molecules is catalyzed by specific desaturases either aerobically, with the help of molecular oxygen, or anaerobically (for reference on fatty acid synthesis, see F.C. Neidhardt *et al*. (1996) E. coli and Salmonella. ASM Press: Washington, D.C., p. 612-636 and references contained therein; Lengeler *et al*. (eds) (1999) Biology of Procaryotes. Thieme: Stuttgart, New York, and references contained therein; and Magnuson, K. *et al*., (1993) *Microbiological Reviews* 57: 522-542, and references contained therein). The cyclopropane fatty acids (CFA) are synthesized by a specific CFA-synthase using SAM as a cosubstrate. Branched chain fatty acids are synthesized from branched chain amino acids that are deaminated to yield branched chain 2-oxo-acids (see Lengeler *et al*., eds. (1999) Biology of Procaryotes. Thieme: Stuttgart, New York, and references contained therein). Another essential step in lipid synthesis is the transfer of fatty acids onto the polar head groups by, for example, glycerol-phosphate-acyltransferases. The combination of various precursor molecules and biosynthetic enzymes results in the production of different fatty acid molecules, which has a profound effect on the composition of the membrane.

### III. Elements and Methods of the Invention

The present invention is based, at least in part, on the discovery of novel molecules, referred to herein as MCT nucleic acid and protein molecules, which control the production of cellular membranes in *C. glutamicum* and govern the movement of molecules across such membranes. In one embodiment, the MCT molecules participate in the metabolism of compounds necessary for the construction of cellular membranes in *C. glutamicum,* or in the transport of molecules across these membranes. In a preferred embodiment, the activity of the MCT molecules of the present invention to regulate membrane component production and membrane transport has an impact on the production of a desired fine chemical by this organism. In a particularly preferred embodiment, the MCT molecules of the invention are modulated in activity, such that the *C glutamicum* metabolic pathways which the MCT proteins of the invention regulate are modulated in yield, production, and/or efficiency of production and the transport of compounds through the membranes is altered in efficiency, which either directly or indirectly modulates the yield, production, and/or efficiency of production of a desired fine chemical by *C. glutamicum.*

The language, "MCT protein" or "MCT polypeptide" includes proteins which participate in the metabolism of compounds necessary for the construction of cellular membranes in *C. glutamicum,* or in the transport of molecules across these membranes. Examples of MCT proteins include those encoded by the MCT genes set forth in Table 1 and by the odd-numbered SEQ ID NOs. The terms "MCT gene" or "MCT nucleic acid sequence" include nucleic acid sequences encoding an MCT protein, which consist of a coding region and also corresponding untranslated 5' and 3' sequence regions. Examples of MCT genes include those set forth in Table 1. The terms "production" or "productivity" are art-recognized and include the concentration of the fermentation product (for example, the desired fine chemical) formed within a given time and a given fermentation volume (*e*.*g*., kg product per hour per liter). The term "efficiency of production" includes the time required for a particular level of production to be achieved (for example, how long it takes for the cell to attain a particular rate of output of a fine chemical). The term "yield" or "product/carbon yield" is art-recognized and includes the efficiency of the conversion of the carbon source into the product (*i*.*e*., fine chemical). This is generally written as, for example, kg product per kg carbon source. By increasing the yield or production of the compound, the quantity of recovered molecules, or of useful recovered molecules of that compound in a given amount of culture over a given amount of time is increased. The terms "biosynthesis" or a "biosynthetic pathway" are art-recognized and include the synthesis of a compound, preferably an organic compound, by a cell from intermediate compounds in what may be a multistep and highly regulated process. The terms "degradation" or a "degradation pathway" are art-recognized and include the breakdown of a compound, preferably an organic compound, by a cell to degradation products (generally speaking, smaller or less complex molecules) in what may be a multistep and highly regulated process. The language "metabolism" is art-recognized and includes the totality of the biochemical reactions that take place in an organism. The metabolism of a particular compound, then, (*e*.*g*., the metabolism of an amino acid such as glycine) comprises the overall biosynthetic, modification, and degradation pathways in the cell related to this compound.

In another embodiment, the MCT molecules of the invention are capable of modulating the production of a desired molecule, such as a fine chemical, in a microorganism such as *C. glutamicum.* There are a number of mechanisms by which the alteration of an MCT protein of the invention may directly affect the yield, production, and/or efficiency of production of a fine chemical from a *C glutamicum* strain incorporating such an altered protein. Those MCT proteins involved in the export of fine chemical molecules from the cell may be increased in number or activity such that greater quantities of these compounds are secreted to the extracellular medium, from which they are more readily recovered. Similarly, those MCT proteins involved in the import of nutrients necessary for the biosynthesis of one or more fine chemicals (*e*.*g*., phosphate, sulfate, nitrogen compounds, etc.) may be increased in number or activity such that these precursor, cofactor, or intermediate compounds are increased in concentration within the cell. Further, fatty acids and lipids themselves are desirable fine chemicals; by optimizing the activity or increasing the number of one or more MCT proteins of the invention which participate in the biosynthesis of these compounds, or by impairing the activity of one or more MCT proteins which are involved in the degradation of these compounds, it may be possible to increase the yield, production, and/or efficiency of production of fatty acid and lipid molecules from *C*. *glutamicum.*

The mutagenesis of one or more MCT genes of the invention may also result in MCT proteins having altered activities which indirectly impact the production of one or more desired fine chemicals from *C. glutamicum.* For example, MCT proteins of the invention involved in the export of waste products may be increased in number or activity such that the normal metabolic wastes of the cell (possibly increased in quantity due to the overproduction of the desired fine chemical) are efficiently exported before they are able to damage nucleotides and proteins within the cell (which would decrease the viability of the cell) or to interfere with fine chemical biosynthetic pathways (which would decrease the yield, production, or efficiency of production of the desired fine chemical). Further, the relatively large intracellular quantities of the desired fine chemical may in itself be toxic to the cell, so by increasing the activity or number of transporters able to export this compound from the cell, one may increase the viability of the cell in culture, in turn leading to a greater number of cells in the culture producing the desired fine chemical. The MCT proteins of the invention may also be manipulated such that the relative amounts of different lipid and fatty acid molecules are produced. This may have a profound effect on the lipid composition of the membrane of the cell. Since each type of lipid has different physical properties, an alteration in the lipid composition of a membrane may significantly alter membrane fluidity. Changes in membrane fluidity can impact the transport of molecules across the membrane, as well as the integrity of the cell, both of which have a profound effect on the production of fine chemicals from *C. glutamicum* in large-scale fermentative culture.

The isolated nucleic acid sequences of the invention are contained within the genome of a *Corynebacterium glutamicum* strain available through the American Type Culture Collection, given designation ATCC 13032. The nucleotide sequence of the isolated *C. glutamicum* MCT DNAs and the predicted amino acid sequences of the *C*. *glutamicum* MCT proteins are shown in the Sequence Listing as odd-numbered SEQ ID NOs and even-numbered SEQ ID NOs, respectively. Computational analyses were performed which classified and/or identified these nucleotide sequences as sequences which encode proteins involved in the metabolism of cellular membrane components or proteins involved in the transport of compounds across such membranes.

The present invention also pertains to proteins which have an amino acid sequence which is substantially homologous to an amino acid sequence of the invention (*e*.*g*., the sequence of an even-numbered SEQ ID NO of the Sequence Listing).. As used herein, a protein which has an amino acid sequence which is substantially homologous to a selected amino acid sequence is least about 50% homologous to the selected amino acid sequence, e.g., the entire selected amino acid sequence. A protein which has an amino acid sequence which is substantially homologous to a selected amino acid sequence can also be least about 50-60%, preferably at least about 60-70%, and more preferably at least about 70-80%, 80-90%, or 90-95%, and most preferably at least about 96%, 97%, 98%, 99% or more homologous to the selected amino acid sequence.

The MCT protein or a biologically active portion or fragment thereof of the invention can participate in the metabolism of compounds necessary for the construction of cellular membranes in *C*. *glutamicum,* or in the transport of molecules across these membranes, or have one or more of the activities set forth in Table 1.

Various aspects of the invention are described in further detail in the following subsections:

### A. Isolated Nucleic Acid Molecules

One aspect of the invention pertains to isolated nucleic acid molecules that encode MCT polypeptides or biologically active portions thereof, as well as nucleic acid fragments sufficient for use as hybridization probes or primers for the identification or amplification of MCT-encoding nucleic acid (*e*.*g*., MCT DNA). As used herein, the term "nucleic acid molecule" is intended to include DNA molecules (*e*.*g*., cDNA or genomic DNA) and RNA molecules (*e*.*g*., mRNA) and analogs of the DNA or RNA generated using nucleotide analogs. This term also encompasses untranslated sequence located at both the 3' and 5' ends of the coding region of the gene: at least about 100 nucleotides of sequence upstream from the 5' end of the coding region and at least about 20 nucleotides of sequence downstream from the 3'end of the coding region of the gene. The nucleic acid molecule can be single-stranded or double-stranded, but preferably is double-stranded DNA. An "isolated" nucleic acid molecule is one which is separated from other nucleic acid molecules which are present in the natural source of the nucleic acid. Preferably, an "isolated" nucleic acid is free of sequences which naturally flank the nucleic acid (*i*.*e*., sequences located at the 5' and 3' ends of the nucleic acid) in the genomic DNA of the organism from which the nucleic acid is derived. For example, in various embodiments, the isolated MCT nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived (*e*.*g*, a *C. glutamicum* cell). Moreover, an "isolated" nucleic acid molecule, such as a DNA molecule, can be substantially free of other cellular material, or culture medium when produced by recombinant techniques, or chemical precursors or other chemicals when chemically synthesized.

A nucleic acid molecule of the present invention, e.g., a nucleic acid molecule having a nucleotide sequence of an odd-numbered SEQ ID NO of the Sequence Listing, or a portion thereof, can be isolated using standard molecular biology techniques and the sequence information provided herein. For example, a *C*. *glutamicum* MCT DNA can be isolated from a *C*. *glutamicum* library using all or portion of one of the odd-numbered SEQ ID NO sequences of the Sequence Listing as a hybridization probe and standard hybridization techniques (*e*.*g*., as described in Sambrook, J., Fritsh, E. F., and Maniatis, *T. Molecular Cloning: A Laboratory Manual. 2nd*, *ed*, *Cold Spring Harbor Laboratory*, Cold Spring Harbor Laboratory Press, Cold Spring Harbor, NY, 1989). Moreover, a nucleic acid molecule encompassing all or a portion of one of the nucleic acid sequences of the invention (*e*.*g*., an odd-numbered SEQ ID NO:) can be isolated by the polymerase chain reaction using oligonucleotide primers designed based upon this sequence (*e*.*g*., a nucleic acid molecule encompassing all or a portion of one of the nucleic acid sequences of the invention (*e*.*g*., an odd-numbered SEQ ID NO of the Sequence Listing) can be isolated by the polymerase chain reaction using oligonucleotide primers designed based upon this same sequence). For example, mRNA can be isolated from normal endothelial cells (*e*.*g*., by the guanidinium-thiocyanate extraction procedure of Chirgwin *et al*. (1979) *Biochemistry* 18: 5294-5299) and DNA can be prepared using reverse transcriptase (*e*.*g*., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences shown in the Sequence Listing. A nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and
characterized by DNA sequence analysis. Furthermore, oligonucleotides corresponding to an MCT nucleotide sequence can be prepared by standard synthetic techniques, e.g., using an automated DNA synthesizer.

In a preferred embodiment, an isolated nucleic acid molecule of the invention comprises one of the nucleotide sequences shown in the Sequence Listing. The nucleic acid sequences of the invention, as set forth in the Sequence Listing correspond to the *Corynebacterium glutamicum* MCT DNAs of the invention. This DNA comprises sequences encoding MCT proteins (*i*.*e*., the "coding region", indicated in each odd-numbered SEQ ID NO: in the Sequence Listing), as well as 5' untranslated sequences and 3' untranslated sequences, also indicated in each odd-numbered SEQ ID NO: in the Sequence Listing. Alternatively, the nucleic acid molecule can comprise only the coding region of any of the nucleic acid sequences of the Sequence Listing.

For the purposes of this application, it will be understood that each of the nucleic acid and amino acid sequences set forth in the Sequence Listing has an identifying RXA, RXN, RXS, or RXC number having the designation "RXA", "RXN", "RXS" or "RXC" followed by 5 digits (*i*.*e*., RXA02099, RXN03097, RXS00148, or RXC01748). Each of the nucleic acid sequences comprises up to three parts: a 5' upstream region, a coding region, and a downstream region. Each of these three regions is identified by the same RXA, RXN, RXS, or RXC designation to eliminate confusion. The recitation "one of the odd-numbered sequences in of the Sequence Listing", then, refers to any of the nucleic acid sequences in the Sequence Listing, which may also be distinguished by their differing RXA, RXN, RXS, or RXC designations. The coding region of each of these sequences is translated into a corresponding amino acid sequence, which is also set forth in the Sequence Listing, as an even-numbered SEQ ID NO: immediately following the corresponding nucleic acid sequence. For example, the coding region for RXA03097 is set forth in SEQ ID NO:1, while the amino acid sequence which it encodes is set forth as SEQ ID NO:2. The sequences of the nucleic acid molecules of the invention are identified by the same RXA, RXN, RXS, or RXC designations as the amino acid molecules which they encode, such that they can be readily correlated. For example, the amino acid sequences designated RXA02099, RX1V03097, RXS00148, and RXC01748 are translations of the coding region of the nucleotide sequences of nucleic acid molecules RXA02099, RXN03097, RXS00148, and RXC01748, respectively. The correspondence between the RXA, RXN, RXS, and RXC nucleotide and amino acid sequences of the invention and their assigned SEQ ID NOs is set forth in Table 1. For example, as set forth in Table I, the nucleotide sequence of RXA00104 is SEQ ID NO:5, and the amino acid sequence of RXA00104 is SEQ ID NO:6.

Several of the genes of the invention are "F-designated genes". An F-designated gene includes those genes set forth in Table 1 which have an 'F' in front of the RXA, RXN, RXS, or RXC designation. For example, SEQ ID NO:11, designated, as indicated on Table 1, as "F RXA02581 ", is an F-designated gene, as are SEQ ID NOs: 31, 33, and 43 (designated on Table 1 as "F RXA02487", "F RXA02490", and "F RXA02809", respectively).

In one embodiment, the nucleic acid molecules of the present invention are not intended to include those compiled in Table 2. In the case of the dapD gene, a sequence for this gene was published in Wehrmann, A., *et al*. (1998) *J*. *Bacteriol*. 180(12): 3159-3165. However, the sequence obtained by the inventors of the present application is significantly longer than the published version. It is believed that the published version relied on an incorrect start codon, and thus represents only a fragment of the actual coding region.

In another preferred embodiment, an isolated nucleic acid molecule of the invention comprises a nucleic acid molecule which is a complement of one of the nucleotide sequences of the invention (*e*.*g*., a sequence of an odd-numbered SEQ ID NO: of the Sequence Listing), or a portion thereof. A nucleic acid molecule which is complementary to one of the nucleotide sequences shown in of the invention is one which is sufficiently complementary to one of the nucleotide sequences shown in the Sequence Listing (*e*.*g*., the sequence of an odd-numbered SEQ ID NO:)such that it can hybridize to one of the nucleotide sequences of the invention, thereby forming a stable duplex.

In still another preferred embodiment, an isolated nucleic acid molecule of the invention comprises a nucleotide sequence which is at least about 50%, 51 %, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more homologous to a nucleotide sequence of the invention (*e*.*g*., a sequence of an odd-numbered SEQ ID NO: of the Sequence Listing), or a portion thereof. Ranges and identity values intermediate to the above-recited ranges, (*e*.*g*., 70-90% identical or 80-95% identical) are also intended to be encompassed by the present invention. For example, ranges of identity values using a combination of any of the above values recited as upper and/or lower limits are intended to be included. In an additional preferred embodiment, an isolated nucleic acid molecule of the invention comprises a nucleotide sequence which hybridizes, e.g., hybridizes under stringent conditions, to one of the nucleotide sequences of the invention, or a portion thereof.

Moreover, the nucleic acid molecule of the invention can comprise only a portion of the coding region of the sequence of one of the odd-numbered SEQ ID NOs of the Sequence Listing, for example a fragment which can be used as a probe or primer or a fragment encoding a biologically active portion of an MCT protein. The nucleotide sequences determined from the cloning of the MCT genes from *C*. *glutamicum* allows for the generation of probes and primers designed for use in identifying and/or cloning MCT homologues in other cell types and organisms, as well as MCT homologues from other *Corynebacteria* or related species. The probelprimer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of one of the nucleotide sequences of the invention (*e*.*g*., a sequence of one of the odd-numbered SEQ ID NOs of the Sequence Listing), an anti-sense sequence of one of these sequences, or naturally occurring mutants thereof. Primers based on a nucleotide sequence of the invention can be used in PCR reactions to clone MCT homologues. Probes based on the MCT nucleotide sequences can be used to detect transcripts or genomic sequences encoding the same or homologous proteins. In preferred embodiments, the probe further comprises a label group attached thereto, *e*.*g*. the label group can be a radioisotope, a fluorescent compound, an enzyme, or an enzyme co-factor. Such probes can be used as a part of a diagnostic test kit for identifying cells which misexpress an MCT protein, such as by measuring a level of an MCT-encoding nucleic acid in a sample of cells, e.g., detecting MCT mRNA levels or determining whether a genomic MCT gene has been mutated or deleted.

In one embodiment, the nucleic acid molecule of the invention encodes a protein or portion thereof which includes an amino acid sequence which is sufficiently homologous to an amino acid sequence of the invention (*e*.*g*., a sequence of an even-numbered SEQ ID NO of the Sequence Listing) such that the protein or portion thereof maintains the ability to participate in the metabolism of compounds necessary for the construction of cellular membranes in *C*. *glutamicum,* or in the transport of molecules across these membranes. As used herein, the language "sufficiently homologous" refers to proteins or portions thereof which have amino acid sequences which include a minimum number of identical or equivalent (*e*.*g*., an amino acid residue which has a similar side chain as an amino acid residue in a sequence of one of the even-numbered SEQ ID NOs of the Sequence Listing) amino acid residues to an amino acid sequence of the invention such that the protein or portion thereof is able to participate in the metabolism of compounds necessary for the construction of cellular membranes in *C*. *glutamicum*, or in the transport of molecules across these membranes. Protein members of such membrane component metabolic pathways or membrane transport systems, as described herein, may play a role in the production and secretion of one or more fine chemicals. Examples of such activities are also described herein. Thus, "the function of an MCT protein" contributes either directly or indirectly to the yield, production, and/or efficiency of production of one or more fine chemicals. Examples of MCT protein activities are set forth in Table 1.

In another embodiment, the protein is at least about 50-60%, preferably at least about 60-70%, and more preferably at least about 70-80%, 80-90%, 90-95%, and most preferably at least about 96%, 97%, 98%, 99% or more homologous to an entire amino acid sequence of the invention (*e*.*g*., a sequence of an even-numbered SEQ ID NO: of the Sequence Listing).

Portions of proteins encoded by the MCT nucleic acid molecules of the invention are preferably biologically active portions of one of the MCT proteins. As used herein, the term "biologically active portion of an MCT protein" is intended to include a portion, *e*.*g*., a domain/motif, of an MCT protein that participates in the metabolism of compounds necessary for the construction of cellular membranes in *C glutamicum,* or in the transport of molecules across these membranes, or has an activity as set forth in Table 1. To determine whether an MCT protein or a biologically active portion thereof can participate in the metabolism of compounds necessary for the construction of cellular membranes in *C. glutamicum,* or in the transport of molecules across these membranes, an assay of enzymatic activity may be performed. Such assay methods are well known to those of ordinary skill in the art, as detailed in Example 8 of the Exemplification.

Additional nucleic acid fragments encoding biologically active portions of an MCT protein can be prepared by isolating a portion of one of the amino acid sequences of the invention (*e*.*g*., a sequence of an even-numbered SEQ ID NO: of the Sequence Listing), expressing the encoded portion of the MCT protein or peptide (*e*.*g*., by recombinant expression *in vitro)* and assessing the activity of the encoded portion of the MCT protein or peptide.

The invention further encompasses nucleic acid molecules that differ from one of the nucleotide sequences shown of the invention (*e*.*g*., a sequence of an odd-numbered SEQ ID NO: of the Sequence Listing) (and portions thereof) due to degeneracy of the genetic code and thus encode the same MCT protein as that encoded by the nucleotide sequences of the invention. In another embodiment, an isolated nucleic acid molecule of the invention has a nucleotide sequence encoding a protein having an amino acid sequence shown in the Sequence Listing (*e*.*g*., an even-numbered SEQ ID NO:). In a still further embodiment, the nucleic acid molecule of the invention encodes a full length *C*. *glutamicum* protein which is substantially homologous to an amino acid sequence of the invention (encoded by an open reading frame shown in an odd-numbered SEQ ID NO: of the Sequence Listing).

It will be understood by one of ordinary skill in the art that in one embodiment the sequences of the invention are not meant to include the sequences of the prior art, such as those Genbank sequences set forth in Tables 2 or 4 which were available prior to the present invention. In one embodiment, the invention includes nucleotide and amino acid sequences having a percent identity to a nucleotide or amino acid sequence of the invention which is greater than that of a sequence of the prior art (*e*.*g*., a Genbank sequence (or the protein encoded by such a sequence) set forth in Tables 2 or 4). For example, the invention includes a nucleotide sequence which is greater than and/or at least 38% identical to the nucleotide sequence designated RXA01420 (SEQ ID NO:7), a nucleotide sequence which is greater than and/or at least 43% identical to the nucleotide sequence designated RXA00104 (SEQ ID NO:5), and a nucleotide sequence which is greater than and/or at least 45% identical to the nucleotide sequence designated RXA02173 (SEQ ID NO:25). One of ordinary skill in the art would be able to calculate the lower threshold of percent identity for any given sequence of the invention by examining the GAP-calculated percent identity scores set forth in Table 4 for each of the three top hits for the given sequence, and by subtracting the highest GAP-calculated percent identity from 100 percent. One of ordinary skill in the art will also appreciate that nucleic acid and amino acid sequences having percent identities greater than the lower threshold so calculated (*e*.*g*., at least 50%, 51%, 52%, 53%, 54%, 55%, 56%, 57%, 58%, 59%, or 60%, preferably at least about 61 %, 62%, 63%, 64%, 65%, 66%, 67%, 68%, 69%, or 70%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more identical) are also encompassed by the invention.

In addition to the *C*. *glutamicum* MCT nucleotide sequences set forth in the Sequence Listing as odd-numbered SEQ ID NOs, it will be appreciated by one of ordinary skill in the art that DNA sequence polymorphisms that lead to changes in the amino acid sequences of MCT proteins may exist within a population (*e*.*g*., the *C*. *glutomicum* population). Such genetic polymorphism in the MCT gene may exist among individuals within a population due to natural variation. As used herein, the terms "gene" and "recombinant gene" refer to nucleic acid molecules comprising an open reading frame encoding an MCT protein, preferably a *C glutamicum* MCT protein. Such natural variations can typically result in 1-5% variance in the nucleotide sequence of the MCT gene. Any and all such nucleotide variations and resulting amino acid polymorphisms in MCT that are the result of natural variation and that do not alter the functional activity of MCT proteins are intended to be within the scope of the invention.

Nucleic acid molecules corresponding to natural variants and non-*C*. *glutamicum* homologues of the *C. glutamicum* MCT DNA of the invention can be isolated based on their homology to the *C. glutamicum* MCT nucleic acid disclosed herein using the *C*. *glutamicum* DNA, or a portion thereof, as a hybridization probe according to standard hybridization techniques under stringent hybridization conditions. Accordingly, in another embodiment, an isolated nucleic acid molecule of the invention is at least 15 nucleotides in length and hybridizes under stringent conditions to the nucleic acid molecule comprising a nucleotide sequence of an odd-numbered SEQ ID NO: of the Sequence Listing. In other embodiments, the nucleic acid is at least 30, 50, 100, 250 or more nucleotides in length. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 65%, more preferably at least about 70%, and even more preferably at least about 75% or more homologous to each other typically remain hybridized to each other. Such stringent conditions are known to those of ordinary skill in the art and can be found in *Current Protocols in Molecular Biology,* John Wiley & Sons, N.Y. (1989), 6.3.1-63.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to a nucleotide sequence of the invention corresponds to a naturally-occurring nucleic acid molecule. As used herein, a "naturally-occurring" nucleic acid molecule refers to an RNA or DNA molecule having a nucleotide sequence that occurs in nature (*e*.*g*., encodes a natural protein). In one embodiment, the nucleic acid encodes a natural *C*. *glutamicum* MCT protein.

In addition to naturally-occurring variants of the MCT sequence that may exist in the population, one of ordinary skill in the art will further appreciate that changes can be introduced by mutation into a nucleotide sequence of the invention, thereby leading to changes in the amino acid sequence of the encoded MCT protein, without altering the functional ability of the MCT protein. For example, nucleotide substitutions leading to amino acid substitutions at "non-essential" amino acid residues can be made in a nucleotide sequence of the invention. A "non-essential" amino acid residue is a residue that can be altered from the wild-type sequence of one of the MCT proteins (*e*.*g*., an even-numbered SEQ ID NO: of the Sequence Listing) without altering the activity of said MCT protein, whereas an "essential" amino acid residue is required for MCT protein activity. Other amino acid residues, however, (*e*.*g*., those that are not conserved or only semi-conserved in the domain having MCT activity) may not be essential for activity and thus are likely to be amenable to alteration without altering MCT activity.

Accordingly, another aspect of the invention pertains to nucleic acid molecules encoding MCT proteins that contain changes in amino acid residues that are not essential for MCT activity. Such MCT proteins differ in amino acid sequence from a sequence of an even-numbered SEQ ID NO: of the Sequence Listing yet retain at least one of the MCT activities described herein. In one embodiment, the isolated nucleic acid molecule comprises a nucleotide sequence encoding a protein, wherein the protein comprises an amino acid sequence at least about 50% homologous to an amino acid sequence of the invention and is capable of participate in the metabolism of compounds necessary for the construction of cellular membranes in *C*. *glutamicum,* or in the transport of molecules across these membranes, or has one or more activities set forth in Table 1. Preferably, the protein encoded by the nucleic acid molecule is at least about 50-60% homologous to the amino acid sequence of one of the odd-numbered SEQ ID NOs of the Sequence Listing , more preferably at least about 60-70% homologous to one of these sequences, even more preferably at least about 70-80%, 80-90%, 90-95% homologous to one of these sequences, and most preferably at least about 96%, 97%, 98%, or 99% homologous to one of the amino acid sequences of the invention..

To determine the percent homology of two amino acid sequences (*e*.*g*., one of the amino acid sequences of the invention and a mutant form thereof) or of two nucleic acids, the sequences are aligned for optimal comparison purposes (*e*.*g*., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence (*e*.*g*., one of the amino acid sequences of the invention) is occupied by the same amino acid residue or nucleotide as the corresponding position in the other sequence (*e*.*g*., a mutant form of the amino acid sequence), then the molecules are homologous at that position (*i*.*e*., as used herein amino acid or nucleic acid "homology" is equivalent to amino acid or nucleic acid "identity"). The percent homology between the two sequences is a function of the number of identical positions shared by the sequences (*i*.*e*., % homology = # of identical positions/total # of positions x 100).

An isolated nucleic acid molecule encoding an MCT protein homologous to a protein sequence of the invention (*e*.*g*., a sequence of an even-numbered SEQ ID NO: of the Sequence Listing) can be created by introducing one or more nucleotide substitutions, additions or deletions into a nucleotide sequence of the invention such that one or more amino acid substitutions, additions or deletions are introduced into the encoded protein. Mutations can be introduced into one of the nucleotide sequences of the invention by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e*.*g*., lysine, arginine, histidine), acidic side chains (*e*.*g*., aspartic acid, glutamic acid), uncharged polar side chains (*e*.*g*., glycine, asparagine, glutamine, serine, threonine, tyrosine, cysteine), nonpolar side chains (*e*.*g*., alanine, valine, leucine, isoleucine, proline, phenylalanine, methionine, tryptophan), beta-branched side chains (*e*.*g*., threortine, valine, isoleucine) and aromatic side chains *(e.g.,* tyrosine, phenylalanine, tryptophan, histidine). Thus, a predicted nonessential amino acid residue in an MCT protein is preferably replaced with another amino acid residue from the same side chain family. Alternatively, in another embodiment, mutations can be introduced randomly along all or part of an MCT coding sequence, such as by saturation mutagenesis, and the resultant mutants can be screened for an MCT activity described herein to identify mutants that retain MCT activity. Following mutagenesis of the nucleotide sequence of one of the odd-numbered SEQ ID NOs of the Sequence Listing, the encoded protein can be expressed recombinantly and the activity of the protein can be determined using, for example, assays described herein (see Example 8 of the Exemplification).

In addition to the nucleic acid molecules encoding MCT proteins described above, another aspect of the invention pertains to isolated nucleic acid molecules which are antisense thereto. An "antisense" nucleic acid comprises a nucleotide sequence which is complementary to a "sense" nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire MCT coding strand, or to only a portion thereof. In one embodiment, an antisense nucleic acid molecule is antisense to a "coding region" of the coding strand of a nucleotide sequence encoding an MCT protein. The term "coding region" refers to the region of the nucleotide sequence comprising codons which are translated into amino acid residues (*e*.*g*., the entire coding region of SEQ ID NO:5 (RXA00 104) comprises nucleotides 1 to 756). In another embodiment, the antisense nucleic acid molecule is antisense to a "noncoding region" of the coding strand of a nucleotide sequence encoding MCT. The term "noncoding region" refers to 5' and 3' sequences which flank the coding region that are not translated into amino acids (*i*.*e*., also referred to as 5' and 3' untranslated regions).

Given the coding strand sequences encoding MCT disclosed herein (*e*.*g*., the sequences set forth as odd-numbered SEQ ID NOs in the Sequence Listing), antisense nucleic acids of the invention can be designed according to the rules of Watson and Crick base pairing. The antisense nucleic acid molecule can be complementary to the entire coding region of MCT mRNA, but more preferably is an oligonucleotide which is antisense to only a portion of the coding or noncoding region of MCT mRNA. For example, the antisense oligonucleotide can be complementary to the region surrounding the translation start site of MCT mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a cell or generated *in situ* such that they hybridize with or bind to cellular mRNA and/or genomic DNA encoding an MCT protein to thereby inhibit expression of the protein, *e*.*g*., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al*. (1987) *Nucleic Acids. Res.* 15:6625-6641). The antisense nucleic acid molecule can also comprise a 2'-o-methylribonucleotide (Inoue *et al*. (1987) *Nucleic Acids Res.* 15:613I-6148) or a chimeric RNA-DNA analogue (Inoue *et al*. (1987) *FEBS Lett.* 215:327-330).

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (*e*.*g*., hammerhead ribozymes (described in Haselhoffand Gerlach (1988) *Nature* 334:585-591)) can be used to catalytically cleave MCT mRNA transcripts to thereby inhibit translation of MCT mRNA. A ribozyme having specificity for an MCT-encoding nucleic acid can be designed based upon the nucleotide sequence of an MCT DNA disclosed herein (*i*.*e*., SEQ ID NO. 5 (RXA00104). For example, a derivative of a *Tetrahymena* L*-*19 IVS RNA can be constructed in which the nucleotide sequence of the active site is complementary to the nucleotide sequence to be cleaved in an MCT-encoding mRNA. See, *e*.*g*., Cech *et al*. U.S. Patent No. 4,987,071 and Cech *et al*. U.S. Patent No. 5,116,742. Alternatively, MCT mRNA can be used to select a catalytic RNA having a specific ribonuclease activity from a pool of RNA molecules. See, *e*.*g*., Bartel, D. and Szostak, J.W. (1993) *Science* 261:1411-1418.

Alternatively, MCT gene expression can be inhibited by targeting nucleotide sequences complementary to the regulatory region of an MCT nucleotide sequence (*e*.*g*., an MCT promoter and/or enhancers) to form triple helical structures that prevent transcription of an MCT gene in target cells. See generally, Helene, C. (1991) *Anticancer Drug Des.* 6(6):569-84; Helene, C. *et al.* (1992) *Ann. N. Y. Acad Sci*. 660:27-36; and Maher, L.J. (1992) *Bioassays* 14(12):807-15.

### B. Recombinant Expression Vectors and Host Cells

Another aspect of the invention pertains to vectors, preferably expression vectors, containing a nucleic acid encoding an MCT protein (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (*e*.*g*., in an *in vitro* transcription/trartstation system or in a host cell when the vector is introduced into the host cell). The term "regulatory sequence" is intended to include promoters, enhancers and other expression control elements (*e*.*g*., polyadenylation signals). Such regulatory sequences are described, for example, in Goeddel; *Gene Expression Technology: Methods in Enzymology 185,* Academic Press, San Diego, CA (1990). Regulatory sequences include those which direct constitutive expression of a nucleotide sequence in many types of host cell and those which direct expression of the nucleotide sequence only in certain host cells. Preferred regulatory sequences are, for example, promoters such as cos-, tac-, trp-, tet-, trp-tet-, Ipp-, lac-, Ipp-lac-, IacI^{q}-, T7-, T5-, T3-, gal-, trc-, ara-, SP6-, amy, SPO2, λ-P_{R}-or λ P_{L}, which are used preferably in bacteria. Additional regulatory sequences are, for example, promoters from yeasts and fungi, such as ADC1, MFα, AC, P-60, CYC1, GAPDH, TEF, rp28, ADH, promoters from plants such as CaMV/35S, SSU, OCS, lib4, usp, STLS1, B33, nos or ubiquitin- or phaseolin-promoters. It is also possible to use artificial promoters. It will be appreciated by one of ordinary skill in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e*.*g*., MCT proteins, mutant forms of MCT proteins, fusion proteins, etc.).

The recombinant expression vectors of the invention can be designed for expression of MCT proteins in prokaryotic or eukaryotic cells. For example, MCT genes can be expressed in bacterial cells such as *C*. *glutamicum,* insect cells (using baculovirus expression vectors), yeast and other fungal cells (see Romanos, M.A. *et al*. (1992) "Foreign gene expression in yeast: a review", *Yeast* 8: 423-488; van den Hondel, C.A.M.J.J. *et al.* (1991) "Heterologous gene expression in filamentous fungi" in: More Gene Manipulations in Fungi, J.W. Bennet & L.L. Lasure, eds., p. 396-428: Academic Press: San Diego; and van den Hondel, C.A.M.J.J. & Punt, P.J. (1991) "Gene transfer systems and vector development for filamentous fungi, in: Applied Molecular Genetics of Fungi, Peberdy, J.F. *et al*., eds., p. 1-28, Cambridge University Press: Cambridge), algae and multicellular plant cells (see Schmidt, R. and Willmitzer, L. (1988) High efficiency *Agrobacterium tumefaciens* -mediated transformation *of Arabidopsis thaliana* leaf and cotyledon explants" *Plant Cell Rep.:* 583-586), or mammalian cells. Suitable host cells are discussed further in Goeddel, *Gene Expression Technology: Methods in Enzymology* 185, Academic Press, San Diego, CA ( 1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. In one embodiment, the coding sequence of the MCT protein is cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. Recombinant MCT protein unfused to GST can be recovered by cleavage of the fusion protein with thrombin.

Examples of suitable inducible non-fusion *E. coli* expression vectors include pTrc (Amann *et al*., (1988) *Gene* 69:301-315) pLG338, pACYC184, pBR322, pUC18, pUC19, pKC30, pRep4, pHS1, pHS2, pPLc236, pMBL24, pLG200, pUR290, pIN-IIII 13-B1, λgt11, pBdC1, and pET 11d (Studier *et al*., *Gene Expression Technology*: *Methods in Enzymology* 185, Academic Press, San Diego, California (1990) 60-89 ; and Pouwels *et al*., eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21 (DE3) or HMS 174(DE3) from a resident λ prophage harboring a T7 gnl gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmids pUB110, pC194, or pBD214 are suited for transformation of Bacillus species. Several plasmids of use in the transfer of genetic information into Corynebacterium include pHM1519, pBL1, pSA77, or pAJ667 (Pouwels *et al*., eds. (1985) Cloning Vectors. Elsevier: New York IBSN 0 444 904018).

One strategy to maximize recombinant protein expression is to express the protein in a host bacteria with an impaired capacity to proteolytically cleave the recombinant protein (Gottesman, S., *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in the bacterium chosen for expression, such as *C*. *glutamicum* (Wada *et al*. (1992) *Nucleic Acids Res.* 20:2111-2118). Such alteration ofnucleic acid sequences of the invention can be carried out by standard DNA synthesis techniques.

In another embodiment, the MCT protein expression vector is a yeast expression vector. Examples of vectors for expression in yeast S. *cerevisiae* include pYepSec1 (Baldari, *et al*., (1987) *Embo J.* 6:229-234), 2µ, pAG-1, Yep6, Yep13, pEMBLYe23, pMFa (Kurjan and Herskowitz, (1982) *Cell* 30:933-943), pJRY88 (Schultz *et al*., (1987) *Gene* 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Gene transfer systems and vector development for filamentous fungi, in: Applied Molecular Genetics of Fungi, J.F. *Peberdy, et al*., eds., p. 1-28, Cambridge University Press: Cambridge, and Pouwels *et al*., eds. (1985) Cloning Vectors. Elsevier: New York (IBSN 0 444 904018).

Alternatively, the MCT proteins of the invention can be expressed in insect cells using baculovirus expression vectors. Baculovirus vectors available for expression of proteins in cultured insect cells (*e*.*g*., Sf9 cells) include the pAc series (Smith *et al*. (1983) *Mol. Cell Biol*. 3:2156-2165) and the pVL series (Lucklow and Summers (1989) *Virology* 170:31-39).

In another embodiment, the MCT proteins of the invention may be expressed in unicellular plant cells (such as algae) or in plant cells from higher plants (*e*.*g*., the spermatophytes, such as crop plants). Examples of plant expression vectors include those detailed in: Becker, D., Kemper, E., Schell, J. and Masterson, R. (1992) "New plant binary vectors with selectable markers located proximal to the left border", *Plant Mol. Biol.* 20: 1195-1197; and Bevan, M.W. (1984) "Binary *Agrobacterium* vectors for plant transformation", *Nucl*. *Acid Res.* 12: 8711-8721, and include pLGV23, pGHlac+, pBIN19, pAK2004, and pDH51 (Pouwels *et al*., eds. (1985) Cloning Vectors. Elsevier: New York IBSN 0 444 904018).

In yet another embodiment, a nucleic acid of the invention is expressed in mammalian cells using a mammalian expression vector. Examples of mammalian expression vectors include pCDM8 (Seed, B. (1987) *Nature* 329:840) and pMT2PC (Kaufman *et al*. (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989.

In another embodiment, the recombinant mammalian expression vector is capable of directing expression of the nucleic acid preferentially in a particular cell type (*e*.*g*., tissue-specific regulatory elements are used to express the nucleic acid). Tissue-specific regulatory elements are known in the art. Non-limiting examples of suitable tissue-specific promoters include the albumin promoter (liver-specific; Pinkert *et al*. (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji *et al*. (1983) *Cell* 33:729-740; Queen and Baltimore (1983) *Cell 33:741-748),* neuron-specific promoters (*e*.*g*., the neurofilament promoter; Byrne and Ruddle (1989) *PNAS* 86:5473-5477), pancreas-specific promoters (Edlund *et al*. (1985) *Science* 230:912-916), and mammary gland-specific promoters (*e*.*g*., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to MCT mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, *H. et al*., Antisense RNA as a molecular tool for genetic analysis, *Reviews - Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, an MCT protein can be expressed in bacterial cells such as *C. glutamicum,* insect cells, yeast or mammalian cells (such as Chinese hamster ovary cells (CHO) or COS cells). Other suitable host cells are known to one of ordinary skill in the art. Microorganisms related to *Corynebacterium glutamicum* which may be conveniently used as host cells for the nucleic acid and protein molecules of the invention are set forth in Table 3.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (*e*.*g*., linear DNA or RNA (*e*.*g*., a linearized vector or a gene construct alone without a vector) or nucleic acid in the form of a vector (*e*.*g*., a plasmid, phage, phasmid, phagemid, transposon or other DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells can be found in Sambrook, *et al*. (*Molecular Cloning: A Laboratory Manual. 2nd*, *ed*., *Cold Spring Harbor Laboratory,* Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e*.*g*., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding an MCT protein or can be introduced on a separate vector. Cells stably transfected with the introduced nucleic acid can be identified by, for example, drug selection (*e*.*g*., cells that have incorporated the selectable marker gene will survive, while the other cells die).

To create a homologous recombinant microorganism, a vector is prepared which contains at least a portion of an MCT gene into which a deletion, addition or substitution has been introduced to thereby alter, *e*.*g*., functionally disrupt, the MCT gene. Preferably, this MCT gene is a *Corynebacterium glutamicum* MCT gene, but it can be a homologue from a related bacterium or even from a mammalian, yeast, or insect source. In a preferred embodiment, the vector is designed such that, upon homologous recombination, the endogenous MCT gene is functionally disrupted (*i*.*e*., no longer encodes a functional protein; also referred to as a "knock out" vector). Alternatively, the vector can be designed such that, upon homologous recombination, the endogenous MCT gene is mutated or otherwise altered but still encodes functional protein (*e*.*g*., the upstream regulatory region can be altered to thereby alter the expression of the endogenous MCT protein). In the homologous recombination vector, the altered portion of the MCT gene is flanked at its 5' and 3' ends by additional nucleic acid of the MCT gene to allow for homologous recombination to occur between the exogenous MCT gene carried by the vector and an endogenous MCT gene in a microorganism. The additional flanking MCT nucleic acid is of sufficient length for successful homologous recombination with the endogenous gene. Typically, several kilobases of flanking DNA (both at the 5' and 3' ends) are included in the vector (see e.g., Thomas, K.R., and Capecchi, M.R. (1987) Cell 51: 503 for a description of homologous recombination vectors). The vector is introduced into a microorganism (*e*.*g*., by electroporation) and cells in which the introduced MCT gene has homologously recombined with the endogenous MCT gene are selected, using art-known techniques.

In another embodiment, recombinant microorganisms can be produced which contain selected systems which allow for regulated expression of the introduced gene. For example, inclusion of an MCT gene on a vector placing it under control of the lac operon permits expression of the MCT gene only in the presence of IPTG. Such regulatory systems are well known in the art.

In another embodiment, an endogenous MCT gene in a host cell is disrupted (*e*.*g*., by homologous recombination or other genetic means known in the art) such that expression of its protein product does not occur. In another embodiment, an endogenous or introduced MCT gene in a host cell has been altered by one or more point mutations, deletions, or inversions, but still encodes a functional MCT protein. In still another embodiment, one or more of the regulatory regions (*e*.*g*., a promoter, repressor, or inducer) of an MCT gene in a microorganism has been altered (*e*.*g*., by deletion, truncation, inversion, or point mutation) such that the expression of the MCT gene is modulated. One of ordinary skill in the art will appreciate that host cells containing more than one of the described MCT gene and protein modifications may be readily produced using the methods of the invention, and are meant to be included in the present invention.

A host cell of the invention, such as a prokaryotic or eukaryotic host cell in culture, can be used to produce (*i*.*e*., express) an MCT protein. Accordingly, the invention further provides methods for producing MCT proteins using the host cells of the invention. In one embodiment, the method comprises culturing the host cell of invention (into which a recombinant expression vector encoding an MCT protein has been introduced, or into which genome has been introduced a gene encoding a wild-type or altered MCT protein) in a suitable medium until MCT protein is produced. In another embodiment, the method further comprises isolating MCT proteins from the medium or the host cell.

### C. Isolated MCT Proteins

Another aspect of the invention pertains to isolated MCT proteins, and biologically active portions thereof. An "isolated" or "purified" protein or biologically active portion thereof is substantially free of cellular material when produced by recombinant DNA techniques, or chemical precursors or other chemicals when chemically synthesized. The language "substantially free of cellular material" includes preparations of MCT protein in which the protein is separated from cellular components of the cells in which it is naturally or recombinantly produced. In one embodiment, the language "substantially free of cellular material" includes preparations of MCT protein having less than about 30% (by dry weight) of non-MCT protein (also referred to herein as a "contaminating protein"), more preferably less than about 20% of non-MCT protein, still more preferably less than about 10% of non-MCT protein, and most preferably less than about 5% non-MCT protein. When the MCT protein or biologically active portion thereof is recombinantly produced, it is also preferably substantially free of culture medium, *i*.*e*., culture medium represents less than about 20%, more preferably less than about 10%, and most preferably less than about 5% of the volume of the protein preparation. The language "substantially free of chemical precursors or other chemicals" includes preparations of MCT protein in which the protein is separated from chemical precursors or other chemicals which are involved in the synthesis of the protein. In one embodiment, the language "substantially free of chemical precursors or other chemicals" includes preparations of MCT protein having less than about 30% (by dry weight) of chemical precursors or non-MCT chemicals, more preferably less than about 20% chemical precursors or non-MCT chemicals, still more preferably less than about 10% chemical precursors or non-MCT chemicals, and most preferably less than about 5% chemical precursors or non-MCT chemicals. In preferred embodiments, isolated proteins or biologically active portions thereof lack contaminating proteins from the same organism from which the MCT protein is derived. Typically, such proteins are produced by recombinant expression of, for example, a *C. glutamicum* MCT protein in a microorganism such as *C*. *glutamicum.*

An isolated MCT protein or a portion thereof of the invention can participate in the metabolism of compounds necessary for the construction of cellular membranes in *C. glutamicum,* or in the transport of molecules across these membranes, or has one or more of the activities set forth in Table 1. In preferred embodiments, the protein or portion thereof comprises an amino acid sequence which is sufficiently homologous to an amino acid sequence of the invention (*e*.*g*., a sequence of an even-numbered SEQ ID NO: of the Sequence Listing) such that the protein or portion thereof maintains the ability participate in the metabolism of compounds necessary for the construction of cellular membranes in C *glutamicum,* or in the transport of molecules across these membranes. The portion of the protein is preferably a biologically active portion as described herein. In another preferred embodiment, an MCT protein of the invention has an amino acid sequence set forth as an even-numbered SEQ ID NO: of the Sequence Listing.. In yet another preferred embodiment, the MCT protein has an amino acid sequence which is encoded by a nucleotide sequence which hybridizes, e.g., hybridizes under stringent conditions, to a nucleotide sequence of the invention (*e*.*g*., a sequence of an odd-numbered SEQ ID NO: of the Sequence Listing). In still another preferred embodiment, the MCT protein has an amino acid sequence which is encoded by a nucleotide sequence that is at least about 50%, 51%, 52%, 53%, 54%, 55%, 56%, 57%, 58%, 59%, or 60%, preferably at least about 61%, 62%, 63%, 64%, 65%, 66%, 67%, 58%, 69%, or 70%, more preferably at least about 71 %, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more homologous to one of the nucleic acid sequences of the invention, or a portion thereof. Ranges and identity values intermediate to the above-recited values, (*e*.*g*., 70-90% identical or 80-95% identical) are also intended to be encompassed by the present invention. For example, ranges of identity values using a combination of any of the above values recited as upper and/or lower limits are intended to be included. The preferred MCT proteins of the present invention also preferably possess at least one of the MCT activities described herein. For example, a preferred MCT protein of the present invention includes an amino acid sequence encoded by a nucleotide sequence which hybridizes, e.g., hybridizes under stringent conditions, to a nucleotide sequence of the invention, and which can participate in the metabolism of compounds necessary for the construction of cellular membranes in *C. glutamicum,* or in the transport of molecules across these membranes, or which has one or more of the activities set forth in Table 1.

In other embodiments, the MCT protein is substantially homologous to an amino acid sequence of the invention (*e*.*g*., a sequence of an even-numbered SEQ ID NO: of the Sequence Listing) and retains the functional activity of the protein of one of the amino acid sequences of the invention yet differs in amino acid sequence due to natural variation or mutagenesis, as described in detail in subsection I above. Accordingly, in another embodiment, the MCT protein is a protein which comprises an amino acid sequence which is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81 %, 82%, 83%, 84%, 85%, 86%, 87%, 88%, 89%, or 90%, or 91%, 92%, 93%, 94%, and even more preferably at least about 95%, 96%, 97%, 98%, 99% or more homologous to an entire amino acid sequence of the invention and which has at least one of the MCT activities described herein. Ranges and identity values intermediate to the above-recited values, (*e*.*g*., 70-90% identical or 80-95% identical) are also intended to be encompassed by the present invention. For example, ranges of identity values using a combination of any of the above values recited as upper and/or lower limits are intended to be included. In another embodiment, the invention pertains to a full length *C*. *glutamicum* protein which is substantially homologous to an entire amino acid sequence of the invention.

Biologically active portions of an MCT protein include peptides comprising amino acid sequences derived from the amino acid sequence of an MCT protein, *e*.*g*., the an amino acid sequence of an even-numbered SEQ ID NO: of the Sequence Listing or the amino acid sequence of a protein homologous to an MCT protein, which include fewer amino acids than a full length MCT protein or the full length protein which is homologous to an MCT protein, and exhibit at least one activity of an MCT protein. Typically, biologically active portions (peptides, e.g., peptides which are, for example, 5, 10, 15, 20, 30, 35, 36, 37, 38, 39, 40, 50, 100 or more amino acids in length) comprise a domain or motif with at least one activity of an MCT protein. Moreover, other biologically active portions, in which other regions of the protein are deleted, can be prepared by recombinant techniques and evaluated for one or more of the activities described herein. Preferably, the biologically active portions of an MCT protein include one or more selected domains/motifs or portions thereof having biological activity.

MCT proteins are preferably produced by recombinant DNA techniques. For example, a nucleic acid molecule encoding the protein is cloned into an expression vector (as described above), the expression vector is introduced into a host cell (as described above) and the MCT protein is expressed in the host cell. The MCT protein can then be isolated from the cells by an appropriate purification scheme using standard protein purification techniques. Alternative to recombinant expression, an MCT protein, polypeptide, or peptide can be synthesized chemically using standard peptide synthesis techniques. Moreover, native MCT protein can be isolated from cells (*e*.*g*., endothelial cells), for example using an anti-MCT antibody, which can be produced by standard techniques utilizing an MCT protein or fragment thereof of this invention.

The invention also provides MCT chimeric or fusion proteins. As used herein, an MCT "chimeric protein" or "fusion protein" comprises an MCT polypeptide operatively linked to a non-MCT polypeptide. An "MCT polypeptide" refers to a polypeptide having an amino acid sequence corresponding to an MCT protein, whereas a "non-MCT polypeptide" refers to a polypeptide having an amino acid sequence corresponding to a protein which is not substantially homologous to the MCT protein, *e.g.,* a protein which is different from the MCT protein and which is derived from the same or a different organism. Within the fusion protein, the term "operatively linked" is intended to indicate that the MCT polypeptide and the non-MCT polypeptide are fused in-frame to each other. The non-MCT polypeptide can be fused to the N-terminus or C-terminus of the MCT polypeptide. For example, in one embodiment the fusion protein is a GST-MCT fusion protein in which the MCT sequences are fused to the C-terminus of the GST sequences. Such fusion proteins can facilitate the purification of recombinant MCT proteins. In another embodiment, the fusion protein is an MCT protein containing a heterologous signal sequence at its N-terminus. In certain host cells (*e*.*g*., mammalian host cells), expression and/or secretion of an MCT protein can be increased through use of a heterologous signal sequence.

Preferably, an MCT chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al*. John Wiley & Sons: 1992). Moreover, many expression vectors are commercially available that already encode a fusion moiety *(e.g.,* a GST polypeptide). An MCT-encoding nucleic acid can be cloned into such an expression vector such that the fusion moiety is linked in-frame to the MCT protein.

Homologues of the MCT protein can be generated by mutagenesis, *e*.*g*., discrete point mutation or truncation of the MCT protein. As used herein, the term "homologue" refers to a variant form of the MCT protein which acts as an agonist or antagonist of the activity of the MCT protein. An agonist of the MCT protein can retain substantially the same, or a subset, of the biological activities of the MCT protein. An antagonist of the MCT protein can inhibit one or more of the activities of the naturally occurring form of the MCT protein, by, for example, competitively binding to a downstream or upstream member of the cell membrane component metabolic cascade which includes the MCT protein, or by binding to an MCT protein which mediates transport of compounds across such membranes, thereby preventing translocation from taking place.

In an alternative embodiment, homologues of the MCT protein can be identified by screening combinatorial libraries of mutants, e.g., truncation mutants, of the MCT protein for MCT protein agonist or antagonist activity. In one embodiment, a variegated library of MCT variants is generated by combinatorial mutagenesis at the nucleic acid level and is encoded by a variegated gene library. A variegated library of MCT variants can be produced by, for example, enzymatically ligating a mixture of synthetic oligonucleotides into gene sequences such that a degenerate set of potential MCT sequences is expressible as individual polypeptides, or alternatively, as a set of larger fusion proteins (*e*.*g*., for phage display) containing the set of MCT sequences therein. There are a variety of methods which can be used to produce libraries of potential MCT homologues from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential MCT sequences. Methods for synthesizing degenerate oligonucleotides are known in the art (see, *e.g.,* Narang, S.A. (1983) *Tetrahedron* 39:3; Itakura *et al*. (1984) *Annu. Rev. Biochem.* 53:323; Itakura *et al.* (1984) *Science* 198:1056; Ike *et al*. (1983) *Nucleic Acid Res.* 11:477.

In addition, libraries of fragments of the MCT protein coding can be used to generate a variegated population of MCT fragments for screening and subsequent selection of homologues of an MCT protein. In one embodiment, a library of coding sequence fragments can be generated by treating a double stranded PCR fragment of an MCT coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the MCT protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of MCT homologues. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique which enhances the frequency of functional mutants in the libraries, can be used in combination with the screening assays to identify MCT homologues (Arkin and Yourvan (1992) *PNAS* 89:7811-7815; Delgrave *et al*. (1993) *Protein Engineering* 6(3):327-331).

In another embodiment, cell based assays can be exploited to analyze a variegated MCT library, using methods well known in the art.

### D. Uses and Methods of the Invention

The nucleic acid molecules, proteins, protein homologues, fusion proteins, primers, vectors, and host cells described herein can be used in one or more of the following methods: identification of *C*. *glutamicum* and related organisms; mapping of genomes of organisms related to *C. glutamicum;* identification and localization of *C*. *glulamicum* sequences of interest; evolutionary studies; determination of MCT protein regions required for function; modulation of an MCT protein activity; modulation of the metabolism of one or more cell membrane components; modulation of the transmembrane transport of one or more compounds; and modulation of cellular production of a desired compound, such as a fine chemical.

The MCT nucleic acid molecules of the invention have a variety of uses. First, they may be used to identify an organism as being *Corynebacterium glutamicum* or a close relative thereof. Also, they may be used to identify the presence of *C*. *glulamicum* or a relative thereof in a mixed population of microorganisms. The invention provides the nucleic acid sequences of a number of *C. glutamicum* genes; by probing the extracted genomic DNA of a culture of a unique or mixed population of microorganisms under stringent conditions with a probe spanning a region of a *C*. *glutamicum* gene which is unique to this organism, one can ascertain whether this organism is present.

Although *Corynebacterium glutamicum* itself is nonpathogenic, it is related to pathogenic species, such as *Corynebacterium diphtheriae. Corynebacterium diphtheriae* is the causative agent of diphtheria, a rapidly developing, acute, febrile infection which involves both local and systemic pathology. In this disease, a local lesion develops in the upper respiratory tract and involves necrotic injury to epithelial cells; the bacilli secrete toxin which is disseminated through this lesion to distal susceptible tissues of the body. Degenerative changes brought about by the inhibition of protein synthesis in these tissues, which include heart, muscle, peripheral nerves, adrenals, kidneys, liver and spleen, result in the systemic pathology of the disease. Diphtheria continues to have high incidence in many parts of the world, including Africa, Asia, Eastern Europe and the independent states of the former Soviet Union. An ongoing epidemic of diphtheria in the latter two regions has resulted in at least 5,000 deaths since 1990.

In one embodiment, the invention provides a method of identifying the presence or activity of *Correyebacterium diphtheriae* in a subject. This method includes detection of one or more of the nucleic acid or amino acid sequences of the invention *(e.g.,* the sequences set forth as odd-numbered or even-numbered SEQ ID NOs, respectively, in the Sequence Listing) in a subject, thereby detecting the presence or activity of *Corynebacterium diphtheriae* in the subject. *C*. *glutamicum* and *C*. *diphtheriae* are related bacteria, and many of the nucleic acid and protein molecules in *C*. *glutamicum* are homologous to *C*. *diphtheriae* nucleic acid and protein molecules, and can therefore be used to detect *C*. *diphtheriae* in a subject.

The nucleic acid and protein molecules of the invention may also serve as markers for specific regions of the genome. This has utility not only in the mapping of the genome, but also for functional studies of *C*. *glutamicum* proteins. For example, to identify the region of the genome to which a particular *C*. *glutamicum* DNA-binding protein binds, the *C*. *glutamicum* genome could be digested, and the fragments incubated with the DNA-binding protein. Those which bind the protein may be additionally probed with the nucleic acid molecules of the invention, preferably with readily detectable labels; binding of such a nucleic acid molecule to the genome fragment enables the localization of the fragment to the genome map of *C*. *glutamicum,* and, when performed multiple times with different enzymes, facilitates a rapid determination of the nucleic acid sequence to which the protein binds. Further, the nucleic acid molecules of the invention may be sufficiently homologous to the sequences of related species such that these nucleic acid molecules may serve as markers for the construction of a genomic map in related bacteria, such as *Brevibacterium lactofermentum.*

The MCT nucleic acid molecules of the invention are also useful for evolutionary and protein structural studies. The metabolic and transport processes in which the molecules of the invention participate are utilized by a wide variety of prokaryotic and eukaryotic cells; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Manipulation of the MCT nucleic acid molecules of the invention may result in the production of MCT proteins having functional differences from the wild-type MCT proteins. These proteins may be improved in efficiency or activity, may be present in greater numbers in the cell than is usual, or may be decreased in efficiency or activity.

The invention provides methods for screening molecules which modulate the activity of an MCT protein, either by interacting with the protein itself or a substrate or binding partner of the MCT protein, or by modulating the transcription or translation of an MCT nucleic acid molecule of the invention. In such methods, a microorganism expressing one or more MCT proteins of the invention is contacted with one or more test compounds, and the effect of each test compound on the activity or level of expression of the MCT protein is assessed.

There are a number of mechanisms by which the alteration of an MCT protein of the invention may directly affect the yield, production, and/or efficiency of production of a fine chemical from a *C glutamicum* strain incorporating such an altered protein. Recovery of fine chemical compounds from large-scale cultures of *C*. *glutamicum* is significantly improved if *C*. *glutamicum* secretes the desired compounds, since such compounds may be readily purified from the culture medium (as opposed to extracted from the mass of *C*. *glutamicum* cells). By either increasing the number or the activity of transporter molecules which export fine chemicals from the cell, it may be possible to increase the amount of the produced fine chemical which is present in the extracellular medium, thus permitting greater ease of harvesting and purification. Conversely, in order to efficiently overproduce one or more fine chemicals, increased amounts of the cofactors, precursor molecules, and intermediate compounds for the appropriate biosynthetic pathways are required. Therefore, by increasing the number and/or activity of transporter proteins involved in the import of nutrients, such as carbon sources (*i*.*e*., sugars), nitrogen sources (*i*.*e*., amino acids, ammonium salts), phosphate, and sulfur, it may be possible to improve the production of a fine chemical, due to the removal of any nutrient supply limitations on the biosynthetic process. Further, fatty acids and lipids are themselves desirable. fine chemicals, so by optimizing the activity or increasing the number of one or more MCT proteins of the invention which participate in the biosynthesis of these compounds, or by impairing the activity of one or more MCT proteins which are involved in the degradation of these compounds, it may be possible to increase the yield, production, and/or efficiency of production of fatty acid and lipid molecules from *C. glutamicum.*

The engineering of one or more MCT genes of the invention may also result in MCT proteins having altered activities which indirectly impact the production of one or more desired fine chemicals from *C. glutamicum.* For example, the normal biochemical processes of metabolism result in the production of a variety of waste products (*e*.*g*., hydrogen peroxide and other reactive oxygen species) which may actively interfere with these same metabolic processes (for example, peroxynitrite is known to nitrate tyrosine side chains, thereby inactivating some enzymes having tyrosine in the active site (Groves, J.T. (1999) *Curr. Opin. Chem. Biol.* 3(2): 226-235). While these waste products are typically excreted, the *C. glutamicum* strains utilized for large-scale fermentative production are optimized for the overproduction of one or more fine chemicals, and thus may produce more waste products than is typical for a wild-type *C*. *glutamicum.* By optimizing the activity of one or more MCT proteins of the invention which are involved in the export of waste molecules, it may be possible to improve the viability of the cell and to maintain efficient metabolic activity. Also, the presence of high intracellular levels of the desired fine chemical may actually be toxic to the cell, so by increasing the ability of the cell to secrete these compounds, one may improve the viability of the cell.

Further, the MCT proteins of the invention may be manipulated such that the relative amounts of various lipid and fatty acid molecules produced are altered. This may have a profound effect on the lipid composition of the membrane of the cell. Since each type of lipid has different physical properties, an alteration in the lipid composition of a membrane may significantly alter membrane fluidity. Changes in membrane fluidity can impact the transport of molecules across the membrane, which, as previously explicated, may modify the export of waste products or the produced fine chemical or the import of necessary nutrients. Such membrane fluidity changes may also profoundly affect the integrity of the cell; cells with relatively weaker membranes are more vulnerable in the large-scale fermentor environment to mechanical stresses which may damage or kill the cell. By manipulating MCT proteins involved in the production of fatty acids and lipids for membrane construction such that the resulting membrane has a membrane composition more amenable to the environmental conditions extant in the cultures utilized to produce fine chemicals, a greater proportion of the *C. glutamicum* cells should survive and multiply. Greater numbers of *C*. *glutamicum* cells in a culture should translate into greater yields, production, or efficiency of production of the fine chemical from the culture.

The aforementioned mutagenesis strategies for MCT proteins to result in increased yields of a fine chemical from *C*. *glutamicum* are not meant to be limiting; variations on these strategies will be readily apparent to one of ordinary skill in the art. Using such strategies, and incorporating the mechanisms disclosed herein, the nucleic acid and protein molecules of the invention may be utilized to generate *C*. *glutamicum* or related strains of bacteria expressing mutated MCT nucleic acid and protein molecules such that the yield, production, and/or efficiency of production of a desired compound is improved. This desired compound may be any natural product of *C*. *glutamicum,* which includes the final products of biosynthesis pathways and intermediates of naturally-occurring metabolic pathways, as well as molecules which do not naturally occur in the metabolism of *C. glutamicum,* but which are produced by a *C. glutamicum* strain of the invention.

This invention is further illustrated by the following examples which should not be construed as limiting. The contents of all references, patent applications, patents, published patent applications, Tables, and the sequence listing cited throughout this application are hereby incorporated by reference.

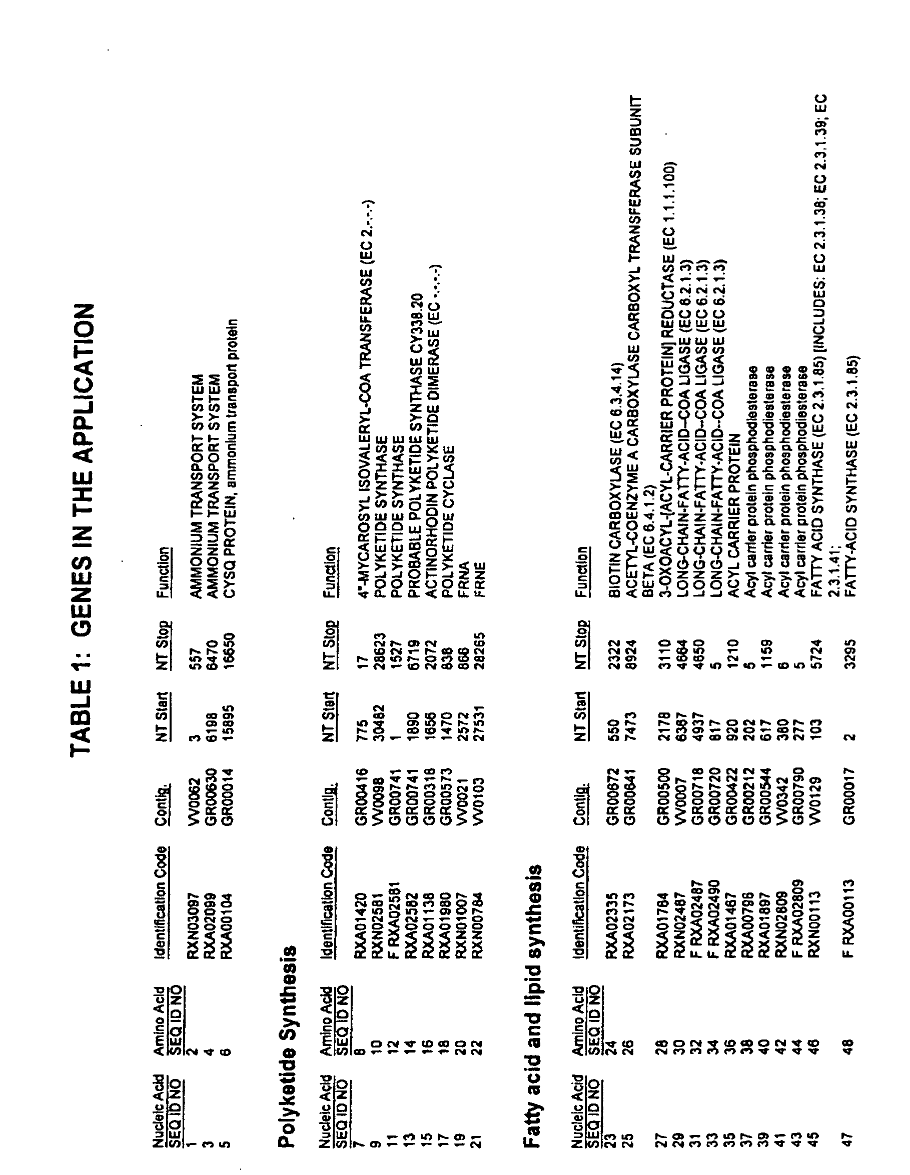

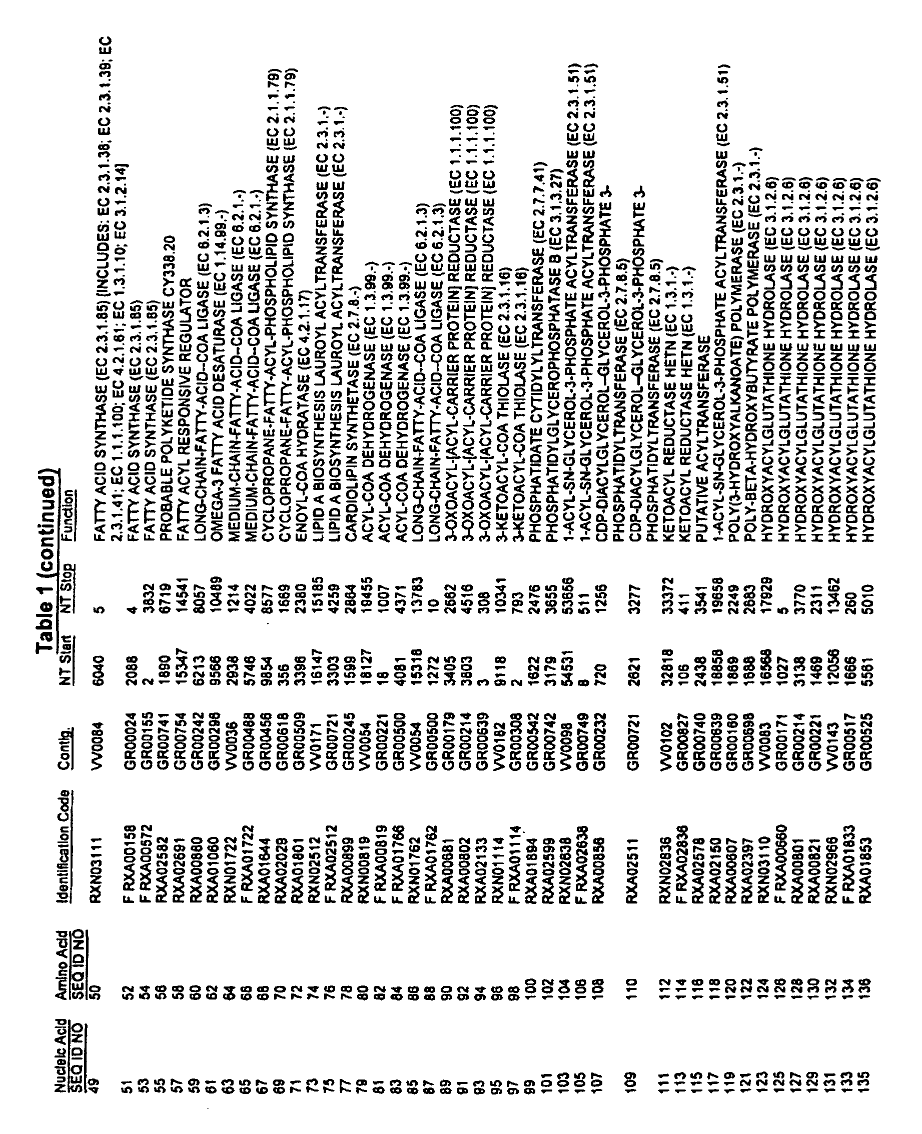

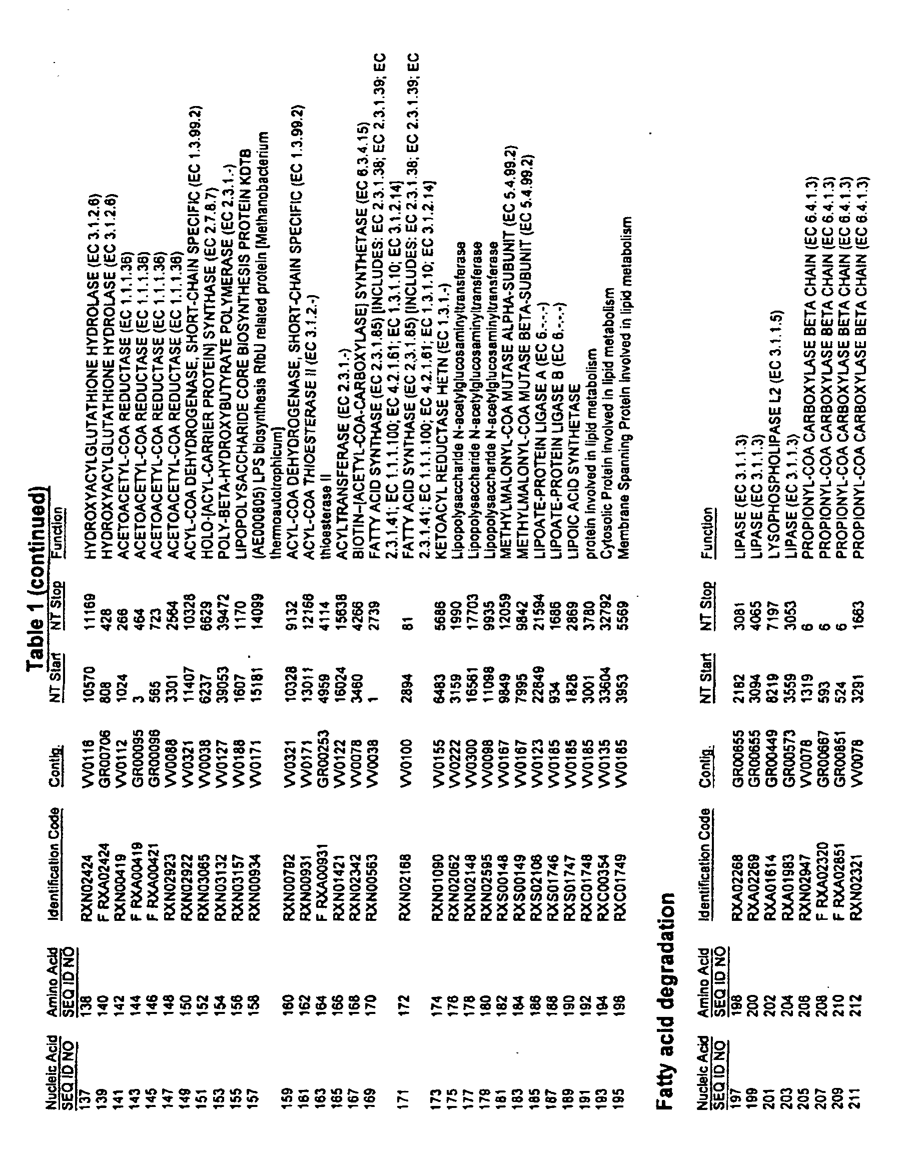

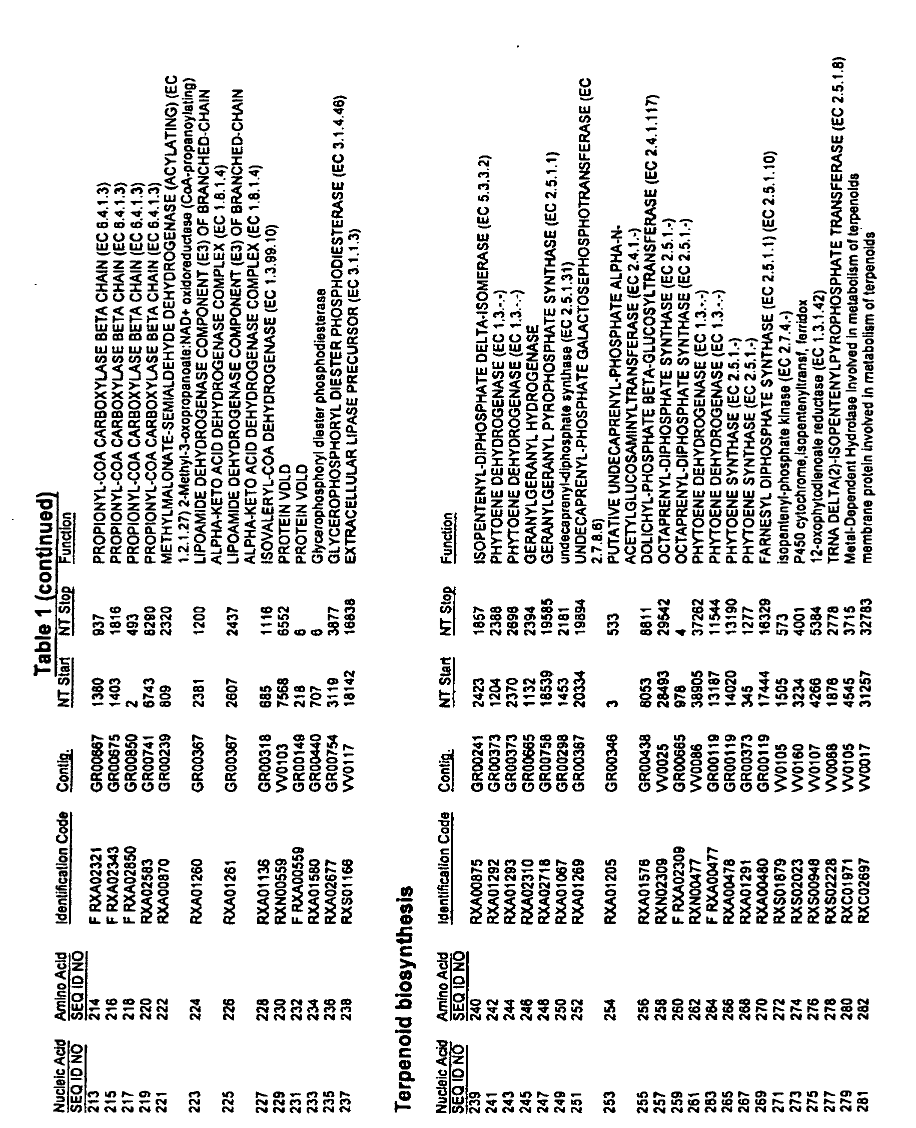

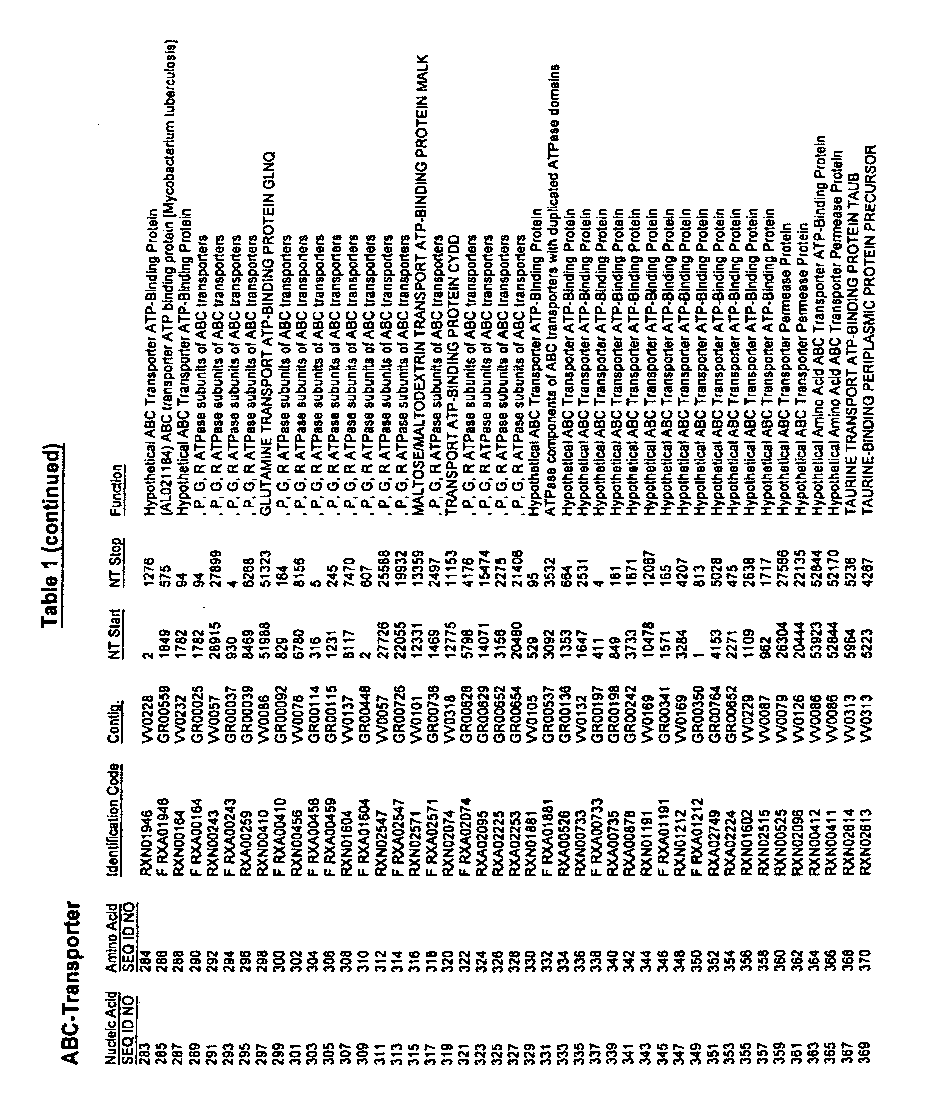

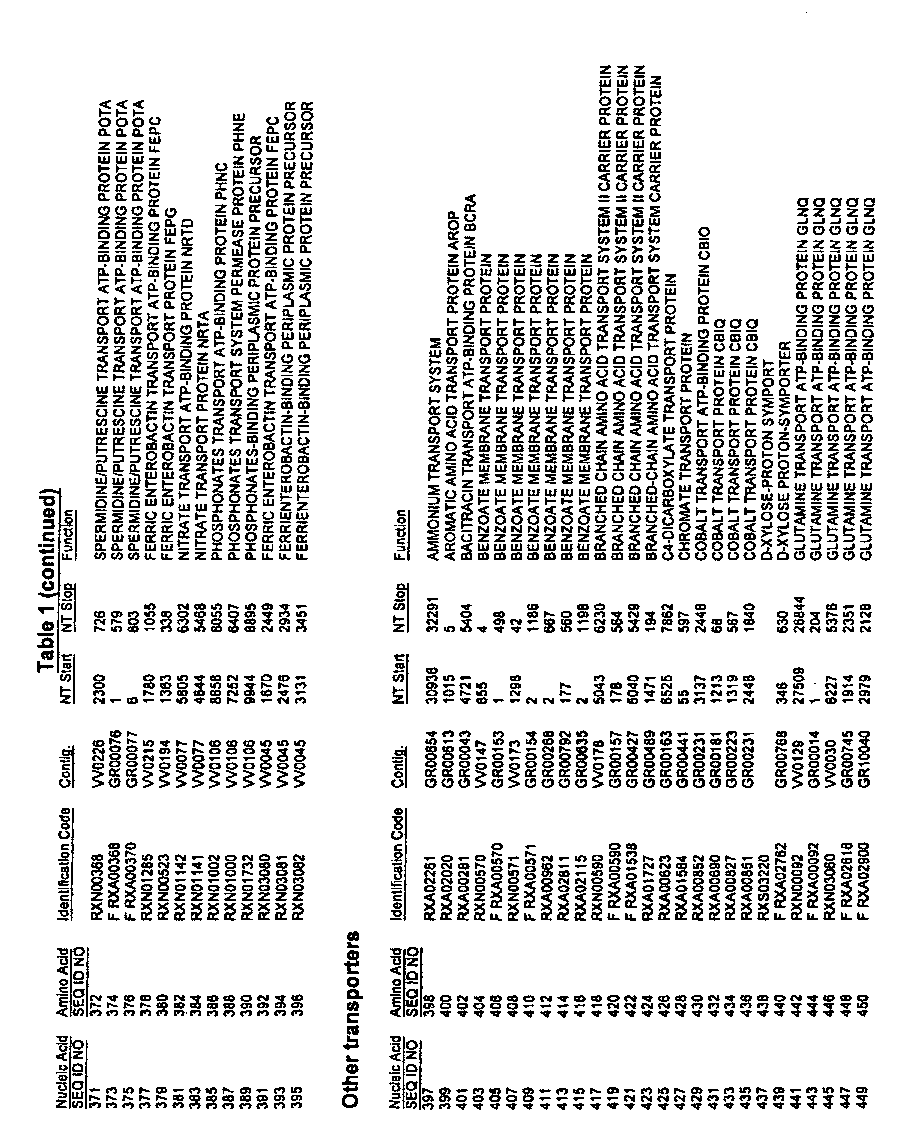

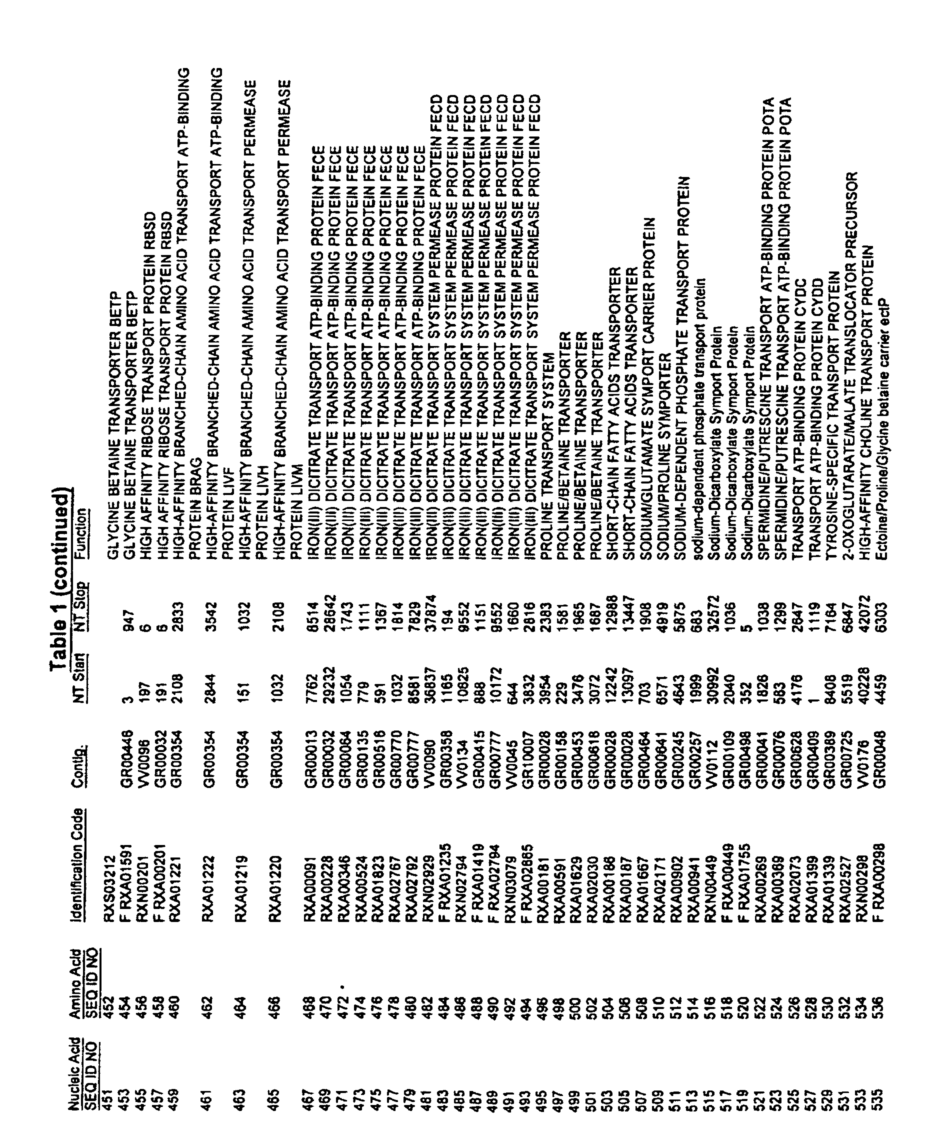

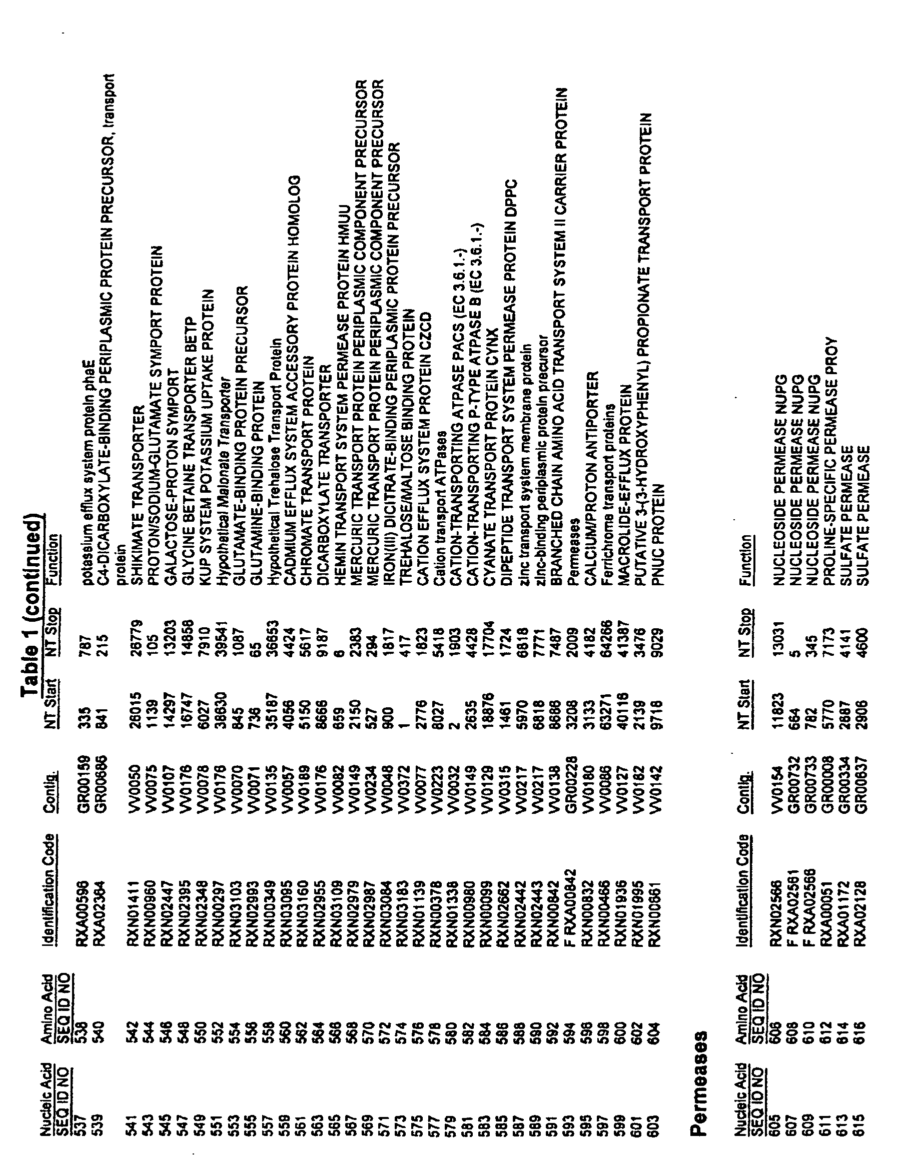

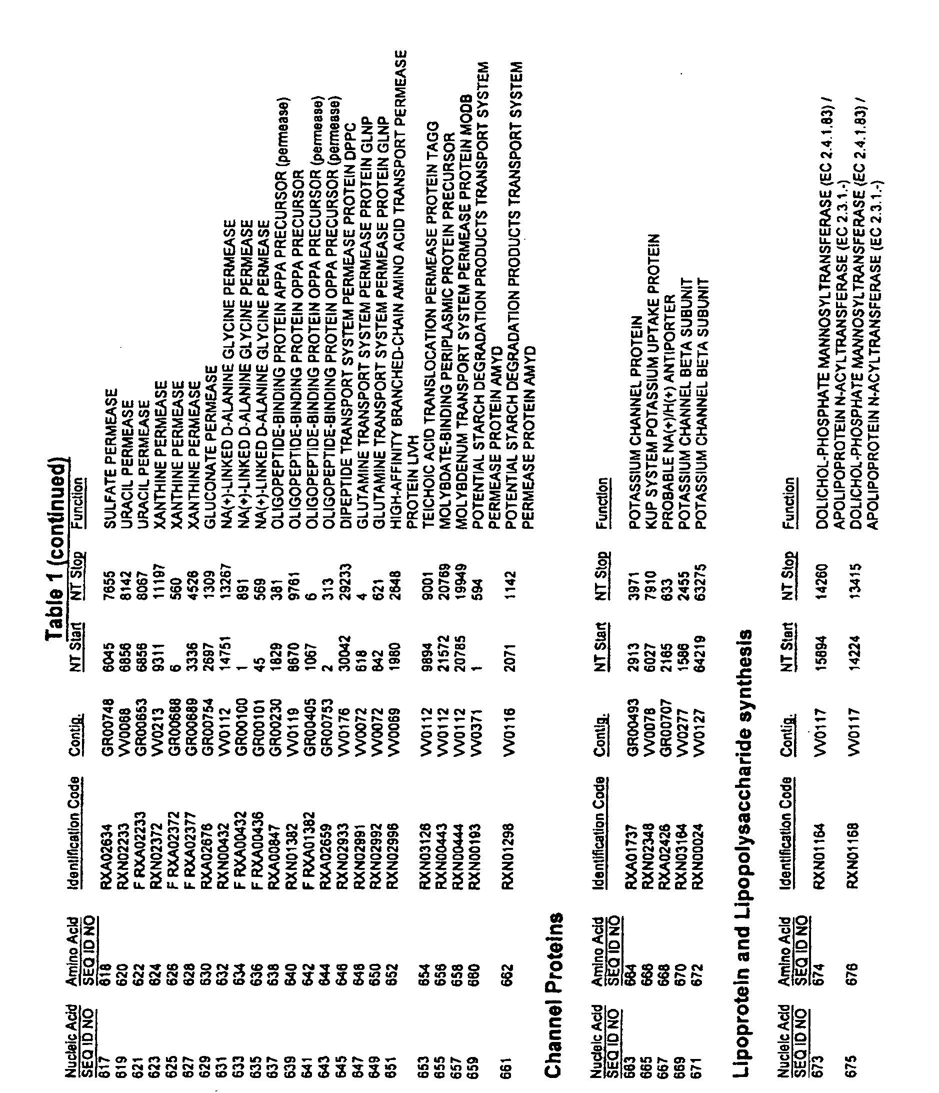

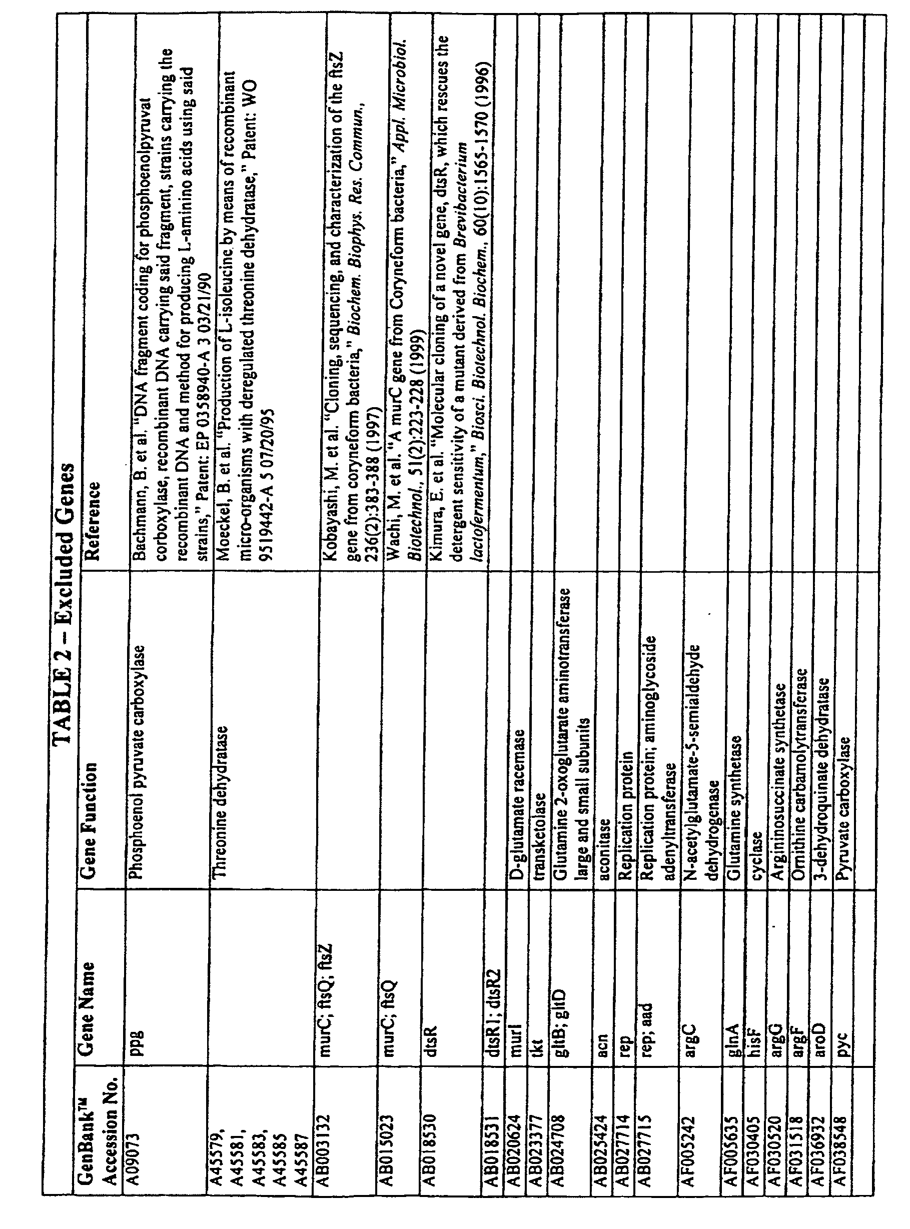

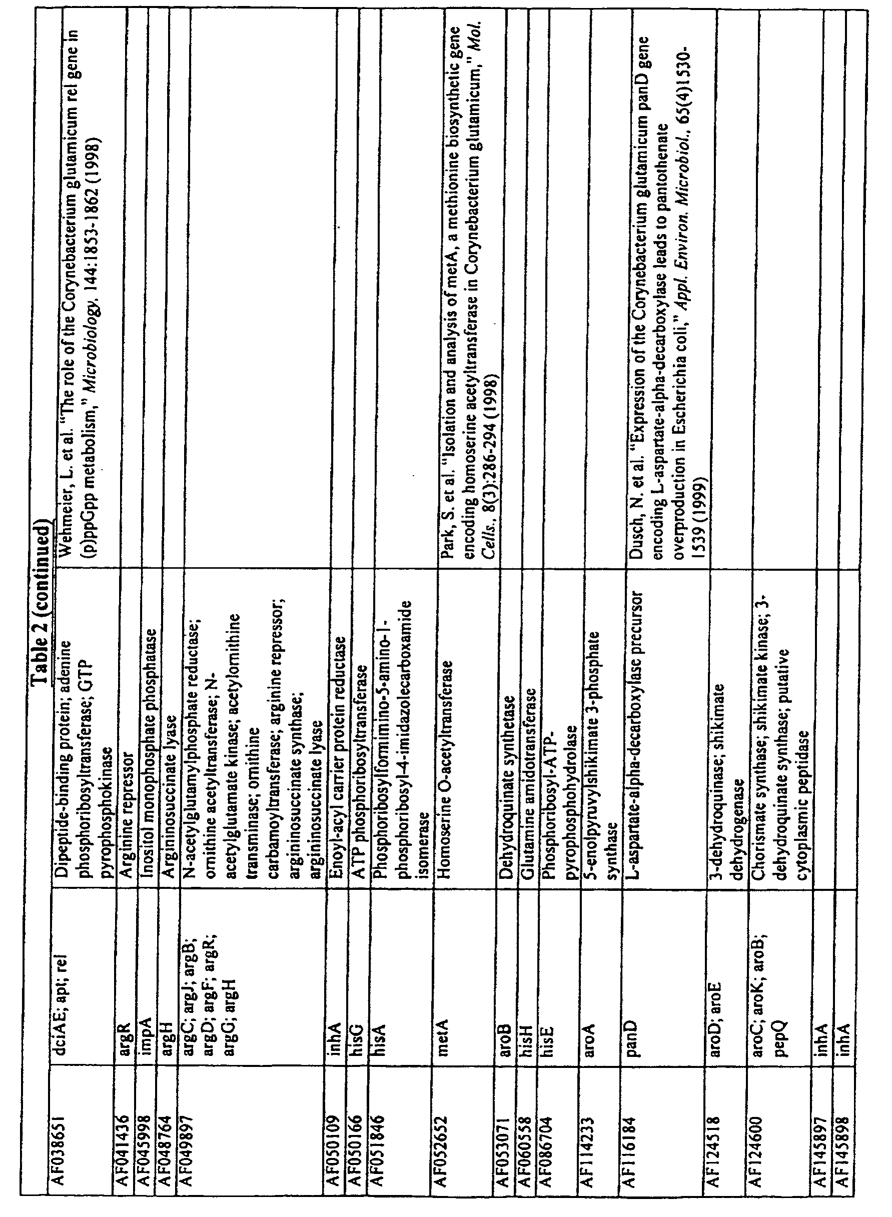

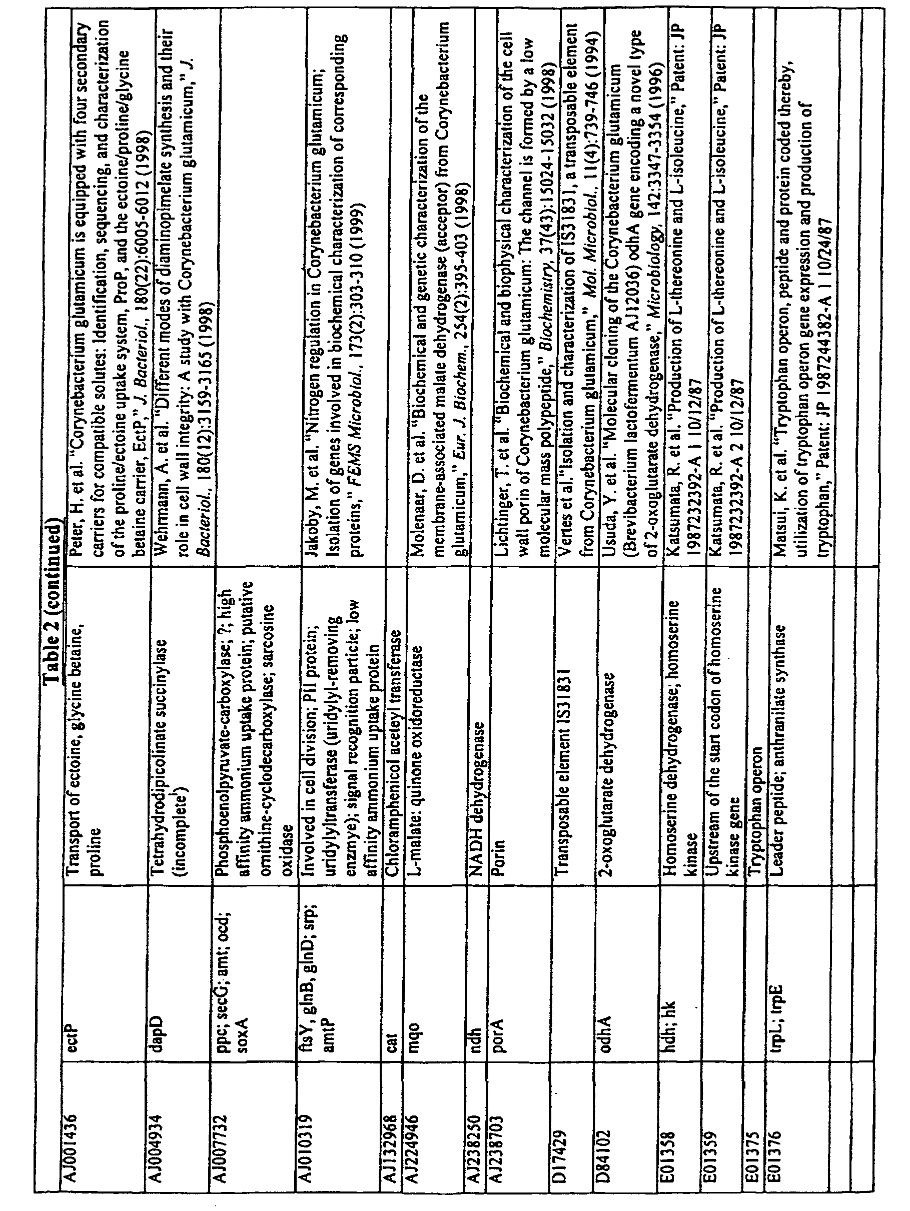

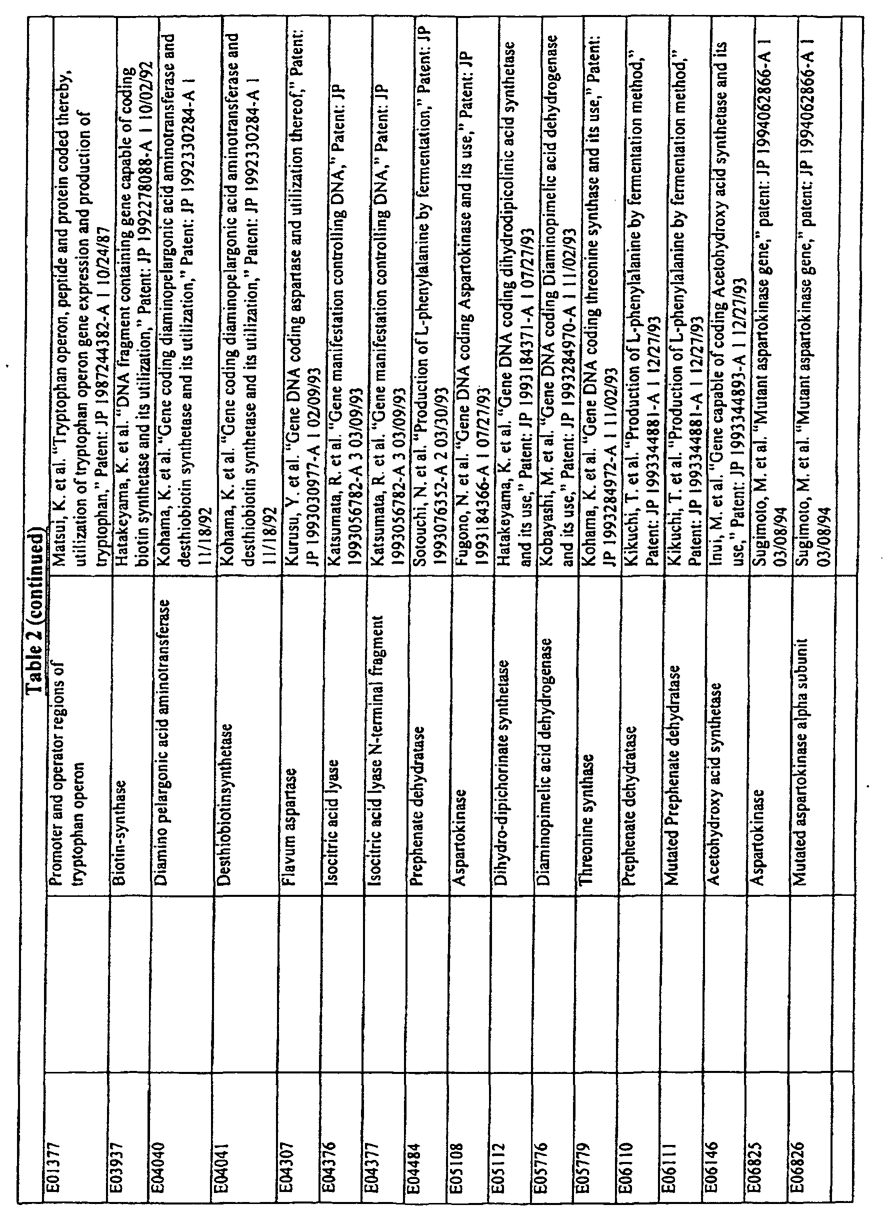

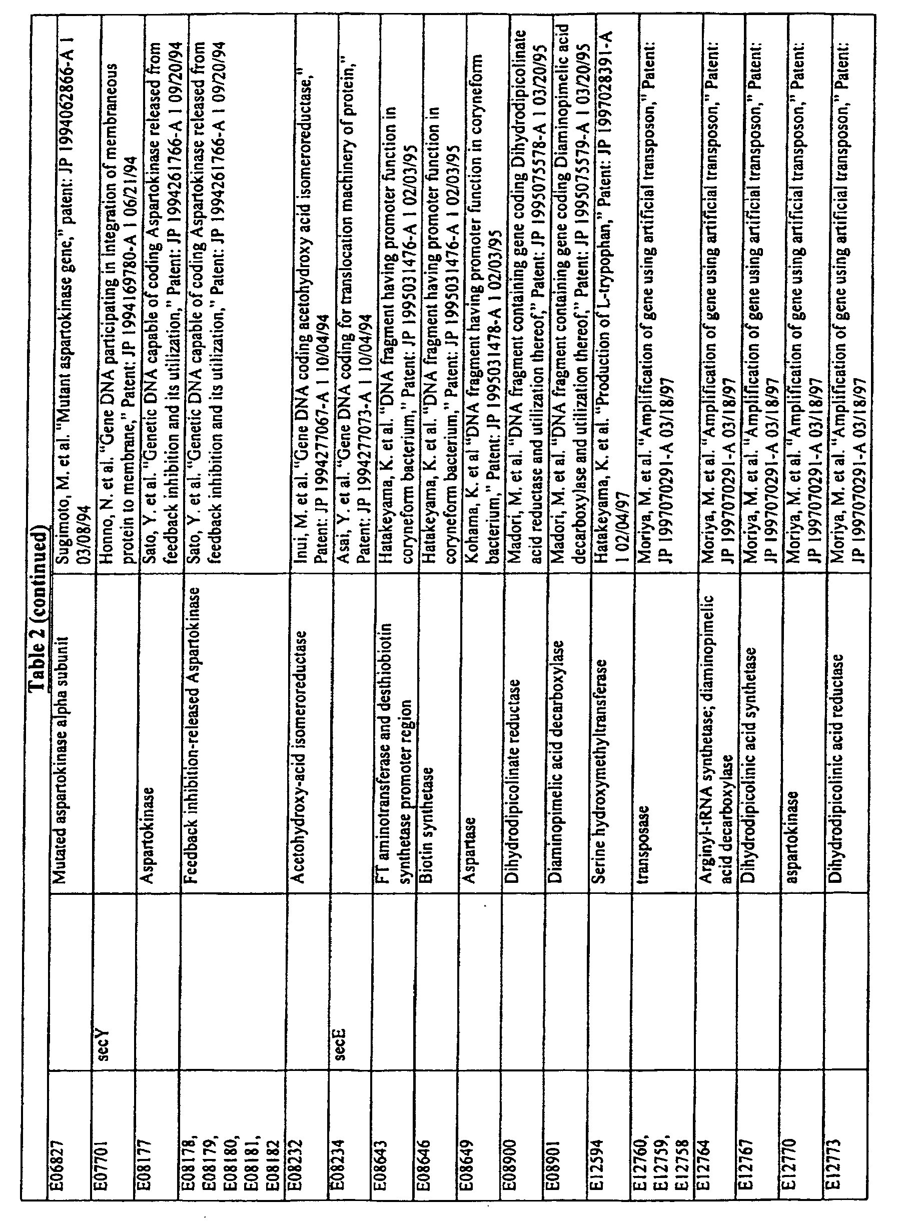

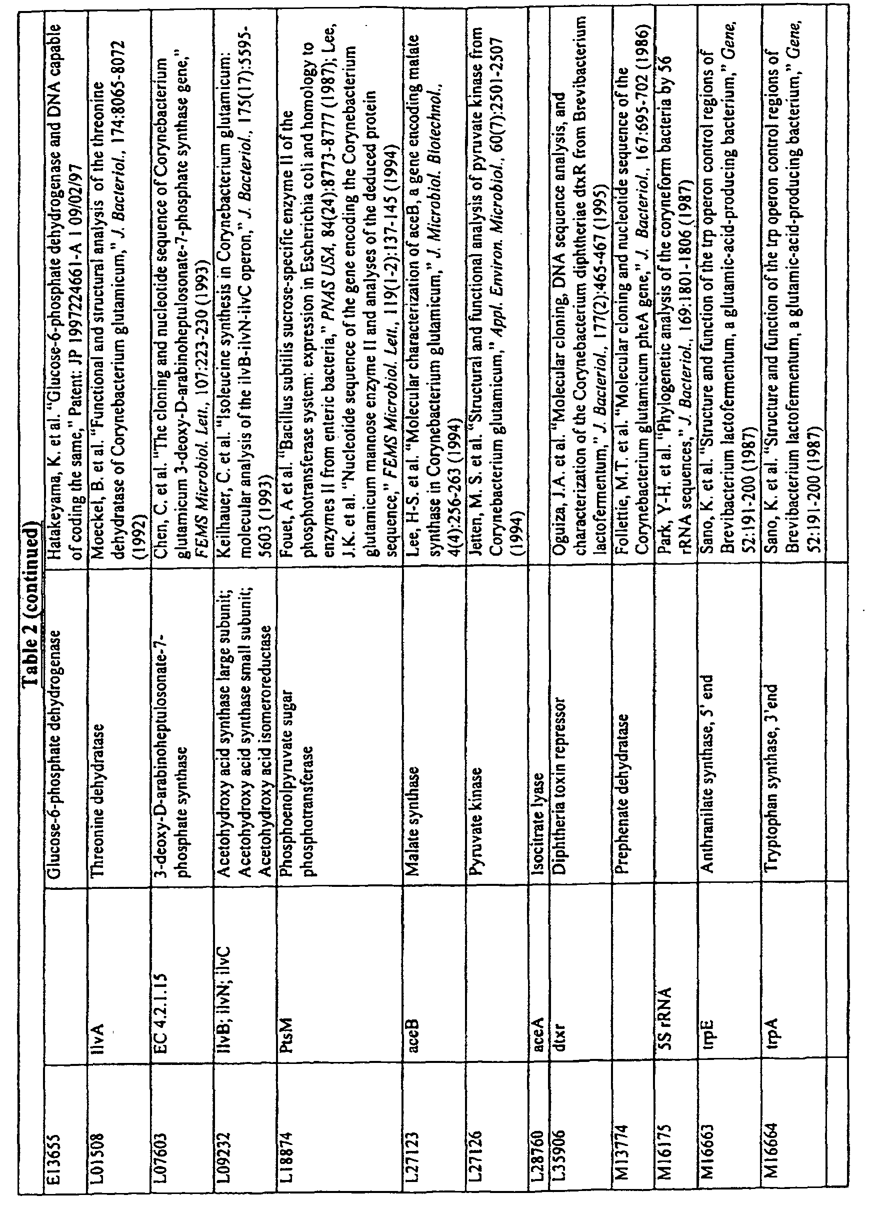

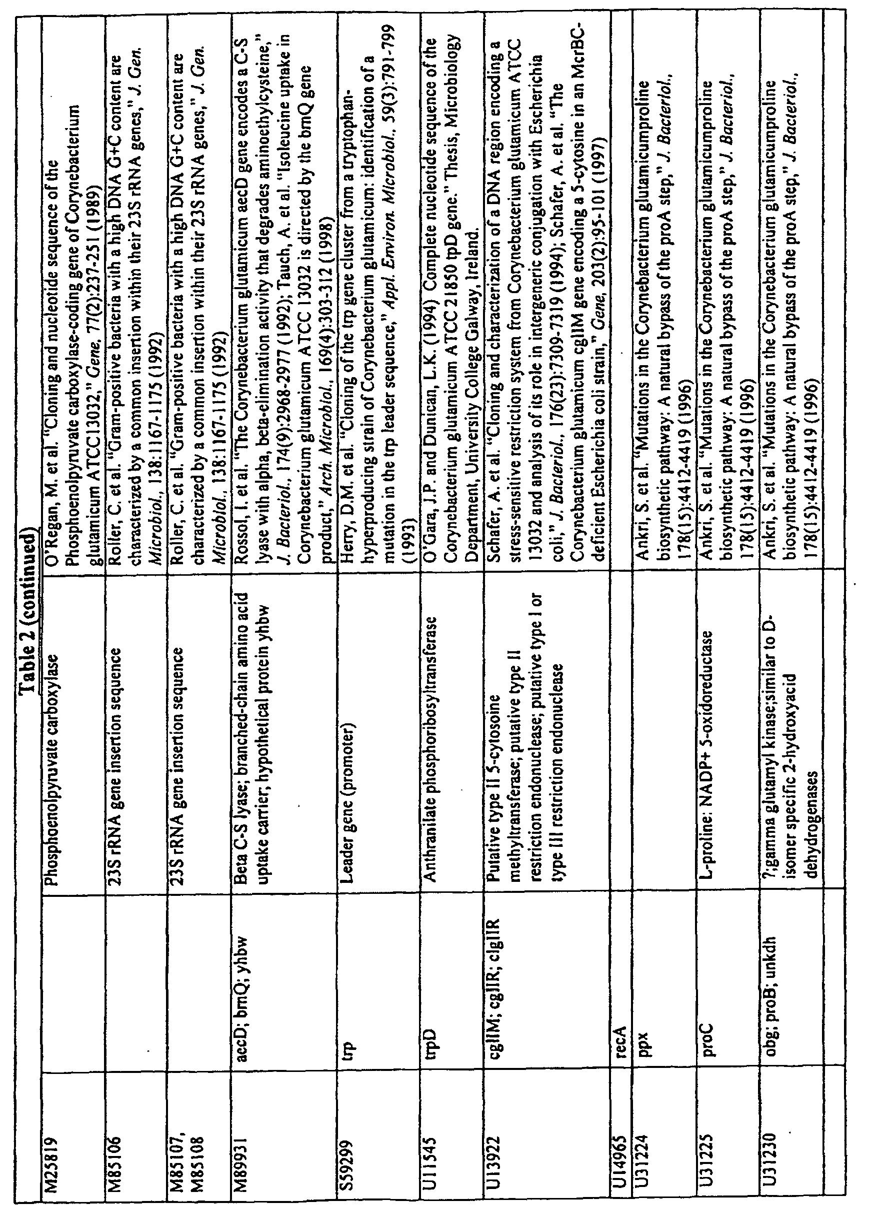

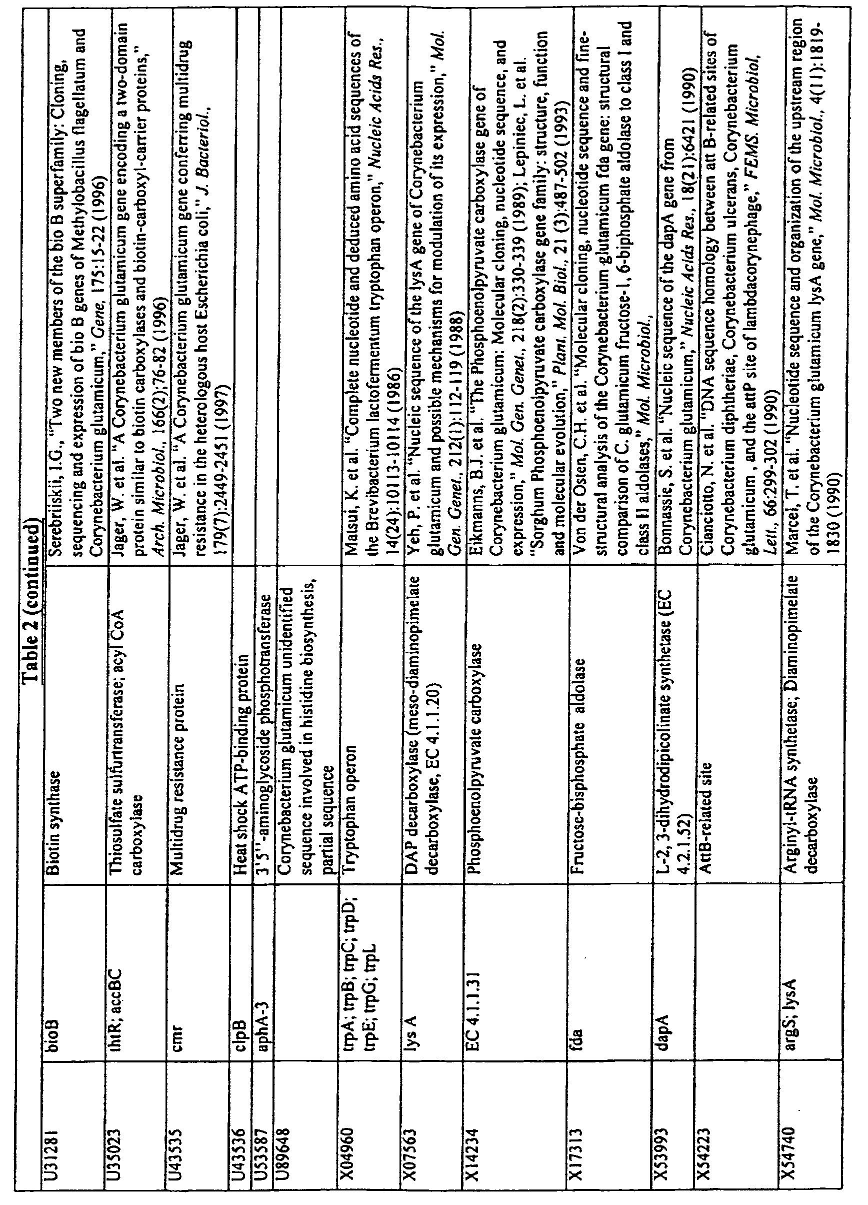

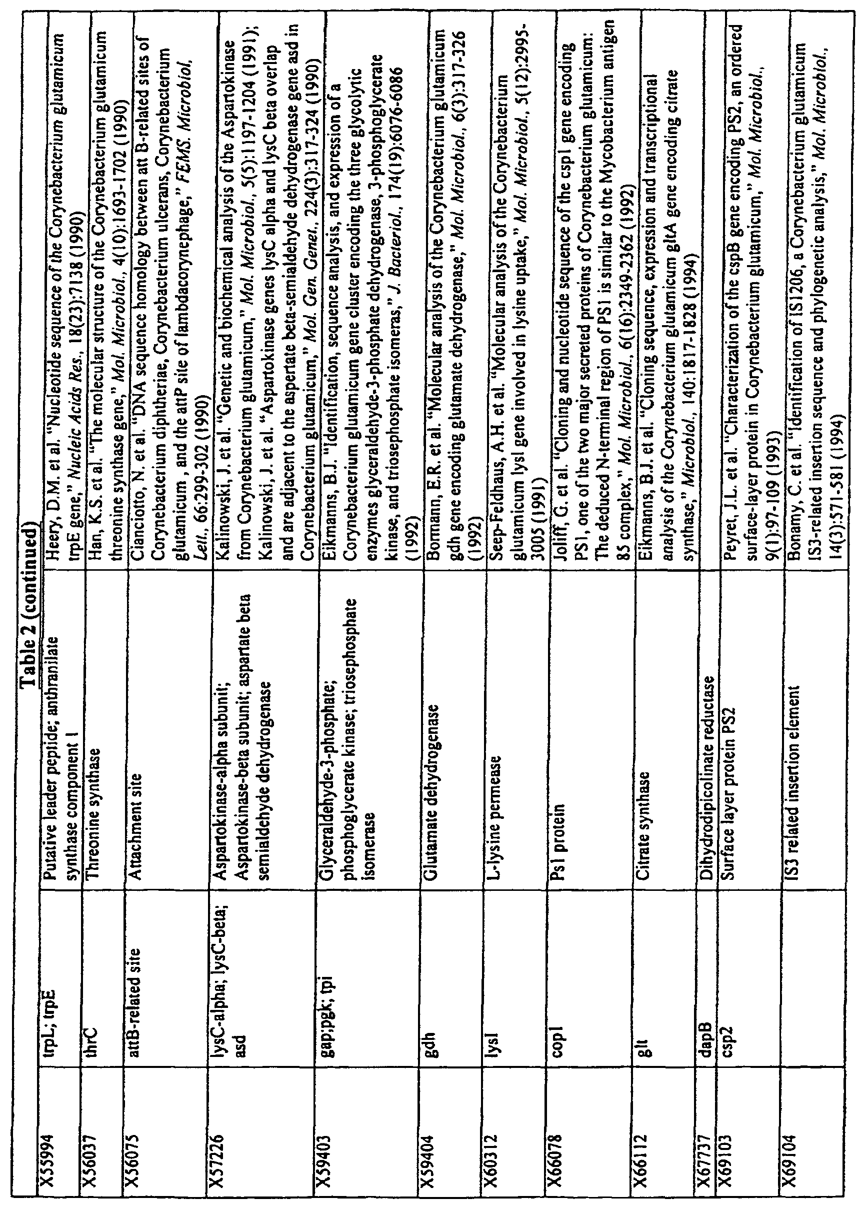

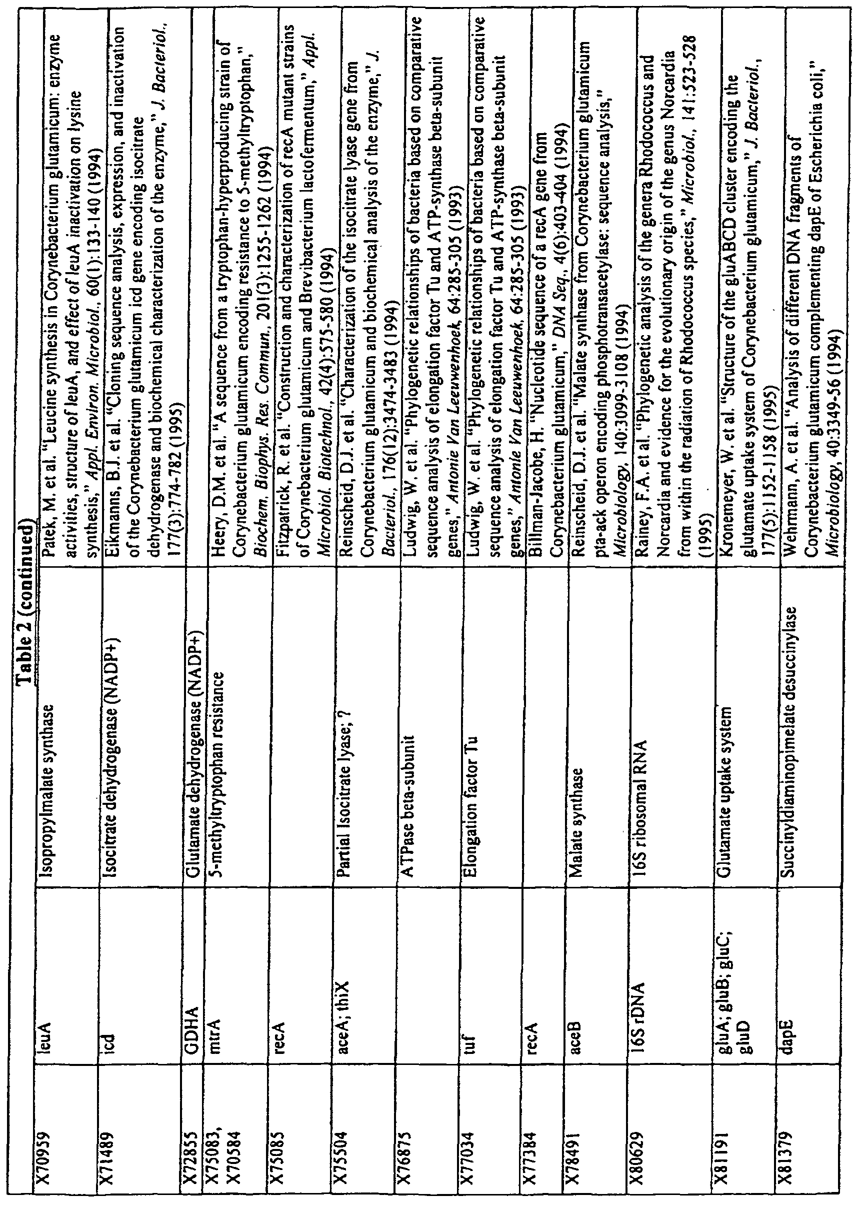

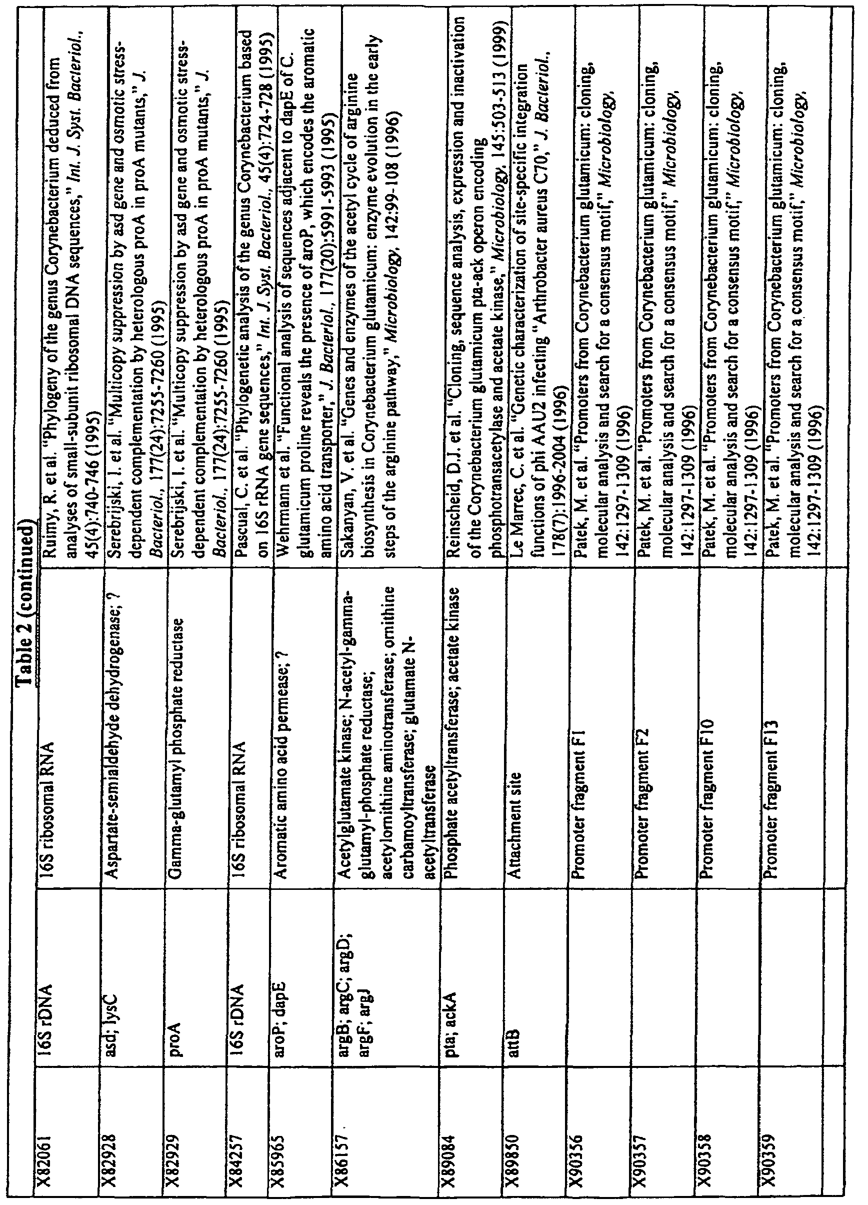

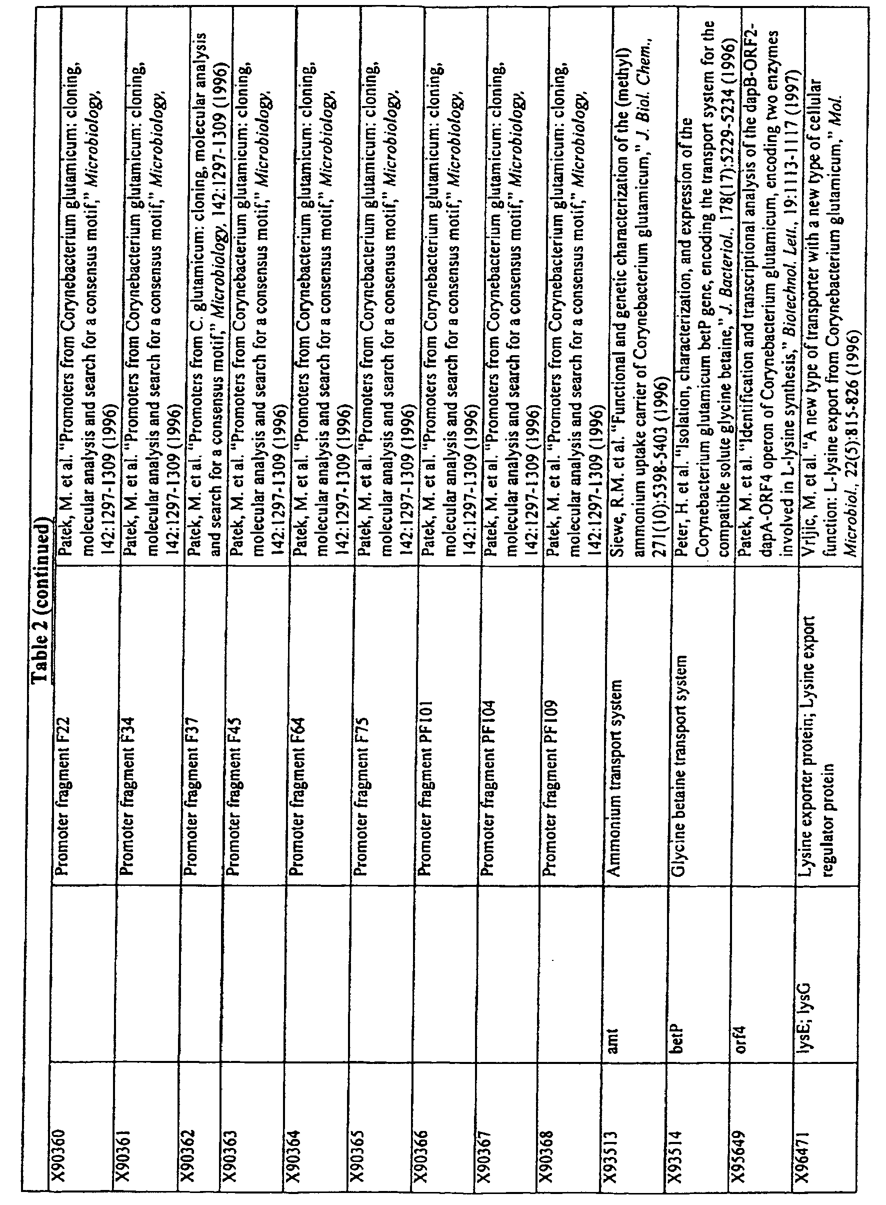

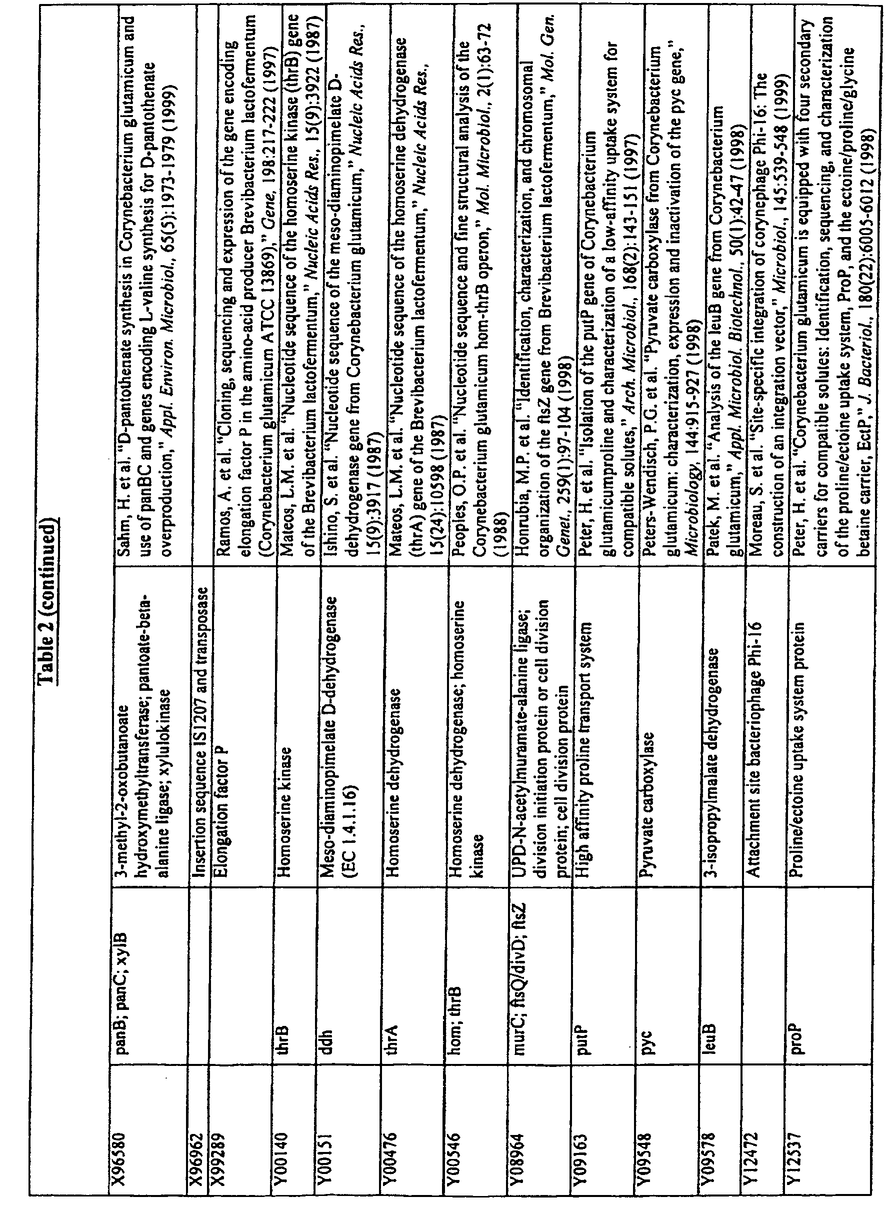

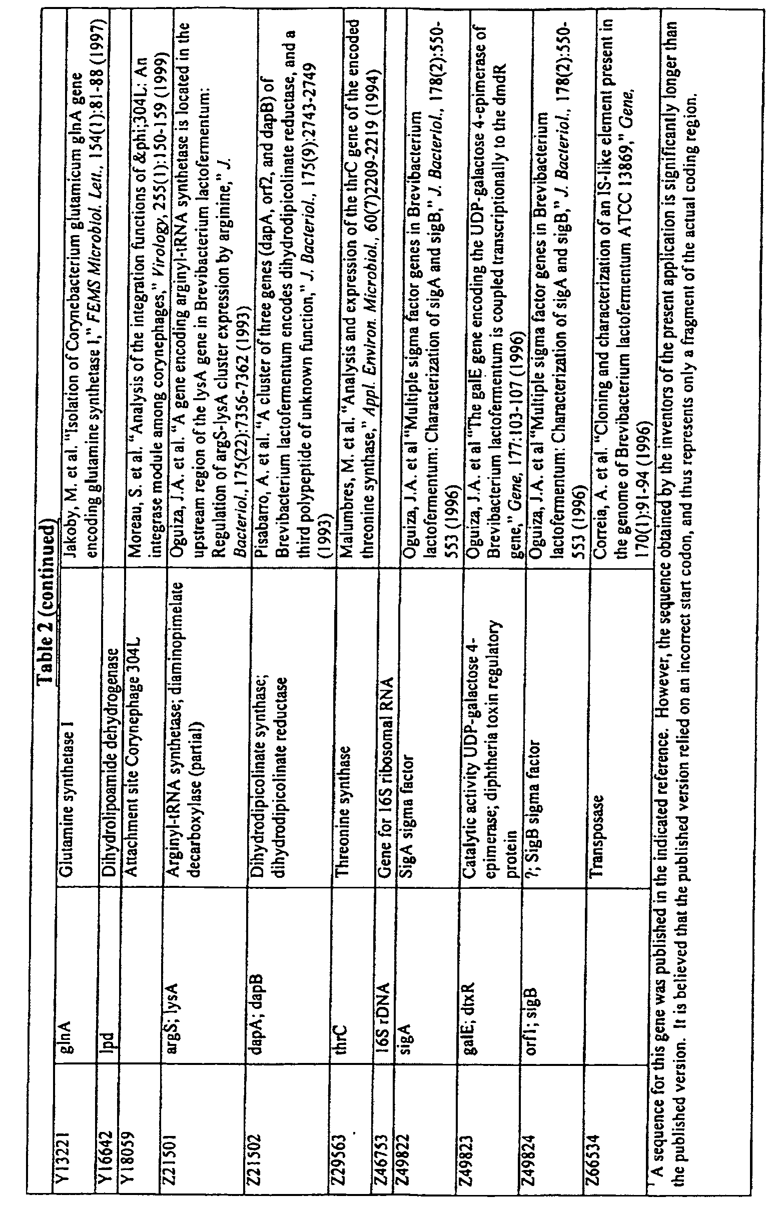

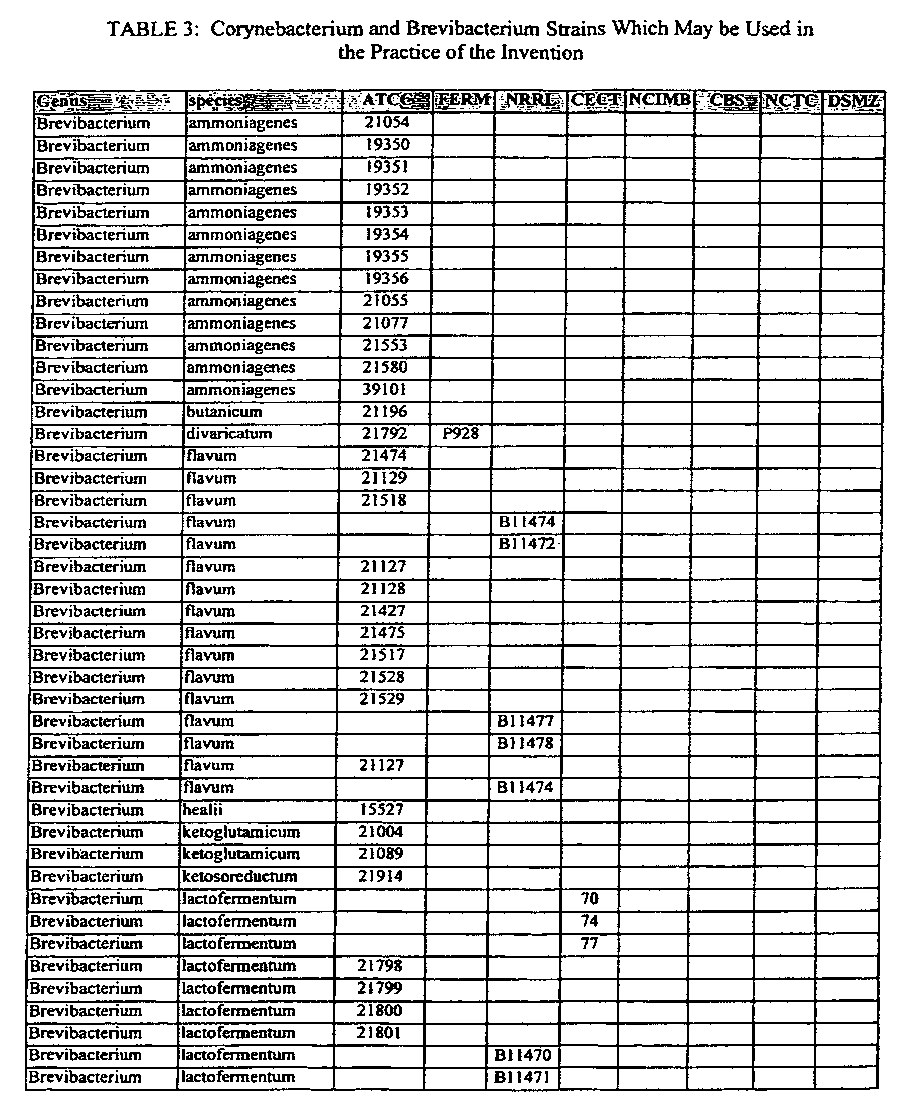

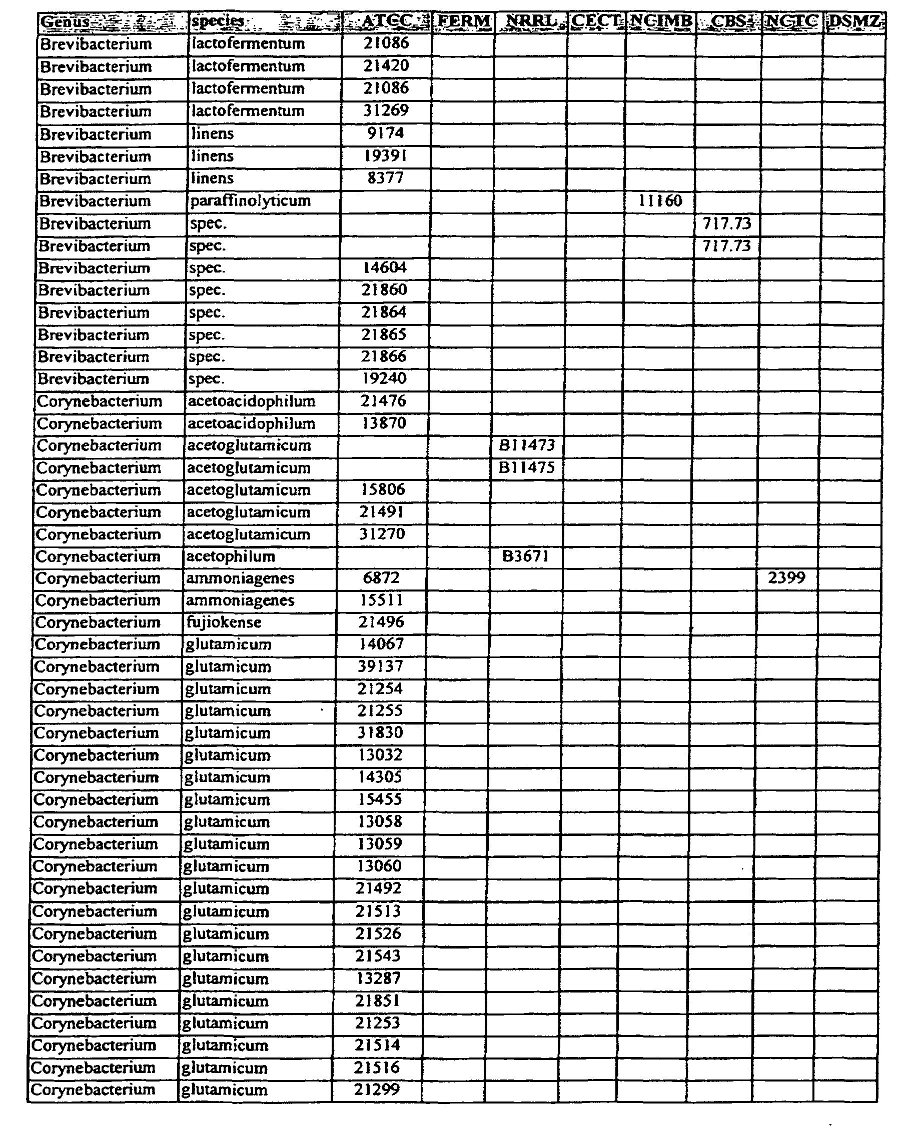

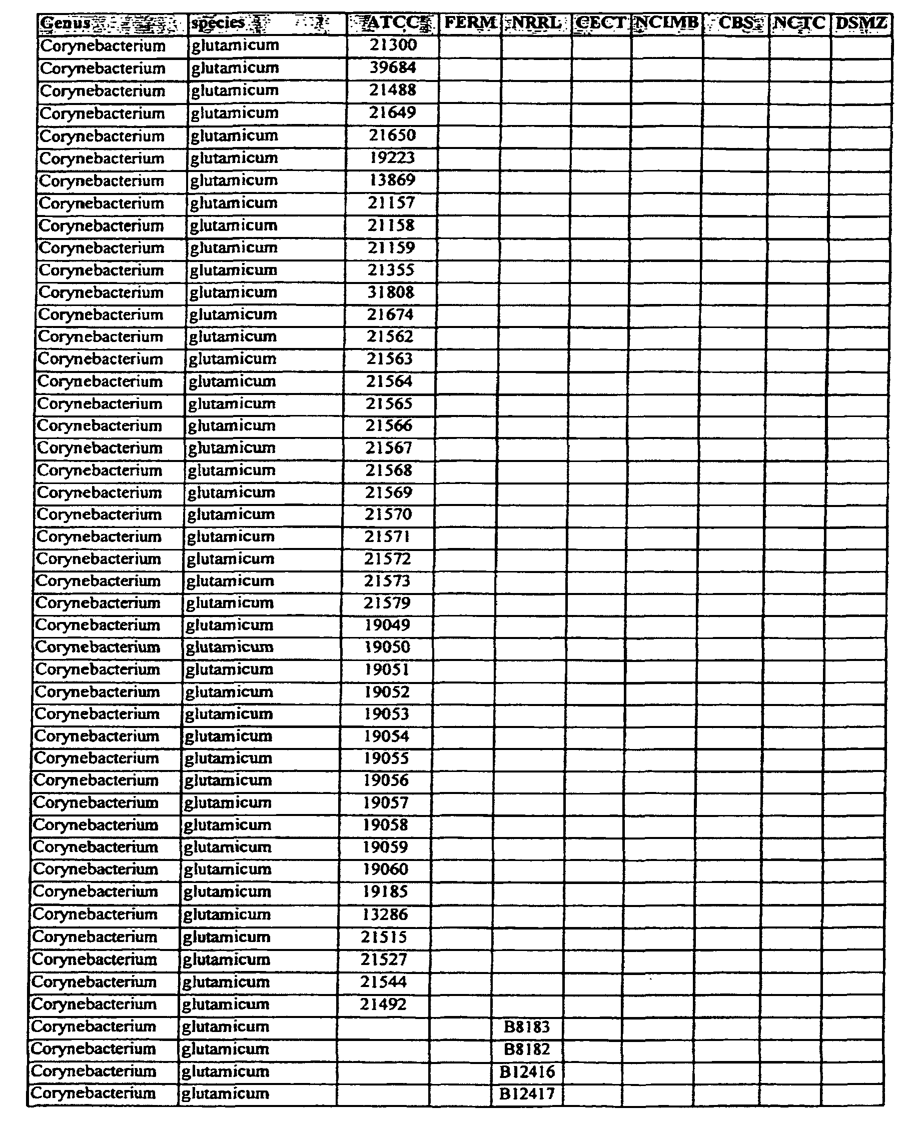

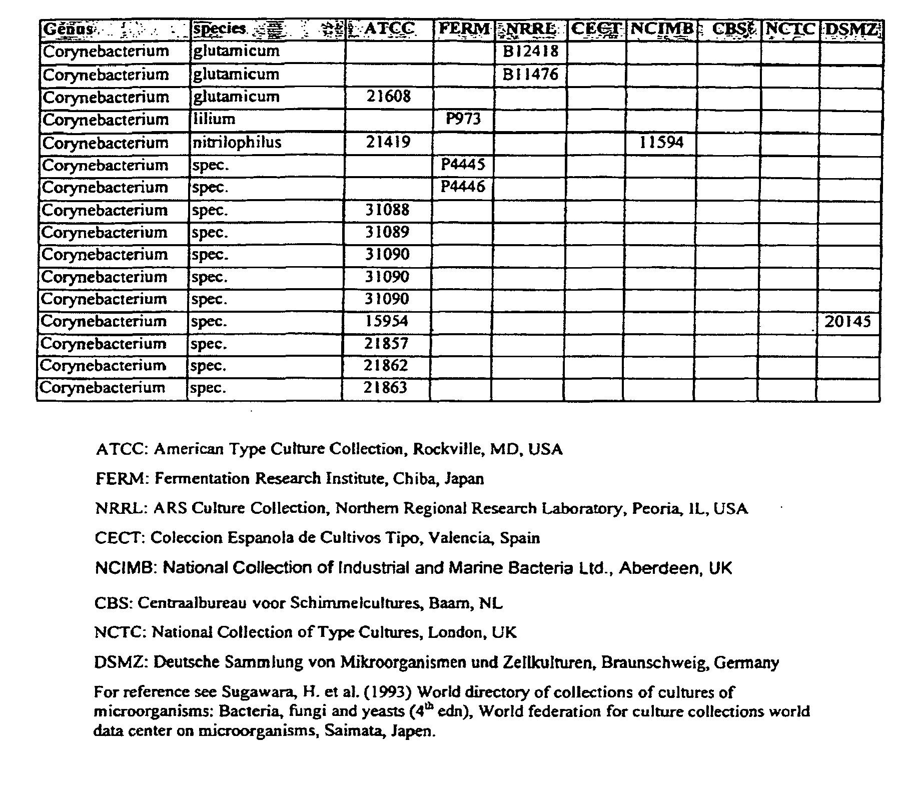

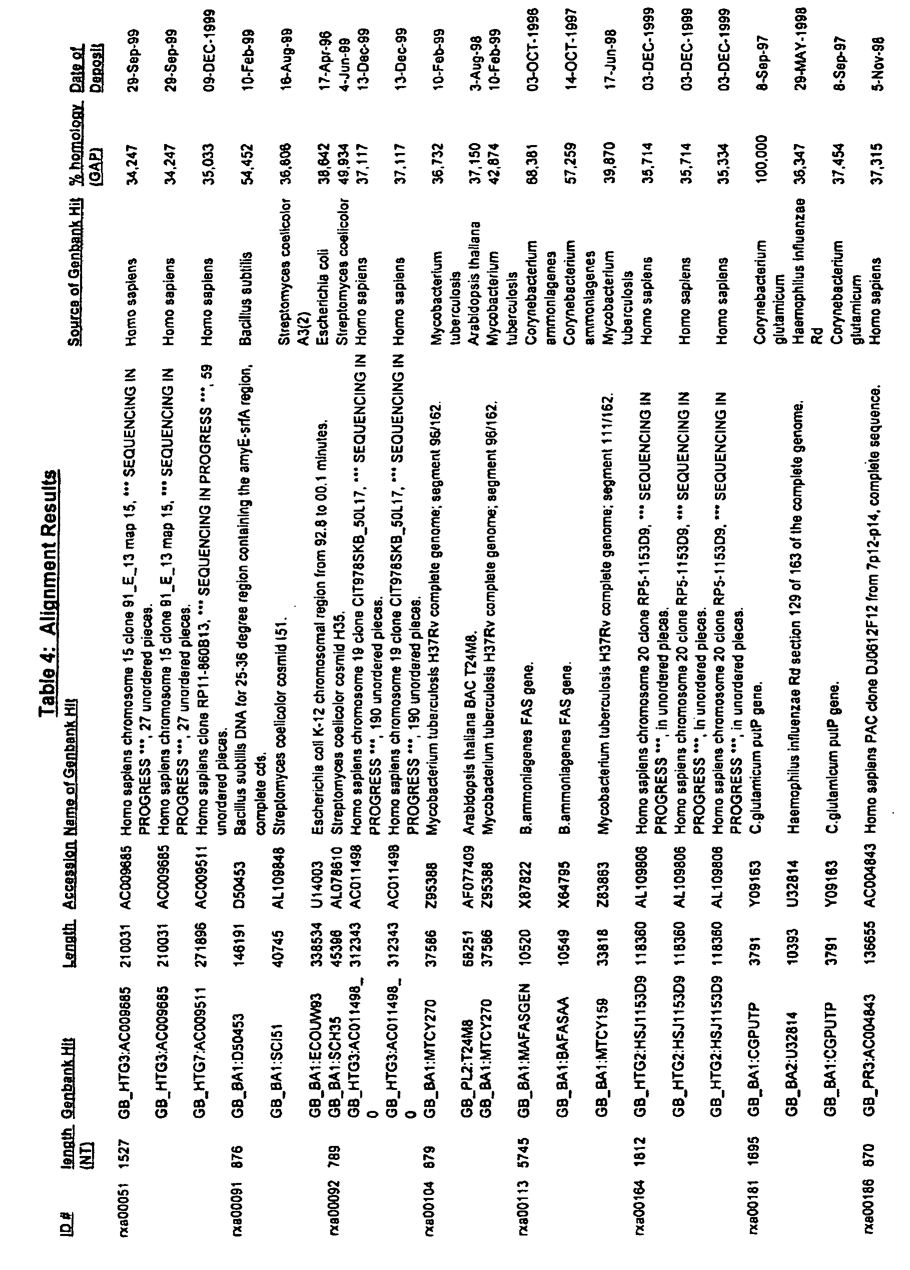

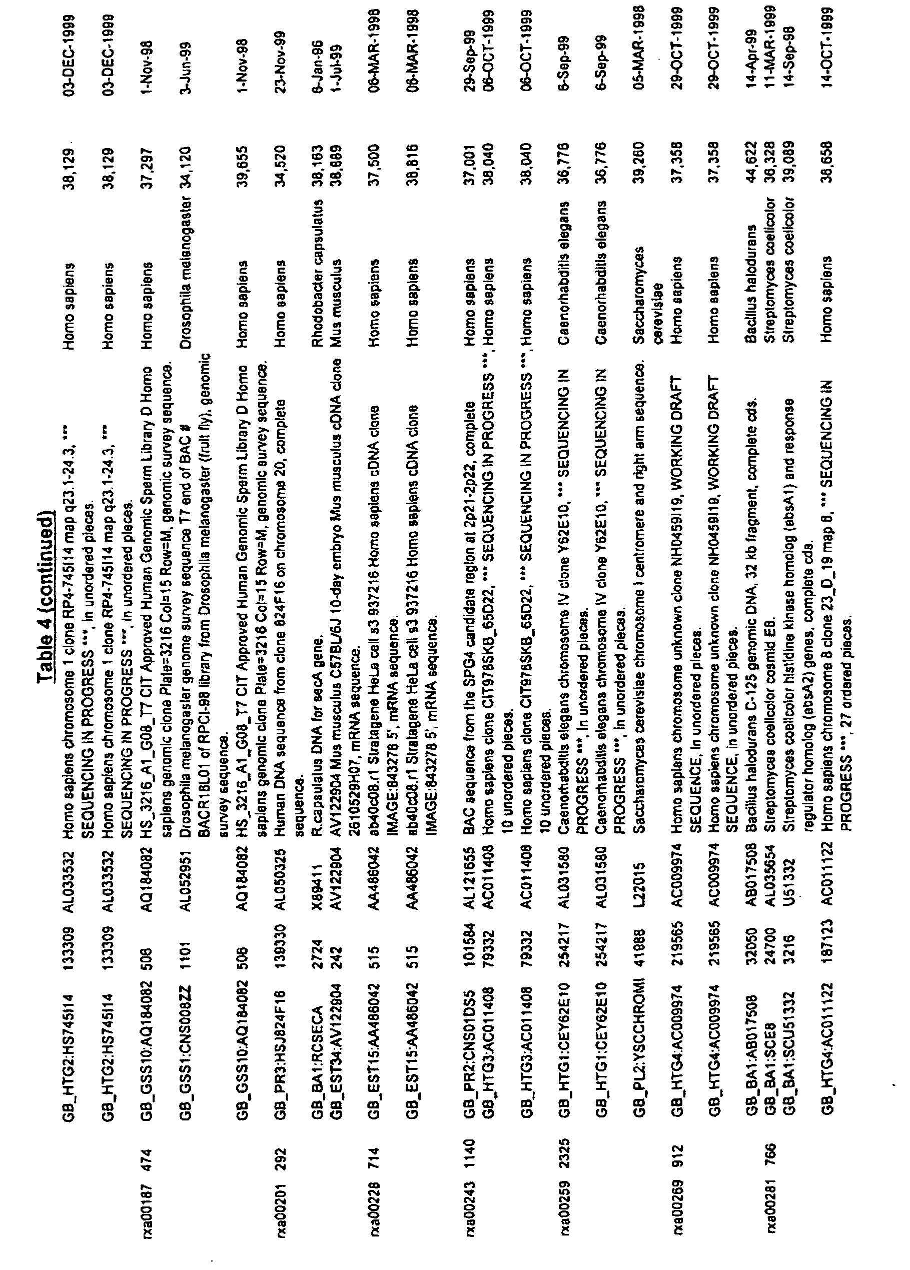

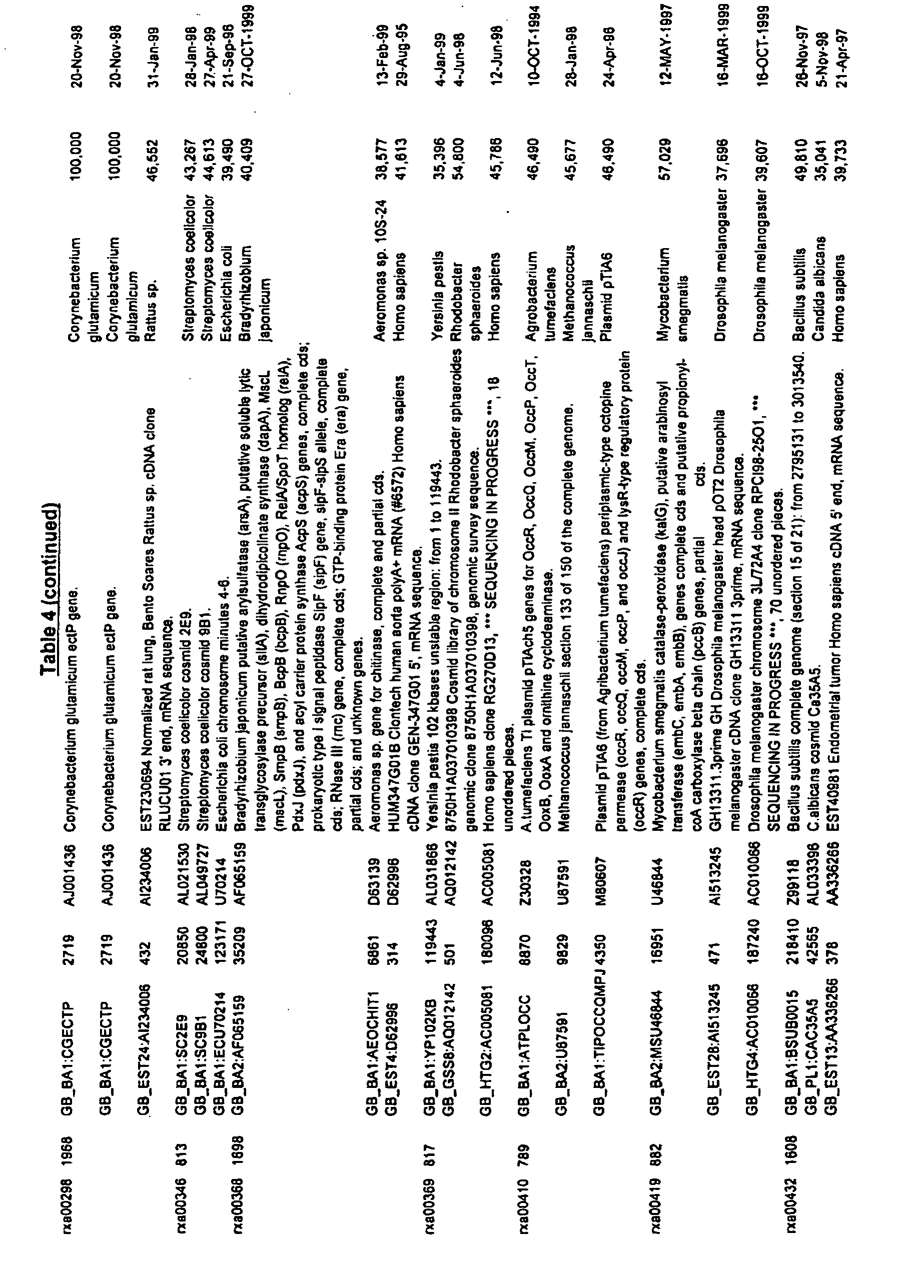

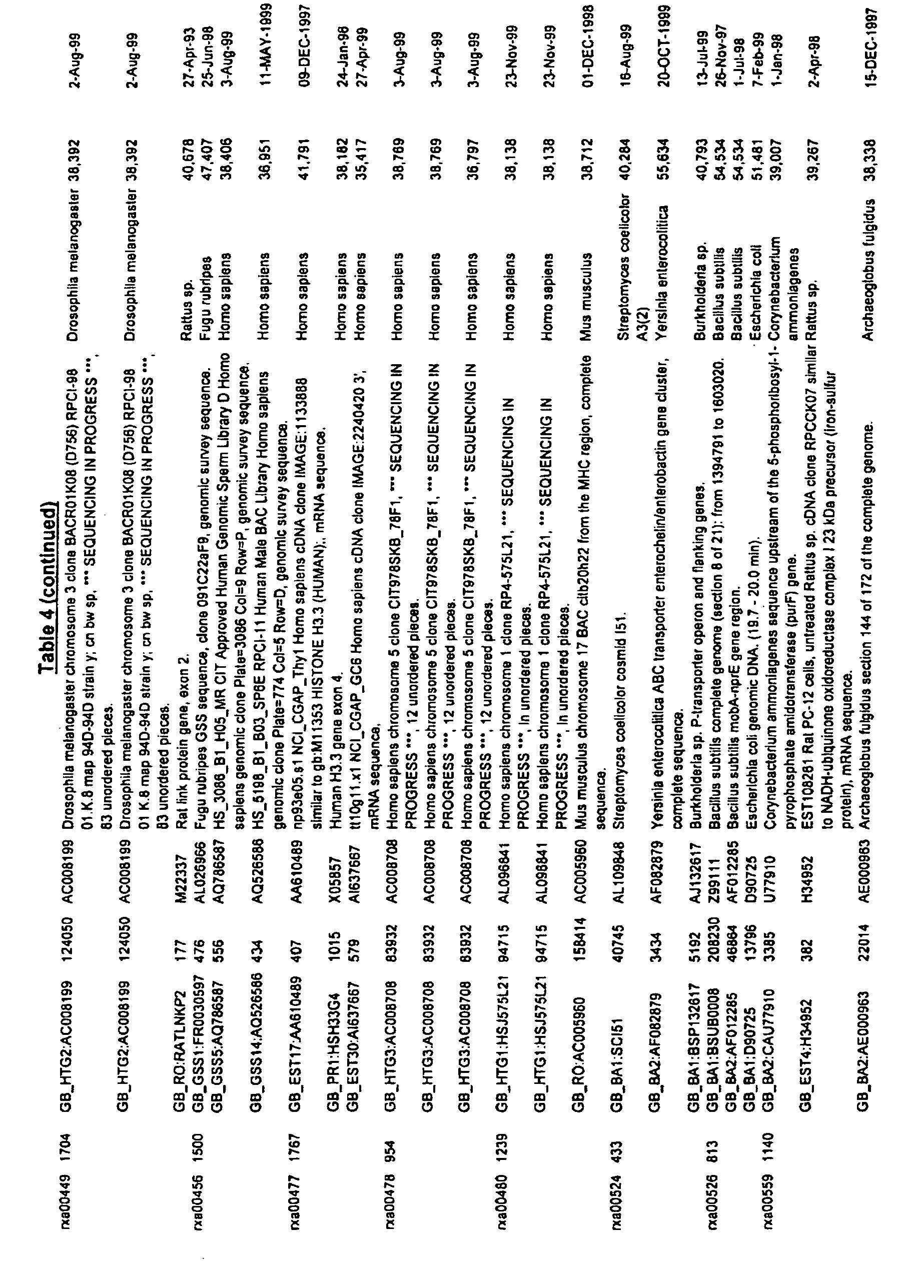

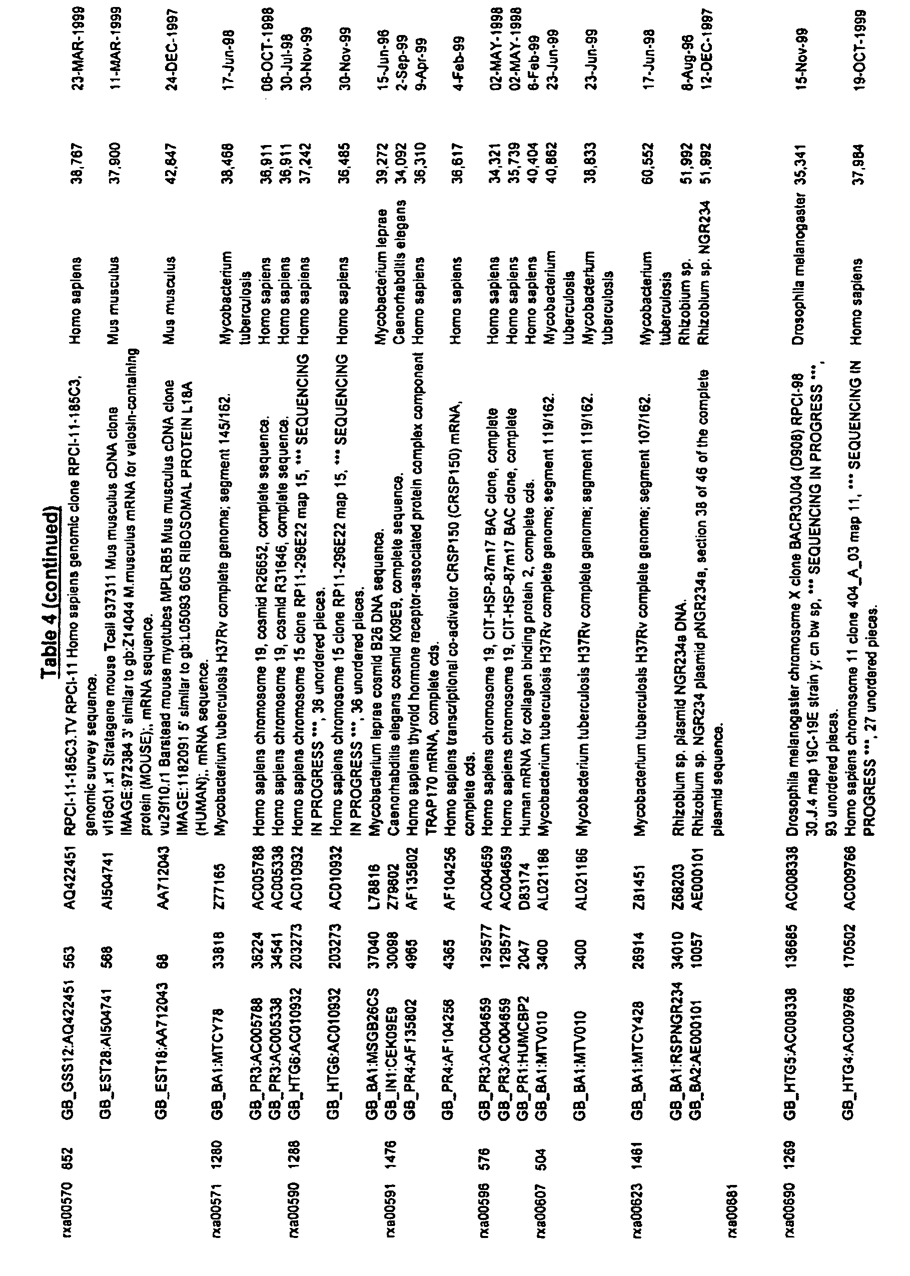

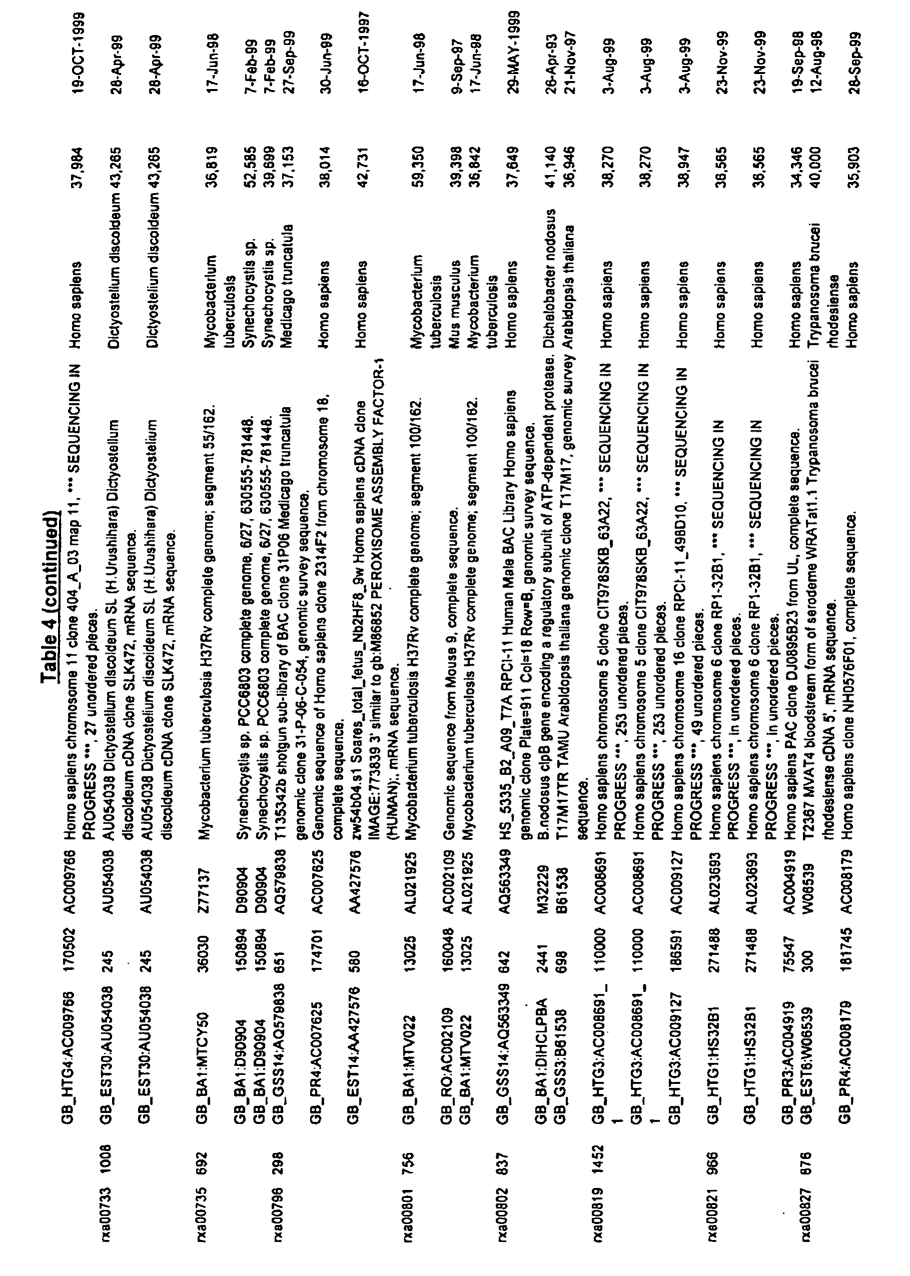

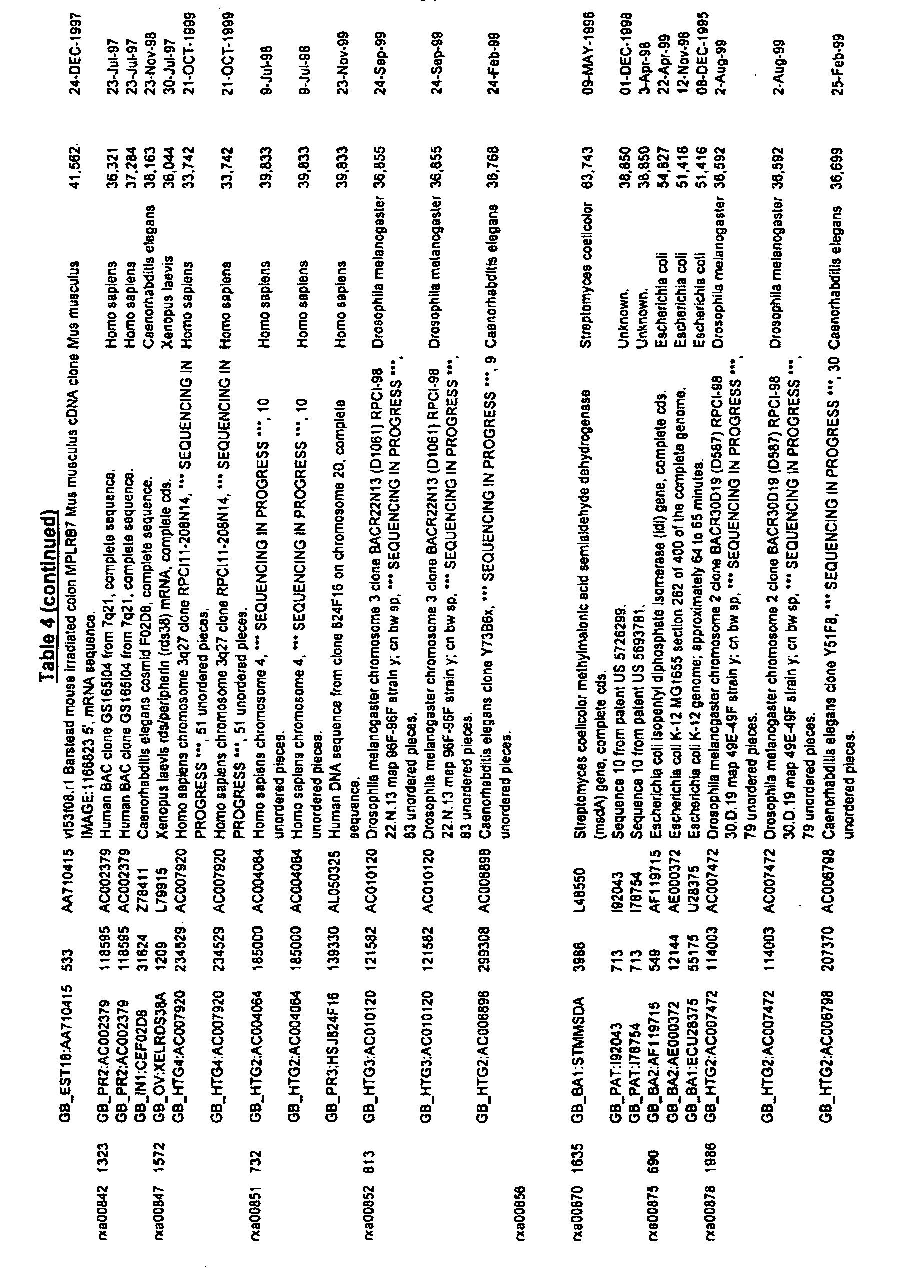

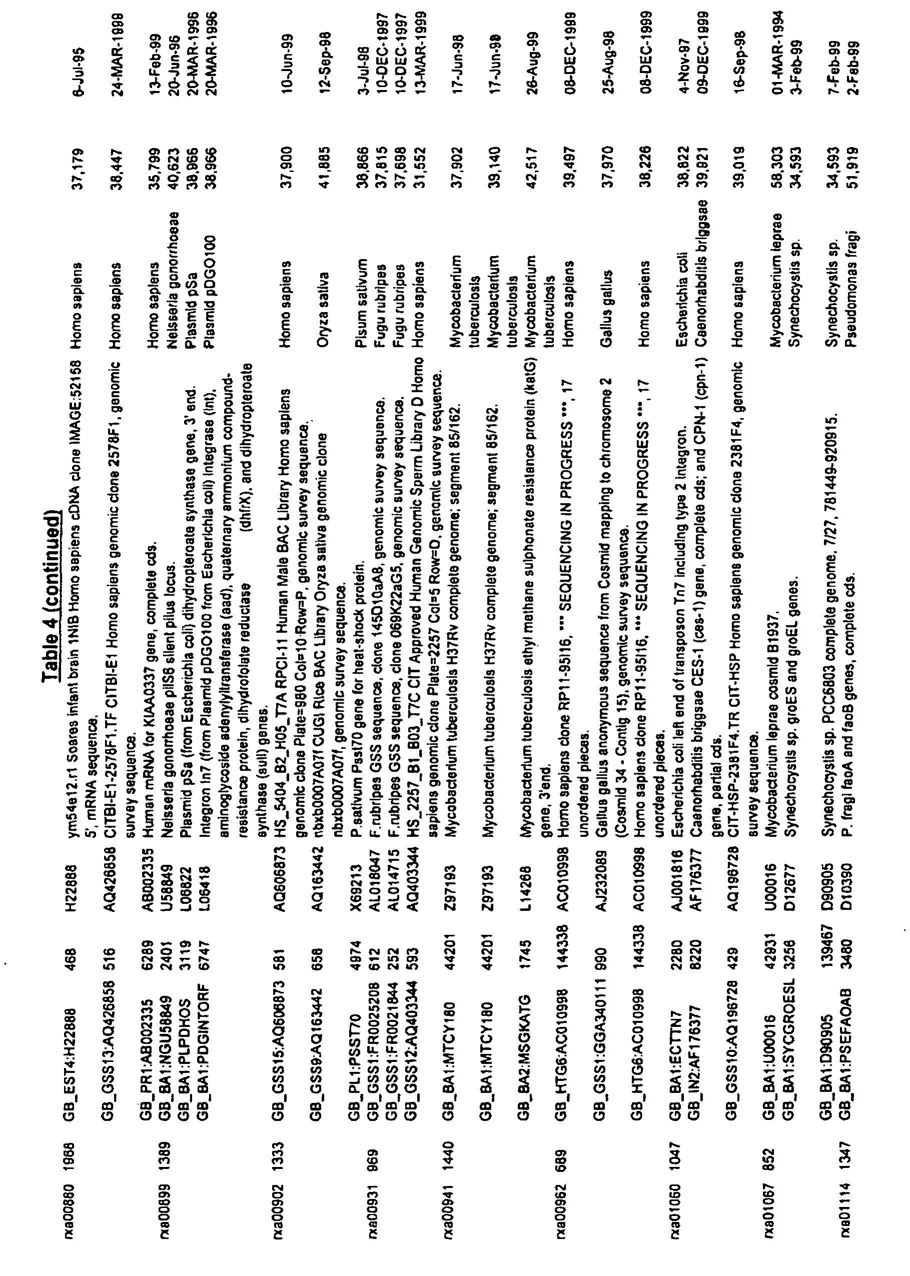

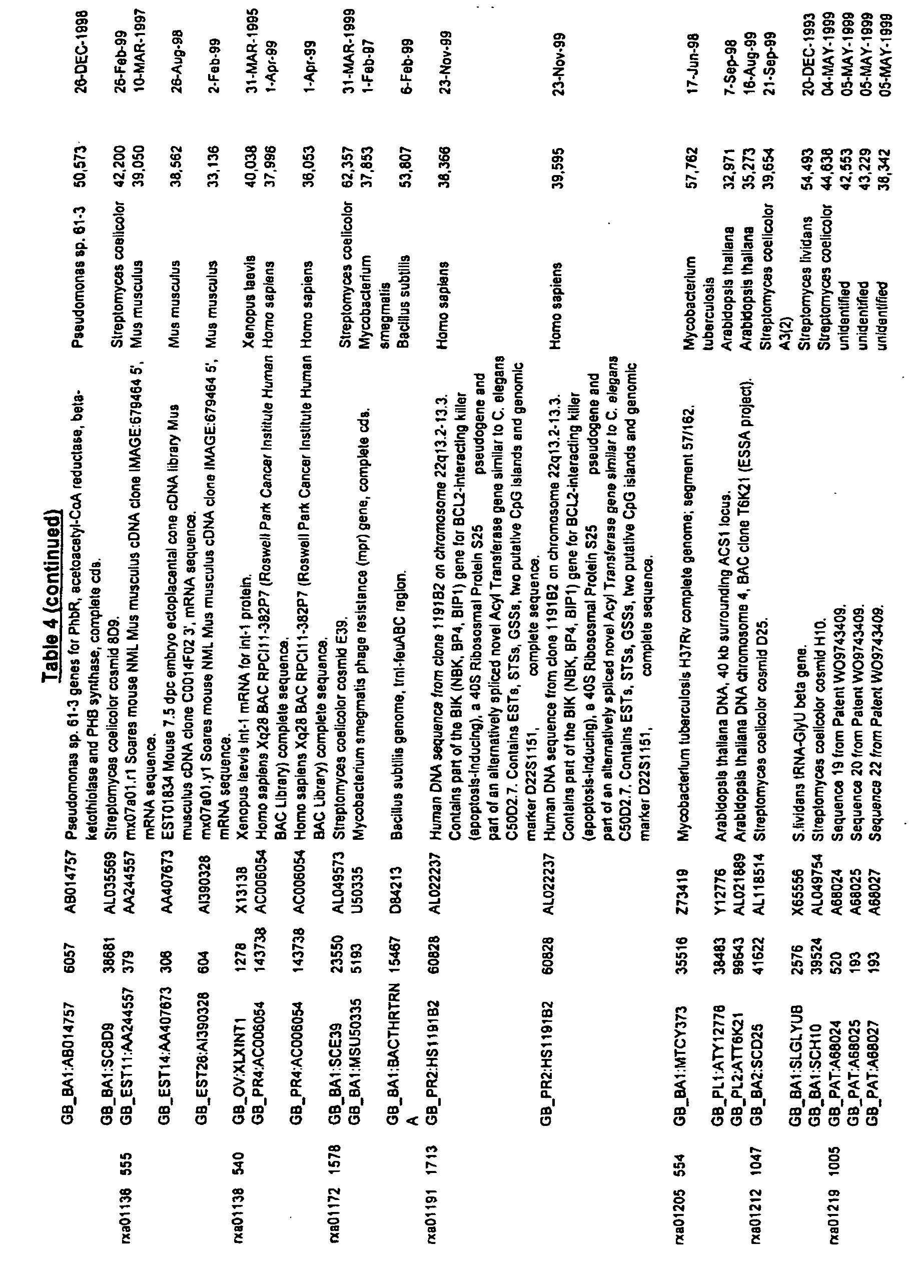

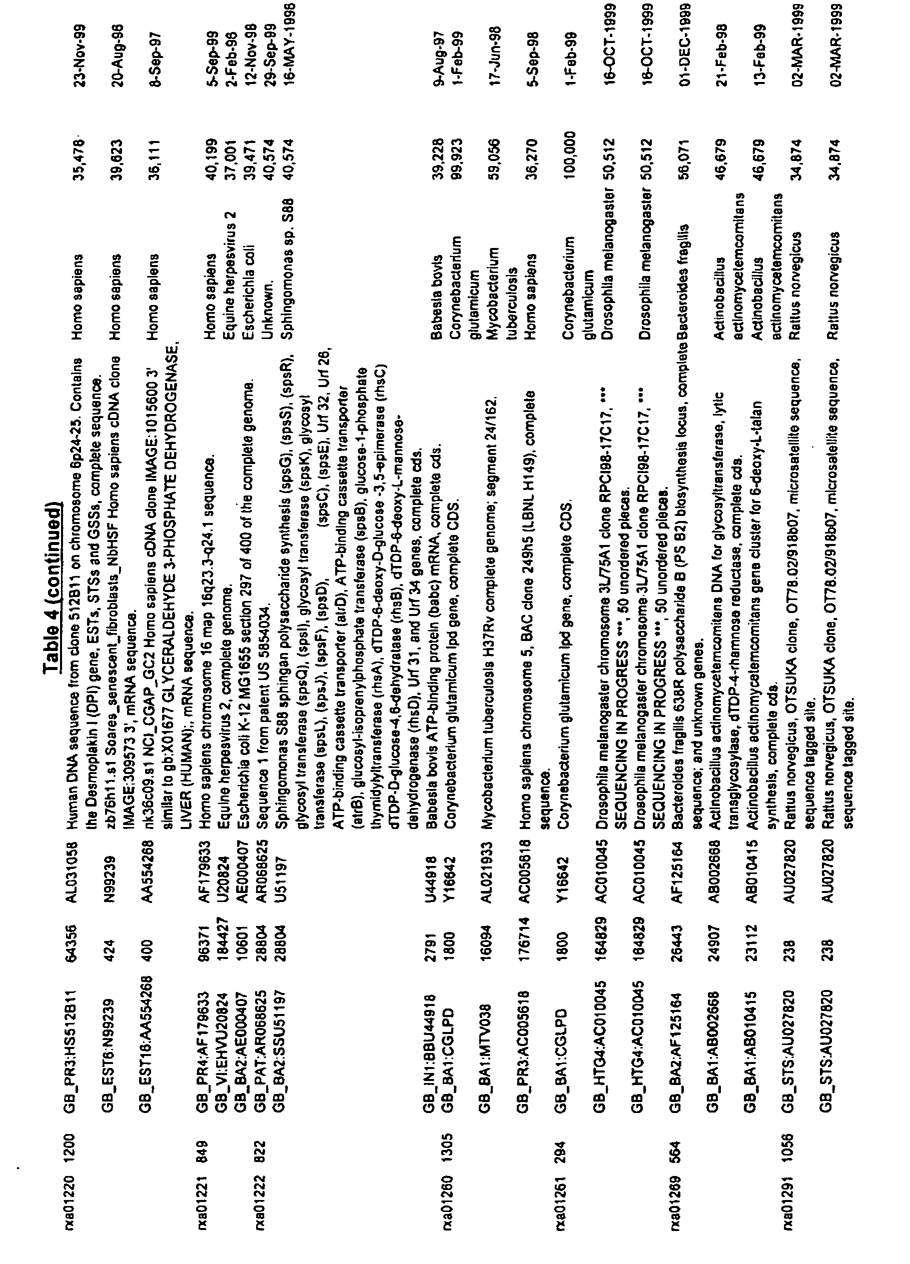

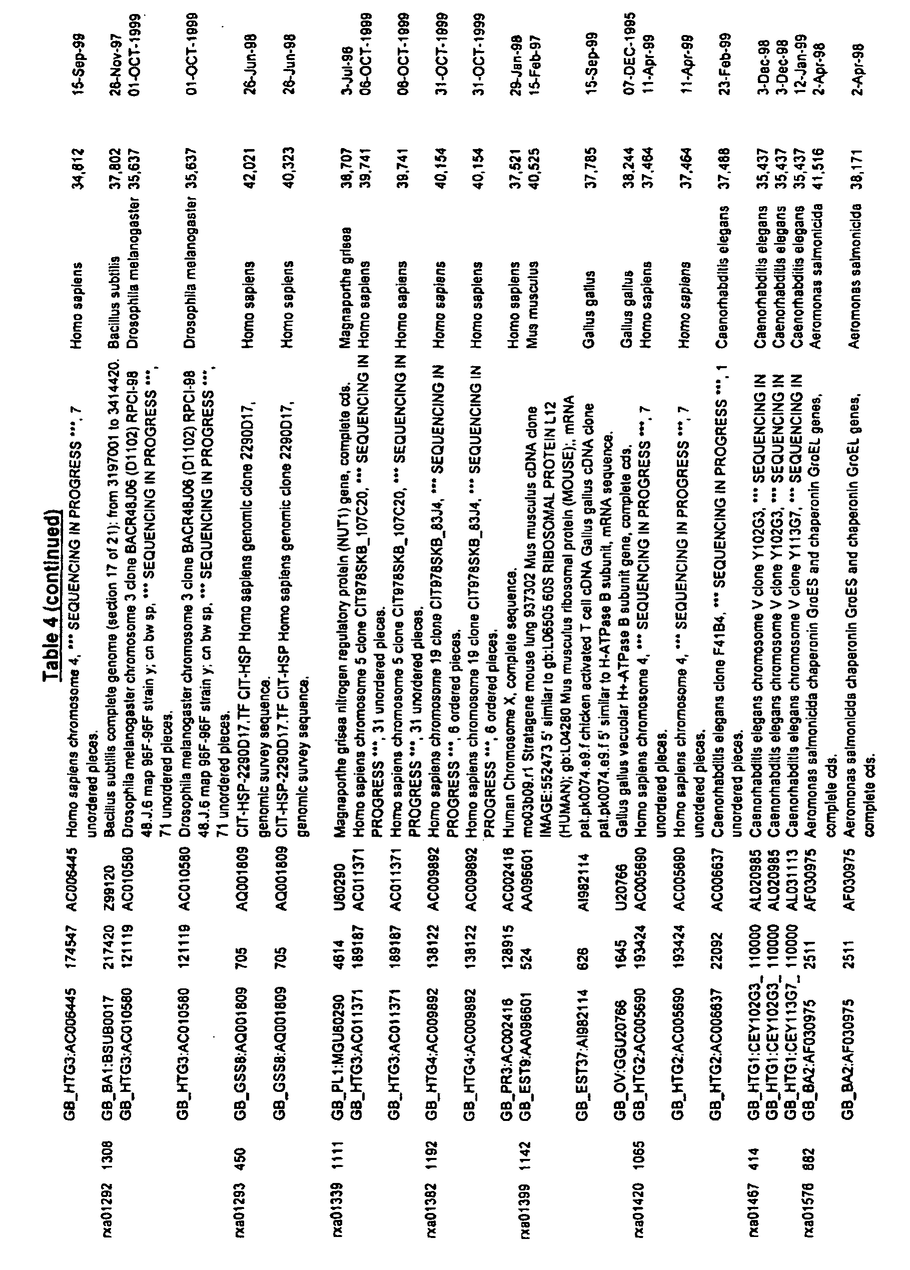

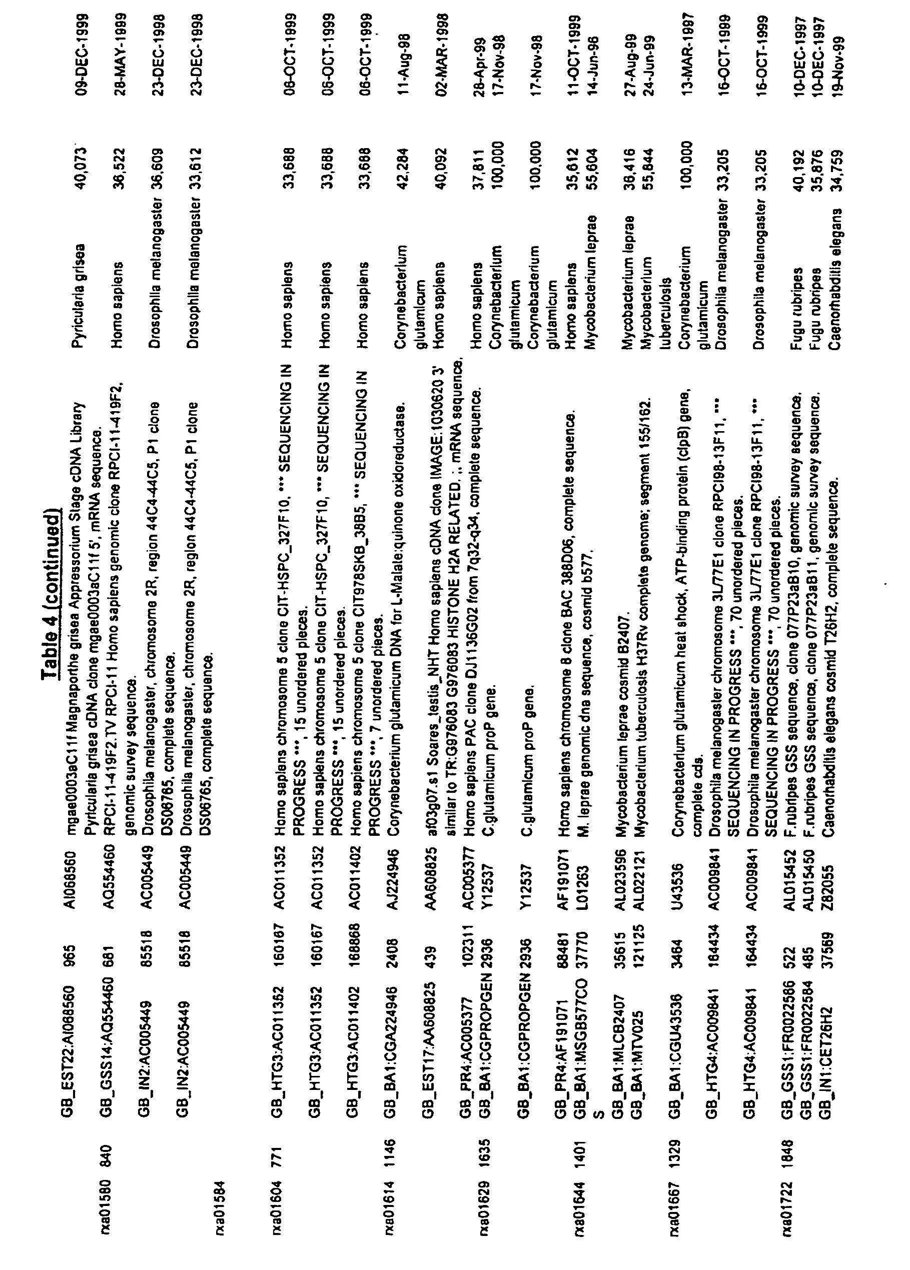

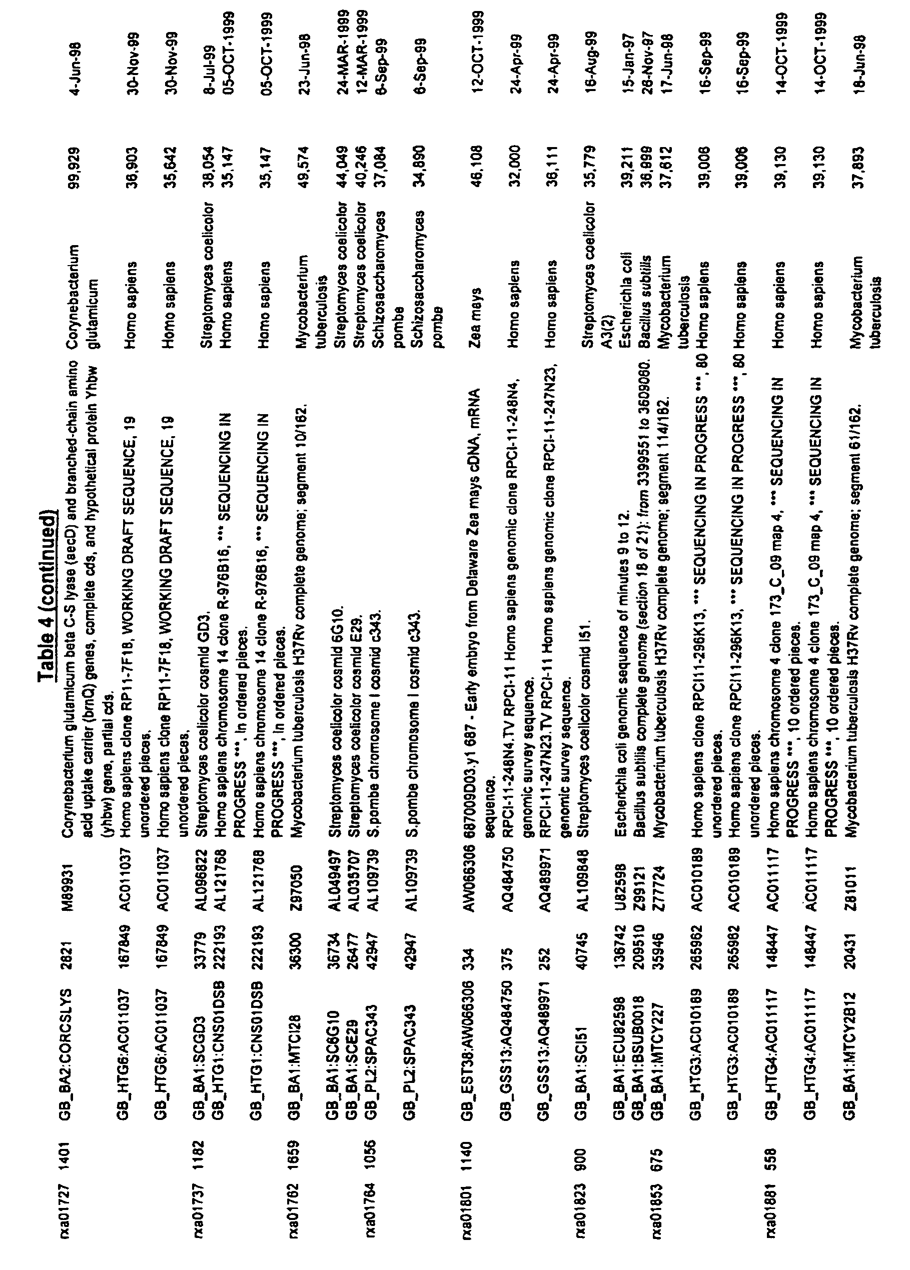

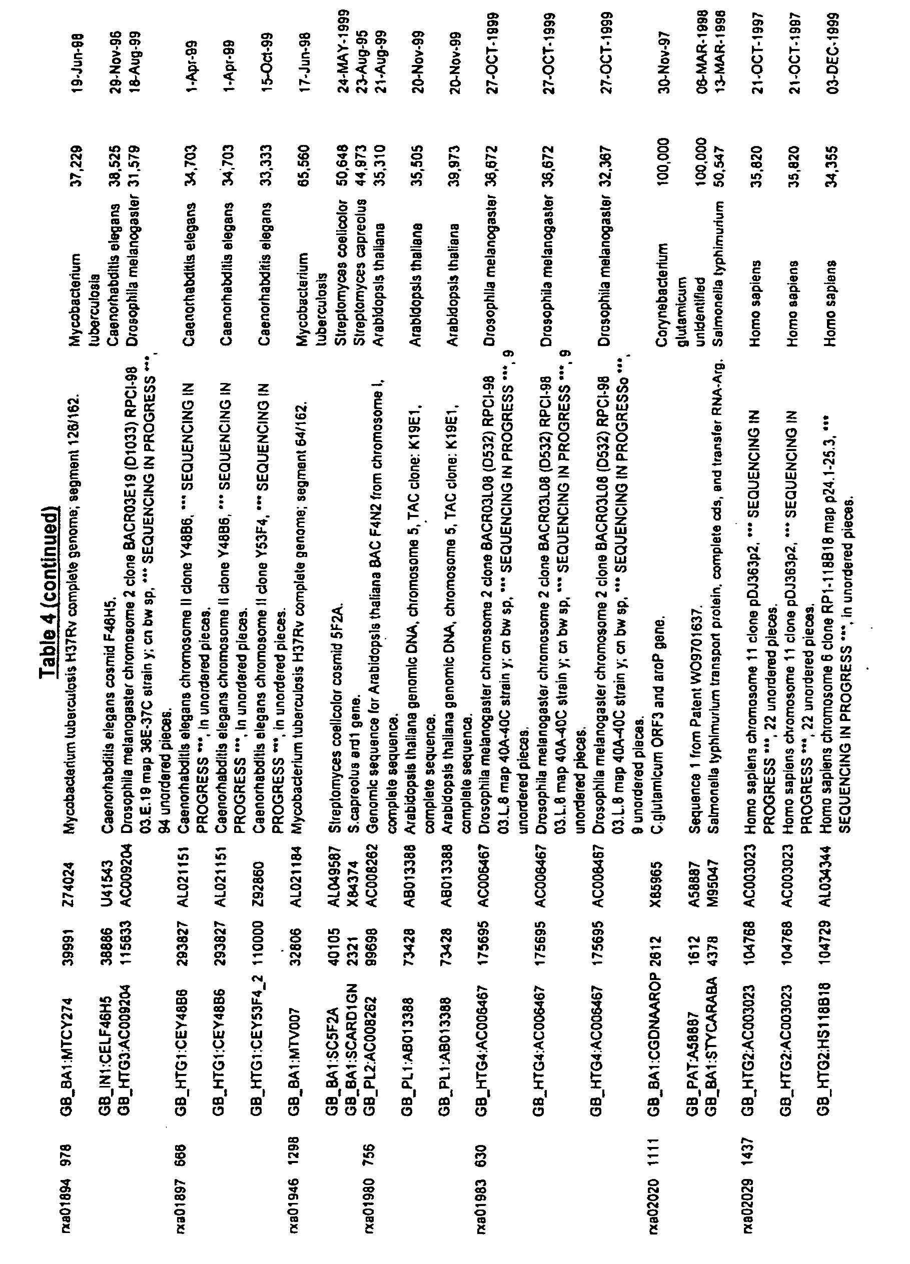

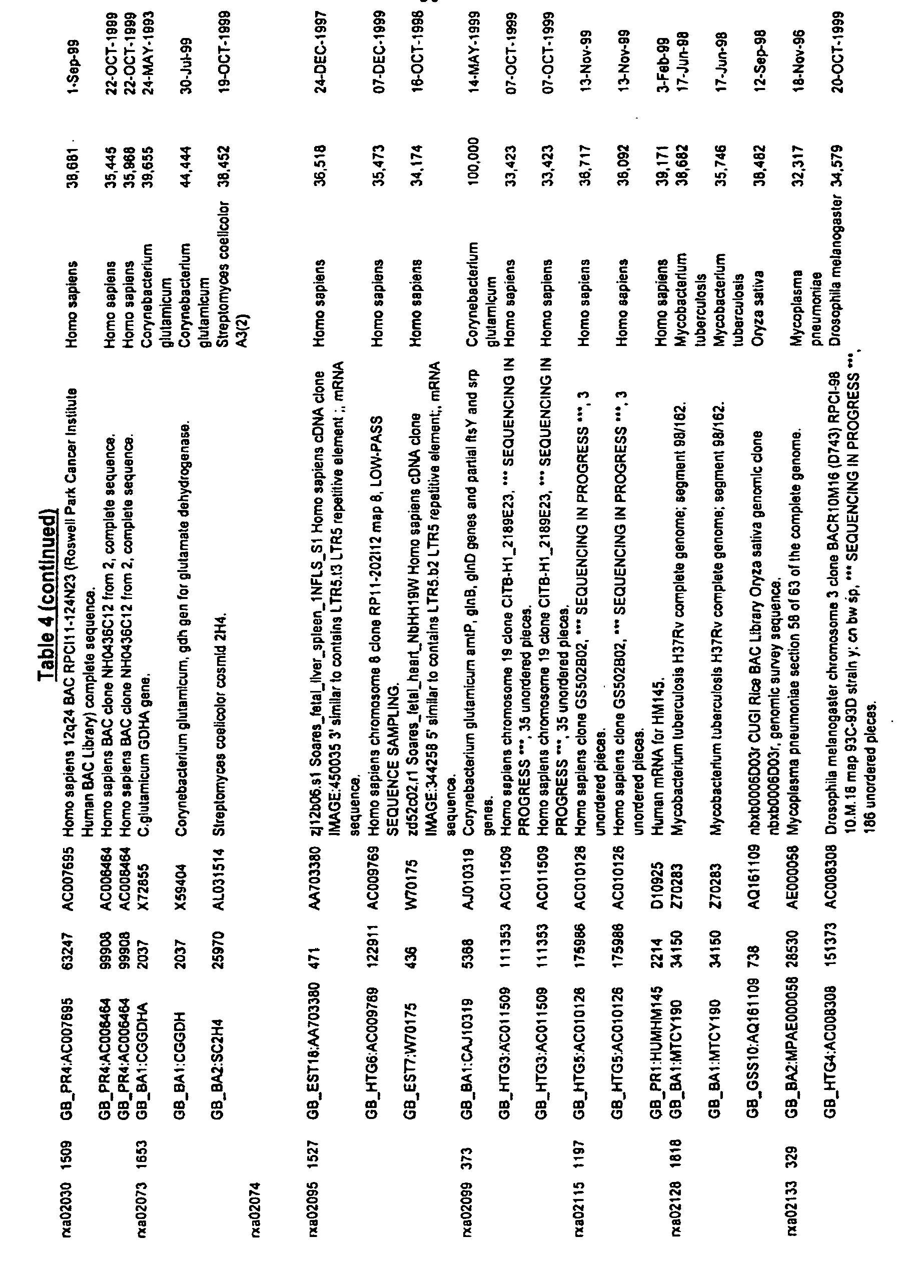

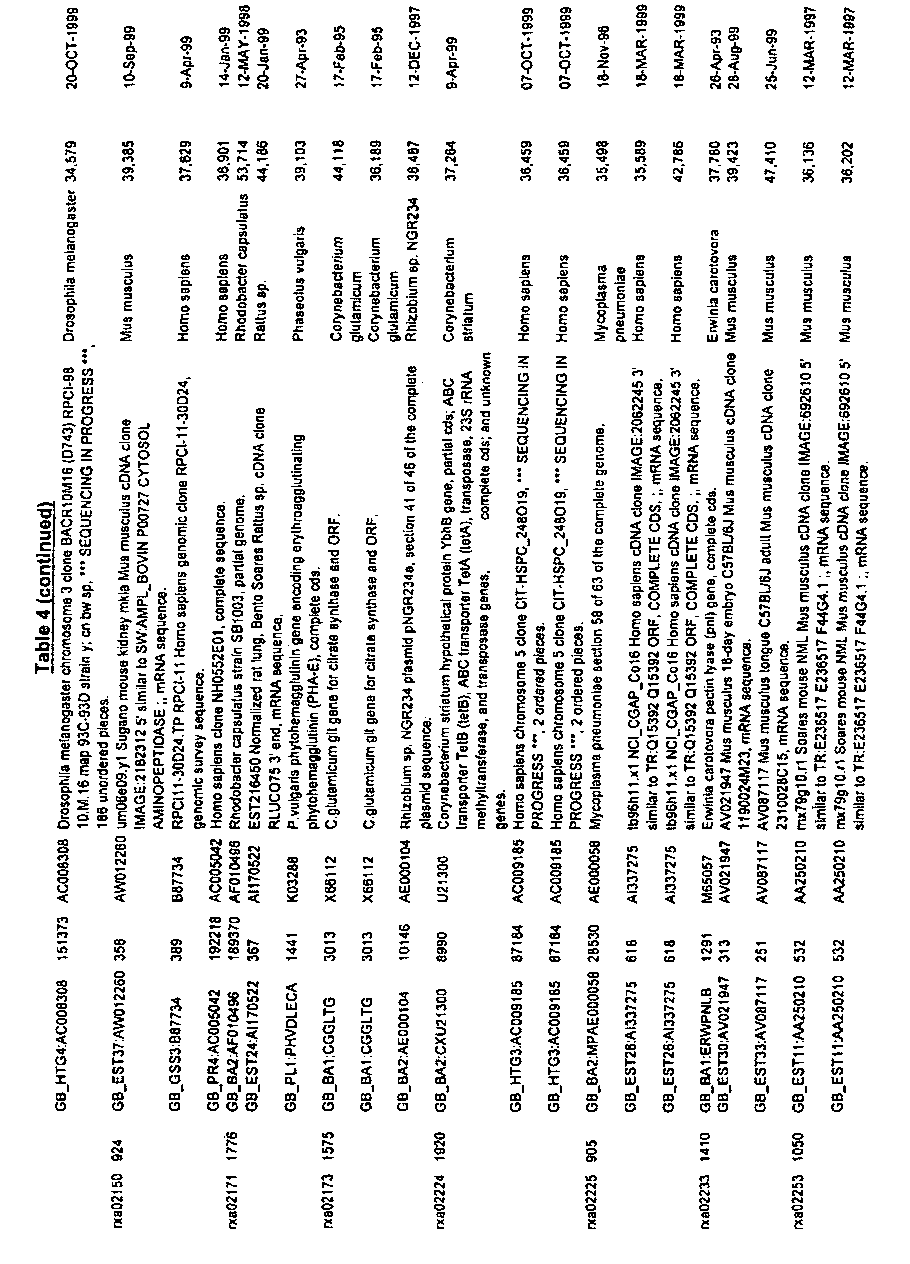

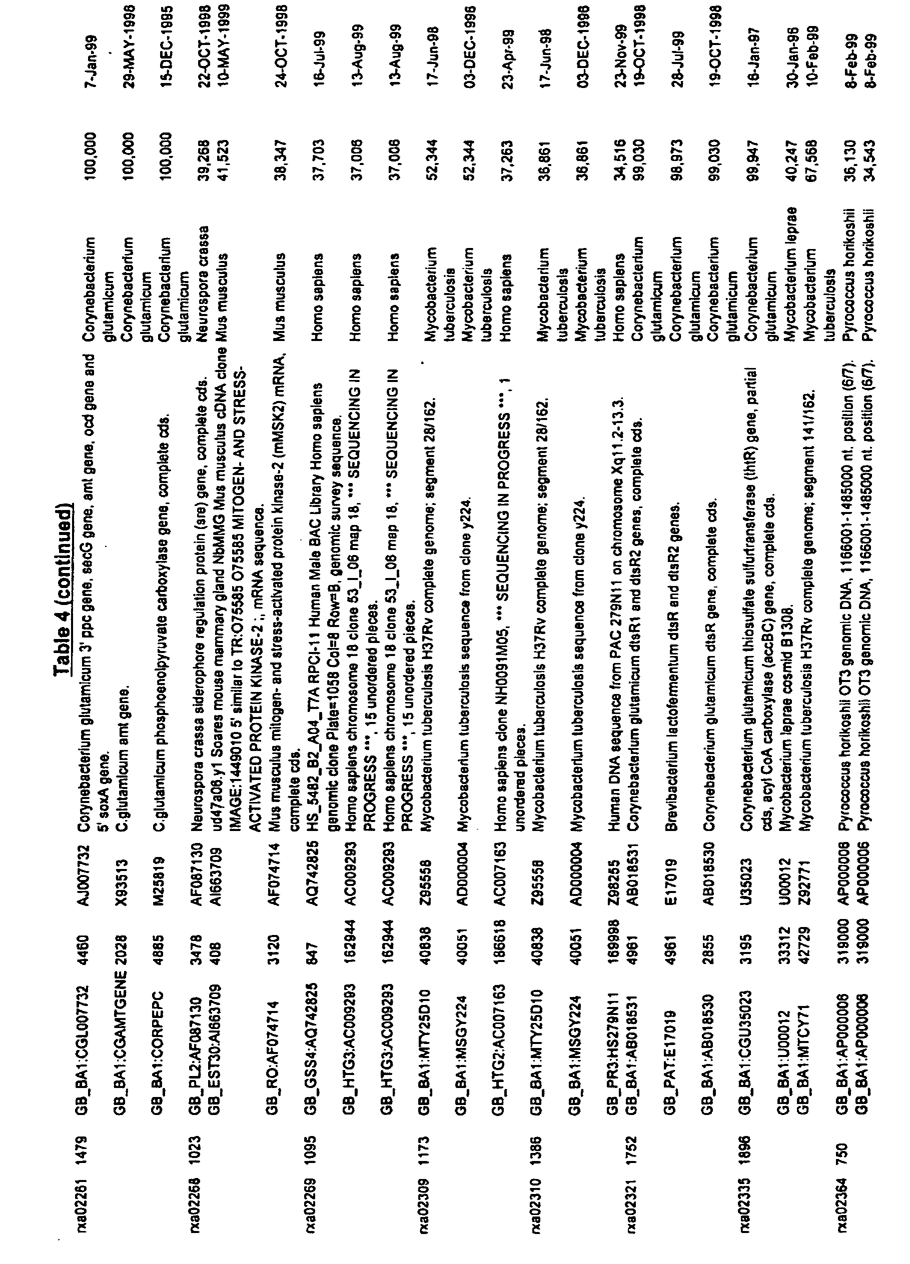

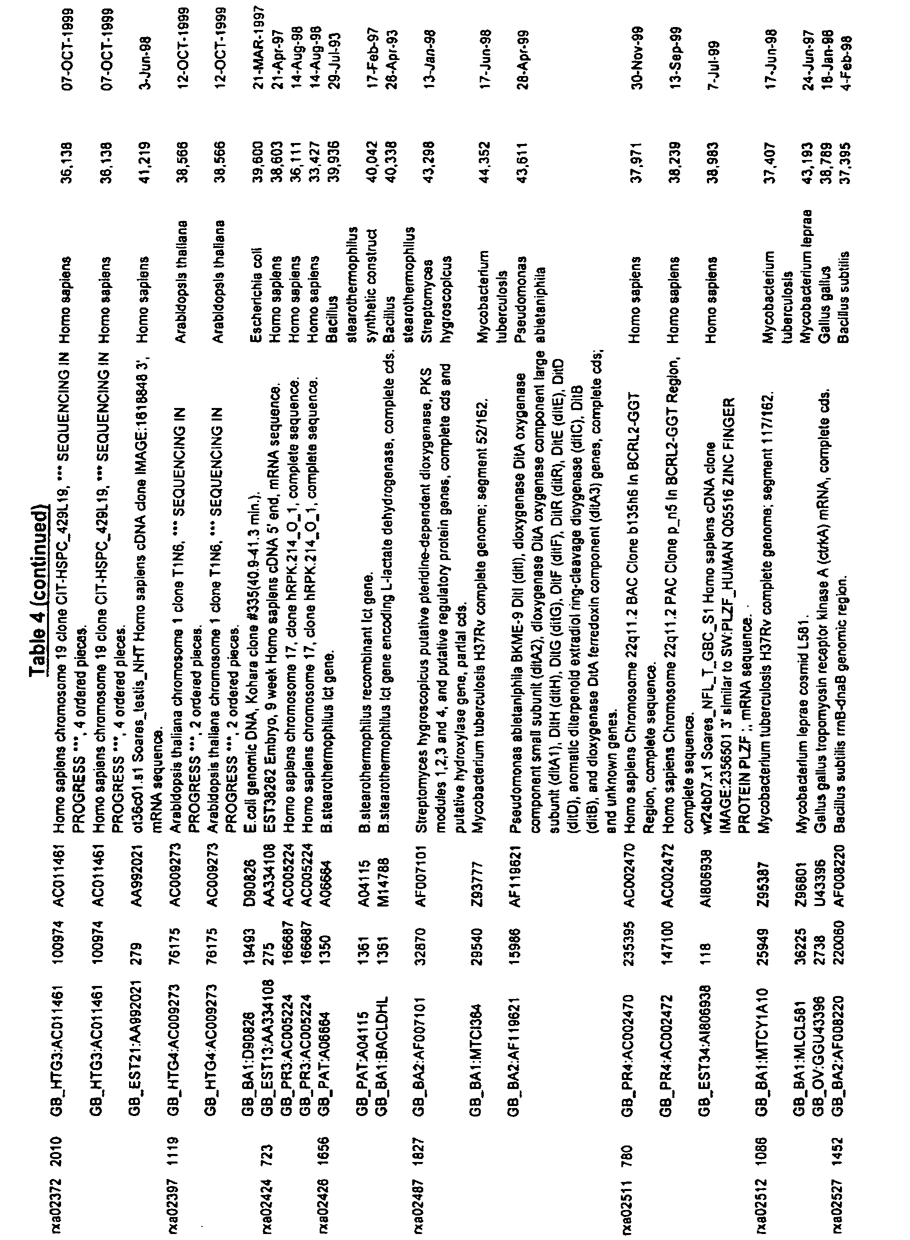

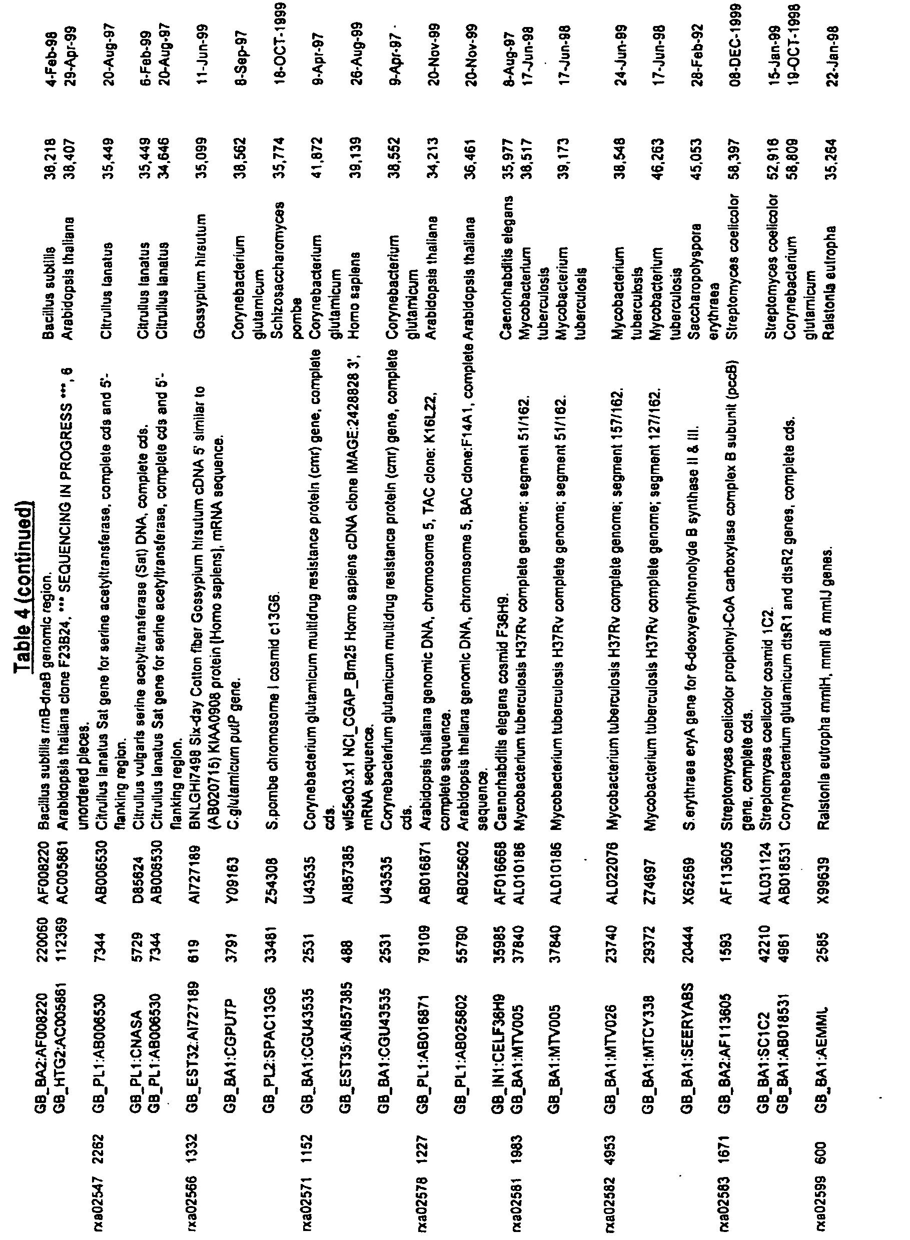

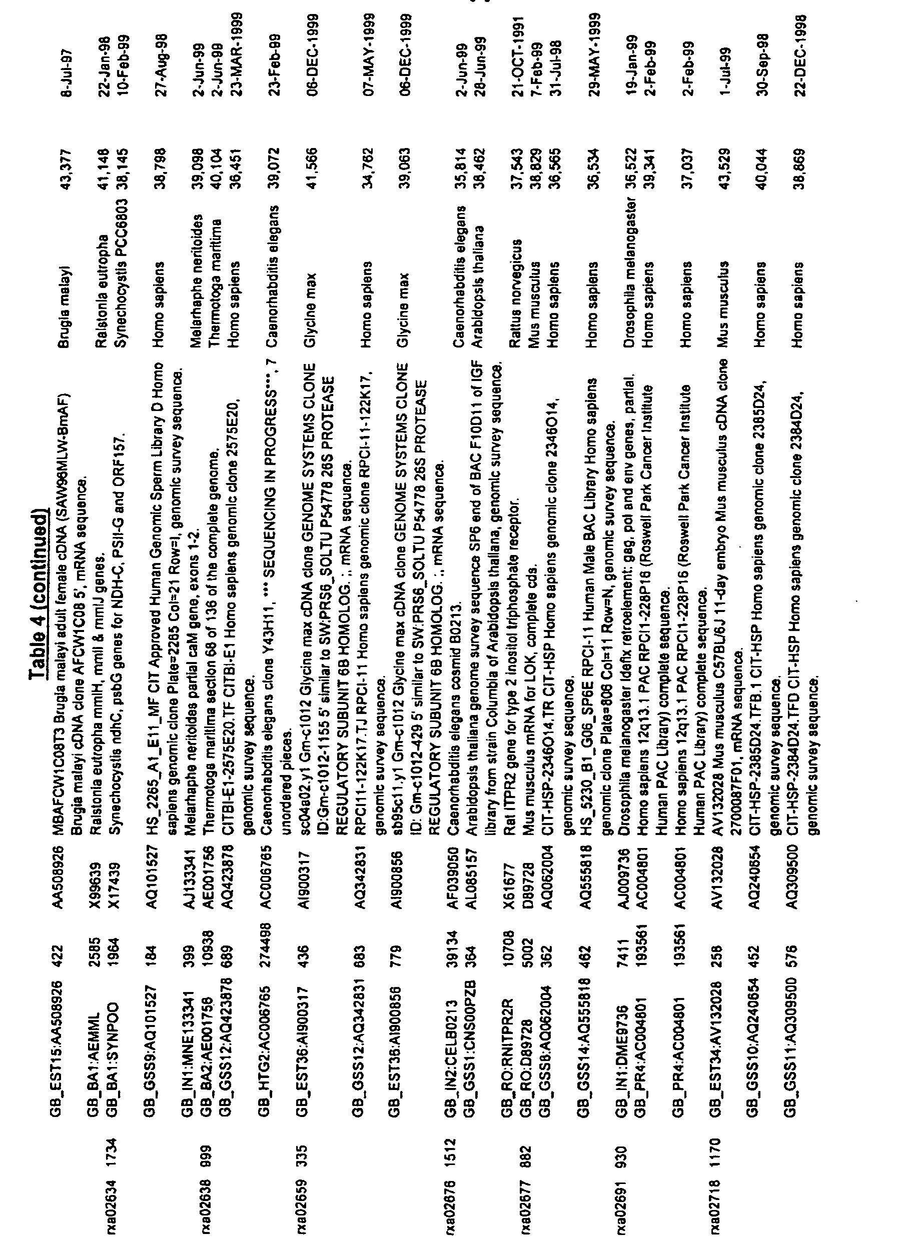

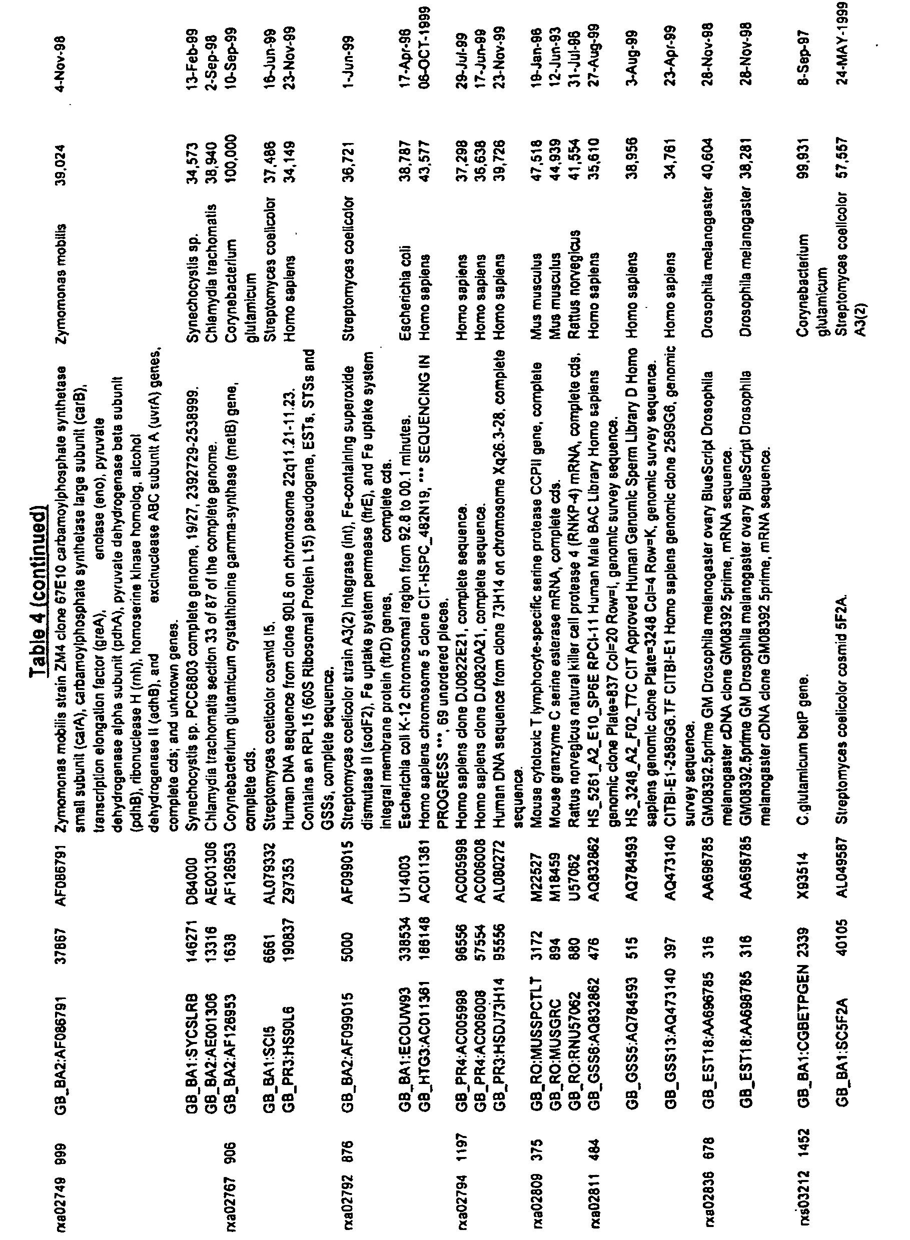

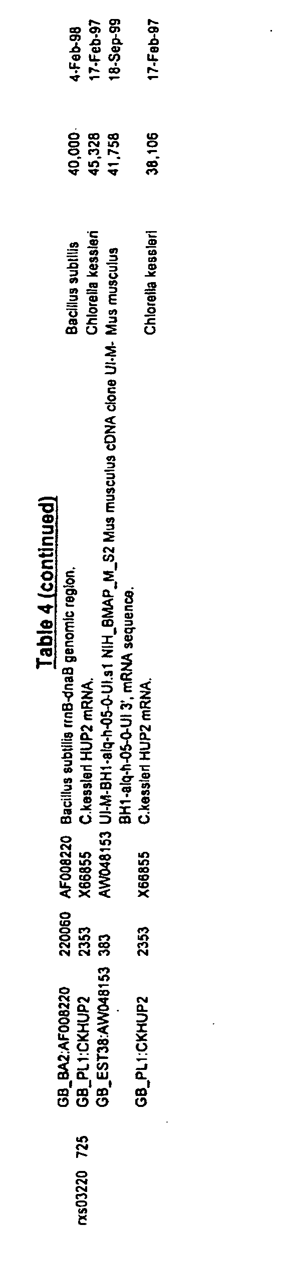

### Exemplification

### Example 1: Preparation of total genomic DNA of Corynebacterium glutamicum ATCC 13032

A culture of *Corynebacterium glutamicum* (ATCC 13032) was grown overnight at 30°C with vigorous shaking in BHI medium (Difco). The cells were harvested by centrifugation, the supernatant was discarded and the cells were resuspended in 5 ml buffer-I (5% of the original volume of the culture ― all indicated volumes have been calculated for 100 ml of culture volume). Composition of buffer-I: 140.34 g/l sucrose, 2.46 g/l M_{g}SO₄ x 7H₂O, 10 ml/l KH₂PO₄ solution (100 g/l, adjusted to pH 6.7 with KOH), 50 ml/l M12 concentrate (10 g/l (NH₄)₂SO₄, 1 g/l NaCl, 2 g/l MgSO₄ x 7H₂O, 0.2 g/l CaCl₂, 0.5 g/l yeast extract (Difco), 10 ml/l trace-elements-mix (200 mg/l FeSO₄ x H₂O, 10 mg/l ZnSO₄ x 7 H₂O, 3 mg/l MnCl₂ x 4 H₂O, 30 mg/l H₃BO₃ 20 mg/l CoCl, x 6 H₂O, 1 mg/l NiCl₂ x 6 H₂O, 3 mg/l Na₂MoO₄ x 2 H₂O, 500 mg/l complexing agent (EDTA or critic acid), 100 ml/l vitamins-mix (0.2 mg/l biotin, 0.2 mg/l folic acid, 20 mg/l p-amino benzoic acid, 20 mg/l riboflavin, 40 mg/l ca-panthothenate, 140 mg/l nicotinic acid, 40 mg/l pyridoxole hydrochloride, 200 mg/l myo-inositol). Lysozyme was added to the suspension to a final concentration of 2.5 mg/ml. After an approximately 4 h incubation at 37°C, the cell wall was degraded and the resulting protoplasts are harvested by centrifugation. The pellet was washed once with 5 ml buffer-I and once with 5 ml TE-buffer (10 mM Tris-HCl, 1 mM EDTA, pH 8). The pellet was resuspended in 4 ml TE-buffer and 0.5 ml SDS solution (10%) and 0.5 ml NaCl solution (5 M) are added. After adding of proteinase K to a final concentration of 200 µg/ml, the suspension is incubated for ca.18 h at 37°C. The DNA was purified by extraction with phenol, phenol-chloroform-isoamylalcohol and chloroform-isoamylalcohol using standard procedures. Then, the DNA was precipitated by adding 1/50 volume of 3 M sodium acetate and 2 volumes of ethanol, followed by a 30 min incubation at -20°C and a 30 min centrifugation at 12,000 rpm in a high speed centrifuge using a SS34 rotor (Sorvall). The DNA was dissolved in 1 ml TE-buffer containing 20 µg/ml RNaseA and dialysed at 4°C against 1000 ml TE-buffer for at least 3 hours. During this time, the buffer was exchanged 3 times. To aliquots of 0.4 ml of the dialysed DNA solution, 0.4 ml of 2 M LiCl and 0.8 ml of ethanol are added. After a 30 min incubation at -20°C, the DNA was collected by centrifugation (13,000 rpm, Biofuge Fresco, Heraeus, Hanau, Germany). The DNA pellet was dissolved in TE-buffer. DNA prepared by this procedure could be used for all purposes, including southern blotting or construction of genomic libraries.

### Example 2: Construction of genomic libraries in Escherichia coli of Corynebacterium glutamicum ATCC13032.

Using DNA prepared as described in Example 1, cosmid and plasmid libraries were constructed according to known and well established methods *(see e.g.,* Sambrook, J. *et al.* (1989) "Molecular Cloning : A Laboratory Manual", Cold Spring Harbor Laboratory Press, or Ausubel, F.M. *et al.* (1994) "Current Protocols in Molecular Biology", John Wiley & Sons.)

Any plasmid or cosmid could be used. Of particular use were the plasmids pBR322 (Sutcliffe, J.G. (1979) *Proc*. *Natl*. *Acad*. *Sci. USA*, 75:3737-3741); pACYC177 (Change & Cohen (1978) *J*: *Bocteriol* 134:1141-1156), plasmids of the pBS series (pBSSK+, pBSSK- and others; Stratagene, Lalolla, USA), or cosmids as SuperCosl (Stratagene, Lalolla, USA) or Lorist6 (Gibson, T.J., Rosenthal A. and Waterson, R.H. (1987) *Gene* 53:283-286. Gene libraries specifically for use in *C*. *glutamicum* may be constructed using plasmid pSL109 (Lee, H.-S. and A. J. Sinskey (1994) *J. Microbiol. Biotechnol.* 4: 256-263).

### Example 3: DNA Sequencing and Computational Functional Analysis

Genomic libraries as described in Example 2 were used for DNA sequencing according to standard methods, in particular by the chain termination method using ABI377 sequencing machines (see e.g., Fleischman, R.D. *et al*. (1995) "Whole-genome Random Sequencing and Assembly of Haemophilus Influenzae Rd., *Science,* 269:496-512). Sequencing primers with the following nucleotide sequences were used: 5'-GGAAACAGTATGACCATG-3' or 5'-GTAAAACGACGGCCAGT-3'.

### Example 4: In vivo Mutagenesis

*In vivo* mutagenesis of *Corynebacterium glutamicum* can be performed by passage of plasmid (or other vector) DNA through *E. coli* or other microorganisms (*e*.*g*. *Bacillus* spp. or yeasts such as *Saccharomyces cerevisiae)* which are impaired in their capabilities to maintain the integrity of their genetic information. Typical mutator strains have mutations in the genes for the DNA repair system (*e*.*g*., mutHLS, mutD, mutT, etc.; for reference, see Rupp, W.D. (1996) DNA repair mechanisms, in: *Escherichia coli* and *Salmonella,* p. 2277-2294, ASM: Washington.) Such strains are well known to those of ordinary skill in the art. The use of such strains is illustrated, for example, in Greener, A. and Callahan, M. (1994) *Strategies* 7: 32-34.

### Example 5: DNA Transfer Between Escherichia coli and Corynebacterium glutamicum

Several *Corynebacterium* and *Brevibacterium* species contain endogenous plasmids (as e.g., pHM1519 or pBL1) which replicate autonomously (for review see, *e.g.,* Martin, J.F. *et al*. (1987) *Biotechnology,* 5:137-146). Shuttle vectors for *Escherichia coli* and *Corynebacterium glutamicum* can be readily constructed by using standard vectors for *E. coli* (Sambrook, J. *et al*. (1989), "Molecular Cloning: A Laboratory Manual", Cold Spring Harbor Laboratory Press or Ausubel, F.M. *et al*. (1994) "Current Protocols in Molecular Biology", John Wiley & Sons) to which a origin or replication for and a suitable marker from *Corynebacterium glutamicum* is added. Such origins of replication are preferably taken from endogenous plasmids isolated from *Corynebacterium* and *Brevibacterium* species. Of particular use as transformation markers for these species are genes for kanamycin resistance (such as those derived from the Tn5 or Tn903 transposons) or chloramphenicol (Winnacker, E.L. (1987) "From Genes to Clones ― Introduction to Gene Technology, VCH, Weinheim). There are numerous examples in the literature of the construction of a wide variety of shuttle vectors which replicate in both E. *coli and C. glutamicum.* and which can be used for several purposes, including gene overexpression (for reference, see *e.g.,* Yoshihama, M. *et al*. (1985) *J*. *Bacteriol.* 162:591-597, Martin J.F. *et al*. (1987) *Biotechnology,* 5:137-146 and Eikmanns, B.J. *et al*. (1991) *Gene,* 102:93-98).
Using standard methods, it is possible to clone a gene of interest into one of the shuttle vectors described above and to introduce such a hybrid vectors into strains of *Corynebacterium glutamicum.* Transformation of *C*. *glutamicum* can be achieved by protoplast transformation (Kastsumata, R. *et al*. (1984) *J. Bacteriol.* 159306-311), electroporation (Liebl, E. *et al.* (1989) *FEMS Microbiol. Letters,* 53:399-303) and in cases where special vectors are used, also by conjugation (as described e.g. in Schäfer, A *et al*. (1990) *J. Bacteriol.* 172:1663-1666). It is also possible to transfer the shuttle vectors for *C*. *glutamicum* to *E. coli* by preparing plasmid DNA from *C. glutamicum* (using standard methods well-known in the art) and transforming it into *E*. *coli.* This transformation step can be performed using standard methods, but it is advantageous to use an Mcr-deficient *E. coli* strain, such as NM522 (Gough & Murray (1983) *J. Mol. Biol.* 166:1-19).

Genes may be overexpressed in *C glulamicum* strains using plasmids which comprise pCG (U.S. Patent No. 4,617,267) or fragments thereof, and optionally the gene for kanamycin resistance from TN903 (Grindley, N.D. and Joyce, C.M. (1980) *Proc. Natl. Acad Sci. USA* 77(12): 7176-7180). In addition, genes may be overexpressed in *C. glutamicum* strains using plasmid pSL109 (Lee, H.-S. and A. J. Sinskey (1994) *J. Microbiol Biotechnol.* 4: 256-263).

Aside from the use of replicative plasmids, gene overexpression can also be achieved by integration into the genome. Genomic integration in *C*. *glutamicum* or other Corynebacterium or Brevibacterium species may be accomplished by well-known methods, such as homologous recombination with genomic region(s), restriction endonuclease mediated integration (REMI) (see, *e.g.,* DE Patent 19823834), or through the use of transposons. It is also possible to modulate the activity of a gene of interest by modifying the regulatory regions (*e*.*g*., a promoter, a repressor, and/or an enhancer) by sequence modification, insertion, or deletion using site-directed methods (such as homologous recombination) or methods based on random events (such as transposon mutagenesis or REMI). Nucleic acid sequences which function as transcriptional terminators may also be inserted 3' to the coding region of one or more genes of the invention; such terminators are well-known in the art and are described, for example, in Winnacker, E.L. (1987) From Genes to Clones - Introduction to Gene Technology. VCH: Weinheim.

### Example 6: Assessment of the Expression of the Mutant Protein

Observations of the activity of a mutated protein in a transformed host cell rely on the fact that the mutant protein is expressed in a similar fashion and in a similar quantity to that of the wild-type protein. A useful method to ascertain the level of transcription of the mutant gene (an indicator of the amount of mRNA available for translation to the gene product) is to perform a Northern blot (for reference see, for example, Ausubel *et al*. (1988) Current Protocols in Molecular Biology, Wiley: New York), in which a primer designed to bind to the gene of interest is labeled with a detectable tag (usually radioactive or chemiluminescent), such that when the total RNA of a culture of the organism is extracted, run on gel, transferred to a stable matrix and incubated with this probe, the binding and quantity of binding of the probe indicates the presence and also the quantity of mRNA for this gene. This information is evidence of the degree of transcription of the mutant gene. Total cellular RNA can be prepared from *Corynebacterium glutamicum* by several methods, all well-known in the art, such as that described in Bormann, E.R. *et al*. (1992) *Mol. Microbiol.* 6: 317-326.

To assess the presence or relative quantity of protein translated from this mRNA, standard techniques, such as a Western blot, may be employed (see, for example, Ausubel *et al*. (1988) Current Protocols in Molecular Biology, Wiley: New York). In this process, total cellular proteins are extracted, separated by gel electrophoresis, transferred to a matrix such as nitrocellulose, and incubated with a probe, such as an antibody, which specifically binds to the desired protein. This probe is generally tagged with a chemiluminescent or colorimetric label which may be readily detected. The presence and quantity of label observed indicates the presence and quantity of the desired mutant protein present in the cell.

### Example 7: Growth of Genetically Modified Corynebacterium glutamicum ― Media and Culture Conditions

Genetically modified *Corynebacteria* are cultured in synthetic or natural growth media. A number of different growth media for Corynebacteria are both well-known and readily available (Lieb *et al*. (1989) *Appl. Microbiol. Biotechnol.,* 32:205-210; von der Osten *et al*. (1998) *Biotechnology Letters,* 11:11-16; Patent DE 4,120,867; Liebl (1992) "The Genus *Corynebacterium,* in: The Procaryotes, Volume II, Balows, A. *et al*., eds. Springer-Verlag). These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribulose, lactose, maltose, sucrose, raffinose, starch or cellulose serve as very good carbon sources. It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂SO₄, NH₄OH, nitrates, urea, amino acids or complex nitrogen sources like corn steep liquor, soy bean flour, soy bean protein, yeast extract, meat extract and others.

Inorganic salt compounds which may be included in the media include the chloride-, phosphorous- or sulfate- salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamin, folic acid, nicotinic acid, pantothenate and pyridoxin. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (*eds.* P.M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components are sterilized, either by heat (20 minutes at 1.5 bar and 121'C) or by sterile filtration. The components can either be sterilized together or, if necessary, separately. All media components can be present at the beginning of growth, or they can optionally be added continuously or batchwise.

Culture conditions are defined separately for each experiment. The temperature should be in a range between 15°C and 45°C. The temperature can be kept constant or can be altered during the experiment. The pH of the medium should be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of NaOH or NH₄OH during growth. If complex medium components such as yeast extract are utilized, the necessity for additional buffers may be reduced, due to the fact that many complex compounds have high buffer capacities. If a fermentor is utilized for culturing the microorganisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml shake flasks are used, filled with 10% (by volume) of the required growth medium. The flasks should be shaken on a rotary shaker (amplitude 25 mm) using a speed-range of 100 - 300 rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD₆₀₀ of O.5 - 1.5 using cells grown on agar plates, such as CM plates (10 g/l glucose, 2,5 g/l NaCl, 2 g/l urea, 10 g/l polypeptone, 5 g/l yeast extract, 5 g/l meat extract, 22 g/l NaCl, 2 g/l urea, 10 g/l polypeptone, 5 g/l yeast extract, 5 g/l meat extract, 22 g/l agar, pH 6.8 with 2M NaOH) that had been incubated at 30°C. Inoculation of the media is accomplished by either introduction of a saline suspension of *C*. *glutamicum* cells from CM plates or addition of a liquid preculture of this bacterium.

### Example 8 -In vitro Analysis of the Function of Mutant Proteins

The determination of activities and kinetic parameters of enzymes is well established in the art. Experiments to determine the activity of any given altered enzyme must be tailored to the specific activity of the wild-type enzyme, which is well within the ability of one of ordinary skill in the art. Overviews about enzymes in general, as well as specific details concerning structure, kinetics, principles, methods, applications and examples for the determination of many enzyme activities may be found, for example, in the following references: Dixon, M., and Webb, E.C., (1979) Enzymes. Longmans: London; Fersht, (1985) Enzyme Structure and Mechanism. Freeman: New York; Walsh, (1979) Enzymatic Reaction Mechanisms. Freeman: San Francisco; Price, N.C., Stevens, L. (1982) Fundamentals of Enzymology. Oxford Univ. Press: Oxford; Boyer, P.D., ed. (1983) The Enzymes, 3^{rd} ed. Academic Press: New York; Bisswanger, H., (1994) Enzymkinetik, 2^{nd} ed. VCH: Weinheim (ISBN 3527300325); Bergmeyer, H.U., Bergmeyer, J., Graß1, M., eds. (1983-1986) Methods of Enzymatic Analysis, 3^{rd} ed., vol. I-XII, Verlag Chemie: Weinheim; and Ullmann's Encyclopedia of Industrial Chemistry (1987) vol. A9, "Enzymes". VCH: Weinheim, p. 352-363.

The activity of proteins which bind to DNA can be measured by several well-established methods, such as DNA band-shift assays (also called gel retardation assays). The effect of such proteins on the expression of other molecules can be measured using reporter gene assays (such as that described in Kolmar, H. *et al*. (1995) *EMBO J.* 14: 3895-3904 and references cited therein). Reporter gene test systems are well known and established for applications in both pro- and eukaryotic cells, using enzymes such as beta-galactosidase, green fluorescent protein, and several others.

The determination of activity of membrane-transport proteins can be performed according to techniques such as those described in Gennis, R.B. (1989) "Pores, Channels and Transporters", in Biomembranes, Molecular Structure and Function, Springer: Heidelberg, p. 85-137; 199-234; and 270-322.

### Example 9: Analysis of Impact of Mutant Protein on the Production of the Desired Product

The effect of the genetic modification in *C*. *glutamicum* on production of a desired compound (such as an amino acid) can be assessed by growing the modified microorganism under suitable conditions (such as those described above) and analyzing the medium and/or the cellular component for increased production of the desired product (*i*.*e*., an amino acid). Such analysis techniques are well known to one of ordinary skill in the art, and include spectroscopy, thin layer chromatography, staining methods of various kinds, enzymatic and microbiological methods, and analytical chromatography such as high performance liquid chromatography (see, for example, Ullman, Encyclopedia of Industrial Chemistry, vol. A2, p. 89-90 and p. 443-613, VCH: Weinheim (1985); Fallon, A. *et al*., (1987) "Applications of HPLC in Biochemistry" in: Laboratory Techniques in Biochemistry and Molecular Biology, vol. 17; Rehm *et al*. (1993) Biotechnology, vol. 3, Chapter III: "Product recovery and purification", page 469-714, VCH: Weinheim; Belter, P.A. *et al*. (1988) Bioseparations: downstream processing for biotechnology, John Wiley and Sons; Kennedy, J.F. and Cabral, J.M.S. (1992) Recovery processes for biological materials, John Wiley and Sons; Shaeiwitz, J.A. and Henry, J.D. (1988) Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications.)

In addition to the measurement of the final product of fermentation, it is also possible to analyze other components of the metabolic pathways utilized for the production of the desired compound, such as intermediates and side-products, to determine the overall efficiency of production of the compound. Analysis methods include measurements of nutrient levels in the medium (*e*.*g*., sugars, hydrocarbons, nitrogen sources, phosphate, and other ions), measurements of biomass composition and growth, analysis of the production of common metabolites of biosynthetic pathways, and measurement of gasses produced during fermentation. Standard methods for these measurements are outlined in Applied Microbial Physiology, A Practical Approach, P.M. Rhodes and P.F. Stanbury, eds., IRL Press, p. 103-129; 131-163; and 165-192 (ISBN: 0199635773) and references cited therein.

### Example 10: Purification of the Desired Product from C. glutamicum Culture

Recovery of the desired product from the *C*. *glutamicum* cells or supernatant of the above-described culture can be performed by various methods well known in the art. If the desired product is not secreted from the cells, the cells can be harvested from the culture by low-speed centrifugation, the cells can be lysed by standard techniques, such as mechanical force or sonication. The cellular debris is removed by centrifugation, and the supernatant fraction containing the soluble proteins is retained for further purification of the desired compound. If the product is secreted from the *C*. *glutamicum* cells, then the cells are removed from the culture by low-speed centrifugation, and the supernate fraction is retained for further purification.

The supernatant fraction from either purification method is subjected to chromatography with a suitable resin, in which the desired molecule is either retained on a chromatography resin while many of the impurities in the sample are not, or where the impurities are retained by the resin while the sample is not. Such chromatography steps may be repeated as necessary, using the same or different chromatography resins. One of ordinary skill in the art would be well-versed in the selection of appropriate chromatography resins and in their most efficacious application for a particular molecule to be purified. The purified product may be concentrated by filtration or ultrafiltration, and stored at a temperature at which the stability of the product is maximized.

There are a wide array of purification methods known to the art and the preceding method of purification is not meant to be limiting. Such purification techniques are described, for example, in Bailey, J.E. & Ollis, D.F. Biochemical Engineering Fundamentals, McGraw-Hill: New York (1986).

The identity and purity of the isolated compounds may be assessed by techniques standard in the art. These include high-performance liquid chromatography (HPLC), spectroscopic methods, staining methods, thin layer chromatography, NIRS, enzymatic assay, or microbiologically. Such analysis methods are reviewed in: Patek *et al.* (1994) *Appl. Environ. Microbiol.* 60: 133-140; Malakhova *et al.* (1996) *Biotekhnologiya* 11: 27-32; and Schmidt *et al*. (1998) *Bioprocess Engineer.* 19: 67-70. Ulmann's Encyclopedia ofIndustrial Chemistry, (1996) vol. A27, VCH: Weinheim, p. 89-90, p. 521-540, p. 540-547, p. 559-566, 575-581 and p. 581-587; Michal, G. (1999) Biochemical Pathways: An Atlas of Biochemistry and Molecular Biology, John Wiley and Sons; Fallon, A. *et al*. (1987) Applications ofHPLC in Biochemistry in: Laboratory Techniques in Biochemistry and Molecular Biology, vol. 17.

### Example 11: Analysis of the Gene Sequences of the Invention

The comparison of sequences and determination of percent homology between two sequences are art-known techniques, and can be accomplished using a mathematical algorithm, such as the algorithm of Karlin and Altschul (1990) *Proc. Natl. Acad Sci.* USA 87:2264-68, modified as in Karlin and Altschul (1993) *Proc. Natl. Acad Sci.* USA 90:5873-77. Such an algorithm is incorporated into the NBLAST and XBLAST programs (version 2.0) of Altschul, *et al*. (1990) *J*. *Mol. Biol.* 215:403-10. BLAST nucleotide searches can be performed with the NBLAST program, score = 100, wordlength = 12 to obtain nucleotide sequences homologous to MCT nucleic acid molecules of the invention. BLAST protein searches can be performed with the XBLAST program, score = 50, wordlength = 3 to obtain amino acid sequences homologous to MCT protein molecules of the invention. To obtain gapped alignments for comparison purposes, Gapped BLAST can be utilized as described in Altschul *et al*., (1997) *Nucleic Acids Res.* 25(17):3389-3402. When utilizing BLAST and Gapped BLAST programs, one of ordinary skill in the art will know how to optimize the parameters of the program (*e*.*g*., XBLAST and NBLAST) for the specific sequence being analyzed.

Another example of a mathematical algorithm utilized for the comparison of sequences is the algorithm of Meyers and Miller ((1988) *Comput. Appl. Biosci.* 4: 11-17). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art, and include ADVANCE and ADAM. described in Torelli and Robotti (1994) *Comput. Appl. Biosci.* 10:3-5; and FASTA, described in Pearson and Lipman (1988) *P.N.A.S.* 85:2444-8.

The percent homology between two amino acid sequences can also be accomplished using the GAP program in the GCG software package (available at http://www.gcg.com), using either a Blosum 62 matrix or a PAM250 matrix, and a gap weight of 12,10, 8,6, or 4 and a length weight of 2, 3, or 4. The percent homology between two nucleic acid sequences can be accomplished using the GAP program in the GCG software package, using standard parameters, such as a gap weight of 50 and a length weight of 3.

A comparative analysis of the gene sequences of the invention with those present in Genbank has been performed using techniques known in the art (see, *e*.*g*., Bexevanis and Ouellette, eds. (1998) Bioinformatics: A Practical Guide to the Analysis of Genes and Proteins. John Wiley and Sons: New York). The gene sequences of the invention were compared to genes present in Genbank in a three-step process. In a first step, a BLASTN analysis (*e*.*g*., a local alignment analysis) was performed for each of the sequences of the invention against the nucleotide sequences present in Genbank, and the top 500 hits were retained for further analysis. A subsequent FASTA search (*e*.*g*., a combined local and global alignment analysis, in which limited regions of the sequences are aligned) was performed on these 500 hits. Each gene sequence of the invention was subsequently globally aligned to each of the top three FASTA hits, using the GAP program in the GCG software package (using standard parameters). In order to obtain correct results, the length of the sequences extracted from Genbank were adjusted to the length of the query sequences by methods well-known in the art. The results of this analysis are set forth in Table 4. The resulting data is identical to that which would have been obtained had a GAP (global) analysis alone been performed on each of the genes of the invention in comparison with each of the references in Genbank, but required significantly reduced computational time as compared to such a database-wide GAP (global) analysis. Sequences of the invention for which no alignments above the cutoff values were obtained are indicated on Table 4 by the absence of alignment information. It will further be understood by one of ordinary skill in the art that the GAP alignment homology percentages set forth in Table 4 under the heading "% homology (GAP)" are listed in the European numerical format, wherein a ',' represents a decimal point. For example, a value of "40,345" in this column represents "40.345%".

### Example 12: Construction and Operation of DNA Microarrays

The sequences of the invention may additionally be used in the construction and application of DNA microarrays (the design, methodology, and uses of DNA arrays are well known in the art, and are described, for example, in Schena, M. *et al*. (1995) *Science* 270: 467-470; Wodicka, L. *et al*. (1997) *Nature Biotechnology* 15: 1359-1367; DeSaizieu, *A. et al*. (1998) *Nature Biotechnology* 16: 45-48; and DeRisi, J.L. *et al*. (1997) *Science* 278: 680-686).

DNA microarrays are solid or flexible supports consisting of nitrocellulose, nylon, glass, silicone, or other materials. Nucleic acid molecules may be attached to the surface in an ordered manner. After appropriate labeling, other nucleic acids or nucleic acid mixtures can be hybridized to the immobilized nucleic acid molecules, and the label may be used to monitor and measure the individual signal intensities of the hybridized molecules at defined regions. This methodology allows the simultaneous quantification of the relative or absolute amount of all or selected nucleic acids in the applied nucleic acid sample or mixture. DNA microarrays, therefore, permit an analysis of the expression of multiple (as many as 6800 or more) nucleic acids in parallel (see, e.g., Schena, M. (1996) *BioEssays* 18(5): 427-431).

The sequences of the invention may be used to design oligonucleotide primers which are able to amplify defined regions of one or more *C*. *glutamicum* genes by a nucleic acid amplification reaction such as the polymerase chain reaction. The choice and design of the 5' or 3' oligonucleotide primers or of appropriate linkers allows the covalent attachment of the resulting PCR products to the surface of a support medium described above (and also described, for example, Schena, M. *et al*. (1995) *Science* 270: 467-470).

Nucleic acid microarrays may also be constructed by *in situ* oligonucleotide synthesis as described by Wodicka, L. *et al*. (1997) *Nature Biotechnology* 15: 1359-1367. By photolithographic methods, precisely defined regions of the matrix are exposed to light. Protective groups which are photolabile are thereby activated and undergo nucleotide addition, whereas regions that are masked from light do not undergo any modification. Subsequent cycles of protection and light activation permit the synthesis of different oligonucleotides at defined positions. Small, defined regions of the genes of the invention may be synthesized on microarrays by solid phase oligonucleotide synthesis.

The nucleic acid molecules of the invention present in a sample or mixture of nucleotides may be hybridized to the micmarrays. These nucleic acid molecules can be labeled according to standard methods. In brief, nucleic acid molecules (*e*.*g*., mRNA molecules or DNA molecules) are labeled by the incorporation of isotopically or fluorescently labeled nucleotides, *e*.*g*., during reverse transcription or DNA synthesis. Hybridization of labeled nucleic acids to microarrays is described (*e*.*g*., in Schena, M. *et al*. (1995) *supra;* Wodicka, L. *et al*. (1997), *supra;* and DeSaizieu A. *et al*. (1998), *supra*)*.* The detection and quantification of the hybridized molecule are tailored to the specific incorporated label. Radioactive labels can be detected, for example, as described in Schena, *M. et al*. (1995) *supra*) and fluorescent labels may be detected, for example, by the method of Shalon *et al*. (1996) *Genome Research* 6: 639-645).

The application of the sequences of the invention to DNA microarray technology, as described above, permits comparative analyses of different strains of *C*. *glutamicum* or other Corynebacteria. For example, studies of inter-strain variations based on individual transcript profiles and the identification of genes that are important for specific and/or desired strain properties such as pathogenicity, productivity and stress tolerance are facilitated by nucleic acid array methodologies. Also, comparisons of the profile of expression of genes of the invention during the course of a fermentation reaction are possible using nucleic acid array technology.

### Example 13: Analysis of the Dynamics of Cellular Protein Populations (Proteomics)

The genes, compositions, and methods of the invention may be applied to study the interactions and dynamics of populations of proteins, termed 'proteomics'. Protein populations of interest include, but are not limited to, the total protein population of *C*. *glutamicum* (*e*.*g*., in comparison with the protein populations of other organisms), those proteins which are active under specific environmental or metabolic conditions (*e*.*g*., during fermentation, at high or low temperature, or at high or low pH), or those proteins which are active during specific phases of growth and development.

Protein populations can be analyzed by various well-known techniques, such as gel electrophoresis. Cellular proteins may be obtained, for example, by lysis or extraction, and may be separated from one another using a variety of electrophoretic techniques. Sodium dodecyl sulfate polyacrylamide gel electrophoresis (SDS-PAGE) separates proteins largely on the basis of their molecular weight. Isociectric focusing polyacrylamide gel electrophoresis (IEF-PAGE) separates proteins by their isoelectric point (which reflects not only the amino acid sequence but also posttranslational modifications of the protein). Another, more preferred method of protein analysis is the consecutive combination of both IEF-PAGE and SDS-PAGE, known as 2-D-gel electrophoresis (described, for example, in Hermann *et al.* (1998) *Electrophoresis 19:* 3217-3221; Fountoulakis *et al*. (1998) *Electrophoresis* 19: 1193-1202; Langen *et al*. (1997) *Electrophoresis* 18: 1184-1192; Antelmann *et al*. (1997) *Electrophoresis 18:* 1451-1463). Other separation techniques may also be utilized for protein separation, such as capillary gel electrophoresis; such techniques are well known in the art.

Proteins separated by these methodologies can be visualized by standard techniques, such as by staining or labeling. Suitable stains are known in the art, and include Coomassie Brilliant Blue, silver stain, or fluorescent dyes such as Sypro Ruby (Molecular Probes). The inclusion of radioactively labeled amino acids or other protein precursors (e.g., ³⁵S-methionine, ³⁵S-cysteine, ¹⁴ C-labelled amino acids, ¹⁵N-amino acids, ¹⁵NO₃ or ¹⁵NH₄⁺ or ¹³C-labelled amino acids) in the medium of *C*. *glutamicum* permits the labeling of proteins from these cells prior to their separation. Similarly, fluorescent labels may be employed. These labeled proteins can be extracted, isolated and separated according to the previously described techniques.

Proteins visualized by these techniques can be further analyzed by measuring the amount of dye or label used. The amount of a given protein can be determined quantitatively using, for example, optical methods and can be compared to the amount of other proteins in the same gel or in other gels. Comparisons of proteins on gels can be made, for example, by optical comparison, by spectroscopy, by image scanning and analysis of gels, or through the use of photographic films and screens. Such techniques are well-known in the art.

To determine the identity of any given protein, direct sequencing or other standard techniques may be employed. For example, N- and/or C-terminal amino acid sequencing (such as Edman degradation) may be used, as may mass spectrometry (in particular MALDI or ESI techniques (see, *e*.*g*., Langen *et al*. (1997) *Electrophoresis* 18: 1184-1192)). The protein sequences provided herein can be used for the identification of *C*. *glutamicum* proteins by these techniques.

The information obtained by these methods can be used to compare patterns of protein presence, activity, or modification between different samples from various biological conditions (*e*.*g*., different organisms, time points of fermentation, media conditions, or different biotopes, among others). Data obtained from such experiments alone, or in combination with other techniques, can be used for various applications, such as to compare the behavior of various organisms in a given (*e*.*g*., metabolic) situation, to increase the productivity of strains which produce fine chemicals or to increase the efficiency of the production of fine chemicals.

### Equivalents

Those of ordinary skill in the art will recognize, or will be able to ascertain using no more than routine experimentation, many equivalents to the specific embodiments of the invention described herein. Such equivalents are intended to be encompassed by the following claims.

## Claims

1. An isolated *Corynebacterium glutamicum* nucleic acid molecule comprising the nucleic acid sequence set forth in SEQ ID NO:5, or a complement thereof.

2. An isolated nucleic acid molecule which encodes a polypeptide comprising the amino acid sequence set forth in SEQ ID NO:6, or a complement thereof.

3. An isolated nucleic acid molecule which encodes a naturally occurring allelic variant of a polypeptide comprising the amino acid sequence set forth in SEQ ID NO:6, or a complement thereof.

4. An isolated nucleic acid molecule comprising a nucleotide sequence which is at least 50% identical to the entire nucleotide sequence set forth in SEQ ID NO:5, or a complement thereof.

5. An isolated nucleic acid molecule comprising a fragment of at least 15 contiguous nucleotides of the nucleotide sequence set forth in SEQ ID NO:5, or a complement thereof.

6. A vector comprising the nucleic acid molecule of any one of claims 1-5.

7. The vector of claim 6, which is an expression vector.

8. A host cell transfected with the expression vector of claim 7.

9. The host cell of claim 8, wherein said cell is a microorganism.

10. The host cell of claim 9, wherein said cell belongs to the genus *Corynebacterium* or *Brevibacterium.*

11. The host cell of claim 8, wherein the expression of said nucleic acid molecule results in the modulation in production of a fine chemical from said cell.

12. The host cell of claim 11, wherein said fine chemical is selected from the group consisting of: organic acids, proteinogenic and nonproteinogenic amino acids, purine and pyrimidine bases, nucleosides, nucleotides, lipids, saturated and unsaturated fatty acids, diols, carbohydrates, aromatic compounds, vitamins, cofactors, polyketides, and enzymes.

13. A method of producing a polypeptide comprising culturing the host cell of claim 8 in an appropriate culture medium to, thereby, produce the polypeptide.

14. An isolated polypeptide comprising the amino acid sequence set forth in SEQ ID NO:6.

15. An isolated polypeptide comprising a naturally occurring allelic variant of a polypeptide comprising the amino acid sequence set forth in SEQ ID NO:6.

16. An isolated polypeptide which is encoded by a nucleic acid molecule comprising a nucleotide sequence which is at least 50% identical to the entire nucleic acid sequence set forth in SEQ ID NO:5.

17. An isolated polypeptide comprising an amino acid sequence which is at least 50% identical to the entire amino acid sequence set forth in SEQ ID NO:6.

18. An isolated polypeptide comprising a fragment of a polypeptide comprising the amino acid sequence set forth in SEQ ID NO:6, wherein said polypeptide fragment maintains a biological activity of the polypeptide comprising the amino sequence.

19. The isolated polypeptide of any of claims 14-18, further comprising heterologous amino acid sequences.

20. A method for producing a fine chemical, comprising culturing the cell of claim 8 such that the fine chemical is produced.

21. The method of claim 20, wherein said method further comprises the step of recovering the fine chemical from said culture.

22. The method of claim 20, wherein said cell belongs to the genus *Corynebacterium* or *Brevibacterium.*

23. The method of claim 20, wherein said cell is selected from the group consisting of: *Corynebacterium glutamicum, Corynebacterium herculis, Corynebacterium, lilium*, *Corynebacterium aceloacidophilum, Corynebacterium aceloglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Brevibacterium divaricatum, Brevibacterium flavum, Brevibacterium healii, Brevibacterium ketoglutamicum, Brevibacterium ketosoreductum, Brevibacterium lactofermentum, Brevibacterium linens, Brevibacterium paraffinolyticum,* and those strains set forth in Table 3.

24. The method of claim 20, wherein expression of the nucleic acid molecule from said vector results in modulation of production of said fine chemical.

25. The method of claim 20, wherein said fine chemical is selected from the group consisting of: organic acids, proteinogenic and nonproteinogenic amino acids, purine and pyrimidine bases, nucleosides, nucleotides, lipids, saturated and unsaturated fatty acids, diols, carbohydrates, aromatic compounds, vitamins, cofactors, polyketides and enzymes.

26. The method of claim 20, wherein said fine chemical is an amino acid.

27. The method of claim 26, wherein said amino acid is drawn from the group consisting of: lysine, glutamate, glutamine, alanine, aspartate, glycine, serine, threonine, methionine, cysteine, valine, leucine, isoleucine, arginine, proline, histidine, tyrosine, phenylalanine, and tryptophan.

28. A method for producing a fine chemical, comprising culturing a cell whose genomic DNA has been altered by the introduction of a nucleic acid molecule of any one of claims 1-5.

29. A method for diagnosing the presence or activity of Corynebacterium diphtheriae in a subject, comprising detecting the presence of at least one of the nucleic acid molecules of claims 1-5 or the polypeptide molecules of claims 14-18, thereby diagnosing the presence or activity of Corynebacterium diphtheriae in the subject.

30. A host cell comprising the nucleic acid molecule set forth in SEQ ID NO:5, wherein the nucleic acid molecule is disrupted.

31. A host cell comprising the nucleic acid molecule set forth in SEQ ID NO:5, wherein the nucleic acid molecule comprises one or more nucleic acid modifications as compared to the sequence set forth in SEQ ID NO:5.

32. A host cell comprising the nucleic acid molecule set forth in SEQ ID NO:5, wherein the regulatory region of the nucleic acid molecule is modified relative to the wild-type regulatory region of the molecule.


SEQUENCE LISTING

<110> BASF Aktiengesellschaft
<120> CORYNEBACTERIUM GLUTAMICUM GENES ENCODING PROTEINS
      INVOLVED IN MEMBRANE SYNTHESIS AND MEMBRANE
      TRANSPORT
<130> BGI-125CPPC
<140>
<141>
<160> 676

<210> 1
<211> 578
<212> DNA
<213> Corynebacterium glutamicum

<220>
<221> CDS
<222> (1)..(555)
<223> RXN03097

<400> 1
att tca gcc agt gca ggt gct ata ggt tgg tta att tta gaa tat att    48
Ile Ser Ala Ser Ala Gly Ala Ile Gly Trp Leu Ile Leu Glu Tyr Ile
  1               5                  10                  15

ttt aaa aag acg aca agt tta ctt gga ctt tta ctc ggt gca tta gca    96
Phe Lys Lys Thr Thr Ser Leu Leu Gly Leu Leu Leu Gly Ala Leu Ala
             20                  25                  30

gga tta gtt gtt att act cct gca gca gga tat gta aca tat ctt agt    144
Gly Leu Val Val Ile Thr Pro Ala Ala Gly Tyr Val Thr Tyr Leu Ser
         35                  40                  45

gca aca ata atg gct tta ata gga ggt atc tgt tgt tat att gtc att    192
Ala Thr Ile Met Ala Leu Ile Gly Gly Ile Cys Cys Tyr Ile Val Ile
     50                  55                  60

aat tac atc aag gta aaa cta aaa tat cat gat gca tta gat gca ttt    240
Asn Tyr Ile Lys Val Lys Leu Lys Tyr His Asp Ala Leu Asp Ala Phe
 65                  70                  75                  80

ggt att cat ggt gtt ggt ggt att att ggt gct gtt tta aca gca gtt    288
Gly Ile His Gly Val Gly Gly Ile Ile Gly Ala Val Leu Thr Ala Val
                 85                  90                  95

ttc caa agt aaa aaa gcc aat cct gac att gag aat ggc ttt att tat    336
Phe Gln Ser Lys Lys Ala Asn Pro Asp Ile Glu Asn Gly Phe Ile Tyr
            100                 105                 110

act ggt gac ata cat att ata ctt gta caa ata tta tgt gta aca gca    384
Thr Gly Asp Ile His Ile Ile Leu Val Gln Ile Leu Cys Val Thr Ala
        115                 120                 125

gtt gta att ttt agt atc gtc atg acg ttt att att gcg aaa gta att    432
Val Val Ile Phe Ser Ile Val Met Thr Phe Ile Ile Ala Lys Val Ile
    130                 135                 140

aaa tta att aca cca tta tct gtt acg gaa caa gaa acg aat ata gga    480
Lys Leu Ile Thr Pro Leu Ser Val Thr Glu Gln Glu Thr Asn Ile Gly
145                 150                 155                 160

tta gac aag att gtt cac ggt gaa cat gct tac ttt gaa ggt gag cta     528
Leu Asp Lys Ile Val His Gly Glu His Ala Tyr Phe Glu Gly Glu Leu
                165                 170                 175

aat aga ttc aat aaa cat att cga tat tagaatatat ttacatagaa          575
Asn Arg Phe Asn Lys His Ile Arg Tyr
            180                 185

tat                                                                578


<210> 2
<211> 185
<212> PRT
<213> Corynebacterium glutamicum

<400> 2
Ile Ser Ala Ser Ala Gly Ala Ile Gly Trp Leu Ile Leu Glu Tyr Ile
  1               5                  10                  15

Phe Lys Lys Thr Thr Ser Leu Leu Gly Leu Leu Leu Gly Ala Leu Ala
            20                  25                  30

Gly Leu Val Val Ile Thr Pro Ala Ala Gly Tyr Val Thr Tyr Leu Ser
        35                  40                  45

Ala Thr Ile Met Ala Leu Ile Gly Gly Ile Cys Cys Tyr Ile Val Ile
    50                  55                  60

Asn Tyr Ile Lys Val Lys Leu Lys Tyr His Asp Ala Leu Asp Ala Phe
 65                  70                  75                  80

Gly Ile His Gly Val Gly Gly Ile Ile Gly Ala Val Leu Thr Ala Val
                85                  90                  95

Phe Gln Ser Lys Lys Ala Asn Pro Asp Ile Glu Asn Gly Phe Ile Tyr
            100                 105                 110

Thr Gly Asp Ile His Ile Ile Leu Val Gln Ile Leu Cys Val Thr Ala
        115                 120                 125

Val Val Ile Phe Ser Ile Val Met Thr Phe Ile Ile Ala Lys Val Ile
    130                 135                 140

Lys Leu Ile Thr Pro Leu Ser Val Thr Glu Gln Glu Thr Asn Ile Gly
145                 150                 155                 160

Leu Asp Lys Ile Val His Gly Glu His Ala Tyr Phe Glu Gly Glu Leu
                165                 170                 175

Asn Arg Phe Asn Lys His Ile Arg Tyr
            180                 185


<210> 3
<211> 373
<212> DNA
<213> Corynebacterium glutamicum

<220>

<221> CDS
<222> (101)..(373)
<223> RXA02099

<400> 3
tagttagagc tggttcaagg ggtgtcaatc ccaaaaggca ctccttgaac tcatgaaaaa 60

gcttgacaaa acttcaacgt caaaggaggt catccacgct atg ggt gca gat caa 115
Met Gly Ala Asp Gln
1               5

att gca gca gtc tcc ggc aat tca gct tgg atg ctg atg tcc gcg tcg 163
Ile Ala Ala Val Ser Gly Asn Ser Ala Trp Met Leu Met Ser Ala Ser
                10                  15                  20

ctc gtg ctg cta atg aca cca gca ctg gca ctt ttc tat ggc ggc atg 211
Leu Val Leu Leu Met Thr Pro Ala Leu Ala Leu Phe Tyr Gly Gly Met
            25                  30                  35

tct cgt caa aag tcc gtg ctc aac atg atg atg atg tcc ttt gga gca 259
Ser Arg Gln Lys Ser Val Leu Asn Met Met Met Met Ser Phe Gly Ala
        40                  45                  50

ttg ggc gtc gtt act gtt att tac ctc ttg tgg gga tgg tcg atg tct 307
Leu Gly Val Val Thr Val Ile Tyr Leu Leu Trp Gly Trp Ser Met Ser
    55                  60                  65

tat gga acc caa tca atc gcg gga atc ttt gct aac cct ttt gag ttc 355
Tyr Gly Thr Gln Ser Ile Ala Gly Ile Phe Ala Asn Pro Phe Glu Phe
70                  75                  80                  85

ttc ggt ctt aaa gat tcc 373
Phe Gly Leu Lys Asp Ser
                90

<210> 4
<211> 91
<212> PRT
<213> Corynebacterium glutamicum

<400> 4
Met Gly Ala Asp Gln Ile Ala Ala Val Ser Gly Asn Ser Ala Trp Met
1               5                   10                  15

Leu Met Ser Ala Ser Leu Val Leu Leu Met Thr Pro Ala Leu Ala Leu
            20                  25                  30

Phe Tyr Gly Gly Met Ser Arg Gln Lys Ser Val Leu Asn Met Met Met
        35                  40                  45

Met Ser Phe Gly Ala Leu Gly Val Val Thr Val Ile Tyr Leu Leu Trp
    50                  55                  60

Gly Trp Ser Met Ser Tyr Gly Thr Gln Ser Ile Ala Gly Ile Phe Ala
65                  70                  75                  80

Asn Pro Phe Glu Phe Phe Gly Leu Lys Asp Ser
                85                  90

<210> 5
<211> 879
<212> DNA
<213> Corynebacterium glutamicum

<220>
<221> CDS
<222> (101)..(856)
<223> RXA00104

<400> 5
tgcaaagaca tcccggaacc actccgctac ttgaggtcgg aatcgggaaa gaatgctttt 60

agccatgcct taatgtaacc aaacatctag aattgagaac atg act gct cag att 115
Met Thr Ala Gln Ile
1               5

gat gat tcg atc ctc acc cat cgt ctc gcc caa ggc acc gga gaa atc 163
Asp Asp Ser Ile Leu Thr His Arg Leu Ala Gln Gly Thr Gly Glu Ile
                10                  15                  20

ctc aaa ggt gtc cgc aat gtt ggg gtg tta aga ggt cgg aat ctc ggt 211
Leu Lys Gly Val Arg Asn Val Gly Val Leu Arg Gly Arg Asn Leu Gly
            25                  30                  35

gat gcc ggc gac gaa ctc gca caa agt tgg att gct cga gtg ttg gag 259
Asp Ala Gly Asp Glu Leu Ala Gln Ser Trp Ile Ala Arg Val Leu Glu
        40                  45                  50

cag cac cgc ccc aac gat gga ttc ctg tct gaa gaa gcc gcc gac aac 307
Gln His Arg Pro Asn Asp Gly Phe Leu Ser Glu Glu Ala Ala Asp Asn
    55                  60                  65

cca gac cgc cta tcc aag gac cgc gtg tgg atc atc gat ccc ctc gac 355
Pro Asp Arg Leu Ser Lys Asp Arg Val Trp Ile Ile Asp Pro Leu Asp
70                  75                  80                  85

ggc acc aaa gaa ttc gcc acc ggc cgc cag gac tgg gca gta cac atc 403
Gly Thr Lys Glu Phe Ala Thr Gly Arg Gln Asp Trp Ala Val His Ile
                90                  95                  100

gca ctg gta gaa aac ggt gtt ccc acc cac gcc gct gtt ggc ctc ccc 451
Ala Leu Val Glu Asn Gly Val Pro Thr His Ala Ala Val Gly Leu Pro
            105                 110                 115

gac ctt ggc gtg gtg ttc cac tcc gct gat gcc cgc gcc gtg act ggc 499
Asp Leu Gly Val Val Phe His Ser Ala Asp Ala Arg Ala Val Thr Gly
        120                 125                 130

cct tac tcc aag gtc atc gcc atc tcc cac aac cgc cca cca aag gtt 547
Pro Tyr Ser Lys Val Ile Ala Ile Ser His Asn Arg Pro Pro Lys Val
    135                 140                 145

gct cta tct tgc gca gag cag ctc ggc ttt gaa acc aag gcc ctt gga 595
Ala Leu Ser Cys Ala Glu Gln Leu Gly Phe Glu Thr Lys Ala Leu Gly
150                 155                 160                 165

tcc gca ggc gct aaa gca atg cac gtt ctc ctc ggt gac tac gac gcc 643
Ser Ala Gly Ala Lys Ala Met His Val Leu Leu Gly Asp Tyr Asp Ala
                170                 175                 180

tac atc cac gcc ggc ggc caa tac gag tgg gat tcc gca gca cca gtc 691
Tyr Ile His Ala Gly Gly Gln Tyr Glu Trp Asp Ser Ala Ala Pro Val
            185                 190                 195

ggc gtc tgc aag gca gca ggc ttg cac tgc tcc agg ctc gac ggt tcc 739
Gly Val Cys Lys Ala Ala Gly Leu His Cys Ser Arg Leu Asp Gly Ser
        200                 205                 210

gag ctg acc tac aac aac aaa gac acc tac atg cca gac atc ttg atc 787
Glu Leu Thr Tyr Asn Asn Lys Asp Thr Tyr Met Pro Asp Ile Leu Ile
    215                 220                 225

tgt cgc cct gaa ctt gca gat gaa ctt ctc gag atg tgc gcg aag ttc 835
Cys Arg Pro Glu Leu Ala Asp Glu Leu Leu Glu Met Cys Ala Lys Phe
230                 235                 240                 245

tac gag gag aat gga act tac taacgctgtt atgatgacgg cat 879
Tyr Glu Glu Asn Gly Thr Tyr
                250

<210> 6
<211> 252
<212> PRT
<213> Corynebacterium glutamicum

<400> 6
Met Thr Ala Gln Ile Asp Asp Ser Ile Leu Thr His Arg Leu Ala Gln
1               5                   10                  15

Gly Thr Gly Glu Ile Leu Lys Gly Val Arg Asn Val Gly Val Leu Arg
            20                  25                  30

Gly Arg Asn Leu Gly Asp Ala Gly Asp Glu Leu Ala Gln Ser Trp Ile
        35                  40                  45

Ala Arg Val Leu Glu Gln His Arg Pro Asn Asp Gly Phe Leu Ser Glu
    50                  55                  60

Glu Ala Ala Asp Asn Pro Asp Arg Leu Ser Lys Asp Arg Val Trp Ile
65                  70                  75                  80

Ile Asp Pro Leu Asp Gly Thr Lys Glu Phe Ala Thr Gly Arg Gln Asp
                85                  90                  95

Trp Ala Val His Ile Ala Leu Val Glu Asn Gly Val Pro Thr His Ala
            100                 105                 110

Ala Val Gly Leu Pro Asp Leu Gly Val Val Phe His Ser Ala Asp Ala
        115                 120                 125

Arg Ala Val Thr Gly Pro Tyr Ser Lys Val Ile Ala Ile Ser His Asn
    130                 135                 140

Arg Pro Pro Lys Val Ala Leu Ser Cys Ala Glu Gln Leu Gly Phe Glu
145                 150                 155                 160

Thr Lys Ala Leu Gly Ser Ala Gly Ala Lys Ala Met His Val Leu Leu
                165                 170                 175

Gly Asp Tyr Asp Ala Tyr Ile His Ala Gly Gly Gln Tyr Glu Trp Asp

180                 185                 190

Ser Ala Ala Pro Val Gly Val Cys Lys Ala Ala Gly Leu His Cys Ser
        195                 200                 205

Arg Leu Asp Gly Ser Glu Leu Thr Tyr Asn Asn Lys Asp Thr Tyr Met
    210                 215                 220

Pro Asp Ile Leu Ile Cys Arg Pro Glu Leu Ala Asp Glu Leu Leu Glu
225                 230                 235                 240

Met Cys Ala Lys Phe Tyr Glu Glu Asn Gly Thr Tyr
                245                 250

<210> 7
<211> 875
<212> DNA
<213> Corynebacterium glutamicum

<220>
<221> CDS
<222> (101)..(859)
<223> RXA01420

<400> 7
cgaagttcac gactacaggc attacctggg tgctgatttt gcgcgagtaa agctgtggct 60

gcggaatgcg ctgctcatcc acacgtggaa tcctgattgg gtg acg ctg ggt gga   115
                                           Val Thr Leu Gly Gly
                                           1               5

ttg aat gta cca tcg tgg tcg ctg ggc gcg gaa atg ctg ttc tac ctg   163
Leu Asn Val Pro Ser Trp Ser Leu Gly Ala Glu Met Leu Phe Tyr Leu
                10                  15                  20

acg ttc ccg ctg ttt att ccg tta gtg cgc aaa gtg aag ggc gtg ggc   211
Thr Phe Pro Leu Phe Ile Pro Leu Val Arg Lys Val Lys Gly Val Gly
            25                  30                  35

aac tgg tgg gca ttt ggc atc acc ttt gcc gtg agc ctg gcg ctg att   259
Asn Trp Trp Ala Phe Gly Ile Thr Phe Ala Val Ser Leu Ala Leu Ile
        40                  45                  50

aca gtg att cac ttt tat gcg gac gga cca aag ggg att gag aac ttc   307
Thr Val Ile His Phe Tyr Ala Asp Gly Pro Lys Gly Ile Glu Asn Phe
    55                  60                  65

ttt gtt cct cgc ctg tgg gac acc aat gtg tca ccg gtc gcg gaa gtt   355
Phe Val Pro Arg Leu Trp Asp Thr Asn Val Ser Pro Val Ala Glu Val
70                  75                  80                  85

cac gcc gat cca gtg tgg ttt atg cag gaa gaa att ccc gtg ctg gaa   403
His Ala Asp Pro Val Trp Phe Met Gln Glu Glu Ile Pro Val Leu Glu
                90                  95                  100

tct tac tgg ctg tct tac tac ttc ccg ctg acc aga ctc atc gag ttc   451
Ser Tyr Trp Leu Ser Tyr Tyr Phe Pro Leu Thr Arg Leu Ile Glu Phe
            105                 110                 115

tac ctc ggt gtg ttt ggc gcg aag ctg gtt gct gaa ggc atg ttt aaa   499

Tyr Leu Gly Val Phe Gly Ala Lys Leu Val Ala Glu Gly Met Phe Lys
        120                 125                 130

aac acc aac atc acc atc ccg ctg atc gca ctg gct gtt tct ttt gtt 547
Asn Thr Asn Ile Thr Ile Pro Leu Ile Ala Leu Ala Val Ser Phe Val
    135                 140                 145

gct act tgg ttt gtg cca ctg gca ttc aag atg tct gtc atc atg tcc 595
Ala Thr Trp Phe Val Pro Leu Ala Phe Lys Met Ser Val Ile Met Ser
150                 155                 160                 165

ctg cca atg gct ttt gtt gtg gca acg ctt gcg gtg aga gac att gaa 643
Leu Pro Met Ala Phe Val Val Ala Thr Leu Ala Val Arg Asp Ile Glu
                170                 175                 180

ggc aag agt ggg gaa atc gcc tcg cct cgc gca gtt ttg ttg ggt aat 691
Gly Lys Ser Gly Glu Ile Ala Ser Pro Arg Ala Val Leu Leu Gly Asn
            185                 190                 195

att tcc ttt gcc ttc tac atg gtg caa ttc ccc gtc atg gtg ttt gtg 739
Ile Ser Phe Ala Phe Tyr Met Val Gln Phe Pro Val Met Val Phe Val
        200                 205                 210

cag cgc tat ttc att gct ggc aaa gaa tac ggc ttc ctt ggc tgg gca 787
Gln Arg Tyr Phe Ile Ala Gly Lys Glu Tyr Gly Phe Leu Gly Trp Ala
    215                 220                 225

ttc tat gca gtg gtg tgt ttc atc gtg tcg gtc att ctc gcg tgg gtg 835
Phe Tyr Ala Val Val Cys Phe Ile Val Ser Val Ile Leu Ala Trp Val
230                 235                 240                 245

ctg tta cct tcg ttg atg atc ctt taataaggcc acggaa 875
Leu Leu Pro Ser Leu Met Ile Leu
                250

<210> 8
<211> 253
<212> PRT
<213> Corynebacterium glutamicum

<400> 8
Val Thr Leu Gly Gly Leu Asn Val Pro Ser Trp Ser Leu Gly Ala Glu
1               5                   10                  15

Met Leu Phe Tyr Leu Thr Phe Pro Leu Phe Ile Pro Leu Val Arg Lys
            20                  25                  30

Val Lys Gly Val Gly Asn Trp Trp Ala Phe Gly Ile Thr Phe Ala Val
        35                  40                  45

Ser Leu Ala Leu Ile Thr Val Ile His Phe Tyr Ala Asp Gly Pro Lys
    50                  55                  60

Gly Ile Glu Asn Phe Phe Val Pro Arg Leu Trp Asp Thr Asn Val Ser
65                  70                  75                  80

Pro Val Ala Glu Val His Ala Asp Pro Val Trp Phe Met Gln Glu Glu
                85                  90                  95

Ile Pro Val Leu Glu Ser Tyr Trp Leu Ser Tyr Tyr Phe Pro Leu Thr

                100                 105                 110

Arg Leu Ile Glu Phe Tyr Leu Gly Val Phe Gly Ala Lys Leu Val Ala
        115                 120                 125

Glu Gly Met Phe Lys Asn Thr Asn Ile Thr Ile Pro Leu Ile Ala Leu
    130                 135                 140

Ala Val Ser Phe Val Ala Thr Trp Phe Val Pro Leu Ala Phe Lys Met
145                 150                 155                 160

Ser Val Ile Met Ser Leu Pro Met Ala Phe Val Val Ala Thr Leu Ala
                165                 170                 175

Val Arg Asp Ile Glu Gly Lys Ser Gly Glu Ile Ala Ser Pro Arg Ala
            180                 185                 190

Val Leu Leu Gly Asn Ile Ser Phe Ala Phe Tyr Met Val Gln Phe Pro
        195                 200                 205

Val Met Val Phe Val Gln Arg Tyr Phe Ile Ala Gly Lys Glu Tyr Gly
    210                 215                 220

Phe Leu Gly Trp Ala Phe Tyr Ala Val Val Cys Phe Ile Val Ser Val
225                 230                 235                 240

Ile Leu Ala Trp Val Leu Leu Pro Ser Leu Met Ile Leu
                245                 250

<210> 9
<211> 1983
<212> DNA
<213> Corynebacterium glutamicum

<220>
<221> CDS
<222> (101)..(1960)
<223> RXN02581

<400> 9
acggtcactc gctcaagtag tgttagtttg caaaagtaat aaaatgttca tctttgtcga 60

tggtcacaat agtcattaaa gaattgtaag aggggttcac atg gat tta gat aaa   115
                                           Met Asp Leu Asp Lys
                                           1               5

gcg att ggt tca ttc ttc gat gag aat gga gaa atc aac ctt cct cca   163
Ala Ile Gly Ser Phe Phe Asp Glu Asn Gly Glu Ile Asn Leu Pro Pro
                10                  15                  20

ttc cta act ttg gca gcc atg ggt gag ttc atg tac cag gct gac atc   211
Phe Leu Thr Leu Ala Ala Met Gly Glu Phe Met Tyr Gln Ala Asp Ile
            25                  30                  35

gct gaa ggc ggc ggg gat aaa cca cgc atg cat ttc tgg gac ttc tcc   259
Ala Glu Gly Gly Gly Asp Lys Pro Arg Met His Phe Trp Asp Phe Ser
        40                  45                  50

gaa gac cgc gat ggc aag ctg att cag tac acc cga aac gag atc gat   307
Glu Asp Arg Asp Gly Lys Leu Ile Gln Tyr Thr Arg Asn Glu Ile Asp

    55                  60                  65

act cgt atc aag gct gta gca ggc cgt ttg cag cag gtc gcc acc ctg 355
Thr Arg Ile Lys Ala Val Ala Gly Arg Leu Gln Gln Val Ala Thr Leu
70                  75                  80                  85

ggt gat cgt gca gcg atc ctg gct aac aac agc cct gag tac att ttc 403
Gly Asp Arg Ala Ala Ile Leu Ala Asn Asn Ser Pro Glu Tyr Ile Phe
                90                  95                  100

agc ttc ctc ggc gcg atc tac gct ggc atg gtc cct gtg ccg ctt tat 451
Ser Phe Leu Gly Ala Ile Tyr Ala Gly Met Val Pro Val Pro Leu Tyr
            105                 110                 115

gat cca aac gag cca gga cac gca gac cac ctc aac gct gtt ttc gca 499
Asp Pro Asn Glu Pro Gly His Ala Asp His Leu Asn Ala Val Phe Ala
        120                 125                 130

gac agc gag cca gtt gtc gtt ctg acc aac tcc aag tcc gca ggt gcc 547
Asp Ser Glu Pro Val Val Val Leu Thr Asn Ser Lys Ser Ala Gly Ala
    135                 140                 145

gtg cgc aag cac ttc tcc agc ctt cca gct gca gaa cgc cca cgc atc 595
Val Arg Lys His Phe Ser Ser Leu Pro Ala Ala Glu Arg Pro Arg Ile
150                 155                 160                 165

ctc tct gta gat tcc ttg cct gat tct ctc gcg gat tct tac gag aac 643
Leu Ser Val Asp Ser Leu Pro Asp Ser Leu Ala Asp Ser Tyr Glu Asn
                170                 175                 180

cca atg ctg acc gaa gcc ggc cgc cgc ctg gct gct ctg cgc cag tcc 691
Pro Met Leu Thr Glu Ala Gly Arg Arg Leu Ala Ala Leu Arg Gln Ser
            185                 190                 195

gcg ccc att gat ctg acc gca ttc ctg cag tac acc tcc ggc tcc acc 739
Ala Pro Ile Asp Leu Thr Ala Phe Leu Gln Tyr Thr Ser Gly Ser Thr
        200                 205                 210

cga acc cca gct ggc gtt gtt ctg acc aac cgc tcc atc ctg acc aac 787
Arg Thr Pro Ala Gly Val Val Leu Thr Asn Arg Ser Ile Leu Thr Asn
    215                 220                 225

gtc ttg cag atc ttc agc gcc gca cag ctg aaa acc cca ctg cgc ctg 835
Val Leu Gln Ile Phe Ser Ala Ala Gln Leu Lys Thr Pro Leu Arg Leu
230                 235                 240                 245

gtt tca tgg ctg cca ctg cac cac gac atg ggc att atc ctc gcg gcg 883
Val Ser Trp Leu Pro Leu His His Asp Met Gly Ile Ile Leu Ala Ala
                250                 255                 260

ttt gtc act atg ctt ggc ctg gac aac gag ttc atg aac cca cgc gat 931
Phe Val Thr Met Leu Gly Leu Asp Asn Glu Phe Met Asn Pro Arg Asp
            265                 270                 275

ttc gtg cag cag cct tcc cgc tgg att aag cag ctc aac cgt cgc gaa 979
Phe Val Gln Gln Pro Ser Arg Trp Ile Lys Gln Leu Asn Arg Arg Glu
        280                 285                 290

agc gac gtg gac gtt aac gtc tac acc gtg gtt cct aac ttc gcc ctc 1027
Ser Asp Val Asp Val Asn Val Tyr Thr Val Val Pro Asn Phe Ala Leu
    295                 300                 305

gag ctt gca gca cgc tac gca aag cca gca gag gga gag acc ctg gat 1075
Glu Leu Ala Ala Arg Tyr Ala Lys Pro Ala Glu Gly Glu Thr Leu Asp
310                 315                 320                 325

ctt tcc gca ttg gat gcc atc att atc ggt tcc gag cca gtc aca gaa 1123
Leu Ser Ala Leu Asp Ala Ile Ile Ile Gly Ser Glu Pro Val Thr Glu
                330                 335                 340

aac gct ctg acc acc ttc cgt gaa gct ttc gag cct tac ggc ctg cct 1171
Asn Ala Leu Thr Thr Phe Arg Glu Ala Phe Glu Pro Tyr Gly Leu Pro
            345                 350                 355

gtt cag acc ctg cgt cct tcc tac ggt ctt gca gaa gca tcc ctg ctg 1219
Val Gln Thr Leu Arg Pro Ser Tyr Gly Leu Ala Glu Ala Ser Leu Leu
        360                 365                 370

gtc acc acc cca cag acc gaa aac cgc cca ctg atc tcc tac ttc gac 1267
Val Thr Thr Pro Gln Thr Glu Asn Arg Pro Leu Ile Ser Tyr Phe Asp
    375                 380                 385

cgc gag gcc ttg gcc gaa aac cgc gtt gag ctt gta gaa aag ggc aat 1315
Arg Glu Ala Leu Ala Glu Asn Arg Val Glu Leu Val Glu Lys Gly Asn
390                 395                 400                 405

aac aag gct gtt gct ttc gtc tcc aac ggc cag gtt gca gcc cca cag 1363
Asn Lys Ala Val Ala Phe Val Ser Asn Gly Gln Val Ala Ala Pro Gln
                410                 415                 420

cag ctg gtc atc gtt gat tcc gaa acc gga acc gag ctg gca gac ggc 1411
Gln Leu Val Ile Val Asp Ser Glu Thr Gly Thr Glu Leu Ala Asp Gly
            425                 430                 435

cag atc ggc gaa atc tgg acc cac ggc gaa aac act gct gca ggt tac 1459
Gln Ile Gly Glu Ile Trp Thr His Gly Glu Asn Thr Ala Ala Gly Tyr
        440                 445                 450

ctc gac cgc gag gaa gac acc gca gaa acc ttc cgc aac cgt ctg acc 1507
Leu Asp Arg Glu Glu Asp Thr Ala Glu Thr Phe Arg Asn Arg Leu Thr
    455                 460                 465

acc cgc ctg gaa gaa aac tcc cgc gca gaa ggt gct gcc gac gac aac 1555
Thr Arg Leu Glu Glu Asn Ser Arg Ala Glu Gly Ala Ala Asp Asp Asn
470                 475                 480                 485

tac tgg atg gcc acc ggt gac ctc ggc gtc atc gta gac aac gag ctc 1603
Tyr Trp Met Ala Thr Gly Asp Leu Gly Val Ile Val Asp Asn Glu Leu
                490                 495                 500

tac atc acc ggt cgt ctg aag gac ctc atc gtt gtc gca ggc cga aac 1651
Tyr Ile Thr Gly Arg Leu Lys Asp Leu Ile Val Val Ala Gly Arg Asn
            505                 510                 515

cac tac cca cag gac atc gag tac acc gtc cag gct gct tcc gca cac 1699
His Tyr Pro Gln Asp Ile Glu Tyr Thr Val Gln Ala Ala Ser Ala His
        520                 525                 530

atc cgt gca gat tcc gtc gca gca ttc gca gtc cca ggc gat gac att 1747
Ile Arg Ala Asp Ser Val Ala Ala Phe Ala Val Pro Gly Asp Asp Ile
    535                 540                 545

gaa aag ctc atc atc ctg gca gaa cgc gac acc act gca aac gaa gcc    1795
Glu Lys Leu Ile Ile Leu Ala Glu Arg Asp Thr Thr Ala Asn Glu Ala
550                 555                 560                 565

gac gat gca gct gct gaa gaa gca atc cgc tcc gcc gtt ggc act gca    1843
Asp Asp Ala Ala Ala Glu Glu Ala Ile Arg Ser Ala Val Gly Thr Ala
                570                 575                 580

cac ggt gtt gtt cca gaa gag atc cgt atc ctc gca cct gac gag atc    1891
His Gly Val Val Pro Glu Glu Ile Arg Ile Leu Ala Pro Asp Glu Ile
            585                 590                 595

gcg cgt tcc tcc tcc gga aag atc gca cgc cgc gtc aac cag cgc aac    1939
Ala Arg Ser Ser Ser Gly Lys Ile Ala Arg Arg Val Asn Gln Arg Asn
        600                 605                 610

tac att cag gaa caa gct aac tagttctttg cagacaccgc agg              1983
Tyr Ile Gln Glu Gln Ala Asn
    615                 620


<210> 10
<211> 620
<212> PRT
<213> Corynebacterium glutamicum

<400> 10
Met Asp Leu Asp Lys Ala Ile Gly Ser Phe Phe Asp Glu Asn Gly Glu
  1               5                  10                  15

Ile Asn Leu Pro Pro Phe Leu Thr Leu Ala Ala Met Gly Glu Phe Met
             20                  25                  30

Tyr Gln Ala Asp Ile Ala Glu Gly Gly Gly Asp Lys Pro Arg Met His
         35                  40                  45

Phe Trp Asp Phe Ser Glu Asp Arg Asp Gly Lys Leu Ile Gln Tyr Thr
     50                  55                  60

Arg Asn Glu Ile Asp Thr Arg Ile Lys Ala Val Ala Gly Arg Leu Gln
 65                  70                  75                  80

Gln Val Ala Thr Leu Gly Asp Arg Ala Ala Ile Leu Ala Asn Asn Ser
                 85                  90                  95

Pro Glu Tyr Ile Phe Ser Phe Leu Gly Ala Ile Tyr Ala Gly Met Val
            100                 105                 110

Pro Val Pro Leu Tyr Asp Pro Asn Glu Pro Gly His Ala Asp His Leu
        115                 120                 125

Asn Ala Val Phe Ala Asp Ser Glu Pro Val Val Val Leu Thr Asn Ser
    130                 135                 140

Lys Ser Ala Gly Ala Val Arg Lys His Phe Ser Ser Leu Pro Ala Ala
145                 150                 155                 160

Glu Arg Pro Arg Ile Leu Ser Val Asp Ser Leu Pro Asp Ser Leu Ala
                165                 170                 175

Asp Ser Tyr Glu Asn Pro Met Leu Thr Glu Ala Gly Arg Arg Leu Ala

            180                 185                 190

Ala Leu Arg Gln Ser Ala Pro Ile Asp Leu Thr Ala Phe Leu Gln Tyr
        195                 200                 205

Thr Ser Gly Ser Thr Arg Thr Pro Ala Gly Val Val Leu Thr Asn Arg
    210                 215                 220

Ser Ile Leu Thr Asn Val Leu Gln Ile Phe Ser Ala Ala Gln Leu Lys
225                 230                 235                 240

Thr Pro Leu Arg Leu Val Ser Trp Leu Pro Leu His His Asp Met Gly
                245                 250                 255

Ile Ile Leu Ala Ala Phe Val Thr Met Leu Gly Leu Asp Asn Glu Phe
            260                 265                 270

Met Asn Pro Arg Asp Phe Val Gln Gln Pro Ser Arg Trp Ile Lys Gln
        275                 280                 285

Leu Asn Arg Arg Glu Ser Asp Val Asp Val Asn Val Tyr Thr Val Val
    290                 295                 300

Pro Asn Phe Ala Leu Glu Leu Ala Ala Arg Tyr Ala Lys Pro Ala Glu
305                 310                 315                 320

Gly Glu Thr Leu Asp Leu Ser Ala Leu Asp Ala Ile Ile Ile Gly Ser
                325                 330                 335

Glu Pro Val Thr Glu Asn Ala Leu Thr Thr Phe Arg Glu Ala Phe Glu
            340                 345                 350

Pro Tyr Gly Leu Pro Val Gln Thr Leu Arg Pro Ser Tyr Gly Leu Ala
        355                 360                 365

Glu Ala Ser Leu Leu Val Thr Thr Pro Gln Thr Glu Asn Arg Pro Leu
    370                 375                 380

Ile Ser Tyr Phe Asp Arg Glu Ala Leu Ala Glu Asn Arg Val Glu Leu
385                 390                 395                 400

Val Glu Lys Gly Asn Asn Lys Ala Val Ala Phe Val Ser Asn Gly Gln
                405                 410                 415

Val Ala Ala Pro Gln Gln Leu Val Ile Val Asp Ser Glu Thr Gly Thr
            420                 425                 430

Glu Leu Ala Asp Gly Gln Ile Gly Glu Ile Trp Thr His Gly Glu Asn
        435                 440                 445

Thr Ala Ala Gly Tyr Leu Asp Arg Glu Glu Asp Thr Ala Glu Thr Phe
    450                 455                 460

Arg Asn Arg Leu Thr Thr Arg Leu Glu Glu Asn Ser Arg Ala Glu Gly
465                 470                 475                 480

Ala Ala Asp Asp Asn Tyr Trp Met Ala Thr Gly Asp Leu Gly Val Ile
                485                 490                 495

Val Asp Asn Glu Leu Tyr Ile Thr Gly Arg Leu Lys Asp Leu Ile Val
            500                 505                 510

Val Ala Gly Arg Asn His Tyr Pro Gln Asp Ile Glu Tyr Thr Val Gln
        515                 520                 525

Ala Ala Ser Ala His Ile Arg Ala Asp Ser Val Ala Ala Phe Ala Val
    530                 535                 540

Pro Gly Asp Asp Ile Glu Lys Leu Ile Ile Leu Ala Glu Arg Asp Thr
545                 550                 555                 560

Thr Ala Asn Glu Ala Asp Asp Ala Ala Ala Glu Glu Ala Ile Arg Ser
                565                 570                 575

Ala Val Gly Thr Ala His Gly Val Val Pro Glu Glu Ile Arg Ile Leu
            580                 585                 590

Ala Pro Asp Glu Ile Ala Arg Ser Ser Ser Gly Lys Ile Ala Arg Arg
        595                 600                 605

Val Asn Gln Arg Asn Tyr Ile Gln Glu Gln Ala Asn
    610                 615                 620

<210> 11
<211> 1550
<212> DNA
<213> Corynebacterium glutamicum

<220>
<221> CDS
<222> (1)..(1527)
<223> FRXA02581

<400> 11
gtc cct gtg ccg ctt tat gat cca aac gag cca gga cac gca gac cac      48
Val Pro Val Pro Leu Tyr Asp Pro Asn Glu Pro Gly His Ala Asp His
  1               5                  10                  15

ctc aac gct gtt ttc gca gac agc gag cca gtt gtc gtt ctg acc aac      96
Leu Asn Ala Val Phe Ala Asp Ser Glu Pro Val Val Val Leu Thr Asn
             20                  25                  30

tcc aag tcc gca ggt gcc gtg cgc aag cac ttc tcc agc ctt cca gct     144
Ser Lys Ser Ala Gly Ala Val Arg Lys His Phe Ser Ser Leu Pro Ala
         35                  40                  45

gca gaa cgc cca cgc atc ctc tct gta gat tcc ttg cct gat tct ctc     192
Ala Glu Arg Pro Arg Ile Leu Ser Val Asp Ser Leu Pro Asp Ser Leu
     50                  55                  60

gcg gat tct tac gag aac cca atg ctg acc gaa gcc ggc cgc cgc ctg     240
Ala Asp Ser Tyr Glu Asn Pro Met Leu Thr Glu Ala Gly Arg Arg Leu
 65                  70                  75                  80

gct gct ctg cgc cag tcc gcg ccc att gat ctg acc gca ttc ctg cag     288
Ala Ala Leu Arg Gln Ser Ala Pro Ile Asp Leu Thr Ala Phe Leu Gln
                 85                  90                  95

tac acc tcc ggc tcc acc cga acc cca gct ggc gtt gtt ctg acc aac     336
Tyr Thr Ser Gly Ser Thr Arg Thr Pro Ala Gly Val Val Leu Thr Asn
            100                 105                 110

cgc tcc atc ctg acc aac gtc ttg cag atc ttc agc gcc gca cag ctg 384
Arg Ser Ile Leu Thr Asn Val Leu Gln Ile Phe Ser Ala Ala Gln Leu
        115                 120                 125

aaa acc cca ctg cgc ctg gtt tca tgg ctg cca ctg cac cac gac atg 432
Lys Thr Pro Leu Arg Leu Val Ser Trp Leu Pro Leu His His Asp Met
    130                 135                 140

ggc att atc ctc gcg gcg ttt gtc act atg ctt ggc ctg gac aac gag 480
Gly Ile Ile Leu Ala Ala Phe Val Thr Met Leu Gly Leu Asp Asn Glu
145                 150                 155                 160

ttc atg aac cca cgc gat ttc gtg cag cag cct tcc cgc tgg att aag 528
Phe Met Asn Pro Arg Asp Phe Val Gln Gln Pro Ser Arg Trp Ile Lys
                165                 170                 175

cag ctc aac cgt cgc gaa agc gac gtg gac gtt aac gtc tac acc gtg 576
Gln Leu Asn Arg Arg Glu Ser Asp Val Asp Val Asn Val Tyr Thr Val
            180                 185                 190

gtt cct aac ttc gcc ctc gag ctt gca gca cgc tac gca aag cca gca 624
Val Pro Asn Phe Ala Leu Glu Leu Ala Ala Arg Tyr Ala Lys Pro Ala
        195                 200                 205

gag gga gag acc ctg gat ctt tcc gca ttg gat gcc atc att atc ggt 672
Glu Gly Glu Thr Leu Asp Leu Ser Ala Leu Asp Ala Ile Ile Ile Gly
    210                 215                 220

tcc gag cca gtc aca gaa aac gct ctg acc acc ttc cgt gaa gct ttc 720
Ser Glu Pro Val Thr Glu Asn Ala Leu Thr Thr Phe Arg Glu Ala Phe
225                 230                 235                 240

gag cct tac ggc ctg cct gtt cag acc ctg cgt cct tcc tac ggt ctt 768
Glu Pro Tyr Gly Leu Pro Val Gln Thr Leu Arg Pro Ser Tyr Gly Leu
                245                 250                 255

gca gaa gca tcc ctg ctg gtc acc acc cca cag acc gaa aac cgc cca 816
Ala Glu Ala Ser Leu Leu Val Thr Thr Pro Gln Thr Glu Asn Arg Pro
            260                 265                 270

ctg atc tcc tac ttc gac cgc gag gcc ttg gcc gaa aac cgc gtt gag 864
Leu Ile Ser Tyr Phe Asp Arg Glu Ala Leu Ala Glu Asn Arg Val Glu
        275                 280                 285

ctt gta gaa aag ggc aat aac aag gct gtt gct ttc gtc tcc aac ggc 912
Leu Val Glu Lys Gly Asn Asn Lys Ala Val Ala Phe Val Ser Asn Gly
    290                 295                 300

cag gtt gca gcc cca cag cag ctg gtc atc gtt gat tcc gaa acc gga 960
Gln Val Ala Ala Pro Gln Gln Leu Val Ile Val Asp Ser Glu Thr Gly
305                 310                 315                 320

acc gag ctg gca gac ggc cag atc ggc gaa atc tgg acc cac ggc gaa 1008
Thr Glu Leu Ala Asp Gly Gln Ile Gly Glu Ile Trp Thr His Gly Glu
                325                 330                 335

aac act gct gca ggt tac ctc gac cgc gag gaa gac acc gca gaa acc 1056
Asn Thr Ala Ala Gly Tyr Leu Asp Arg Glu Glu Asp Thr Ala Glu Thr
            340                 345                 350

ttc cgc aac cgt ctg acc acc cgc ctg gaa gaa aac tcc cgc gca gaa 1104
Phe Arg Asn Arg Leu Thr Thr Arg Leu Glu Glu Asn Ser Arg Ala Glu
        355                 360                 365

ggt gct gcc gac gac aac tac tgg atg gcc acc ggt gac ctc ggc gtc 1152
Gly Ala Ala Asp Asp Asn Tyr Trp Met Ala Thr Gly Asp Leu Gly Val
    370                 375                 380

atc gta gac aac gag ctc tac atc acc ggt cgt ctg aag gac ctc atc 1200
Ile Val Asp Asn Glu Leu Tyr Ile Thr Gly Arg Leu Lys Asp Leu Ile
385                 390                 395                 400

gtt gtc gca ggc cga aac cac tac cca cag gac atc gag tac acc gtc 1248
Val Val Ala Gly Arg Asn His Tyr Pro Gln Asp Ile Glu Tyr Thr Val
                405                 410                 415

cag gct gct tcc gca cac atc cgt gca gat tcc gtc gca gca ttc gca 1296
Gln Ala Ala Ser Ala His Ile Arg Ala Asp Ser Val Ala Ala Phe Ala
            420                 425                 430

gtc cca ggc gat gac att gaa aag ctc atc atc ctg gca gaa cgc gac 1344
Val Pro Gly Asp Asp Ile Glu Lys Leu Ile Ile Leu Ala Glu Arg Asp
        435                 440                 445

acc act gca aac gaa gcc gac gat gca gct gct gaa gaa gca atc cgc 1392
Thr Thr Ala Asn Glu Ala Asp Asp Ala Ala Ala Glu Glu Ala Ile Arg
    450                 455                 460

tcc gcc gtt ggc act gca cac ggt gtt gtt cca gaa gag atc cgt atc 1440
Ser Ala Val Gly Thr Ala His Gly Val Val Pro Glu Glu Ile Arg Ile
465                 470                 475                 480

ctc gca cct gac gag atc gcg cgt tcc tcc tcc gga aag atc gca cgc 1488
Leu Ala Pro Asp Glu Ile Ala Arg Ser Ser Ser Gly Lys Ile Ala Arg
                485                 490                 495

cgc gtc aac cag cgc aac tac att cag gaa caa gct aac tagttctttg 1537
Arg Val Asn Gln Arg Asn Tyr Ile Gln Glu Gln Ala Asn
            500                 505

cagacaccgc agg 1550

<210> 12
<211> 509
<212> PRT
<213> Corynebacterium glutamicum

<400> 12
Val Pro Val Pro Leu Tyr Asp Pro Asn Glu Pro Gly His Ala Asp His
1               5                   10                  15

Leu Asn Ala Val Phe Ala Asp Ser Glu Pro Val Val Val Leu Thr Asn
            20                  25                  30

Ser Lys Ser Ala Gly Ala Val Arg Lys His Phe Ser Ser Leu Pro Ala
        35                  40                  45

Ala Glu Arg Pro Arg Ile Leu Ser Val Asp Ser Leu Pro Asp Ser Leu
    50                  55                  60

Ala Asp Ser Tyr Glu Asn Pro Met Leu Thr Glu Ala Gly Arg Arg Leu
65                  70                  75                  80

Ala Ala Leu Arg Gln Ser Ala Pro Ile Asp Leu Thr Ala Phe Leu Gln
                85                  90                  95

Tyr Thr Ser Gly Ser Thr Arg Thr Pro Ala Gly Val Val Leu Thr Asn
            100                 105                 110

Arg Ser Ile Leu Thr Asn Val Leu Gln Ile Phe Ser Ala Ala Gln Leu
        115                 120                 125

Lys Thr Pro Leu Arg Leu Val Ser Trp Leu Pro Leu His His Asp Met
    130                 135                 140

Gly Ile Ile Leu Ala Ala Phe Val Thr Met Leu Gly Leu Asp Asn Glu
145                 150                 155                 160

Phe Met Asn Pro Arg Asp Phe Val Gln Gln Pro Ser Arg Trp Ile Lys
                165                 170                 175

Gln Leu Asn Arg Arg Glu Ser Asp Val Asp Val Asn Val Tyr Thr Val
            180                 185                 190

Val Pro Asn Phe Ala Leu Glu Leu Ala Ala Arg Tyr Ala Lys Pro Ala
        195                 200                 205

Glu Gly Glu Thr Leu Asp Leu Ser Ala Leu Asp Ala Ile Ile Ile Gly
    210                 215                 220

Ser Glu Pro Val Thr Glu Asn Ala Leu Thr Thr Phe Arg Glu Ala Phe
225                 230                 235                 240

Glu Pro Tyr Gly Leu Pro Val Gln Thr Leu Arg Pro Ser Tyr Gly Leu
                245                 250                 255

Ala Glu Ala Ser Leu Leu Val Thr Thr Pro Gln Thr Glu Asn Arg Pro
            260                 265                 270

Leu Ile Ser Tyr Phe Asp Arg Glu Ala Leu Ala Glu Asn Arg Val Glu
        275                 280                 285

Leu Val Glu Lys Gly Asn Asn Lys Ala Val Ala Phe Val Ser Asn Gly
    290                 295                 300

Gln Val Ala Ala Pro Gln Gln Leu Val Ile Val Asp Ser Glu Thr Gly
305                 310                 315                 320

Thr Glu Leu Ala Asp Gly Gln Ile Gly Glu Ile Trp Thr His Gly Glu
                325                 330                 335

Asn Thr Ala Ala Gly Tyr Leu Asp Arg Glu Glu Asp Thr Ala Glu Thr
            340                 345                 350

Phe Arg Asn Arg Leu Thr Thr Arg Leu Glu Glu Asn Ser Arg Ala Glu
        355                 360                 365

Gly Ala Ala Asp Asp Asn Tyr Trp Met Ala Thr Gly Asp Leu Gly Val
    370                 375                 380

Ile Val Asp Asn Glu Leu Tyr Ile Thr Gly Arg Leu Lys Asp Leu Ile

385                 390                 395                 400

Val Val Ala Gly Arg Asn His Tyr Pro Gln Asp Ile Glu Tyr Thr Val
                405                 410                 415

Gln Ala Ala Ser Ala His Ile Arg Ala Asp Ser Val Ala Ala Phe Ala
            420                 425                 430

Val Pro Gly Asp Asp Ile Glu Lys Leu Ile Ile Leu Ala Glu Arg Asp
        435                 440                 445

Thr Thr Ala Asn Glu Ala Asp Asp Ala Ala Ala Glu Glu Ala Ile Arg
    450                 455                 460

Ser Ala Val Gly Thr Ala His Gly Val Val Pro Glu Glu Ile Arg Ile
465                 470                 475                 480

Leu Ala Pro Asp Glu Ile Ala Arg Ser Ser Ser Gly Lys Ile Ala Arg
                485                 490                 495

Arg Val Asn Gln Arg Asn Tyr Ile Gln Glu Gln Ala Asn
            500                 505

<210> 13
<211> 4953
<212> DNA
<213> Corynebacterium glutamicum

<220>
<221> CDS
<222> (101)..(4930)
<223> RXA02582

<400> 13
tggatgtcta ttctcctcag cctcgttttg gttgaggcgg atagttttaa tattttttta 60

agtttaagtt gtaatcgagc tgaaaggctg aggcctcaat atg gaa cag agc caa   115
                                           Met Glu Gln Ser Gln
                                           1               5

tcg tcg gat cag aag atg acc gtt gaa cag gtt cgc acc tgg ctc cgt   163
Ser Ser Asp Gln Lys Met Thr Val Glu Gln Val Arg Thr Trp Leu Arg
                10                  15                  20

gat tgg gtt gtc cgc acc acg ggt att ccg gtg gag gaa gtt acg gat   211
Asp Trp Val Val Arg Thr Thr Gly Ile Pro Val Glu Glu Val Thr Asp
            25                  30                  35

gac aag gca atg gag acc ttt ggc ctt tcc tct cgc gat gtt gtt gtg   259
Asp Lys Ala Met Glu Thr Phe Gly Leu Ser Ser Arg Asp Val Val Val
        40                  45                  50

ttg tct ggt gag ctg gaa aac ctg ctg gac acc tcc ttg gat gcc acc   307
Leu Ser Gly Glu Leu Glu Asn Leu Leu Asp Thr Ser Leu Asp Ala Thr
    55                  60                  65

atc gct tat gag tac cca acg atc cgt agt ttg gcg cag cgc ctt gtt   355
Ile Ala Tyr Glu Tyr Pro Thr Ile Arg Ser Leu Ala Gln Arg Leu Val
70                  75                  80                  85

gag ggc gag cct cgt cgt gca cat acc cag cgt gaa ttg aat ttc tcc 403
Glu Gly Glu Pro Arg Arg Ala His Thr Gln Arg Glu Leu Asn Phe Ser
                90                  95                  100

gcg gtg agc gat tcc cca ggt tcc cac gat att gcg gtt gtc ggt atg 451
Ala Val Ser Asp Ser Pro Gly Ser His Asp Ile Ala Val Val Gly Met
            105                 110                 115

gct gcg cgt tac cca ggc gct gag agc ctg gag gat atg tgg aag cta 499
Ala Ala Arg Tyr Pro Gly Ala Glu Ser Leu Glu Asp Met Trp Lys Leu
        120                 125                 130

ctc gtc gag ggc cgt gac ggt atc tcg gat cta ccg att ggc cgt tgg 547
Leu Val Glu Gly Arg Asp Gly Ile Ser Asp Leu Pro Ile Gly Arg Trp
    135                 140                 145

tct gag tat gca ggc gat gag gtt atg tct cgg aag atg gaa gag ttt 595
Ser Glu Tyr Ala Gly Asp Glu Val Met Ser Arg Lys Met Glu Glu Phe
150                 155                 160                 165

tct acc atc ggt ggc tac ctg tca gat atc tct agc ttt gat gcg gag 643
Ser Thr Ile Gly Gly Tyr Leu Ser Asp Ile Ser Ser Phe Asp Ala Glu
                170                 175                 180

ttc ttt ggt ctg tct ccg ctc gag gcc gcc aac atg gat cct cag cag 691
Phe Phe Gly Leu Ser Pro Leu Glu Ala Ala Asn Met Asp Pro Gln Gln
            185                 190                 195

cgt att ttg ctg gag cta acg tgg gag gct ttg gag tac gct cgc atc 739
Arg Ile Leu Leu Glu Leu Thr Trp Glu Ala Leu Glu Tyr Ala Arg Ile
        200                 205                 210

gca cca aac act ttg cgt ggc gaa gcc gtg ggc gtg ttc ata ggt tcc 787
Ala Pro Asn Thr Leu Arg Gly Glu Ala Val Gly Val Phe Ile Gly Ser
    215                 220                 225

tcc aac aac gat tac ggc atg atg atc gct gcc gat cca gca gaa gca 835
Ser Asn Asn Asp Tyr Gly Met Met Ile Ala Ala Asp Pro Ala Glu Ala
230                 235                 240                 245

cat cct tat gcg ctg act ggt act tct agt gcg att gtc gct aac cgc 883
His Pro Tyr Ala Leu Thr Gly Thr Ser Ser Ala Ile Val Ala Asn Arg
                250                 255                 260

att aac tac gct ttc gat ttc cgc ggt cct tcc gtc aac gtg gat acc 931
Ile Asn Tyr Ala Phe Asp Phe Arg Gly Pro Ser Val Asn Val Asp Thr
            265                 270                 275

gca tgt tct tct tct ctg gta gcg gtt cac cag gct gtc cgt gcg ctg 979
Ala Cys Ser Ser Ser Leu Val Ala Val His Gln Ala Val Arg Ala Leu
        280                 285                 290

cgt aat ggc gaa gcg gat cac gct atc gct ggt gga gtg aac att ttg 1027
Arg Asn Gly Glu Ala Asp His Ala Ile Ala Gly Gly Val Asn Ile Leu
    295                 300                 305

gca tct cca ttt gtc aca act gca ttc gct gag ctc ggt gtg atc agc 1075
Ala Ser Pro Phe Val Thr Thr Ala Phe Ala Glu Leu Gly Val Ile Ser
310                 315                 320                 325

cca acc ggc aag atc cac gca ttc tct gat gat gcc gat ggt ttc gtg 1123

Pro Thr Gly Lys Ile His Ala Phe Ser Asp Asp Ala Asp Gly Phe Val
                330                 335                 340

cgt tcc gac ggc gcc gga gtc gtt gtg ctg aag cgc gtg gat gac gca     1171
Arg Ser Asp Gly Ala Gly Val Val Val Leu Lys Arg Val Asp Asp Ala
            345                 350                 355

atc cgc gac ggc gac aag atc atc ggt gtg atc aag ggt tct gca gtg     1219
Ile Arg Asp Gly Asp Lys Ile Ile Gly Val Ile Lys Gly Ser Ala Val
        360                 365                 370

aac tcc gat ggt cac tcc aac gga ctg acc gct cca aac cct gac gca     1267
Asn Ser Asp Gly His Ser Asn Gly Leu Thr Ala Pro Asn Pro Asp Ala
    375                 380                 385

cag gtt gat gtg ctg cag cgt gca tat gtt gac gct cag gtt gat ccc     1315
Gln Val Asp Val Leu Gln Arg Ala Tyr Val Asp Ala Gln Val Asp Pro
390                 395                 400                 405

acc acc gtg gat tac gtt gag gct cac ggc acc ggc acc atc ctg ggt     1363
Thr Thr Val Asp Tyr Val Glu Ala His Gly Thr Gly Thr Ile Leu Gly
                410                 415                 420

gac ccc att gaa gca act gcc ctg ggt gct gtt ttg ggc tat ggc cgt     1411
Asp Pro Ile Glu Ala Thr Ala Leu Gly Ala Val Leu Gly Tyr Gly Arg
            425                 430                 435

gac gca tcc acc cca act ctt ctg ggc tct gca aag tcc aac ttc ggc     1459
Asp Ala Ser Thr Pro Thr Leu Leu Gly Ser Ala Lys Ser Asn Phe Gly
        440                 445                 450

cac act gag tca gct gcg ggt att gca ggt gtc atc aag gtg ctg ctt     1507
His Thr Glu Ser Ala Ala Gly Ile Ala Gly Val Ile Lys Val Leu Leu
    455                 460                 465

gct ttg cag aac aag act ctg cca cca acc gtg aac ttt gca ggt cca     1555
Ala Leu Gln Asn Lys Thr Leu Pro Pro Thr Val Asn Phe Ala Gly Pro
470                 475                 480                 485

aac cgc tac atc gat ttc gat gct gag cgt ttg gaa gtt gtg gaa gat     1603
Asn Arg Tyr Ile Asp Phe Asp Ala Glu Arg Leu Glu Val Val Glu Asp
                490                 495                 500

cca cgt gaa tgg ccg gaa tac aac ggt cac gca gta gct ggt gtg tct     1651
Pro Arg Glu Trp Pro Glu Tyr Asn Gly His Ala Val Ala Gly Val Ser
            505                 510                 515

gcg ttc ggg ttc ggt gga acc aac gcg cac gtg gtg atc tct gag tac     1699
Ala Phe Gly Phe Gly Gly Thr Asn Ala His Val Val Ile Ser Glu Tyr
        520                 525                 530

aac gca gaa gac tac gaa acc cgt gcc ccg aag gag gcg ttg ctt cct     1747
Asn Ala Glu Asp Tyr Glu Thr Arg Ala Pro Lys Glu Ala Leu Leu Pro
    535                 540                 545

gac cag cag gtt gcc ctg ccg gtg tct ggt cac ctg cca tcc agg cgt     1795
Asp Gln Gln Val Ala Leu Pro Val Ser Gly His Leu Pro Ser Arg Arg
550                 555                 560                 565

cga caa gca gct gct gac ttg gcg gac ttc ttg gag ggc cgc aaa gat     1843
Arg Gln Ala Ala Ala Asp Leu Ala Asp Phe Leu Glu Gly Arg Lys Asp

570                 575                 580

tgc gac cta acc cca gta gcc cgc gcg ctg gca ggc cgc aat cat ggc    1891
Cys Asp Leu Thr Pro Val Ala Arg Ala Leu Ala Gly Arg Asn His Gly
            585                 590                 595

cgc tcc cgc gca gtc gtg ctt gca agc act atc gag gaa gcc gta aag    1939
Arg Ser Arg Ala Val Val Leu Ala Ser Thr Ile Glu Glu Ala Val Lys
        600                 605                 610

cgc ctg cgc cag gtc gca gaa ggc aag gtc agc gtc ggt atc tcc gca    1987
Arg Leu Arg Gln Val Ala Glu Gly Lys Val Ser Val Gly Ile Ser Ala
    615                 620                 625

gct gat tcc cca gcg gcc aac ggt cct gtg ttt gtg tac tcc ggt ttc    2035
Ala Asp Ser Pro Ala Ala Asn Gly Pro Val Phe Val Tyr Ser Gly Phe
630                 635                 640                 645

gga tcc cag cac cgc ctc atg atc aag gaa ttg tgc tca att tcg cca    2083
Gly Ser Gln His Arg Leu Met Ile Lys Glu Leu Cys Ser Ile Ser Pro
                650                 655                 660

cag ttc cgc gag cgc atc gaa gag ctc gat gaa atg gtt aag ttc gag    2131
Gln Phe Arg Glu Arg Ile Glu Glu Leu Asp Glu Met Val Lys Phe Glu
            665                 670                 675

tcc ggc tgg tcc atc atg aaa ctt gtt ttg gat gat gag caa act tac    2179
Ser Gly Trp Ser Ile Met Lys Leu Val Leu Asp Asp Glu Gln Thr Tyr
        680                 685                 690

gac acc gag acc gcg cag gtt gtg atc acc gca atc cag atc gca ctg    2227
Asp Thr Glu Thr Ala Gln Val Val Ile Thr Ala Ile Gln Ile Ala Leu
    695                 700                 705

act gac ctg ttg gca agc ttc ggt gta aag cca gct gcc gtc atg ggc    2275
Thr Asp Leu Leu Ala Ser Phe Gly Val Lys Pro Ala Ala Val Met Gly
710                 715                 720                 725

atg tcg atg ggt gag atc gct gcc gct tac gct gcc ggt gga ctc agc    2323
Met Ser Met Gly Glu Ile Ala Ala Ala Tyr Ala Ala Gly Gly Leu Ser
                730                 735                 740

gac cgc gat acc atg ctc att gcc agc cac cgt tcc cgc cta atg ggc    2371
Asp Arg Asp Thr Met Leu Ile Ala Ser His Arg Ser Arg Leu Met Gly
            745                 750                 755

gag ggc gag aag tcc ctg gct gag gat cag ctg ggt gcc atg gct gtg    2419
Glu Gly Glu Lys Ser Leu Ala Glu Asp Gln Leu Gly Ala Met Ala Val
        760                 765                 770

gtg gaa ttc gcc gct gcg gac ctg gat aag ttc atc gaa gaa aac cct    2467
Val Glu Phe Ala Ala Ala Asp Leu Asp Lys Phe Ile Glu Glu Asn Pro
    775                 780                 785

gag tac aag ggc att gag cct gct gtt tat gca ggc cca ggc atg act    2515
Glu Tyr Lys Gly Ile Glu Pro Ala Val Tyr Ala Gly Pro Gly Met Thr
790                 795                 800                 805

acc gtg ggt gga cct cgc gac gcc gtg gtt cag ttc gta gag aag ctg    2563
Thr Val Gly Gly Pro Arg Asp Ala Val Val Gln Phe Val Glu Lys Leu
                810                 815                 820

gaa tct gaa gac aag ttt gcc cgc ctg ctc aac gtc aag ggc gct ggc 2611
Glu Ser Glu Asp Lys Phe Ala Arg Leu Leu Asn Val Lys Gly Ala Gly
            825                 830                 835

cac acc tct gct gtg gaa cca ctt ctt ggt gag ctc gct ggc gaa atc 2659
His Thr Ser Ala Val Glu Pro Leu Leu Gly Glu Leu Ala Gly Glu Ile
        840                 845                 850

gct ggc atc gag cca ctt ccg ctg cag atc ccg ttg ttc agc tct gtc 2707
Ala Gly Ile Glu Pro Leu Pro Leu Gln Ile Pro Leu Phe Ser Ser Val
    855                 860                 865

gat cag ggt gtt acc tac cca gtt gga gca gtg gtc cac gac gcc gat 2755
Asp Gln Gly Val Thr Tyr Pro Val Gly Ala Val Val His Asp Ala Asp
870                 875                 880                 885

tac atg ctc cgc tgt acc cgc cag tcc gtg tac ttc cag gac tcc acc 2803
Tyr Met Leu Arg Cys Thr Arg Gln Ser Val Tyr Phe Gln Asp Ser Thr
                890                 895                 900

gaa gct gca ttc gct gca ggc cac aac acc ttg gtg gaa att tcc ccg 2851
Glu Ala Ala Phe Ala Ala Gly His Asn Thr Leu Val Glu Ile Ser Pro
            905                 910                 915

aac cca gtt gca ctc atg ggc atg atg aac acc gcg ttc acc gtg ggc 2899
Asn Pro Val Ala Leu Met Gly Met Met Asn Thr Ala Phe Thr Val Gly
        920                 925                 930

aag cct gat gca cag ctg ctg ttc agc ctg aag cga aaa gtc cca gag 2947
Lys Pro Asp Ala Gln Leu Leu Phe Ser Leu Lys Arg Lys Val Pro Glu
    935                 940                 945

gca gaa tcc ctc cgc gac ctt ctg gcg aag ctg tac gtc aac ggc gca 2995
Ala Glu Ser Leu Arg Asp Leu Leu Ala Lys Leu Tyr Val Asn Gly Ala
950                 955                 960                 965

aac gtt gat ttc tcc gcg ctg tac ggc gaa ggt gaa acc atc gat ccg 3043
Asn Val Asp Phe Ser Ala Leu Tyr Gly Glu Gly Glu Thr Ile Asp Pro
                970                 975                 980

cca cac atc acc tgg aag cac cag cgc ttc tgg act tcc gca cgc cca 3091
Pro His Ile Thr Trp Lys His Gln Arg Phe Trp Thr Ser Ala Arg Pro
            985                 990                 995

tcc tct ggc gca tcc ctg gat ctg ccg ggc ttc cgc gtg aat ctg cca 3139
Ser Ser Gly Ala Ser Leu Asp Leu Pro Gly Phe Arg Val Asn Leu Pro
        1000                1005                1010

aac aac act gtg gcg ttt tcc acc gca gcc gaa ctg gca cca tcc gca 3187
Asn Asn Thr Val Ala Phe Ser Thr Ala Ala Glu Leu Ala Pro Ser Ala
    1015                1020                1025

gtg gca atc atg gaa gca gcc gcc atg gct gtc acc cca ggt tcc tcc 3235
Val Ala Ile Met Glu Ala Ala Ala Met Ala Val Thr Pro Gly Ser Ser
1030                1035                1040                1045

gtc gac gca gtg gac gag cgc gac atg ctg cca cca agc ggc gaa atc 3283
Val Asp Ala Val Asp Glu Arg Asp Met Leu Pro Pro Ser Gly Glu Ile
                1050                1055                1060

acc acc atc gtg act cgc tcc ttg ggc ggt ttg agc ctg tcc gta tac 3331
Thr Thr Ile Val Thr Arg Ser Leu Gly Gly Leu Ser Leu Ser Val Tyr
            1065                1070                1075

aag att gag gga acc acc agc act ctt gtc gcc gaa ggt ttc gca gcc 3379
Lys Ile Glu Gly Thr Thr Ser Thr Leu Val Ala Glu Gly Phe Ala Ala
        1080                1085                1090

aac cca gga ttc gcc gca gca tct tcc ttc gac ggt cca ggc tac gac 3427
Asn Pro Gly Phe Ala Ala Ala Ser Ser Phe Asp Gly Pro Gly Tyr Asp
    1095                1100                1105

gga ttc aac acc gat tac agc gac cag cca gac cct cgc tcc gac ctg 3475
Gly Phe Asn Thr Asp Tyr Ser Asp Gln Pro Asp Pro Arg Ser Asp Leu
1110                1115                1120                1125

cct ttg gac atc gaa gca gtc cgc tgg gac cca gcg acc gaa acc gtc 3523
Pro Leu Asp Ile Glu Ala Val Arg Trp Asp Pro Ala Thr Glu Thr Val
                1130                1135                1140

gaa gag cgc atg cgc gcc atc gtc tcc gaa gca atg ggc tac gac gtg 3571
Glu Glu Arg Met Arg Ala Ile Val Ser Glu Ala Met Gly Tyr Asp Val
            1145                1150                1155

gat gac ctc cca cgc gaa ctc cca ctg att gac ctc ggc ctc gac tcc 3619
Asp Asp Leu Pro Arg Glu Leu Pro Leu Ile Asp Leu Gly Leu Asp Ser
        1160                1165                1170

ctc atg ggc atg cgc atc aaa aac cgc atc gaa aat gac ttc cag atc 3667
Leu Met Gly Met Arg Ile Lys Asn Arg Ile Glu Asn Asp Phe Gln Ile
    1175                1180                1185

cca cca ctc cag gtc caa gcg ctc cgc gat gca tcc gtc gct gac gtg 3715
Pro Pro Leu Gln Val Gln Ala Leu Arg Asp Ala Ser Val Ala Asp Val
1190                1195                1200                1205

gta atc atg gtg gaa aac atg gtc gct ggc cgc tcc tct gag acg ctt 3763
Val Ile Met Val Glu Asn Met Val Ala Gly Arg Ser Ser Glu Thr Leu
                1210                1215                1220

gtc gac gcc acc ccg cag gtg cca gct gag gca gca ggg gag gct caa 3811
Val Asp Ala Thr Pro Gln Val Pro Ala Glu Ala Ala Gly Glu Ala Gln
            1225                1230                1235

gct gct gag tct tct gct tcg ggc gag gac gtg cag ggc gtt ggc gtt 3859
Ala Ala Glu Ser Ser Ala Ser Gly Glu Asp Val Gln Gly Val Gly Val
        1240                1245                1250

gca ccg cga gat gcg tcg gaa cgc atg gtc ttt ggt act tgg gca ggc 3907
Ala Pro Arg Asp Ala Ser Glu Arg Met Val Phe Gly Thr Trp Ala Gly
    1255                1260                1265

ctg act ggc gct gcg gca gct ggc gtg acc agc aag ttg cca cag atc 3955
Leu Thr Gly Ala Ala Ala Ala Gly Val Thr Ser Lys Leu Pro Gln Ile
1270                1275                1280                1285

gat gtc gat act gca acc gca att gcc gag cgc ctc acc gag cgt tcc 4003
Asp Val Asp Thr Ala Thr Ala Ile Ala Glu Arg Leu Thr Glu Arg Ser
                1290                1295                1300

gga att gaa atc agc act gaa cag gtg ttg gct gca gaa acc ctc gaa 4051

Gly Ile Glu Ile Ser Thr Glu Gln Val Leu Ala Ala Glu Thr Leu Glu
            1305                1310                1315

cca ctg tct gac ctg gtg cgt gaa ggc ctc gaa act gaa gtt cag ggc    4099
Pro Leu Ser Asp Leu Val Arg Glu Gly Leu Glu Thr Glu Val Gln Gly
        1320                1325                1330

aac att cgt gtg ctg cgt gga cgt gca gaa ggc tcc acc aag cct gca    4147
Asn Ile Arg Val Leu Arg Gly Arg Ala Glu Gly Ser Thr Lys Pro Ala
    1335                1340                1345

gtg ttc atg ttc cac cca gct ggc ggt tct tcc gtg gtc tac caa cca    4195
Val Phe Met Phe His Pro Ala Gly Gly Ser Ser Val Val Tyr Gln Pro
1350                1355                1360                1365

cta atg cgt cgt ctg cct gaa gat gtc cct gtc tat ggc gtt gag cgt    4243
Leu Met Arg Arg Leu Pro Glu Asp Val Pro Val Tyr Gly Val Glu Arg
                1370                1375                1380

ctg gaa ggc gat ctc gct gac cgc gcg gca gca tat gtt gat gac atc    4291
Leu Glu Gly Asp Leu Ala Asp Arg Ala Ala Ala Tyr Val Asp Asp Ile
            1385                1390                1395

aag aag tac tcc gat ggc ttc cca gta gtt ttg ggt ggc tgg agc ttc    4339
Lys Lys Tyr Ser Asp Gly Phe Pro Val Val Leu Gly Gly Trp Ser Phe
        1400                1405                1410

ggc ggt gcc gtt gcc ttc gag gtt gcc cac caa ctg gtt ggc tcc gat    4387
Gly Gly Ala Val Ala Phe Glu Val Ala His Gln Leu Val Gly Ser Asp
    1415                1420                1425

gtt gag gta gct acc gtg gcg ttg ctg gat act gtg cag cct tca aac    4435
Val Glu Val Ala Thr Val Ala Leu Leu Asp Thr Val Gln Pro Ser Asn
1430                1435                1440                1445

cca gca cca gat acc gct gag gaa act cgt gca cgc tgg act cgc tac    4483
Pro Ala Pro Asp Thr Ala Glu Glu Thr Arg Ala Arg Trp Thr Arg Tyr
                1450                1455                1460

gcg gac ttc gcc aag aag acc tac ggc ctt gat ttc gag gta cct ttt    4531
Ala Asp Phe Ala Lys Lys Thr Tyr Gly Leu Asp Phe Glu Val Pro Phe
            1465                1470                1475

gaa atc ttg gac acc atc ggt gaa gac gga atg ctg tcc atg atg acc    4579
Glu Ile Leu Asp Thr Ile Gly Glu Asp Gly Met Leu Ser Met Met Thr
        1480                1485                1490

gac ttc ctg gcc aac acc gac gcc tcc gag cac gga ttg tcc gca ggt    4627
Asp Phe Leu Ala Asn Thr Asp Ala Ser Glu His Gly Leu Ser Ala Gly
    1495                1500                1505

gtg ctt gaa cac cag cgc gct tct ttt gtg gac aac cgc atc ctg gct    4675
Val Leu Glu His Gln Arg Ala Ser Phe Val Asp Asn Arg Ile Leu Ala
1510                1515                1520                1525

aaa ctt aat ttt gca gac tgg gcc aac gtt gaa gcc cct gtc atc ctg    4723
Lys Leu Asn Phe Ala Asp Trp Ala Asn Val Glu Ala Pro Val Ile Leu
                1530                1535                1540

ttc cgc gcg gaa cgc atg cat gat gga gct atc gaa ctt gaa cca aac    4771
Phe Arg Ala Glu Arg Met His Asp Gly Ala Ile Glu Leu Glu Pro Asn

1545                1550                1555

tat gcc aag att gat caa gat gga gga tgg tcc gga att gtc aac gat    4819
Tyr Ala Lys Ile Asp Gln Asp Gly Gly Trp Ser Gly Ile Val Asn Asp
        1560                1565                1570

ttg gaa att gtt cag ctg aat ggt gac cac ctg gca gtt gtc gat gaa    4867
Leu Glu Ile Val Gln Leu Asn Gly Asp His Leu Ala Val Val Asp Glu
    1575                1580                1585

cca gaa atc ggc aca gtc gga gct cat ttg agt cgc cgc att gat gag    4915
Pro Glu Ile Gly Thr Val Gly Ala His Leu Ser Arg Arg Ile Asp Glu
1590                1595                1600                1605

att tct cgg aag aat tagtaacgga gagctgacgg aag                      4953
Ile Ser Arg Lys Asn
                1610

<210> 14
<211> 1610
<212> PRT
<213> Corynebacterium glutamicum

<400> 14
Met Glu Gln Ser Gln Ser Ser Asp Gln Lys Met Thr Val Glu Gln Val
1               5                   10                  15

Arg Thr Trp Leu Arg Asp Trp Val Val Arg Thr Thr Gly Ile Pro Val
            20                  25                  30

Glu Glu Val Thr Asp Asp Lys Ala Met Glu Thr Phe Gly Leu Ser Ser
        35                  40                  45

Arg Asp Val Val Val Leu Ser Gly Glu Leu Glu Asn Leu Leu Asp Thr
    50                  55                  60

Ser Leu Asp Ala Thr Ile Ala Tyr Glu Tyr Pro Thr Ile Arg Ser Leu
65                  70                  75                  80

Ala Gln Arg Leu Val Glu Gly Glu Pro Arg Arg Ala His Thr Gln Arg
                85                  90                  95

Glu Leu Asn Phe Ser Ala Val Ser Asp Ser Pro Gly Ser His Asp Ile
            100                 105                 110

Ala Val Val Gly Met Ala Ala Arg Tyr Pro Gly Ala Glu Ser Leu Glu
        115                 120                 125

Asp Met Trp Lys Leu Leu Val Glu Gly Arg Asp Gly Ile Ser Asp Leu
    130                 135                 140

Pro Ile Gly Arg Trp Ser Glu Tyr Ala Gly Asp Glu Val Met Ser Arg
145                 150                 155                 160

Lys Met Glu Glu Phe Ser Thr Ile Gly Gly Tyr Leu Ser Asp Ile Ser
                165                 170                 175

Ser Phe Asp Ala Glu Phe Phe Gly Leu Ser Pro Leu Glu Ala Ala Asn
            180                 185                 190

Met Asp Pro Gln Gln Arg Ile Leu Leu Glu Leu Thr Trp Glu Ala Leu
        195                 200                 205

Glu Tyr Ala Arg Ile Ala Pro Asn Thr Leu Arg Gly Glu Ala Val Gly
    210                 215                 220

Val Phe Ile Gly Ser Ser Asn Asn Asp Tyr Gly Met Met Ile Ala Ala
225                 230                 235                 240

Asp Pro Ala Glu Ala His Pro Tyr Ala Leu Thr Gly Thr Ser Ser Ala
                245                 250                 255

Ile Val Ala Asn Arg Ile Asn Tyr Ala Phe Asp Phe Arg Gly Pro Ser
            260                 265                 270

Val Asn Val Asp Thr Ala Cys Ser Ser Ser Leu Val Ala Val His Gln
        275                 280                 285

Ala Val Arg Ala Leu Arg Asn Gly Glu Ala Asp His Ala Ile Ala Gly
    290                 295                 300

Gly Val Asn Ile Leu Ala Ser Pro Phe Val Thr Thr Ala Phe Ala Glu
305                 310                 315                 320

Leu Gly Val Ile Ser Pro Thr Gly Lys Ile His Ala Phe Ser Asp Asp
                325                 330                 335

Ala Asp Gly Phe Val Arg Ser Asp Gly Ala Gly Val Val Val Leu Lys
            340                 345                 350

Arg Val Asp Asp Ala Ile Arg Asp Gly Asp Lys Ile Ile Gly Val Ile
        355                 360                 365

Lys Gly Ser Ala Val Asn Ser Asp Gly His Ser Asn Gly Leu Thr Ala
    370                 375                 380

Pro Asn Pro Asp Ala Gln Val Asp Val Leu Gln Arg Ala Tyr Val Asp
385                 390                 395                 400

Ala Gln Val Asp Pro Thr Thr Val Asp Tyr Val Glu Ala His Gly Thr
                405                 410                 415

Gly Thr Ile Leu Gly Asp Pro Ile Glu Ala Thr Ala Leu Gly Ala Val
            420                 425                 430

Leu Gly Tyr Gly Arg Asp Ala Ser Thr Pro Thr Leu Leu Gly Ser Ala
        435                 440                 445

Lys Ser Asn Phe Gly His Thr Glu Ser Ala Ala Gly Ile Ala Gly Val
    450                 455                 460

Ile Lys Val Leu Leu Ala Leu Gln Asn Lys Thr Leu Pro Pro Thr Val
465                 470                 475                 480

Asn Phe Ala Gly Pro Asn Arg Tyr Ile Asp Phe Asp Ala Glu Arg Leu
                485                 490                 495

Glu Val Val Glu Asp Pro Arg Glu Trp Pro Glu Tyr Asn Gly His Ala
            500                 505                 510

Val Ala Gly Val Ser Ala Phe Gly Phe Gly Gly Thr Asn Ala His Val

        515                 520                 525

Val Ile Ser Glu Tyr Asn Ala Glu Asp Tyr Glu Thr Arg Ala Pro Lys
    530                 535                 540

Glu Ala Leu Leu Pro Asp Gln Gln Val Ala Leu Pro Val Ser Gly His
545                 550                 555                 560

Leu Pro Ser Arg Arg Arg Gln Ala Ala Ala Asp Leu Ala Asp Phe Leu
                565                 570                 575

Glu Gly Arg Lys Asp Cys Asp Leu Thr Pro Val Ala Arg Ala Leu Ala
            580                 585                 590

Gly Arg Asn His Gly Arg Ser Arg Ala Val Val Leu Ala Ser Thr Ile
        595                 600                 605

Glu Glu Ala Val Lys Arg Leu Arg Gln Val Ala Glu Gly Lys Val Ser
    610                 615                 620

Val Gly Ile Ser Ala Ala Asp Ser Pro Ala Ala Asn Gly Pro Val Phe
625                 630                 635                 640

Val Tyr Ser Gly Phe Gly Ser Gln His Arg Leu Met Ile Lys Glu Leu
                645                 650                 655

Cys Ser Ile Ser Pro Gln Phe Arg Glu Arg Ile Glu Glu Leu Asp Glu
            660                 665                 670

Met Val Lys Phe Glu Ser Gly Trp Ser Ile Met Lys Leu Val Leu Asp
        675                 680                 685

Asp Glu Gln Thr Tyr Asp Thr Glu Thr Ala Gln Val Val Ile Thr Ala
    690                 695                 700

Ile Gln Ile Ala Leu Thr Asp Leu Leu Ala Ser Phe Gly Val Lys Pro
705                 710                 715                 720

Ala Ala Val Met Gly Met Ser Met Gly Glu Ile Ala Ala Ala Tyr Ala
                725                 730                 735

Ala Gly Gly Leu Ser Asp Arg Asp Thr Met Leu Ile Ala Ser His Arg
            740                 745                 750

Ser Arg Leu Met Gly Glu Gly Glu Lys Ser Leu Ala Glu Asp Gln Leu
        755                 760                 765

Gly Ala Met Ala Val Val Glu Phe Ala Ala Ala Asp Leu Asp Lys Phe
    770                 775                 780

Ile Glu Glu Asn Pro Glu Tyr Lys Gly Ile Glu Pro Ala Val Tyr Ala
785                 790                 795                 800

Gly Pro Gly Met Thr Thr Val Gly Gly Pro Arg Asp Ala Val Val Gln
                805                 810                 815

Phe Val Glu Lys Leu Glu Ser Glu Asp Lys Phe Ala Arg Leu Leu Asn
            820                 825                 830

Val Lys Gly Ala Gly His Thr Ser Ala Val Glu Pro Leu Leu Gly Glu
        835                 840                 845

Leu Ala Gly Glu Ile Ala Gly Ile Glu Pro Leu Pro Leu Gln Ile Pro
    850                 855                 860

Leu Phe Ser Ser Val Asp Gln Gly Val Thr Tyr Pro Val Gly Ala Val
865                 870                 875                 880

Val His Asp Ala Asp Tyr Met Leu Arg Cys Thr Arg Gln Ser Val Tyr
                885                 890                 895

Phe Gln Asp Ser Thr Glu Ala Ala Phe Ala Ala Gly His Asn Thr Leu
            900                 905                 910

Val Glu Ile Ser Pro Asn Pro Val Ala Leu Met Gly Met Met Asn Thr
        915                 920                 925

Ala Phe Thr Val Gly Lys Pro Asp Ala Gln Leu Leu Phe Ser Leu Lys
    930                 935                 940

Arg Lys Val Pro Glu Ala Glu Ser Leu Arg Asp Leu Leu Ala Lys Leu
945                 950                 955                 960

Tyr Val Asn Gly Ala Asn Val Asp Phe Ser Ala Leu Tyr Gly Glu Gly
                965                 970                 975

Glu Thr Ile Asp Pro Pro His Ile Thr Trp Lys His Gln Arg Phe Trp
            980                 985                 990

Thr Ser Ala Arg Pro Ser Ser Gly Ala Ser Leu Asp Leu Pro Gly Phe
        995                 1000                1005

Arg Val Asn Leu Pro Asn Asn Thr Val Ala Phe Ser Thr Ala Ala Glu
    1010                1015                1020

Leu Ala Pro Ser Ala Val Ala Ile Met Glu Ala Ala Ala Met Ala Val
1025                1030                1035                1040

Thr Pro Gly Ser Ser Val Asp Ala Val Asp Glu Arg Asp Met Leu Pro
                1045                1050                1055

Pro Ser Gly Glu Ile Thr Thr Ile Val Thr Arg Ser Leu Gly Gly Leu
            1060                1065                1070

Ser Leu Ser Val Tyr Lys Ile Glu Gly Thr Thr Ser Thr Leu Val Ala
        1075                1080                1085

Glu Gly Phe Ala Ala Asn Pro Gly Phe Ala Ala Ala Ser Ser Phe Asp
    1090                1095                1100

Gly Pro Gly Tyr Asp Gly Phe Asn Thr Asp Tyr Ser Asp Gln Pro Asp
1105                1110                1115                1120

Pro Arg Ser Asp Leu Pro Leu Asp Ile Glu Ala Val Arg Trp Asp Pro
                1125                1130                1135

Ala Thr Glu Thr Val Glu Glu Arg Met Arg Ala Ile Val Ser Glu Ala
            1140                1145                1150

Met Gly Tyr Asp Val Asp Asp Leu Pro Arg Glu Leu Pro Leu Ile Asp
        1155                1160                1165

Leu Gly Leu Asp Ser Leu Met Gly Met Arg Ile Lys Asn Arg Ile Glu
    1170                1175                1180

Asn Asp Phe Gln Ile Pro Pro Leu Gln Val Gln Ala Leu Arg Asp Ala
1185                1190                1195                1200

Ser Val Ala Asp Val Val Ile Met Val Glu Asn Met Val Ala Gly Arg
                1205                1210                1215

Ser Ser Glu Thr Leu Val Asp Ala Thr Pro Gln Val Pro Ala Glu Ala
            1220                1225                1230

Ala Gly Glu Ala Gln Ala Ala Glu Ser Ser Ala Ser Gly Glu Asp Val
        1235                1240                1245

Gln Gly Val Gly Val Ala Pro Arg Asp Ala Ser Glu Arg Met Val Phe
    1250                1255                1260

Gly Thr Trp Ala Gly Leu Thr Gly Ala Ala Ala Ala Gly Val Thr Ser
1265                1270                1275                1280

Lys Leu Pro Gln Ile Asp Val Asp Thr Ala Thr Ala Ile Ala Glu Arg
                1285                1290                1295

Leu Thr Glu Arg Ser Gly Ile Glu Ile Ser Thr Glu Gln Val Leu Ala
            1300                1305                1310

Ala Glu Thr Leu Glu Pro Leu Ser Asp Leu Val Arg Glu Gly Leu Glu
        1315                1320                1325

Thr Glu Val Gln Gly Asn Ile Arg Val Leu Arg Gly Arg Ala Glu Gly
    1330                1335                1340

Ser Thr Lys Pro Ala Val Phe Met Phe His Pro Ala Gly Gly Ser Ser
1345                1350                1355                1360

Val Val Tyr Gln Pro Leu Met Arg Arg Leu Pro Glu Asp Val Pro Val
                1365                1370                1375

Tyr Gly Val Glu Arg Leu Glu Gly Asp Leu Ala Asp Arg Ala Ala Ala
            1380                1385                1390

Tyr Val Asp Asp Ile Lys Lys Tyr Ser Asp Gly Phe Pro Val Val Leu
        1395                1400                1405

Gly Gly Trp Ser Phe Gly Gly Ala Val Ala Phe Glu Val Ala His Gln
    1410                1415                1420

Leu Val Gly Ser Asp Val Glu Val Ala Thr Val Ala Leu Leu Asp Thr
1425                1430                1435                1440

Val Gln Pro Ser Asn Pro Ala Pro Asp Thr Ala Glu Glu Thr Arg Ala
                1445                1450                1455

Arg Trp Thr Arg Tyr Ala Asp Phe Ala Lys Lys Thr Tyr Gly Leu Asp
            1460                1465                1470

Phe Glu Val Pro Phe Glu Ile Leu Asp Thr Ile Gly Glu Asp Gly Met
        1475                1480                1485

Leu Ser Met Met Thr Asp Phe Leu Ala Asn Thr Asp Ala Ser Glu His

1490                1495                1500

Gly Leu Ser Ala Gly Val Leu Glu His Gln Arg Ala Ser Phe Val Asp
1505                1510                1515                1520

Asn Arg Ile Leu Ala Lys Leu Asn Phe Ala Asp Trp Ala Asn Val Glu
                1525                1530                1535

Ala Pro Val Ile Leu Phe Arg Ala Glu Arg Met His Asp Gly Ala Ile
            1540                1545                1550

Glu Leu Glu Pro Asn Tyr Ala Lys Ile Asp Gln Asp Gly Gly Trp Ser
        1555                1560                1565

Gly Ile Val Asn Asp Leu Glu Ile Val Gln Leu Asn Gly Asp His Leu
    1570                1575                1580

Ala Val Val Asp Glu Pro Glu Ile Gly Thr Val Gly Ala His Leu Ser
1585                1590                1595                1600

Arg Arg Ile Asp Glu Ile Ser Arg Lys Asn
                1605                1610


<210> 15
<211> 540
<212> DNA
<213> Corynebacterium glutamicum

<220>
<221> CDS
<222> (101)..(517)
<223> RXA01138

<400> 15
cttattcaag tttttggaaa gataattaac tatgacttta aaaacagtaa acggaacgca 60

gctgcgagac actgtcggat cgttcccttc aggtgtcacg gtg gtc acc act acc   115
                                            Val Val Thr Thr Thr
                                            1               5

gat ggg gaa gtg gac cac ggg ttg act gtg agt gcg ttc gtg tcc ttg   163
Asp Gly Glu Val Asp His Gly Leu Thr Val Ser Ala Phe Val Ser Leu
                10                  15                  20

tcg ttg gag cct gcc atg gtg ttg gtg agt atc gat aag aaa tca agc   211
Ser Leu Glu Pro Ala Met Val Leu Val Ser Ile Asp Lys Lys Ser Ser
            25                  30                  35

gtt gtg ccg ttt ttg gag cag ggt tca cca gtt gct gtg tct gtg tta   259
Val Val Pro Phe Leu Glu Gln Gly Ser Pro Val Ala Val Ser Val Leu
        40                  45                  50

tcg gaa gag cag agc gat ttg gcc atc aca ttt ggc cgt cat ctg gaa   307
Ser Glu Glu Gln Ser Asp Leu Ala Ile Thr Phe Gly Arg His Leu Glu
    55                  60                  65

aac aaa ttc gac ggc gtt tcc att aag cgt tca aca aac agg gca gcg   355
Asn Lys Phe Asp Gly Val Ser Ile Lys Arg Ser Thr Asn Arg Ala Ala
70                  75                  80                  85

gtc ttg gaa ggt gcg tca gca tgg ttg agt ggc gct gtg gtg gat aaa    403
Val Leu Glu Gly Ala Ser Ala Trp Leu Ser Gly Ala Val Val Asp Lys
                90                  95                  100

tac cca ggt gga gat cac ttt att atc acc att gcc gtg gaa gag tgt    451
Tyr Pro Gly Gly Asp His Phe Ile Ile Thr Ile Ala Val Glu Glu Cys
            105                 110                 115

gct cac gac gag gag caa aag cca ctt ctt tac cac cgt ggc agg ctt    499
Ala His Asp Glu Glu Gln Lys Pro Leu Leu Tyr His Arg Gly Arg Leu
        120                 125                 130

ttt cag tgg caa gaa gat taattctcca ccccttcatt ttc                  540
Phe Gln Trp Gln Glu Asp
    135

<210> 16
<211> 139
<212> PRT
<213> Corynebacterium glutamicum

<400> 16
Val Val Thr Thr Thr Asp Gly Glu Val Asp His Gly Leu Thr Val Ser
  1               5                  10                  15

Ala Phe Val Ser Leu Ser Leu Glu Pro Ala Met Val Leu Val Ser Ile
             20                  25                  30

Asp Lys Lys Ser Ser Val Val Pro Phe Leu Glu Gln Gly Ser Pro Val
         35                  40                  45

Ala Val Ser Val Leu Ser Glu Glu Gln Ser Asp Leu Ala Ile Thr Phe
     50                  55                  60

Gly Arg His Leu Glu Asn Lys Phe Asp Gly Val Ser Ile Lys Arg Ser
 65                  70                  75                  80

Thr Asn Arg Ala Ala Val Leu Glu Gly Ala Ser Ala Trp Leu Ser Gly
                 85                  90                  95

Ala Val Val Asp Lys Tyr Pro Gly Gly Asp His Phe Ile Ile Thr Ile
            100                 105                 110

Ala Val Glu Glu Cys Ala His Asp Glu Glu Gln Lys Pro Leu Leu Tyr
        115                 120                 125

His Arg Gly Arg Leu Phe Gln Trp Gln Glu Asp
    130                 135

<210> 17
<211> 756
<212> DNA
<213> Corynebacterium glutamicum

<220>
<221> CDS
<222> (101)..(733)
<223> RXA01980

<400> 17
aggatcgtac caaataaggc aataaactct ttttactttt cctcaacttc ctgaaaagtc 60

gccgccctag aattcactaa gtttccgata tctttaaccc atg cag atc att gat 115
Met Gln Ile Ile Asp
1               5

ctc tct cat gcg ttc gcg ccc gga caa ccc cac tac cct ggg gat cca 163
Leu Ser His Ala Phe Ala Pro Gly Gln Pro His Tyr Pro Gly Asp Pro
                10                  15                  20

gat caa gaa att aag acg gtc tcc acg att gaa aac gat ggc ttt tta 211
Asp Gln Glu Ile Lys Thr Val Ser Thr Ile Glu Asn Asp Gly Phe Leu
            25                  30                  35

atg cat caa tac aga ctt gtt ggt ccc tgg gga acg cat gta gat gca 259
Met His Gln Tyr Arg Leu Val Gly Pro Trp Gly Thr His Val Asp Ala
        40                  45                  50

cct gca cat ttc gat cca caa ggt cgg acg ctt gat cag atc cct gtg 307
Pro Ala His Phe Asp Pro Gln Gly Arg Thr Leu Asp Gln Ile Pro Val
    55                  60                  65

gag gaa acg cat tta ccc ctt tat tgc ctg agg ttt tct cgc ccc gat 355
Glu Glu Thr His Leu Pro Leu Tyr Cys Leu Arg Phe Ser Arg Pro Asp
70                  75                  80                  85

cta tgt act gct gct gat ata gaa gcg ttt gaa cat aca cac ggg aaa 403
Leu Cys Thr Ala Ala Asp Ile Glu Ala Phe Glu His Thr His Gly Lys
                90                  95                  100

atc gaa cca gga tcc ttc gtc gca ctc cac act gga tgg gaa tgg ggt 451
Ile Glu Pro Gly Ser Phe Val Ala Leu His Thr Gly Trp Glu Trp Gly
            105                 110                 115

aaa caa ggg atc gca ccc ggc tgg tct atc gag gct tta gaa atc ctc 499
Lys Gln Gly Ile Ala Pro Gly Trp Ser Ile Glu Ala Leu Glu Ile Leu
        120                 125                 130

cat gcc cgt gga gtc att gcc att ggc cac gat ctt ccc gat aca gat 547
His Ala Arg Gly Val Ile Ala Ile Gly His Asp Leu Pro Asp Thr Asp
    135                 140                 145

cct tca ctg gag gca cag cgc tgg tgg ctg tgc cgt gac cat tgg cag 595
Pro Ser Leu Glu Ala Gln Arg Trp Trp Leu Cys Arg Asp His Trp Gln
150                 155                 160                 165

att gaa aac ctc acc aat ttg gac aag gtt cct gca acg ggt gcg atg 643
Ile Glu Asn Leu Thr Asn Leu Asp Lys Val Pro Ala Thr Gly Ala Met
                170                 175                 180

att gct tgt cct tgg cca gtt cca aaa gat ggt gct agt ttc cca gtg 691
Ile Ala Cys Pro Trp Pro Val Pro Lys Asp Gly Ala Ser Phe Pro Val
            185                 190                 195

cgt cca att gct ctc gtc cca gag cac cta tcc cct act cgc 733
Arg Pro Ile Ala Leu Val Pro Glu His Leu Ser Pro Thr Arg
        200                 205                 210

taaagcgaac ggcagcacct tct 756

<210> 18
<211> 211
<212> PRT
<213> Corynebacterium glutamicum

<400> 18
Met Gln Ile Ile Asp Leu Ser His Ala Phe Ala Pro Gly Gln Pro His
1               5                   10                  15

Tyr Pro Gly Asp Pro Asp Gln Glu Ile Lys Thr Val Ser Thr Ile Glu
            20                  25                  30

Asn Asp Gly Phe Leu Met His Gln Tyr Arg Leu Val Gly Pro Trp Gly
        35                  40                  45

Thr His Val Asp Ala Pro Ala His Phe Asp Pro Gln Gly Arg Thr Leu
    50                  55                  60

Asp Gln Ile Pro Val Glu Glu Thr His Leu Pro Leu Tyr Cys Leu Arg
65                  70                  75                  80

Phe Ser Arg Pro Asp Leu Cys Thr Ala Ala Asp Ile Glu Ala Phe Glu
                85                  90                  95

His Thr His Gly Lys Ile Glu Pro Gly Ser Phe Val Ala Leu His Thr
            100                 105                 110

Gly Trp Glu Trp Gly Lys Gln Gly Ile Ala Pro Gly Trp Ser Ile Glu
        115                 120                 125

Ala Leu Glu Ile Leu His Ala Arg Gly Val Ile Ala Ile Gly His Asp
    130                 135                 140

Leu Pro Asp Thr Asp Pro Ser Leu Glu Ala Gln Arg Trp Trp Leu Cys
145                 150                 155                 160

Arg Asp His Trp Gln Ile Glu Asn Leu Thr Asn Leu Asp Lys Val Pro
                165                 170                 175

Ala Thr Gly Ala Met Ile Ala Cys Pro Trp Pro Val Pro Lys Asp Gly
            180                 185                 190

Ala Ser Phe Pro Val Arg Pro Ile Ala Leu Val Pro Glu His Leu Ser
        195                 200                 205

Pro Thr Arg
    210


<210> 19
<211> 1830
<212> DNA
<213> Corynebacterium glutamicum

<220>
<221> CDS
<222> (101)..(1807)
<223> RXN01007

<400> 19

tctgaaccaa tataccgatc agtctaaaag tgtgttaagt tctggaacat aaattagctg 60

acacgtaaag taacttaaag attcactgga ggtaagccta gtg ttt aaa aag cac 115
Val Phe Lys Lys His
1               5

aga cac ggt ctc ggc tcc ccc gaa acc aaa cca cgc tca ata acc cgc 163
Arg His Gly Leu Gly Ser Pro Glu Thr Lys Pro Arg Ser Ile Thr Arg
                10                  15                  20

cgg ttt ttc acc gcg gcc gcc gct acg ctg gca gga ttg gca gtc ctg 211
Arg Phe Phe Thr Ala Ala Ala Ala Thr Leu Ala Gly Leu Ala Val Leu
            25                  30                  35

tcc ggc tgc aca gca caa ccc tca caa gca gaa gac aac acg ctc act 259
Ser Gly Cys Thr Ala Gln Pro Ser Gln Ala Glu Asp Asn Thr Leu Thr
        40                  45                  50

tac tta gag cca cag ttc ttc cgc acc ctg tac cca cca tca gcg ggc 307
Tyr Leu Glu Pro Gln Phe Phe Arg Thr Leu Tyr Pro Pro Ser Ala Gly
    55                  60                  65

ttt tac ccc aac ggc agt gtg gtg aac aac att gca gac cgc ttg ctc 355
Phe Tyr Pro Asn Gly Ser Val Val Asn Asn Ile Ala Asp Arg Leu Leu
70                  75                  80                  85

tac cag gat cct gaa acc ttg gaa ctc aag ccg tgg atc gcc acc gaa 403
Tyr Gln Asp Pro Glu Thr Leu Glu Leu Lys Pro Trp Ile Ala Thr Glu
                90                  95                  100

ctc cca gaa gta aac gaa gac gcc acg gaa ttt acc ttc aac atc cgc 451
Leu Pro Glu Val Asn Glu Asp Ala Thr Glu Phe Thr Phe Asn Ile Arg
            105                 110                 115

acc gat gtc acc tac tcc gat ggc acc ccg ctg acg gct gaa aac gtg 499
Thr Asp Val Thr Tyr Ser Asp Gly Thr Pro Leu Thr Ala Glu Asn Val
        120                 125                 130

gtg aaa aac ttc gat ctc tat ggc ctc ggc gat caa gat cga cgc ctc 547
Val Lys Asn Phe Asp Leu Tyr Gly Leu Gly Asp Gln Asp Arg Arg Leu
    135                 140                 145

acc atc tct gag cag atc acc aac tac gac cac ggc gaa gta gta gat 595
Thr Ile Ser Glu Gln Ile Thr Asn Tyr Asp His Gly Glu Val Val Asp
150                 155                 160                 165

gag gac acc gtc cga ttc cac ttc tct gag cct gca cct ggt ttt gct 643
Glu Asp Thr Val Arg Phe His Phe Ser Glu Pro Ala Pro Gly Phe Ala
                170                 175                 180

cag gcc acc agc tcc ttc aac gct ggc ctt tat gcc gat tcc acc ttg 691
Gln Ala Thr Ser Ser Phe Asn Ala Gly Leu Tyr Ala Asp Ser Thr Leu
            185                 190                 195

gag ttc gcc aat gag gat ttc gcg cca ggc aac gcc caa aac gtc atc 739
Glu Phe Ala Asn Glu Asp Phe Ala Pro Gly Asn Ala Gln Asn Val Ile
        200                 205                 210

ggc tcc ggt cct ttc gtg atc acc gat gaa acc cta ggc acc aac ctc 787
Gly Ser Gly Pro Phe Val Ile Thr Asp Glu Thr Leu Gly Thr Asn Leu
    215                 220                 225

acc ttg act gcg cgt gag gat tac gat tgg gca cca cca tca cgc gaa 835
Thr Leu Thr Ala Arg Glu Asp Tyr Asp Trp Ala Pro Pro Ser Arg Glu
230                 235                 240                 245

cat caa ggt cgc gcg aag ctt gac gcc gtc aat tat gtc ctc gcg ggt 883
His Gln Gly Arg Ala Lys Leu Asp Ala Val Asn Tyr Val Leu Ala Gly
                250                 255                 260

gaa gaa tcc gtc cgc atc gga gcc atc gtt gct ggc caa ggt gat atc 931
Glu Glu Ser Val Arg Ile Gly Ala Ile Val Ala Gly Gln Gly Asp Ile
            265                 270                 275

gcc cgc cag atc gaa gcg cca gtg gag gca cac ctg aag gat gca ggc 979
Ala Arg Gln Ile Glu Ala Pro Val Glu Ala His Leu Lys Asp Ala Gly
        280                 285                 290

atc ccg atc atc tcc gca gcc acc aac ggt gtg aac aac agc ttc aac 1027
Ile Pro Ile Ile Ser Ala Ala Thr Asn Gly Val Asn Asn Ser Phe Asn
    295                 300                 305

ttc cgc ttc aaa aac gag ctg ctc tca gac atc cgt gtt cgc caa gct 1075
Phe Arg Phe Lys Asn Glu Leu Leu Ser Asp Ile Arg Val Arg Gln Ala
310                 315                 320                 325

ctg atc cac gcg atc gac cgc gaa aag atc atg cgt gtg ctg ttt agt 1123
Leu Ile His Ala Ile Asp Arg Glu Lys Ile Met Arg Val Leu Phe Ser
                330                 335                 340

gat tcc tat ccg ctg gca act tct gtg ctt gcg caa aat gca ctg ggc 1171
Asp Ser Tyr Pro Leu Ala Thr Ser Val Leu Ala Gln Asn Ala Leu Gly
            345                 350                 355

tac aaa gaa caa gta gat gcc tat gtc tac gac cta gac aaa gca aca 1219
Tyr Lys Glu Gln Val Asp Ala Tyr Val Tyr Asp Leu Asp Lys Ala Thr
        360                 365                 370

gct ctg ctt gac gaa gcc ggc tgg acc ctt gat agc gac ggc atg cgt 1267
Ala Leu Leu Asp Glu Ala Gly Trp Thr Leu Asp Ser Asp Gly Met Arg
    375                 380                 385

cgc aag gac ggt gaa ctt cta gag ctc acc ttc aac gaa gcc ctc cca 1315
Arg Lys Asp Gly Glu Leu Leu Glu Leu Thr Phe Asn Glu Ala Leu Pro
390                 395                 400                 405

cag cct cgt tca cgc gaa gtt gtc acc atg gtc caa gaa cag ctc ggt 1363
Gln Pro Arg Ser Arg Glu Val Val Thr Met Val Gln Glu Gln Leu Gly
                410                 415                 420

gat ctg ggc atc aag gtc aac ctc aac cca ggt gac caa gca gcc cag 1411
Asp Leu Gly Ile Lys Val Asn Leu Asn Pro Gly Asp Gln Ala Ala Gln
            425                 430                 435

gac gct gac tcc aag gat ctc aac aag atc cag gtt cgc cac acc atg 1459
Asp Ala Asp Ser Lys Asp Leu Asn Lys Ile Gln Val Arg His Thr Met
        440                 445                 450

gtg ggt cgc gca gac tat gac gtg ctg aaa tcc cag ctg tac tcc acc 1507
Val Gly Arg Ala Asp Tyr Asp Val Leu Lys Ser Gln Leu Tyr Ser Thr
    455                 460                 465

aac cgc aac gag ctg ttg aac atg acc gtg gaa ggg gag acc gcc gat 1555
Asn Arg Asn Glu Leu Leu Asn Met Thr Val Glu Gly Glu Thr Ala Asp
470                 475                 480                 485

att ggc gat cct cat ttg gag gaa ctc ctc atg gct att gca tcc agc 1603
Ile Gly Asp Pro His Leu Glu Glu Leu Leu Met Ala Ile Ala Ser Ser
                490                 495                 500

cca cgc gaa gag gac cgt gca gca gca tct gcc gca gca cag gat tac 1651
Pro Arg Glu Glu Asp Arg Ala Ala Ala Ser Ala Ala Ala Gln Asp Tyr
            505                 510                 515

atc acc gag cag gca tat gtt ctt cca ctg ttt gaa gag cca gtt gtc 1699
Ile Thr Glu Gln Ala Tyr Val Leu Pro Leu Phe Glu Glu Pro Val Val
        520                 525                 530

tac ggc gtg cag cct tac gtg aag ggc ttt agc ccc gaa gtg atc ggc 1747
Tyr Gly Val Gln Pro Tyr Val Lys Gly Phe Ser Pro Glu Val Ile Gly
    535                 540                 545

cgc ccc agc ttc tat gag acc tac att gac cat tcc agc gac cat tcc 1795
Arg Pro Ser Phe Tyr Glu Thr Tyr Ile Asp His Ser Ser Asp His Ser
550                 555                 560                 565

agt gag gag gac taaatgacta cctcgcagat tct 1830
Ser Glu Glu Asp

<210> 20
<211> 569
<212> PRT
<213> Corynebacterium glutamicum

<400> 20
Val Phe Lys Lys His Arg His Gly Leu Gly Ser Pro Glu Thr Lys Pro
1               5                   10                  15

Arg Ser Ile Thr Arg Arg Phe Phe Thr Ala Ala Ala Ala Thr Leu Ala
            20                  25                  30

Gly Leu Ala Val Leu Ser Gly Cys Thr Ala Gln Pro Ser Gln Ala Glu
        35                  40                  45

Asp Asn Thr Leu Thr Tyr Leu Glu Pro Gln Phe Phe Arg Thr Leu Tyr
    50                  55                  60

Pro Pro Ser Ala Gly Phe Tyr Pro Asn Gly Ser Val Val Asn Asn Ile
65                  70                  75                  80

Ala Asp Arg Leu Leu Tyr Gln Asp Pro Glu Thr Leu Glu Leu Lys Pro
                85                  90                  95

Trp Ile Ala Thr Glu Leu Pro Glu Val Asn Glu Asp Ala Thr Glu Phe
            100                 105                 110

Thr Phe Asn Ile Arg Thr Asp Val Thr Tyr Ser Asp Gly Thr Pro Leu
        115                 120                 125

Thr Ala Glu Asn Val Val Lys Asn Phe Asp Leu Tyr Gly Leu Gly Asp
    130                 135                 140

Gln Asp Arg Arg Leu Thr Ile Ser Glu Gln Ile Thr Asn Tyr Asp His
145                 150                 155                 160

Gly Glu Val Val Asp Glu Asp Thr Val Arg Phe His Phe Ser Glu Pro
                165                 170                 175

Ala Pro Gly Phe Ala Gln Ala Thr Ser Ser Phe Asn Ala Gly Leu Tyr
            180                 185                 190

Ala Asp Ser Thr Leu Glu Phe Ala Asn Glu Asp Phe Ala Pro Gly Asn
        195                 200                 205

Ala Gln Asn Val Ile Gly Ser Gly Pro Phe Val Ile Thr Asp Glu Thr
    210                 215                 220

Leu Gly Thr Asn Leu Thr Leu Thr Ala Arg Glu Asp Tyr Asp Trp Ala
225                 230                 235                 240

Pro Pro Ser Arg Glu His Gln Gly Arg Ala Lys Leu Asp Ala Val Asn
                245                 250                 255

Tyr Val Leu Ala Gly Glu Glu Ser Val Arg Ile Gly Ala Ile Val Ala
            260                 265                 270

Gly Gln Gly Asp Ile Ala Arg Gln Ile Glu Ala Pro Val Glu Ala His
        275                 280                 285

Leu Lys Asp Ala Gly Ile Pro Ile Ile Ser Ala Ala Thr Asn Gly Val
    290                 295                 300

Asn Asn Ser Phe Asn Phe Arg Phe Lys Asn Glu Leu Leu Ser Asp Ile
305                 310                 315                 320

Arg Val Arg Gln Ala Leu Ile His Ala Ile Asp Arg Glu Lys Ile Met
                325                 330                 335

Arg Val Leu Phe Ser Asp Ser Tyr Pro Leu Ala Thr Ser Val Leu Ala
            340                 345                 350

Gln Asn Ala Leu Gly Tyr Lys Glu Gln Val Asp Ala Tyr Val Tyr Asp
        355                 360                 365

Leu Asp Lys Ala Thr Ala Leu Leu Asp Glu Ala Gly Trp Thr Leu Asp
    370                 375                 380

Ser Asp Gly Met Arg Arg Lys Asp Gly Glu Leu Leu Glu Leu Thr Phe
385                 390                 395                 400

Asn Glu Ala Leu Pro Gln Pro Arg Ser Arg Glu Val Val Thr Met Val
                405                 410                 415

Gln Glu Gln Leu Gly Asp Leu Gly Ile Lys Val Asn Leu Asn Pro Gly
            420                 425                 430

Asp Gln Ala Ala Gln Asp Ala Asp Ser Lys Asp Leu Asn Lys Ile Gln
        435                 440                 445

Val Arg His Thr Met Val Gly Arg Ala Asp Tyr Asp Val Leu Lys Ser
    450                 455                 460

Gln Leu Tyr Ser Thr Asn Arg Asn Glu Leu Leu Asn Met Thr Val Glu
465                 470                 475                 480

Gly Glu Thr Ala Asp Ile Gly Asp Pro His Leu Glu Glu Leu Leu Met
                485                 490                 495

Ala Ile Ala Ser Ser Pro Arg Glu Glu Asp Arg Ala Ala Ala Ser Ala
            500                 505                 510

Ala Ala Gln Asp Tyr Ile Thr Glu Gln Ala Tyr Val Leu Pro Leu Phe
        515                 520                 525

Glu Glu Pro Val Val Tyr Gly Val Gln Pro Tyr Val Lys Gly Phe Ser
    530                 535                 540

Pro Glu Val Ile Gly Arg Pro Ser Phe Tyr Glu Thr Tyr Ile Asp His
545                 550                 555                 560

Ser Ser Asp His Ser Ser Glu Glu Asp
                565

<210> 21
<211> 858
<212> DNA
<213> Corynebacterium glutamicum

<220>
<221> CDS
<222> (101)..(835)
<223> RXN00784

<400> 21
gtcacatcag ttatcgcgcg gaatagttag cgggcgcatg tggttgggat atatgaataa 60

atctattcca actacgcgta aaacgaggac gtttaaagct atg tct att gaa ttc   115
                                           Met Ser Ile Glu Phe
                                           1               5

tcc gca cca gca aaa atg aaa atc gaa gtg tgg agc gac atc atg tgc   163
Ser Ala Pro Ala Lys Met Lys Ile Glu Val Trp Ser Asp Ile Met Cys
                10                  15                  20

ccc ttc tgc tac atc ggc aaa aag cgc ctc gac gac gcc cta agt acc   211
Pro Phe Cys Tyr Ile Gly Lys Lys Arg Leu Asp Asp Ala Leu Ser Thr
            25                  30                  35

ttt gac cag gcc gga cgc atc gaa gtg gaa tac aag agc ttc gaa ctc   259
Phe Asp Gln Ala Gly Arg Ile Glu Val Glu Tyr Lys Ser Phe Glu Leu
        40                  45                  50

atg cca ggc cta gaa acc cac cca ctg cgt tcc gac gtt gaa tac ctc   307
Met Pro Gly Leu Glu Thr His Pro Leu Arg Ser Asp Val Glu Tyr Leu
    55                  60                  65

gcc gac gcc aag ggc atg agc ctc gag cag gcc cgc caa atg aac ggc   355
Ala Asp Ala Lys Gly Met Ser Leu Glu Gln Ala Arg Gln Met Asn Gly
70                  75                  80                  85

caa gtc caa gca atg gca caa gcc acc gga ctt gaa atg aat cct gac   403
Gln Val Gln Ala Met Ala Gln Ala Thr Gly Leu Glu Met Asn Pro Asp

                90                  95                  100

gaa acc atc gcg gcc aac acc atc aac gcg cac cgc ctt acc cac ttc 451
Glu Thr Ile Ala Ala Asn Thr Ile Asn Ala His Arg Leu Thr His Phe
            105                 110                 115

gcg aaa gcc cac ggc aag caa caa gaa gtg gcg cag gaa ctc ttc aag 499
Ala Lys Ala His Gly Lys Gln Gln Glu Val Ala Gln Glu Leu Phe Lys
        120                 125                 130

gct cac ttc gta gac ggc aag aac gtt gat gac ctc gat gtg ctg gtc 547
Ala His Phe Val Asp Gly Lys Asn Val Asp Asp Leu Asp Val Leu Val
    135                 140                 145

tcc att gct gca gag gtt ggt ctc gat gcc agt gca gcc cgc gaa gct 595
Ser Ile Ala Ala Glu Val Gly Leu Asp Ala Ser Ala Ala Arg Glu Ala
150                 155                 160                 165

ctc gaa tcc gac gtg tac acc aac gaa gtc caa caa gac gtc cac gaa 643
Leu Glu Ser Asp Val Tyr Thr Asn Glu Val Gln Gln Asp Val His Glu
                170                 175                 180

gcc cgc caa ctc ggc gtc caa ggt gtg ccc ttc ttt gta ttc gac cgc 691
Ala Arg Gln Leu Gly Val Gln Gly Val Pro Phe Phe Val Phe Asp Arg
            185                 190                 195

aaa tac gcc atc aac ggc gcc caa caa gaa gaa gta ttc acc ggc acc 739
Lys Tyr Ala Ile Asn Gly Ala Gln Gln Glu Glu Val Phe Thr Gly Thr
        200                 205                 210

gta gaa aaa gcc ttc gaa gag tgg gca gcc gaa aac cca gtc agc cca 787
Val Glu Lys Ala Phe Glu Glu Trp Ala Ala Glu Asn Pro Val Ser Pro
    215                 220                 225

ttt gag gtc att gac ggc caa agc tgc tcc gtc gac ggc acc tgc aac 835
Phe Glu Val Ile Asp Gly Gln Ser Cys Ser Val Asp Gly Thr Cys Asn
230                 235                 240                 245

taacttttgg gacctatgtg cgt 858

<210> 22
<211> 245
<212> PRT
<213> Corynebacterium glutamicum

<400> 22
Met Ser Ile Glu Phe Ser Ala Pro Ala Lys Met Lys Ile Glu Val Trp
1               5                   10                  15

Ser Asp Ile Met Cys Pro Phe Cys Tyr Ile Gly Lys Lys Arg Leu Asp
            20                  25                  30

Asp Ala Leu Ser Thr Phe Asp Gln Ala Gly Arg Ile Glu Val Glu Tyr
        35                  40                  45

Lys Ser Phe Glu Leu Met Pro Gly Leu Glu Thr His Pro Leu Arg Ser
    50                  55                  60

Asp Val Glu Tyr Leu Ala Asp Ala Lys Gly Met Ser Leu Glu Gln Ala
65                  70                  75                  80

Arg Gln Met Asn Gly Gln Val Gln Ala Met Ala Gln Ala Thr Gly Leu
                85                  90                  95

Glu Met Asn Pro Asp Glu Thr Ile Ala Ala Asn Thr Ile Asn Ala His
            100                 105                 110

Arg Leu Thr His Phe Ala Lys Ala His Gly Lys Gln Gln Glu Val Ala
        115                 120                 125

Gln Glu Leu Phe Lys Ala His Phe Val Asp Gly Lys Asn Val Asp Asp
    130                 135                 140

Leu Asp Val Leu Val Ser Ile Ala Ala Glu Val Gly Leu Asp Ala Ser
145                 150                 155                 160

Ala Ala Arg Glu Ala Leu Glu Ser Asp Val Tyr Thr Asn Glu Val Gln
                165                 170                 175

Gln Asp Val His Glu Ala Arg Gln Leu Gly Val Gln Gly Val Pro Phe
            180                 185                 190

Phe Val Phe Asp Arg Lys Tyr Ala Ile Asn Gly Ala Gln Gln Glu Glu
        195                 200                 205

Val Phe Thr Gly Thr Val Glu Lys Ala Phe Glu Glu Trp Ala Ala Glu
    210                 215                 220

Asn Pro Val Ser Pro Phe Glu Val Ile Asp Gly Gln Ser Cys Ser Val
225                 230                 235                 240

Asp Gly Thr Cys Asn
                245

<210> 23
<211> 1896
<212> DNA
<213> Corynebacterium glutamicum

<220>
<221> CDS
<222> (101)..(1873)
<223> RXA02335

<400> 23
tggttgagtt cttcggggtt gagtgtgcaa gaatattcac tattggtcag gcaactatgt 60

gtctacccac tgagtcatca atttaaatca ggagttatta gtg tca gtc gag act   115
                                          Val Ser Val Glu Thr
                                          1               5

agg aag atc acc aag gtt ctt gtc gct aac cgt ggt gag att gca atc   163
Arg Lys Ile Thr Lys Val Leu Val Ala Asn Arg Gly Glu Ile Ala Ile
                10                  15                  20

cgc gtg ttc cgt gca gct cga gat gaa ggc atg gga tct gtc gcc gtc   211
Arg Val Phe Arg Ala Ala Arg Asp Glu Gly Met Gly Ser Val Ala Val
            25                  30                  35

tac gca gag cca gat gca gat gca cca ttc gtg tca tat gca gac gag   259

Tyr Ala Glu Pro Asp Ala Asp Ala Pro Phe Val Ser Tyr Ala Asp Glu
        40                  45                  50

gct ttt gcc ctc ggt ggc caa aca tcc gct gag tcc tac ctt gtc att     307
Ala Phe Ala Leu Gly Gly Gln Thr Ser Ala Glu Ser Tyr Leu Val Ile
    55                  60                  65

gac aag atc atc gat gcg gcc cgc aag tcc ggc gcc gac gcc atc cac     355
Asp Lys Ile Ile Asp Ala Ala Arg Lys Ser Gly Ala Asp Ala Ile His
70                  75                  80                  85

ccc ggc tac ggc ttc ctc gca gaa aac gct gac ttc gca gaa gca gtc     403
Pro Gly Tyr Gly Phe Leu Ala Glu Asn Ala Asp Phe Ala Glu Ala Val
                90                  95                  100

atc aac gaa ggc ctg atc tgg att gga cct tca cct gag tcc atc cgc     451
Ile Asn Glu Gly Leu Ile Trp Ile Gly Pro Ser Pro Glu Ser Ile Arg
            105                 110                 115

tcc ctc ggc gac aag gtc acc gct cgc cac atc gca gat acc gcc aag     499
Ser Leu Gly Asp Lys Val Thr Ala Arg His Ile Ala Asp Thr Ala Lys
        120                 125                 130

gct cca atg gct cct ggc acc aag gaa cca gta aaa gac gca gca gaa     547
Ala Pro Met Ala Pro Gly Thr Lys Glu Pro Val Lys Asp Ala Ala Glu
    135                 140                 145

gtt gtg gct ttc gct gaa gaa ttc ggt ctc cca atc gcc atc aag gca     595
Val Val Ala Phe Ala Glu Glu Phe Gly Leu Pro Ile Ala Ile Lys Ala
150                 155                 160                 165

gct ttc ggt ggc ggc gga cgt ggc atg aag gtt gcc tac aag atg gaa     643
Ala Phe Gly Gly Gly Gly Arg Gly Met Lys Val Ala Tyr Lys Met Glu
                170                 175                 180

gaa gtc gct gac ctc ttc gag tcc gca acc cgt gaa gca acc gca gcg     691
Glu Val Ala Asp Leu Phe Glu Ser Ala Thr Arg Glu Ala Thr Ala Ala
            185                 190                 195

ttc ggc cgc ggc gag tgc ttc gtg gag cgc tac ctg gac aag gca cgc     739
Phe Gly Arg Gly Glu Cys Phe Val Glu Arg Tyr Leu Asp Lys Ala Arg
        200                 205                 210

cac gtt gag gct cag gtc atc gcc gat aag cac ggc aac gtt gtt gtc     787
His Val Glu Ala Gln Val Ile Ala Asp Lys His Gly Asn Val Val Val
    215                 220                 225

gcc gga acc cgt gac tgc tcc ctg cag cgc cgt ttc cag aag ctc gtc     835
Ala Gly Thr Arg Asp Cys Ser Leu Gln Arg Arg Phe Gln Lys Leu Val
230                 235                 240                 245

gaa gaa gca cca gca cca ttc ctc acc gat gac cag cgc gag cgt ctc     883
Glu Glu Ala Pro Ala Pro Phe Leu Thr Asp Asp Gln Arg Glu Arg Leu
                250                 255                 260

cac tcc tcc gcg aag gct atc tgt aag gaa gct ggc tac tac ggt gca     931
His Ser Ser Ala Lys Ala Ile Cys Lys Glu Ala Gly Tyr Tyr Gly Ala
            265                 270                 275

ggc acc gtt gag tac ctc gtt ggc tcc gac ggc ctg atc tcc ttc ctc     979
Gly Thr Val Glu Tyr Leu Val Gly Ser Asp Gly Leu Ile Ser Phe Leu

280                 285                 290

gag gtc aac acc cgc ctc cag gtg gaa cac cca gtc acc gaa gag acc    1027
Glu Val Asn Thr Arg Leu Gln Val Glu His Pro Val Thr Glu Glu Thr
    295                 300                 305

acc ggc atc gac ctg gtc cgc gaa atg ttc cgc atc gca gaa ggc cac    1075
Thr Gly Ile Asp Leu Val Arg Glu Met Phe Arg Ile Ala Glu Gly His
310                 315                 320                 325

gag ctc tcc atc aag gaa gat cca gct cca cgc ggc cac gca ttc gag    1123
Glu Leu Ser Ile Lys Glu Asp Pro Ala Pro Arg Gly His Ala Phe Glu
                330                 335                 340

ttc cgc atc aac ggc gaa gac gct ggc tcc aac ttc atg cct gca cca    1171
Phe Arg Ile Asn Gly Glu Asp Ala Gly Ser Asn Phe Met Pro Ala Pro
            345                 350                 355

ggc aag atc acc agc tac cgc gag cca cag ggc cca ggc gtc cgc atg    1219
Gly Lys Ile Thr Ser Tyr Arg Glu Pro Gln Gly Pro Gly Val Arg Met
        360                 365                 370

gac tcc ggt gtc gtt gaa ggt tcc gaa atc tcc gga cag ttc gac tcc    1267
Asp Ser Gly Val Val Glu Gly Ser Glu Ile Ser Gly Gln Phe Asp Ser
    375                 380                 385

atg ctg gca aag ctg atc gtt tgg ggc gac acc cgc gag cag gct ctc    1315
Met Leu Ala Lys Leu Ile Val Trp Gly Asp Thr Arg Glu Gln Ala Leu
390                 395                 400                 405

cag cgc tcc cgc cgt gca ctt gca gag tac gtt gtc gag ggc atg cca    1363
Gln Arg Ser Arg Arg Ala Leu Ala Glu Tyr Val Val Glu Gly Met Pro
                410                 415                 420

acc gtt atc cca ttc cac cag cac atc gtg gaa aac cca gca ttc gtg    1411
Thr Val Ile Pro Phe His Gln His Ile Val Glu Asn Pro Ala Phe Val
            425                 430                 435

ggc aac gac gaa ggc ttc gag atc tac acc aag tgg atc gaa gag gtt    1459
Gly Asn Asp Glu Gly Phe Glu Ile Tyr Thr Lys Trp Ile Glu Glu Val
        440                 445                 450

tgg gat aac cca atc gca cct tac gtt gac gct tcc gag ctc gac gaa    1507
Trp Asp Asn Pro Ile Ala Pro Tyr Val Asp Ala Ser Glu Leu Asp Glu
    455                 460                 465

gat gag gac aag acc cca gca cag aag gtt gtt gtg gag atc aac ggc    1555
Asp Glu Asp Lys Thr Pro Ala Gln Lys Val Val Val Glu Ile Asn Gly
470                 475                 480                 485

cgt cgc gtt gag gtt gca ctc cca ggc gat ctg gca ctc ggt ggc acc    1603
Arg Arg Val Glu Val Ala Leu Pro Gly Asp Leu Ala Leu Gly Gly Thr
                490                 495                 500

gct ggt cct aag aag aag gcc aag aag cgt cgc gca ggt ggt gca aag    1651
Ala Gly Pro Lys Lys Lys Ala Lys Lys Arg Arg Ala Gly Gly Ala Lys
            505                 510                 515

gct ggc gta tcc ggc gat gca gtg gca gct cca atg cag ggc act gtc    1699
Ala Gly Val Ser Gly Asp Ala Val Ala Ala Pro Met Gln Gly Thr Val
        520                 525                 530

atc aag gtc aac gtc gaa gaa ggc gct gaa gtc aac gaa ggc gac acc     1747
Ile Lys Val Asn Val Glu Glu Gly Ala Glu Val Asn Glu Gly Asp Thr
    535                 540                 545

gtt gtt gtc ctc gag gct atg aag atg gaa aac cct gtg aag gct cat     1795
Val Val Val Leu Glu Ala Met Lys Met Glu Asn Pro Val Lys Ala His
550                 555                 560                 565

aag tcc gga acc gta acc ggc ctt act gtc gct gca ggc gag ggt gtc     1843
Lys Ser Gly Thr Val Thr Gly Leu Thr Val Ala Ala Gly Glu Gly Val
                570                 575                 580

aac aag ggc gtt gtt ctc ctc gag atc aag taagttcttg gattcctgtt     1893
Asn Lys Gly Val Val Leu Leu Glu Ile Lys
            585                 590

agt                                                                 1896


<210> 24
<211> 591
<212> PRT
<213> Corynebacterium glutamicum

<400> 24
Val Ser Val Glu Thr Arg Lys Ile Thr Lys Val Leu Val Ala Asn Arg
  1               5                  10                  15

Gly Glu Ile Ala Ile Arg Val Phe Arg Ala Ala Arg Asp Glu Gly Met
             20                  25                  30

Gly Ser Val Ala Val Tyr Ala Glu Pro Asp Ala Asp Ala Pro Phe Val
         35                  40                  45

Ser Tyr Ala Asp Glu Ala Phe Ala Leu Gly Gly Gln Thr Ser Ala Glu
     50                  55                  60

Ser Tyr Leu Val Ile Asp Lys Ile Ile Asp Ala Ala Arg Lys Ser Gly
 65                  70                  75                  80

Ala Asp Ala Ile His Pro Gly Tyr Gly Phe Leu Ala Glu Asn Ala Asp
                 85                  90                  95

Phe Ala Glu Ala Val Ile Asn Glu Gly Leu Ile Trp Ile Gly Pro Ser
            100                 105                 110

Pro Glu Ser Ile Arg Ser Leu Gly Asp Lys Val Thr Ala Arg His Ile
        115                 120                 125

Ala Asp Thr Ala Lys Ala Pro Met Ala Pro Gly Thr Lys Glu Pro Val
    130                 135                 140

Lys Asp Ala Ala Glu Val Val Ala Phe Ala Glu Glu Phe Gly Leu Pro
145                 150                 155                 160

Ile Ala Ile Lys Ala Ala Phe Gly Gly Gly Gly Arg Gly Met Lys Val
                165                 170                 175

Ala Tyr Lys Met Glu Glu Val Ala Asp Leu Phe Glu Ser Ala Thr Arg
            180                 185                 190

Glu Ala Thr Ala Ala Phe Gly Arg Gly Glu Cys Phe Val Glu Arg Tyr
        195                 200                 205

Leu Asp Lys Ala Arg His Val Glu Ala Gln Val Ile Ala Asp Lys His
    210                 215                 220

Gly Asn Val Val Val Ala Gly Thr Arg Asp Cys Ser Leu Gln Arg Arg
225                 230                 235                 240

Phe Gln Lys Leu Val Glu Glu Ala Pro Ala Pro Phe Leu Thr Asp Asp
                245                 250                 255

Gln Arg Glu Arg Leu His Ser Ser Ala Lys Ala Ile Cys Lys Glu Ala
            260                 265                 270

Gly Tyr Tyr Gly Ala Gly Thr Val Glu Tyr Leu Val Gly Ser Asp Gly
        275                 280                 285

Leu Ile Ser Phe Leu Glu Val Asn Thr Arg Leu Gln Val Glu His Pro
    290                 295                 300

Val Thr Glu Glu Thr Thr Gly Ile Asp Leu Val Arg Glu Met Phe Arg
305                 310                 315                 320

Ile Ala Glu Gly His Glu Leu Ser Ile Lys Glu Asp Pro Ala Pro Arg
                325                 330                 335

Gly His Ala Phe Glu Phe Arg Ile Asn Gly Glu Asp Ala Gly Ser Asn
            340                 345                 350

Phe Met Pro Ala Pro Gly Lys Ile Thr Ser Tyr Arg Glu Pro Gln Gly
        355                 360                 365

Pro Gly Val Arg Met Asp Ser Gly Val Val Glu Gly Ser Glu Ile Ser
    370                 375                 380

Gly Gln Phe Asp Ser Met Leu Ala Lys Leu Ile Val Trp Gly Asp Thr
385                 390                 395                 400

Arg Glu Gln Ala Leu Gln Arg Ser Arg Arg Ala Leu Ala Glu Tyr Val
                405                 410                 415

Val Glu Gly Met Pro Thr Val Ile Pro Phe His Gln His Ile Val Glu
            420                 425                 430

Asn Pro Ala Phe Val Gly Asn Asp Glu Gly Phe Glu Ile Tyr Thr Lys
        435                 440                 445

Trp Ile Glu Glu Val Trp Asp Asn Pro Ile Ala Pro Tyr Val Asp Ala
    450                 455                 460

Ser Glu Leu Asp Glu Asp Glu Asp Lys Thr Pro Ala Gln Lys Val Val
465                 470                 475                 480

Val Glu Ile Asn Gly Arg Arg Val Glu Val Ala Leu Pro Gly Asp Leu
                485                 490                 495

Ala Leu Gly Gly Thr Ala Gly Pro Lys Lys Ala Lys Lys Arg Arg
            500                 505                 510

Ala Gly Gly Ala Lys Ala Gly Val Ser Gly Asp Ala Val Ala Ala Pro
        515                 520                 525

Met Gln Gly Thr Val Ile Lys Val Asn Val Glu Glu Gly Ala Glu Val
    530                 535                 540

Asn Glu Gly Asp Thr Val Val Val Leu Glu Ala Met Lys Met Glu Asn
545                 550                 555                 560

Pro Val Lys Ala His Lys Ser Gly Thr Val Thr Gly Leu Thr Val Ala
                565                 570                 575

Ala Gly Glu Gly Val Asn Lys Gly Val Val Leu Leu Glu Ile Lys
            580                 585                 590


<210> 25
<211> 1575
<212> DNA
<213> Corynebacterium glutamicum

<220>
<221> CDS
<222> (101)..(1552)
<223> RXA02173

<400> 25
ctaaattggg cttagatctt ccgcctctaa ataggtatgc agagacattc gaattaatta 60

acaaagccat ttttcggccg tggagaagcg ttttccgact atg gtg tgg ggc atg   115
                                           Met Val Trp Gly Met
                                           1               5

gaa cac act tca gca ttg acg ctc ata gac tcg gtt ttg gac cct gac   163
Glu His Thr Ser Ala Leu Thr Leu Ile Asp Ser Val Leu Asp Pro Asp
                10                  15                  20

agc ttc att tct tgg aat gaa act ccc caa tat gac aac ctc aat caa   211
Ser Phe Ile Ser Trp Asn Glu Thr Pro Gln Tyr Asp Asn Leu Asn Gln
            25                  30                  35

ggc tat gca gag acc ttg gag cgg gct cga agc aag gcc aaa tgc gat   259
Gly Tyr Ala Glu Thr Leu Glu Arg Ala Arg Ser Lys Ala Lys Cys Asp
        40                  45                  50

gaa tcg gta att act gga gaa ggc acc gtg gag ggc att ccg gta gcc   307
Glu Ser Val Ile Thr Gly Glu Gly Thr Val Glu Gly Ile Pro Val Ala
    55                  60                  65

gtt att ttg tcc gat ttt tcc ttc ctc ggc ggt tct ttg ggc acg gtc   355
Val Ile Leu Ser Asp Phe Ser Phe Leu Gly Gly Ser Leu Gly Thr Val
70                  75                  80                  85

gcg tcg gtg cgc atc atg aag gcg att cac cgc gcc aca gag ctg aaa   403
Ala Ser Val Arg Ile Met Lys Ala Ile His Arg Ala Thr Glu Leu Lys
                90                  95                  100

ctc cca ctg ctg gtc tcc cct gct tcc ggt ggt gcg cgc atg cag gaa   451
Leu Pro Leu Leu Val Ser Pro Ala Ser Gly Gly Ala Arg Met Gln Glu
            105                 110                 115

gac aat cga gct ttt gtc atg atg gtg tcc ata acc gcg gct gtg cag 499
Asp Asn Arg Ala Phe Val Met Met Val Ser Ile Thr Ala Ala Val Gln
        120                 125                 130

cgt cac cgc gag gcg cat ttg ccg ttc ctg gtg tat ttg cgc aat ccc 547
Arg His Arg Glu Ala His Leu Pro Phe Leu Val Tyr Leu Arg Asn Pro
    135                 140                 145

acg atg ggt ggc gcc atg gcc tcg tgg ggt tca tct ggg cat ctc act 595
Thr Met Gly Gly Ala Met Ala Ser Trp Gly Ser Ser Gly His Leu Thr
150                 155                 160                 165

ttt gcg gaa ccc ggc gcg cag ata ggt ttc ctg ggt cct cgc gtg gtg 643
Phe Ala Glu Pro Gly Ala Gln Ile Gly Phe Leu Gly Pro Arg Val Val
                170                 175                 180

gag tta acc act ggg cat gcg ctt cca gac ggt gtg cag cag gcg gag 691
Glu Leu Thr Thr Gly His Ala Leu Pro Asp Gly Val Gln Gln Ala Glu
            185                 190                 195

aat ttg gtg aaa act ggt gtg att gat gga att gtg tcg cca ctc caa 739
Asn Leu Val Lys Thr Gly Val Ile Asp Gly Ile Val Ser Pro Leu Gln
        200                 205                 210

ttg cgt gca gcg gtg gca aaa acc ctc aag gtt att cag ccg gta gag 787
Leu Arg Ala Ala Val Ala Lys Thr Leu Lys Val Ile Gln Pro Val Glu
    215                 220                 225

gca acg gat cgt ttt tct cca aca act cct ggc gtg gca ctt ccg gtg 835
Ala Thr Asp Arg Phe Ser Pro Thr Thr Pro Gly Val Ala Leu Pro Val
230                 235                 240                 245

atg gag gcg att gcg cgt tct cgt gac ccg cag agg cct gga atc ggg 883
Met Glu Ala Ile Ala Arg Ser Arg Asp Pro Gln Arg Pro Gly Ile Gly
                250                 255                 260

gag att atg gaa acg ttg ggg gca gac gtc gtc aag ctt tct ggt gcg 931
Glu Ile Met Glu Thr Leu Gly Ala Asp Val Val Lys Leu Ser Gly Ala
            265                 270                 275

cgt gct ggc gca ttg agc ccg gct gtg cgc gtt gcc ctg gcg cgc atc 979
Arg Ala Gly Ala Leu Ser Pro Ala Val Arg Val Ala Leu Ala Arg Ile
        280                 285                 290

ggg ggc cgg ccc gtg gtg ctg att ggg cag gat cgc cgc ttc acg ctt 1027
Gly Gly Arg Pro Val Val Leu Ile Gly Gln Asp Arg Arg Phe Thr Leu
    295                 300                 305

ggg ccg cag gag ctg cgt ttt gcg cgt cgt ggc att tcg ctg gcg cgc 1075
Gly Pro Gln Glu Leu Arg Phe Ala Arg Arg Gly Ile Ser Leu Ala Arg
310                 315                 320                 325

gag cta aac ctg ccg atc gtg tcc atc atc gac acc tcc ggc gcc gaa 1123
Glu Leu Asn Leu Pro Ile Val Ser Ile Ile Asp Thr Ser Gly Ala Glu
                330                 335                 340

ttg tcg cag gcg gct gag gag ctc ggc atc gca agc tcg att gcg cgc 1171
Leu Ser Gln Ala Ala Glu Glu Leu Gly Ile Ala Ser Ser Ile Ala Arg
            345                 350                 355

acc ttg tcc aag ctt atc gac gct ccc ctc ccc acc gtt tcg gtc att 1219

Thr Leu Ser Lys Leu Ile Asp Ala Pro Leu Pro Thr Val Ser Val Ile
        360                 365                 370

att ggt cag ggc gtt ggc ggt ggc gcg ctg gcc atg ctg ccc gcc gat    1267
Ile Gly Gln Gly Val Gly Gly Gly Ala Leu Ala Met Leu Pro Ala Asp
    375                 380                 385

ctg gtc tac gcg gcc gaa aac gcg tgg ctg tcc gca ttg cca cca gag    1315
Leu Val Tyr Ala Ala Glu Asn Ala Trp Leu Ser Ala Leu Pro Pro Glu
390                 395                 400                 405

ggc gcc tcg gcc atc ctc ttc cgc gac acc aac cac gcc gcg gaa atc    1363
Gly Ala Ser Ala Ile Leu Phe Arg Asp Thr Asn His Ala Ala Glu Ile
                410                 415                 420

ata gag cga caa ggc gtg cag gcg cac gca ctt tta agc caa ggg ctt    1411
Ile Glu Arg Gln Gly Val Gln Ala His Ala Leu Leu Ser Gln Gly Leu
            425                 430                 435

atc gac ggg atc gtc gcc gaa acc gag cac ttt gtt gaa gaa att ctc    1459
Ile Asp Gly Ile Val Ala Glu Thr Glu His Phe Val Glu Glu Ile Leu
        440                 445                 450

ggc aca atc agc aac gcc ctc tcc gaa ttg gat aac aat ccg gag agg    1507
Gly Thr Ile Ser Asn Ala Leu Ser Glu Leu Asp Asn Asn Pro Glu Arg
    455                 460                 465

gcg gga cgc gac agt cgc ttc aca cga ttt gag cgt tta gcg cag        1552
Ala Gly Arg Asp Ser Arg Phe Thr Arg Phe Glu Arg Leu Ala Gln
470                 475                 480

taaagaaaat tatgcgctga tca                                          1575

<210> 26
<211> 484
<212> PRT
<213> Corynebacterium glutamicum

<400> 26
Met Val Trp Gly Met Glu His Thr Ser Ala Leu Thr Leu Ile Asp Ser
  1               5                  10                  15

Val Leu Asp Pro Asp Ser Phe Ile Ser Trp Asn Glu Thr Pro Gln Tyr
             20                  25                  30

Asp Asn Leu Asn Gln Gly Tyr Ala Glu Thr Leu Glu Arg Ala Arg Ser
         35                  40                  45

Lys Ala Lys Cys Asp Glu Ser Val Ile Thr Gly Glu Gly Thr Val Glu
     50                  55                  60

Gly Ile Pro Val Ala Val Ile Leu Ser Asp Phe Ser Phe Leu Gly Gly
 65                  70                  75                  80

Ser Leu Gly Thr Val Ala Ser Val Arg Ile Met Lys Ala Ile His Arg
                 85                  90                  95

Ala Thr Glu Leu Lys Leu Pro Leu Leu Val Ser Pro Ala Ser Gly Gly
            100                 105                 110

Ala Arg Met Gln Glu Asp Asn Arg Ala Phe Val Met Met Val Ser Ile
        115                 120                 125

Thr Ala Ala Val Gln Arg His Arg Glu Ala His Leu Pro Phe Leu Val
    130                 135                 140

Tyr Leu Arg Asn Pro Thr Met Gly Gly Ala Met Ala Ser Trp Gly Ser
145                 150                 155                 160

Ser Gly His Leu Thr Phe Ala Glu Pro Gly Ala Gln Ile Gly Phe Leu
                165                 170                 175

Gly Pro Arg Val Val Glu Leu Thr Thr Gly His Ala Leu Pro Asp Gly
            180                 185                 190

Val Gln Gln Ala Glu Asn Leu Val Lys Thr Gly Val Ile Asp Gly Ile
        195                 200                 205

Val Ser Pro Leu Gln Leu Arg Ala Ala Val Ala Lys Thr Leu Lys Val
    210                 215                 220

Ile Gln Pro Val Glu Ala Thr Asp Arg Phe Ser Pro Thr Thr Pro Gly
225                 230                 235                 240

Val Ala Leu Pro Val Met Glu Ala Ile Ala Arg Ser Arg Asp Pro Gln
                245                 250                 255

Arg Pro Gly Ile Gly Glu Ile Met Glu Thr Leu Gly Ala Asp Val Val
            260                 265                 270

Lys Leu Ser Gly Ala Arg Ala Gly Ala Leu Ser Pro Ala Val Arg Val
        275                 280                 285

Ala Leu Ala Arg Ile Gly Gly Arg Pro Val Val Leu Ile Gly Gln Asp
    290                 295                 300

Arg Arg Phe Thr Leu Gly Pro Gln Glu Leu Arg Phe Ala Arg Arg Gly
305                 310                 315                 320

Ile Ser Leu Ala Arg Glu Leu Asn Leu Pro Ile Val Ser Ile Ile Asp
                325                 330                 335

Thr Ser Gly Ala Glu Leu Ser Gln Ala Ala Glu Glu Leu Gly Ile Ala
            340                 345                 350

Ser Ser Ile Ala Arg Thr Leu Ser Lys Leu Ile Asp Ala Pro Leu Pro
        355                 360                 365

Thr Val Ser Val Ile Ile Gly Gln Gly Val Gly Gly Gly Ala Leu Ala
    370                 375                 380

Met Leu Pro Ala Asp Leu Val Tyr Ala Ala Glu Asn Ala Trp Leu Ser
385                 390                 395                 400

Ala Leu Pro Pro Glu Gly Ala Ser Ala Ile Leu Phe Arg Asp Thr Asn
                405                 410                 415

His Ala Ala Glu Ile Ile Glu Arg Gln Gly Val Gln Ala His Ala Leu
            420                 425                 430

Leu Ser Gln Gly Leu Ile Asp Gly Ile Val Ala Glu Thr Glu His Phe

435                 440                 445

Val Glu Glu Ile Leu Gly Thr Ile Ser Asn Ala Leu Ser Glu Leu Asp
    450                 455                 460

Asn Asn Pro Glu Arg Ala Gly Arg Asp Ser Arg Phe Thr Arg Phe Glu
465                 470                 475                 480

Arg Leu Ala Gln

<210> 27
<211> 1056
<212> DNA
<213> Corynebacterium glutamicum

<220>
<221> CDS
<222> (101)..(1033)
<223> RXA01764

<400> 27
ttttcaaaat taatagttga cattttcaac gttatgagtt ttcattggta tcacgccccg 60

acgaagtgtc tgggatcaca aaccttcaaa ggagtttgaa atg tct ctc aat gga   115
                                           Met Ser Leu Asn Gly
                                           1               5

aaa gtc gcc atc gtt acc gga tct ggt gca gga ctt ggt cgt tcc ttc   163
Lys Val Ala Ile Val Thr Gly Ser Gly Ala Gly Leu Gly Arg Ser Phe
                10                  15                  20

gct cag gag ctt gcc cgt cag ggt gca tct gtc atc gtc aat gac gta   211
Ala Gln Glu Leu Ala Arg Gln Gly Ala Ser Val Ile Val Asn Asp Val
            25                  30                  35

aac cag gca gcc gca gat gag act gtc gca gca atc acc gaa gcc ggc   259
Asn Gln Ala Ala Ala Asp Glu Thr Val Ala Ala Ile Thr Glu Ala Gly
        40                  45                  50

ggc aaa gcc gcc gcc gtt atc gcc ccc gtt gga ccc tct gaa agc gcc   307
Gly Lys Ala Ala Ala Val Ile Ala Pro Val Gly Pro Ser Glu Ser Ala
    55                  60                  65

gca ttg ctg gtg cgg gag gcc gtc gac aag ttc ggt tct ttg gac att   355
Ala Leu Leu Val Arg Glu Ala Val Asp Lys Phe Gly Ser Leu Asp Ile
70                  75                  80                  85

ctt gtc aca aac gcg ggc atc ctt cgt gat agg tcc ctg ctg aag atg   403
Leu Val Thr Asn Ala Gly Ile Leu Arg Asp Arg Ser Leu Leu Lys Met
                90                  95                  100

acg gac gat gat ttc gat gca gtc att aac gtg cac ctc aag ggc act   451
Thr Asp Asp Asp Phe Asp Ala Val Ile Asn Val His Leu Lys Gly Thr
            105                 110                 115

ttc acc tgt gtt cgc gag gca ttt gga tac ttc aag gag aat gga atc   499
Phe Thr Cys Val Arg Glu Ala Phe Gly Tyr Phe Lys Glu Asn Gly Ile
        120                 125                 130

gcg ggg cgc atc gtc acg att ggt tct ccc acc ggg cag cgc ggc aac 547
Ala Gly Arg Ile Val Thr Ile Gly Ser Pro Thr Gly Gln Arg Gly Asn
    135                 140                 145

ttc gga cag agc aat tac gct gca gct aag gcg ggc att gtg ggt atg 595
Phe Gly Gln Ser Asn Tyr Ala Ala Ala Lys Ala Gly Ile Val Gly Met
150                 155                 160                 165

gtt cgc acg tgg gcg ctg gag atg aag cgc gca ggt gtc acc att aac 643
Val Arg Thr Trp Ala Leu Glu Met Lys Arg Ala Gly Val Thr Ile Asn
                170                 175                 180

gcg atc att ccg gaa gca gcc acc gat atg acc aag acg gtg cca tat 691
Ala Ile Ile Pro Glu Ala Ala Thr Asp Met Thr Lys Thr Val Pro Tyr
            185                 190                 195

ttc cag aag gct gta gag gcc gat gag cgt ggc gag gcc atg cca gca 739
Phe Gln Lys Ala Val Glu Ala Asp Glu Arg Gly Glu Ala Met Pro Ala
        200                 205                 210

ttc ttc cgc gag acc cta ggt ttt ggc act cct cag gat gtt gcg gga 787
Phe Phe Arg Glu Thr Leu Gly Phe Gly Thr Pro Gln Asp Val Ala Gly
    215                 220                 225

ctt gtg gcc ttc ctt tcc tct gat gag gca gcg aat att tct gga cag 835
Leu Val Ala Phe Leu Ser Ser Asp Glu Ala Ala Asn Ile Ser Gly Gln
230                 235                 240                 245

gcc atc ggt gca ggc ggc gac cgc atg cag gtg tgg aag cac cca gag 883
Ala Ile Gly Ala Gly Gly Asp Arg Met Gln Val Trp Lys His Pro Glu
                250                 255                 260

cca gca gtt act gaa ttt aac cca ggt ggc tgg acc tat gaa gca ctg 931
Pro Ala Val Thr Glu Phe Asn Pro Gly Gly Trp Thr Tyr Glu Ala Leu
            265                 270                 275

cag gaa cgt ggc aag aac att att gag ggc aac ctg cag tcc gtc ggt 979
Gln Glu Arg Gly Lys Asn Ile Ile Glu Gly Asn Leu Gln Ser Val Gly
        280                 285                 290

gtc gtt ttc cct gaa ctg ccg gca gag ctt cag cca caa atc cca gtc 1027
Val Val Phe Pro Glu Leu Pro Ala Glu Leu Gln Pro Gln Ile Pro Val
    295                 300                 305

aag gca taacaccgca caccaagaat ttt 1056
Lys Ala
310

<210> 28
<211> 311
<212> PRT
<213> Corynebacterium glutamicum

<400> 28
Met Ser Leu Asn Gly Lys Val Ala Ile Val Thr Gly Ser Gly Ala Gly
1               5                   10                  15

Leu Gly Arg Ser Phe Ala Gln Glu Leu Ala Arg Gln Gly Ala Ser Val
            20                  25                  30

Ile Val Asn Asp Val Asn Gln Ala Ala Ala Asp Glu Thr Val Ala Ala
        35                  40                  45

Ile Thr Glu Ala Gly Gly Lys Ala Ala Ala Val Ile Ala Pro Val Gly
    50                  55                  60

Pro Ser Glu Ser Ala Ala Leu Leu Val Arg Glu Ala Val Asp Lys Phe
65                  70                  75                  80

Gly Ser Leu Asp Ile Leu Val Thr Asn Ala Gly Ile Leu Arg Asp Arg
                85                  90                  95

Ser Leu Leu Lys Met Thr Asp Asp Asp Phe Asp Ala Val Ile Asn Val
            100                 105                 110

His Leu Lys Gly Thr Phe Thr Cys Val Arg Glu Ala Phe Gly Tyr Phe
        115                 120                 125

Lys Glu Asn Gly Ile Ala Gly Arg Ile Val Thr Ile Gly Ser Pro Thr
    130                 135                 140

Gly Gln Arg Gly Asn Phe Gly Gln Ser Asn Tyr Ala Ala Ala Lys Ala
145                 150                 155                 160

Gly Ile Val Gly Met Val Arg Thr Trp Ala Leu Glu Met Lys Arg Ala
                165                 170                 175

Gly Val Thr Ile Asn Ala Ile Ile Pro Glu Ala Ala Thr Asp Met Thr
            180                 185                 190

Lys Thr Val Pro Tyr Phe Gln Lys Ala Val Glu Ala Asp Glu Arg Gly
        195                 200                 205

Glu Ala Met Pro Ala Phe Phe Arg Glu Thr Leu Gly Phe Gly Thr Pro
    210                 215                 220

Gln Asp Val Ala Gly Leu Val Ala Phe Leu Ser Ser Asp Glu Ala Ala
225                 230                 235                 240

Asn Ile Ser Gly Gln Ala Ile Gly Ala Gly Gly Asp Arg Met Gln Val
                245                 250                 255

Trp Lys His Pro Glu Pro Ala Val Thr Glu Phe Asn Pro Gly Gly Trp
            260                 265                 270

Thr Tyr Glu Ala Leu Gln Glu Arg Gly Lys Asn Ile Ile Glu Gly Asn
        275                 280                 285

Leu Gln Ser Val Gly Val Val Phe Pro Glu Leu Pro Ala Glu Leu Gln
    290                 295                 300

Pro Gln Ile Pro Val Lys Ala
305                 310


<210> 29
<211> 1827
<212> DNA
<213> Corynebacterium glutamicum

<220>

<221> CDS
<222> (101)..(1804)
<223> RXN02487

<400> 29
tcatagctac gcgcatgccc acattctaga tcgccgaaga aagcagcggg acgtctctat 60

atactaaagg gcactaaagc aacgcagttg aagggacacc atg tca gca tac gaa 115
Met Ser Ala Tyr Glu
1               5

acc aaa gaa tgg ctc cag cac tac cca gag tgg acg cca cac tcg ctg 163
Thr Lys Glu Trp Leu Gln His Tyr Pro Glu Trp Thr Pro His Ser Leu
                10                  15                  20

gaa tat ggc gac acc acc ctg ctg gac gtt tac gac aac aac ctg gcc 211
Glu Tyr Gly Asp Thr Thr Leu Leu Asp Val Tyr Asp Asn Asn Leu Ala
            25                  30                  35

att aac gca gac aag cca gcc acc tac ttt ttc ggt cgt tca caa acc 259
Ile Asn Ala Asp Lys Pro Ala Thr Tyr Phe Phe Gly Arg Ser Gln Thr
        40                  45                  50

tac ggt gaa ctg gac aaa gaa gtc cgc aaa act gcc gct ggc ctg cgc 307
Tyr Gly Glu Leu Asp Lys Glu Val Arg Lys Thr Ala Ala Gly Leu Arg
    55                  60                  65

gca cta ggt gtc cgc ccc ggc gat cac gta gcg att atc ctc ccc aac 355
Ala Leu Gly Val Arg Pro Gly Asp His Val Ala Ile Ile Leu Pro Asn
70                  75                  80                  85

tgc cca cag cac atc gca gct ttc tac gca gtg ctg aaa ctc ggc gca 403
Cys Pro Gln His Ile Ala Ala Phe Tyr Ala Val Leu Lys Leu Gly Ala
                90                  95                  100

gta gtc att gag cac aac ccg ctc tac acc gcc cac gaa ctg ctc gaa 451
Val Val Ile Glu His Asn Pro Leu Tyr Thr Ala His Glu Leu Leu Glu
            105                 110                 115

ccc ttc aaa gac cac ggt gcc cgc gtt gcc atc gtc tgg gac aaa gcc 499
Pro Phe Lys Asp His Gly Ala Arg Val Ala Ile Val Trp Asp Lys Ala
        120                 125                 130

tcc ccc acc gtc gaa cag cta cgt gga cag acc cag ttg gaa acc atc 547
Ser Pro Thr Val Glu Gln Leu Arg Gly Gln Thr Gln Leu Glu Thr Ile
    135                 140                 145

gtg tcg gtc aac atg atc aac gcg atg cca cca ctc cag cgc cta gca 595
Val Ser Val Asn Met Ile Asn Ala Met Pro Pro Leu Gln Arg Leu Ala
150                 155                 160                 165

ctt cgg ctc cca atc cct gca ctg cgc aag agc cgc gaa tcc ctc tcc 643
Leu Arg Leu Pro Ile Pro Ala Leu Arg Lys Ser Arg Glu Ser Leu Ser
                170                 175                 180

ggc gca gcc ccc aac acc gtt cct ttt gaa acc ctg acc agc gca gca 691
Gly Ala Ala Pro Asn Thr Val Pro Phe Glu Thr Leu Thr Ser Ala Ala
            185                 190                 195

atg ggc ggc gac ggc gac gac gta gtt tca gaa ccc acc gtg acc aaa 739
Met Gly Gly Asp Gly Asp Asp Val Val Ser Glu Pro Thr Val Thr Lys

200                 205                 210

gaa tcc gtc gcg ctg atc ctc tac acc tcc ggc acc acc gga cgc ccc     787
Glu Ser Val Ala Leu Ile Leu Tyr Thr Ser Gly Thr Thr Gly Arg Pro
    215                 220                 225

aag ggt gcc cag ctc acc cac gga aac ctg ttc ttc aat ctt ctt caa     835
Lys Gly Ala Gln Leu Thr His Gly Asn Leu Phe Phe Asn Leu Leu Gln
230                 235                 240                 245

gga aag cac tgg gtt cca ggt ctc gga gac aaa cca gaa cgc atg ctt     883
Gly Lys His Trp Val Pro Gly Leu Gly Asp Lys Pro Glu Arg Met Leu
                250                 255                 260

gca gcc cta cca atg ttc cac gca tac ggt ctg acc atg gtc ggc aca     931
Ala Ala Leu Pro Met Phe His Ala Tyr Gly Leu Thr Met Val Gly Thr
            265                 270                 275

ctg tcc gtg ttc atc ggt ggc gaa atg gtg cta ctt ccc acc cca cgc     979
Leu Ser Val Phe Ile Gly Gly Glu Met Val Leu Leu Pro Thr Pro Arg
        280                 285                 290

atc gac ctg atc atg aac gta atg aaa aag cac acc cca acc tgg cta     1027
Ile Asp Leu Ile Met Asn Val Met Lys Lys His Thr Pro Thr Trp Leu
    295                 300                 305

cca ggc gtg ccc acc ctt tac gaa aaa atc gtc gac gcc tcc gaa aaa     1075
Pro Gly Val Pro Thr Leu Tyr Glu Lys Ile Val Asp Ala Ser Glu Lys
310                 315                 320                 325

gaa gga atc ccc atc aag gga gtc cgc aac gcc ttc tcc ggt gca tcc     1123
Glu Gly Ile Pro Ile Lys Gly Val Arg Asn Ala Phe Ser Gly Ala Ser
                330                 335                 340

aca ctc tcc cag cgc acc gtt gaa cgc tgg gaa aag cac acc ggc gga     1171
Thr Leu Ser Gln Arg Thr Val Glu Arg Trp Glu Lys His Thr Gly Gly
            345                 350                 355

cgc ctc gtc gaa ggc tac ggc ctc acc gaa acc tcc ccc atc atc gtg     1219
Arg Leu Val Glu Gly Tyr Gly Leu Thr Glu Thr Ser Pro Ile Ile Val
        360                 365                 370

ggt aac ccc atg agc gat cac cga cgc caa ggc tac gta gga atc ccc     1267
Gly Asn Pro Met Ser Asp His Arg Arg Gln Gly Tyr Val Gly Ile Pro
    375                 380                 385

ttc ccc gac acc atc gtg cgc atc gca aac cca gaa aac ctc gac gaa     1315
Phe Pro Asp Thr Ile Val Arg Ile Ala Asn Pro Glu Asn Leu Asp Glu
390                 395                 400                 405

acc atg ccc gac ggc agc gaa ggc gaa gtc cta gtc aag ggc cca cag     1363
Thr Met Pro Asp Gly Ser Glu Gly Glu Val Leu Val Lys Gly Pro Gln
                410                 415                 420

gtg ttc aag ggt tac ctc aac cag gaa gaa gcc acc aag aac agc ttc     1411
Val Phe Lys Gly Tyr Leu Asn Gln Glu Glu Ala Thr Lys Asn Ser Phe
            425                 430                 435

cac ggc gag tgg tac cgc acc ggc gac gtc gga gtg atg gaa gaa gac     1459
His Gly Glu Trp Tyr Arg Thr Gly Asp Val Gly Val Met Glu Glu Asp
        440                 445                 450

ggg ttc atc cgc cta gtt gct cgc atc aag gaa gtc atc atc act ggc 1507
Gly Phe Ile Arg Leu Val Ala Arg Ile Lys Glu Val Ile Ile Thr Gly
    455                 460                 465

ggt ttc aac gtg tac cca gct gag gtt gaa gaa gtc ctc gca gag cac 1555
Gly Phe Asn Val Tyr Pro Ala Glu Val Glu Glu Val Leu Ala Glu His
470                 475                 480                 485

cca gac att gaa gat tcc gca gtc gtt ggt atc ccg cgt gaa gac ggc 1603
Pro Asp Ile Glu Asp Ser Ala Val Val Gly Ile Pro Arg Glu Asp Gly
                490                 495                 500

tcc gaa aac gtc gtt gct gcc atc act ttg gtg gaa ggt gca gcg ctg 1651
Ser Glu Asn Val Val Ala Ala Ile Thr Leu Val Glu Gly Ala Ala Leu
            505                 510                 515

gat ccg gat ggc ctg aag gaa ttc gcc cgc aag aac ctc acc cgc tac 1699
Asp Pro Asp Gly Leu Lys Glu Phe Ala Arg Lys Asn Leu Thr Arg Tyr
        520                 525                 530

aag gtt ccg cgc act ttc tac cac ttt gag gag atg ccg cgg gat cag 1747
Lys Val Pro Arg Thr Phe Tyr His Phe Glu Glu Met Pro Arg Asp Gln
    535                 540                 545

atg ggc aag att agg cgt cgt gaa gtg cag gcg gag ttg ttg aag aag 1795
Met Gly Lys Ile Arg Arg Arg Glu Val Gln Ala Glu Leu Leu Lys Lys
550                 555                 560                 565

ctc ggc aag tagacgccga tttaagaggt cga 1827
Leu Gly Lys

<210> 30
<211> 568
<212> PRT
<213> Corynebacterium glutamicum

<400> 30
Met Ser Ala Tyr Glu Thr Lys Glu Trp Leu Gln His Tyr Pro Glu Trp
1               5                   10                  15

Thr Pro His Ser Leu Glu Tyr Gly Asp Thr Thr Leu Leu Asp Val Tyr
            20                  25                  30

Asp Asn Asn Leu Ala Ile Asn Ala Asp Lys Pro Ala Thr Tyr Phe Phe
        35                  40                  45

Gly Arg Ser Gln Thr Tyr Gly Glu Leu Asp Lys Glu Val Arg Lys Thr
    50                  55                  60

Ala Ala Gly Leu Arg Ala Leu Gly Val Arg Pro Gly Asp His Val Ala
65                  70                  75                  80

Ile Ile Leu Pro Asn Cys Pro Gln His Ile Ala Ala Phe Tyr Ala Val
                85                  90                  95

Leu Lys Leu Gly Ala Val Val Ile Glu His Asn Pro Leu Tyr Thr Ala
            100                 105                 110

His Glu Leu Leu Glu Pro Phe Lys Asp His Gly Ala Arg Val Ala Ile
        115                 120                 125

Val Trp Asp Lys Ala Ser Pro Thr Val Glu Gln Leu Arg Gly Gln Thr
    130                 135                 140

Gln Leu Glu Thr Ile Val Ser Val Asn Met Ile Asn Ala Met Pro Pro
145                 150                 155                 160

Leu Gln Arg Leu Ala Leu Arg Leu Pro Ile Pro Ala Leu Arg Lys Ser
                165                 170                 175

Arg Glu Ser Leu Ser Gly Ala Ala Pro Asn Thr Val Pro Phe Glu Thr
            180                 185                 190

Leu Thr Ser Ala Ala Met Gly Gly Asp Gly Asp Asp Val Val Ser Glu
        195                 200                 205

Pro Thr Val Thr Lys Glu Ser Val Ala Leu Ile Leu Tyr Thr Ser Gly
    210                 215                 220

Thr Thr Gly Arg Pro Lys Gly Ala Gln Leu Thr His Gly Asn Leu Phe
225                 230                 235                 240

Phe Asn Leu Leu Gln Gly Lys His Trp Val Pro Gly Leu Gly Asp Lys
                245                 250                 255

Pro Glu Arg Met Leu Ala Ala Leu Pro Met Phe His Ala Tyr Gly Leu
            260                 265                 270

Thr Met Val Gly Thr Leu Ser Val Phe Ile Gly Gly Glu Met Val Leu
        275                 280                 285

Leu Pro Thr Pro Arg Ile Asp Leu Ile Met Asn Val Met Lys Lys His
    290                 295                 300

Thr Pro Thr Trp Leu Pro Gly Val Pro Thr Leu Tyr Glu Lys Ile Val
305                 310                 315                 320

Asp Ala Ser Glu Lys Glu Gly Ile Pro Ile Lys Gly Val Arg Asn Ala
                325                 330                 335

Phe Ser Gly Ala Ser Thr Leu Ser Gln Arg Thr Val Glu Arg Trp Glu
            340                 345                 350

Lys His Thr Gly Gly Arg Leu Val Glu Gly Tyr Gly Leu Thr Glu Thr
        355                 360                 365

Ser Pro Ile Ile Val Gly Asn Pro Met Ser Asp His Arg Arg Gln Gly
    370                 375                 380

Tyr Val Gly Ile Pro Phe Pro Asp Thr Ile Val Arg Ile Ala Asn Pro
385                 390                 395                 400

Glu Asn Leu Asp Glu Thr Met Pro Asp Gly Ser Glu Gly Glu Val Leu
                405                 410                 415

Val Lys Gly Pro Gln Val Phe Lys Gly Tyr Leu Asn Gln Glu Glu Ala
            420                 425                 430

Thr Lys Asn Ser Phe His Gly Glu Trp Tyr Arg Thr Gly Asp Val Gly

        435                 440                 445

Val Met Glu Glu Asp Gly Phe Ile Arg Leu Val Ala Arg Ile Lys Glu
    450                 455                 460

Val Ile Ile Thr Gly Gly Phe Asn Val Tyr Pro Ala Glu Val Glu Glu
465                 470                 475                 480

Val Leu Ala Glu His Pro Asp Ile Glu Asp Ser Ala Val Val Gly Ile
                485                 490                 495

Pro Arg Glu Asp Gly Ser Glu Asn Val Val Ala Ala Ile Thr Leu Val
            500                 505                 510

Glu Gly Ala Ala Leu Asp Pro Asp Gly Leu Lys Glu Phe Ala Arg Lys
        515                 520                 525

Asn Leu Thr Arg Tyr Lys Val Pro Arg Thr Phe Tyr His Phe Glu Glu
    530                 535                 540

Met Pro Arg Asp Gln Met Gly Lys Ile Arg Arg Arg Glu Val Gln Ala
545                 550                 555                 560

Glu Leu Leu Lys Lys Leu Gly Lys
                565


<210> 31
<211> 411
<212> DNA
<213> Corynebacterium glutamicum

<220>
<221> CDS
<222> (101)..(388)
<223> FRXA02487

<400> 31
ggcgagtggt accgcaccgc gacgtcggag tgatgaagaa gccgggttca tccgcctagt 60

tgctcgcatc aagaaagtca tcatcactgg cggtttcaac gtg tac cca gct gag   115
                                            Val Tyr Pro Ala Glu
                                            1               5

gtt gaa gaa gtc ctc gca gag cac cca gac att gaa gat tcc gca gtc   163
Val Glu Glu Val Leu Ala Glu His Pro Asp Ile Glu Asp Ser Ala Val
                10                  15                  20

gtt ggt atc ccg cgt gaa gac ggc tcc gaa aac gtc gtt gct gcc atc   211
Val Gly Ile Pro Arg Glu Asp Gly Ser Glu Asn Val Val Ala Ala Ile
            25                  30                  35

act ttg gtg gaa ggt gca gcg ctg gat ccg gat ggc ctg aag gaa ttc   259
Thr Leu Val Glu Gly Ala Ala Leu Asp Pro Asp Gly Leu Lys Glu Phe
        40                  45                  50

gcc cgc aag aac ctc acc cgc tac aag gtt ccg cgc act ttc tac cac   307
Ala Arg Lys Asn Leu Thr Arg Tyr Lys Val Pro Arg Thr Phe Tyr His
    55                  60                  65

ttt gag gag atg ccg cgg gat cag atg ggc aag att agg cgt cgt gaa   355

Phe Glu Glu Met Pro Arg Asp Gln Met Gly Lys Ile Arg Arg Arg Glu
70                  75                  80                  85

gtg cag gcg gag ttg ttg aag aag ctc ggc aag tagacgccga tttaagaggt 408
Val Gln Ala Glu Leu Leu Lys Lys Leu Gly Lys
                90                  95

cga                                                               411

<210> 32
<211> 96
<212> PRT
<213> Corynebacterium glutamicum

<400> 32
Val Tyr Pro Ala Glu Val Glu Glu Val Leu Ala Glu His Pro Asp Ile
1               5                   10                  15

Glu Asp Ser Ala Val Val Gly Ile Pro Arg Glu Asp Gly Ser Glu Asn
            20                  25                  30

Val Val Ala Ala Ile Thr Leu Val Glu Gly Ala Ala Leu Asp Pro Asp
        35                  40                  45

Gly Leu Lys Glu Phe Ala Arg Lys Asn Leu Thr Arg Tyr Lys Val Pro
    50                  55                  60

Arg Thr Phe Tyr His Phe Glu Glu Met Pro Arg Asp Gln Met Gly Lys
65                  70                  75                  80

Ile Arg Arg Arg Glu Val Gln Ala Glu Leu Leu Lys Lys Leu Gly Lys
                85                  90                  95

<210> 33
<211> 913
<212> DNA
<213> Corynebacterium glutamicum

<220>
<221> CDS
<222> (101)..(913)
<223> FRXA02490

<400> 33
tcatagctac gcgcatgccc acattctaga tcgccgaaga aagcagcggg acgtctctat 60

atactaaagg gcactaaagc aacgcagttg aagggacacc atg tca gca tac gaa   115
                                            Met Ser Ala Tyr Glu
                                            1               5

acc aaa gaa tgg ctc cag cac tac cca gag tgg acg cca cac tcg ctg   163
Thr Lys Glu Trp Leu Gln His Tyr Pro Glu Trp Thr Pro His Ser Leu
                10                  15                  20

gaa tat ggc gac acc acc ctg ctg gac gtt tac gac aac aac ctg gcc   211
Glu Tyr Gly Asp Thr Thr Leu Leu Asp Val Tyr Asp Asn Asn Leu Ala

25                  30                  35

att aac gca gac aag cca gcc acc tac ttt ttc ggt cgt tca caa acc     259
Ile Asn Ala Asp Lys Pro Ala Thr Tyr Phe Phe Gly Arg Ser Gln Thr
         40                  45                  50

tac ggt gaa ctg gac aaa gaa gtc cgc aaa act gcc gct ggc ctg cgc     307
Tyr Gly Glu Leu Asp Lys Glu Val Arg Lys Thr Ala Ala Gly Leu Arg
     55                  60                  65

gca cta ggt gtc cgc ccc ggc gat cac gta gcg att atc ctc ccc aac     355
Ala Leu Gly Val Arg Pro Gly Asp His Val Ala Ile Ile Leu Pro Asn
 70                  75                  80                  85

tgc cca cag cac atc gca gct ttc tac gca gtg ctg aaa ctc ggc gca     403
Cys Pro Gln His Ile Ala Ala Phe Tyr Ala Val Leu Lys Leu Gly Ala
                 90                  95                 100

gta gtc att gag cac aac ccg ctc tac acc gcc cac gaa ctg ctc gaa     451
Val Val Ile Glu His Asn Pro Leu Tyr Thr Ala His Glu Leu Leu Glu
            105                 110                 115

ccc ttc aaa gac cac ggt gcc cgc gtt gcc atc gtc tgg gac aaa gcc     499
Pro Phe Lys Asp His Gly Ala Arg Val Ala Ile Val Trp Asp Lys Ala
        120                 125                 130

tcc ccc acc gtc gaa cag cta cgt gga cag acc cag ttg gaa acc atc     547
Ser Pro Thr Val Glu Gln Leu Arg Gly Gln Thr Gln Leu Glu Thr Ile
    135                 140                 145

gtg tcg gtc aac atg atc aac gcg atg cca cca ctc cag cgc cta gca     595
Val Ser Val Asn Met Ile Asn Ala Met Pro Pro Leu Gln Arg Leu Ala
150                 155                 160                 165

ctt cgg ctc cca atc cct gca ctg cgc aag agc cgc gaa tcc ctc tcc     643
Leu Arg Leu Pro Ile Pro Ala Leu Arg Lys Ser Arg Glu Ser Leu Ser
                170                 175                 180

ggc gca gcc ccc aac acc gtt cct ttt gaa acc ctg acc agc gca gca     691
Gly Ala Ala Pro Asn Thr Val Pro Phe Glu Thr Leu Thr Ser Ala Ala
            185                 190                 195

atg ggc ggc gac ggc gac gac gta gtt tca gaa ccc acc gtg acc aaa     739
Met Gly Gly Asp Gly Asp Asp Val Val Ser Glu Pro Thr Val Thr Lys
        200                 205                 210

gaa tcc gtc gcg ctg atc ctc tac acc tcc ggc acc acc gga cgc ccc     787
Glu Ser Val Ala Leu Ile Leu Tyr Thr Ser Gly Thr Thr Gly Arg Pro
    215                 220                 225

aag ggt gcc cag ctc acc cac gga aac ctg ttc tcc aat ctc ctc caa     835
Lys Gly Ala Gln Leu Thr His Gly Asn Leu Phe Ser Asn Leu Leu Gln
230                 235                 240                 245

gga aag cac tgg gtt cca ggt ctc gga gac aaa cca gaa cgc atg ctt     883
Gly Lys His Trp Val Pro Gly Leu Gly Asp Lys Pro Glu Arg Met Leu
                250                 255                 260

gca gcc cta cca atg ttc cac gca tac ggt                             913
Ala Ala Leu Pro Met Phe His Ala Tyr Gly
            265                 270

<210> 34
<211> 271
<212> PRT
<213> Corynebacterium glutamicum

<400> 34
Met Ser Ala Tyr Glu Thr Lys Glu Trp Leu Gln His Tyr Pro Glu Trp
1               5                   10                  15

Thr Pro His Ser Leu Glu Tyr Gly Asp Thr Thr Leu Leu Asp Val Tyr
            20                  25                  30

Asp Asn Asn Leu Ala Ile Asn Ala Asp Lys Pro Ala Thr Tyr Phe Phe
        35                  40                  45

Gly Arg Ser Gln Thr Tyr Gly Glu Leu Asp Lys Glu Val Arg Lys Thr
    50                  55                  60

Ala Ala Gly Leu Arg Ala Leu Gly Val Arg Pro Gly Asp His Val Ala
65                  70                  75                  80

Ile Ile Leu Pro Asn Cys Pro Gln His Ile Ala Ala Phe Tyr Ala Val
                85                  90                  95

Leu Lys Leu Gly Ala Val Val Ile Glu His Asn Pro Leu Tyr Thr Ala
            100                 105                 110

His Glu Leu Leu Glu Pro Phe Lys Asp His Gly Ala Arg Val Ala Ile
        115                 120                 125

Val Trp Asp Lys Ala Ser Pro Thr Val Glu Gln Leu Arg Gly Gln Thr
    130                 135                 140

Gln Leu Glu Thr Ile Val Ser Val Asn Met Ile Asn Ala Met Pro Pro
145                 150                 155                 160

Leu Gln Arg Leu Ala Leu Arg Leu Pro Ile Pro Ala Leu Arg Lys Ser
                165                 170                 175

Arg Glu Ser Leu Ser Gly Ala Ala Pro Asn Thr Val Pro Phe Glu Thr
            180                 185                 190

Leu Thr Ser Ala Ala Met Gly Gly Asp Gly Asp Asp Val Val Ser Glu
        195                 200                 205

Pro Thr Val Thr Lys Glu Ser Val Ala Leu Ile Leu Tyr Thr Ser Gly
    210                 215                 220

Thr Thr Gly Arg Pro Lys Gly Ala Gln Leu Thr His Gly Asn Leu Phe
225                 230                 235                 240

Ser Asn Leu Leu Gln Gly Lys His Trp Val Pro Gly Leu Gly Asp Lys
                245                 250                 255

Pro Glu Arg Met Leu Ala Ala Leu Pro Met Phe His Ala Tyr Gly
            260                 265                 270

<210> 35

<211> 414
<212> DNA
<213> Corynebacterium glutamicum

<220>
<221> CDS
<222> (101)..(391)
<223> RXA01467

<400> 35
gcgtcatctt tagccttgct ttatgagctt ttccccgcaa tgagccgcgt tgtgtatcca 60

taagtcttag cctacaagcg ctttgaggta gtttgggatc atg gat ttc aac gac 115
Met Asp Phe Asn Asp
1               5

aaa gcc gct tca gaa aac gct gta aag act ggc gca gaa ggc ccc aac 163
Lys Ala Ala Ser Glu Asn Ala Val Lys Thr Gly Ala Glu Gly Pro Asn
                10                  15                  20

gtt ttc gcg agc gtg gcc aag att ttg cag gat gtt ggc gga att tca 211
Val Phe Ala Ser Val Ala Lys Ile Leu Gln Asp Val Gly Gly Ile Ser
            25                  30                  35

gcc gaa gac gtc act ccg gaa tct cgt ttt act gag gat ttg gca gtg 259
Ala Glu Asp Val Thr Pro Glu Ser Arg Phe Thr Glu Asp Leu Ala Val
        40                  45                  50

agc tca ctc aat tac atc gag ttg att gtc aat gcg gag gac gca ttt 307
Ser Ser Leu Asn Tyr Ile Glu Leu Ile Val Asn Ala Glu Asp Ala Phe
    55                  60                  65

ggt gtt cgc att gag gac gcc gat gcc aag gat ttg acc acc gtg cag 355
Gly Val Arg Ile Glu Asp Ala Asp Ala Lys Asp Leu Thr Thr Val Gln
70                  75                  80                  85

gat ttg att gac ttt att aac acc aat aag gct gat tagcgggaaa 401
Asp Leu Ile Asp Phe Ile Asn Thr Asn Lys Ala Asp
                90                  95

atttcgccca aaa 414

<210> 36
<211> 97
<212> PRT
<213> Corynebacterium glutamicum

<400> 36
Met Asp Phe Asn Asp Lys Ala Ala Ser Glu Asn Ala Val Lys Thr Gly
1               5                   10                  15

Ala Glu Gly Pro Asn Val Phe Ala Ser Val Ala Lys Ile Leu Gln Asp
            20                  25                  30

Val Gly Gly Ile Ser Ala Glu Asp Val Thr Pro Glu Ser Arg Phe Thr
        35                  40                  45

Glu Asp Leu Ala Val Ser Ser Leu Asn Tyr Ile Glu Leu Ile Val Asn
    50                  55                  60

Ala Glu Asp Ala Phe Gly Val Arg Ile Glu Asp Ala Asp Ala Lys Asp
65                  70                  75                  80

Leu Thr Thr Val Gln Asp Leu Ile Asp Phe Ile Asn Thr Asn Lys Ala
                85                  90                  95

Asp

<210> 37
<211> 298
<212> DNA
<213> Corynebacterium glutamicum

<220>
<221> CDS
<222> (101)..(298)
<223> RXA00796

<400> 37
ctggaatccc aaagcgcttg catgacgcgg aaagttaaca taggctaggt acgtcaagtt 60

gttgatacat caacttaatt aacttttaga ggagtacacc atg agc aag atc gcc   115
                                            Met Ser Lys Ile Ala
                                            1               5

atc atc acc ggt tcc acc cgt cca ggc cgc gtc aac att gac gta gcc   163
Ile Ile Thr Gly Ser Thr Arg Pro Gly Arg Val Asn Ile Asp Val Ala
                10                  15                  20

aac tgg gtt ctc gag cgc gca caa gag cgc aac gat gca cag tac gag   211
Asn Trp Val Leu Glu Arg Ala Gln Glu Arg Asn Asp Ala Gln Tyr Glu
            25                  30                  35

ctc gtt gat atc gcc gat ttc aac ttc ccc gtc ctc gac gaa gca atg   259
Leu Val Asp Ile Ala Asp Phe Asn Phe Pro Val Leu Asp Glu Ala Met
        40                  45                  50

cca gcc ggc tac ggc cag tat gca aac gag cac acc aag               298
Pro Ala Gly Tyr Gly Gln Tyr Ala Asn Glu His Thr Lys
    55                  60                  65

<210> 38
<211> 66
<212> PRT
<213> Corynebacterium glutamicum

<400> 38
Met Ser Lys Ile Ala Ile Ile Thr Gly Ser Thr Arg Pro Gly Arg Val
1               5                   10                  15

Asn Ile Asp Val Ala Asn Trp Val Leu Glu Arg Ala Gln Glu Arg Asn
            20                  25                  30

Asp Ala Gln Tyr Glu Leu Val Asp Ile Ala Asp Phe Asn Phe Pro Val
        35                  40                  45

Leu Asp Glu Ala Met Pro Ala Gly Tyr Gly Gln Tyr Ala Asn Glu His
    50                  55                  60

Thr Lys
65

<210> 39
<211> 666
<212> DNA
<213> Corynebacterium glutamicum

<220>
<221> CDS
<222> (101)..(643)
<223> RXA01897

<400> 39
ctatttagat cgttccggaa ccgatccacg cgccgatatc cactccctgg atgaactctt 60

tcactaggct ggcctttatt gtttccggaa aggggttgct atg aaa att ggt gtc 115
Met Lys Ile Gly Val
1               5

atc ctg ggc agt atc cgc gaa ggc cgc ttc ggc caa ggc gtt gcc gat 163
Ile Leu Gly Ser Ile Arg Glu Gly Arg Phe Gly Gln Gly Val Ala Asp
                10                  15                  20

tgg gtc atg gaa caa atc ggg gct tat gac gca ccc gat gtg gaa ttt 211
Trp Val Met Glu Gln Ile Gly Ala Tyr Asp Ala Pro Asp Val Glu Phe
            25                  30                  35

gaa ctc atc gac ctc aaa gct ttc aac gtg ccc ctg ttg gaa tcc gcg 259
Glu Leu Ile Asp Leu Lys Ala Phe Asn Val Pro Leu Leu Glu Ser Ala
        40                  45                  50

aca gtt cca ggt tcc gcg gat aaa cag tac gac gac ccg cgc gta act 307
Thr Val Pro Gly Ser Ala Asp Lys Gln Tyr Asp Asp Pro Arg Val Thr
    55                  60                  65

gcc tgg tca cag gcc att gat gcc tgc gat gcc ttc ctt ttc atc acc 355
Ala Trp Ser Gln Ala Ile Asp Ala Cys Asp Ala Phe Leu Phe Ile Thr
70                  75                  80                  85

ccg gaa tac aac cac ggt gtg ccc ggc gcg ttc aaa aat gcg tat gac 403
Pro Glu Tyr Asn His Gly Val Pro Gly Ala Phe Lys Asn Ala Tyr Asp
                90                  95                  100

atc ctg ggc aat gaa tgg ctg aac aaa acc gtc ggt ttc att tcc tac 451
Ile Leu Gly Asn Glu Trp Leu Asn Lys Thr Val Gly Phe Ile Ser Tyr
            105                 110                 115

ggt gca gtc gaa ggg atc cgt gtt gtc gaa cag tgg cgt cag atc gtc 499
Gly Ala Val Glu Gly Ile Arg Val Val Glu Gln Trp Arg Gln Ile Val
        120                 125                 130

gcc acc ttc aac atg tac gat atc cgc agc cag cta tcc ttt tcc acc 547
Ala Thr Phe Asn Met Tyr Asp Ile Arg Ser Gln Leu Ser Phe Ser Thr
    135                 140                 145

ttc acc gag aac aac aac ggc act ttt gcg ccc aat gat cga cgc ccc 595
Phe Thr Glu Asn Asn Asn Gly Thr Phe Ala Pro Asn Asp Arg Arg Pro
150                 155                 160                 165

ggt gaa cta atc cgc ctc ctt gat agc ctc cta acg gct gtc cgc gat 643
Gly Glu Leu Ile Arg Leu Leu Asp Ser Leu Leu Thr Ala Val Arg Asp
                170                 175                 180

taaggctctg aaatactaat gag 666

<210> 40
<211> 181
<212> PRT
<213> Corynebacterium glutamicum

<400> 40
Met Lys Ile Gly Val Ile Leu Gly Ser Ile Arg Glu Gly Arg Phe Gly
1               5                   10                  15

Gln Gly Val Ala Asp Trp Val Met Glu Gln Ile Gly Ala Tyr Asp Ala
            20                  25                  30

Pro Asp Val Glu Phe Glu Leu Ile Asp Leu Lys Ala Phe Asn Val Pro
        35                  40                  45

Leu Leu Glu Ser Ala Thr Val Pro Gly Ser Ala Asp Lys Gln Tyr Asp
    50                  55                  60

Asp Pro Arg Val Thr Ala Trp Ser Gln Ala Ile Asp Ala Cys Asp Ala
65                  70                  75                  80

Phe Leu Phe Ile Thr Pro Glu Tyr Asn His Gly Val Pro Gly Ala Phe
                85                  90                  95

Lys Asn Ala Tyr Asp Ile Leu Gly Asn Glu Trp Leu Asn Lys Thr Val
            100                 105                 110

Gly Phe Ile Ser Tyr Gly Ala Val Glu Gly Ile Arg Val Val Glu Gln
        115                 120                 125

Trp Arg Gln Ile Val Ala Thr Phe Asn Met Tyr Asp Ile Arg Ser Gln
    130                 135                 140

Leu Ser Phe Ser Thr Phe Thr Glu Asn Asn Asn Gly Thr Phe Ala Pro
145                 150                 155                 160

Asn Asp Arg Arg Pro Gly Glu Leu Ile Arg Leu Leu Asp Ser Leu Leu
                165                 170                 175

Thr Ala Val Arg Asp
            180

<210> 41
<211> 375
<212> DNA
<213> Corynebacterium glutamicum

<220>
<221> CDS
<222> (1)..(375)
<223> RXN02809

<400> 41
aac ctc tcc gtc cca gca gca cta acc aac gcc ctt tcc tac ctc tcc     48
Asn Leu Ser Val Pro Ala Ala Leu Thr Asn Ala Leu Ser Tyr Leu Ser
1               5                   10                  15

gca gag tgg aac aac aag gct gca ggc atc gtc tcc tac ggc tcc gca     96
Ala Glu Trp Asn Asn Lys Ala Ala Gly Ile Val Ser Tyr Gly Ser Ala
            20                  25                  30

atg ggc gtt cgc gca gct gag cac ctc cgc ggc atc ctt tcc gag ctt     144
Met Gly Val Arg Ala Ala Glu His Leu Arg Gly Ile Leu Ser Glu Leu
        35                  40                  45

cag atc gca cac gtt caa aag acc ggc ctg ctg agc atc ttc acc gac     192
Gln Ile Ala His Val Gln Lys Thr Gly Leu Leu Ser Ile Phe Thr Asp
    50                  55                  60

ttc gaa tac cct aac ttc aag cct tcc gag cag ggc atc tcc tct gtg     240
Phe Glu Tyr Pro Asn Phe Lys Pro Ser Glu Gln Gly Ile Ser Ser Val
65                  70                  75                  80

gac gct atg ctt gag cag ctt gtt gtc tgg acc aag gca atg tcc acc     288
Asp Ala Met Leu Glu Gln Leu Val Val Trp Thr Lys Ala Met Ser Thr
                85                  90                  95

att cgc gag tct gcg aac gtc tat cac tta aga acc cct cac aaa agt     336
Ile Arg Glu Ser Ala Asn Val Tyr His Leu Arg Thr Pro His Lys Ser
            100                 105                 110

ggc gag ctc ccc gac tgg gac tcg cct ctt ttc gta ttc                 375
Gly Glu Leu Pro Asp Trp Asp Ser Pro Leu Phe Val Phe
        115                 120                 125


<210> 42
<211> 125
<212> PRT
<213> Corynebacterium glutamicum

<400> 42
Asn Leu Ser Val Pro Ala Ala Leu Thr Asn Ala Leu Ser Tyr Leu Ser
1               5                   10                  15

Ala Glu Trp Asn Asn Lys Ala Ala Gly Ile Val Ser Tyr Gly Ser Ala
            20                  25                  30

Met Gly Val Arg Ala Ala Glu His Leu Arg Gly Ile Leu Ser Glu Leu
        35                  40                  45

Gln Ile Ala His Val Gln Lys Thr Gly Leu Leu Ser Ile Phe Thr Asp
    50                  55                  60

Phe Glu Tyr Pro Asn Phe Lys Pro Ser Glu Gln Gly Ile Ser Ser Val
65                  70                  75                  80

Asp Ala Met Leu Glu Gln Leu Val Val Trp Thr Lys Ala Met Ser Thr
                85                  90                  95

Ile Arg Glu Ser Ala Asn Val Tyr His Leu Arg Thr Pro His Lys Ser
            100                 105                 110

Gly Glu Leu Pro Asp Trp Asp Ser Pro Leu Phe Val Phe
        115                 120                 125

<210> 43
<211> 273
<212> DNA
<213> Corynebacterium glutamicum

<220>
<221> CDS
<222> (1)..(273)
<223> FRXA02809

<400> 43
gca gca cta acc aac gcc ctt tcc tac ctc tcc gca gag tgg aac aac      48
Ala Ala Leu Thr Asn Ala Leu Ser Tyr Leu Ser Ala Glu Trp Asn Asn
  1               5                  10                  15

aag gct gca ggc atc gtc tcc tac ggc tcc gca atg ggc gtt cgc gca      96
Lys Ala Ala Gly Ile Val Ser Tyr Gly Ser Ala Met Gly Val Arg Ala
             20                  25                  30

gct gag cac ctc cgc ggc atc ctt tcc gag ctt cag atc gca cac gtt     144
Ala Glu His Leu Arg Gly Ile Leu Ser Glu Leu Gln Ile Ala His Val
         35                  40                  45

caa aag acc ggc ctg ctg agc atc ttc acc gac ttc gaa tac cct aac     192
Gln Lys Thr Gly Leu Leu Ser Ile Phe Thr Asp Phe Glu Tyr Pro Asn
     50                  55                  60

ttc aag cct tcc gag cag ggc atc tcc tct gtg gac gct atg ctt gag     240
Phe Lys Pro Ser Glu Gln Gly Ile Ser Ser Val Asp Ala Met Leu Glu
 65                  70                  75                  80

cag ctt gtt gtc tgg acc aag gca atg tcc acc                         273
Gln Leu Val Val Trp Thr Lys Ala Met Ser Thr
                 85                  90

<210> 44
<211> 91
<212> PRT
<213> Corynebacterium glutamicum

<400> 44
Ala Ala Leu Thr Asn Ala Leu Ser Tyr Leu Ser Ala Glu Trp Asn Asn
  1               5                  10                  15

Lys Ala Ala Gly Ile Val Ser Tyr Gly Ser Ala Met Gly Val Arg Ala
             20                  25                  30

Ala Glu His Leu Arg Gly Ile Leu Ser Glu Leu Gln Ile Ala His Val
         35                  40                  45

Gln Lys Thr Gly Leu Leu Ser Ile Phe Thr Asp Phe Glu Tyr Pro Asn
     50                  55                  60

Phe Lys Pro Ser Glu Gln Gly Ile Ser Ser Val Asp Ala Met Leu Glu
 65                  70                  75                  80

Gln Leu Val Val Trp Thr Lys Ala Met Ser Thr
                85                  90

<210> 45
<211> 5745
<212> DNA
<213> Corynebacterium glutamicum

<220>
<221> CDS
<222> (101)..(5722)
<223> RXN00113

<400> 45
tctgcgcatc ccactcgagg agctggcaaa cggccgcacc atcgatgttg aggcacgcac 60

cgactccatc gaagagtccg catccggccg tatcgttacc gtg cgc atc gtg ctc   115
                                            Val Arg Ile Val Leu
                                            1               5

acc acc gaa ggc gaa gta gca gcc aag ctg gtt acc cgc ttc gca att   163
Thr Thr Glu Gly Glu Val Ala Ala Lys Leu Val Thr Arg Phe Ala Ile
                10                  15                  20

cgt gga cgc atc acc acc aac gaa atg gct gca cca gca gat tcc tac   211
Arg Gly Arg Ile Thr Thr Asn Glu Met Ala Ala Pro Ala Asp Ser Tyr
            25                  30                  35

ggc gca cgc gac gaa gtt gtc gag gca acc cct cgt tcc ttc atc cgc   259
Gly Ala Arg Asp Glu Val Val Glu Ala Thr Pro Arg Ser Phe Ile Arg
        40                  45                  50

cag gca act gtt tct gca cct gca gac atg acc cca ttc gcc atg gtt   307
Gln Ala Thr Val Ser Ala Pro Ala Asp Met Thr Pro Phe Ala Met Val
    55                  60                  65

tct ggt gac tac aac cca att cac acc tct gac aac gct gca aag ctt   355
Ser Gly Asp Tyr Asn Pro Ile His Thr Ser Asp Asn Ala Ala Lys Leu
70                  75                  80                  85

gtt ggc ctg gac gca gct ctt gtc cac ggc atg tgg ctg tcc gct acc   403
Val Gly Leu Asp Ala Ala Leu Val His Gly Met Trp Leu Ser Ala Thr
                90                  95                  100

gca cag cac ctc gct ggt ctt ggc tct gag gtc att ggt tgg acc tac   451
Ala Gln His Leu Ala Gly Leu Gly Ser Glu Val Ile Gly Trp Thr Tyr
            105                 110                 115

tcc atg tac ggc atg gtt caa ctc aac gac gtt gtt gac atc acc gtc   499
Ser Met Tyr Gly Met Val Gln Leu Asn Asp Val Val Asp Ile Thr Val
        120                 125                 130

gag cgt gtt ggc cgc gca ggt ctg aag cct gca tac gag gtc acc tgc   547
Glu Arg Val Gly Arg Ala Gly Leu Lys Pro Ala Tyr Glu Val Thr Cys
    135                 140                 145

cgc att gat ggc aac gtt gtc tcc cgc gga cag gca ctg ctc aag gct   595
Arg Ile Asp Gly Asn Val Val Ser Arg Gly Gln Ala Leu Leu Lys Ala
150                 155                 160                 165

cct tcc acc gct tat gtt tac cca agc cag ggc atc cag gct aag ggc 643
Pro Ser Thr Ala Tyr Val Tyr Pro Ser Gln Gly Ile Gln Ala Lys Gly
                170                 175                 180

atg ggc caa ggc gat cgc acc gca agc gca gag gct cgc gct gtg tgg 691
Met Gly Gln Gly Asp Arg Thr Ala Ser Ala Glu Ala Arg Ala Val Trp
            185                 190                 195

gag cgt gca gat gca cac acc cgc gca aac ctg ggc ttc tcc atc cag 739
Glu Arg Ala Asp Ala His Thr Arg Ala Asn Leu Gly Phe Ser Ile Gln
        200                 205                 210

cag gtc att gat gaa aac cca act gag ctg aag gtc ggg gac acc acc 787
Gln Val Ile Asp Glu Asn Pro Thr Glu Leu Lys Val Gly Asp Thr Thr
    215                 220                 225

ttc gtg cac cca gct ggg gtt ttg aac ctc acc cag ttc acg cag gtc 835
Phe Val His Pro Ala Gly Val Leu Asn Leu Thr Gln Phe Thr Gln Val
230                 235                 240                 245

gcg ctc gcc gtg gtt gcc tac gct cag acc gag cgc ctc aag gct gca 883
Ala Leu Ala Val Val Ala Tyr Ala Gln Thr Glu Arg Leu Lys Ala Ala
                250                 255                 260

aac gca att gtc gac ggc tcc ctc tac gca ggc cac tcc ctc ggc gag 931
Asn Ala Ile Val Asp Gly Ser Leu Tyr Ala Gly His Ser Leu Gly Glu
            265                 270                 275

tac acc gca ctg gca tcc ttg ggc aac atc ttc gaa ctc gaa ggc gtc 979
Tyr Thr Ala Leu Ala Ser Leu Gly Asn Ile Phe Glu Leu Glu Gly Val
        280                 285                 290

atc gac gtt gtg ttc tcc cgc ggt tcc gca atg cac tcc ctg gtt cca 1027
Ile Asp Val Val Phe Ser Arg Gly Ser Ala Met His Ser Leu Val Pro
    295                 300                 305

cgt gat gag aag ggc cgt tcc aac tac ggt ctt gcc gca ttc cgt ccg 1075
Arg Asp Glu Lys Gly Arg Ser Asn Tyr Gly Leu Ala Ala Phe Arg Pro
310                 315                 320                 325

aac atg atc aac gtt gca gcc acc gag gtg gag aac tgg gtt gac cgt 1123
Asn Met Ile Asn Val Ala Ala Thr Glu Val Glu Asn Trp Val Asp Arg
                330                 335                 340

gtc gct gaa gaa tcc ggc gaa ttc ctg cag atc gtt aac tac aac gtt 1171
Val Ala Glu Glu Ser Gly Glu Phe Leu Gln Ile Val Asn Tyr Asn Val
            345                 350                 355

gat ggc cag cag tac gca gtc gca ggt acc ttg gct ggc ctg aag gcc 1219
Asp Gly Gln Gln Tyr Ala Val Ala Gly Thr Leu Ala Gly Leu Lys Ala
        360                 365                 370

ctc aag gct tct gca tct gca aac cca cgt gct tac gtg aac att cca 1267
Leu Lys Ala Ser Ala Ser Ala Asn Pro Arg Ala Tyr Val Asn Ile Pro
    375                 380                 385

ggc att gac gtg cca ttc cac tcc agc gtc ctg cgc cca ggc gtt cct 1315
Gly Ile Asp Val Pro Phe His Ser Ser Val Leu Arg Pro Gly Val Pro
390                 395                 400                 405

gct ttc gca gag aag ctg gac gag ctg ctg cca gag acc atc gac att 1363

Ala Phe Ala Glu Lys Leu Asp Glu Leu Leu Pro Glu Thr Ile Asp Ile
                410                 415                 420

gat gct ctt cgc ggc cgc tac atc ccg aac ctg gtt gct cgt cct ttc    1411
Asp Ala Leu Arg Gly Arg Tyr Ile Pro Asn Leu Val Ala Arg Pro Phe
            425                 430                 435

gag ctc acc cag agc ttc gtg gat gcc atc ctt gct gtt gtt cca tcc    1459
Glu Leu Thr Gln Ser Phe Val Asp Ala Ile Leu Ala Val Val Pro Ser
        440                 445                 450

gag cgc ctc aag ggc atc aag gtg gag gac acc gac gag aac acc ctg    1507
Glu Arg Leu Lys Gly Ile Lys Val Glu Asp Thr Asp Glu Asn Thr Leu
    455                 460                 465

gca cgt ctg ctc ctc atc gag ctg ctg tct tgg cag ttc gca tcc cct    1555
Ala Arg Leu Leu Leu Ile Glu Leu Leu Ser Trp Gln Phe Ala Ser Pro
470                 475                 480                 485

gtg cgt tgg atc gaa acc cag gct ctg atc atc gat acc gtc gat cag    1603
Val Arg Trp Ile Glu Thr Gln Ala Leu Ile Ile Asp Thr Val Asp Gln
                490                 495                 500

atc atc gag gtc ggc ttg gca gca tcg cca acc ctg acc aac ctg gca    1651
Ile Ile Glu Val Gly Leu Ala Ala Ser Pro Thr Leu Thr Asn Leu Ala
            505                 510                 515

ctg cgc acc atg gat gtc att ggc aag tcc cgc cca gta ttc aac gtg    1699
Leu Arg Thr Met Asp Val Ile Gly Lys Ser Arg Pro Val Phe Asn Val
        520                 525                 530

gag cgc gac cag gac acc gtt atg ctc aac gat gtt cgc cag gca cct    1747
Glu Arg Asp Gln Asp Thr Val Met Leu Asn Asp Val Arg Gln Ala Pro
    535                 540                 545

gtt gct gag gtt gaa gaa gaa gca gtt gag gaa gca cct gca gca gcc    1795
Val Ala Glu Val Glu Glu Glu Ala Val Glu Glu Ala Pro Ala Ala Ala
550                 555                 560                 565

gca gct cca gca gct gag gca cca gtt gct gca gct cca gta gcc gca    1843
Ala Ala Pro Ala Ala Glu Ala Pro Val Ala Ala Ala Pro Val Ala Ala
                570                 575                 580

gcc gca cct gca cct gtt gga aac gca cct gaa ctg aag ttc aac gct    1891
Ala Ala Pro Ala Pro Val Gly Asn Ala Pro Glu Leu Lys Phe Asn Ala
            585                 590                 595

gcc aat gcc atc atg gtt ctc ttc gct gtc cag aac aag atc aac att    1939
Ala Asn Ala Ile Met Val Leu Phe Ala Val Gln Asn Lys Ile Asn Ile
        600                 605                 610

gat cag atc acc gca gcg gat acc tct gag acc ctg acc aac ggt gtg    1987
Asp Gln Ile Thr Ala Ala Asp Thr Ser Glu Thr Leu Thr Asn Gly Val
    615                 620                 625

tcc tca cgc cgt aac cag atg ctc atg gac atg tcc acc gag ctg tcc    2035
Ser Ser Arg Arg Asn Gln Met Leu Met Asp Met Ser Thr Glu Leu Ser
630                 635                 640                 645

gtc cca acc atc gac ggc gca gct gac gct gac gta gct acc ctg cag    2083
Val Pro Thr Ile Asp Gly Ala Ala Asp Ala Asp Val Ala Thr Leu Gln

                    650                 655                 660

gga cgt gtt gtc acc gca gct cct ggc tac aag cct ttc gga cca gtt     2131
Gly Arg Val Val Thr Ala Ala Pro Gly Tyr Lys Pro Phe Gly Pro Val
                665                 670                 675

ctt tcc gag acc gtt cgt gca cgt ctt cgc gca ctg acc ggt gca gca     2179
Leu Ser Glu Thr Val Arg Ala Arg Leu Arg Ala Leu Thr Gly Ala Ala
            680                 685                 690

ggt ctg aag acc tcc tac atc ggc gat cgc gtg acc ggc acc tgg gga     2227
Gly Leu Lys Thr Ser Tyr Ile Gly Asp Arg Val Thr Gly Thr Trp Gly
    695                 700                 705

ctt cca gaa agc tgg acc gca cac gtt gag gtt gaa ttg ctg ctg ggc     2275
Leu Pro Glu Ser Trp Thr Ala His Val Glu Val Glu Leu Leu Leu Gly
710                 715                 720                 725

acc cgc gaa ggc gag tcc gtc cgc ggc ggc aac ctg ggt agc ctg cct     2323
Thr Arg Glu Gly Glu Ser Val Arg Gly Gly Asn Leu Gly Ser Leu Pro
                730                 735                 740

gct aac gca tcc agc aag ggc gac gtc gat gcg ctt atc gac gca gcc     2371
Ala Asn Ala Ser Ser Lys Gly Asp Val Asp Ala Leu Ile Asp Ala Ala
            745                 750                 755

gtg cag aat gtt gct gca gcc aac ggc acc agc gtc tcc atg tcc tcc     2419
Val Gln Asn Val Ala Ala Ala Asn Gly Thr Ser Val Ser Met Ser Ser
        760                 765                 770

ggc ggt gca gct tca ggt ggc gga gtt gtt gat tcc gca gca ctt gat     2467
Gly Gly Ala Ala Ser Gly Gly Gly Val Val Asp Ser Ala Ala Leu Asp
    775                 780                 785

gcc tac gca tcc acc gtc act ggt gaa gaa ggc gtc ctg gca aac gtt     2515
Ala Tyr Ala Ser Thr Val Thr Gly Glu Glu Gly Val Leu Ala Asn Val
790                 795                 800                 805

gct cgc ggc atc ctg tct cag ctt ggt ctc gac acc aag gac gag gtt     2563
Ala Arg Gly Ile Leu Ser Gln Leu Gly Leu Asp Thr Lys Asp Glu Val
                810                 815                 820

gaa ggc gca gag atc gac acc gaa ctc tac gac gct gtc gaa gca gaa     2611
Glu Gly Ala Glu Ile Asp Thr Glu Leu Tyr Asp Ala Val Glu Ala Glu
            825                 830                 835

ctg ggc acc ggc tgg ctg aag ctt gtc acc cca gtg ttc tcc gct gat     2659
Leu Gly Thr Gly Trp Leu Lys Leu Val Thr Pro Val Phe Ser Ala Asp
        840                 845                 850

cgt gcg atc ttg ttc gac gac cgt tgg gca tct gca cgt gaa gat ctg     2707
Arg Ala Ile Leu Phe Asp Asp Arg Trp Ala Ser Ala Arg Glu Asp Leu
    855                 860                 865

gca cgc ctt gcc aac ggc gag gat att gcc gtc gag cgc ttt gct gga     2755
Ala Arg Leu Ala Asn Gly Glu Asp Ile Ala Val Glu Arg Phe Ala Gly
870                 875                 880                 885

acg ggg gag acc gtc gtc aag caa gct gca tgg tgg gct gag cac gtt     2803
Thr Gly Glu Thr Val Val Lys Gln Ala Ala Trp Trp Ala Glu His Val
                890                 895                 900

gaa gac acc gct ctc gct gca acc ctg aag cag gtt tcc gag gtg gct 2851
Glu Asp Thr Ala Leu Ala Ala Thr Leu Lys Gln Val Ser Glu Val Ala
            905                 910                 915

gcg aag cca gcc aac gag cca cac atc gac gat gtt gcg ctg gtt acc 2899
Ala Lys Pro Ala Asn Glu Pro His Ile Asp Asp Val Ala Leu Val Thr
        920                 925                 930

ggt gcg gct cct gag tcg atc gcc ggt gca gtt gcg gct cgc ctg ctg 2947
Gly Ala Ala Pro Glu Ser Ile Ala Gly Ala Val Ala Ala Arg Leu Leu
    935                 940                 945

tcc cag ggc gcg acc gtc att ctc acc gca tcg aac gtc tcc cag gcg 2995
Ser Gln Gly Ala Thr Val Ile Leu Thr Ala Ser Asn Val Ser Gln Ala
950                 955                 960                 965

cgt aag gaa tac gca cgc aag ctc tac gct gcg aac gca acc cct aac 3043
Arg Lys Glu Tyr Ala Arg Lys Leu Tyr Ala Ala Asn Ala Thr Pro Asn
                970                 975                 980

gca aag ctg tgg att gtt cct gcg aac atg tcc tcc tac cgc gat gtt 3091
Ala Lys Leu Trp Ile Val Pro Ala Asn Met Ser Ser Tyr Arg Asp Val
            985                 990                 995

gat gca gtc atc gat tgg atc ggc aac gag cag cgc gtc acc gtc ggc 3139
Asp Ala Val Ile Asp Trp Ile Gly Asn Glu Gln Arg Val Thr Val Gly
        1000                1005                1010

agc acc gtc acc gtg acc aag cca gct ctg acc cca acc ctt gcg tac 3187
Ser Thr Val Thr Val Thr Lys Pro Ala Leu Thr Pro Thr Leu Ala Tyr
    1015                1020                1025

cca ttc gca gct cca tcc gta tcc ggt acc ttg gcg gat gca ggc cca 3235
Pro Phe Ala Ala Pro Ser Val Ser Gly Thr Leu Ala Asp Ala Gly Pro
1030                1035                1040                1045

cag gct gaa aac cag gca cgc ctg ctc ctc tgg tcc gtg gag cgc acc 3283
Gln Ala Glu Asn Gln Ala Arg Leu Leu Leu Trp Ser Val Glu Arg Thr
                1050                1055                1060

atc gca gga ctt gca gat ctt gca tcc cgc ggt gtc gat gga cgc gtc 3331
Ile Ala Gly Leu Ala Asp Leu Ala Ser Arg Gly Val Asp Gly Arg Val
            1065                1070                1075

cac gtt gta ctc cca ggt tcc ccg aac cgc gga atg ttc ggt ggc gac 3379
His Val Val Leu Pro Gly Ser Pro Asn Arg Gly Met Phe Gly Gly Asp
        1080                1085                1090

ggc gct tac ggc gaa gtc aag gct gct ttc gac gcc atc ctt gcc aag 3427
Gly Ala Tyr Gly Glu Val Lys Ala Ala Phe Asp Ala Ile Leu Ala Lys
    1095                1100                1105

tgg ggc tcc gag acc ggc tgg cca cag ttt gtc tcc ctc gca cag gca 3475
Trp Gly Ser Glu Thr Gly Trp Pro Gln Phe Val Ser Leu Ala Gln Ala
1110                1115                1120                1125

cgc atc ggc tgg gtc gca ggc acc ggc ctc atg ggt cgc aac gac gtg 3523
Arg Ile Gly Trp Val Ala Gly Thr Gly Leu Met Gly Arg Asn Asp Val
                1130                1135                1140

ctc atc cct gcc gct gaa aag ctg ggc atc cac gtc tac acc cct gaa 3571
Leu Ile Pro Ala Ala Glu Lys Leu Gly Ile His Val Tyr Thr Pro Glu
            1145                1150                1155

gag atc tct tcc gaa ctg ctg ggt ctt gca tcc gca gaa tcc cgc gaa 3619
Glu Ile Ser Ser Glu Leu Leu Gly Leu Ala Ser Ala Glu Ser Arg Glu
        1160                1165                1170

aag gct ctg gaa gca ccg atc gat tac gac ctg acc ggt gga ctt tcc 3667
Lys Ala Leu Glu Ala Pro Ile Asp Tyr Asp Leu Thr Gly Gly Leu Ser
    1175                1180                1185

ggt ggc gta tcc atc gca gca ctg gca gca tcc ctc gag tcc gac gca 3715
Gly Gly Val Ser Ile Ala Ala Leu Ala Ala Ser Leu Glu Ser Asp Ala
1190                1195                1200                1205

gta gag acc acc tct gca gca gaa gac acc atc aag gcg ctt cca tca 3763
Val Glu Thr Thr Ser Ala Ala Glu Asp Thr Ile Lys Ala Leu Pro Ser
                1210                1215                1220

cct aag cac cca gag cag cca gtg ggc acg cca gtt gga gag gtc aag 3811
Pro Lys His Pro Glu Gln Pro Val Gly Thr Pro Val Gly Glu Val Lys
            1225                1230                1235

acc gat ctc gaa gac atg gtt gtc atg gtt ggc gtt ggc gaa gtc tcc 3859
Thr Asp Leu Glu Asp Met Val Val Met Val Gly Val Gly Glu Val Ser
        1240                1245                1250

tca tgg ggc tcc gga cgt acc cgc ttc gaa gct gag tac ggc atc cag 3907
Ser Trp Gly Ser Gly Arg Thr Arg Phe Glu Ala Glu Tyr Gly Ile Gln
    1255                1260                1265

cgc gac ggc tcc gtt gac ctc acc gca gca ggc gtc ctt gag ctt gca 3955
Arg Asp Gly Ser Val Asp Leu Thr Ala Ala Gly Val Leu Glu Leu Ala
1270                1275                1280                1285

tgg atg atg ggt ctg atc tcc tgg agc gaa gat cca aag cca gcc tgg 4003
Trp Met Met Gly Leu Ile Ser Trp Ser Glu Asp Pro Lys Pro Ala Trp
                1290                1295                1300

tac gac gct gac ggc acc gaa gtg cct gaa gaa gag atc tac gag cgc 4051
Tyr Asp Ala Asp Gly Thr Glu Val Pro Glu Glu Glu Ile Tyr Glu Arg
            1305                1310                1315

ttc cgc gac gaa gtc atc gca cga tgc ggt gtt cgt gag ctt gtc gac 4099
Phe Arg Asp Glu Val Ile Ala Arg Cys Gly Val Arg Glu Leu Val Asp
        1320                1325                1330

gac gca ttc ctc gtc gac ggc ggc tcc ctc gac gca gct gaa gtc ttc 4147
Asp Ala Phe Leu Val Asp Gly Gly Ser Leu Asp Ala Ala Glu Val Phe
    1335                1340                1345

ctc gac cgc gac atc tcc ttc tcc gta acc tct gct gaa gaa gca cag 4195
Leu Asp Arg Asp Ile Ser Phe Ser Val Thr Ser Ala Glu Glu Ala Gln
1350                1355                1360                1365

gcc tac gtc gat gca gat gct tcc gtg acc gtt gaa gaa gca gac ggc 4243
Ala Tyr Val Asp Ala Asp Ala Ser Val Thr Val Glu Glu Ala Asp Gly
                1370                1375                1380

gaa tgg atc gtg acc aag aag aag ggc tcc acc tcc ttc gtg cca cgc 4291

Glu Trp Ile Val Thr Lys Lys Lys Gly Ser Thr Ser Phe Val Pro Arg
        1385                1390                1395

aag gca acc ctg acc cgc tcc gta gca ggc cag ctg cca acc gac ttc    4339
Lys Ala Thr Leu Thr Arg Ser Val Ala Gly Gln Leu Pro Thr Asp Phe
    1400                1405                1410

gac cct gcc aag tgg ggt atc cca gcc tcc atg atc gat gca ctc gac    4387
Asp Pro Ala Lys Trp Gly Ile Pro Ala Ser Met Ile Asp Ala Leu Asp
    1415                1420                1425

aac atc gca gcg tgg aac ctg gtc act gca gtc gac gcc ttc ctg tcc    4435
Asn Ile Ala Ala Trp Asn Leu Val Thr Ala Val Asp Ala Phe Leu Ser
1430                1435                1440                1445

tcc ggc ttc agc cca gca gaa ctc ctg cag tcc atc cac cca gct gac    4483
Ser Gly Phe Ser Pro Ala Glu Leu Leu Gln Ser Ile His Pro Ala Asp
                1450                1455                1460

gtg tcc tcc acc cag ggc acc ggt atc ggt ggc atg cag tcc cta cgc    4531
Val Ser Ser Thr Gln Gly Thr Gly Ile Gly Gly Met Gln Ser Leu Arg
            1465                1470                1475

aag ctg ttc gtc aac cgc ttc ctc ggc cag gat cgt cca tcc gac atc    4579
Lys Leu Phe Val Asn Arg Phe Leu Gly Gln Asp Arg Pro Ser Asp Ile
        1480                1485                1490

ctc cag gag acc ctg cca aac gtt gtg gct gca cac acc atg cag tcc    4627
Leu Gln Glu Thr Leu Pro Asn Val Val Ala Ala His Thr Met Gln Ser
    1495                1500                1505

tac gtc ggt ggc tac ggc cag atg atc cac cca gtg gca gca tgt gca    4675
Tyr Val Gly Gly Tyr Gly Gln Met Ile His Pro Val Ala Ala Cys Ala
1510                1515                1520                1525

acc gca gct gtc tcc gtg gaa gaa ggc gtg gac aag atc cgc ctc aac    4723
Thr Ala Ala Val Ser Val Glu Glu Gly Val Asp Lys Ile Arg Leu Asn
                1530                1535                1540

aag gca gat ttc gtt gtc gcc ggt ggt atc gat gac atc cag gtt gaa    4771
Lys Ala Asp Phe Val Val Ala Gly Gly Ile Asp Asp Ile Gln Val Glu
            1545                1550                1555

tcc ctg acc ggc ttc ggt gac atg aac gcc acc gca gac acc cag gca    4819
Ser Leu Thr Gly Phe Gly Asp Met Asn Ala Thr Ala Asp Thr Gln Ala
        1560                1565                1570

atg ctg gac aag ggc atc gac cca cgc ttc atc tcc cgc gca aac gat    4867
Met Leu Asp Lys Gly Ile Asp Pro Arg Phe Ile Ser Arg Ala Asn Asp
    1575                1580                1585

cga cgt cgc gca ggc ttc ctc gaa gca gca ggt ggc ggt acc gtc ctc    4915
Arg Arg Arg Ala Gly Phe Leu Glu Ala Ala Gly Gly Gly Thr Val Leu
1590                1595                1600                1605

ctg gca cgt gca tcc gtt gct gct gaa ctg gga ctg cca gtt ctc gca    4963
Leu Ala Arg Ala Ser Val Ala Ala Glu Leu Gly Leu Pro Val Leu Ala
                1610                1615                1620

gtt gtt gca cac gca cag tcc tac gcc gat ggt gct cac acc tcc atc    5011
Val Val Ala His Ala Gln Ser Tyr Ala Asp Gly Ala His Thr Ser Ile

1625                1630                1635

cca gca ccg gga ctt ggc gca ctg ggt gca gca cgt ggt ggc aag aag    5059
Pro Ala Pro Gly Leu Gly Ala Leu Gly Ala Ala Arg Gly Gly Lys Lys
        1640                1645                1650

tcc gta ctt gct cgc gaa ctg aac aaa ttg ggt ctg acc cca gat gac    5107
Ser Val Leu Ala Arg Glu Leu Asn Lys Leu Gly Leu Thr Pro Asp Asp
    1655                1660                1665

gtt cgc gtg gtc tcg aag cac gac acc tcc acc aac gcc aac gat cca    5155
Val Arg Val Val Ser Lys His Asp Thr Ser Thr Asn Ala Asn Asp Pro
1670                1675                1680                1685

aac gag tcc gag ctg cac aac ctg ctg tgg aag acc att gga cgc gaa    5203
Asn Glu Ser Glu Leu His Asn Leu Leu Trp Lys Thr Ile Gly Arg Glu
                1690                1695                1700

gcc gac aac ccg atg ttc gtc gtc tcc cag aag tcc ctt acc gga cac    5251
Ala Asp Asn Pro Met Phe Val Val Ser Gln Lys Ser Leu Thr Gly His
            1705                1710                1715

tca aag ggc ggt gca gca ctc ttc cag atc ggt gga ctt gtc tcc atc    5299
Ser Lys Gly Gly Ala Ala Leu Phe Gln Ile Gly Gly Leu Val Ser Ile
        1720                1725                1730

ctg gaa acc ggc aag ctg cca cag aac gca tcc ctt gac tgc gtt gac    5347
Leu Glu Thr Gly Lys Leu Pro Gln Asn Ala Ser Leu Asp Cys Val Asp
    1735                1740                1745

cca gag atg gaa gca aag ggc gag aac ttc gtc tgg ctg cgc aag cca    5395
Pro Glu Met Glu Ala Lys Gly Glu Asn Phe Val Trp Leu Arg Lys Pro
1750                1755                1760                1765

ctg gat ctc ggc gca ggc tcc att aag gcc ggc gta ctt acc tca ctg    5443
Leu Asp Leu Gly Ala Gly Ser Ile Lys Ala Gly Val Leu Thr Ser Leu
                1770                1775                1780

ggc ttc ggc cac gtg gct gca gtc gtc gtg ctg gca acc agc ggc atc    5491
Gly Phe Gly His Val Ala Ala Val Val Val Leu Ala Thr Ser Gly Ile
            1785                1790                1795

ttc gag cag gca atg cgc aac gca ggc ctc gac gtc gaa gca tgg cgt    5539
Phe Glu Gln Ala Met Arg Asn Ala Gly Leu Asp Val Glu Ala Trp Arg
        1800                1805                1810

gca cgc gca acc cag cgc ctg cgc acc ggt gca aac cgc cta gaa gct    5587
Ala Arg Ala Thr Gln Arg Leu Arg Thr Gly Ala Asn Arg Leu Glu Ala
    1815                1820                1825

ggc atg gtt ggc cga gca cca ttg ttc gag cag gtc gac gga cgt cgc    5635
Gly Met Val Gly Arg Ala Pro Leu Phe Glu Gln Val Asp Gly Arg Arg
1830                1835                1840                1845

ctg cca gag cat ggc gct cac caa gca gag atc aac ttg ctt atc gac    5683
Leu Pro Glu His Gly Ala His Gln Ala Glu Ile Asn Leu Leu Ile Asp
                1850                1855                1860

gct gac gct cgc ctc ggt gct gac ggc atc tac cag ggc taaacgttag    5732
Ala Asp Ala Arg Leu Gly Ala Asp Gly Ile Tyr Gln Gly
            1865                1870

atagctaaga aag                                                          5745

<210> 46
<211> 1874
<212> PRT
<213> Corynebacterium glutamicum

<400> 46
Val Arg Ile Val Leu Thr Thr Glu Gly Glu Val Ala Ala Lys Leu Val
1               5                   10                  15

Thr Arg Phe Ala Ile Arg Gly Arg Ile Thr Thr Asn Glu Met Ala Ala
            20                  25                  30

Pro Ala Asp Ser Tyr Gly Ala Arg Asp Glu Val Val Glu Ala Thr Pro
        35                  40                  45

Arg Ser Phe Ile Arg Gln Ala Thr Val Ser Ala Pro Ala Asp Met Thr
    50                  55                  60

Pro Phe Ala Met Val Ser Gly Asp Tyr Asn Pro Ile His Thr Ser Asp
65                  70                  75                  80

Asn Ala Ala Lys Leu Val Gly Leu Asp Ala Ala Leu Val His Gly Met
                85                  90                  95

Trp Leu Ser Ala Thr Ala Gln His Leu Ala Gly Leu Gly Ser Glu Val
            100                 105                 110

Ile Gly Trp Thr Tyr Ser Met Tyr Gly Met Val Gln Leu Asn Asp Val
        115                 120                 125

Val Asp Ile Thr Val Glu Arg Val Gly Arg Ala Gly Leu Lys Pro Ala
    130                 135                 140

Tyr Glu Val Thr Cys Arg Ile Asp Gly Asn Val Val Ser Arg Gly Gln
145                 150                 155                 160

Ala Leu Leu Lys Ala Pro Ser Thr Ala Tyr Val Tyr Pro Ser Gln Gly
                165                 170                 175

Ile Gln Ala Lys Gly Met Gly Gln Gly Asp Arg Thr Ala Ser Ala Glu
            180                 185                 190

Ala Arg Ala Val Trp Glu Arg Ala Asp Ala His Thr Arg Ala Asn Leu
        195                 200                 205

Gly Phe Ser Ile Gln Gln Val Ile Asp Glu Asn Pro Thr Glu Leu Lys
    210                 215                 220

Val Gly Asp Thr Thr Phe Val His Pro Ala Gly Val Leu Asn Leu Thr
225                 230                 235                 240

Gln Phe Thr Gln Val Ala Leu Ala Val Val Ala Tyr Ala Gln Thr Glu
                245                 250                 255

Arg Leu Lys Ala Ala Asn Ala Ile Val Asp Gly Ser Leu Tyr Ala Gly
            260                 265                 270

His Ser Leu Gly Glu Tyr Thr Ala Leu Ala Ser Leu Gly Asn Ile Phe
        275                 280                 285

Glu Leu Glu Gly Val Ile Asp Val Val Phe Ser Arg Gly Ser Ala Met
    290                 295                 300

His Ser Leu Val Pro Arg Asp Glu Lys Gly Arg Ser Asn Tyr Gly Leu
305                 310                 315                 320

Ala Ala Phe Arg Pro Asn Met Ile Asn Val Ala Ala Thr Glu Val Glu
                325                 330                 335

Asn Trp Val Asp Arg Val Ala Glu Glu Ser Gly Glu Phe Leu Gln Ile
            340                 345                 350

Val Asn Tyr Asn Val Asp Gly Gln Gln Tyr Ala Val Ala Gly Thr Leu
        355                 360                 365

Ala Gly Leu Lys Ala Leu Lys Ala Ser Ala Ser Ala Asn Pro Arg Ala
    370                 375                 380

Tyr Val Asn Ile Pro Gly Ile Asp Val Pro Phe His Ser Ser Val Leu
385                 390                 395                 400

Arg Pro Gly Val Pro Ala Phe Ala Glu Lys Leu Asp Glu Leu Leu Pro
                405                 410                 415

Glu Thr Ile Asp Ile Asp Ala Leu Arg Gly Arg Tyr Ile Pro Asn Leu
            420                 425                 430

Val Ala Arg Pro Phe Glu Leu Thr Gln Ser Phe Val Asp Ala Ile Leu
        435                 440                 445

Ala Val Val Pro Ser Glu Arg Leu Lys Gly Ile Lys Val Glu Asp Thr
    450                 455                 460

Asp Glu Asn Thr Leu Ala Arg Leu Leu Leu Ile Glu Leu Leu Ser Trp
465                 470                 475                 480

Gln Phe Ala Ser Pro Val Arg Trp Ile Glu Thr Gln Ala Leu Ile Ile
                485                 490                 495

Asp Thr Val Asp Gln Ile Ile Glu Val Gly Leu Ala Ala Ser Pro Thr
            500                 505                 510

Leu Thr Asn Leu Ala Leu Arg Thr Met Asp Val Ile Gly Lys Ser Arg
        515                 520                 525

Pro Val Phe Asn Val Glu Arg Asp Gln Asp Thr Val Met Leu Asn Asp
    530                 535                 540

Val Arg Gln Ala Pro Val Ala Glu Val Glu Glu Glu Ala Val Glu Glu
545                 550                 555                 560

Ala Pro Ala Ala Ala Ala Ala Pro Ala Ala Glu Ala Pro Val Ala Ala
                565                 570                 575

Ala Pro Val Ala Ala Ala Ala Pro Ala Pro Val Gly Asn Ala Pro Glu
            580                 585                 590

Leu Lys Phe Asn Ala Ala Asn Ala Ile Met Val Leu Phe Ala Val Gln

595                 600                 605

Asn Lys Ile Asn Ile Asp Gln Ile Thr Ala Ala Asp Thr Ser Glu Thr
    610                 615                 620

Leu Thr Asn Gly Val Ser Ser Arg Arg Asn Gln Met Leu Met Asp Met
625                 630                 635                 640

Ser Thr Glu Leu Ser Val Pro Thr Ile Asp Gly Ala Ala Asp Ala Asp
                645                 650                 655

Val Ala Thr Leu Gln Gly Arg Val Val Thr Ala Ala Pro Gly Tyr Lys
            660                 665                 670

Pro Phe Gly Pro Val Leu Ser Glu Thr Val Arg Ala Arg Leu Arg Ala
        675                 680                 685

Leu Thr Gly Ala Ala Gly Leu Lys Thr Ser Tyr Ile Gly Asp Arg Val
    690                 695                 700

Thr Gly Thr Trp Gly Leu Pro Glu Ser Trp Thr Ala His Val Glu Val
705                 710                 715                 720

Glu Leu Leu Leu Gly Thr Arg Glu Gly Glu Ser Val Arg Gly Gly Asn
                725                 730                 735

Leu Gly Ser Leu Pro Ala Asn Ala Ser Ser Lys Gly Asp Val Asp Ala
            740                 745                 750

Leu Ile Asp Ala Ala Val Gln Asn Val Ala Ala Ala Asn Gly Thr Ser
        755                 760                 765

Val Ser Met Ser Ser Gly Gly Ala Ala Ser Gly Gly Gly Val Val Asp
    770                 775                 780

Ser Ala Ala Leu Asp Ala Tyr Ala Ser Thr Val Thr Gly Glu Glu Gly
785                 790                 795                 800

Val Leu Ala Asn Val Ala Arg Gly Ile Leu Ser Gln Leu Gly Leu Asp
                805                 810                 815

Thr Lys Asp Glu Val Glu Gly Ala Glu Ile Asp Thr Glu Leu Tyr Asp
            820                 825                 830

Ala Val Glu Ala Glu Leu Gly Thr Gly Trp Leu Lys Leu Val Thr Pro
        835                 840                 845

Val Phe Ser Ala Asp Arg Ala Ile Leu Phe Asp Asp Arg Trp Ala Ser
    850                 855                 860

Ala Arg Glu Asp Leu Ala Arg Leu Ala Asn Gly Glu Asp Ile Ala Val
865                 870                 875                 880

Glu Arg Phe Ala Gly Thr Gly Glu Thr Val Val Lys Gln Ala Ala Trp
                885                 890                 895

Trp Ala Glu His Val Glu Asp Thr Ala Leu Ala Ala Thr Leu Lys Gln
            900                 905                 910

Val Ser Glu Val Ala Ala Lys Pro Ala Asn Glu Pro His Ile Asp Asp
        915                 920                 925

Val Ala Leu Val Thr Gly Ala Ala Pro Glu Ser Ile Ala Gly Ala Val
    930                 935                 940

Ala Ala Arg Leu Leu Ser Gln Gly Ala Thr Val Ile Leu Thr Ala Ser
945                 950                 955                 960

Asn Val Ser Gln Ala Arg Lys Glu Tyr Ala Arg Lys Leu Tyr Ala Ala
                965                 970                 975

Asn Ala Thr Pro Asn Ala Lys Leu Trp Ile Val Pro Ala Asn Met Ser
            980                 985                 990

Ser Tyr Arg Asp Val Asp Ala Val Ile Asp Trp Ile Gly Asn Glu Gln
        995                 1000                1005

Arg Val Thr Val Gly Ser Thr Val Thr Val Thr Lys Pro Ala Leu Thr
    1010                1015                1020

Pro Thr Leu Ala Tyr Pro Phe Ala Ala Pro Ser Val Ser Gly Thr Leu
1025                1030                1035                1040

Ala Asp Ala Gly Pro Gln Ala Glu Asn Gln Ala Arg Leu Leu Leu Trp
                1045                1050                1055

Ser Val Glu Arg Thr Ile Ala Gly Leu Ala Asp Leu Ala Ser Arg Gly
            1060                1065                1070

Val Asp Gly Arg Val His Val Val Leu Pro Gly Ser Pro Asn Arg Gly
        1075                1080                1085

Met Phe Gly Gly Asp Gly Ala Tyr Gly Glu Val Lys Ala Ala Phe Asp
    1090                1095                1100

Ala Ile Leu Ala Lys Trp Gly Ser Glu Thr Gly Trp Pro Gln Phe Val
1105                1110                1115                1120

Ser Leu Ala Gln Ala Arg Ile Gly Trp Val Ala Gly Thr Gly Leu Met
                1125                1130                1135

Gly Arg Asn Asp Val Leu Ile Pro Ala Ala Glu Lys Leu Gly Ile His
            1140                1145                1150

Val Tyr Thr Pro Glu Glu Ile Ser Ser Glu Leu Leu Gly Leu Ala Ser
        1155                1160                1165

Ala Glu Ser Arg Glu Lys Ala Leu Glu Ala Pro Ile Asp Tyr Asp Leu
    1170                1175                1180

Thr Gly Gly Leu Ser Gly Gly Val Ser Ile Ala Ala Leu Ala Ala Ser
1185                1190                1195                1200

Leu Glu Ser Asp Ala Val Glu Thr Thr Ser Ala Ala Glu Asp Thr Ile
                1205                1210                1215

Lys Ala Leu Pro Ser Pro Lys His Pro Glu Gln Pro Val Gly Thr Pro
            1220                1225                1230

Val Gly Glu Val Lys Thr Asp Leu Glu Asp Met Val Val Met Val Gly
        1235                1240                1245

Val Gly Glu Val Ser Ser Trp Gly Ser Gly Arg Thr Arg Phe Glu Ala
    1250                1255                1260

Glu Tyr Gly Ile Gln Arg Asp Gly Ser Val Asp Leu Thr Ala Ala Gly
1265                1270                1275                1280

Val Leu Glu Leu Ala Trp Met Met Gly Leu Ile Ser Trp Ser Glu Asp
                1285                1290                1295

Pro Lys Pro Ala Trp Tyr Asp Ala Asp Gly Thr Glu Val Pro Glu Glu
            1300                1305                1310

Glu Ile Tyr Glu Arg Phe Arg Asp Glu Val Ile Ala Arg Cys Gly Val
        1315                1320                1325

Arg Glu Leu Val Asp Asp Ala Phe Leu Val Asp Gly Gly Ser Leu Asp
    1330                1335                1340

Ala Ala Glu Val Phe Leu Asp Arg Asp Ile Ser Phe Ser Val Thr Ser
1345                1350                1355                1360

Ala Glu Glu Ala Gln Ala Tyr Val Asp Ala Asp Ala Ser Val Thr Val
                1365                1370                1375

Glu Glu Ala Asp Gly Glu Trp Ile Val Thr Lys Lys Lys Gly Ser Thr
            1380                1385                1390

Ser Phe Val Pro Arg Lys Ala Thr Leu Thr Arg Ser Val Ala Gly Gln
        1395                1400                1405

Leu Pro Thr Asp Phe Asp Pro Ala Lys Trp Gly Ile Pro Ala Ser Met
    1410                1415                1420

Ile Asp Ala Leu Asp Asn Ile Ala Ala Trp Asn Leu Val Thr Ala Val
1425                1430                1435                1440

Asp Ala Phe Leu Ser Ser Gly Phe Ser Pro Ala Glu Leu Leu Gln Ser
                1445                1450                1455

Ile His Pro Ala Asp Val Ser Ser Thr Gln Gly Thr Gly Ile Gly Gly
            1460                1465                1470

Met Gln Ser Leu Arg Lys Leu Phe Val Asn Arg Phe Leu Gly Gln Asp
        1475                1480                1485

Arg Pro Ser Asp Ile Leu Gln Glu Thr Leu Pro Asn Val Val Ala Ala
    1490                1495                1500

His Thr Met Gln Ser Tyr Val Gly Gly Tyr Gly Gln Met Ile His Pro
1505                1510                1515                1520

Val Ala Ala Cys Ala Thr Ala Ala Val Ser Val Glu Glu Gly Val Asp
                1525                1530                1535

Lys Ile Arg Leu Asn Lys Ala Asp Phe Val Val Ala Gly Gly Ile Asp
            1540                1545                1550

Asp Ile Gln Val Glu Ser Leu Thr Gly Phe Gly Asp Met Asn Ala Thr
        1555                1560                1565

Ala Asp Thr Gln Ala Met Leu Asp Lys Gly Ile Asp Pro Arg Phe Ile

1570                    1575                    1580

Ser Arg Ala Asn Asp Arg Arg Arg Ala Gly Phe Leu Glu Ala Ala Gly
1585                    1590                    1595                    1600

Gly Gly Thr Val Leu Leu Ala Arg Ala Ser Val Ala Ala Glu Leu Gly
                1605                    1610                    1615

Leu Pro Val Leu Ala Val Val Ala His Ala Gln Ser Tyr Ala Asp Gly
            1620                    1625                    1630

Ala His Thr Ser Ile Pro Ala Pro Gly Leu Gly Ala Leu Gly Ala Ala
        1635                    1640                    1645

Arg Gly Gly Lys Lys Ser Val Leu Ala Arg Glu Leu Asn Lys Leu Gly
    1650                    1655                    1660

Leu Thr Pro Asp Asp Val Arg Val Val Ser Lys His Asp Thr Ser Thr
1665                    1670                    1675                    1680

Asn Ala Asn Asp Pro Asn Glu Ser Glu Leu His Asn Leu Leu Trp Lys
                1685                    1690                    1695

Thr Ile Gly Arg Glu Ala Asp Asn Pro Met Phe Val Val Ser Gln Lys
            1700                    1705                    1710

Ser Leu Thr Gly His Ser Lys Gly Gly Ala Ala Leu Phe Gln Ile Gly
        1715                    1720                    1725

Gly Leu Val Ser Ile Leu Glu Thr Gly Lys Leu Pro Gln Asn Ala Ser
    1730                    1735                    1740

Leu Asp Cys Val Asp Pro Glu Met Glu Ala Lys Gly Glu Asn Phe Val
1745                    1750                    1755                    1760

Trp Leu Arg Lys Pro Leu Asp Leu Gly Ala Gly Ser Ile Lys Ala Gly
                1765                    1770                    1775

Val Leu Thr Ser Leu Gly Phe Gly His Val Ala Ala Val Val Val Leu
            1780                    1785                    1790

Ala Thr Ser Gly Ile Phe Glu Gln Ala Met Arg Asn Ala Gly Leu Asp
        1795                    1800                    1805

Val Glu Ala Trp Arg Ala Arg Ala Thr Gln Arg Leu Arg Thr Gly Ala
    1810                    1815                    1820

Asn Arg Leu Glu Ala Gly Met Val Gly Arg Ala Pro Leu Phe Glu Gln
1825                    1830                    1835                    1840

Val Asp Gly Arg Arg Leu Pro Glu His Gly Ala His Gln Ala Glu Ile
                1845                    1850                    1855

Asn Leu Leu Ile Asp Ala Asp Ala Arg Leu Gly Ala Asp Gly Ile Tyr
            1860                    1865                    1870

Gln Gly

<210> 47

<211> 3317
<212> DNA
<213> Corynebacterium glutamicum

<220>
<221> CDS
<222> (1)..(3294)
<223> FRXA00113

<400> 47
gct tca ggt ggc gga gtt gtt gat tcc gca gca ctt gat gcc tac gca 48
Ala Ser Gly Gly Gly Val Val Asp Ser Ala Ala Leu Asp Ala Tyr Ala
1               5                   10                  15

tcc acc gtc act ggt gaa gaa ggc gtc ctg gca aac gtt gct cgc ggc 96
Ser Thr Val Thr Gly Glu Glu Gly Val Leu Ala Asn Val Ala Arg Gly
            20                  25                  30

att ctg tct cag ctt ggt ctc gac acc aag gac gag gtt gaa ggc gca 144
Ile Leu Ser Gln Leu Gly Leu Asp Thr Lys Asp Glu Val Glu Gly Ala
        35                  40                  45

gag atc gac acc gaa ctc tac gac gct gtc gaa gca gaa ctg ggc acc 192
Glu Ile Asp Thr Glu Leu Tyr Asp Ala Val Glu Ala Glu Leu Gly Thr
    50                  55                  60

ggc tgg ctg aag ctt gtc acc cca gtg ttc tcc gct gat cgt gcg atc 240
Gly Trp Leu Lys Leu Val Thr Pro Val Phe Ser Ala Asp Arg Ala Ile
65                  70                  75                  80

ttg ttc gac gac cgt tgg gca tct gca cgt gaa gat ctg gca cgc ctt 288
Leu Phe Asp Asp Arg Trp Ala Ser Ala Arg Glu Asp Leu Ala Arg Leu
                85                  90                  95

gcc aac ggc gag gat att gcc gtc gag cgc ttt gct gga acg ggg gag 336
Ala Asn Gly Glu Asp Ile Ala Val Glu Arg Phe Ala Gly Thr Gly Glu
            100                 105                 110

acc gtc gtc aag caa gct gca tgg tgg gct gag cac gtt gaa gac acc 384
Thr Val Val Lys Gln Ala Ala Trp Trp Ala Glu His Val Glu Asp Thr
        115                 120                 125

gct ctc gct gca acc ctg aag cag gtt tcc gag gtg gct gcg aag cca 432
Ala Leu Ala Ala Thr Leu Lys Gln Val Ser Glu Val Ala Ala Lys Pro
    130                 135                 140

gcc aac gag cca cac atc gac gat gtt gcg ctg gtt acc ggt gcg gct 480
Ala Asn Glu Pro His Ile Asp Asp Val Ala Leu Val Thr Gly Ala Ala
145                 150                 155                 160

cct gag tcg atc gcc ggt gca gtt gcg gct cgc ctg ctg tcc cag ggc 528
Pro Glu Ser Ile Ala Gly Ala Val Ala Ala Arg Leu Leu Ser Gln Gly
                165                 170                 175

gcg acc gtc att ctc acc gca tcg aac gtc tcc cag gcg cgt aag gaa 576
Ala Thr Val Ile Leu Thr Ala Ser Asn Val Ser Gln Ala Arg Lys Glu
            180                 185                 190

tac gca cgc aag ctc tac gct gcg aac gca acc cct aac gca aag ctg 624
Tyr Ala Arg Lys Leu Tyr Ala Ala Asn Ala Thr Pro Asn Ala Lys Leu
        195                 200                 205

tgg att gtt cct gcg aac atg tcc tcc tac cgc gat gtt gat gca gtc 672
Trp Ile Val Pro Ala Asn Met Ser Ser Tyr Arg Asp Val Asp Ala Val
    210                 215                 220

atc gat tgg atc ggc aac gag cag cgc gtc acc gtc ggc agc acc gtc 720
Ile Asp Trp Ile Gly Asn Glu Gln Arg Val Thr Val Gly Ser Thr Val
225                 230                 235                 240

acc gtg acc aag cca gct ctg acc cca acc ctt gcg tac cca ttc gca 768
Thr Val Thr Lys Pro Ala Leu Thr Pro Thr Leu Ala Tyr Pro Phe Ala
                245                 250                 255

gct cca tcc gta tcc ggt acc ttg gcg gat gca ggc cca cag gct gaa 816
Ala Pro Ser Val Ser Gly Thr Leu Ala Asp Ala Gly Pro Gln Ala Glu
            260                 265                 270

aac cag gca cgc ctg ctc ctc tgg tcc gtg gag cgc acc atc gca gga 864
Asn Gln Ala Arg Leu Leu Leu Trp Ser Val Glu Arg Thr Ile Ala Gly
        275                 280                 285

ctt gca gat ctt gca tcc cgc ggt gtc gat gga cgc gtc cac gtt gta 912
Leu Ala Asp Leu Ala Ser Arg Gly Val Asp Gly Arg Val His Val Val
    290                 295                 300

ctc cca ggt tcc ccg aac cgc gga atg ttc ggt ggc gac ggc gct tac 960
Leu Pro Gly Ser Pro Asn Arg Gly Met Phe Gly Gly Asp Gly Ala Tyr
305                 310                 315                 320

ggc gaa gtc aag gct gct ttc gac gcc atc ctt gcc aag tgg ggc tcc 1008
Gly Glu Val Lys Ala Ala Phe Asp Ala Ile Leu Ala Lys Trp Gly Ser
                325                 330                 335

gag acc ggc tgg cca cag ttt gtc tcc ctc gca cag gca cgc atc ggc 1056
Glu Thr Gly Trp Pro Gln Phe Val Ser Leu Ala Gln Ala Arg Ile Gly
            340                 345                 350

tgg gtc gca ggc acc ggc ctc atg ggt cgc aac gac gtg ctc atc cct 1104
Trp Val Ala Gly Thr Gly Leu Met Gly Arg Asn Asp Val Leu Ile Pro
        355                 360                 365

gcc gct gaa aag ctg ggc atc cac gtc tac acc cct gaa gag atc tct 1152
Ala Ala Glu Lys Leu Gly Ile His Val Tyr Thr Pro Glu Glu Ile Ser
    370                 375                 380

tcc gaa ctg ctg ggt ctt gca tcc gca gaa tcc cgc gaa aag gct ctg 1200
Ser Glu Leu Leu Gly Leu Ala Ser Ala Glu Ser Arg Glu Lys Ala Leu
385                 390                 395                 400

gaa gca ccg atc gat tac gac ctg acc ggt gga ctt tcc ggt ggc gta 1248
Glu Ala Pro Ile Asp Tyr Asp Leu Thr Gly Gly Leu Ser Gly Gly Val
                405                 410                 415

tcc atc gca gca ctg gca gca tcc ctc gag tcc gac gca gta gag acc 1296
Ser Ile Ala Ala Leu Ala Ala Ser Leu Glu Ser Asp Ala Val Glu Thr
            420                 425                 430

acc tct gca gca gaa gac acc atc aag gcg ctt cca tca cct aag cac 1344
Thr Ser Ala Ala Glu Asp Thr Ile Lys Ala Leu Pro Ser Pro Lys His
        435                 440                 445

cca gag cag cca gtg ggc acg cca gtt gga gag gtc aag acc gat ctc 1392
Pro Glu Gln Pro Val Gly Thr Pro Val Gly Glu Val Lys Thr Asp Leu
    450                 455                 460

gaa gac atg gtt gtc atg gtt ggc gtt ggc gaa gtc tcc tca tgg ggc 1440
Glu Asp Met Val Val Met Val Gly Val Gly Glu Val Ser Ser Trp Gly
465                 470                 475                 480

tcc gga cgt acc cgc ttc gaa gct gag tac ggc atc cag cgc gac ggc 1488
Ser Gly Arg Thr Arg Phe Glu Ala Glu Tyr Gly Ile Gln Arg Asp Gly
                485                 490                 495

tcc gtt gac ctc acc gca gca ggc gtc ctt gag ctt gca tgg atg atg 1536
Ser Val Asp Leu Thr Ala Ala Gly Val Leu Glu Leu Ala Trp Met Met
            500                 505                 510

ggt ctg atc tcc tgg agc gaa gat cca aag cca gcc tgg tac gac gct 1584
Gly Leu Ile Ser Trp Ser Glu Asp Pro Lys Pro Ala Trp Tyr Asp Ala
        515                 520                 525

gac ggc acc gaa gtg cct gaa gaa gag atc tac gag cgc ttc cgc gac 1632
Asp Gly Thr Glu Val Pro Glu Glu Glu Ile Tyr Glu Arg Phe Arg Asp
    530                 535                 540

gaa gtc atc gca cga tgc ggt gtt cgt gag ctt gtc gac gac gca ttc 1680
Glu Val Ile Ala Arg Cys Gly Val Arg Glu Leu Val Asp Asp Ala Phe
545                 550                 555                 560

ctc gtc gac ggc ggc tcc ctc gac gca gct gaa gtc ttc ctc gac cgc 1728
Leu Val Asp Gly Gly Ser Leu Asp Ala Ala Glu Val Phe Leu Asp Arg
                565                 570                 575

gac atc tcc ttc tcc gta acc tct gct gaa gaa gca cag gcc tac gtc 1776
Asp Ile Ser Phe Ser Val Thr Ser Ala Glu Glu Ala Gln Ala Tyr Val
            580                 585                 590

gat gca gat gct tcc gtg acc gtt gaa gaa gca gac ggc gaa tgg atc 1824
Asp Ala Asp Ala Ser Val Thr Val Glu Glu Ala Asp Gly Glu Trp Ile
        595                 600                 605

gtg acc aag aag aag ggc tcc acc tcc ttc gtg cca cgc aag gca acc 1872
Val Thr Lys Lys Lys Gly Ser Thr Ser Phe Val Pro Arg Lys Ala Thr
    610                 615                 620

ctg acc cgc tcc gta gca ggc cag ctg cca acc gac ttc gac cct gcc 1920
Leu Thr Arg Ser Val Ala Gly Gln Leu Pro Thr Asp Phe Asp Pro Ala
625                 630                 635                 640

aag tgg ggt atc cca gcc tcc atg atc gat gca ctc gac aac atc gca 1968
Lys Trp Gly Ile Pro Ala Ser Met Ile Asp Ala Leu Asp Asn Ile Ala
                645                 650                 655

gcg tgg aac ctg gtc act gca gtc gac gcc ttc ctg tcc tcc ggc ttc 2016
Ala Trp Asn Leu Val Thr Ala Val Asp Ala Phe Leu Ser Ser Gly Phe
            660                 665                 670

agc cca gca gaa ctc ctg cag tcc atc cac cca gct gac gtg tcc tcc 2064
Ser Pro Ala Glu Leu Leu Gln Ser Ile His Pro Ala Asp Val Ser Ser
        675                 680                 685

acc cag ggc acc ggt atc ggt ggc atg cag tcc cta cgc aag ctg ttc 2112

Thr Gln Gly Thr Gly Ile Gly Gly Met Gln Ser Leu Arg Lys Leu Phe
    690                 695                 700

gtc aac cgc ttc ctc ggc cag gat cgt cca tcc gac atc ctc cag gag    2160
Val Asn Arg Phe Leu Gly Gln Asp Arg Pro Ser Asp Ile Leu Gln Glu
705                 710                 715                 720

acc ctg cca aac gtt gtg gct gca cac acc atg cag tcc tac gtc ggt    2208
Thr Leu Pro Asn Val Val Ala Ala His Thr Met Gln Ser Tyr Val Gly
                725                 730                 735

ggc tac ggc cag atg atc cac cca gtg gca gca tgt gca acc gca gct    2256
Gly Tyr Gly Gln Met Ile His Pro Val Ala Ala Cys Ala Thr Ala Ala
            740                 745                 750

gtc tcc gtg gaa gaa ggc gtg gac aag atc cgc ctc aac aag gca gat    2304
Val Ser Val Glu Glu Gly Val Asp Lys Ile Arg Leu Asn Lys Ala Asp
        755                 760                 765

ttc gtt gtc gcc ggt ggt atc gat gac atc cag gtt gaa tcc ctg acc    2352
Phe Val Val Ala Gly Gly Ile Asp Asp Ile Gln Val Glu Ser Leu Thr
    770                 775                 780

ggc ttc ggt gac atg aac gcc acc gca gac acc cag gca atg ctg gac    2400
Gly Phe Gly Asp Met Asn Ala Thr Ala Asp Thr Gln Ala Met Leu Asp
785                 790                 795                 800

aag ggc atc gac cca cgc ttc atc tcc cgc gca aac gat cga cgt cgc    2448
Lys Gly Ile Asp Pro Arg Phe Ile Ser Arg Ala Asn Asp Arg Arg Arg
                805                 810                 815

gca ggc ttc ctc gaa gca gca ggt ggc ggt acc gtc ctc ctg gca cgt    2496
Ala Gly Phe Leu Glu Ala Ala Gly Gly Gly Thr Val Leu Leu Ala Arg
            820                 825                 830

gca tcc gtt gct gct gaa ctg gga ctg cca gtt ctc gca gtt gtt gca    2544
Ala Ser Val Ala Ala Glu Leu Gly Leu Pro Val Leu Ala Val Val Ala
        835                 840                 845

cac gca cag tcc tac gcc gat ggt gct cac acc tcc atc cca gca ccg    2592
His Ala Gln Ser Tyr Ala Asp Gly Ala His Thr Ser Ile Pro Ala Pro
    850                 855                 860

gga ctt ggc gca ctg ggt gca gca cgt ggt ggc aag aag tcc gta ctt    2640
Gly Leu Gly Ala Leu Gly Ala Ala Arg Gly Gly Lys Lys Ser Val Leu
865                 870                 875                 880

gct cgc gaa ctg aac aaa ttg ggt ctg acc cca gat gac gtt cgc gtg    2688
Ala Arg Glu Leu Asn Lys Leu Gly Leu Thr Pro Asp Asp Val Arg Val
                885                 890                 895

gtc tcg aag cac gac acc tcc acc aac gcc aac gat cca aac gag tcc    2736
Val Ser Lys His Asp Thr Ser Thr Asn Ala Asn Asp Pro Asn Glu Ser
            900                 905                 910

gag ctg cac aac ctg ctg tgg aag acc att gga cgc gaa gcc gac aac    2784
Glu Leu His Asn Leu Leu Trp Lys Thr Ile Gly Arg Glu Ala Asp Asn
        915                 920                 925

ccg atg ttc gtc gtc tcc cag aag tcc ctt acc gga cac tca aag ggc    2832
Pro Met Phe Val Val Ser Gln Lys Ser Leu Thr Gly His Ser Lys Gly

930                 935                 940

ggt gca gca ctc ttc cag atc ggt gga ctt gtc tcc atc ctg gaa acc     2880
Gly Ala Ala Leu Phe Gln Ile Gly Gly Leu Val Ser Ile Leu Glu Thr
945                 950                 955                 960

ggc aag ctg cca cag aac gca tcc ctt gac tgc gtt gac cca gag atg     2928
Gly Lys Leu Pro Gln Asn Ala Ser Leu Asp Cys Val Asp Pro Glu Met
                965                 970                 975

gaa gca aag ggc gag aac ttc gtc tgg ctg cgc aag cca ctg gat ctc     2976
Glu Ala Lys Gly Glu Asn Phe Val Trp Leu Arg Lys Pro Leu Asp Leu
            980                 985                 990

ggc gca ggc tcc att aag gcc ggc gta ctt acc tca ctg ggc ttc ggc     3024
Gly Ala Gly Ser Ile Lys Ala Gly Val Leu Thr Ser Leu Gly Phe Gly
        995                 1000                1005

cac gtg gct gca gtc gtc gtg ctg gca acc agc ggc atc ttc gag cag     3072
His Val Ala Ala Val Val Val Leu Ala Thr Ser Gly Ile Phe Glu Gln
    1010                1015                1020

gca atg cgc aac gca ggc ctc gac gtc gaa gca tgg cgt gca cgc gca     3120
Ala Met Arg Asn Ala Gly Leu Asp Val Glu Ala Trp Arg Ala Arg Ala
1025                1030                1035                1040

acc cag cgc ctg cgc acc ggt gca aac cgc cta gaa gct ggc atg gtt     3168
Thr Gln Arg Leu Arg Thr Gly Ala Asn Arg Leu Glu Ala Gly Met Val
                1045                1050                1055

ggc cga gca cca ttg ttc gag cag gtc gac gga cgt cgc ctg cca gag     3216
Gly Arg Ala Pro Leu Phe Glu Gln Val Asp Gly Arg Arg Leu Pro Glu
            1060                1065                1070

cat ggc gct cac caa gca gag atc aac ttg ctt atc gac gct gac gct     3264
His Gly Ala His Gln Ala Glu Ile Asn Leu Leu Ile Asp Ala Asp Ala
        1075                1080                1085

cgc ctc ggt gct gac ggc atc tac cag ggc taaacgttag atagctaaga     3314
Arg Leu Gly Ala Asp Gly Ile Tyr Gln Gly
    1090                1095

aag                                                                 3317

<210> 48
<211> 1098
<212> PRT
<213> Corynebacterium glutamicum

<400> 48
Ala Ser Gly Gly Gly Val Val Asp Ser Ala Ala Leu Asp Ala Tyr Ala
1               5                   10                  15

Ser Thr Val Thr Gly Glu Glu Gly Val Leu Ala Asn Val Ala Arg Gly
            20                  25                  30

Ile Leu Ser Gln Leu Gly Leu Asp Thr Lys Asp Glu Val Glu Gly Ala
        35                  40                  45

Glu Ile Asp Thr Glu Leu Tyr Asp Ala Val Glu Ala Glu Leu Gly Thr

50                  55                  60

Gly Trp Leu Lys Leu Val Thr Pro Val Phe Ser Ala Asp Arg Ala Ile
65                  70                  75                  80

Leu Phe Asp Asp Arg Trp Ala Ser Ala Arg Glu Asp Leu Ala Arg Leu
                85                  90                  95

Ala Asn Gly Glu Asp Ile Ala Val Glu Arg Phe Ala Gly Thr Gly Glu
            100                 105                 110

Thr Val Val Lys Gln Ala Ala Trp Trp Ala Glu His Val Glu Asp Thr
        115                 120                 125

Ala Leu Ala Ala Thr Leu Lys Gln Val Ser Glu Val Ala Ala Lys Pro
    130                 135                 140

Ala Asn Glu Pro His Ile Asp Asp Val Ala Leu Val Thr Gly Ala Ala
145                 150                 155                 160

Pro Glu Ser Ile Ala Gly Ala Val Ala Ala Arg Leu Leu Ser Gln Gly
                165                 170                 175

Ala Thr Val Ile Leu Thr Ala Ser Asn Val Ser Gln Ala Arg Lys Glu
            180                 185                 190

Tyr Ala Arg Lys Leu Tyr Ala Ala Asn Ala Thr Pro Asn Ala Lys Leu
        195                 200                 205

Trp Ile Val Pro Ala Asn Met Ser Ser Tyr Arg Asp Val Asp Ala Val
    210                 215                 220

Ile Asp Trp Ile Gly Asn Glu Gln Arg Val Thr Val Gly Ser Thr Val
225                 230                 235                 240

Thr Val Thr Lys Pro Ala Leu Thr Pro Thr Leu Ala Tyr Pro Phe Ala
                245                 250                 255

Ala Pro Ser Val Ser Gly Thr Leu Ala Asp Ala Gly Pro Gln Ala Glu
            260                 265                 270

Asn Gln Ala Arg Leu Leu Leu Trp Ser Val Glu Arg Thr Ile Ala Gly
        275                 280                 285

Leu Ala Asp Leu Ala Ser Arg Gly Val Asp Gly Arg Val His Val Val
    290                 295                 300

Leu Pro Gly Ser Pro Asn Arg Gly Met Phe Gly Gly Asp Gly Ala Tyr
305                 310                 315                 320

Gly Glu Val Lys Ala Ala Phe Asp Ala Ile Leu Ala Lys Trp Gly Ser
                325                 330                 335

Glu Thr Gly Trp Pro Gln Phe Val Ser Leu Ala Gln Ala Arg Ile Gly
            340                 345                 350

Trp Val Ala Gly Thr Gly Leu Met Gly Arg Asn Asp Val Leu Ile Pro
        355                 360                 365

Ala Ala Glu Lys Leu Gly Ile His Val Tyr Thr Pro Glu Glu Ile Ser
    370                 375                 380

Ser Glu Leu Leu Gly Leu Ala Ser Ala Glu Ser Arg Glu Lys Ala Leu
385                 390                 395                 400

Glu Ala Pro Ile Asp Tyr Asp Leu Thr Gly Gly Leu Ser Gly Gly Val
                405                 410                 415

Ser Ile Ala Ala Leu Ala Ala Ser Leu Glu Ser Asp Ala Val Glu Thr
            420                 425                 430

Thr Ser Ala Ala Glu Asp Thr Ile Lys Ala Leu Pro Ser Pro Lys His
        435                 440                 445

Pro Glu Gln Pro Val Gly Thr Pro Val Gly Glu Val Lys Thr Asp Leu
    450                 455                 460

Glu Asp Met Val Val Met Val Gly Val Gly Glu Val Ser Ser Trp Gly
465                 470                 475                 480

Ser Gly Arg Thr Arg Phe Glu Ala Glu Tyr Gly Ile Gln Arg Asp Gly
                485                 490                 495

Ser Val Asp Leu Thr Ala Ala Gly Val Leu Glu Leu Ala Trp Met Met
            500                 505                 510

Gly Leu Ile Ser Trp Ser Glu Asp Pro Lys Pro Ala Trp Tyr Asp Ala
        515                 520                 525

Asp Gly Thr Glu Val Pro Glu Glu Glu Ile Tyr Glu Arg Phe Arg Asp
    530                 535                 540

Glu Val Ile Ala Arg Cys Gly Val Arg Glu Leu Val Asp Asp Ala Phe
545                 550                 555                 560

Leu Val Asp Gly Gly Ser Leu Asp Ala Ala Glu Val Phe Leu Asp Arg
                565                 570                 575

Asp Ile Ser Phe Ser Val Thr Ser Ala Glu Glu Ala Gln Ala Tyr Val
            580                 585                 590

Asp Ala Asp Ala Ser Val Thr Val Glu Glu Ala Asp Gly Glu Trp Ile
        595                 600                 605

Val Thr Lys Lys Lys Gly Ser Thr Ser Phe Val Pro Arg Lys Ala Thr
    610                 615                 620

Leu Thr Arg Ser Val Ala Gly Gln Leu Pro Thr Asp Phe Asp Pro Ala
625                 630                 635                 640

Lys Trp Gly Ile Pro Ala Ser Met Ile Asp Ala Leu Asp Asn Ile Ala
                645                 650                 655

Ala Trp Asn Leu Val Thr Ala Val Asp Ala Phe Leu Ser Ser Gly Phe
            660                 665                 670

Ser Pro Ala Glu Leu Leu Gln Ser Ile His Pro Ala Asp Val Ser Ser
        675                 680                 685

Thr Gln Gly Thr Gly Ile Gly Gly Met Gln Ser Leu Arg Lys Leu Phe
    690                 695                 700

Val Asn Arg Phe Leu Gly Gln Asp Arg Pro Ser Asp Ile Leu Gln Glu
705                 710                 715                 720

Thr Leu Pro Asn Val Val Ala Ala His Thr Met Gln Ser Tyr Val Gly
                725                 730                 735

Gly Tyr Gly Gln Met Ile His Pro Val Ala Ala Cys Ala Thr Ala Ala
            740                 745                 750

Val Ser Val Glu Glu Gly Val Asp Lys Ile Arg Leu Asn Lys Ala Asp
        755                 760                 765

Phe Val Val Ala Gly Gly Ile Asp Asp Ile Gln Val Glu Ser Leu Thr
    770                 775                 780

Gly Phe Gly Asp Met Asn Ala Thr Ala Asp Thr Gln Ala Met Leu Asp
785                 790                 795                 800

Lys Gly Ile Asp Pro Arg Phe Ile Ser Arg Ala Asn Asp Arg Arg Arg
                805                 810                 815

Ala Gly Phe Leu Glu Ala Ala Gly Gly Gly Thr Val Leu Leu Ala Arg
            820                 825                 830

Ala Ser Val Ala Ala Glu Leu Gly Leu Pro Val Leu Ala Val Val Ala
        835                 840                 845

His Ala Gln Ser Tyr Ala Asp Gly Ala His Thr Ser Ile Pro Ala Pro
    850                 855                 860

Gly Leu Gly Ala Leu Gly Ala Ala Arg Gly Gly Lys Lys Ser Val Leu
865                 870                 875                 880

Ala Arg Glu Leu Asn Lys Leu Gly Leu Thr Pro Asp Asp Val Arg Val
                885                 890                 895

Val Ser Lys His Asp Thr Ser Thr Asn Ala Asn Asp Pro Asn Glu Ser
            900                 905                 910

Glu Leu His Asn Leu Leu Trp Lys Thr Ile Gly Arg Glu Ala Asp Asn
        915                 920                 925

Pro Met Phe Val Val Ser Gln Lys Ser Leu Thr Gly His Ser Lys Gly
    930                 935                 940

Gly Ala Ala Leu Phe Gln Ile Gly Gly Leu Val Ser Ile Leu Glu Thr
945                 950                 955                 960

Gly Lys Leu Pro Gln Asn Ala Ser Leu Asp Cys Val Asp Pro Glu Met
                965                 970                 975

Glu Ala Lys Gly Glu Asn Phe Val Trp Leu Arg Lys Pro Leu Asp Leu
            980                 985                 990

Gly Ala Gly Ser Ile Lys Ala Gly Val Leu Thr Ser Leu Gly Phe Gly
        995                 1000                1005

His Val Ala Ala Val Val Val Leu Ala Thr Ser Gly Ile Phe Glu Gln
    1010                1015                1020

Ala Met Arg Asn Ala Gly Leu Asp Val Glu Ala Trp Arg Ala Arg Ala

1025                1030                1035                1040

Thr Gln Arg Leu Arg Thr Gly Ala Asn Arg Leu Glu Ala Gly Met Val
                1045                1050                1055

Gly Arg Ala Pro Leu Phe Glu Gln Val Asp Gly Arg Arg Leu Pro Glu
            1060                1065                1070

His Gly Ala His Gln Ala Glu Ile Asn Leu Leu Ile Asp Ala Asp Ala
        1075                1080                1085

Arg Leu Gly Ala Asp Gly Ile Tyr Gln Gly
    1090                1095


<210> 49
<211> 6136
<212> DNA
<213> Corynebacterium glutamicum

<220>
<221> CDS
<222> (101)..(6136)
<223> RXN03111

<400> 49
cgtgaccgaa ttgagcagga acttcggggc cagccgactg attaaccgct ttggccagga 60

gccttttgcc ttcgctttcg ccggccaagg atatgactgg ttg aag acc ctt cgt   115
                                           Leu Lys Thr Leu Arg
                                           1               5

gcc gcg gtt gcc gca ggt gca ggc acc aat gtt agt gac atc gtc gag   163
Ala Ala Val Ala Ala Gly Ala Gly Thr Asn Val Ser Asp Ile Val Glu
                10                  15                  20

cgc gca aat gcg ctg ctt gca cta gtt gca gat gat ctc att ggc acc   211
Arg Ala Asn Ala Leu Leu Ala Leu Val Ala Asp Asp Leu Ile Gly Thr
            25                  30                  35

ctt cca ttt ggt ttc gat cca gtg gct tgg gct aac aac tcc gaa gat   259
Leu Pro Phe Gly Phe Asp Pro Val Ala Trp Ala Asn Asn Ser Glu Asp
        40                  45                  50

cca gct ttc gat act gca caa tct gca gtg agc gtg ccg ggt atc ttt   307
Pro Ala Phe Asp Thr Ala Gln Ser Ala Val Ser Val Pro Gly Ile Phe
    55                  60                  65

gtc tcc cag atc gca acc ctg gat tcc ctt gag gcg cag cgc ctt gat   355
Val Ser Gln Ile Ala Thr Leu Asp Ser Leu Glu Ala Gln Arg Leu Asp
70                  75                  80                  85

gtg gat cag gct gtg tcc agc att ggt cat tcc cag ggc gta ttg ggc   403
Val Asp Gln Ala Val Ser Ser Ile Gly His Ser Gln Gly Val Leu Gly
                90                  95                  100

gtg cac ctg ctc aat gat gcg act cgt gct gat gaa ctc gtt gcc att   451
Val His Leu Leu Asn Asp Ala Thr Arg Ala Asp Glu Leu Val Ala Ile
            105                 110                 115

gcg cag ttg atc ggt gca gcg atc acc cgc acc gca cgc atg acg ggc   499

Ala Gln Leu Ile Gly Ala Ala Ile Thr Arg Thr Ala Arg Met Thr Gly
        120                 125                 130

ctg atc gcg cag ggc gac aac atg ccg atg ctg tcg atc gcc gga att     547
Leu Ile Ala Gln Gly Asp Asn Met Pro Met Leu Ser Ile Ala Gly Ile
    135                 140                 145

tcc cgc gaa cag ctt cag caa gct atc gac gcg gcc tgc gcc gaa gtc     595
Ser Arg Glu Gln Leu Gln Gln Ala Ile Asp Ala Ala Cys Ala Glu Val
150                 155                 160                 165

cct gcg gag atc cgc ccg gtt atc ggt ctg cgc aac tca cgc gat tct     643
Pro Ala Glu Ile Arg Pro Val Ile Gly Leu Arg Asn Ser Arg Asp Ser
                170                 175                 180

tat gtt ttg gtt ggc cgc cca gac gac aac gct cgc gtt gtt aag gtc     691
Tyr Val Leu Val Gly Arg Pro Asp Asp Asn Ala Arg Val Val Lys Val
            185                 190                 195

att gag gca atg gct gcc aag gat aag aag gcc att gaa gat aag ctg     739
Ile Glu Ala Met Ala Ala Lys Asp Lys Lys Ala Ile Glu Asp Lys Leu
        200                 205                 210

cgc ggc ggt tcc gcg ttc agc ccc cgt att act ccg ctg aag gtg cag     787
Arg Gly Gly Ser Ala Phe Ser Pro Arg Ile Thr Pro Leu Lys Val Gln
    215                 220                 225

gct gct ttc cat cac cca gct atg aac atg gct gtg gag cag acc gtg     835
Ala Ala Phe His His Pro Ala Met Asn Met Ala Val Glu Gln Thr Val
230                 235                 240                 245

gcg tgg gca acc act gct ggt ttg gat gtg gaa ctc acc cgc gag atc     883
Ala Trp Ala Thr Thr Ala Gly Leu Asp Val Glu Leu Thr Arg Glu Ile
                250                 255                 260

gcc gct gat gtt ttg gtt aac cct gtc gat tgg gta gca cgc gtc aac     931
Ala Ala Asp Val Leu Val Asn Pro Val Asp Trp Val Ala Arg Val Asn
            265                 270                 275

gaa gcg tat gag gct ggc gct cgc tgg ttc ctc gac gtt gga cca gat     979
Glu Ala Tyr Glu Ala Gly Ala Arg Trp Phe Leu Asp Val Gly Pro Asp
        280                 285                 290

ggt ggc atc gtt aag ctg act gcc aac atc ctt gag ggc cgc ggc gcg     1027
Gly Gly Ile Val Lys Leu Thr Ala Asn Ile Leu Glu Gly Arg Gly Ala
    295                 300                 305

gat tcc ttc tat gtt ggt gac gcc gca ggc cag gcc aag ata ttt gat     1075
Asp Ser Phe Tyr Val Gly Asp Ala Ala Gly Gln Ala Lys Ile Phe Asp
310                 315                 320                 325

gct ggc atg gca cct gaa ctt cca gtg gat tac cag gag ttc gca cca     1123
Ala Gly Met Ala Pro Glu Leu Pro Val Asp Tyr Gln Glu Phe Ala Pro
                330                 335                 340

cgc gtt gag cac gtt gat gga acc cca cgc ctg gtt acc aag ttc act     1171
Arg Val Glu His Val Asp Gly Thr Pro Arg Leu Val Thr Lys Phe Thr
            345                 350                 355

gag ctg acc ggc cgc acc cca atg atg ctg gct ggc atg acc cca acc     1219
Glu Leu Thr Gly Arg Thr Pro Met Met Leu Ala Gly Met Thr Pro Thr

        360                 365                 370

acc gtt gac cct gcc att gtt gca gcc gct gca aac ggt gga cac tgg 1267
Thr Val Asp Pro Ala Ile Val Ala Ala Ala Ala Asn Gly Gly His Trp
    375                 380                 385

gct gag ctc gct ggt ggc gga cag gtt acc cca gag ctg ctg gaa acc 1315
Ala Glu Leu Ala Gly Gly Gly Gln Val Thr Pro Glu Leu Leu Glu Thr
390                 395                 400                 405

cac atc gca cag ctc acc gac atg ctt gag cca ggt atc aac gcc cag 1363
His Ile Ala Gln Leu Thr Asp Met Leu Glu Pro Gly Ile Asn Ala Gln
                410                 415                 420

ttc aac tcc atg ttc ttg gat cca tac ctg tgg aag atg cag att ggt 1411
Phe Asn Ser Met Phe Leu Asp Pro Tyr Leu Trp Lys Met Gln Ile Gly
            425                 430                 435

ggc aag cgc ctt gtt cct aag gcc cgc gct aat ggt gca tcc atc gac 1459
Gly Lys Arg Leu Val Pro Lys Ala Arg Ala Asn Gly Ala Ser Ile Asp
        440                 445                 450

ggc atc gtc atc acc gcc ggc att cct gaa aag gat gaa gct gtt gca 1507
Gly Ile Val Ile Thr Ala Gly Ile Pro Glu Lys Asp Glu Ala Val Ala
    455                 460                 465

ttg gtc aag gaa ctg atg cgt gat ggt ttc cct tgg atc gca ttc aag 1555
Leu Val Lys Glu Leu Met Arg Asp Gly Phe Pro Trp Ile Ala Phe Lys
470                 475                 480                 485

cca ggt gcc atc aag cag gtt aac tct gtg ttg gct atc gct aag gaa 1603
Pro Gly Ala Ile Lys Gln Val Asn Ser Val Leu Ala Ile Ala Lys Glu
                490                 495                 500

gtt cca gaa ctc ccc atc atc att cag att gag ggt ggc gtt gca ggt 1651
Val Pro Glu Leu Pro Ile Ile Ile Gln Ile Glu Gly Gly Val Ala Gly
            505                 510                 515

gga cac cac tct tgg gaa gac ctc gat gag ctg ctg atc gcc acc tac 1699
Gly His His Ser Trp Glu Asp Leu Asp Glu Leu Leu Ile Ala Thr Tyr
        520                 525                 530

ggc aag gtc cgc gca ctg gat aac gtg gtg ctg tgt gtc ggc ggt ggc 1747
Gly Lys Val Arg Ala Leu Asp Asn Val Val Leu Cys Val Gly Gly Gly
    535                 540                 545

att ggc tca cct gag cgc gct gct gat tac gtc acc ggt tcc tgg tcc 1795
Ile Gly Ser Pro Glu Arg Ala Ala Asp Tyr Val Thr Gly Ser Trp Ser
550                 555                 560                 565

act tcc tac ggc ctg cca gct atg cct gtt gat ggc atc ttg gtg ggt 1843
Thr Ser Tyr Gly Leu Pro Ala Met Pro Val Asp Gly Ile Leu Val Gly
                570                 575                 580

acc gct gcg atg gca acc aag gaa gca acc acc tcc cag gcc gtc aag 1891
Thr Ala Ala Met Ala Thr Lys Glu Ala Thr Thr Ser Gln Ala Val Lys
            585                 590                 595

gaa ctt ctt gtt tcc acc cag ggc tct gat gaa tgg gtt cct gct ggt 1939
Glu Leu Leu Val Ser Thr Gln Gly Ser Asp Glu Trp Val Pro Ala Gly
        600                 605                 610

ggc gca aag aac gga atg gca tct ggc cgt tcc cag ctt ggc gca gac 1987
Gly Ala Lys Asn Gly Met Ala Ser Gly Arg Ser Gln Leu Gly Ala Asp
    615                 620                 625

atc cac gag atc gac aac tcc ttt gct aag gct gga cgc ctt ctt gat 2035
Ile His Glu Ile Asp Asn Ser Phe Ala Lys Ala Gly Arg Leu Leu Asp
630                 635                 640                 645

gag gtt gca ggc gat gag acg gct gtg cag gcg cgc cgg gat gag atc 2083
Glu Val Ala Gly Asp Glu Thr Ala Val Gln Ala Arg Arg Asp Glu Ile
                650                 655                 660

att gaa gcg att ggc aag acc gcc aag gtg tac ttc ggt gac atc gga 2131
Ile Glu Ala Ile Gly Lys Thr Ala Lys Val Tyr Phe Gly Asp Ile Gly
            665                 670                 675

tcc atg act tac gag cag tgg ctc aac cgc tac ctc gag ctg tct ggc 2179
Ser Met Thr Tyr Glu Gln Trp Leu Asn Arg Tyr Leu Glu Leu Ser Gly
        680                 685                 690

cct gtt gat ggt cag tgg att gat gct tcc tgg gct gca cgt ttt gcc 2227
Pro Val Asp Gly Gln Trp Ile Asp Ala Ser Trp Ala Ala Arg Phe Ala
    695                 700                 705

cag atg ctg gag cgt gcc gag gcg cgt ttg atc gag cag gat cat ggc 2275
Gln Met Leu Glu Arg Ala Glu Ala Arg Leu Ile Glu Gln Asp His Gly
710                 715                 720                 725

caa ttt gag cca agc ctg acg gtg gag gat ggc gtc gac aag ctt gtt 2323
Gln Phe Glu Pro Ser Leu Thr Val Glu Asp Gly Val Asp Lys Leu Val
                730                 735                 740

gct gct tac ccg cat gcc gca acc gac ctg ctc acc ccg gct gat gtc 2371
Ala Ala Tyr Pro His Ala Ala Thr Asp Leu Leu Thr Pro Ala Asp Val
            745                 750                 755

gcc tgg ttc ttg ggc ctg tgc cgc acg ccg ggc aag cct gtg aac ttt 2419
Ala Trp Phe Leu Gly Leu Cys Arg Thr Pro Gly Lys Pro Val Asn Phe
        760                 765                 770

gtg ccc gtc att gat aag gac gtg cgt cgc tgg tgg cgc tcg gac tcc 2467
Val Pro Val Ile Asp Lys Asp Val Arg Arg Trp Trp Arg Ser Asp Ser
    775                 780                 785

ctg tgg cag tcc cac gat gat cgc tac acc gct gat cag gtg gct att 2515
Leu Trp Gln Ser His Asp Asp Arg Tyr Thr Ala Asp Gln Val Ala Ile
790                 795                 800                 805

atc cct ggt gtc gtc gcc gtt gct ggc atc acc aag gcc aac gaa cct 2563
Ile Pro Gly Val Val Ala Val Ala Gly Ile Thr Lys Ala Asn Glu Pro
                810                 815                 820

gtc gct gac ctg ctt gat cgc ttt gtc gac gcc acc atc gag cgc atc 2611
Val Ala Asp Leu Leu Asp Arg Phe Val Asp Ala Thr Ile Glu Arg Ile
            825                 830                 835

gat gag cac gat tcc cgc tcc cgc gac atc atg ggc aaa gtg ctt tcc 2659
Asp Glu His Asp Ser Arg Ser Arg Asp Ile Met Gly Lys Val Leu Ser
        840                 845                 850

tca cct ggc aca ttc tgg gct ggc cgc aac atc cca tcg gtg atc cac 2707
Ser Pro Gly Thr Phe Trp Ala Gly Arg Asn Ile Pro Ser Val Ile His
    855                 860                 865

agc ctt ggg cat gct gac aag tgg tcc cgc tcc gaa ttc gaa gca ttc 2755
Ser Leu Gly His Ala Asp Lys Trp Ser Arg Ser Glu Phe Glu Ala Phe
870                 875                 880                 885

cat agc cca acc ggc gcc aac ttg gtg tac gaa gac gcc gag cac gcg 2803
His Ser Pro Thr Gly Ala Asn Leu Val Tyr Glu Asp Ala Glu His Ala
                890                 895                 900

atg ctg act gtg cct ttg gcg ggt tcc acc gca ttc ggc acc acc gct 2851
Met Leu Thr Val Pro Leu Ala Gly Ser Thr Ala Phe Gly Thr Thr Ala
            905                 910                 915

gag ctg aaa atc cgt ttc acc agc ccc atc gac gct ctg cca agc gct 2899
Glu Leu Lys Ile Arg Phe Thr Ser Pro Ile Asp Ala Leu Pro Ser Ala
        920                 925                 930

gtc cca ctg gtc acc cag gaa gac gct gaa gcc gcg atg ggt gaa ctg 2947
Val Pro Leu Val Thr Gln Glu Asp Ala Glu Ala Ala Met Gly Glu Leu
    935                 940                 945

acc cgc atc gca gct ggc ggc acc ctg gca act gtg aac aat ggc acc 2995
Thr Arg Ile Ala Ala Gly Gly Thr Leu Ala Thr Val Asn Asn Gly Thr
950                 955                 960                 965

gct acc tgg gaa acc tcc gtc gat gcc ggc gtc atc gct gac tac aac 3043
Ala Thr Trp Glu Thr Ser Val Asp Ala Gly Val Ile Ala Asp Tyr Asn
                970                 975                 980

aac gtc acc gca ggc tac ctg cca gca tcc gtt gtt cct gca cac acc 3091
Asn Val Thr Ala Gly Tyr Leu Pro Ala Ser Val Val Pro Ala His Thr
            985                 990                 995

gca cct gac gtg ctg gtt ggc cgc gca tgg cca gca gtt ttc gct gcc 3139
Ala Pro Asp Val Leu Val Gly Arg Ala Trp Pro Ala Val Phe Ala Ala
        1000                1005                1010

gta aag tcc gca gtc atc cca ggc acc gat tcc gca tcc gtt gtg gaa 3187
Val Lys Ser Ala Val Ile Pro Gly Thr Asp Ser Ala Ser Val Val Glu
    1015                1020                1025

ggc atg ctg tcc ctg gtt cac ctg gag cac cac att gtg ctc aag tcc 3235
Gly Met Leu Ser Leu Val His Leu Glu His His Ile Val Leu Lys Ser
1030                1035                1040                1045

gat gtc cca acc gac ggc gcg ctg aag gtt tcc gcg act gcc gat gag 3283
Asp Val Pro Thr Asp Gly Ala Leu Lys Val Ser Ala Thr Ala Asp Glu
                1050                1055                1060

gta gtc gat acc gac ctg ggt cgc ctc gtg atc gtg cgc gca gaa atc 3331
Val Val Asp Thr Asp Leu Gly Arg Leu Val Ile Val Arg Ala Glu Ile
            1065                1070                1075

gcc gac gca gaa ggc aac ctg att gct acg ttg gct gag cgt ttc gcg 3379
Ala Asp Ala Glu Gly Asn Leu Ile Ala Thr Leu Ala Glu Arg Phe Ala
        1080                1085                1090

atc cgc gga cgc aag ggc aac gct gtc gca cgc acc aac acc tcc gca 3427

Ile Arg Gly Arg Lys Gly Asn Ala Val Ala Arg Thr Asn Thr Ser Ala
    1095                    1100                    1105

ctg cca acc acc gtg gac acc cca cgc tca gct cgc gca gtg gca acc    3475
Leu Pro Thr Thr Val Asp Thr Pro Arg Ser Ala Arg Ala Val Ala Thr
1110                    1115                    1120                    1125

gtt gtt gca cct gaa tcc atg cgc cca ttc gct gtg atc tcc ggt gac    3523
Val Val Ala Pro Glu Ser Met Arg Pro Phe Ala Val Ile Ser Gly Asp
                1130                    1135                    1140

cgc aac cca att cac gtc tct gat gtt gcg gct tcc ctg gct ggt ctg    3571
Arg Asn Pro Ile His Val Ser Asp Val Ala Ala Ser Leu Ala Gly Leu
            1145                    1150                    1155

cca ggt gtg atc gtg cac ggc atg tgg acc tct gcc atc ggt gaa ctg    3619
Pro Gly Val Ile Val His Gly Met Trp Thr Ser Ala Ile Gly Glu Leu
        1160                    1165                    1170

atc gcc ggt gca gca ttc aac gat gag cag atc caa act ccc gca gcc    3667
Ile Ala Gly Ala Ala Phe Asn Asp Glu Gln Ile Gln Thr Pro Ala Ala
    1175                    1180                    1185

aag gtc gtg gaa tac acc gca acc atg ctg gca cca gtt ctt cca ggt    3715
Lys Val Val Glu Tyr Thr Ala Thr Met Leu Ala Pro Val Leu Pro Gly
1190                    1195                    1200                    1205

gaa gaa att gag ttc agc gtt gag cgc tcc gca gtg gac aac cgc cca    3763
Glu Glu Ile Glu Phe Ser Val Glu Arg Ser Ala Val Asp Asn Arg Pro
                1210                    1215                    1220

gga atg gga gag gtc cgc acc gtt acc gca acc gtc aac ggc aac tta    3811
Gly Met Gly Glu Val Arg Thr Val Thr Ala Thr Val Asn Gly Asn Leu
            1225                    1230                    1235

gtg ctt acc gcc acc gct gtt gtg gca gct cca tct act ttc tac gca    3859
Val Leu Thr Ala Thr Ala Val Val Ala Ala Pro Ser Thr Phe Tyr Ala
        1240                    1245                    1250

ttc cca ggc cag ggc att cag tcc cag ggc atg ggt atg gaa gca cgc    3907
Phe Pro Gly Gln Gly Ile Gln Ser Gln Gly Met Gly Met Glu Ala Arg
    1255                    1260                    1265

cgt aac tct cag gca gct cgc gct atc tgg gac cgc gcc gat gca cac    3955
Arg Asn Ser Gln Ala Ala Arg Ala Ile Trp Asp Arg Ala Asp Ala His
1270                    1275                    1280                    1285

acc cgc aat aag ctg ggc ttc tcc atc gtg gaa atc gtg gaa aac aac    4003
Thr Arg Asn Lys Leu Gly Phe Ser Ile Val Glu Ile Val Glu Asn Asn
                1290                    1295                    1300

cca cgc gaa gta acc gtg gca ggg gag aag ttc ttc cac cca gac ggc    4051
Pro Arg Glu Val Thr Val Ala Gly Glu Lys Phe Phe His Pro Asp Gly
            1305                    1310                    1315

gtt ttg tac ctc acc cag ttc acc cag gtg ggc atg gca act ctg ggc    4099
Val Leu Tyr Leu Thr Gln Phe Thr Gln Val Gly Met Ala Thr Leu Gly
        1320                    1325                    1330

gtt gct cag atc gct gaa atg cgt gaa gca cat gcc ttg aac cag cgt    4147
Val Ala Gln Ile Ala Glu Met Arg Glu Ala His Ala Leu Asn Gln Arg

1335                1340                1345

gca tac ttt gct gga cac tcc gtt ggt gag tac aac gcg ctt gct gca    4195
Ala Tyr Phe Ala Gly His Ser Val Gly Glu Tyr Asn Ala Leu Ala Ala
1350                1355                1360                1365

tat gct ggt gtg ctg tcc ctg gaa tcc gtt ctg gag atc gtt tac cgt    4243
Tyr Ala Gly Val Leu Ser Leu Glu Ser Val Leu Glu Ile Val Tyr Arg
                1370                1375                1380

cgt ggc ttg acc atg cac cgc ttg gtg gat cgc gat gaa aac ggt ctg    4291
Arg Gly Leu Thr Met His Arg Leu Val Asp Arg Asp Glu Asn Gly Leu
            1385                1390                1395

tcc aac tac gcg ctc gca gct ctt cgc ccc aac aag atg ggt ctg acc    4339
Ser Asn Tyr Ala Leu Ala Ala Leu Arg Pro Asn Lys Met Gly Leu Thr
        1400                1405                1410

gca gac aac gtt ttc gat tac gtt gcg tct gtt tcc gaa gct tcc ggt    4387
Ala Asp Asn Val Phe Asp Tyr Val Ala Ser Val Ser Glu Ala Ser Gly
    1415                1420                1425

gaa ttc ctg gag atc gtt aac tac aac ttg gct ggc ctg cag tac gca    4435
Glu Phe Leu Glu Ile Val Asn Tyr Asn Leu Ala Gly Leu Gln Tyr Ala
1430                1435                1440                1445

gtt gct gga acc cag gct ggt ctt gcc gcc ctt cgt gcc gat gtt gag    4483
Val Ala Gly Thr Gln Ala Gly Leu Ala Ala Leu Arg Ala Asp Val Glu
                1450                1455                1460

aac cgt gca cca ggt cag cgt gcc ttc att ttg atc cct ggc att gac    4531
Asn Arg Ala Pro Gly Gln Arg Ala Phe Ile Leu Ile Pro Gly Ile Asp
            1465                1470                1475

gtg cca ttc cac tcc tcc aag ctg cgc gac ggt gtg ggc gcg ttc cgt    4579
Val Pro Phe His Ser Ser Lys Leu Arg Asp Gly Val Gly Ala Phe Arg
        1480                1485                1490

gag cac ctt gat tcc ctg atc cca gct gag ctg gat ctg gat gtg ctg    4627
Glu His Leu Asp Ser Leu Ile Pro Ala Glu Leu Asp Leu Asp Val Leu
    1495                1500                1505

gtt ggc cgc tac att cca aac ttg gtg gct cgc cca ttc gaa ctc act    4675
Val Gly Arg Tyr Ile Pro Asn Leu Val Ala Arg Pro Phe Glu Leu Thr
1510                1515                1520                1525

gaa gag ttc gtg gca tcc atg gca gaa gtg gtg gag tcc acc tat gtc    4723
Glu Glu Phe Val Ala Ser Met Ala Glu Val Val Glu Ser Thr Tyr Val
                1530                1535                1540

aat gag atc ttg gct gat ttc aag gct gct tcc gcc gat aag cag aag    4771
Asn Glu Ile Leu Ala Asp Phe Lys Ala Ala Ser Ala Asp Lys Gln Lys
            1545                1550                1555

ctt gcc cgc acg ttg ctt att gag ctg ctt gca tgg cag ttc gca tca    4819
Leu Ala Arg Thr Leu Leu Ile Glu Leu Leu Ala Trp Gln Phe Ala Ser
        1560                1565                1570

cct gtg cgc tgg atc gag act cag gat ctg ttg atc aag ggc ctt caa    4867
Pro Val Arg Trp Ile Glu Thr Gln Asp Leu Leu Ile Lys Gly Leu Gln
    1575                1580                1585

gct gag cgt ttc gtg gag gtc ggt gtt ggc tct gct cca acg ctt gcc 4915
Ala Glu Arg Phe Val Glu Val Gly Val Gly Ser Ala Pro Thr Leu Ala
1590                1595                1600                1605

aac atg atg ggc cag acc ctg cgc ctt cct cag tac gcg gac gcc acc 4963
Asn Met Met Gly Gln Thr Leu Arg Leu Pro Gln Tyr Ala Asp Ala Thr
                1610                1615                1620

att gag gtg tta aac att gag cgc gat cgc cca gtt gtg ttc gct acc 5011
Ile Glu Val Leu Asn Ile Glu Arg Asp Arg Pro Val Val Phe Ala Thr
            1625                1630                1635

gat gag gtt gtg cgt gaa gtg gcg gtt gaa gag acc cca gca gct cct 5059
Asp Glu Val Val Arg Glu Val Ala Val Glu Glu Thr Pro Ala Ala Pro
        1640                1645                1650

gca gaa acc act gaa acc cca gca acc cca gca acc cca gcc cct gtt 5107
Ala Glu Thr Thr Glu Thr Pro Ala Thr Pro Ala Thr Pro Ala Pro Val
    1655                1660                1665

gca gct gca gcc cct gcc acc ggc ggc cct cgc cca gat gac atc agc 5155
Ala Ala Ala Ala Pro Ala Thr Gly Gly Pro Arg Pro Asp Asp Ile Ser
1670                1675                1680                1685

ttc act cct tct gat gcc act gaa atg ctc atc gct atc tgg acc aag 5203
Phe Thr Pro Ser Asp Ala Thr Glu Met Leu Ile Ala Ile Trp Thr Lys
                1690                1695                1700

gtt cgc cca gat cag atg ggt gcc act gat tcc atc gag acc ctg gtt 5251
Val Arg Pro Asp Gln Met Gly Ala Thr Asp Ser Ile Glu Thr Leu Val
            1705                1710                1715

gag ggc gtg tcc tct cgc cgt aac cag ctc ctg ctg gat ctt ggt gtg 5299
Glu Gly Val Ser Ser Arg Arg Asn Gln Leu Leu Leu Asp Leu Gly Val
        1720                1725                1730

gag ttc ggc ctc ggc gca att gac gga gca gcc gat gct gag ctc ggt 5347
Glu Phe Gly Leu Gly Ala Ile Asp Gly Ala Ala Asp Ala Glu Leu Gly
    1735                1740                1745

gat cta aag gtc acc gtg tcc aag atg gct aag ggc tac aag gcg ttt 5395
Asp Leu Lys Val Thr Val Ser Lys Met Ala Lys Gly Tyr Lys Ala Phe
1750                1755                1760                1765

ggc cct gtg ctc tcc gat gct gca gct gat gcc ctg cgt cgc ctc act 5443
Gly Pro Val Leu Ser Asp Ala Ala Ala Asp Ala Leu Arg Arg Leu Thr
                1770                1775                1780

ggt cct acc ggt aag cgc ccg gga tac atc gca gag cgc gtc acc ggc 5491
Gly Pro Thr Gly Lys Arg Pro Gly Tyr Ile Ala Glu Arg Val Thr Gly
            1785                1790                1795

acg tgg gaa ttg ggc cag ggc tgg gct gac cac gtg gtc gct gaa gtt 5539
Thr Trp Glu Leu Gly Gln Gly Trp Ala Asp His Val Val Ala Glu Val
        1800                1805                1810

gtg atc ggc gcc cgc gaa ggc gca tcc ctg cgc ggc ggc gac ctg gcg 5587
Val Ile Gly Ala Arg Glu Gly Ala Ser Leu Arg Gly Gly Asp Leu Ala
    1815                1820                1825

tca ctg tct cct gca agc cca gcg tct gca tca gat ctt gat tcg ctt     5635
Ser Leu Ser Pro Ala Ser Pro Ala Ser Ala Ser Asp Leu Asp Ser Leu
1830                1835                1840                1845

atc gac gca gcc gtc cag gcc gta gcc tcc cgc cgc ggc gtt gcg gtc     5683
Ile Asp Ala Ala Val Gln Ala Val Ala Ser Arg Arg Gly Val Ala Val
                1850                1855                1860

tcc ctg cct tca gca ggc ggc gct gcc ggt ggc gtg gtt gat tcc gca     5731
Ser Leu Pro Ser Ala Gly Gly Ala Ala Gly Gly Val Val Asp Ser Ala
            1865                1870                1875

gct ctt ggc gag ttt gca gag cag gtc acc gga cac gat ggt gtg ctt     5779
Ala Leu Gly Glu Phe Ala Glu Gln Val Thr Gly His Asp Gly Val Leu
        1880                1885                1890

gct cag gca gcc cgc acc atc ttg acc cag ttg ggt ctt gat aag cca     5827
Ala Gln Ala Ala Arg Thr Ile Leu Thr Gln Leu Gly Leu Asp Lys Pro
    1895                1900                1905

gca acc gtt tcc gtg gaa gac acc gca gag gaa gac ctc tac gag ttg     5875
Ala Thr Val Ser Val Glu Asp Thr Ala Glu Glu Asp Leu Tyr Glu Leu
1910                1915                1920                1925

gtc tcc aag gaa ctc ggt tct gat tgg cca cgt cag gtt gca cca agc     5923
Val Ser Lys Glu Leu Gly Ser Asp Trp Pro Arg Gln Val Ala Pro Ser
                1930                1935                1940

ttc gat gaa gaa aag gtt gtt ctg ctt gat gac cgt tgg gct tct gcg     5971
Phe Asp Glu Glu Lys Val Val Leu Leu Asp Asp Arg Trp Ala Ser Ala
            1945                1950                1955

cgt gag gat ctc tcc gcg ttg ctc ttg gcg aac tcg cag caa ctg ata     6019
Arg Glu Asp Leu Ser Ala Leu Leu Leu Ala Asn Ser Gln Gln Leu Ile
        1960                1965                1970

tcg atg tca cag gcg cag gcg aag ctg ttg cag cac aag ctg aat tct     6067
Ser Met Ser Gln Ala Gln Ala Lys Leu Leu Gln His Lys Leu Asn Ser
    1975                1980                1985

ttg gac ttg atg atc tcg cag cta ggc tcg cga aca aag ctt ctt gga     6115
Leu Asp Leu Met Ile Ser Gln Leu Gly Ser Arg Thr Lys Leu Leu Gly
1990                1995                2000                2005

cta cgc cga caa tgt tgc ggc                                         6136
Leu Arg Arg Gln Cys Cys Gly
                2010


<210> 50
<211> 2012
<212> PRT
<213> Corynebacterium glutamicum

<400> 50
Leu Lys Thr Leu Arg Ala Ala Val Ala Ala Gly Ala Gly Thr Asn Val
1               5                   10                  15

Ser Asp Ile Val Glu Arg Ala Asn Ala Leu Leu Ala Leu Val Ala Asp
            20                  25                  30

Asp Leu Ile Gly Thr Leu Pro Phe Gly Phe Asp Pro Val Ala Trp Ala
        35                  40                  45

Asn Asn Ser Glu Asp Pro Ala Phe Asp Thr Ala Gln Ser Ala Val Ser
    50                  55                  60

Val Pro Gly Ile Phe Val Ser Gln Ile Ala Thr Leu Asp Ser Leu Glu
65                  70                  75                  80

Ala Gln Arg Leu Asp Val Asp Gln Ala Val Ser Ser Ile Gly His Ser
                85                  90                  95

Gln Gly Val Leu Gly Val His Leu Leu Asn Asp Ala Thr Arg Ala Asp
            100                 105                 110

Glu Leu Val Ala Ile Ala Gln Leu Ile Gly Ala Ala Ile Thr Arg Thr
        115                 120                 125

Ala Arg Met Thr Gly Leu Ile Ala Gln Gly Asp Asn Met Pro Met Leu
    130                 135                 140

Ser Ile Ala Gly Ile Ser Arg Glu Gln Leu Gln Gln Ala Ile Asp Ala
145                 150                 155                 160

Ala Cys Ala Glu Val Pro Ala Glu Ile Arg Pro Val Ile Gly Leu Arg
                165                 170                 175

Asn Ser Arg Asp Ser Tyr Val Leu Val Gly Arg Pro Asp Asp Asn Ala
            180                 185                 190

Arg Val Val Lys Val Ile Glu Ala Met Ala Ala Lys Asp Lys Lys Ala
        195                 200                 205

Ile Glu Asp Lys Leu Arg Gly Gly Ser Ala Phe Ser Pro Arg Ile Thr
    210                 215                 220

Pro Leu Lys Val Gln Ala Ala Phe His His Pro Ala Met Asn Met Ala
225                 230                 235                 240

Val Glu Gln Thr Val Ala Trp Ala Thr Thr Ala Gly Leu Asp Val Glu
                245                 250                 255

Leu Thr Arg Glu Ile Ala Ala Asp Val Leu Val Asn Pro Val Asp Trp
            260                 265                 270

Val Ala Arg Val Asn Glu Ala Tyr Glu Ala Gly Ala Arg Trp Phe Leu
        275                 280                 285

Asp Val Gly Pro Asp Gly Gly Ile Val Lys Leu Thr Ala Asn Ile Leu
    290                 295                 300

Glu Gly Arg Gly Ala Asp Ser Phe Tyr Val Gly Asp Ala Ala Gly Gln
305                 310                 315                 320

Ala Lys Ile Phe Asp Ala Gly Met Ala Pro Glu Leu Pro Val Asp Tyr
                325                 330                 335

Gln Glu Phe Ala Pro Arg Val Glu His Val Asp Gly Thr Pro Arg Leu
            340                 345                 350

Val Thr Lys Phe Thr Glu Leu Thr Gly Arg Thr Pro Met Met Leu Ala

        355                 360                 365

Gly Met Thr Pro Thr Thr Val Asp Pro Ala Ile Val Ala Ala Ala Ala
    370                 375                 380

Asn Gly Gly His Trp Ala Glu Leu Ala Gly Gly Gly Gln Val Thr Pro
385                 390                 395                 400

Glu Leu Leu Glu Thr His Ile Ala Gln Leu Thr Asp Met Leu Glu Pro
                405                 410                 415

Gly Ile Asn Ala Gln Phe Asn Ser Met Phe Leu Asp Pro Tyr Leu Trp
            420                 425                 430

Lys Met Gln Ile Gly Gly Lys Arg Leu Val Pro Lys Ala Arg Ala Asn
        435                 440                 445

Gly Ala Ser Ile Asp Gly Ile Val Ile Thr Ala Gly Ile Pro Glu Lys
    450                 455                 460

Asp Glu Ala Val Ala Leu Val Lys Glu Leu Met Arg Asp Gly Phe Pro
465                 470                 475                 480

Trp Ile Ala Phe Lys Pro Gly Ala Ile Lys Gln Val Asn Ser Val Leu
                485                 490                 495

Ala Ile Ala Lys Glu Val Pro Glu Leu Pro Ile Ile Ile Gln Ile Glu
            500                 505                 510

Gly Gly Val Ala Gly Gly His His Ser Trp Glu Asp Leu Asp Glu Leu
        515                 520                 525

Leu Ile Ala Thr Tyr Gly Lys Val Arg Ala Leu Asp Asn Val Val Leu
    530                 535                 540

Cys Val Gly Gly Gly Ile Gly Ser Pro Glu Arg Ala Ala Asp Tyr Val
545                 550                 555                 560

Thr Gly Ser Trp Ser Thr Ser Tyr Gly Leu Pro Ala Met Pro Val Asp
                565                 570                 575

Gly Ile Leu Val Gly Thr Ala Ala Met Ala Thr Lys Glu Ala Thr Thr
            580                 585                 590

Ser Gln Ala Val Lys Glu Leu Leu Val Ser Thr Gln Gly Ser Asp Glu
        595                 600                 605

Trp Val Pro Ala Gly Gly Ala Lys Asn Gly Met Ala Ser Gly Arg Ser
    610                 615                 620

Gln Leu Gly Ala Asp Ile His Glu Ile Asp Asn Ser Phe Ala Lys Ala
625                 630                 635                 640

Gly Arg Leu Leu Asp Glu Val Ala Gly Asp Glu Thr Ala Val Gln Ala
                645                 650                 655

Arg Arg Asp Glu Ile Ile Glu Ala Ile Gly Lys Thr Ala Lys Val Tyr
            660                 665                 670

Phe Gly Asp Ile Gly Ser Met Thr Tyr Glu Gln Trp Leu Asn Arg Tyr
        675                 680                 685

Leu Glu Leu Ser Gly Pro Val Asp Gly Gln Trp Ile Asp Ala Ser Trp
    690                 695                 700

Ala Ala Arg Phe Ala Gln Met Leu Glu Arg Ala Glu Ala Arg Leu Ile
705                 710                 715                 720

Glu Gln Asp His Gly Gln Phe Glu Pro Ser Leu Thr Val Glu Asp Gly
                725                 730                 735

Val Asp Lys Leu Val Ala Ala Tyr Pro His Ala Ala Thr Asp Leu Leu
            740                 745                 750

Thr Pro Ala Asp Val Ala Trp Phe Leu Gly Leu Cys Arg Thr Pro Gly
        755                 760                 765

Lys Pro Val Asn Phe Val Pro Val Ile Asp Lys Asp Val Arg Arg Trp
    770                 775                 780

Trp Arg Ser Asp Ser Leu Trp Gln Ser His Asp Asp Arg Tyr Thr Ala
785                 790                 795                 800

Asp Gln Val Ala Ile Ile Pro Gly Val Val Ala Val Ala Gly Ile Thr
                805                 810                 815

Lys Ala Asn Glu Pro Val Ala Asp Leu Leu Asp Arg Phe Val Asp Ala
            820                 825                 830

Thr Ile Glu Arg Ile Asp Glu His Asp Ser Arg Ser Arg Asp Ile Met
        835                 840                 845

Gly Lys Val Leu Ser Ser Pro Gly Thr Phe Trp Ala Gly Arg Asn Ile
    850                 855                 860

Pro Ser Val Ile His Ser Leu Gly His Ala Asp Lys Trp Ser Arg Ser
865                 870                 875                 880

Glu Phe Glu Ala Phe His Ser Pro Thr Gly Ala Asn Leu Val Tyr Glu
                885                 890                 895

Asp Ala Glu His Ala Met Leu Thr Val Pro Leu Ala Gly Ser Thr Ala
            900                 905                 910

Phe Gly Thr Thr Ala Glu Leu Lys Ile Arg Phe Thr Ser Pro Ile Asp
        915                 920                 925

Ala Leu Pro Ser Ala Val Pro Leu Val Thr Gln Glu Asp Ala Glu Ala
    930                 935                 940

Ala Met Gly Glu Leu Thr Arg Ile Ala Ala Gly Gly Thr Leu Ala Thr
945                 950                 955                 960

Val Asn Asn Gly Thr Ala Thr Trp Glu Thr Ser Val Asp Ala Gly Val
                965                 970                 975

Ile Ala Asp Tyr Asn Asn Val Thr Ala Gly Tyr Leu Pro Ala Ser Val
            980                 985                 990

Val Pro Ala His Thr Ala Pro Asp Val Leu Val Gly Arg Ala Trp Pro
        995                 1000                1005

Ala Val Phe Ala Ala Val Lys Ser Ala Val Ile Pro Gly Thr Asp Ser
    1010                1015                1020

Ala Ser Val Val Glu Gly Met Leu Ser Leu Val His Leu Glu His His
1025                1030                1035                1040

Ile Val Leu Lys Ser Asp Val Pro Thr Asp Gly Ala Leu Lys Val Ser
                1045                1050                1055

Ala Thr Ala Asp Glu Val Val Asp Thr Asp Leu Gly Arg Leu Val Ile
            1060                1065                1070

Val Arg Ala Glu Ile Ala Asp Ala Glu Gly Asn Leu Ile Ala Thr Leu
        1075                1080                1085

Ala Glu Arg Phe Ala Ile Arg Gly Arg Lys Gly Asn Ala Val Ala Arg
    1090                1095                1100

Thr Asn Thr Ser Ala Leu Pro Thr Thr Val Asp Thr Pro Arg Ser Ala
1105                1110                1115                1120

Arg Ala Val Ala Thr Val Val Ala Pro Glu Ser Met Arg Pro Phe Ala
                1125                1130                1135

Val Ile Ser Gly Asp Arg Asn Pro Ile His Val Ser Asp Val Ala Ala
            1140                1145                1150

Ser Leu Ala Gly Leu Pro Gly Val Ile Val His Gly Met Trp Thr Ser
        1155                1160                1165

Ala Ile Gly Glu Leu Ile Ala Gly Ala Ala Phe Asn Asp Glu Gln Ile
    1170                1175                1180

Gln Thr Pro Ala Ala Lys Val Val Glu Tyr Thr Ala Thr Met Leu Ala
1185                1190                1195                1200

Pro Val Leu Pro Gly Glu Glu Ile Glu Phe Ser Val Glu Arg Ser Ala
                1205                1210                1215

Val Asp Asn Arg Pro Gly Met Gly Glu Val Arg Thr Val Thr Ala Thr
            1220                1225                1230

Val Asn Gly Asn Leu Val Leu Thr Ala Thr Ala Val Val Ala Ala Pro
        1235                1240                1245

Ser Thr Phe Tyr Ala Phe Pro Gly Gln Gly Ile Gln Ser Gln Gly Met
    1250                1255                1260

Gly Met Glu Ala Arg Arg Asn Ser Gln Ala Ala Arg Ala Ile Trp Asp
1265                1270                1275                1280

Arg Ala Asp Ala His Thr Arg Asn Lys Leu Gly Phe Ser Ile Val Glu
                1285                1290                1295

Ile Val Glu Asn Asn Pro Arg Glu Val Thr Val Ala Gly Glu Lys Phe
            1300                1305                1310

Phe His Pro Asp Gly Val Leu Tyr Leu Thr Gln Phe Thr Gln Val Gly
        1315                1320                1325

Met Ala Thr Leu Gly Val Ala Gln Ile Ala Glu Met Arg Glu Ala His

1330                    1335                    1340

Ala Leu Asn Gln Arg Ala Tyr Phe Ala Gly His Ser Val Gly Glu Tyr
1345                    1350                    1355                    1360

Asn Ala Leu Ala Ala Tyr Ala Gly Val Leu Ser Leu Glu Ser Val Leu
                1365                    1370                    1375

Glu Ile Val Tyr Arg Arg Gly Leu Thr Met His Arg Leu Val Asp Arg
            1380                    1385                    1390

Asp Glu Asn Gly Leu Ser Asn Tyr Ala Leu Ala Ala Leu Arg Pro Asn
        1395                    1400                    1405

Lys Met Gly Leu Thr Ala Asp Asn Val Phe Asp Tyr Val Ala Ser Val
    1410                    1415                    1420

Ser Glu Ala Ser Gly Glu Phe Leu Glu Ile Val Asn Tyr Asn Leu Ala
1425                    1430                    1435                    1440

Gly Leu Gln Tyr Ala Val Ala Gly Thr Gln Ala Gly Leu Ala Ala Leu
                1445                    1450                    1455

Arg Ala Asp Val Glu Asn Arg Ala Pro Gly Gln Arg Ala Phe Ile Leu
            1460                    1465                    1470

Ile Pro Gly Ile Asp Val Pro Phe His Ser Ser Lys Leu Arg Asp Gly
        1475                    1480                    1485

Val Gly Ala Phe Arg Glu His Leu Asp Ser Leu Ile Pro Ala Glu Leu
    1490                    1495                    1500

Asp Leu Asp Val Leu Val Gly Arg Tyr Ile Pro Asn Leu Val Ala Arg
1505                    1510                    1515                    1520

Pro Phe Glu Leu Thr Glu Glu Phe Val Ala Ser Met Ala Glu Val Val
                1525                    1530                    1535

Glu Ser Thr Tyr Val Asn Glu Ile Leu Ala Asp Phe Lys Ala Ala Ser
            1540                    1545                    1550

Ala Asp Lys Gln Lys Leu Ala Arg Thr Leu Leu Ile Glu Leu Leu Ala
        1555                    1560                    1565

Trp Gln Phe Ala Ser Pro Val Arg Trp Ile Glu Thr Gln Asp Leu Leu
    1570                    1575                    1580

Ile Lys Gly Leu Gln Ala Glu Arg Phe Val Glu Val Gly Val Gly Ser
1585                    1590                    1595                    1600

Ala Pro Thr Leu Ala Asn Met Met Gly Gln Thr Leu Arg Leu Pro Gln
                1605                    1610                    1615

Tyr Ala Asp Ala Thr Ile Glu Val Leu Asn Ile Glu Arg Asp Arg Pro
            1620                    1625                    1630

Val Val Phe Ala Thr Asp Glu Val Val Arg Glu Val Ala Val Glu Glu
        1635                    1640                    1645

Thr Pro Ala Ala Pro Ala Glu Thr Thr Glu Thr Pro Ala Thr Pro Ala
    1650                    1655                    1660

Thr Pro Ala Pro Val Ala Ala Ala Ala Pro Ala Thr Gly Gly Pro Arg
1665                1670                1675                1680

Pro Asp Asp Ile Ser Phe Thr Pro Ser Asp Ala Thr Glu Met Leu Ile
                1685                1690                1695

Ala Ile Trp Thr Lys Val Arg Pro Asp Gln Met Gly Ala Thr Asp Ser
            1700                1705                1710

Ile Glu Thr Leu Val Glu Gly Val Ser Ser Arg Arg Asn Gln Leu Leu
        1715                1720                1725

Leu Asp Leu Gly Val Glu Phe Gly Leu Gly Ala Ile Asp Gly Ala Ala
    1730                1735                1740

Asp Ala Glu Leu Gly Asp Leu Lys Val Thr Val Ser Lys Met Ala Lys
1745                1750                1755                1760

Gly Tyr Lys Ala Phe Gly Pro Val Leu Ser Asp Ala Ala Ala Asp Ala
                1765                1770                1775

Leu Arg Arg Leu Thr Gly Pro Thr Gly Lys Arg Pro Gly Tyr Ile Ala
            1780                1785                1790

Glu Arg Val Thr Gly Thr Trp Glu Leu Gly Gln Gly Trp Ala Asp His
        1795                1800                1805

Val Val Ala Glu Val Val Ile Gly Ala Arg Glu Gly Ala Ser Leu Arg
    1810                1815                1820

Gly Gly Asp Leu Ala Ser Leu Ser Pro Ala Ser Pro Ala Ser Ala Ser
1825                1830                1835                1840

Asp Leu Asp Ser Leu Ile Asp Ala Ala Val Gln Ala Val Ala Ser Arg
                1845                1850                1855

Arg Gly Val Ala Val Ser Leu Pro Ser Ala Gly Gly Ala Ala Gly Gly
            1860                1865                1870

Val Val Asp Ser Ala Ala Leu Gly Glu Phe Ala Glu Gln Val Thr Gly
        1875                1880                1885

His Asp Gly Val Leu Ala Gln Ala Ala Arg Thr Ile Leu Thr Gln Leu
    1890                1895                1900

Gly Leu Asp Lys Pro Ala Thr Val Ser Val Glu Asp Thr Ala Glu Glu
1905                1910                1915                1920

Asp Leu Tyr Glu Leu Val Ser Lys Glu Leu Gly Ser Asp Trp Pro Arg
                1925                1930                1935

Gln Val Ala Pro Ser Phe Asp Glu Glu Lys Val Val Leu Leu Asp Asp
            1940                1945                1950

Arg Trp Ala Ser Ala Arg Glu Asp Leu Ser Ala Leu Leu Leu Ala Asn
        1955                1960                1965

Ser Gln Gln Leu Ile Ser Met Ser Gln Ala Gln Ala Lys Leu Leu Gln
    1970                1975                1980

His Lys Leu Asn Ser Leu Asp Leu Met Ile Ser Gln Leu Gly Ser Arg
1985                1990                1995                2000

Thr Lys Leu Leu Gly Leu Arg Arg Gln Cys Cys Gly
                2005                2010

<210> 51
<211> 2185
<212> DNA
<213> Corynebacterium glutamicum

<220>
<221> CDS
<222> (101)..(2185)
<223> FRXA00158

<400> 51
cgtgccattt tctttacaaa agagtattaa ccgaaacttc aggtcaggat ccacgccctg 60

cgttcatccc ggctaagtta tacaaggagc gagttctcac gtg acc gaa ttg agc    115
                                           Val Thr Glu Leu Ser
                                           1               5

agg aac ttc ggg gcc agc cga ctg att aac cgc ttt ggc cag gag cct    163
Arg Asn Phe Gly Ala Ser Arg Leu Ile Asn Arg Phe Gly Gln Glu Pro
                10                  15                  20

ttt gcc ttc gct ttc gcc ggc caa gga tat gac tgg ttg aag acc ctt    211
Phe Ala Phe Ala Phe Ala Gly Gln Gly Tyr Asp Trp Leu Lys Thr Leu
            25                  30                  35

cgt gcc gcg gtt gcc gca ggt gca ggc acc aat gtt agt gac atc gtc    259
Arg Ala Ala Val Ala Ala Gly Ala Gly Thr Asn Val Ser Asp Ile Val
        40                  45                  50

gag cgc gca aat gcg ctg ctt gca cta gtt gca gat gat ctc att ggc    307
Glu Arg Ala Asn Ala Leu Leu Ala Leu Val Ala Asp Asp Leu Ile Gly
    55                  60                  65

acc ctt cca ttt ggt ttc gat cca gtg gct tgg gct aac aac tcc gaa    355
Thr Leu Pro Phe Gly Phe Asp Pro Val Ala Trp Ala Asn Asn Ser Glu
70                  75                  80                  85

gat cca gct ttc gat act gca caa tct gca gtg agc gtg ccg ggt atc    403
Asp Pro Ala Phe Asp Thr Ala Gln Ser Ala Val Ser Val Pro Gly Ile
                90                  95                  100

ttt gtc tcc cag atc gca acc ctg gat tcc ctt gag gcg cag cgc ctt    451
Phe Val Ser Gln Ile Ala Thr Leu Asp Ser Leu Glu Ala Gln Arg Leu
            105                 110                 115

gat gtg gat cag gct gtg tcc agc att ggt cat tcc cag ggc gta ttg    499
Asp Val Asp Gln Ala Val Ser Ser Ile Gly His Ser Gln Gly Val Leu
        120                 125                 130

ggc gtg cac ctg ctc aat gat gcg act cgt gct gat gaa ctc gtt gcc    547
Gly Val His Leu Leu Asn Asp Ala Thr Arg Ala Asp Glu Leu Val Ala
    135                 140                 145

att gcg cag ttg atc ggt gca gcg atc acc cgc acc gca cgc atg acg    595

Ile Ala Gln Leu Ile Gly Ala Ala Ile Thr Arg Thr Ala Arg Met Thr
150                 155                 160                 165

ggc ctg atc gcg cag ggc gac aac atg ccg atg ctg tcg atc gcc gga     643
Gly Leu Ile Ala Gln Gly Asp Asn Met Pro Met Leu Ser Ile Ala Gly
                170                 175                 180

att tcc cgc gaa cag ctt cag caa gct atc gac gcg gcc tgc gcc gaa     691
Ile Ser Arg Glu Gln Leu Gln Gln Ala Ile Asp Ala Ala Cys Ala Glu
            185                 190                 195

gtc cct gcg gag atc cgc ccg gtt atc ggt ctg cgc aac tca cgc gat     739
Val Pro Ala Glu Ile Arg Pro Val Ile Gly Leu Arg Asn Ser Arg Asp
        200                 205                 210

tct tat gtt ttg gtt ggc cgc cca gac gac aac gct cgc gtt gtt aag     787
Ser Tyr Val Leu Val Gly Arg Pro Asp Asp Asn Ala Arg Val Val Lys
    215                 220                 225

gtc att gag gca atg gct gcc aag gat aag aag gcc att gaa gat aag     835
Val Ile Glu Ala Met Ala Ala Lys Asp Lys Lys Ala Ile Glu Asp Lys
230                 235                 240                 245

ctg cgc ggc ggt tcc gcg ttc agc ccc cgt att act ccg ctg aag gtg     883
Leu Arg Gly Gly Ser Ala Phe Ser Pro Arg Ile Thr Pro Leu Lys Val
                250                 255                 260

cag gct gct ttc cat cac cca gct atg aac atg gct gtg gag cag acc     931
Gln Ala Ala Phe His His Pro Ala Met Asn Met Ala Val Glu Gln Thr
            265                 270                 275

gtg gcg tgg gca acc act gct ggt ttg gat gtg gaa ctc acc cgc gag     979
Val Ala Trp Ala Thr Thr Ala Gly Leu Asp Val Glu Leu Thr Arg Glu
        280                 285                 290

atc gcc gct gat gtt ttg gtt aac cct gtc gat tgg gta gca cgc gtc     1027
Ile Ala Ala Asp Val Leu Val Asn Pro Val Asp Trp Val Ala Arg Val
    295                 300                 305

aac gaa gcg tat gag gct ggc gct cgc tgg ttc ctc gac gtt gga cca     1075
Asn Glu Ala Tyr Glu Ala Gly Ala Arg Trp Phe Leu Asp Val Gly Pro
310                 315                 320                 325

gat ggt ggc atc gtt aag ctg act gcc aac atc ctt gag ggc cgc ggc     1123
Asp Gly Gly Ile Val Lys Leu Thr Ala Asn Ile Leu Glu Gly Arg Gly
                330                 335                 340

gcg gat tcc ttc tat gtt ggt gac gcc gca ggc cag gcc aag ata ttt     1171
Ala Asp Ser Phe Tyr Val Gly Asp Ala Ala Gly Gln Ala Lys Ile Phe
            345                 350                 355

gat gct ggc atg gca cct gaa ctt cca gtg gat tac cag gag ttc gca     1219
Asp Ala Gly Met Ala Pro Glu Leu Pro Val Asp Tyr Gln Glu Phe Ala
        360                 365                 370

cca cgc gtt gag cac gtt gat gga acc cca cgc ctg gtt acc aag ttc     1267
Pro Arg Val Glu His Val Asp Gly Thr Pro Arg Leu Val Thr Lys Phe
    375                 380                 385

act gag ctg acc ggc cgc acc cca atg atg ctg gct ggc atg acc cca     1315
Thr Glu Leu Thr Gly Arg Thr Pro Met Met Leu Ala Gly Met Thr Pro

390                 395                 400                 405

acc acc gtt gac cct gcc att gtt gca gcc gct gca aac ggt gga cac     1363
Thr Thr Val Asp Pro Ala Ile Val Ala Ala Ala Ala Asn Gly Gly His
                410                 415                 420

tgg gct gag ctc gct ggt ggc gga cag gtt acc cca gag ctg ctg gaa     1411
Trp Ala Glu Leu Ala Gly Gly Gly Gln Val Thr Pro Glu Leu Leu Glu
            425                 430                 435

acc cac atc gca cag ctc acc gac atg ctt gag cca ggt atc aac gcc     1459
Thr His Ile Ala Gln Leu Thr Asp Met Leu Glu Pro Gly Ile Asn Ala
        440                 445                 450

cag ttc aac tcc atg ttc ttg gat cca tac ctg tgg aag atg cag att     1507
Gln Phe Asn Ser Met Phe Leu Asp Pro Tyr Leu Trp Lys Met Gln Ile
    455                 460                 465

ggt ggc aag cgc ctt gtt cct aag gcc cgc gct aat ggt gca tcc atc     1555
Gly Gly Lys Arg Leu Val Pro Lys Ala Arg Ala Asn Gly Ala Ser Ile
470                 475                 480                 485

gac ggc atc gtc atc acc gcc ggc att cct gaa aag gat gaa gct gtt     1603
Asp Gly Ile Val Ile Thr Ala Gly Ile Pro Glu Lys Asp Glu Ala Val
                490                 495                 500

gca ttg gtc aag gaa ctg atg cgt gat ggt ttc cct tgg atc gca ttc     1651
Ala Leu Val Lys Glu Leu Met Arg Asp Gly Phe Pro Trp Ile Ala Phe
            505                 510                 515

aag cca ggt gcc atc aag cag gtt aac tct gtg ttg gct atc gct aag     1699
Lys Pro Gly Ala Ile Lys Gln Val Asn Ser Val Leu Ala Ile Ala Lys
        520                 525                 530

gaa gtt cca gaa ctc ccc atc atc att cag att gag ggt ggc gtt gca     1747
Glu Val Pro Glu Leu Pro Ile Ile Ile Gln Ile Glu Gly Gly Val Ala
    535                 540                 545

ggt gga cac cac tct tgg gaa gac ctc gat gag ctg ctg atc gcc acc     1795
Gly Gly His His Ser Trp Glu Asp Leu Asp Glu Leu Leu Ile Ala Thr
550                 555                 560                 565

tac ggc aag gtc cgc gca ctg gat aac gtg gtg ctg tgt gtc ggc ggt     1843
Tyr Gly Lys Val Arg Ala Leu Asp Asn Val Val Leu Cys Val Gly Gly
                570                 575                 580

ggc att ggc tca cct gag cgc gct gct gat tac gtc acc ggt tcc tgg     1891
Gly Ile Gly Ser Pro Glu Arg Ala Ala Asp Tyr Val Thr Gly Ser Trp
            585                 590                 595

tcc act tcc tac ggc ctg cca gct atg cct gtt gat ggc atc ttg gtg     1939
Ser Thr Ser Tyr Gly Leu Pro Ala Met Pro Val Asp Gly Ile Leu Val
        600                 605                 610

ggt acc gct gcg atg gca acc aag gaa gca acc acc tcc cag gcc gtc     1987
Gly Thr Ala Ala Met Ala Thr Lys Glu Ala Thr Thr Ser Gln Ala Val
    615                 620                 625

aag gaa ctt ctt gtt tcc acc cag ggc tct gat gaa tgg gtt cct gct     2035
Lys Glu Leu Leu Val Ser Thr Gln Gly Ser Asp Glu Trp Val Pro Ala
630                 635                 640                 645

ggt ggc gca aag aac gga atg gca tat ggc cgt tcc cag ctt ggc gca 2083
Gly Gly Ala Lys Asn Gly Met Ala Tyr Gly Arg Ser Gln Leu Gly Ala
                650                 655                 660

gac atc cac gag atc gac aac tcc ttt gct aag gct gga cgc ctt ctt 2131
Asp Ile His Glu Ile Asp Asn Ser Phe Ala Lys Ala Gly Arg Leu Leu
            665                 670                 675

gat gag gtt gca ggc cat gaa acg gat ttg caa gcg cgc ccg gat gag 2179
Asp Glu Val Ala Gly His Glu Thr Asp Leu Gln Ala Arg Pro Asp Glu
        680                 685                 690

atc att 2185
Ile Ile
    695

<210> 52
<211> 695
<212> PRT
<213> Corynebacterium glutamicum

<400> 52
Val Thr Glu Leu Ser Arg Asn Phe Gly Ala Ser Arg Leu Ile Asn Arg
1               5                   10                  15

Phe Gly Gln Glu Pro Phe Ala Phe Ala Phe Ala Gly Gln Gly Tyr Asp
            20                  25                  30

Trp Leu Lys Thr Leu Arg Ala Ala Val Ala Ala Gly Ala Gly Thr Asn
        35                  40                  45

Val Ser Asp Ile Val Glu Arg Ala Asn Ala Leu Leu Ala Leu Val Ala
    50                  55                  60

Asp Asp Leu Ile Gly Thr Leu Pro Phe Gly Phe Asp Pro Val Ala Trp
65                  70                  75                  80

Ala Asn Asn Ser Glu Asp Pro Ala Phe Asp Thr Ala Gln Ser Ala Val
                85                  90                  95

Ser Val Pro Gly Ile Phe Val Ser Gln Ile Ala Thr Leu Asp Ser Leu
            100                 105                 110

Glu Ala Gln Arg Leu Asp Val Asp Gln Ala Val Ser Ser Ile Gly His
        115                 120                 125

Ser Gln Gly Val Leu Gly Val His Leu Leu Asn Asp Ala Thr Arg Ala
    130                 135                 140

Asp Glu Leu Val Ala Ile Ala Gln Leu Ile Gly Ala Ala Ile Thr Arg
145                 150                 155                 160

Thr Ala Arg Met Thr Gly Leu Ile Ala Gln Gly Asp Asn Met Pro Met
                165                 170                 175

Leu Ser Ile Ala Gly Ile Ser Arg Glu Gln Leu Gln Gln Ala Ile Asp
            180                 185                 190

Ala Ala Cys Ala Glu Val Pro Ala Glu Ile Arg Pro Val Ile Gly Leu

        195                 200                 205

Arg Asn Ser Arg Asp Ser Tyr Val Leu Val Gly Arg Pro Asp Asp Asn
    210                 215                 220

Ala Arg Val Val Lys Val Ile Glu Ala Met Ala Ala Lys Asp Lys Lys
225                 230                 235                 240

Ala Ile Glu Asp Lys Leu Arg Gly Gly Ser Ala Phe Ser Pro Arg Ile
                245                 250                 255

Thr Pro Leu Lys Val Gln Ala Ala Phe His His Pro Ala Met Asn Met
            260                 265                 270

Ala Val Glu Gln Thr Val Ala Trp Ala Thr Thr Ala Gly Leu Asp Val
        275                 280                 285

Glu Leu Thr Arg Glu Ile Ala Ala Asp Val Leu Val Asn Pro Val Asp
    290                 295                 300

Trp Val Ala Arg Val Asn Glu Ala Tyr Glu Ala Gly Ala Arg Trp Phe
305                 310                 315                 320

Leu Asp Val Gly Pro Asp Gly Gly Ile Val Lys Leu Thr Ala Asn Ile
                325                 330                 335

Leu Glu Gly Arg Gly Ala Asp Ser Phe Tyr Val Gly Asp Ala Ala Gly
            340                 345                 350

Gln Ala Lys Ile Phe Asp Ala Gly Met Ala Pro Glu Leu Pro Val Asp
        355                 360                 365

Tyr Gln Glu Phe Ala Pro Arg Val Glu His Val Asp Gly Thr Pro Arg
    370                 375                 380

Leu Val Thr Lys Phe Thr Glu Leu Thr Gly Arg Thr Pro Met Met Leu
385                 390                 395                 400

Ala Gly Met Thr Pro Thr Thr Val Asp Pro Ala Ile Val Ala Ala Ala
                405                 410                 415

Ala Asn Gly Gly His Trp Ala Glu Leu Ala Gly Gly Gly Gln Val Thr
            420                 425                 430

Pro Glu Leu Leu Glu Thr His Ile Ala Gln Leu Thr Asp Met Leu Glu
        435                 440                 445

Pro Gly Ile Asn Ala Gln Phe Asn Ser Met Phe Leu Asp Pro Tyr Leu
    450                 455                 460

Trp Lys Met Gln Ile Gly Gly Lys Arg Leu Val Pro Lys Ala Arg Ala
465                 470                 475                 480

Asn Gly Ala Ser Ile Asp Gly Ile Val Ile Thr Ala Gly Ile Pro Glu
                485                 490                 495

Lys Asp Glu Ala Val Ala Leu Val Lys Glu Leu Met Arg Asp Gly Phe
            500                 505                 510

Pro Trp Ile Ala Phe Lys Pro Gly Ala Ile Lys Gln Val Asn Ser Val
        515                 520                 525

Leu Ala Ile Ala Lys Glu Val Pro Glu Leu Pro Ile Ile Ile Gln Ile
    530                 535                 540

Glu Gly Gly Val Ala Gly Gly His His Ser Trp Glu Asp Leu Asp Glu
545                 550                 555                 560

Leu Leu Ile Ala Thr Tyr Gly Lys Val Arg Ala Leu Asp Asn Val Val
                565                 570                 575

Leu Cys Val Gly Gly Gly Ile Gly Ser Pro Glu Arg Ala Ala Asp Tyr
            580                 585                 590

Val Thr Gly Ser Trp Ser Thr Ser Tyr Gly Leu Pro Ala Met Pro Val
        595                 600                 605

Asp Gly Ile Leu Val Gly Thr Ala Ala Met Ala Thr Lys Glu Ala Thr
    610                 615                 620

Thr Ser Gln Ala Val Lys Glu Leu Leu Val Ser Thr Gln Gly Ser Asp
625                 630                 635                 640

Glu Trp Val Pro Ala Gly Gly Ala Lys Asn Gly Met Ala Tyr Gly Arg
                645                 650                 655

Ser Gln Leu Gly Ala Asp Ile His Glu Ile Asp Asn Ser Phe Ala Lys
            660                 665                 670

Ala Gly Arg Leu Leu Asp Glu Val Ala Gly His Glu Thr Asp Leu Gln
        675                 680                 685

Ala Arg Pro Asp Glu Ile Ile
    690                 695


<210> 53
<211> 3831
<212> DNA
<213> Corynebacterium glutamicum

<220>
<221> CDS
<222> (1)..(3831)
<223> FRXA00572

<400> 53
cag tgg ctc aac cgc tac ctc gag ctg tct ggc cct gtt gat ggt cag      48
Gln Trp Leu Asn Arg Tyr Leu Glu Leu Ser Gly Pro Val Asp Gly Gln
  1               5                  10                  15

tgg att gat gct tcc tgg gct gca cgt ttt gcc cag atg ctg gag cgt      96
Trp Ile Asp Ala Ser Trp Ala Ala Arg Phe Ala Gln Met Leu Glu Arg
             20                  25                  30

gcc gag gcg cgt ttg atc gag cag gat cat ggc caa ttt gag cca agc     144
Ala Glu Ala Arg Leu Ile Glu Gln Asp His Gly Gln Phe Glu Pro Ser
         35                  40                  45

ctg acg gtg gag gat ggc gtc gac aag ctt gtt gct gct tac ccg cat     192
Leu Thr Val Glu Asp Gly Val Asp Lys Leu Val Ala Ala Tyr Pro His
     50                  55                  60

gcc gca acc gac ctg ctc acc ccg gct gat gtc gcc tgg ttc ttg ggc 240
Ala Ala Thr Asp Leu Leu Thr Pro Ala Asp Val Ala Trp Phe Leu Gly
65                  70                  75                  80

ctg tgc cgc acg ccg ggc aag cct gtg aac ttt gtg ccc gtc att gat 288
Leu Cys Arg Thr Pro Gly Lys Pro Val Asn Phe Val Pro Val Ile Asp
                85                  90                  95

aag gac gtg cgt cgc tgg tgg cgc tcg gac tcc ctg tgg cag tcc cac 336
Lys Asp Val Arg Arg Trp Trp Arg Ser Asp Ser Leu Trp Gln Ser His
            100                 105                 110

gat gat cgc tac acc gct gat cag gtg gct att atc cct ggt gtc gtc 384
Asp Asp Arg Tyr Thr Ala Asp Gln Val Ala Ile Ile Pro Gly Val Val
        115                 120                 125

gcc gtt gct ggc atc acc aag gcc aac gaa cct gtc gct gac ctg ctt 432
Ala Val Ala Gly Ile Thr Lys Ala Asn Glu Pro Val Ala Asp Leu Leu
    130                 135                 140

gat cgc ttt gtc gac gcc acc atc gag cgc atc gat gag cac gat tcc 480
Asp Arg Phe Val Asp Ala Thr Ile Glu Arg Ile Asp Glu His Asp Ser
145                 150                 155                 160

cgc tcc cgc gac atc atg ggc aaa gtg ctt tcc tca cct ggc aca ttc 528
Arg Ser Arg Asp Ile Met Gly Lys Val Leu Ser Ser Pro Gly Thr Phe
                165                 170                 175

tgg gct ggc cgc aac atc cca tcg gtg atc cac agc ctt ggg cat gct 576
Trp Ala Gly Arg Asn Ile Pro Ser Val Ile His Ser Leu Gly His Ala
            180                 185                 190

gac aag tgg tcc cgc tcc gaa ttc gaa gca ttc cat agc cca acc ggc 624
Asp Lys Trp Ser Arg Ser Glu Phe Glu Ala Phe His Ser Pro Thr Gly
        195                 200                 205

gcc aac ttg gtg tac gaa gac gcc gag cac gcg atg ctg act gtg cct 672
Ala Asn Leu Val Tyr Glu Asp Ala Glu His Ala Met Leu Thr Val Pro
    210                 215                 220

ttg gcg ggt tcc acc gca ttc ggc acc acc gct gag ctg aaa atc cgt 720
Leu Ala Gly Ser Thr Ala Phe Gly Thr Thr Ala Glu Leu Lys Ile Arg
225                 230                 235                 240

ttc acc agc ccc atc gac gct ctg cca agc gct gtc cca ctg gtc acc 768
Phe Thr Ser Pro Ile Asp Ala Leu Pro Ser Ala Val Pro Leu Val Thr
                245                 250                 255

cag gaa gac gct gaa gcc gcg atg ggt gaa ctg acc cgc atc gca gct 816
Gln Glu Asp Ala Glu Ala Ala Met Gly Glu Leu Thr Arg Ile Ala Ala
            260                 265                 270

ggc ggc acc ctg gca act gtg aac aat ggc acc gct acc tgg gaa acc 864
Gly Gly Thr Leu Ala Thr Val Asn Asn Gly Thr Ala Thr Trp Glu Thr
        275                 280                 285

tcc gtc gat gcc ggc gtc atc gct gac tac aac aac gtc acc gca ggc 912
Ser Val Asp Ala Gly Val Ile Ala Asp Tyr Asn Asn Val Thr Ala Gly
    290                 295                 300

tac ctg cca gca tcc gtt gtt cct gca cac acc gca cct gac gtg ctg 960
Tyr Leu Pro Ala Ser Val Val Pro Ala His Thr Ala Pro Asp Val Leu
305                 310                 315                 320

gtt ggc cgc gca tgg cca gca gtt ttc gct gcc gta aag tcc gca gtc 1008
Val Gly Arg Ala Trp Pro Ala Val Phe Ala Ala Val Lys Ser Ala Val
                325                 330                 335

atc cca ggc acc gat tcc gca tcc gtt gtg gaa ggc atg ctg tcc ctg 1056
Ile Pro Gly Thr Asp Ser Ala Ser Val Val Glu Gly Met Leu Ser Leu
            340                 345                 350

gtt cac ctg gag cac cac att gtg ctc aag tcc gat gtc cca acc gac 1104
Val His Leu Glu His His Ile Val Leu Lys Ser Asp Val Pro Thr Asp
        355                 360                 365

ggc gcg ctg aag gtt tcc gcg act gcc gat gag gta gtc gat acc gac 1152
Gly Ala Leu Lys Val Ser Ala Thr Ala Asp Glu Val Val Asp Thr Asp
    370                 375                 380

ctg ggt cgc ctc gtg atc gtg cgc gca gaa atc gcc gac gca gaa ggc 1200
Leu Gly Arg Leu Val Ile Val Arg Ala Glu Ile Ala Asp Ala Glu Gly
385                 390                 395                 400

aac ctg att gct acg ttg gct gag cgt ttc gcg atc cgc gga cgc aag 1248
Asn Leu Ile Ala Thr Leu Ala Glu Arg Phe Ala Ile Arg Gly Arg Lys
                405                 410                 415

ggc aac gct gtc gca cgc acc aac acc tcc gca ctg cca acc acc gtg 1296
Gly Asn Ala Val Ala Arg Thr Asn Thr Ser Ala Leu Pro Thr Thr Val
            420                 425                 430

gac acc cca cgc tca gct cgc gca gtg gca acc gtt gtt gca cct gaa 1344
Asp Thr Pro Arg Ser Ala Arg Ala Val Ala Thr Val Val Ala Pro Glu
        435                 440                 445

tcc atg cgc cca ttc gct gtg atc tcc ggt gac cgc aac cca att cac 1392
Ser Met Arg Pro Phe Ala Val Ile Ser Gly Asp Arg Asn Pro Ile His
    450                 455                 460

gtc tct gat gtt gcg gct tcc ctg gct ggt ctg cca ggt gtg atc gtg 1440
Val Ser Asp Val Ala Ala Ser Leu Ala Gly Leu Pro Gly Val Ile Val
465                 470                 475                 480

cac ggc atg tgg acc tct gcc atc ggt gaa ctg atc gcc ggt gca gca 1488
His Gly Met Trp Thr Ser Ala Ile Gly Glu Leu Ile Ala Gly Ala Ala
                485                 490                 495

ttc aac gat gag cag atc caa act ccc gca gcc aag gtc gtg gaa tac 1536
Phe Asn Asp Glu Gln Ile Gln Thr Pro Ala Ala Lys Val Val Glu Tyr
            500                 505                 510

acc gca acc atg ctg gca cca gtt ctt cca ggt gaa gaa att gag ttc 1584
Thr Ala Thr Met Leu Ala Pro Val Leu Pro Gly Glu Glu Ile Glu Phe
        515                 520                 525

agc gtt gag cgc tcc gca gtg gac aac cgc cca gga atg gga gag gtc 1632
Ser Val Glu Arg Ser Ala Val Asp Asn Arg Pro Gly Met Gly Glu Val
    530                 535                 540

cgc acc gtt acc gca acc gtc aac ggc aac tta gtg ctt acc gcc acc 1680

Arg Thr Val Thr Ala Thr Val Asn Gly Asn Leu Val Leu Thr Ala Thr
545                 550                 555                 560

gct gtt gtg gca gct cca tct act ttc tac gca ttc cca ggc cag ggc     1728
Ala Val Val Ala Ala Pro Ser Thr Phe Tyr Ala Phe Pro Gly Gln Gly
                565                 570                 575

att cag tcc cag ggc atg ggt atg gaa gca cgc cgt aac tct cag gca     1776
Ile Gln Ser Gln Gly Met Gly Met Glu Ala Arg Arg Asn Ser Gln Ala
            580                 585                 590

gct cgc gct atc tgg gac cgc gcc gat gca cac acc cgc aat aag ctg     1824
Ala Arg Ala Ile Trp Asp Arg Ala Asp Ala His Thr Arg Asn Lys Leu
        595                 600                 605

ggc ttc tcc atc gtg gaa atc gtg gaa aac aac cca cgc gaa gta acc     1872
Gly Phe Ser Ile Val Glu Ile Val Glu Asn Asn Pro Arg Glu Val Thr
    610                 615                 620

gtg gca ggg gag aag ttc ttc cac cca gac ggc gtt ttg tac ctc acc     1920
Val Ala Gly Glu Lys Phe Phe His Pro Asp Gly Val Leu Tyr Leu Thr
625                 630                 635                 640

cag ttc acc cag gtg ggc atg gca act ctg ggc gtt gct cag atc gct     1968
Gln Phe Thr Gln Val Gly Met Ala Thr Leu Gly Val Ala Gln Ile Ala
                645                 650                 655

gaa atg cgt gaa gca cat gcc ttg aac cag cgt gca tac ttt gct gga     2016
Glu Met Arg Glu Ala His Ala Leu Asn Gln Arg Ala Tyr Phe Ala Gly
            660                 665                 670

cac tcc gtt ggt gag tac aac gcg ctt gct gca tat gct ggt gtg ctg     2064
His Ser Val Gly Glu Tyr Asn Ala Leu Ala Ala Tyr Ala Gly Val Leu
        675                 680                 685

tcc ctg gaa tcc gtt ctg gag atc gtt tac cgt cgt ggc ttg acc atg     2112
Ser Leu Glu Ser Val Leu Glu Ile Val Tyr Arg Arg Gly Leu Thr Met
    690                 695                 700

cac cgc ttg gtg gat cgc gat gaa aac ggt ctg tcc aac tac gcg ctc     2160
His Arg Leu Val Asp Arg Asp Glu Asn Gly Leu Ser Asn Tyr Ala Leu
705                 710                 715                 720

gca gct ctt cgc ccc aac aag atg ggt ctg acc gca gac aac gtt ttc     2208
Ala Ala Leu Arg Pro Asn Lys Met Gly Leu Thr Ala Asp Asn Val Phe
                725                 730                 735

gat tac gtt gcg tct gtt tcc gaa gct tcc ggt gaa ttc ctg gag atc     2256
Asp Tyr Val Ala Ser Val Ser Glu Ala Ser Gly Glu Phe Leu Glu Ile
            740                 745                 750

gtt aac tac aac ttg gct ggc ctg cag tac gca gtt gct gga acc cag     2304
Val Asn Tyr Asn Leu Ala Gly Leu Gln Tyr Ala Val Ala Gly Thr Gln
        755                 760                 765

gct ggt ctt gcc gcc ctt cgt gcc gat gtt gag aac cgt gca cca ggt     2352
Ala Gly Leu Ala Ala Leu Arg Ala Asp Val Glu Asn Arg Ala Pro Gly
    770                 775                 780

cag cgt gcc ttc att ttg atc cct ggc att gac gtg cca ttc cac tcc     2400
Gln Arg Ala Phe Ile Leu Ile Pro Gly Ile Asp Val Pro Phe His Ser

785                 790                 795                 800

tcc aag ctg cgc gac ggt gtg ggc gcg ttc cgt gag cac ctt gat tcc     2448
Ser Lys Leu Arg Asp Gly Val Gly Ala Phe Arg Glu His Leu Asp Ser
                805                 810                 815

ctg atc cca gct gag ctg gat ctg gat gtg ctg gtt ggc cgc tac att     2496
Leu Ile Pro Ala Glu Leu Asp Leu Asp Val Leu Val Gly Arg Tyr Ile
            820                 825                 830

cca aac ttg gtg gct cgc cca ttc gaa ctc act gaa gag ttc gtg gca     2544
Pro Asn Leu Val Ala Arg Pro Phe Glu Leu Thr Glu Glu Phe Val Ala
        835                 840                 845

tcc atg gca gaa gtg gtg gag tcc acc tat gtc aat gag atc ttg gct     2592
Ser Met Ala Glu Val Val Glu Ser Thr Tyr Val Asn Glu Ile Leu Ala
    850                 855                 860

gat ttc aag gct gct tcc gcc gat aag cag aag ctt gcc cgc acg ttg     2640
Asp Phe Lys Ala Ala Ser Ala Asp Lys Gln Lys Leu Ala Arg Thr Leu
865                 870                 875                 880

ctt att gag ctg ctt gca tgg cag ttc gca tca cct gtg cgc tgg atc     2688
Leu Ile Glu Leu Leu Ala Trp Gln Phe Ala Ser Pro Val Arg Trp Ile
                885                 890                 895

gag act cag gat ctg ttg atc aag ggc ctt caa gct gag cgt ttc gtg     2736
Glu Thr Gln Asp Leu Leu Ile Lys Gly Leu Gln Ala Glu Arg Phe Val
            900                 905                 910

gag gtc ggt gtt ggc tct gct cca acg ctt gcc aac atg atg ggc cag     2784
Glu Val Gly Val Gly Ser Ala Pro Thr Leu Ala Asn Met Met Gly Gln
        915                 920                 925

acc ctg cgc ctt cct cag tac gcg gac gcc acc att gag gtg tta aac     2832
Thr Leu Arg Leu Pro Gln Tyr Ala Asp Ala Thr Ile Glu Val Leu Asn
    930                 935                 940

att gag cgc gat cgc cca gtt gtg ttc gct acc gat gag gtt gtg cgt     2880
Ile Glu Arg Asp Arg Pro Val Val Phe Ala Thr Asp Glu Val Val Arg
945                 950                 955                 960

gaa gtg gcg gtt gaa gag acc cca gca gct cct gca gaa acc act gaa     2928
Glu Val Ala Val Glu Glu Thr Pro Ala Ala Pro Ala Glu Thr Thr Glu
                965                 970                 975

acc cca gca acc cca gca acc cca gcc cct gtt gca gct gca gcc cct     2976
Thr Pro Ala Thr Pro Ala Thr Pro Ala Pro Val Ala Ala Ala Ala Pro
            980                 985                 990

gcc acc ggc ggc cct cgc cca gat gac atc agc ttc act cct tct gat     3024
Ala Thr Gly Gly Pro Arg Pro Asp Asp Ile Ser Phe Thr Pro Ser Asp
        995                 1000                1005

gcc act gaa atg ctc atc gct atc tgg acc aag gtt cgc cca gat cag     3072
Ala Thr Glu Met Leu Ile Ala Ile Trp Thr Lys Val Arg Pro Asp Gln
    1010                1015                1020

atg ggt gcc act gat tcc atc gag acc ctg gtt gag ggc gtg tcc tct     3120
Met Gly Ala Thr Asp Ser Ile Glu Thr Leu Val Glu Gly Val Ser Ser
1025                1030                1035                1040

cgc cgt aac cag ctc ctg ctg gat ctt ggt gtg gag ttc ggc ctc ggc 3168
Arg Arg Asn Gln Leu Leu Leu Asp Leu Gly Val Glu Phe Gly Leu Gly
                1045                1050                1055

gca att gac gga gca gcc gat gct gag ctc ggt gat cta aag gtc acc 3216
Ala Ile Asp Gly Ala Ala Asp Ala Glu Leu Gly Asp Leu Lys Val Thr
            1060                1065                1070

gtg tcc aag atg gct aag ggc tac aag gcg ttt ggc cct gtg ctc tcc 3264
Val Ser Lys Met Ala Lys Gly Tyr Lys Ala Phe Gly Pro Val Leu Ser
        1075                1080                1085

gat gct gca gct gat gcc ctg cgt cgc ctc act ggt cct acc ggt aag 3312
Asp Ala Ala Ala Asp Ala Leu Arg Arg Leu Thr Gly Pro Thr Gly Lys
    1090                1095                1100

cgc ccg gga tac atc gca gag cgc gtc acc ggc acg tgg gaa ttg ggc 3360
Arg Pro Gly Tyr Ile Ala Glu Arg Val Thr Gly Thr Trp Glu Leu Gly
1105                1110                1115                1120

cag ggc tgg gct gac cac gtg gtc gct gaa gtt gtg atc ggc gcc cgc 3408
Gln Gly Trp Ala Asp His Val Val Ala Glu Val Val Ile Gly Ala Arg
                1125                1130                1135

gaa ggc gca tcc ctg cgc ggc ggc gac ctg gcg tca ctg tct cct gca 3456
Glu Gly Ala Ser Leu Arg Gly Gly Asp Leu Ala Ser Leu Ser Pro Ala
            1140                1145                1150

agc cca gcg tct gca tca gat ctt gat tcg ctt atc gac gca gcc gtc 3504
Ser Pro Ala Ser Ala Ser Asp Leu Asp Ser Leu Ile Asp Ala Ala Val
        1155                1160                1165

cag gcc gta gcc tcc cgc cgc ggc gtt gcg gtc tcc ctg cct tca gca 3552
Gln Ala Val Ala Ser Arg Arg Gly Val Ala Val Ser Leu Pro Ser Ala
    1170                1175                1180

ggc ggc gct gcc ggt ggc gtg gtt gat tcc gca gct ctt ggc gag ttt 3600
Gly Gly Ala Ala Gly Gly Val Val Asp Ser Ala Ala Leu Gly Glu Phe
1185                1190                1195                1200

gca gag cag gtc acc gga cac gat ggt gtg ctt gct cag gca gcc cgc 3648
Ala Glu Gln Val Thr Gly His Asp Gly Val Leu Ala Gln Ala Ala Arg
                1205                1210                1215

acc atc ttg acc cag ttg ggt ctt gat aag cca gca acc gtt tcc gtg 3696
Thr Ile Leu Thr Gln Leu Gly Leu Asp Lys Pro Ala Thr Val Ser Val
            1220                1225                1230

gaa gac acc gca gag gaa gac ctc tac gag ttg gtc tcc aag gaa ctc 3744
Glu Asp Thr Ala Glu Glu Asp Leu Tyr Glu Leu Val Ser Lys Glu Leu
        1235                1240                1245

ggt tct gat tgg cca cgt cag gtt gca cca agc ttc gat gaa gaa aag 3792
Gly Ser Asp Trp Pro Arg Gln Val Ala Pro Ser Phe Asp Glu Glu Lys
    1250                1255                1260

gtt gtt ctg ctt gat gac cgt tgg gct tct gcg cgt gag 3831
Val Val Leu Leu Asp Asp Arg Trp Ala Ser Ala Arg Glu
1265                1270                1275

<210> 54
<211> 1277
<212> PRT
<213> Corynebacterium glutamicum

<400> 54
Gln Trp Leu Asn Arg Tyr Leu Glu Leu Ser Gly Pro Val Asp Gly Gln
1               5                   10                  15

Trp Ile Asp Ala Ser Trp Ala Ala Arg Phe Ala Gln Met Leu Glu Arg
            20                  25                  30

Ala Glu Ala Arg Leu Ile Glu Gln Asp His Gly Gln Phe Glu Pro Ser
        35                  40                  45

Leu Thr Val Glu Asp Gly Val Asp Lys Leu Val Ala Ala Tyr Pro His
    50                  55                  60

Ala Ala Thr Asp Leu Leu Thr Pro Ala Asp Val Ala Trp Phe Leu Gly
65                  70                  75                  80

Leu Cys Arg Thr Pro Gly Lys Pro Val Asn Phe Val Pro Val Ile Asp
                85                  90                  95

Lys Asp Val Arg Arg Trp Trp Arg Ser Asp Ser Leu Trp Gln Ser His
            100                 105                 110

Asp Asp Arg Tyr Thr Ala Asp Gln Val Ala Ile Ile Pro Gly Val Val
        115                 120                 125

Ala Val Ala Gly Ile Thr Lys Ala Asn Glu Pro Val Ala Asp Leu Leu
    130                 135                 140

Asp Arg Phe Val Asp Ala Thr Ile Glu Arg Ile Asp Glu His Asp Ser
145                 150                 155                 160

Arg Ser Arg Asp Ile Met Gly Lys Val Leu Ser Ser Pro Gly Thr Phe
                165                 170                 175

Trp Ala Gly Arg Asn Ile Pro Ser Val Ile His Ser Leu Gly His Ala
            180                 185                 190

Asp Lys Trp Ser Arg Ser Glu Phe Glu Ala Phe His Ser Pro Thr Gly
        195                 200                 205

Ala Asn Leu Val Tyr Glu Asp Ala Glu His Ala Met Leu Thr Val Pro
    210                 215                 220

Leu Ala Gly Ser Thr Ala Phe Gly Thr Thr Ala Glu Leu Lys Ile Arg
225                 230                 235                 240

Phe Thr Ser Pro Ile Asp Ala Leu Pro Ser Ala Val Pro Leu Val Thr
                245                 250                 255

Gln Glu Asp Ala Glu Ala Ala Met Gly Glu Leu Thr Arg Ile Ala Ala
            260                 265                 270

Gly Gly Thr Leu Ala Thr Val Asn Asn Gly Thr Ala Thr Trp Glu Thr
        275                 280                 285

Ser Val Asp Ala Gly Val Ile Ala Asp Tyr Asn Asn Val Thr Ala Gly
    290                 295                 300

Tyr Leu Pro Ala Ser Val Val Pro Ala His Thr Ala Pro Asp Val Leu
305                 310                 315                 320

Val Gly Arg Ala Trp Pro Ala Val Phe Ala Ala Val Lys Ser Ala Val
                325                 330                 335

Ile Pro Gly Thr Asp Ser Ala Ser Val Val Glu Gly Met Leu Ser Leu
            340                 345                 350

Val His Leu Glu His His Ile Val Leu Lys Ser Asp Val Pro Thr Asp
        355                 360                 365

Gly Ala Leu Lys Val Ser Ala Thr Ala Asp Glu Val Val Asp Thr Asp
    370                 375                 380

Leu Gly Arg Leu Val Ile Val Arg Ala Glu Ile Ala Asp Ala Glu Gly
385                 390                 395                 400

Asn Leu Ile Ala Thr Leu Ala Glu Arg Phe Ala Ile Arg Gly Arg Lys
                405                 410                 415

Gly Asn Ala Val Ala Arg Thr Asn Thr Ser Ala Leu Pro Thr Thr Val
            420                 425                 430

Asp Thr Pro Arg Ser Ala Arg Ala Val Ala Thr Val Val Ala Pro Glu
        435                 440                 445

Ser Met Arg Pro Phe Ala Val Ile Ser Gly Asp Arg Asn Pro Ile His
    450                 455                 460

Val Ser Asp Val Ala Ala Ser Leu Ala Gly Leu Pro Gly Val Ile Val
465                 470                 475                 480

His Gly Met Trp Thr Ser Ala Ile Gly Glu Leu Ile Ala Gly Ala Ala
                485                 490                 495

Phe Asn Asp Glu Gln Ile Gln Thr Pro Ala Ala Lys Val Val Glu Tyr
            500                 505                 510

Thr Ala Thr Met Leu Ala Pro Val Leu Pro Gly Glu Glu Ile Glu Phe
        515                 520                 525

Ser Val Glu Arg Ser Ala Val Asp Asn Arg Pro Gly Met Gly Glu Val
    530                 535                 540

Arg Thr Val Thr Ala Thr Val Asn Gly Asn Leu Val Leu Thr Ala Thr
545                 550                 555                 560

Ala Val Val Ala Ala Pro Ser Thr Phe Tyr Ala Phe Pro Gly Gln Gly
                565                 570                 575

Ile Gln Ser Gln Gly Met Gly Met Glu Ala Arg Arg Asn Ser Gln Ala
            580                 585                 590

Ala Arg Ala Ile Trp Asp Arg Ala Asp Ala His Thr Arg Asn Lys Leu
        595                 600                 605

Gly Phe Ser Ile Val Glu Ile Val Glu Asn Asn Pro Arg Glu Val Thr

610                 615                 620

Val Ala Gly Glu Lys Phe Phe His Pro Asp Gly Val Leu Tyr Leu Thr
625                 630                 635                 640

Gln Phe Thr Gln Val Gly Met Ala Thr Leu Gly Val Ala Gln Ile Ala
                645                 650                 655

Glu Met Arg Glu Ala His Ala Leu Asn Gln Arg Ala Tyr Phe Ala Gly
            660                 665                 670

His Ser Val Gly Glu Tyr Asn Ala Leu Ala Ala Tyr Ala Gly Val Leu
        675                 680                 685

Ser Leu Glu Ser Val Leu Glu Ile Val Tyr Arg Arg Gly Leu Thr Met
    690                 695                 700

His Arg Leu Val Asp Arg Asp Glu Asn Gly Leu Ser Asn Tyr Ala Leu
705                 710                 715                 720

Ala Ala Leu Arg Pro Asn Lys Met Gly Leu Thr Ala Asp Asn Val Phe
                725                 730                 735

Asp Tyr Val Ala Ser Val Ser Glu Ala Ser Gly Glu Phe Leu Glu Ile
            740                 745                 750

Val Asn Tyr Asn Leu Ala Gly Leu Gln Tyr Ala Val Ala Gly Thr Gln
        755                 760                 765

Ala Gly Leu Ala Ala Leu Arg Ala Asp Val Glu Asn Arg Ala Pro Gly
    770                 775                 780

Gln Arg Ala Phe Ile Leu Ile Pro Gly Ile Asp Val Pro Phe His Ser
785                 790                 795                 800

Ser Lys Leu Arg Asp Gly Val Gly Ala Phe Arg Glu His Leu Asp Ser
                805                 810                 815

Leu Ile Pro Ala Glu Leu Asp Leu Asp Val Leu Val Gly Arg Tyr Ile
            820                 825                 830

Pro Asn Leu Val Ala Arg Pro Phe Glu Leu Thr Glu Glu Phe Val Ala
        835                 840                 845

Ser Met Ala Glu Val Val Glu Ser Thr Tyr Val Asn Glu Ile Leu Ala
    850                 855                 860

Asp Phe Lys Ala Ala Ser Ala Asp Lys Gln Lys Leu Ala Arg Thr Leu
865                 870                 875                 880

Leu Ile Glu Leu Leu Ala Trp Gln Phe Ala Ser Pro Val Arg Trp Ile
                885                 890                 895

Glu Thr Gln Asp Leu Leu Ile Lys Gly Leu Gln Ala Glu Arg Phe Val
            900                 905                 910

Glu Val Gly Val Gly Ser Ala Pro Thr Leu Ala Asn Met Met Gly Gln
        915                 920                 925

Thr Leu Arg Leu Pro Gln Tyr Ala Asp Ala Thr Ile Glu Val Leu Asn
    930                 935                 940

Ile Glu Arg Asp Arg Pro Val Val Phe Ala Thr Asp Glu Val Val Arg
945                 950                 955                 960

Glu Val Ala Val Glu Glu Thr Pro Ala Ala Pro Ala Glu Thr Thr Glu
                965                 970                 975

Thr Pro Ala Thr Pro Ala Thr Pro Ala Pro Val Ala Ala Ala Ala Pro
            980                 985                 990

Ala Thr Gly Gly Pro Arg Pro Asp Asp Ile Ser Phe Thr Pro Ser Asp
        995                 1000                1005

Ala Thr Glu Met Leu Ile Ala Ile Trp Thr Lys Val Arg Pro Asp Gln
    1010                1015                1020

Met Gly Ala Thr Asp Ser Ile Glu Thr Leu Val Glu Gly Val Ser Ser
1025                1030                1035                1040

Arg Arg Asn Gln Leu Leu Leu Asp Leu Gly Val Glu Phe Gly Leu Gly
                1045                1050                1055

Ala Ile Asp Gly Ala Ala Asp Ala Glu Leu Gly Asp Leu Lys Val Thr
            1060                1065                1070

Val Ser Lys Met Ala Lys Gly Tyr Lys Ala Phe Gly Pro Val Leu Ser
        1075                1080                1085

Asp Ala Ala Ala Asp Ala Leu Arg Arg Leu Thr Gly Pro Thr Gly Lys
    1090                1095                1100

Arg Pro Gly Tyr Ile Ala Glu Arg Val Thr Gly Thr Trp Glu Leu Gly
1105                1110                1115                1120

Gln Gly Trp Ala Asp His Val Val Ala Glu Val Val Ile Gly Ala Arg
                1125                1130                1135

Glu Gly Ala Ser Leu Arg Gly Gly Asp Leu Ala Ser Leu Ser Pro Ala
            1140                1145                1150

Ser Pro Ala Ser Ala Ser Asp Leu Asp Ser Leu Ile Asp Ala Ala Val
        1155                1160                1165

Gln Ala Val Ala Ser Arg Arg Gly Val Ala Val Ser Leu Pro Ser Ala
    1170                1175                1180

Gly Gly Ala Ala Gly Gly Val Val Asp Ser Ala Ala Leu Gly Glu Phe
1185                1190                1195                1200

Ala Glu Gln Val Thr Gly His Asp Gly Val Leu Ala Gln Ala Ala Arg
                1205                1210                1215

Thr Ile Leu Thr Gln Leu Gly Leu Asp Lys Pro Ala Thr Val Ser Val
            1220                1225                1230

Glu Asp Thr Ala Glu Glu Asp Leu Tyr Glu Leu Val Ser Lys Glu Leu
        1235                1240                1245

Gly Ser Asp Trp Pro Arg Gln Val Ala Pro Ser Phe Asp Glu Glu Lys
    1250                1255                1260

Val Val Leu Leu Asp Asp Arg Trp Ala Ser Ala Arg Glu
1265                1270                1275

<210> 55
<211> 4953
<212> DNA
<213> Corynebacterium glutamicum

<220>
<221> CDS
<222> (101)..(4930)
<223> RXA02582

<400> 55
tggatgtcta ttctcctcag cctcgttttg gttgaggcgg atagttttaa tattttttta 60

agtttaagtt gtaatcgagc tgaaaggctg aggcctcaat atg gaa cag agc caa   115
                                           Met Glu Gln Ser Gln
                                           1               5

tcg tcg gat cag aag atg acc gtt gaa cag gtt cgc acc tgg ctc cgt   163
Ser Ser Asp Gln Lys Met Thr Val Glu Gln Val Arg Thr Trp Leu Arg
                10                  15                  20

gat tgg gtt gtc cgc acc acg ggt att ccg gtg gag gaa gtt acg gat   211
Asp Trp Val Val Arg Thr Thr Gly Ile Pro Val Glu Glu Val Thr Asp
            25                  30                  35

gac aag gca atg gag acc ttt ggc ctt tcc tct cgc gat gtt gtt gtg   259
Asp Lys Ala Met Glu Thr Phe Gly Leu Ser Ser Arg Asp Val Val Val
        40                  45                  50

ttg tct ggt gag ctg gaa aac ctg ctg gac acc tcc ttg gat gcc acc   307
Leu Ser Gly Glu Leu Glu Asn Leu Leu Asp Thr Ser Leu Asp Ala Thr
    55                  60                  65

atc gct tat gag tac cca acg atc cgt agt ttg gcg cag cgc ctt gtt   355
Ile Ala Tyr Glu Tyr Pro Thr Ile Arg Ser Leu Ala Gln Arg Leu Val
70                  75                  80                  85

gag ggc gag cct cgt cgt gca cat acc cag cgt gaa ttg aat ttc tcc   403
Glu Gly Glu Pro Arg Arg Ala His Thr Gln Arg Glu Leu Asn Phe Ser
                90                  95                  100

gcg gtg agc gat tcc cca ggt tcc cac gat att gcg gtt gtc ggt atg   451
Ala Val Ser Asp Ser Pro Gly Ser His Asp Ile Ala Val Val Gly Met
            105                 110                 115

gct gcg cgt tac cca ggc gct gag agc ctg gag gat atg tgg aag cta   499
Ala Ala Arg Tyr Pro Gly Ala Glu Ser Leu Glu Asp Met Trp Lys Leu
        120                 125                 130

ctc gtc gag ggc cgt gac ggt atc tcg gat cta ccg att ggc cgt tgg   547
Leu Val Glu Gly Arg Asp Gly Ile Ser Asp Leu Pro Ile Gly Arg Trp
    135                 140                 145

tct gag tat gca ggc gat gag gtt atg tct cgg aag atg gaa gag ttt   595
Ser Glu Tyr Ala Gly Asp Glu Val Met Ser Arg Lys Met Glu Glu Phe
150                 155                 160                 165

tct acc atc ggt ggc tac ctg tca gat atc tct agc ttt gat gcg gag 643
Ser Thr Ile Gly Gly Tyr Leu Ser Asp Ile Ser Ser Phe Asp Ala Glu
                170                 175                 180

ttc ttt ggt ctg tct ccg ctc gag gcc gcc aac atg gat cct cag cag 691
Phe Phe Gly Leu Ser Pro Leu Glu Ala Ala Asn Met Asp Pro Gln Gln
            185                 190                 195

cgt att ttg ctg gag cta acg tgg gag gct ttg gag tac gct cgc atc 739
Arg Ile Leu Leu Glu Leu Thr Trp Glu Ala Leu Glu Tyr Ala Arg Ile
        200                 205                 210

gca cca aac act ttg cgt ggc gaa gcc gtg ggc gtg ttc ata ggt tcc 787
Ala Pro Asn Thr Leu Arg Gly Glu Ala Val Gly Val Phe Ile Gly Ser
    215                 220                 225

tcc aac aac gat tac ggc atg atg atc gct gcc gat cca gca gaa gca 835
Ser Asn Asn Asp Tyr Gly Met Met Ile Ala Ala Asp Pro Ala Glu Ala
230                 235                 240                 245

cat cct tat gcg ctg act ggt act tct agt gcg att gtc gct aac cgc 883
His Pro Tyr Ala Leu Thr Gly Thr Ser Ser Ala Ile Val Ala Asn Arg
                250                 255                 260

att aac tac gct ttc gat ttc cgc ggt cct tcc gtc aac gtg gat acc 931
Ile Asn Tyr Ala Phe Asp Phe Arg Gly Pro Ser Val Asn Val Asp Thr
            265                 270                 275

gca tgt tct tct tct ctg gta gcg gtt cac cag gct gtc cgt gcg ctg 979
Ala Cys Ser Ser Ser Leu Val Ala Val His Gln Ala Val Arg Ala Leu
        280                 285                 290

cgt aat ggc gaa gcg gat cac gct atc gct ggt gga gtg aac att ttg 1027
Arg Asn Gly Glu Ala Asp His Ala Ile Ala Gly Gly Val Asn Ile Leu
    295                 300                 305

gca tct cca ttt gtc aca act gca ttc gct gag ctc ggt gtg atc agc 1075
Ala Ser Pro Phe Val Thr Thr Ala Phe Ala Glu Leu Gly Val Ile Ser
310                 315                 320                 325

cca acc ggc aag atc cac gca ttc tct gat gat gcc gat ggt ttc gtg 1123
Pro Thr Gly Lys Ile His Ala Phe Ser Asp Asp Ala Asp Gly Phe Val
                330                 335                 340

cgt tcc gac ggc gcc gga gtc gtt gtg ctg aag cgc gtg gat gac gca 1171
Arg Ser Asp Gly Ala Gly Val Val Val Leu Lys Arg Val Asp Asp Ala
            345                 350                 355

atc cgc gac ggc gac aag atc atc ggt gtg atc aag ggt tct gca gtg 1219
Ile Arg Asp Gly Asp Lys Ile Ile Gly Val Ile Lys Gly Ser Ala Val
        360                 365                 370

aac tcc gat ggt cac tcc aac gga ctg acc gct cca aac cct gac gca 1267
Asn Ser Asp Gly His Ser Asn Gly Leu Thr Ala Pro Asn Pro Asp Ala
    375                 380                 385

cag gtt gat gtg ctg cag cgt gca tat gtt gac gct cag gtt gat ccc 1315
Gln Val Asp Val Leu Gln Arg Ala Tyr Val Asp Ala Gln Val Asp Pro
390                 395                 400                 405

acc acc gtg gat tac gtt gag gct cac ggc acc ggc acc atc ctg ggt 1363

Thr Thr Val Asp Tyr Val Glu Ala His Gly Thr Gly Thr Ile Leu Gly
                410                 415                 420

gac ccc att gaa gca act gcc ctg ggt gct gtt ttg ggc tat ggc cgt 1411
Asp Pro Ile Glu Ala Thr Ala Leu Gly Ala Val Leu Gly Tyr Gly Arg
            425                 430                 435

gac gca tcc acc cca act ctt ctg ggc tct gca aag tcc aac ttc ggc 1459
Asp Ala Ser Thr Pro Thr Leu Leu Gly Ser Ala Lys Ser Asn Phe Gly
        440                 445                 450

cac act gag tca gct gcg ggt att gca ggt gtc atc aag gtg ctg ctt 1507
His Thr Glu Ser Ala Ala Gly Ile Ala Gly Val Ile Lys Val Leu Leu
    455                 460                 465

gct ttg cag aac aag act ctg cca cca acc gtg aac ttt gca ggt cca 1555
Ala Leu Gln Asn Lys Thr Leu Pro Pro Thr Val Asn Phe Ala Gly Pro
470                 475                 480                 485

aac cgc tac atc gat ttc gat gct gag cgt ttg gaa gtt gtg gaa gat 1603
Asn Arg Tyr Ile Asp Phe Asp Ala Glu Arg Leu Glu Val Val Glu Asp
                490                 495                 500

cca cgt gaa tgg ccg gaa tac aac ggt cac gca gta gct ggt gtg tct 1651
Pro Arg Glu Trp Pro Glu Tyr Asn Gly His Ala Val Ala Gly Val Ser
            505                 510                 515

gcg ttc ggg ttc ggt gga acc aac gcg cac gtg gtg atc tct gag tac 1699
Ala Phe Gly Phe Gly Gly Thr Asn Ala His Val Val Ile Ser Glu Tyr
        520                 525                 530

aac gca gaa gac tac gaa acc cgt gcc ccg aag gag gcg ttg ctt cct 1747
Asn Ala Glu Asp Tyr Glu Thr Arg Ala Pro Lys Glu Ala Leu Leu Pro
    535                 540                 545

gac cag cag gtt gcc ctg ccg gtg tct ggt cac ctg cca tcc agg cgt 1795
Asp Gln Gln Val Ala Leu Pro Val Ser Gly His Leu Pro Ser Arg Arg
550                 555                 560                 565

cga caa gca gct gct gac ttg gcg gac ttc ttg gag ggc cgc aaa gat 1843
Arg Gln Ala Ala Ala Asp Leu Ala Asp Phe Leu Glu Gly Arg Lys Asp
                570                 575                 580

tgc gac cta acc cca gta gcc cgc gcg ctg gca ggc cgc aat cat ggc 1891
Cys Asp Leu Thr Pro Val Ala Arg Ala Leu Ala Gly Arg Asn His Gly
            585                 590                 595

cgc tcc cgc gca gtc gtg ctt gca agc act atc gag gaa gcc gta aag 1939
Arg Ser Arg Ala Val Val Leu Ala Ser Thr Ile Glu Glu Ala Val Lys
        600                 605                 610

cgc ctg cgc cag gtc gca gaa ggc aag gtc agc gtc ggt atc tcc gca 1987
Arg Leu Arg Gln Val Ala Glu Gly Lys Val Ser Val Gly Ile Ser Ala
    615                 620                 625

gct gat tcc cca gcg gcc aac ggt cct gtg ttt gtg tac tcc ggt ttc 2035
Ala Asp Ser Pro Ala Ala Asn Gly Pro Val Phe Val Tyr Ser Gly Phe
630                 635                 640                 645

gga tcc cag cac cgc ctc atg atc aag gaa ttg tgc tca att tcg cca 2083
Gly Ser Gln His Arg Leu Met Ile Lys Glu Leu Cys Ser Ile Ser Pro

                650                 655                 660

cag ttc cgc gag cgc atc gaa gag ctc gat gaa atg gtt aag ttc gag 2131
Gln Phe Arg Glu Arg Ile Glu Glu Leu Asp Glu Met Val Lys Phe Glu
            665                 670                 675

tcc ggc tgg tcc atc atg aaa ctt gtt ttg gat gat gag caa act tac 2179
Ser Gly Trp Ser Ile Met Lys Leu Val Leu Asp Asp Glu Gln Thr Tyr
        680                 685                 690

gac acc gag acc gcg cag gtt gtg atc acc gca atc cag atc gca ctg 2227
Asp Thr Glu Thr Ala Gln Val Val Ile Thr Ala Ile Gln Ile Ala Leu
    695                 700                 705

act gac ctg ttg gca agc ttc ggt gta aag cca gct gcc gtc atg ggc 2275
Thr Asp Leu Leu Ala Ser Phe Gly Val Lys Pro Ala Ala Val Met Gly
710                 715                 720                 725

atg tcg atg ggt gag atc gct gcc gct tac gct gcc ggt gga ctc agc 2323
Met Ser Met Gly Glu Ile Ala Ala Ala Tyr Ala Ala Gly Gly Leu Ser
                730                 735                 740

gac cgc gat acc atg ctc att gcc agc cac cgt tcc cgc cta atg ggc 2371
Asp Arg Asp Thr Met Leu Ile Ala Ser His Arg Ser Arg Leu Met Gly
            745                 750                 755

gag ggc gag aag tcc ctg gct gag gat cag ctg ggt gcc atg gct gtg 2419
Glu Gly Glu Lys Ser Leu Ala Glu Asp Gln Leu Gly Ala Met Ala Val
        760                 765                 770

gtg gaa ttc gcc gct gcg gac ctg gat aag ttc atc gaa gaa aac cct 2467
Val Glu Phe Ala Ala Ala Asp Leu Asp Lys Phe Ile Glu Glu Asn Pro
    775                 780                 785

gag tac aag ggc att gag cct gct gtt tat gca ggc cca ggc atg act 2515
Glu Tyr Lys Gly Ile Glu Pro Ala Val Tyr Ala Gly Pro Gly Met Thr
790                 795                 800                 805

acc gtg ggt gga cct cgc gac gcc gtg gtt cag ttc gta gag aag ctg 2563
Thr Val Gly Gly Pro Arg Asp Ala Val Val Gln Phe Val Glu Lys Leu
                810                 815                 820

gaa tct gaa gac aag ttt gcc cgc ctg ctc aac gtc aag ggc gct ggc 2611
Glu Ser Glu Asp Lys Phe Ala Arg Leu Leu Asn Val Lys Gly Ala Gly
            825                 830                 835

cac acc tct gct gtg gaa cca ctt ctt ggt gag ctc gct ggc gaa atc 2659
His Thr Ser Ala Val Glu Pro Leu Leu Gly Glu Leu Ala Gly Glu Ile
        840                 845                 850

gct ggc atc gag cca ctt ccg ctg cag atc ccg ttg ttc agc tct gtc 2707
Ala Gly Ile Glu Pro Leu Pro Leu Gln Ile Pro Leu Phe Ser Ser Val
    855                 860                 865

gat cag ggt gtt acc tac cca gtt gga gca gtg gtc cac gac gcc gat 2755
Asp Gln Gly Val Thr Tyr Pro Val Gly Ala Val Val His Asp Ala Asp
870                 875                 880                 885

tac atg ctc cgc tgt acc cgc cag tcc gtg tac ttc cag gac tcc acc 2803
Tyr Met Leu Arg Cys Thr Arg Gln Ser Val Tyr Phe Gln Asp Ser Thr
                890                 895                 900

gaa gct gca ttc gct gca ggc cac aac acc ttg gtg gaa att tcc ccg 2851
Glu Ala Ala Phe Ala Ala Gly His Asn Thr Leu Val Glu Ile Ser Pro
            905                 910                 915

aac cca gtt gca ctc atg ggc atg atg aac acc gcg ttc acc gtg ggc 2899
Asn Pro Val Ala Leu Met Gly Met Met Asn Thr Ala Phe Thr Val Gly
        920                 925                 930

aag cct gat gca cag ctg ctg ttc agc ctg aag cga aaa gtc cca gag 2947
Lys Pro Asp Ala Gln Leu Leu Phe Ser Leu Lys Arg Lys Val Pro Glu
    935                 940                 945

gca gaa tcc ctc cgc gac ctt ctg gcg aag ctg tac gtc aac ggc gca 2995
Ala Glu Ser Leu Arg Asp Leu Leu Ala Lys Leu Tyr Val Asn Gly Ala
950                 955                 960                 965

aac gtt gat ttc tcc gcg ctg tac ggc gaa ggt gaa acc atc gat ccg 3043
Asn Val Asp Phe Ser Ala Leu Tyr Gly Glu Gly Glu Thr Ile Asp Pro
                970                 975                 980

cca cac atc acc tgg aag cac cag cgc ttc tgg act tcc gca cgc cca 3091
Pro His Ile Thr Trp Lys His Gln Arg Phe Trp Thr Ser Ala Arg Pro
            985                 990                 995

tcc tct ggc gca tcc ctg gat ctg ccg ggc ttc cgc gtg aat ctg cca 3139
Ser Ser Gly Ala Ser Leu Asp Leu Pro Gly Phe Arg Val Asn Leu Pro
        1000                1005                1010

aac aac act gtg gcg ttt tcc acc gca gcc gaa ctg gca cca tcc gca 3187
Asn Asn Thr Val Ala Phe Ser Thr Ala Ala Glu Leu Ala Pro Ser Ala
    1015                1020                1025

gtg gca atc atg gaa gca gcc gcc atg gct gtc acc cca ggt tcc tcc 3235
Val Ala Ile Met Glu Ala Ala Ala Met Ala Val Thr Pro Gly Ser Ser
1030                1035                1040                1045

gtc gac gca gtg gac gag cgc gac atg ctg cca cca agc ggc gaa atc 3283
Val Asp Ala Val Asp Glu Arg Asp Met Leu Pro Pro Ser Gly Glu Ile
                1050                1055                1060

acc acc atc gtg act cgc tcc ttg ggc ggt ttg agc ctg tcc gta tac 3331
Thr Thr Ile Val Thr Arg Ser Leu Gly Gly Leu Ser Leu Ser Val Tyr
            1065                1070                1075

aag att gag gga acc acc agc act ctt gtc gcc gaa ggt ttc gca gcc 3379
Lys Ile Glu Gly Thr Thr Ser Thr Leu Val Ala Glu Gly Phe Ala Ala
        1080                1085                1090

aac cca gga ttc gcc gca gca tct tcc ttc gac ggt cca ggc tac gac 3427
Asn Pro Gly Phe Ala Ala Ala Ser Ser Phe Asp Gly Pro Gly Tyr Asp
    1095                1100                1105

gga ttc aac acc gat tac agc gac cag cca gac cct cgc tcc gac ctg 3475
Gly Phe Asn Thr Asp Tyr Ser Asp Gln Pro Asp Pro Arg Ser Asp Leu
1110                1115                1120                1125

cct ttg gac atc gaa gca gtc cgc tgg gac cca gcg acc gaa acc gtc 3523
Pro Leu Asp Ile Glu Ala Val Arg Trp Asp Pro Ala Thr Glu Thr Val
                1130                1135                1140

gaa gag cgc atg cgc gcc atc gtc tcc gaa gca atg ggc tac gac gtg    3571
Glu Glu Arg Met Arg Ala Ile Val Ser Glu Ala Met Gly Tyr Asp Val
            1145                1150                1155

gat gac ctc cca cgc gaa ctc cca ctg att gac ctc ggc ctc gac tcc    3619
Asp Asp Leu Pro Arg Glu Leu Pro Leu Ile Asp Leu Gly Leu Asp Ser
        1160                1165                1170

ctc atg ggc atg cgc atc aaa aac cgc atc gaa aat gac ttc cag atc    3667
Leu Met Gly Met Arg Ile Lys Asn Arg Ile Glu Asn Asp Phe Gln Ile
    1175                1180                1185

cca cca ctc cag gtc caa gcg ctc cgc gat gca tcc gtc gct gac gtg    3715
Pro Pro Leu Gln Val Gln Ala Leu Arg Asp Ala Ser Val Ala Asp Val
1190                1195                1200                1205

gta atc atg gtg gaa aac atg gtc gct ggc cgc tcc tct gag acg ctt    3763
Val Ile Met Val Glu Asn Met Val Ala Gly Arg Ser Ser Glu Thr Leu
                1210                1215                1220

gtc gac gcc acc ccg cag gtg cca gct gag gca gca ggg gag gct caa    3811
Val Asp Ala Thr Pro Gln Val Pro Ala Glu Ala Ala Gly Glu Ala Gln
            1225                1230                1235

gct gct gag tct tct gct tcg ggc gag gac gtg cag ggc gtt ggc gtt    3859
Ala Ala Glu Ser Ser Ala Ser Gly Glu Asp Val Gln Gly Val Gly Val
        1240                1245                1250

gca ccg cga gat gcg tcg gaa cgc atg gtc ttt ggt act tgg gca ggc    3907
Ala Pro Arg Asp Ala Ser Glu Arg Met Val Phe Gly Thr Trp Ala Gly
    1255                1260                1265

ctg act ggc gct gcg gca gct ggc gtg acc agc aag ttg cca cag atc    3955
Leu Thr Gly Ala Ala Ala Ala Gly Val Thr Ser Lys Leu Pro Gln Ile
1270                1275                1280                1285

gat gtc gat act gca acc gca att gcc gag cgc ctc acc gag cgt tcc    4003
Asp Val Asp Thr Ala Thr Ala Ile Ala Glu Arg Leu Thr Glu Arg Ser
                1290                1295                1300

gga att gaa atc agc act gaa cag gtg ttg gct gca gaa acc ctc gaa    4051
Gly Ile Glu Ile Ser Thr Glu Gln Val Leu Ala Ala Glu Thr Leu Glu
            1305                1310                1315

cca ctg tct gac ctg gtg cgt gaa ggc ctc gaa act gaa gtt cag ggc    4099
Pro Leu Ser Asp Leu Val Arg Glu Gly Leu Glu Thr Glu Val Gln Gly
        1320                1325                1330

aac att cgt gtg ctg cgt gga cgt gca gaa ggc tcc acc aag cct gca    4147
Asn Ile Arg Val Leu Arg Gly Arg Ala Glu Gly Ser Thr Lys Pro Ala
    1335                1340                1345

gtg ttc atg ttc cac cca gct ggc ggt tct tcc gtg gtc tac caa cca    4195
Val Phe Met Phe His Pro Ala Gly Gly Ser Ser Val Val Tyr Gln Pro
1350                1355                1360                1365

cta atg cgt cgt ctg cct gaa gat gtc cct gtc tat ggc gtt gag cgt    4243
Leu Met Arg Arg Leu Pro Glu Asp Val Pro Val Tyr Gly Val Glu Arg
                1370                1375                1380

ctg gaa ggc gat ctc gct gac cgc gcg gca gca tat gtt gat gac atc    4291

Leu Glu Gly Asp Leu Ala Asp Arg Ala Ala Ala Tyr Val Asp Asp Ile
            1385                1390                1395

aag aag tac tcc gat ggc ttc cca gta gtt ttg ggt ggc tgg agc ttc 4339
Lys Lys Tyr Ser Asp Gly Phe Pro Val Val Leu Gly Gly Trp Ser Phe
        1400                1405                1410

ggc ggt gcc gtt gcc ttc gag gtt gcc cac caa ctg gtt ggc tcc gat 4387
Gly Gly Ala Val Ala Phe Glu Val Ala His Gln Leu Val Gly Ser Asp
    1415                1420                1425

gtt gag gta gct acc gtg gcg ttg ctg gat act gtg cag cct tca aac 4435
Val Glu Val Ala Thr Val Ala Leu Leu Asp Thr Val Gln Pro Ser Asn
1430                1435                1440                1445

cca gca cca gat acc gct gag gaa act cgt gca cgc tgg act cgc tac 4483
Pro Ala Pro Asp Thr Ala Glu Glu Thr Arg Ala Arg Trp Thr Arg Tyr
                1450                1455                1460

gcg gac ttc gcc aag aag acc tac ggc ctt gat ttc gag gta cct ttt 4531
Ala Asp Phe Ala Lys Lys Thr Tyr Gly Leu Asp Phe Glu Val Pro Phe
            1465                1470                1475

gaa atc ttg gac acc atc ggt gaa gac gga atg ctg tcc atg atg acc 4579
Glu Ile Leu Asp Thr Ile Gly Glu Asp Gly Met Leu Ser Met Met Thr
        1480                1485                1490

gac ttc ctg gcc aac acc gac gcc tcc gag cac gga ttg tcc gca ggt 4627
Asp Phe Leu Ala Asn Thr Asp Ala Ser Glu His Gly Leu Ser Ala Gly
    1495                1500                1505

gtg ctt gaa cac cag cgc gct tct ttt gtg gac aac cgc atc ctg gct 4675
Val Leu Glu His Gln Arg Ala Ser Phe Val Asp Asn Arg Ile Leu Ala
1510                1515                1520                1525

aaa ctt aat ttt gca gac tgg gcc aac gtt gaa gcc cct gtc atc ctg 4723
Lys Leu Asn Phe Ala Asp Trp Ala Asn Val Glu Ala Pro Val Ile Leu
                1530                1535                1540

ttc cgc gcg gaa cgc atg cat gat gga gct atc gaa ctt gaa cca aac 4771
Phe Arg Ala Glu Arg Met His Asp Gly Ala Ile Glu Leu Glu Pro Asn
            1545                1550                1555

tat gcc aag att gat caa gat gga gga tgg tcc gga att gtc aac gat 4819
Tyr Ala Lys Ile Asp Gln Asp Gly Gly Trp Ser Gly Ile Val Asn Asp
        1560                1565                1570

ttg gaa att gtt cag ctg aat ggt gac cac ctg gca gtt gtc gat gaa 4867
Leu Glu Ile Val Gln Leu Asn Gly Asp His Leu Ala Val Val Asp Glu
    1575                1580                1585

cca gaa atc ggc aca gtc gga gct cat ttg agt cgc cgc att gat gag 4915
Pro Glu Ile Gly Thr Val Gly Ala His Leu Ser Arg Arg Ile Asp Glu
1590                1595                1600                1605

att tct cgg aag aat tagtaacgga gagctgacgg aag 4953
Ile Ser Arg Lys Asn
                1610

<210> 56

<211> 1610
<212> PRT
<213> Corynebacterium glutamicum

<400> 56
Met Glu Gln Ser Gln Ser Ser Asp Gln Lys Met Thr Val Glu Gln Val
1               5                   10                  15

Arg Thr Trp Leu Arg Asp Trp Val Val Arg Thr Thr Gly Ile Pro Val
            20                  25                  30

Glu Glu Val Thr Asp Asp Lys Ala Met Glu Thr Phe Gly Leu Ser Ser
        35                  40                  45

Arg Asp Val Val Val Leu Ser Gly Glu Leu Glu Asn Leu Leu Asp Thr
    50                  55                  60

Ser Leu Asp Ala Thr Ile Ala Tyr Glu Tyr Pro Thr Ile Arg Ser Leu
65                  70                  75                  80

Ala Gln Arg Leu Val Glu Gly Glu Pro Arg Arg Ala His Thr Gln Arg
                85                  90                  95

Glu Leu Asn Phe Ser Ala Val Ser Asp Ser Pro Gly Ser His Asp Ile
            100                 105                 110

Ala Val Val Gly Met Ala Ala Arg Tyr Pro Gly Ala Glu Ser Leu Glu
        115                 120                 125

Asp Met Trp Lys Leu Leu Val Glu Gly Arg Asp Gly Ile Ser Asp Leu
    130                 135                 140

Pro Ile Gly Arg Trp Ser Glu Tyr Ala Gly Asp Glu Val Met Ser Arg
145                 150                 155                 160

Lys Met Glu Glu Phe Ser Thr Ile Gly Gly Tyr Leu Ser Asp Ile Ser
                165                 170                 175

Ser Phe Asp Ala Glu Phe Phe Gly Leu Ser Pro Leu Glu Ala Ala Asn
            180                 185                 190

Met Asp Pro Gln Gln Arg Ile Leu Leu Glu Leu Thr Trp Glu Ala Leu
        195                 200                 205

Glu Tyr Ala Arg Ile Ala Pro Asn Thr Leu Arg Gly Glu Ala Val Gly
    210                 215                 220

Val Phe Ile Gly Ser Ser Asn Asn Asp Tyr Gly Met Met Ile Ala Ala
225                 230                 235                 240

Asp Pro Ala Glu Ala His Pro Tyr Ala Leu Thr Gly Thr Ser Ser Ala
                245                 250                 255

Ile Val Ala Asn Arg Ile Asn Tyr Ala Phe Asp Phe Arg Gly Pro Ser
            260                 265                 270

Val Asn Val Asp Thr Ala Cys Ser Ser Ser Leu Val Ala Val His Gln
        275                 280                 285

Ala Val Arg Ala Leu Arg Asn Gly Glu Ala Asp His Ala Ile Ala Gly
    290                 295                 300

Gly Val Asn Ile Leu Ala Ser Pro Phe Val Thr Thr Ala Phe Ala Glu
305                 310                 315                 320

Leu Gly Val Ile Ser Pro Thr Gly Lys Ile His Ala Phe Ser Asp Asp
                325                 330                 335

Ala Asp Gly Phe Val Arg Ser Asp Gly Ala Gly Val Val Val Leu Lys
            340                 345                 350

Arg Val Asp Asp Ala Ile Arg Asp Gly Asp Lys Ile Ile Gly Val Ile
        355                 360                 365

Lys Gly Ser Ala Val Asn Ser Asp Gly His Ser Asn Gly Leu Thr Ala
    370                 375                 380

Pro Asn Pro Asp Ala Gln Val Asp Val Leu Gln Arg Ala Tyr Val Asp
385                 390                 395                 400

Ala Gln Val Asp Pro Thr Thr Val Asp Tyr Val Glu Ala His Gly Thr
                405                 410                 415

Gly Thr Ile Leu Gly Asp Pro Ile Glu Ala Thr Ala Leu Gly Ala Val
            420                 425                 430

Leu Gly Tyr Gly Arg Asp Ala Ser Thr Pro Thr Leu Leu Gly Ser Ala
        435                 440                 445

Lys Ser Asn Phe Gly His Thr Glu Ser Ala Ala Gly Ile Ala Gly Val
    450                 455                 460

Ile Lys Val Leu Leu Ala Leu Gln Asn Lys Thr Leu Pro Pro Thr Val
465                 470                 475                 480

Asn Phe Ala Gly Pro Asn Arg Tyr Ile Asp Phe Asp Ala Glu Arg Leu
                485                 490                 495

Glu Val Val Glu Asp Pro Arg Glu Trp Pro Glu Tyr Asn Gly His Ala
            500                 505                 510

Val Ala Gly Val Ser Ala Phe Gly Phe Gly Gly Thr Asn Ala His Val
        515                 520                 525

Val Ile Ser Glu Tyr Asn Ala Glu Asp Tyr Glu Thr Arg Ala Pro Lys
    530                 535                 540

Glu Ala Leu Leu Pro Asp Gln Gln Val Ala Leu Pro Val Ser Gly His
545                 550                 555                 560

Leu Pro Ser Arg Arg Arg Gln Ala Ala Ala Asp Leu Ala Asp Phe Leu
                565                 570                 575

Glu Gly Arg Lys Asp Cys Asp Leu Thr Pro Val Ala Arg Ala Leu Ala
            580                 585                 590

Gly Arg Asn His Gly Arg Ser Arg Ala Val Val Leu Ala Ser Thr Ile
        595                 600                 605

Glu Glu Ala Val Lys Arg Leu Arg Gln Val Ala Glu Gly Lys Val Ser
    610                 615                 620

Val Gly Ile Ser Ala Ala Asp Ser Pro Ala Ala Asn Gly Pro Val Phe
625                 630                 635                 640

Val Tyr Ser Gly Phe Gly Ser Gln His Arg Leu Met Ile Lys Glu Leu
                645                 650                 655

Cys Ser Ile Ser Pro Gln Phe Arg Glu Arg Ile Glu Glu Leu Asp Glu
            660                 665                 670

Met Val Lys Phe Glu Ser Gly Trp Ser Ile Met Lys Leu Val Leu Asp
        675                 680                 685

Asp Glu Gln Thr Tyr Asp Thr Glu Thr Ala Gln Val Val Ile Thr Ala
    690                 695                 700

Ile Gln Ile Ala Leu Thr Asp Leu Leu Ala Ser Phe Gly Val Lys Pro
705                 710                 715                 720

Ala Ala Val Met Gly Met Ser Met Gly Glu Ile Ala Ala Ala Tyr Ala
                725                 730                 735

Ala Gly Gly Leu Ser Asp Arg Asp Thr Met Leu Ile Ala Ser His Arg
            740                 745                 750

Ser Arg Leu Met Gly Glu Gly Glu Lys Ser Leu Ala Glu Asp Gln Leu
        755                 760                 765

Gly Ala Met Ala Val Val Glu Phe Ala Ala Ala Asp Leu Asp Lys Phe
    770                 775                 780

Ile Glu Glu Asn Pro Glu Tyr Lys Gly Ile Glu Pro Ala Val Tyr Ala
785                 790                 795                 800

Gly Pro Gly Met Thr Thr Val Gly Gly Pro Arg Asp Ala Val Val Gln
                805                 810                 815

Phe Val Glu Lys Leu Glu Ser Glu Asp Lys Phe Ala Arg Leu Leu Asn
            820                 825                 830

Val Lys Gly Ala Gly His Thr Ser Ala Val Glu Pro Leu Leu Gly Glu
        835                 840                 845

Leu Ala Gly Glu Ile Ala Gly Ile Glu Pro Leu Pro Leu Gln Ile Pro
    850                 855                 860

Leu Phe Ser Ser Val Asp Gln Gly Val Thr Tyr Pro Val Gly Ala Val
865                 870                 875                 880

Val His Asp Ala Asp Tyr Met Leu Arg Cys Thr Arg Gln Ser Val Tyr
                885                 890                 895

Phe Gln Asp Ser Thr Glu Ala Ala Phe Ala Ala Gly His Asn Thr Leu
            900                 905                 910

Val Glu Ile Ser Pro Asn Pro Val Ala Leu Met Gly Met Met Asn Thr
        915                 920                 925

Ala Phe Thr Val Gly Lys Pro Asp Ala Gln Leu Leu Phe Ser Leu Lys
    930                 935                 940

Arg Lys Val Pro Glu Ala Glu Ser Leu Arg Asp Leu Leu Ala Lys Leu

945                 950                 955                 960

Tyr Val Asn Gly Ala Asn Val Asp Phe Ser Ala Leu Tyr Gly Glu Gly
                965                 970                 975

Glu Thr Ile Asp Pro Pro His Ile Thr Trp Lys His Gln Arg Phe Trp
            980                 985                 990

Thr Ser Ala Arg Pro Ser Ser Gly Ala Ser Leu Asp Leu Pro Gly Phe
        995                 1000                1005

Arg Val Asn Leu Pro Asn Asn Thr Val Ala Phe Ser Thr Ala Ala Glu
    1010                1015                1020

Leu Ala Pro Ser Ala Val Ala Ile Met Glu Ala Ala Ala Met Ala Val
1025                1030                1035                1040

Thr Pro Gly Ser Ser Val Asp Ala Val Asp Glu Arg Asp Met Leu Pro
                1045                1050                1055

Pro Ser Gly Glu Ile Thr Thr Ile Val Thr Arg Ser Leu Gly Gly Leu
            1060                1065                1070

Ser Leu Ser Val Tyr Lys Ile Glu Gly Thr Thr Ser Thr Leu Val Ala
        1075                1080                1085

Glu Gly Phe Ala Ala Asn Pro Gly Phe Ala Ala Ala Ser Ser Phe Asp
    1090                1095                1100

Gly Pro Gly Tyr Asp Gly Phe Asn Thr Asp Tyr Ser Asp Gln Pro Asp
1105                1110                1115                1120

Pro Arg Ser Asp Leu Pro Leu Asp Ile Glu Ala Val Arg Trp Asp Pro
                1125                1130                1135

Ala Thr Glu Thr Val Glu Glu Arg Met Arg Ala Ile Val Ser Glu Ala
            1140                1145                1150

Met Gly Tyr Asp Val Asp Asp Leu Pro Arg Glu Leu Pro Leu Ile Asp
        1155                1160                1165

Leu Gly Leu Asp Ser Leu Met Gly Met Arg Ile Lys Asn Arg Ile Glu
    1170                1175                1180

Asn Asp Phe Gln Ile Pro Pro Leu Gln Val Gln Ala Leu Arg Asp Ala
1185                1190                1195                1200

Ser Val Ala Asp Val Val Ile Met Val Glu Asn Met Val Ala Gly Arg
                1205                1210                1215

Ser Ser Glu Thr Leu Val Asp Ala Thr Pro Gln Val Pro Ala Glu Ala
            1220                1225                1230

Ala Gly Glu Ala Gln Ala Ala Glu Ser Ser Ala Ser Gly Glu Asp Val
        1235                1240                1245

Gln Gly Val Gly Val Ala Pro Arg Asp Ala Ser Glu Arg Met Val Phe
    1250                1255                1260

Gly Thr Trp Ala Gly Leu Thr Gly Ala Ala Ala Ala Gly Val Thr Ser
1265                1270                1275                1280

Lys Leu Pro Gln Ile Asp Val Asp Thr Ala Thr Ala Ile Ala Glu Arg
                1285                1290                1295

Leu Thr Glu Arg Ser Gly Ile Glu Ile Ser Thr Glu Gln Val Leu Ala
            1300                1305                1310

Ala Glu Thr Leu Glu Pro Leu Ser Asp Leu Val Arg Glu Gly Leu Glu
        1315                1320                1325

Thr Glu Val Gln Gly Asn Ile Arg Val Leu Arg Gly Arg Ala Glu Gly
    1330                1335                1340

Ser Thr Lys Pro Ala Val Phe Met Phe His Pro Ala Gly Gly Ser Ser
1345                1350                1355                1360

Val Val Tyr Gln Pro Leu Met Arg Arg Leu Pro Glu Asp Val Pro Val
                1365                1370                1375

Tyr Gly Val Glu Arg Leu Glu Gly Asp Leu Ala Asp Arg Ala Ala Ala
            1380                1385                1390

Tyr Val Asp Asp Ile Lys Lys Tyr Ser Asp Gly Phe Pro Val Val Leu
        1395                1400                1405

Gly Gly Trp Ser Phe Gly Gly Ala Val Ala Phe Glu Val Ala His Gln
    1410                1415                1420

Leu Val Gly Ser Asp Val Glu Val Ala Thr Val Ala Leu Leu Asp Thr
1425                1430                1435                1440

Val Gln Pro Ser Asn Pro Ala Pro Asp Thr Ala Glu Glu Thr Arg Ala
                1445                1450                1455

Arg Trp Thr Arg Tyr Ala Asp Phe Ala Lys Lys Thr Tyr Gly Leu Asp
            1460                1465                1470

Phe Glu Val Pro Phe Glu Ile Leu Asp Thr Ile Gly Glu Asp Gly Met
        1475                1480                1485

Leu Ser Met Met Thr Asp Phe Leu Ala Asn Thr Asp Ala Ser Glu His
    1490                1495                1500

Gly Leu Ser Ala Gly Val Leu Glu His Gln Arg Ala Ser Phe Val Asp
1505                1510                1515                1520

Asn Arg Ile Leu Ala Lys Leu Asn Phe Ala Asp Trp Ala Asn Val Glu
                1525                1530                1535

Ala Pro Val Ile Leu Phe Arg Ala Glu Arg Met His Asp Gly Ala Ile
            1540                1545                1550

Glu Leu Glu Pro Asn Tyr Ala Lys Ile Asp Gln Asp Gly Gly Trp Ser
        1555                1560                1565

Gly Ile Val Asn Asp Leu Glu Ile Val Gln Leu Asn Gly Asp His Leu
    1570                1575                1580

Ala Val Val Asp Glu Pro Glu Ile Gly Thr Val Gly Ala His Leu Ser
1585                1590                1595                1600

Arg Arg Ile Asp Glu Ile Ser Arg Lys Asn
                1605                1610

<210> 57
<211> 930
<212> DNA
<213> Corynebacterium glutamicum

<220>
<221> CDS
<222> (101)..(907)
<223> RXA02691

<400> 57
cttagggctt atctgttttc cagccttgct ttttactagg cgctcctgtc ccgcttcagt 60

caccaaaacc acacccctgg ttatgaccag atcggctaaa atg aac acc atg cct   115
                                           Met Asn Thr Met Pro
                                           1               5

gac caa ccg ctc aac cag gac gga ttc cct acc gca tcc aaa ggg gtg   163
Asp Gln Pro Leu Asn Gln Asp Gly Phe Pro Thr Ala Ser Lys Gly Val
                10                  15                  20

gaa ccc gac aac ctc ccc gac cgc gtt ctc gtg gac ggc ctt aaa cca   211
Glu Pro Asp Asn Leu Pro Asp Arg Val Leu Val Asp Gly Leu Lys Pro
            25                  30                  35

aag cat cag cag ctt cgt gaa att ttg gag gaa atc tgc acc acc cag   259
Lys His Gln Gln Leu Arg Glu Ile Leu Glu Glu Ile Cys Thr Thr Gln
        40                  45                  50

ctt cag cct ggg gac atg ctg cct ggt gag cgc atc ctg gaa gaa aag   307
Leu Gln Pro Gly Asp Met Leu Pro Gly Glu Arg Ile Leu Glu Glu Lys
    55                  60                  65

tat ggc gtc agc cga att acg gtt cgt cgg gcg att ggt gat ctg gtc   355
Tyr Gly Val Ser Arg Ile Thr Val Arg Arg Ala Ile Gly Asp Leu Val
70                  75                  80                  85

gcg tcc ggc agg ttg aag cga gct cgc ggc aaa ggt acc ttc gtg gcc   403
Ala Ser Gly Arg Leu Lys Arg Ala Arg Gly Lys Gly Thr Phe Val Ala
                90                  95                  100

cac tcg ccg ttg att tcc cgc ctg cat ttg gcc tcg ttt tcc gca gag   451
His Ser Pro Leu Ile Ser Arg Leu His Leu Ala Ser Phe Ser Ala Glu
            105                 110                 115

atg gcc gcc cag aag cta tcg gct acc agc agg att ttg agt tct tcc   499
Met Ala Ala Gln Lys Leu Ser Ala Thr Ser Arg Ile Leu Ser Ser Ser
        120                 125                 130

cgc ggt ccc gcc cca gat gat att gct gat ttc ttt ggt acc gat cgc   547
Arg Gly Pro Ala Pro Asp Asp Ile Ala Asp Phe Phe Gly Thr Asp Arg
    135                 140                 145

gcg gcc cag cac atc acg ttg cgc cgc ctg cgc ttt gga aat ggt cga   595
Ala Ala Gln His Ile Thr Leu Arg Arg Leu Arg Phe Gly Asn Gly Arg
150                 155                 160                 165

ccc tat gcc att gac aac ggt tgg tac aac tcc gaa ttc gca cct gac     643
Pro Tyr Ala Ile Asp Asn Gly Trp Tyr Asn Ser Glu Phe Ala Pro Asp
                170                 175                 180

ctg ctg gaa aat gat gtg tac aac tcc gtg tac tcc atc ctg gac cgc     691
Leu Leu Glu Asn Asp Val Tyr Asn Ser Val Tyr Ser Ile Leu Asp Arg
            185                 190                 195

gtc tat ggc gtc ccc gtc acc cag gcc gag caa acg gtc acc gcc gta     739
Val Tyr Gly Val Pro Val Thr Gln Ala Glu Gln Thr Val Thr Ala Val
        200                 205                 210

gca gcc gac gaa gac acc gca cgg ctt ctg gac gtc acc ccc ggc gcc     787
Ala Ala Asp Glu Asp Thr Ala Arg Leu Leu Asp Val Thr Pro Gly Ala
    215                 220                 225

cca ctc ctt cgt atc ctt cga cag tca ctt tct ggc gat aag ccc gtg     835
Pro Leu Leu Arg Ile Leu Arg Gln Ser Leu Ser Gly Asp Lys Pro Val
230                 235                 240                 245

gaa tgg tgc gtt tcc ttg tac cga acc gac cga tat tct tta aaa aca     883
Glu Trp Cys Val Ser Leu Tyr Arg Thr Asp Arg Tyr Ser Leu Lys Thr
                250                 255                 260

ttg gtt aca cgc tcc gaa gat ctc tgacgtgaac ccattttggt ggc           930
Leu Val Thr Arg Ser Glu Asp Leu
            265


<210> 58
<211> 269
<212> PRT
<213> Corynebacterium glutamicum

<400> 58
Met Asn Thr Met Pro Asp Gln Pro Leu Asn Gln Asp Gly Phe Pro Thr
1               5                   10                  15

Ala Ser Lys Gly Val Glu Pro Asp Asn Leu Pro Asp Arg Val Leu Val
            20                  25                  30

Asp Gly Leu Lys Pro Lys His Gln Gln Leu Arg Glu Ile Leu Glu Glu
        35                  40                  45

Ile Cys Thr Thr Gln Leu Gln Pro Gly Asp Met Leu Pro Gly Glu Arg
    50                  55                  60

Ile Leu Glu Glu Lys Tyr Gly Val Ser Arg Ile Thr Val Arg Arg Ala
65                  70                  75                  80

Ile Gly Asp Leu Val Ala Ser Gly Arg Leu Lys Arg Ala Arg Gly Lys
                85                  90                  95

Gly Thr Phe Val Ala His Ser Pro Leu Ile Ser Arg Leu His Leu Ala
            100                 105                 110

Ser Phe Ser Ala Glu Met Ala Ala Gln Lys Leu Ser Ala Thr Ser Arg
        115                 120                 125

Ile Leu Ser Ser Ser Arg Gly Pro Ala Pro Asp Asp Ile Ala Asp Phe
    130                 135                 140

Phe Gly Thr Asp Arg Ala Ala Gln His Ile Thr Leu Arg Arg Leu Arg
145                 150                 155                 160

Phe Gly Asn Gly Arg Pro Tyr Ala Ile Asp Asn Gly Trp Tyr Asn Ser
                165                 170                 175

Glu Phe Ala Pro Asp Leu Leu Glu Asn Asp Val Tyr Asn Ser Val Tyr
            180                 185                 190

Ser Ile Leu Asp Arg Val Tyr Gly Val Pro Val Thr Gln Ala Glu Gln
        195                 200                 205

Thr Val Thr Ala Val Ala Ala Asp Glu Asp Thr Ala Arg Leu Leu Asp
    210                 215                 220

Val Thr Pro Gly Ala Pro Leu Leu Arg Ile Leu Arg Gln Ser Leu Ser
225                 230                 235                 240

Gly Asp Lys Pro Val Glu Trp Cys Val Ser Leu Tyr Arg Thr Asp Arg
                245                 250                 255

Tyr Ser Leu Lys Thr Leu Val Thr Arg Ser Glu Asp Leu
            260                 265

<210> 59
<211> 1968
<212> DNA
<213> Corynebacterium glutamicum

<220>
<221> CDS
<222> (101)..(1945)
<223> RXA00880

<400> 59
tccatgtggt taaagatatg cctaaagatc tgaccaaaaa cgtgactaaa gacgtgacga 60

cacaagtaca gccaaattaa aggaaaggtt gaatttgacc atg act tca cct aat   115
                                            Met Thr Ser Pro Asn
                                            1               5

acc ctg cag gaa tac act gaa cct gcc aag tac acc atc gga gaa tct   163
Thr Leu Gln Glu Tyr Thr Glu Pro Ala Lys Tyr Thr Ile Gly Glu Ser
                10                  15                  20

gaa acc tgc ctg acc gcc ctt cta gat cag att aag act cga cct tac   211
Glu Thr Cys Leu Thr Ala Leu Leu Asp Gln Ile Lys Thr Arg Pro Tyr
            25                  30                  35

gga gtt ttg ttc agc aag cct gcc aac tat gag tgg gtg aat gta act   259
Gly Val Leu Phe Ser Lys Pro Ala Asn Tyr Glu Trp Val Asn Val Thr
        40                  45                  50

gcc aaa gaa ttt cag gac gag gtt ttt gcg gtt gca aaa gga att att   307
Ala Lys Glu Phe Gln Asp Glu Val Phe Ala Val Ala Lys Gly Ile Ile
    55                  60                  65

tca gtc ggc gta gag cag gga gac cgt gtc gcg ctg ctg tcc aat act   355
Ser Val Gly Val Glu Gln Gly Asp Arg Val Ala Leu Leu Ser Asn Thr

70                    75                    80                    85

cgc tat gag tgg gct gtg ctt gat ttc gct atc tgg gcc gct ggc gca     403
Arg Tyr Glu Trp Ala Val Leu Asp Phe Ala Ile Trp Ala Ala Gly Ala
                90                  95                  100

gtg agc gtg cct atc tac agc tcc tct tca ctg tcc caa att gag tgg     451
Val Ser Val Pro Ile Tyr Ser Ser Ser Ser Leu Ser Gln Ile Glu Trp
            105                 110                 115

atc att gag gat tcc ggc gct gtt ttg gcc att acc gaa acc cct gat     499
Ile Ile Glu Asp Ser Gly Ala Val Leu Ala Ile Thr Glu Thr Pro Asp
        120                 125                 130

cat acc gac ttg atg aag aac ctg gtc atc ggt gaa gac gga act cca     547
His Thr Asp Leu Met Lys Asn Leu Val Ile Gly Glu Asp Gly Thr Pro
    135                 140                 145

gcg att aag ggt tca cct tcc aag ctg cgc cgc att cta gag atc aac     595
Ala Ile Lys Gly Ser Pro Ser Lys Leu Arg Arg Ile Leu Glu Ile Asn
150                 155                 160                 165

tct tcg gcg ttg gag acc ttg aag ttt gag ggc cgc gag ctt tct gat     643
Ser Ser Ala Leu Glu Thr Leu Lys Phe Glu Gly Arg Glu Leu Ser Asp
                170                 175                 180

gag ctg gtg tgg gaa cgc att cat gca acc aag gcc gct gac ctg gcg     691
Glu Leu Val Trp Glu Arg Ile His Ala Thr Lys Ala Ala Asp Leu Ala
            185                 190                 195

tct ttg gtg tac acc tct ggc aca act ggt agg ccg aag ggc tgc gag     739
Ser Leu Val Tyr Thr Ser Gly Thr Thr Gly Arg Pro Lys Gly Cys Glu
        200                 205                 210

ttg tcc cac tac cac tgg ttg gct gag gtc cga gcg ctg atc acc aat     787
Leu Ser His Tyr His Trp Leu Ala Glu Val Arg Ala Leu Ile Thr Asn
    215                 220                 225

gac atc gga gcg atc gcg atg cca ggt tca agg ttg ctc acc ttc ctt     835
Asp Ile Gly Ala Ile Ala Met Pro Gly Ser Arg Leu Leu Thr Phe Leu
230                 235                 240                 245

cct ttg gcg cac gtt ctt gct cgc gca gtg cac ttg gcc ttc gct gtc     883
Pro Leu Ala His Val Leu Ala Arg Ala Val His Leu Ala Phe Ala Val
                250                 255                 260

acc ggt gca acc cag tcc cac tgg tct gat ttc agc acc ctt act ttg     931
Thr Gly Ala Thr Gln Ser His Trp Ser Asp Phe Ser Thr Leu Thr Leu
            265                 270                 275

gaa ctg cag cgt tcc cgc ccg aac ctg att ttg ggt gtt cca cgc gtg     979
Glu Leu Gln Arg Ser Arg Pro Asn Leu Ile Leu Gly Val Pro Arg Val
        280                 285                 290

ttt gaa aag gtc cgc aac gcc gct gct gct aat gct gct gac ggt ggc     1027
Phe Glu Lys Val Arg Asn Ala Ala Ala Ala Asn Ala Ala Asp Gly Gly
    295                 300                 305

gca atc aag cgc atc atg ttt gag cgt gcc gaa aag gcg gcc att gaa     1075
Ala Ile Lys Arg Ile Met Phe Glu Arg Ala Glu Lys Ala Ala Ile Glu
310                 315                 320                 325

tac tcc atg gct ctt gat act gca gaa ggc cca agc aag tcc cag gtt 1123
Tyr Ser Met Ala Leu Asp Thr Ala Glu Gly Pro Ser Lys Ser Gln Val
                330                 335                 340

atg gca cat aaa gcg ttt gac aag ctg gtg tac tcc aag atc cgt gca 1171
Met Ala His Lys Ala Phe Asp Lys Leu Val Tyr Ser Lys Ile Arg Ala
            345                 350                 355

gct gtc ggt ggc gat gtg cag tac gcc atc acc ggt ggt tca gcg atg 1219
Ala Val Gly Gly Asp Val Gln Tyr Ala Ile Thr Gly Gly Ser Ala Met
        360                 365                 370

ggg cag gag ctg ctg cac ttc ttc cgc ggt gtg ggc atg acc atc tac 1267
Gly Gln Glu Leu Leu His Phe Phe Arg Gly Val Gly Met Thr Ile Tyr
    375                 380                 385

gaa ggt tat ggt ctg acg gaa tct gcg gct gct gca gcg gtg gac ttc 1315
Glu Gly Tyr Gly Leu Thr Glu Ser Ala Ala Ala Ala Ala Val Asp Phe
390                 395                 400                 405

act gat caa aag atc ggc act gtg ggt aag ccg atg ggt ggc atg acc 1363
Thr Asp Gln Lys Ile Gly Thr Val Gly Lys Pro Met Gly Gly Met Thr
                410                 415                 420

atc aag atc aat gaa gat ggc gaa atc atg cta aaa ggc gag atg ttg 1411
Ile Lys Ile Asn Glu Asp Gly Glu Ile Met Leu Lys Gly Glu Met Leu
            425                 430                 435

ttc cag gga tat tgg aac aac cca gaa gcc aca gca gaa gcc ctc cac 1459
Phe Gln Gly Tyr Trp Asn Asn Pro Glu Ala Thr Ala Glu Ala Leu His
        440                 445                 450

gac ggt tgg ttc aac acc ggc gat ctg ggt gag ctg ttg gag tct gga 1507
Asp Gly Trp Phe Asn Thr Gly Asp Leu Gly Glu Leu Leu Glu Ser Gly
    455                 460                 465

cac ctg gtg atc acc gga cgt aag aaa gat ctg atc gtg acc gcg ggc 1555
His Leu Val Ile Thr Gly Arg Lys Lys Asp Leu Ile Val Thr Ala Gly
470                 475                 480                 485

ggc aag aac gtt tcc cca gga ccc atg gaa gac atc atc cgc gca cac 1603
Gly Lys Asn Val Ser Pro Gly Pro Met Glu Asp Ile Ile Arg Ala His
                490                 495                 500

cca ctg gtc agc cag gcc atg gtg gtg ggc gat ggt aaa cca ttc gtt 1651
Pro Leu Val Ser Gln Ala Met Val Val Gly Asp Gly Lys Pro Phe Val
            505                 510                 515

ggc ctg ctg gtg acc ttg gat cca gat atg ttg aag cgg tgg aag ctg 1699
Gly Leu Leu Val Thr Leu Asp Pro Asp Met Leu Lys Arg Trp Lys Leu
        520                 525                 530

aac cac aac att gcg gaa tcc cgc acg gtt tct gag att gct act gat 1747
Asn His Asn Ile Ala Glu Ser Arg Thr Val Ser Glu Ile Ala Thr Asp
    535                 540                 545

cct gca ctg cgt gcg gaa atc cag gat gca gtc aac aac gct aat gcc 1795
Pro Ala Leu Arg Ala Glu Ile Gln Asp Ala Val Asn Asn Ala Asn Ala
550                 555                 560                 565

acg gtg tct cat tca gag gcg atc aag cgg ttc tac atc ctt gat cgc 1843
Thr Val Ser His Ser Glu Ala Ile Lys Arg Phe Tyr Ile Leu Asp Arg
                570                 575                 580

gac ctg acc gag gaa gcc gac gag ctg acc cca acg ctg aag gtc aag 1891
Asp Leu Thr Glu Glu Ala Asp Glu Leu Thr Pro Thr Leu Lys Val Lys
            585                 590                 595

cgc aac gtt gtt gtt cgc cgt tac gca gac gcc atc gac cac atc tac 1939
Arg Asn Val Val Val Arg Arg Tyr Ala Asp Ala Ile Asp His Ile Tyr
        600                 605                 610

aac cga tgagtaacac agagacccaa ttt 1968
Asn Arg
    615

<210> 60
<211> 615
<212> PRT
<213> Corynebacterium glutamicum

<400> 60
Met Thr Ser Pro Asn Thr Leu Gln Glu Tyr Thr Glu Pro Ala Lys Tyr
1               5                   10                  15

Thr Ile Gly Glu Ser Glu Thr Cys Leu Thr Ala Leu Leu Asp Gln Ile
            20                  25                  30

Lys Thr Arg Pro Tyr Gly Val Leu Phe Ser Lys Pro Ala Asn Tyr Glu
        35                  40                  45

Trp Val Asn Val Thr Ala Lys Glu Phe Gln Asp Glu Val Phe Ala Val
    50                  55                  60

Ala Lys Gly Ile Ile Ser Val Gly Val Glu Gln Gly Asp Arg Val Ala
65                  70                  75                  80

Leu Leu Ser Asn Thr Arg Tyr Glu Trp Ala Val Leu Asp Phe Ala Ile
                85                  90                  95

Trp Ala Ala Gly Ala Val Ser Val Pro Ile Tyr Ser Ser Ser Ser Leu
            100                 105                 110

Ser Gln Ile Glu Trp Ile Ile Glu Asp Ser Gly Ala Val Leu Ala Ile
        115                 120                 125

Thr Glu Thr Pro Asp His Thr Asp Leu Met Lys Asn Leu Val Ile Gly
    130                 135                 140

Glu Asp Gly Thr Pro Ala Ile Lys Gly Ser Pro Ser Lys Leu Arg Arg
145                 150                 155                 160

Ile Leu Glu Ile Asn Ser Ser Ala Leu Glu Thr Leu Lys Phe Glu Gly
                165                 170                 175

Arg Glu Leu Ser Asp Glu Leu Val Trp Glu Arg Ile His Ala Thr Lys
            180                 185                 190

Ala Ala Asp Leu Ala Ser Leu Val Tyr Thr Ser Gly Thr Thr Gly Arg
        195                 200                 205

Pro Lys Gly Cys Glu Leu Ser His Tyr His Trp Leu Ala Glu Val Arg
    210                 215                 220

Ala Leu Ile Thr Asn Asp Ile Gly Ala Ile Ala Met Pro Gly Ser Arg
225                 230                 235                 240

Leu Leu Thr Phe Leu Pro Leu Ala His Val Leu Ala Arg Ala Val His
                245                 250                 255

Leu Ala Phe Ala Val Thr Gly Ala Thr Gln Ser His Trp Ser Asp Phe
            260                 265                 270

Ser Thr Leu Thr Leu Glu Leu Gln Arg Ser Arg Pro Asn Leu Ile Leu
        275                 280                 285

Gly Val Pro Arg Val Phe Glu Lys Val Arg Asn Ala Ala Ala Ala Asn
    290                 295                 300

Ala Ala Asp Gly Gly Ala Ile Lys Arg Ile Met Phe Glu Arg Ala Glu
305                 310                 315                 320

Lys Ala Ala Ile Glu Tyr Ser Met Ala Leu Asp Thr Ala Glu Gly Pro
                325                 330                 335

Ser Lys Ser Gln Val Met Ala His Lys Ala Phe Asp Lys Leu Val Tyr
            340                 345                 350

Ser Lys Ile Arg Ala Ala Val Gly Gly Asp Val Gln Tyr Ala Ile Thr
        355                 360                 365

Gly Gly Ser Ala Met Gly Gln Glu Leu Leu His Phe Phe Arg Gly Val
    370                 375                 380

Gly Met Thr Ile Tyr Glu Gly Tyr Gly Leu Thr Glu Ser Ala Ala Ala
385                 390                 395                 400

Ala Ala Val Asp Phe Thr Asp Gln Lys Ile Gly Thr Val Gly Lys Pro
                405                 410                 415

Met Gly Gly Met Thr Ile Lys Ile Asn Glu Asp Gly Glu Ile Met Leu
            420                 425                 430

Lys Gly Glu Met Leu Phe Gln Gly Tyr Trp Asn Asn Pro Glu Ala Thr
        435                 440                 445

Ala Glu Ala Leu His Asp Gly Trp Phe Asn Thr Gly Asp Leu Gly Glu
    450                 455                 460

Leu Leu Glu Ser Gly His Leu Val Ile Thr Gly Arg Lys Lys Asp Leu
465                 470                 475                 480

Ile Val Thr Ala Gly Gly Lys Asn Val Ser Pro Gly Pro Met Glu Asp
                485                 490                 495

Ile Ile Arg Ala His Pro Leu Val Ser Gln Ala Met Val Val Gly Asp
            500                 505                 510

Gly Lys Pro Phe Val Gly Leu Leu Val Thr Leu Asp Pro Asp Met Leu
        515                 520                 525

Lys Arg Trp Lys Leu Asn His Asn Ile Ala Glu Ser Arg Thr Val Ser
    530                 535                 540

Glu Ile Ala Thr Asp Pro Ala Leu Arg Ala Glu Ile Gln Asp Ala Val
545                 550                 555                 560

Asn Asn Ala Asn Ala Thr Val Ser His Ser Glu Ala Ile Lys Arg Phe
                565                 570                 575

Tyr Ile Leu Asp Arg Asp Leu Thr Glu Glu Ala Asp Glu Leu Thr Pro
            580                 585                 590

Thr Leu Lys Val Lys Arg Asn Val Val Val Arg Arg Tyr Ala Asp Ala
        595                 600                 605

Ile Asp His Ile Tyr Asn Arg
    610                 615


<210> 61
<211> 1047
<212> DNA
<213> Corynebacterium glutamicum

<220>
<221> CDS
<222> (101)..(1024)
<223> RXA01060

<400> 61
ttccatttac acatctgttc gactcgcgcc cgaatgatgt cgcatcaaca tgagaagcta 60

cttccatact tttactgatt gataagaatg gacaaagaca atg aac cgc aca ctc   115
                                           Met Asn Arg Thr Leu
                                           1               5

cga aca ctt ggc tgg ctt gct gcc gta att caa gaa gat ccg gag ccc   163
Arg Thr Leu Gly Trp Leu Ala Ala Val Ile Gln Glu Asp Pro Glu Pro
                10                  15                  20

tgg ttc acc act gat ccc gac acc gat tat gtc cct tat gta aat agt   211
Trp Phe Thr Thr Asp Pro Asp Thr Asp Tyr Val Pro Tyr Val Asn Ser
            25                  30                  35

ttc tca ttt gaa tcc ctt tcc ctc gtt cca gat gct ctg atg ctg ctc   259
Phe Ser Phe Glu Ser Leu Ser Leu Val Pro Asp Ala Leu Met Leu Leu
        40                  45                  50

aaa cgt tct ctt cac ctt gcg atg gag caa cag gac ctc cca gtg aag   307
Lys Arg Ser Leu His Leu Ala Met Glu Gln Gln Asp Leu Pro Val Lys
    55                  60                  65

gat cta caa gaa gca ctg agg cac gta cta gtt ttc aaa ttc cac ttc   355
Asp Leu Gln Glu Ala Leu Arg His Val Leu Val Phe Lys Phe His Phe
70                  75                  80                  85

agg gaa gaa tgg gag ctg gaa tta gca tgg gac tcc gag cgg acc aag   403
Arg Glu Glu Trp Glu Leu Glu Leu Ala Trp Asp Ser Glu Arg Thr Lys
                90                  95                  100

tct gcg gtg aga att att gag agc aca aaa gaa tct ctt gcc gat caa   451

Ser Ala Val Arg Ile Ile Glu Ser Thr Lys Glu Ser Leu Ala Asp Gln
            105                 110                 115

tac aga gat tac aaa tac gca ttc ttg cct gag cta att ttc cag gaa     499
Tyr Arg Asp Tyr Lys Tyr Ala Phe Leu Pro Glu Leu Ile Phe Gln Glu
        120                 125                 130

tca cgc ggg atc ttt gac ttt gag ctg gag gga tac acc ctc aaa gta     547
Ser Arg Gly Ile Phe Asp Phe Glu Leu Glu Gly Tyr Thr Leu Lys Val
    135                 140                 145

gga caa agc acg ctt tcc att cct tgg gac atg atc gcc aat ggt tat     595
Gly Gln Ser Thr Leu Ser Ile Pro Trp Asp Met Ile Ala Asn Gly Tyr
150                 155                 160                 165

gtc cct gca agt ctt cgg aat ttt gga gaa tta atg gac cgt gac acc     643
Val Pro Ala Ser Leu Arg Asn Phe Gly Glu Leu Met Asp Arg Asp Thr
                170                 175                 180

gga gat ctt gat gca gat ccc att ctg aga cct cga gaa ctc aaa ttt     691
Gly Asp Leu Asp Ala Asp Pro Ile Leu Arg Pro Arg Glu Leu Lys Phe
            185                 190                 195

gag atc cat aat tgt cca gat ctt aat cct tgg ata atg cgg gaa act     739
Glu Ile His Asn Cys Pro Asp Leu Asn Pro Trp Ile Met Arg Glu Thr
        200                 205                 210

ttt gat ttc atg atg gaa atc gca act gaa aca gga tgg ttc cat gct     787
Phe Asp Phe Met Met Glu Ile Ala Thr Glu Thr Gly Trp Phe His Ala
    215                 220                 225

ctc aac ccc gca tat aat tcc gtc tac acc tac gat ctt att tct cgc     835
Leu Asn Pro Ala Tyr Asn Ser Val Tyr Thr Tyr Asp Leu Ile Ser Arg
230                 235                 240                 245

atg cca gat ttc ctt gtt gaa ggg agc ttt cgt ccg cat tca gtg aaa     883
Met Pro Asp Phe Leu Val Glu Gly Ser Phe Arg Pro His Ser Val Lys
                250                 255                 260

cga tct tgg gaa aaa att caa aag ata gct aaa gct gtt gag tct tat     931
Arg Ser Trp Glu Lys Ile Gln Lys Ile Ala Lys Ala Val Glu Ser Tyr
            265                 270                 275

gca tct cac gat tac tgc atg tca acg ctg aca cat gac tac agg gca     979
Ala Ser His Asp Tyr Cys Met Ser Thr Leu Thr His Asp Tyr Arg Ala
        280                 285                 290

atc gag tta tcc ctg acg cct aca aaa act gag gaa cca agc aca         1024
Ile Glu Leu Ser Leu Thr Pro Thr Lys Thr Glu Glu Pro Ser Thr
    295                 300                 305

taagaaaacc tcgcctgccc act                                           1047

<210> 62
<211> 308
<212> PRT
<213> Corynebacterium glutamicum

<400> 62
Met Asn Arg Thr Leu Arg Thr Leu Gly Trp Leu Ala Ala Val Ile Gln

1                 5                   10                  15

Glu Asp Pro Glu Pro Trp Phe Thr Thr Asp Pro Asp Thr Asp Tyr Val
            20                  25                  30

Pro Tyr Val Asn Ser Phe Ser Phe Glu Ser Leu Ser Leu Val Pro Asp
        35                  40                  45

Ala Leu Met Leu Leu Lys Arg Ser Leu His Leu Ala Met Glu Gln Gln
    50                  55                  60

Asp Leu Pro Val Lys Asp Leu Gln Glu Ala Leu Arg His Val Leu Val
65                  70                  75                  80

Phe Lys Phe His Phe Arg Glu Glu Trp Glu Leu Glu Leu Ala Trp Asp
                85                  90                  95

Ser Glu Arg Thr Lys Ser Ala Val Arg Ile Ile Glu Ser Thr Lys Glu
            100                 105                 110

Ser Leu Ala Asp Gln Tyr Arg Asp Tyr Lys Tyr Ala Phe Leu Pro Glu
        115                 120                 125

Leu Ile Phe Gln Glu Ser Arg Gly Ile Phe Asp Phe Glu Leu Glu Gly
    130                 135                 140

Tyr Thr Leu Lys Val Gly Gln Ser Thr Leu Ser Ile Pro Trp Asp Met
145                 150                 155                 160

Ile Ala Asn Gly Tyr Val Pro Ala Ser Leu Arg Asn Phe Gly Glu Leu
                165                 170                 175

Met Asp Arg Asp Thr Gly Asp Leu Asp Ala Asp Pro Ile Leu Arg Pro
            180                 185                 190

Arg Glu Leu Lys Phe Glu Ile His Asn Cys Pro Asp Leu Asn Pro Trp
        195                 200                 205

Ile Met Arg Glu Thr Phe Asp Phe Met Met Glu Ile Ala Thr Glu Thr
    210                 215                 220

Gly Trp Phe His Ala Leu Asn Pro Ala Tyr Asn Ser Val Tyr Thr Tyr
225                 230                 235                 240

Asp Leu Ile Ser Arg Met Pro Asp Phe Leu Val Glu Gly Ser Phe Arg
                245                 250                 255

Pro His Ser Val Lys Arg Ser Trp Glu Lys Ile Gln Lys Ile Ala Lys
            260                 265                 270

Ala Val Glu Ser Tyr Ala Ser His Asp Tyr Cys Met Ser Thr Leu Thr
        275                 280                 285

His Asp Tyr Arg Ala Ile Glu Leu Ser Leu Thr Pro Thr Lys Thr Glu
    290                 295                 300

Glu Pro Ser Thr
305

<210> 63

<211> 1848
<212> DNA
<213> Corynebacterium glutamicum

<220>
<221> CDS
<222> (101)..(1825)
<223> RXN01722

<400> 63
ctcattcccc tcgctaaaag ctgcataaag tttcgacgtt ttcaaagttg attgcttgca 60

ctgtcgttgc gtgtcgcatg ccccggctat tgttgattgc atg ctc agc acg atg 115
Met Leu Ser Thr Met
1               5

cag gac gtc cca ctg tca tta acc aga atc ctc gag tac ggg tcc act 163
Gln Asp Val Pro Leu Ser Leu Thr Arg Ile Leu Glu Tyr Gly Ser Thr
                10                  15                  20

gtt cac ggt gat act ttg atc acc acg tgg ggc ggt gcc gat ggc att 211
Val His Gly Asp Thr Leu Ile Thr Thr Trp Gly Gly Ala Asp Gly Ile
            25                  30                  35

gaa caa gca cag caa act ttt agt gct gtg ggg gct aga gct gcg gct 259
Glu Gln Ala Gln Gln Thr Phe Ser Ala Val Gly Ala Arg Ala Ala Ala
        40                  45                  50

ttg gct cat gcg ctg cat gat tct tta ggt att act ggg gat cag cga 307
Leu Ala His Ala Leu His Asp Ser Leu Gly Ile Thr Gly Asp Gln Arg
    55                  60                  65

gtg gcg tcg atg ctc tat aac tgc gcg gaa cac atg gaa act atg ttt 355
Val Ala Ser Met Leu Tyr Asn Cys Ala Glu His Met Glu Thr Met Phe
70                  75                  80                  85

gca gtc gca tgc atg ggt gcc gtg ttt aat ccg ctg aac aag cag ttg 403
Ala Val Ala Cys Met Gly Ala Val Phe Asn Pro Leu Asn Lys Gln Leu
                90                  95                  100

atg aat gat cag att gtg ttc att ctc aat cac tct gaa gct gaa gtg 451
Met Asn Asp Gln Ile Val Phe Ile Leu Asn His Ser Glu Ala Glu Val
            105                 110                 115

gtt atc gct gat ccg cgc atg gct gaa caa ttg ggt gag atc tta aaa 499
Val Ile Ala Asp Pro Arg Met Ala Glu Gln Leu Gly Glu Ile Leu Lys
        120                 125                 130

gaa aca cca aaa gtt cgt gcc gtg gtg ttt att gga ccg aat gat ttc 547
Glu Thr Pro Lys Val Arg Ala Val Val Phe Ile Gly Pro Asn Asp Phe
    135                 140                 145

tct agc gcg gcg gcc cac atg ccg gag gga atg aag ctg tat tcc tat 595
Ser Ser Ala Ala Ala His Met Pro Glu Gly Met Lys Leu Tyr Ser Tyr
150                 155                 160                 165

gaa gcg ctc ctt gac ggc cgt tcc act gtt tac aac tgg ccc gag cag 643
Glu Ala Leu Leu Asp Gly Arg Ser Thr Val Tyr Asn Trp Pro Glu Gln
                170                 175                 180

gat gaa cgc act gct gct gca att tgc tat tcc acc ggt aca tcg gga 691

Asp Glu Arg Thr Ala Ala Ala Ile Cys Tyr Ser Thr Gly Thr Ser Gly
            185                 190                 195

ccg ccg aag ggt gtg gtg tat tcc cac cgc tcg ctt tat tta cag tcg 739
Pro Pro Lys Gly Val Val Tyr Ser His Arg Ser Leu Tyr Leu Gln Ser
        200                 205                 210

ttg agc ttg cgc acc acg gat tcc ctc gca gtg gaa cac ggc gaa acg 787
Leu Ser Leu Arg Thr Thr Asp Ser Leu Ala Val Glu His Gly Glu Thr
    215                 220                 225

ttc ctg tgc tgt gtg ccg att tac cac gtc ctc agc tgg ggc gtg ccg 835
Phe Leu Cys Cys Val Pro Ile Tyr His Val Leu Ser Trp Gly Val Pro
230                 235                 240                 245

atc gca gcg ttt atg tcc ggc act ccc ctg gtg ctt cct gga ccg gat 883
Ile Ala Ala Phe Met Ser Gly Thr Pro Leu Val Leu Pro Gly Pro Asp
                250                 255                 260

ctt tct gcg ccg aca ttg gca aag atc att tcc acc acg ctg cct cgc 931
Leu Ser Ala Pro Thr Leu Ala Lys Ile Ile Ser Thr Thr Leu Pro Arg
            265                 270                 275

gtg gcg cat ggc gtg cct act ctc tgg att cag ctg atg gtt cac tac 979
Val Ala His Gly Val Pro Thr Leu Trp Ile Gln Leu Met Val His Tyr
        280                 285                 290

ctg aaa aat ccc cca gag cgt atg tct ctg cgt gag cta tac gtg ggc 1027
Leu Lys Asn Pro Pro Glu Arg Met Ser Leu Arg Glu Leu Tyr Val Gly
    295                 300                 305

ggt tct gcg gtg cct cca atc gtg atc acc atg tgg gag cag cgc tat 1075
Gly Ser Ala Val Pro Pro Ile Val Ile Thr Met Trp Glu Gln Arg Tyr
310                 315                 320                 325

ggc gtg gat gtt gtc cac gtg tgg ggt atg acg gaa acc tcc acc gtg 1123
Gly Val Asp Val Val His Val Trp Gly Met Thr Glu Thr Ser Thr Val
                330                 335                 340

ggt act gtg tct cgc cca cca tca ggt gtt tct ggt gaa agc cgg tgg 1171
Gly Thr Val Ser Arg Pro Pro Ser Gly Val Ser Gly Glu Ser Arg Trp
            345                 350                 355

aat tat cga gtc tcc cag ggc cga ttc ccc gcc tcc ctg cag tac cgc 1219
Asn Tyr Arg Val Ser Gln Gly Arg Phe Pro Ala Ser Leu Gln Tyr Arg
        360                 365                 370

att gtc aac gac ggc cag gtc atg gcg tcc acc gac cgc aac gag ggc 1267
Ile Val Asn Asp Gly Gln Val Met Ala Ser Thr Asp Arg Asn Glu Gly
    375                 380                 385

gag att cag gtc cgc ggt ccg tgg gtg act gca agt tac ttc cac ccc 1315
Glu Ile Gln Val Arg Gly Pro Trp Val Thr Ala Ser Tyr Phe His Pro
390                 395                 400                 405

gat gtg gaa aaa gaa ggt ggc acc gcc tca aca ttc cgc gac cat gac 1363
Asp Val Glu Lys Glu Gly Gly Thr Ala Ser Thr Phe Arg Asp His Asp
                410                 415                 420

gtt gaa gag gaa aac gat gag ctc ttc acc gcc gac ggc tgg ctg cgc 1411
Val Glu Glu Glu Asn Asp Glu Leu Phe Thr Ala Asp Gly Trp Leu Arg

            425                 430                 435

acc ggg gac gtt gga tct gtc acc agc gat gga ttc ctc acc atc caa 1459
Thr Gly Asp Val Gly Ser Val Thr Ser Asp Gly Phe Leu Thr Ile Gln
        440                 445                 450

gac cgc gcc cgc gat gtc atc cgt tct ggc ggc gag tgg att tac tcc 1507
Asp Arg Ala Arg Asp Val Ile Arg Ser Gly Gly Glu Trp Ile Tyr Ser
    455                 460                 465

gct cag ctg gaa aac ctc atc gtg gct acg gaa gag gtt gtc gaa tgc 1555
Ala Gln Leu Glu Asn Leu Ile Val Ala Thr Glu Glu Val Val Glu Cys
470                 475                 480                 485

gcc gtc att ggc ttc ccc gat gac aag tgg gtg gaa cgt ccc ctc gca 1603
Ala Val Ile Gly Phe Pro Asp Asp Lys Trp Val Glu Arg Pro Leu Ala
                490                 495                 500

gtc acc atg ctc tac ccc ggc att gaa cgc acc cgg gaa acc gcc gag 1651
Val Thr Met Leu Tyr Pro Gly Ile Glu Arg Thr Arg Glu Thr Ala Glu
            505                 510                 515

cgc ctc cgc gat caa ctt cgc gac cgc cta ccc aac tgg atg ctg cca 1699
Arg Leu Arg Asp Gln Leu Arg Asp Arg Leu Pro Asn Trp Met Leu Pro
        520                 525                 530

gaa tat tgg aca ttt gtt gat gag gtg gat aaa acc tcc gtc ggt aaa 1747
Glu Tyr Trp Thr Phe Val Asp Glu Val Asp Lys Thr Ser Val Gly Lys
    535                 540                 545

tat gac aag aag gac ctc cgc aac cac ctg cgc aac ggc gat ttc gaa 1795
Tyr Asp Lys Lys Asp Leu Arg Asn His Leu Arg Asn Gly Asp Phe Glu
550                 555                 560                 565

gta att aag ctt aaa ggg cca ggt gaa aaa taacttccct atttattccg 1845
Val Ile Lys Leu Lys Gly Pro Gly Glu Lys
                570                 575

gct 1848

<210> 64
<211> 575
<212> PRT
<213> Corynebacterium glutamicum

<400> 64
Met Leu Ser Thr Met Gln Asp Val Pro Leu Ser Leu Thr Arg Ile Leu
1               5                   10                  15

Glu Tyr Gly Ser Thr Val His Gly Asp Thr Leu Ile Thr Thr Trp Gly
            20                  25                  30

Gly Ala Asp Gly Ile Glu Gln Ala Gln Gln Thr Phe Ser Ala Val Gly
        35                  40                  45

Ala Arg Ala Ala Ala Leu Ala His Ala Leu His Asp Ser Leu Gly Ile
    50                  55                  60

Thr Gly Asp Gln Arg Val Ala Ser Met Leu Tyr Asn Cys Ala Glu His
65                  70                  75                  80

Met Glu Thr Met Phe Ala Val Ala Cys Met Gly Ala Val Phe Asn Pro
                85                  90                  95

Leu Asn Lys Gln Leu Met Asn Asp Gln Ile Val Phe Ile Leu Asn His
            100                 105                 110

Ser Glu Ala Glu Val Val Ile Ala Asp Pro Arg Met Ala Glu Gln Leu
        115                 120                 125

Gly Glu Ile Leu Lys Glu Thr Pro Lys Val Arg Ala Val Val Phe Ile
    130                 135                 140

Gly Pro Asn Asp Phe Ser Ser Ala Ala Ala His Met Pro Glu Gly Met
145                 150                 155                 160

Lys Leu Tyr Ser Tyr Glu Ala Leu Leu Asp Gly Arg Ser Thr Val Tyr
                165                 170                 175

Asn Trp Pro Glu Gln Asp Glu Arg Thr Ala Ala Ala Ile Cys Tyr Ser
            180                 185                 190

Thr Gly Thr Ser Gly Pro Pro Lys Gly Val Val Tyr Ser His Arg Ser
        195                 200                 205

Leu Tyr Leu Gln Ser Leu Ser Leu Arg Thr Thr Asp Ser Leu Ala Val
    210                 215                 220

Glu His Gly Glu Thr Phe Leu Cys Cys Val Pro Ile Tyr His Val Leu
225                 230                 235                 240

Ser Trp Gly Val Pro Ile Ala Ala Phe Met Ser Gly Thr Pro Leu Val
                245                 250                 255

Leu Pro Gly Pro Asp Leu Ser Ala Pro Thr Leu Ala Lys Ile Ile Ser
            260                 265                 270

Thr Thr Leu Pro Arg Val Ala His Gly Val Pro Thr Leu Trp Ile Gln
        275                 280                 285

Leu Met Val His Tyr Leu Lys Asn Pro Pro Glu Arg Met Ser Leu Arg
    290                 295                 300

Glu Leu Tyr Val Gly Gly Ser Ala Val Pro Pro Ile Val Ile Thr Met
305                 310                 315                 320

Trp Glu Gln Arg Tyr Gly Val Asp Val Val His Val Trp Gly Met Thr
                325                 330                 335

Glu Thr Ser Thr Val Gly Thr Val Ser Arg Pro Pro Ser Gly Val Ser
            340                 345                 350

Gly Glu Ser Arg Trp Asn Tyr Arg Val Ser Gln Gly Arg Phe Pro Ala
        355                 360                 365

Ser Leu Gln Tyr Arg Ile Val Asn Asp Gly Gln Val Met Ala Ser Thr
    370                 375                 380

Asp Arg Asn Glu Gly Glu Ile Gln Val Arg Gly Pro Trp Val Thr Ala
385                 390                 395                 400

Ser Tyr Phe His Pro Asp Val Glu Lys Glu Gly Gly Thr Ala Ser Thr
                405                 410                 415

Phe Arg Asp His Asp Val Glu Glu Glu Asn Asp Glu Leu Phe Thr Ala
            420                 425                 430

Asp Gly Trp Leu Arg Thr Gly Asp Val Gly Ser Val Thr Ser Asp Gly
        435                 440                 445

Phe Leu Thr Ile Gln Asp Arg Ala Arg Asp Val Ile Arg Ser Gly Gly
    450                 455                 460

Glu Trp Ile Tyr Ser Ala Gln Leu Glu Asn Leu Ile Val Ala Thr Glu
465                 470                 475                 480

Glu Val Val Glu Cys Ala Val Ile Gly Phe Pro Asp Asp Lys Trp Val
                485                 490                 495

Glu Arg Pro Leu Ala Val Thr Met Leu Tyr Pro Gly Ile Glu Arg Thr
            500                 505                 510

Arg Glu Thr Ala Glu Arg Leu Arg Asp Gln Leu Arg Asp Arg Leu Pro
        515                 520                 525

Asn Trp Met Leu Pro Glu Tyr Trp Thr Phe Val Asp Glu Val Asp Lys
    530                 535                 540

Thr Ser Val Gly Lys Tyr Asp Lys Lys Asp Leu Arg Asn His Leu Arg
545                 550                 555                 560

Asn Gly Asp Phe Glu Val Ile Lys Leu Lys Gly Pro Gly Glu Lys
                565                 570                 575

<210> 65
<211> 1848
<212> DNA
<213> Corynebacterium glutamicum

<220>
<221> CDS
<222> (101)..(1825)
<223> FRXA01722

<400> 65
ctcattcccc tcgctaaaag ctgcataaag tttcgacgtt ttcaaagttg attgcttgca 60

ctgtcgttgc gtgtcgcatg ccccggctat tgttgattgc atg ctc agc acg atg   115
                                           Met Leu Ser Thr Met
                                           1               5

cag gac gtc cca ctg tca tta acc aga atc ctc gag tac ggg tcc act   163
Gln Asp Val Pro Leu Ser Leu Thr Arg Ile Leu Glu Tyr Gly Ser Thr
                10                  15                  20

gtt cac ggt gat act ttg atc acc acg tgg ggc ggt gcc gat ggc att   211
Val His Gly Asp Thr Leu Ile Thr Thr Trp Gly Gly Ala Asp Gly Ile
            25                  30                  35

gaa caa gca cag caa act ttt agt gct gtg ggg gct aga gct gcg gct   259
Glu Gln Ala Gln Gln Thr Phe Ser Ala Val Gly Ala Arg Ala Ala Ala

        40                  45                  50

ttg gct cat gcg ctg cat gat tct tta ggt att act ggg gat cag cga 307
Leu Ala His Ala Leu His Asp Ser Leu Gly Ile Thr Gly Asp Gln Arg
    55                  60                  65

gtg gcg tcg atg ctc tat aac tgc gcg gaa cac atg gaa act atg ttt 355
Val Ala Ser Met Leu Tyr Asn Cys Ala Glu His Met Glu Thr Met Phe
70                  75                  80                  85

gca gtc gca tgc atg ggt gcc gtg ttt aat ccg ctg aac aag cag ttg 403
Ala Val Ala Cys Met Gly Ala Val Phe Asn Pro Leu Asn Lys Gln Leu
                90                  95                  100

atg aat gat cag att gtg ttc att ctc aat cac tct gaa gct gaa gtg 451
Met Asn Asp Gln Ile Val Phe Ile Leu Asn His Ser Glu Ala Glu Val
            105                 110                 115

gtt atc gct gat ccg cgc atg gct gaa caa ttg ggt gag atc tta aaa 499
Val Ile Ala Asp Pro Arg Met Ala Glu Gln Leu Gly Glu Ile Leu Lys
        120                 125                 130

gaa aca cca aaa gtt cgt gcc gtg gtg ttt att gga ccg aat gat ttc 547
Glu Thr Pro Lys Val Arg Ala Val Val Phe Ile Gly Pro Asn Asp Phe
    135                 140                 145

tct agc gcg gcg gcc cac atg ccg gag gga atg aag ctg tat tcc tat 595
Ser Ser Ala Ala Ala His Met Pro Glu Gly Met Lys Leu Tyr Ser Tyr
150                 155                 160                 165

gaa gcg ctc ctt gac ggc cgt tcc act gtt tac aac tgg ccc gag cag 643
Glu Ala Leu Leu Asp Gly Arg Ser Thr Val Tyr Asn Trp Pro Glu Gln
                170                 175                 180

gat gaa cgc act gct gct gca att tgc tat tcc acc ggt aca tcg gga 691
Asp Glu Arg Thr Ala Ala Ala Ile Cys Tyr Ser Thr Gly Thr Ser Gly
            185                 190                 195

ccg ccg aag ggt gtg gtg tat tcc cac cgc tcg ctt tat tta cag tcg 739
Pro Pro Lys Gly Val Val Tyr Ser His Arg Ser Leu Tyr Leu Gln Ser
        200                 205                 210

ttg agc ttg cgc acc acg gat tcc ctc gca gtg gaa cac ggc gaa acg 787
Leu Ser Leu Arg Thr Thr Asp Ser Leu Ala Val Glu His Gly Glu Thr
    215                 220                 225

ttc ctg tgc tgt gtg ccg att tac cac gtc ctc agc tgg ggc gtg ccg 835
Phe Leu Cys Cys Val Pro Ile Tyr His Val Leu Ser Trp Gly Val Pro
230                 235                 240                 245

atc gca gcg ttt atg tcc ggc act ccc ctg gtg ctt cct gga ccg gat 883
Ile Ala Ala Phe Met Ser Gly Thr Pro Leu Val Leu Pro Gly Pro Asp
                250                 255                 260

ctt tct gcg ccg aca ttg gca aag atc att tcc acc acg ctg cct cgc 931
Leu Ser Ala Pro Thr Leu Ala Lys Ile Ile Ser Thr Thr Leu Pro Arg
            265                 270                 275

gtg gcg cat ggc gtg cct act ctc tgg att cag ctg atg gtt cac tac 979
Val Ala His Gly Val Pro Thr Leu Trp Ile Gln Leu Met Val His Tyr
        280                 285                 290

ctg aaa aat ccc cca gag cgt atg tct ctg cgt gag cta tac gtg ggc 1027
Leu Lys Asn Pro Pro Glu Arg Met Ser Leu Arg Glu Leu Tyr Val Gly
    295                 300                 305

ggt tct gcg gtg cct cca atc gtg atc acc atg tgg gag cag cgc tat 1075
Gly Ser Ala Val Pro Pro Ile Val Ile Thr Met Trp Glu Gln Arg Tyr
310                 315                 320                 325

ggc gtg gat gtt gtc cac gtg tgg ggt atg acg gaa acc tcc acc gtg 1123
Gly Val Asp Val Val His Val Trp Gly Met Thr Glu Thr Ser Thr Val
                330                 335                 340

ggt act gtg tct cgc cca cca tca ggt gtt tct ggt gaa agc cgg tgg 1171
Gly Thr Val Ser Arg Pro Pro Ser Gly Val Ser Gly Glu Ser Arg Trp
            345                 350                 355

aat tat cga gtc tcc cag ggc cga ttc ccc gcc tcc ctg cag tac cgc 1219
Asn Tyr Arg Val Ser Gln Gly Arg Phe Pro Ala Ser Leu Gln Tyr Arg
        360                 365                 370

att gtc aac gac ggc cag gtc atg gcg tcc acc gac cgc aac gag ggc 1267
Ile Val Asn Asp Gly Gln Val Met Ala Ser Thr Asp Arg Asn Glu Gly
    375                 380                 385

gag att cag gtc cgc ggt ccg tgg gtg act gca agt tac ttc cac ccc 1315
Glu Ile Gln Val Arg Gly Pro Trp Val Thr Ala Ser Tyr Phe His Pro
390                 395                 400                 405

gat gtg gaa aaa gaa ggt ggc acc gcc tca aca ttc cgc gac cat gac 1363
Asp Val Glu Lys Glu Gly Gly Thr Ala Ser Thr Phe Arg Asp His Asp
                410                 415                 420

gtt gaa gag gaa aac gat gag ctc ttc acc gcc gac ggc tgg ctg cgc 1411
Val Glu Glu Glu Asn Asp Glu Leu Phe Thr Ala Asp Gly Trp Leu Arg
            425                 430                 435

acc ggg gac gtt gga tct gtc acc agc gat gga ttc ctc acc atc caa 1459
Thr Gly Asp Val Gly Ser Val Thr Ser Asp Gly Phe Leu Thr Ile Gln
        440                 445                 450

gac cgc gcc cgc gat gtc atc cgt tct ggc ggc gag tgg att tac tcc 1507
Asp Arg Ala Arg Asp Val Ile Arg Ser Gly Gly Glu Trp Ile Tyr Ser
    455                 460                 465

gct cag ctg gaa aac ctc atc gtg gct acs gaa gag gtt gtc gaa tgc 1555
Ala Gln Leu Glu Asn Leu Ile Val Ala Xaa Glu Glu Val Val Glu Cys
470                 475                 480                 485

gcc gtc att ggc ttc ccc gat gac aag tgg gtg gaa cgt ccc ctc gca 1603
Ala Val Ile Gly Phe Pro Asp Asp Lys Trp Val Glu Arg Pro Leu Ala
                490                 495                 500

gtc acc atg ctc tac ccc ggc att gaa cgc acc cgg gaa acc gcc gag 1651
Val Thr Met Leu Tyr Pro Gly Ile Glu Arg Thr Arg Glu Thr Ala Glu
            505                 510                 515

cgc ctc cgc gat caa ctt cgc gac cgc cta ccc aac tgg atg ctg cca 1699
Arg Leu Arg Asp Gln Leu Arg Asp Arg Leu Pro Asn Trp Met Leu Pro
        520                 525                 530

gaa tat tgg aca ttt gtt gat gag gtg gat aaa acc tcc gtc ggt aaa 1747
Glu Tyr Trp Thr Phe Val Asp Glu Val Asp Lys Thr Ser Val Gly Lys
    535                 540                 545

tat gac aag aag gac ctc cgc aac cac ctg cgc aac ggc gat ttc gaa 1795
Tyr Asp Lys Lys Asp Leu Arg Asn His Leu Arg Asn Gly Asp Phe Glu
550                 555                 560                 565

gta att aag ctt aaa ggg cca ggt gar aaa taacttccct atttattccg 1845
Val Ile Lys Leu Lys Gly Pro Gly Xaa Lys
                570                 575

gct 1848

<210> 66
<211> 575
<212> PRT
<213> Corynebacterium glutamicum

<400> 66
Met Leu Ser Thr Met Gln Asp Val Pro Leu Ser Leu Thr Arg Ile Leu
1               5                   10                  15

Glu Tyr Gly Ser Thr Val His Gly Asp Thr Leu Ile Thr Thr Trp Gly
            20                  25                  30

Gly Ala Asp Gly Ile Glu Gln Ala Gln Gln Thr Phe Ser Ala Val Gly
        35                  40                  45

Ala Arg Ala Ala Ala Leu Ala His Ala Leu His Asp Ser Leu Gly Ile
    50                  55                  60

Thr Gly Asp Gln Arg Val Ala Ser Met Leu Tyr Asn Cys Ala Glu His
65                  70                  75                  80

Met Glu Thr Met Phe Ala Val Ala Cys Met Gly Ala Val Phe Asn Pro
                85                  90                  95

Leu Asn Lys Gln Leu Met Asn Asp Gln Ile Val Phe Ile Leu Asn His
            100                 105                 110

Ser Glu Ala Glu Val Val Ile Ala Asp Pro Arg Met Ala Glu Gln Leu
        115                 120                 125

Gly Glu Ile Leu Lys Glu Thr Pro Lys Val Arg Ala Val Val Phe Ile
    130                 135                 140

Gly Pro Asn Asp Phe Ser Ser Ala Ala Ala His Met Pro Glu Gly Met
145                 150                 155                 160

Lys Leu Tyr Ser Tyr Glu Ala Leu Leu Asp Gly Arg Ser Thr Val Tyr
                165                 170                 175

Asn Trp Pro Glu Gln Asp Glu Arg Thr Ala Ala Ala Ile Cys Tyr Ser
            180                 185                 190

Thr Gly Thr Ser Gly Pro Pro Lys Gly Val Val Tyr Ser His Arg Ser
        195                 200                 205

Leu Tyr Leu Gln Ser Leu Ser Leu Arg Thr Thr Asp Ser Leu Ala Val

210                 215                 220

Glu His Gly Glu Thr Phe Leu Cys Cys Val Pro Ile Tyr His Val Leu
225                 230                 235                 240

Ser Trp Gly Val Pro Ile Ala Ala Phe Met Ser Gly Thr Pro Leu Val
                245                 250                 255

Leu Pro Gly Pro Asp Leu Ser Ala Pro Thr Leu Ala Lys Ile Ile Ser
            260                 265                 270

Thr Thr Leu Pro Arg Val Ala His Gly Val Pro Thr Leu Trp Ile Gln
        275                 280                 285

Leu Met Val His Tyr Leu Lys Asn Pro Pro Glu Arg Met Ser Leu Arg
    290                 295                 300

Glu Leu Tyr Val Gly Gly Ser Ala Val Pro Pro Ile Val Ile Thr Met
305                 310                 315                 320

Trp Glu Gln Arg Tyr Gly Val Asp Val Val His Val Trp Gly Met Thr
                325                 330                 335

Glu Thr Ser Thr Val Gly Thr Val Ser Arg Pro Pro Ser Gly Val Ser
            340                 345                 350

Gly Glu Ser Arg Trp Asn Tyr Arg Val Ser Gln Gly Arg Phe Pro Ala
        355                 360                 365

Ser Leu Gln Tyr Arg Ile Val Asn Asp Gly Gln Val Met Ala Ser Thr
    370                 375                 380

Asp Arg Asn Glu Gly Glu Ile Gln Val Arg Gly Pro Trp Val Thr Ala
385                 390                 395                 400

Ser Tyr Phe His Pro Asp Val Glu Lys Glu Gly Gly Thr Ala Ser Thr
                405                 410                 415

Phe Arg Asp His Asp Val Glu Glu Glu Asn Asp Glu Leu Phe Thr Ala
            420                 425                 430

Asp Gly Trp Leu Arg Thr Gly Asp Val Gly Ser Val Thr Ser Asp Gly
        435                 440                 445

Phe Leu Thr Ile Gln Asp Arg Ala Arg Asp Val Ile Arg Ser Gly Gly
    450                 455                 460

Glu Trp Ile Tyr Ser Ala Gln Leu Glu Asn Leu Ile Val Ala Xaa Glu
465                 470                 475                 480

Glu Val Val Glu Cys Ala Val Ile Gly Phe Pro Asp Asp Lys Trp Val
                485                 490                 495

Glu Arg Pro Leu Ala Val Thr Met Leu Tyr Pro Gly Ile Glu Arg Thr
            500                 505                 510

Arg Glu Thr Ala Glu Arg Leu Arg Asp Gln Leu Arg Asp Arg Leu Pro
        515                 520                 525

Asn Trp Met Leu Pro Glu Tyr Trp Thr Phe Val Asp Glu Val Asp Lys
    530                 535                 540

Thr Ser Val Gly Lys Tyr Asp Lys Lys Asp Leu Arg Asn His Leu Arg
545                 550                 555                 560

Asn Gly Asp Phe Glu Val Ile Lys Leu Lys Gly Pro Gly Xaa Lys
                565                 570                 575

<210> 67
<211> 1401
<212> DNA
<213> Corynebacterium glutamicum

<220>
<221> CDS
<222> (101)..(1378)
<223> RXA01644

<400> 67
gtaggtagag gcttgtggtc actacttgtg gccacatttt aaaaaaatgc acaagaagag 60

aaagcaaagc attatgagta acgccgtagc gcaggacctc atg acc atc gcc gac    115
                                           Met Thr Ile Ala Asp
                                           1               5

atc gtc gag gcc acg acc act gca ccc atc cca ttc cac atc act gcc    163
Ile Val Glu Ala Thr Thr Thr Ala Pro Ile Pro Phe His Ile Thr Ala
                10                  15                  20

ttc gat gga agc ttc act ggc cct gaa gat gct ccc tac cag ctg ttt    211
Phe Asp Gly Ser Phe Thr Gly Pro Glu Asp Ala Pro Tyr Gln Leu Phe
            25                  30                  35

gtt gcc aac acg gat gca gta tcc tac atc gca aca gcg cca gga gat    259
Val Ala Asn Thr Asp Ala Val Ser Tyr Ile Ala Thr Ala Pro Gly Asp
        40                  45                  50

ttg ggt ttg gca cgt gcc tac ctc atg gga gac ctc atc gtg gaa ggt    307
Leu Gly Leu Ala Arg Ala Tyr Leu Met Gly Asp Leu Ile Val Glu Gly
    55                  60                  65

gag cat ccc ggc cat cct tat ggg atc ttt gat gcg ttg aag gag ttc    355
Glu His Pro Gly His Pro Tyr Gly Ile Phe Asp Ala Leu Lys Glu Phe
70                  75                  80                  85

tac cgc tgc ttc aaa cgc cca gat gca tcc acc acc ttg cag atc atg    403
Tyr Arg Cys Phe Lys Arg Pro Asp Ala Ser Thr Thr Leu Gln Ile Met
                90                  95                  100

tgg act ctg cgg aaa atg aat gcc tta aaa ttc cag gaa att cca cca    451
Trp Thr Leu Arg Lys Met Asn Ala Leu Lys Phe Gln Glu Ile Pro Pro
            105                 110                 115

atg gaa caa gcc cct gca tgg cgt aaa gca ctg atc aac ggg cta gca    499
Met Glu Gln Ala Pro Ala Trp Arg Lys Ala Leu Ile Asn Gly Leu Ala
        120                 125                 130

tcc agg cac tcg aaa tcc cgc gac aag aaa gcc att agc tac cac tac    547
Ser Arg His Ser Lys Ser Arg Asp Lys Lys Ala Ile Ser Tyr His Tyr
    135                 140                 145

gac gtg ggc aat gag ttc tac tcc ctg ttt tta gat gat tcc atg acc 595
Asp Val Gly Asn Glu Phe Tyr Ser Leu Phe Leu Asp Asp Ser Met Thr
150                 155                 160                 165

tat acc tgc gcg tat tat cca acg cca gaa tca agt ttg gaa gaa gcc 643
Tyr Thr Cys Ala Tyr Tyr Pro Thr Pro Glu Ser Ser Leu Glu Glu Ala
                170                 175                 180

caa gaa aac aaa tac cgc ctc atc ttt gaa aaa ctg cgt ctg aaa gaa 691
Gln Glu Asn Lys Tyr Arg Leu Ile Phe Glu Lys Leu Arg Leu Lys Glu
            185                 190                 195

ggc gat cgc ctc cta gac gtg gga tgc ggt tgg gga ggc atg gtc cgc 739
Gly Asp Arg Leu Leu Asp Val Gly Cys Gly Trp Gly Gly Met Val Arg
        200                 205                 210

tac gcc gcc aaa cac ggt gtg aaa gcc atc gga gtt acg ctg tct gaa 787
Tyr Ala Ala Lys His Gly Val Lys Ala Ile Gly Val Thr Leu Ser Glu
    215                 220                 225

cag caa tat gag tgg ggt caa gca gag atc aaa cgc caa ggt ttg gaa 835
Gln Gln Tyr Glu Trp Gly Gln Ala Glu Ile Lys Arg Gln Gly Leu Glu
230                 235                 240                 245

gac ctc gcg gaa att cgc ttc atg gat tac cgc gat gtt cca gaa act 883
Asp Leu Ala Glu Ile Arg Phe Met Asp Tyr Arg Asp Val Pro Glu Thr
                250                 255                 260

gga ttc gat gcg atc tca gca atc ggc atc att gaa cac atc ggt gtg 931
Gly Phe Asp Ala Ile Ser Ala Ile Gly Ile Ile Glu His Ile Gly Val
            265                 270                 275

aac aac tat ccc gac tac ttt gaa ttg ctc agc agc aaa ctc aaa aca 979
Asn Asn Tyr Pro Asp Tyr Phe Glu Leu Leu Ser Ser Lys Leu Lys Thr
        280                 285                 290

ggc gga ctg atg ctc aac cac agc atc acc tac cca gac aac cgc ccc 1027
Gly Gly Leu Met Leu Asn His Ser Ile Thr Tyr Pro Asp Asn Arg Pro
    295                 300                 305

cgc cac gca ggt gca ttt att gat cgc tac att ttc ccc gac ggt gaa 1075
Arg His Ala Gly Ala Phe Ile Asp Arg Tyr Ile Phe Pro Asp Gly Glu
310                 315                 320                 325

ctc act ggc tct ggc acc ctg atc aag cac atg cag gac aac ggt ttc 1123
Leu Thr Gly Ser Gly Thr Leu Ile Lys His Met Gln Asp Asn Gly Phe
                330                 335                 340

gaa gtg ctg cac gaa gaa aac ctc cgc ttt gat tac caa cgc acc ctg 1171
Glu Val Leu His Glu Glu Asn Leu Arg Phe Asp Tyr Gln Arg Thr Leu
            345                 350                 355

cac gcg tgg tgc gaa aac ctc aaa gaa aat tgg gag gaa gca gtt gaa 1219
His Ala Trp Cys Glu Asn Leu Lys Glu Asn Trp Glu Glu Ala Val Glu
        360                 365                 370

ctc gcc ggt gaa ccc act gca cga ctc ttt ggc ctg tac atg gca ggt 1267
Leu Ala Gly Glu Pro Thr Ala Arg Leu Phe Gly Leu Tyr Met Ala Gly
    375                 380                 385

tcg gaa tgg gga ttt gcc cac aac atc gtc cag ctg cac caa gta ctg 1315

Ser Glu Trp Gly Phe Ala His Asn Ile Val Gln Leu His Gln Val Leu
390                 395                 400                 405

ggt gtg aaa ctc gat gag cag gga agt cgc gga gaa gtt cct gaa aga     1363
Gly Val Lys Leu Asp Glu Gln Gly Ser Arg Gly Glu Val Pro Glu Arg
                410                 415                 420

atg tgg tgg act atc taaagaaaca atgtttcttt tta                       1401
Met Trp Trp Thr Ile
            425

<210> 68
<211> 426
<212> PRT
<213> Corynebacterium glutamicum

<400> 68
Met Thr Ile Ala Asp Ile Val Glu Ala Thr Thr Thr Ala Pro Ile Pro
1               5                   10                  15

Phe His Ile Thr Ala Phe Asp Gly Ser Phe Thr Gly Pro Glu Asp Ala
            20                  25                  30

Pro Tyr Gln Leu Phe Val Ala Asn Thr Asp Ala Val Ser Tyr Ile Ala
        35                  40                  45

Thr Ala Pro Gly Asp Leu Gly Leu Ala Arg Ala Tyr Leu Met Gly Asp
    50                  55                  60

Leu Ile Val Glu Gly Glu His Pro Gly His Pro Tyr Gly Ile Phe Asp
65                  70                  75                  80

Ala Leu Lys Glu Phe Tyr Arg Cys Phe Lys Arg Pro Asp Ala Ser Thr
                85                  90                  95

Thr Leu Gln Ile Met Trp Thr Leu Arg Lys Met Asn Ala Leu Lys Phe
            100                 105                 110

Gln Glu Ile Pro Pro Met Glu Gln Ala Pro Ala Trp Arg Lys Ala Leu
        115                 120                 125

Ile Asn Gly Leu Ala Ser Arg His Ser Lys Ser Arg Asp Lys Lys Ala
    130                 135                 140

Ile Ser Tyr His Tyr Asp Val Gly Asn Glu Phe Tyr Ser Leu Phe Leu
145                 150                 155                 160

Asp Asp Ser Met Thr Tyr Thr Cys Ala Tyr Tyr Pro Thr Pro Glu Ser
                165                 170                 175

Ser Leu Glu Glu Ala Gln Glu Asn Lys Tyr Arg Leu Ile Phe Glu Lys
            180                 185                 190

Leu Arg Leu Lys Glu Gly Asp Arg Leu Leu Asp Val Gly Cys Gly Trp
        195                 200                 205

Gly Gly Met Val Arg Tyr Ala Ala Lys His Gly Val Lys Ala Ile Gly
    210                 215                 220

Val Thr Leu Ser Glu Gln Gln Tyr Glu Trp Gly Gln Ala Glu Ile Lys

225                 230                 235                 240

Arg Gln Gly Leu Glu Asp Leu Ala Glu Ile Arg Phe Met Asp Tyr Arg
                245                 250                 255

Asp Val Pro Glu Thr Gly Phe Asp Ala Ile Ser Ala Ile Gly Ile Ile
            260                 265                 270

Glu His Ile Gly Val Asn Asn Tyr Pro Asp Tyr Phe Glu Leu Leu Ser
        275                 280                 285

Ser Lys Leu Lys Thr Gly Gly Leu Met Leu Asn His Ser Ile Thr Tyr
    290                 295                 300

Pro Asp Asn Arg Pro Arg His Ala Gly Ala Phe Ile Asp Arg Tyr Ile
305                 310                 315                 320

Phe Pro Asp Gly Glu Leu Thr Gly Ser Gly Thr Leu Ile Lys His Met
                325                 330                 335

Gln Asp Asn Gly Phe Glu Val Leu His Glu Glu Asn Leu Arg Phe Asp
            340                 345                 350

Tyr Gln Arg Thr Leu His Ala Trp Cys Glu Asn Leu Lys Glu Asn Trp
        355                 360                 365

Glu Glu Ala Val Glu Leu Ala Gly Glu Pro Thr Ala Arg Leu Phe Gly
    370                 375                 380

Leu Tyr Met Ala Gly Ser Glu Trp Gly Phe Ala His Asn Ile Val Gln
385                 390                 395                 400

Leu His Gln Val Leu Gly Val Lys Leu Asp Glu Gln Gly Ser Arg Gly
                405                 410                 415

Glu Val Pro Glu Arg Met Trp Trp Thr Ile
            420                 425

<210> 69
<211> 1437
<212> DNA
<213> Corynebacterium glutamicum

<220>
<221> CDS
<222> (101)..(1414)
<223> RXA02029

<400> 69
cgcacgtaca ctacttttag cagttaaccc gccgaacttt cttgattaag tattcattga 60

gcgttcttgg cataaatccc actggaggtg catcaaacgt atg gca gag gca cgt   115
                                           Met Ala Glu Ala Arg
                                           1               5

ttg cgc cat ctt gaa ccc att gat gtg gaa gag tgg cct ggg gtg gcg   163
Leu Arg His Leu Glu Pro Ile Asp Val Glu Glu Trp Pro Gly Val Ala
                10                  15                  20

tca gtg ccc aac cta gcg ttt gct ggt gca cgc gca cgg caa gca gaa   211

Ser Val Pro Asn Leu Ala Phe Ala Gly Ala Arg Ala Arg Gln Ala Glu
            25                  30                  35

tat cga ttt gcc aag gcc tgc agc aac gca ggt tta gtt ctg cta gga     259
Tyr Arg Phe Ala Lys Ala Cys Ser Asn Ala Gly Leu Val Leu Leu Gly
        40                  45                  50

aat gac ccg gat ctc att att gat cat gag gaa tta ttc tca cgt ttg     307
Asn Asp Pro Asp Leu Ile Ile Asp His Glu Glu Leu Phe Ser Arg Leu
    55                  60                  65

gcc gca tcg ggc tgg ctg ggg ctc gct gag agc tac atg gca ggc gag     355
Ala Ala Ser Gly Trp Leu Gly Leu Ala Glu Ser Tyr Met Ala Gly Glu
70                  75                  80                  85

tgg cgc agc gag agg ctt gcc gac gtc ttg acc gct ttg ttg gga act     403
Trp Arg Ser Glu Arg Leu Ala Asp Val Leu Thr Ala Leu Leu Gly Thr
                90                  95                  100

ggg ttt aaa ccc cgc ggc aaa cta tct gga tcg ttc acc ctg cca ggg     451
Gly Phe Lys Pro Arg Gly Lys Leu Ser Gly Ser Phe Thr Leu Pro Gly
            105                 110                 115

caa gct gtg gat gcc gga ggc gca cta ccc aat gaa ctc att cgt tta     499
Gln Ala Val Asp Ala Gly Gly Ala Leu Pro Asn Glu Leu Ile Arg Leu
        120                 125                 130

agt tca ggt gat ggc atg agc gca ttt ggt gga gtt ttt gcc tcc ggt     547
Ser Ser Gly Asp Gly Met Ser Ala Phe Gly Gly Val Phe Ala Ser Gly
    135                 140                 145

gtt cca acc act tta cgt acc gca gtg aaa agc cat gtg ccg gga gct     595
Val Pro Thr Thr Leu Arg Thr Ala Val Lys Ser His Val Pro Gly Ala
150                 155                 160                 165

ggt cgc aat agg gag ccc gca tct cac ttt gtg gat atc acc aag atc     643
Gly Arg Asn Arg Glu Pro Ala Ser His Phe Val Asp Ile Thr Lys Ile
                170                 175                 180

tcg gag cct gtg gcc gtg gag cgt gaa gac ctc ggc gag gct cag cgc     691
Ser Glu Pro Val Ala Val Glu Arg Glu Asp Leu Gly Glu Ala Gln Arg
            185                 190                 195

cga gca gcg tcc ttt ttg ctt gac ggc gcc aaa gtt aaa gcc gga agc     739
Arg Ala Ala Ser Phe Leu Leu Asp Gly Ala Lys Val Lys Ala Gly Ser
        200                 205                 210

cat gtg ttg gag ttt ccc agc agt ggt gga gct tta gct att ttg gct     787
His Val Leu Glu Phe Pro Ser Ser Gly Gly Ala Leu Ala Ile Leu Ala
    215                 220                 225

gct cga cgc cag gga act gtt gac gct tta acg gct gat ccc gcg caa     835
Ala Arg Arg Gln Gly Thr Val Asp Ala Leu Thr Ala Asp Pro Ala Gln
230                 235                 240                 245

gtt tca agc ctg gag gag acg ttt gtg ctc gcc ggt gtg gag gag gat     883
Val Ser Ser Leu Glu Glu Thr Phe Val Leu Ala Gly Val Glu Glu Asp
                250                 255                 260

att cac atc gag gtc att ccc cag gcg att ccc tcg cca cgc gaa tgg     931
Ile His Ile Glu Val Ile Pro Gln Ala Ile Pro Ser Pro Arg Glu Trp

265                 270                 275

ggt ggc gcc tac gat tcg ata gtc gcc atg gag aag cta gaa gtg gtg     979
Gly Gly Ala Tyr Asp Ser Ile Val Ala Met Glu Lys Leu Glu Val Val
        280                 285                 290

ggc aag cac ggt tcc aag cgg ttt att aaa gct atc gac aga atg ctc     1027
Gly Lys His Gly Ser Lys Arg Phe Ile Lys Ala Ile Asp Arg Met Leu
    295                 300                 305

acc acc ggc ggc aac gta gcc atg caa tcc ttg gta gct act gac caa     1075
Thr Thr Gly Gly Asn Val Ala Met Gln Ser Leu Val Ala Thr Asp Gln
310                 315                 320                 325

tgg agt cct gtg tgt tct gaa gcg att tct ttg ctc aag gcc tat att     1123
Trp Ser Pro Val Cys Ser Glu Ala Ile Ser Leu Leu Lys Ala Tyr Ile
                330                 335                 340

tgg cct gcg ctg cat tac cca act gtt gat gaa gtt cat cag ctt gtt     1171
Trp Pro Ala Leu His Tyr Pro Thr Val Asp Glu Val His Gln Leu Val
            345                 350                 355

gat agg gat tct tct ctg cgc gtg gtg aag gaa aca cac ttt gcg ggc     1219
Asp Arg Asp Ser Ser Leu Arg Val Val Lys Glu Thr His Phe Ala Gly
        360                 365                 370

cat tac cta aaa agc gtg cag ttg caa cgt gaa gtg ttt gaa ggc cag     1267
His Tyr Leu Lys Ser Val Gln Leu Gln Arg Glu Val Phe Glu Gly Gln
    375                 380                 385

ata cgc gaa gcg gcg gca gat ggc ttt gat gcc gtc tac cgc cgc atg     1315
Ile Arg Glu Ala Ala Ala Asp Gly Phe Asp Ala Val Tyr Arg Arg Met
390                 395                 400                 405

tgg gtg tat cac tac gcg ctt att gaa gcc ctg tta cgc ctg gga tgt     1363
Trp Val Tyr His Tyr Ala Leu Ile Glu Ala Leu Leu Arg Leu Gly Cys
                410                 415                 420

ctc aat gca gtg caa ttt gcg ttg acg aca aga aac aga agg ggg cgt     1411
Leu Asn Ala Val Gln Phe Ala Leu Thr Thr Arg Asn Arg Arg Gly Arg
            425                 430                 435

cga taagcaaaaa tcttttagac ctc                                       1437
Arg

<210> 70
<211> 438
<212> PRT
<213> Corynebacterium glutamicum

<400> 70
Met Ala Glu Ala Arg Leu Arg His Leu Glu Pro Ile Asp Val Glu Glu
1               5                   10                  15

Trp Pro Gly Val Ala Ser Val Pro Asn Leu Ala Phe Ala Gly Ala Arg
            20                  25                  30

Ala Arg Gln Ala Glu Tyr Arg Phe Ala Lys Ala Cys Ser Asn Ala Gly
        35                  40                  45

Leu Val Leu Leu Gly Asn Asp Pro Asp Leu Ile Ile Asp His Glu Glu
50                  55                  60

Leu Phe Ser Arg Leu Ala Ala Ser Gly Trp Leu Gly Leu Ala Glu Ser
65                  70                  75                  80

Tyr Met Ala Gly Glu Trp Arg Ser Glu Arg Leu Ala Asp Val Leu Thr
                85                  90                  95

Ala Leu Leu Gly Thr Gly Phe Lys Pro Arg Gly Lys Leu Ser Gly Ser
            100                 105                 110

Phe Thr Leu Pro Gly Gln Ala Val Asp Ala Gly Gly Ala Leu Pro Asn
        115                 120                 125

Glu Leu Ile Arg Leu Ser Ser Gly Asp Gly Met Ser Ala Phe Gly Gly
    130                 135                 140

Val Phe Ala Ser Gly Val Pro Thr Thr Leu Arg Thr Ala Val Lys Ser
145                 150                 155                 160

His Val Pro Gly Ala Gly Arg Asn Arg Glu Pro Ala Ser His Phe Val
                165                 170                 175

Asp Ile Thr Lys Ile Ser Glu Pro Val Ala Val Glu Arg Glu Asp Leu
            180                 185                 190

Gly Glu Ala Gln Arg Arg Ala Ala Ser Phe Leu Leu Asp Gly Ala Lys
        195                 200                 205

Val Lys Ala Gly Ser His Val Leu Glu Phe Pro Ser Ser Gly Gly Ala
    210                 215                 220

Leu Ala Ile Leu Ala Ala Arg Arg Gln Gly Thr Val Asp Ala Leu Thr
225                 230                 235                 240

Ala Asp Pro Ala Gln Val Ser Ser Leu Glu Glu Thr Phe Val Leu Ala
                245                 250                 255

Gly Val Glu Glu Asp Ile His Ile Glu Val Ile Pro Gln Ala Ile Pro
            260                 265                 270

Ser Pro Arg Glu Trp Gly Gly Ala Tyr Asp Ser Ile Val Ala Met Glu
        275                 280                 285

Lys Leu Glu Val Val Gly Lys His Gly Ser Lys Arg Phe Ile Lys Ala
    290                 295                 300

Ile Asp Arg Met Leu Thr Thr Gly Gly Asn Val Ala Met Gln Ser Leu
305                 310                 315                 320

Val Ala Thr Asp Gln Trp Ser Pro Val Cys Ser Glu Ala Ile Ser Leu
                325                 330                 335

Leu Lys Ala Tyr Ile Trp Pro Ala Leu His Tyr Pro Thr Val Asp Glu
            340                 345                 350

Val His Gln Leu Val Asp Arg Asp Ser Ser Leu Arg Val Val Lys Glu
        355                 360                 365

Thr His Phe Ala Gly His Tyr Leu Lys Ser Val Gln Leu Gln Arg Glu
    370                 375                 380

Val Phe Glu Gly Gln Ile Arg Glu Ala Ala Ala Asp Gly Phe Asp Ala
385                 390                 395                 400

Val Tyr Arg Arg Met Trp Val Tyr His Tyr Ala Leu Ile Glu Ala Leu
                405                 410                 415

Leu Arg Leu Gly Cys Leu Asn Ala Val Gln Phe Ala Leu Thr Thr Arg
            420                 425                 430

Asn Arg Arg Gly Arg Arg
        435


<210> 71
<211> 1140
<212> DNA
<213> Corynebacterium glutamicum

<220>
<221> CDS
<222> (101)..(1117)
<223> RXA01801

<400> 71
tttggattcc tttccgatta tgtcttgatc gcccattctg tcacatccgg gttgtctaaa 60

cacccgcgaa gatttcctgt gatgtgccac actggttctc atg tct aac gta gtg   115
                                            Met Ser Asn Val Val
                                              1               5

aac act ttt gtc cag aat tcc acc ggt atg gtg gag ctt aat cgt ccc   163
Asn Thr Phe Val Gln Asn Ser Thr Gly Met Val Glu Leu Asn Arg Pro
                 10                  15                  20

aaa gcg ctc aat tct ctt aat cag gaa atg atc gat ctc gtt cag gaa   211
Lys Ala Leu Asn Ser Leu Asn Gln Glu Met Ile Asp Leu Val Gln Glu
             25                  30                  35

gct cta acc act tgg gct gat gat gat cag gtg cag cag gtt ctc att   259
Ala Leu Thr Thr Trp Ala Asp Asp Asp Gln Val Gln Gln Val Leu Ile
         40                  45                  50

tac tca tcc tcg gag cgt gca ttt tgt gcc ggc ggt gat gtg cgc gcg   307
Tyr Ser Ser Ser Glu Arg Ala Phe Cys Ala Gly Gly Asp Val Arg Ala
     55                  60                  65

gtc cgt gag agc gtg ttg gag ggc gat gtt gcg gcc ggc gat aag tat   355
Val Arg Glu Ser Val Leu Glu Gly Asp Val Ala Ala Gly Asp Lys Tyr
 70                  75                  80                  85

ttc atc gat gag ttc gcg atg aac aac acg ttg gga acc tat ccg aag   403
Phe Ile Asp Glu Phe Ala Met Asn Asn Thr Leu Gly Thr Tyr Pro Lys
                 90                  95                 100

ccg gtc att tct gtg atc aac ggc gtc gcg atg ggt ggc gga atg gga   451
Pro Val Ile Ser Val Ile Asn Gly Val Ala Met Gly Gly Gly Met Gly
            105                 110                 115

att tcc atg cat gga tcg cac cgg atc gtc acg gaa aaa gcg ttc gcg     499
Ile Ser Met His Gly Ser His Arg Ile Val Thr Glu Lys Ala Phe Ala
        120                 125                 130

tcg atg cct gag atg gcg atc ggc tat gtt ccc gat gtg ggc ttt act     547
Ser Met Pro Glu Met Ala Ile Gly Tyr Val Pro Asp Val Gly Phe Thr
    135                 140                 145

tat ttc ggt cag cgt gca tcg tcg ctg gcc atc gcc aca ttt ttg gcg     595
Tyr Phe Gly Gln Arg Ala Ser Ser Leu Ala Ile Ala Thr Phe Leu Ala
150                 155                 160                 165

gtg acc ggg tgg cgc atg agc cct gcc gat atg ctg tgg gct ggc gtc     643
Val Thr Gly Trp Arg Met Ser Pro Ala Asp Met Leu Trp Ala Gly Val
                170                 175                 180

gca acg cat ttt gtt gag gat gcg cag ggg ttc att gat gcg gtc ttg     691
Ala Thr His Phe Val Glu Asp Ala Gln Gly Phe Ile Asp Ala Val Leu
            185                 190                 195

aac gag tcg ctt gat ggc gcg ctg gag aaa ttc tcc acg caa cct acg     739
Asn Glu Ser Leu Asp Gly Ala Leu Glu Lys Phe Ser Thr Gln Pro Thr
        200                 205                 210

ggc agc agc gag ctg gcc ggc gtc gcc agc cag att gag gag acc ttt     787
Gly Ser Ser Glu Leu Ala Gly Val Ala Ser Gln Ile Glu Glu Thr Phe
    215                 220                 225

ggg cac agc tct tgg gcg ctt atc gac gcc tcc ctc cgg tct cac ccc     835
Gly His Ser Ser Trp Ala Leu Ile Asp Ala Ser Leu Arg Ser His Pro
230                 235                 240                 245

gat gct gaa ttt gtt gcc aag gtg gat ggg ctg atg gcg tcg gca gca     883
Asp Ala Glu Phe Val Ala Lys Val Asp Gly Leu Met Ala Ser Ala Ala
                250                 255                 260

ccg gca tcg gtg gtg gct acc gtg aag ctg atg cat caa aac agt gag     931
Pro Ala Ser Val Val Ala Thr Val Lys Leu Met His Gln Asn Ser Glu
            265                 270                 275

gcg acc act ctg cgt gaa ggc ttg gac aat gaa ttg gcg atg tct ctt     979
Ala Thr Thr Leu Arg Glu Gly Leu Asp Asn Glu Leu Ala Met Ser Leu
        280                 285                 290

tat atg atc cgc cag cct gat ttt gct gaa ggt gtg cgt gca gtg ttg     1027
Tyr Met Ile Arg Gln Pro Asp Phe Ala Glu Gly Val Arg Ala Val Leu
    295                 300                 305

gtt gat aaa gac cgc aat gca gcc ttc tcc cca gcc aac tac gaa gat     1075
Val Asp Lys Asp Arg Asn Ala Ala Phe Ser Pro Ala Asn Tyr Glu Asp
310                 315                 320                 325

gtt gat gag tca cat ttt gtg acc ttg ttc cag cgc agt tca             1117
Val Asp Glu Ser His Phe Val Thr Leu Phe Gln Arg Ser Ser
                330                 335

taaaaccgcc aaacttttga tag                                           1140

<210> 72
<211> 339

<212> PRT
<213> Corynebacterium glutamicum

<400> 72
Met Ser Asn Val Val Asn Thr Phe Val Gln Asn Ser Thr Gly Met Val
1               5                   10                  15

Glu Leu Asn Arg Pro Lys Ala Leu Asn Ser Leu Asn Gln Glu Met Ile
            20                  25                  30

Asp Leu Val Gln Glu Ala Leu Thr Thr Trp Ala Asp Asp Asp Gln Val
        35                  40                  45

Gln Gln Val Leu Ile Tyr Ser Ser Ser Glu Arg Ala Phe Cys Ala Gly
    50                  55                  60

Gly Asp Val Arg Ala Val Arg Glu Ser Val Leu Glu Gly Asp Val Ala
65                  70                  75                  80

Ala Gly Asp Lys Tyr Phe Ile Asp Glu Phe Ala Met Asn Asn Thr Leu
                85                  90                  95

Gly Thr Tyr Pro Lys Pro Val Ile Ser Val Ile Asn Gly Val Ala Met
            100                 105                 110

Gly Gly Gly Met Gly Ile Ser Met His Gly Ser His Arg Ile Val Thr
        115                 120                 125

Glu Lys Ala Phe Ala Ser Met Pro Glu Met Ala Ile Gly Tyr Val Pro
    130                 135                 140

Asp Val Gly Phe Thr Tyr Phe Gly Gln Arg Ala Ser Ser Leu Ala Ile
145                 150                 155                 160

Ala Thr Phe Leu Ala Val Thr Gly Trp Arg Met Ser Pro Ala Asp Met
                165                 170                 175

Leu Trp Ala Gly Val Ala Thr His Phe Val Glu Asp Ala Gln Gly Phe
            180                 185                 190

Ile Asp Ala Val Leu Asn Glu Ser Leu Asp Gly Ala Leu Glu Lys Phe
        195                 200                 205

Ser Thr Gln Pro Thr Gly Ser Ser Glu Leu Ala Gly Val Ala Ser Gln
    210                 215                 220

Ile Glu Glu Thr Phe Gly His Ser Ser Trp Ala Leu Ile Asp Ala Ser
225                 230                 235                 240

Leu Arg Ser His Pro Asp Ala Glu Phe Val Ala Lys Val Asp Gly Leu
                245                 250                 255

Met Ala Ser Ala Ala Pro Ala Ser Val Val Ala Thr Val Lys Leu Met
            260                 265                 270

His Gln Asn Ser Glu Ala Thr Thr Leu Arg Glu Gly Leu Asp Asn Glu
        275                 280                 285

Leu Ala Met Ser Leu Tyr Met Ile Arg Gln Pro Asp Phe Ala Glu Gly
    290                 295                 300

Val Arg Ala Val Leu Val Asp Lys Asp Arg Asn Ala Ala Phe Ser Pro
305                 310                 315                 320

Ala Asn Tyr Glu Asp Val Asp Glu Ser His Phe Val Thr Leu Phe Gln
                325                 330                 335

Arg Ser Ser

<210> 73
<211> 1086
<212> DNA
<213> Corynebacterium glutamicum

<220>
<221> CDS
<222> (101)..(1063)
<223> RXN02512

<400> 73
gctggaaagt ccccattggc taaggaattt accaaggcac cagcaggtgc gaaggcagat 60

tacagcaaca ccaaataaaa attagccgag ggagcatcgc atg aag ccg aag gat 115
                                            Met Lys Pro Lys Asp
                                            1               5

ttc tgc aca gcg gaa aat tgg gcg gag aat tta agc gca ctg ggc tat 163
Phe Cys Thr Ala Glu Asn Trp Ala Glu Asn Leu Ser Ala Leu Gly Tyr
                10                  15                  20

cta gct ggt tgg cgt ttt gtc cgg atg ctc cct ttg cct att gct cgc 211
Leu Ala Gly Trp Arg Phe Val Arg Met Leu Pro Leu Pro Ile Ala Arg
            25                  30                  35

cgg gtg ttt gac ctt ggg gcg gat ctg gcg tcg aaa agc gga aaa ggc 259
Arg Val Phe Asp Leu Gly Ala Asp Leu Ala Ser Lys Ser Gly Lys Gly
        40                  45                  50

atg ggg cag cta cgc gct aat ctg gcg cgg gtg gtc ggt gcg gaa aac 307
Met Gly Gln Leu Arg Ala Asn Leu Ala Arg Val Val Gly Ala Glu Asn
    55                  60                  65

gtt acg cag gcg ctg gtg aag caa gca acg cgc agc tat gcg cgg tat 355
Val Thr Gln Ala Leu Val Lys Gln Ala Thr Arg Ser Tyr Ala Arg Tyr
70                  75                  80                  85

tgg ctg gaa gcg ttc cgg cta ccg gcg atc gcg cga gat cct gag ctg 403
Trp Leu Glu Ala Phe Arg Leu Pro Ala Ile Ala Arg Asp Pro Glu Leu
                90                  95                  100

ctt gcg cgg ttg cgt aag gga act gtt ggc cta gat ttg ttg gat gaa 451
Leu Ala Arg Leu Arg Lys Gly Thr Val Gly Leu Asp Leu Leu Asp Glu
            105                 110                 115

tct ttg gct gcc ggc aag ggc gta gtt ttg acg ctc cca cac agc ggc 499
Ser Leu Ala Ala Gly Lys Gly Val Val Leu Thr Leu Pro His Ser Gly
        120                 125                 130

aac tgg gat atg gct ggc gct ttt ctg att agc cat cat ggg caa ttc 547
Asn Trp Asp Met Ala Gly Ala Phe Leu Ile Ser His His Gly Gln Phe

135                 140                 145

acc acc gtt gca gaa agg gtc aag ccg gaa cgc ttg ttt gaa gcg ttc      595
Thr Thr Val Ala Glu Arg Val Lys Pro Glu Arg Leu Phe Glu Ala Phe
150                 155                 160                 165

gtg gag ttt cga gaa agc ctt gga ttt gag gtg ctg cct ctc acc ggt      643
Val Glu Phe Arg Glu Ser Leu Gly Phe Glu Val Leu Pro Leu Thr Gly
                170                 175                 180

ggc gag cgt ccg ccg ttt gaa aag ctg aaa gag cgc ctg aca tct gga      691
Gly Glu Arg Pro Pro Phe Glu Lys Leu Lys Glu Arg Leu Thr Ser Gly
            185                 190                 195

ggt atc gtg tgc ctt ctt ggg gag cgt gac ctg cgg cat tcc ggc gtg      739
Gly Ile Val Cys Leu Leu Gly Glu Arg Asp Leu Arg His Ser Gly Val
        200                 205                 210

gag acc act ttt ttt ggt gag aag acc tcc atg cca gca gga cct gcg      787
Glu Thr Thr Phe Phe Gly Glu Lys Thr Ser Met Pro Ala Gly Pro Ala
    215                 220                 225

cag ctg gcc att gaa aca ggt gcg gcg ctg cac gtg gtg cat cca tgg      835
Gln Leu Ala Ile Glu Thr Gly Ala Ala Leu His Val Val His Pro Trp
230                 235                 240                 245

ttc gat gac gac ggc tgg ggt ctc agc gta tcc gat gcc gtg acc gtg      883
Phe Asp Asp Asp Gly Trp Gly Leu Ser Val Ser Asp Ala Val Thr Val
                250                 255                 260

gat aat tta tcc gac acg gtg cag cgg atc gca cat ctt ttt atg gca      931
Asp Asn Leu Ser Asp Thr Val Gln Arg Ile Ala His Leu Phe Met Ala
            265                 270                 275

aat att acg gcg cac ccc gct gat tgg cat atg ctc caa ccc ctg tgg      979
Asn Ile Thr Ala His Pro Ala Asp Trp His Met Leu Gln Pro Leu Trp
        280                 285                 290

ttt ggt gat ttg gat ccg gag cgt ctc aag cgc tct agg gag cag aca      1027
Phe Gly Asp Leu Asp Pro Glu Arg Leu Lys Arg Ser Arg Glu Gln Thr
    295                 300                 305

aat gtt cac aaa ccg gtg gca tta cag gag gac aat taggtgcgaa           1073
Asn Val His Lys Pro Val Ala Leu Gln Glu Asp Asn
310                 315                 320

ttggaatggt ctg                                                       1086

<210> 74
<211> 321
<212> PRT
<213> Corynebacterium glutamicum

<400> 74
Met Lys Pro Lys Asp Phe Cys Thr Ala Glu Asn Trp Ala Glu Asn Leu
1               5                   10                  15

Ser Ala Leu Gly Tyr Leu Ala Gly Trp Arg Phe Val Arg Met Leu Pro
            20                  25                  30

Leu Pro Ile Ala Arg Arg Val Phe Asp Leu Gly Ala Asp Leu Ala Ser
        35                  40                  45

Lys Ser Gly Lys Gly Met Gly Gln Leu Arg Ala Asn Leu Ala Arg Val
    50                  55                  60

Val Gly Ala Glu Asn Val Thr Gln Ala Leu Val Lys Gln Ala Thr Arg
65                  70                  75                  80

Ser Tyr Ala Arg Tyr Trp Leu Glu Ala Phe Arg Leu Pro Ala Ile Ala
                85                  90                  95

Arg Asp Pro Glu Leu Leu Ala Arg Leu Arg Lys Gly Thr Val Gly Leu
            100                 105                 110

Asp Leu Leu Asp Glu Ser Leu Ala Ala Gly Lys Gly Val Val Leu Thr
        115                 120                 125

Leu Pro His Ser Gly Asn Trp Asp Met Ala Gly Ala Phe Leu Ile Ser
    130                 135                 140

His His Gly Gln Phe Thr Thr Val Ala Glu Arg Val Lys Pro Glu Arg
145                 150                 155                 160

Leu Phe Glu Ala Phe Val Glu Phe Arg Glu Ser Leu Gly Phe Glu Val
                165                 170                 175

Leu Pro Leu Thr Gly Gly Glu Arg Pro Pro Phe Glu Lys Leu Lys Glu
            180                 185                 190

Arg Leu Thr Ser Gly Gly Ile Val Cys Leu Leu Gly Glu Arg Asp Leu
        195                 200                 205

Arg His Ser Gly Val Glu Thr Thr Phe Phe Gly Glu Lys Thr Ser Met
    210                 215                 220

Pro Ala Gly Pro Ala Gln Leu Ala Ile Glu Thr Gly Ala Ala Leu His
225                 230                 235                 240

Val Val His Pro Trp Phe Asp Asp Asp Gly Trp Gly Leu Ser Val Ser
                245                 250                 255

Asp Ala Val Thr Val Asp Asn Leu Ser Asp Thr Val Gln Arg Ile Ala
            260                 265                 270

His Leu Phe Met Ala Asn Ile Thr Ala His Pro Ala Asp Trp His Met
        275                 280                 285

Leu Gln Pro Leu Trp Phe Gly Asp Leu Asp Pro Glu Arg Leu Lys Arg
    290                 295                 300

Ser Arg Glu Gln Thr Asn Val His Lys Pro Val Ala Leu Gln Glu Asp
305                 310                 315                 320

Asn

<210> 75
<211> 1057
<212> DNA

<213> Corynebacterium glutamicum

<220>
<221> CDS
<222> (101)..(1057)
<223> FRXA02512

<400> 75
gctggaaagt ccccattggc taaggaattt accaaggcac cagcaggtgc gaaggcagat 60

tacagcaaca ccaaataaaa attagccgag ggagcatcgc atg aag ccg aag gat 115
Met Lys Pro Lys Asp
1               5

ttc tgc aca gcg gaa aat tgg gcg gag aat tta agc gca ctg ggc tat 163
Phe Cys Thr Ala Glu Asn Trp Ala Glu Asn Leu Ser Ala Leu Gly Tyr
                10                  15                  20

cta gct ggt tgg cgt ttt gtc cgg atg ctc cct ttg cct att gct cgc 211
Leu Ala Gly Trp Arg Phe Val Arg Met Leu Pro Leu Pro Ile Ala Arg
            25                  30                  35

cgg gtg ttt gac ctt ggg gcg gat ctg gcg tcg aaa agc gga aaa ggc 259
Arg Val Phe Asp Leu Gly Ala Asp Leu Ala Ser Lys Ser Gly Lys Gly
        40                  45                  50

atg ggg cag cta cgc gct aat ctg gcg cgg gtg gtc ggt gcg gaa aac 307
Met Gly Gln Leu Arg Ala Asn Leu Ala Arg Val Val Gly Ala Glu Asn
    55                  60                  65

gtt acg cag gcg ctg gtg aag caa gca acg cgc agc tat gcg cgg tat 355
Val Thr Gln Ala Leu Val Lys Gln Ala Thr Arg Ser Tyr Ala Arg Tyr
70                  75                  80                  85

tgg ctg gaa gcg ttc cgg cta ccg gcg atc gcg cga gat cct gag ctg 403
Trp Leu Glu Ala Phe Arg Leu Pro Ala Ile Ala Arg Asp Pro Glu Leu
                90                  95                  100

ctt gcg cgg ttg cgt aag gga act gtt ggc cta gat ttg ttg gat gaa 451
Leu Ala Arg Leu Arg Lys Gly Thr Val Gly Leu Asp Leu Leu Asp Glu
            105                 110                 115

tct ttg gct gcc ggc aag ggc gta gtt ttg acg ctc cca cac agc ggc 499
Ser Leu Ala Ala Gly Lys Gly Val Val Leu Thr Leu Pro His Ser Gly
        120                 125                 130

aac tgg gat atg gct ggc gct ttt ctg att agc cat cat ggg caa ttc 547
Asn Trp Asp Met Ala Gly Ala Phe Leu Ile Ser His His Gly Gln Phe
    135                 140                 145

acc acc gtt gca gaa agg gtc aag ccg gaa cgc ttg ttt gaa gcg ttc 595
Thr Thr Val Ala Glu Arg Val Lys Pro Glu Arg Leu Phe Glu Ala Phe
150                 155                 160                 165

gtg gag ttt cga gaa agc ctt gga ttt gag gtg ctg cct ctc acc ggt 643
Val Glu Phe Arg Glu Ser Leu Gly Phe Glu Val Leu Pro Leu Thr Gly
                170                 175                 180

ggc gag cgt ccg ccg ttt gaa aag ctg aaa gag cgc ctg aca tct gga 691
Gly Glu Arg Pro Pro Phe Glu Lys Leu Lys Glu Arg Leu Thr Ser Gly
            185                 190                 195

ggt atc gtg tgc ctt ctt ggg gag cgt gac ctg cgg cat tcc ggc gtg 739
Gly Ile Val Cys Leu Leu Gly Glu Arg Asp Leu Arg His Ser Gly Val
        200                 205                 210

gag acc act ttt ttt ggt gag aag acc tcc atg cca gca gga cct gcg 787
Glu Thr Thr Phe Phe Gly Glu Lys Thr Ser Met Pro Ala Gly Pro Ala
    215                 220                 225

cag ctg gcc att gaa aca ggt gcg gcg ctg cac gtg gtg cat cca tgg 835
Gln Leu Ala Ile Glu Thr Gly Ala Ala Leu His Val Val His Pro Trp
230                 235                 240                 245

ttc gat gac gac ggc tgg ggt ctc agc gta tcc gat gcc gtg acc gtg 883
Phe Asp Asp Asp Gly Trp Gly Leu Ser Val Ser Asp Ala Val Thr Val
                250                 255                 260

gat aat tta tcc gac acg gtg cag cgg atc gca cat ctt ttt atg gca 931
Asp Asn Leu Ser Asp Thr Val Gln Arg Ile Ala His Leu Phe Met Ala
            265                 270                 275

aat att acg gcg cac ccc gct gat tgg cat atg ctc caa ccc ctg tgg 979
Asn Ile Thr Ala His Pro Ala Asp Trp His Met Leu Gln Pro Leu Trp
        280                 285                 290

ttt ggt gat ttg gat ccg gag cgt ctc aag cgc tct agg gag cag aca 1027
Phe Gly Asp Leu Asp Pro Glu Arg Leu Lys Arg Ser Arg Glu Gln Thr
    295                 300                 305

aat gtt cac aaa ccg gtg gca tta cag gag 1057
Asn Val His Lys Pro Val Ala Leu Gln Glu
310                 315

<210> 76
<211> 319
<212> PRT
<213> Corynebacterium glutamicum

<400> 76
Met Lys Pro Lys Asp Phe Cys Thr Ala Glu Asn Trp Ala Glu Asn Leu
1               5                   10                  15

Ser Ala Leu Gly Tyr Leu Ala Gly Trp Arg Phe Val Arg Met Leu Pro
            20                  25                  30

Leu Pro Ile Ala Arg Arg Val Phe Asp Leu Gly Ala Asp Leu Ala Ser
        35                  40                  45

Lys Ser Gly Lys Gly Met Gly Gln Leu Arg Ala Asn Leu Ala Arg Val
    50                  55                  60

Val Gly Ala Glu Asn Val Thr Gln Ala Leu Val Lys Gln Ala Thr Arg
65                  70                  75                  80

Ser Tyr Ala Arg Tyr Trp Leu Glu Ala Phe Arg Leu Pro Ala Ile Ala
                85                  90                  95

Arg Asp Pro Glu Leu Leu Ala Arg Leu Arg Lys Gly Thr Val Gly Leu
            100                 105                 110

Asp Leu Leu Asp Glu Ser Leu Ala Ala Gly Lys Gly Val Val Leu Thr
        115                 120                 125

Leu Pro His Ser Gly Asn Trp Asp Met Ala Gly Ala Phe Leu Ile Ser
    130                 135                 140

His His Gly Gln Phe Thr Thr Val Ala Glu Arg Val Lys Pro Glu Arg
145                 150                 155                 160

Leu Phe Glu Ala Phe Val Glu Phe Arg Glu Ser Leu Gly Phe Glu Val
                165                 170                 175

Leu Pro Leu Thr Gly Gly Glu Arg Pro Pro Phe Glu Lys Leu Lys Glu
            180                 185                 190

Arg Leu Thr Ser Gly Gly Ile Val Cys Leu Leu Gly Glu Arg Asp Leu
        195                 200                 205

Arg His Ser Gly Val Glu Thr Thr Phe Phe Gly Glu Lys Thr Ser Met
    210                 215                 220

Pro Ala Gly Pro Ala Gln Leu Ala Ile Glu Thr Gly Ala Ala Leu His
225                 230                 235                 240

Val Val His Pro Trp Phe Asp Asp Asp Gly Trp Gly Leu Ser Val Ser
                245                 250                 255

Asp Ala Val Thr Val Asp Asn Leu Ser Asp Thr Val Gln Arg Ile Ala
            260                 265                 270

His Leu Phe Met Ala Asn Ile Thr Ala His Pro Ala Asp Trp His Met
        275                 280                 285

Leu Gln Pro Leu Trp Phe Gly Asp Leu Asp Pro Glu Arg Leu Lys Arg
    290                 295                 300

Ser Arg Glu Gln Thr Asn Val His Lys Pro Val Ala Leu Gln Glu
305                 310                 315


<210> 77
<211> 1389
<212> DNA
<213> Corynebacterium glutamicum

<220>
<221> CDS
<222> (101)..(1366)
<223> RXA00899

<400> 77
tttgctgctc ccctacgtcg gactcccact gttcctgctt atgggatcgc catacatcaa 60

ccggtgacgc caccgcatcc aacaagaaat caacgacctc atg gaa gac gtc cac   115
                                          Met Glu Asp Val His
                                          1               5

gac gac gtc ccc gac atc ccc acc gga atg gat gtc tcc gcg gaa gtt   163
Asp Asp Val Pro Asp Ile Pro Thr Gly Met Asp Val Ser Ala Glu Val
                10                  15                  20

gaa tct gtc atc aaa ctc aac cgc cgc ctc acc cgc atg cca gca gtg 211
Glu Ser Val Ile Lys Leu Asn Arg Arg Leu Thr Arg Met Pro Ala Val
            25                  30                  35

acc ggc gga aac aac ggc ttc tac tcc gac tac cgt gaa tcc ctc aaa 259
Thr Gly Gly Asn Asn Gly Phe Tyr Ser Asp Tyr Arg Glu Ser Leu Lys
        40                  45                  50

cgg atg acc gcc gca atc gac gaa gcc gaa gaa tac atc tac gtc gag 307
Arg Met Thr Ala Ala Ile Asp Glu Ala Glu Glu Tyr Ile Tyr Val Glu
    55                  60                  65

atc tac atc atg gcc tgg gat tcc tac acc caa cca ttc ttc gca gca 355
Ile Tyr Ile Met Ala Trp Asp Ser Tyr Thr Gln Pro Phe Phe Ala Ala
70                  75                  80                  85

ctc gaa cga gcc cac aac cgc ggc gtc aaa gtc cga ctc ctt ttc gac 403
Leu Glu Arg Ala His Asn Arg Gly Val Lys Val Arg Leu Leu Phe Asp
                90                  95                  100

cac gtc ggc agc tgg aaa tac ccc ggc tac cac cgc ctc aaa aaa gaa 451
His Val Gly Ser Trp Lys Tyr Pro Gly Tyr His Arg Leu Lys Lys Glu
            105                 110                 115

ctc aac cgc atg ggc ttc gcc tgg tac ctc atg ctc ccc ctc caa ccc 499
Leu Asn Arg Met Gly Phe Ala Trp Tyr Leu Met Leu Pro Leu Gln Pro
        120                 125                 130

tgg cga cgc cgc ttc cgc cga ccc gac ctg cgc aac cac cgc aaa atg 547
Trp Arg Arg Arg Phe Arg Arg Pro Asp Leu Arg Asn His Arg Lys Met
    135                 140                 145

ctc atc atc gac ggc cac acc gca ttc atg ggc tcc caa aat ctc atc 595
Leu Ile Ile Asp Gly His Thr Ala Phe Met Gly Ser Gln Asn Leu Ile
150                 155                 160                 165

gcc ccg agt tac cta caa aag aaa aac atc aaa ctc ggc cgc gaa tgg 643
Ala Pro Ser Tyr Leu Gln Lys Lys Asn Ile Lys Leu Gly Arg Glu Trp
                170                 175                 180

aaa gac ctc atg gtc gaa ctc acc ggc ccc atc gtc tcc tcc atg gaa 691
Lys Asp Leu Met Val Glu Leu Thr Gly Pro Ile Val Ser Ser Met Glu
            185                 190                 195

atg atc ttc gcc ggc gac tgg tac gtc gaa tcc aac gaa gcc ctc gac 739
Met Ile Phe Ala Gly Asp Trp Tyr Val Glu Ser Asn Glu Ala Leu Asp
        200                 205                 210

atc cgc gac cac gca gaa gcc cac ggc tac atc ggc aac act caa aaa 787
Ile Arg Asp His Ala Glu Ala His Gly Tyr Ile Gly Asn Thr Gln Lys
    215                 220                 225

gac tcc gcc acc aac ctc gtg cag ctc atc ccc tcc ggc cct ggt tac 835
Asp Ser Ala Thr Asn Leu Val Gln Leu Ile Pro Ser Gly Pro Gly Tyr
230                 235                 240                 245

acc aca gaa ccc aac ctg cgc atg ttc aac tcc atc gtt cac cac gcc 883
Thr Thr Glu Pro Asn Leu Arg Met Phe Asn Ser Ile Val His His Ala
                250                 255                 260

aaa gaa cga ctc atc ttg tgc agc ccc tac ttc atc ccc gac gaa tcc 931

Lys Glu Arg Leu Ile Leu Cys Ser Pro Tyr Phe Ile Pro Asp Glu Ser
            265                 270                 275

ctc ctc gaa gcc gtc acc tca gcc tgc tac cgc gga gta acc gtc gaa     979
Leu Leu Glu Ala Val Thr Ser Ala Cys Tyr Arg Gly Val Thr Val Glu
        280                 285                 290

cta ttc gtc tct gaa caa gcc gac caa ttc gcc atc gac cac gcc caa     1027
Leu Phe Val Ser Glu Gln Ala Asp Gln Phe Ala Ile Asp His Ala Gln
    295                 300                 305

tcc tcc tac tac cag gca ctc ctt gaa gcc ggc gtg aaa atc tac caa     1075
Ser Ser Tyr Tyr Gln Ala Leu Leu Glu Ala Gly Val Lys Ile Tyr Gln
310                 315                 320                 325

ttc ccc aaa ccc gac gtc ctc cac acc aag tac atg atc gcc gac ccc     1123
Phe Pro Lys Pro Asp Val Leu His Thr Lys Tyr Met Ile Ala Asp Pro
                330                 335                 340

gac gac acc acc ggc aac gaa gcc ctc gga gtc ctc gga tcc tcc aac     1171
Asp Asp Thr Thr Gly Asn Glu Ala Leu Gly Val Leu Gly Ser Ser Asn
            345                 350                 355

ctc gac atc cgc agc ttt ggc ctc aac tac gaa atc tcc ctg atg atc     1219
Leu Asp Ile Arg Ser Phe Gly Leu Asn Tyr Glu Ile Ser Leu Met Ile
        360                 365                 370

gcc aaa ggc aac ctc atc cac gaa ctc aac gcc ctc acc gac cgt tac     1267
Ala Lys Gly Asn Leu Ile His Glu Leu Asn Ala Leu Thr Asp Arg Tyr
    375                 380                 385

cgc aca gta agt ttc aag ctc acc ttg gat aag tgg aac cag cgc agt     1315
Arg Thr Val Ser Phe Lys Leu Thr Leu Asp Lys Trp Asn Gln Arg Ser
390                 395                 400                 405

tgg cgg cgc cgc tac gtg gac aat gtc atg cgt ttg acc tcg gcg ctg     1363
Trp Arg Arg Arg Tyr Val Asp Asn Val Met Arg Leu Thr Ser Ala Leu
                410                 415                 420

cag tagtttggcg cgtttggagt gcg                                       1389
Gln



<210> 78
<211> 422
<212> PRT
<213> Corynebacterium glutamicum

<400> 78
Met Glu Asp Val His Asp Asp Val Pro Asp Ile Pro Thr Gly Met Asp
1               5                   10                  15

Val Ser Ala Glu Val Glu Ser Val Ile Lys Leu Asn Arg Arg Leu Thr
            20                  25                  30

Arg Met Pro Ala Val Thr Gly Gly Asn Asn Gly Phe Tyr Ser Asp Tyr
        35                  40                  45

Arg Glu Ser Leu Lys Arg Met Thr Ala Ala Ile Asp Glu Ala Glu Glu
    50                  55                  60

Tyr Ile Tyr Val Glu Ile Tyr Ile Met Ala Trp Asp Ser Tyr Thr Gln
65                  70                  75                  80

Pro Phe Phe Ala Ala Leu Glu Arg Ala His Asn Arg Gly Val Lys Val
                85                  90                  95

Arg Leu Leu Phe Asp His Val Gly Ser Trp Lys Tyr Pro Gly Tyr His
            100                 105                 110

Arg Leu Lys Lys Glu Leu Asn Arg Met Gly Phe Ala Trp Tyr Leu Met
        115                 120                 125

Leu Pro Leu Gln Pro Trp Arg Arg Arg Phe Arg Arg Pro Asp Leu Arg
    130                 135                 140

Asn His Arg Lys Met Leu Ile Ile Asp Gly His Thr Ala Phe Met Gly
145                 150                 155                 160

Ser Gln Asn Leu Ile Ala Pro Ser Tyr Leu Gln Lys Lys Asn Ile Lys
                165                 170                 175

Leu Gly Arg Glu Trp Lys Asp Leu Met Val Glu Leu Thr Gly Pro Ile
            180                 185                 190

Val Ser Ser Met Glu Met Ile Phe Ala Gly Asp Trp Tyr Val Glu Ser
        195                 200                 205

Asn Glu Ala Leu Asp Ile Arg Asp His Ala Glu Ala His Gly Tyr Ile
    210                 215                 220

Gly Asn Thr Gln Lys Asp Ser Ala Thr Asn Leu Val Gln Leu Ile Pro
225                 230                 235                 240

Ser Gly Pro Gly Tyr Thr Thr Glu Pro Asn Leu Arg Met Phe Asn Ser
                245                 250                 255

Ile Val His His Ala Lys Glu Arg Leu Ile Leu Cys Ser Pro Tyr Phe
            260                 265                 270

Ile Pro Asp Glu Ser Leu Leu Glu Ala Val Thr Ser Ala Cys Tyr Arg
        275                 280                 285

Gly Val Thr Val Glu Leu Phe Val Ser Glu Gln Ala Asp Gln Phe Ala
    290                 295                 300

Ile Asp His Ala Gln Ser Ser Tyr Tyr Gln Ala Leu Leu Glu Ala Gly
305                 310                 315                 320

Val Lys Ile Tyr Gln Phe Pro Lys Pro Asp Val Leu His Thr Lys Tyr
                325                 330                 335

Met Ile Ala Asp Pro Asp Asp Thr Thr Gly Asn Glu Ala Leu Gly Val
            340                 345                 350

Leu Gly Ser Ser Asn Leu Asp Ile Arg Ser Phe Gly Leu Asn Tyr Glu
        355                 360                 365

Ile Ser Leu Met Ile Ala Lys Gly Asn Leu Ile His Glu Leu Asn Ala
    370                 375                 380

Leu Thr Asp Arg Tyr Arg Thr Val Ser Phe Lys Leu Thr Leu Asp Lys
385                 390                 395                 400

Trp Asn Gln Arg Ser Trp Arg Arg Arg Tyr Val Asp Asn Val Met Arg
                405                 410                 415

Leu Thr Ser Ala Leu Gln
            420

<210> 79
<211> 1452
<212> DNA
<213> Corynebacterium glutamicum

<220>
<221> CDS
<222> (101)..(1429)
<223> RXN00819

<400> 79
gatgaggttc gtccgggaat cctcaaagac aatgcggtga aggtacttgg cctagccgct 60

agcactgagc gcggatctca agcagaaaag gtcgtgcaac atg cgt gat ccc att   115
                                           Met Arg Asp Pro Ile
                                           1               5

caa ggt gct gtt att cct tct gat ctt ttt ggt ttc gca gaa gtt ctc   163
Gln Gly Ala Val Ile Pro Ser Asp Leu Phe Gly Phe Ala Glu Val Leu
                10                  15                  20

acc gaa gcc gaa cgc gca gtt ctt ctg gaa acc cgc agg gtg ctt gag   211
Thr Glu Ala Glu Arg Ala Val Leu Leu Glu Thr Arg Arg Val Leu Glu
            25                  30                  35

gaa gag gtg aag cct tat att aat gag gcc tgg gat aag gca gtc ttc   259
Glu Glu Val Lys Pro Tyr Ile Asn Glu Ala Trp Asp Lys Ala Val Phe
        40                  45                  50

ccc gat gag atc gtg cag ccc ctc caa gat ctg caa ttg ctt gat ccg   307
Pro Asp Glu Ile Val Gln Pro Leu Gln Asp Leu Gln Leu Leu Asp Pro
    55                  60                  65

cct gca ctt cgg gaa gca ggg gag tcg gtt cga gac att ttc act ggt   355
Pro Ala Leu Arg Glu Ala Gly Glu Ser Val Arg Asp Ile Phe Thr Gly
70                  75                  80                  85

ttc cgc aat ttt gaa ctc gcg cgc tgt gac atc aat gtt ggt acc tat   403
Phe Arg Asn Phe Glu Leu Ala Arg Cys Asp Ile Asn Val Gly Thr Tyr
                90                  95                  100

tac aac gca tct gct ggt ctc ttc cga acg gcc tgc atg gtt ggt ggc   451
Tyr Asn Ala Ser Ala Gly Leu Phe Arg Thr Ala Cys Met Val Gly Gly
            105                 110                 115

tcc ccg gag cag gcg cag cga ttg gat gcg cag atc aaa tct ggt gag   499
Ser Pro Glu Gln Ala Gln Arg Leu Asp Ala Gln Ile Lys Ser Gly Glu
        120                 125                 130

gtc aag ggc gtt ttt gca ctg acg gaa cct gat cat ggc tct gat atc   547
Val Lys Gly Val Phe Ala Leu Thr Glu Pro Asp His Gly Ser Asp Ile

135                 140                 145

gca ggt ggt ctg gca acc acg gcc act aag gac gca gac acc ggc gag    595
Ala Gly Gly Leu Ala Thr Thr Ala Thr Lys Asp Ala Asp Thr Gly Glu
150                 155                 160                 165

tgg att atc aat ggt gaa aaa cgg tgg atc ggt ggt gct tcc act gct    643
Trp Ile Ile Asn Gly Glu Lys Arg Trp Ile Gly Gly Ala Ser Thr Ala
                170                 175                 180

gat ttg atc gct acc ttc gcc agg gat aca gcc gat aac cag gtg aaa    691
Asp Leu Ile Ala Thr Phe Ala Arg Asp Thr Ala Asp Asn Gln Val Lys
            185                 190                 195

tgc ttc ctc gtg gca cct cag gca gag ggc gtg tcc atg gag att att    739
Cys Phe Leu Val Ala Pro Gln Ala Glu Gly Val Ser Met Glu Ile Ile
        200                 205                 210

gat cgc aaa gcc tca ctg cgc atc atg caa aat gca cac att acc tat    787
Asp Arg Lys Ala Ser Leu Arg Ile Met Gln Asn Ala His Ile Thr Tyr
    215                 220                 225

aac aat gtc cgg gtg tct ggg gat gcg cgg ctg cac aac atc aat tct    835
Asn Asn Val Arg Val Ser Gly Asp Ala Arg Leu His Asn Ile Asn Ser
230                 235                 240                 245

ttc aag gat gtt tcg gaa tgc ctg cgc cgt atg cgt tcc gat gtg gcg    883
Phe Lys Asp Val Ser Glu Cys Leu Arg Arg Met Arg Ser Asp Val Ala
                250                 255                 260

tgg atg gcg gtc ggt gcg cag gca ggt gcc tat gaa gca gcc gtg aag    931
Trp Met Ala Val Gly Ala Gln Ala Gly Ala Tyr Glu Ala Ala Val Lys
            265                 270                 275

tat gtg cgc agc agg gaa cag ttt ggc cgt ccg atc gcg ggg ttc cag    979
Tyr Val Arg Ser Arg Glu Gln Phe Gly Arg Pro Ile Ala Gly Phe Gln
        280                 285                 290

ttg att cag gaa aag ctc gcg ctc atg ctg ggc aat ctc acg gcg tcg    1027
Leu Ile Gln Glu Lys Leu Ala Leu Met Leu Gly Asn Leu Thr Ala Ser
    295                 300                 305

ctg ggc atg atg gtc aaa ctc acc gat cag cag cag gcg gga att ttc    1075
Leu Gly Met Met Val Lys Leu Thr Asp Gln Gln Gln Ala Gly Ile Phe
310                 315                 320                 325

aaa gag gaa aac tcc gcg ctg gcg aaa atg ttt acc tcg ctc aaa ctt    1123
Lys Glu Glu Asn Ser Ala Leu Ala Lys Met Phe Thr Ser Leu Lys Leu
                330                 335                 340

cgg gag acc gct agt tgg gcg cgg gaa atc tgc gga ggc aac ggc atc    1171
Arg Glu Thr Ala Ser Trp Ala Arg Glu Ile Cys Gly Gly Asn Gly Ile
            345                 350                 355

att ttg gac aac gat gtt gcc cgg ttc cat gcc gat gca gaa gcc gtc    1219
Ile Leu Asp Asn Asp Val Ala Arg Phe His Ala Asp Ala Glu Ala Val
        360                 365                 370

tat tca tat gaa ggc acc cac gaa atc aat gca ctc atc gtt ggn cgn    1267
Tyr Ser Tyr Glu Gly Thr His Glu Ile Asn Ala Leu Ile Val Gly Arg
    375                 380                 385

ncc att ctg ggn cnt ctn ttc ttt tta tat tat nac nct ttt gag gag     1315
Xaa Ile Leu Gly Xaa Leu Phe Phe Leu Tyr Tyr Xaa Xaa Phe Glu Glu
390                 395                 400                 405

gat ctt cat gac tac ttc cac cac ccc aaa cca tcg ttt ctt tcg aag     1363
Asp Leu His Asp Tyr Phe His His Pro Lys Pro Ser Phe Leu Ser Lys
                410                 415                 420

acg cac caa ccc tca ccg gcc agg acc tgg gct ttt cgc agt ggc gca     1411
Thr His Gln Pro Ser Pro Ala Arg Thr Trp Ala Phe Arg Ser Gly Ala
            425                 430                 435

ctg tca ccc agg aga tgg tgaacacctt ggcggacgca act                   1452
Leu Ser Pro Arg Arg Trp
        440


<210> 80
<211> 443
<212> PRT
<213> Corynebacterium glutamicum

<400> 80
Met Arg Asp Pro Ile Gln Gly Ala Val Ile Pro Ser Asp Leu Phe Gly
  1               5                  10                  15

Phe Ala Glu Val Leu Thr Glu Ala Glu Arg Ala Val Leu Leu Glu Thr
             20                  25                  30

Arg Arg Val Leu Glu Glu Glu Val Lys Pro Tyr Ile Asn Glu Ala Trp
         35                  40                  45

Asp Lys Ala Val Phe Pro Asp Glu Ile Val Gln Pro Leu Gln Asp Leu
     50                  55                  60

Gln Leu Leu Asp Pro Pro Ala Leu Arg Glu Ala Gly Glu Ser Val Arg
 65                  70                  75                  80

Asp Ile Phe Thr Gly Phe Arg Asn Phe Glu Leu Ala Arg Cys Asp Ile
                 85                  90                  95

Asn Val Gly Thr Tyr Tyr Asn Ala Ser Ala Gly Leu Phe Arg Thr Ala
            100                 105                 110

Cys Met Val Gly Gly Ser Pro Glu Gln Ala Gln Arg Leu Asp Ala Gln
        115                 120                 125

Ile Lys Ser Gly Glu Val Lys Gly Val Phe Ala Leu Thr Glu Pro Asp
    130                 135                 140

His Gly Ser Asp Ile Ala Gly Gly Leu Ala Thr Thr Ala Thr Lys Asp
145                 150                 155                 160

Ala Asp Thr Gly Glu Trp Ile Ile Asn Gly Glu Lys Arg Trp Ile Gly
                165                 170                 175

Gly Ala Ser Thr Ala Asp Leu Ile Ala Thr Phe Ala Arg Asp Thr Ala
            180                 185                 190

Asp Asn Gln Val Lys Cys Phe Leu Val Ala Pro Gln Ala Glu Gly Val

195                 200                 205

Ser Met Glu Ile Ile Asp Arg Lys Ala Ser Leu Arg Ile Met Gln Asn
    210                 215                 220

Ala His Ile Thr Tyr Asn Asn Val Arg Val Ser Gly Asp Ala Arg Leu
225                 230                 235                 240

His Asn Ile Asn Ser Phe Lys Asp Val Ser Glu Cys Leu Arg Arg Met
                245                 250                 255

Arg Ser Asp Val Ala Trp Met Ala Val Gly Ala Gln Ala Gly Ala Tyr
            260                 265                 270

Glu Ala Ala Val Lys Tyr Val Arg Ser Arg Glu Gln Phe Gly Arg Pro
        275                 280                 285

Ile Ala Gly Phe Gln Leu Ile Gln Glu Lys Leu Ala Leu Met Leu Gly
    290                 295                 300

Asn Leu Thr Ala Ser Leu Gly Met Met Val Lys Leu Thr Asp Gln Gln
305                 310                 315                 320

Gln Ala Gly Ile Phe Lys Glu Glu Asn Ser Ala Leu Ala Lys Met Phe
                325                 330                 335

Thr Ser Leu Lys Leu Arg Glu Thr Ala Ser Trp Ala Arg Glu Ile Cys
            340                 345                 350

Gly Gly Asn Gly Ile Ile Leu Asp Asn Asp Val Ala Arg Phe His Ala
        355                 360                 365

Asp Ala Glu Ala Val Tyr Ser Tyr Glu Gly Thr His Glu Ile Asn Ala
    370                 375                 380

Leu Ile Val Gly Arg Xaa Ile Leu Gly Xaa Leu Phe Phe Leu Tyr Tyr
385                 390                 395                 400

Xaa Xaa Phe Glu Glu Asp Leu His Asp Tyr Phe His His Pro Lys Pro
                405                 410                 415

Ser Phe Leu Ser Lys Thr His Gln Pro Ser Pro Ala Arg Thr Trp Ala
            420                 425                 430

Phe Arg Ser Gly Ala Leu Ser Pro Arg Arg Trp
        435                 440

<210> 81
<211> 1030
<212> DNA
<213> Corynebacterium glutamicum

<220>
<221> CDS
<222> (18)..(1007)
<223> FRXA00819

<400> 81
tcttccgaac ggcctgcatg gtt ggt ggc tcc ccg gag cag gcg cag cga ttg 53
                  Met Val Gly Gly Ser Pro Glu Gln Ala Gln Arg Leu

1               5                   10

gat gcg cag atc aaa tct ggt gag gtc aag ggc gtt ttt gca ctg acg     101
Asp Ala Gln Ile Lys Ser Gly Glu Val Lys Gly Val Phe Ala Leu Thr
        15                  20                  25

gaa cct gat cat ggc tct gat atc gca ggt ggt ctg gca acc acg gcc     149
Glu Pro Asp His Gly Ser Asp Ile Ala Gly Gly Leu Ala Thr Thr Ala
    30                  35                  40

act aag gac gca gac acc ggc gag tgg att atc aat ggt gaa aaa cgg     197
Thr Lys Asp Ala Asp Thr Gly Glu Trp Ile Ile Asn Gly Glu Lys Arg
45                  50                  55                  60

tgg atc ggt ggt gct tcc act gct gat ttg atc gct acc ttc gcc agg     245
Trp Ile Gly Gly Ala Ser Thr Ala Asp Leu Ile Ala Thr Phe Ala Arg
                65                  70                  75

gat aca gcc gat aac cag gtg aaa tgc ttc ctc gtg gca cct cag gca     293
Asp Thr Ala Asp Asn Gln Val Lys Cys Phe Leu Val Ala Pro Gln Ala
            80                  85                  90

gag ggc gtg tcc atg gag att att gat cgc aaa gcc tca ctg cgc atc     341
Glu Gly Val Ser Met Glu Ile Ile Asp Arg Lys Ala Ser Leu Arg Ile
        95                  100                 105

atg caa aat gca cac att acc tat aac aat gtc cgg gtg tct ggg gat     389
Met Gln Asn Ala His Ile Thr Tyr Asn Asn Val Arg Val Ser Gly Asp
    110                 115                 120

gcg cgg ctg cac aac atc aat tct ttc aag gat gtt tcg gaa tgc ctg     437
Ala Arg Leu His Asn Ile Asn Ser Phe Lys Asp Val Ser Glu Cys Leu
125                 130                 135                 140

cgc cgt atg cgt tcc gat gtg gcg tgg atg gcg gtc ggt gcg cag gca     485
Arg Arg Met Arg Ser Asp Val Ala Trp Met Ala Val Gly Ala Gln Ala
                145                 150                 155

ggt gcc tat gaa gca gcc gtg aag tat gtg cgc agc agg gaa cag ttt     533
Gly Ala Tyr Glu Ala Ala Val Lys Tyr Val Arg Ser Arg Glu Gln Phe
            160                 165                 170

ggc cgt ccg atc gcg ggg ttc cag ttg att cag gaa aag ctc gcg ctc     581
Gly Arg Pro Ile Ala Gly Phe Gln Leu Ile Gln Glu Lys Leu Ala Leu
        175                 180                 185

atg ctg ggc aat ctc acg gcg tcg ctg ggc atg atg gtc aaa ctc acc     629
Met Leu Gly Asn Leu Thr Ala Ser Leu Gly Met Met Val Lys Leu Thr
    190                 195                 200

gat cag cag cag gcg gga att ttc aaa gag gaa aac tcc gcg ctg gcg     677
Asp Gln Gln Gln Ala Gly Ile Phe Lys Glu Glu Asn Ser Ala Leu Ala
205                 210                 215                 220

aaa atg ttt acc tcg ctc aaa ctt cgg gag acc gct agt tgg gcg cgg     725
Lys Met Phe Thr Ser Leu Lys Leu Arg Glu Thr Ala Ser Trp Ala Arg
                225                 230                 235

gaa atc tgc gga ggc aac ggc atc att ttg gac aac gat gtt gcc cgg     773
Glu Ile Cys Gly Gly Asn Gly Ile Ile Leu Asp Asn Asp Val Ala Arg
            240                 245                 250

ttc cat gcc gat gca gaa gcc gtc tat tca tat gaa ggc acc cac gaa     821
Phe His Ala Asp Ala Glu Ala Val Tyr Ser Tyr Glu Gly Thr His Glu
        255                 260                 265

atc aat gca ctc atc gtt ggn cgn ncc att ctg ggn cnt ctn ttc ttt     869
Ile Asn Ala Leu Ile Val Gly Arg Xaa Ile Leu Gly Xaa Leu Phe Phe
    270                 275                 280

tta tat tat nac nct ttt gag gag gat ctt cat gac tac ttc cac cac     917
Leu Tyr Tyr Xaa Xaa Phe Glu Glu Asp Leu His Asp Tyr Phe His His
285                 290                 295                 300

ccc aaa cca tcg ttt ctt tcg aag acg cac caa ccc tca ccg gcc agg     965
Pro Lys Pro Ser Phe Leu Ser Lys Thr His Gln Pro Ser Pro Ala Arg
                305                 310                 315

acc tgg gct ttt cgc agt ggc gca ctg tca ccc agg aga tgg             1007
Thr Trp Ala Phe Arg Ser Gly Ala Leu Ser Pro Arg Arg Trp
            320                 325                 330

tgaacacctt cgcggacgca act                                           1030

<210> 82
<211> 330
<212> PRT
<213> Corynebacterium glutamicum

<400> 82
Met Val Gly Gly Ser Pro Glu Gln Ala Gln Arg Leu Asp Ala Gln Ile
  1               5                  10                  15

Lys Ser Gly Glu Val Lys Gly Val Phe Ala Leu Thr Glu Pro Asp His
             20                  25                  30

Gly Ser Asp Ile Ala Gly Gly Leu Ala Thr Thr Ala Thr Lys Asp Ala
         35                  40                  45

Asp Thr Gly Glu Trp Ile Ile Asn Gly Glu Lys Arg Trp Ile Gly Gly
     50                  55                  60

Ala Ser Thr Ala Asp Leu Ile Ala Thr Phe Ala Arg Asp Thr Ala Asp
 65                  70                  75                  80

Asn Gln Val Lys Cys Phe Leu Val Ala Pro Gln Ala Glu Gly Val Ser
                 85                  90                  95

Met Glu Ile Ile Asp Arg Lys Ala Ser Leu Arg Ile Met Gln Asn Ala
            100                 105                 110

His Ile Thr Tyr Asn Asn Val Arg Val Ser Gly Asp Ala Arg Leu His
        115                 120                 125

Asn Ile Asn Ser Phe Lys Asp Val Ser Glu Cys Leu Arg Arg Met Arg
    130                 135                 140

Ser Asp Val Ala Trp Met Ala Val Gly Ala Gln Ala Gly Ala Tyr Glu
145                 150                 155                 160

Ala Ala Val Lys Tyr Val Arg Ser Arg Glu Gln Phe Gly Arg Pro Ile

                    165                     170                     175

Ala Gly Phe Gln Leu Ile Gln Glu Lys Leu Ala Leu Met Leu Gly Asn
            180                 185                 190

Leu Thr Ala Ser Leu Gly Met Met Val Lys Leu Thr Asp Gln Gln Gln
        195                 200                 205

Ala Gly Ile Phe Lys Glu Glu Asn Ser Ala Leu Ala Lys Met Phe Thr
    210                 215                 220

Ser Leu Lys Leu Arg Glu Thr Ala Ser Trp Ala Arg Glu Ile Cys Gly
225                 230                 235                 240

Gly Asn Gly Ile Ile Leu Asp Asn Asp Val Ala Arg Phe His Ala Asp
                245                 250                 255

Ala Glu Ala Val Tyr Ser Tyr Glu Gly Thr His Glu Ile Asn Ala Leu
            260                 265                 270

Ile Val Gly Arg Xaa Ile Leu Gly Xaa Leu Phe Phe Leu Tyr Tyr Xaa
        275                 280                 285

Xaa Phe Glu Glu Asp Leu His Asp Tyr Phe His His Pro Lys Pro Ser
    290                 295                 300

Phe Leu Ser Lys Thr His Gln Pro Ser Pro Ala Arg Thr Trp Ala Phe
305                 310                 315                 320

Arg Ser Gly Ala Leu Ser Pro Arg Arg Trp
                325                 330


<210> 83
<211> 391
<212> DNA
<213> Corynebacterium glutamicum

<220>
<221> CDS
<222> (101)..(391)
<223> FRXA01766

<400> 83
gatgaggttc gtccgggaat cctcaaagac aatgcggtga aggtacttgg cctagccgct 60

agcactgagc gcggatctca agcagaaaag gtcgtgcaac atg cgt gat ccc att    115
                                           Met Arg Asp Pro Ile
                                           1               5

caa ggt gct gtt att cct tct gat ctt ttt ggt ttc gca gaa gtt ctc    163
Gln Gly Ala Val Ile Pro Ser Asp Leu Phe Gly Phe Ala Glu Val Leu
                10                  15                  20

acc gaa gcc gaa cgc gca gtt ctt ctg gaa acc cgc agg gtg ctt gag    211
Thr Glu Ala Glu Arg Ala Val Leu Leu Glu Thr Arg Arg Val Leu Glu
            25                  30                  35

gaa gag gtg aag cct tat att aat gag gcc tgg gat aag gca gtc ttc    259
Glu Glu Val Lys Pro Tyr Ile Asn Glu Ala Trp Asp Lys Ala Val Phe
        40                  45                  50

ccc gat gag atc gtg cag ccc ctc caa gat ctg caa ttg ctt gat ccg 307
Pro Asp Glu Ile Val Gln Pro Leu Gln Asp Leu Gln Leu Leu Asp Pro
    55                  60                  65

cct gca ctt cgg gaa gca ggg gag tcg gtt cga gac att ttc act ggt 355
Pro Ala Leu Arg Glu Ala Gly Glu Ser Val Arg Asp Ile Phe Thr Gly
70                  75                  80                  85

ttc cgc aat ttt gaa ctc gcg cgc tgt gac atc aat 391
Phe Arg Asn Phe Glu Leu Ala Arg Cys Asp Ile Asn
                90                  95

<210> 84
<211> 97
<212> PRT
<213> Corynebacterium glutamicum

<400> 84
Met Arg Asp Pro Ile Gln Gly Ala Val Ile Pro Ser Asp Leu Phe Gly
1               5                   10                  15

Phe Ala Glu Val Leu Thr Glu Ala Glu Arg Ala Val Leu Leu Glu Thr
            20                  25                  30

Arg Arg Val Leu Glu Glu Glu Val Lys Pro Tyr Ile Asn Glu Ala Trp
        35                  40                  45

Asp Lys Ala Val Phe Pro Asp Glu Ile Val Gln Pro Leu Gln Asp Leu
    50                  55                  60

Gln Leu Leu Asp Pro Pro Ala Leu Arg Glu Ala Gly Glu Ser Val Arg
65                  70                  75                  80

Asp Ile Phe Thr Gly Phe Arg Asn Phe Glu Leu Ala Arg Cys Asp Ile
                85                  90                  95

Asn

<210> 85
<211> 1659
<212> DNA
<213> Corynebacterium glutamicum

<220>
<221> CDS
<222> (101)..(1636)
<223> RXN01762

<400> 85
tggagtgatg aattttccta tagaacgttt tttaaacgat tgacttttta aacgtttacg 60

cttttaatga cttcaaacgt gatctaaagc acaaaggaga atg aaa gtg aac ctc 115
Met Lys Val Asn Leu
1               5

gga ata gga agc tac cca cga cgc agg gca act gtt cga cca gag tcc 163
Gly Ile Gly Ser Tyr Pro Arg Arg Arg Ala Thr Val Arg Pro Glu Ser

10                  15                  20

act gca atc gaa ttc gaa ggc acc agc atc acc tac gga gaa ttc agc     211
Thr Ala Ile Glu Phe Glu Gly Thr Ser Ile Thr Tyr Gly Glu Phe Ser
                25                  30                  35

aaa cga gtc aat cgg ctt gga cat gct ctt cta gac ctc ggc gtt gcg     259
Lys Arg Val Asn Arg Leu Gly His Ala Leu Leu Asp Leu Gly Val Ala
            40                  45                  50

cac caa gat cga gta gct tat gtc gga ttc aac cac cct gcc ctg cta     307
His Gln Asp Arg Val Ala Tyr Val Gly Phe Asn His Pro Ala Leu Leu
        55                  60                  65

gaa gtg ttc ttt tca acg aac ctc att ggg gca aca ccc gtg ctt gtt     355
Glu Val Phe Phe Ser Thr Asn Leu Ile Gly Ala Thr Pro Val Leu Val
    70                  75                  80                  85

aac cct cgc cta tcg gca aac gaa atc gat tac atc atc caa gac agc     403
Asn Pro Arg Leu Ser Ala Asn Glu Ile Asp Tyr Ile Ile Gln Asp Ser
                    90                  95                  100

ggt gcg agc atc gtg ttt tac gga atc gac ctc atc gag cac gcc act     451
Gly Ala Ser Ile Val Phe Tyr Gly Ile Asp Leu Ile Glu His Ala Thr
                105                 110                 115

tac ctc caa gaa ctc cat cca gag atc atc atg gtg gcc gtt gaa ggc     499
Tyr Leu Gln Glu Leu His Pro Glu Ile Ile Met Val Ala Val Glu Gly
            120                 125                 130

gat gag ggt cca ggt ttg cgt cga aaa gcg ctt att gaa gcg gcg agc     547
Asp Glu Gly Pro Gly Leu Arg Arg Lys Ala Leu Ile Glu Ala Ala Ser
        135                 140                 145

gac gcc gac atc gac cta gaa gtc agc gat gat gac ctg gtg ctg ctc     595
Asp Ala Asp Ile Asp Leu Glu Val Ser Asp Asp Asp Leu Val Leu Leu
    150                 155                 160                 165

atg tac acc tcc gga acc act ggc cgc cca aag ggc gcc atg tta tcc     643
Met Tyr Thr Ser Gly Thr Thr Gly Arg Pro Lys Gly Ala Met Leu Ser
                    170                 175                 180

cac cga aac ctc ttc ttt aac tac ttc aat gcc ctg ctc agc cag gaa     691
His Arg Asn Leu Phe Phe Asn Tyr Phe Asn Ala Leu Leu Ser Gln Glu
                185                 190                 195

att gaa caa ggc gcg gta ctt tta tcc act gcg ccg tta ttc cac atc     739
Ile Glu Gln Gly Ala Val Leu Leu Ser Thr Ala Pro Leu Phe His Ile
            200                 205                 210

gcg ggc ctc aac atg acc acc atc ccg gtg atg atg aag ggc gga aag     787
Ala Gly Leu Asn Met Thr Thr Ile Pro Val Met Met Lys Gly Gly Lys
        215                 220                 225

gtg atc atc cac cgc gaa ttc cgg gca gag cac gtc ctc gac gaa atc     835
Val Ile Ile His Arg Glu Phe Arg Ala Glu His Val Leu Asp Glu Ile
    230                 235                 240                 245

gaa cgc tcc aag gta tcc gaa tcc ttc atg gtg cca gcg atg atc gac     883
Glu Arg Ser Lys Val Ser Glu Ser Phe Met Val Pro Ala Met Ile Asp
                    250                 255                 260

atg ttg tcc aac cac cca tca ttt gcc gaa cgc gac ctt tct tcc ctt 931
Met Leu Ser Asn His Pro Ser Phe Ala Glu Arg Asp Leu Ser Ser Leu
            265                 270                 275

cgc gcc atc atg gtg ggc ggc tct ccc ctt agc gaa cgt gcg ctg cga 979
Arg Ala Ile Met Val Gly Gly Ser Pro Leu Ser Glu Arg Ala Leu Arg
        280                 285                 290

atc tgg caa gga cgc gac gta aaa att gtc caa ggc ttc ggc atg aca 1027
Ile Trp Gln Gly Arg Asp Val Lys Ile Val Gln Gly Phe Gly Met Thr
    295                 300                 305

gaa acc gca ccg ggc gcc tgt atc ctc gag gca aca gac aca agc aca 1075
Glu Thr Ala Pro Gly Ala Cys Ile Leu Glu Ala Thr Asp Thr Ser Thr
310                 315                 320                 325

cac ctt gga acc gca ggt cga gcc cac ttc ttc acc gac atc aaa cta 1123
His Leu Gly Thr Ala Gly Arg Ala His Phe Phe Thr Asp Ile Lys Leu
                330                 335                 340

gtg gac ccg aaa acc ggc gaa gaa gtc ccc acc gga gag gcc ggc gaa 1171
Val Asp Pro Lys Thr Gly Glu Glu Val Pro Thr Gly Glu Ala Gly Glu
            345                 350                 355

gtt ctc atc cgc gga cca cat gtg atg acc gga tac tgg aac cga cca 1219
Val Leu Ile Arg Gly Pro His Val Met Thr Gly Tyr Trp Asn Arg Pro
        360                 365                 370

gaa gac acc gcc agc gca cta caa aat ggc tgg tac cac tcc gga gat 1267
Glu Asp Thr Ala Ser Ala Leu Gln Asn Gly Trp Tyr His Ser Gly Asp
    375                 380                 385

atc gcc atc aaa gat gaa gac ggc tac tac acc atc aaa gac cgc atc 1315
Ile Ala Ile Lys Asp Glu Asp Gly Tyr Tyr Thr Ile Lys Asp Arg Ile
390                 395                 400                 405

aaa gac atg tac atc tcc ggc ggc gaa aac att tac ccc gca gaa gtc 1363
Lys Asp Met Tyr Ile Ser Gly Gly Glu Asn Ile Tyr Pro Ala Glu Val
                410                 415                 420

gaa caa gca ctc cag gaa ctg gaa gca gtc ctt gat gcc gca gtc atc 1411
Glu Gln Ala Leu Gln Glu Leu Glu Ala Val Leu Asp Ala Ala Val Ile
            425                 430                 435

gga gtc ccc gat gaa cga tgg ggc gaa act ggc atc gcc ttt gtc tcc 1459
Gly Val Pro Asp Glu Arg Trp Gly Glu Thr Gly Ile Ala Phe Val Ser
        440                 445                 450

atc cga gaa agc tac ctg acc aac cca cca acc gga ccg gaa cta cga 1507
Ile Arg Glu Ser Tyr Leu Thr Asn Pro Pro Thr Gly Pro Glu Leu Arg
    455                 460                 465

gaa cta cta ggc agt gtc cta gcc aga tac aaa ctt cca cga gaa atc 1555
Glu Leu Leu Gly Ser Val Leu Ala Arg Tyr Lys Leu Pro Arg Glu Ile
470                 475                 480                 485

cac atc atc gaa gaa ctc ccc cgc aac gcc aca ggc aaa atc caa aag 1603
His Ile Ile Glu Glu Leu Pro Arg Asn Ala Thr Gly Lys Ile Gln Lys
                490                 495                 500

aac atc ctg cga gac ttc acc atc ccc gtt tca taaacccccc aacgtcactt 1656
Asn Ile Leu Arg Asp Phe Thr Ile Pro Val Ser
            505                 510

tga                                                               1659


<210> 86
<211> 512
<212> PRT
<213> Corynebacterium glutamicum

<400> 86
Met Lys Val Asn Leu Gly Ile Gly Ser Tyr Pro Arg Arg Arg Ala Thr
1               5                   10                  15

Val Arg Pro Glu Ser Thr Ala Ile Glu Phe Glu Gly Thr Ser Ile Thr
            20                  25                  30

Tyr Gly Glu Phe Ser Lys Arg Val Asn Arg Leu Gly His Ala Leu Leu
        35                  40                  45

Asp Leu Gly Val Ala His Gln Asp Arg Val Ala Tyr Val Gly Phe Asn
    50                  55                  60

His Pro Ala Leu Leu Glu Val Phe Phe Ser Thr Asn Leu Ile Gly Ala
65                  70                  75                  80

Thr Pro Val Leu Val Asn Pro Arg Leu Ser Ala Asn Glu Ile Asp Tyr
                85                  90                  95

Ile Ile Gln Asp Ser Gly Ala Ser Ile Val Phe Tyr Gly Ile Asp Leu
            100                 105                 110

Ile Glu His Ala Thr Tyr Leu Gln Glu Leu His Pro Glu Ile Ile Met
        115                 120                 125

Val Ala Val Glu Gly Asp Glu Gly Pro Gly Leu Arg Arg Lys Ala Leu
    130                 135                 140

Ile Glu Ala Ala Ser Asp Ala Asp Ile Asp Leu Glu Val Ser Asp Asp
145                 150                 155                 160

Asp Leu Val Leu Leu Met Tyr Thr Ser Gly Thr Thr Gly Arg Pro Lys
                165                 170                 175

Gly Ala Met Leu Ser His Arg Asn Leu Phe Phe Asn Tyr Phe Asn Ala
            180                 185                 190

Leu Leu Ser Gln Glu Ile Glu Gln Gly Ala Val Leu Leu Ser Thr Ala
        195                 200                 205

Pro Leu Phe His Ile Ala Gly Leu Asn Met Thr Thr Ile Pro Val Met
    210                 215                 220

Met Lys Gly Gly Lys Val Ile Ile His Arg Glu Phe Arg Ala Glu His
225                 230                 235                 240

Val Leu Asp Glu Ile Glu Arg Ser Lys Val Ser Glu Ser Phe Met Val
                245                 250                 255

Pro Ala Met Ile Asp Met Leu Ser Asn His Pro Ser Phe Ala Glu Arg
            260                 265                 270

Asp Leu Ser Ser Leu Arg Ala Ile Met Val Gly Gly Ser Pro Leu Ser
        275                 280                 285

Glu Arg Ala Leu Arg Ile Trp Gln Gly Arg Asp Val Lys Ile Val Gln
    290                 295                 300

Gly Phe Gly Met Thr Glu Thr Ala Pro Gly Ala Cys Ile Leu Glu Ala
305                 310                 315                 320

Thr Asp Thr Ser Thr His Leu Gly Thr Ala Gly Arg Ala His Phe Phe
                325                 330                 335

Thr Asp Ile Lys Leu Val Asp Pro Lys Thr Gly Glu Glu Val Pro Thr
            340                 345                 350

Gly Glu Ala Gly Glu Val Leu Ile Arg Gly Pro His Val Met Thr Gly
        355                 360                 365

Tyr Trp Asn Arg Pro Glu Asp Thr Ala Ser Ala Leu Gln Asn Gly Trp
    370                 375                 380

Tyr His Ser Gly Asp Ile Ala Ile Lys Asp Glu Asp Gly Tyr Tyr Thr
385                 390                 395                 400

Ile Lys Asp Arg Ile Lys Asp Met Tyr Ile Ser Gly Gly Glu Asn Ile
                405                 410                 415

Tyr Pro Ala Glu Val Glu Gln Ala Leu Gln Glu Leu Glu Ala Val Leu
            420                 425                 430

Asp Ala Ala Val Ile Gly Val Pro Asp Glu Arg Trp Gly Glu Thr Gly
        435                 440                 445

Ile Ala Phe Val Ser Ile Arg Glu Ser Tyr Leu Thr Asn Pro Pro Thr
    450                 455                 460

Gly Pro Glu Leu Arg Glu Leu Leu Gly Ser Val Leu Ala Arg Tyr Lys
465                 470                 475                 480

Leu Pro Arg Glu Ile His Ile Ile Glu Glu Leu Pro Arg Asn Ala Thr
                485                 490                 495

Gly Lys Ile Gln Lys Asn Ile Leu Arg Asp Phe Thr Ile Pro Val Ser
            500                 505                 510

<210> 87
<211> 1372
<212> DNA
<213> Corynebacterium glutamicum

<220>
<221> CDS
<222> (101)..(1363)
<223> FRXA01762

<400> 87
tggagtgatg aattttccta tagaacgttt tttaaacgat tgacttttta aacgtttacg 60

cttttaatga cttcaaacgt gatctaaagc acaaaggaga atg aaa gtg aac ctc 115
Met Lys Val Asn Leu
1               5

gga ata gga agc tac cca cga cgc agg gca act gtt cga cca gag tcc 163
Gly Ile Gly Ser Tyr Pro Arg Arg Arg Ala Thr Val Arg Pro Glu Ser
                10                  15                  20

act gca atc gaa ttc gaa ggc acc agc atc acc tac gga gaa ttc agc 211
Thr Ala Ile Glu Phe Glu Gly Thr Ser Ile Thr Tyr Gly Glu Phe Ser
            25                  30                  35

aaa cga gtc aat cgg ctt gga cat gct ctt cta gac ctc ggc gtt gcg 259
Lys Arg Val Asn Arg Leu Gly His Ala Leu Leu Asp Leu Gly Val Ala
        40                  45                  50

cac caa gat cga gta gct tat gtc gga ttc aac cac cct gcc ctg cta 307
His Gln Asp Arg Val Ala Tyr Val Gly Phe Asn His Pro Ala Leu Leu
    55                  60                  65

gaa gtg ttc ttt tca acg aac ctc att ggg gca aca ccc gtg ctt gtt 355
Glu Val Phe Phe Ser Thr Asn Leu Ile Gly Ala Thr Pro Val Leu Val
70                  75                  80                  85

aac cct cgc cta tcg gca aac gaa atc gat tac atc atc caa gac agc 403
Asn Pro Arg Leu Ser Ala Asn Glu Ile Asp Tyr Ile Ile Gln Asp Ser
                90                  95                  100

ggt gcg agc atc gtg ttt tac gga atc gac ctc atc gag cac gcc act 451
Gly Ala Ser Ile Val Phe Tyr Gly Ile Asp Leu Ile Glu His Ala Thr
            105                 110                 115

tac ctc caa gaa ctc cat cca gag atc atc atg gtg gcc gtt gaa ggc 499
Tyr Leu Gln Glu Leu His Pro Glu Ile Ile Met Val Ala Val Glu Gly
        120                 125                 130

gat gag ggt cca ggt ttg cgt cga aaa gcg ctt att gaa gcg gcg agc 547
Asp Glu Gly Pro Gly Leu Arg Arg Lys Ala Leu Ile Glu Ala Ala Ser
    135                 140                 145

gac gcc gac atc gac cta gaa gtc agc gat gat gac ctg gtg ctg ctc 595
Asp Ala Asp Ile Asp Leu Glu Val Ser Asp Asp Asp Leu Val Leu Leu
150                 155                 160                 165

atg tac acc tcc gga acc act ggc cgc cca aag ggc gcc atg tta tcc 643
Met Tyr Thr Ser Gly Thr Thr Gly Arg Pro Lys Gly Ala Met Leu Ser
                170                 175                 180

cac cga aac ctc ttc ttt aac tac ttc aat gcc ctg ctc agc cag gaa 691
His Arg Asn Leu Phe Phe Asn Tyr Phe Asn Ala Leu Leu Ser Gln Glu
            185                 190                 195

att gaa caa ggc gcg gta ctt tta tcc act gcg ccg tta ttc cac atc 739
Ile Glu Gln Gly Ala Val Leu Leu Ser Thr Ala Pro Leu Phe His Ile
        200                 205                 210

gcg ggc ctc aac atg acc acc atc ccg gtg atg atg aag ggc gga aag 787

Ala Gly Leu Asn Met Thr Thr Ile Pro Val Met Met Lys Gly Gly Lys
    215                 220                 225

gtg atc atc cac cgc gaa ttc cgg gca gag cac gtc ctc gac gaa atc     835
Val Ile Ile His Arg Glu Phe Arg Ala Glu His Val Leu Asp Glu Ile
230                 235                 240                 245

gaa cgc tcc aag gta tcc gaa tcc ttc atg gtg cca gcg atg atc gac     883
Glu Arg Ser Lys Val Ser Glu Ser Phe Met Val Pro Ala Met Ile Asp
                250                 255                 260

atg ttg tcc aac cac cca tca ttt gcc gaa cgc gac ctt tct tcc ctt     931
Met Leu Ser Asn His Pro Ser Phe Ala Glu Arg Asp Leu Ser Ser Leu
            265                 270                 275

cgc gcc atc atg gtg ggc ggc tct ccc ctt agc gaa cgt gcg ctg cga     979
Arg Ala Ile Met Val Gly Gly Ser Pro Leu Ser Glu Arg Ala Leu Arg
        280                 285                 290

atc tgg caa gga cgc gac gta aaa att gtc caa ggc ttc ggc atg aca     1027
Ile Trp Gln Gly Arg Asp Val Lys Ile Val Gln Gly Phe Gly Met Thr
    295                 300                 305

gaa acc gca ccg ggc gcc tgt atc ctc gag gca aca gac aca agc aca     1075
Glu Thr Ala Pro Gly Ala Cys Ile Leu Glu Ala Thr Asp Thr Ser Thr
310                 315                 320                 325

cac ctt gga acc gca ggt cga gcc cac ttc ttc acc gac atc aaa cta     1123
His Leu Gly Thr Ala Gly Arg Ala His Phe Phe Thr Asp Ile Lys Leu
                330                 335                 340

gtg gac ccg aaa acc ggc gaa gaa gtc ccc acc gga gag gcc ggc gaa     1171
Val Asp Pro Lys Thr Gly Glu Glu Val Pro Thr Gly Glu Ala Gly Glu
            345                 350                 355

gtt ctc atc cgc gga cca cat gtg atg acc gga tac tgg aac cga cca     1219
Val Leu Ile Arg Gly Pro His Val Met Thr Gly Tyr Trp Asn Arg Pro
        360                 365                 370

gaa gac acc gcc agc gca cta caa aat ggc tgg tac cac tcc gga gat     1267
Glu Asp Thr Ala Ser Ala Leu Gln Asn Gly Trp Tyr His Ser Gly Asp
    375                 380                 385

atc gcc atc aaa gat gaa gac ggc tac tac acc atc aaa gac cgc atc     1315
Ile Ala Ile Lys Asp Glu Asp Gly Tyr Tyr Thr Ile Lys Asp Arg Ile
390                 395                 400                 405

aaa gac atg tac atc tcc ggc ggc gaa aac att tac ccc gca gaa gtc     1363
Lys Asp Met Tyr Ile Ser Gly Gly Glu Asn Ile Tyr Pro Ala Glu Val
                410                 415                 420

taagtaccc                                                           1372

<210> 88
<211> 421
<212> PRT
<213> Corynebacterium glutamicum

<400> 88
Met Lys Val Asn Leu Gly Ile Gly Ser Tyr Pro Arg Arg Arg Ala Thr

1               5                   10                  15

Val Arg Pro Glu Ser Thr Ala Ile Glu Phe Glu Gly Thr Ser Ile Thr
            20                  25                  30

Tyr Gly Glu Phe Ser Lys Arg Val Asn Arg Leu Gly His Ala Leu Leu
        35                  40                  45

Asp Leu Gly Val Ala His Gln Asp Arg Val Ala Tyr Val Gly Phe Asn
    50                  55                  60

His Pro Ala Leu Leu Glu Val Phe Phe Ser Thr Asn Leu Ile Gly Ala
65                  70                  75                  80

Thr Pro Val Leu Val Asn Pro Arg Leu Ser Ala Asn Glu Ile Asp Tyr
                85                  90                  95

Ile Ile Gln Asp Ser Gly Ala Ser Ile Val Phe Tyr Gly Ile Asp Leu
            100                 105                 110

Ile Glu His Ala Thr Tyr Leu Gln Glu Leu His Pro Glu Ile Ile Met
        115                 120                 125

Val Ala Val Glu Gly Asp Glu Gly Pro Gly Leu Arg Arg Lys Ala Leu
    130                 135                 140

Ile Glu Ala Ala Ser Asp Ala Asp Ile Asp Leu Glu Val Ser Asp Asp
145                 150                 155                 160

Asp Leu Val Leu Leu Met Tyr Thr Ser Gly Thr Thr Gly Arg Pro Lys
                165                 170                 175

Gly Ala Met Leu Ser His Arg Asn Leu Phe Phe Asn Tyr Phe Asn Ala
            180                 185                 190

Leu Leu Ser Gln Glu Ile Glu Gln Gly Ala Val Leu Leu Ser Thr Ala
        195                 200                 205

Pro Leu Phe His Ile Ala Gly Leu Asn Met Thr Thr Ile Pro Val Met
    210                 215                 220

Met Lys Gly Gly Lys Val Ile Ile His Arg Glu Phe Arg Ala Glu His
225                 230                 235                 240

Val Leu Asp Glu Ile Glu Arg Ser Lys Val Ser Glu Ser Phe Met Val
                245                 250                 255

Pro Ala Met Ile Asp Met Leu Ser Asn His Pro Ser Phe Ala Glu Arg
            260                 265                 270

Asp Leu Ser Ser Leu Arg Ala Ile Met Val Gly Gly Ser Pro Leu Ser
        275                 280                 285

Glu Arg Ala Leu Arg Ile Trp Gln Gly Arg Asp Val Lys Ile Val Gln
    290                 295                 300

Gly Phe Gly Met Thr Glu Thr Ala Pro Gly Ala Cys Ile Leu Glu Ala
305                 310                 315                 320

Thr Asp Thr Ser Thr His Leu Gly Thr Ala Gly Arg Ala His Phe Phe
                325                 330                 335

Thr Asp Ile Lys Leu Val Asp Pro Lys Thr Gly Glu Glu Val Pro Thr
            340                 345                 350

Gly Glu Ala Gly Glu Val Leu Ile Arg Gly Pro His Val Met Thr Gly
        355                 360                 365

Tyr Trp Asn Arg Pro Glu Asp Thr Ala Ser Ala Leu Gln Asn Gly Trp
    370                 375                 380

Tyr His Ser Gly Asp Ile Ala Ile Lys Asp Glu Asp Gly Tyr Tyr Thr
385                 390                 395                 400

Ile Lys Asp Arg Ile Lys Asp Met Tyr Ile Ser Gly Gly Glu Asn Ile
                405                 410                 415

Tyr Pro Ala Glu Val
            420

<210> 89
<211> 867
<212> DNA
<213> Corynebacterium glutamicum

<220>
<221> CDS
<222> (101)..(844)
<223> RXA00681

<400> 89
aagggcaagg ttgtcattgt tgatccagat gcgccacgca tcgaaggacc cggcgcgcac 60

agccatgcgc actcagtagc agcacatggg gtggatacac atg cct agt cca cgc   115
                                           Met Pro Ser Pro Arg
                                           1               5

act gtt ctt atc act ggt gcc gct ggc ggt ttg ggt cgg gca ttc gct   163
Thr Val Leu Ile Thr Gly Ala Ala Gly Gly Leu Gly Arg Ala Phe Ala
                10                  15                  20

gaa ggt ttc gca gcc caa gga gac cgt atc gcg gtg gcg gat atc aat   211
Glu Gly Phe Ala Ala Gln Gly Asp Arg Ile Ala Val Ala Asp Ile Asn
            25                  30                  35

ctg gat ggg gcg caa gag acc gtt gac aag ctg aaa gca ttg ggc gca   259
Leu Asp Gly Ala Gln Glu Thr Val Asp Lys Leu Lys Ala Leu Gly Ala
        40                  45                  50

gat gcc gca gcc ttt gaa gtt gat gtc acg tct ttg gag tcc acc gag   307
Asp Ala Ala Ala Phe Glu Val Asp Val Thr Ser Leu Glu Ser Thr Glu
    55                  60                  65

gcc cta gcc gcc ggt gcc gct gag ttt ggc ggt ggc cga att gat gtc   355
Ala Leu Ala Ala Gly Ala Ala Glu Phe Gly Gly Gly Arg Ile Asp Val
70                  75                  80                  85

ctt att aat aac gca gcg ata tat gcg aca gtg act cgt tca ccg ttt   403
Leu Ile Asn Asn Ala Ala Ile Tyr Ala Thr Val Thr Arg Ser Pro Phe
                90                  95                  100

gag gat att gac cct gcg gag tgg gat ttg gtc atg gga gtc aat ctc    451
Glu Asp Ile Asp Pro Ala Glu Trp Asp Leu Val Met Gly Val Asn Leu
            105                 110                 115

aaa ggc ccg tgg ttg gtg acg cgt tct gtg agt ccg ttt ttg tcc gat    499
Lys Gly Pro Trp Leu Val Thr Arg Ser Val Ser Pro Phe Leu Ser Asp
        120                 125                 130

aat gcc cgt gtg gtc aat ctt tcc agc gcg act gtg ttt tca gga tct    547
Asn Ala Arg Val Val Asn Leu Ser Ser Ala Thr Val Phe Ser Gly Ser
    135                 140                 145

gca cac tgg gcg cac tac gtg gca tcc aaa ggt ggg gtc att gct tta    595
Ala His Trp Ala His Tyr Val Ala Ser Lys Gly Gly Val Ile Ala Leu
150                 155                 160                 165

acc agg gtg ctt gct aaa gag ctg ggt ggt cgt ggg atc acg gtc aat    643
Thr Arg Val Leu Ala Lys Glu Leu Gly Gly Arg Gly Ile Thr Val Asn
                170                 175                 180

gcg gtt gcg cct ggg ttt acg ctg act gaa gcc agc ttg gga ctc atg    691
Ala Val Ala Pro Gly Phe Thr Leu Thr Glu Ala Ser Leu Gly Leu Met
            185                 190                 195

gat agc gcg gaa acg tac ggt gtc gat cgc gga tcc atc aag cgc gca    739
Asp Ser Ala Glu Thr Tyr Gly Val Asp Arg Gly Ser Ile Lys Arg Ala
        200                 205                 210

agc caa ccg aaa gac atc gtc ggc acc acc atg ttt ctt gca tcc cca    787
Ser Gln Pro Lys Asp Ile Val Gly Thr Thr Met Phe Leu Ala Ser Pro
    215                 220                 225

gaa gcc gaa tac atc act ggg caa aca ctc atc gtt gat ggt ggc cga    835
Glu Ala Glu Tyr Ile Thr Gly Gln Thr Leu Ile Val Asp Gly Gly Arg
230                 235                 240                 245

cag ttc atc taagtactaa aagttctaag gag                              867
Gln Phe Ile

<210> 90
<211> 248
<212> PRT
<213> Corynebacterium glutamicum

<400> 90
Met Pro Ser Pro Arg Thr Val Leu Ile Thr Gly Ala Ala Gly Gly Leu
1               5                   10                  15

Gly Arg Ala Phe Ala Glu Gly Phe Ala Ala Gln Gly Asp Arg Ile Ala
            20                  25                  30

Val Ala Asp Ile Asn Leu Asp Gly Ala Gln Glu Thr Val Asp Lys Leu
        35                  40                  45

Lys Ala Leu Gly Ala Asp Ala Ala Ala Phe Glu Val Asp Val Thr Ser
    50                  55                  60

Leu Glu Ser Thr Glu Ala Leu Ala Ala Gly Ala Ala Glu Phe Gly Gly
65                  70                  75                  80

Gly Arg Ile Asp Val Leu Ile Asn Asn Ala Ala Ile Tyr Ala Thr Val
                85                  90                  95

Thr Arg Ser Pro Phe Glu Asp Ile Asp Pro Ala Glu Trp Asp Leu Val
            100                 105                 110

Met Gly Val Asn Leu Lys Gly Pro Trp Leu Val Thr Arg Ser Val Ser
        115                 120                 125

Pro Phe Leu Ser Asp Asn Ala Arg Val Val Asn Leu Ser Ser Ala Thr
    130                 135                 140

Val Phe Ser Gly Ser Ala His Trp Ala His Tyr Val Ala Ser Lys Gly
145                 150                 155                 160

Gly Val Ile Ala Leu Thr Arg Val Leu Ala Lys Glu Leu Gly Gly Arg
                165                 170                 175

Gly Ile Thr Val Asn Ala Val Ala Pro Gly Phe Thr Leu Thr Glu Ala
            180                 185                 190

Ser Leu Gly Leu Met Asp Ser Ala Glu Thr Tyr Gly Val Asp Arg Gly
        195                 200                 205

Ser Ile Lys Arg Ala Ser Gln Pro Lys Asp Ile Val Gly Thr Thr Met
    210                 215                 220

Phe Leu Ala Ser Pro Glu Ala Glu Tyr Ile Thr Gly Gln Thr Leu Ile
225                 230                 235                 240

Val Asp Gly Gly Arg Gln Phe Ile
                245

<210> 91
<211> 837
<212> DNA
<213> Corynebacterium glutamicum

<220>
<221> CDS
<222> (101)..(814)
<223> RXA00802

<400> 91
gccatggtga tcacaccagt gtgggggctg aggctccaca cttggaggaa tggattaaac 60

gcgggcacta agccccgaac gattagtagg cttgggcacc atg gat ctt aaa ctt   115
                                            Met Asp Leu Lys Leu
                                            1               5

ggt ggc caa gtc ata ctt gtt gtt ggc ggt gca gga act att ggt tct   163
Gly Gly Gln Val Ile Leu Val Val Gly Gly Ala Gly Thr Ile Gly Ser
                10                  15                  20

gaa gtt gta aaa ctc tta act gaa gaa ggc gca acc gcg gta gcg gcg   211
Glu Val Val Lys Leu Leu Thr Glu Glu Gly Ala Thr Ala Val Ala Ala
            25                  30                  35

tcg aga agc acg ccc tta tct att gac gct tcg gat gaa gcg tcc gtc   259

Ser Arg Ser Thr Pro Leu Ser Ile Asp Ala Ser Asp Glu Ala Ser Val
        40                  45                  50

cgt gcg ggc att gat cag gtg atc gca gaa cat ggt cgc ctg gat ggg     307
Arg Ala Gly Ile Asp Gln Val Ile Ala Glu His Gly Arg Leu Asp Gly
    55                  60                  65

ctg gtt gtt tct tct gca ccc gct gcg caa acg ctc agc gcg gag aca     355
Leu Val Val Ser Ser Ala Pro Ala Ala Gln Thr Leu Ser Ala Glu Thr
70                  75                  80                  85

gca gat gat ccg gac act gtg ttg gct gct att gaa ggc aaa gcc atc     403
Ala Asp Asp Pro Asp Thr Val Leu Ala Ala Ile Glu Gly Lys Ala Ile
                90                  95                  100

acg ttt atg aag gct gca acc gtg gcg ctc gag aag atg cgt gag gct     451
Thr Phe Met Lys Ala Ala Thr Val Ala Leu Glu Lys Met Arg Glu Ala
            105                 110                 115

gga cat ggg cgc atc gtt gca ctt tcc ggc atg aac tca tac aaa aca     499
Gly His Gly Arg Ile Val Ala Leu Ser Gly Met Asn Ser Tyr Lys Thr
        120                 125                 130

ttg agt act act gcg tcg gcg cga aat gct gcg ctg aat gtc gtg gtg     547
Leu Ser Thr Thr Ala Ser Ala Arg Asn Ala Ala Leu Asn Val Val Val
    135                 140                 145

aaa aat ttg gcg gat cgt cac gcg ggc acc gga att aca gta aat gcg     595
Lys Asn Leu Ala Asp Arg His Ala Gly Thr Gly Ile Thr Val Asn Ala
150                 155                 160                 165

att agc ccg gga ttc gtg gta gct gag cca gac gct gag gta aac cgc     643
Ile Ser Pro Gly Phe Val Val Ala Glu Pro Asp Ala Glu Val Asn Arg
                170                 175                 180

gca aat ggt gac acc acg tgg agg agg tcg cgg agg cga tcg cgt ttt     691
Ala Asn Gly Asp Thr Thr Trp Arg Arg Ser Arg Arg Arg Ser Arg Phe
            185                 190                 195

tgt tgt cgc cgc gca ccg cat caa ttt ctg gag aga tta ttt cgg tgg     739
Cys Cys Arg Arg Ala Pro His Gln Phe Leu Glu Arg Leu Phe Arg Trp
        200                 205                 210

gac ata agg cga agg gca tca tcc ttc ctt agc tcg cgt gag ctt ccc     787
Asp Ile Arg Arg Arg Ala Ser Ser Phe Leu Ser Ser Arg Glu Leu Pro
    215                 220                 225

aag cgt aag cac ccc cgt gtg agg gca taacggccgt tctgttaaag           834
Lys Arg Lys His Pro Arg Val Arg Ala
230                 235

att                                                                 837


<210> 92
<211> 238
<212> PRT
<213> Corynebacterium glutamicum

<400> 92
Met Asp Leu Lys Leu Gly Gly Gln Val Ile Leu Val Val Gly Gly Ala

1               5                   10                  15

Gly Thr Ile Gly Ser Glu Val Val Lys Leu Leu Thr Glu Glu Gly Ala
            20                  25                  30

Thr Ala Val Ala Ala Ser Arg Ser Thr Pro Leu Ser Ile Asp Ala Ser
        35                  40                  45

Asp Glu Ala Ser Val Arg Ala Gly Ile Asp Gln Val Ile Ala Glu His
    50                  55                  60

Gly Arg Leu Asp Gly Leu Val Val Ser Ser Ala Pro Ala Ala Gln Thr
65                  70                  75                  80

Leu Ser Ala Glu Thr Ala Asp Asp Pro Asp Thr Val Leu Ala Ala Ile
                85                  90                  95

Glu Gly Lys Ala Ile Thr Phe Met Lys Ala Ala Thr Val Ala Leu Glu
            100                 105                 110

Lys Met Arg Glu Ala Gly His Gly Arg Ile Val Ala Leu Ser Gly Met
        115                 120                 125

Asn Ser Tyr Lys Thr Leu Ser Thr Thr Ala Ser Ala Arg Asn Ala Ala
    130                 135                 140

Leu Asn Val Val Val Lys Asn Leu Ala Asp Arg His Ala Gly Thr Gly
145                 150                 155                 160

Ile Thr Val Asn Ala Ile Ser Pro Gly Phe Val Val Ala Glu Pro Asp
                165                 170                 175

Ala Glu Val Asn Arg Ala Asn Gly Asp Thr Thr Trp Arg Arg Ser Arg
            180                 185                 190

Arg Arg Ser Arg Phe Cys Cys Arg Arg Ala Pro His Gln Phe Leu Glu
        195                 200                 205

Arg Leu Phe Arg Trp Asp Ile Arg Arg Arg Ala Ser Ser Phe Leu Ser
    210                 215                 220

Ser Arg Glu Leu Pro Lys Arg Lys His Pro Arg Val Arg Ala
225                 230                 235

<210> 93
<211> 329
<212> DNA
<213> Corynebacterium glutamicum

<220>
<221> CDS
<222> (1)..(306)
<223> RXA02133

<400> 93
gaa aac ccc tac att ggc gga gct gga tac aac gca gca aaa ttc ggt      48
Glu Asn Pro Tyr Ile Gly Gly Ala Gly Tyr Asn Ala Ala Lys Phe Gly
 1               5                  10                  15

gta gca gca ttc aac cgt gtg ctt cgc ttg gaa acc cac cag cag acc      96

Val Ala Ala Phe Asn Arg Val Leu Arg Leu Glu Thr His Gln Gln Thr
            20                  25                  30

ctt cgc gta tct gag atc gat cca ggt cga gtt gcc acg gaa gaa ttc 144
Leu Arg Val Ser Glu Ile Asp Pro Gly Arg Val Ala Thr Glu Glu Phe
        35                  40                  45

tcc ctc gtt cgt ttc ggc gga gat aaa gaa cgc gca gaa gca gtc tat 192
Ser Leu Val Arg Phe Gly Gly Asp Lys Glu Arg Ala Glu Ala Val Tyr
    50                  55                  60

gac gac gtc ctc aac ctc acc gct gaa gac atc gca gag tct gtg cgt 240
Asp Asp Val Leu Asn Leu Thr Ala Glu Asp Ile Ala Glu Ser Val Arg
65                  70                  75                  80

tgg gtc gcg agc ctt cca aag cac atg aac att gac cgc atg cgt att 288
Trp Val Ala Ser Leu Pro Lys His Met Asn Ile Asp Arg Met Arg Ile
                85                  90                  95

aca cct cgc gat cag gtc taaaacccgc actcttttga aat 329
Thr Pro Arg Asp Gln Val
            100

<210> 94
<211> 102
<212> PRT
<213> Corynebacterium glutamicum

<400> 94
Glu Asn Pro Tyr Ile Gly Gly Ala Gly Tyr Asn Ala Ala Lys Phe Gly
1               5                   10                  15

Val Ala Ala Phe Asn Arg Val Leu Arg Leu Glu Thr His Gln Gln Thr
            20                  25                  30

Leu Arg Val Ser Glu Ile Asp Pro Gly Arg Val Ala Thr Glu Glu Phe
        35                  40                  45

Ser Leu Val Arg Phe Gly Gly Asp Lys Glu Arg Ala Glu Ala Val Tyr
    50                  55                  60

Asp Asp Val Leu Asn Leu Thr Ala Glu Asp Ile Ala Glu Ser Val Arg
65                  70                  75                  80

Trp Val Ala Ser Leu Pro Lys His Met Asn Ile Asp Arg Met Arg Ile
                85                  90                  95

Thr Pro Arg Asp Gln Val
            100

<210> 95
<211> 1347
<212> DNA
<213> Corynebacterium glutamicum

<220>
<221> CDS
<222> (101)..(1324)
<223> RXN01114

<400> 95
ttcggtggaa gatatccgca agctggtgct gccgcagctt ttagaaacgg ctcaagcaat 60

ttcgacagat ctctctgcac tctaaattaa ggatcaaaaa atg aac cct caa gat 115
Met Asn Pro Gln Asp
1               5

att gtc atc tgt tcc cca ttg cgc acc cca gtt ggt gct tac ggc gga 163
Ile Val Ile Cys Ser Pro Leu Arg Thr Pro Val Gly Ala Tyr Gly Gly
                10                  15                  20

tcc ttc acc ggc gtc cct gtt gaa gaa ttg gcc acc acc gtg atc aac 211
Ser Phe Thr Gly Val Pro Val Glu Glu Leu Ala Thr Thr Val Ile Asn
            25                  30                  35

gcg atc gtt gag gca acc ggc atc acc ggc gac gat gtg gac gat ctg 259
Ala Ile Val Glu Ala Thr Gly Ile Thr Gly Asp Asp Val Asp Asp Leu
        40                  45                  50

atc ctc ggc cag gca tcc ccc aac ggt gcg gct cca gca ctg ggc cgt 307
Ile Leu Gly Gln Ala Ser Pro Asn Gly Ala Ala Pro Ala Leu Gly Arg
    55                  60                  65

gtt gtt gct cta gat tcc aag ctt ggc caa aac gtt cca ggc atg cag 355
Val Val Ala Leu Asp Ser Lys Leu Gly Gln Asn Val Pro Gly Met Gln
70                  75                  80                  85

ctt gat cgc cgc tgt ggt tcc ggc ctg cag gca atc gtc acc gct gct 403
Leu Asp Arg Arg Cys Gly Ser Gly Leu Gln Ala Ile Val Thr Ala Ala
                90                  95                  100

gca cac gtt gca tcc ggc gct gct gat ctg atc atc gca ggt ggc gca 451
Ala His Val Ala Ser Gly Ala Ala Asp Leu Ile Ile Ala Gly Gly Ala
            105                 110                 115

gaa tcc atg agc cgc gtt gag tac acc gtg tcc ggc gat atc cgt tgg 499
Glu Ser Met Ser Arg Val Glu Tyr Thr Val Ser Gly Asp Ile Arg Trp
        120                 125                 130

ggt gtc aag ggc ggc gac atg cag ctt cgt gac cgc ctt gca gaa gca 547
Gly Val Lys Gly Gly Asp Met Gln Leu Arg Asp Arg Leu Ala Glu Ala
    135                 140                 145

cgc gaa acc gct ggc gga cgc aac cac ccg atc cct ggt ggc atg atc 595
Arg Glu Thr Ala Gly Gly Arg Asn His Pro Ile Pro Gly Gly Met Ile
150                 155                 160                 165

gag acc gct gag aac ctg cgt cgc gaa tac ggc atc tcc cgc gag gag 643
Glu Thr Ala Glu Asn Leu Arg Arg Glu Tyr Gly Ile Ser Arg Glu Glu
                170                 175                 180

cag gac aag atc tcc gca gcg tcc cag cag cgt tgg ggc aag gct gct 691
Gln Asp Lys Ile Ser Ala Ala Ser Gln Gln Arg Trp Gly Lys Ala Ala
            185                 190                 195

gat gcg ggg ctt ttc gac gac gag atc gtg cca gtc acc gtc cct gcc 739
Asp Ala Gly Leu Phe Asp Asp Glu Ile Val Pro Val Thr Val Pro Ala
        200                 205                 210

aag aag cgc ggc cag gag cca acc atc gtt tct cga gac gag cat ggt 787

Lys Lys Arg Gly Gln Glu Pro Thr Ile Val Ser Arg Asp Glu His Gly
    215                 220                 225

cga cca gga aca acc gtc gaa aag ctt gct gct ttg cgc ccc atc atg     835
Arg Pro Gly Thr Thr Val Glu Lys Leu Ala Ala Leu Arg Pro Ile Met
230                 235                 240                 245

ggc cgc cag gat gcg gaa gca acc gtc acc gct ggc aac gcg tcc ggc     883
Gly Arg Gln Asp Ala Glu Ala Thr Val Thr Ala Gly Asn Ala Ser Gly
                250                 255                 260

caa aat gat ggc gct gct gcc gtc atc gtg acc act cgc gcc aag gcc     931
Gln Asn Asp Gly Ala Ala Ala Val Ile Val Thr Thr Arg Ala Lys Ala
            265                 270                 275

gag gag aag ggc ctg cgc cca gtc atg cgt ttg gct ggc tgg tct gtg     979
Glu Glu Lys Gly Leu Arg Pro Val Met Arg Leu Ala Gly Trp Ser Val
        280                 285                 290

gct gct gtt ccc cca gag acc atg ggt att gga cct gtt cct gcc acc     1027
Ala Ala Val Pro Pro Glu Thr Met Gly Ile Gly Pro Val Pro Ala Thr
    295                 300                 305

aag aag gtc ctg gat cgt ttg ggc ctt acc ctg gag gac atc ggc gcg     1075
Lys Lys Val Leu Asp Arg Leu Gly Leu Thr Leu Glu Asp Ile Gly Ala
310                 315                 320                 325

atc gaa ctc aac gaa gct ttc gca gct cag gca ctg tct gtg ctg aag     1123
Ile Glu Leu Asn Glu Ala Phe Ala Ala Gln Ala Leu Ser Val Leu Lys
                330                 335                 340

gaa tgg aac att tct tgg gaa gat gag cgc gtc aac cca ctg ggt tcc     1171
Glu Trp Asn Ile Ser Trp Glu Asp Glu Arg Val Asn Pro Leu Gly Ser
            345                 350                 355

ggt att tcc atg gga cac cca gtc ggt gcc acc ggt gct cgc atg gca     1219
Gly Ile Ser Met Gly His Pro Val Gly Ala Thr Gly Ala Arg Met Ala
        360                 365                 370

gta acc ttg gct cac cgc atg cag cgt gaa aac act cag tac gga ctg     1267
Val Thr Leu Ala His Arg Met Gln Arg Glu Asn Thr Gln Tyr Gly Leu
    375                 380                 385

gcc acc atg tgc atc ggt ggc ggc cag ggt ctt gca gct gtc ttt gaa     1315
Ala Thr Met Cys Ile Gly Gly Gly Gln Gly Leu Ala Ala Val Phe Glu
390                 395                 400                 405

aag gag aac taaaaatggc tattttgcac agc                               1347
Lys Glu Asn

<210> 96
<211> 408
<212> PRT
<213> Corynebacterium glutamicum

<400> 96
Met Asn Pro Gln Asp Ile Val Ile Cys Ser Pro Leu Arg Thr Pro Val
  1               5                  10                  15

Gly Ala Tyr Gly Gly Ser Phe Thr Gly Val Pro Val Glu Glu Leu Ala
            20                  25                  30

Thr Thr Val Ile Asn Ala Ile Val Glu Ala Thr Gly Ile Thr Gly Asp
        35                  40                  45

Asp Val Asp Asp Leu Ile Leu Gly Gln Ala Ser Pro Asn Gly Ala Ala
    50                  55                  60

Pro Ala Leu Gly Arg Val Val Ala Leu Asp Ser Lys Leu Gly Gln Asn
65                  70                  75                  80

Val Pro Gly Met Gln Leu Asp Arg Arg Cys Gly Ser Gly Leu Gln Ala
                85                  90                  95

Ile Val Thr Ala Ala Ala His Val Ala Ser Gly Ala Ala Asp Leu Ile
            100                 105                 110

Ile Ala Gly Gly Ala Glu Ser Met Ser Arg Val Glu Tyr Thr Val Ser
        115                 120                 125

Gly Asp Ile Arg Trp Gly Val Lys Gly Gly Asp Met Gln Leu Arg Asp
    130                 135                 140

Arg Leu Ala Glu Ala Arg Glu Thr Ala Gly Gly Arg Asn His Pro Ile
145                 150                 155                 160

Pro Gly Gly Met Ile Glu Thr Ala Glu Asn Leu Arg Arg Glu Tyr Gly
                165                 170                 175

Ile Ser Arg Glu Glu Gln Asp Lys Ile Ser Ala Ala Ser Gln Gln Arg
            180                 185                 190

Trp Gly Lys Ala Ala Asp Ala Gly Leu Phe Asp Asp Glu Ile Val Pro
        195                 200                 205

Val Thr Val Pro Ala Lys Lys Arg Gly Gln Glu Pro Thr Ile Val Ser
    210                 215                 220

Arg Asp Glu His Gly Arg Pro Gly Thr Thr Val Glu Lys Leu Ala Ala
225                 230                 235                 240

Leu Arg Pro Ile Met Gly Arg Gln Asp Ala Glu Ala Thr Val Thr Ala
                245                 250                 255

Gly Asn Ala Ser Gly Gln Asn Asp Gly Ala Ala Ala Val Ile Val Thr
            260                 265                 270

Thr Arg Ala Lys Ala Glu Glu Lys Gly Leu Arg Pro Val Met Arg Leu
        275                 280                 285

Ala Gly Trp Ser Val Ala Ala Val Pro Pro Glu Thr Met Gly Ile Gly
    290                 295                 300

Pro Val Pro Ala Thr Lys Lys Val Leu Asp Arg Leu Gly Leu Thr Leu
305                 310                 315                 320

Glu Asp Ile Gly Ala Ile Glu Leu Asn Glu Ala Phe Ala Ala Gln Ala
                325                 330                 335

Leu Ser Val Leu Lys Glu Trp Asn Ile Ser Trp Glu Asp Glu Arg Val

340                 345                 350

Asn Pro Leu Gly Ser Gly Ile Ser Met Gly His Pro Val Gly Ala Thr
        355                 360                 365

Gly Ala Arg Met Ala Val Thr Leu Ala His Arg Met Gln Arg Glu Asn
    370                 375                 380

Thr Gln Tyr Gly Leu Ala Thr Met Cys Ile Gly Gly Gly Gln Gly Leu
385                 390                 395                 400

Ala Ala Val Phe Glu Lys Glu Asn
                405

<210> 97
<211> 815
<212> DNA
<213> Corynebacterium glutamicum

<220>
<221> CDS
<222> (1)..(792)
<223> FRXA01114

<400> 97
cgc ctt gca gaa gca cgc gaa acc gct ggc gga cgc aac cac ccg atc      48
Arg Leu Ala Glu Ala Arg Glu Thr Ala Gly Gly Arg Asn His Pro Ile
  1               5                  10                  15

cct ggt ggc atg atc gag acc gct gag aac ctg cgt cgc gaa tac ggc      96
Pro Gly Gly Met Ile Glu Thr Ala Glu Asn Leu Arg Arg Glu Tyr Gly
             20                  25                  30

atc tcc cgc gag gag cag gac aag atc tcc gca gcg tcc cag cag cgt     144
Ile Ser Arg Glu Glu Gln Asp Lys Ile Ser Ala Ala Ser Gln Gln Arg
         35                  40                  45

tgg ggc aag gct gct gat gcg ggg ctt ttc gac gac gag atc gtg cca     192
Trp Gly Lys Ala Ala Asp Ala Gly Leu Phe Asp Asp Glu Ile Val Pro
     50                  55                  60

gtc acc gtc cct gcc aag aag cgc ggc cag gag cca acc atc gtt tct     240
Val Thr Val Pro Ala Lys Lys Arg Gly Gln Glu Pro Thr Ile Val Ser
 65                  70                  75                  80

cga gac gag cat ggt cga cca gga aca acc gtc gaa aag ctt gct gct     288
Arg Asp Glu His Gly Arg Pro Gly Thr Thr Val Glu Lys Leu Ala Ala
                 85                  90                  95

ttg cgc ccc atc atg ggc cgc cag gat gcg gaa gca acc gtc acc gct     336
Leu Arg Pro Ile Met Gly Arg Gln Asp Ala Glu Ala Thr Val Thr Ala
            100                 105                 110

ggc aac gcg tcc ggc caa aat gat ggc gct gct gcc gtc atc gtg acc     384
Gly Asn Ala Ser Gly Gln Asn Asp Gly Ala Ala Ala Val Ile Val Thr
        115                 120                 125

act cgc gcc aag gcc gag gag aag ggc ctg cgc cca gtc atg cgt ttg     432
Thr Arg Ala Lys Ala Glu Glu Lys Gly Leu Arg Pro Val Met Arg Leu
    130                 135                 140

gct ggc tgg tct gtg gct gct gtt ccc cca gag acc atg ggt att gga     480
Ala Gly Trp Ser Val Ala Ala Val Pro Pro Glu Thr Met Gly Ile Gly
145                 150                 155                 160

cct gtt cct gcc acc aag aag gtc ctg gat cgt ttg ggc ctt acc ctg     528
Pro Val Pro Ala Thr Lys Lys Val Leu Asp Arg Leu Gly Leu Thr Leu
                165                 170                 175

gag gac atc ggc gcg atc gaa ctc aac gaa gct ttc gca gct cag gca     576
Glu Asp Ile Gly Ala Ile Glu Leu Asn Glu Ala Phe Ala Ala Gln Ala
            180                 185                 190

ctg tct gtg ctg aag gaa tgg aac att tct tgg gaa gat gag cgc gtc     624
Leu Ser Val Leu Lys Glu Trp Asn Ile Ser Trp Glu Asp Glu Arg Val
        195                 200                 205

aac cca ctg ggt tcc ggt att tcc atg gga cac cca gtc ggt gcc acc     672
Asn Pro Leu Gly Ser Gly Ile Ser Met Gly His Pro Val Gly Ala Thr
    210                 215                 220

ggt gct cgc atg gca gta acc ttg gct cac cgc atg cag cgt gaa aac     720
Gly Ala Arg Met Ala Val Thr Leu Ala His Arg Met Gln Arg Glu Asn
225                 230                 235                 240

act cag tac gga ctg gcc acc atg tgc atc ggt ggc ggc cag ggt ctt     768
Thr Gln Tyr Gly Leu Ala Thr Met Cys Ile Gly Gly Gly Gln Gly Leu
                245                 250                 255

gca gct gtc ttt gaa aag gag aac taaaaatggc tattttgcac agc          815
Ala Ala Val Phe Glu Lys Glu Asn
            260


<210> 98
<211> 264
<212> PRT
<213> Corynebacterium glutamicum

<400> 98
Arg Leu Ala Glu Ala Arg Glu Thr Ala Gly Gly Arg Asn His Pro Ile
1               5                   10                  15

Pro Gly Gly Met Ile Glu Thr Ala Glu Asn Leu Arg Arg Glu Tyr Gly
            20                  25                  30

Ile Ser Arg Glu Glu Gln Asp Lys Ile Ser Ala Ala Ser Gln Gln Arg
        35                  40                  45

Trp Gly Lys Ala Ala Asp Ala Gly Leu Phe Asp Asp Glu Ile Val Pro
    50                  55                  60

Val Thr Val Pro Ala Lys Lys Arg Gly Gln Glu Pro Thr Ile Val Ser
65                  70                  75                  80

Arg Asp Glu His Gly Arg Pro Gly Thr Thr Val Glu Lys Leu Ala Ala
                85                  90                  95

Leu Arg Pro Ile Met Gly Arg Gln Asp Ala Glu Ala Thr Val Thr Ala
            100                 105                 110

Gly Asn Ala Ser Gly Gln Asn Asp Gly Ala Ala Ala Val Ile Val Thr
        115                 120                 125

Thr Arg Ala Lys Ala Glu Glu Lys Gly Leu Arg Pro Val Met Arg Leu
    130                 135                 140

Ala Gly Trp Ser Val Ala Ala Val Pro Pro Glu Thr Met Gly Ile Gly
145                 150                 155                 160

Pro Val Pro Ala Thr Lys Lys Val Leu Asp Arg Leu Gly Leu Thr Leu
                165                 170                 175

Glu Asp Ile Gly Ala Ile Glu Leu Asn Glu Ala Phe Ala Ala Gln Ala
            180                 185                 190

Leu Ser Val Leu Lys Glu Trp Asn Ile Ser Trp Glu Asp Glu Arg Val
        195                 200                 205

Asn Pro Leu Gly Ser Gly Ile Ser Met Gly His Pro Val Gly Ala Thr
    210                 215                 220

Gly Ala Arg Met Ala Val Thr Leu Ala His Arg Met Gln Arg Glu Asn
225                 230                 235                 240

Thr Gln Tyr Gly Leu Ala Thr Met Cys Ile Gly Gly Gly Gln Gly Leu
                245                 250                 255

Ala Ala Val Phe Glu Lys Glu Asn
            260

<210> 99
<211> 978
<212> DNA
<213> Corynebacterium glutamicum

<220>
<221> CDS
<222> (101)..(955)
<223> RXA01894

<400> 99
agaatttttt cgaaaatgct ggcaccatca acagtgacat tgttagaaac ttcaaggaga 60

acccatgaat gaaccggagc aacatcaccg gtccatgagg atg ccc aaa ccc aaa    115
                                          Met Pro Lys Pro Lys
                                          1               5

aat aat gcg ggt cga gat ctc aaa gct gcc att gct gtg ggg atc gga    163
Asn Asn Ala Gly Arg Asp Leu Lys Ala Ala Ile Ala Val Gly Ile Gly
                10                  15                  20

ctg ggg gtc ctg gtt ctt ttg ggg att gtc cta agc cca tgg ggt tgg    211
Leu Gly Val Leu Val Leu Leu Gly Ile Val Leu Ser Pro Trp Gly Trp
            25                  30                  35

tac atc ctc gtt gca ggt ttt atg gct gca gca aca tgg gaa gtt ggt    259
Tyr Ile Leu Val Ala Gly Phe Met Ala Ala Ala Thr Trp Glu Val Gly
        40                  45                  50

agc aga ctt aaa gaa ggc ggc tat cat ttg cca ctg ccg att atg atc    307

Ser Arg Leu Lys Glu Gly Gly Tyr His Leu Pro Leu Pro Ile Met Ile
    55                  60                  65

atc ggc ggt cag gca atc atc tgg ctg tca tgg cca ttt ggc acg atg 355
Ile Gly Gly Gln Ala Ile Ile Trp Leu Ser Trp Pro Phe Gly Thr Met
70                  75                  80                  85

ggc att ttg gcg tct ttt gtg gcc act gtg ttg gtg ctg atg tat ttc 403
Gly Ile Leu Ala Ser Phe Val Ala Thr Val Leu Val Leu Met Tyr Phe
                90                  95                  100

cga att ttc tac aat ggc acg gaa aaa gaa gcc cgc aac tat ttg agg 451
Arg Ile Phe Tyr Asn Gly Thr Glu Lys Glu Ala Arg Asn Tyr Leu Arg
            105                 110                 115

gac acc tct gtg ggc atc ttc gtg ctc acc tgg att cca ttg ttc gga 499
Asp Thr Ser Val Gly Ile Phe Val Leu Thr Trp Ile Pro Leu Phe Gly
        120                 125                 130

agc ttc gct gcg atg ctg tcg ctg atg caa aac aat tcc atc ccg ggt 547
Ser Phe Ala Ala Met Leu Ser Leu Met Gln Asn Asn Ser Ile Pro Gly
    135                 140                 145

aca tat ttc att ttg acg ttc atg ctg tgt gtg atc gca tcg gat gtg 595
Thr Tyr Phe Ile Leu Thr Phe Met Leu Cys Val Ile Ala Ser Asp Val
150                 155                 160                 165

ggc ggg tat atc gcg ggt gtg ttc ttt gga tcg cac cca atg gcg ccg 643
Gly Gly Tyr Ile Ala Gly Val Phe Phe Gly Ser His Pro Met Ala Pro
                170                 175                 180

ttg gtg agt ccg aag aag tct tgg gaa ggc ttt gcc ggc tcc att gtc 691
Leu Val Ser Pro Lys Lys Ser Trp Glu Gly Phe Ala Gly Ser Ile Val
            185                 190                 195

tta gga tcg gtc act ggt gca ctc agt gtt cac ttc ctg ctc gat cac 739
Leu Gly Ser Val Thr Gly Ala Leu Ser Val His Phe Leu Leu Asp His
        200                 205                 210

cac tgg tgg atg ggt gtg atc ttg ggt tgt gcc cta gtt gtg tgc gcc 787
His Trp Trp Met Gly Val Ile Leu Gly Cys Ala Leu Val Val Cys Ala
    215                 220                 225

acg ttg ggt gac ttg gtt gag tcg cag ttc aaa cgc gat ttg ggc atc 835
Thr Leu Gly Asp Leu Val Glu Ser Gln Phe Lys Arg Asp Leu Gly Ile
230                 235                 240                 245

aag gat atg tcg aac ctt ctt cca ggc cac ggc gga ttg atg gac cgt 883
Lys Asp Met Ser Asn Leu Leu Pro Gly His Gly Gly Leu Met Asp Arg
                250                 255                 260

ttg gat ggc atg ctc ccg gcc gcg atg gtg acg tgg ttg atc ctg agt 931
Leu Asp Gly Met Leu Pro Ala Ala Met Val Thr Trp Leu Ile Leu Ser
            265                 270                 275

gtg atc agc agc tcg tat ccg tcg taaagcttgg gccagcttta agt 978
Val Ile Ser Ser Ser Tyr Pro Ser
        280                 285

<210> 100

<211> 285
<212> PRT
<213> Corynebacterium glutamicum

<400> 100
Met Pro Lys Pro Lys Asn Asn Ala Gly Arg Asp Leu Lys Ala Ala Ile
1               5                   10                  15

Ala Val Gly Ile Gly Leu Gly Val Leu Val Leu Leu Gly Ile Val Leu
            20                  25                  30

Ser Pro Trp Gly Trp Tyr Ile Leu Val Ala Gly Phe Met Ala Ala Ala
        35                  40                  45

Thr Trp Glu Val Gly Ser Arg Leu Lys Glu Gly Gly Tyr His Leu Pro
    50                  55                  60

Leu Pro Ile Met Ile Ile Gly Gly Gln Ala Ile Ile Trp Leu Ser Trp
65                  70                  75                  80

Pro Phe Gly Thr Met Gly Ile Leu Ala Ser Phe Val Ala Thr Val Leu
                85                  90                  95

Val Leu Met Tyr Phe Arg Ile Phe Tyr Asn Gly Thr Glu Lys Glu Ala
            100                 105                 110

Arg Asn Tyr Leu Arg Asp Thr Ser Val Gly Ile Phe Val Leu Thr Trp
        115                 120                 125

Ile Pro Leu Phe Gly Ser Phe Ala Ala Met Leu Ser Leu Met Gln Asn
    130                 135                 140

Asn Ser Ile Pro Gly Thr Tyr Phe Ile Leu Thr Phe Met Leu Cys Val
145                 150                 155                 160

Ile Ala Ser Asp Val Gly Gly Tyr Ile Ala Gly Val Phe Phe Gly Ser
                165                 170                 175

His Pro Met Ala Pro Leu Val Ser Pro Lys Lys Ser Trp Glu Gly Phe
            180                 185                 190

Ala Gly Ser Ile Val Leu Gly Ser Val Thr Gly Ala Leu Ser Val His
        195                 200                 205

Phe Leu Leu Asp His His Trp Trp Met Gly Val Ile Leu Gly Cys Ala
    210                 215                 220

Leu Val Val Cys Ala Thr Leu Gly Asp Leu Val Glu Ser Gln Phe Lys
225                 230                 235                 240

Arg Asp Leu Gly Ile Lys Asp Met Ser Asn Leu Leu Pro Gly His Gly
                245                 250                 255

Gly Leu Met Asp Arg Leu Asp Gly Met Leu Pro Ala Ala Met Val Thr
            260                 265                 270

Trp Leu Ile Leu Ser Val Ile Ser Ser Ser Tyr Pro Ser
        275                 280                 285

<210> 101

<211> 600
<212> DNA
<213> Corynebacterium glutamicum

<220>
<221> CDS
<222> (101)..(577)
<223> RXA02599

<400> 101
gatcaagcat catacgatgt cggagattgt ggcgggcgtt gttacaggag cagttgcaac 60

cggcatttgt tatgcactcc tacttgcgta aaggagaatt atg gat cag cta atc 115
Met Asp Gln Leu Ile
1               5

ctc gac gca ttt atc ggc ttg aga gtg act tgg ctg agt ccg gtg att 163
Leu Asp Ala Phe Ile Gly Leu Arg Val Thr Trp Leu Ser Pro Val Ile
                10                  15                  20

att ttg ttc acc cag ctc acc ggg cca aca ctg atg ttt gtg tat gcg 211
Ile Leu Phe Thr Gln Leu Thr Gly Pro Thr Leu Met Phe Val Tyr Ala
            25                  30                  35

ctt gtg tgg ggc ttg ttg cgc aag agc gcc act gcc ccg atc gcg gtg 259
Leu Val Trp Gly Leu Leu Arg Lys Ser Ala Thr Ala Pro Ile Ala Val
        40                  45                  50

ggg ctg gct aat ctg atc agt cat ttt ctc aag agg gcg ttt gaa cgg 307
Gly Leu Ala Asn Leu Ile Ser His Phe Leu Lys Arg Ala Phe Glu Arg
    55                  60                  65

cct cga cca aat aca gca gag cac ttg gtt gta gaa act aac ttt tca 355
Pro Arg Pro Asn Thr Ala Glu His Leu Val Val Glu Thr Asn Phe Ser
70                  75                  80                  85

ttc cct tct ggt cat gct gtg ggc gct gca gca tgt gcc gtg gca gtg 403
Phe Pro Ser Gly His Ala Val Gly Ala Ala Ala Cys Ala Val Ala Val
                90                  95                  100

ggg tac tcc gtg aac cgg tgg tgg aaa ctc acg ctg tgg gta atc gcg 451
Gly Tyr Ser Val Asn Arg Trp Trp Lys Leu Thr Leu Trp Val Ile Ala
            105                 110                 115

ctg ctt gtg ggg ctg tct cgg ttg tat gtc ggt gtg cat tgg ccc agc 499
Leu Leu Val Gly Leu Ser Arg Leu Tyr Val Gly Val His Trp Pro Ser
        120                 125                 130

gat gtg ctt gcc ggc tgg gcc atc ggt gcg ttg act tca gtg gtg gtg 547
Asp Val Leu Ala Gly Trp Ala Ile Gly Ala Leu Thr Ser Val Val Val
    135                 140                 145

ttt acc agc tgg aac ctc ctc cag cgc cgc tgaaaccact gctgaaagtg 597
Phe Thr Ser Trp Asn Leu Leu Gln Arg Arg
150                 155

gta 600


<210> 102
<211> 159

<212> PRT
<213> Corynebacterium glutamicum

<400> 102
Met Asp Gln Leu Ile Leu Asp Ala Phe Ile Gly Leu Arg Val Thr Trp
1               5                   10                  15

Leu Ser Pro Val Ile Ile Leu Phe Thr Gln Leu Thr Gly Pro Thr Leu
            20                  25                  30

Met Phe Val Tyr Ala Leu Val Trp Gly Leu Leu Arg Lys Ser Ala Thr
        35                  40                  45

Ala Pro Ile Ala Val Gly Leu Ala Asn Leu Ile Ser His Phe Leu Lys
    50                  55                  60

Arg Ala Phe Glu Arg Pro Arg Pro Asn Thr Ala Glu His Leu Val Val
65                  70                  75                  80

Glu Thr Asn Phe Ser Phe Pro Ser Gly His Ala Val Gly Ala Ala Ala
                85                  90                  95

Cys Ala Val Ala Val Gly Tyr Ser Val Asn Arg Trp Trp Lys Leu Thr
            100                 105                 110

Leu Trp Val Ile Ala Leu Leu Val Gly Leu Ser Arg Leu Tyr Val Gly
        115                 120                 125

Val His Trp Pro Ser Asp Val Leu Ala Gly Trp Ala Ile Gly Ala Leu
    130                 135                 140

Thr Ser Val Val Val Phe Thr Ser Trp Asn Leu Leu Gln Arg Arg
145                 150                 155


<210> 103
<211> 999
<212> DNA
<213> Corynebacterium glutamicum

<220>
<221> CDS
<222> (101)..(976)
<223> RXN02638

<400> 103
ctctgttgtt gttcctgagg cgcttcgtcc attcgtgggc aaagacgtgc tcaagccagt 60

gaaacaagcc ggttaattaa gcggaaagct cgtggtcgac atg gtc aaa agg ttt   115
                                           Met Val Lys Arg Phe
                                           1               5

ggc ttt ttc gta gag gat tcc ctg cct aag gtt ccg ctg cat ccc gaa   163
Gly Phe Phe Val Glu Asp Ser Leu Pro Lys Val Pro Leu His Pro Glu
                10                  15                  20

gag tca cgg gag acg ttt tat ggg cgc atc atc att agt gct gtg cgg   211
Glu Ser Arg Glu Thr Phe Tyr Gly Arg Ile Ile Ile Ser Ala Val Arg
            25                  30                  35

acg gtg atg aaa gcc cag gat gtg cag att tcc atc ttc ggt gcg gag   259

Thr Val Met Lys Ala Gln Asp Val Gln Ile Ser Ile Phe Gly Ala Glu
        40                  45                  50

aac att ccg acc acc ggc ggc gca ctt ttc gca tca acc aca ctg gtt     307
Asn Ile Pro Thr Thr Gly Gly Ala Leu Phe Ala Ser Thr Thr Leu Val
    55                  60                  65

att atg act tca ttc tgg gtg gta tcc ccc gca ttc gtg cgg ggt aag     355
Ile Met Thr Ser Phe Trp Val Val Ser Pro Ala Phe Val Arg Gly Lys
70                  75                  80                  85

cgc ctg gtt cga ttc atg gcg aag aag gaa att ttc gac acc cca gtt     403
Arg Leu Val Arg Phe Met Ala Lys Lys Glu Ile Phe Asp Thr Pro Val
                90                  95                  100

gtc ggc acc ctc atg cgc tgg atg aag cac gtc tct gtg gac cgc tcc     451
Val Gly Thr Leu Met Arg Trp Met Lys His Val Ser Val Asp Arg Ser
            105                 110                 115

gca ggt gcc ggt tcc atg gaa gat gcg cgg aag cgt ctc gac gcc ggc     499
Ala Gly Ala Gly Ser Met Glu Asp Ala Arg Lys Arg Leu Asp Ala Gly
        120                 125                 130

agc ctc gtc ggt atc ttc cct gag gcg acg gtg tca cgg tcc ttt gaa     547
Ser Leu Val Gly Ile Phe Pro Glu Ala Thr Val Ser Arg Ser Phe Glu
    135                 140                 145

atc aag gaa cta aaa act ggc gcc gtc cgc atc gcc gac agc gct aac     595
Ile Lys Glu Leu Lys Thr Gly Ala Val Arg Ile Ala Asp Ser Ala Asn
150                 155                 160                 165

gtt ccg ctg ctg cca ctt att att tgg ggc ggc cag cgc atc atc acc     643
Val Pro Leu Leu Pro Leu Ile Ile Trp Gly Gly Gln Arg Ile Ile Thr
                170                 175                 180

aaa gac atc gag cgc gac ttc ggc cgc tcc cac atc ccc gta ttc atc     691
Lys Asp Ile Glu Arg Asp Phe Gly Arg Ser His Ile Pro Val Phe Ile
            185                 190                 195

agc gtg ggt gaa ccc gtc gac gcc agc ggc gat ccc gac gaa gca acg     739
Ser Val Gly Glu Pro Val Asp Ala Ser Gly Asp Pro Asp Glu Ala Thr
        200                 205                 210

gaa cgc ctc tac gag gct atg aaa aag ctt ctc gac gaa acc cgc acc     787
Glu Arg Leu Tyr Glu Ala Met Lys Lys Leu Leu Asp Glu Thr Arg Thr
    215                 220                 225

gcc tac gaa caa aag tat ggc cca ttc gaa ggt gga gaa ttg tgg cgc     835
Ala Tyr Glu Gln Lys Tyr Gly Pro Phe Glu Gly Gly Glu Leu Trp Arg
230                 235                 240                 245

ccg aaa tcc ctc ggc ggc ggc gcc cca acg ttg gag cag gcg aaa atg     883
Pro Lys Ser Leu Gly Gly Gly Ala Pro Thr Leu Glu Gln Ala Lys Met
                250                 255                 260

ttg gaa atc gcc gaa cgg gaa cgt cga caa gca aaa cgc gcg gca aag     931
Leu Glu Ile Ala Glu Arg Glu Arg Arg Gln Ala Lys Arg Ala Ala Lys
            265                 270                 275

gtc gcc aag aaa cgc acc acc ttt ata agg aaa atc ttt aaa aaa         976
Val Ala Lys Lys Arg Thr Thr Phe Ile Arg Lys Ile Phe Lys Lys

280                 285                 290

tgattgcact gggttcagcg ccc                                              999

<210> 104
<211> 292
<212> PRT
<213> Corynebacterium glutamicum

<400> 104
Met Val Lys Arg Phe Gly Phe Phe Val Glu Asp Ser Leu Pro Lys Val
1               5                   10                  15

Pro Leu His Pro Glu Glu Ser Arg Glu Thr Phe Tyr Gly Arg Ile Ile
            20                  25                  30

Ile Ser Ala Val Arg Thr Val Met Lys Ala Gln Asp Val Gln Ile Ser
        35                  40                  45

Ile Phe Gly Ala Glu Asn Ile Pro Thr Thr Gly Gly Ala Leu Phe Ala
    50                  55                  60

Ser Thr Thr Leu Val Ile Met Thr Ser Phe Trp Val Val Ser Pro Ala
65                  70                  75                  80

Phe Val Arg Gly Lys Arg Leu Val Arg Phe Met Ala Lys Lys Glu Ile
                85                  90                  95

Phe Asp Thr Pro Val Val Gly Thr Leu Met Arg Trp Met Lys His Val
            100                 105                 110

Ser Val Asp Arg Ser Ala Gly Ala Gly Ser Met Glu Asp Ala Arg Lys
        115                 120                 125

Arg Leu Asp Ala Gly Ser Leu Val Gly Ile Phe Pro Glu Ala Thr Val
    130                 135                 140

Ser Arg Ser Phe Glu Ile Lys Glu Leu Lys Thr Gly Ala Val Arg Ile
145                 150                 155                 160

Ala Asp Ser Ala Asn Val Pro Leu Leu Pro Leu Ile Ile Trp Gly Gly
                165                 170                 175

Gln Arg Ile Ile Thr Lys Asp Ile Glu Arg Asp Phe Gly Arg Ser His
            180                 185                 190

Ile Pro Val Phe Ile Ser Val Gly Glu Pro Val Asp Ala Ser Gly Asp
        195                 200                 205

Pro Asp Glu Ala Thr Glu Arg Leu Tyr Glu Ala Met Lys Lys Leu Leu
    210                 215                 220

Asp Glu Thr Arg Thr Ala Tyr Glu Gln Lys Tyr Gly Pro Phe Glu Gly
225                 230                 235                 240

Gly Glu Leu Trp Arg Pro Lys Ser Leu Gly Gly Gly Ala Pro Thr Leu
                245                 250                 255

Glu Gln Ala Lys Met Leu Glu Ile Ala Glu Arg Glu Arg Arg Gln Ala
            260                 265                 270

Lys Arg Ala Ala Lys Val Ala Lys Lys Arg Thr Thr Phe Ile Arg Lys
        275                 280                 285

Ile Phe Lys Lys
    290


<210> 105
<211> 534
<212> DNA
<213> Corynebacterium glutamicum

<220>
<221> CDS
<222> (8)..(511)
<223> FRXA02638

<400> 105
atgaagaatg gcg cgg aag cgt ctt aac gcc ggc agc ctc gtc ggt atc      49
           Met Ala Arg Lys Arg Leu Asn Ala Gly Ser Leu Val Gly Ile
           1               5                   10

ttc cct gag gcg acg gtg tca cgg tcc ttt gaa atc aag gaa cta aaa      97
Phe Pro Glu Ala Thr Val Ser Arg Ser Phe Glu Ile Lys Glu Leu Lys
15                  20                  25                  30

act ggc gcc gtc cgc atc gcc gac agc gct aac gtt ccg ctg ctg cca     145
Thr Gly Ala Val Arg Ile Ala Asp Ser Ala Asn Val Pro Leu Leu Pro
                35                  40                  45

ctt att att tgg ggc ggc cag cgc atc atc acc aaa gac atc gag cgc     193
Leu Ile Ile Trp Gly Gly Gln Arg Ile Ile Thr Lys Asp Ile Glu Arg
            50                  55                  60

gac ttc ggc cgc tcc cac atc ccc gta ttc atc agc gtg ggt gaa ccc     241
Asp Phe Gly Arg Ser His Ile Pro Val Phe Ile Ser Val Gly Glu Pro
        65                  70                  75

gtc gac gcc agc ggc gat ccc gac gaa gca acg gaa cgc ctc tac gag     289
Val Asp Ala Ser Gly Asp Pro Asp Glu Ala Thr Glu Arg Leu Tyr Glu
    80                  85                  90

gct atg aaa aag ctt ctc gac gaa acc cgc acc gcc tac gaa caa aag     337
Ala Met Lys Lys Leu Leu Asp Glu Thr Arg Thr Ala Tyr Glu Gln Lys
95                  100                 105                 110

tat ggc cca ttc gaa ggt gga gaa ttg tgg cgc ccg aaa tcc ctc ggc     385
Tyr Gly Pro Phe Glu Gly Gly Glu Leu Trp Arg Pro Lys Ser Leu Gly
                115                 120                 125

ggc ggc gcc cca acg ttg gag cag gcg aaa atg ttg gaa atc gcc gaa     433
Gly Gly Ala Pro Thr Leu Glu Gln Ala Lys Met Leu Glu Ile Ala Glu
            130                 135                 140

cgg gaa cgt cga caa gca aaa cgc gcg gca aag gtc gcc aag aaa cgc     481
Arg Glu Arg Arg Gln Ala Lys Arg Ala Ala Lys Val Ala Lys Lys Arg
        145                 150                 155

acc acc ttt ata agg aaa atc ttt aaa aaa tgattgcact gggttcagcg       531
Thr Thr Phe Ile Arg Lys Ile Phe Lys Lys

160                 165

ccc                                                                    534

<210> 106
<211> 168
<212> PRT
<213> Corynebacterium glutamicum

<400> 106
Met Ala Arg Lys Arg Leu Asn Ala Gly Ser Leu Val Gly Ile Phe Pro
1               5                   10                  15

Glu Ala Thr Val Ser Arg Ser Phe Glu Ile Lys Glu Leu Lys Thr Gly
            20                  25                  30

Ala Val Arg Ile Ala Asp Ser Ala Asn Val Pro Leu Leu Pro Leu Ile
        35                  40                  45

Ile Trp Gly Gly Gln Arg Ile Ile Thr Lys Asp Ile Glu Arg Asp Phe
    50                  55                  60

Gly Arg Ser His Ile Pro Val Phe Ile Ser Val Gly Glu Pro Val Asp
65                  70                  75                  80

Ala Ser Gly Asp Pro Asp Glu Ala Thr Glu Arg Leu Tyr Glu Ala Met
                85                  90                  95

Lys Lys Leu Leu Asp Glu Thr Arg Thr Ala Tyr Glu Gln Lys Tyr Gly
            100                 105                 110

Pro Phe Glu Gly Gly Glu Leu Trp Arg Pro Lys Ser Leu Gly Gly Gly
        115                 120                 125

Ala Pro Thr Leu Glu Gln Ala Lys Met Leu Glu Ile Ala Glu Arg Glu
    130                 135                 140

Arg Arg Gln Ala Lys Arg Ala Ala Lys Val Ala Lys Lys Arg Thr Thr
145                 150                 155                 160

Phe Ile Arg Lys Ile Phe Lys Lys
                165

<210> 107
<211> 637
<212> DNA
<213> Corynebacterium glutamicum

<220>
<221> CDS
<222> (101)..(637)
<223> RXA00856

<400> 107
gtttttagga atagagtggg ctcaagcttt gtgacaagtt ttttggagaa atcattacta 60

gtcgtagtct tcaatttggg tgcaggtagg gtggaacacc gtg agt gat gta tca   115
                                          Val Ser Asp Val Ser
                                          1               5

gca ggc gta aat ggc gca caa gat cca agc aat caa gcg gtc aag cct 163
Ala Gly Val Asn Gly Ala Gln Asp Pro Ser Asn Gln Ala Val Lys Pro
                10                  15                  20

tcc aac tgg aac ctt ccg aac ttc ttg acc agc ttg cgt atc att gtc 211
Ser Asn Trp Asn Leu Pro Asn Phe Leu Thr Ser Leu Arg Ile Ile Val
            25                  30                  35

atc cct ttg ttt gcg tgg ctt acg ctt aaa ggt gag acg gaa aac aat 259
Ile Pro Leu Phe Ala Trp Leu Thr Leu Lys Gly Glu Thr Glu Asn Asn
        40                  45                  50

gct ttt gcc tgg tgg gcg ttg gtt gtt ttc att ttg ctc atg atc acc 307
Ala Phe Ala Trp Trp Ala Leu Val Val Phe Ile Leu Leu Met Ile Thr
    55                  60                  65

gac aag ctt gac ggc gat att gcg cga gca cgt ggc ctg gtc act gac 355
Asp Lys Leu Asp Gly Asp Ile Ala Arg Ala Arg Gly Leu Val Thr Asp
70                  75                  80                  85

ttt ggc aag atc gcg gat ccg att gcc gat aag gcg ttg atg acc aca 403
Phe Gly Lys Ile Ala Asp Pro Ile Ala Asp Lys Ala Leu Met Thr Thr
                90                  95                  100

gca ttt gtc tgt ttc aac atc atc ggc att ttg ccc tgg tgg gtc act 451
Ala Phe Val Cys Phe Asn Ile Ile Gly Ile Leu Pro Trp Trp Val Thr
            105                 110                 115

gcg ttg att gtg ctt cga gag ttc ggc att acc atc tgg cgt ttc ttc 499
Ala Leu Ile Val Leu Arg Glu Phe Gly Ile Thr Ile Trp Arg Phe Phe
        120                 125                 130

caa ctg cgc gct gga aat gtt gtg cct gca tca aag ggg ggc aag ctt 547
Gln Leu Arg Ala Gly Asn Val Val Pro Ala Ser Lys Gly Gly Lys Leu
    135                 140                 145

aag act gct ctg cag act gtt gcc gtt gct ctg tat ctg tgc cct ttc 595
Lys Thr Ala Leu Gln Thr Val Ala Val Ala Leu Tyr Leu Cys Pro Phe
150                 155                 160                 165

cca agt tgg atg gat att cca agc cag atc gtc atg tat gca 637
Pro Ser Trp Met Asp Ile Pro Ser Gln Ile Val Met Tyr Ala
                170                 175

<210> 108
<211> 179
<212> PRT
<213> Corynebacterium glutamicum

<400> 108
Val Ser Asp Val Ser Ala Gly Val Asn Gly Ala Gln Asp Pro Ser Asn
1               5                   10                  15

Gln Ala Val Lys Pro Ser Asn Trp Asn Leu Pro Asn Phe Leu Thr Ser
            20                  25                  30

Leu Arg Ile Ile Val Ile Pro Leu Phe Ala Trp Leu Thr Leu Lys Gly
        35                  40                  45

Glu Thr Glu Asn Asn Ala Phe Ala Trp Trp Ala Leu Val Val Phe Ile
50                  55                  60

Leu Leu Met Ile Thr Asp Lys Leu Asp Gly Asp Ile Ala Arg Ala Arg
65                  70                  75                  80

Gly Leu Val Thr Asp Phe Gly Lys Ile Ala Asp Pro Ile Ala Asp Lys
                85                  90                  95

Ala Leu Met Thr Thr Ala Phe Val Cys Phe Asn Ile Ile Gly Ile Leu
            100                 105                 110

Pro Trp Trp Val Thr Ala Leu Ile Val Leu Arg Glu Phe Gly Ile Thr
        115                 120                 125

Ile Trp Arg Phe Phe Gln Leu Arg Ala Gly Asn Val Val Pro Ala Ser
    130                 135                 140

Lys Gly Gly Lys Leu Lys Thr Ala Leu Gln Thr Val Ala Val Ala Leu
145                 150                 155                 160

Tyr Leu Cys Pro Phe Pro Ser Trp Met Asp Ile Pro Ser Gln Ile Val
                165                 170                 175

Met Tyr Ala

<210> 109
<211> 780
<212> DNA
<213> Corynebacterium glutamicum

<220>
<221> CDS
<222> (101)..(757)
<223> RXA02511

<400> 109
ccatgcttgc gcaagcatgg ggcaccattg atggggcacc aggcactgtc gacccaacgc 60

tgacttcagc gatccgtacc gcagcaccga aggagcactg atg ctg gga ctt cat   115
                                           Met Leu Gly Leu His
                                           1               5

gga cgt aag cct gcg cag gtt att gtt gag cct gtt gcc aaa ttg atg   163
Gly Arg Lys Pro Ala Gln Val Ile Val Glu Pro Val Ala Lys Leu Met
                10                  15                  20

atc aag ttg aag gtg acg cct aat cag ctc acc tta gtc agc gct ggc   211
Ile Lys Leu Lys Val Thr Pro Asn Gln Leu Thr Leu Val Ser Ala Gly
            25                  30                  35

ctc acc gtt ggg gtg gct ttg ctg ctg att cct acg ggg cat ttg att   259
Leu Thr Val Gly Val Ala Leu Leu Leu Ile Pro Thr Gly His Leu Ile
        40                  45                  50

tgg gcg gca gtt ttg acg ggc ctg ttt gcg gct ttc gac atg att gat   307
Trp Ala Ala Val Leu Thr Gly Leu Phe Ala Ala Phe Asp Met Ile Asp
    55                  60                  65

ggc acg gtt gct cgc atg caa ggt ggt ggc acc aaa ttt ggt gcc acc 355
Gly Thr Val Ala Arg Met Gln Gly Gly Gly Thr Lys Phe Gly Ala Thr
70                  75                  80                  85

ttg gat gcc acg tgt gac cgc atc act gat ggt gca cta ttt ggt gcg 403
Leu Asp Ala Thr Cys Asp Arg Ile Thr Asp Gly Ala Leu Phe Gly Ala
                90                  95                  100

att acc tgg tgg ctg gtg tat tcc tac gat gca cca cag gca ttg gtc 451
Ile Thr Trp Trp Leu Val Tyr Ser Tyr Asp Ala Pro Gln Ala Leu Val
            105                 110                 115

gct gcc tcc ttg gtt tgt ttg gtt gcc tcc cag gtg atc tct tac gtg 499
Ala Ala Ser Leu Val Cys Leu Val Ala Ser Gln Val Ile Ser Tyr Val
        120                 125                 130

aaa gcc agg gga gag gcc tcc gga ttc acc atg gac ggc ggt ctc gtg 547
Lys Ala Arg Gly Glu Ala Ser Gly Phe Thr Met Asp Gly Gly Leu Val
    135                 140                 145

gaa cgc cct gag cgt ctg att gtc agc ctt gtt ggt ttg ggg ctg acc 595
Glu Arg Pro Glu Arg Leu Ile Val Ser Leu Val Gly Leu Gly Leu Thr
150                 155                 160                 165

gga atg ggc gtt cca tat gcc atc gat gtg gca ctg tgg gcc ctt gca 643
Gly Met Gly Val Pro Tyr Ala Ile Asp Val Ala Leu Trp Ala Leu Ala
                170                 175                 180

gct ggc agt att tac act gtt gtg cag cgc ttg gtc atg gct gga aag 691
Ala Gly Ser Ile Tyr Thr Val Val Gln Arg Leu Val Met Ala Gly Lys
            185                 190                 195

tcc cca ttg gct aag gaa ttt acc aag gca cca gca ggt gcg aag gca 739
Ser Pro Leu Ala Lys Glu Phe Thr Lys Ala Pro Ala Gly Ala Lys Ala
        200                 205                 210

gat tac agc aac acc aaa taaaaattag ccgagggagc atc 780
Asp Tyr Ser Asn Thr Lys
    215

<210> 110
<211> 219
<212> PRT
<213> Corynebacterium glutamicum

<400> 110
Met Leu Gly Leu His Gly Arg Lys Pro Ala Gln Val Ile Val Glu Pro
1               5                   10                  15

Val Ala Lys Leu Met Ile Lys Leu Lys Val Thr Pro Asn Gln Leu Thr
            20                  25                  30

Leu Val Ser Ala Gly Leu Thr Val Gly Val Ala Leu Leu Leu Ile Pro
        35                  40                  45

Thr Gly His Leu Ile Trp Ala Ala Val Leu Thr Gly Leu Phe Ala Ala
    50                  55                  60

Phe Asp Met Ile Asp Gly Thr Val Ala Arg Met Gln Gly Gly Gly Thr
65                  70                  75                  80

Lys Phe Gly Ala Thr Leu Asp Ala Thr Cys Asp Arg Ile Thr Asp Gly
                85                  90                  95

Ala Leu Phe Gly Ala Ile Thr Trp Trp Leu Val Tyr Ser Tyr Asp Ala
            100                 105                 110

Pro Gln Ala Leu Val Ala Ala Ser Leu Val Cys Leu Val Ala Ser Gln
        115                 120                 125

Val Ile Ser Tyr Val Lys Ala Arg Gly Glu Ala Ser Gly Phe Thr Met
    130                 135                 140

Asp Gly Gly Leu Val Glu Arg Pro Glu Arg Leu Ile Val Ser Leu Val
145                 150                 155                 160

Gly Leu Gly Leu Thr Gly Met Gly Val Pro Tyr Ala Ile Asp Val Ala
                165                 170                 175

Leu Trp Ala Leu Ala Ala Gly Ser Ile Tyr Thr Val Val Gln Arg Leu
            180                 185                 190

Val Met Ala Gly Lys Ser Pro Leu Ala Lys Glu Phe Thr Lys Ala Pro
        195                 200                 205

Ala Gly Ala Lys Ala Asp Tyr Ser Asn Thr Lys
    210                 215

<210> 111
<211> 678
<212> DNA
<213> Corynebacterium glutamicum

<220>
<221> CDS
<222> (101)..(655)
<223> RXN02836

<400> 111
ccatcactcg gcttcaccgt ctacggtgct gcccgcagga cagagcgccc ccaaaagctc 60

gcttcagacg ggatccaccc cctcgagatg gacgtcaccg atg aca atc gat gaa   115
                                            Met Thr Ile Asp Glu
                                            1               5

ggc cgt cgc cag ttc gag gtc aat gta ttc ggc gcg atg gcc ctc acc   163
Gly Arg Arg Gln Phe Glu Val Asn Val Phe Gly Ala Met Ala Leu Thr
                10                  15                  20

cga ctc gtc ctg ccc cac atg cag aaa caa aag tgg ggg acg atc gtg   211
Arg Leu Val Leu Pro His Met Gln Lys Gln Lys Trp Gly Thr Ile Val
            25                  30                  35

aac atc aca tcg atg ggc ggg aag atc tac acg cct ctc ggc ggc tgg   259
Asn Ile Thr Ser Met Gly Gly Lys Ile Tyr Thr Pro Leu Gly Gly Trp
        40                  45                  50

tat cac ggc acc aag ttc gcc ctc gag gcc ctc tcg gac gcc ctc cgc   307
Tyr His Gly Thr Lys Phe Ala Leu Glu Ala Leu Ser Asp Ala Leu Arg
    55                  60                  65

ctg gag gtc gcc cca ttc ggc atc gac gtt gtt gtc atc gaa ccg ggc 355
Leu Glu Val Ala Pro Phe Gly Ile Asp Val Val Val Ile Glu Pro Gly
70                  75                  80                  85

ggc atc gcc acc gag tgg gga gga atc gct gcc gac aat ctc gac gca 403
Gly Ile Ala Thr Glu Trp Gly Gly Ile Ala Ala Asp Asn Leu Asp Ala
                90                  95                  100

gtg tcg aaa gac agc gca tac aag cgc cag gct gac gca gta tcg aag 451
Val Ser Lys Asp Ser Ala Tyr Lys Arg Gln Ala Asp Ala Val Ser Lys
            105                 110                 115

tcg ttg cga tct gag gcg aac agc aac cgc aac tca cca ccg tcg gtt 499
Ser Leu Arg Ser Glu Ala Asn Ser Asn Arg Asn Ser Pro Pro Ser Val
        120                 125                 130

gtc gcc gat gcg att gga aag gcc gtg acg gca cgt cac ccc aag acc 547
Val Ala Asp Ala Ile Gly Lys Ala Val Thr Ala Arg His Pro Lys Thr
    135                 140                 145

cgc tat gcc atc ggc ttc ggt gcc aaa ccg ctg att gcc tcg cgc aac 595
Arg Tyr Ala Ile Gly Phe Gly Ala Lys Pro Leu Ile Ala Ser Arg Asn
150                 155                 160                 165

atc ctc acc gat cgc cag ttc gac cca gtg atc act cga gcg act ggc 643
Ile Leu Thr Asp Arg Gln Phe Asp Pro Val Ile Thr Arg Ala Thr Gly
                170                 175                 180

gtc ccc cgc gac tgaccactct tctgcgcccg tca 678
Val Pro Arg Asp
            185

<210> 112
<211> 185
<212> PRT
<213> Corynebacterium glutamicum

<400> 112
Met Thr Ile Asp Glu Gly Arg Arg Gln Phe Glu Val Asn Val Phe Gly
1               5                   10                  15

Ala Met Ala Leu Thr Arg Leu Val Leu Pro His Met Gln Lys Gln Lys
            20                  25                  30

Trp Gly Thr Ile Val Asn Ile Thr Ser Met Gly Gly Lys Ile Tyr Thr
        35                  40                  45

Pro Leu Gly Gly Trp Tyr His Gly Thr Lys Phe Ala Leu Glu Ala Leu
    50                  55                  60

Ser Asp Ala Leu Arg Leu Glu Val Ala Pro Phe Gly Ile Asp Val Val
65                  70                  75                  80

Val Ile Glu Pro Gly Gly Ile Ala Thr Glu Trp Gly Gly Ile Ala Ala
                85                  90                  95

Asp Asn Leu Asp Ala Val Ser Lys Asp Ser Ala Tyr Lys Arg Gln Ala
            100                 105                 110

Asp Ala Val Ser Lys Ser Leu Arg Ser Glu Ala Asn Ser Asn Arg Asn
        115                 120                 125

Ser Pro Pro Ser Val Val Ala Asp Ala Ile Gly Lys Ala Val Thr Ala
    130                 135                 140

Arg His Pro Lys Thr Arg Tyr Ala Ile Gly Phe Gly Ala Lys Pro Leu
145                 150                 155                 160

Ile Ala Ser Arg Asn Ile Leu Thr Asp Arg Gln Phe Asp Pro Val Ile
                165                 170                 175

Thr Arg Ala Thr Gly Val Pro Arg Asp
            180                 185


<210> 113
<211> 406
<212> DNA
<213> Corynebacterium glutamicum

<220>
<221> CDS
<222> (101)..(406)
<223> FRXA02836

<400> 113
ccatcactcg gcttcaccgt ctacggtgct gcccgcagga cagagcgccc ccaaaagctc 60

gcttcagacg ggatccaccc cctcgagatg gacgtcaccg atg aca atc gat gaa   115
                                            Met Thr Ile Asp Glu
                                            1               5

ggc cgt cgc cag ttc gag gtc aat gta ttc ggc gcg atg gcc ctc acc   163
Gly Arg Arg Gln Phe Glu Val Asn Val Phe Gly Ala Met Ala Leu Thr
                10                  15                  20

cga ctc gtc ctg ccc cac atg cag aaa caa aag tgg ggg acg atc gtg   211
Arg Leu Val Leu Pro His Met Gln Lys Gln Lys Trp Gly Thr Ile Val
            25                  30                  35

aac atc aca tcg atg ggc ggg aag atc tac acg cct ctc ggc ggc tgg   259
Asn Ile Thr Ser Met Gly Gly Lys Ile Tyr Thr Pro Leu Gly Gly Trp
        40                  45                  50

tat cac ggc acc aag ttc gcc ctc gag gcc ctc tcg gac gcc ctc cgc   307
Tyr His Gly Thr Lys Phe Ala Leu Glu Ala Leu Ser Asp Ala Leu Arg
    55                  60                  65

ctg gag gtc gcc cca ttc ggc atc gac gtt gtt gtc atc gaa ccg ggc   355
Leu Glu Val Ala Pro Phe Gly Ile Asp Val Val Val Ile Glu Pro Gly
70                  75                  80                  85

ggc atc gcc acc gag tgg gga gga atc gct gcc gac aat ctc gac gca   403
Gly Ile Ala Thr Glu Trp Gly Gly Ile Ala Ala Asp Asn Leu Asp Ala
                90                  95                  100

gtg                                                               406
Val

<210> 114
<211> 102
<212> PRT
<213> Corynebacterium glutamicum

<400> 114
Met Thr Ile Asp Glu Gly Arg Arg Gln Phe Glu Val Asn Val Phe Gly
1               5                   10                  15

Ala Met Ala Leu Thr Arg Leu Val Leu Pro His Met Gln Lys Gln Lys
            20                  25                  30

Trp Gly Thr Ile Val Asn Ile Thr Ser Met Gly Gly Lys Ile Tyr Thr
        35                  40                  45

Pro Leu Gly Gly Trp Tyr His Gly Thr Lys Phe Ala Leu Glu Ala Leu
    50                  55                  60

Ser Asp Ala Leu Arg Leu Glu Val Ala Pro Phe Gly Ile Asp Val Val
65                  70                  75                  80

Val Ile Glu Pro Gly Gly Ile Ala Thr Glu Trp Gly Gly Ile Ala Ala
                85                  90                  95

Asp Asn Leu Asp Ala Val
            100


<210> 115
<211> 1227
<212> DNA
<213> Corynebacterium glutamicum

<220>
<221> CDS
<222> (101)..(1204)
<223> RXA02578

<400> 115
ggcaaaaatg aggaacagca cgcccgcaat aatgaggacc gttgcagatc gcttcataaa 60

aacagcccac acctttccgc taaactcgca tgttgaaata atg tct acc caa tca   115
                                           Met Ser Thr Gln Ser
                                           1               5

tat gca ccc atc cgc cat cgc gga ttc atc agc tca ctc gag gga cta   163
Tyr Ala Pro Ile Arg His Arg Gly Phe Ile Ser Ser Leu Glu Gly Leu
                10                  15                  20

cgc gca atc gcc tcc ctg gga gtc ttg gcg acc cac gtt gca ttc caa   211
Arg Ala Ile Ala Ser Leu Gly Val Leu Ala Thr His Val Ala Phe Gln
            25                  30                  35

acc tcc gtc gac ccc gcc agc aac atc ggt gca gta ctc gcg cgt ttc   259
Thr Ser Val Asp Pro Ala Ser Asn Ile Gly Ala Val Leu Ala Arg Phe
        40                  45                  50

gac ttt ttc gtc gcc gtc ttc ttc gcc ctc tcc gcc ttc gtt ctt tgg   307
Asp Phe Phe Val Ala Val Phe Phe Ala Leu Ser Ala Phe Val Leu Trp
    55                  60                  65

cga cgc cgc gcc ggg caa cca gtg gga ctg tac tac ctc aaa cgc cta 355
Arg Arg Arg Ala Gly Gln Pro Val Gly Leu Tyr Tyr Leu Lys Arg Leu
70                  75                  80                  85

gcc cgc atc atg ccc gca tac tgg gca acg gtc att gca gtc ctg ctg 403
Ala Arg Ile Met Pro Ala Tyr Trp Ala Thr Val Ile Ala Val Leu Leu
                90                  95                  100

ttt att ccc acc ggc ccc tgg tta gcc aac ctg acg atg acc caa atc 451
Phe Ile Pro Thr Gly Pro Trp Leu Ala Asn Leu Thr Met Thr Gln Ile
            105                 110                 115

tac tgg cca gac ggg ctc atg aca ggc ctc acc cac ctt tgg tcc ctg 499
Tyr Trp Pro Asp Gly Leu Met Thr Gly Leu Thr His Leu Trp Ser Leu
        120                 125                 130

tgc gtg gaa gtg gcg ttt tac ctg gtg atg ccg ctt ctc gcg tgg gtg 547
Cys Val Glu Val Ala Phe Tyr Leu Val Met Pro Leu Leu Ala Trp Val
    135                 140                 145

ttg gat agg ttt ggt cgg ccg gtg cgc atc ctg ttg att gtt ggt ggg 595
Leu Asp Arg Phe Gly Arg Pro Val Arg Ile Leu Leu Ile Val Gly Gly
150                 155                 160                 165

gca gtg ttg agt ctg gcg tgg ccg tgg att ccc ctt gtg gag cat gcg 643
Ala Val Leu Ser Leu Ala Trp Pro Trp Ile Pro Leu Val Glu His Ala
                170                 175                 180

ttg gac gag ggg tgg gcg aac atg cag atc tgg cca ccc gct tac gct 691
Leu Asp Glu Gly Trp Ala Asn Met Gln Ile Trp Pro Pro Ala Tyr Ala
            185                 190                 195

tgc tgg ttt gca gtc ggc atg atc gcc gca gaa att gaa gga gtt cga 739
Cys Trp Phe Ala Val Gly Met Ile Ala Ala Glu Ile Glu Gly Val Arg
        200                 205                 210

ttc cca cgg gtt ccg agc ttt gtg tgg gtg ggt tta gct tta gtg gtc 787
Phe Pro Arg Val Pro Ser Phe Val Trp Val Gly Leu Ala Leu Val Val
    215                 220                 225

gct tgg atc gcg ggc caa gaa tgg ttc gga cca cta ggt tta gtg cac 835
Ala Trp Ile Ala Gly Gln Glu Trp Phe Gly Pro Leu Gly Leu Val His
230                 235                 240                 245

ccc agc ccc tgg gaa ttc aac tta aga gtc ctc gcg ggc aca ctt ttc 883
Pro Ser Pro Trp Glu Phe Asn Leu Arg Val Leu Ala Gly Thr Leu Phe
                250                 255                 260

gct gta ttt ctg gtg gtt ccc tac gcg ctg ggt acg ccc tct cgg ctt 931
Ala Val Phe Leu Val Val Pro Tyr Ala Leu Gly Thr Pro Ser Arg Leu
            265                 270                 275

ctt gat tcc agt tgg atg aaa acg ctc ggc acc tgg tcg tat tcc atc 979
Leu Asp Ser Ser Trp Met Lys Thr Leu Gly Thr Trp Ser Tyr Ser Ile
        280                 285                 290

ttc ctc tgg cac ctt ccc gtg ctg acg att gtg ttc cca ctg ctc ggg 1027
Phe Leu Trp His Leu Pro Val Leu Thr Ile Val Phe Pro Leu Leu Gly
    295                 300                 305

ttg cct tta ttt agt gga aat ttc ctg ttg gtg ttc atc gtg acg gtc 1075
Leu Pro Leu Phe Ser Gly Asn Phe Leu Leu Val Phe Ile Val Thr Val
310                 315                 320                 325

ttg ttg acg atc cca gtt gcc gcc atc agc tac acc ttc atc gaa gag 1123
Leu Leu Thr Ile Pro Val Ala Ala Ile Ser Tyr Thr Phe Ile Glu Glu
                330                 335                 340

ccc atc agc ggt gga ccc ggc gcg cca ttc agg ctg ggg gtc gtt agg 1171
Pro Ile Ser Gly Gly Pro Gly Ala Pro Phe Arg Leu Gly Val Val Arg
            345                 350                 355

att cac cat ttt tct ggg ggt agg tct gga aaa tgatgaattg gcaccacgtc 1224
Ile His His Phe Ser Gly Gly Arg Ser Gly Lys
        360                 365

aag 1227

<210> 116
<211> 368
<212> PRT
<213> Corynebacterium glutamicum

<400> 116
Met Ser Thr Gln Ser Tyr Ala Pro Ile Arg His Arg Gly Phe Ile Ser
1               5                   10                  15

Ser Leu Glu Gly Leu Arg Ala Ile Ala Ser Leu Gly Val Leu Ala Thr
            20                  25                  30

His Val Ala Phe Gln Thr Ser Val Asp Pro Ala Ser Asn Ile Gly Ala
        35                  40                  45

Val Leu Ala Arg Phe Asp Phe Phe Val Ala Val Phe Phe Ala Leu Ser
    50                  55                  60

Ala Phe Val Leu Trp Arg Arg Arg Ala Gly Gln Pro Val Gly Leu Tyr
65                  70                  75                  80

Tyr Leu Lys Arg Leu Ala Arg Ile Met Pro Ala Tyr Trp Ala Thr Val
                85                  90                  95

Ile Ala Val Leu Leu Phe Ile Pro Thr Gly Pro Trp Leu Ala Asn Leu
            100                 105                 110

Thr Met Thr Gln Ile Tyr Trp Pro Asp Gly Leu Met Thr Gly Leu Thr
        115                 120                 125

His Leu Trp Ser Leu Cys Val Glu Val Ala Phe Tyr Leu Val Met Pro
    130                 135                 140

Leu Leu Ala Trp Val Leu Asp Arg Phe Gly Arg Pro Val Arg Ile Leu
145                 150                 155                 160

Leu Ile Val Gly Gly Ala Val Leu Ser Leu Ala Trp Pro Trp Ile Pro
                165                 170                 175

Leu Val Glu His Ala Leu Asp Glu Gly Trp Ala Asn Met Gln Ile Trp
            180                 185                 190

Pro Pro Ala Tyr Ala Cys Trp Phe Ala Val Gly Met Ile Ala Ala Glu
        195                 200                 205

Ile Glu Gly Val Arg Phe Pro Arg Val Pro Ser Phe Val Trp Val Gly
    210                 215                 220

Leu Ala Leu Val Val Ala Trp Ile Ala Gly Gln Glu Trp Phe Gly Pro
225                 230                 235                 240

Leu Gly Leu Val His Pro Ser Pro Trp Glu Phe Asn Leu Arg Val Leu
                245                 250                 255

Ala Gly Thr Leu Phe Ala Val Phe Leu Val Val Pro Tyr Ala Leu Gly
            260                 265                 270

Thr Pro Ser Arg Leu Leu Asp Ser Ser Trp Met Lys Thr Leu Gly Thr
        275                 280                 285

Trp Ser Tyr Ser Ile Phe Leu Trp His Leu Pro Val Leu Thr Ile Val
    290                 295                 300

Phe Pro Leu Leu Gly Leu Pro Leu Phe Ser Gly Asn Phe Leu Leu Val
305                 310                 315                 320

Phe Ile Val Thr Val Leu Leu Thr Ile Pro Val Ala Ala Ile Ser Tyr
                325                 330                 335

Thr Phe Ile Glu Glu Pro Ile Ser Gly Gly Pro Gly Ala Pro Phe Arg
            340                 345                 350

Leu Gly Val Val Arg Ile His His Phe Ser Gly Gly Arg Ser Gly Lys
        355                 360                 365

<210> 117
<211> 924
<212> DNA
<213> Corynebacterium glutamicum

<220>
<221> CDS
<222> (101)..(901)
<223> RXA02150

<400> 117
gtgtttttcg gtgggctgcg atgacgcatg tccaccaaaa gagccacccc ttaaagaaat 60

taaaaagtgg ttttggtagc ttcgcagcaa aatacacatc gtg ggt aac gta ttc 115
Val Gly Asn Val Phe
1               5

tta gaa gtt cct aca gca gta aag cgc gaa gaa ggg gta aac cca aac 163
Leu Glu Val Pro Thr Ala Val Lys Arg Glu Glu Gly Val Asn Pro Asn
                10                  15                  20

atc atg aaa aac aac tgg tat cgg ctt ttc aag tat gtg cta att ggc 211
Ile Met Lys Asn Asn Trp Tyr Arg Leu Phe Lys Tyr Val Leu Ile Gly
            25                  30                  35

ccg ttt ttg cgt gtg tac aac cgc ccg gag atc gaa ggc aaa gaa aac 259
Pro Phe Leu Arg Val Tyr Asn Arg Pro Glu Ile Glu Gly Lys Glu Asn
        40                  45                  50

atc cct gca gaa ggt gcc gcg atc atg gcg tcc aac cac gaa gca gtg 307
Ile Pro Ala Glu Gly Ala Ala Ile Met Ala Ser Asn His Glu Ala Val
    55                  60                  65

atg gat tcc ttt tat ttt ccc ctg ctg tgc cca cgg cag ctg acc ttc 355
Met Asp Ser Phe Tyr Phe Pro Leu Leu Cys Pro Arg Gln Leu Thr Phe
70                  75                  80                  85

cca gcg aag gcc gaa tac ttc aca tca cca ggt att aaa ggc aag atg 403
Pro Ala Lys Ala Glu Tyr Phe Thr Ser Pro Gly Ile Lys Gly Lys Met
                90                  95                  100

cag aag tgg ttt ttt act tct gtg ggg caa gta ccc ctg gac cgc acc 451
Gln Lys Trp Phe Phe Thr Ser Val Gly Gln Val Pro Leu Asp Arg Thr
            105                 110                 115

gca gat aat gcc atg gat tct ttg atg aat acc gcc aaa atg gtg ctg 499
Ala Asp Asn Ala Met Asp Ser Leu Met Asn Thr Ala Lys Met Val Leu
        120                 125                 130

gat cgg gga gac ctc ttc ggt att tac cct gaa gga tct cgt tcg ccc 547
Asp Arg Gly Asp Leu Phe Gly Ile Tyr Pro Glu Gly Ser Arg Ser Pro
    135                 140                 145

gat ggt cgc atc tac aag ggc aaa acc gga atg gcc tat gtt gcg atg 595
Asp Gly Arg Ile Tyr Lys Gly Lys Thr Gly Met Ala Tyr Val Ala Met
150                 155                 160                 165

gaa act ggt acg aca gtt atc ccc gtt gcc atg att ggc agc cgg gac 643
Glu Thr Gly Thr Thr Val Ile Pro Val Ala Met Ile Gly Ser Arg Asp
                170                 175                 180

gcg aac cct atc gga agt tgg ttt ccg aaa ccc gca aaa gtc agg atc 691
Ala Asn Pro Ile Gly Ser Trp Phe Pro Lys Pro Ala Lys Val Arg Ile
            185                 190                 195

aag gta gga agc cca att gat ccc ctc gca ttc gtc aaa gaa cat ggg 739
Lys Val Gly Ser Pro Ile Asp Pro Leu Ala Phe Val Lys Glu His Gly
        200                 205                 210

ttg aag cct gga acc tac gaa gca gcg cgc aag ctg aca gat cac gtt 787
Leu Lys Pro Gly Thr Tyr Glu Ala Ala Arg Lys Leu Thr Asp His Val
    215                 220                 225

atg ttc att ctt gct gat ctc act ggt cag ccg tat gtt gat gcg tac 835
Met Phe Ile Leu Ala Asp Leu Thr Gly Gln Pro Tyr Val Asp Ala Tyr
230                 235                 240                 245

tct aaa gat gtg aaa aac gct ctg gag gaa gga aaa gga tac ccg gag 883
Ser Lys Asp Val Lys Asn Ala Leu Glu Glu Gly Lys Gly Tyr Pro Glu
                250                 255                 260

ggc aca gct cct tca cag taatcgggtc ttttctgtta aaa 924
Gly Thr Ala Pro Ser Gln
            265

<210> 118
<211> 267
<212> PRT
<213> Corynebacterium glutamicum

<400> 118
Val Gly Asn Val Phe Leu Glu Val Pro Thr Ala Val Lys Arg Glu Glu
1               5                   10                  15

Gly Val Asn Pro Asn Ile Met Lys Asn Asn Trp Tyr Arg Leu Phe Lys
            20                  25                  30

Tyr Val Leu Ile Gly Pro Phe Leu Arg Val Tyr Asn Arg Pro Glu Ile
        35                  40                  45

Glu Gly Lys Glu Asn Ile Pro Ala Glu Gly Ala Ala Ile Met Ala Ser
    50                  55                  60

Asn His Glu Ala Val Met Asp Ser Phe Tyr Phe Pro Leu Leu Cys Pro
65                  70                  75                  80

Arg Gln Leu Thr Phe Pro Ala Lys Ala Glu Tyr Phe Thr Ser Pro Gly
                85                  90                  95

Ile Lys Gly Lys Met Gln Lys Trp Phe Phe Thr Ser Val Gly Gln Val
            100                 105                 110

Pro Leu Asp Arg Thr Ala Asp Asn Ala Met Asp Ser Leu Met Asn Thr
        115                 120                 125

Ala Lys Met Val Leu Asp Arg Gly Asp Leu Phe Gly Ile Tyr Pro Glu
    130                 135                 140

Gly Ser Arg Ser Pro Asp Gly Arg Ile Tyr Lys Gly Lys Thr Gly Met
145                 150                 155                 160

Ala Tyr Val Ala Met Glu Thr Gly Thr Thr Val Ile Pro Val Ala Met
                165                 170                 175

Ile Gly Ser Arg Asp Ala Asn Pro Ile Gly Ser Trp Phe Pro Lys Pro
            180                 185                 190

Ala Lys Val Arg Ile Lys Val Gly Ser Pro Ile Asp Pro Leu Ala Phe
        195                 200                 205

Val Lys Glu His Gly Leu Lys Pro Gly Thr Tyr Glu Ala Ala Arg Lys
    210                 215                 220

Leu Thr Asp His Val Met Phe Ile Leu Ala Asp Leu Thr Gly Gln Pro
225                 230                 235                 240

Tyr Val Asp Ala Tyr Ser Lys Asp Val Lys Asn Ala Leu Glu Glu Gly
                245                 250                 255

Lys Gly Tyr Pro Glu Gly Thr Ala Pro Ser Gln
            260                 265


<210> 119
<211> 504

<212> DNA
<213> Corynebacterium glutamicum

<220>
<221> CDS
<222> (101)..(481)
<223> RXA00607

<400> 119
cactgccagc aacgccaacg gttgaacccg aaccagaagg cgatgaagac tggcccgaac 60

ccatcaaccc cgcaggcgat aacaaagagg aggcaaaccg atg att ctc gca ctg 115
Met Ile Leu Ala Leu
1               5

aca gtc gcg ata ctt ttc ggt gga ggt gtc tac ctc att cag caa cgc 163
Thr Val Ala Ile Leu Phe Gly Gly Gly Val Tyr Leu Ile Gln Gln Arg
                10                  15                  20

gga atg gtg cgc atc gtc ttc ggc atg tca ctg atc ggc cac gca gcg 211
Gly Met Val Arg Ile Val Phe Gly Met Ser Leu Ile Gly His Ala Ala
            25                  30                  35

aac ctg acc atc ctg tac gcc ggt gtg ccc acg tgg cgc ggc gaa gcc 259
Asn Leu Thr Ile Leu Tyr Ala Gly Val Pro Thr Trp Arg Gly Glu Ala
        40                  45                  50

ttc ccc gac agg acc ccg ctt acc gac gcc gcc gat cca ctc ccc cag 307
Phe Pro Asp Arg Thr Pro Leu Thr Asp Ala Ala Asp Pro Leu Pro Gln
    55                  60                  65

gcc ttc gtc ctc acc gcc atc gtc atc gcg atg gcc acc aca acc atc 355
Ala Phe Val Leu Thr Ala Ile Val Ile Ala Met Ala Thr Thr Thr Ile
70                  75                  80                  85

atg ttg gcc ttg gca gca ctg gga cgc agc gac gac acc cgg tcc atc 403
Met Leu Ala Leu Ala Ala Leu Gly Arg Ser Asp Asp Thr Arg Ser Ile
                90                  95                  100

gaa cca gat gac gat caa tcg cct ttg act act agc gct cgt tca gtc 451
Glu Pro Asp Asp Asp Gln Ser Pro Leu Thr Thr Ser Ala Arg Ser Val
            105                 110                 115

acc aac cca aca gat cag gag gat aaa gct taaatggcca tggatgttct 501
Thr Asn Pro Thr Asp Gln Glu Asp Lys Ala
        120                 125

cct 504

<210> 120
<211> 127
<212> PRT
<213> Corynebacterium glutamicum

<400> 120
Met Ile Leu Ala Leu Thr Val Ala Ile Leu Phe Gly Gly Gly Val Tyr
1               5                   10                  15

Leu Ile Gln Gln Arg Gly Met Val Arg Ile Val Phe Gly Met Ser Leu
            20                  25                  30

Ile Gly His Ala Ala Asn Leu Thr Ile Leu Tyr Ala Gly Val Pro Thr
        35                  40                  45

Trp Arg Gly Glu Ala Phe Pro Asp Arg Thr Pro Leu Thr Asp Ala Ala
    50                  55                  60

Asp Pro Leu Pro Gln Ala Phe Val Leu Thr Ala Ile Val Ile Ala Met
65                  70                  75                  80

Ala Thr Thr Thr Ile Met Leu Ala Leu Ala Ala Leu Gly Arg Ser Asp
                85                  90                  95

Asp Thr Arg Ser Ile Glu Pro Asp Asp Asp Gln Ser Pro Leu Thr Thr
            100                 105                 110

Ser Ala Arg Ser Val Thr Asn Pro Thr Asp Gln Glu Asp Lys Ala
        115                 120                 125


<210> 121
<211> 1119
<212> DNA
<213> Corynebacterium glutamicum

<220>
<221> CDS
<222> (101)..(1096)
<223> RXA02397

<400> 121
gtgtgaataa aacttgtttc ctggtcattt cccctactga actgcgctta tgcctatgct 60

tagaaaccag agacaagctt aagaaggaca ggggccggcc atg aat gat ttc gaa   115
                                            Met Asn Asp Phe Glu
                                            1               5

aca acc atc gat cgg atc tct aaa gaa caa gat ccc gca gcc cga agc   163
Thr Thr Ile Asp Arg Ile Ser Lys Glu Gln Asp Pro Ala Ala Arg Ser
                10                  15                  20

cgc gtg gaa cag ttc att gtg gaa aca gta cgt gca cta ccc aac ttg   211
Arg Val Glu Gln Phe Ile Val Glu Thr Val Arg Ala Leu Pro Asn Leu
            25                  30                  35

acc acc aaa caa ggt gca tcg ttg gct atc caa ctt ctt gat gcg gta   259
Thr Thr Lys Gln Gly Ala Ser Leu Ala Ile Gln Leu Leu Asp Ala Val
        40                  45                  50

cag ctc gcg gat gcg gcg gga acc aag ggg ggt gcg tcg aca agc aat   307
Gln Leu Ala Asp Ala Ala Gly Thr Lys Gly Gly Ala Ser Thr Ser Asn
    55                  60                  65

gct tca tcg ctg cct gac acc ttt gac gcg ctg acc agc ctg att ggc   355
Ala Ser Ser Leu Pro Asp Thr Phe Asp Ala Leu Thr Ser Leu Ile Gly
70                  75                  80                  85

aag ctc gat gtg cgc agc gat tct gaa tgg cgc tcg ttt ggg ttc cag   403
Lys Leu Asp Val Arg Ser Asp Ser Glu Trp Arg Ser Phe Gly Phe Gln
                90                  95                  100

cct tct gaa act gcg cac ccg cta atg atc gct atc cct gag att gag 451
Pro Ser Glu Thr Ala His Pro Leu Met Ile Ala Ile Pro Glu Ile Glu
            105                 110                 115

att ttt tat cag cac acc gat gtg gag ccg gga agc gat gac gcc gtg 499
Ile Phe Tyr Gln His Thr Asp Val Glu Pro Gly Ser Asp Asp Ala Val
        120                 125                 130

gcg ccg gac ttt cag gaa aat cag gat atg tgg cgc agg cgt ctc gga 547
Ala Pro Asp Phe Gln Glu Asn Gln Asp Met Trp Arg Arg Arg Leu Gly
    135                 140                 145

tct gtc acc gaa cca aac ctt ata tat aaa gag ttt tcc gga ccc ggc 595
Ser Val Thr Glu Pro Asn Leu Ile Tyr Lys Glu Phe Ser Gly Pro Gly
150                 155                 160                 165

aaa gcg cag cgt gcc gta gaa atg ctg ggc aat ctg tgg aag atc ggc 643
Lys Ala Gln Arg Ala Val Glu Met Leu Gly Asn Leu Trp Lys Ile Gly
                170                 175                 180

gtg gtg gtg agt agg aat acg gaa agt cgc ctt ggg tta act cgt gtg 691
Val Val Val Ser Arg Asn Thr Glu Ser Arg Leu Gly Leu Thr Arg Val
            185                 190                 195

gaa tac acc ccc acc cct ggc gaa gta ccc gtg ccg ctg atg tcg gag 739
Glu Tyr Thr Pro Thr Pro Gly Glu Val Pro Val Pro Leu Met Ser Glu
        200                 205                 210

aag aac tgt tgg tac agc att cgg gtg tct gaa acc att ggt gag aac 787
Lys Asn Cys Trp Tyr Ser Ile Arg Val Ser Glu Thr Ile Gly Glu Asn
    215                 220                 225

cag gtt ccg gaa att gtg cgc tgc tta ggc gag att ttc tgt ggc tat 835
Gln Val Pro Glu Ile Val Arg Cys Leu Gly Glu Ile Phe Cys Gly Tyr
230                 235                 240                 245

ctt ccc cag atg tgg ctc aaa gaa cca gta aag gcc ggc aag ttg cga 883
Leu Pro Gln Met Trp Leu Lys Glu Pro Val Lys Ala Gly Lys Leu Arg
                250                 255                 260

atc caa gaa tcg gaa gca gca gcg tat atc gcg atg gcg cga cta gat 931
Ile Gln Glu Ser Glu Ala Ala Ala Tyr Ile Ala Met Ala Arg Leu Asp
            265                 270                 275

ctt tcc cca cgc acc ggc aac acc act tgg acc aac agc tat att tcc 979
Leu Ser Pro Arg Thr Gly Asn Thr Thr Trp Thr Asn Ser Tyr Ile Ser
        280                 285                 290

acg cgt cct ctc tcc ccc gct ttt agg tgg gac gtg gtg ctg gag gct 1027
Thr Arg Pro Leu Ser Pro Ala Phe Arg Trp Asp Val Val Leu Glu Ala
    295                 300                 305

tcc cac caa ttg gag aac ctg ctg cgt gga gac aca ggg ccg gtc act 1075
Ser His Gln Leu Glu Asn Leu Leu Arg Gly Asp Thr Gly Pro Val Thr
310                 315                 320                 325

gcc aca caa tcg gca gct ggt taaccaaaag ttcacgtgga gat 1119
Ala Thr Gln Ser Ala Ala Gly
                330

<210> 122
<211> 332
<212> PRT
<213> Corynebacterium glutamicum

<400> 122
Met Asn Asp Phe Glu Thr Thr Ile Asp Arg Ile Ser Lys Glu Gln Asp
1               5                   10                  15

Pro Ala Ala Arg Ser Arg Val Glu Gln Phe Ile Val Glu Thr Val Arg
            20                  25                  30

Ala Leu Pro Asn Leu Thr Thr Lys Gln Gly Ala Ser Leu Ala Ile Gln
        35                  40                  45

Leu Leu Asp Ala Val Gln Leu Ala Asp Ala Ala Gly Thr Lys Gly Gly
    50                  55                  60

Ala Ser Thr Ser Asn Ala Ser Ser Leu Pro Asp Thr Phe Asp Ala Leu
65                  70                  75                  80

Thr Ser Leu Ile Gly Lys Leu Asp Val Arg Ser Asp Ser Glu Trp Arg
                85                  90                  95

Ser Phe Gly Phe Gln Pro Ser Glu Thr Ala His Pro Leu Met Ile Ala
            100                 105                 110

Ile Pro Glu Ile Glu Ile Phe Tyr Gln His Thr Asp Val Glu Pro Gly
        115                 120                 125

Ser Asp Asp Ala Val Ala Pro Asp Phe Gln Glu Asn Gln Asp Met Trp
    130                 135                 140

Arg Arg Arg Leu Gly Ser Val Thr Glu Pro Asn Leu Ile Tyr Lys Glu
145                 150                 155                 160

Phe Ser Gly Pro Gly Lys Ala Gln Arg Ala Val Glu Met Leu Gly Asn
                165                 170                 175

Leu Trp Lys Ile Gly Val Val Val Ser Arg Asn Thr Glu Ser Arg Leu
            180                 185                 190

Gly Leu Thr Arg Val Glu Tyr Thr Pro Thr Pro Gly Glu Val Pro Val
        195                 200                 205

Pro Leu Met Ser Glu Lys Asn Cys Trp Tyr Ser Ile Arg Val Ser Glu
    210                 215                 220

Thr Ile Gly Glu Asn Gln Val Pro Glu Ile Val Arg Cys Leu Gly Glu
225                 230                 235                 240

Ile Phe Cys Gly Tyr Leu Pro Gln Met Trp Leu Lys Glu Pro Val Lys
                245                 250                 255

Ala Gly Lys Leu Arg Ile Gln Glu Ser Glu Ala Ala Ala Tyr Ile Ala
            260                 265                 270

Met Ala Arg Leu Asp Leu Ser Pro Arg Thr Gly Asn Thr Thr Trp Thr
        275                 280                 285

Asn Ser Tyr Ile Ser Thr Arg Pro Leu Ser Pro Ala Phe Arg Trp Asp

290                 295                 300

Val Val Leu Glu Ala Ser His Gln Leu Glu Asn Leu Leu Arg Gly Asp
305                 310                 315                 320

Thr Gly Pro Val Thr Ala Thr Gln Ser Ala Ala Gly
                325                 330

<210> 123
<211> 1462
<212> DNA
<213> Corynebacterium glutamicum

<220>
<221> CDS
<222> (101)..(1462)
<223> RXN03110

<400> 123
aatgagtgaa cctgcgcttg aactataccc cctggggtat tactgtagat tctcaatacc 60

ccgaggggta tctagttttt acgttgagaa ggagagctca gtg ctt att gaa cgc   115
                                           Val Leu Ile Glu Arg
                                           1               5

atc tac gac gaa gac ctt gcg cag gcc agc tat ttc att ggc tgc caa   163
Ile Tyr Asp Glu Asp Leu Ala Gln Ala Ser Tyr Phe Ile Gly Cys Gln
                10                  15                  20

gcc cac aac acc gca gtc gtg gtt gat ccg cgt cgc gat att gcc gtc   211
Ala His Asn Thr Ala Val Val Val Asp Pro Arg Arg Asp Ile Ala Val
            25                  30                  35

tat ctg gac atg gcc aag aaa aac gga atg gag att gtt gga gtt acc   259
Tyr Leu Asp Met Ala Lys Lys Asn Gly Met Glu Ile Val Gly Val Thr
        40                  45                  50

gaa acc cat atc cat gcg gac tat ttg tca gga acc cgt gag tta gct   307
Glu Thr His Ile His Ala Asp Tyr Leu Ser Gly Thr Arg Glu Leu Ala
    55                  60                  65

gct gca act aat gcc acc atg tac gtc tca ggg gag ggc ggc gcc gat   355
Ala Ala Thr Asn Ala Thr Met Tyr Val Ser Gly Glu Gly Gly Ala Asp
70                  75                  80                  85

tgg cag tat gaa ttc gac gcc gag cga ata tgc gac ggc agc gag att   403
Trp Gln Tyr Glu Phe Asp Ala Glu Arg Ile Cys Asp Gly Ser Glu Ile
                90                  95                  100

cgc ctg gga aat ctg gtg ctc aca gct gtt cac acc cca ggc cat acc   451
Arg Leu Gly Asn Leu Val Leu Thr Ala Val His Thr Pro Gly His Thr
            105                 110                 115

ccg gaa cac tta tcg ttc ctg ctg aag gac ggc gcg ttc gca gat gag   499
Pro Glu His Leu Ser Phe Leu Leu Lys Asp Gly Ala Phe Ala Asp Glu
        120                 125                 130

cca gga ttc atg ctc act ggc gat ttc gtt ttc gcg ggt gat ctt ggc   547
Pro Gly Phe Met Leu Thr Gly Asp Phe Val Phe Ala Gly Asp Leu Gly
    135                 140                 145

cga cca gat ttg ctc gat gaa gca gct ggg gga gtg gac act cgt ttt 595
Arg Pro Asp Leu Leu Asp Glu Ala Ala Gly Gly Val Asp Thr Arg Phe
150                 155                 160                 165

gag ggg gct cgc caa atg ttc aag agc ttg aag gaa aaa ttc ctg aca 643
Glu Gly Ala Arg Gln Met Phe Lys Ser Leu Lys Glu Lys Phe Leu Thr
                170                 175                 180

ttg cct gat cac atc cag atc ttc cct ggt cat ggt tcc ggt tcc gcg 691
Leu Pro Asp His Ile Gln Ile Phe Pro Gly His Gly Ser Gly Ser Ala
            185                 190                 195

tgt ggc aaa gcc ttg ggt tcg gtt cct tca aca aca ctt gga tat gaa 739
Cys Gly Lys Ala Leu Gly Ser Val Pro Ser Thr Thr Leu Gly Tyr Glu
        200                 205                 210

cgt caa ttt gcg tgg tgg gga aag tat ctg gag gca gat gat gaa caa 787
Arg Gln Phe Ala Trp Trp Gly Lys Tyr Leu Glu Ala Asp Asp Glu Gln
    215                 220                 225

gga ttc att gat gag ctt ctg gaa ggc caa cct gat gca cct gca tac 835
Gly Phe Ile Asp Glu Leu Leu Glu Gly Gln Pro Asp Ala Pro Ala Tyr
230                 235                 240                 245

ttc ggc agg atg aag agg caa aat agg caa ggg ccc gca att atg ggc 883
Phe Gly Arg Met Lys Arg Gln Asn Arg Gln Gly Pro Ala Ile Met Gly
                250                 255                 260

gct cgc gag ctg ttg cca cag ctg gaa gct tct gat ctg cac gac gtc 931
Ala Arg Glu Leu Leu Pro Gln Leu Glu Ala Ser Asp Leu His Asp Val
            265                 270                 275

att gtt gtt gat acc cgc tca gcc gat gaa gtt cac cag ggc act gta 979
Ile Val Val Asp Thr Arg Ser Ala Asp Glu Val His Gln Gly Thr Val
        280                 285                 290

gct ggt gca gtg aat att cct gcg ggc aat tcg atg gcg aaa ttt ggc 1027
Ala Gly Ala Val Asn Ile Pro Ala Gly Asn Ser Met Ala Lys Phe Gly
    295                 300                 305

tcg tgg acc gtt gat ccc gag aag gat tcc cga gct ttg gtt ctg ctc 1075
Ser Trp Thr Val Asp Pro Glu Lys Asp Ser Arg Ala Leu Val Leu Leu
310                 315                 320                 325

gcg gca agc caa att ggt gcc atg gag atg tgg gac cac atg gtt cgc 1123
Ala Ala Ser Gln Ile Gly Ala Met Glu Met Trp Asp His Met Val Arg
                330                 335                 340

gtg gga atc gat aat gtt gct ggt ttt atc acc aac ttt gat ggg gtg 1171
Val Gly Ile Asp Asn Val Ala Gly Phe Ile Thr Asn Phe Asp Gly Val
            345                 350                 355

gac cta gtt gca ccg caa act gtg tcc cca gat cag ctg gat gaa ttg 1219
Asp Leu Val Ala Pro Gln Thr Val Ser Pro Asp Gln Leu Asp Glu Leu
        360                 365                 370

gaa tac gat cta ctt ctt gat gtc cgc aac cgc agt gaa gtc gaa gaa 1267
Glu Tyr Asp Leu Leu Leu Asp Val Arg Asn Arg Ser Glu Val Glu Glu
    375                 380                 385

ggc tac atc cca gga gca ctc cat att aat ggt gca tcc gtg ctg tgg 1315
Gly Tyr Ile Pro Gly Ala Leu His Ile Asn Gly Ala Ser Val Leu Trp
390                 395                 400                 405

aat ctg gag aaa ctg cca cgt gac gga aag atc gtg agc tac tgc aag 1363
Asn Leu Glu Lys Leu Pro Arg Asp Gly Lys Ile Val Ser Tyr Cys Lys
                410                 415                 420

agt gga aca cgc agc tca atc gcc gca agc acc ctg cgt aat gct ggt 1411
Ser Gly Thr Arg Ser Ser Ile Ala Ala Ser Thr Leu Arg Asn Ala Gly
            425                 430                 435

ttt gat gtg gtg gaa ctt caa gga tcc tat gac aac tgg gtc cgg cac 1459
Phe Asp Val Val Glu Leu Gln Gly Ser Tyr Asp Asn Trp Val Arg His
        440                 445                 450

aac 1462
Asn

<210> 124
<211> 454
<212> PRT
<213> Corynebacterium glutamicum

<400> 124
Val Leu Ile Glu Arg Ile Tyr Asp Glu Asp Leu Ala Gln Ala Ser Tyr
1               5                   10                  15

Phe Ile Gly Cys Gln Ala His Asn Thr Ala Val Val Val Asp Pro Arg
            20                  25                  30

Arg Asp Ile Ala Val Tyr Leu Asp Met Ala Lys Lys Asn Gly Met Glu
        35                  40                  45

Ile Val Gly Val Thr Glu Thr His Ile His Ala Asp Tyr Leu Ser Gly
    50                  55                  60

Thr Arg Glu Leu Ala Ala Ala Thr Asn Ala Thr Met Tyr Val Ser Gly
65                  70                  75                  80

Glu Gly Gly Ala Asp Trp Gln Tyr Glu Phe Asp Ala Glu Arg Ile Cys
                85                  90                  95

Asp Gly Ser Glu Ile Arg Leu Gly Asn Leu Val Leu Thr Ala Val His
            100                 105                 110

Thr Pro Gly His Thr Pro Glu His Leu Ser Phe Leu Leu Lys Asp Gly
        115                 120                 125

Ala Phe Ala Asp Glu Pro Gly Phe Met Leu Thr Gly Asp Phe Val Phe
    130                 135                 140

Ala Gly Asp Leu Gly Arg Pro Asp Leu Leu Asp Glu Ala Ala Gly Gly
145                 150                 155                 160

Val Asp Thr Arg Phe Glu Gly Ala Arg Gln Met Phe Lys Ser Leu Lys
                165                 170                 175

Glu Lys Phe Leu Thr Leu Pro Asp His Ile Gln Ile Phe Pro Gly His

            180                 185                 190

Gly Ser Gly Ser Ala Cys Gly Lys Ala Leu Gly Ser Val Pro Ser Thr
        195                 200                 205

Thr Leu Gly Tyr Glu Arg Gln Phe Ala Trp Trp Gly Lys Tyr Leu Glu
    210                 215                 220

Ala Asp Asp Glu Gln Gly Phe Ile Asp Glu Leu Leu Glu Gly Gln Pro
225                 230                 235                 240

Asp Ala Pro Ala Tyr Phe Gly Arg Met Lys Arg Gln Asn Arg Gln Gly
                245                 250                 255

Pro Ala Ile Met Gly Ala Arg Glu Leu Leu Pro Gln Leu Glu Ala Ser
            260                 265                 270

Asp Leu His Asp Val Ile Val Val Asp Thr Arg Ser Ala Asp Glu Val
        275                 280                 285

His Gln Gly Thr Val Ala Gly Ala Val Asn Ile Pro Ala Gly Asn Ser
    290                 295                 300

Met Ala Lys Phe Gly Ser Trp Thr Val Asp Pro Glu Lys Asp Ser Arg
305                 310                 315                 320

Ala Leu Val Leu Leu Ala Ala Ser Gln Ile Gly Ala Met Glu Met Trp
                325                 330                 335

Asp His Met Val Arg Val Gly Ile Asp Asn Val Ala Gly Phe Ile Thr
            340                 345                 350

Asn Phe Asp Gly Val Asp Leu Val Ala Pro Gln Thr Val Ser Pro Asp
        355                 360                 365

Gln Leu Asp Glu Leu Glu Tyr Asp Leu Leu Leu Asp Val Arg Asn Arg
    370                 375                 380

Ser Glu Val Glu Glu Gly Tyr Ile Pro Gly Ala Leu His Ile Asn Gly
385                 390                 395                 400

Ala Ser Val Leu Trp Asn Leu Glu Lys Leu Pro Arg Asp Gly Lys Ile
                405                 410                 415

Val Ser Tyr Cys Lys Ser Gly Thr Arg Ser Ser Ile Ala Ala Ser Thr
            420                 425                 430

Leu Arg Asn Ala Gly Phe Asp Val Val Glu Leu Gln Gly Ser Tyr Asp
        435                 440                 445

Asn Trp Val Arg His Asn
    450

<210> 125
<211> 1023
<212> DNA
<213> Corynebacterium glutamicum

<220>
<221> CDS

<222> (1)..(1023)
<223> FRXA00660

<400> 125
cca ggc cat acc ccg gaa cac tta tcg ttc ctg ctg aag gac ggc gcg     48
Pro Gly His Thr Pro Glu His Leu Ser Phe Leu Leu Lys Asp Gly Ala
1               5                   10                  15

ttc gca cat gag cca gga ttc atg ctc act ggc gat ttc gtt ttc gcg     96
Phe Ala His Glu Pro Gly Phe Met Leu Thr Gly Asp Phe Val Phe Ala
            20                  25                  30

ggt gat ctt ggc cga cca gat ttg ctc gat gaa gca gct ggg gga gtg     144
Gly Asp Leu Gly Arg Pro Asp Leu Leu Asp Glu Ala Ala Gly Gly Val
        35                  40                  45

gac act cgt ttt gag ggg gct cgc caa atg ttc aag agc ttg aag gaa     192
Asp Thr Arg Phe Glu Gly Ala Arg Gln Met Phe Lys Ser Leu Lys Glu
    50                  55                  60

aaa ttc ctg aca ttg cct gat cac atc cag atc ttc cct ggt cat ggt     240
Lys Phe Leu Thr Leu Pro Asp His Ile Gln Ile Phe Pro Gly His Gly
65                  70                  75                  80

tcc ggt tcc gcg tgt ggc aaa gcc ttg ggt tcg gtt cct tca aca aca     288
Ser Gly Ser Ala Cys Gly Lys Ala Leu Gly Ser Val Pro Ser Thr Thr
                85                  90                  95

ctt gga tat gaa cgt caa ttt gcg tgg tgg gga aag tat ctg gag gca     336
Leu Gly Tyr Glu Arg Gln Phe Ala Trp Trp Gly Lys Tyr Leu Glu Ala
            100                 105                 110

gat gat gaa caa gga ttc att gat gag ctt ctg gaa ggc caa cct gat     384
Asp Asp Glu Gln Gly Phe Ile Asp Glu Leu Leu Glu Gly Gln Pro Asp
        115                 120                 125

gca cct gca tac ttc ggc agg atg aag agg caa aat agg caa ggg ccc     432
Ala Pro Ala Tyr Phe Gly Arg Met Lys Arg Gln Asn Arg Gln Gly Pro
    130                 135                 140

gca att atg ggc gct cgc gag ctg ttg cca cag ctg gaa gct tct gat     480
Ala Ile Met Gly Ala Arg Glu Leu Leu Pro Gln Leu Glu Ala Ser Asp
145                 150                 155                 160

ctg cac gac gtc att gtt gtt gat acc cgc tca gcc gat gaa gtt cac     528
Leu His Asp Val Ile Val Val Asp Thr Arg Ser Ala Asp Glu Val His
                165                 170                 175

cag ggc act gta gct ggt gca gtg aat att cct gcg ggc aat tcg atg     576
Gln Gly Thr Val Ala Gly Ala Val Asn Ile Pro Ala Gly Asn Ser Met
            180                 185                 190

gcg aaa ttt ggc tcg tgg acc gtt gat ccc gag aag gat tcc cga gct     624
Ala Lys Phe Gly Ser Trp Thr Val Asp Pro Glu Lys Asp Ser Arg Ala
        195                 200                 205

ttg gtt ctg ctc gcg gca agc caa att ggt gcc atg gag atg tgg gac     672
Leu Val Leu Leu Ala Ala Ser Gln Ile Gly Ala Met Glu Met Trp Asp
    210                 215                 220

cac atg gtt cgc gtg gga atc gat aat gtt gct ggt ttt atc acc aac     720

His Met Val Arg Val Gly Ile Asp Asn Val Ala Gly Phe Ile Thr Asn
225                 230                 235                 240

ttt gat ggg gtg gac cta gtt gca ccg caa act gtg tcc cca gat cag     768
Phe Asp Gly Val Asp Leu Val Ala Pro Gln Thr Val Ser Pro Asp Gln
                245                 250                 255

ctg gat gaa ttg gaa tac gat cta ctt ctt gat gtc cgc aac cgc agt     816
Leu Asp Glu Leu Glu Tyr Asp Leu Leu Leu Asp Val Arg Asn Arg Ser
            260                 265                 270

gaa gtc gaa gaa ggc tac atc cca gga gca ctc cat att aat ggt gca     864
Glu Val Glu Glu Gly Tyr Ile Pro Gly Ala Leu His Ile Asn Gly Ala
        275                 280                 285

tcc gtg ctg tgg aat ctg gag aaa ctg cca cgt gac gga aag atc gtg     912
Ser Val Leu Trp Asn Leu Glu Lys Leu Pro Arg Asp Gly Lys Ile Val
    290                 295                 300

agc tac tgc aag agt gga aca cgc agc tca atc gcc gca agc acc ctg     960
Ser Tyr Cys Lys Ser Gly Thr Arg Ser Ser Ile Ala Ala Ser Thr Leu
305                 310                 315                 320

cgt aat gct ggt ttt gat gtg gtg gaa ctt caa gga tcc tat gac aac     1008
Arg Asn Ala Gly Phe Asp Val Val Glu Leu Gln Gly Ser Tyr Asp Asn
                325                 330                 335

tgg gtc cgg cac aac                                                 1023
Trp Val Arg His Asn
            340

<210> 126
<211> 341
<212> PRT
<213> Corynebacterium glutamicum

<400> 126
Pro Gly His Thr Pro Glu His Leu Ser Phe Leu Leu Lys Asp Gly Ala
  1               5                  10                  15

Phe Ala His Glu Pro Gly Phe Met Leu Thr Gly Asp Phe Val Phe Ala
            20                  25                  30

Gly Asp Leu Gly Arg Pro Asp Leu Leu Asp Glu Ala Ala Gly Gly Val
        35                  40                  45

Asp Thr Arg Phe Glu Gly Ala Arg Gln Met Phe Lys Ser Leu Lys Glu
    50                  55                  60

Lys Phe Leu Thr Leu Pro Asp His Ile Gln Ile Phe Pro Gly His Gly
 65                  70                  75                  80

Ser Gly Ser Ala Cys Gly Lys Ala Leu Gly Ser Val Pro Ser Thr Thr
                 85                  90                  95

Leu Gly Tyr Glu Arg Gln Phe Ala Trp Trp Gly Lys Tyr Leu Glu Ala
            100                 105                 110

Asp Asp Glu Gln Gly Phe Ile Asp Glu Leu Leu Glu Gly Gln Pro Asp
        115                 120                 125

Ala Pro Ala Tyr Phe Gly Arg Met Lys Arg Gln Asn Arg Gln Gly Pro
    130                 135                 140

Ala Ile Met Gly Ala Arg Glu Leu Leu Pro Gln Leu Glu Ala Ser Asp
145                 150                 155                 160

Leu His Asp Val Ile Val Val Asp Thr Arg Ser Ala Asp Glu Val His
                165                 170                 175

Gln Gly Thr Val Ala Gly Ala Val Asn Ile Pro Ala Gly Asn Ser Met
            180                 185                 190

Ala Lys Phe Gly Ser Trp Thr Val Asp Pro Glu Lys Asp Ser Arg Ala
        195                 200                 205

Leu Val Leu Leu Ala Ala Ser Gln Ile Gly Ala Met Glu Met Trp Asp
    210                 215                 220

His Met Val Arg Val Gly Ile Asp Asn Val Ala Gly Phe Ile Thr Asn
225                 230                 235                 240

Phe Asp Gly Val Asp Leu Val Ala Pro Gln Thr Val Ser Pro Asp Gln
                245                 250                 255

Leu Asp Glu Leu Glu Tyr Asp Leu Leu Leu Asp Val Arg Asn Arg Ser
            260                 265                 270

Glu Val Glu Glu Gly Tyr Ile Pro Gly Ala Leu His Ile Asn Gly Ala
        275                 280                 285

Ser Val Leu Trp Asn Leu Glu Lys Leu Pro Arg Asp Gly Lys Ile Val
    290                 295                 300

Ser Tyr Cys Lys Ser Gly Thr Arg Ser Ser Ile Ala Ala Ser Thr Leu
305                 310                 315                 320

Arg Asn Ala Gly Phe Asp Val Val Glu Leu Gln Gly Ser Tyr Asp Asn
                325                 330                 335

Trp Val Arg His Asn
            340


<210> 127
<211> 756
<212> DNA
<213> Corynebacterium glutamicum

<220>
<221> CDS
<222> (101)..(733)
<223> RXA00801

<400> 127
ggataagttt gtttctgagc gtattggtct tgatgatgtt gaagaggctt tcaacaccat 60

gaaggctggc gacgtgctgc gttctgtggt ggagatctaa atg gct cac gac gga   115
                                            Met Ala His Asp Gly
                                            1               5

ttg cgc gta gaa aac att gtc acc tca ggc atc ttt gcc ctt gat ggt     163
Leu Arg Val Glu Asn Ile Val Thr Ser Gly Ile Phe Ala Leu Asp Gly
                10                  15                  20

ggc gaa tgg gaa gtc gac aac aac atc tgg gtt gtg gga aat gat gat     211
Gly Glu Trp Glu Val Asp Asn Asn Ile Trp Val Val Gly Asn Asp Asp
            25                  30                  35

gag gtt ttc atc atc gat gcg gca cac act gca gca ccc atc atc gag     259
Glu Val Phe Ile Ile Asp Ala Ala His Thr Ala Ala Pro Ile Ile Glu
        40                  45                  50

gct gtc ggt gga cgt gct gtg aag ggc att ttg tgc acc cac gca cac     307
Ala Val Gly Gly Arg Ala Val Lys Gly Ile Leu Cys Thr His Ala His
    55                  60                  65

aat gac cac atc act gtc gca cca gag cta tcc aag gaa ttt gat gca     355
Asn Asp His Ile Thr Val Ala Pro Glu Leu Ser Lys Glu Phe Asp Ala
70                  75                  80                  85

cca atc ttc gtg cac cca ggt gac caa atg ctg tgg gag gaa acc cac     403
Pro Ile Phe Val His Pro Gly Asp Gln Met Leu Trp Glu Glu Thr His
                90                  95                  100

gga aac ctg acc cac gag gat ttg gca gat cag cag aag ttc caa atc     451
Gly Asn Leu Thr His Glu Asp Leu Ala Asp Gln Gln Lys Phe Gln Ile
            105                 110                 115

gct gga act gaa ctg atc gtg ctt aat acc cct gga cac tca cct gga     499
Ala Gly Thr Glu Leu Ile Val Leu Asn Thr Pro Gly His Ser Pro Gly
        120                 125                 130

tcc agc tgc ttc tac ctc cct gaa gca aac gag ctc ttc tct gga gac     547
Ser Ser Cys Phe Tyr Leu Pro Glu Ala Asn Glu Leu Phe Ser Gly Asp
    135                 140                 145

act ttg ttc cag ggt ggg ccg gga gca act ggc cgt aag tac agc tcc     595
Thr Leu Phe Gln Gly Gly Pro Gly Ala Thr Gly Arg Lys Tyr Ser Ser
150                 155                 160                 165

ttt gac acc atc att gag tcc ctc aag acc tca att ttg gat cta cca     643
Phe Asp Thr Ile Ile Glu Ser Leu Lys Thr Ser Ile Leu Asp Leu Pro
                170                 175                 180

gcg gaa acc acc gtg cgc act ggc cat ggt gat cac acc agt gtg ggg     691
Ala Glu Thr Thr Val Arg Thr Gly His Gly Asp His Thr Ser Val Gly
            185                 190                 195

gct gag gct cca cac ttg gag gaa tgg att aaa cgc ggg cac             733
Ala Glu Ala Pro His Leu Glu Glu Trp Ile Lys Arg Gly His
        200                 205                 210

taagccccga acgattagta ggc                                           756

<210> 128
<211> 211
<212> PRT
<213> Corynebacterium glutamicum

<400> 128

Met Ala His Asp Gly Leu Arg Val Glu Asn Ile Val Thr Ser Gly Ile
1               5                   10                  15

Phe Ala Leu Asp Gly Gly Glu Trp Glu Val Asp Asn Asn Ile Trp Val
            20                  25                  30

Val Gly Asn Asp Asp Glu Val Phe Ile Ile Asp Ala Ala His Thr Ala
        35                  40                  45

Ala Pro Ile Ile Glu Ala Val Gly Gly Arg Ala Val Lys Gly Ile Leu
    50                  55                  60

Cys Thr His Ala His Asn Asp His Ile Thr Val Ala Pro Glu Leu Ser
65                  70                  75                  80

Lys Glu Phe Asp Ala Pro Ile Phe Val His Pro Gly Asp Gln Met Leu
                85                  90                  95

Trp Glu Glu Thr His Gly Asn Leu Thr His Glu Asp Leu Ala Asp Gln
            100                 105                 110

Gln Lys Phe Gln Ile Ala Gly Thr Glu Leu Ile Val Leu Asn Thr Pro
        115                 120                 125

Gly His Ser Pro Gly Ser Ser Cys Phe Tyr Leu Pro Glu Ala Asn Glu
    130                 135                 140

Leu Phe Ser Gly Asp Thr Leu Phe Gln Gly Gly Pro Gly Ala Thr Gly
145                 150                 155                 160

Arg Lys Tyr Ser Ser Phe Asp Thr Ile Ile Glu Ser Leu Lys Thr Ser
                165                 170                 175

Ile Leu Asp Leu Pro Ala Glu Thr Thr Val Arg Thr Gly His Gly Asp
            180                 185                 190

His Thr Ser Val Gly Ala Glu Ala Pro His Leu Glu Glu Trp Ile Lys
        195                 200                 205

Arg Gly His
    210

<210> 129
<211> 966
<212> DNA
<213> Corynebacterium glutamicum

<220>
<221> CDS
<222> (101)..(943)
<223> RXA00821

<400> 129
ttaaaagctt gcttctcgac gcaaaaaccc atccggcgca tcccttcaat gttaggggtg 60

cgctgctatt tttctcccca gttctacgaa atgacttatt gtg act aag ctt gag   115
                                            Val Thr Lys Leu Glu
                                            1               5

cgc atg gag cat cct gct tac agc caa ttg cgg ccg gtt acc ccg tcc   163

Arg Met Glu His Pro Ala Tyr Ser Gln Leu Arg Pro Val Thr Pro Ser
                10                  15                  20

gca tct gtt gtt ttg tgc cct aat ccc ggt tac agc tcg ctg gaa ggc     211
Ala Ser Val Val Leu Cys Pro Asn Pro Gly Tyr Ser Ser Leu Glu Gly
            25                  30                  35

act aat tct tgg gtt atc cgg gca cca gaa gac ccc cgg agc att gtc     259
Thr Asn Ser Trp Val Ile Arg Ala Pro Glu Asp Pro Arg Ser Ile Val
        40                  45                  50

atc gat cca ggt cct gaa gat gag ggc cac ctt aac gtc ttg cat tcc     307
Ile Asp Pro Gly Pro Glu Asp Glu Gly His Leu Asn Val Leu His Ser
    55                  60                  65

aag gct gag gag gtg ggt ttg att ctt ctg acc cac cgt cac tat gat     355
Lys Ala Glu Glu Val Gly Leu Ile Leu Leu Thr His Arg His Tyr Asp
70                  75                  80                  85

cat gct gac ggc gca cag cgt ttc cgt cag ctg acc aat gca cct gtg     403
His Ala Asp Gly Ala Gln Arg Phe Arg Gln Leu Thr Asn Ala Pro Val
                90                  95                  100

cgt gcg atg gac cct tcg tac tgt gct ggt gcg gag gag att cat gat     451
Arg Ala Met Asp Pro Ser Tyr Cys Ala Gly Ala Glu Glu Ile His Asp
            105                 110                 115

ggt gag atc atc acg atc gac ggt gtc acc cca cag att gag gtg gtg     499
Gly Glu Ile Ile Thr Ile Asp Gly Val Thr Pro Gln Ile Glu Val Val
        120                 125                 130

gcc aca cct ggt cat acc cgt gat tct gtg tct tat ttc atc tgg agt     547
Ala Thr Pro Gly His Thr Arg Asp Ser Val Ser Tyr Phe Ile Trp Ser
    135                 140                 145

gga gtc cct cat gag tcc act ttg gag ggc atc gtt tct ggc gac acc     595
Gly Val Pro His Glu Ser Thr Leu Glu Gly Ile Val Ser Gly Asp Thr
150                 155                 160                 165

att gcg ggt cgt cac acc acg atg att tca gag acc gac ggc gat ttg     643
Ile Ala Gly Arg His Thr Thr Met Ile Ser Glu Thr Asp Gly Asp Leu
                170                 175                 180

ggt gag tac ctg aat tct ttg gcc att ttg gag gag cgc ggc aag gat     691
Gly Glu Tyr Leu Asn Ser Leu Ala Ile Leu Glu Glu Arg Gly Lys Asp
            185                 190                 195

att ccg ctg ctt cca gga cat ggt cca gat gga cag gac gtg tcc tcc     739
Ile Pro Leu Leu Pro Gly His Gly Pro Asp Gly Gln Asp Val Ser Ser
        200                 205                 210

ttc gcg cgt aag tac att gag cgt cgt gag ctg cgt ctg aac cag atc     787
Phe Ala Arg Lys Tyr Ile Glu Arg Arg Glu Leu Arg Leu Asn Gln Ile
    215                 220                 225

cgt gag gta tgg gag acc cgt ggc cgt gac gtg tcc atg aag gat ctc     835
Arg Glu Val Trp Glu Thr Arg Gly Arg Asp Val Ser Met Lys Asp Leu
230                 235                 240                 245

atc gac gcc atc tac gat gat gtt gat cca gtt ctg cgt ggt gca gcc     883
Ile Asp Ala Ile Tyr Asp Asp Val Asp Pro Val Leu Arg Gly Ala Ala

                250                 255                 260

gag cag tcc act cat gtg gct att cgt tac ctg cag gct cag gaa gct 931
Glu Gln Ser Thr His Val Ala Ile Arg Tyr Leu Gln Ala Gln Glu Ala
            265                 270                 275

tcc gcc tca aac taaacacttt taactaaaca aca 966
Ser Ala Ser Asn
        280

<210> 130
<211> 281
<212> PRT
<213> Corynebacterium glutamicum

<400> 130
Val Thr Lys Leu Glu Arg Met Glu His Pro Ala Tyr Ser Gln Leu Arg
1               5                   10                  15

Pro Val Thr Pro Ser Ala Ser Val Val Leu Cys Pro Asn Pro Gly Tyr
            20                  25                  30

Ser Ser Leu Glu Gly Thr Asn Ser Trp Val Ile Arg Ala Pro Glu Asp
        35                  40                  45

Pro Arg Ser Ile Val Ile Asp Pro Gly Pro Glu Asp Glu Gly His Leu
    50                  55                  60

Asn Val Leu His Ser Lys Ala Glu Glu Val Gly Leu Ile Leu Leu Thr
65                  70                  75                  80

His Arg His Tyr Asp His Ala Asp Gly Ala Gln Arg Phe Arg Gln Leu
                85                  90                  95

Thr Asn Ala Pro Val Arg Ala Met Asp Pro Ser Tyr Cys Ala Gly Ala
            100                 105                 110

Glu Glu Ile His Asp Gly Glu Ile Ile Thr Ile Asp Gly Val Thr Pro
        115                 120                 125

Gln Ile Glu Val Val Ala Thr Pro Gly His Thr Arg Asp Ser Val Ser
    130                 135                 140

Tyr Phe Ile Trp Ser Gly Val Pro His Glu Ser Thr Leu Glu Gly Ile
145                 150                 155                 160

Val Ser Gly Asp Thr Ile Ala Gly Arg His Thr Thr Met Ile Ser Glu
                165                 170                 175

Thr Asp Gly Asp Leu Gly Glu Tyr Leu Asn Ser Leu Ala Ile Leu Glu
            180                 185                 190

Glu Arg Gly Lys Asp Ile Pro Leu Leu Pro Gly His Gly Pro Asp Gly
        195                 200                 205

Gln Asp Val Ser Ser Phe Ala Arg Lys Tyr Ile Glu Arg Arg Glu Leu
    210                 215                 220

Arg Leu Asn Gln Ile Arg Glu Val Trp Glu Thr Arg Gly Arg Asp Val
225                 230                 235                 240

Ser Met Lys Asp Leu Ile Asp Ala Ile Tyr Asp Asp Val Asp Pro Val
                245                 250                 255

Leu Arg Gly Ala Ala Glu Gln Ser Thr His Val Ala Ile Arg Tyr Leu
            260                 265                 270

Gln Ala Gln Glu Ala Ser Ala Ser Asn
        275                 280


<210> 131
<211> 1530
<212> DNA
<213> Corynebacterium glutamicum

<220>
<221> CDS
<222> (101)..(1507)
<223> RXN02966

<400> 131
aaatataccc cccagggtat cttgacagat taaagctcga tgttttaggc tctacatata 60

cccccacggg tatcccctca actttgatct aaggtgtcac atg ctt ttt gaa cgc   115
                                            Met Leu Phe Glu Arg
                                              1               5

atc tac gaa gaa ggc ctc gcc caa gcc agc tat ttc att ggc tgc caa   163
Ile Tyr Glu Glu Gly Leu Ala Gln Ala Ser Tyr Phe Ile Gly Cys Gln
                10                  15                  20

cgc gaa ggc aaa gcg att gtt gtt gat gct cgc cga gat atc cag acc   211
Arg Glu Gly Lys Ala Ile Val Val Asp Ala Arg Arg Asp Ile Gln Thr
            25                  30                  35

tat ctg gac ctt gca gca aaa aac aac atg gtc att agc gcc gta acc   259
Tyr Leu Asp Leu Ala Ala Lys Asn Asn Met Val Ile Ser Ala Val Thr
        40                  45                  50

gaa acc cat att cat gcc gat tat ctc tcc ggt act cgc gaa ctt gca   307
Glu Thr His Ile His Ala Asp Tyr Leu Ser Gly Thr Arg Glu Leu Ala
    55                  60                  65

gct gcc acc ggc gcc gag att ttc ctc tct ggc gaa ggc gga gct gat   355
Ala Ala Thr Gly Ala Glu Ile Phe Leu Ser Gly Glu Gly Gly Ala Asp
70                  75                  80                  85

tgg caa tat ggc ttt aca ggc acc acc ttg atg cac aat tcc acc atc   403
Trp Gln Tyr Gly Phe Thr Gly Thr Thr Leu Met His Asn Ser Thr Ile
                90                  95                  100

aag ctg gga aat atc acc atc aca gcc aag cac act ccc gga cac act   451
Lys Leu Gly Asn Ile Thr Ile Thr Ala Lys His Thr Pro Gly His Thr
            105                 110                 115

cca gag cac ctg tca ttt ttg att act gat ggt gcg gtc tca aag gat   499
Pro Glu His Leu Ser Phe Leu Ile Thr Asp Gly Ala Val Ser Lys Asp
        120                 125                 130

ccc ggt ttt atg ctc agc ggt gac ttc gtc ttc gta ggt gac gtg gga   547

Pro Gly Phe Met Leu Ser Gly Asp Phe Val Phe Val Gly Asp Val Gly
    135                 140                 145

cgt cca gat tta ctt gat gag gca gct ggc ggc gtg gac acc cgc ttc     595
Arg Pro Asp Leu Leu Asp Glu Ala Ala Gly Gly Val Asp Thr Arg Phe
150                 155                 160                 165

gcc gga gca cag caa ctc ttc cat agc cta aaa gag cag ttc ctt gca     643
Ala Gly Ala Gln Gln Leu Phe His Ser Leu Lys Glu Gln Phe Leu Ala
                170                 175                 180

ctc ccc gac cac att cag gtt tat cca ggt cat ggt gct ggc agc cct     691
Leu Pro Asp His Ile Gln Val Tyr Pro Gly His Gly Ala Gly Ser Pro
            185                 190                 195

tgt ggc aag gca ttg ggc gcg atc cct agc acc acc gtg gga tat gaa     739
Cys Gly Lys Ala Leu Gly Ala Ile Pro Ser Thr Thr Val Gly Tyr Glu
        200                 205                 210

aag gct aat gcg tgg tgg gct cca tat ctg cgc agt gat gat gaa gcc     787
Lys Ala Asn Ala Trp Trp Ala Pro Tyr Leu Arg Ser Asp Asp Glu Ala
    215                 220                 225

ggc ttt gtg gaa gag ctt ctc gac ggc cag cca gat gcc cac gct tac     835
Gly Phe Val Glu Glu Leu Leu Asp Gly Gln Pro Asp Ala His Ala Tyr
230                 235                 240                 245

ttt gct cgc atg aaa aag cag aac aag cag gga cct gca gta ctt agt     883
Phe Ala Arg Met Lys Lys Gln Asn Lys Gln Gly Pro Ala Val Leu Ser
                250                 255                 260

aca tta tcc ccg ctt gtg aag cta gaa gcc gag gaa gtc gtc gaa aag     931
Thr Leu Ser Pro Leu Val Lys Leu Glu Ala Glu Glu Val Val Glu Lys
            265                 270                 275

ctt ggt tct gaa gca gta ttt gtg gat acc cgc gag caa aac caa gtc     979
Leu Gly Ser Glu Ala Val Phe Val Asp Thr Arg Glu Gln Asn Gln Val
        280                 285                 290

cat ctc gga acc gtt gtt ggg gca ttg aat att ccg cgc ggc gcc aag     1027
His Leu Gly Thr Val Val Gly Ala Leu Asn Ile Pro Arg Gly Ala Lys
    295                 300                 305

gcg tcc aat ttt gcg gcg tgg gtt att gat cct caa aag gat gct cag     1075
Ala Ser Asn Phe Ala Ala Trp Val Ile Asp Pro Gln Lys Asp Ala Gln
310                 315                 320                 325

gac ctt att gtt ttg gct ccg gac gcc aat acc gct gcg gat ttc cgc     1123
Asp Leu Ile Val Leu Ala Pro Asp Ala Asn Thr Ala Ala Asp Phe Arg
                330                 335                 340

gac gct ttg ctg cgg gtt ggc atc gac act gtg cgt tat ttc acc aac     1171
Asp Ala Leu Leu Arg Val Gly Ile Asp Thr Val Arg Tyr Phe Thr Asn
            345                 350                 355

agt atc gat gga ttg cct acc ttt gtg cca gaa ctc atc tcc ccc gct     1219
Ser Ile Asp Gly Leu Pro Thr Phe Val Pro Glu Leu Ile Ser Pro Ala
        360                 365                 370

gag cta gct gag acc aac tat gac gca ctg att gat atc cgt gca aag     1267
Glu Leu Ala Glu Thr Asn Tyr Asp Ala Leu Ile Asp Ile Arg Ala Lys

375                 380                 385

tcc gaa ttt gcc gct ggc agc att ccc ggc gcg cag cag ctt tct gga     1315
Ser Glu Phe Ala Ala Gly Ser Ile Pro Gly Ala Gln Gln Leu Ser Gly
390                 395                 400                 405

ggt tcg gcc atg tgg cgc ctc aat gag ctg cct gcg ggt ggc act ttg     1363
Gly Ser Ala Met Trp Arg Leu Asn Glu Leu Pro Ala Gly Gly Thr Leu
                410                 415                 420

gta acc ttc tgc caa tca gga gcg cga aat acc gtg gta gcc aat gct     1411
Val Thr Phe Cys Gln Ser Gly Ala Arg Asn Thr Val Val Ala Asn Ala
            425                 430                 435

ttg cga cgc gcc gga ttc acc gtt atc gag ctc gag ggc agc tac gcc     1459
Leu Arg Arg Ala Gly Phe Thr Val Ile Glu Leu Glu Gly Ser Tyr Ala
        440                 445                 450

gcg tgg gaa aaa tca gct gcc aat cct aaa aac ttg cag act gcc gtc     1507
Ala Trp Glu Lys Ser Ala Ala Asn Pro Lys Asn Leu Gln Thr Ala Val
    455                 460                 465

tagttttaga tccggcgctg tat                                           1530

<210> 132
<211> 469
<212> PRT
<213> Corynebacterium glutamicum

<400> 132
Met Leu Phe Glu Arg Ile Tyr Glu Glu Gly Leu Ala Gln Ala Ser Tyr
1               5                   10                  15

Phe Ile Gly Cys Gln Arg Glu Gly Lys Ala Ile Val Val Asp Ala Arg
            20                  25                  30

Arg Asp Ile Gln Thr Tyr Leu Asp Leu Ala Ala Lys Asn Asn Met Val
        35                  40                  45

Ile Ser Ala Val Thr Glu Thr His Ile His Ala Asp Tyr Leu Ser Gly
    50                  55                  60

Thr Arg Glu Leu Ala Ala Ala Thr Gly Ala Glu Ile Phe Leu Ser Gly
65                  70                  75                  80

Glu Gly Gly Ala Asp Trp Gln Tyr Gly Phe Thr Gly Thr Thr Leu Met
                85                  90                  95

His Asn Ser Thr Ile Lys Leu Gly Asn Ile Thr Ile Thr Ala Lys His
            100                 105                 110

Thr Pro Gly His Thr Pro Glu His Leu Ser Phe Leu Ile Thr Asp Gly
        115                 120                 125

Ala Val Ser Lys Asp Pro Gly Phe Met Leu Ser Gly Asp Phe Val Phe
    130                 135                 140

Val Gly Asp Val Gly Arg Pro Asp Leu Leu Asp Glu Ala Ala Gly Gly
145                 150                 155                 160

Val Asp Thr Arg Phe Ala Gly Ala Gln Gln Leu Phe His Ser Leu Lys
                165                 170                 175

Glu Gln Phe Leu Ala Leu Pro Asp His Ile Gln Val Tyr Pro Gly His
            180                 185                 190

Gly Ala Gly Ser Pro Cys Gly Lys Ala Leu Gly Ala Ile Pro Ser Thr
        195                 200                 205

Thr Val Gly Tyr Glu Lys Ala Asn Ala Trp Trp Ala Pro Tyr Leu Arg
    210                 215                 220

Ser Asp Asp Glu Ala Gly Phe Val Glu Glu Leu Leu Asp Gly Gln Pro
225                 230                 235                 240

Asp Ala His Ala Tyr Phe Ala Arg Met Lys Lys Gln Asn Lys Gln Gly
                245                 250                 255

Pro Ala Val Leu Ser Thr Leu Ser Pro Leu Val Lys Leu Glu Ala Glu
            260                 265                 270

Glu Val Val Glu Lys Leu Gly Ser Glu Ala Val Phe Val Asp Thr Arg
        275                 280                 285

Glu Gln Asn Gln Val His Leu Gly Thr Val Val Gly Ala Leu Asn Ile
    290                 295                 300

Pro Arg Gly Ala Lys Ala Ser Asn Phe Ala Ala Trp Val Ile Asp Pro
305                 310                 315                 320

Gln Lys Asp Ala Gln Asp Leu Ile Val Leu Ala Pro Asp Ala Asn Thr
                325                 330                 335

Ala Ala Asp Phe Arg Asp Ala Leu Leu Arg Val Gly Ile Asp Thr Val
            340                 345                 350

Arg Tyr Phe Thr Asn Ser Ile Asp Gly Leu Pro Thr Phe Val Pro Glu
        355                 360                 365

Leu Ile Ser Pro Ala Glu Leu Ala Glu Thr Asn Tyr Asp Ala Leu Ile
    370                 375                 380

Asp Ile Arg Ala Lys Ser Glu Phe Ala Ala Gly Ser Ile Pro Gly Ala
385                 390                 395                 400

Gln Gln Leu Ser Gly Gly Ser Ala Met Trp Arg Leu Asn Glu Leu Pro
                405                 410                 415

Ala Gly Gly Thr Leu Val Thr Phe Cys Gln Ser Gly Ala Arg Asn Thr
            420                 425                 430

Val Val Ala Asn Ala Leu Arg Arg Ala Gly Phe Thr Val Ile Glu Leu
        435                 440                 445

Glu Gly Ser Tyr Ala Ala Trp Glu Lys Ser Ala Ala Asn Pro Lys Asn
    450                 455                 460

Leu Gln Thr Ala Val
465

<210> 133
<211> 1530
<212> DNA
<213> Corynebacterium glutamicum

<220>
<221> CDS
<222> (101)..(1507)
<223> FRXA01833

<400> 133
aaatataccc cccagggtat cttgacagat taaagctcga tgttttaggc tctacatata 60

cccccacggg tatcccctca actttgatct aaggtgtcac atg ctt ttt gaa cgc 115
Met Leu Phe Glu Arg
1               5

atc tac gaa gaa ggc ctc gcc caa gcc agc tat ttc att ggc tgc caa 163
Ile Tyr Glu Glu Gly Leu Ala Gln Ala Ser Tyr Phe Ile Gly Cys Gln
                10                  15                  20

cgc gaa ggc aaa gcg att gtt gtt gat gct cgc cga gat atc cag acc 211
Arg Glu Gly Lys Ala Ile Val Val Asp Ala Arg Arg Asp Ile Gln Thr
            25                  30                  35

tat ctg gac ctt gca gca aaa aac aac atg gtc att agc gcc gta acc 259
Tyr Leu Asp Leu Ala Ala Lys Asn Asn Met Val Ile Ser Ala Val Thr
        40                  45                  50

gaa acc cat att cat gcc gat tat ctc tcc ggt act cgc gaa ctt gca 307
Glu Thr His Ile His Ala Asp Tyr Leu Ser Gly Thr Arg Glu Leu Ala
    55                  60                  65

gct gcc acc ggc gcc gag att ttc ctc tct ggc gaa ggc gga gct gat 355
Ala Ala Thr Gly Ala Glu Ile Phe Leu Ser Gly Glu Gly Gly Ala Asp
70                  75                  80                  85

tgg caa tat ggc ttt aca ggc acc acc ttg atg cac aat tcc acc atc 403
Trp Gln Tyr Gly Phe Thr Gly Thr Thr Leu Met His Asn Ser Thr Ile
                90                  95                  100

aag ctg gaa aat atc acc atc aca gcc aag cac act ccc gga cac act 451
Lys Leu Glu Asn Ile Thr Ile Thr Ala Lys His Thr Pro Gly His Thr
            105                 110                 115

cca gag cac ctg tca ttt ttg att act gat ggt gcg gtc tca aag gat 499
Pro Glu His Leu Ser Phe Leu Ile Thr Asp Gly Ala Val Ser Lys Asp
        120                 125                 130

ccc ggt ttt atg ctc agc ggt gac ttc gtc ttc gta ggt gac gtg gga 547
Pro Gly Phe Met Leu Ser Gly Asp Phe Val Phe Val Gly Asp Val Gly
    135                 140                 145

cgt cca gat tta ctt gat gag gca gct ggc ggc gtg gac acc cgc ttc 595
Arg Pro Asp Leu Leu Asp Glu Ala Ala Gly Gly Val Asp Thr Arg Phe
150                 155                 160                 165

gcc gga gca cag caa ctc ttc cat agc cta aaa gag cag ttc ctt gca 643
Ala Gly Ala Gln Gln Leu Phe His Ser Leu Lys Glu Gln Phe Leu Ala
                170                 175                 180

ctc ccc gac cac att cag gtt tat cca ggt cat ggt gct ggc agc cct 691
Leu Pro Asp His Ile Gln Val Tyr Pro Gly His Gly Ala Gly Ser Pro
            185                 190                 195

tgt ggc aag gca ttg ggc gcg atc cct agc acc acc gtg gga tat gaa 739
Cys Gly Lys Ala Leu Gly Ala Ile Pro Ser Thr Thr Val Gly Tyr Glu
        200                 205                 210

aag gct aat gcg tgg tgg gct cca tat ctg cgc agt gat gat gaa gcc 787
Lys Ala Asn Ala Trp Trp Ala Pro Tyr Leu Arg Ser Asp Asp Glu Ala
    215                 220                 225

ggc ttt gtg gaa gag ctt ctc gac ggc cag cca gat gcc cac gct tac 835
Gly Phe Val Glu Glu Leu Leu Asp Gly Gln Pro Asp Ala His Ala Tyr
230                 235                 240                 245

ttt gct cgc atg aaa aag cag aac aag cag gga cct gca gta ctt agt 883
Phe Ala Arg Met Lys Lys Gln Asn Lys Gln Gly Pro Ala Val Leu Ser
                250                 255                 260

aca tta tcc ccg ctt gtg aag cta gaa gcc gag gaa gtc gtc gaa aag 931
Thr Leu Ser Pro Leu Val Lys Leu Glu Ala Glu Glu Val Val Glu Lys
            265                 270                 275

ctt ggt tct gaa gca gta ttt gtg gat acc cgc gag caa aac caa gtc 979
Leu Gly Ser Glu Ala Val Phe Val Asp Thr Arg Glu Gln Asn Gln Val
        280                 285                 290

cat ctc gga acc gtt gtt ggg gca ttg aat att ccg cgc ggc gcc aag 1027
His Leu Gly Thr Val Val Gly Ala Leu Asn Ile Pro Arg Gly Ala Lys
    295                 300                 305

gcg tcc aat ttt gcg gcg tgg gtt att gat cct caa aag gat gct cag 1075
Ala Ser Asn Phe Ala Ala Trp Val Ile Asp Pro Gln Lys Asp Ala Gln
310                 315                 320                 325

gac ctt att gtt ttg gct ccg gac gcc aat acc gct gcg gat ttc cgc 1123
Asp Leu Ile Val Leu Ala Pro Asp Ala Asn Thr Ala Ala Asp Phe Arg
                330                 335                 340

gac gct ttg ctg cgg gtt ggc atc gac act gtg cgt tat ttc acc aac 1171
Asp Ala Leu Leu Arg Val Gly Ile Asp Thr Val Arg Tyr Phe Thr Asn
            345                 350                 355

agt atc gat gga ttg cct acc ttt gtg cca gaa ctc atc tcc ccc gct 1219
Ser Ile Asp Gly Leu Pro Thr Phe Val Pro Glu Leu Ile Ser Pro Ala
        360                 365                 370

gag cta gct gag acc aac tat gac gca ctg att gat atc cgt gca aag 1267
Glu Leu Ala Glu Thr Asn Tyr Asp Ala Leu Ile Asp Ile Arg Ala Lys
    375                 380                 385

tcc gaa ttt gcc gct ggc agc att ccc ggc gcg cag cag ctt tct gga 1315
Ser Glu Phe Ala Ala Gly Ser Ile Pro Gly Ala Gln Gln Leu Ser Gly
390                 395                 400                 405

ggt tcg gcc atg tgg cgc ctc aat gag ctg cct gcg ggt ggc act ttg 1363
Gly Ser Ala Met Trp Arg Leu Asn Glu Leu Pro Ala Gly Gly Thr Leu
                410                 415                 420

gta acc ttc tgc caa tca gga gcg cga aat acc gtg gta gcc aat gct 1411

Val Thr Phe Cys Gln Ser Gly Ala Arg Asn Thr Val Val Ala Asn Ala
            425                 430                 435

ttg cga cgc gcc gga ttc acc gtt atc gag ctc gag ggc agc tac gcc    1459
Leu Arg Arg Ala Gly Phe Thr Val Ile Glu Leu Glu Gly Ser Tyr Ala
        440                 445                 450

gcg tgg gaa aaa tca gct gcc aat cct aaa aac ttg cag act gcc gtc    1507
Ala Trp Glu Lys Ser Ala Ala Asn Pro Lys Asn Leu Gln Thr Ala Val
    455                 460                 465

tagttttaga tccggcgctg tat                                          1530

<210> 134
<211> 469
<212> PRT
<213> Corynebacterium glutamicum

<400> 134
Met Leu Phe Glu Arg Ile Tyr Glu Glu Gly Leu Ala Gln Ala Ser Tyr
1               5                   10                  15

Phe Ile Gly Cys Gln Arg Glu Gly Lys Ala Ile Val Val Asp Ala Arg
            20                  25                  30

Arg Asp Ile Gln Thr Tyr Leu Asp Leu Ala Ala Lys Asn Asn Met Val
        35                  40                  45

Ile Ser Ala Val Thr Glu Thr His Ile His Ala Asp Tyr Leu Ser Gly
    50                  55                  60

Thr Arg Glu Leu Ala Ala Ala Thr Gly Ala Glu Ile Phe Leu Ser Gly
65                  70                  75                  80

Glu Gly Gly Ala Asp Trp Gln Tyr Gly Phe Thr Gly Thr Thr Leu Met
                85                  90                  95

His Asn Ser Thr Ile Lys Leu Glu Asn Ile Thr Ile Thr Ala Lys His
            100                 105                 110

Thr Pro Gly His Thr Pro Glu His Leu Ser Phe Leu Ile Thr Asp Gly
        115                 120                 125

Ala Val Ser Lys Asp Pro Gly Phe Met Leu Ser Gly Asp Phe Val Phe
    130                 135                 140

Val Gly Asp Val Gly Arg Pro Asp Leu Leu Asp Glu Ala Ala Gly Gly
145                 150                 155                 160

Val Asp Thr Arg Phe Ala Gly Ala Gln Gln Leu Phe His Ser Leu Lys
                165                 170                 175

Glu Gln Phe Leu Ala Leu Pro Asp His Ile Gln Val Tyr Pro Gly His
            180                 185                 190

Gly Ala Gly Ser Pro Cys Gly Lys Ala Leu Gly Ala Ile Pro Ser Thr
        195                 200                 205

Thr Val Gly Tyr Glu Lys Ala Asn Ala Trp Trp Ala Pro Tyr Leu Arg
    210                 215                 220

Ser Asp Asp Glu Ala Gly Phe Val Glu Glu Leu Leu Asp Gly Gln Pro
225                 230                 235                 240

Asp Ala His Ala Tyr Phe Ala Arg Met Lys Lys Gln Asn Lys Gln Gly
                245                 250                 255

Pro Ala Val Leu Ser Thr Leu Ser Pro Leu Val Lys Leu Glu Ala Glu
            260                 265                 270

Glu Val Val Glu Lys Leu Gly Ser Glu Ala Val Phe Val Asp Thr Arg
        275                 280                 285

Glu Gln Asn Gln Val His Leu Gly Thr Val Val Gly Ala Leu Asn Ile
    290                 295                 300

Pro Arg Gly Ala Lys Ala Ser Asn Phe Ala Ala Trp Val Ile Asp Pro
305                 310                 315                 320

Gln Lys Asp Ala Gln Asp Leu Ile Val Leu Ala Pro Asp Ala Asn Thr
                325                 330                 335

Ala Ala Asp Phe Arg Asp Ala Leu Leu Arg Val Gly Ile Asp Thr Val
            340                 345                 350

Arg Tyr Phe Thr Asn Ser Ile Asp Gly Leu Pro Thr Phe Val Pro Glu
        355                 360                 365

Leu Ile Ser Pro Ala Glu Leu Ala Glu Thr Asn Tyr Asp Ala Leu Ile
    370                 375                 380

Asp Ile Arg Ala Lys Ser Glu Phe Ala Ala Gly Ser Ile Pro Gly Ala
385                 390                 395                 400

Gln Gln Leu Ser Gly Gly Ser Ala Met Trp Arg Leu Asn Glu Leu Pro
                405                 410                 415

Ala Gly Gly Thr Leu Val Thr Phe Cys Gln Ser Gly Ala Arg Asn Thr
            420                 425                 430

Val Val Ala Asn Ala Leu Arg Arg Ala Gly Phe Thr Val Ile Glu Leu
        435                 440                 445

Glu Gly Ser Tyr Ala Ala Trp Glu Lys Ser Ala Ala Asn Pro Lys Asn
    450                 455                 460

Leu Gln Thr Ala Val
465

<210> 135
<211> 675
<212> DNA
<213> Corynebacterium glutamicum

<220>
<221> CDS
<222> (101)..(652)
<223> RXA01853

<400> 135

gaatgtccaa cgtcaaaaaa attcttttct atcctaaccg tatcttcaca ccttgaggga 60

atgatggggg agcgccgctt acgcactaca ctgtttattc atg gag atc ctc gga 115
Met Glu Ile Leu Gly
1               5

ttc gcg gct ggt ccg tat aaa aca aat tgc tat gtg gtg cgc ggg gag 163
Phe Ala Ala Gly Pro Tyr Lys Thr Asn Cys Tyr Val Val Arg Gly Glu
                10                  15                  20

aat gag gtc gcg atc att gat cct ggc atg cat gcc cac gat gat ttg 211
Asn Glu Val Ala Ile Ile Asp Pro Gly Met His Ala His Asp Asp Leu
            25                  30                  35

gtg gaa tac atc acc acg aat aat ttg agc gtg gac aag att gtg ctc 259
Val Glu Tyr Ile Thr Thr Asn Asn Leu Ser Val Asp Lys Ile Val Leu
        40                  45                  50

acc cac gga cac att gat cac acc cgt gat gct ggt gtt gtg gca aag 307
Thr His Gly His Ile Asp His Thr Arg Asp Ala Gly Val Val Ala Lys
    55                  60                  65

cgt ttc aat gcg ccg gtc tat atc cat cct gat gat gcg ttt ttc ctt 355
Arg Phe Asn Ala Pro Val Tyr Ile His Pro Asp Asp Ala Phe Phe Leu
70                  75                  80                  85

gag gtc tac aag gga tct gga aca aag acg gcc atg ctt ttc gac gcc 403
Glu Val Tyr Lys Gly Ser Gly Thr Lys Thr Ala Met Leu Phe Asp Ala
                90                  95                  100

gac aac atg gtg tcc cct gat cct gag tca ctg cgt gat ctg gtt gat 451
Asp Asn Met Val Ser Pro Asp Pro Glu Ser Leu Arg Asp Leu Val Asp
            105                 110                 115

ggt gag aca atc act ttg gct ggc gaa gaa ttc acg ttg aag cat gca 499
Gly Glu Thr Ile Thr Leu Ala Gly Glu Glu Phe Thr Leu Lys His Ala
        120                 125                 130

cca ggg cat tca cct ggt tgt acg ctg atc gtc ggc aag gaa tac tgc 547
Pro Gly His Ser Pro Gly Cys Thr Leu Ile Val Gly Lys Glu Tyr Cys
    135                 140                 145

ttt agc ggt gat gtg ttg ttc aag ggc tct att ggt cgc act gat ttt 595
Phe Ser Gly Asp Val Leu Phe Lys Gly Ser Ile Gly Arg Thr Asp Phe
150                 155                 160                 165

gag tgg tct gat gca gat gcc atg aat gag tca cta cgc acg gca gtg 643
Glu Trp Ser Asp Ala Asp Ala Met Asn Glu Ser Leu Arg Thr Ala Val
                170                 175                 180

ctc cca ctt tgatgatgca ttgcagattc ttc 675
Leu Pro Leu

<210> 136
<211> 184
<212> PRT
<213> Corynebacterium glutamicum

<400> 136

Met Glu Ile Leu Gly Phe Ala Ala Gly Pro Tyr Lys Thr Asn Cys Tyr
1               5                   10                  15

Val Val Arg Gly Glu Asn Glu Val Ala Ile Ile Asp Pro Gly Met His
            20                  25                  30

Ala His Asp Asp Leu Val Glu Tyr Ile Thr Thr Asn Asn Leu Ser Val
        35                  40                  45

Asp Lys Ile Val Leu Thr His Gly His Ile Asp His Thr Arg Asp Ala
    50                  55                  60

Gly Val Val Ala Lys Arg Phe Asn Ala Pro Val Tyr Ile His Pro Asp
65                  70                  75                  80

Asp Ala Phe Phe Leu Glu Val Tyr Lys Gly Ser Gly Thr Lys Thr Ala
                85                  90                  95

Met Leu Phe Asp Ala Asp Asn Met Val Ser Pro Asp Pro Glu Ser Leu
            100                 105                 110

Arg Asp Leu Val Asp Gly Glu Thr Ile Thr Leu Ala Gly Glu Glu Phe
        115                 120                 125

Thr Leu Lys His Ala Pro Gly His Ser Pro Gly Cys Thr Leu Ile Val
    130                 135                 140

Gly Lys Glu Tyr Cys Phe Ser Gly Asp Val Leu Phe Lys Gly Ser Ile
145                 150                 155                 160

Gly Arg Thr Asp Phe Glu Trp Ser Asp Ala Asp Ala Met Asn Glu Ser
                165                 170                 175

Leu Arg Thr Ala Val Leu Pro Leu
            180

<210> 137
<211> 723
<212> DNA
<213> Corynebacterium glutamicum

<220>
<221> CDS
<222> (101)..(700)
<223> RXN02424

<400> 137
agccaatgct caactgccct tcaaaatcga tttttgtcgc ttcaaaaaat acaccttcga 60

acatagcatc gaacaaggtg tttccgctag tctgaaccac atg act aac gag ctc   115
                                           Met Thr Asn Glu Leu
                                           1               5

act ctt cac cat att tcc gtg tcc caa atg gac aac aat tgc tac ctt   163
Thr Leu His His Ile Ser Val Ser Gln Met Asp Asn Asn Cys Tyr Leu
                10                  15                  20

ttg gcc gca aat ggc aac ggt tta ctc att gat gct gca gat gac gca   211
Leu Ala Ala Asn Gly Asn Gly Leu Leu Ile Asp Ala Ala Asp Asp Ala
            25                  30                  35

gct gca cta ctt aaa tta gcc gaa gat gcc ggt gtg acc atc acc aaa    259
Ala Ala Leu Leu Lys Leu Ala Glu Asp Ala Gly Val Thr Ile Thr Lys
        40                  45                  50

gtg ttg acc acc cac cgc cac gca gac cac gtc cgt gca ttg ccg gag    307
Val Leu Thr Thr His Arg His Ala Asp His Val Arg Ala Leu Pro Glu
    55                  60                  65

gtt ctc cag aag acc gga gca act cac tac gcg cct ttc ctt gag gtg    355
Val Leu Gln Lys Thr Gly Ala Thr His Tyr Ala Pro Phe Leu Glu Val
70                  75                  80                  85

cca gct ttg ccc tcc gct gtt gat gtg gaa ctg cat cat ggt gat tca    403
Pro Ala Leu Pro Ser Ala Val Asp Val Glu Leu His His Gly Asp Ser
                90                  95                  100

att gaa ttt gag ggt cat gta ttc cct atc agc att ctg cgc ggc cac    451
Ile Glu Phe Glu Gly His Val Phe Pro Ile Ser Ile Leu Arg Gly His
            105                 110                 115

acc cca ggc ggt gca gta ctc acc gct gag atc gac ggt aaa act cac    499
Thr Pro Gly Gly Ala Val Leu Thr Ala Glu Ile Asp Gly Lys Thr His
        120                 125                 130

ctt ttc gtg ggt gac agc ctc ttc ccc ggc ggt ttg ggc aaa acc agc    547
Leu Phe Val Gly Asp Ser Leu Phe Pro Gly Gly Leu Gly Lys Thr Ser
    135                 140                 145

agc gaa ggc gac ttc gtc cga ctg ttc aac gat gtc aaa gag cgc atc    595
Ser Glu Gly Asp Phe Val Arg Leu Phe Asn Asp Val Lys Glu Arg Ile
150                 155                 160                 165

ttt gac acc tac gac gat gac agc atc gtg tgg cca ggt cac ggc aag    643
Phe Asp Thr Tyr Asp Asp Asp Ser Ile Val Trp Pro Gly His Gly Lys
                170                 175                 180

gaa acc acc ctt gga gcc gag cgt cca cag ctg gaa atc tgg tgg gag    691
Glu Thr Thr Leu Gly Ala Glu Arg Pro Gln Leu Glu Ile Trp Trp Glu
            185                 190                 195

cgt cgc tgg taagcgcttt tctcaaccag gca                              723
Arg Arg Trp
        200


<210> 138
<211> 200
<212> PRT
<213> Corynebacterium glutamicum

<400> 138
Met Thr Asn Glu Leu Thr Leu His His Ile Ser Val Ser Gln Met Asp
 1               5                  10                  15

Asn Asn Cys Tyr Leu Leu Ala Ala Asn Gly Asn Gly Leu Leu Ile Asp
            20                  25                  30

Ala Ala Asp Asp Ala Ala Ala Leu Leu Lys Leu Ala Glu Asp Ala Gly
        35                  40                  45

Val Thr Ile Thr Lys Val Leu Thr Thr His Arg His Ala Asp His Val
50                  55                  60

Arg Ala Leu Pro Glu Val Leu Gln Lys Thr Gly Ala Thr His Tyr Ala
65                  70                  75                  80

Pro Phe Leu Glu Val Pro Ala Leu Pro Ser Ala Val Asp Val Glu Leu
                85                  90                  95

His His Gly Asp Ser Ile Glu Phe Glu Gly His Val Phe Pro Ile Ser
            100                 105                 110

Ile Leu Arg Gly His Thr Pro Gly Gly Ala Val Leu Thr Ala Glu Ile
        115                 120                 125

Asp Gly Lys Thr His Leu Phe Val Gly Asp Ser Leu Phe Pro Gly Gly
    130                 135                 140

Leu Gly Lys Thr Ser Ser Glu Gly Asp Phe Val Arg Leu Phe Asn Asp
145                 150                 155                 160

Val Lys Glu Arg Ile Phe Asp Thr Tyr Asp Asp Asp Ser Ile Val Trp
                165                 170                 175

Pro Gly His Gly Lys Glu Thr Thr Leu Gly Ala Glu Arg Pro Gln Leu
            180                 185                 190

Glu Ile Trp Trp Glu Arg Arg Trp
        195                 200

<210> 139
<211> 404
<212> DNA
<213> Corynebacterium glutamicum

<220>
<221> CDS
<222> (1)..(381)
<223> FRXA02424

<400> 139
acc gga gca act cac tac gcg cct ttc ctt gag gtg cca gct ttg ccc      48
Thr Gly Ala Thr His Tyr Ala Pro Phe Leu Glu Val Pro Ala Leu Pro
  1               5                  10                  15

tcc gct gtt gat gtg gaa ctg cat cat ggt gat tca att gaa ttt gag      96
Ser Ala Val Asp Val Glu Leu His His Gly Asp Ser Ile Glu Phe Glu
             20                  25                  30

ggt cat gta ttc cct atc agc att ctg cgc ggc cac acc cca ggc ggt      144
Gly His Val Phe Pro Ile Ser Ile Leu Arg Gly His Thr Pro Gly Gly
         35                  40                  45

gca gta ctc acc gct gag atc gac ggt aaa act cac ctt ttc gtg ggt      192
Ala Val Leu Thr Ala Glu Ile Asp Gly Lys Thr His Leu Phe Val Gly
     50                  55                  60

gac agc ctc ttc ccc ggc ggt ttg ggc aaa acc agc agc gaa ggc gac      240
Asp Ser Leu Phe Pro Gly Gly Leu Gly Lys Thr Ser Ser Glu Gly Asp
 65                  70                  75                  80

ttc gtc cga ctg ttc aac gat gtc aaa gag cgc atc ttt gac acc tac    288
Phe Val Arg Leu Phe Asn Asp Val Lys Glu Arg Ile Phe Asp Thr Tyr
                85                  90                  95

gac gat gac agc atc gtg tgg cca ggt cac ggc aag gaa acc acc ctt    336
Asp Asp Asp Ser Ile Val Trp Pro Gly His Gly Lys Glu Thr Thr Leu
            100                 105                 110

gga gcc gag cgt cca cag ctg gaa atc tgg tgg gag cgt cgc tgg        381
Gly Ala Glu Arg Pro Gln Leu Glu Ile Trp Trp Glu Arg Arg Trp
        115                 120                 125

taagcgcttt tctcaaccag gca                                          404

<210> 140
<211> 127
<212> PRT
<213> Corynebacterium glutamicum

<400> 140
Thr Gly Ala Thr His Tyr Ala Pro Phe Leu Glu Val Pro Ala Leu Pro
  1               5                  10                  15

Ser Ala Val Asp Val Glu Leu His His Gly Asp Ser Ile Glu Phe Glu
             20                  25                  30

Gly His Val Phe Pro Ile Ser Ile Leu Arg Gly His Thr Pro Gly Gly
         35                  40                  45

Ala Val Leu Thr Ala Glu Ile Asp Gly Lys Thr His Leu Phe Val Gly
     50                  55                  60

Asp Ser Leu Phe Pro Gly Gly Leu Gly Lys Thr Ser Ser Glu Gly Asp
 65                  70                  75                  80

Phe Val Arg Leu Phe Asn Asp Val Lys Glu Arg Ile Phe Asp Thr Tyr
                 85                  90                  95

Asp Asp Asp Ser Ile Val Trp Pro Gly His Gly Lys Glu Thr Thr Leu
            100                 105                 110

Gly Ala Glu Arg Pro Gln Leu Glu Ile Trp Trp Glu Arg Arg Trp
        115                 120                 125

<210> 141
<211> 882
<212> DNA
<213> Corynebacterium glutamicum

<220>
<221> CDS
<222> (101)..(859)
<223> RXN00419

<400> 141
gctggttgaa gactcgaaat gagatcgacc caaccggagt ctttgcatct gacatgtccc 60

gccgacttga gctttcttaa gaaagggctt gaactaaaca atg ctt aac gca gtg   115

Met Leu Asn Ala Val
1               5

ggc aaa gcc caa aac att ctc ctt ctt ggt gga acc tct gag atc ggt     163
Gly Lys Ala Gln Asn Ile Leu Leu Leu Gly Gly Thr Ser Glu Ile Gly
                10                  15                  20

att tcc att gtc tcc cgc ttc ctc aag cag ggt cca tcc cat gtg acc     211
Ile Ser Ile Val Ser Arg Phe Leu Lys Gln Gly Pro Ser His Val Thr
            25                  30                  35

ttg gca gcg cgt aaa gat tcc cca cgc gtg gac gca gca gtc gca gag     259
Leu Ala Ala Arg Lys Asp Ser Pro Arg Val Asp Ala Ala Val Ala Glu
        40                  45                  50

atc aaa gca gct ggc gct gct tcc gtt gct gtt gtt gat ttc gat gcg     307
Ile Lys Ala Ala Gly Ala Ala Ser Val Ala Val Val Asp Phe Asp Ala
    55                  60                  65

ctc gac acc gaa tcc cac cct gca gcc atc gac gca gcc ttt gaa aac     355
Leu Asp Thr Glu Ser His Pro Ala Ala Ile Asp Ala Ala Phe Glu Asn
70                  75                  80                  85

ggc gac gtt gac gta gca atc gtg gct ttc ggc atc ctc ggc gac aac     403
Gly Asp Val Asp Val Ala Ile Val Ala Phe Gly Ile Leu Gly Asp Asn
                90                  95                  100

gaa gca cag tgg cgc gac caa gca cta gca gtg gaa gca acc acc gtg     451
Glu Ala Gln Trp Arg Asp Gln Ala Leu Ala Val Glu Ala Thr Thr Val
            105                 110                 115

aac tac acc gcc ggc gtt tcc gta ggt gta ctg ctg ggc cag aaa ttt     499
Asn Tyr Thr Ala Gly Val Ser Val Gly Val Leu Leu Gly Gln Lys Phe
        120                 125                 130

gag cag cag ggc cac ggc acc atc gtg gca ttg tcc tct gtg gca ggc     547
Glu Gln Gln Gly His Gly Thr Ile Val Ala Leu Ser Ser Val Ala Gly
    135                 140                 145

cag cga gtc cgc cgc tcc aac ttt gtc tac ggc tcc gcc aag gca ggt     595
Gln Arg Val Arg Arg Ser Asn Phe Val Tyr Gly Ser Ala Lys Ala Gly
150                 155                 160                 165

ttc gac ggt ttc tac acc cag ctc ggc gaa gcc ctg cgt gga tcc ggt     643
Phe Asp Gly Phe Tyr Thr Gln Leu Gly Glu Ala Leu Arg Gly Ser Gly
                170                 175                 180

gcc aac gta ttg gtg gtt cgc cca ggc cag gta cgc acc aag atg tcc     691
Ala Asn Val Leu Val Val Arg Pro Gly Gln Val Arg Thr Lys Met Ser
            185                 190                 195

gca gat ggt ggc gaa gcc cca ctg acc gtc aac cgc gaa gac gtg gca     739
Ala Asp Gly Gly Glu Ala Pro Leu Thr Val Asn Arg Glu Asp Val Ala
        200                 205                 210

gat gct gtt tat gat gca gtg gtg aac aag aag gac atc atc ttt gtc     787
Asp Ala Val Tyr Asp Ala Val Val Asn Lys Lys Asp Ile Ile Phe Val
    215                 220                 225

cac cca ctg ttc cag tac gtc tct ttt gcg ttc caa ttc att ccg cga     835
His Pro Leu Phe Gln Tyr Val Ser Phe Ala Phe Gln Phe Ile Pro Arg

230                 235                 240                 245

gca atc ttc cgc aag ctg ccg ttc taacggaagt tacggaagtt acg          882
Ala Ile Phe Arg Lys Leu Pro Phe
                250

<210> 142
<211> 253
<212> PRT
<213> Corynebacterium glutamicum

<400> 142
Met Leu Asn Ala Val Gly Lys Ala Gln Asn Ile Leu Leu Leu Gly Gly
1               5                   10                  15

Thr Ser Glu Ile Gly Ile Ser Ile Val Ser Arg Phe Leu Lys Gln Gly
            20                  25                  30

Pro Ser His Val Thr Leu Ala Ala Arg Lys Asp Ser Pro Arg Val Asp
        35                  40                  45

Ala Ala Val Ala Glu Ile Lys Ala Ala Gly Ala Ala Ser Val Ala Val
    50                  55                  60

Val Asp Phe Asp Ala Leu Asp Thr Glu Ser His Pro Ala Ala Ile Asp
65                  70                  75                  80

Ala Ala Phe Glu Asn Gly Asp Val Asp Val Ala Ile Val Ala Phe Gly
                85                  90                  95

Ile Leu Gly Asp Asn Glu Ala Gln Trp Arg Asp Gln Ala Leu Ala Val
            100                 105                 110

Glu Ala Thr Thr Val Asn Tyr Thr Ala Gly Val Ser Val Gly Val Leu
        115                 120                 125

Leu Gly Gln Lys Phe Glu Gln Gln Gly His Gly Thr Ile Val Ala Leu
    130                 135                 140

Ser Ser Val Ala Gly Gln Arg Val Arg Arg Ser Asn Phe Val Tyr Gly
145                 150                 155                 160

Ser Ala Lys Ala Gly Phe Asp Gly Phe Tyr Thr Gln Leu Gly Glu Ala
                165                 170                 175

Leu Arg Gly Ser Gly Ala Asn Val Leu Val Val Arg Pro Gly Gln Val
            180                 185                 190

Arg Thr Lys Met Ser Ala Asp Gly Gly Glu Ala Pro Leu Thr Val Asn
        195                 200                 205

Arg Glu Asp Val Ala Asp Ala Val Tyr Asp Ala Val Val Asn Lys Lys
    210                 215                 220

Asp Ile Ile Phe Val His Pro Leu Phe Gln Tyr Val Ser Phe Ala Phe
225                 230                 235                 240

Gln Phe Ile Pro Arg Ala Ile Phe Arg Lys Leu Pro Phe
                245                 250

<210> 143
<211> 485
<212> DNA
<213> Corynebacterium glutamicum

<220>
<221> CDS
<222> (1)..(462)
<223> FRXA00419

<400> 143
gac aac gaa gca cag tgg cgc gac caa gca cta gca gtg gaa gca acc 48
Asp Asn Glu Ala Gln Trp Arg Asp Gln Ala Leu Ala Val Glu Ala Thr
1               5                   10                  15

acc gtg aac tac acc gcc ggc gtt tcc gta ggt gta ctg ctg ggc cag 96
Thr Val Asn Tyr Thr Ala Gly Val Ser Val Gly Val Leu Leu Gly Gln
            20                  25                  30

aaa ttt gag cag cag ggc cac ggc acc atc gtg gca ttg tcc tct gtg 144
Lys Phe Glu Gln Gln Gly His Gly Thr Ile Val Ala Leu Ser Ser Val
        35                  40                  45

gca ggc cag cga gtc cgc cgc tcc aac ttt gtc tac ggc tcc gcc aag 192
Ala Gly Gln Arg Val Arg Arg Ser Asn Phe Val Tyr Gly Ser Ala Lys
    50                  55                  60

gca ggt ttc gac ggt ttc tac acc cag ctc ggc gaa gcc ctg cgt gga 240
Ala Gly Phe Asp Gly Phe Tyr Thr Gln Leu Gly Glu Ala Leu Arg Gly
65                  70                  75                  80

tcc ggt gcc aac gta ttg gtg gtt cgc cca ggc cag gta cgc acc aag 288
Ser Gly Ala Asn Val Leu Val Val Arg Pro Gly Gln Val Arg Thr Lys
                85                  90                  95

atg tcc gca gat ggt ggc gaa gcc cca ctg acc gtc aac cgc gaa gac 336
Met Ser Ala Asp Gly Gly Glu Ala Pro Leu Thr Val Asn Arg Glu Asp
            100                 105                 110

gtg gca gat gct gtt tat gat gca gtg gtg aac aag aag gac atc atc 384
Val Ala Asp Ala Val Tyr Asp Ala Val Val Asn Lys Lys Asp Ile Ile
        115                 120                 125

ttt gtc cac cca ctg ttc cag tac gtc tct ttt gcg ttc caa ttc att 432
Phe Val His Pro Leu Phe Gln Tyr Val Ser Phe Ala Phe Gln Phe Ile
    130                 135                 140

ccg cga gca atc ttc cgc aag ctg ccg ttc taacggaagt tacggaagtt 482
Pro Arg Ala Ile Phe Arg Lys Leu Pro Phe
145                 150

acg 485


<210> 144
<211> 154
<212> PRT
<213> Corynebacterium glutamicum

<400> 144

Asp Asn Glu Ala Gln Trp Arg Asp Gln Ala Leu Ala Val Glu Ala Thr
1               5                   10                  15

Thr Val Asn Tyr Thr Ala Gly Val Ser Val Gly Val Leu Leu Gly Gln
            20                  25                  30

Lys Phe Glu Gln Gln Gly His Gly Thr Ile Val Ala Leu Ser Ser Val
        35                  40                  45

Ala Gly Gln Arg Val Arg Arg Ser Asn Phe Val Tyr Gly Ser Ala Lys
    50                  55                  60

Ala Gly Phe Asp Gly Phe Tyr Thr Gln Leu Gly Glu Ala Leu Arg Gly
65                  70                  75                  80

Ser Gly Ala Asn Val Leu Val Val Arg Pro Gly Gln Val Arg Thr Lys
                85                  90                  95

Met Ser Ala Asp Gly Gly Glu Ala Pro Leu Thr Val Asn Arg Glu Asp
            100                 105                 110

Val Ala Asp Ala Val Tyr Asp Ala Val Val Asn Lys Lys Asp Ile Ile
        115                 120                 125

Phe Val His Pro Leu Phe Gln Tyr Val Ser Phe Ala Phe Gln Phe Ile
    130                 135                 140

Pro Arg Ala Ile Phe Arg Lys Leu Pro Phe
145                 150


<210> 145
<211> 259
<212> DNA
<213> Corynebacterium glutamicum

<220>
<221> CDS
<222> (101)..(259)
<223> FRXA00421

<400> 145
gctggttgaa gactcgaaat gagatcgacc caaccggagt ctttgcatct gacatgtccc 60

gccgacttga gctttcttaa gaaagggctt gaactaaaca atg ctt aac gca gtg   115
                                           Met Leu Asn Ala Val
                                           1               5

ggc aaa gcc caa aac att ctc ctt ctt ggt gga acc tct gag atc ggt   163
Gly Lys Ala Gln Asn Ile Leu Leu Leu Gly Gly Thr Ser Glu Ile Gly
                10                  15                  20

att tcc att gtc tcc cgc ttc ctc aag cag ggt cca tcc cat gtg acc   211
Ile Ser Ile Val Ser Arg Phe Leu Lys Gln Gly Pro Ser His Val Thr
            25                  30                  35

ttg gca gcg cgt aaa gat tcc cca cgc gtg gac gca gca gtc gca gag   259
Leu Ala Ala Arg Lys Asp Ser Pro Arg Val Asp Ala Ala Val Ala Glu
        40                  45                  50

<210> 146
<211> 53
<212> PRT
<213> Corynebacterium glutamicum

<400> 146
Met Leu Asn Ala Val Gly Lys Ala Gln Asn Ile Leu Leu Leu Gly Gly
1               5                   10                  15

Thr Ser Glu Ile Gly Ile Ser Ile Val Ser Arg Phe Leu Lys Gln Gly
            20                  25                  30

Pro Ser His Val Thr Leu Ala Ala Arg Lys Asp Ser Pro Arg Val Asp
        35                  40                  45

Ala Ala Val Ala Glu
    50


<210> 147
<211> 861
<212> DNA
<213> Corynebacterium glutamicum

<220>
<221> CDS
<222> (101)..(838)
<223> RXN02923

<400> 147
gcatctacat tgaaggaatc cacggagcgc gcatcgaaga catcgttgtg gtgaatgaag 60

acggttgtga aaccctcaac aaccagccca aggaactgcg ttg agc att ctt ctc   115
                                           Leu Ser Ile Leu Leu
                                           1               5

cta ggc gga acc tct gac att gcc ggt gag att gcc acg ttg acg tgt   163
Leu Gly Gly Thr Ser Asp Ile Ala Gly Glu Ile Ala Thr Leu Thr Cys
                10                  15                  20

cac ggc gaa gac gtt gtt ttg gct gct cgt cga cca gag gct gca cag   211
His Gly Glu Asp Val Val Leu Ala Ala Arg Arg Pro Glu Ala Ala Gln
            25                  30                  35

ggc tta gcg gaa gat ctt cga cag cgc ggt gcc aca tct gtt cat gtt   259
Gly Leu Ala Glu Asp Leu Arg Gln Arg Gly Ala Thr Ser Val His Val
        40                  45                  50

ttg agc ttt gac gcc caa gta cta gac acg cac cga gaa ctt gtg aag   307
Leu Ser Phe Asp Ala Gln Val Leu Asp Thr His Arg Glu Leu Val Lys
    55                  60                  65

aaa aca caa gag cta gct ggc gag att tcc ctt gcc gtg gtt gct ttt   355
Lys Thr Gln Glu Leu Ala Gly Glu Ile Ser Leu Ala Val Val Ala Phe
70                  75                  80                  85

ggc atc ttg gga gac caa gaa cgc gca gaa acc gat gag acc cat gcg   403
Gly Ile Leu Gly Asp Gln Glu Arg Ala Glu Thr Asp Glu Thr His Ala
                90                  95                  100

gta gag atc gcc acc gtg gat tac acc gct cag gtc tcc atg ctc act   451

Val Glu Ile Ala Thr Val Asp Tyr Thr Ala Gln Val Ser Met Leu Thr
            105                 110                 115

gtg ctt gct gat gag ctc cgc gca caa act act cca gca gcg atc gtg 499
Val Leu Ala Asp Glu Leu Arg Ala Gln Thr Thr Pro Ala Ala Ile Val
        120                 125                 130

gcg ttt tcc tcg att gct ggg tgg cgg gcg cgc cgg cct aac tat gtc 547
Ala Phe Ser Ser Ile Ala Gly Trp Arg Ala Arg Arg Pro Asn Tyr Val
    135                 140                 145

tat gga tcc acc aag gct ggt ctc gat gca ttt tgc caa ggg ctt gca 595
Tyr Gly Ser Thr Lys Ala Gly Leu Asp Ala Phe Cys Gln Gly Leu Ala
150                 155                 160                 165

gat agc ctg cat ggg aca cac gtg cga ttg att att gcg cgt cct ggc 643
Asp Ser Leu His Gly Thr His Val Arg Leu Ile Ile Ala Arg Pro Gly
                170                 175                 180

ttt gtt att ggt tct atg acc acg ggg atg aag cct gct ccg atg tcg 691
Phe Val Ile Gly Ser Met Thr Thr Gly Met Lys Pro Ala Pro Met Ser
            185                 190                 195

gtg tat cca cga gat gtt gcc gca gct gtt gtt agt gct tat acc tct 739
Val Tyr Pro Arg Asp Val Ala Ala Ala Val Val Ser Ala Tyr Thr Ser
        200                 205                 210

aag aag agg agc acg acc ttg tgg att ccg gga cgt ctg cgg gtt ctc 787
Lys Lys Arg Ser Thr Thr Leu Trp Ile Pro Gly Arg Leu Arg Val Leu
    215                 220                 225

gcc tgg att atg cgg atg gtt cct cgt ccg gtg tgg cgg aag atg cca 835
Ala Trp Ile Met Arg Met Val Pro Arg Pro Val Trp Arg Lys Met Pro
230                 235                 240                 245

cgc tagatacccg ttcagccctc aca 861
Arg

<210> 148
<211> 246
<212> PRT
<213> Corynebacterium glutamicum

<400> 148
Leu Ser Ile Leu Leu Leu Gly Gly Thr Ser Asp Ile Ala Gly Glu Ile
1               5                   10                  15

Ala Thr Leu Thr Cys His Gly Glu Asp Val Val Leu Ala Ala Arg Arg
            20                  25                  30

Pro Glu Ala Ala Gln Gly Leu Ala Glu Asp Leu Arg Gln Arg Gly Ala
        35                  40                  45

Thr Ser Val His Val Leu Ser Phe Asp Ala Gln Val Leu Asp Thr His
    50                  55                  60

Arg Glu Leu Val Lys Lys Thr Gln Glu Leu Ala Gly Glu Ile Ser Leu
65                  70                  75                  80

Ala Val Val Ala Phe Gly Ile Leu Gly Asp Gln Glu Arg Ala Glu Thr
                85                  90                  95

Asp Glu Thr His Ala Val Glu Ile Ala Thr Val Asp Tyr Thr Ala Gln
            100                 105                 110

Val Ser Met Leu Thr Val Leu Ala Asp Glu Leu Arg Ala Gln Thr Thr
        115                 120                 125

Pro Ala Ala Ile Val Ala Phe Ser Ser Ile Ala Gly Trp Arg Ala Arg
    130                 135                 140

Arg Pro Asn Tyr Val Tyr Gly Ser Thr Lys Ala Gly Leu Asp Ala Phe
145                 150                 155                 160

Cys Gln Gly Leu Ala Asp Ser Leu His Gly Thr His Val Arg Leu Ile
                165                 170                 175

Ile Ala Arg Pro Gly Phe Val Ile Gly Ser Met Thr Thr Gly Met Lys
            180                 185                 190

Pro Ala Pro Met Ser Val Tyr Pro Arg Asp Val Ala Ala Ala Val Val
        195                 200                 205

Ser Ala Tyr Thr Ser Lys Lys Arg Ser Thr Thr Leu Trp Ile Pro Gly
    210                 215                 220

Arg Leu Arg Val Leu Ala Trp Ile Met Arg Met Val Pro Arg Pro Val
225                 230                 235                 240

Trp Arg Lys Met Pro Arg
                245


<210> 149
<211> 1203
<212> DNA
<213> Corynebacterium glutamicum

<220>
<221> CDS
<222> (101)..(1180)
<223> RXN02922

<400> 149
cccaccgcgg cgcaagctac cgcctaggcg ctcggaactc caccgccact attgatctca 60

gttccatatc cgcccaacta gtttcccagg gagcccactc atg atc tca ccg caa   115
                                            Met Ile Ser Pro Gln
                                            1               5

aca atc atc gac aat ctt gct cca gtc ctc gcg gag atc gca gca acg   163
Thr Ile Ile Asp Asn Leu Ala Pro Val Leu Ala Glu Ile Ala Ala Thr
                10                  15                  20

gct gcg cag cgc gaa cag gat cga gaa ttc agc cgt gat ttg gca aag   211
Ala Ala Gln Arg Glu Gln Asp Arg Glu Phe Ser Arg Asp Leu Ala Lys
            25                  30                  35

cag ctt agt gcc ggt ggg ttc act aaa ctc cgc att cca gtt gaa ttt   259
Gln Leu Ser Ala Gly Gly Phe Thr Lys Leu Arg Ile Pro Val Glu Phe

40                  45                  50

ggc ggt ttg ggt ttt agc ctt cca gaa gca ttt gag gtg ctg gtg gca    307
Gly Gly Leu Gly Phe Ser Leu Pro Glu Ala Phe Glu Val Leu Val Ala
    55                  60                  65

gcg gcc gct gca gat tca aat atc gcg cag gga ttg cgg cct cac ttc    355
Ala Ala Ala Ala Asp Ser Asn Ile Ala Gln Gly Leu Arg Pro His Phe
70                  75                  80                  85

ttg gca gtg gag agc tta ttg atc gct cct tat tca gag cac cgc acc    403
Leu Ala Val Glu Ser Leu Leu Ile Ala Pro Tyr Ser Glu His Arg Thr
                90                  95                  100

aag tgg ctg cga aaa atc gct gag aaa ggc gtg gtc att ggc aat gcg    451
Lys Trp Leu Arg Lys Ile Ala Glu Lys Gly Val Val Ile Gly Asn Ala
            105                 110                 115

ttg act gaa gtg gga aac aag cca ggt gag ctg aaa acc aag atc cga    499
Leu Thr Glu Val Gly Asn Lys Pro Gly Glu Leu Lys Thr Lys Ile Arg
        120                 125                 130

aag gaa ggc gag tct tac gtt ctc agc gac acc aag ttc tat tcc acg    547
Lys Glu Gly Glu Ser Tyr Val Leu Ser Asp Thr Lys Phe Tyr Ser Thr
    135                 140                 145

ggc agt ctt tac gcc gac tgg att caa gta cat gcg aaa gat gag gaa    595
Gly Ser Leu Tyr Ala Asp Trp Ile Gln Val His Ala Lys Asp Glu Glu
150                 155                 160                 165

gat caa gat gtt ttc gcc ttt gtt gat cgc gac gca tca ggc gtt gtt    643
Asp Gln Asp Val Phe Ala Phe Val Asp Arg Asp Ala Ser Gly Val Val
                170                 175                 180

ttg gtg gat tac tgg gac gaa ttt ggg caa caa cta ttt gcc tcc gga    691
Leu Val Asp Tyr Trp Asp Glu Phe Gly Gln Gln Leu Phe Ala Ser Gly
            185                 190                 195

acc agc ttc ttt gaa aaa gtc gtg gta gat cca ctg gac att gtc acc    739
Thr Ser Phe Phe Glu Lys Val Val Val Asp Pro Leu Asp Ile Val Thr
        200                 205                 210

cgt gat tac acc gca ccc agc gct ttc cag gcg ctg gcg cag tca cat    787
Arg Asp Tyr Thr Ala Pro Ser Ala Phe Gln Ala Leu Ala Gln Ser His
    215                 220                 225

cat ttg tct acg ttg act ggt att tcg cag gca atc acc cgt gac att    835
His Leu Ser Thr Leu Thr Gly Ile Ser Gln Ala Ile Thr Arg Asp Ile
230                 235                 240                 245

gtt acc tat gta caa aac cgc acc cgt att ttc agc cac gga agt ggt    883
Val Thr Tyr Val Gln Asn Arg Thr Arg Ile Phe Ser His Gly Ser Gly
                250                 255                 260

gac ctt cca cgc ttt gat ccg cag gtg caa cag gtg gtg ggt gag gtg    931
Asp Leu Pro Arg Phe Asp Pro Gln Val Gln Gln Val Val Gly Glu Val
            265                 270                 275

aaa gcc aag tcg tat gca gtc gag aaa atc ttt cag ggt ttc gca caa    979
Lys Ala Lys Ser Tyr Ala Val Glu Lys Ile Phe Gln Gly Phe Ala Gln
        280                 285                 290

gaa ctg gat ctt gtc gtc gat aag gca aaa gcc ggc act gct acg gag     1027
Glu Leu Asp Leu Val Val Asp Lys Ala Lys Ala Gly Thr Ala Thr Glu
    295                 300                 305

gtc gat ctg gcc gct gtc gac ctg agc gcc tac caa gcc cag tta gcg     1075
Val Asp Leu Ala Ala Val Asp Leu Ser Ala Tyr Gln Ala Gln Leu Ala
310                 315                 320                 325

gtg gca cct ttg gtg ctg agc cag gcc acc cag gcc ttt gag gtt ggc     1123
Val Ala Pro Leu Val Leu Ser Gln Ala Thr Gln Ala Phe Glu Val Gly
                330                 335                 340

gca gat gcc tta aac ggc ggc cac gtg gct gcc caa tac acg atc gga     1171
Ala Asp Ala Leu Asn Gly Gly His Val Ala Ala Gln Tyr Thr Ile Gly
            345                 350                 355

tcc ttg tca tgagccaaga aattttgagc cat                               1203
Ser Leu Ser
        360

<210> 150
<211> 360
<212> PRT
<213> Corynebacterium glutamicum

<400> 150
Met Ile Ser Pro Gln Thr Ile Ile Asp Asn Leu Ala Pro Val Leu Ala
1               5                   10                  15

Glu Ile Ala Ala Thr Ala Ala Gln Arg Glu Gln Asp Arg Glu Phe Ser
            20                  25                  30

Arg Asp Leu Ala Lys Gln Leu Ser Ala Gly Gly Phe Thr Lys Leu Arg
        35                  40                  45

Ile Pro Val Glu Phe Gly Gly Leu Gly Phe Ser Leu Pro Glu Ala Phe
    50                  55                  60

Glu Val Leu Val Ala Ala Ala Ala Ala Asp Ser Asn Ile Ala Gln Gly
65                  70                  75                  80

Leu Arg Pro His Phe Leu Ala Val Glu Ser Leu Leu Ile Ala Pro Tyr
                85                  90                  95

Ser Glu His Arg Thr Lys Trp Leu Arg Lys Ile Ala Glu Lys Gly Val
            100                 105                 110

Val Ile Gly Asn Ala Leu Thr Glu Val Gly Asn Lys Pro Gly Glu Leu
        115                 120                 125

Lys Thr Lys Ile Arg Lys Glu Gly Glu Ser Tyr Val Leu Ser Asp Thr
    130                 135                 140

Lys Phe Tyr Ser Thr Gly Ser Leu Tyr Ala Asp Trp Ile Gln Val His
145                 150                 155                 160

Ala Lys Asp Glu Glu Asp Gln Asp Val Phe Ala Phe Val Asp Arg Asp
                165                 170                 175

Ala Ser Gly Val Val Leu Val Asp Tyr Trp Asp Glu Phe Gly Gln Gln
            180                 185                 190

Leu Phe Ala Ser Gly Thr Ser Phe Phe Glu Lys Val Val Val Asp Pro
        195                 200                 205

Leu Asp Ile Val Thr Arg Asp Tyr Thr Ala Pro Ser Ala Phe Gln Ala
    210                 215                 220

Leu Ala Gln Ser His His Leu Ser Thr Leu Thr Gly Ile Ser Gln Ala
225                 230                 235                 240

Ile Thr Arg Asp Ile Val Thr Tyr Val Gln Asn Arg Thr Arg Ile Phe
                245                 250                 255

Ser His Gly Ser Gly Asp Leu Pro Arg Phe Asp Pro Gln Val Gln Gln
            260                 265                 270

Val Val Gly Glu Val Lys Ala Lys Ser Tyr Ala Val Glu Lys Ile Phe
        275                 280                 285

Gln Gly Phe Ala Gln Glu Leu Asp Leu Val Val Asp Lys Ala Lys Ala
    290                 295                 300

Gly Thr Ala Thr Glu Val Asp Leu Ala Ala Val Asp Leu Ser Ala Tyr
305                 310                 315                 320

Gln Ala Gln Leu Ala Val Ala Pro Leu Val Leu Ser Gln Ala Thr Gln
                325                 330                 335

Ala Phe Glu Val Gly Ala Asp Ala Leu Asn Gly Gly His Val Ala Ala
            340                 345                 350

Gln Tyr Thr Ile Gly Ser Leu Ser
        355                 360


<210> 151
<211> 516
<212> DNA
<213> Corynebacterium glutamicum

<220>
<221> CDS
<222> (101)..(493)
<223> RXN03065

<400> 151
gacggcgcca actctgcatt cttcgcctca gcgtgcgtgg cagtgtttgc actgatcgtg 60

ggcttctttg taaagaggcc agcccgctaa gctaggtcgc atg atc agc att gga   115
                                            Met Ile Ser Ile Gly
                                            1               5

acc gac ctc gtt cac att tcg gcg ttc gca gag cag ctt gca cag ccg   163
Thr Asp Leu Val His Ile Ser Ala Phe Ala Glu Gln Leu Ala Gln Pro
                10                  15                  20

gga agt tcc ttc atg gag gtg ttt tcc gca ggg gag cgt cgt aaa gca   211
Gly Ser Ser Phe Met Glu Val Phe Ser Ala Gly Glu Arg Arg Lys Ala
            25                  30                  35

aat gaa cgc cag gca agc cgc tac gcc gag cat ttg gcg ggg cgg tgg 259
Asn Glu Arg Gln Ala Ser Arg Tyr Ala Glu His Leu Ala Gly Arg Trp
        40                  45                  50

gcg gca aaa gaa tcc ttt att aag gcc tgg tca cag gcc att tat ggc 307
Ala Ala Lys Glu Ser Phe Ile Lys Ala Trp Ser Gln Ala Ile Tyr Gly
    55                  60                  65

cag cca ccc gtg atc gcc gaa gaa gcc gtg gtg tgg cgg gac atc gaa 355
Gln Pro Pro Val Ile Ala Glu Glu Ala Val Val Trp Arg Asp Ile Glu
70                  75                  80                  85

gtt cgc gca gac gcg tgg ggg cgc gtc gcg atc gaa ttg gcg ccc gaa 403
Val Arg Ala Asp Ala Trp Gly Arg Val Ala Ile Glu Leu Ala Pro Glu
                90                  95                  100

ttg gcc gca gtg gtc cgg gaa tcc atc ggg gag ttt tcc agc agc ttg 451
Leu Ala Ala Val Val Arg Glu Ser Ile Gly Glu Phe Ser Ser Ser Leu
            105                 110                 115

agc atc agc cac gac ggc gac tac gcg gtg gaa cgt gcg tgt 493
Ser Ile Ser His Asp Gly Asp Tyr Ala Val Glu Arg Ala Cys
        120                 125                 130

tgactatcca gtagccacgg aga 516

<210> 152
<211> 131
<212> PRT
<213> Corynebacterium glutamicum

<400> 152
Met Ile Ser Ile Gly Thr Asp Leu Val His Ile Ser Ala Phe Ala Glu
1               5                   10                  15

Gln Leu Ala Gln Pro Gly Ser Ser Phe Met Glu Val Phe Ser Ala Gly
            20                  25                  30

Glu Arg Arg Lys Ala Asn Glu Arg Gln Ala Ser Arg Tyr Ala Glu His
        35                  40                  45

Leu Ala Gly Arg Trp Ala Ala Lys Glu Ser Phe Ile Lys Ala Trp Ser
    50                  55                  60

Gln Ala Ile Tyr Gly Gln Pro Pro Val Ile Ala Glu Glu Ala Val Val
65                  70                  75                  80

Trp Arg Asp Ile Glu Val Arg Ala Asp Ala Trp Gly Arg Val Ala Ile
                85                  90                  95

Glu Leu Ala Pro Glu Leu Ala Ala Val Val Arg Glu Ser Ile Gly Glu
            100                 105                 110

Phe Ser Ser Ser Leu Ser Ile Ser His Asp Gly Asp Tyr Ala Val Glu
        115                 120                 125

Arg Ala Cys
    130

<210> 153
<211> 543
<212> DNA
<213> Corynebacterium glutamicum

<220>
<221> CDS
<222> (101)..(520)
<223> RXN03132

<400> 153
taacttattt atacataaaa acacaataaa aaccgcacac gcggcgaaac ttcaataaat 60

ccctagactg caaaaaatgg ttaggctaac cttatttcca atg aat gtg aat caa 115
Met Asn Val Asn Gln
1               5

cag ttg gga gcc cgc acc gcc atg aac cac cct gag acc gca acc gtc 163
Gln Leu Gly Ala Arg Thr Ala Met Asn His Pro Glu Thr Ala Thr Val
                10                  15                  20

ctt cgc tcc atc tct gac atg gtt agc acc gaa acc aat ccg cgc cgt 211
Leu Arg Ser Ile Ser Asp Met Val Ser Thr Glu Thr Asn Pro Arg Arg
            25                  30                  35

aaa agc cgc ctc gag cag ctc att tac gcc acc gcc agc gcc tgg ccc 259
Lys Ser Arg Leu Glu Gln Leu Ile Tyr Ala Thr Ala Ser Ala Trp Pro
        40                  45                  50

cac tac ccc att gcc cac gca gcc cag gcc gcg gtg caa tta gcc agg 307
His Tyr Pro Ile Ala His Ala Ala Gln Ala Ala Val Gln Leu Ala Arg
    55                  60                  65

cca atg cgg gtt ttt gag ctc cag agc ttt gaa ggc gtc aag cac gca 355
Pro Met Arg Val Phe Glu Leu Gln Ser Phe Glu Gly Val Lys His Ala
70                  75                  80                  85

ctc cac cac atc gat ctt cgc ccc gcc ctc gaa tgg gac atc atg gga 403
Leu His His Ile Asp Leu Arg Pro Ala Leu Glu Trp Asp Ile Met Gly
                90                  95                  100

ttt ccc gaa tcc ccc gac acg ctg ccc att ttg ctc agt gac ctg cgc 451
Phe Pro Glu Ser Pro Asp Thr Leu Pro Ile Leu Leu Ser Asp Leu Arg
            105                 110                 115

gac ccc cct tac gcc acc act gct gtg cca cct caa tcc acg cca cgc 499
Asp Pro Pro Tyr Ala Thr Thr Ala Val Pro Pro Gln Ser Thr Pro Arg
        120                 125                 130

tcc gct cac cga tcc aca cat tgacgcttcc aggcgccacc gcg 543
Ser Ala His Arg Ser Thr His
    135                 140


<210> 154
<211> 140
<212> PRT
<213> Corynebacterium glutamicum

<400> 154

Met Asn Val Asn Gln Gln Leu Gly Ala Arg Thr Ala Met Asn His Pro
1               5                   10                  15

Glu Thr Ala Thr Val Leu Arg Ser Ile Ser Asp Met Val Ser Thr Glu
            20                  25                  30

Thr Asn Pro Arg Arg Lys Ser Arg Leu Glu Gln Leu Ile Tyr Ala Thr
        35                  40                  45

Ala Ser Ala Trp Pro His Tyr Pro Ile Ala His Ala Ala Gln Ala Ala
    50                  55                  60

Val Gln Leu Ala Arg Pro Met Arg Val Phe Glu Leu Gln Ser Phe Glu
65                  70                  75                  80

Gly Val Lys His Ala Leu His His Ile Asp Leu Arg Pro Ala Leu Glu
                85                  90                  95

Trp Asp Ile Met Gly Phe Pro Glu Ser Pro Asp Thr Leu Pro Ile Leu
            100                 105                 110

Leu Ser Asp Leu Arg Asp Pro Pro Tyr Ala Thr Thr Ala Val Pro Pro
        115                 120                 125

Gln Ser Thr Pro Arg Ser Ala His Arg Ser Thr His
    130                 135                 140


<210> 155
<211> 461
<212> DNA
<213> Corynebacterium glutamicum

<220>
<221> CDS
<222> (1)..(438)
<223> RXN03157

<400> 155
ggc cac ctc gac atc gtg acc cgc gcc gcc gcg caa ttt agc gaa gtc      48
Gly His Leu Asp Ile Val Thr Arg Ala Ala Ala Gln Phe Ser Glu Val
1               5                   10                  15

acc atc ttg gtc acc gcc aac ccc aac aaa aac tca gga ctg ttc acc      96
Thr Ile Leu Val Thr Ala Asn Pro Asn Lys Asn Ser Gly Leu Phe Thr
            20                  25                  30

gtc gca gaa cgc atg gat ctc atc cgc gaa tcc acc gca cac ctg gac      144
Val Ala Glu Arg Met Asp Leu Ile Arg Glu Ser Thr Ala His Leu Asp
        35                  40                  45

aac gta aaa gta gac act tgg gcc tca ctg ctc gtg gat tac acc acc      192
Asn Val Lys Val Asp Thr Trp Ala Ser Leu Leu Val Asp Tyr Thr Thr
    50                  55                  60

gaa cat ggc atc ggc gcc cta gtc aaa ggc ctg cga agc tcc ctc gac      240
Glu His Gly Ile Gly Ala Leu Val Lys Gly Leu Arg Ser Ser Leu Asp
65                  70                  75                  80

tac gaa tac gag ctc ccc atg gca cag atg aac agg cga ctc acg ggc      288
Tyr Glu Tyr Glu Leu Pro Met Ala Gln Met Asn Arg Arg Leu Thr Gly

                85                  90                  95

gta gac acc ttc ttc ctc ctc acc gac gag aaa tac ggc tac gtc agc 336
Val Asp Thr Phe Phe Leu Leu Thr Asp Glu Lys Tyr Gly Tyr Val Ser
            100                 105                 110

tcc aca ctg tgc aaa gaa gtt gcc cgc ttc ggt ggc gat gtc tcc ggc 384
Ser Thr Leu Cys Lys Glu Val Ala Arg Phe Gly Gly Asp Val Ser Gly
        115                 120                 125

ctc cta cct gag gta gtg gca aaa gcc gtg aca gaa aaa tac agc aac 432
Leu Leu Pro Glu Val Val Ala Lys Ala Val Thr Glu Lys Tyr Ser Asn
    130                 135                 140

cag cac tagtttcttt tgcatcaagc gtc 461
Gln His
145

<210> 156
<211> 146
<212> PRT
<213> Corynebacterium glutamicum

<400> 156
Gly His Leu Asp Ile Val Thr Arg Ala Ala Ala Gln Phe Ser Glu Val
1               5                   10                  15

Thr Ile Leu Val Thr Ala Asn Pro Asn Lys Asn Ser Gly Leu Phe Thr
            20                  25                  30

Val Ala Glu Arg Met Asp Leu Ile Arg Glu Ser Thr Ala His Leu Asp
        35                  40                  45

Asn Val Lys Val Asp Thr Trp Ala Ser Leu Leu Val Asp Tyr Thr Thr
    50                  55                  60

Glu His Gly Ile Gly Ala Leu Val Lys Gly Leu Arg Ser Ser Leu Asp
65                  70                  75                  80

Tyr Glu Tyr Glu Leu Pro Met Ala Gln Met Asn Arg Arg Leu Thr Gly
                85                  90                  95

Val Asp Thr Phe Phe Leu Leu Thr Asp Glu Lys Tyr Gly Tyr Val Ser
            100                 105                 110

Ser Thr Leu Cys Lys Glu Val Ala Arg Phe Gly Gly Asp Val Ser Gly
        115                 120                 125

Leu Leu Pro Glu Val Val Ala Lys Ala Val Thr Glu Lys Tyr Ser Asn
    130                 135                 140

Gln His
145

<210> 157
<211> 1206
<212> DNA
<213> Corynebacterium glutamicum

<220>
<221> CDS
<222> (101)..(1183)
<223> RXN00934

<400> 157
ccaacccctg tggtttggtg atttggatcc ggagcgtctc aagcgctcta gggagcagac 60

aaatgttcac aaaccggtgg cattacagga ggacaattag gtg cga att gga atg 115
Val Arg Ile Gly Met
1               5

gtc tgc ccg tac tcc ttc gat gag ccg ggc ggt gtt caa gcg cat atc 163
Val Cys Pro Tyr Ser Phe Asp Glu Pro Gly Gly Val Gln Ala His Ile
                10                  15                  20

ctt gac tta gcg cga acc ttc att gcc caa ggc cat gag gtt cag gtg 211
Leu Asp Leu Ala Arg Thr Phe Ile Ala Gln Gly His Glu Val Gln Val
            25                  30                  35

ctt ggt ccg tgt agt gcg gat acg cag gtg ccc gat ttc gtg gtg cgc 259
Leu Gly Pro Cys Ser Ala Asp Thr Gln Val Pro Asp Phe Val Val Arg
        40                  45                  50

ggt ggt ggc agc atc ccg att ccg tac aat ggc tcg gtt gcc cgc ttg 307
Gly Gly Gly Ser Ile Pro Ile Pro Tyr Asn Gly Ser Val Ala Arg Leu
    55                  60                  65

agc ttt ggg ccg aaa atg ttc aag gcc gtg cgc acg ttc ctc cgc gaa 355
Ser Phe Gly Pro Lys Met Phe Lys Ala Val Arg Thr Phe Leu Arg Glu
70                  75                  80                  85

ggc aac ttc gat gtg ctg cat atc cat gaa ccg aat tca cca agt ttt 403
Gly Asn Phe Asp Val Leu His Ile His Glu Pro Asn Ser Pro Ser Phe
                90                  95                  100

tcc atg gcg gcg cta cgc ttt gcg gaa ggc ccc atc gtt gct act tac 451
Ser Met Ala Ala Leu Arg Phe Ala Glu Gly Pro Ile Val Ala Thr Tyr
            105                 110                 115

cac gcc tcc agt agc gga tcg aag ctg ctc aag gct ttc tta cca gtg 499
His Ala Ser Ser Ser Gly Ser Lys Leu Leu Lys Ala Phe Leu Pro Val
        120                 125                 130

ctt tcg ccc atg ctg gag aaa gtg cgc gca ggc atc gcc gtg tct gaa 547
Leu Ser Pro Met Leu Glu Lys Val Arg Ala Gly Ile Ala Val Ser Glu
    135                 140                 145

atg gct cgg cgc tgg cag gtg gag caa gtc ggc ggc gat ccc gtg ctg 595
Met Ala Arg Arg Trp Gln Val Glu Gln Val Gly Gly Asp Pro Val Leu
150                 155                 160                 165

atc ccc aac ggg gta gag acc tcc atg ttc aaa gcc gcg cgc caa atc 643
Ile Pro Asn Gly Val Glu Thr Ser Met Phe Lys Ala Ala Arg Gln Ile
                170                 175                 180

gaa ccg aat gat cct gta gag atc gtc ttt ttg ggt cgc ctc gat gag 691
Glu Pro Asn Asp Pro Val Glu Ile Val Phe Leu Gly Arg Leu Asp Glu
            185                 190                 195

tcc cgc aaa ggc ctc gac atc ctc ctg cgc gct ctg acc agg ctg gat 739

Ser Arg Lys Gly Leu Asp Ile Leu Leu Arg Ala Leu Thr Arg Leu Asp
        200                 205                 210

cgc ccg ttt acc tgc acc gtc att ggc ggc ggc acc ccg cga gaa gtc      787
Arg Pro Phe Thr Cys Thr Val Ile Gly Gly Gly Thr Pro Arg Glu Val
    215                 220                 225

gcc ggc atc aac ttt gtg ggc cgc gtc agc gat gag gaa aag gca gca      835
Ala Gly Ile Asn Phe Val Gly Arg Val Ser Asp Glu Glu Lys Ala Ala
230                 235                 240                 245

atc tta ggt cgc gca gac atc tat gtc gca ccc aac acc ggc ggc gaa      883
Ile Leu Gly Arg Ala Asp Ile Tyr Val Ala Pro Asn Thr Gly Gly Glu
                250                 255                 260

agc ttc ggc atc gtg cta gtt gaa gcg atg gcc gcg gga tgc gct gtc      931
Ser Phe Gly Ile Val Leu Val Glu Ala Met Ala Ala Gly Cys Ala Val
            265                 270                 275

gtc gcc agc gac cta gaa gcg ttc tcc ctg gtc acc gat tct gaa gcc      979
Val Ala Ser Asp Leu Glu Ala Phe Ser Leu Val Thr Asp Ser Glu Ala
        280                 285                 290

gca cag cca gcg ggc gtg cta ttt aaa acc ggc tca gac gcc gac cta      1027
Ala Gln Pro Ala Gly Val Leu Phe Lys Thr Gly Ser Asp Ala Asp Leu
    295                 300                 305

gcc aaa aaa ctt caa gcg ctt atc gac gac ccc tcc tcc cgt tcc acg      1075
Ala Lys Lys Leu Gln Ala Leu Ile Asp Asp Pro Ser Ser Arg Ser Thr
310                 315                 320                 325

ctt atc gcc gcg ggg cta aag cgc gca aac gcc tac gac tgg tcg aca      1123
Leu Ile Ala Ala Gly Leu Lys Arg Ala Asn Ala Tyr Asp Trp Ser Thr
                330                 335                 340

gta tcc acc cag gtc atg gca gtc tat gaa acc att gcg atc gac aaa      1171
Val Ser Thr Gln Val Met Ala Val Tyr Glu Thr Ile Ala Ile Asp Lys
            345                 350                 355

gtg agg ctt gga tgacccttgt ttacctcctc atc                            1206
Val Arg Leu Gly
        360

<210> 158
<211> 361
<212> PRT
<213> Corynebacterium glutamicum

<400> 158
Val Arg Ile Gly Met Val Cys Pro Tyr Ser Phe Asp Glu Pro Gly Gly
1               5                   10                  15

Val Gln Ala His Ile Leu Asp Leu Ala Arg Thr Phe Ile Ala Gln Gly
            20                  25                  30

His Glu Val Gln Val Leu Gly Pro Cys Ser Ala Asp Thr Gln Val Pro
        35                  40                  45

Asp Phe Val Val Arg Gly Gly Gly Ser Ile Pro Ile Pro Tyr Asn Gly
    50                  55                  60

Ser Val Ala Arg Leu Ser Phe Gly Pro Lys Met Phe Lys Ala Val Arg
65                  70                  75                  80

Thr Phe Leu Arg Glu Gly Asn Phe Asp Val Leu His Ile His Glu Pro
                85                  90                  95

Asn Ser Pro Ser Phe Ser Met Ala Ala Leu Arg Phe Ala Glu Gly Pro
            100                 105                 110

Ile Val Ala Thr Tyr His Ala Ser Ser Ser Gly Ser Lys Leu Leu Lys
        115                 120                 125

Ala Phe Leu Pro Val Leu Ser Pro Met Leu Glu Lys Val Arg Ala Gly
    130                 135                 140

Ile Ala Val Ser Glu Met Ala Arg Arg Trp Gln Val Glu Gln Val Gly
145                 150                 155                 160

Gly Asp Pro Val Leu Ile Pro Asn Gly Val Glu Thr Ser Met Phe Lys
                165                 170                 175

Ala Ala Arg Gln Ile Glu Pro Asn Asp Pro Val Glu Ile Val Phe Leu
            180                 185                 190

Gly Arg Leu Asp Glu Ser Arg Lys Gly Leu Asp Ile Leu Leu Arg Ala
        195                 200                 205

Leu Thr Arg Leu Asp Arg Pro Phe Thr Cys Thr Val Ile Gly Gly Gly
    210                 215                 220

Thr Pro Arg Glu Val Ala Gly Ile Asn Phe Val Gly Arg Val Ser Asp
225                 230                 235                 240

Glu Glu Lys Ala Ala Ile Leu Gly Arg Ala Asp Ile Tyr Val Ala Pro
                245                 250                 255

Asn Thr Gly Gly Glu Ser Phe Gly Ile Val Leu Val Glu Ala Met Ala
            260                 265                 270

Ala Gly Cys Ala Val Val Ala Ser Asp Leu Glu Ala Phe Ser Leu Val
        275                 280                 285

Thr Asp Ser Glu Ala Ala Gln Pro Ala Gly Val Leu Phe Lys Thr Gly
    290                 295                 300

Ser Asp Ala Asp Leu Ala Lys Lys Leu Gln Ala Leu Ile Asp Asp Pro
305                 310                 315                 320

Ser Ser Arg Ser Thr Leu Ile Ala Ala Gly Leu Lys Arg Ala Asn Ala
                325                 330                 335

Tyr Asp Trp Ser Thr Val Ser Thr Gln Val Met Ala Val Tyr Glu Thr
            340                 345                 350

Ile Ala Ile Asp Lys Val Arg Leu Gly
        355                 360

<210> 159
<211> 1320

<212> DNA
<213> Corynebacterium glutamicum

<220>
<221> CDS
<222> (101)..(1297)
<223> RXN00792

<400> 159
cacctttggt gctgagccag gccacccagg cctttgaggt tggcgcagat gccttaaacg 60

gcggccacgt ggctgcccaa tacacgatcg gatccttgtc atg agc caa gaa att 115
Met Ser Gln Glu Ile
1               5

ttg agc cat ttt gca ccc gca tta gag cgc att cga agc ggc gcc gtc 163
Leu Ser His Phe Ala Pro Ala Leu Glu Arg Ile Arg Ser Gly Ala Val
                10                  15                  20

gag cgc gaa cag cag cgc gcc ttg cca gtg gaa gag att aaa gag ctg 211
Glu Arg Glu Gln Gln Arg Ala Leu Pro Val Glu Glu Ile Lys Glu Leu
            25                  30                  35

gta gag cta ggt ttt act ggg ctt cga gtg ccc gaa gaa cta ggc ggt 259
Val Glu Leu Gly Phe Thr Gly Leu Arg Val Pro Glu Glu Leu Gly Gly
        40                  45                  50

gcg ggc gct tcc ctg gaa agc gta gtt gag tta ctg atc gag atc gcg 307
Ala Gly Ala Ser Leu Glu Ser Val Val Glu Leu Leu Ile Glu Ile Ala
    55                  60                  65

ggc gcc gat tcc aat atc gcc caa gcc ctg cgc gga cat ttt gcc ttc 355
Gly Ala Asp Ser Asn Ile Ala Gln Ala Leu Arg Gly His Phe Ala Phe
70                  75                  80                  85

gtg gaa cta ctc ctg gaa gcg ccg gag agc gaa ttc cgc acc cat tgg 403
Val Glu Leu Leu Leu Glu Ala Pro Glu Ser Glu Phe Arg Thr His Trp
                90                  95                  100

ctg cgc gaa gtc gcc acc gga aga ctt gtg ggc aac gcc gaa agt gag 451
Leu Arg Glu Val Ala Thr Gly Arg Leu Val Gly Asn Ala Glu Ser Glu
            105                 110                 115

aaa cgc ggc gtt tac ggc gat ccg cag acc ttc atc gat gag gtg gag 499
Lys Arg Gly Val Tyr Gly Asp Pro Gln Thr Phe Ile Asp Glu Val Glu
        120                 125                 130

act gaa aac gga ccg att ttc gtg ctc aac ggc act aag ttt tat acc 547
Thr Glu Asn Gly Pro Ile Phe Val Leu Asn Gly Thr Lys Phe Tyr Thr
    135                 140                 145

acc ggc acc tat ttt gcg gac tac acc tgg acc acc gcg ctg ctg cgc 595
Thr Gly Thr Tyr Phe Ala Asp Tyr Thr Trp Thr Thr Ala Leu Leu Arg
150                 155                 160                 165

aac ctt aac ggc caa gaa act ttg gtc agt ttg ccg gtc gat ctg cac 643
Asn Leu Asn Gly Gln Glu Thr Leu Val Ser Leu Pro Val Asp Leu His
                170                 175                 180

gcg ccg ggc gtg gat gtt gct gat gat tgg agc ggg ttt ggg caa aag 691
Ala Pro Gly Val Asp Val Ala Asp Asp Trp Ser Gly Phe Gly Gln Lys

                185                 190                 195

ctc acc gcc tct gga acg acc acg ttt aaa gac ctg gag gtg gat ccg     739
Leu Thr Ala Ser Gly Thr Thr Thr Phe Lys Asp Leu Glu Val Asp Pro
        200                 205                 210

cgg tgg atc att cca cgc act gat gcg ccc acg ctg gtg tgg acg tat     787
Arg Trp Ile Ile Pro Arg Thr Asp Ala Pro Thr Leu Val Trp Thr Tyr
    215                 220                 225

ctg cag ctg agc ctg ctg acc gtg ctg gtt ggc agt gcc gca gca gct     835
Leu Gln Leu Ser Leu Leu Thr Val Leu Val Gly Ser Ala Ala Ala Ala
230                 235                 240                 245

gtc gat gag gtg gtt gcc cgc gcc caa tcc tcc acc aga aat gcg tgg     883
Val Asp Glu Val Val Ala Arg Ala Gln Ser Ser Thr Arg Asn Ala Trp
                250                 255                 260

aac cct ggc gtc gag cgc cgc agc gat ccg gcc gca acc ata gcg atc     931
Asn Pro Gly Val Glu Arg Arg Ser Asp Pro Ala Ala Thr Ile Ala Ile
            265                 270                 275

ggc gac gca cgc agc cga gtc acc gtt att cgt gga gcg ctt ctc gac     979
Gly Asp Ala Arg Ser Arg Val Thr Val Ile Arg Gly Ala Leu Leu Asp
        280                 285                 290

gcc acc cgc cac gtt tcc aac gcc gcc acg atc gta acc ccc gaa gcc     1027
Ala Thr Arg His Val Ser Asn Ala Ala Thr Ile Val Thr Pro Glu Ala
    295                 300                 305

ttc aac gag gcg gac gct att gtt gca gcg ctc tgg ccc atc gtc tcc     1075
Phe Asn Glu Ala Asp Ala Ile Val Ala Ala Leu Trp Pro Ile Val Ser
310                 315                 320                 325

gga caa gct ttg gtg gtg aca tcc aac gtt ttc gat gcg gtg ggt gca     1123
Gly Gln Ala Leu Val Val Thr Ser Asn Val Phe Asp Ala Val Gly Ala
                330                 335                 340

tct gca gtg ctt ggt gag cat tcc att gat cgc cac tgg cgc aat gtg     1171
Ser Ala Val Leu Gly Glu His Ser Ile Asp Arg His Trp Arg Asn Val
            345                 350                 355

cgt acc gtg tcc tca aac aac ccg gtg ttc ctg gcc aag aat gca gtg     1219
Arg Thr Val Ser Ser Asn Asn Pro Val Phe Leu Ala Lys Asn Ala Val
        360                 365                 370

ggg gag tat gcc ctc aac ggc act cct gtg ggt acc aac att ggt aaa     1267
Gly Glu Tyr Ala Leu Asn Gly Thr Pro Val Gly Thr Asn Ile Gly Lys
    375                 380                 385

gca ctg agc cgt ccg gtg agc cta agc agc tagacgtgtg atttcgctgg     1317
Ala Leu Ser Arg Pro Val Ser Leu Ser Ser
390                 395

ttt                                                                 1320

<210> 160
<211> 399
<212> PRT
<213> Corynebacterium glutamicum

<400> 160
Met Ser Gln Glu Ile Leu Ser His Phe Ala Pro Ala Leu Glu Arg Ile
1               5                   10                  15

Arg Ser Gly Ala Val Glu Arg Glu Gln Gln Arg Ala Leu Pro Val Glu
            20                  25                  30

Glu Ile Lys Glu Leu Val Glu Leu Gly Phe Thr Gly Leu Arg Val Pro
        35                  40                  45

Glu Glu Leu Gly Gly Ala Gly Ala Ser Leu Glu Ser Val Val Glu Leu
    50                  55                  60

Leu Ile Glu Ile Ala Gly Ala Asp Ser Asn Ile Ala Gln Ala Leu Arg
65                  70                  75                  80

Gly His Phe Ala Phe Val Glu Leu Leu Leu Glu Ala Pro Glu Ser Glu
                85                  90                  95

Phe Arg Thr His Trp Leu Arg Glu Val Ala Thr Gly Arg Leu Val Gly
            100                 105                 110

Asn Ala Glu Ser Glu Lys Arg Gly Val Tyr Gly Asp Pro Gln Thr Phe
        115                 120                 125

Ile Asp Glu Val Glu Thr Glu Asn Gly Pro Ile Phe Val Leu Asn Gly
    130                 135                 140

Thr Lys Phe Tyr Thr Thr Gly Thr Tyr Phe Ala Asp Tyr Thr Trp Thr
145                 150                 155                 160

Thr Ala Leu Leu Arg Asn Leu Asn Gly Gln Glu Thr Leu Val Ser Leu
                165                 170                 175

Pro Val Asp Leu His Ala Pro Gly Val Asp Val Ala Asp Asp Trp Ser
            180                 185                 190

Gly Phe Gly Gln Lys Leu Thr Ala Ser Gly Thr Thr Thr Phe Lys Asp
        195                 200                 205

Leu Glu Val Asp Pro Arg Trp Ile Ile Pro Arg Thr Asp Ala Pro Thr
    210                 215                 220

Leu Val Trp Thr Tyr Leu Gln Leu Ser Leu Leu Thr Val Leu Val Gly
225                 230                 235                 240

Ser Ala Ala Ala Ala Val Asp Glu Val Val Ala Arg Ala Gln Ser Ser
                245                 250                 255

Thr Arg Asn Ala Trp Asn Pro Gly Val Glu Arg Arg Ser Asp Pro Ala
            260                 265                 270

Ala Thr Ile Ala Ile Gly Asp Ala Arg Ser Arg Val Thr Val Ile Arg
        275                 280                 285

Gly Ala Leu Leu Asp Ala Thr Arg His Val Ser Asn Ala Ala Thr Ile
    290                 295                 300

Val Thr Pro Glu Ala Phe Asn Glu Ala Asp Ala Ile Val Ala Ala Leu
305                 310                 315                 320

Trp Pro Ile Val Ser Gly Gln Ala Leu Val Val Thr Ser Asn Val Phe
                325                 330                 335

Asp Ala Val Gly Ala Ser Ala Val Leu Gly Glu His Ser Ile Asp Arg
            340                 345                 350

His Trp Arg Asn Val Arg Thr Val Ser Ser Asn Asn Pro Val Phe Leu
        355                 360                 365

Ala Lys Asn Ala Val Gly Glu Tyr Ala Leu Asn Gly Thr Pro Val Gly
    370                 375                 380

Thr Asn Ile Gly Lys Ala Leu Ser Arg Pro Val Ser Leu Ser Ser
385                 390                 395

<210> 161
<211> 969
<212> DNA
<213> Corynebacterium glutamicum

<220>
<221> CDS
<222> (101)..(946)
<223> RXN00931

<400> 161
ccgtaaccta atcgttgaaa catcacctta ttgctgggct ttgcacgcta ctctttgtga 60

gtaacctcac cgaagtgcat aaattaattg ggagtgatca gtg aaa act att gaa    115
                                           Val Lys Thr Ile Glu
                                           1               5

gat att ttg acc ttg gaa gaa atc gac cgc gat att tac cgt ggt ccc    163
Asp Ile Leu Thr Leu Glu Glu Ile Asp Arg Asp Ile Tyr Arg Gly Pro
                10                  15                  20

gtt atc gaa tct tat tta gcc agg act ttc ggt ggc cag gtc gct gcc    211
Val Ile Glu Ser Tyr Leu Ala Arg Thr Phe Gly Gly Gln Val Ala Ala
            25                  30                  35

caa gct tta gta gca gca acg cat act gtt gat aaa gcc ttt act gtg    259
Gln Ala Leu Val Ala Ala Thr His Thr Val Asp Lys Ala Phe Thr Val
        40                  45                  50

cat tct ttg cat ggc tac ttt ata gct cct ggt gat cca aca gca ccc    307
His Ser Leu His Gly Tyr Phe Ile Ala Pro Gly Asp Pro Thr Ala Pro
    55                  60                  65

gca att tat tta gtg gat cga gtt cgc gac gga aaa agc tac gtc acc    355
Ala Ile Tyr Leu Val Asp Arg Val Arg Asp Gly Lys Ser Tyr Val Thr
70                  75                  80                  85

cgc tcg gtg cgt ggc atc caa gac ggc gaa gta atc ttc agc atg cag    403
Arg Ser Val Arg Gly Ile Gln Asp Gly Glu Val Ile Phe Ser Met Gln
                90                  95                  100

gcc agc ttt cat cgt ggg gat gaa ggc att gag cac atg gac aag atg    451
Ala Ser Phe His Arg Gly Asp Glu Gly Ile Glu His Met Asp Lys Met
            105                 110                 115

cgt aaa gtt cca gct cct gat gag atc aag gga aca gta gaa cgt atg 499
Arg Lys Val Pro Ala Pro Asp Glu Ile Lys Gly Thr Val Glu Arg Met
        120                 125                 130

ccg atc tca agt agg cga gtg ctt gat gaa tgg gcg gaa tgg gat atc 547
Pro Ile Ser Ser Arg Arg Val Leu Asp Glu Trp Ala Glu Trp Asp Ile
    135                 140                 145

cgc gtt att ccg cag gat caa tta gaa ctc agc gat ttc acc gct act 595
Arg Val Ile Pro Gln Asp Gln Leu Glu Leu Ser Asp Phe Thr Ala Thr
150                 155                 160                 165

gag caa gct gtg tgg att cgg tgc acc gct gat ctt ccg gat aat ccc 643
Glu Gln Ala Val Trp Ile Arg Cys Thr Ala Asp Leu Pro Asp Asn Pro
                170                 175                 180

acc ttc cac cag tgc tca ctg act tat ctg tcc gat atg act ttg ctg 691
Thr Phe His Gln Cys Ser Leu Thr Tyr Leu Ser Asp Met Thr Leu Leu
            185                 190                 195

cat agt gcc ctg gtg cca cac cca ggt gag aaa atg cag atg gcc tca 739
His Ser Ala Leu Val Pro His Pro Gly Glu Lys Met Gln Met Ala Ser
        200                 205                 210

ctt gat cac gct gtg tgg ttc ctg cgt ccc ttc cgc gtc gat gaa tgg 787
Leu Asp His Ala Val Trp Phe Leu Arg Pro Phe Arg Val Asp Glu Trp
    215                 220                 225

ttg ctt tat gat cag cgc tct cca tcg gcc tca agt ggg cga gcc ttg 835
Leu Leu Tyr Asp Gln Arg Ser Pro Ser Ala Ser Ser Gly Arg Ala Leu
230                 235                 240                 245

act cac ggg cgg ctt ttc aac cag cag gga gat ttg gtc gct att gtc 883
Thr His Gly Arg Leu Phe Asn Gln Gln Gly Asp Leu Val Ala Ile Val
                250                 255                 260

aat caa gag gga atg acc cgc aca ctc cac gag ggt gcg caa tca att 931
Asn Gln Glu Gly Met Thr Arg Thr Leu His Glu Gly Ala Gln Ser Ile
            265                 270                 275

ccg atg cgc aaa gac taaaatgcag cgaacttgaa gat 969
Pro Met Arg Lys Asp
        280

<210> 162
<211> 282
<212> PRT
<213> Corynebacterium glutamicum

<400> 162
Val Lys Thr Ile Glu Asp Ile Leu Thr Leu Glu Glu Ile Asp Arg Asp
1               5                   10                  15

Ile Tyr Arg Gly Pro Val Ile Glu Ser Tyr Leu Ala Arg Thr Phe Gly
            20                  25                  30

Gly Gln Val Ala Ala Gln Ala Leu Val Ala Ala Thr His Thr Val Asp
        35                  40                  45

Lys Ala Phe Thr Val His Ser Leu His Gly Tyr Phe Ile Ala Pro Gly
50                  55                  60

Asp Pro Thr Ala Pro Ala Ile Tyr Leu Val Asp Arg Val Arg Asp Gly
65                  70                  75                  80

Lys Ser Tyr Val Thr Arg Ser Val Arg Gly Ile Gln Asp Gly Glu Val
                85                  90                  95

Ile Phe Ser Met Gln Ala Ser Phe His Arg Gly Asp Glu Gly Ile Glu
            100                 105                 110

His Met Asp Lys Met Arg Lys Val Pro Ala Pro Asp Glu Ile Lys Gly
        115                 120                 125

Thr Val Glu Arg Met Pro Ile Ser Ser Arg Arg Val Leu Asp Glu Trp
    130                 135                 140

Ala Glu Trp Asp Ile Arg Val Ile Pro Gln Asp Gln Leu Glu Leu Ser
145                 150                 155                 160

Asp Phe Thr Ala Thr Glu Gln Ala Val Trp Ile Arg Cys Thr Ala Asp
                165                 170                 175

Leu Pro Asp Asn Pro Thr Phe His Gln Cys Ser Leu Thr Tyr Leu Ser
            180                 185                 190

Asp Met Thr Leu Leu His Ser Ala Leu Val Pro His Pro Gly Glu Lys
        195                 200                 205

Met Gln Met Ala Ser Leu Asp His Ala Val Trp Phe Leu Arg Pro Phe
    210                 215                 220

Arg Val Asp Glu Trp Leu Leu Tyr Asp Gln Arg Ser Pro Ser Ala Ser
225                 230                 235                 240

Ser Gly Arg Ala Leu Thr His Gly Arg Leu Phe Asn Gln Gln Gly Asp
                245                 250                 255

Leu Val Ala Ile Val Asn Gln Glu Gly Met Thr Arg Thr Leu His Glu
            260                 265                 270

Gly Ala Gln Ser Ile Pro Met Arg Lys Asp
        275                 280

<210> 163
<211> 969
<212> DNA
<213> Corynebacterium glutamicum

<220>
<221> CDS
<222> (101)..(946)
<223> FRXA00931

<400> 163
ccgtaaccta atcgttgaaa catcacctta ttgctgggct ttgcacgcta ctctttgtga 60

gtaacctcac cgaagtgcat aaattaattg ggagtgatca gtg aaa act att gaa   115
                                          Val Lys Thr Ile Glu

                                                    1               5

gat att ttg acc ttg gaa gaa atc gac cgc gat att tac cgt ggt ccc    163
Asp Ile Leu Thr Leu Glu Glu Ile Asp Arg Asp Ile Tyr Arg Gly Pro
                10                  15                  20

gtt atc gaa tct tat tta gcc agg act ttc ggt ggc cag gtc gct gcc    211
Val Ile Glu Ser Tyr Leu Ala Arg Thr Phe Gly Gly Gln Val Ala Ala
            25                  30                  35

caa gct tta gta gca gca acg cat act gtt gat aaa gcc ttt act gtg    259
Gln Ala Leu Val Ala Ala Thr His Thr Val Asp Lys Ala Phe Thr Val
        40                  45                  50

cat tct ttg cat ggc tac ttt ata gct cct ggt gat cca aca gca ccc    307
His Ser Leu His Gly Tyr Phe Ile Ala Pro Gly Asp Pro Thr Ala Pro
    55                  60                  65

gca att tat tta gtg gat cga gtt cgc gac gga aaa agc tac gtc acc    355
Ala Ile Tyr Leu Val Asp Arg Val Arg Asp Gly Lys Ser Tyr Val Thr
70                  75                  80                  85

cgc tcg gtg cgt ggc atc caa gac ggc gaa gta atc ttc agc atg cag    403
Arg Ser Val Arg Gly Ile Gln Asp Gly Glu Val Ile Phe Ser Met Gln
                90                  95                  100

gcc agc ttt cat cgt ggg gat gaa ggc att gag cac atg gac aag atg    451
Ala Ser Phe His Arg Gly Asp Glu Gly Ile Glu His Met Asp Lys Met
            105                 110                 115

cgt aaa gtt cca gct cct gat gag atc aag gga aca gta gaa cgt atg    499
Arg Lys Val Pro Ala Pro Asp Glu Ile Lys Gly Thr Val Glu Arg Met
        120                 125                 130

ccg atc tca agt agg cga gtg ctt gat gaa tgg gcg gaa tgg gat atc    547
Pro Ile Ser Ser Arg Arg Val Leu Asp Glu Trp Ala Glu Trp Asp Ile
    135                 140                 145

cgc gtt att ccg cag gat caa tta gaa ctc agc gat ttc acc gct act    595
Arg Val Ile Pro Gln Asp Gln Leu Glu Leu Ser Asp Phe Thr Ala Thr
150                 155                 160                 165

gag caa gct gtg tgg att cgg tgc acc gct gat ctt ccg gat aat ccc    643
Glu Gln Ala Val Trp Ile Arg Cys Thr Ala Asp Leu Pro Asp Asn Pro
                170                 175                 180

acc ttc cac cag tgc tca ctg act tat ctg tcc gat atg act ttg ctg    691
Thr Phe His Gln Cys Ser Leu Thr Tyr Leu Ser Asp Met Thr Leu Leu
            185                 190                 195

cat agt gcc ctg gtg cca cac cca ggt gag aaa atg cag atg gcc tca    739
His Ser Ala Leu Val Pro His Pro Gly Glu Lys Met Gln Met Ala Ser
        200                 205                 210

ctt gat cac gct gtg tgg ttc ctg cgt ccc ttc cgc gtc gat gaa tgg    787
Leu Asp His Ala Val Trp Phe Leu Arg Pro Phe Arg Val Asp Glu Trp
    215                 220                 225

ttg ctt tat gat cag cgc tct cca tcg gcc tca agt ggg cga gcc ttg    835
Leu Leu Tyr Asp Gln Arg Ser Pro Ser Ala Ser Ser Gly Arg Ala Leu
230                 235                 240                 245

act cac ggg cgg ctt ttc aac cag cag gga gat ttg gtc gct att gtc 883
Thr His Gly Arg Leu Phe Asn Gln Gln Gly Asp Leu Val Ala Ile Val
                250                 255                 260

aat caa gag gga atg acc cgc aca ctc cac gag ggt gcg caa tca att 931
Asn Gln Glu Gly Met Thr Arg Thr Leu His Glu Gly Ala Gln Ser Ile
            265                 270                 275

ccg atg cgc aaa gac taaaatgcag cgaacttgaa gat 969
Pro Met Arg Lys Asp
        280

<210> 164
<211> 282
<212> PRT
<213> Corynebacterium glutamicum

<400> 164
Val Lys Thr Ile Glu Asp Ile Leu Thr Leu Glu Glu Ile Asp Arg Asp
1               5                   10                  15

Ile Tyr Arg Gly Pro Val Ile Glu Ser Tyr Leu Ala Arg Thr Phe Gly
            20                  25                  30

Gly Gln Val Ala Ala Gln Ala Leu Val Ala Ala Thr His Thr Val Asp
        35                  40                  45

Lys Ala Phe Thr Val His Ser Leu His Gly Tyr Phe Ile Ala Pro Gly
    50                  55                  60

Asp Pro Thr Ala Pro Ala Ile Tyr Leu Val Asp Arg Val Arg Asp Gly
65                  70                  75                  80

Lys Ser Tyr Val Thr Arg Ser Val Arg Gly Ile Gln Asp Gly Glu Val
                85                  90                  95

Ile Phe Ser Met Gln Ala Ser Phe His Arg Gly Asp Glu Gly Ile Glu
            100                 105                 110

His Met Asp Lys Met Arg Lys Val Pro Ala Pro Asp Glu Ile Lys Gly
        115                 120                 125

Thr Val Glu Arg Met Pro Ile Ser Ser Arg Arg Val Leu Asp Glu Trp
    130                 135                 140

Ala Glu Trp Asp Ile Arg Val Ile Pro Gln Asp Gln Leu Glu Leu Ser
145                 150                 155                 160

Asp Phe Thr Ala Thr Glu Gln Ala Val Trp Ile Arg Cys Thr Ala Asp
                165                 170                 175

Leu Pro Asp Asn Pro Thr Phe His Gln Cys Ser Leu Thr Tyr Leu Ser
            180                 185                 190

Asp Met Thr Leu Leu His Ser Ala Leu Val Pro His Pro Gly Glu Lys
        195                 200                 205

Met Gln Met Ala Ser Leu Asp His Ala Val Trp Phe Leu Arg Pro Phe
    210                 215                 220

Arg Val Asp Glu Trp Leu Leu Tyr Asp Gln Arg Ser Pro Ser Ala Ser
225                 230                 235                 240

Ser Gly Arg Ala Leu Thr His Gly Arg Leu Phe Asn Gln Gln Gly Asp
                245                 250                 255

Leu Val Ala Ile Val Asn Gln Glu Gly Met Thr Arg Thr Leu His Glu
            260                 265                 270

Gly Ala Gln Ser Ile Pro Met Arg Lys Asp
        275                 280

<210> 165
<211> 510
<212> DNA
<213> Corynebacterium glutamicum

<220>
<221> CDS
<222> (101)..(487)
<223> RXN01421

<400> 165
ttgatgcacg tgcagaaatc gtcggcggtc cgtggcaccc atctgttaag ggagactcgg 60

ttactgcagg gatcctgcga gatcgagtaa acgcctaaag atg acg tcg aaa agc   115
                                            Met Thr Ser Lys Ser
                                            1               5

att agc ggc aag cgc ccg aat ctg ccg tcg ctc act gga gcg cgg tgg   163
Ile Ser Gly Lys Arg Pro Asn Leu Pro Ser Leu Thr Gly Ala Arg Trp
                10                  15                  20

ctc gcg gcg ctc gct gtt tat ttt ttg cat gcg ttg gtg ttt ttg tcg   211
Leu Ala Ala Leu Ala Val Tyr Phe Leu His Ala Leu Val Phe Leu Ser
            25                  30                  35

gtg tat ccg ttc cag cag tcg gaa ctg ttt gcc aca atc cat aaa ttt   259
Val Tyr Pro Phe Gln Gln Ser Glu Leu Phe Ala Thr Ile His Lys Phe
        40                  45                  50

gtc ccc atg cag ctg ggt tca gct ggt gta acc ttc ttc ttt atc ttg   307
Val Pro Met Gln Leu Gly Ser Ala Gly Val Thr Phe Phe Phe Ile Leu
    55                  60                  65

tcc gga ttt ttg atc tat tgg tca aat agc cag ctc aag ggc atg aag   355
Ser Gly Phe Leu Ile Tyr Trp Ser Asn Ser Gln Leu Lys Gly Met Lys
70                  75                  80                  85

aat gtg ctg tat tac tgc aag cgc cgc atc acc aag att tat ccc atg   403
Asn Val Leu Tyr Tyr Cys Lys Arg Arg Ile Thr Lys Ile Tyr Pro Met
                90                  95                  100

cac ttg att gcg ttg ccg atg ttt att gag gcg tcg gcg aag ttc acg   451
His Leu Ile Ala Leu Pro Met Phe Ile Glu Ala Ser Ala Lys Phe Thr
            105                 110                 115

act aca ggc att acc tgg gtg ctg att ttg cgc gag taaagctgtg        497
Thr Thr Gly Ile Thr Trp Val Leu Ile Leu Arg Glu

120                 125

gctgcggaat gcg                                                    510

<210> 166
<211> 129
<212> PRT
<213> Corynebacterium glutamicum

<400> 166
Met Thr Ser Lys Ser Ile Ser Gly Lys Arg Pro Asn Leu Pro Ser Leu
1               5                   10                  15

Thr Gly Ala Arg Trp Leu Ala Ala Leu Ala Val Tyr Phe Leu His Ala
            20                  25                  30

Leu Val Phe Leu Ser Val Tyr Pro Phe Gln Gln Ser Glu Leu Phe Ala
        35                  40                  45

Thr Ile His Lys Phe Val Pro Met Gln Leu Gly Ser Ala Gly Val Thr
    50                  55                  60

Phe Phe Phe Ile Leu Ser Gly Phe Leu Ile Tyr Trp Ser Asn Ser Gln
65                  70                  75                  80

Leu Lys Gly Met Lys Asn Val Leu Tyr Tyr Cys Lys Arg Arg Ile Thr
                85                  90                  95

Lys Ile Tyr Pro Met His Leu Ile Ala Leu Pro Met Phe Ile Glu Ala
            100                 105                 110

Ser Ala Lys Phe Thr Thr Thr Gly Ile Thr Trp Val Leu Ile Leu Arg
        115                 120                 125

Glu

<210> 167
<211> 930
<212> DNA
<213> Corynebacterium glutamicum

<220>
<221> CDS
<222> (101)..(907)
<223> RXN02342

<400> 167
caggttcgtg ctgcgtgcgg gtagttttta aaggtaaaac tttttgggcg tgtcgccctt 60

aaagcgcgct tttcgacgcg accccactac attggcttcc atg aac gtt gac att   115
                                            Met Asn Val Asp Ile
                                            1               5

tca cga tcc aga gag ccg cta aac gtt gag ctc ctg aag gaa aaa ttg   163
Ser Arg Ser Arg Glu Pro Leu Asn Val Glu Leu Leu Lys Glu Lys Leu
                10                  15                  20

ctc caa aac ggt gac ttt ggc cag gtc att tac gaa aaa gtg aca ggc   211

Leu Gln Asn Gly Asp Phe Gly Gln Val Ile Tyr Glu Lys Val Thr Gly
            25                  30                  35

tcc act aat gct gac ttg ctg gca ctt gca ggt tct ggc gct cca aac 259
Ser Thr Asn Ala Asp Leu Leu Ala Leu Ala Gly Ser Gly Ala Pro Asn
        40                  45                  50

tgg acg gtg aaa act gtc gag ttt caa gat cat gcg cgt ggg cga ctc 307
Trp Thr Val Lys Thr Val Glu Phe Gln Asp His Ala Arg Gly Arg Leu
    55                  60                  65

ggc cgc ccg tgg tct gcc cct gag ggt tcc caa aca atc gtg tct gtg 355
Gly Arg Pro Trp Ser Ala Pro Glu Gly Ser Gln Thr Ile Val Ser Val
70                  75                  80                  85

ctc gtt caa cta tct att gat caa gtg gac cgg att ggc act att cca 403
Leu Val Gln Leu Ser Ile Asp Gln Val Asp Arg Ile Gly Thr Ile Pro
                90                  95                  100

ctc gcg gcg gga ctc gct gtc atg gat gcg ttg aat gac ctc ggt gtg 451
Leu Ala Ala Gly Leu Ala Val Met Asp Ala Leu Asn Asp Leu Gly Val
            105                 110                 115

gaa ggt gcc gga ctg aaa tgg ccc aac gat gtt caa atc cac ggc aag 499
Glu Gly Ala Gly Leu Lys Trp Pro Asn Asp Val Gln Ile His Gly Lys
        120                 125                 130

aaa ctc tgc ggc atc ctg gtg gaa gcc acc ggc ttt gat tcc acc cca 547
Lys Leu Cys Gly Ile Leu Val Glu Ala Thr Gly Phe Asp Ser Thr Pro
    135                 140                 145

aca gtt gtc atc ggt tgg ggc act aat atc agc ctg act aaa gag gag 595
Thr Val Val Ile Gly Trp Gly Thr Asn Ile Ser Leu Thr Lys Glu Glu
150                 155                 160                 165

ctt cct gtt cct cat gca act tcc ctc gca ttg gaa ggt gtt gaa gtc 643
Leu Pro Val Pro His Ala Thr Ser Leu Ala Leu Glu Gly Val Glu Val
                170                 175                 180

gac aga acc aca ttc ctt att aat atg ctc aca cat ctg cat act cga 691
Asp Arg Thr Thr Phe Leu Ile Asn Met Leu Thr His Leu His Thr Arg
            185                 190                 195

ctg gac cag tgg cag ggt cca agt gtg gat tgg ctc gat gat tac cgt 739
Leu Asp Gln Trp Gln Gly Pro Ser Val Asp Trp Leu Asp Asp Tyr Arg
        200                 205                 210

gcg gta tgt tcc agt att ggc caa gat gtt cga gtg ctt cta cct ggg 787
Ala Val Cys Ser Ser Ile Gly Gln Asp Val Arg Val Leu Leu Pro Gly
    215                 220                 225

gat aaa gaa ctc tta ggt gaa gcg atc ggt gtc gcg act ggc gga gaa 835
Asp Lys Glu Leu Leu Gly Glu Ala Ile Gly Val Ala Thr Gly Gly Glu
230                 235                 240                 245

att cgt gtt cgc gat gct tcg ggc acc gtt cac acc ctc aac gcc ggt 883
Ile Arg Val Arg Asp Ala Ser Gly Thr Val His Thr Leu Asn Ala Gly
                250                 255                 260

gaa att acg cac ctt cgc ctg cag taaggtgacg gtatggggaa ttc 930
Glu Ile Thr His Leu Arg Leu Gln

            265

<210> 168
<211> 269
<212> PRT
<213> Corynebacterium glutamicum

<400> 168
Met Asn Val Asp Ile Ser Arg Ser Arg Glu Pro Leu Asn Val Glu Leu
1               5                   10                  15

Leu Lys Glu Lys Leu Leu Gln Asn Gly Asp Phe Gly Gln Val Ile Tyr
            20                  25                  30

Glu Lys Val Thr Gly Ser Thr Asn Ala Asp Leu Leu Ala Leu Ala Gly
        35                  40                  45

Ser Gly Ala Pro Asn Trp Thr Val Lys Thr Val Glu Phe Gln Asp His
    50                  55                  60

Ala Arg Gly Arg Leu Gly Arg Pro Trp Ser Ala Pro Glu Gly Ser Gln
65                  70                  75                  80

Thr Ile Val Ser Val Leu Val Gln Leu Ser Ile Asp Gln Val Asp Arg
                85                  90                  95

Ile Gly Thr Ile Pro Leu Ala Ala Gly Leu Ala Val Met Asp Ala Leu
            100                 105                 110

Asn Asp Leu Gly Val Glu Gly Ala Gly Leu Lys Trp Pro Asn Asp Val
        115                 120                 125

Gln Ile His Gly Lys Lys Leu Cys Gly Ile Leu Val Glu Ala Thr Gly
    130                 135                 140

Phe Asp Ser Thr Pro Thr Val Val Ile Gly Trp Gly Thr Asn Ile Ser
145                 150                 155                 160

Leu Thr Lys Glu Glu Leu Pro Val Pro His Ala Thr Ser Leu Ala Leu
                165                 170                 175

Glu Gly Val Glu Val Asp Arg Thr Thr Phe Leu Ile Asn Met Leu Thr
            180                 185                 190

His Leu His Thr Arg Leu Asp Gln Trp Gln Gly Pro Ser Val Asp Trp
        195                 200                 205

Leu Asp Asp Tyr Arg Ala Val Cys Ser Ser Ile Gly Gln Asp Val Arg
    210                 215                 220

Val Leu Leu Pro Gly Asp Lys Glu Leu Leu Gly Glu Ala Ile Gly Val
225                 230                 235                 240

Ala Thr Gly Gly Glu Ile Arg Val Arg Asp Ala Ser Gly Thr Val His
                245                 250                 255

Thr Leu Asn Ala Gly Glu Ile Thr His Leu Arg Leu Gln
            260                 265

<210> 169
<211> 2762
<212> DNA
<213> Corynebacterium glutamicum

<220>
<221> CDS
<222> (1)..(2739)
<223> RXN00563

<400> 169
ttc tac aag gat ctc tac gca cgt tcc gca cgc ggc acg gca gca ctg     48
Phe Tyr Lys Asp Leu Tyr Ala Arg Ser Ala Arg Gly Thr Ala Ala Leu
1               5                   10                  15

tgg atc gtg gcg gct aac ttg agc tcc tac tca gac atc gac gcc atc     96
Trp Ile Val Ala Ala Asn Leu Ser Ser Tyr Ser Asp Ile Asp Ala Ile
            20                  25                  30

atc aac tgg gtc gga tcc gag cag acc acc acc gtc aac ggc gca tcc     144
Ile Asn Trp Val Gly Ser Glu Gln Thr Thr Thr Val Asn Gly Ala Ser
        35                  40                  45

aag ctg gtc aag cca gct ttg gtc cct acc ttg ctg ttc cca ttc gcg     192
Lys Leu Val Lys Pro Ala Leu Val Pro Thr Leu Leu Phe Pro Phe Ala
    50                  55                  60

gca cct cgc gtg tcc gga tcc atg gca gat gca ggc cca cag gca gaa     240
Ala Pro Arg Val Ser Gly Ser Met Ala Asp Ala Gly Pro Gln Ala Glu
65                  70                  75                  80

tcc cag atg cga ctt ctg ctc tgg tct gtt gag cgc ctc atc gca ggt     288
Ser Gln Met Arg Leu Leu Leu Trp Ser Val Glu Arg Leu Ile Ala Gly
                85                  90                  95

ctt gcg cca ttg ggc tcc tcc atc aac gtg ggt cac cgc ctg cac gtg     336
Leu Ala Pro Leu Gly Ser Ser Ile Asn Val Gly His Arg Leu His Val
            100                 105                 110

gtc atc cca ggt tca cca aac cgt gga cgc ttc ggt ggc gat ggt gca     384
Val Ile Pro Gly Ser Pro Asn Arg Gly Arg Phe Gly Gly Asp Gly Ala
        115                 120                 125

tac ggt gaa tcc aag gca gct ctc gac gcc gtg gtt acc cgt tgg aac     432
Tyr Gly Glu Ser Lys Ala Ala Leu Asp Ala Val Val Thr Arg Trp Asn
    130                 135                 140

gca gag caa gct gca tgg gga gca cac acc tcc ctc gtg cac gct cac     480
Ala Glu Gln Ala Ala Trp Gly Ala His Thr Ser Leu Val His Ala His
145                 150                 155                 160

atc ggt tgg gtt cgc ggc acc ggc ctc atg ggc ggc aac gat cct ttg     528
Ile Gly Trp Val Arg Gly Thr Gly Leu Met Gly Gly Asn Asp Pro Leu
                165                 170                 175

gtc aag gca gct gaa gaa gca ggc gtg gaa acc tac tcc acc caa gaa     576
Val Lys Ala Ala Glu Glu Ala Gly Val Glu Thr Tyr Ser Thr Gln Glu
            180                 185                 190

att gca gag aaa ctg ctg tcc cag gca act tcc act gtt cgc gag cag     624
Ile Ala Glu Lys Leu Leu Ser Gln Ala Thr Ser Thr Val Arg Glu Gln

        195                 200                 205

gca gca tcc gcg cca atc acc gtc gac ttc act ggc gga ctt ggt gaa 672
Ala Ala Ser Ala Pro Ile Thr Val Asp Phe Thr Gly Gly Leu Gly Glu
    210                 215                 220

tct gat ctg aac ctg gcg gaa atg gca cgt gca gaa gca gct aag gca 720
Ser Asp Leu Asn Leu Ala Glu Met Ala Arg Ala Glu Ala Ala Lys Ala
225                 230                 235                 240

gct aac gca cca gtg gtt gag gct cca cgc aca gtg gca gca ctg cca 768
Ala Asn Ala Pro Val Val Glu Ala Pro Arg Thr Val Ala Ala Leu Pro
                245                 250                 255

act cct tac cga cca gtg gtt caa acc acc cct gat ttc gca ggt caa 816
Thr Pro Tyr Arg Pro Val Val Gln Thr Thr Pro Asp Phe Ala Gly Gln
            260                 265                 270

gtc acc caa aac ctt gac gag atg gtc gtc atc gtt ggc gcc ggc gag 864
Val Thr Gln Asn Leu Asp Glu Met Val Val Ile Val Gly Ala Gly Glu
        275                 280                 285

ctc ggc cca ctg ggt tct gca cgt acg cgt ttc gac gcc gaa ctc aac 912
Leu Gly Pro Leu Gly Ser Ala Arg Thr Arg Phe Asp Ala Glu Leu Asn
    290                 295                 300

ggt tcc ctc tcc gcc gcg ggt gtc atc gaa ctt gca tgg acg atg gga 960
Gly Ser Leu Ser Ala Ala Gly Val Ile Glu Leu Ala Trp Thr Met Gly
305                 310                 315                 320

ctt atc cac tgg gat gaa gat cca aag cca ggc tgg tac gac gac tcc 1008
Leu Ile His Trp Asp Glu Asp Pro Lys Pro Gly Trp Tyr Asp Asp Ser
                325                 330                 335

gac gac gca gtg gcc gaa gaa gac atc ttc gac cgc tac cac gac gaa 1056
Asp Asp Ala Val Ala Glu Glu Asp Ile Phe Asp Arg Tyr His Asp Glu
            340                 345                 350

gtc atg gca cgc gtt ggt gtc cgc aag tac aat gac atg cct gag tac 1104
Val Met Ala Arg Val Gly Val Arg Lys Tyr Asn Asp Met Pro Glu Tyr
        355                 360                 365

ggc atg atc gac aac ttt gca cca gag ctg acc acc gtc tac ctc gac 1152
Gly Met Ile Asp Asn Phe Ala Pro Glu Leu Thr Thr Val Tyr Leu Asp
    370                 375                 380

cag gac ctc acc ttc aac gtg gga tcc cgc gaa gag gca ctg acc tac 1200
Gln Asp Leu Thr Phe Asn Val Gly Ser Arg Glu Glu Ala Leu Thr Tyr
385                 390                 395                 400

gtc gac tcc gag cca gaa ctc acc ttt gct tct ttc gac gaa gca gca 1248
Val Asp Ser Glu Pro Glu Leu Thr Phe Ala Ser Phe Asp Glu Ala Ala
                405                 410                 415

ggg gag tgg aag gtc act cgc aag gca ggc tcc gca atc cgc gta cct 1296
Gly Glu Trp Lys Val Thr Arg Lys Ala Gly Ser Ala Ile Arg Val Pro
            420                 425                 430

cgc cgc atg gcg atg acc cgc ttc gtt ggt gga cag gtt cct aag gac 1344
Arg Arg Met Ala Met Thr Arg Phe Val Gly Gly Gln Val Pro Lys Asp
        435                 440                 445

ttc gac cca gct gtg tgg ggc att cca gct gac atg gtg gac aac ctg 1392
Phe Asp Pro Ala Val Trp Gly Ile Pro Ala Asp Met Val Asp Asn Leu
    450                 455                 460

gac acc gtc gcg ctg tgg aac att gtc tgt act gtc gac gcc ttc ctg 1440
Asp Thr Val Ala Leu Trp Asn Ile Val Cys Thr Val Asp Ala Phe Leu
465                 470                 475                 480

tcc gct gga ttc acc cca gca gag ctg ctt gct tcc gtt cac cca gca 1488
Ser Ala Gly Phe Thr Pro Ala Glu Leu Leu Ala Ser Val His Pro Ala
                485                 490                 495

cgc gtg tcc tct acc caa ggc acc ggc atg ggc ggc atg gaa tcc ctc 1536
Arg Val Ser Ser Thr Gln Gly Thr Gly Met Gly Gly Met Glu Ser Leu
            500                 505                 510

cgt ggc atc tac gtc gac cgc att ctg gca gag cca cgc gcc aac gac 1584
Arg Gly Ile Tyr Val Asp Arg Ile Leu Ala Glu Pro Arg Ala Asn Asp
        515                 520                 525

gtt ctg cag gaa gca ctg ccc aac gtt gtt gca gct cac gtc atg cag 1632
Val Leu Gln Glu Ala Leu Pro Asn Val Val Ala Ala His Val Met Gln
    530                 535                 540

tcc tac gtc ggt ggc tac gga cag atg atc cac cca gtc gca gct tgt 1680
Ser Tyr Val Gly Gly Tyr Gly Gln Met Ile His Pro Val Ala Ala Cys
545                 550                 555                 560

gca acc gca gct gtt tct gtg gaa gaa gca ctg gac aag atc cgc atc 1728
Ala Thr Ala Ala Val Ser Val Glu Glu Ala Leu Asp Lys Ile Arg Ile
                565                 570                 575

ggc aag tcc gac ttc gtt gtc gca ggt ggc ttc gat gcc ctg tcc gtt 1776
Gly Lys Ser Asp Phe Val Val Ala Gly Gly Phe Asp Ala Leu Ser Val
            580                 585                 590

gaa ggc atc acc ggc ttc ggc gac atg gca gca acc gcc gac tcc gca 1824
Glu Gly Ile Thr Gly Phe Gly Asp Met Ala Ala Thr Ala Asp Ser Ala
        595                 600                 605

gag atg gaa ggc aag gga att gag cac cgc ttc ttc tcc cgc gcc aac 1872
Glu Met Glu Gly Lys Gly Ile Glu His Arg Phe Phe Ser Arg Ala Asn
    610                 615                 620

gac cgg cgc cgc ggt gga ttc atc gaa tcc gaa ggt ggc gga acc gtc 1920
Asp Arg Arg Arg Gly Gly Phe Ile Glu Ser Glu Gly Gly Gly Thr Val
625                 630                 635                 640

ctt ctg gca cgc gga tca ctc gca gct gac ctg ggc ctt cca gta ctc 1968
Leu Leu Ala Arg Gly Ser Leu Ala Ala Asp Leu Gly Leu Pro Val Leu
                645                 650                 655

ggt gtc atc gga ttc gca gag tcc ttt gca gat ggt gcc cac acc tcc 2016
Gly Val Ile Gly Phe Ala Glu Ser Phe Ala Asp Gly Ala His Thr Ser
            660                 665                 670

atc cca gcc cca ggc ctc ggt gcc ctt ggt gct gct cgc gat ggt gtg 2064
Ile Pro Ala Pro Gly Leu Gly Ala Leu Gly Ala Ala Arg Asp Gly Val
        675                 680                 685

gaa tct cgc ctt gca gta gca ctg cgt tcc gtc ggt gtc tct gct gat     2112
Glu Ser Arg Leu Ala Val Ala Leu Arg Ser Val Gly Val Ser Ala Asp
    690                 695                 700

gag atc tcc att atc tcc aag cac gac acc tcc acc aac gcg aat gat     2160
Glu Ile Ser Ile Ile Ser Lys His Asp Thr Ser Thr Asn Ala Asn Asp
705                 710                 715                 720

cca aac gag tcc gac ctg cac gag cgc atc gca tcc gct atc ggt cgt     2208
Pro Asn Glu Ser Asp Leu His Glu Arg Ile Ala Ser Ala Ile Gly Arg
                725                 730                 735

gca gac ggc aac ccg atg tac gtg att tcc cag aag tca ctc acc gga     2256
Ala Asp Gly Asn Pro Met Tyr Val Ile Ser Gln Lys Ser Leu Thr Gly
            740                 745                 750

cac gcc aag ggt ggt gca gca gca ttc cag atg atc ggt ctc acc cag     2304
His Ala Lys Gly Gly Ala Ala Ala Phe Gln Met Ile Gly Leu Thr Gln
        755                 760                 765

gtc ctc cga tcc gga ctg gtg cca gcc aac cgc gca ctc gac tgc gtt     2352
Val Leu Arg Ser Gly Leu Val Pro Ala Asn Arg Ala Leu Asp Cys Val
    770                 775                 780

gac cca gta ctg tcc aag cat tcc cac ctc gtc tgg ctg cgc aag cca     2400
Asp Pro Val Leu Ser Lys His Ser His Leu Val Trp Leu Arg Lys Pro
785                 790                 795                 800

cta gac ctt cgt gcg aag gca cca aag gca ggt ctt gtt acc tcc ctt     2448
Leu Asp Leu Arg Ala Lys Ala Pro Lys Ala Gly Leu Val Thr Ser Leu
                805                 810                 815

ggc ttc gga cac gtc tcc gct ctg gtt gcg att gtt cac cca gac gcc     2496
Gly Phe Gly His Val Ser Ala Leu Val Ala Ile Val His Pro Asp Ala
            820                 825                 830

ttc tat gag gca gtt cgt gtg gca cgt ggt gct gag gca gct gac gta     2544
Phe Tyr Glu Ala Val Arg Val Ala Arg Gly Ala Glu Ala Ala Asp Val
        835                 840                 845

tgg cgc gca tcc gcg atc gct cgc gaa gaa gca ggc ctt cgt acc atc     2592
Trp Arg Ala Ser Ala Ile Ala Arg Glu Glu Ala Gly Leu Arg Thr Ile
    850                 855                 860

gtc gcc ggt atg cac ggt ggc gta ctg tac gaa cgc cca gtc gag cgc     2640
Val Ala Gly Met His Gly Gly Val Leu Tyr Glu Arg Pro Val Glu Arg
865                 870                 875                 880

aac ctc ggt gtc cac gga gac gca gct aag gaa gtt gaa gct gca gtc     2688
Asn Leu Gly Val His Gly Asp Ala Ala Lys Glu Val Glu Ala Ala Val
                885                 890                 895

ctc ctg gat tcc cgc gcc cgc cta gtt gac ggt gtc ctc cgc gcc gaa     2736
Leu Leu Asp Ser Arg Ala Arg Leu Val Asp Gly Val Leu Arg Ala Glu
            900                 905                 910

ggc tagttggtta ttgcgttgag ccc                                       2762
Gly

<210> 170
<211> 913
<212> PRT
<213> Corynebacterium glutamicum

<400> 170
Phe Tyr Lys Asp Leu Tyr Ala Arg Ser Ala Arg Gly Thr Ala Ala Leu
1               5                   10                  15

Trp Ile Val Ala Ala Asn Leu Ser Ser Tyr Ser Asp Ile Asp Ala Ile
            20                  25                  30

Ile Asn Trp Val Gly Ser Glu Gln Thr Thr Thr Val Asn Gly Ala Ser
        35                  40                  45

Lys Leu Val Lys Pro Ala Leu Val Pro Thr Leu Leu Phe Pro Phe Ala
    50                  55                  60

Ala Pro Arg Val Ser Gly Ser Met Ala Asp Ala Gly Pro Gln Ala Glu
65                  70                  75                  80

Ser Gln Met Arg Leu Leu Leu Trp Ser Val Glu Arg Leu Ile Ala Gly
                85                  90                  95

Leu Ala Pro Leu Gly Ser Ser Ile Asn Val Gly His Arg Leu His Val
            100                 105                 110

Val Ile Pro Gly Ser Pro Asn Arg Gly Arg Phe Gly Gly Asp Gly Ala
        115                 120                 125

Tyr Gly Glu Ser Lys Ala Ala Leu Asp Ala Val Val Thr Arg Trp Asn
    130                 135                 140

Ala Glu Gln Ala Ala Trp Gly Ala His Thr Ser Leu Val His Ala His
145                 150                 155                 160

Ile Gly Trp Val Arg Gly Thr Gly Leu Met Gly Gly Asn Asp Pro Leu
                165                 170                 175

Val Lys Ala Ala Glu Glu Ala Gly Val Glu Thr Tyr Ser Thr Gln Glu
            180                 185                 190

Ile Ala Glu Lys Leu Leu Ser Gln Ala Thr Ser Thr Val Arg Glu Gln
        195                 200                 205

Ala Ala Ser Ala Pro Ile Thr Val Asp Phe Thr Gly Gly Leu Gly Glu
    210                 215                 220

Ser Asp Leu Asn Leu Ala Glu Met Ala Arg Ala Glu Ala Ala Lys Ala
225                 230                 235                 240

Ala Asn Ala Pro Val Val Glu Ala Pro Arg Thr Val Ala Ala Leu Pro
                245                 250                 255

Thr Pro Tyr Arg Pro Val Val Gln Thr Thr Pro Asp Phe Ala Gly Gln
            260                 265                 270

Val Thr Gln Asn Leu Asp Glu Met Val Val Ile Val Gly Ala Gly Glu
        275                 280                 285

Leu Gly Pro Leu Gly Ser Ala Arg Thr Arg Phe Asp Ala Glu Leu Asn

290                 295                 300

Gly Ser Leu Ser Ala Ala Gly Val Ile Glu Leu Ala Trp Thr Met Gly
305                 310                 315                 320

Leu Ile His Trp Asp Glu Asp Pro Lys Pro Gly Trp Tyr Asp Asp Ser
                325                 330                 335

Asp Asp Ala Val Ala Glu Glu Asp Ile Phe Asp Arg Tyr His Asp Glu
            340                 345                 350

Val Met Ala Arg Val Gly Val Arg Lys Tyr Asn Asp Met Pro Glu Tyr
        355                 360                 365

Gly Met Ile Asp Asn Phe Ala Pro Glu Leu Thr Thr Val Tyr Leu Asp
    370                 375                 380

Gln Asp Leu Thr Phe Asn Val Gly Ser Arg Glu Glu Ala Leu Thr Tyr
385                 390                 395                 400

Val Asp Ser Glu Pro Glu Leu Thr Phe Ala Ser Phe Asp Glu Ala Ala
                405                 410                 415

Gly Glu Trp Lys Val Thr Arg Lys Ala Gly Ser Ala Ile Arg Val Pro
            420                 425                 430

Arg Arg Met Ala Met Thr Arg Phe Val Gly Gly Gln Val Pro Lys Asp
        435                 440                 445

Phe Asp Pro Ala Val Trp Gly Ile Pro Ala Asp Met Val Asp Asn Leu
    450                 455                 460

Asp Thr Val Ala Leu Trp Asn Ile Val Cys Thr Val Asp Ala Phe Leu
465                 470                 475                 480

Ser Ala Gly Phe Thr Pro Ala Glu Leu Leu Ala Ser Val His Pro Ala
                485                 490                 495

Arg Val Ser Ser Thr Gln Gly Thr Gly Met Gly Gly Met Glu Ser Leu
            500                 505                 510

Arg Gly Ile Tyr Val Asp Arg Ile Leu Ala Glu Pro Arg Ala Asn Asp
        515                 520                 525

Val Leu Gln Glu Ala Leu Pro Asn Val Val Ala Ala His Val Met Gln
    530                 535                 540

Ser Tyr Val Gly Gly Tyr Gly Gln Met Ile His Pro Val Ala Ala Cys
545                 550                 555                 560

Ala Thr Ala Ala Val Ser Val Glu Glu Ala Leu Asp Lys Ile Arg Ile
                565                 570                 575

Gly Lys Ser Asp Phe Val Val Ala Gly Gly Phe Asp Ala Leu Ser Val
            580                 585                 590

Glu Gly Ile Thr Gly Phe Gly Asp Met Ala Ala Thr Ala Asp Ser Ala
        595                 600                 605

Glu Met Glu Gly Lys Gly Ile Glu His Arg Phe Phe Ser Arg Ala Asn
    610                 615                 620

Asp Arg Arg Arg Gly Gly Phe Ile Glu Ser Glu Gly Gly Gly Thr Val
625                 630                 635                 640

Leu Leu Ala Arg Gly Ser Leu Ala Ala Asp Leu Gly Leu Pro Val Leu
                645                 650                 655

Gly Val Ile Gly Phe Ala Glu Ser Phe Ala Asp Gly Ala His Thr Ser
            660                 665                 670

Ile Pro Ala Pro Gly Leu Gly Ala Leu Gly Ala Ala Arg Asp Gly Val
        675                 680                 685

Glu Ser Arg Leu Ala Val Ala Leu Arg Ser Val Gly Val Ser Ala Asp
    690                 695                 700

Glu Ile Ser Ile Ile Ser Lys His Asp Thr Ser Thr Asn Ala Asn Asp
705                 710                 715                 720

Pro Asn Glu Ser Asp Leu His Glu Arg Ile Ala Ser Ala Ile Gly Arg
                725                 730                 735

Ala Asp Gly Asn Pro Met Tyr Val Ile Ser Gln Lys Ser Leu Thr Gly
            740                 745                 750

His Ala Lys Gly Gly Ala Ala Ala Phe Gln Met Ile Gly Leu Thr Gln
        755                 760                 765

Val Leu Arg Ser Gly Leu Val Pro Ala Asn Arg Ala Leu Asp Cys Val
    770                 775                 780

Asp Pro Val Leu Ser Lys His Ser His Leu Val Trp Leu Arg Lys Pro
785                 790                 795                 800

Leu Asp Leu Arg Ala Lys Ala Pro Lys Ala Gly Leu Val Thr Ser Leu
                805                 810                 815

Gly Phe Gly His Val Ser Ala Leu Val Ala Ile Val His Pro Asp Ala
            820                 825                 830

Phe Tyr Glu Ala Val Arg Val Ala Arg Gly Ala Glu Ala Ala Asp Val
        835                 840                 845

Trp Arg Ala Ser Ala Ile Ala Arg Glu Glu Ala Gly Leu Arg Thr Ile
    850                 855                 860

Val Ala Gly Met His Gly Gly Val Leu Tyr Glu Arg Pro Val Glu Arg
865                 870                 875                 880

Asn Leu Gly Val His Gly Asp Ala Ala Lys Glu Val Glu Ala Ala Val
                885                 890                 895

Leu Leu Asp Ser Arg Ala Arg Leu Val Asp Gly Val Leu Arg Ala Glu
            900                 905                 910

Gly

<210> 171
<211> 2937

<212> DNA
<213> Corynebacterium glutamicum

<220>
<221> CDS
<222> (101)..(2914)
<223> RXN02168

<400> 171
agggtttagt catctatttc ggctacgctc acgtaaccta cgcattcgta agtggtcatg 60

ggtccaagaa gcccattcca agacttagga gctttatttc gtg agt att tct tca 115
Val Ser Ile Ser Ser
1               5

ctg aca ccg ctg cac tct ttc aaa gag cca gca att ctg tac gcc ggt 163
Leu Thr Pro Leu His Ser Phe Lys Glu Pro Ala Ile Leu Tyr Ala Gly
                10                  15                  20

cag gct tct gcc tgg cag cag gtg atc gct gat tcc agc gaa gac cac 211
Gln Ala Ser Ala Trp Gln Gln Val Ile Ala Asp Ser Ser Glu Asp His
            25                  30                  35

atc acc gca acg cac ctg cgc gag ctc ctg tct cgc tcc cgt gca aag 259
Ile Thr Ala Thr His Leu Arg Glu Leu Leu Ser Arg Ser Arg Ala Lys
        40                  45                  50

act gca cct ttc gct cgc caa atc acc gcc atc gtg cct ggc tca ctt 307
Thr Ala Pro Phe Ala Arg Gln Ile Thr Ala Ile Val Pro Gly Ser Leu
    55                  60                  65

gct cgt ctt gag gaa ctg acc cgc gaa gac gca caa atc ggt gca gac 355
Ala Arg Leu Glu Glu Leu Thr Arg Glu Asp Ala Gln Ile Gly Ala Asp
70                  75                  80                  85

atc gac gca cag cct gcc gtt tcc att cca ggc att ctg ctg gga cag 403
Ile Asp Ala Gln Pro Ala Val Ser Ile Pro Gly Ile Leu Leu Gly Gln
                90                  95                  100

atc gct gca acc cgt cag ctg cgt gac ctc gga ctc gat gtc gca gca 451
Ile Ala Ala Thr Arg Gln Leu Arg Asp Leu Gly Leu Asp Val Ala Ala
            105                 110                 115

gct tcc cgc ctt gga cac tcc cag ggc att ttg ggc gtt gaa gca gta 499
Ala Ser Arg Leu Gly His Ser Gln Gly Ile Leu Gly Val Glu Ala Val
        120                 125                 130

gac aat gaa gaa gac gtt tta gct ttc gcc atc ctg ctg ggc gca gca 547
Asp Asn Glu Glu Asp Val Leu Ala Phe Ala Ile Leu Leu Gly Ala Ala
    135                 140                 145

gct tcc cag ttc gct ggc aag ggc gca cat atg ctc tct gtt cgc ggc 595
Ala Ser Gln Phe Ala Gly Lys Gly Ala His Met Leu Ser Val Arg Gly
150                 155                 160                 165

ctg tcc cgt gag atc atc cag gac acc atc gct ggt gtc gat ggg gta 643
Leu Ser Arg Glu Ile Ile Gln Asp Thr Ile Ala Gly Val Asp Gly Val
                170                 175                 180

gag gtc tcc ctg cgc aac gct cgt gca cac ttt gtt gtc tct ggt aag 691
Glu Val Ser Leu Arg Asn Ala Arg Ala His Phe Val Val Ser Gly Lys

            185                 190                 195

cca gag gca ctg aag aag gct gct gct gct cta cag cgc gca gct gat 739
Pro Glu Ala Leu Lys Lys Ala Ala Ala Ala Leu Gln Arg Ala Ala Asp
        200                 205                 210

gtt tac aac gaa gac atc aac gaa aag cgc aag ggt gga tcc ctg gca 787
Val Tyr Asn Glu Asp Ile Asn Glu Lys Arg Lys Gly Gly Ser Leu Ala
    215                 220                 225

gag cct aag ttt gac tac ttg gat gtg gcc att cct ttc cac cac tcc 835
Glu Pro Lys Phe Asp Tyr Leu Asp Val Ala Ile Pro Phe His His Ser
230                 235                 240                 245

tcc atg cag gac gca gcc gac ttg gct gtc gag tgg gca acc acc tgt 883
Ser Met Gln Asp Ala Ala Asp Leu Ala Val Glu Trp Ala Thr Thr Cys
                250                 255                 260

ggc cta aac gtc aac gcg cgc gcg ttg gca gaa gca att cta gtt aac 931
Gly Leu Asn Val Asn Ala Arg Ala Leu Ala Glu Ala Ile Leu Val Asn
            265                 270                 275

cca gct gac tgg gtt gag cag atc gca aac ctc aag gct gat tac gtt 979
Pro Ala Asp Trp Val Glu Gln Ile Ala Asn Leu Lys Ala Asp Tyr Val
        280                 285                 290

ctt tcc ctc gat gca ggc gtc agc cgt ttc acc gct cca ttg cta gac 1027
Leu Ser Leu Asp Ala Gly Val Ser Arg Phe Thr Ala Pro Leu Leu Asp
    295                 300                 305

ggt cgc gga atc tct ttg gtt cct gcg ttc tcc gct gca gag cgc gac 1075
Gly Arg Gly Ile Ser Leu Val Pro Ala Phe Ser Ala Ala Glu Arg Asp
310                 315                 320                 325

aac ttg gct cgc cct ggc ttc cac gtt cct acc gct gag gat tgg tcc 1123
Asn Leu Ala Arg Pro Gly Phe His Val Pro Thr Ala Glu Asp Trp Ser
                330                 335                 340

gag ttc gct cca aag ctg gtt aag ctt cca aac ggt gag cac aag gtt 1171
Glu Phe Ala Pro Lys Leu Val Lys Leu Pro Asn Gly Glu His Lys Val
            345                 350                 355

ctc acc ggg ttc tcc cgc ctg act ggt tat tcc cca atc gtc ctg gct 1219
Leu Thr Gly Phe Ser Arg Leu Thr Gly Tyr Ser Pro Ile Val Leu Ala
        360                 365                 370

ggc atg acc cca acc acc gtt gat cct gag atc gtt gca gct gca gcg 1267
Gly Met Thr Pro Thr Thr Val Asp Pro Glu Ile Val Ala Ala Ala Ala
    375                 380                 385

aac gct gga cac tgg gcc gaa atg gcc ggt ggc gga cag tac tct gaa 1315
Asn Ala Gly His Trp Ala Glu Met Ala Gly Gly Gly Gln Tyr Ser Glu
390                 395                 400                 405

gaa gtc ttc acc aag aac aag gaa aag ctc gtt tcc ctg ctc aag gtt 1363
Glu Val Phe Thr Lys Asn Lys Glu Lys Leu Val Ser Leu Leu Lys Val
                410                 415                 420

gga cgc tcc gca cag ttc aac tcc atg ttc ttc gac cgc tac atg tgg 1411
Gly Arg Ser Ala Gln Phe Asn Ser Met Phe Phe Asp Arg Tyr Met Trp
            425                 430                 435

aac ctg cag ttc ggt gca cag cgc atc gtt tcc aag gca cgt gca acc 1459
Asn Leu Gln Phe Gly Ala Gln Arg Ile Val Ser Lys Ala Arg Ala Thr
        440                 445                 450

ggt acc tcc atc aac ggt gtt gtt gtc tcc gct ggt atc cca gag gtt 1507
Gly Thr Ser Ile Asn Gly Val Val Val Ser Ala Gly Ile Pro Glu Val
    455                 460                 465

gag gaa gca act gag ctg atc aac gat ctg aac gct gat ggc ttc cca 1555
Glu Glu Ala Thr Glu Leu Ile Asn Asp Leu Asn Ala Asp Gly Phe Pro
470                 475                 480                 485

tac gtt gca ttc aag cca ggc acc gtg gat cag atc cgc gca acc ctg 1603
Tyr Val Ala Phe Lys Pro Gly Thr Val Asp Gln Ile Arg Ala Thr Leu
                490                 495                 500

aag att gct gat gca aac cca gag acc aag atc atc atc cag atc gag 1651
Lys Ile Ala Asp Ala Asn Pro Glu Thr Lys Ile Ile Ile Gln Ile Glu
            505                 510                 515

gac gga cac gct ggt ggc cac cac tcc tgg gtc aac ttg gac gat ctg 1699
Asp Gly His Ala Gly Gly His His Ser Trp Val Asn Leu Asp Asp Leu
        520                 525                 530

ctc ctg acc acc tac gca gag ctg cgt tcc cgc aag aac gtt gtc gtc 1747
Leu Leu Thr Thr Tyr Ala Glu Leu Arg Ser Arg Lys Asn Val Val Val
    535                 540                 545

atg atc ggt ggc ggc atc gga acc cct gca aag gct gct tac tac ctg 1795
Met Ile Gly Gly Gly Ile Gly Thr Pro Ala Lys Ala Ala Tyr Tyr Leu
550                 555                 560                 565

acc ggt gaa tgg tcc acc gat ttg ggc ttc cca gca atg cca gtg gac 1843
Thr Gly Glu Trp Ser Thr Asp Leu Gly Phe Pro Ala Met Pro Val Asp
                570                 575                 580

ggc atc ctc gtg ggt acc gct gcc atg gca acc aag gaa gca acc act 1891
Gly Ile Leu Val Gly Thr Ala Ala Met Ala Thr Lys Glu Ala Thr Thr
            585                 590                 595

tct cct cag gtc aag cag gca ctg gtc gac acc cca ggt gtt gat cca 1939
Ser Pro Gln Val Lys Gln Ala Leu Val Asp Thr Pro Gly Val Asp Pro
        600                 605                 610

cac gac gct ggc ggc tgg gtt ggc cgt ggc gat gct cgt ggt ggc gtg 1987
His Asp Ala Gly Gly Trp Val Gly Arg Gly Asp Ala Arg Gly Gly Val
    615                 620                 625

acc tct ggt ctg tca cac ctg cac gct gac atg tac gag ctg gac aac 2035
Thr Ser Gly Leu Ser His Leu His Ala Asp Met Tyr Glu Leu Asp Asn
630                 635                 640                 645

gat tct gct gca gct tcc cgc ctg atc tct tcc atc gat tct gat gat 2083
Asp Ser Ala Ala Ala Ser Arg Leu Ile Ser Ser Ile Asp Ser Asp Asp
                650                 655                 660

tac gca gat cac cgc gaa gag ctc atc gag gct atc aac aag acc gct 2131
Tyr Ala Asp His Arg Glu Glu Leu Ile Glu Ala Ile Asn Lys Thr Ala
            665                 670                 675

aag cct ttc ttc ggc gag gtc gaa gag atg act tac gca gag tgg atc     2179
Lys Pro Phe Phe Gly Glu Val Glu Glu Met Thr Tyr Ala Glu Trp Ile
        680                 685                 690

cag cgt tgg gtt gag ctt gct tac cca act cag gac cca acc tgg gat     2227
Gln Arg Trp Val Glu Leu Ala Tyr Pro Thr Gln Asp Pro Thr Trp Asp
    695                 700                 705

gat cgt ttc ctc gat ttg gtt cac cgc att gaa gct cgt ctc aac gag     2275
Asp Arg Phe Leu Asp Leu Val His Arg Ile Glu Ala Arg Leu Asn Glu
710                 715                 720                 725

gca gag cac ggc gcc atc acc aca ctg ttc cca gac cat gcg tct gtg     2323
Ala Glu His Gly Ala Ile Thr Thr Leu Phe Pro Asp His Ala Ser Val
                730                 735                 740

gaa aat gag gaa gag gcc gtc gaa aag ctt ctt gct gct tac ccg cag     2371
Glu Asn Glu Glu Glu Ala Val Glu Lys Leu Leu Ala Ala Tyr Pro Gln
            745                 750                 755

gcc cgc gag atc cag gtc tct gcg cgc gac gcc gcg tgg ttt att ggt     2419
Ala Arg Glu Ile Gln Val Ser Ala Arg Asp Ala Ala Trp Phe Ile Gly
        760                 765                 770

ctg tgc cgc aag cac cac aag cct atg cct tgg gtt cca gca atc gat     2467
Leu Cys Arg Lys His His Lys Pro Met Pro Trp Val Pro Ala Ile Asp
    775                 780                 785

gct gac cta gca cgc tgg tgg ggc ctt gac acc ctg tgg cag tcc cag     2515
Ala Asp Leu Ala Arg Trp Trp Gly Leu Asp Thr Leu Trp Gln Ser Gln
790                 795                 800                 805

aac gag cgc tac ggc gcg aac tca gtc cgc gtt atc cca gga cca gtc     2563
Asn Glu Arg Tyr Gly Ala Asn Ser Val Arg Val Ile Pro Gly Pro Val
                810                 815                 820

tcc gtc gcc ggc atc gac cgt gtt gac gag cca gtt gca gag ctg ctc     2611
Ser Val Ala Gly Ile Asp Arg Val Asp Glu Pro Val Ala Glu Leu Leu
            825                 830                 835

ggc cgc ttc gaa gct gcc tgc gtt gac gct ctc gac ggc gag cca gaa     2659
Gly Arg Phe Glu Ala Ala Cys Val Asp Ala Leu Asp Gly Glu Pro Glu
        840                 845                 850

gag atc ttc gct cgc ctc aat gag tcc aag aac gag cgc gaa ttc ctg     2707
Glu Ile Phe Ala Arg Leu Asn Glu Ser Lys Asn Glu Arg Glu Phe Leu
    855                 860                 865

ctg gct acc cca cac atc gtg tgg cac ggc aac ctg atc gac aac cca     2755
Leu Ala Thr Pro His Ile Val Trp His Gly Asn Leu Ile Asp Asn Pro
870                 875                 880                 885

gct cac gtc ctc aac gag ggt gct ttc gag ctc atc gag gag gat ggc     2803
Ala His Val Leu Asn Glu Gly Ala Phe Glu Leu Ile Glu Glu Asp Gly
                890                 895                 900

tac tgg gtc atc cgt atc ctg gct gat tcc tac ttc gac gat ctg cca     2851
Tyr Trp Val Ile Arg Ile Leu Ala Asp Ser Tyr Phe Asp Asp Leu Pro
            905                 910                 915

gtt gag cag cgc cca tac ctg gtt cag cat gtt gac atc cca gtt gag     2899

Val Glu Gln Arg Pro Tyr Leu Val Gln His Val Asp Ile Pro Val Glu
        920                 925                 930

ctg ggt gac gct ggt tgaaccggtg gtttcccaat tgg                    2937
Leu Gly Asp Ala Gly
    935

<210> 172
<211> 938
<212> PRT
<213> Corynebacterium glutamicum

<400> 172
   Val Ser Ile Ser Ser Leu Thr Pro Leu His Ser Phe Lys Glu Pro Ala
     1               5                  10                  15
   Ile Leu Tyr Ala Gly Gln Ala Ser Ala Trp Gln Gln Val Ile Ala Asp
                20                  25                  30
   Ser Ser Glu Asp His Ile Thr Ala Thr His Leu Arg Glu Leu Leu Ser
            35                  40                  45
   Arg Ser Arg Ala Lys Thr Ala Pro Phe Ala Arg Gln Ile Thr Ala Ile
        50                  55                  60
   Val Pro Gly Ser Leu Ala Arg Leu Glu Glu Leu Thr Arg Glu Asp Ala
    65                  70                  75                  80
   Gln Ile Gly Ala Asp Ile Asp Ala Gln Pro Ala Val Ser Ile Pro Gly
                    85                  90                  95
   Ile Leu Leu Gly Gln Ile Ala Ala Thr Arg Gln Leu Arg Asp Leu Gly
               100                 105                 110
   Leu Asp Val Ala Ala Ala Ser Arg Leu Gly His Ser Gln Gly Ile Leu
           115                 120                 125
   Gly Val Glu Ala Val Asp Asn Glu Glu Asp Val Leu Ala Phe Ala Ile
       130                 135                 140
   Leu Leu Gly Ala Ala Ala Ser Gln Phe Ala Gly Lys Gly Ala His Met
   145                 150                 155                 160
   Leu Ser Val Arg Gly Leu Ser Arg Glu Ile Ile Gln Asp Thr Ile Ala
                   165                 170                 175
   Gly Val Asp Gly Val Glu Val Ser Leu Arg Asn Ala Arg Ala His Phe
               180                 185                 190
   Val Val Ser Gly Lys Pro Glu Ala Leu Lys Lys Ala Ala Ala Ala Leu
           195                 200                 205
   Gln Arg Ala Ala Asp Val Tyr Asn Glu Asp Ile Asn Glu Lys Arg Lys
       210                 215                 220
   Gly Gly Ser Leu Ala Glu Pro Lys Phe Asp Tyr Leu Asp Val Ala Ile
   225                 230                 235                 240
   Pro Phe His His Ser Ser Met Gln Asp Ala Ala Asp Leu Ala Val Glu
                   245                 250                 255
   Trp Ala Thr Thr Cys Gly Leu Asn Val Asn Ala Arg Ala Leu Ala Glu
               260                 265                 270
   Ala Ile Leu Val Asn Pro Ala Asp Trp Val Glu Gln Ile Ala Asn Leu
           275                 280                 285
   Lys Ala Asp Tyr Val Leu Ser Leu Asp Ala Gly Val Ser Arg Phe Thr
       290                 295                 300
   Ala Pro Leu Leu Asp Gly Arg Gly Ile Ser Leu Val Pro Ala Phe Ser
   305                 310                 315                 320
   Ala Ala Glu Arg Asp Asn Leu Ala Arg Pro Gly Phe His Val Pro Thr
                   325                 330                 335
   Ala Glu Asp Trp Ser Glu Phe Ala Pro Lys Leu Val Lys Leu Pro Asn
               340                 345                 350
   Gly Glu His Lys Val Leu Thr Gly Phe Ser Arg Leu Thr Gly Tyr Ser
           355                 360                 365
   Pro Ile Val Leu Ala Gly Met Thr Pro Thr Thr Val Asp Pro Glu Ile
       370                 375                 380
   Val Ala Ala Ala Ala Asn Ala Gly His Trp Ala Glu Met Ala Gly Gly
   385                 390                 395                 400
   Gly Gln Tyr Ser Glu Glu Val Phe Thr Lys Asn Lys Glu Lys Leu Val
                   405                 410                 415
   Ser Leu Leu Lys Val Gly Arg Ser Ala Gln Phe Asn Ser Met Phe Phe
               420                 425                 430
   Asp Arg Tyr Met Trp Asn Leu Gln Phe Gly Ala Gln Arg Ile Val Ser
           435                 440                 445
   Lys Ala Arg Ala Thr Gly Thr Ser Ile Asn Gly Val Val Val Ser Ala
       450                 455                 460
   Gly Ile Pro Glu Val Glu Glu Ala Thr Glu Leu Ile Asn Asp Leu Asn
   465                 470                 475                 480
   Ala Asp Gly Phe Pro Tyr Val Ala Phe Lys Pro Gly Thr Val Asp Gln
                   485                 490                 495
   Ile Arg Ala Thr Leu Lys Ile Ala Asp Ala Asn Pro Glu Thr Lys Ile
               500                 505                 510
   Ile Ile Gln Ile Glu Asp Gly His Ala Gly Gly His His Ser Trp Val
           515                 520                 525
   Asn Leu Asp Asp Leu Leu Leu Thr Thr Tyr Ala Glu Leu Arg Ser Arg
       530                 535                 540
   Lys Asn Val Val Val Met Ile Gly Gly Gly Ile Gly Thr Pro Ala Lys
   545                 550                 555                 560
   Ala Ala Tyr Tyr Leu Thr Gly Glu Trp Ser Thr Asp Leu Gly Phe Pro
                   565                 570                 575
   Ala Met Pro Val Asp Gly Ile Leu Val Gly Thr Ala Ala Met Ala Thr
               580                 585                 590
   Lys Glu Ala Thr Thr Ser Pro Gln Val Lys Gln Ala Leu Val Asp Thr
           595                 600                 605
   Pro Gly Val Asp Pro His Asp Ala Gly Gly Trp Val Gly Arg Gly Asp
       610                 615                 620
   Ala Arg Gly Gly Val Thr Ser Gly Leu Ser His Leu His Ala Asp Met
   625                 630                 635                 640
   Tyr Glu Leu Asp Asn Asp Ser Ala Ala Ala Ser Arg Leu Ile Ser Ser
                   645                 650                 655
   Ile Asp Ser Asp Asp Tyr Ala Asp His Arg Glu Glu Leu Ile Glu Ala
               660                 665                 670
   Ile Asn Lys Thr Ala Lys Pro Phe Phe Gly Glu Val Glu Glu Met Thr
           675                 680                 685
   Tyr Ala Glu Trp Ile Gln Arg Trp Val Glu Leu Ala Tyr Pro Thr Gln
       690                 695                 700
   Asp Pro Thr Trp Asp Asp Arg Phe Leu Asp Leu Val His Arg Ile Glu
   705                 710                 715                 720
   Ala Arg Leu Asn Glu Ala Glu His Gly Ala Ile Thr Thr Leu Phe Pro
                   725                 730                 735
   Asp His Ala Ser Val Glu Asn Glu Glu Glu Ala Val Glu Lys Leu Leu
               740                 745                 750
   Ala Ala Tyr Pro Gln Ala Arg Glu Ile Gln Val Ser Ala Arg Asp Ala
           755                 760                 765
   Ala Trp Phe Ile Gly Leu Cys Arg Lys His His Lys Pro Met Pro Trp
       770                 775                 780
   Val Pro Ala Ile Asp Ala Asp Leu Ala Arg Trp Trp Gly Leu Asp Thr
   785                 790                 795                 800
   Leu Trp Gln Ser Gln Asn Glu Arg Tyr Gly Ala Asn Ser Val Arg Val
                   805                 810                 815
   Ile Pro Gly Pro Val Ser Val Ala Gly Ile Asp Arg Val Asp Glu Pro
               820                 825                 830
   Val Ala Glu Leu Leu Gly Arg Phe Glu Ala Ala Cys Val Asp Ala Leu
           835                 840                 845
   Asp Gly Glu Pro Glu Glu Ile Phe Ala Arg Leu Asn Glu Ser Lys Asn
       850                 855                 860
   Glu Arg Glu Phe Leu Leu Ala Thr Pro His Ile Val Trp His Gly Asn
   865                 870                 875                 880
   Leu Ile Asp Asn Pro Ala His Val Leu Asn Glu Gly Ala Phe Glu Leu
                   885                 890                 895
   Ile Glu Glu Asp Gly Tyr Trp Val Ile Arg Ile Leu Ala Asp Ser Tyr
   900                 905                 910
   Phe Asp Asp Leu Pro Val Glu Gln Arg Pro Tyr Leu Val Gln His Val
           915                 920                 925
   Asp Ile Pro Val Glu Leu Gly Asp Ala Gly
       930                 935

<210> 173
<211> 921
<212> DNA
<213> Corynebacterium glutamicum

<220>
<221> CDS
<222> (101)..(898)
<223> RXN01090

<400> 173
gccggtttgg gctggttgga gctctagatc gtaagtggtg tcgtacccat gacgtaccat 60

taaccacgaa cgttttaaag aagccacgaa ggagcctgac atg gcg tta cca cta   115
                                           Met Ala Leu Pro Leu
                                           1               5

ccc agc aag agc gct cga gca ctt gtt act ggg gca agc caa ggc att   163
Pro Ser Lys Ser Ala Arg Ala Leu Val Thr Gly Ala Ser Gln Gly Ile
                10                  15                  20

ggc ctc gcc atc gcc aaa gat ttg gcg cgg tat ggg cac aac ctc att   211
Gly Leu Ala Ile Ala Lys Asp Leu Ala Arg Tyr Gly His Asn Leu Ile
            25                  30                  35

ttg gtt gct cgc cgc gag gat gtc ctc aaa gag atc gcc gca gat cta   259
Leu Val Ala Arg Arg Glu Asp Val Leu Lys Glu Ile Ala Ala Asp Leu
        40                  45                  50

gag aag aag cac ggc gtg atc gtt gag gtc cgc ccg gtg gat ttg agt   307
Glu Lys Lys His Gly Val Ile Val Glu Val Arg Pro Val Asp Leu Ser
    55                  60                  65

gat gag cca gcc cgc aag gtg ttg atc gat gag atc aag aca agg gaa   355
Asp Glu Pro Ala Arg Lys Val Leu Ile Asp Glu Ile Lys Thr Arg Glu
70                  75                  80                  85

atc aac atc atc att aac tct gct ggc atc gca agc ttt ggg ccg ttc   403
Ile Asn Ile Ile Ile Asn Ser Ala Gly Ile Ala Ser Phe Gly Pro Phe
                90                  95                  100

aag gac cag gat tgg tct tat gag act gcc cag ttc tca ctt aat gcc   451
Lys Asp Gln Asp Trp Ser Tyr Glu Thr Ala Gln Phe Ser Leu Asn Ala
            105                 110                 115

aca gcc gtt ttt gag ctc acc cac gcg gtg ttg ggt ggc atg att gac   499
Thr Ala Val Phe Glu Leu Thr His Ala Val Leu Gly Gly Met Ile Asp
        120                 125                 130

cgt ggc acg ggc gct att tgc aat gtg gga tct gcg gct ggc aat gtg   547
Arg Gly Thr Gly Ala Ile Cys Asn Val Gly Ser Ala Ala Gly Asn Val
    135                 140                 145

cca atc ccc aac aac gcc acg tat gtg ctc acc aag gct ggc gtg aac 595
Pro Ile Pro Asn Asn Ala Thr Tyr Val Leu Thr Lys Ala Gly Val Asn
150                 155                 160                 165

gcg ttc acc gag gca atg cat tat gag ctg cgc gga act ggt gtg gcg 643
Ala Phe Thr Glu Ala Met His Tyr Glu Leu Arg Gly Thr Gly Val Ala
                170                 175                 180

tgt act ttg ctc gca ccg gga cct gtc cgt gag gcg gag atc cct gag 691
Cys Thr Leu Leu Ala Pro Gly Pro Val Arg Glu Ala Glu Ile Pro Glu
            185                 190                 195

tct gag aag tcg atc gtg gac aag gtt gtc cct gat ttc ttg tgg acc 739
Ser Glu Lys Ser Ile Val Asp Lys Val Val Pro Asp Phe Leu Trp Thr
        200                 205                 210

acc tat gag tcc tgc tcc gca gag acc ttg cgt gcg ctg tct aag aat 787
Thr Tyr Glu Ser Cys Ser Ala Glu Thr Leu Arg Ala Leu Ser Lys Asn
    215                 220                 225

cag cgt cgc gtt gtt cca ggt ccg ctg tcc aag gcc atg aat ttt gtg 835
Gln Arg Arg Val Val Pro Gly Pro Leu Ser Lys Ala Met Asn Phe Val
230                 235                 240                 245

tcc tct gtt gct cca acc gct gtg ctc tcc cct gtt atg ggc tgg gtt 883
Ser Ser Val Ala Pro Thr Ala Val Leu Ser Pro Val Met Gly Trp Val
                250                 255                 260

tat aag aag atg ggt tagtttttaa aagtgtctga atc 921
Tyr Lys Lys Met Gly
            265

<210> 174
<211> 266
<212> PRT
<213> Corynebacterium glutamicum

<400> 174
Met Ala Leu Pro Leu Pro Ser Lys Ser Ala Arg Ala Leu Val Thr Gly
1               5                   10                  15

Ala Ser Gln Gly Ile Gly Leu Ala Ile Ala Lys Asp Leu Ala Arg Tyr
            20                  25                  30

Gly His Asn Leu Ile Leu Val Ala Arg Arg Glu Asp Val Leu Lys Glu
        35                  40                  45

Ile Ala Ala Asp Leu Glu Lys Lys His Gly Val Ile Val Glu Val Arg
    50                  55                  60

Pro Val Asp Leu Ser Asp Glu Pro Ala Arg Lys Val Leu Ile Asp Glu
65                  70                  75                  80

Ile Lys Thr Arg Glu Ile Asn Ile Ile Ile Asn Ser Ala Gly Ile Ala
                85                  90                  95

Ser Phe Gly Pro Phe Lys Asp Gln Asp Trp Ser Tyr Glu Thr Ala Gln
            100                 105                 110

Phe Ser Leu Asn Ala Thr Ala Val Phe Glu Leu Thr His Ala Val Leu
        115                 120                 125

Gly Gly Met Ile Asp Arg Gly Thr Gly Ala Ile Cys Asn Val Gly Ser
    130                 135                 140

Ala Ala Gly Asn Val Pro Ile Pro Asn Asn Ala Thr Tyr Val Leu Thr
145                 150                 155                 160

Lys Ala Gly Val Asn Ala Phe Thr Glu Ala Met His Tyr Glu Leu Arg
                165                 170                 175

Gly Thr Gly Val Ala Cys Thr Leu Leu Ala Pro Gly Pro Val Arg Glu
            180                 185                 190

Ala Glu Ile Pro Glu Ser Glu Lys Ser Ile Val Asp Lys Val Val Pro
        195                 200                 205

Asp Phe Leu Trp Thr Thr Tyr Glu Ser Cys Ser Ala Glu Thr Leu Arg
    210                 215                 220

Ala Leu Ser Lys Asn Gln Arg Arg Val Val Pro Gly Pro Leu Ser Lys
225                 230                 235                 240

Ala Met Asn Phe Val Ser Ser Val Ala Pro Thr Ala Val Leu Ser Pro
                245                 250                 255

Val Met Gly Trp Val Tyr Lys Lys Met Gly
            260                 265

<210> 175
<211> 1293
<212> DNA
<213> Corynebacterium glutamicum

<220>
<221> CDS
<222> (101)..(1270)
<223> RXN02062

<400> 175
ttgtctaaac atcgttttgg ggtccgaatg atagcccctt ttaatgcccc catttcggta 60

tcgctgcgca actgttttta gatggctaat ctttgaaatt atg aga gtc gga atg 115
Met Arg Val Gly Met
1               5

atg aca aga gag tat cca cca gag gtt tac ggc ggc gct ggc gtg cac 163
Met Thr Arg Glu Tyr Pro Pro Glu Val Tyr Gly Gly Ala Gly Val His
                10                  15                  20

gtc acc gaa ttg acc cga ttc atg cgt gag atc gct gaa gtt gat gtt 211
Val Thr Glu Leu Thr Arg Phe Met Arg Glu Ile Ala Glu Val Asp Val
            25                  30                  35

cac tgc atg ggt gca cct cgc gat atg gag gga gtt ttc gtc cac ggc 259
His Cys Met Gly Ala Pro Arg Asp Met Glu Gly Val Phe Val His Gly
        40                  45                  50

gtc gat cct gcc ttg gaa agc gcg aac cct gcg att aag aca ctg tcc 307

Val Asp Pro Ala Leu Glu Ser Ala Asn Pro Ala Ile Lys Thr Leu Ser
    55                  60                  65

acc ggt tta cgc atg gca gaa gct gca aac aac gtg gat gtc gtg cac     355
Thr Gly Leu Arg Met Ala Glu Ala Ala Asn Asn Val Asp Val Val His
 70                  75                  80                  85

tca cac act tgg tat gca ggt ctt ggc ggc cac ctt gca gct cgt ctc     403
Ser His Thr Trp Tyr Ala Gly Leu Gly Gly His Leu Ala Ala Arg Leu
                 90                  95                 100

cac ggc att cct cac gtg gct acc gcg cac tct ttg gag cca gat cgc     451
His Gly Ile Pro His Val Ala Thr Ala His Ser Leu Glu Pro Asp Arg
            105                 110                 115

cca tgg aag cgt gag cag ctt ggc ggt gga tac gac gtg tcc tcc tgg     499
Pro Trp Lys Arg Glu Gln Leu Gly Gly Gly Tyr Asp Val Ser Ser Trp
        120                 125                 130

tct gaa aaa aat gcc atg gaa tac gct gac gcg gtc atc gct gtg tcg     547
Ser Glu Lys Asn Ala Met Glu Tyr Ala Asp Ala Val Ile Ala Val Ser
    135                 140                 145

gct cgc atg aaa gat tcc atc ctc gct gcg tac cct cgc atc gag ccg     595
Ala Arg Met Lys Asp Ser Ile Leu Ala Ala Tyr Pro Arg Ile Glu Pro
150                 155                 160                 165

gac aac gtg cgt gtt gtc ctc aac ggc atc gac act gag ttg tgg cag     643
Asp Asn Val Arg Val Val Leu Asn Gly Ile Asp Thr Glu Leu Trp Gln
                170                 175                 180

cct cgc ccg act ttc gat gac gcg gaa gat tcc gta ctc cgc tcc cta     691
Pro Arg Pro Thr Phe Asp Asp Ala Glu Asp Ser Val Leu Arg Ser Leu
            185                 190                 195

ggc gtt gac cca cag cgg ccc atc gtc gca ttt gtc ggc cgc atc acc     739
Gly Val Asp Pro Gln Arg Pro Ile Val Ala Phe Val Gly Arg Ile Thr
        200                 205                 210

cgc caa aaa ggc gtc gag cac ctc atc aag gca gca gcg ctt ttc gac     787
Arg Gln Lys Gly Val Glu His Leu Ile Lys Ala Ala Ala Leu Phe Asp
    215                 220                 225

gag tcc gtg cag ctt gtg ctc tgt gcc ggc gcg cca gac acc ccc gaa     835
Glu Ser Val Gln Leu Val Leu Cys Ala Gly Ala Pro Asp Thr Pro Glu
230                 235                 240                 245

atc gca gct cgc acc acc gcc ctg gtg gaa gaa ctc cag gca aag cgc     883
Ile Ala Ala Arg Thr Thr Ala Leu Val Glu Glu Leu Gln Ala Lys Arg
                250                 255                 260

gaa ggc att ttc tgg gtt cag gac atg ctg ggc aag gac aaa atc caa     931
Glu Gly Ile Phe Trp Val Gln Asp Met Leu Gly Lys Asp Lys Ile Gln
            265                 270                 275

gag att ctc acc gct gct gac acc ttc gtg tgc cca tcc att tac gag     979
Glu Ile Leu Thr Ala Ala Asp Thr Phe Val Cys Pro Ser Ile Tyr Glu
        280                 285                 290

cca ctg ggc atc gtg aac ttg gaa gca atg gcc tgc aac acc gca gtt     1027
Pro Leu Gly Ile Val Asn Leu Glu Ala Met Ala Cys Asn Thr Ala Val

295                 300                 305

gtc gca tcc gac gtt gga ggc atc cct gag gtt gtt gtc gac ggc acc      1075
Val Ala Ser Asp Val Gly Gly Ile Pro Glu Val Val Val Asp Gly Thr
310                 315                 320                 325

acc ggc gcc ctc gtt cac tac gac gaa aat gat gtc gaa acc ttc gag      1123
Thr Gly Ala Leu Val His Tyr Asp Glu Asn Asp Val Glu Thr Phe Glu
                330                 335                 340

cgc gat atc gcc gaa gcg gtg aat aaa atg gtc gct gat cga gag acc      1171
Arg Asp Ile Ala Glu Ala Val Asn Lys Met Val Ala Asp Arg Glu Thr
            345                 350                 355

gca gcc aaa ttt ggt ctc gca ggg cgc gaa cgt gct atc aat gat ttc      1219
Ala Ala Lys Phe Gly Leu Ala Gly Arg Glu Arg Ala Ile Asn Asp Phe
        360                 365                 370

tcc tgg gca acg att gct cag cag acc att gat gtg tac aaa tcc ttg      1267
Ser Trp Ala Thr Ile Ala Gln Gln Thr Ile Asp Val Tyr Lys Ser Leu
    375                 380                 385

atg taaaaccgaa agccggggaa cct                                        1293
Met
390


<210> 176
<211> 390
<212> PRT
<213> Corynebacterium glutamicum

<400> 176
Met Arg Val Gly Met Met Thr Arg Glu Tyr Pro Pro Glu Val Tyr Gly
1               5                   10                  15

Gly Ala Gly Val His Val Thr Glu Leu Thr Arg Phe Met Arg Glu Ile
            20                  25                  30

Ala Glu Val Asp Val His Cys Met Gly Ala Pro Arg Asp Met Glu Gly
        35                  40                  45

Val Phe Val His Gly Val Asp Pro Ala Leu Glu Ser Ala Asn Pro Ala
    50                  55                  60

Ile Lys Thr Leu Ser Thr Gly Leu Arg Met Ala Glu Ala Ala Asn Asn
65                  70                  75                  80

Val Asp Val Val His Ser His Thr Trp Tyr Ala Gly Leu Gly Gly His
                85                  90                  95

Leu Ala Ala Arg Leu His Gly Ile Pro His Val Ala Thr Ala His Ser
            100                 105                 110

Leu Glu Pro Asp Arg Pro Trp Lys Arg Glu Gln Leu Gly Gly Gly Tyr
        115                 120                 125

Asp Val Ser Ser Trp Ser Glu Lys Asn Ala Met Glu Tyr Ala Asp Ala
    130                 135                 140

Val Ile Ala Val Ser Ala Arg Met Lys Asp Ser Ile Leu Ala Ala Tyr

145                 150                 155                 160

Pro Arg Ile Glu Pro Asp Asn Val Arg Val Val Leu Asn Gly Ile Asp
                165                 170                 175

Thr Glu Leu Trp Gln Pro Arg Pro Thr Phe Asp Asp Ala Glu Asp Ser
            180                 185                 190

Val Leu Arg Ser Leu Gly Val Asp Pro Gln Arg Pro Ile Val Ala Phe
        195                 200                 205

Val Gly Arg Ile Thr Arg Gln Lys Gly Val Glu His Leu Ile Lys Ala
    210                 215                 220

Ala Ala Leu Phe Asp Glu Ser Val Gln Leu Val Leu Cys Ala Gly Ala
225                 230                 235                 240

Pro Asp Thr Pro Glu Ile Ala Ala Arg Thr Thr Ala Leu Val Glu Glu
                245                 250                 255

Leu Gln Ala Lys Arg Glu Gly Ile Phe Trp Val Gln Asp Met Leu Gly
            260                 265                 270

Lys Asp Lys Ile Gln Glu Ile Leu Thr Ala Ala Asp Thr Phe Val Cys
        275                 280                 285

Pro Ser Ile Tyr Glu Pro Leu Gly Ile Val Asn Leu Glu Ala Met Ala
    290                 295                 300

Cys Asn Thr Ala Val Val Ala Ser Asp Val Gly Gly Ile Pro Glu Val
305                 310                 315                 320

Val Val Asp Gly Thr Thr Gly Ala Leu Val His Tyr Asp Glu Asn Asp
                325                 330                 335

Val Glu Thr Phe Glu Arg Asp Ile Ala Glu Ala Val Asn Lys Met Val
            340                 345                 350

Ala Asp Arg Glu Thr Ala Ala Lys Phe Gly Leu Ala Gly Arg Glu Arg
        355                 360                 365

Ala Ile Asn Asp Phe Ser Trp Ala Thr Ile Ala Gln Gln Thr Ile Asp
    370                 375                 380

Val Tyr Lys Ser Leu Met
385                 390


<210> 177
<211> 1266
<212> DNA
<213> Corynebacterium glutamicum

<220>
<221> CDS
<222> (101)..(1243)
<223> RXN02148

<400> 177
gcctcagact acggaatccc tgtgcaggtg gtatctgttg attcagcacc gttttatggt 60

gcgcgtcgct actaagaaat agttcgtcag gagaatcttt gtg tct gca tcc cga 115
Val Ser Ala Ser Arg
1               5

aaa act ctc gtt gtg acc aat gat ttt cct cca cgg atc ggc gga atc 163
Lys Thr Leu Val Val Thr Asn Asp Phe Pro Pro Arg Ile Gly Gly Ile
                10                  15                  20

caa agc tat ttg agg gat ttc atc gct act caa gat cct gag tcg atc 211
Gln Ser Tyr Leu Arg Asp Phe Ile Ala Thr Gln Asp Pro Glu Ser Ile
            25                  30                  35

gtg gtg ttt gcg tcg act caa aac gct gag gaa gcg cat gcc tac gac 259
Val Val Phe Ala Ser Thr Gln Asn Ala Glu Glu Ala His Ala Tyr Asp
        40                  45                  50

aag act ttg gac tat gag gtc att agg tgg cct cgt tcg gtg atg ctg 307
Lys Thr Leu Asp Tyr Glu Val Ile Arg Trp Pro Arg Ser Val Met Leu
    55                  60                  65

ccc acc cca aca acg gca cac gct atg gcg gag atc att cgt gag cga 355
Pro Thr Pro Thr Thr Ala His Ala Met Ala Glu Ile Ile Arg Glu Arg
70                  75                  80                  85

gag atc gat aat gtg tgg ttc ggt gct gcg gct ccg ttg gcg ttg atg 403
Glu Ile Asp Asn Val Trp Phe Gly Ala Ala Ala Pro Leu Ala Leu Met
                90                  95                  100

gca ggc aca gcg aag cag gca ggt gcg agc aag gtt att gcc tcc act 451
Ala Gly Thr Ala Lys Gln Ala Gly Ala Ser Lys Val Ile Ala Ser Thr
            105                 110                 115

cat ggg cat gag gtg ggg tgg tca atg ctt cct gga tcg aga caa tcg 499
His Gly His Glu Val Gly Trp Ser Met Leu Pro Gly Ser Arg Gln Ser
        120                 125                 130

ttg cgc aaa atc ggc act gag gtg gac gtg ctg act tat atc tcg cag 547
Leu Arg Lys Ile Gly Thr Glu Val Asp Val Leu Thr Tyr Ile Ser Gln
    135                 140                 145

tac acg ctg cgc aga ttc aaa agt gct ttt gga tcg cac ccg act ttt 595
Tyr Thr Leu Arg Arg Phe Lys Ser Ala Phe Gly Ser His Pro Thr Phe
150                 155                 160                 165

gaa cat ttg cct tcc ggc gtg gac gtt aag aga ttc act ccg gcc acg 643
Glu His Leu Pro Ser Gly Val Asp Val Lys Arg Phe Thr Pro Ala Thr
                170                 175                 180

cca gag gac aaa agc gca aca agg aaa aag cta gga ttc acg gac acc 691
Pro Glu Asp Lys Ser Ala Thr Arg Lys Lys Leu Gly Phe Thr Asp Thr
            185                 190                 195

acc ccg gtt att gcc tgt aac tcg cgt tta gtg ccg agg aaa ggc cag 739
Thr Pro Val Ile Ala Cys Asn Ser Arg Leu Val Pro Arg Lys Gly Gln
        200                 205                 210

gac tcg ctg atc aag gcg atg cca cag gtg att gcg gcg cgc cca gat 787
Asp Ser Leu Ile Lys Ala Met Pro Gln Val Ile Ala Ala Arg Pro Asp
    215                 220                 225

gcg cag ttg ctc att gtg ggc agt ggg cga tac gag tcg act ttg cgg 835

Ala Gln Leu Leu Ile Val Gly Ser Gly Arg Tyr Glu Ser Thr Leu Arg
230                 235                 240                 245

cgc ctt gcc act gac gtg agc cag aac gtg aaa ttc ctt ggg cgc ctt     883
Arg Leu Ala Thr Asp Val Ser Gln Asn Val Lys Phe Leu Gly Arg Leu
                250                 255                 260

gaa tat caa gat atg atc aac acg ctt gcc gcc gca gat att ttc gcg     931
Glu Tyr Gln Asp Met Ile Asn Thr Leu Ala Ala Ala Asp Ile Phe Ala
            265                 270                 275

atg cca gcg cgc acc cgc ggt ggc gga ctt gat gtt gaa ggc ttg ggc     979
Met Pro Ala Arg Thr Arg Gly Gly Gly Leu Asp Val Glu Gly Leu Gly
        280                 285                 290

att gtc tat ctc gag gca caa gcc tgc gga gtg ccg gtg ata gcc ggc     1027
Ile Val Tyr Leu Glu Ala Gln Ala Cys Gly Val Pro Val Ile Ala Gly
    295                 300                 305

acc tct ggc ggc gcg cca gag acg gtg act ccg gca act ggc ctg gtt     1075
Thr Ser Gly Gly Ala Pro Glu Thr Val Thr Pro Ala Thr Gly Leu Val
310                 315                 320                 325

gtg gag ggg tcg gac gtc gat aag ctg tct gag ctt tta att gag ctt     1123
Val Glu Gly Ser Asp Val Asp Lys Leu Ser Glu Leu Leu Ile Glu Leu
                330                 335                 340

ctc gac gat ccg atc cgc cgc gcc gcg atg ggc gct gca ggt agg gcg     1171
Leu Asp Asp Pro Ile Arg Arg Ala Ala Met Gly Ala Ala Gly Arg Ala
            345                 350                 355

cat gtg gag gcc gaa tgg tcg tgg gaa atc atg ggg gag cgg ttg acc     1219
His Val Glu Ala Glu Trp Ser Trp Glu Ile Met Gly Glu Arg Leu Thr
        360                 365                 370

aat att ttg cag agt gaa cca cga tgatggttgg acagctgttg ata          1266
Asn Ile Leu Gln Ser Glu Pro Arg
    375                 380

<210> 178
<211> 381
<212> PRT
<213> Corynebacterium glutamicum

<400> 178
Val Ser Ala Ser Arg Lys Thr Leu Val Val Thr Asn Asp Phe Pro Pro
1               5                   10                  15

Arg Ile Gly Gly Ile Gln Ser Tyr Leu Arg Asp Phe Ile Ala Thr Gln
            20                  25                  30

Asp Pro Glu Ser Ile Val Val Phe Ala Ser Thr Gln Asn Ala Glu Glu
        35                  40                  45

Ala His Ala Tyr Asp Lys Thr Leu Asp Tyr Glu Val Ile Arg Trp Pro
    50                  55                  60

Arg Ser Val Met Leu Pro Thr Pro Thr Thr Ala His Ala Met Ala Glu
65                  70                  75                  80

Ile Ile Arg Glu Arg Glu Ile Asp Asn Val Trp Phe Gly Ala Ala Ala
                85                  90                  95

Pro Leu Ala Leu Met Ala Gly Thr Ala Lys Gln Ala Gly Ala Ser Lys
            100                 105                 110

Val Ile Ala Ser Thr His Gly His Glu Val Gly Trp Ser Met Leu Pro
        115                 120                 125

Gly Ser Arg Gln Ser Leu Arg Lys Ile Gly Thr Glu Val Asp Val Leu
    130                 135                 140

Thr Tyr Ile Ser Gln Tyr Thr Leu Arg Arg Phe Lys Ser Ala Phe Gly
145                 150                 155                 160

Ser His Pro Thr Phe Glu His Leu Pro Ser Gly Val Asp Val Lys Arg
                165                 170                 175

Phe Thr Pro Ala Thr Pro Glu Asp Lys Ser Ala Thr Arg Lys Lys Leu
            180                 185                 190

Gly Phe Thr Asp Thr Thr Pro Val Ile Ala Cys Asn Ser Arg Leu Val
        195                 200                 205

Pro Arg Lys Gly Gln Asp Ser Leu Ile Lys Ala Met Pro Gln Val Ile
    210                 215                 220

Ala Ala Arg Pro Asp Ala Gln Leu Leu Ile Val Gly Ser Gly Arg Tyr
225                 230                 235                 240

Glu Ser Thr Leu Arg Arg Leu Ala Thr Asp Val Ser Gln Asn Val Lys
                245                 250                 255

Phe Leu Gly Arg Leu Glu Tyr Gln Asp Met Ile Asn Thr Leu Ala Ala
            260                 265                 270

Ala Asp Ile Phe Ala Met Pro Ala Arg Thr Arg Gly Gly Gly Leu Asp
        275                 280                 285

Val Glu Gly Leu Gly Ile Val Tyr Leu Glu Ala Gln Ala Cys Gly Val
    290                 295                 300

Pro Val Ile Ala Gly Thr Ser Gly Gly Ala Pro Glu Thr Val Thr Pro
305                 310                 315                 320

Ala Thr Gly Leu Val Val Glu Gly Ser Asp Val Asp Lys Leu Ser Glu
                325                 330                 335

Leu Leu Ile Glu Leu Leu Asp Asp Pro Ile Arg Arg Ala Ala Met Gly
            340                 345                 350

Ala Ala Gly Arg Ala His Val Glu Ala Glu Trp Ser Trp Glu Ile Met
        355                 360                 365

Gly Glu Arg Leu Thr Asn Ile Leu Gln Ser Glu Pro Arg
    370                 375                 380

<210> 179
<211> 1287
<212> DNA

<213> Corynebacterium glutamicum

<220>
<221> CDS
<222> (101)..(1264)
<223> RXN02595

<400> 179
gtgggtaaag gggactccga ggaagtccac gtcgtcttct ttcgcggcgc tgaggatggt 60

ttcgcggatt tgtgcggggg agtgggtggg agagaaaacg gtg atc gtt gtg gcc 115
Val Ile Val Val Ala
1               5

atg gct tcc att atg gct tgt tta aaa gca gct aga ctg aat aac cct 163
Met Ala Ser Ile Met Ala Cys Leu Lys Ala Ala Arg Leu Asn Asn Pro
                10                  15                  20

atg aag atc ctt ttg ttg tgc tgg cgt gat acc act cat cct caa ggt 211
Met Lys Ile Leu Leu Leu Cys Trp Arg Asp Thr Thr His Pro Gln Gly
            25                  30                  35

ggc gga agt gaa cgc tat ctg gag cgg gtg ggt gag ttt ttg gcg gat 259
Gly Gly Ser Glu Arg Tyr Leu Glu Arg Val Gly Glu Phe Leu Ala Asp
        40                  45                  50

cag ggc cat gag gtg gtg ttt cgt act gct ggg cac acg gat gcg cca 307
Gln Gly His Glu Val Val Phe Arg Thr Ala Gly His Thr Asp Ala Pro
    55                  60                  65

cgg cgt tct ttc cgc gat ggt gtg agg tat tcc agg agc ggt ggg aag 355
Arg Arg Ser Phe Arg Asp Gly Val Arg Tyr Ser Arg Ser Gly Gly Lys
70                  75                  80                  85

ttt agt gtg tat ccc aag gcg tgg gtg gcc atg atg ttg ggt cgt gtg 403
Phe Ser Val Tyr Pro Lys Ala Trp Val Ala Met Met Leu Gly Arg Val
                90                  95                  100

ggg att ggc acg ttt tcc aag gtt gat gtg gtg gtg gat acg cag aat 451
Gly Ile Gly Thr Phe Ser Lys Val Asp Val Val Val Asp Thr Gln Asn
            105                 110                 115

ggc att ccg ttt ttt gga aag ttt ttc tcc ggt aag ccg act gtg ttg 499
Gly Ile Pro Phe Phe Gly Lys Phe Phe Ser Gly Lys Pro Thr Val Leu
        120                 125                 130

ctc acg cat cat tgc cat aag gag cag tgg ccg gtg gtg ggt cgg gtg 547
Leu Thr His His Cys His Lys Glu Gln Trp Pro Val Val Gly Arg Val
    135                 140                 145

ctg gcg aag gtt ggt tgg ctg att gag agc cag atc gcg ccg cgc gct 595
Leu Ala Lys Val Gly Trp Leu Ile Glu Ser Gln Ile Ala Pro Arg Ala
150                 155                 160                 165

tac aaa act gcg ccg tat gtg act gtt tca gag ccg agc gct gag gag 643
Tyr Lys Thr Ala Pro Tyr Val Thr Val Ser Glu Pro Ser Ala Glu Glu
                170                 175                 180

ctc att gcg ttg ggt gtg gat cag cag cgg att cat atc gtg cgc aat 691
Leu Ile Ala Leu Gly Val Asp Gln Gln Arg Ile His Ile Val Arg Asn
            185                 190                 195

ggc gtg gat ccc gtg ccg ctg cac acg ccg aag ctg gat cgc gat ggc 739
Gly Val Asp Pro Val Pro Leu His Thr Pro Lys Leu Asp Arg Asp Gly
        200                 205                 210

cag cat gcg gtg acg ttg tcg cgc ctg gtt ccg cac aag cag att gag 787
Gln His Ala Val Thr Leu Ser Arg Leu Val Pro His Lys Gln Ile Glu
    215                 220                 225

cat gcg atg gat gtc gtc gcg gcg ctc gac ggc gtg gtg ctg gat gta 835
His Ala Met Asp Val Val Ala Ala Leu Asp Gly Val Val Leu Asp Val
230                 235                 240                 245

gtc gaa agc ggt tgg tgg cag aag gaa ctg gtc gat tat gcc cgc acg 883
Val Glu Ser Gly Trp Trp Gln Lys Glu Leu Val Asp Tyr Ala Arg Thr
                250                 255                 260

ctg ggt gtg agc gat cgc gtg gtt ttc cac ggc cag gtc gcc gag gat 931
Leu Gly Val Ser Asp Arg Val Val Phe His Gly Gln Val Ala Glu Asp
            265                 270                 275

cac aag cac gcc ctg ttg gag cgc gcc acg att cat ctc atg cct tcg 979
His Lys His Ala Leu Leu Glu Arg Ala Thr Ile His Leu Met Pro Ser
        280                 285                 290

cgc aag gaa ggc tgg ggc ctg gcg gtc acg gag gcg gcg cag cac ggc 1027
Arg Lys Glu Gly Trp Gly Leu Ala Val Thr Glu Ala Ala Gln His Gly
    295                 300                 305

gtt ccg acg atc ggt tac cga agc tca ggc ggc ctg cgc gat tcc gtc 1075
Val Pro Thr Ile Gly Tyr Arg Ser Ser Gly Gly Leu Arg Asp Ser Val
310                 315                 320                 325

gtc gac ggc gaa acc ggc ctg ctt gtc gac tcc aag gcc gag ctt att 1123
Val Asp Gly Glu Thr Gly Leu Leu Val Asp Ser Lys Ala Glu Leu Ile
                330                 335                 340

tca gcc acc aaa acc ctg ctt atc gac gcc tcc ctc cgc tcc aag ctc 1171
Ser Ala Thr Lys Thr Leu Leu Ile Asp Ala Ser Leu Arg Ser Lys Leu
            345                 350                 355

ggc gcc agc gcg aag cag cgc gcc gaa aac tac aag tgg gac acc gcg 1219
Gly Ala Ser Ala Lys Gln Arg Ala Glu Asn Tyr Lys Trp Asp Thr Ala
        360                 365                 370

gga gcg cag ttc gag gaa cta ctt ctt ggt ctt gcg tcg aaa aag 1264
Gly Ala Gln Phe Glu Glu Leu Leu Leu Gly Leu Ala Ser Lys Lys
    375                 380                 385

tagtcccagc ggcaacgcca tcc 1287

<210> 180
<211> 388
<212> PRT
<213> Corynebacterium glutamicum

<400> 180
Val Ile Val Val Ala Met Ala Ser Ile Met Ala Cys Leu Lys Ala Ala
1               5                   10                  15

Arg Leu Asn Asn Pro Met Lys Ile Leu Leu Leu Cys Trp Arg Asp Thr
            20                  25                  30

Thr His Pro Gln Gly Gly Gly Ser Glu Arg Tyr Leu Glu Arg Val Gly
        35                  40                  45

Glu Phe Leu Ala Asp Gln Gly His Glu Val Val Phe Arg Thr Ala Gly
    50                  55                  60

His Thr Asp Ala Pro Arg Arg Ser Phe Arg Asp Gly Val Arg Tyr Ser
65                  70                  75                  80

Arg Ser Gly Gly Lys Phe Ser Val Tyr Pro Lys Ala Trp Val Ala Met
                85                  90                  95

Met Leu Gly Arg Val Gly Ile Gly Thr Phe Ser Lys Val Asp Val Val
            100                 105                 110

Val Asp Thr Gln Asn Gly Ile Pro Phe Phe Gly Lys Phe Phe Ser Gly
        115                 120                 125

Lys Pro Thr Val Leu Leu Thr His His Cys His Lys Glu Gln Trp Pro
    130                 135                 140

Val Val Gly Arg Val Leu Ala Lys Val Gly Trp Leu Ile Glu Ser Gln
145                 150                 155                 160

Ile Ala Pro Arg Ala Tyr Lys Thr Ala Pro Tyr Val Thr Val Ser Glu
                165                 170                 175

Pro Ser Ala Glu Glu Leu Ile Ala Leu Gly Val Asp Gln Gln Arg Ile
            180                 185                 190

His Ile Val Arg Asn Gly Val Asp Pro Val Pro Leu His Thr Pro Lys
        195                 200                 205

Leu Asp Arg Asp Gly Gln His Ala Val Thr Leu Ser Arg Leu Val Pro
    210                 215                 220

His Lys Gln Ile Glu His Ala Met Asp Val Val Ala Ala Leu Asp Gly
225                 230                 235                 240

Val Val Leu Asp Val Val Glu Ser Gly Trp Trp Gln Lys Glu Leu Val
                245                 250                 255

Asp Tyr Ala Arg Thr Leu Gly Val Ser Asp Arg Val Val Phe His Gly
            260                 265                 270

Gln Val Ala Glu Asp His Lys His Ala Leu Leu Glu Arg Ala Thr Ile
        275                 280                 285

His Leu Met Pro Ser Arg Lys Glu Gly Trp Gly Leu Ala Val Thr Glu
    290                 295                 300

Ala Ala Gln His Gly Val Pro Thr Ile Gly Tyr Arg Ser Ser Gly Gly
305                 310                 315                 320

Leu Arg Asp Ser Val Val Asp Gly Glu Thr Gly Leu Leu Val Asp Ser
                325                 330                 335

Lys Ala Glu Leu Ile Ser Ala Thr Lys Thr Leu Leu Ile Asp Ala Ser

340                 345                 350

Leu Arg Ser Lys Leu Gly Ala Ser Ala Lys Gln Arg Ala Glu Asn Tyr
        355                 360                 365

Lys Trp Asp Thr Ala Gly Ala Gln Phe Glu Glu Leu Leu Leu Gly Leu
    370                 375                 380

Ala Ser Lys Lys
385

<210> 181
<211> 2334
<212> DNA
<213> Corynebacterium glutamicum

<220>
<221> CDS
<222> (101)..(2311)
<223> RXS00148

<400> 181
ctttgagggc agcgcgcatg cgcccgatgg ttatttgaac atgacaattg atgccgcggc 60

gacgctggct gacctgctag atgctttggg agcttaaatc atg acg tcg atc cct   115
                                            Met Thr Ser Ile Pro
                                            1               5

aat ttt tca gac atc cca ttg act gct gag aca cgt gca tcg gag tca   163
Asn Phe Ser Asp Ile Pro Leu Thr Ala Glu Thr Arg Ala Ser Glu Ser
                10                  15                  20

cac aac gtt gac gcc ggc aag gtg tgg aac act ccc gaa ggc att gat   211
His Asn Val Asp Ala Gly Lys Val Trp Asn Thr Pro Glu Gly Ile Asp
            25                  30                  35

gtc aag cgc gta ttc acg cag gct gac cgc gac gag gcg caa gcg gcg   259
Val Lys Arg Val Phe Thr Gln Ala Asp Arg Asp Glu Ala Gln Ala Ala
        40                  45                  50

gga cat ccg gtg gat tct ttg cca ggt caa aag cca ttt atg cgc ggg   307
Gly His Pro Val Asp Ser Leu Pro Gly Gln Lys Pro Phe Met Arg Gly
    55                  60                  65

ccg tac cca act atg tac acc aat cag ccg tgg acg att cgc cag tac   355
Pro Tyr Pro Thr Met Tyr Thr Asn Gln Pro Trp Thr Ile Arg Gln Tyr
70                  75                  80                  85

gca ggc ttt tca acc gcc gcg gaa tcc aat gcg ttt tat cgg agg aac   403
Ala Gly Phe Ser Thr Ala Ala Glu Ser Asn Ala Phe Tyr Arg Arg Asn
                90                  95                  100

ctt gct gcg ggt caa aaa ggt ttg tcg gtt gcg ttc gat cta gcg acc   451
Leu Ala Ala Gly Gln Lys Gly Leu Ser Val Ala Phe Asp Leu Ala Thr
            105                 110                 115

cac cgc ggt tat gac tcg gat aat gag cgc gtg gtc ggc gat gtg ggt   499
His Arg Gly Tyr Asp Ser Asp Asn Glu Arg Val Val Gly Asp Val Gly
        120                 125                 130

atg gcc ggc gtg gcg att gat tcg att ttg gat atg cgt cag ctg ttt 547
Met Ala Gly Val Ala Ile Asp Ser Ile Leu Asp Met Arg Gln Leu Phe
    135                 140                 145

gat ggc att gat ttg tcc agc gtg tcg gtg tcg atg acc atg aat ggc 595
Asp Gly Ile Asp Leu Ser Ser Val Ser Val Ser Met Thr Met Asn Gly
150                 155                 160                 165

gct gtg ctg ccg att ctt gcg ttc tat atc gtg gcg gct gag gaa caa 643
Ala Val Leu Pro Ile Leu Ala Phe Tyr Ile Val Ala Ala Glu Glu Gln
                170                 175                 180

ggt gtg ggt ccg gag cag ctt gcg ggc acg atc cag aat gac atc ttg 691
Gly Val Gly Pro Glu Gln Leu Ala Gly Thr Ile Gln Asn Asp Ile Leu
            185                 190                 195

aaa gaa ttt atg gtg cgc aac acc tat att tat ccg ccg aag ccg tcg 739
Lys Glu Phe Met Val Arg Asn Thr Tyr Ile Tyr Pro Pro Lys Pro Ser
        200                 205                 210

atg cgc atc att tcc aac atc ttt gag tac acc tcc ttg aag atg cca 787
Met Arg Ile Ile Ser Asn Ile Phe Glu Tyr Thr Ser Leu Lys Met Pro
    215                 220                 225

cgt ttt aac tcc att tcg att tct ggc tat cac atc cag gaa gcg gga 835
Arg Phe Asn Ser Ile Ser Ile Ser Gly Tyr His Ile Gln Glu Ala Gly
230                 235                 240                 245

gcg act gcc gat ttg gag ctg gcc tac act ctg gcg gat ggt att gaa 883
Ala Thr Ala Asp Leu Glu Leu Ala Tyr Thr Leu Ala Asp Gly Ile Glu
                250                 255                 260

tac atc cgt gca ggt aaa gag gta ggc ctt gac gtg gat aag ttc gcg 931
Tyr Ile Arg Ala Gly Lys Glu Val Gly Leu Asp Val Asp Lys Phe Ala
            265                 270                 275

cct cgt ctg tcc ttc ttc tgg ggt att tct atg tac acc ttc atg gag 979
Pro Arg Leu Ser Phe Phe Trp Gly Ile Ser Met Tyr Thr Phe Met Glu
        280                 285                 290

atc gca aag ctg cgt gcg gga cga ctg ctg tgg agc gag ttg gtg gca 1027
Ile Ala Lys Leu Arg Ala Gly Arg Leu Leu Trp Ser Glu Leu Val Ala
    295                 300                 305

aaa ttc gat ccg aaa aac gcc aag tcc cag tcg ctg cgc acg cac tcg 1075
Lys Phe Asp Pro Lys Asn Ala Lys Ser Gln Ser Leu Arg Thr His Ser
310                 315                 320                 325

cag acc tct ggt tgg tcg ttg acc gcg cag gat gtg tac aac aac gtc 1123
Gln Thr Ser Gly Trp Ser Leu Thr Ala Gln Asp Val Tyr Asn Asn Val
                330                 335                 340

gcc cgc acc gcg att gag gcg atg gct gca acc cag ggc cac acc cag 1171
Ala Arg Thr Ala Ile Glu Ala Met Ala Ala Thr Gln Gly His Thr Gln
            345                 350                 355

tcg ctg cac acc aat gca ctt gat gag gcg ttg gcg ctg ccc acc gat 1219
Ser Leu His Thr Asn Ala Leu Asp Glu Ala Leu Ala Leu Pro Thr Asp
        360                 365                 370

ttc tct gct cgt atc gcc cga aac acc cag ctg ttg ctg cag cag gaa 1267

Phe Ser Ala Arg Ile Ala Arg Asn Thr Gln Leu Leu Leu Gln Gln Glu
    375                 380                 385

tct ggc acg gtg cgt cca gtt gat cca tgg gcg ggc tcc tat tac gtg    1315
Ser Gly Thr Val Arg Pro Val Asp Pro Trp Ala Gly Ser Tyr Tyr Val
390                 395                 400                 405

gag tgg ttg acc aat gag ctg gct aac cgc gcg cgc aag cac atc gat    1363
Glu Trp Leu Thr Asn Glu Leu Ala Asn Arg Ala Arg Lys His Ile Asp
                410                 415                 420

gag gtg gag gaa gcc ggc gga atg gcg cag gcc acc gcg cag gga att    1411
Glu Val Glu Glu Ala Gly Gly Met Ala Gln Ala Thr Ala Gln Gly Ile
            425                 430                 435

cct aag ctg cgc att gag gaa tca gcg gca cgc acc cag gct cgc att    1459
Pro Lys Leu Arg Ile Glu Glu Ser Ala Ala Arg Thr Gln Ala Arg Ile
        440                 445                 450

gat tcc ggc cgc cag gcg ctg atc ggc gtg aat cgc tac gtg gcg gaa    1507
Asp Ser Gly Arg Gln Ala Leu Ile Gly Val Asn Arg Tyr Val Ala Glu
    455                 460                 465

gaa gat gag gaa att gaa gtc ctc aag gtt gac aac acc aag gtt cgc    1555
Glu Asp Glu Glu Ile Glu Val Leu Lys Val Asp Asn Thr Lys Val Arg
470                 475                 480                 485

gca gaa cag ttg gct aaa ctc gcg caa ctg aaa gca gag cgc aac gat    1603
Ala Glu Gln Leu Ala Lys Leu Ala Gln Leu Lys Ala Glu Arg Asn Asp
                490                 495                 500

gcg gaa gtc aag gct gcg ctg gat gcg ttg aca gct gct gcc cgc aac    1651
Ala Glu Val Lys Ala Ala Leu Asp Ala Leu Thr Ala Ala Ala Arg Asn
            505                 510                 515

gag cat aaa gag cca ggg gat ttg gat cag aac ctg ctc aaa ctt gcc    1699
Glu His Lys Glu Pro Gly Asp Leu Asp Gln Asn Leu Leu Lys Leu Ala
        520                 525                 530

gtc gat gct gcg cgc gca aaa gct acc att gga gag atc tcc gat gct    1747
Val Asp Ala Ala Arg Ala Lys Ala Thr Ile Gly Glu Ile Ser Asp Ala
    535                 540                 545

ttg gaa gtt gtc ttt ggc cgc cac gaa gca gaa atc agg acg ctg tct    1795
Leu Glu Val Val Phe Gly Arg His Glu Ala Glu Ile Arg Thr Leu Ser
550                 555                 560                 565

ggc gtg tac aag gat gag gtt gga aag gaa ggc aca gtg agc aac gtc    1843
Gly Val Tyr Lys Asp Glu Val Gly Lys Glu Gly Thr Val Ser Asn Val
                570                 575                 580

gaa cgc gcg atc gcc ctg gct gac gcc ttt gag gct gag gaa ggc cgc    1891
Glu Arg Ala Ile Ala Leu Ala Asp Ala Phe Glu Ala Glu Glu Gly Arg
            585                 590                 595

cgc cca cgt atc ttt att gcc aag atg ggc cag gat gga cat gac cgt    1939
Arg Pro Arg Ile Phe Ile Ala Lys Met Gly Gln Asp Gly His Asp Arg
        600                 605                 610

gga cag aag gtt gtc gcg tct gcc tat gct gac ctg ggc atg gac gtg    1987
Gly Gln Lys Val Val Ala Ser Ala Tyr Ala Asp Leu Gly Met Asp Val

615                 620                 625

gat gtt gga ccg ctg ttt caa act cca gcc gaa gct gcc cgc gcc gcc     2035
Asp Val Gly Pro Leu Phe Gln Thr Pro Ala Glu Ala Ala Arg Ala Ala
630                 635                 640                 645

gtg gac gcc gat gtt cac gtg gtg ggt atg tct tcg ctg gca gca ggc     2083
Val Asp Ala Asp Val His Val Val Gly Met Ser Ser Leu Ala Ala Gly
                650                 655                 660

cac ctc acc ttg ctg ccc gag ctg aag aaa gaa ctt gca gct ctt ggc     2131
His Leu Thr Leu Leu Pro Glu Leu Lys Lys Glu Leu Ala Ala Leu Gly
            665                 670                 675

cgc gat gac att ctg gtc acc gtg ggc ggc gtc att ccg ccg ggc gat     2179
Arg Asp Asp Ile Leu Val Thr Val Gly Gly Val Ile Pro Pro Gly Asp
        680                 685                 690

ttc cag gat ctc tac gat atg ggt gcc gcc gcg att tac cct tca gga     2227
Phe Gln Asp Leu Tyr Asp Met Gly Ala Ala Ala Ile Tyr Pro Ser Gly
    695                 700                 705

acc gtc atc gcg gag tcg gcg atc gat ctg atc acc cga ctc gcc gca     2275
Thr Val Ile Ala Glu Ser Ala Ile Asp Leu Ile Thr Arg Leu Ala Ala
710                 715                 720                 725

cac ctg ggc ttt gac ctg gat gtg gat gtg aat gaa tgatcacggt          2321
His Leu Gly Phe Asp Leu Asp Val Asp Val Asn Glu
                730                 735

ttcctagaag aca                                                      2334


<210> 182
<211> 737
<212> PRT
<213> Corynebacterium glutamicum

<400> 182
Met Thr Ser Ile Pro Asn Phe Ser Asp Ile Pro Leu Thr Ala Glu Thr
  1               5                  10                  15

Arg Ala Ser Glu Ser His Asn Val Asp Ala Gly Lys Val Trp Asn Thr
             20                  25                  30

Pro Glu Gly Ile Asp Val Lys Arg Val Phe Thr Gln Ala Asp Arg Asp
         35                  40                  45

Glu Ala Gln Ala Ala Gly His Pro Val Asp Ser Leu Pro Gly Gln Lys
     50                  55                  60

Pro Phe Met Arg Gly Pro Tyr Pro Thr Met Tyr Thr Asn Gln Pro Trp
 65                  70                  75                  80

Thr Ile Arg Gln Tyr Ala Gly Phe Ser Thr Ala Ala Glu Ser Asn Ala
                 85                  90                  95

Phe Tyr Arg Arg Asn Leu Ala Ala Gly Gln Lys Gly Leu Ser Val Ala
            100                 105                 110

Phe Asp Leu Ala Thr His Arg Gly Tyr Asp Ser Asp Asn Glu Arg Val

115                 120                 125

Val Gly Asp Val Gly Met Ala Gly Val Ala Ile Asp Ser Ile Leu Asp
    130                 135                 140

Met Arg Gln Leu Phe Asp Gly Ile Asp Leu Ser Ser Val Ser Val Ser
145                 150                 155                 160

Met Thr Met Asn Gly Ala Val Leu Pro Ile Leu Ala Phe Tyr Ile Val
                165                 170                 175

Ala Ala Glu Glu Gln Gly Val Gly Pro Glu Gln Leu Ala Gly Thr Ile
            180                 185                 190

Gln Asn Asp Ile Leu Lys Glu Phe Met Val Arg Asn Thr Tyr Ile Tyr
        195                 200                 205

Pro Pro Lys Pro Ser Met Arg Ile Ile Ser Asn Ile Phe Glu Tyr Thr
    210                 215                 220

Ser Leu Lys Met Pro Arg Phe Asn Ser Ile Ser Ile Ser Gly Tyr His
225                 230                 235                 240

Ile Gln Glu Ala Gly Ala Thr Ala Asp Leu Glu Leu Ala Tyr Thr Leu
                245                 250                 255

Ala Asp Gly Ile Glu Tyr Ile Arg Ala Gly Lys Glu Val Gly Leu Asp
            260                 265                 270

Val Asp Lys Phe Ala Pro Arg Leu Ser Phe Phe Trp Gly Ile Ser Met
        275                 280                 285

Tyr Thr Phe Met Glu Ile Ala Lys Leu Arg Ala Gly Arg Leu Leu Trp
    290                 295                 300

Ser Glu Leu Val Ala Lys Phe Asp Pro Lys Asn Ala Lys Ser Gln Ser
305                 310                 315                 320

Leu Arg Thr His Ser Gln Thr Ser Gly Trp Ser Leu Thr Ala Gln Asp
                325                 330                 335

Val Tyr Asn Asn Val Ala Arg Thr Ala Ile Glu Ala Met Ala Ala Thr
            340                 345                 350

Gln Gly His Thr Gln Ser Leu His Thr Asn Ala Leu Asp Glu Ala Leu
        355                 360                 365

Ala Leu Pro Thr Asp Phe Ser Ala Arg Ile Ala Arg Asn Thr Gln Leu
    370                 375                 380

Leu Leu Gln Gln Glu Ser Gly Thr Val Arg Pro Val Asp Pro Trp Ala
385                 390                 395                 400

Gly Ser Tyr Tyr Val Glu Trp Leu Thr Asn Glu Leu Ala Asn Arg Ala
                405                 410                 415

Arg Lys His Ile Asp Glu Val Glu Glu Ala Gly Gly Met Ala Gln Ala
            420                 425                 430

Thr Ala Gln Gly Ile Pro Lys Leu Arg Ile Glu Glu Ser Ala Ala Arg
        435                 440                 445

Thr Gln Ala Arg Ile Asp Ser Gly Arg Gln Ala Leu Ile Gly Val Asn
    450                 455                 460

Arg Tyr Val Ala Glu Glu Asp Glu Glu Ile Glu Val Leu Lys Val Asp
465                 470                 475                 480

Asn Thr Lys Val Arg Ala Glu Gln Leu Ala Lys Leu Ala Gln Leu Lys
                485                 490                 495

Ala Glu Arg Asn Asp Ala Glu Val Lys Ala Ala Leu Asp Ala Leu Thr
            500                 505                 510

Ala Ala Ala Arg Asn Glu His Lys Glu Pro Gly Asp Leu Asp Gln Asn
        515                 520                 525

Leu Leu Lys Leu Ala Val Asp Ala Ala Arg Ala Lys Ala Thr Ile Gly
    530                 535                 540

Glu Ile Ser Asp Ala Leu Glu Val Val Phe Gly Arg His Glu Ala Glu
545                 550                 555                 560

Ile Arg Thr Leu Ser Gly Val Tyr Lys Asp Glu Val Gly Lys Glu Gly
                565                 570                 575

Thr Val Ser Asn Val Glu Arg Ala Ile Ala Leu Ala Asp Ala Phe Glu
            580                 585                 590

Ala Glu Glu Gly Arg Arg Pro Arg Ile Phe Ile Ala Lys Met Gly Gln
        595                 600                 605

Asp Gly His Asp Arg Gly Gln Lys Val Val Ala Ser Ala Tyr Ala Asp
    610                 615                 620

Leu Gly Met Asp Val Asp Val Gly Pro Leu Phe Gln Thr Pro Ala Glu
625                 630                 635                 640

Ala Ala Arg Ala Ala Val Asp Ala Asp Val His Val Val Gly Met Ser
                645                 650                 655

Ser Leu Ala Ala Gly His Leu Thr Leu Leu Pro Glu Leu Lys Lys Glu
            660                 665                 670

Leu Ala Ala Leu Gly Arg Asp Asp Ile Leu Val Thr Val Gly Gly Val
        675                 680                 685

Ile Pro Pro Gly Asp Phe Gln Asp Leu Tyr Asp Met Gly Ala Ala Ala
    690                 695                 700

Ile Tyr Pro Ser Gly Thr Val Ile Ala Glu Ser Ala Ile Asp Leu Ile
705                 710                 715                 720

Thr Arg Leu Ala Ala His Leu Gly Phe Asp Leu Asp Val Asp Val Asn
                725                 730                 735

Glu

<210> 183
<211> 1971

<212> DNA
<213> Corynebacterium glutamicum

<220>
<221> CDS
<222> (101)..(1948)
<223> RXS00149

<400> 183
ttttcgtagg taaacacagg tgaaggcttt acaagcttgt gaactcccta cacaaaagca 60

atccaatagc tatccataag caagagaaag taagtctacg ttg act gat ctc aca 115
Leu Thr Asp Leu Thr
1               5

aag act gcg gtg ccc gag gaa ctt tca gag aac ctc gaa act tgg tac 163
Lys Thr Ala Val Pro Glu Glu Leu Ser Glu Asn Leu Glu Thr Trp Tyr
                10                  15                  20

aag gct gtg gcc ggt gtt ttc gcg cgc aca cag aaa aaa gac atc ggc 211
Lys Ala Val Ala Gly Val Phe Ala Arg Thr Gln Lys Lys Asp Ile Gly
            25                  30                  35

gac att gcc gta gat gtg tgg aag aaa ctc atc gtc act aca ccg gat 259
Asp Ile Ala Val Asp Val Trp Lys Lys Leu Ile Val Thr Thr Pro Asp
        40                  45                  50

ggt gtt gat atc aat ccg ctg tac acc aga gca gat gag tcc cag agg 307
Gly Val Asp Ile Asn Pro Leu Tyr Thr Arg Ala Asp Glu Ser Gln Arg
    55                  60                  65

aaa ttc act gag gtt cct ggt gag ttt ccc ttc act agg gga acc act 355
Lys Phe Thr Glu Val Pro Gly Glu Phe Pro Phe Thr Arg Gly Thr Thr
70                  75                  80                  85

gtt gat ggt gaa cgc gtt ggt tgg ggt gtt act gag act ttc gga cat 403
Val Asp Gly Glu Arg Val Gly Trp Gly Val Thr Glu Thr Phe Gly His
                90                  95                  100

gac agc ccg aag aat atc aac gct gcg gtg ctg aat gct ctg aat tct 451
Asp Ser Pro Lys Asn Ile Asn Ala Ala Val Leu Asn Ala Leu Asn Ser
            105                 110                 115

ggc acc acc aca ttg ggt ttt gag ttc tct gag gaa ttc acg gca gct 499
Gly Thr Thr Thr Leu Gly Phe Glu Phe Ser Glu Glu Phe Thr Ala Ala
        120                 125                 130

gat ctt aaa gtt gct ctc gaa ggc gtg tat ctc aac atg gct ccg ttg 547
Asp Leu Lys Val Ala Leu Glu Gly Val Tyr Leu Asn Met Ala Pro Leu
    135                 140                 145

ctg att cat gcg ggt gga tcc acg tca gag gtt gca gcg gcg ttg tat 595
Leu Ile His Ala Gly Gly Ser Thr Ser Glu Val Ala Ala Ala Leu Tyr
150                 155                 160                 165

acg ttg gcg gag gaa gcc gga acg ttt ttt gct gcg ttg acc ttg ggt 643
Thr Leu Ala Glu Glu Ala Gly Thr Phe Phe Ala Ala Leu Thr Leu Gly
                170                 175                 180

tct cgt cct ttg acg gcg cag gtt gat ggt tcg cac agt gac acc att 691
Ser Arg Pro Leu Thr Ala Gln Val Asp Gly Ser His Ser Asp Thr Ile

            185                 190                 195

gaa gaa gca gtt cag ttg gca gtg aat gct tcc aag cgt gcg aat gtg 739
Glu Glu Ala Val Gln Leu Ala Val Asn Ala Ser Lys Arg Ala Asn Val
        200                 205                 210

cgc gct atc ttg gtg gat ggt tcc agt ttt tcc aac cag ggc gcg tcg 787
Arg Ala Ile Leu Val Asp Gly Ser Ser Phe Ser Asn Gln Gly Ala Ser
    215                 220                 225

gat gct caa gaa att ggt cta agt atc gcc gcc ggt gtg gat tat gtc 835
Asp Ala Gln Glu Ile Gly Leu Ser Ile Ala Ala Gly Val Asp Tyr Val
230                 235                 240                 245

cgt cgc ttg gtc gat gca ggc ctt tcc acg gaa gct gca ctt aag cag 883
Arg Arg Leu Val Asp Ala Gly Leu Ser Thr Glu Ala Ala Leu Lys Gln
                250                 255                 260

gtg gcg ttc cgt ttt gcg gtc acc gat gag cag ttc gcg cag att tct 931
Val Ala Phe Arg Phe Ala Val Thr Asp Glu Gln Phe Ala Gln Ile Ser
            265                 270                 275

aag ctg cgt gtg gct cga cgt ctg tgg gcc agg gtg tgt gag gtg ctt 979
Lys Leu Arg Val Ala Arg Arg Leu Trp Ala Arg Val Cys Glu Val Leu
        280                 285                 290

ggt ttt cca gag ctg gcc gta gca cca cag cat gcg gtg act gca cga 1027
Gly Phe Pro Glu Leu Ala Val Ala Pro Gln His Ala Val Thr Ala Arg
    295                 300                 305

gcg atg ttt agc cag cgt gat ccg tgg gtg aat atg ctg cgc agt act 1075
Ala Met Phe Ser Gln Arg Asp Pro Trp Val Asn Met Leu Arg Ser Thr
310                 315                 320                 325

gtt gca gct ttc gct gca ggc gtc ggt gga gca acc gat gtg gag gtt 1123
Val Ala Ala Phe Ala Ala Gly Val Gly Gly Ala Thr Asp Val Glu Val
                330                 335                 340

cgt act ttt gat gat gcg atc cca gat gga gtt cct gga gtg tcg agg 1171
Arg Thr Phe Asp Asp Ala Ile Pro Asp Gly Val Pro Gly Val Ser Arg
            345                 350                 355

aat ttc gct cac cgc atc gcg cgc aat act aat ttg ttg ttg cta gaa 1219
Asn Phe Ala His Arg Ile Ala Arg Asn Thr Asn Leu Leu Leu Leu Glu
        360                 365                 370

gag tca cat ctt ggt cac gtg gtt gat cct gct ggt gga tca tat ttc 1267
Glu Ser His Leu Gly His Val Val Asp Pro Ala Gly Gly Ser Tyr Phe
    375                 380                 385

gtg gag agc ttc acc gat gat cta gcg gag aag gcg tgg gct gtg ttc 1315
Val Glu Ser Phe Thr Asp Asp Leu Ala Glu Lys Ala Trp Ala Val Phe
390                 395                 400                 405

agt ggc atc gaa gct gag ggc gga tac agt gca gct tgt gca tcc ggc 1363
Ser Gly Ile Glu Ala Glu Gly Gly Tyr Ser Ala Ala Cys Ala Ser Gly
                410                 415                 420

acg gtg act gcc atg ctt gat cag acg tgg gag cag act cgc gct gat 1411
Thr Val Thr Ala Met Leu Asp Gln Thr Trp Glu Gln Thr Arg Ala Asp
            425                 430                 435

gtg gcg tcg aga aag aag aag ctc act gga att aat gag ttc ccg aac 1459
Val Ala Ser Arg Lys Lys Lys Leu Thr Gly Ile Asn Glu Phe Pro Asn
        440                 445                 450

ttg gcg gag tct ccg ctg cca gct gat cgt cgg gta gaa cct gca ggt 1507
Leu Ala Glu Ser Pro Leu Pro Ala Asp Arg Arg Val Glu Pro Ala Gly
    455                 460                 465

gtg cgt cga tgg gca gcg gat ttt gaa gcg ctg cgc aat cgt tcg gat 1555
Val Arg Arg Trp Ala Ala Asp Phe Glu Ala Leu Arg Asn Arg Ser Asp
470                 475                 480                 485

gct ttc ttg gaa aag aac ggc gcg agg cca cag atc acg atg att cct 1603
Ala Phe Leu Glu Lys Asn Gly Ala Arg Pro Gln Ile Thr Met Ile Pro
                490                 495                 500

ctg gga ccg ttg tcc aag cac aat att cgc act ggt ttt act tcc aac 1651
Leu Gly Pro Leu Ser Lys His Asn Ile Arg Thr Gly Phe Thr Ser Asn
            505                 510                 515

ctg ttg gct tcc ggt ggc att gaa gca atc aac ccg ggt caa ctt gtt 1699
Leu Leu Ala Ser Gly Gly Ile Glu Ala Ile Asn Pro Gly Gln Leu Val
        520                 525                 530

ccc ggc act gac gct ttt gca gaa gct gca cag gcc gca ggc att gta 1747
Pro Gly Thr Asp Ala Phe Ala Glu Ala Ala Gln Ala Ala Gly Ile Val
    535                 540                 545

gtg gtg tgt gga acg gac caa gag tat gcc gaa acg ggg gag gga gcc 1795
Val Val Cys Gly Thr Asp Gln Glu Tyr Ala Glu Thr Gly Glu Gly Ala
550                 555                 560                 565

gtc gaa aag ctc cgc gaa gcg ggc gtt gag cgc atc ctg ctt gct ggc 1843
Val Glu Lys Leu Arg Glu Ala Gly Val Glu Arg Ile Leu Leu Ala Gly
                570                 575                 580

gcg ccg aag agc ttt gag ggc agc gcg cat gcg ccc gat ggt tat ttg 1891
Ala Pro Lys Ser Phe Glu Gly Ser Ala His Ala Pro Asp Gly Tyr Leu
            585                 590                 595

aac atg aca att gat gcc gcg gcg acg ctg gct gac ctg cta gat gct 1939
Asn Met Thr Ile Asp Ala Ala Ala Thr Leu Ala Asp Leu Leu Asp Ala
        600                 605                 610

ttg gga gct taaatcatga cgtcgatccc taa 1971
Leu Gly Ala
    615

<210> 184
<211> 616
<212> PRT
<213> Corynebacterium glutamicum

<400> 184
Leu Thr Asp Leu Thr Lys Thr Ala Val Pro Glu Glu Leu Ser Glu Asn
1               5                   10                  15

Leu Glu Thr Trp Tyr Lys Ala Val Ala Gly Val Phe Ala Arg Thr Gln
            20                  25                  30

Lys Lys Asp Ile Gly Asp Ile Ala Val Asp Val Trp Lys Lys Leu Ile
        35                  40                  45

Val Thr Thr Pro Asp Gly Val Asp Ile Asn Pro Leu Tyr Thr Arg Ala
    50                  55                  60

Asp Glu Ser Gln Arg Lys Phe Thr Glu Val Pro Gly Glu Phe Pro Phe
65                  70                  75                  80

Thr Arg Gly Thr Thr Val Asp Gly Glu Arg Val Gly Trp Gly Val Thr
                85                  90                  95

Glu Thr Phe Gly His Asp Ser Pro Lys Asn Ile Asn Ala Ala Val Leu
            100                 105                 110

Asn Ala Leu Asn Ser Gly Thr Thr Thr Leu Gly Phe Glu Phe Ser Glu
        115                 120                 125

Glu Phe Thr Ala Ala Asp Leu Lys Val Ala Leu Glu Gly Val Tyr Leu
    130                 135                 140

Asn Met Ala Pro Leu Leu Ile His Ala Gly Gly Ser Thr Ser Glu Val
145                 150                 155                 160

Ala Ala Ala Leu Tyr Thr Leu Ala Glu Glu Ala Gly Thr Phe Phe Ala
                165                 170                 175

Ala Leu Thr Leu Gly Ser Arg Pro Leu Thr Ala Gln Val Asp Gly Ser
            180                 185                 190

His Ser Asp Thr Ile Glu Glu Ala Val Gln Leu Ala Val Asn Ala Ser
        195                 200                 205

Lys Arg Ala Asn Val Arg Ala Ile Leu Val Asp Gly Ser Ser Phe Ser
    210                 215                 220

Asn Gln Gly Ala Ser Asp Ala Gln Glu Ile Gly Leu Ser Ile Ala Ala
225                 230                 235                 240

Gly Val Asp Tyr Val Arg Arg Leu Val Asp Ala Gly Leu Ser Thr Glu
                245                 250                 255

Ala Ala Leu Lys Gln Val Ala Phe Arg Phe Ala Val Thr Asp Glu Gln
            260                 265                 270

Phe Ala Gln Ile Ser Lys Leu Arg Val Ala Arg Arg Leu Trp Ala Arg
        275                 280                 285

Val Cys Glu Val Leu Gly Phe Pro Glu Leu Ala Val Ala Pro Gln His
    290                 295                 300

Ala Val Thr Ala Arg Ala Met Phe Ser Gln Arg Asp Pro Trp Val Asn
305                 310                 315                 320

Met Leu Arg Ser Thr Val Ala Ala Phe Ala Ala Gly Val Gly Gly Ala
                325                 330                 335

Thr Asp Val Glu Val Arg Thr Phe Asp Asp Ala Ile Pro Asp Gly Val
            340                 345                 350

Pro Gly Val Ser Arg Asn Phe Ala His Arg Ile Ala Arg Asn Thr Asn
        355                 360                 365

Leu Leu Leu Leu Glu Glu Ser His Leu Gly His Val Val Asp Pro Ala
    370                 375                 380

Gly Gly Ser Tyr Phe Val Glu Ser Phe Thr Asp Asp Leu Ala Glu Lys
385                 390                 395                 400

Ala Trp Ala Val Phe Ser Gly Ile Glu Ala Glu Gly Gly Tyr Ser Ala
                405                 410                 415

Ala Cys Ala Ser Gly Thr Val Thr Ala Met Leu Asp Gln Thr Trp Glu
            420                 425                 430

Gln Thr Arg Ala Asp Val Ala Ser Arg Lys Lys Lys Leu Thr Gly Ile
        435                 440                 445

Asn Glu Phe Pro Asn Leu Ala Glu Ser Pro Leu Pro Ala Asp Arg Arg
    450                 455                 460

Val Glu Pro Ala Gly Val Arg Arg Trp Ala Ala Asp Phe Glu Ala Leu
465                 470                 475                 480

Arg Asn Arg Ser Asp Ala Phe Leu Glu Lys Asn Gly Ala Arg Pro Gln
                485                 490                 495

Ile Thr Met Ile Pro Leu Gly Pro Leu Ser Lys His Asn Ile Arg Thr
            500                 505                 510

Gly Phe Thr Ser Asn Leu Leu Ala Ser Gly Gly Ile Glu Ala Ile Asn
        515                 520                 525

Pro Gly Gln Leu Val Pro Gly Thr Asp Ala Phe Ala Glu Ala Ala Gln
    530                 535                 540

Ala Ala Gly Ile Val Val Val Cys Gly Thr Asp Gln Glu Tyr Ala Glu
545                 550                 555                 560

Thr Gly Glu Gly Ala Val Glu Lys Leu Arg Glu Ala Gly Val Glu Arg
                565                 570                 575

Ile Leu Leu Ala Gly Ala Pro Lys Ser Phe Glu Gly Ser Ala His Ala
            580                 585                 590

Pro Asp Gly Tyr Leu Asn Met Thr Ile Asp Ala Ala Ala Thr Leu Ala
        595                 600                 605

Asp Leu Leu Asp Ala Leu Gly Ala
    610                 615


<210> 185
<211> 1179
<212> DNA
<213> Corynebacterium glutamicum

<220>
<221> CDS
<222> (101)..(1156)
<223> RXS02106

<400> 185
   ggtcgccgct tttcgacgcc cgcctgcggc gggatgactg tgatggaggg gcgcgtcgac 60
   aagcaaaatc tctttagcaa attcggttac tgtggggcgc atg aat aac cat ttt 115
   Met Asn Asn His Phe
   1 5
   gag ctc aaa gta cct ggt gga aag ctt gtc gtc gtt gat gtg acc acc 163
   Glu Leu Lys Val Pro Gly Gly Lys Leu Val Val Val Asp Val Thr Thr
   10 15 20
   gat ctg gat tcc att gct gac gtg aag att tcc ggc gat ttc ttc ctc 211
   Asp Leu Asp Ser Ile Ala Asp Val Lys Ile Ser Gly Asp Phe Phe Leu
   25 30 35
   gaa ccc gat gag gca ttc ttc gcc ctt ggc cgg gcg ctg cag ggg gcg 259
   Glu Pro Asp Glu Ala Phe Phe Ala Leu Gly Arg Ala Leu Gln Gly Ala
   40 45 50
   tcg gtg ggt gat aac act gat cgt ttg cag gca aag ttg gat gca gcg 307
   Ser Val Gly Asp Asn Thr Asp Arg Leu Gln Ala Lys Leu Asp Ala Ala
   55 60 65
   ttg gcg gaa tat gat gac gtt gag cta cac ggc ttt agc act gcg gat 355
   Leu Ala Glu Tyr Asp Asp Val Glu Leu His Gly Phe Ser Thr Ala Asp
   70 75 80 85
   att gct tta gct gtg cgt cgg gca gtc acc ggc gcg caa gat ttc acc 403
   Ile Ala Leu Ala Val Arg Arg Ala Val Thr Gly Ala Gln Asp Phe Thr
   90 95 100
   gat tat gaa tgg gaa atc ctg cac cca ggg gtg ctt cct acc cca ctt 451
   Asp Tyr Glu Trp Glu Ile Leu His Pro Gly Val Leu Pro Thr Pro Leu
   105 110 115
   aac gtt gcg ttg gat gag ctc ctt ttg gac caa gtt gcc agt ggt cag 499
   Asn Val Ala Leu Asp Glu Leu Leu Leu Asp Gln Val Ala Ser Gly Gln
   120 125 130
   cgt ggc ccg acg atg cgc att tgg gat tgg gat gat cgc gcc aca gtg 547
   Arg Gly Pro Thr Met Arg Ile Trp Asp Trp Asp Asp Arg Ala Thr Val
   135 140 145
   atc ggt agt ttc cag tca tat gtc aat gaa atc aac caa gaa ggc gtt 595
   Ile Gly Ser Phe Gln Ser Tyr Val Asn Glu Ile Asn Gln Glu Gly Val
   150 155 160 165
   aat gaa cat ggt gtg acc gtg gta cga cgc atg tct ggt ggc ggt gca 643
   Asn Glu His Gly Val Thr Val Val Arg Arg Met Ser Gly Gly Gly Ala
   170 175 180
   atg ttt atg gag ggc ggc aac tgc atc acc tat tcc ctg tat gca ccg 691
   Met Phe Met Glu Gly Gly Asn Cys Ile Thr Tyr Ser Leu Tyr Ala Pro
   185 190 195
   gaa tct ctc gtt gct ggt ttg agc tat gag cag tcc tat gaa tat ttg 739
   Glu Ser Leu Val Ala Gly Leu Ser Tyr Glu Gln Ser Tyr Glu Tyr Leu
   200 205 210
   gat cgt tgg gtg att gct gcg ctg aag aca cac gat gtt gac gct tgg 787
   Asp Arg Trp Val Ile Ala Ala Leu Lys Thr His Asp Val Asp Ala Trp
       215                 220                 225
   tac gtg cct atc aat gac atc acc tcc acc ggc gga aaa atc ggt ggc     835
   Tyr Val Pro Ile Asn Asp Ile Thr Ser Thr Gly Gly Lys Ile Gly Gly
   230                 235                 240                 245
   gct gca cag aaa cgt cgc agt ggc gca gtc ctc cac cac gtg acc atg     883
   Ala Ala Gln Lys Arg Arg Ser Gly Ala Val Leu His His Val Thr Met
                   250                 255                 260
   tcc tat gac atc gat gcg gac atg atg acc cag gtg ttg cgc att gga     931
   Ser Tyr Asp Ile Asp Ala Asp Met Met Thr Gln Val Leu Arg Ile Gly
               265                 270                 275
   aag gtg aag att tcc gac aag ggt ctt cgc agc gca aag aag cgc gtt     979
   Lys Val Lys Ile Ser Asp Lys Gly Leu Arg Ser Ala Lys Lys Arg Val
           280                 285                 290
   gat cct ctg cgc cgc caa aca ggt gca tca cgt gag caa atc atc gac     1027
   Asp Pro Leu Arg Arg Gln Thr Gly Ala Ser Arg Glu Gln Ile Ile Asp
       295                 300                 305
   acc cta aag tcc aca ttc agt gct agg tac ggc gcg caa gaa gta gag     1075
   Thr Leu Lys Ser Thr Phe Ser Ala Arg Tyr Gly Ala Gln Glu Val Glu
   310                 315                 320                 325
   ctc agc gat gaa gat ttc gcg gca ggc cac gac cta gta aaa acc aaa     1123
   Leu Ser Asp Glu Asp Phe Ala Ala Gly His Asp Leu Val Lys Thr Lys
                   330                 335                 340
   tac gcc acc gag gag tgg act aag cga gtt caa tagtttctat ggatctgcac 1176
   Tyr Ala Thr Glu Glu Trp Thr Lys Arg Val Gln
               345                 350
   aag                                                                 1179


<210> 186
<211> 352
<212> PRT
<213> Corynebacterium glutamicum

<400> 186
Met Asn Asn His Phe Glu Leu Lys Val Pro Gly Gly Lys Leu Val Val
  1               5                  10                  15

Val Asp Val Thr Thr Asp Leu Asp Ser Ile Ala Asp Val Lys Ile Ser
             20                  25                  30

Gly Asp Phe Phe Leu Glu Pro Asp Glu Ala Phe Phe Ala Leu Gly Arg
         35                  40                  45

Ala Leu Gln Gly Ala Ser Val Gly Asp Asn Thr Asp Arg Leu Gln Ala
     50                  55                  60

Lys Leu Asp Ala Ala Leu Ala Glu Tyr Asp Asp Val Glu Leu His Gly
 65                  70                  75                  80

Phe Ser Thr Ala Asp Ile Ala Leu Ala Val Arg Arg Ala Val Thr Gly
                 85                  90                  95

Ala Gln Asp Phe Thr Asp Tyr Glu Trp Glu Ile Leu His Pro Gly Val
            100                 105                 110

Leu Pro Thr Pro Leu Asn Val Ala Leu Asp Glu Leu Leu Leu Asp Gln
        115                 120                 125

Val Ala Ser Gly Gln Arg Gly Pro Thr Met Arg Ile Trp Asp Trp Asp
    130                 135                 140

Asp Arg Ala Thr Val Ile Gly Ser Phe Gln Ser Tyr Val Asn Glu Ile
145                 150                 155                 160

Asn Gln Glu Gly Val Asn Glu His Gly Val Thr Val Val Arg Arg Met
                165                 170                 175

Ser Gly Gly Gly Ala Met Phe Met Glu Gly Gly Asn Cys Ile Thr Tyr
            180                 185                 190

Ser Leu Tyr Ala Pro Glu Ser Leu Val Ala Gly Leu Ser Tyr Glu Gln
        195                 200                 205

Ser Tyr Glu Tyr Leu Asp Arg Trp Val Ile Ala Ala Leu Lys Thr His
    210                 215                 220

Asp Val Asp Ala Trp Tyr Val Pro Ile Asn Asp Ile Thr Ser Thr Gly
225                 230                 235                 240

Gly Lys Ile Gly Gly Ala Ala Gln Lys Arg Arg Ser Gly Ala Val Leu
                245                 250                 255

His His Val Thr Met Ser Tyr Asp Ile Asp Ala Asp Met Met Thr Gln
            260                 265                 270

Val Leu Arg Ile Gly Lys Val Lys Ile Ser Asp Lys Gly Leu Arg Ser
        275                 280                 285

Ala Lys Lys Arg Val Asp Pro Leu Arg Arg Gln Thr Gly Ala Ser Arg
    290                 295                 300

Glu Gln Ile Ile Asp Thr Leu Lys Ser Thr Phe Ser Ala Arg Tyr Gly
305                 310                 315                 320

Ala Gln Glu Val Glu Leu Ser Asp Glu Asp Phe Ala Ala Gly His Asp
                325                 330                 335

Leu Val Lys Thr Lys Tyr Ala Thr Glu Glu Trp Thr Lys Arg Val Gln
            340                 345                 350

<210> 187
<211> 876
<212> DNA
<213> Corynebacterium glutamicum

<220>
<221> CDS
<222> (101)..(853)

<223> RXS01746

<400> 187
aaaccgccac tcccctttca ctggggagtg gcggttttgt cgtttcatgc atgcagtgtg 60

tgacttatca accttgttag ggctagggtg gatatctatc atg act gca cca aga 115
Met Thr Ala Pro Arg
1               5

gat cct ttt ttc ccc gca gat ctt tct atc cgc gcg tct gca gag ccc 163
Asp Pro Phe Phe Pro Ala Asp Leu Ser Ile Arg Ala Ser Ala Glu Pro
                10                  15                  20

att gaa att cag cgg ttg ggt ttg atc gat tat caa gag gcc tgg gat 211
Ile Glu Ile Gln Arg Leu Gly Leu Ile Asp Tyr Gln Glu Ala Trp Asp
            25                  30                  35

tat caa gca gag ctt gct acc cgt agg gct aat gat gaa atc cct gat 259
Tyr Gln Ala Glu Leu Ala Thr Arg Arg Ala Asn Asp Glu Ile Pro Asp
        40                  45                  50

cag ctg ctt att ttg gag cac ccg tcg gtg tat acc gca ggt aag cgc 307
Gln Leu Leu Ile Leu Glu His Pro Ser Val Tyr Thr Ala Gly Lys Arg
    55                  60                  65

acc cag ccg gaa gat ctt ccc acc aac gga ctg ccg gtg atc aat gct 355
Thr Gln Pro Glu Asp Leu Pro Thr Asn Gly Leu Pro Val Ile Asn Ala
70                  75                  80                  85

gat cgt ggt ggt cgc atc acg tgg cat ggt cct ggc caa ttg gtg atc 403
Asp Arg Gly Gly Arg Ile Thr Trp His Gly Pro Gly Gln Leu Val Ile
                90                  95                  100

tat ccg atc atc aaa tta gcc gat ccg atc gat gtg gtt gat tac gta 451
Tyr Pro Ile Ile Lys Leu Ala Asp Pro Ile Asp Val Val Asp Tyr Val
            105                 110                 115

aga cgc ctc gag gaa gcg ctc atc caa gtt gtc ggc gat atg ggt gtt 499
Arg Arg Leu Glu Glu Ala Leu Ile Gln Val Val Gly Asp Met Gly Val
        120                 125                 130

gcc ggc gct ggg cgc att gat ggg cgt tcg ggt gtg tgg gtg cca gct 547
Ala Gly Ala Gly Arg Ile Asp Gly Arg Ser Gly Val Trp Val Pro Ala
    135                 140                 145

cat gat ggt tgg gtg gac agc aag gtt gcg gcc atc ggc att cga ata 595
His Asp Gly Trp Val Asp Ser Lys Val Ala Ala Ile Gly Ile Arg Ile
150                 155                 160                 165

act cgt ggt gtt gca atg cac ggt gtg gcc atc aac tgc aac aac acg 643
Thr Arg Gly Val Ala Met His Gly Val Ala Ile Asn Cys Asn Asn Thr
                170                 175                 180

ttg gat ttc tat gag cac atc att ccg tgt ggc att gct gat gca ggc 691
Leu Asp Phe Tyr Glu His Ile Ile Pro Cys Gly Ile Ala Asp Ala Gly
            185                 190                 195

ttg agc aca ctc tcg agg gaa ctg aaa agg gac gtt tca gtt gag gaa 739
Leu Ser Thr Leu Ser Arg Glu Leu Lys Arg Asp Val Ser Val Glu Glu
        200                 205                 210

tta gtc gag cca tcg atc cgc gca ttg gat gat gct ttg gct ggt cgg     787
Leu Val Glu Pro Ser Ile Arg Ala Leu Asp Asp Ala Leu Ala Gly Arg
    215                 220                 225

ctg gtt gtt tct gat cat tct ttc ggc agc gcg ccc gac cca act aag     835
Leu Val Val Ser Asp His Ser Phe Gly Ser Ala Pro Asp Pro Thr Lys
230                 235                 240                 245

aat ctc cct aaa cgg ggg tagtacgagg aattttgtcg gtg                   876
Asn Leu Pro Lys Arg Gly
                250

<210> 188
<211> 251
<212> PRT
<213> Corynebacterium glutamicum

<400> 188
Met Thr Ala Pro Arg Asp Pro Phe Phe Pro Ala Asp Leu Ser Ile Arg
  1               5                  10                  15

Ala Ser Ala Glu Pro Ile Glu Ile Gln Arg Leu Gly Leu Ile Asp Tyr
             20                  25                  30

Gln Glu Ala Trp Asp Tyr Gln Ala Glu Leu Ala Thr Arg Arg Ala Asn
         35                  40                  45

Asp Glu Ile Pro Asp Gln Leu Leu Ile Leu Glu His Pro Ser Val Tyr
     50                  55                  60

Thr Ala Gly Lys Arg Thr Gln Pro Glu Asp Leu Pro Thr Asn Gly Leu
 65                  70                  75                  80

Pro Val Ile Asn Ala Asp Arg Gly Gly Arg Ile Thr Trp His Gly Pro
                 85                  90                  95

Gly Gln Leu Val Ile Tyr Pro Ile Ile Lys Leu Ala Asp Pro Ile Asp
            100                 105                 110

Val Val Asp Tyr Val Arg Arg Leu Glu Glu Ala Leu Ile Gln Val Val
        115                 120                 125

Gly Asp Met Gly Val Ala Gly Ala Gly Arg Ile Asp Gly Arg Ser Gly
    130                 135                 140

Val Trp Val Pro Ala His Asp Gly Trp Val Asp Ser Lys Val Ala Ala
145                 150                 155                 160

Ile Gly Ile Arg Ile Thr Arg Gly Val Ala Met His Gly Val Ala Ile
                165                 170                 175

Asn Cys Asn Asn Thr Leu Asp Phe Tyr Glu His Ile Ile Pro Cys Gly
            180                 185                 190

Ile Ala Asp Ala Gly Leu Ser Thr Leu Ser Arg Glu Leu Lys Arg Asp
        195                 200                 205

Val Ser Val Glu Glu Leu Val Glu Pro Ser Ile Arg Ala Leu Asp Asp
    210                 215                 220

Ala Leu Ala Gly Arg Leu Val Val Ser Asp His Ser Phe Gly Ser Ala
225                 230                 235                 240

Pro Asp Pro Thr Lys Asn Leu Pro Lys Arg Gly
                245                 250

<210> 189
<211> 1167
<212> DNA
<213> Corynebacterium glutamicum

<220>
<221> CDS
<222> (101)..(1144)
<223> RXS01747

<400> 189
cgaagtagag ccgattgcag aatcggcgga atgagacgtc gaaaagcgtt taagctttcc 60

ctaaaaatat cactaactcg aaagatgtaa ggttgcattt gtg act atc gca cct   115
                                           Val Thr Ile Ala Pro
                                           1               5

gaa gga cga cga ctg cta cgc gtc gaa gct cga aac tca gaa acc ccg   163
Glu Gly Arg Arg Leu Leu Arg Val Glu Ala Arg Asn Ser Glu Thr Pro
                10                  15                  20

att gag acg aag cct cga tgg att aga aac cag gtc aaa aac gga cct   211
Ile Glu Thr Lys Pro Arg Trp Ile Arg Asn Gln Val Lys Asn Gly Pro
            25                  30                  35

gag tat cag gat atg aag gaa cgt gtc gct ggc gca tca cta cac act   259
Glu Tyr Gln Asp Met Lys Glu Arg Val Ala Gly Ala Ser Leu His Thr
        40                  45                  50

gtg tgt cag gag gct ggc tgt cct aat atc cat gag tgt tgg gaa tcc   307
Val Cys Gln Glu Ala Gly Cys Pro Asn Ile His Glu Cys Trp Glu Ser
    55                  60                  65

cgt gag gca acc ttc ctc att ggt ggc gcc aac tgc tct cgc cgc tgt   355
Arg Glu Ala Thr Phe Leu Ile Gly Gly Ala Asn Cys Ser Arg Arg Cys
70                  75                  80                  85

gat ttc tgc atg atc aac tcg gct cgc cct gag cca ctc gac cgc ggt   403
Asp Phe Cys Met Ile Asn Ser Ala Arg Pro Glu Pro Leu Asp Arg Gly
                90                  95                  100

gag cca ctg cgt gtc gct gag tct gtt cgt gag atg cag ctg aat tac   451
Glu Pro Leu Arg Val Ala Glu Ser Val Arg Glu Met Gln Leu Asn Tyr
            105                 110                 115

tcc acc atc acc ggt gtt acc cgt gat gat ctg gat gat gaa ggc gca   499
Ser Thr Ile Thr Gly Val Thr Arg Asp Asp Leu Asp Asp Glu Gly Ala
        120                 125                 130

tgg ctg tac tca gaa gtg gtt cgt aag atc cac gag ctg aac cca cac   547
Trp Leu Tyr Ser Glu Val Val Arg Lys Ile His Glu Leu Asn Pro His
    135                 140                 145

acc ggt gtg gaa aac ctg gtg cct gat ttc tcc ggc aag aag gat ctg   595

Thr Gly Val Glu Asn Leu Val Pro Asp Phe Ser Gly Lys Lys Asp Leu
150                 155                 160                 165

ctg cag gaa gtt ttt gaa tcc cgc cca gag gtt ttc gct cac aac gtg     643
Leu Gln Glu Val Phe Glu Ser Arg Pro Glu Val Phe Ala His Asn Val
                170                 175                 180

gaa act gtg cca cgt att ttc aag cgc att cgc cca gca ttc cgc tac     691
Glu Thr Val Pro Arg Ile Phe Lys Arg Ile Arg Pro Ala Phe Arg Tyr
            185                 190                 195

gag cgt tca ctt gat gtg atc cgt cag gct cgc gat ttc ggt ctg gtg     739
Glu Arg Ser Leu Asp Val Ile Arg Gln Ala Arg Asp Phe Gly Leu Val
        200                 205                 210

acc aag tcc aac ctg att ttg ggc atg ggt gaa acc aag gaa gaa atc     787
Thr Lys Ser Asn Leu Ile Leu Gly Met Gly Glu Thr Lys Glu Glu Ile
    215                 220                 225

acc gag gcg ctg cag gat ctg cac gac gct ggc tgt gac atc atc acc     835
Thr Glu Ala Leu Gln Asp Leu His Asp Ala Gly Cys Asp Ile Ile Thr
230                 235                 240                 245

atc acc cag tac ctg cgt cct ggt cct ttg ttc cac ccc atc gag cgt     883
Ile Thr Gln Tyr Leu Arg Pro Gly Pro Leu Phe His Pro Ile Glu Arg
                250                 255                 260

tgg gtg aag cct gag gag ttc ctc gag cac gct gat gct gca aag gaa     931
Trp Val Lys Pro Glu Glu Phe Leu Glu His Ala Asp Ala Ala Lys Glu
            265                 270                 275

atg ggc ttc gct gct gtt atg tcc ggc cca ttg gtt cgt tcc tct tac     979
Met Gly Phe Ala Ala Val Met Ser Gly Pro Leu Val Arg Ser Ser Tyr
        280                 285                 290

cgt gca ggc cgt ctg tac gcg cag gcc atg gag ttc cgt ggc gag gaa     1027
Arg Ala Gly Arg Leu Tyr Ala Gln Ala Met Glu Phe Arg Gly Glu Glu
    295                 300                 305

atc cca gca cac ctc gcg cac ctg aag gat act tcc gga gga tcc acc     1075
Ile Pro Ala His Leu Ala His Leu Lys Asp Thr Ser Gly Gly Ser Thr
310                 315                 320                 325

gcc cag gaa gca tct aca ctt ctg gag cgt tac ggt gct tcc gaa gac     1123
Ala Gln Glu Ala Ser Thr Leu Leu Glu Arg Tyr Gly Ala Ser Glu Asp
                330                 335                 340

acc cca gtg gtg tcc ttc aac taagcccgaa gtttttaac cgc                1167
Thr Pro Val Val Ser Phe Asn
            345

<210> 190
<211> 348
<212> PRT
<213> Corynebacterium glutamicum

<400> 190
Val Thr Ile Ala Pro Glu Gly Arg Arg Leu Leu Arg Val Glu Ala Arg
1               5                   10                  15

Asn Ser Glu Thr Pro Ile Glu Thr Lys Pro Arg Trp Ile Arg Asn Gln
                20                  25                  30

Val Lys Asn Gly Pro Glu Tyr Gln Asp Met Lys Glu Arg Val Ala Gly
            35                  40                  45

Ala Ser Leu His Thr Val Cys Gln Glu Ala Gly Cys Pro Asn Ile His
    50                  55                  60

Glu Cys Trp Glu Ser Arg Glu Ala Thr Phe Leu Ile Gly Gly Ala Asn
65                  70                  75                  80

Cys Ser Arg Arg Cys Asp Phe Cys Met Ile Asn Ser Ala Arg Pro Glu
                85                  90                  95

Pro Leu Asp Arg Gly Glu Pro Leu Arg Val Ala Glu Ser Val Arg Glu
            100                 105                 110

Met Gln Leu Asn Tyr Ser Thr Ile Thr Gly Val Thr Arg Asp Asp Leu
        115                 120                 125

Asp Asp Glu Gly Ala Trp Leu Tyr Ser Glu Val Val Arg Lys Ile His
    130                 135                 140

Glu Leu Asn Pro His Thr Gly Val Glu Asn Leu Val Pro Asp Phe Ser
145                 150                 155                 160

Gly Lys Lys Asp Leu Leu Gln Glu Val Phe Glu Ser Arg Pro Glu Val
                165                 170                 175

Phe Ala His Asn Val Glu Thr Val Pro Arg Ile Phe Lys Arg Ile Arg
            180                 185                 190

Pro Ala Phe Arg Tyr Glu Arg Ser Leu Asp Val Ile Arg Gln Ala Arg
        195                 200                 205

Asp Phe Gly Leu Val Thr Lys Ser Asn Leu Ile Leu Gly Met Gly Glu
    210                 215                 220

Thr Lys Glu Glu Ile Thr Glu Ala Leu Gln Asp Leu His Asp Ala Gly
225                 230                 235                 240

Cys Asp Ile Ile Thr Ile Thr Gln Tyr Leu Arg Pro Gly Pro Leu Phe
                245                 250                 255

His Pro Ile Glu Arg Trp Val Lys Pro Glu Glu Phe Leu Glu His Ala
            260                 265                 270

Asp Ala Ala Lys Glu Met Gly Phe Ala Ala Val Met Ser Gly Pro Leu
        275                 280                 285

Val Arg Ser Ser Tyr Arg Ala Gly Arg Leu Tyr Ala Gln Ala Met Glu
    290                 295                 300

Phe Arg Gly Glu Glu Ile Pro Ala His Leu Ala His Leu Lys Asp Thr
305                 310                 315                 320

Ser Gly Gly Ser Thr Ala Gln Glu Ala Ser Thr Leu Leu Glu Arg Tyr
                325                 330                 335

Gly Ala Ser Glu Asp Thr Pro Val Val Ser Phe Asn

340                345

<210> 191
<211> 903
<212> DNA
<213> Corynebacterium glutamicum

<220>
<221> CDS
<222> (101)..(880)
<223> RXC01748

<400> 191
atcaccaaat gtggcggttt tgcgtcgaaa agcgtgctct ttctacacct ctttgaggtt 60

cattttcgcg gtttcctcac aatcgcctat tgttaagtac atg gca gac gcg aaa 115
                                           Met Ala Asp Ala Lys
                                           1               5

aag cag gcg gat aaa gcc gcc aag aag cag gta aga gca gcc aag aag 163
Lys Gln Ala Asp Lys Ala Ala Lys Lys Gln Val Arg Ala Ala Lys Lys
                10                  15                  20

gca cag cgc aag gag act cgc tca caa atg tgg cag gtc ttc aac atg 211
Ala Gln Arg Lys Glu Thr Arg Ser Gln Met Trp Gln Val Phe Asn Met
            25                  30                  35

caa cgc aag cag gat aag gct ctt att ccg ctt ctg ttg ctc gct att 259
Gln Arg Lys Gln Asp Lys Ala Leu Ile Pro Leu Leu Leu Leu Ala Ile
        40                  45                  50

ctt ggt atc ccg ctg gtc ctt ttc ctc atc ggt ttg att tgg ggt ggt 307
Leu Gly Ile Pro Leu Val Leu Phe Leu Ile Gly Leu Ile Trp Gly Gly
    55                  60                  65

cag tgg tgg atg ctt ccg atc ggc att gct gca ggt gtt gta gct gca 355
Gln Trp Trp Met Leu Pro Ile Gly Ile Ala Ala Gly Val Val Ala Ala
70                  75                  80                  85

atg ttt att ttc acc cgt cgc gtt gag cgt gac gtg tac aag cgc gcc 403
Met Phe Ile Phe Thr Arg Arg Val Glu Arg Asp Val Tyr Lys Arg Ala
                90                  95                  100

gaa ggt cag cag ggt gct gct ggt tgg gct gtg gag aac ctc cgc tct 451
Glu Gly Gln Gln Gly Ala Ala Gly Trp Ala Val Glu Asn Leu Arg Ser
            105                 110                 115

ggc gtg ggc atg acc tgg cgc acc aag acc gct gtt gca gtg acc act 499
Gly Val Gly Met Thr Trp Arg Thr Lys Thr Ala Val Ala Val Thr Thr
        120                 125                 130

cag atg gat gca gtg cac cgc gtc att ggt ctg tgt ggt gtt gtg ctg 547
Gln Met Asp Ala Val His Arg Val Ile Gly Leu Cys Gly Val Val Leu
    135                 140                 145

gtc ggc gag ggc tcc cct cac cgc ctg aag cca atg ctt gcg cag caa 595
Val Gly Glu Gly Ser Pro His Arg Leu Lys Pro Met Leu Ala Gln Gln
150                 155                 160                 165

aag aag cgc ctg aac cgc gtg gca cct ggt gtt cca gtg tat gaa atc 643

Lys Lys Arg Leu Asn Arg Val Ala Pro Gly Val Pro Val Tyr Glu Ile
                170                 175                 180

atc acg ggc aac ggc gaa ggc cag acc cct atc gcg aag ctg cag cgt     691
Ile Thr Gly Asn Gly Glu Gly Gln Thr Pro Ile Ala Lys Leu Gln Arg
            185                 190                 195

gaa ctg gtc aag ctg cct cgc aac tac aag aag aac gac gtc gct gcc     739
Glu Leu Val Lys Leu Pro Arg Asn Tyr Lys Lys Asn Asp Val Ala Ala
        200                 205                 210

ctg gcc gct cgc att gag gct atg gac aat gtc gga aac gct cct ggc     787
Leu Ala Ala Arg Ile Glu Ala Met Asp Asn Val Gly Asn Ala Pro Gly
    215                 220                 225

gga tct ttg cct aag ggt cca ttg cca aag ggc gca agc atg tcc ggt     835
Gly Ser Leu Pro Lys Gly Pro Leu Pro Lys Gly Ala Ser Met Ser Gly
230                 235                 240                 245

atg aac cgc cgc gct cgc cga cag gct gaa cgc aag ggc gag gct         880
Met Asn Arg Arg Ala Arg Arg Gln Ala Glu Arg Lys Gly Glu Ala
                250                 255                 260

taaagccttt tcgctttcgc gtc                                           903

<210> 192
<211> 260
<212> PRT
<213> Corynebacterium glutamicum

<400> 192
Met Ala Asp Ala Lys Lys Gln Ala Asp Lys Ala Ala Lys Lys Gln Val
  1               5                  10                  15

Arg Ala Ala Lys Lys Ala Gln Arg Lys Glu Thr Arg Ser Gln Met Trp
             20                  25                  30

Gln Val Phe Asn Met Gln Arg Lys Gln Asp Lys Ala Leu Ile Pro Leu
         35                  40                  45

Leu Leu Leu Ala Ile Leu Gly Ile Pro Leu Val Leu Phe Leu Ile Gly
     50                  55                  60

Leu Ile Trp Gly Gly Gln Trp Trp Met Leu Pro Ile Gly Ile Ala Ala
 65                  70                  75                  80

Gly Val Val Ala Ala Met Phe Ile Phe Thr Arg Arg Val Glu Arg Asp
                 85                  90                  95

Val Tyr Lys Arg Ala Glu Gly Gln Gln Gly Ala Ala Gly Trp Ala Val
            100                 105                 110

Glu Asn Leu Arg Ser Gly Val Gly Met Thr Trp Arg Thr Lys Thr Ala
        115                 120                 125

Val Ala Val Thr Thr Gln Met Asp Ala Val His Arg Val Ile Gly Leu
    130                 135                 140

Cys Gly Val Val Leu Val Gly Glu Gly Ser Pro His Arg Leu Lys Pro
145                 150                 155                 160

Met Leu Ala Gln Gln Lys Lys Arg Leu Asn Arg Val Ala Pro Gly Val
                165                 170                 175

Pro Val Tyr Glu Ile Ile Thr Gly Asn Gly Glu Gly Gln Thr Pro Ile
            180                 185                 190

Ala Lys Leu Gln Arg Glu Leu Val Lys Leu Pro Arg Asn Tyr Lys Lys
        195                 200                 205

Asn Asp Val Ala Ala Leu Ala Ala Arg Ile Glu Ala Met Asp Asn Val
    210                 215                 220

Gly Asn Ala Pro Gly Gly Ser Leu Pro Lys Gly Pro Leu Pro Lys Gly
225                 230                 235                 240

Ala Ser Met Ser Gly Met Asn Arg Arg Ala Arg Arg Gln Ala Glu Arg
                245                 250                 255

Lys Gly Glu Ala
            260


<210> 193
<211> 936
<212> DNA
<213> Corynebacterium glutamicum

<220>
<221> CDS
<222> (101)..(913)
<223> RXC00354

<400> 193
ggcttgtcgg taaggctgca gggtagcggg agtttccttc agattggaag ttctttaatt 60

ttctcgatta tgtacctcat ttacgcgtaa agtttggggc atg gga aag tta ctt   115
                                           Met Gly Lys Leu Leu
                                           1               5

ttc gta gac atc ggt ggc aca ctg ctg gat tac tca aat gaa gtt ccg   163
Phe Val Asp Ile Gly Gly Thr Leu Leu Asp Tyr Ser Asn Glu Val Pro
                10                  15                  20

cgt tcg gcc gtt gac gcg atc cgt aag gca cgc gcc aaa gga cac cgc   211
Arg Ser Ala Val Asp Ala Ile Arg Lys Ala Arg Ala Lys Gly His Arg
            25                  30                  35

gtg tac ttg agc tct ggt cga agc agc gct gag gtg act tct cag ttg   259
Val Tyr Leu Ser Ser Gly Arg Ser Ser Ala Glu Val Thr Ser Gln Leu
        40                  45                  50

tgg gat atc gga gtg gat ggc ctc att ggc gca aat ggt gga tat gtg   307
Trp Asp Ile Gly Val Asp Gly Leu Ile Gly Ala Asn Gly Gly Tyr Val
    55                  60                  65

gaa agc gca cag gag tct gtg ttc cac cgc cgt ttg tcg ggt gag gag   355
Glu Ser Ala Gln Glu Ser Val Phe His Arg Arg Leu Ser Gly Glu Glu
70                  75                  80                  85

acc cgc cac att gtg gag tgg ctc tac aac cgt ggt ttg gag ttt tat   403

Thr Arg His Ile Val Glu Trp Leu Tyr Asn Arg Gly Leu Glu Phe Tyr
                90                  95                  100

ctc gag tcc aac aac ggt ttg tat gca agc cgt ggt ttc cgt gag gct     451
Leu Glu Ser Asn Asn Gly Leu Tyr Ala Ser Arg Gly Phe Arg Glu Ala
            105                 110                 115

tct aag cca gtg ctg tct cgc ctt tcg gag aag acc gac gtg aca gtc     499
Ser Lys Pro Val Leu Ser Arg Leu Ser Glu Lys Thr Asp Val Thr Val
        120                 125                 130

gat agc atg tac ccg gat atg ttc tgg ggc gcg agc ctt gat cgt gac     547
Asp Ser Met Tyr Pro Asp Met Phe Trp Gly Ala Ser Leu Asp Arg Asp
    135                 140                 145

gat gtg aac aag atc agt tac atc ttc aat tct cag gaa gat ttg gac     595
Asp Val Asn Lys Ile Ser Tyr Ile Phe Asn Ser Gln Glu Asp Leu Asp
150                 155                 160                 165

gca gcg cgt gag gcg ttc cct aac ctg gag cac acc acg tgg ggt ggt     643
Ala Ala Arg Glu Ala Phe Pro Asn Leu Glu His Thr Thr Trp Gly Gly
                170                 175                 180

cag acg ggt gcg ttg ttc ggc acg atc ggt gtg tct gtc aac aag aag     691
Gln Thr Gly Ala Leu Phe Gly Thr Ile Gly Val Ser Val Asn Lys Lys
            185                 190                 195

atc ggc gtg gat cgc ctg ctg aag tac ctg aac gca gat cgc gca aac     739
Ile Gly Val Asp Arg Leu Leu Lys Tyr Leu Asn Ala Asp Arg Ala Asn
        200                 205                 210

acc att gcg ttc ggc gac agc gat gag gat ctc tcc cta ttt gag gcg     787
Thr Ile Ala Phe Gly Asp Ser Asp Glu Asp Leu Ser Leu Phe Glu Ala
    215                 220                 225

agc gct tac ggc gtc gcg atg ggc gag gcc acc gaa tcg ctc aag gct     835
Ser Ala Tyr Gly Val Ala Met Gly Glu Ala Thr Glu Ser Leu Lys Ala
230                 235                 240                 245

gct gct gac ctg gtc acg gat gct gtt ggg cag gac ggc ttg cgc aat     883
Ala Ala Asp Leu Val Thr Asp Ala Val Gly Gln Asp Gly Leu Arg Asn
                250                 255                 260

gcg ttt tta aag ctt gag ctt atc gac gcc tgaccccatc aaagaacttc       933
Ala Phe Leu Lys Leu Glu Leu Ile Asp Ala
            265                 270

cca                                                                 936


<210> 194
<211> 271
<212> PRT
<213> Corynebacterium glutamicum

<400> 194
Met Gly Lys Leu Leu Phe Val Asp Ile Gly Gly Thr Leu Leu Asp Tyr
  1               5                  10                  15

Ser Asn Glu Val Pro Arg Ser Ala Val Asp Ala Ile Arg Lys Ala Arg
             20                  25                  30

Ala Lys Gly His Arg Val Tyr Leu Ser Ser Gly Arg Ser Ser Ala Glu
        35                  40                  45

Val Thr Ser Gln Leu Trp Asp Ile Gly Val Asp Gly Leu Ile Gly Ala
    50                  55                  60

Asn Gly Gly Tyr Val Glu Ser Ala Gln Glu Ser Val Phe His Arg Arg
65                  70                  75                  80

Leu Ser Gly Glu Glu Thr Arg His Ile Val Glu Trp Leu Tyr Asn Arg
                85                  90                  95

Gly Leu Glu Phe Tyr Leu Glu Ser Asn Asn Gly Leu Tyr Ala Ser Arg
            100                 105                 110

Gly Phe Arg Glu Ala Ser Lys Pro Val Leu Ser Arg Leu Ser Glu Lys
        115                 120                 125

Thr Asp Val Thr Val Asp Ser Met Tyr Pro Asp Met Phe Trp Gly Ala
    130                 135                 140

Ser Leu Asp Arg Asp Asp Val Asn Lys Ile Ser Tyr Ile Phe Asn Ser
145                 150                 155                 160

Gln Glu Asp Leu Asp Ala Ala Arg Glu Ala Phe Pro Asn Leu Glu His
                165                 170                 175

Thr Thr Trp Gly Gly Gln Thr Gly Ala Leu Phe Gly Thr Ile Gly Val
            180                 185                 190

Ser Val Asn Lys Lys Ile Gly Val Asp Arg Leu Leu Lys Tyr Leu Asn
        195                 200                 205

Ala Asp Arg Ala Asn Thr Ile Ala Phe Gly Asp Ser Asp Glu Asp Leu
    210                 215                 220

Ser Leu Phe Glu Ala Ser Ala Tyr Gly Val Ala Met Gly Glu Ala Thr
225                 230                 235                 240

Glu Ser Leu Lys Ala Ala Ala Asp Leu Val Thr Asp Ala Val Gly Gln
                245                 250                 255

Asp Gly Leu Arg Asn Ala Phe Leu Lys Leu Glu Leu Ile Asp Ala
            260                 265                 270

<210> 195
<211> 1740
<212> DNA
<213> Corynebacterium glutamicum

<220>
<221> CDS
<222> (101)..(1717)
<223> RXC01749

<400> 195
ggtttttgcg tgctctggtt tagggactgg ttttgggaac gtgcccagtt ccacatcaaa 60

taacgctgag gtcgtactta atccatgaga tcatgaatgg gtg agc ttc ctt gta 115

Val Ser Phe Leu Val
1               5

gaa aat caa tta ctc gcg ttg gtt gtc atc atg acg gtc gga cta ttg     163
Glu Asn Gln Leu Leu Ala Leu Val Val Ile Met Thr Val Gly Leu Leu
                10                  15                  20

ctc ggc cgc atc aaa att ttc ggg ttc cgt ctc ggc gtc gcc gct gta     211
Leu Gly Arg Ile Lys Ile Phe Gly Phe Arg Leu Gly Val Ala Ala Val
            25                  30                  35

ctg ttt gta ggt cta gcg cta tcc acc att gag ccg gat att tcc gtc     259
Leu Phe Val Gly Leu Ala Leu Ser Thr Ile Glu Pro Asp Ile Ser Val
        40                  45                  50

cca tcc ctc att tac gtg gtt gga ctg tcg ctt ttt gtc tac acg atc     307
Pro Ser Leu Ile Tyr Val Val Gly Leu Ser Leu Phe Val Tyr Thr Ile
    55                  60                  65

ggt ctg gaa gcc ggc cct gga ttc ttc acc tcc atg aaa acc act ggt     355
Gly Leu Glu Ala Gly Pro Gly Phe Phe Thr Ser Met Lys Thr Thr Gly
70                  75                  80                  85

ctg cgc aac aac gca ctg acc ttg ggc gcc atc atc gcc acc acg gca     403
Leu Arg Asn Asn Ala Leu Thr Leu Gly Ala Ile Ile Ala Thr Thr Ala
                90                  95                  100

ctc gca tgg gca ctc atc aca gtt ttg aac atc gat gcc gcc tcc ggc     451
Leu Ala Trp Ala Leu Ile Thr Val Leu Asn Ile Asp Ala Ala Ser Gly
            105                 110                 115

gcc ggc atg ctc acc ggc gcg ctc acc aac acc cca gcc atg gcc gca     499
Ala Gly Met Leu Thr Gly Ala Leu Thr Asn Thr Pro Ala Met Ala Ala
        120                 125                 130

gtt gtt gac gca ctt cct tcg ctt atc gac gac acc ggc cag ctt cac     547
Val Val Asp Ala Leu Pro Ser Leu Ile Asp Asp Thr Gly Gln Leu His
    135                 140                 145

ctc atc gcc gag ctg ccc gtc gtc gca tat tcc ttg gca tac ccc ctc     595
Leu Ile Ala Glu Leu Pro Val Val Ala Tyr Ser Leu Ala Tyr Pro Leu
150                 155                 160                 165

ggt gtg ctc atc gtt att ctc tcc atc gcc atc ttc agc tct gtg ttc     643
Gly Val Leu Ile Val Ile Leu Ser Ile Ala Ile Phe Ser Ser Val Phe
                170                 175                 180

aaa gtc gac cac aac aaa gaa gcc gaa gaa gcg ggc gtt gcg gtc cag     691
Lys Val Asp His Asn Lys Glu Ala Glu Glu Ala Gly Val Ala Val Gln
            185                 190                 195

gaa ctc aaa ggc cgt cgc atc cgc gtc acc gtc gct gat ctt cca gcc     739
Glu Leu Lys Gly Arg Arg Ile Arg Val Thr Val Ala Asp Leu Pro Ala
        200                 205                 210

ctg gag aac atc cca gag ctg ctc aac ctc cac gtc att gtg tcc cga     787
Leu Glu Asn Ile Pro Glu Leu Leu Asn Leu His Val Ile Val Ser Arg
    215                 220                 225

gtg gaa cga gac ggt gag caa ttc atc ccg ctt tat ggc gaa cac gca     835
Val Glu Arg Asp Gly Glu Gln Phe Ile Pro Leu Tyr Gly Glu His Ala

230                 235                 240                 245

cgc atc ggc gat gtc tta aca gtg gtg ggt gcc gat gaa gaa ctc aac     883
Arg Ile Gly Asp Val Leu Thr Val Val Gly Ala Asp Glu Glu Leu Asn
                250                 255                 260

cgc gcg gaa aaa gcc atc ggt gaa ctc att gac ggc gac ccc tac agc     931
Arg Ala Glu Lys Ala Ile Gly Glu Leu Ile Asp Gly Asp Pro Tyr Ser
            265                 270                 275

aat gtg gaa ctt gat tac cga cgc atc ttc gtc tca aac aca gca gtc     979
Asn Val Glu Leu Asp Tyr Arg Arg Ile Phe Val Ser Asn Thr Ala Val
        280                 285                 290

gtg ggc act ccc cta tcc aag ctc cag cca ctg ttt aaa gac atg ctg     1027
Val Gly Thr Pro Leu Ser Lys Leu Gln Pro Leu Phe Lys Asp Met Leu
    295                 300                 305

atc acc cgc atc agg cgc ggc gac aca gat ttg gtg gcc tcc tcc gac     1075
Ile Thr Arg Ile Arg Arg Gly Asp Thr Asp Leu Val Ala Ser Ser Asp
310                 315                 320                 325

atg act ttg cag ctc ggt gac cgt gtc cgc gtt gtc gca cca gca gaa     1123
Met Thr Leu Gln Leu Gly Asp Arg Val Arg Val Val Ala Pro Ala Glu
                330                 335                 340

aaa ctc cgc gaa gca acc caa ttg ctc ggc gat tcc tac aag aaa ctc     1171
Lys Leu Arg Glu Ala Thr Gln Leu Leu Gly Asp Ser Tyr Lys Lys Leu
            345                 350                 355

tcc gat ttc aac ctg ctc cca ctc gct gcc ggc ctc atg atc ggt gtg     1219
Ser Asp Phe Asn Leu Leu Pro Leu Ala Ala Gly Leu Met Ile Gly Val
        360                 365                 370

ctt gtc ggc atg gtg gag ttc cca cta cca ggt gga agc tcc ctg aaa     1267
Leu Val Gly Met Val Glu Phe Pro Leu Pro Gly Gly Ser Ser Leu Lys
    375                 380                 385

ctg ggt aac gca ggt gga ccg cta gtt gtt gcg ctg ctg ctc ggc atg     1315
Leu Gly Asn Ala Gly Gly Pro Leu Val Val Ala Leu Leu Leu Gly Met
390                 395                 400                 405

atc aat cgc aca ggc aag ttc gtc tgg caa atc ccc tac gga gca aac     1363
Ile Asn Arg Thr Gly Lys Phe Val Trp Gln Ile Pro Tyr Gly Ala Asn
                410                 415                 420

ctt gcc ctt cgc caa ctg ggc atc aca cta ttt ttg gct gcc atc ggt     1411
Leu Ala Leu Arg Gln Leu Gly Ile Thr Leu Phe Leu Ala Ala Ile Gly
            425                 430                 435

acc tca gcg ggc gca gga ttt cga tca gcg atc agc gac ccc caa tca     1459
Thr Ser Ala Gly Ala Gly Phe Arg Ser Ala Ile Ser Asp Pro Gln Ser
        440                 445                 450

ctc acc atc atc ggc ttc ggt gcg ctg ctc act ttg ttc atc tcc atc     1507
Leu Thr Ile Ile Gly Phe Gly Ala Leu Leu Thr Leu Phe Ile Ser Ile
    455                 460                 465

acg gtg ctg ttc gtt ggc cac aaa ctg atg aaa atc ccc ttc ggt gaa     1555
Thr Val Leu Phe Val Gly His Lys Leu Met Lys Ile Pro Phe Gly Glu
470                 475                 480                 485

acc gct ggc atc ctc gcc ggt acg caa acc cac cct gct gtg ctg agt     1603
Thr Ala Gly Ile Leu Ala Gly Thr Gln Thr His Pro Ala Val Leu Ser
                490                 495                 500

tat gtg tca gat gcc tcc cgc aac gag ctc cct gcc atg ggt tat acc     1651
Tyr Val Ser Asp Ala Ser Arg Asn Glu Leu Pro Ala Met Gly Tyr Thr
            505                 510                 515

tct gtg tat ccg ctg gcg atg atc gca aag atc ctg gcc gcc caa acg     1699
Ser Val Tyr Pro Leu Ala Met Ile Ala Lys Ile Leu Ala Ala Gln Thr
        520                 525                 530

ttg ttg ttc cta ctt atc tagcattgac cccttaagcg cag                   1740
Leu Leu Phe Leu Leu Ile
    535

<210> 196
<211> 539
<212> PRT
<213> Corynebacterium glutamicum

<400> 196
Val Ser Phe Leu Val Glu Asn Gln Leu Leu Ala Leu Val Val Ile Met
  1               5                  10                  15

Thr Val Gly Leu Leu Leu Gly Arg Ile Lys Ile Phe Gly Phe Arg Leu
             20                  25                  30

Gly Val Ala Ala Val Leu Phe Val Gly Leu Ala Leu Ser Thr Ile Glu
         35                  40                  45

Pro Asp Ile Ser Val Pro Ser Leu Ile Tyr Val Val Gly Leu Ser Leu
     50                  55                  60

Phe Val Tyr Thr Ile Gly Leu Glu Ala Gly Pro Gly Phe Phe Thr Ser
 65                  70                  75                  80

Met Lys Thr Thr Gly Leu Arg Asn Asn Ala Leu Thr Leu Gly Ala Ile
                 85                  90                  95

Ile Ala Thr Thr Ala Leu Ala Trp Ala Leu Ile Thr Val Leu Asn Ile
            100                 105                 110

Asp Ala Ala Ser Gly Ala Gly Met Leu Thr Gly Ala Leu Thr Asn Thr
        115                 120                 125

Pro Ala Met Ala Ala Val Val Asp Ala Leu Pro Ser Leu Ile Asp Asp
    130                 135                 140

Thr Gly Gln Leu His Leu Ile Ala Glu Leu Pro Val Val Ala Tyr Ser
145                 150                 155                 160

Leu Ala Tyr Pro Leu Gly Val Leu Ile Val Ile Leu Ser Ile Ala Ile
                165                 170                 175

Phe Ser Ser Val Phe Lys Val Asp His Asn Lys Glu Ala Glu Glu Ala
            180                 185                 190

Gly Val Ala Val Gln Glu Leu Lys Gly Arg Arg Ile Arg Val Thr Val

195                 200                 205

Ala Asp Leu Pro Ala Leu Glu Asn Ile Pro Glu Leu Leu Asn Leu His
    210                 215                 220

Val Ile Val Ser Arg Val Glu Arg Asp Gly Glu Gln Phe Ile Pro Leu
225                 230                 235                 240

Tyr Gly Glu His Ala Arg Ile Gly Asp Val Leu Thr Val Val Gly Ala
                245                 250                 255

Asp Glu Glu Leu Asn Arg Ala Glu Lys Ala Ile Gly Glu Leu Ile Asp
            260                 265                 270

Gly Asp Pro Tyr Ser Asn Val Glu Leu Asp Tyr Arg Arg Ile Phe Val
        275                 280                 285

Ser Asn Thr Ala Val Val Gly Thr Pro Leu Ser Lys Leu Gln Pro Leu
    290                 295                 300

Phe Lys Asp Met Leu Ile Thr Arg Ile Arg Arg Gly Asp Thr Asp Leu
305                 310                 315                 320

Val Ala Ser Ser Asp Met Thr Leu Gln Leu Gly Asp Arg Val Arg Val
                325                 330                 335

Val Ala Pro Ala Glu Lys Leu Arg Glu Ala Thr Gln Leu Leu Gly Asp
            340                 345                 350

Ser Tyr Lys Lys Leu Ser Asp Phe Asn Leu Leu Pro Leu Ala Ala Gly
        355                 360                 365

Leu Met Ile Gly Val Leu Val Gly Met Val Glu Phe Pro Leu Pro Gly
    370                 375                 380

Gly Ser Ser Leu Lys Leu Gly Asn Ala Gly Gly Pro Leu Val Val Ala
385                 390                 395                 400

Leu Leu Leu Gly Met Ile Asn Arg Thr Gly Lys Phe Val Trp Gln Ile
                405                 410                 415

Pro Tyr Gly Ala Asn Leu Ala Leu Arg Gln Leu Gly Ile Thr Leu Phe
            420                 425                 430

Leu Ala Ala Ile Gly Thr Ser Ala Gly Ala Gly Phe Arg Ser Ala Ile
        435                 440                 445

Ser Asp Pro Gln Ser Leu Thr Ile Ile Gly Phe Gly Ala Leu Leu Thr
    450                 455                 460

Leu Phe Ile Ser Ile Thr Val Leu Phe Val Gly His Lys Leu Met Lys
465                 470                 475                 480

Ile Pro Phe Gly Glu Thr Ala Gly Ile Leu Ala Gly Thr Gln Thr His
                485                 490                 495

Pro Ala Val Leu Ser Tyr Val Ser Asp Ala Ser Arg Asn Glu Leu Pro
            500                 505                 510

Ala Met Gly Tyr Thr Ser Val Tyr Pro Leu Ala Met Ile Ala Lys Ile
        515                 520                 525

Leu Ala Ala Gln Thr Leu Leu Phe Leu Leu Ile
    530                 535

<210> 197
<211> 1023
<212> DNA
<213> Corynebacterium glutamicum

<220>
<221> CDS
<222> (101)..(1000)
<223> RXA02268

<400> 197
tgaatcgttg gtgcttcgag ttgggattgt tatgtgggga gacgtcgata agcaaaacac 60

ttgccgagcg caagccggcc tacggcgcta gtgtgagcac atg tcc cag gaa aat   115
                                           Met Ser Gln Glu Asn
                                           1               5

tct ggt ttg ttc aag cgc gcg att aca cgt ggg gtg gct aag gtg cgc   163
Ser Gly Leu Phe Lys Arg Ala Ile Thr Arg Gly Val Ala Lys Val Arg
                10                  15                  20

cgg aat ccg cgc gag gat ttt gcg gag gaa ttc acc caa gaa ctc tac   211
Arg Asn Pro Arg Glu Asp Phe Ala Glu Glu Phe Thr Gln Glu Leu Tyr
            25                  30                  35

gat cac gca aca aat atc acc ctg ccc ctg acg gcg cgg ctg aag ccg   259
Asp His Ala Thr Asn Ile Thr Leu Pro Leu Thr Ala Arg Leu Lys Pro
        40                  45                  50

aat ggg ttt ttc cag gat gat tgg cgg gcg cga cca agt ggt gcg cga   307
Asn Gly Phe Phe Gln Asp Asp Trp Arg Ala Arg Pro Ser Gly Ala Arg
    55                  60                  65

ccg tgg ccg atc gtg cta att cac gga tcc ggg gcc agc aag ggt tca   355
Pro Trp Pro Ile Val Leu Ile His Gly Ser Gly Ala Ser Lys Gly Ser
70                  75                  80                  85

tgg gag gaa atg ggc gct gag ctg cgc agc aaa ggt tgg gcc gtg ttt   403
Trp Glu Glu Met Gly Ala Glu Leu Arg Ser Lys Gly Trp Ala Val Phe
                90                  95                  100

gcc cct gac ttt gga acg cgt gcc acc gag cca att gcg gcg tcg gct   451
Ala Pro Asp Phe Gly Thr Arg Ala Thr Glu Pro Ile Ala Ala Ser Ala
            105                 110                 115

gcc caa att ggt gcg tat att gat gcc gtt ttg ttg gtg acg ggc gct   499
Ala Gln Ile Gly Ala Tyr Ile Asp Ala Val Leu Leu Val Thr Gly Ala
        120                 125                 130

gcg cag att gtg ctg gtt ggg cat tcg caa ggc ggt gtc gtg gcg cgg   547
Ala Gln Ile Val Leu Val Gly His Ser Gln Gly Gly Val Val Ala Arg
    135                 140                 145

tat tgg atg cgc acc tac ggc gga tac atg aag gtc agg cac atg att   595
Tyr Trp Met Arg Thr Tyr Gly Gly Tyr Met Lys Val Arg His Met Ile
150                 155                 160                 165

tcc atc tct acg cca aat cac gga acg ctc atg gga ggc att tta aac     643
Ser Ile Ser Thr Pro Asn His Gly Thr Leu Met Gly Gly Ile Leu Asn
                170                 175                 180

ccg atg acg aag gtg aaa tcg gga gag gga acg atc gaa aag ctg atg     691
Pro Met Thr Lys Val Lys Ser Gly Glu Gly Thr Ile Glu Lys Leu Met
            185                 190                 195

cac aga cta ttc ggg ccc act ggt ttt gaa cag ctg cgc gga cac gac     739
His Arg Leu Phe Gly Pro Thr Gly Phe Glu Gln Leu Arg Gly His Asp
        200                 205                 210

atc atc gag ttt ttg gcc gac ggt ggg gac ctc gat cca ggc gtc acc     787
Ile Ile Glu Phe Leu Ala Asp Gly Gly Asp Leu Asp Pro Gly Val Thr
    215                 220                 225

tac acc tgc att ggt acc cat ttt gat cct ttc atc caa cct ccg gag     835
Tyr Thr Cys Ile Gly Thr His Phe Asp Pro Phe Ile Gln Pro Pro Glu
230                 235                 240                 245

gtg gcc ttt ttg gag gtc aac gag gac gat gat cca aat cga gtc cac     883
Val Ala Phe Leu Glu Val Asn Glu Asp Asp Asp Pro Asn Arg Val His
                250                 255                 260

aat att tgg gtc gaa gat gaa cac ccg cgc gca atg att gcc cac aac     931
Asn Ile Trp Val Glu Asp Glu His Pro Arg Ala Met Ile Ala His Asn
            265                 270                 275

gat atg gtg cgc gat ccc agg gtg atc gaa atc gta cgc gca gag ctc     979
Asp Met Val Arg Asp Pro Arg Val Ile Glu Ile Val Arg Ala Glu Leu
        280                 285                 290

gac cgg gtg gca cgc ctc ggc taagttgggg acatggttga cgc              1023
Asp Arg Val Ala Arg Leu Gly
    295                 300


<210> 198
<211> 300
<212> PRT
<213> Corynebacterium glutamicum

<400> 198
Met Ser Gln Glu Asn Ser Gly Leu Phe Lys Arg Ala Ile Thr Arg Gly
  1               5                  10                  15

Val Ala Lys Val Arg Arg Asn Pro Arg Glu Asp Phe Ala Glu Glu Phe
             20                  25                  30

Thr Gln Glu Leu Tyr Asp His Ala Thr Asn Ile Thr Leu Pro Leu Thr
         35                  40                  45

Ala Arg Leu Lys Pro Asn Gly Phe Phe Gln Asp Asp Trp Arg Ala Arg
     50                  55                  60

Pro Ser Gly Ala Arg Pro Trp Pro Ile Val Leu Ile His Gly Ser Gly
 65                  70                  75                  80

Ala Ser Lys Gly Ser Trp Glu Glu Met Gly Ala Glu Leu Arg Ser Lys
                 85                  90                  95

Gly Trp Ala Val Phe Ala Pro Asp Phe Gly Thr Arg Ala Thr Glu Pro
            100                 105                 110

Ile Ala Ala Ser Ala Ala Gln Ile Gly Ala Tyr Ile Asp Ala Val Leu
        115                 120                 125

Leu Val Thr Gly Ala Ala Gln Ile Val Leu Val Gly His Ser Gln Gly
    130                 135                 140

Gly Val Val Ala Arg Tyr Trp Met Arg Thr Tyr Gly Gly Tyr Met Lys
145                 150                 155                 160

Val Arg His Met Ile Ser Ile Ser Thr Pro Asn His Gly Thr Leu Met
                165                 170                 175

Gly Gly Ile Leu Asn Pro Met Thr Lys Val Lys Ser Gly Glu Gly Thr
            180                 185                 190

Ile Glu Lys Leu Met His Arg Leu Phe Gly Pro Thr Gly Phe Glu Gln
        195                 200                 205

Leu Arg Gly His Asp Ile Ile Glu Phe Leu Ala Asp Gly Gly Asp Leu
    210                 215                 220

Asp Pro Gly Val Thr Tyr Thr Cys Ile Gly Thr His Phe Asp Pro Phe
225                 230                 235                 240

Ile Gln Pro Pro Glu Val Ala Phe Leu Glu Val Asn Glu Asp Asp Asp
                245                 250                 255

Pro Asn Arg Val His Asn Ile Trp Val Glu Asp Glu His Pro Arg Ala
            260                 265                 270

Met Ile Ala His Asn Asp Met Val Arg Asp Pro Arg Val Ile Glu Ile
        275                 280                 285

Val Arg Ala Glu Leu Asp Arg Val Ala Arg Leu Gly
    290                 295                 300


<210> 199
<211> 1095
<212> DNA
<213> Corynebacterium glutamicum

<220>
<221> CDS
<222> (101)..(1072)
<223> RXA02269

<400> 199
cgcaatgatt gcccacaacg atatggtgcg cgatcccagg gtgatcgaaa tcgtacgcgc 60

agagctcgac cgggtggcac gcctcggcta agttggggac atg gtt gac gcc ctc   115
                                           Met Val Asp Ala Leu
                                           1               5

aat gat ctc cgc cga gaa ctc aca aac gcg tta agg tcc gtg tgg aaa   163
Asn Asp Leu Arg Arg Glu Leu Thr Asn Ala Leu Arg Ser Val Trp Lys
                10                  15                  20

aac ctc ccc act gat aac gcc ccg cag gcc gat gcc ttg cca gac gat 211
Asn Leu Pro Thr Asp Asn Ala Pro Gln Ala Asp Ala Leu Pro Asp Asp
            25                  30                  35

gta gtg gaa gag att gcg ata aat ttc tac cgt gat ccc aaa aac cgc 259
Val Val Glu Glu Ile Ala Ile Asn Phe Tyr Arg Asp Pro Lys Asn Arg
        40                  45                  50

ggc aaa ctc aac gaa gac aaa aca gat tcc ttg ccg atg ctc gcg cgc 307
Gly Lys Leu Asn Glu Asp Lys Thr Asp Ser Leu Pro Met Leu Ala Arg
    55                  60                  65

ata cgt tca cgt gga ctt ttt gaa gac gat tgg cgc gcc cgc ccc acc 355
Ile Arg Ser Arg Gly Leu Phe Glu Asp Asp Trp Arg Ala Arg Pro Thr
70                  75                  80                  85

gaa gac cgc ccc tgg cca gtg gta tta gtc cac gga act gga tca aca 403
Glu Asp Arg Pro Trp Pro Val Val Leu Val His Gly Thr Gly Ser Thr
                90                  95                  100

aaa ggt gat tgg caa gac ttg gga gcc gat cta cgc cgc gac ggc tgg 451
Lys Gly Asp Trp Gln Asp Leu Gly Ala Asp Leu Arg Arg Asp Gly Trp
            105                 110                 115

gca gtg ttt gca ccc gaa ttt ggc caa cgc gcc acc ggt tca gtc gca 499
Ala Val Phe Ala Pro Glu Phe Gly Gln Arg Ala Thr Gly Ser Val Ala
        120                 125                 130

gaa tca tcc gca caa att ggc gcc tat ata gat aca gta ttg ctt gct 547
Glu Ser Ser Ala Gln Ile Gly Ala Tyr Ile Asp Thr Val Leu Leu Ala
    135                 140                 145

aca gga gcc tca aaa gtc att gtc gtt ggc cac tcc caa ggc ggc gtg 595
Thr Gly Ala Ser Lys Val Ile Val Val Gly His Ser Gln Gly Gly Val
150                 155                 160                 165

ttg ctg aga tac tgg atg cgt gtt ttg ggt ggt gca tcc aaa gtc aaa 643
Leu Leu Arg Tyr Trp Met Arg Val Leu Gly Gly Ala Ser Lys Val Lys
                170                 175                 180

cac atg gtc tcc ctc gct gtc ccc aat cac ggc acc acc atg ggc gga 691
His Met Val Ser Leu Ala Val Pro Asn His Gly Thr Thr Met Gly Gly
            185                 190                 195

atc gtc agc ccg cta atc cgt aac aat cgt ggc gaa agt gtg gtt aat 739
Ile Val Ser Pro Leu Ile Arg Asn Asn Arg Gly Glu Ser Val Val Asn
        200                 205                 210

tct gtc gtt caa tca tgg ttc ggc gaa gct gga ttt gaa atg atc cgc 787
Ser Val Val Gln Ser Trp Phe Gly Glu Ala Gly Phe Glu Met Ile Arg
    215                 220                 225

gga cac gac acc atc aac gcc atc aat gaa ggc ggc gat ttg gat cca 835
Gly His Asp Thr Ile Asn Ala Ile Asn Glu Gly Gly Asp Leu Asp Pro
230                 235                 240                 245

gac gtg aca tat ctg tgc atc gcc acc cac ttt gac acc gtg att cag 883
Asp Val Thr Tyr Leu Cys Ile Ala Thr His Phe Asp Thr Val Ile Gln
                250                 255                 260

ccc cct gaa acc tgc ttc cta gag gcc cgg aac ccc gaa gaa ctc aag     931
Pro Pro Glu Thr Cys Phe Leu Glu Ala Arg Asn Pro Glu Glu Leu Lys
            265                 270                 275

cgg gtc caa aac atc tgg gtg gaa aac ctc gac ccc aat tca gtc gtg     979
Arg Val Gln Asn Ile Trp Val Glu Asn Leu Asp Pro Asn Ser Val Val
        280                 285                 290

ctc cac gaa gca atg cct tac gat ccc cgc gta cgc gca ctg gtc agg     1027
Leu His Glu Ala Met Pro Tyr Asp Pro Arg Val Arg Ala Leu Val Arg
    295                 300                 305

gcg gat ttg agc aaa ttg gtg gag att tcc gag act gcg gag aac         1072
Ala Asp Leu Ser Lys Leu Val Glu Ile Ser Glu Thr Ala Glu Asn
310                 315                 320

taggggtttt ggtggttgtc taa                                           1095

<210> 200
<211> 324
<212> PRT
<213> Corynebacterium glutamicum

<400> 200
Met Val Asp Ala Leu Asn Asp Leu Arg Arg Glu Leu Thr Asn Ala Leu
1               5                   10                  15

Arg Ser Val Trp Lys Asn Leu Pro Thr Asp Asn Ala Pro Gln Ala Asp
            20                  25                  30

Ala Leu Pro Asp Asp Val Val Glu Glu Ile Ala Ile Asn Phe Tyr Arg
        35                  40                  45

Asp Pro Lys Asn Arg Gly Lys Leu Asn Glu Asp Lys Thr Asp Ser Leu
    50                  55                  60

Pro Met Leu Ala Arg Ile Arg Ser Arg Gly Leu Phe Glu Asp Asp Trp
65                  70                  75                  80

Arg Ala Arg Pro Thr Glu Asp Arg Pro Trp Pro Val Val Leu Val His
                85                  90                  95

Gly Thr Gly Ser Thr Lys Gly Asp Trp Gln Asp Leu Gly Ala Asp Leu
            100                 105                 110

Arg Arg Asp Gly Trp Ala Val Phe Ala Pro Glu Phe Gly Gln Arg Ala
        115                 120                 125

Thr Gly Ser Val Ala Glu Ser Ser Ala Gln Ile Gly Ala Tyr Ile Asp
    130                 135                 140

Thr Val Leu Leu Ala Thr Gly Ala Ser Lys Val Ile Val Val Gly His
145                 150                 155                 160

Ser Gln Gly Gly Val Leu Leu Arg Tyr Trp Met Arg Val Leu Gly Gly
                165                 170                 175

Ala Ser Lys Val Lys His Met Val Ser Leu Ala Val Pro Asn His Gly
            180                 185                 190

Thr Thr Met Gly Gly Ile Val Ser Pro Leu Ile Arg Asn Asn Arg Gly
        195                 200                 205

Glu Ser Val Val Asn Ser Val Val Gln Ser Trp Phe Gly Glu Ala Gly
    210                 215                 220

Phe Glu Met Ile Arg Gly His Asp Thr Ile Asn Ala Ile Asn Glu Gly
225                 230                 235                 240

Gly Asp Leu Asp Pro Asp Val Thr Tyr Leu Cys Ile Ala Thr His Phe
                245                 250                 255

Asp Thr Val Ile Gln Pro Pro Glu Thr Cys Phe Leu Glu Ala Arg Asn
            260                 265                 270

Pro Glu Glu Leu Lys Arg Val Gln Asn Ile Trp Val Glu Asn Leu Asp
        275                 280                 285

Pro Asn Ser Val Val Leu His Glu Ala Met Pro Tyr Asp Pro Arg Val
    290                 295                 300

Arg Ala Leu Val Arg Ala Asp Leu Ser Lys Leu Val Glu Ile Ser Glu
305                 310                 315                 320

Thr Ala Glu Asn

<210> 201
<211> 1146
<212> DNA
<213> Corynebacterium glutamicum

<220>
<221> CDS
<222> (101)..(1123)
<223> RXA01614

<400> 201
tgcaattcgc aaacatgagt agtatcgcgg aagtttcacc acgtgaacat ttcagtcgac 60

tcgcgcacac cacaccaaca atcgacctat cgttatacgt atg aat cag atg cag    115
                                            Met Asn Gln Met Gln
                                            1               5

cag tgg aaa cca gac ttc ctg gga gag ggc tac caa aac ctc acc atc    163
Gln Trp Lys Pro Asp Phe Leu Gly Glu Gly Tyr Gln Asn Leu Thr Ile
                10                  15                  20

gag ctc ggc gac gac ccg gat aat gaa aca gat gtt gtg aca acg gtt    211
Glu Leu Gly Asp Asp Pro Asp Asn Glu Thr Asp Val Val Thr Thr Val
            25                  30                  35

gtg cgc tac aac cca gac aat cac gcg gac gag tct ttt gct gcc cgc    259
Val Arg Tyr Asn Pro Asp Asn His Ala Asp Glu Ser Phe Ala Ala Arg
        40                  45                  50

cca gcg ttg ctg tgg gtt cac ggc atg acg gac tac ttc ttc cac act    307
Pro Ala Leu Leu Trp Val His Gly Met Thr Asp Tyr Phe Phe His Thr
    55                  60                  65

gaa ttc gcg gag ttt ttc cac aat gcc ggt ttt gct gtg tac ggc att 355
Glu Phe Ala Glu Phe Phe His Asn Ala Gly Phe Ala Val Tyr Gly Ile
70                  75                  80                  85

gat ctt aga aaa tgt gga cgc tcc tac cgt cca gga cag cag tgg cac 403
Asp Leu Arg Lys Cys Gly Arg Ser Tyr Arg Pro Gly Gln Gln Trp His
                90                  95                  100

tac acc tct gat ctt gcc cat tac ttc cct gac tta aca gct gct gcc 451
Tyr Thr Ser Asp Leu Ala His Tyr Phe Pro Asp Leu Thr Ala Ala Ala
            105                 110                 115

gag gtc atc tct tcc acc cac cct gag cta gtc ccc gtc gcc cat tcc 499
Glu Val Ile Ser Ser Thr His Pro Glu Leu Val Pro Val Ala His Ser
        120                 125                 130

act ggt gga ctc atc gtt cct ctg tgg atg tcc cag atg cgc aca agc 547
Thr Gly Gly Leu Ile Val Pro Leu Trp Met Ser Gln Met Arg Thr Ser
    135                 140                 145

aat cca gct gcc att gag aag att cca gcg ctg gtc ctc aac agt ccg 595
Asn Pro Ala Ala Ile Glu Lys Ile Pro Ala Leu Val Leu Asn Ser Pro
150                 155                 160                 165

tgg ctg gac atg atg tat cca cca ctg ttc atc aag ctg atc acc cct 643
Trp Leu Asp Met Met Tyr Pro Pro Leu Phe Ile Lys Leu Ile Thr Pro
                170                 175                 180

atg gtg agg gtg ttg ggc aaa cgc tcc ccc aca acc atc atc cca ggc 691
Met Val Arg Val Leu Gly Lys Arg Ser Pro Thr Thr Ile Ile Pro Gly
            185                 190                 195

gga ggt ttg gga gca tac gga aaa tcg atc cat aag aac ttt tac ggc 739
Gly Gly Leu Gly Ala Tyr Gly Lys Ser Ile His Lys Asn Phe Tyr Gly
        200                 205                 210

gaa tgg gac ttt gac acc acc atc aag cct gta gaa gga cat aaa aag 787
Glu Trp Asp Phe Asp Thr Thr Ile Lys Pro Val Glu Gly His Lys Lys
    215                 220                 225

agc atc gga tgg ctt cgg gca gtc atg gct ggc caa gca gaa atc cat 835
Ser Ile Gly Trp Leu Arg Ala Val Met Ala Gly Gln Ala Glu Ile His
230                 235                 240                 245

cac gac cac gtg aat gtc gga gtg gac gtg ctc acg ctg tgt tca aat 883
His Asp His Val Asn Val Gly Val Asp Val Leu Thr Leu Cys Ser Asn
                250                 255                 260

aag tcc tgg ttg aag tct gaa tac aca gag gac acc aac act tca gac 931
Lys Ser Trp Leu Lys Ser Glu Tyr Thr Glu Asp Thr Asn Thr Ser Asp
            265                 270                 275

gcg gtt ttg gat gtg aaa cac att caa aag tgg gct cct cat ttg agc 979
Ala Val Leu Asp Val Lys His Ile Gln Lys Trp Ala Pro His Leu Ser
        280                 285                 290

tcg cca tcg tcc agg gtt gat gtt gag atc atc gac aac gct cgc cac 1027
Ser Pro Ser Ser Arg Val Asp Val Glu Ile Ile Asp Asn Ala Arg His
    295                 300                 305

gat att ttc ctc tca agg aaa ccc gcc aga gat cac gcc tct gaa gta 1075

Asp Ile Phe Leu Ser Arg Lys Pro Ala Arg Asp His Ala Ser Glu Val
310                 315                 320                 325

ctc aac aac tgg ctg caa tcg aag ctt tcc agc ctc aaa cca tct caa    1123
Leu Asn Asn Trp Leu Gln Ser Lys Leu Ser Ser Leu Lys Pro Ser Gln
                330                 335                 340

taacaccgcg aattatagac tga                                          1146

<210> 202
<211> 341
<212> PRT
<213> Corynebacterium glutamicum

<400> 202
Met Asn Gln Met Gln Gln Trp Lys Pro Asp Phe Leu Gly Glu Gly Tyr
  1               5                  10                  15

Gln Asn Leu Thr Ile Glu Leu Gly Asp Asp Pro Asp Asn Glu Thr Asp
             20                  25                  30

Val Val Thr Thr Val Val Arg Tyr Asn Pro Asp Asn His Ala Asp Glu
         35                  40                  45

Ser Phe Ala Ala Arg Pro Ala Leu Leu Trp Val His Gly Met Thr Asp
     50                  55                  60

Tyr Phe Phe His Thr Glu Phe Ala Glu Phe Phe His Asn Ala Gly Phe
 65                  70                  75                  80

Ala Val Tyr Gly Ile Asp Leu Arg Lys Cys Gly Arg Ser Tyr Arg Pro
                 85                  90                  95

Gly Gln Gln Trp His Tyr Thr Ser Asp Leu Ala His Tyr Phe Pro Asp
            100                 105                 110

Leu Thr Ala Ala Ala Glu Val Ile Ser Ser Thr His Pro Glu Leu Val
        115                 120                 125

Pro Val Ala His Ser Thr Gly Gly Leu Ile Val Pro Leu Trp Met Ser
    130                 135                 140

Gln Met Arg Thr Ser Asn Pro Ala Ala Ile Glu Lys Ile Pro Ala Leu
145                 150                 155                 160

Val Leu Asn Ser Pro Trp Leu Asp Met Met Tyr Pro Pro Leu Phe Ile
                165                 170                 175

Lys Leu Ile Thr Pro Met Val Arg Val Leu Gly Lys Arg Ser Pro Thr
            180                 185                 190

Thr Ile Ile Pro Gly Gly Gly Leu Gly Ala Tyr Gly Lys Ser Ile His
        195                 200                 205

Lys Asn Phe Tyr Gly Glu Trp Asp Phe Asp Thr Thr Ile Lys Pro Val
    210                 215                 220

Glu Gly His Lys Lys Ser Ile Gly Trp Leu Arg Ala Val Met Ala Gly
225                 230                 235                 240

Gln Ala Glu Ile His His Asp His Val Asn Val Gly Val Asp Val Leu
                245                 250                 255

Thr Leu Cys Ser Asn Lys Ser Trp Leu Lys Ser Glu Tyr Thr Glu Asp
            260                 265                 270

Thr Asn Thr Ser Asp Ala Val Leu Asp Val Lys His Ile Gln Lys Trp
        275                 280                 285

Ala Pro His Leu Ser Ser Pro Ser Ser Arg Val Asp Val Glu Ile Ile
    290                 295                 300

Asp Asn Ala Arg His Asp Ile Phe Leu Ser Arg Lys Pro Ala Arg Asp
305                 310                 315                 320

His Ala Ser Glu Val Leu Asn Asn Trp Leu Gln Ser Lys Leu Ser Ser
                325                 330                 335

Leu Lys Pro Ser Gln
            340

<210> 203
<211> 630
<212> DNA
<213> Corynebacterium glutamicum

<220>
<221> CDS
<222> (101)..(607)
<223> RXA01983

<400> 203
atatcgctgc catggcagcc ttgctgggca acctcaagca cactgaccta gaagagctcc 60

ccaccgatta ccaggggtgt cctcccatgt ccgctgcgtt atg gaa ggc tac gga   115
                                           Met Glu Gly Tyr Gly
                                           1               5

cct acc cag atc gaa aag ctc tta cct gca tac aca cag gtc aac aca   163
Pro Thr Gln Ile Glu Lys Leu Leu Pro Ala Tyr Thr Gln Val Asn Thr
                10                  15                  20

gcc ggg aat aat cca gcg acg acg cct gag caa gat ctc ctc ggc gga   211
Ala Gly Asn Asn Pro Ala Thr Thr Pro Glu Gln Asp Leu Leu Gly Gly
            25                  30                  35

gct gca acc tcg ccg gaa aac tac gac cac cag ctg cag tac gca gtc   259
Ala Ala Thr Ser Pro Glu Asn Tyr Asp His Gln Leu Gln Tyr Ala Val
        40                  45                  50

gac gcc agt ccg gtg cat cag aat gcg gca cag gca ccg ccc ttc ctg   307
Asp Ala Ser Pro Val His Gln Asn Ala Ala Gln Ala Pro Pro Phe Leu
    55                  60                  65

atc atg cac ggc act ggt gac cgg atg gtc cct ccg gag caa tcg gct   355
Ile Met His Gly Thr Gly Asp Arg Met Val Pro Pro Glu Gln Ser Ala
70                  75                  80                  85

gcg ctg cac acc cat ctt gtg cag gct ggt cgg cag tcc acc ctg gta   403
Ala Leu His Thr His Leu Val Gln Ala Gly Arg Gln Ser Thr Leu Val

                90                  95                  100

ctc att gag ggc ttt ggc cac ggt ttc ctc aat ccc ggg gaa gtc gcg 451
Leu Ile Glu Gly Phe Gly His Gly Phe Leu Asn Pro Gly Glu Val Ala
            105                 110                 115

gag ctg ggg cca aac gtt cga cta gac aat ggt cgg ctc gag cgg gag 499
Glu Leu Gly Pro Asn Val Arg Leu Asp Asn Gly Arg Leu Glu Arg Glu
        120                 125                 130

cct cag aca aat ttc agc gcg cag cag agt ccg gga aac ccc ttt gaa 547
Pro Gln Thr Asn Phe Ser Ala Gln Gln Ser Pro Gly Asn Pro Phe Glu
    135                 140                 145

cta cag gga ctt gcc gcc gac cat gag atg atc aag cgc ttt ttc acc 595
Leu Gln Gly Leu Ala Ala Asp His Glu Met Ile Lys Arg Phe Phe Thr
150                 155                 160                 165

ctg cac ctt cgc taagactcta ccttcaccca act 630
Leu His Leu Arg

<210> 204
<211> 169
<212> PRT
<213> Corynebacterium glutamicum

<400> 204
Met Glu Gly Tyr Gly Pro Thr Gln Ile Glu Lys Leu Leu Pro Ala Tyr
1               5                   10                  15

Thr Gln Val Asn Thr Ala Gly Asn Asn Pro Ala Thr Thr Pro Glu Gln
            20                  25                  30

Asp Leu Leu Gly Gly Ala Ala Thr Ser Pro Glu Asn Tyr Asp His Gln
        35                  40                  45

Leu Gln Tyr Ala Val Asp Ala Ser Pro Val His Gln Asn Ala Ala Gln
    50                  55                  60

Ala Pro Pro Phe Leu Ile Met His Gly Thr Gly Asp Arg Met Val Pro
65                  70                  75                  80

Pro Glu Gln Ser Ala Ala Leu His Thr His Leu Val Gln Ala Gly Arg
                85                  90                  95

Gln Ser Thr Leu Val Leu Ile Glu Gly Phe Gly His Gly Phe Leu Asn
            100                 105                 110

Pro Gly Glu Val Ala Glu Leu Gly Pro Asn Val Arg Leu Asp Asn Gly
        115                 120                 125

Arg Leu Glu Arg Glu Pro Gln Thr Asn Phe Ser Ala Gln Gln Ser Pro
    130                 135                 140

Gly Asn Pro Phe Glu Leu Gln Gly Leu Ala Ala Asp His Glu Met Ile
145                 150                 155                 160

Lys Arg Phe Phe Thr Leu His Leu Arg
                165

<210> 205
<211> 1414
<212> DNA
<213> Corynebacterium glutamicum

<220>
<221> CDS
<222> (101)..(1414)
<223> RXN02947

<400> 205
gtatgttcac acaagaaccc tgcacaacgc cttcaaagta cgtcgaccac gaccaagcgc 60

attattcact ctcacccttc aggatttaga ctaagaaacc atg act gca gca cag 115
Met Thr Ala Ala Gln
1               5

acc aaa cct gac ctc acc acc acg gct gga aag ctg tcc gat ctt cgc 163
Thr Lys Pro Asp Leu Thr Thr Thr Ala Gly Lys Leu Ser Asp Leu Arg
                10                  15                  20

tcc cgt ctt gca gaa gct caa gct cca atg ggc gaa gca act gta gaa 211
Ser Arg Leu Ala Glu Ala Gln Ala Pro Met Gly Glu Ala Thr Val Glu
            25                  30                  35

aaa gtg cac gct gct ggc agg aag act gcc cgc gaa cgt atc gag tat 259
Lys Val His Ala Ala Gly Arg Lys Thr Ala Arg Glu Arg Ile Glu Tyr
        40                  45                  50

ttg ctc gat gag ggc tct ttc gta gag atc gat gct ctt gct cgt cac 307
Leu Leu Asp Glu Gly Ser Phe Val Glu Ile Asp Ala Leu Ala Arg His
    55                  60                  65

cgt tcc aag aac ttc ggc ctg gat gcc aag cgt cca gtt act gac ggt 355
Arg Ser Lys Asn Phe Gly Leu Asp Ala Lys Arg Pro Val Thr Asp Gly
70                  75                  80                  85

gtt gtg act ggt tac ggc acc atc gat ggc cgt aag gtc tgt gtg ttc 403
Val Val Thr Gly Tyr Gly Thr Ile Asp Gly Arg Lys Val Cys Val Phe
                90                  95                  100

tcc cag gac ggc gct gta ttc ggt ggc gct ttg ggt gaa gtt tat ggt 451
Ser Gln Asp Gly Ala Val Phe Gly Gly Ala Leu Gly Glu Val Tyr Gly
            105                 110                 115

gaa aag atc gtt aag gtt atg gat ctt gcg atc aag acc ggt gtg cct 499
Glu Lys Ile Val Lys Val Met Asp Leu Ala Ile Lys Thr Gly Val Pro
        120                 125                 130

ttg atc gga atc aat gag ggt gct ggt gcg cgt atc cag gaa ggt gtt 547
Leu Ile Gly Ile Asn Glu Gly Ala Gly Ala Arg Ile Gln Glu Gly Val
    135                 140                 145

gtg tct ctg ggt ctg tac tca cag atc ttc tac cgc aac acc cag gcg 595
Val Ser Leu Gly Leu Tyr Ser Gln Ile Phe Tyr Arg Asn Thr Gln Ala
150                 155                 160                 165

tct ggc gtt atc cca cag atc tct ttg atc atg ggt gcc tgc gct ggt 643
Ser Gly Val Ile Pro Gln Ile Ser Leu Ile Met Gly Ala Cys Ala Gly

                170                 175                 180

ggt cac gtg tac tcc cct gct ctg act gac ttc atc gtc atg gtg gat 691
Gly His Val Tyr Ser Pro Ala Leu Thr Asp Phe Ile Val Met Val Asp
            185                 190                 195

cag act tcc aag atg ttt atc acc ggc cct gat gtc atc aag act gtc 739
Gln Thr Ser Lys Met Phe Ile Thr Gly Pro Asp Val Ile Lys Thr Val
        200                 205                 210

acc ggt gaa gat gtc acc cag gag gag ctc ggt ggc gct cac acc cac 787
Thr Gly Glu Asp Val Thr Gln Glu Glu Leu Gly Gly Ala His Thr His
    215                 220                 225

atg gct acc tcc ggt acc tcc cat tac tct gct tct gat gat tca gat 835
Met Ala Thr Ser Gly Thr Ser His Tyr Ser Ala Ser Asp Asp Ser Asp
230                 235                 240                 245

gct ttg gat tgg gtt cgc gag ctg acc tct tat ctt cca tcc aac aac 883
Ala Leu Asp Trp Val Arg Glu Leu Thr Ser Tyr Leu Pro Ser Asn Asn
                250                 255                 260

cgt gcg gaa act cct cgc cag gag gcc gac atc atg atc ggt tcc atc 931
Arg Ala Glu Thr Pro Arg Gln Glu Ala Asp Ile Met Ile Gly Ser Ile
            265                 270                 275

cag gaa aac atc aac gat gtg gat ctg gaa ttg gac acc atc atc ccg 979
Gln Glu Asn Ile Asn Asp Val Asp Leu Glu Leu Asp Thr Ile Ile Pro
        280                 285                 290

gat tcc ccg aac cag cct tat gac atg aag gaa gtt att tcc cgc atc 1027
Asp Ser Pro Asn Gln Pro Tyr Asp Met Lys Glu Val Ile Ser Arg Ile
    295                 300                 305

gtc gac gac gcc gag ttc ttc gag atc cag gaa gac tac gca gag aac 1075
Val Asp Asp Ala Glu Phe Phe Glu Ile Gln Glu Asp Tyr Ala Glu Asn
310                 315                 320                 325

atc ctg tgt ggc ttc gct cgc gtt gag gtc cgt tct gtt ggc atc gtg 1123
Ile Leu Cys Gly Phe Ala Arg Val Glu Val Arg Ser Val Gly Ile Val
                330                 335                 340

gct aac cag cca acc cag ttc gct ggc tgc ttg gat att aag gca tct 1171
Ala Asn Gln Pro Thr Gln Phe Ala Gly Cys Leu Asp Ile Lys Ala Ser
            345                 350                 355

gag aag gct gcc cgt ttc atc cgc acc tgc gat gcc ttc aac atc cca 1219
Glu Lys Ala Ala Arg Phe Ile Arg Thr Cys Asp Ala Phe Asn Ile Pro
        360                 365                 370

atc ctt gag ttc gtg gac gtt cca ggc ttc ctg cct ggc acc aac cag 1267
Ile Leu Glu Phe Val Asp Val Pro Gly Phe Leu Pro Gly Thr Asn Gln
    375                 380                 385

gaa ttc gac ggc atc atc cgc cgc ggc gca aag ctg ctt tac gct tac 1315
Glu Phe Asp Gly Ile Ile Arg Arg Gly Ala Lys Leu Leu Tyr Ala Tyr
390                 395                 400                 405

gct gaa gca acc gtc ggc aag atc acc gtc atc acc cgc aag tcc tac 1363
Ala Glu Ala Thr Val Gly Lys Ile Thr Val Ile Thr Arg Lys Ser Tyr
                410                 415                 420

ggc gga gcg tac tgc gtg atg ggt tcc aag gat atg ggc gct ggc ctg     1411
Gly Gly Ala Tyr Cys Val Met Gly Ser Lys Asp Met Gly Ala Gly Leu
            425                 430                 435

gta                                                                 1414
Val

<210> 206
<211> 438
<212> PRT
<213> Corynebacterium glutamicum

<400> 206
Met Thr Ala Ala Gln Thr Lys Pro Asp Leu Thr Thr Thr Ala Gly Lys
1               5                   10                  15

Leu Ser Asp Leu Arg Ser Arg Leu Ala Glu Ala Gln Ala Pro Met Gly
            20                  25                  30

Glu Ala Thr Val Glu Lys Val His Ala Ala Gly Arg Lys Thr Ala Arg
        35                  40                  45

Glu Arg Ile Glu Tyr Leu Leu Asp Glu Gly Ser Phe Val Glu Ile Asp
    50                  55                  60

Ala Leu Ala Arg His Arg Ser Lys Asn Phe Gly Leu Asp Ala Lys Arg
65                  70                  75                  80

Pro Val Thr Asp Gly Val Val Thr Gly Tyr Gly Thr Ile Asp Gly Arg
                85                  90                  95

Lys Val Cys Val Phe Ser Gln Asp Gly Ala Val Phe Gly Gly Ala Leu
            100                 105                 110

Gly Glu Val Tyr Gly Glu Lys Ile Val Lys Val Met Asp Leu Ala Ile
        115                 120                 125

Lys Thr Gly Val Pro Leu Ile Gly Ile Asn Glu Gly Ala Gly Ala Arg
    130                 135                 140

Ile Gln Glu Gly Val Val Ser Leu Gly Leu Tyr Ser Gln Ile Phe Tyr
145                 150                 155                 160

Arg Asn Thr Gln Ala Ser Gly Val Ile Pro Gln Ile Ser Leu Ile Met
                165                 170                 175

Gly Ala Cys Ala Gly Gly His Val Tyr Ser Pro Ala Leu Thr Asp Phe
            180                 185                 190

Ile Val Met Val Asp Gln Thr Ser Lys Met Phe Ile Thr Gly Pro Asp
        195                 200                 205

Val Ile Lys Thr Val Thr Gly Glu Asp Val Thr Gln Glu Glu Leu Gly
    210                 215                 220

Gly Ala His Thr His Met Ala Thr Ser Gly Thr Ser His Tyr Ser Ala
225                 230                 235                 240

Ser Asp Asp Ser Asp Ala Leu Asp Trp Val Arg Glu Leu Thr Ser Tyr
                245                 250                 255

Leu Pro Ser Asn Asn Arg Ala Glu Thr Pro Arg Gln Glu Ala Asp Ile
            260                 265                 270

Met Ile Gly Ser Ile Gln Glu Asn Ile Asn Asp Val Asp Leu Glu Leu
        275                 280                 285

Asp Thr Ile Ile Pro Asp Ser Pro Asn Gln Pro Tyr Asp Met Lys Glu
    290                 295                 300

Val Ile Ser Arg Ile Val Asp Asp Ala Glu Phe Phe Glu Ile Gln Glu
305                 310                 315                 320

Asp Tyr Ala Glu Asn Ile Leu Cys Gly Phe Ala Arg Val Glu Val Arg
                325                 330                 335

Ser Val Gly Ile Val Ala Asn Gln Pro Thr Gln Phe Ala Gly Cys Leu
            340                 345                 350

Asp Ile Lys Ala Ser Glu Lys Ala Ala Arg Phe Ile Arg Thr Cys Asp
        355                 360                 365

Ala Phe Asn Ile Pro Ile Leu Glu Phe Val Asp Val Pro Gly Phe Leu
    370                 375                 380

Pro Gly Thr Asn Gln Glu Phe Asp Gly Ile Ile Arg Arg Gly Ala Lys
385                 390                 395                 400

Leu Leu Tyr Ala Tyr Ala Glu Ala Thr Val Gly Lys Ile Thr Val Ile
                405                 410                 415

Thr Arg Lys Ser Tyr Gly Gly Ala Tyr Cys Val Met Gly Ser Lys Asp
            420                 425                 430

Met Gly Ala Gly Leu Val
        435


<210> 207
<211> 688
<212> DNA
<213> Corynebacterium glutamicum

<220>
<221> CDS
<222> (101)..(688)
<223> FRXA02320

<400> 207
gtatgttcac acaagaaccc tgcacaacgc cttcaaagta cgtcgaccac gaccaagcgc 60

attattcact ctcacccttc aggatttaga ctaagaaacc atg act gca gca cag   115
                                            Met Thr Ala Ala Gln
                                            1               5

acc aaa cct gac ctc acc acc acg gct gga aag ctg tcc gat ctt cgc   163
Thr Lys Pro Asp Leu Thr Thr Thr Ala Gly Lys Leu Ser Asp Leu Arg
                10                  15                  20

tcc cgt ctt gca gaa gct caa gct cca atg ggc gaa gca act gta gaa    211
Ser Arg Leu Ala Glu Ala Gln Ala Pro Met Gly Glu Ala Thr Val Glu
            25                  30                  35

aaa gtg cac gct gct ggc agg aag act gcc cgc gaa cgt atc gag tat    259
Lys Val His Ala Ala Gly Arg Lys Thr Ala Arg Glu Arg Ile Glu Tyr
        40                  45                  50

ttg ctc gat gag ggc tct ttc gta gag atc gat gct ctt gct cgt cac    307
Leu Leu Asp Glu Gly Ser Phe Val Glu Ile Asp Ala Leu Ala Arg His
    55                  60                  65

cgt tcc aag aac ttc ggc ctg gat gcc aag cgt cca gtt act gac ggt    355
Arg Ser Lys Asn Phe Gly Leu Asp Ala Lys Arg Pro Val Thr Asp Gly
70                  75                  80                  85

gtt gtg act ggt tac ggc acc atc gat ggc cgt aag gtc tgt gtg ttc    403
Val Val Thr Gly Tyr Gly Thr Ile Asp Gly Arg Lys Val Cys Val Phe
                90                  95                  100

tcc cag gac ggc gct gta ttc ggt ggc gct ttg ggt gaa gtt tat ggt    451
Ser Gln Asp Gly Ala Val Phe Gly Gly Ala Leu Gly Glu Val Tyr Gly
            105                 110                 115

gaa aag atc gtt aag gtt atg gat ctt gcg atc aag acc ggt gtg cct    499
Glu Lys Ile Val Lys Val Met Asp Leu Ala Ile Lys Thr Gly Val Pro
        120                 125                 130

ttg atc gga atc aat gag ggt gct ggt gcg cgt atc cag gaa ggt gtt    547
Leu Ile Gly Ile Asn Glu Gly Ala Gly Ala Arg Ile Gln Glu Gly Val
    135                 140                 145

gtg tct ctg ggt ctg tac tca cag atc ttc tac cgc aac acc cag gcg    595
Val Ser Leu Gly Leu Tyr Ser Gln Ile Phe Tyr Arg Asn Thr Gln Ala
150                 155                 160                 165

tct ggc gtt atc cca cag atc tct ttg atc atg ggt gcc tgc gct ggt    643
Ser Gly Val Ile Pro Gln Ile Ser Leu Ile Met Gly Ala Cys Ala Gly
                170                 175                 180

ggt cac gtg tac tcc cct gct ctg act gac ttc atc gtc atg gtg        688
Gly His Val Tyr Ser Pro Ala Leu Thr Asp Phe Ile Val Met Val
            185                 190                 195


<210> 208
<211> 196
<212> PRT
<213> Corynebacterium glutamicum

<400> 208
Met Thr Ala Ala Gln Thr Lys Pro Asp Leu Thr Thr Thr Ala Gly Lys
1               5                   10                  15

Leu Ser Asp Leu Arg Ser Arg Leu Ala Glu Ala Gln Ala Pro Met Gly
            20                  25                  30

Glu Ala Thr Val Glu Lys Val His Ala Ala Gly Arg Lys Thr Ala Arg
        35                  40                  45

Glu Arg Ile Glu Tyr Leu Leu Asp Glu Gly Ser Phe Val Glu Ile Asp

50                  55                  60

Ala Leu Ala Arg His Arg Ser Lys Asn Phe Gly Leu Asp Ala Lys Arg
65                  70                  75                  80

Pro Val Thr Asp Gly Val Val Thr Gly Tyr Gly Thr Ile Asp Gly Arg
                85                  90                  95

Lys Val Cys Val Phe Ser Gln Asp Gly Ala Val Phe Gly Gly Ala Leu
            100                 105                 110

Gly Glu Val Tyr Gly Glu Lys Ile Val Lys Val Met Asp Leu Ala Ile
        115                 120                 125

Lys Thr Gly Val Pro Leu Ile Gly Ile Asn Glu Gly Ala Gly Ala Arg
    130                 135                 140

Ile Gln Glu Gly Val Val Ser Leu Gly Leu Tyr Ser Gln Ile Phe Tyr
145                 150                 155                 160

Arg Asn Thr Gln Ala Ser Gly Val Ile Pro Gln Ile Ser Leu Ile Met
                165                 170                 175

Gly Ala Cys Ala Gly Gly His Val Tyr Ser Pro Ala Leu Thr Asp Phe
            180                 185                 190

Ile Val Met Val
        195

<210> 209
<211> 519
<212> DNA
<213> Corynebacterium glutamicum

<220>
<221> CDS
<222> (1)..(519)
<223> FRXA02851

<400> 209
cct cgc cag aag gcc gac atc atg atc ggt tcc atc cag gaa aac atc      48
Pro Arg Gln Lys Ala Asp Ile Met Ile Gly Ser Ile Gln Glu Asn Ile
1               5                   10                  15

aac gat gtg gat ctg gaa ttg gac acc atc atc ccg gat tcc ccg aac      96
Asn Asp Val Asp Leu Glu Leu Asp Thr Ile Ile Pro Asp Ser Pro Asn
            20                  25                  30

cag cct tat gac atg aag gaa gtt att tcc cgc atc gtn gac gac gcc      144
Gln Pro Tyr Asp Met Lys Glu Val Ile Ser Arg Ile Val Asp Asp Ala
        35                  40                  45

gag ttc ttc gag atc cag gaa gac tac gca gag aac atc ctg tgt ggc      192
Glu Phe Phe Glu Ile Gln Glu Asp Tyr Ala Glu Asn Ile Leu Cys Gly
    50                  55                  60

ttc gct cgc gtt gag gnc cgt tnt gtt ggc atc gtg gct aac cag cca      240
Phe Ala Arg Val Glu Xaa Arg Xaa Val Gly Ile Val Ala Asn Gln Pro
65                  70                  75                  80

acc cag ttc gct ggc tgn ttg gat att aag gca tct gag aag gct gcc     288
Thr Gln Phe Ala Gly Xaa Leu Asp Ile Lys Ala Ser Glu Lys Ala Ala
                85                  90                  95

cgt ttc atc cgc acc tgc gat gcc ttc aac atc cca atc ctt gag ttc     336
Arg Phe Ile Arg Thr Cys Asp Ala Phe Asn Ile Pro Ile Leu Glu Phe
            100                 105                 110

gtg gac gtt cca ggc ttc ctg cct ggc acc aac cag gaa ttc gac ggc     384
Val Asp Val Pro Gly Phe Leu Pro Gly Thr Asn Gln Glu Phe Asp Gly
        115                 120                 125

atc atc cgc cgc ggc gca aag ctg ctt tac gct tac gct gaa gca acc     432
Ile Ile Arg Arg Gly Ala Lys Leu Leu Tyr Ala Tyr Ala Glu Ala Thr
    130                 135                 140

gtc ggc aag atc acc gtc atc acc cgc aag tcc tac ggc gga gcg tac     480
Val Gly Lys Ile Thr Val Ile Thr Arg Lys Ser Tyr Gly Gly Ala Tyr
145                 150                 155                 160

tgc gtg atg ggt tcc aag gat atg ggc gct ggc ctg gta                 519
Cys Val Met Gly Ser Lys Asp Met Gly Ala Gly Leu Val
                165                 170


<210> 210
<211> 173
<212> PRT
<213> Corynebacterium glutamicum

<400> 210
Pro Arg Gln Lys Ala Asp Ile Met Ile Gly Ser Ile Gln Glu Asn Ile
  1               5                  10                  15

Asn Asp Val Asp Leu Glu Leu Asp Thr Ile Ile Pro Asp Ser Pro Asn
             20                  25                  30

Gln Pro Tyr Asp Met Lys Glu Val Ile Ser Arg Ile Val Asp Asp Ala
         35                  40                  45

Glu Phe Phe Glu Ile Gln Glu Asp Tyr Ala Glu Asn Ile Leu Cys Gly
     50                  55                  60

Phe Ala Arg Val Glu Xaa Arg Xaa Val Gly Ile Val Ala Asn Gln Pro
 65                  70                  75                  80

Thr Gln Phe Ala Gly Xaa Leu Asp Ile Lys Ala Ser Glu Lys Ala Ala
                 85                  90                  95

Arg Phe Ile Arg Thr Cys Asp Ala Phe Asn Ile Pro Ile Leu Glu Phe
            100                 105                 110

Val Asp Val Pro Gly Phe Leu Pro Gly Thr Asn Gln Glu Phe Asp Gly
        115                 120                 125

Ile Ile Arg Arg Gly Ala Lys Leu Leu Tyr Ala Tyr Ala Glu Ala Thr
    130                 135                 140

Val Gly Lys Ile Thr Val Ile Thr Arg Lys Ser Tyr Gly Gly Ala Tyr
145                 150                 155                 160

Cys Val Met Gly Ser Lys Asp Met Gly Ala Gly Leu Val
                165                 170

<210> 211
<211> 1752
<212> DNA
<213> Corynebacterium glutamicum

<220>
<221> CDS
<222> (101)..(1729)
<223> RXN02321

<400> 211
tttaaaaact acccgcacgc agcacgaacc tgttcagtga tgtaaatcac cgcggaaata 60

ttgtggacgt tacccccgcc taccgctacg atttcaaaac atg acc att tcc tca 115
                                           Met Thr Ile Ser Ser
                                           1               5

cct ttg att gac gtc gcc aac ctt cca gac atc aac acc act gcc ggc 163
Pro Leu Ile Asp Val Ala Asn Leu Pro Asp Ile Asn Thr Thr Ala Gly
                10                  15                  20

aag atc gcc gac ctt aag gct cgc cgc gcg gaa gcc cat ttc ccc atg 211
Lys Ile Ala Asp Leu Lys Ala Arg Arg Ala Glu Ala His Phe Pro Met
            25                  30                  35

ggt gaa aag gca gta gag aag gtc cac gct gct gga cgc ctc act gcc 259
Gly Glu Lys Ala Val Glu Lys Val His Ala Ala Gly Arg Leu Thr Ala
        40                  45                  50

cgt gag cgc ttg gat tac tta ctc gat gag ggc tcc ttc atc gag acc 307
Arg Glu Arg Leu Asp Tyr Leu Leu Asp Glu Gly Ser Phe Ile Glu Thr
    55                  60                  65

gat cag ctg gct cgc cac cgc acc acc gct ttc tgc ctg ggc gct aag 355
Asp Gln Leu Ala Arg His Arg Thr Thr Ala Phe Cys Leu Gly Ala Lys
70                  75                  80                  85

cgt cct gca acc gac ggt atc gtg acc ggc tgg ggc acc att gat gga 403
Arg Pro Ala Thr Asp Gly Ile Val Thr Gly Trp Gly Thr Ile Asp Gly
                90                  95                  100

cgc gaa gtc tgc atc ttc tcg cag gac ggc acc gta ttc ggt ggc gcg 451
Arg Glu Val Cys Ile Phe Ser Gln Asp Gly Thr Val Phe Gly Gly Ala
            105                 110                 115

ctt ggt gag gtg tac ggc gaa aag atg atc aag atc atg gag ctg gca 499
Leu Gly Glu Val Tyr Gly Glu Lys Met Ile Lys Ile Met Glu Leu Ala
        120                 125                 130

atc gac acc ggc cgc cca ttg atc ggt ctt tac gaa ggc gct ggc gct 547
Ile Asp Thr Gly Arg Pro Leu Ile Gly Leu Tyr Glu Gly Ala Gly Ala
    135                 140                 145

cgt att cag gac ggc gct gtc tcc ctg gac ttc att tcc cag acc ttc 595
Arg Ile Gln Asp Gly Ala Val Ser Leu Asp Phe Ile Ser Gln Thr Phe
150                 155                 160                 165

tac caa aac att cag gct tct ggc gtt atc cca cag atc tcc gtc atc 643
Tyr Gln Asn Ile Gln Ala Ser Gly Val Ile Pro Gln Ile Ser Val Ile
                170                 175                 180

atg ggc gca tgt gca ggt ggc aac gct tac ggc cca gct ctg acc gac 691
Met Gly Ala Cys Ala Gly Gly Asn Ala Tyr Gly Pro Ala Leu Thr Asp
            185                 190                 195

ttc gtg gtc atg gtg gac aag acc tcc aag atg ttc gtt acc ggc cca 739
Phe Val Val Met Val Asp Lys Thr Ser Lys Met Phe Val Thr Gly Pro
        200                 205                 210

gac gtg atc aag acc gtc acc ggc gag gaa atc acc cag gaa gag ctt 787
Asp Val Ile Lys Thr Val Thr Gly Glu Glu Ile Thr Gln Glu Glu Leu
    215                 220                 225

ggc gga gca acc acc cac atg gtg acc gct ggt aac tcc cac tac acc 835
Gly Gly Ala Thr Thr His Met Val Thr Ala Gly Asn Ser His Tyr Thr
230                 235                 240                 245

gct gcg acc gat gag gaa gca ctg gat tgg gta cag gac ctg gtg tcc 883
Ala Ala Thr Asp Glu Glu Ala Leu Asp Trp Val Gln Asp Leu Val Ser
                250                 255                 260

ttc ctc cca tcc aac aat cgc tcc tac gca ccg atg gaa gac ttc gac 931
Phe Leu Pro Ser Asn Asn Arg Ser Tyr Ala Pro Met Glu Asp Phe Asp
            265                 270                 275

gag gaa gaa ggc ggc gtt gaa gaa aac atc acc gct gac gat ctg aag 979
Glu Glu Glu Gly Gly Val Glu Glu Asn Ile Thr Ala Asp Asp Leu Lys
        280                 285                 290

ctc gac gag atc atc cca gat tcc gcg acc gtt cct tac gac gtc cgc 1027
Leu Asp Glu Ile Ile Pro Asp Ser Ala Thr Val Pro Tyr Asp Val Arg
    295                 300                 305

gat gtc atc gaa tgc ctc acc gac gat ggc gaa tac ctg gaa atc cag 1075
Asp Val Ile Glu Cys Leu Thr Asp Asp Gly Glu Tyr Leu Glu Ile Gln
310                 315                 320                 325

gca gac cgc gca gaa aac gtt gtt att gca ttc ggc cgc atc gaa ggc 1123
Ala Asp Arg Ala Glu Asn Val Val Ile Ala Phe Gly Arg Ile Glu Gly
                330                 335                 340

cag tcc gtt ggc ttt gtt gcc aac cag cca acc cag ttc gct ggc tgc 1171
Gln Ser Val Gly Phe Val Ala Asn Gln Pro Thr Gln Phe Ala Gly Cys
            345                 350                 355

ctg gac atc gac tcc tct gag aag gca gct cgc ttc gtc cgc acc tgc 1219
Leu Asp Ile Asp Ser Ser Glu Lys Ala Ala Arg Phe Val Arg Thr Cys
        360                 365                 370

gac gcg ttc aac atc cca atc gtc atg ctt gtc gac gtc ccc ggc ttc 1267
Asp Ala Phe Asn Ile Pro Ile Val Met Leu Val Asp Val Pro Gly Phe
    375                 380                 385

ctc cca ggc gca ggc cag gag tac ggt ggc att ctg cgt cgt ggc gca 1315
Leu Pro Gly Ala Gly Gln Glu Tyr Gly Gly Ile Leu Arg Arg Gly Ala
390                 395                 400                 405

aag ctg ctc tac gca tac ggc gaa gca acc gtt cca aag atc acc gtc 1363

Lys Leu Leu Tyr Ala Tyr Gly Glu Ala Thr Val Pro Lys Ile Thr Val
                410                 415                 420

acc atg cgt aag gct tac ggc gga gcg tac tgc gtg atg ggt tcc aag 1411
Thr Met Arg Lys Ala Tyr Gly Gly Ala Tyr Cys Val Met Gly Ser Lys
            425                 430                 435

ggc ttg ggc tct gac atc aac ctt gca tgg cca acc gca cag atc gcc 1459
Gly Leu Gly Ser Asp Ile Asn Leu Ala Trp Pro Thr Ala Gln Ile Ala
        440                 445                 450

gtc atg ggc gct gct ggc gca gtt gga ttc atc tac cgc aag gag ctc 1507
Val Met Gly Ala Ala Gly Ala Val Gly Phe Ile Tyr Arg Lys Glu Leu
    455                 460                 465

atg gca gct gat gcc aag ggc ctc gat acc gta gct ctg gct aag tcc 1555
Met Ala Ala Asp Ala Lys Gly Leu Asp Thr Val Ala Leu Ala Lys Ser
470                 475                 480                 485

ttc gag cgc gag tat gaa gac cac atg ctc aac ccg tac cac gct gca 1603
Phe Glu Arg Glu Tyr Glu Asp His Met Leu Asn Pro Tyr His Ala Ala
                490                 495                 500

gaa cgt ggc ctg atc gac gcc gtg atc ctg cca agc gaa acc cgc gga 1651
Glu Arg Gly Leu Ile Asp Ala Val Ile Leu Pro Ser Glu Thr Arg Gly
            505                 510                 515

cag att tcc cgc aac ctt cgc ctg ctc aag cac aag aac gtc act cgc 1699
Gln Ile Ser Arg Asn Leu Arg Leu Leu Lys His Lys Asn Val Thr Arg
        520                 525                 530

cct gct cgc aag cac ggc aac atg cca ctg taaatcggcg aatccataaa 1749
Pro Ala Arg Lys His Gly Asn Met Pro Leu
    535                 540

ggt 1752

<210> 212
<211> 543
<212> PRT
<213> Corynebacterium glutamicum

<400> 212
Met Thr Ile Ser Ser Pro Leu Ile Asp Val Ala Asn Leu Pro Asp Ile
1               5                   10                  15

Asn Thr Thr Ala Gly Lys Ile Ala Asp Leu Lys Ala Arg Arg Ala Glu
            20                  25                  30

Ala His Phe Pro Met Gly Glu Lys Ala Val Glu Lys Val His Ala Ala
        35                  40                  45

Gly Arg Leu Thr Ala Arg Glu Arg Leu Asp Tyr Leu Leu Asp Glu Gly
    50                  55                  60

Ser Phe Ile Glu Thr Asp Gln Leu Ala Arg His Arg Thr Thr Ala Phe
65                  70                  75                  80

Cys Leu Gly Ala Lys Arg Pro Ala Thr Asp Gly Ile Val Thr Gly Trp
                85                  90                  95

Gly Thr Ile Asp Gly Arg Glu Val Cys Ile Phe Ser Gln Asp Gly Thr
            100                 105                 110

Val Phe Gly Gly Ala Leu Gly Glu Val Tyr Gly Glu Lys Met Ile Lys
        115                 120                 125

Ile Met Glu Leu Ala Ile Asp Thr Gly Arg Pro Leu Ile Gly Leu Tyr
    130                 135                 140

Glu Gly Ala Gly Ala Arg Ile Gln Asp Gly Ala Val Ser Leu Asp Phe
145                 150                 155                 160

Ile Ser Gln Thr Phe Tyr Gln Asn Ile Gln Ala Ser Gly Val Ile Pro
                165                 170                 175

Gln Ile Ser Val Ile Met Gly Ala Cys Ala Gly Gly Asn Ala Tyr Gly
            180                 185                 190

Pro Ala Leu Thr Asp Phe Val Val Met Val Asp Lys Thr Ser Lys Met
        195                 200                 205

Phe Val Thr Gly Pro Asp Val Ile Lys Thr Val Thr Gly Glu Glu Ile
    210                 215                 220

Thr Gln Glu Glu Leu Gly Gly Ala Thr Thr His Met Val Thr Ala Gly
225                 230                 235                 240

Asn Ser His Tyr Thr Ala Ala Thr Asp Glu Glu Ala Leu Asp Trp Val
                245                 250                 255

Gln Asp Leu Val Ser Phe Leu Pro Ser Asn Asn Arg Ser Tyr Ala Pro
            260                 265                 270

Met Glu Asp Phe Asp Glu Glu Glu Gly Gly Val Glu Glu Asn Ile Thr
        275                 280                 285

Ala Asp Asp Leu Lys Leu Asp Glu Ile Ile Pro Asp Ser Ala Thr Val
    290                 295                 300

Pro Tyr Asp Val Arg Asp Val Ile Glu Cys Leu Thr Asp Asp Gly Glu
305                 310                 315                 320

Tyr Leu Glu Ile Gln Ala Asp Arg Ala Glu Asn Val Val Ile Ala Phe
                325                 330                 335

Gly Arg Ile Glu Gly Gln Ser Val Gly Phe Val Ala Asn Gln Pro Thr
            340                 345                 350

Gln Phe Ala Gly Cys Leu Asp Ile Asp Ser Ser Glu Lys Ala Ala Arg
        355                 360                 365

Phe Val Arg Thr Cys Asp Ala Phe Asn Ile Pro Ile Val Met Leu Val
    370                 375                 380

Asp Val Pro Gly Phe Leu Pro Gly Ala Gly Gln Glu Tyr Gly Gly Ile
385                 390                 395                 400

Leu Arg Arg Gly Ala Lys Leu Leu Tyr Ala Tyr Gly Glu Ala Thr Val
                405                 410                 415

Pro Lys Ile Thr Val Thr Met Arg Lys Ala Tyr Gly Gly Ala Tyr Cys
            420                 425                 430

Val Met Gly Ser Lys Gly Leu Gly Ser Asp Ile Asn Leu Ala Trp Pro
        435                 440                 445

Thr Ala Gln Ile Ala Val Met Gly Ala Ala Gly Ala Val Gly Phe Ile
    450                 455                 460

Tyr Arg Lys Glu Leu Met Ala Ala Asp Ala Lys Gly Leu Asp Thr Val
465                 470                 475                 480

Ala Leu Ala Lys Ser Phe Glu Arg Glu Tyr Glu Asp His Met Leu Asn
                485                 490                 495

Pro Tyr His Ala Ala Glu Arg Gly Leu Ile Asp Ala Val Ile Leu Pro
            500                 505                 510

Ser Glu Thr Arg Gly Gln Ile Ser Arg Asn Leu Arg Leu Leu Lys His
        515                 520                 525

Lys Asn Val Thr Arg Pro Ala Arg Lys His Gly Asn Met Pro Leu
    530                 535                 540

<210> 213
<211> 467
<212> DNA
<213> Corynebacterium glutamicum

<220>
<221> CDS
<222> (1)..(444)
<223> FRXA02321

<400> 213
gag tac ggt ggc att ctg cgt cgt ggc gca aag ctg ctc tac gca tcg      48
Glu Tyr Gly Gly Ile Leu Arg Arg Gly Ala Lys Leu Leu Tyr Ala Ser
  1               5                  10                  15

gnn gaa gca ccg gtt cca aag atc acc gtc acc atg cgt aag gct tac      96
Xaa Glu Ala Pro Val Pro Lys Ile Thr Val Thr Met Arg Lys Ala Tyr
             20                  25                  30

ggc gga gcg tac tgc gtg atg ggt tcc aag ggc ttg ggc tct gac atc     144
Gly Gly Ala Tyr Cys Val Met Gly Ser Lys Gly Leu Gly Ser Asp Ile
         35                  40                  45

aac ctt gca tgg cca acc gca cag atc gcc gtc atg ggc gct gct ggc     192
Asn Leu Ala Trp Pro Thr Ala Gln Ile Ala Val Met Gly Ala Ala Gly
     50                  55                  60

gca gtt gga ttc atc tac cgc aag gag ctc atg gca gct gat gcc aag     240
Ala Val Gly Phe Ile Tyr Arg Lys Glu Leu Met Ala Ala Asp Ala Lys
 65                  70                  75                  80

ggc ctc gat acc gta gct ctg gct aag tcc ttc gag cgc gag tat gaa     288
Gly Leu Asp Thr Val Ala Leu Ala Lys Ser Phe Glu Arg Glu Tyr Glu
                 85                  90                  95

gac cac atg ctc aac ccg tac cac gct gca gaa cgt ggc ctg atc gac     336

Asp His Met Leu Asn Pro Tyr His Ala Ala Glu Arg Gly Leu Ile Asp
            100                 105                 110

gcc gtg atc ctg cca agc gaa acc cgc gga cag att tcc cgc aac ctt    384
Ala Val Ile Leu Pro Ser Glu Thr Arg Gly Gln Ile Ser Arg Asn Leu
        115                 120                 125

cgc ctg ctc aag cac aag aac gtc act cgc cct gct cgc aag cac ggc    432
Arg Leu Leu Lys His Lys Asn Val Thr Arg Pro Ala Arg Lys His Gly
    130                 135                 140

aac atg cca ctg taaatcggcg aatccataaa ggt                          467
Asn Met Pro Leu
145

<210> 214
<211> 148
<212> PRT
<213> Corynebacterium glutamicum

<400> 214
Glu Tyr Gly Gly Ile Leu Arg Arg Gly Ala Lys Leu Leu Tyr Ala Ser
  1               5                  10                  15

Xaa Glu Ala Pro Val Pro Lys Ile Thr Val Thr Met Arg Lys Ala Tyr
             20                  25                  30

Gly Gly Ala Tyr Cys Val Met Gly Ser Lys Gly Leu Gly Ser Asp Ile
         35                  40                  45

Asn Leu Ala Trp Pro Thr Ala Gln Ile Ala Val Met Gly Ala Ala Gly
     50                  55                  60

Ala Val Gly Phe Ile Tyr Arg Lys Glu Leu Met Ala Ala Asp Ala Lys
 65                  70                  75                  80

Gly Leu Asp Thr Val Ala Leu Ala Lys Ser Phe Glu Arg Glu Tyr Glu
                 85                  90                  95

Asp His Met Leu Asn Pro Tyr His Ala Ala Glu Arg Gly Leu Ile Asp
            100                 105                 110

Ala Val Ile Leu Pro Ser Glu Thr Arg Gly Gln Ile Ser Arg Asn Leu
        115                 120                 125

Arg Leu Leu Lys His Lys Asn Val Thr Arg Pro Ala Arg Lys His Gly
    130                 135                 140

Asn Met Pro Leu
145

<210> 215
<211> 514
<212> DNA
<213> Corynebacterium glutamicum

<220>
<221> CDS
<222> (101)..(514)

<223> FRXA02343

<400> 215
tttaaaaact acccgcacgc agcacgaacc tgttcagtga tgtaaatcac cgcggaaata 60

ttgtggacgt tacccccgcc taccgctacg atttcaaaac atg acc att tcc tca 115
Met Thr Ile Ser Ser
1               5

cct ttg att gac gtc gcc aac ctt cca gac atc aac acc act gcc ggc 163
Pro Leu Ile Asp Val Ala Asn Leu Pro Asp Ile Asn Thr Thr Ala Gly
                10                  15                  20

aag atc gcc gac ctt aag gct cgc cgc gcg gaa gcc cat ttc ccc atg 211
Lys Ile Ala Asp Leu Lys Ala Arg Arg Ala Glu Ala His Phe Pro Met
            25                  30                  35

ggt gaa aag gca gta gag aag gtc cac gct gct gga cgc ctc act gcc 259
Gly Glu Lys Ala Val Glu Lys Val His Ala Ala Gly Arg Leu Thr Ala
        40                  45                  50

cgt gag cgc ttg gat tac tta ctc gat gag ggc tcc ttc atc gag acc 307
Arg Glu Arg Leu Asp Tyr Leu Leu Asp Glu Gly Ser Phe Ile Glu Thr
    55                  60                  65

gat cag ctg gct cgc cac cgc acc acc gct ttc tgc ctg ggc gct aag 355
Asp Gln Leu Ala Arg His Arg Thr Thr Ala Phe Cys Leu Gly Ala Lys
70                  75                  80                  85

cgt cct gca acc gac ggc atc gtg acc ggc tgg ggc acc att gat gga 403
Arg Pro Ala Thr Asp Gly Ile Val Thr Gly Trp Gly Thr Ile Asp Gly
                90                  95                  100

cgc gaa gtc tgc atc ttc tcg cag gac ggc acc gta ttc ggt ggc gcg 451
Arg Glu Val Cys Ile Phe Ser Gln Asp Gly Thr Val Phe Gly Gly Ala
            105                 110                 115

ctt ggt gag gtg tac ggc gaa aag atg atc aag atc atg gag ctg gca 499
Leu Gly Glu Val Tyr Gly Glu Lys Met Ile Lys Ile Met Glu Leu Ala
        120                 125                 130

atc gac acc ggc cgc 514
Ile Asp Thr Gly Arg
    135


<210> 216
<211> 138
<212> PRT
<213> Corynebacterium glutamicum

<400> 216
Met Thr Ile Ser Ser Pro Leu Ile Asp Val Ala Asn Leu Pro Asp Ile
1               5                   10                  15

Asn Thr Thr Ala Gly Lys Ile Ala Asp Leu Lys Ala Arg Arg Ala Glu
            20                  25                  30

Ala His Phe Pro Met Gly Glu Lys Ala Val Glu Lys Val His Ala Ala
        35                  40                  45

Gly Arg Leu Thr Ala Arg Glu Arg Leu Asp Tyr Leu Leu Asp Glu Gly
    50                  55                  60

Ser Phe Ile Glu Thr Asp Gln Leu Ala Arg His Arg Thr Thr Ala Phe
65                  70                  75                  80

Cys Leu Gly Ala Lys Arg Pro Ala Thr Asp Gly Ile Val Thr Gly Trp
                85                  90                  95

Gly Thr Ile Asp Gly Arg Glu Val Cys Ile Phe Ser Gln Asp Gly Thr
            100                 105                 110

Val Phe Gly Gly Ala Leu Gly Glu Val Tyr Gly Glu Lys Met Ile Lys
        115                 120                 125

Ile Met Glu Leu Ala Ile Asp Thr Gly Arg
    130                 135

<210> 217
<211> 492
<212> DNA
<213> Corynebacterium glutamicum

<220>
<221> CDS
<222> (1)..(492)
<223> FRXA02850

<400> 217
gaa gag ctt ggc gga gca acc acc cac atg gtg acc gct ggt aac tcc      48
Glu Glu Leu Gly Gly Ala Thr Thr His Met Val Thr Ala Gly Asn Ser
  1               5                  10                  15

cac tac acc gct gcg acc gat gag gaa gca ctg gat tgg gta cag gac      96
His Tyr Thr Ala Ala Thr Asp Glu Glu Ala Leu Asp Trp Val Gln Asp
             20                  25                  30

ctg gtg tcc ttc ctc cca tcc aac aat cgc tcc tac gca ccg atg gaa     144
Leu Val Ser Phe Leu Pro Ser Asn Asn Arg Ser Tyr Ala Pro Met Glu
         35                  40                  45

gac ttc gac gag gaa gaa ggc ggc gtt gaa gaa aac atc acc gct gac     192
Asp Phe Asp Glu Glu Glu Gly Gly Val Glu Glu Asn Ile Thr Ala Asp
     50                  55                  60

gat ctg aag ctc gac gag atc atc cca gat tcc gcg acc gtt cct tac     240
Asp Leu Lys Leu Asp Glu Ile Ile Pro Asp Ser Ala Thr Val Pro Tyr
 65                  70                  75                  80

gac gtc cgc gat gtc atn gaa tgc ctc acc gac gat ggc gaa tac ctg     288
Asp Val Arg Asp Val Xaa Glu Cys Leu Thr Asp Asp Gly Glu Tyr Leu
                 85                  90                  95

gaa atc cag gca gnc cgc gca gaa aac gtt gtt att gca ttc ggc cgc     336
Glu Ile Gln Ala Xaa Arg Ala Glu Asn Val Val Ile Ala Phe Gly Arg
            100                 105                 110

atc gaa ggc cag tcc gtt ggc ttt gtt gcc aac cag cca acc cag ttc     384
Ile Glu Gly Gln Ser Val Gly Phe Val Ala Asn Gln Pro Thr Gln Phe
        115                 120                 125

gct ggc tgc ctg gac atc gac tcc tct gag aag gca gct cgc ttc gtc     432
Ala Gly Cys Leu Asp Ile Asp Ser Ser Glu Lys Ala Ala Arg Phe Val
    130                 135                 140

cgc acc tgc gac gcg ttc aac atc cca atc gtc atg ctt gtg gac gtc     480
Arg Thr Cys Asp Ala Phe Asn Ile Pro Ile Val Met Leu Val Asp Val
145                 150                 155                 160

ccc ggc ttc ctc                                                     492
Pro Gly Phe Leu

<210> 218
<211> 164
<212> PRT
<213> Corynebacterium glutamicum

<400> 218
Glu Glu Leu Gly Gly Ala Thr Thr His Met Val Thr Ala Gly Asn Ser
 1               5                  10                  15

His Tyr Thr Ala Ala Thr Asp Glu Glu Ala Leu Asp Trp Val Gln Asp
            20                  25                  30

Leu Val Ser Phe Leu Pro Ser Asn Asn Arg Ser Tyr Ala Pro Met Glu
        35                  40                  45

Asp Phe Asp Glu Glu Glu Gly Gly Val Glu Glu Asn Ile Thr Ala Asp
    50                  55                  60

Asp Leu Lys Leu Asp Glu Ile Ile Pro Asp Ser Ala Thr Val Pro Tyr
65                  70                  75                  80

Asp Val Arg Asp Val Xaa Glu Cys Leu Thr Asp Asp Gly Glu Tyr Leu
                85                  90                  95

Glu Ile Gln Ala Xaa Arg Ala Glu Asn Val Val Ile Ala Phe Gly Arg
            100                 105                 110

Ile Glu Gly Gln Ser Val Gly Phe Val Ala Asn Gln Pro Thr Gln Phe
        115                 120                 125

Ala Gly Cys Leu Asp Ile Asp Ser Ser Glu Lys Ala Ala Arg Phe Val
    130                 135                 140

Arg Thr Cys Asp Ala Phe Asn Ile Pro Ile Val Met Leu Val Asp Val
145                 150                 155                 160

Pro Gly Phe Leu

<210> 219
<211> 1671
<212> DNA
<213> Corynebacterium glutamicum

<220>
<221> CDS

<222> (101)..(1648)
<223> RXA02583

<400> 219
cagttgtcga tgaaccagaa atcggcacag tcggagctca tttgagtcgc cgcattgatg 60

agatttctcg gaagaattag taacggagag ctgacggaag ttg agt aac acc act 115
Leu Ser Asn Thr Thr
1               5

act gca gag aag cta gcg gat ctg cgc gca cgc ctg gag att gcc aaa 163
Thr Ala Glu Lys Leu Ala Asp Leu Arg Ala Arg Leu Glu Ile Ala Lys
                10                  15                  20

gac cca ggt agt gaa cgc gca cgt aaa aag cgc gac gag gaa ggc cga 211
Asp Pro Gly Ser Glu Arg Ala Arg Lys Lys Arg Asp Glu Glu Gly Arg
            25                  30                  35

acc acc cct cgt cag cgt att gat gct ctg ctt gat gcc gga tcc ttt 259
Thr Thr Pro Arg Gln Arg Ile Asp Ala Leu Leu Asp Ala Gly Ser Phe
        40                  45                  50

gtg gag atc ggc gca cta ggc cgt acc ccg gat gaa ccc gat gcg cct 307
Val Glu Ile Gly Ala Leu Gly Arg Thr Pro Asp Glu Pro Asp Ala Pro
    55                  60                  65

tac tct gac ggt gtg gtg act ggt tat ggt cgc atc gat ggt cgc cca 355
Tyr Ser Asp Gly Val Val Thr Gly Tyr Gly Arg Ile Asp Gly Arg Pro
70                  75                  80                  85

gtg gcc atc tac gcc cat gac aag acc gtt tac ggt ggt tcc gtg ggc 403
Val Ala Ile Tyr Ala His Asp Lys Thr Val Tyr Gly Gly Ser Val Gly
                90                  95                  100

atg act ttc gga cgt aaa gtc agc gaa gtc atg gac atg gct atc cgc 451
Met Thr Phe Gly Arg Lys Val Ser Glu Val Met Asp Met Ala Ile Arg
            105                 110                 115

att ggt tgc cca gtt atc ggt att cag gat tcc ggc gga gcc cgc att 499
Ile Gly Cys Pro Val Ile Gly Ile Gln Asp Ser Gly Gly Ala Arg Ile
        120                 125                 130

cag gat gcg gtg acc tcc ttg gcg atg tac tca gag atc gcg cgt cgt 547
Gln Asp Ala Val Thr Ser Leu Ala Met Tyr Ser Glu Ile Ala Arg Arg
    135                 140                 145

cag ctt ccg ctg tct ggc cgc agc cct cag att tcc atc atg ctg ggt 595
Gln Leu Pro Leu Ser Gly Arg Ser Pro Gln Ile Ser Ile Met Leu Gly
150                 155                 160                 165

aaa tcg gca ggt ggc gca gtg tat gca cct gtg acc act gac ttt gtt 643
Lys Ser Ala Gly Gly Ala Val Tyr Ala Pro Val Thr Thr Asp Phe Val
                170                 175                 180

atc ggc gtt gat ggt gaa aca gaa atg tat gtc acc ggc cca gcc gtg 691
Ile Gly Val Asp Gly Glu Thr Glu Met Tyr Val Thr Gly Pro Ala Val
            185                 190                 195

atc aag gaa gtc acc ggc gag cag atc act tcc gca gac ctc ggt ggc 739
Ile Lys Glu Val Thr Gly Glu Gln Ile Thr Ser Ala Asp Leu Gly Gly
        200                 205                 210

ggt gcg cag cag atg caa aac ggc aac att tcc tat ttg gcg tcc tct 787
Gly Ala Gln Gln Met Gln Asn Gly Asn Ile Ser Tyr Leu Ala Ser Ser
    215                 220                 225

gaa gaa gag gcc ctg aat atg gtc aag gat ttg ctc gac ttc ctg cct 835
Glu Glu Glu Ala Leu Asn Met Val Lys Asp Leu Leu Asp Phe Leu Pro
230                 235                 240                 245

ttg acc tgc aat gat cca gcc cct gtg ttt gca gca cca acg gat gaa 883
Leu Thr Cys Asn Asp Pro Ala Pro Val Phe Ala Ala Pro Thr Asp Glu
                250                 255                 260

gag atc gcc tac gac gaa gct ctg aac tcg ttc atg cct gac gac act 931
Glu Ile Ala Tyr Asp Glu Ala Leu Asn Ser Phe Met Pro Asp Asp Thr
            265                 270                 275

aac cag ggc tac gac atg cat gac ctg ctg gac aag ctt ttc gac gac 979
Asn Gln Gly Tyr Asp Met His Asp Leu Leu Asp Lys Leu Phe Asp Asp
        280                 285                 290

gcc aac ctg ctg gaa atc caa gag gag tac gcc ccc aac ctg atc act 1027
Ala Asn Leu Leu Glu Ile Gln Glu Glu Tyr Ala Pro Asn Leu Ile Thr
    295                 300                 305

acc ttc gcc cgc gtt gat ggc aag gca gtc ggt gtg gtg gcc aac caa 1075
Thr Phe Ala Arg Val Asp Gly Lys Ala Val Gly Val Val Ala Asn Gln
310                 315                 320                 325

cca atg gat aag gca ggc tgc atc gac gct gac gcc gcc gac aag ggc 1123
Pro Met Asp Lys Ala Gly Cys Ile Asp Ala Asp Ala Ala Asp Lys Gly
                330                 335                 340

gcc cgc ttc atc cgt atc tgc gac gcc tac aac atc ccg atc atc ttc 1171
Ala Arg Phe Ile Arg Ile Cys Asp Ala Tyr Asn Ile Pro Ile Ile Phe
            345                 350                 355

gtc gtg gac acc cct ggc tac ctg cct ggc gtg gac caa gag aag gtc 1219
Val Val Asp Thr Pro Gly Tyr Leu Pro Gly Val Asp Gln Glu Lys Val
        360                 365                 370

ggt ttg att cac cgt ggc gca aag cta gcc ttc gca gtg gtg gaa tcg 1267
Gly Leu Ile His Arg Gly Ala Lys Leu Ala Phe Ala Val Val Glu Ser
    375                 380                 385

acc gtc cct aag att tcc ttg atc gtg cgc aag gcc tac ggc gga gca 1315
Thr Val Pro Lys Ile Ser Leu Ile Val Arg Lys Ala Tyr Gly Gly Ala
390                 395                 400                 405

tat gcc gtg atg ggt tcc aag aac ctc acc ggt gac ctc aac ttc gca 1363
Tyr Ala Val Met Gly Ser Lys Asn Leu Thr Gly Asp Leu Asn Phe Ala
                410                 415                 420

tgg cca acc gca cag atc gcc gtg atg ggc gca gcc gca gct gtc gtg 1411
Trp Pro Thr Ala Gln Ile Ala Val Met Gly Ala Ala Ala Ala Val Val
            425                 430                 435

atg atc cag ggc aag cag ctc gaa gcc gcc cca cct gag cag cgt gaa 1459
Met Ile Gln Gly Lys Gln Leu Glu Ala Ala Pro Pro Glu Gln Arg Glu
        440                 445                 450

tac atg aag aaa ctg ttc atg gac ttc tac gat gag aac atg acc agc     1507
Tyr Met Lys Lys Leu Phe Met Asp Phe Tyr Asp Glu Asn Met Thr Ser
    455                 460                 465

cca tat gtg gcc gcc gag cgt ggt tac atc gac gcc atg atc gaa cct     1555
Pro Tyr Val Ala Ala Glu Arg Gly Tyr Ile Asp Ala Met Ile Glu Pro
470                 475                 480                 485

gca gag acc cgt ttg gtg ctt cgc cga gca gtc cgc cag ctg gaa acc     1603
Ala Glu Thr Arg Leu Val Leu Arg Arg Ala Val Arg Gln Leu Glu Thr
                490                 495                 500

aag gct gtg cga gac ctc gac aag aag cac acg atc atg ccg atg         1648
Lys Ala Val Arg Asp Leu Asp Lys Lys His Thr Ile Met Pro Met
            505                 510                 515

taacgtccaa agaattatcc aga                                           1671

<210> 220
<211> 516
<212> PRT
<213> Corynebacterium glutamicum

<400> 220
Leu Ser Asn Thr Thr Thr Ala Glu Lys Leu Ala Asp Leu Arg Ala Arg
1               5                   10                  15

Leu Glu Ile Ala Lys Asp Pro Gly Ser Glu Arg Ala Arg Lys Lys Arg
            20                  25                  30

Asp Glu Glu Gly Arg Thr Thr Pro Arg Gln Arg Ile Asp Ala Leu Leu
        35                  40                  45

Asp Ala Gly Ser Phe Val Glu Ile Gly Ala Leu Gly Arg Thr Pro Asp
    50                  55                  60

Glu Pro Asp Ala Pro Tyr Ser Asp Gly Val Val Thr Gly Tyr Gly Arg
65                  70                  75                  80

Ile Asp Gly Arg Pro Val Ala Ile Tyr Ala His Asp Lys Thr Val Tyr
                85                  90                  95

Gly Gly Ser Val Gly Met Thr Phe Gly Arg Lys Val Ser Glu Val Met
            100                 105                 110

Asp Met Ala Ile Arg Ile Gly Cys Pro Val Ile Gly Ile Gln Asp Ser
        115                 120                 125

Gly Gly Ala Arg Ile Gln Asp Ala Val Thr Ser Leu Ala Met Tyr Ser
    130                 135                 140

Glu Ile Ala Arg Arg Gln Leu Pro Leu Ser Gly Arg Ser Pro Gln Ile
145                 150                 155                 160

Ser Ile Met Leu Gly Lys Ser Ala Gly Gly Ala Val Tyr Ala Pro Val
                165                 170                 175

Thr Thr Asp Phe Val Ile Gly Val Asp Gly Glu Thr Glu Met Tyr Val
            180                 185                 190

Thr Gly Pro Ala Val Ile Lys Glu Val Thr Gly Glu Gln Ile Thr Ser
        195                 200                 205

Ala Asp Leu Gly Gly Gly Ala Gln Gln Met Gln Asn Gly Asn Ile Ser
    210                 215                 220

Tyr Leu Ala Ser Ser Glu Glu Glu Ala Leu Asn Met Val Lys Asp Leu
225                 230                 235                 240

Leu Asp Phe Leu Pro Leu Thr Cys Asn Asp Pro Ala Pro Val Phe Ala
                245                 250                 255

Ala Pro Thr Asp Glu Glu Ile Ala Tyr Asp Glu Ala Leu Asn Ser Phe
            260                 265                 270

Met Pro Asp Asp Thr Asn Gln Gly Tyr Asp Met His Asp Leu Leu Asp
        275                 280                 285

Lys Leu Phe Asp Asp Ala Asn Leu Leu Glu Ile Gln Glu Glu Tyr Ala
    290                 295                 300

Pro Asn Leu Ile Thr Thr Phe Ala Arg Val Asp Gly Lys Ala Val Gly
305                 310                 315                 320

Val Val Ala Asn Gln Pro Met Asp Lys Ala Gly Cys Ile Asp Ala Asp
                325                 330                 335

Ala Ala Asp Lys Gly Ala Arg Phe Ile Arg Ile Cys Asp Ala Tyr Asn
            340                 345                 350

Ile Pro Ile Ile Phe Val Val Asp Thr Pro Gly Tyr Leu Pro Gly Val
        355                 360                 365

Asp Gln Glu Lys Val Gly Leu Ile His Arg Gly Ala Lys Leu Ala Phe
    370                 375                 380

Ala Val Val Glu Ser Thr Val Pro Lys Ile Ser Leu Ile Val Arg Lys
385                 390                 395                 400

Ala Tyr Gly Gly Ala Tyr Ala Val Met Gly Ser Lys Asn Leu Thr Gly
                405                 410                 415

Asp Leu Asn Phe Ala Trp Pro Thr Ala Gln Ile Ala Val Met Gly Ala
            420                 425                 430

Ala Ala Ala Val Val Met Ile Gln Gly Lys Gln Leu Glu Ala Ala Pro
        435                 440                 445

Pro Glu Gln Arg Glu Tyr Met Lys Lys Leu Phe Met Asp Phe Tyr Asp
    450                 455                 460

Glu Asn Met Thr Ser Pro Tyr Val Ala Ala Glu Arg Gly Tyr Ile Asp
465                 470                 475                 480

Ala Met Ile Glu Pro Ala Glu Thr Arg Leu Val Leu Arg Arg Ala Val
                485                 490                 495

Arg Gln Leu Glu Thr Lys Ala Val Arg Asp Leu Asp Lys Lys His Thr
            500                 505                 510

Ile Met Pro Met

        515

<210> 221
<211> 1635
<212> DNA
<213> Corynebacterium glutamicum

<220>
<221> CDS
<222> (101)..(1612)
<223> RXA00870

<400> 221
caagacggcg atgtcgccgc cgctgttgat accgcagcgc gacttgttca cacagatatt 60

caacaattca cttcgcagag catttaagga atttacacac atg tct gaa cca caa 115
Met Ser Glu Pro Gln
1               5

acc atc tcg cac tgg att gac ggc gcg att tcc cca tcc act tcc ggc 163
Thr Ile Ser His Trp Ile Asp Gly Ala Ile Ser Pro Ser Thr Ser Gly
                10                  15                  20

aag acc gct cct gtc tac aat cct gca act ggc cag gtc acc gcc aat 211
Lys Thr Ala Pro Val Tyr Asn Pro Ala Thr Gly Gln Val Thr Ala Asn
            25                  30                  35

gtt gcg ctg gct agc cag gaa gag atc gat gcc acc atc gct tct gcc 259
Val Ala Leu Ala Ser Gln Glu Glu Ile Asp Ala Thr Ile Ala Ser Ala
        40                  45                  50

acc aag gct gct aag acg tgg ggc aac ctg tct atc gct aag cgc caa 307
Thr Lys Ala Ala Lys Thr Trp Gly Asn Leu Ser Ile Ala Lys Arg Gln
    55                  60                  65

gct gtg ctt ttc aac ttc cgt gag ctg ctg aat gct cgc aag ggt gag 355
Ala Val Leu Phe Asn Phe Arg Glu Leu Leu Asn Ala Arg Lys Gly Glu
70                  75                  80                  85

ctg gcg gag atc atc act gca gag cac ggc aag gtc ttg tcc gat gcc 403
Leu Ala Glu Ile Ile Thr Ala Glu His Gly Lys Val Leu Ser Asp Ala
                90                  95                  100

atg ggt gaa atc ctg cgc ggc cag gaa gtc gtg gag ctt gct acc ggt 451
Met Gly Glu Ile Leu Arg Gly Gln Glu Val Val Glu Leu Ala Thr Gly
            105                 110                 115

ttc cca cac ctg ctt aaa ggt gcg ttc aac gag aac gtc tcc acc ggc 499
Phe Pro His Leu Leu Lys Gly Ala Phe Asn Glu Asn Val Ser Thr Gly
        120                 125                 130

att gat gtg tat tcc ttg aag cag cca ctg ggt gtt gtc ggt atc atc 547
Ile Asp Val Tyr Ser Leu Lys Gln Pro Leu Gly Val Val Gly Ile Ile
    135                 140                 145

agc ccg ttc aac ttc cct gcg atg gtg ccg atg tgg ttt ttc cca atc 595
Ser Pro Phe Asn Phe Pro Ala Met Val Pro Met Trp Phe Phe Pro Ile
150                 155                 160                 165

gca atc gct gca ggc aac gca gtt att ttg aag cct tca gag aag gat 643

Ala Ile Ala Ala Gly Asn Ala Val Ile Leu Lys Pro Ser Glu Lys Asp
                170                 175                 180

cct tcg gca gcg ctg tgg atg gct cag atc tgg aag gaa gct ggt ctt      691
Pro Ser Ala Ala Leu Trp Met Ala Gln Ile Trp Lys Glu Ala Gly Leu
            185                 190                 195

cca gac ggc gta ttc aac gtg ctc cag ggc gac aag ctg gct gtt gat      739
Pro Asp Gly Val Phe Asn Val Leu Gln Gly Asp Lys Leu Ala Val Asp
        200                 205                 210

ggt ttg ctg aac agc cct gat gtc tct gcg att tcc ttc gtg ggt tcc      787
Gly Leu Leu Asn Ser Pro Asp Val Ser Ala Ile Ser Phe Val Gly Ser
    215                 220                 225

acc cca atc gca aag tac atc tac gag act tcc gcg aag aac ggc aag      835
Thr Pro Ile Ala Lys Tyr Ile Tyr Glu Thr Ser Ala Lys Asn Gly Lys
230                 235                 240                 245

cgc gtc cag gcg ttg ggc ggc gcg aag aac cac atg ctg gtg ctg cca      883
Arg Val Gln Ala Leu Gly Gly Ala Lys Asn His Met Leu Val Leu Pro
                250                 255                 260

gat gct gat ctg gat ctg gtt gcc gat cag gca atc aac gca ggt tac      931
Asp Ala Asp Leu Asp Leu Val Ala Asp Gln Ala Ile Asn Ala Gly Tyr
            265                 270                 275

ggc gct gcc ggt gag cgt tgc atg gct gtt tct gtg gtc ttg gct att      979
Gly Ala Ala Gly Glu Arg Cys Met Ala Val Ser Val Val Leu Ala Ile
        280                 285                 290

gaa tct gtt gcc gac gag ctc att gag aag atc aag gag cgc atc gac      1027
Glu Ser Val Ala Asp Glu Leu Ile Glu Lys Ile Lys Glu Arg Ile Asp
    295                 300                 305

acc ctg cgc atc ggc aac ggt gcc ggc gac gag cag ggc gag ccg cac      1075
Thr Leu Arg Ile Gly Asn Gly Ala Gly Asp Glu Gln Gly Glu Pro His
310                 315                 320                 325

ctg ggc cca cta atc acc gac gtc cac cgc gac aag gtc gct tct tat      1123
Leu Gly Pro Leu Ile Thr Asp Val His Arg Asp Lys Val Ala Ser Tyr
                330                 335                 340

gtc gac atc gct gag gcc gac ggc gcc aag atc atc gtg gac ggg cgt      1171
Val Asp Ile Ala Glu Ala Asp Gly Ala Lys Ile Ile Val Asp Gly Arg
            345                 350                 355

aac tgc gcc gta gac ggg cac gag gag ggc ttc ttc ttc ggc cct acg      1219
Asn Cys Ala Val Asp Gly His Glu Glu Gly Phe Phe Phe Gly Pro Thr
        360                 365                 370

ctt atc gac gac atc cca ctc acg ttc cgc gcc tac acc gaa gaa atc      1267
Leu Ile Asp Asp Ile Pro Leu Thr Phe Arg Ala Tyr Thr Glu Glu Ile
    375                 380                 385

ttc ggc ccg gtc ctc tct gtc gtt cgt gtc gca tcc ttc gac gag gca      1315
Phe Gly Pro Val Leu Ser Val Val Arg Val Ala Ser Phe Asp Glu Ala
390                 395                 400                 405

att gag ctg atc aac tcc ggt gaa ttc ggc aac gga acc gca atc ttc      1363
Ile Glu Leu Ile Asn Ser Gly Glu Phe Gly Asn Gly Thr Ala Ile Phe

                410                 415                 420

acc aac gat ggt gga gcg gca cgc cgc ttc cag cat gag atc gaa gtg 1411
Thr Asn Asp Gly Gly Ala Ala Arg Arg Phe Gln His Glu Ile Glu Val
            425                 430                 435

ggc atg atc ggc atc aac gta cca atc cca gtg cct gtt gcg tac cac 1459
Gly Met Ile Gly Ile Asn Val Pro Ile Pro Val Pro Val Ala Tyr His
        440                 445                 450

tcc ttc ggt ggt tgg aag aac tcc ctc ttc ggt gac gcc aag gca tat 1507
Ser Phe Gly Gly Trp Lys Asn Ser Leu Phe Gly Asp Ala Lys Ala Tyr
    455                 460                 465

ggc act caa ggt ttt gat ttc ttc acc agg gaa aag gcg atc acc agc 1555
Gly Thr Gln Gly Phe Asp Phe Phe Thr Arg Glu Lys Ala Ile Thr Ser
470                 475                 480                 485

cgt tgg ctc gac cca gca acc cac ggt ggc att aac ctc ggt ttc cca 1603
Arg Trp Leu Asp Pro Ala Thr His Gly Gly Ile Asn Leu Gly Phe Pro
                490                 495                 500

cag aac gat taattgaagg agagcacagg act 1635
Gln Asn Asp

<210> 222
<211> 504
<212> PRT
<213> Corynebacterium glutamicum

<400> 222
Met Ser Glu Pro Gln Thr Ile Ser His Trp Ile Asp Gly Ala Ile Ser
1               5                   10                  15

Pro Ser Thr Ser Gly Lys Thr Ala Pro Val Tyr Asn Pro Ala Thr Gly
            20                  25                  30

Gln Val Thr Ala Asn Val Ala Leu Ala Ser Gln Glu Glu Ile Asp Ala
        35                  40                  45

Thr Ile Ala Ser Ala Thr Lys Ala Ala Lys Thr Trp Gly Asn Leu Ser
    50                  55                  60

Ile Ala Lys Arg Gln Ala Val Leu Phe Asn Phe Arg Glu Leu Leu Asn
65                  70                  75                  80

Ala Arg Lys Gly Glu Leu Ala Glu Ile Ile Thr Ala Glu His Gly Lys
                85                  90                  95

Val Leu Ser Asp Ala Met Gly Glu Ile Leu Arg Gly Gln Glu Val Val
            100                 105                 110

Glu Leu Ala Thr Gly Phe Pro His Leu Leu Lys Gly Ala Phe Asn Glu
        115                 120                 125

Asn Val Ser Thr Gly Ile Asp Val Tyr Ser Leu Lys Gln Pro Leu Gly
    130                 135                 140

Val Val Gly Ile Ile Ser Pro Phe Asn Phe Pro Ala Met Val Pro Met

145                 150                 155                 160

Trp Phe Phe Pro Ile Ala Ile Ala Ala Gly Asn Ala Val Ile Leu Lys
                165                 170                 175

Pro Ser Glu Lys Asp Pro Ser Ala Ala Leu Trp Met Ala Gln Ile Trp
            180                 185                 190

Lys Glu Ala Gly Leu Pro Asp Gly Val Phe Asn Val Leu Gln Gly Asp
        195                 200                 205

Lys Leu Ala Val Asp Gly Leu Leu Asn Ser Pro Asp Val Ser Ala Ile
    210                 215                 220

Ser Phe Val Gly Ser Thr Pro Ile Ala Lys Tyr Ile Tyr Glu Thr Ser
225                 230                 235                 240

Ala Lys Asn Gly Lys Arg Val Gln Ala Leu Gly Gly Ala Lys Asn His
                245                 250                 255

Met Leu Val Leu Pro Asp Ala Asp Leu Asp Leu Val Ala Asp Gln Ala
            260                 265                 270

Ile Asn Ala Gly Tyr Gly Ala Ala Gly Glu Arg Cys Met Ala Val Ser
        275                 280                 285

Val Val Leu Ala Ile Glu Ser Val Ala Asp Glu Leu Ile Glu Lys Ile
    290                 295                 300

Lys Glu Arg Ile Asp Thr Leu Arg Ile Gly Asn Gly Ala Gly Asp Glu
305                 310                 315                 320

Gln Gly Glu Pro His Leu Gly Pro Leu Ile Thr Asp Val His Arg Asp
                325                 330                 335

Lys Val Ala Ser Tyr Val Asp Ile Ala Glu Ala Asp Gly Ala Lys Ile
            340                 345                 350

Ile Val Asp Gly Arg Asn Cys Ala Val Asp Gly His Glu Glu Gly Phe
        355                 360                 365

Phe Phe Gly Pro Thr Leu Ile Asp Asp Ile Pro Leu Thr Phe Arg Ala
    370                 375                 380

Tyr Thr Glu Glu Ile Phe Gly Pro Val Leu Ser Val Val Arg Val Ala
385                 390                 395                 400

Ser Phe Asp Glu Ala Ile Glu Leu Ile Asn Ser Gly Glu Phe Gly Asn
                405                 410                 415

Gly Thr Ala Ile Phe Thr Asn Asp Gly Gly Ala Ala Arg Arg Phe Gln
            420                 425                 430

His Glu Ile Glu Val Gly Met Ile Gly Ile Asn Val Pro Ile Pro Val
        435                 440                 445

Pro Val Ala Tyr His Ser Phe Gly Gly Trp Lys Asn Ser Leu Phe Gly
    450                 455                 460

Asp Ala Lys Ala Tyr Gly Thr Gln Gly Phe Asp Phe Phe Thr Arg Glu
465                 470                 475                 480

Lys Ala Ile Thr Ser Arg Trp Leu Asp Pro Ala Thr His Gly Gly Ile
                485                 490                 495

Asn Leu Gly Phe Pro Gln Asn Asp
            500

<210> 223
<211> 1305
<212> DNA
<213> Corynebacterium glutamicum

<220>
<221> CDS
<222> (101)..(1282)
<223> RXA01260

<400> 223
ctaaacgtgg gctgcattcc ttccaaagtc tctgatcaaa aacgctgaag ttgcccatac 60

ctttacccat gagaagaaga ccttcggcat caatggcgaa gtg acc ttc aac tat   115
                                           Val Thr Phe Asn Tyr
                                           1               5

gag gat gct cac aag cgt tcc cgt ggc gtt tcc gac aag atc gtt gga   163
Glu Asp Ala His Lys Arg Ser Arg Gly Val Ser Asp Lys Ile Val Gly
                10                  15                  20

ggc gtt cat tac ttg atg aag aag aac aag atc atc gaa att cat ggt   211
Gly Val His Tyr Leu Met Lys Lys Asn Lys Ile Ile Glu Ile His Gly
            25                  30                  35

ctt gga aac ttc aag gat gct aag act ctt gag gtc acc gac ggt aag   259
Leu Gly Asn Phe Lys Asp Ala Lys Thr Leu Glu Val Thr Asp Gly Lys
        40                  45                  50

gat gct ggc aag acc atc acc ttt gat gac tgc atc atc gca acc ggt   307
Asp Ala Gly Lys Thr Ile Thr Phe Asp Asp Cys Ile Ile Ala Thr Gly
    55                  60                  65

tcg gta gtc aac acc ctc cgt ggc gtt gac ttc tca gag aac gtt gtg   355
Ser Val Val Asn Thr Leu Arg Gly Val Asp Phe Ser Glu Asn Val Val
70                  75                  80                  85

tct ttt gaa gag cag att ctt aac cct gtt gcg cca aag aag atg gtc   403
Ser Phe Glu Glu Gln Ile Leu Asn Pro Val Ala Pro Lys Lys Met Val
                90                  95                  100

att gtt ggt gca ggc gca att gga atg gaa ttc gcc tac gtt ctt ggt   451
Ile Val Gly Ala Gly Ala Ile Gly Met Glu Phe Ala Tyr Val Leu Gly
            105                 110                 115

aac tac ggt gta gat gta acc gtc atc gag ttc atg gat cgt gtg ctt   499
Asn Tyr Gly Val Asp Val Thr Val Ile Glu Phe Met Asp Arg Val Leu
        120                 125                 130

cca aat gaa gat gct gaa gtc tcc aag gtt att gca aag gcc tac aag   547
Pro Asn Glu Asp Ala Glu Val Ser Lys Val Ile Ala Lys Ala Tyr Lys
    135                 140                 145

aag atg ggc gtt aag ctt ctt cct ggc cat gca acc act gct gtt cgg 595
Lys Met Gly Val Lys Leu Leu Pro Gly His Ala Thr Thr Ala Val Arg
150                 155                 160                 165

gac aac ggt gac ttt gtc gag gtt gat tac cag aag aag ggc tct gac 643
Asp Asn Gly Asp Phe Val Glu Val Asp Tyr Gln Lys Lys Gly Ser Asp
                170                 175                 180

aag aca gag act ctt act gtt gat cga gtc atg gtt tcc gtt ggt ttc 691
Lys Thr Glu Thr Leu Thr Val Asp Arg Val Met Val Ser Val Gly Phe
            185                 190                 195

cgt cca cgc gtt gag gga ttt ggt ctt gaa aac act ggc gtt aag ctc 739
Arg Pro Arg Val Glu Gly Phe Gly Leu Glu Asn Thr Gly Val Lys Leu
        200                 205                 210

acc gag cgt ggc gca atc gag atc gat gat tac atg cgt acc aac gtc 787
Thr Glu Arg Gly Ala Ile Glu Ile Asp Asp Tyr Met Arg Thr Asn Val
    215                 220                 225

gat ggc att tac gcc atc ggt gac gtg acc gcc aag ctt cag ctt gct 835
Asp Gly Ile Tyr Ala Ile Gly Asp Val Thr Ala Lys Leu Gln Leu Ala
230                 235                 240                 245

cac gtc gca gaa gca cag ggc att gtt gcc gca gag act att gct ggt 883
His Val Ala Glu Ala Gln Gly Ile Val Ala Ala Glu Thr Ile Ala Gly
                250                 255                 260

gca gaa act cag act ctt ggt gat tac atg atg atg cca cgt gca acc 931
Ala Glu Thr Gln Thr Leu Gly Asp Tyr Met Met Met Pro Arg Ala Thr
            265                 270                 275

ttc tgc aac cca cag gtt tct tcc ttt ggt tac acc gaa gag cag gcc 979
Phe Cys Asn Pro Gln Val Ser Ser Phe Gly Tyr Thr Glu Glu Gln Ala
        280                 285                 290

aag gag aag tgg cca gat cgt gag atc aag gtt gct tcc ttc cca ttc 1027
Lys Glu Lys Trp Pro Asp Arg Glu Ile Lys Val Ala Ser Phe Pro Phe
    295                 300                 305

tct gca aac ggt aaa gca gtt ggc ctg gca gaa act gat ggt ttc gca 1075
Ser Ala Asn Gly Lys Ala Val Gly Leu Ala Glu Thr Asp Gly Phe Ala
310                 315                 320                 325

aag atc gtt gct gat gca gaa ttc ggt gag ctg ctc ggt gca cac ctg 1123
Lys Ile Val Ala Asp Ala Glu Phe Gly Glu Leu Leu Gly Ala His Leu
                330                 335                 340

gtt gga gca aat gca tca gag ctc atc aat gaa ttg gtg ctt gct cag 1171
Val Gly Ala Asn Ala Ser Glu Leu Ile Asn Glu Leu Val Leu Ala Gln
            345                 350                 355

aac tgg gat ctc acc act gaa gag atc tct cgt agc gtc cat att cac 1219
Asn Trp Asp Leu Thr Thr Glu Glu Ile Ser Arg Ser Val His Ile His
        360                 365                 370

cca acg cta tct gag gca gtt aag gaa gct gca cac ggt atc tct gga 1267
Pro Thr Leu Ser Glu Ala Val Lys Glu Ala Ala His Gly Ile Ser Gly
    375                 380                 385

cac atg atc aac ttc tagaatccac ctcgttggcc ctg 1305

His Met Ile Asn Phe
390

<210> 224
<211> 394
<212> PRT
<213> Corynebacterium glutamicum

<400> 224
Val Thr Phe Asn Tyr Glu Asp Ala His Lys Arg Ser Arg Gly Val Ser
1               5                   10                  15

Asp Lys Ile Val Gly Gly Val His Tyr Leu Met Lys Lys Asn Lys Ile
            20                  25                  30

Ile Glu Ile His Gly Leu Gly Asn Phe Lys Asp Ala Lys Thr Leu Glu
        35                  40                  45

Val Thr Asp Gly Lys Asp Ala Gly Lys Thr Ile Thr Phe Asp Asp Cys
    50                  55                  60

Ile Ile Ala Thr Gly Ser Val Val Asn Thr Leu Arg Gly Val Asp Phe
65                  70                  75                  80

Ser Glu Asn Val Val Ser Phe Glu Glu Gln Ile Leu Asn Pro Val Ala
                85                  90                  95

Pro Lys Lys Met Val Ile Val Gly Ala Gly Ala Ile Gly Met Glu Phe
            100                 105                 110

Ala Tyr Val Leu Gly Asn Tyr Gly Val Asp Val Thr Val Ile Glu Phe
        115                 120                 125

Met Asp Arg Val Leu Pro Asn Glu Asp Ala Glu Val Ser Lys Val Ile
    130                 135                 140

Ala Lys Ala Tyr Lys Lys Met Gly Val Lys Leu Leu Pro Gly His Ala
145                 150                 155                 160

Thr Thr Ala Val Arg Asp Asn Gly Asp Phe Val Glu Val Asp Tyr Gln
                165                 170                 175

Lys Lys Gly Ser Asp Lys Thr Glu Thr Leu Thr Val Asp Arg Val Met
            180                 185                 190

Val Ser Val Gly Phe Arg Pro Arg Val Glu Gly Phe Gly Leu Glu Asn
        195                 200                 205

Thr Gly Val Lys Leu Thr Glu Arg Gly Ala Ile Glu Ile Asp Asp Tyr
    210                 215                 220

Met Arg Thr Asn Val Asp Gly Ile Tyr Ala Ile Gly Asp Val Thr Ala
225                 230                 235                 240

Lys Leu Gln Leu Ala His Val Ala Glu Ala Gln Gly Ile Val Ala Ala
                245                 250                 255

Glu Thr Ile Ala Gly Ala Glu Thr Gln Thr Leu Gly Asp Tyr Met Met
            260                 265                 270

Met Pro Arg Ala Thr Phe Cys Asn Pro Gln Val Ser Ser Phe Gly Tyr
        275                 280                 285

Thr Glu Glu Gln Ala Lys Glu Lys Trp Pro Asp Arg Glu Ile Lys Val
    290                 295                 300

Ala Ser Phe Pro Phe Ser Ala Asn Gly Lys Ala Val Gly Leu Ala Glu
305                 310                 315                 320

Thr Asp Gly Phe Ala Lys Ile Val Ala Asp Ala Glu Phe Gly Glu Leu
                325                 330                 335

Leu Gly Ala His Leu Val Gly Ala Asn Ala Ser Glu Leu Ile Asn Glu
            340                 345                 350

Leu Val Leu Ala Gln Asn Trp Asp Leu Thr Thr Glu Glu Ile Ser Arg
        355                 360                 365

Ser Val His Ile His Pro Thr Leu Ser Glu Ala Val Lys Glu Ala Ala
    370                 375                 380

His Gly Ile Ser Gly His Met Ile Asn Phe
385                 390

<210> 225
<211> 294
<212> DNA
<213> Corynebacterium glutamicum

<220>
<221> CDS
<222> (101)..(271)
<223> RXA01261

<400> 225
gtgggtgttt ttcattttct tccactctaa aattaagtat ggaaaaccaa ccgcacccgg 60

atgcacgaca atgacccact aaacacgtat ccttgaatgc gtg act gaa cat tat 115
Val Thr Glu His Tyr
1               5

gac gta gta gta ctc gga gcc ggc ccc ggt ggc tat gtc tcc gcc atc 163
Asp Val Val Val Leu Gly Ala Gly Pro Gly Gly Tyr Val Ser Ala Ile
                10                  15                  20

cgt gca gcg cag ctt ggc aag aag gtt gct gta att gag aag cag tac 211
Arg Ala Ala Gln Leu Gly Lys Lys Val Ala Val Ile Glu Lys Gln Tyr
            25                  30                  35

tgg ggt ggt gtt tgc cta aac gtg ggc tgc att cct tcc aaa gtc tct 259
Trp Gly Gly Val Cys Leu Asn Val Gly Cys Ile Pro Ser Lys Val Ser
        40                  45                  50

gat caa aaa cgc tgaagttgcc catacctta ccc 294
Asp Gln Lys Arg
    55

<210> 226
<211> 57

<212> PRT
<213> Corynebacterium glutamicum

<400> 226
Val Thr Glu His Tyr Asp Val Val Val Leu Gly Ala Gly Pro Gly Gly
1               5                   10                  15

Tyr Val Ser Ala Ile Arg Ala Ala Gln Leu Gly Lys Lys Val Ala Val
            20                  25                  30

Ile Glu Lys Gln Tyr Trp Gly Gly Val Cys Leu Asn Val Gly Cys Ile
        35                  40                  45

Pro Ser Lys Val Ser Asp Gln Lys Arg
    50                  55


<210> 227
<211> 555
<212> DNA
<213> Corynebacterium glutamicum

<220>
<221> CDS
<222> (101)..(532)
<223> RXA01136

<400> 227
ccctcgcaac accgacggta gcctcttgga aatggcgcgc atcgtgcgtg agctttcccg 60

ctaggacctg tctactgcct tcactttgtg cgaacaccgc atg acc ttg gat tac   115
                                           Met Thr Leu Asp Tyr
                                           1               5

ttc aag gca tcc ggc act gac tat gct ttg gga ttg gct gca gag tcg   163
Phe Lys Ala Ser Gly Thr Asp Tyr Ala Leu Gly Leu Ala Ala Glu Ser
                10                  15                  20

gaa ggg gca cga cgc act ggt atc acc ggc atg gcg agt gca ttc aag   211
Glu Gly Ala Arg Arg Thr Gly Ile Thr Gly Met Ala Ser Ala Phe Lys
            25                  30                  35

gag ttt gct ggt tgt ggt gag atc gac ctt gaa gca acc agg gta gaa   259
Glu Phe Ala Gly Cys Gly Glu Ile Asp Leu Glu Ala Thr Arg Val Glu
        40                  45                  50

ggt ggc ctc aaa gtt agt gga aag ctt cgt tgg gct tcc aac ttg tgc   307
Gly Gly Leu Lys Val Ser Gly Lys Leu Arg Trp Ala Ser Asn Leu Cys
    55                  60                  65

gaa gat cca gtg att gtg cct gct gca aag acc gca gag ggc tta caa   355
Glu Asp Pro Val Ile Val Pro Ala Ala Lys Thr Ala Glu Gly Leu Gln
70                  75                  80                  85

cta ctg ttc gca ttg ggc gca gaa acc gaa ggt gtc acc ctc ggt tct   403
Leu Leu Phe Ala Leu Gly Ala Glu Thr Glu Gly Val Thr Leu Gly Ser
                90                  95                  100

tca ctt gct cta ctc ggt ttg aac gca act gct tgc gct tgg gtg agc   451
Ser Leu Ala Leu Leu Gly Leu Asn Ala Thr Ala Cys Ala Trp Val Ser
            105                 110                 115

ttt gag gat gtc ttc att cct ggg gct cag att cta agc cac gat ttc 499
Phe Glu Asp Val Phe Ile Pro Gly Ala Gln Ile Leu Ser His Asp Phe
        120                 125                 130

ctt acc ttg tgg cat cgg tgc gcc caa cct tcg tgatcctacg gatctccgaa 552
Leu Thr Leu Trp His Arg Cys Ala Gln Pro Ser
    135                 140

tac 555

<210> 228
<211> 144
<212> PRT
<213> Corynebacterium glutamicum

<400> 228
Met Thr Leu Asp Tyr Phe Lys Ala Ser Gly Thr Asp Tyr Ala Leu Gly
1               5                   10                  15

Leu Ala Ala Glu Ser Glu Gly Ala Arg Arg Thr Gly Ile Thr Gly Met
            20                  25                  30

Ala Ser Ala Phe Lys Glu Phe Ala Gly Cys Gly Glu Ile Asp Leu Glu
        35                  40                  45

Ala Thr Arg Val Glu Gly Gly Leu Lys Val Ser Gly Lys Leu Arg Trp
    50                  55                  60

Ala Ser Asn Leu Cys Glu Asp Pro Val Ile Val Pro Ala Ala Lys Thr
65                  70                  75                  80

Ala Glu Gly Leu Gln Leu Leu Phe Ala Leu Gly Ala Glu Thr Glu Gly
                85                  90                  95

Val Thr Leu Gly Ser Ser Leu Ala Leu Leu Gly Leu Asn Ala Thr Ala
            100                 105                 110

Cys Ala Trp Val Ser Phe Glu Asp Val Phe Ile Pro Gly Ala Gln Ile
        115                 120                 125

Leu Ser His Asp Phe Leu Thr Leu Trp His Arg Cys Ala Gln Pro Ser
    130                 135                 140

<210> 229
<211> 1140
<212> DNA
<213> Corynebacterium glutamicum

<220>
<221> CDS
<222> (101)..(1117)
<223> RXN00559

<400> 229
cccttcaatc cagtctttga cggccaatac ggcttgccgg gtttccagcg gatcaatcct 60

catgaagcat cagcctagta cgaaccgtta aagtgtccat atg tct gat aat ccg 115
Met Ser Asp Asn Pro
1               5

cat gag aat ccc cgt gag aat cca cac cgc tcc cca gaa gtc gtc ctt 163
His Glu Asn Pro Arg Glu Asn Pro His Arg Ser Pro Glu Val Val Leu
                10                  15                  20

cgt ttc atg gct gcc cct act gac gtt ttg atg gct ggt agc cat ggc 211
Arg Phe Met Ala Ala Pro Thr Asp Val Leu Met Ala Gly Ser His Gly
            25                  30                  35

gtt ggc ggt ggc cga gtc ctg gaa tgg atc gat aag gct gct tat gct 259
Val Gly Gly Gly Arg Val Leu Glu Trp Ile Asp Lys Ala Ala Tyr Ala
        40                  45                  50

tgt gct acc cag tgg tct gga acc tac tgt gtc act gct tat gtt ggt 307
Cys Ala Thr Gln Trp Ser Gly Thr Tyr Cys Val Thr Ala Tyr Val Gly
    55                  60                  65

cac att cac ttc act cgc cct att ccc tct ggc cac atg gtc gag gtg 355
His Ile His Phe Thr Arg Pro Ile Pro Ser Gly His Met Val Glu Val
70                  75                  80                  85

cgt tcc cgc att gcg atg act ggc cgt tcc tcc atg cac atc gtg aat 403
Arg Ser Arg Ile Ala Met Thr Gly Arg Ser Ser Met His Ile Val Asn
                90                  95                  100

gag gtg ctt tct gcg gat cct cgc gat ggc aac tac acc cgt gcg tgt 451
Glu Val Leu Ser Ala Asp Pro Arg Asp Gly Asn Tyr Thr Arg Ala Cys
            105                 110                 115

gac tgc ttg gtt att ttc gtg gcg aag gac acc gca act ggt cgc gct 499
Asp Cys Leu Val Ile Phe Val Ala Lys Asp Thr Ala Thr Gly Arg Ala
        120                 125                 130

acc cca gtt cct tca ttt acc cct aag aat gaa gaa gag cag cgc gtg 547
Thr Pro Val Pro Ser Phe Thr Pro Lys Asn Glu Glu Glu Gln Arg Val
    135                 140                 145

ttg gaa gct gct aac tcc cgc atc ggg ctg cgc aag gct att gaa gcg 595
Leu Glu Ala Ala Asn Ser Arg Ile Gly Leu Arg Lys Ala Ile Glu Ala
150                 155                 160                 165

gag atg gaa aag cag acg tac aac gga cct tct gag gcc cct cgt ttg 643
Glu Met Glu Lys Gln Thr Tyr Asn Gly Pro Ser Glu Ala Pro Arg Leu
                170                 175                 180

att acc cgc ttc ttg gct aag cca aca gat atc aac tgg ggt ggc aag 691
Ile Thr Arg Phe Leu Ala Lys Pro Thr Asp Ile Asn Trp Gly Gly Lys
            185                 190                 195

gtc cac ggt ggc act gcc atg gaa tgg atc gat gag gcg ggt gct gcg 739
Val His Gly Gly Thr Ala Met Glu Trp Ile Asp Glu Ala Gly Ala Ala
        200                 205                 210

tgc acc atg gag tgg tct ggt aac cac acc gtt gcg gtt tat gct ggt 787
Cys Thr Met Glu Trp Ser Gly Asn His Thr Val Ala Val Tyr Ala Gly
    215                 220                 225

ggt atc cgc ttc tac cag ccc att cag atc ggt gac ctc att gag gtg 835
Gly Ile Arg Phe Tyr Gln Pro Ile Gln Ile Gly Asp Leu Ile Glu Val
230                 235                 240                 245

gac gcc cgc atg atg cgt acc gat aag cgt tcc atg cag atg tcc atc 883
Asp Ala Arg Met Met Arg Thr Asp Lys Arg Ser Met Gln Met Ser Ile
                250                 255                 260

cac gtc cgt gcc ggt gat gct cac cgt ggc cgt gct gag cta gaa acc 931
His Val Arg Ala Gly Asp Ala His Arg Gly Arg Ala Glu Leu Glu Thr
            265                 270                 275

gct att cac gca acg gtg acc tac tta gga att gat gtc gac gga gag 979
Ala Ile His Ala Thr Val Thr Tyr Leu Gly Ile Asp Val Asp Gly Glu
        280                 285                 290

cct ttg cct gca cca cag ttt gtg cct cgt acc cct gag gat atc cag 1027
Pro Leu Pro Ala Pro Gln Phe Val Pro Arg Thr Pro Glu Asp Ile Gln
    295                 300                 305

ttg gct gag cat gca aac atc ctg agg gat ctg cgt gct gat tac acc 1075
Leu Ala Glu His Ala Asn Ile Leu Arg Asp Leu Arg Ala Asp Tyr Thr
310                 315                 320                 325

cca atg ccg ctg ttc cag cgc agg gtt cca ctg cag atc gac 1117
Pro Met Pro Leu Phe Gln Arg Arg Val Pro Leu Gln Ile Asp
                330                 335

tagttagacc cgaaaaagcc ccc 1140

<210> 230
<211> 339
<212> PRT
<213> Corynebacterium glutamicum

<400> 230
Met Ser Asp Asn Pro His Glu Asn Pro Arg Glu Asn Pro His Arg Ser
1               5                   10                  15

Pro Glu Val Val Leu Arg Phe Met Ala Ala Pro Thr Asp Val Leu Met
            20                  25                  30

Ala Gly Ser His Gly Val Gly Gly Gly Arg Val Leu Glu Trp Ile Asp
        35                  40                  45

Lys Ala Ala Tyr Ala Cys Ala Thr Gln Trp Ser Gly Thr Tyr Cys Val
    50                  55                  60

Thr Ala Tyr Val Gly His Ile His Phe Thr Arg Pro Ile Pro Ser Gly
65                  70                  75                  80

His Met Val Glu Val Arg Ser Arg Ile Ala Met Thr Gly Arg Ser Ser
                85                  90                  95

Met His Ile Val Asn Glu Val Leu Ser Ala Asp Pro Arg Asp Gly Asn
            100                 105                 110

Tyr Thr Arg Ala Cys Asp Cys Leu Val Ile Phe Val Ala Lys Asp Thr
        115                 120                 125

Ala Thr Gly Arg Ala Thr Pro Val Pro Ser Phe Thr Pro Lys Asn Glu
    130                 135                 140

Glu Glu Gln Arg Val Leu Glu Ala Ala Asn Ser Arg Ile Gly Leu Arg
145                 150                 155                 160

Lys Ala Ile Glu Ala Glu Met Glu Lys Gln Thr Tyr Asn Gly Pro Ser
                165                 170                 175

Glu Ala Pro Arg Leu Ile Thr Arg Phe Leu Ala Lys Pro Thr Asp Ile
            180                 185                 190

Asn Trp Gly Gly Lys Val His Gly Gly Thr Ala Met Glu Trp Ile Asp
        195                 200                 205

Glu Ala Gly Ala Ala Cys Thr Met Glu Trp Ser Gly Asn His Thr Val
    210                 215                 220

Ala Val Tyr Ala Gly Gly Ile Arg Phe Tyr Gln Pro Ile Gln Ile Gly
225                 230                 235                 240

Asp Leu Ile Glu Val Asp Ala Arg Met Met Arg Thr Asp Lys Arg Ser
                245                 250                 255

Met Gln Met Ser Ile His Val Arg Ala Gly Asp Ala His Arg Gly Arg
            260                 265                 270

Ala Glu Leu Glu Thr Ala Ile His Ala Thr Val Thr Tyr Leu Gly Ile
        275                 280                 285

Asp Val Asp Gly Glu Pro Leu Pro Ala Pro Gln Phe Val Pro Arg Thr
    290                 295                 300

Pro Glu Asp Ile Gln Leu Ala Glu His Ala Asn Ile Leu Arg Asp Leu
305                 310                 315                 320

Arg Ala Asp Tyr Thr Pro Met Pro Leu Phe Gln Arg Arg Val Pro Leu
                325                 330                 335

Gln Ile Asp

<210> 231
<211> 313
<212> DNA
<213> Corynebacterium glutamicum

<220>
<221> CDS
<222> (101)..(313)
<223> FRXA00559

<400> 231
cccttcaatc cagtctttga cggccaatac ggcttgccgg gtttccagcg gatcaatcct 60

catgaagcat cagcctagta cgaaccgtta aagtgtccat atg tct gat aat ccg 115
                                           Met Ser Asp Asn Pro
                                           1               5

cat gag aat ccc cgt gag aat cca cac cgc tcc cca gaa gtc gtc ctt 163

His Glu Asn Pro Arg Glu Asn Pro His Arg Ser Pro Glu Val Val Leu
                10                  15                  20

cgt ttc atg gct gcc cct act gac gtt ttg atg gct ggt agc cat ggc     211
Arg Phe Met Ala Ala Pro Thr Asp Val Leu Met Ala Gly Ser His Gly
            25                  30                  35

gtt ggc ggt ggc cga gtc ctg gaa tgg atc gat aag gct gct tat gct     259
Val Gly Gly Gly Arg Val Leu Glu Trp Ile Asp Lys Ala Ala Tyr Ala
        40                  45                  50

tgt gct acc cac ggg tct gga acc tac tgc gtc act gct tat gtt ggt     307
Cys Ala Thr His Gly Ser Gly Thr Tyr Cys Val Thr Ala Tyr Val Gly
    55                  60                  65

cac att                                                             313
His Ile
70


<210> 232
<211> 71
<212> PRT
<213> Corynebacterium glutamicum

<400> 232
Met Ser Asp Asn Pro His Glu Asn Pro Arg Glu Asn Pro His Arg Ser
1               5                   10                  15

Pro Glu Val Val Leu Arg Phe Met Ala Ala Pro Thr Asp Val Leu Met
            20                  25                  30

Ala Gly Ser His Gly Val Gly Gly Gly Arg Val Leu Glu Trp Ile Asp
        35                  40                  45

Lys Ala Ala Tyr Ala Cys Ala Thr His Gly Ser Gly Thr Tyr Cys Val
    50                  55                  60

Thr Ala Tyr Val Gly His Ile
65                  70


<210> 233
<211> 802
<212> DNA
<213> Corynebacterium glutamicum

<220>
<221> CDS
<222> (101)..(802)
<223> RXA01580

<400> 233
cggtaaacgc ctcattaaag tccaatgcca tgctcataac actaacagtt aaccgtgcgg 60

tcaactttgc tccctatcct taaaaagccc acagaaaagg atg tat aag aat atg     115
                                            Met Tyr Lys Asn Met
                                            1               5

cac att gtt gcc cat cgc ggt gcg gaa gat ctg cac ctc gaa aac acc     163
His Ile Val Ala His Arg Gly Ala Glu Asp Leu His Leu Glu Asn Thr

10                  15                  20

atg acc gct ttc cag gct gcc gcg ccc gct gac gct ttt gag ctg gat     211
Met Thr Ala Phe Gln Ala Ala Ala Pro Ala Asp Ala Phe Glu Leu Asp
            25                  30                  35

atc cac gcc acc gct gac aat cag gtc gtc gtt atc cac gac cgc acc     259
Ile His Ala Thr Ala Asp Asn Gln Val Val Val Ile His Asp Arg Thr
        40                  45                  50

gca gcg cgt gtt gcc gcg cca gat tcc ctg cac cgc gac acc ccg gtt     307
Ala Ala Arg Val Ala Ala Pro Asp Ser Leu His Arg Asp Thr Pro Val
    55                  60                  65

gcg cgc tta agc gcc gcg caa atc aag gag ata acg ctt atc gac gga     355
Ala Arg Leu Ser Ala Ala Gln Ile Lys Glu Ile Thr Leu Ile Asp Gly
70                  75                  80                  85

tcc ccc gta cca acc ctg gag gaa gtt cta ctc cag acg agc ctg ccg     403
Ser Pro Val Pro Thr Leu Glu Glu Val Leu Leu Gln Thr Ser Leu Pro
                90                  95                  100

atc caa gtg gaa atc aaa tct gcc ggt gca gtt cca gca gcc gca gca     451
Ile Gln Val Glu Ile Lys Ser Ala Gly Ala Val Pro Ala Ala Ala Ala
            105                 110                 115

tta ttg cag aaa tac cca gag cac ctg gag cgc ctg ctg ttc atc agt     499
Leu Leu Gln Lys Tyr Pro Glu His Leu Glu Arg Leu Leu Phe Ile Ser
        120                 125                 130

ttc atc gat gca gca ctg gtg gaa atc gtg gat cga ctg cca gaa gct     547
Phe Ile Asp Ala Ala Leu Val Glu Ile Val Asp Arg Leu Pro Glu Ala
    135                 140                 145

cgc gtg gga atc ttg cgc gat gcg tcc atg gat gat ctg cgc att ctt     595
Arg Val Gly Ile Leu Arg Asp Ala Ser Met Asp Asp Leu Arg Ile Leu
150                 155                 160                 165

gat tac atc ccg cta aaa aat gtg ggc gcg atc ttg ccc tcg tgg aaa     643
Asp Tyr Ile Pro Leu Lys Asn Val Gly Ala Ile Leu Pro Ser Trp Lys
                170                 175                 180

gca cta aac gtg gcg tca att gct gat cta cat acc aag gga atc aag     691
Ala Leu Asn Val Ala Ser Ile Ala Asp Leu His Thr Lys Gly Ile Lys
            185                 190                 195

gtt ggc tgc tgg aca att cgg gat gaa aat gcg ttt ggg atc gca caa     739
Val Gly Cys Trp Thr Ile Arg Asp Glu Asn Ala Phe Gly Ile Ala Gln
        200                 205                 210

caa gct ggc gtt gat tac ccc act gtt agc gat ccc tct cgt ttc tcg     787
Gln Ala Gly Val Asp Tyr Pro Thr Val Ser Asp Pro Ser Arg Phe Ser
    215                 220                 225

cgc cct ccc ctg ctg                                                 802
Arg Pro Pro Leu Leu
230

<210> 234
<211> 234

<212> PRT
<213> Corynebacterium glutamicum

<400> 234
Met Tyr Lys Asn Met His Ile Val Ala His Arg Gly Ala Glu Asp Leu
1               5                   10                  15

His Leu Glu Asn Thr Met Thr Ala Phe Gln Ala Ala Ala Pro Ala Asp
            20                  25                  30

Ala Phe Glu Leu Asp Ile His Ala Thr Ala Asp Asn Gln Val Val Val
        35                  40                  45

Ile His Asp Arg Thr Ala Ala Arg Val Ala Ala Pro Asp Ser Leu His
    50                  55                  60

Arg Asp Thr Pro Val Ala Arg Leu Ser Ala Ala Gln Ile Lys Glu Ile
65                  70                  75                  80

Thr Leu Ile Asp Gly Ser Pro Val Pro Thr Leu Glu Glu Val Leu Leu
                85                  90                  95

Gln Thr Ser Leu Pro Ile Gln Val Glu Ile Lys Ser Ala Gly Ala Val
            100                 105                 110

Pro Ala Ala Ala Ala Leu Leu Gln Lys Tyr Pro Glu His Leu Glu Arg
        115                 120                 125

Leu Leu Phe Ile Ser Phe Ile Asp Ala Ala Leu Val Glu Ile Val Asp
    130                 135                 140

Arg Leu Pro Glu Ala Arg Val Gly Ile Leu Arg Asp Ala Ser Met Asp
145                 150                 155                 160

Asp Leu Arg Ile Leu Asp Tyr Ile Pro Leu Lys Asn Val Gly Ala Ile
                165                 170                 175

Leu Pro Ser Trp Lys Ala Leu Asn Val Ala Ser Ile Ala Asp Leu His
            180                 185                 190

Thr Lys Gly Ile Lys Val Gly Cys Trp Thr Ile Arg Asp Glu Asn Ala
        195                 200                 205

Phe Gly Ile Ala Gln Gln Ala Gly Val Asp Tyr Pro Thr Val Ser Asp
    210                 215                 220

Pro Ser Arg Phe Ser Arg Pro Pro Leu Leu
225                 230


<210> 235
<211> 882
<212> DNA
<213> Corynebacterium glutamicum

<220>
<221> CDS
<222> (101)..(859)
<223> RXA02677

<400> 235

ttagtgcagt gtatttattt ccgttcacgc tgcggggctg gtggtttgga gggatactag 60

agtcgatagc aggtatataa aggccaggag agatgggttc atg aaa gtc atc gcg 115
Met Lys Val Ile Ala
1               5

cac cga ggt tta tcg tct cgc ttt ccc gaa tta aca gag tct gcg ttt 163
His Arg Gly Leu Ser Ser Arg Phe Pro Glu Leu Thr Glu Ser Ala Phe
                10                  15                  20

cgg gcg gct cta gag cta ccg att cat gga att gaa act gat gtc cgg 211
Arg Ala Ala Leu Glu Leu Pro Ile His Gly Ile Glu Thr Asp Val Arg
            25                  30                  35

ctg act aaa tgt ggc gaa gtg gtt aac gtc cat gac ccc att gtg gat 259
Leu Thr Lys Cys Gly Glu Val Val Asn Val His Asp Pro Ile Val Asp
        40                  45                  50

cgc gtg tcg aat ggt cgc ggt cgg gtg tcg cgt ttg gac ttg gaa tcc 307
Arg Val Ser Asn Gly Arg Gly Arg Val Ser Arg Leu Asp Leu Glu Ser
    55                  60                  65

ttg ctg agc ttg aac ttt gga acc aaa gaa acc cca gaa aaa gtg ctt 355
Leu Leu Ser Leu Asn Phe Gly Thr Lys Glu Thr Pro Glu Lys Val Leu
70                  75                  80                  85

act tta aac aat cta tta gat att ttt gag gat tat cca gat aag cac 403
Thr Leu Asn Asn Leu Leu Asp Ile Phe Glu Asp Tyr Pro Asp Lys His
                90                  95                  100

ctt tat ata gaa acc aag cac cca atg cgc tac gcg gtc atg ctg gaa 451
Leu Tyr Ile Glu Thr Lys His Pro Met Arg Tyr Ala Val Met Leu Glu
            105                 110                 115

gaa gaa atc aca aaa atc tta aaa tat cgt ggg ctg acg gaa gac cca 499
Glu Glu Ile Thr Lys Ile Leu Lys Tyr Arg Gly Leu Thr Glu Asp Pro
        120                 125                 130

cgc atc cac atc att tct ttt gca ctt ccc gcg atg tat cgc atg gct 547
Arg Ile His Ile Ile Ser Phe Ala Leu Pro Ala Met Tyr Arg Met Ala
    135                 140                 145

cgc ctt gct cca cag ctt gat cgc att cat ctg cgc agg tcg tgg gag 595
Arg Leu Ala Pro Gln Leu Asp Arg Ile His Leu Arg Arg Ser Trp Glu
150                 155                 160                 165

cgt tgg ggt aat ccg cgc gat gtg cgc tgc ggt gta ccc acc ggt ttg 643
Arg Trp Gly Asn Pro Arg Asp Val Arg Cys Gly Val Pro Thr Gly Leu
                170                 175                 180

ggg ttg tcg ctg gag cgg gcg aag atg gat cca agg atg att ggg gcg 691
Gly Leu Ser Leu Glu Arg Ala Lys Met Asp Pro Arg Met Ile Gly Ala
            185                 190                 195

aaa ggg tta ccc acc tat ctt ttc acc gtc gat aag caa aaa gac atg 739
Lys Gly Leu Pro Thr Tyr Leu Phe Thr Val Asp Lys Gln Lys Asp Met
        200                 205                 210

ctg tgg gcg cgc gaa cag ggc gtc gac atg ctc gcc acc aat tat ccg 787
Leu Trp Ala Arg Glu Gln Gly Val Asp Met Leu Ala Thr Asn Tyr Pro
    215                 220                 225

gac cgt gcg gcg gag ctt ttg aac gca cat ccc aag ccc gcc atg tac     835
Asp Arg Ala Ala Glu Leu Leu Asn Ala His Pro Lys Pro Ala Met Tyr
230                 235                 240                 245

gct aat gcg cat ggc aaa gaa gac taagaagaat gaacagctgc cgg          882
Ala Asn Ala His Gly Lys Glu Asp
                250

<210> 236
<211> 253
<212> PRT
<213> Corynebacterium glutamicum

<400> 236
Met Lys Val Ile Ala His Arg Gly Leu Ser Ser Arg Phe Pro Glu Leu
  1               5                  10                  15

Thr Glu Ser Ala Phe Arg Ala Ala Leu Glu Leu Pro Ile His Gly Ile
             20                  25                  30

Glu Thr Asp Val Arg Leu Thr Lys Cys Gly Glu Val Val Asn Val His
         35                  40                  45

Asp Pro Ile Val Asp Arg Val Ser Asn Gly Arg Gly Arg Val Ser Arg
     50                  55                  60

Leu Asp Leu Glu Ser Leu Leu Ser Leu Asn Phe Gly Thr Lys Glu Thr
 65                  70                  75                  80

Pro Glu Lys Val Leu Thr Leu Asn Asn Leu Leu Asp Ile Phe Glu Asp
                 85                  90                  95

Tyr Pro Asp Lys His Leu Tyr Ile Glu Thr Lys His Pro Met Arg Tyr
            100                 105                 110

Ala Val Met Leu Glu Glu Glu Ile Thr Lys Ile Leu Lys Tyr Arg Gly
        115                 120                 125

Leu Thr Glu Asp Pro Arg Ile His Ile Ile Ser Phe Ala Leu Pro Ala
    130                 135                 140

Met Tyr Arg Met Ala Arg Leu Ala Pro Gln Leu Asp Arg Ile His Leu
145                 150                 155                 160

Arg Arg Ser Trp Glu Arg Trp Gly Asn Pro Arg Asp Val Arg Cys Gly
                165                 170                 175

Val Pro Thr Gly Leu Gly Leu Ser Leu Glu Arg Ala Lys Met Asp Pro
            180                 185                 190

Arg Met Ile Gly Ala Lys Gly Leu Pro Thr Tyr Leu Phe Thr Val Asp
        195                 200                 205

Lys Gln Lys Asp Met Leu Trp Ala Arg Glu Gln Gly Val Asp Met Leu
    210                 215                 220

Ala Thr Asn Tyr Pro Asp Arg Ala Ala Glu Leu Leu Asn Ala His Pro
225                 230                 235                 240

Lys Pro Ala Met Tyr Ala Asn Ala His Gly Lys Glu Asp
                245                 250

<210> 237
<211> 1428
<212> DNA
<213> Corynebacterium glutamicum

<220>
<221> CDS
<222> (101)..(1405)
<223> RXS01166

<400> 237
accgtaccca cagacacacc agaattaaca gaaacagact gaaaaacaac atcgctcgac 60

atgcgcgtaa tcctaacccg cgcacactaa tgtggccgat atg ggc tac acc aac    115
                                            Met Gly Tyr Thr Asn
                                              1               5

ctc aac gac aca cgg gtc ttg cgc gcc ggg tca tgt gat gcc tgg tgg    163
Leu Asn Asp Thr Arg Val Leu Arg Ala Gly Ser Cys Asp Ala Trp Trp
                 10                  15                  20

cgc acg atg tct ccg cta gtg cag cag gga agt gag gca gtc ttt cgg    211
Arg Thr Met Ser Pro Leu Val Gln Gln Gly Ser Glu Ala Val Phe Arg
             25                  30                  35

cgc atc atg ggt ctc tcg cgg cgt cct gat cgg aaa cct ggc ttt gac    259
Arg Ile Met Gly Leu Ser Arg Arg Pro Asp Arg Lys Pro Gly Phe Asp
         40                  45                  50

gat gtc cca cat ttc ggc gca gct gtt cga gtt ccc ggt cta aaa cac    307
Asp Val Pro His Phe Gly Ala Ala Val Arg Val Pro Gly Leu Lys His
     55                  60                  65

ggc acg ttg gtc aat gct gca ccc ttg aaa gtt ttg ggc gca cgg ggc    355
Gly Thr Leu Val Asn Ala Ala Pro Leu Lys Val Leu Gly Ala Arg Gly
 70                  75                  80                  85

gag ccc aac ccc gcg agt tcg tac cgt ttt gaa tac atc acc ggt gat    403
Glu Pro Asn Pro Ala Ser Ser Tyr Arg Phe Glu Tyr Ile Thr Gly Asp
                 90                  95                 100

tcc gca ggt cga gcc atc act gcg acc ggc gct gtc ctc ttt tcc aca    451
Ser Ala Gly Arg Ala Ile Thr Ala Thr Gly Ala Val Leu Phe Ser Thr
            105                 110                 115

cgc ccc tgg aca acc ggc ccg cgt ccc gcg atc gcc atg gct cca tcc    499
Arg Pro Trp Thr Thr Gly Pro Arg Pro Ala Ile Ala Met Ala Pro Ser
        120                 125                 130

acc caa ggc gtc gca cag cac tgc gat ccc tcc cac acc tgc gcc atc    547
Thr Gln Gly Val Ala Gln His Cys Asp Pro Ser His Thr Cys Ala Ile
    135                 140                 145

gga ctc aac gca ttc tat gac aaa ccc ttc gac gca atc att gct tac    595
Gly Leu Asn Ala Phe Tyr Asp Lys Pro Phe Asp Ala Ile Ile Ala Tyr
150                 155                 160                 165

gaa ctc ccc gtc atc ctc tgg ttt cta gct cac gga ctt gac gtt gtg 643
Glu Leu Pro Val Ile Leu Trp Phe Leu Ala His Gly Leu Asp Val Val
                170                 175                 180

ttc atc gat tac ccc cgc gac ccc gca acc ggc gtc caa tac tat tgc 691
Phe Ile Asp Tyr Pro Arg Asp Pro Ala Thr Gly Val Gln Tyr Tyr Cys
            185                 190                 195

gat tcc atc gct gca gct aaa tcg ctt ctc gac gcc gtc ctc gcc tcc 739
Asp Ser Ile Ala Ala Ala Lys Ser Leu Leu Asp Ala Val Leu Ala Ser
        200                 205                 210

aga caa ctc ggc ctt tca ccg gaa gca ccg ctt ggc ctg tgg gga ttc 787
Arg Gln Leu Gly Leu Ser Pro Glu Ala Pro Leu Gly Leu Trp Gly Phe
    215                 220                 225

tcc caa gga ggc ggc gcc act ggc tgg gct gca caa ttg cag gat tac 835
Ser Gln Gly Gly Gly Ala Thr Gly Trp Ala Ala Gln Leu Gln Asp Tyr
230                 235                 240                 245

gca cct gat gtc cgc cca aag gca gcg gtc gtg ggc gct cca cca gtg 883
Ala Pro Asp Val Arg Pro Lys Ala Ala Val Val Gly Ala Pro Pro Val
                250                 255                 260

gat ctc ttc cgc gtc ttg gac act gtc gac ggc gga ttg ctc acc gga 931
Asp Leu Phe Arg Val Leu Asp Thr Val Asp Gly Gly Leu Leu Thr Gly
            265                 270                 275

gtg att gcc tac gcc atc gcg gga ctt gca gtg aac tct tca gag atg 979
Val Ile Ala Tyr Ala Ile Ala Gly Leu Ala Val Asn Ser Ser Glu Met
        280                 285                 290

ttt gag gaa atc atg tcg gtg tta aat gaa cgc gga gtc agt gat gtg 1027
Phe Glu Glu Ile Met Ser Val Leu Asn Glu Arg Gly Val Ser Asp Val
    295                 300                 305

ctg aaa aat atc acc agc tgc gcg gga ggt tcc ttg ttg gcc agt ggc 1075
Leu Lys Asn Ile Thr Ser Cys Ala Gly Gly Ser Leu Leu Ala Ser Gly
310                 315                 320                 325

tac tcg tct tcc cgc ggg tgg aca cat cag ggc acg ccg ctg gca gac 1123
Tyr Ser Ser Ser Arg Gly Trp Thr His Gln Gly Thr Pro Leu Ala Asp
                330                 335                 340

att ctg gac gat ctg cca ctt gtt gtc gct gag ttt ggg aag caa aag 1171
Ile Leu Asp Asp Leu Pro Leu Val Val Ala Glu Phe Gly Lys Gln Lys
            345                 350                 355

ctg ggt cgt gtg gcg cca gaa atc cca gtg ctg ttg tgg ggc tct aaa 1219
Leu Gly Arg Val Ala Pro Glu Ile Pro Val Leu Leu Trp Gly Ser Lys
        360                 365                 370

aat gat gat gtc att ccc att gat ccc att agg gaa ttg cgt gat agc 1267
Asn Asp Asp Val Ile Pro Ile Asp Pro Ile Arg Glu Leu Arg Asp Ser
    375                 380                 385

tgg gcg gac aag ggt acg cca ttg acc tgg cat gaa tcc caa gcg ccg 1315
Trp Ala Asp Lys Gly Thr Pro Leu Thr Trp His Glu Ser Gln Ala Pro
390                 395                 400                 405

cgt gtg cca gga cgc aca ggt ctc aac cat ttc ggg ccc tat ttt aga 1363

Arg Val Pro Gly Arg Thr Gly Leu Asn His Phe Gly Pro Tyr Phe Arg
                410                 415                 420

aac ctg gaa aag tac tcg gga tgg ctc ata gat cat ctt gtc 1405
Asn Leu Glu Lys Tyr Ser Gly Trp Leu Ile Asp His Leu Val
            425                 430                 435

tgagtgccgt tttaaaggct cgg 1428

<210> 238
<211> 435
<212> PRT
<213> Corynebacterium glutamicum

<400> 238
Met Gly Tyr Thr Asn Leu Asn Asp Thr Arg Val Leu Arg Ala Gly Ser
1               5                   10                  15

Cys Asp Ala Trp Trp Arg Thr Met Ser Pro Leu Val Gln Gln Gly Ser
            20                  25                  30

Glu Ala Val Phe Arg Arg Ile Met Gly Leu Ser Arg Arg Pro Asp Arg
        35                  40                  45

Lys Pro Gly Phe Asp Asp Val Pro His Phe Gly Ala Ala Val Arg Val
    50                  55                  60

Pro Gly Leu Lys His Gly Thr Leu Val Asn Ala Ala Pro Leu Lys Val
65                  70                  75                  80

Leu Gly Ala Arg Gly Glu Pro Asn Pro Ala Ser Ser Tyr Arg Phe Glu
                85                  90                  95

Tyr Ile Thr Gly Asp Ser Ala Gly Arg Ala Ile Thr Ala Thr Gly Ala
            100                 105                 110

Val Leu Phe Ser Thr Arg Pro Trp Thr Thr Gly Pro Arg Pro Ala Ile
        115                 120                 125

Ala Met Ala Pro Ser Thr Gln Gly Val Ala Gln His Cys Asp Pro Ser
    130                 135                 140

His Thr Cys Ala Ile Gly Leu Asn Ala Phe Tyr Asp Lys Pro Phe Asp
145                 150                 155                 160

Ala Ile Ile Ala Tyr Glu Leu Pro Val Ile Leu Trp Phe Leu Ala His
                165                 170                 175

Gly Leu Asp Val Val Phe Ile Asp Tyr Pro Arg Asp Pro Ala Thr Gly
            180                 185                 190

Val Gln Tyr Tyr Cys Asp Ser Ile Ala Ala Ala Lys Ser Leu Leu Asp
        195                 200                 205

Ala Val Leu Ala Ser Arg Gln Leu Gly Leu Ser Pro Glu Ala Pro Leu
    210                 215                 220

Gly Leu Trp Gly Phe Ser Gln Gly Gly Gly Ala Thr Gly Trp Ala Ala
225                 230                 235                 240

Gln Leu Gln Asp Tyr Ala Pro Asp Val Arg Pro Lys Ala Ala Val Val
                245                 250                 255

Gly Ala Pro Pro Val Asp Leu Phe Arg Val Leu Asp Thr Val Asp Gly
            260                 265                 270

Gly Leu Leu Thr Gly Val Ile Ala Tyr Ala Ile Ala Gly Leu Ala Val
        275                 280                 285

Asn Ser Ser Glu Met Phe Glu Glu Ile Met Ser Val Leu Asn Glu Arg
    290                 295                 300

Gly Val Ser Asp Val Leu Lys Asn Ile Thr Ser Cys Ala Gly Gly Ser
305                 310                 315                 320

Leu Leu Ala Ser Gly Tyr Ser Ser Ser Arg Gly Trp Thr His Gln Gly
                325                 330                 335

Thr Pro Leu Ala Asp Ile Leu Asp Asp Leu Pro Leu Val Val Ala Glu
            340                 345                 350

Phe Gly Lys Gln Lys Leu Gly Arg Val Ala Pro Glu Ile Pro Val Leu
        355                 360                 365

Leu Trp Gly Ser Lys Asn Asp Asp Val Ile Pro Ile Asp Pro Ile Arg
    370                 375                 380

Glu Leu Arg Asp Ser Trp Ala Asp Lys Gly Thr Pro Leu Thr Trp His
385                 390                 395                 400

Glu Ser Gln Ala Pro Arg Val Pro Gly Arg Thr Gly Leu Asn His Phe
                405                 410                 415

Gly Pro Tyr Phe Arg Asn Leu Glu Lys Tyr Ser Gly Trp Leu Ile Asp
            420                 425                 430

His Leu Val
        435


<210> 239
<211> 690
<212> DNA
<213> Corynebacterium glutamicum

<220>
<221> CDS
<222> (101)..(667)
<223> RXA00875

<400> 239
caatttggcg gcgctgctgg gtgatcgggg gccggttgtt ccgggtgtga ttggtgactg 60

ggtgaggggc taacgcaaca atgtgtctaa gcttaggggc atg act act gag gtt   115
                                            Met Thr Thr Glu Val
                                            1               5

gaa ctg gtt gtt tta gct gat tcc gag ggc aat cct att ggt act gcg   163
Glu Leu Val Val Leu Ala Asp Ser Glu Gly Asn Pro Ile Gly Thr Ala
                10                  15                  20

ccg aaa gct acg gtg cac act aag gac acg cct ctg cat ttc gcg ttt 211
Pro Lys Ala Thr Val His Thr Lys Asp Thr Pro Leu His Phe Ala Phe
            25                  30                  35

tcc acc tat att ttg aac ccg cgt ggg gag ctg ttg gtg acg cgt cgt 259
Ser Thr Tyr Ile Leu Asn Pro Arg Gly Glu Leu Leu Val Thr Arg Arg
        40                  45                  50

gca ttg tcg aag aag aca tgg cct ggt gtg tgg acg aac tct atg tgt 307
Ala Leu Ser Lys Lys Thr Trp Pro Gly Val Trp Thr Asn Ser Met Cys
    55                  60                  65

ggg cac cct ggt ccg gat gag aca aac gcg gat gcg att cgt cgc agg 355
Gly His Pro Gly Pro Asp Glu Thr Asn Ala Asp Ala Ile Arg Arg Arg
70                  75                  80                  85

ggt gtc gat gag ttg ggg ctg gag gta gat tct ttc ttg gat att caa 403
Gly Val Asp Glu Leu Gly Leu Glu Val Asp Ser Phe Leu Asp Ile Gln
                90                  95                  100

gag att ctg cct gat tac cag tac cgt gct gtc gac gcg tcc ggc att 451
Glu Ile Leu Pro Asp Tyr Gln Tyr Arg Ala Val Asp Ala Ser Gly Ile
            105                 110                 115

gtg gag tgg gag ttg tgc ccg gtc cac ctc gtg cgt tta gcg gtg ggg 499
Val Glu Trp Glu Leu Cys Pro Val His Leu Val Arg Leu Ala Val Gly
        120                 125                 130

gaa ttt gtg gag cca ctg gat gat gag gtg gag gag ttc gag tgg gcg 547
Glu Phe Val Glu Pro Leu Asp Asp Glu Val Glu Glu Phe Glu Trp Ala
    135                 140                 145

gaa ccg cag aag ctt ttc gac gct gtt gat gcc aca cca ttt gtg ttt 595
Glu Pro Gln Lys Leu Phe Asp Ala Val Asp Ala Thr Pro Phe Val Phe
150                 155                 160                 165

tct cca tgg cta gtg gat cag ctt agc gcc cct gag ctg cgc caa gcc 643
Ser Pro Trp Leu Val Asp Gln Leu Ser Ala Pro Glu Leu Arg Gln Ala
                170                 175                 180

atc ctg gaa gcg ttt gac gca gag taactaacta gtctagaagc ctt 690
Ile Leu Glu Ala Phe Asp Ala Glu
            185

<210> 240
<211> 189
<212> PRT
<213> Corynebacterium glutamicum

<400> 240
Met Thr Thr Glu Val Glu Leu Val Val Leu Ala Asp Ser Glu Gly Asn
1               5                   10                  15

Pro Ile Gly Thr Ala Pro Lys Ala Thr Val His Thr Lys Asp Thr Pro
            20                  25                  30

Leu His Phe Ala Phe Ser Thr Tyr Ile Leu Asn Pro Arg Gly Glu Leu
        35                  40                  45

Leu Val Thr Arg Arg Ala Leu Ser Lys Lys Thr Trp Pro Gly Val Trp

50                  55                  60

Thr Asn Ser Met Cys Gly His Pro Gly Pro Asp Glu Thr Asn Ala Asp
65                  70                  75                  80

Ala Ile Arg Arg Arg Gly Val Asp Glu Leu Gly Leu Glu Val Asp Ser
                85                  90                  95

Phe Leu Asp Ile Gln Glu Ile Leu Pro Asp Tyr Gln Tyr Arg Ala Val
            100                 105                 110

Asp Ala Ser Gly Ile Val Glu Trp Glu Leu Cys Pro Val His Leu Val
        115                 120                 125

Arg Leu Ala Val Gly Glu Phe Val Glu Pro Leu Asp Asp Glu Val Glu
    130                 135                 140

Glu Phe Glu Trp Ala Glu Pro Gln Lys Leu Phe Asp Ala Val Asp Ala
145                 150                 155                 160

Thr Pro Phe Val Phe Ser Pro Trp Leu Val Asp Gln Leu Ser Ala Pro
                165                 170                 175

Glu Leu Arg Gln Ala Ile Leu Glu Ala Phe Asp Ala Glu
            180                 185


<210> 241
<211> 1308
<212> DNA
<213> Corynebacterium glutamicum

<220>
<221> CDS
<222> (101)..(1285)
<223> RXA01292

<400> 241
tccgcgtccc tcgaagactt aaaaaacggg cgggattttg tgccgtcttt aaaaaagcca 60

gcctggcaac caaagcaatg tggaaagaag tgtttcaaaa atg aca aaa gca gtg   115
                                            Met Thr Lys Ala Val
                                            1               5

gtc atc ggc ggg gga cta gca gga cta gcc acc acc gca ctg ctc tta   163
Val Ile Gly Gly Gly Leu Ala Gly Leu Ala Thr Thr Ala Leu Leu Leu
                10                  15                  20

cgc gaa gga tat gaa gtc cac ctc gtc gaa caa aac gag cac ctc ggc   211
Arg Glu Gly Tyr Glu Val His Leu Val Glu Gln Asn Glu His Leu Gly
            25                  30                  35

ggc cgc gcc ggg acc ttc gaa ctc gac ggc ttc cgc tgg gac acc ggc   259
Gly Arg Ala Gly Thr Phe Glu Leu Asp Gly Phe Arg Trp Asp Thr Gly
        40                  45                  50

ccc agc tgg tac ctc atg ccc gac gcc atg tcc cac ttc ttt aag ctc   307
Pro Ser Trp Tyr Leu Met Pro Asp Ala Met Ser His Phe Phe Lys Leu
    55                  60                  65

tgc ggc acc tcc atc gac gac cac ctc gac ctc gtg cca ctt gaa ccc   355

Cys Gly Thr Ser Ile Asp Asp His Leu Asp Leu Val Pro Leu Glu Pro
70                  75                  80                  85

gcc tac cgc gtc atc gac gac cac ggc gaa ttc atc gac gtc acc tcc     403
Ala Tyr Arg Val Ile Asp Asp His Gly Glu Phe Ile Asp Val Thr Ser
                90                  95                  100

gac atc gat gcg atg gcc gag ctt ttc gaa tcc cgc gaa ccc ggc gcc     451
Asp Ile Asp Ala Met Ala Glu Leu Phe Glu Ser Arg Glu Pro Gly Ala
            105                 110                 115

ggc gcc aaa ctg cgc acc tat atc gac tcc gcc aca cag gtc tac aac     499
Gly Ala Lys Leu Arg Thr Tyr Ile Asp Ser Ala Thr Gln Val Tyr Asn
        120                 125                 130

ctg gcc atc gac ggc ttc ctc tac acc aac ttc acc aac ttc atc cca     547
Leu Ala Ile Asp Gly Phe Leu Tyr Thr Asn Phe Thr Asn Phe Ile Pro
    135                 140                 145

tac tta agc ccc ggc atg ctg cgc ctt tta ccc aaa ctt cta gca agc     595
Tyr Leu Ser Pro Gly Met Leu Arg Leu Leu Pro Lys Leu Leu Ala Ser
150                 155                 160                 165

ctg tcg acc tcg cta aaa gtc aag gtc aac acc caa ttc cgc gat aca     643
Leu Ser Thr Ser Leu Lys Val Lys Val Asn Thr Gln Phe Arg Asp Thr
                170                 175                 180

aaa ctg cgc caa atc tta agc tac ccc gca gtt ttc ctc tcc tca gac     691
Lys Leu Arg Gln Ile Leu Ser Tyr Pro Ala Val Phe Leu Ser Ser Asp
            185                 190                 195

cct tcg cac acc ccg gcg ctc tat cac ctc atg agc cac acc gac ctc     739
Pro Ser His Thr Pro Ala Leu Tyr His Leu Met Ser His Thr Asp Leu
        200                 205                 210

gtc caa ggc gtc tcc tat ccc cga ggc ggc ttc acc gcc ttc atc aag     787
Val Gln Gly Val Ser Tyr Pro Arg Gly Gly Phe Thr Ala Phe Ile Lys
    215                 220                 225

gca cta att tcg ctt atc gac gac gcc gtc ctc cac ctc ggc acc ccc     835
Ala Leu Ile Ser Leu Ile Asp Asp Ala Val Leu His Leu Gly Thr Pro
230                 235                 240                 245

gtc agc gca atc acc acc caa ggc cga aac gcc aca ggc gtc caa gtc     883
Val Ser Ala Ile Thr Thr Gln Gly Arg Asn Ala Thr Gly Val Gln Val
                250                 255                 260

ggc tca gag ttc atc gaa gcc gat atc gtg atc agc tgc gct gat cag     931
Gly Ser Glu Phe Ile Glu Ala Asp Ile Val Ile Ser Cys Ala Asp Gln
            265                 270                 275

cac cac acc gaa acc caa ctc cta cct gct tcg ttg tgc gcg aag ccg     979
His His Thr Glu Thr Gln Leu Leu Pro Ala Ser Leu Cys Ala Lys Pro
        280                 285                 290

gag acg agc tgg aag aac aaa caa ccc ggc ctc agt act gtg ctg gtt     1027
Glu Thr Ser Trp Lys Asn Lys Gln Pro Gly Leu Ser Thr Val Leu Val
    295                 300                 305

ttg gca ggc gtg aag ggg gag cac acc ctg ctt ttt cct ccg act ggg     1075
Leu Ala Gly Val Lys Gly Glu His Thr Leu Leu Phe Pro Pro Thr Gly

310                 315                 320                 325

acg aag att tcc gca aag ttt tcg acg gct cca ccc cag aat tcc cgg     1123
Thr Lys Ile Ser Ala Lys Phe Ser Thr Ala Pro Pro Gln Asn Ser Arg
                330                 335                 340

ctt cag aat cca tct aga tct cca aga cct ccg caa cag att ccg atg     1171
Leu Gln Asn Pro Ser Arg Ser Pro Arg Pro Pro Gln Gln Ile Pro Met
            345                 350                 355

ccg cac ccg aag gcc acg aga acc tct tca tcc tgg tcc cag tac ccg     1219
Pro His Pro Lys Ala Thr Arg Thr Ser Ser Ser Trp Ser Gln Tyr Pro
        360                 365                 370

ccg atg tct cca ttg gtc acg ggt ccg ctt acg gag aag aat ctg aca     1267
Pro Met Ser Pro Leu Val Thr Gly Pro Leu Thr Glu Lys Asn Leu Thr
    375                 380                 385

tgg tgg gcc gga tcg caa tagcagcagt ggctcaaatt ggg                   1308
Trp Trp Ala Gly Ser Gln
390                 395


<210> 242
<211> 395
<212> PRT
<213> Corynebacterium glutamicum

<400> 242
Met Thr Lys Ala Val Val Ile Gly Gly Gly Leu Ala Gly Leu Ala Thr
  1               5                  10                  15

Thr Ala Leu Leu Leu Arg Glu Gly Tyr Glu Val His Leu Val Glu Gln
             20                  25                  30

Asn Glu His Leu Gly Gly Arg Ala Gly Thr Phe Glu Leu Asp Gly Phe
         35                  40                  45

Arg Trp Asp Thr Gly Pro Ser Trp Tyr Leu Met Pro Asp Ala Met Ser
     50                  55                  60

His Phe Phe Lys Leu Cys Gly Thr Ser Ile Asp Asp His Leu Asp Leu
 65                  70                  75                  80

Val Pro Leu Glu Pro Ala Tyr Arg Val Ile Asp Asp His Gly Glu Phe
                 85                  90                  95

Ile Asp Val Thr Ser Asp Ile Asp Ala Met Ala Glu Leu Phe Glu Ser
            100                 105                 110

Arg Glu Pro Gly Ala Gly Ala Lys Leu Arg Thr Tyr Ile Asp Ser Ala
        115                 120                 125

Thr Gln Val Tyr Asn Leu Ala Ile Asp Gly Phe Leu Tyr Thr Asn Phe
    130                 135                 140

Thr Asn Phe Ile Pro Tyr Leu Ser Pro Gly Met Leu Arg Leu Leu Pro
145                 150                 155                 160

Lys Leu Leu Ala Ser Leu Ser Thr Ser Leu Lys Val Lys Val Asn Thr
                165                 170                 175

Gln Phe Arg Asp Thr Lys Leu Arg Gln Ile Leu Ser Tyr Pro Ala Val
            180                 185                 190

Phe Leu Ser Ser Asp Pro Ser His Thr Pro Ala Leu Tyr His Leu Met
        195                 200                 205

Ser His Thr Asp Leu Val Gln Gly Val Ser Tyr Pro Arg Gly Gly Phe
    210                 215                 220

Thr Ala Phe Ile Lys Ala Leu Ile Ser Leu Ile Asp Asp Ala Val Leu
225                 230                 235                 240

His Leu Gly Thr Pro Val Ser Ala Ile Thr Thr Gln Gly Arg Asn Ala
                245                 250                 255

Thr Gly Val Gln Val Gly Ser Glu Phe Ile Glu Ala Asp Ile Val Ile
            260                 265                 270

Ser Cys Ala Asp Gln His His Thr Glu Thr Gln Leu Leu Pro Ala Ser
        275                 280                 285

Leu Cys Ala Lys Pro Glu Thr Ser Trp Lys Asn Lys Gln Pro Gly Leu
    290                 295                 300

Ser Thr Val Leu Val Leu Ala Gly Val Lys Gly Glu His Thr Leu Leu
305                 310                 315                 320

Phe Pro Pro Thr Gly Thr Lys Ile Ser Ala Lys Phe Ser Thr Ala Pro
                325                 330                 335

Pro Gln Asn Ser Arg Leu Gln Asn Pro Ser Arg Ser Pro Arg Pro Pro
            340                 345                 350

Gln Gln Ile Pro Met Pro His Pro Lys Ala Thr Arg Thr Ser Ser Ser
        355                 360                 365

Trp Ser Gln Tyr Pro Pro Met Ser Pro Leu Val Thr Gly Pro Leu Thr
    370                 375                 380

Glu Lys Asn Leu Thr Trp Trp Ala Gly Ser Gln
385                 390                 395

<210> 243
<211> 450
<212> DNA
<213> Corynebacterium glutamicum

<220>
<221> CDS
<222> (101)..(427)
<223> RXA01293

<400> 243
   cgatgccgca cccgaaggcc acgagaacct cttcatcctg gtcccagtac ccgccgatgt 60
   ctccattggt cacgggtccg cttacggaga agaatctgac atg gtg ggc cgg atc   115
                                              Met Val Gly Arg Ile
                                              1               5
   gca ata gca gca gtg gct caa att ggg cga tgg gct ggc att gat ggt     163
   Ala Ile Ala Ala Val Ala Gln Ile Gly Arg Trp Ala Gly Ile Asp Gly
                   10                  15                  20
   ttg gaa agc cgc att gtt gtg cag cgc acc atc ggc cct gcc gac ttc     211
   Leu Glu Ser Arg Ile Val Val Gln Arg Thr Ile Gly Pro Ala Asp Phe
               25                  30                  35
   gca gac cga tac aac tcc tgg agc ggc ggg tcc att ggc ccc tca cac     259
   Ala Asp Arg Tyr Asn Ser Trp Ser Gly Gly Ser Ile Gly Pro Ser His
           40                  45                  50
   acc ctg gca caa tcg gcg ttc ttt agg ggt tct aac aaa tcc cgc aaa     307
   Thr Leu Ala Gln Ser Ala Phe Phe Arg Gly Ser Asn Lys Ser Arg Lys
       55                  60                  65
   gta gat ggc ctg tat gac gca ggt gac acc aca gtt cca ggg gtg ggc     355
   Val Asp Gly Leu Tyr Asp Ala Gly Asp Thr Thr Val Pro Gly Val Gly
   70                  75                  80                  85
   gta tct atg tgt ttg atc tct gca gaa aac gtg ctc aag cgt ctc cgc     403
   Val Ser Met Cys Leu Ile Ser Ala Glu Asn Val Leu Lys Arg Leu Arg
                   90                  95                  100
   ggg gac aac agt gtg gat cgg act taacgggact tttttctttt gct 450
   Gly Asp Asn Ser Val Asp Arg Thr
   105

<210> 244
<211> 109
<212> PRT
<213> Corynebacterium glutamicum

<400> 244
Met Val Gly Arg Ile Ala Ile Ala Ala Val Ala Gln Ile Gly Arg Trp
1               5                   10                  15

Ala Gly Ile Asp Gly Leu Glu Ser Arg Ile Val Val Gln Arg Thr Ile
            20                  25                  30

Gly Pro Ala Asp Phe Ala Asp Arg Tyr Asn Ser Trp Ser Gly Gly Ser
        35                  40                  45

Ile Gly Pro Ser His Thr Leu Ala Gln Ser Ala Phe Phe Arg Gly Ser
    50                  55                  60

Asn Lys Ser Arg Lys Val Asp Gly Leu Tyr Asp Ala Gly Asp Thr Thr
65                  70                  75                  80

Val Pro Gly Val Gly Val Ser Met Cys Leu Ile Ser Ala Glu Asn Val
                85                  90                  95

Leu Lys Arg Leu Arg Gly Asp Asn Ser Val Asp Arg Thr
            100                 105

<210> 245
<211> 1386
<212> DNA
<213> Corynebacterium glutamicum

<220>
<221> CDS
<222> (101)..(1363)
<223> RXA02310

<400> 245
gaaatggaca aaaagctttt tgtttaaccg tagtcgacgg aaggcacttg aagcaccttg 60

ggttagtctt cttgctctcc ggactgagac aatggggttt gtg tct aca act ttt 115
Val Ser Thr Thr Phe
1               5

gat gtg ttg atc atc ggc gcg ggc ccc tca ggt gcc agc gcc gcc gtc 163
Asp Val Leu Ile Ile Gly Ala Gly Pro Ser Gly Ala Ser Ala Ala Val
                10                  15                  20

cat gcg gcc agg act ggg ctt caa aca ttg ctt atc gac gcc tcc tcc 211
His Ala Ala Arg Thr Gly Leu Gln Thr Leu Leu Ile Asp Ala Ser Ser
            25                  30                  35

ttc ccg cgg gat aaa acg tgt ggc gat ggc ctt act ccc cgt gcg att 259
Phe Pro Arg Asp Lys Thr Cys Gly Asp Gly Leu Thr Pro Arg Ala Ile
        40                  45                  50

cac cag cta gaa ctt cta ggt gtt gct gat cag gtt acc ggg gat tat 307
His Gln Leu Glu Leu Leu Gly Val Ala Asp Gln Val Thr Gly Asp Tyr
    55                  60                  65

ttc aac aag ggc ttg aaa ctg cat ggt ttt ggt ggc tct gtt gag gcg 355
Phe Asn Lys Gly Leu Lys Leu His Gly Phe Gly Gly Ser Val Glu Ala
70                  75                  80                  85

ccg tgg ccg gag aca tat ttc acg aat aag ggt tcc gcg atg tcg cgg 403
Pro Trp Pro Glu Thr Tyr Phe Thr Asn Lys Gly Ser Ala Met Ser Arg
                90                  95                  100

atg gag ttc gat gat ttg tta ttc cgc ttg gca aaa tct cat gag gaa 451
Met Glu Phe Asp Asp Leu Leu Phe Arg Leu Ala Lys Ser His Glu Glu
            105                 110                 115

gta acc acg tgg gag aac gcg agc gcc caa gac ccc att ttg agg ggg 499
Val Thr Thr Trp Glu Asn Ala Ser Ala Gln Asp Pro Ile Leu Arg Gly
        120                 125                 130

aat ttc ttg gaa ggc gtt gtg att aat cac gca ggg caa gag aaa acc 547
Asn Phe Leu Glu Gly Val Val Ile Asn His Ala Gly Gln Glu Lys Thr
    135                 140                 145

gtc aag gcg aag cat gtg att att gcc gat ggt gtc cgc tcc cct ttc 595
Val Lys Ala Lys His Val Ile Ile Ala Asp Gly Val Arg Ser Pro Phe
150                 155                 160                 165

ggt aag aaa ctg ggt agg cag tgg caa cgc gat gag gtg tat ggc att 643
Gly Lys Lys Leu Gly Arg Gln Trp Gln Arg Asp Glu Val Tyr Gly Ile
                170                 175                 180

gcg gct cgt gct tat tgt gaa act ccg ctg tct gat gaa ccg tgg att 691
Ala Ala Arg Ala Tyr Cys Glu Thr Pro Leu Ser Asp Glu Pro Trp Ile
            185                 190                 195

cac tcc cat gtg gaa ctg cgc gat gaa gat ggt gtg gtg cag cca gga 739
His Ser His Val Glu Leu Arg Asp Glu Asp Gly Val Val Gln Pro Gly
        200                 205                 210

tat ggg tgg att ttc ccg ctg ggc aac ggc acg gtg aat ttg ggt tgt 787
Tyr Gly Trp Ile Phe Pro Leu Gly Asn Gly Thr Val Asn Leu Gly Cys
    215                 220                 225

ggc gcg ctc tcg acg gat acg aga cca gcg aag atc aat acg aag aaa 835
Gly Ala Leu Ser Thr Asp Thr Arg Pro Ala Lys Ile Asn Thr Lys Lys
230                 235                 240                 245

ttg ttg agc ttc tat gcg ggt cag cgt cgt aaa gca tgg caa ctc ggg 883
Leu Leu Ser Phe Tyr Ala Gly Gln Arg Arg Lys Ala Trp Gln Leu Gly
                250                 255                 260

ccc gag cac gac gtc gcc tct gcc ctg ctg cct atg ggc ggc gcg gtg 931
Pro Glu His Asp Val Ala Ser Ala Leu Leu Pro Met Gly Gly Ala Val
            265                 270                 275

tcg aat gtg gct ggc gcg aac tgg atg ctg atc ggc gat tcc gcc gcg 979
Ser Asn Val Ala Gly Ala Asn Trp Met Leu Ile Gly Asp Ser Ala Ala
        280                 285                 290

tgt gtg aac ccg ctg aac ggc gaa ggc atc gac tat ggc ctg gaa acc 1027
Cys Val Asn Pro Leu Asn Gly Glu Gly Ile Asp Tyr Gly Leu Glu Thr
    295                 300                 305

gcg gcg atg gcc gtc gac acg ctt gtg gaa aac ccc aag cgc gat ttg 1075
Ala Ala Met Ala Val Asp Thr Leu Val Glu Asn Pro Lys Arg Asp Leu
310                 315                 320                 325

acc ttg gta tgg cca cat agg ttg cgc gac gcg tac ggc gag acc ttc 1123
Thr Leu Val Trp Pro His Arg Leu Arg Asp Ala Tyr Gly Glu Thr Phe
                330                 335                 340

atg ttg gcg cgc acg gct gct cga ctg ctg acg tac ccg cag ttt ttg 1171
Met Leu Ala Arg Thr Ala Ala Arg Leu Leu Thr Tyr Pro Gln Phe Leu
            345                 350                 355

ccg atg gct ggg ccg ctc gca ttc cgc ggg ccg ctg caa aag gcc atc 1219
Pro Met Ala Gly Pro Leu Ala Phe Arg Gly Pro Leu Gln Lys Ala Ile
        360                 365                 370

atg ccg gcg gct gcg cgt ttg atg ggc aac ctg atc aca gag gag gat 1267
Met Pro Ala Ala Ala Arg Leu Met Gly Asn Leu Ile Thr Glu Glu Asp
    375                 380                 385

aaa gac ctg ctc gcc agg ggt tgg cag gcc gcc gga tcc gcg att agt 1315
Lys Asp Leu Leu Ala Arg Gly Trp Gln Ala Ala Gly Ser Ala Ile Ser
390                 395                 400                 405

tgg gcg cgg aag ggc tcc cct ctg tgg gac tcg act agt tct ctg gtt 1363
Trp Ala Arg Lys Gly Ser Pro Leu Trp Asp Ser Thr Ser Ser Leu Val
                410                 415                 420

taatcgccga gtgcagcgcg acg 1386

<210> 246
<211> 421

<212> PRT
<213> Corynebacterium glutamicum

<400> 246
Val Ser Thr Thr Phe Asp Val Leu Ile Ile Gly Ala Gly Pro Ser Gly
1               5                   10                  15

Ala Ser Ala Ala Val His Ala Ala Arg Thr Gly Leu Gln Thr Leu Leu
            20                  25                  30

Ile Asp Ala Ser Ser Phe Pro Arg Asp Lys Thr Cys Gly Asp Gly Leu
        35                  40                  45

Thr Pro Arg Ala Ile His Gln Leu Glu Leu Leu Gly Val Ala Asp Gln
    50                  55                  60

Val Thr Gly Asp Tyr Phe Asn Lys Gly Leu Lys Leu His Gly Phe Gly
65                  70                  75                  80

Gly Ser Val Glu Ala Pro Trp Pro Glu Thr Tyr Phe Thr Asn Lys Gly
                85                  90                  95

Ser Ala Met Ser Arg Met Glu Phe Asp Asp Leu Leu Phe Arg Leu Ala
            100                 105                 110

Lys Ser His Glu Glu Val Thr Thr Trp Glu Asn Ala Ser Ala Gln Asp
        115                 120                 125

Pro Ile Leu Arg Gly Asn Phe Leu Glu Gly Val Val Ile Asn His Ala
    130                 135                 140

Gly Gln Glu Lys Thr Val Lys Ala Lys His Val Ile Ile Ala Asp Gly
145                 150                 155                 160

Val Arg Ser Pro Phe Gly Lys Lys Leu Gly Arg Gln Trp Gln Arg Asp
                165                 170                 175

Glu Val Tyr Gly Ile Ala Ala Arg Ala Tyr Cys Glu Thr Pro Leu Ser
            180                 185                 190

Asp Glu Pro Trp Ile His Ser His Val Glu Leu Arg Asp Glu Asp Gly
        195                 200                 205

Val Val Gln Pro Gly Tyr Gly Trp Ile Phe Pro Leu Gly Asn Gly Thr
    210                 215                 220

Val Asn Leu Gly Cys Gly Ala Leu Ser Thr Asp Thr Arg Pro Ala Lys
225                 230                 235                 240

Ile Asn Thr Lys Lys Leu Leu Ser Phe Tyr Ala Gly Gln Arg Arg Lys
                245                 250                 255

Ala Trp Gln Leu Gly Pro Glu His Asp Val Ala Ser Ala Leu Leu Pro
            260                 265                 270

Met Gly Gly Ala Val Ser Asn Val Ala Gly Ala Asn Trp Met Leu Ile
        275                 280                 285

Gly Asp Ser Ala Ala Cys Val Asn Pro Leu Asn Gly Glu Gly Ile Asp
    290                 295                 300

Tyr Gly Leu Glu Thr Ala Ala Met Ala Val Asp Thr Leu Val Glu Asn
305                 310                 315                 320

Pro Lys Arg Asp Leu Thr Leu Val Trp Pro His Arg Leu Arg Asp Ala
                325                 330                 335

Tyr Gly Glu Thr Phe Met Leu Ala Arg Thr Ala Ala Arg Leu Leu Thr
            340                 345                 350

Tyr Pro Gln Phe Leu Pro Met Ala Gly Pro Leu Ala Phe Arg Gly Pro
        355                 360                 365

Leu Gln Lys Ala Ile Met Pro Ala Ala Ala Arg Leu Met Gly Asn Leu
    370                 375                 380

Ile Thr Glu Glu Asp Lys Asp Leu Leu Ala Arg Gly Trp Gln Ala Ala
385                 390                 395                 400

Gly Ser Ala Ile Ser Trp Ala Arg Lys Gly Ser Pro Leu Trp Asp Ser
                405                 410                 415

Thr Ser Ser Leu Val
            420

<210> 247
<211> 1170
<212> DNA
<213> Corynebacterium glutamicum

<220>
<221> CDS
<222> (101)..(1147)
<223> RXA02718

<400> 247
tccaccaaaa ttcagcccgc atccccctat tcgattgaag gacgtctcct tgagcagttt 60

cgatgcccat gaccttgacc tcgacaaatt tccggaggtc gtg cga gat cgt ttg   115
                                          Val Arg Asp Arg Leu
                                          1               5

acg cag ttc ctc gat gct caa gag cta aca att gct gat atc ggc gct   163
Thr Gln Phe Leu Asp Ala Gln Glu Leu Thr Ile Ala Asp Ile Gly Ala
                10                  15                  20

cct gtc aca gat gct gtg gcc cat ctt cgc agt ttc gtg ctc aat gga   211
Pro Val Thr Asp Ala Val Ala His Leu Arg Ser Phe Val Leu Asn Gly
            25                  30                  35

gga aag cga atc cgt cct ctt tat gcg tgg gct ggt ttc ctg gcg gcg   259
Gly Lys Arg Ile Arg Pro Leu Tyr Ala Trp Ala Gly Phe Leu Ala Ala
        40                  45                  50

caa ggc cat aag aat tct tct gaa aaa ctt gag tcc gtc ctt gac gcc   307
Gln Gly His Lys Asn Ser Ser Glu Lys Leu Glu Ser Val Leu Asp Ala
    55                  60                  65

gca gcg agt ctc gaa ttc atc cag gct tgt gcc ttg att cat gac gat   355
Ala Ala Ser Leu Glu Phe Ile Gln Ala Cys Ala Leu Ile His Asp Asp
70                  75                  80                  85

att atc gat tct tct gat acc cgg cgc gga gcc ccc aca gtt cac cgg 403
Ile Ile Asp Ser Ser Asp Thr Arg Arg Gly Ala Pro Thr Val His Arg
                90                  95                  100

gct gtg gaa gct gat cac cgc gcc aat aat ttc gaa ggc gat ccc gag 451
Ala Val Glu Ala Asp His Arg Ala Asn Asn Phe Glu Gly Asp Pro Glu
            105                 110                 115

cac ttt ggc gtt tca gtc tcg att ttg gct ggc gat atg gca ttg gtg 499
His Phe Gly Val Ser Val Ser Ile Leu Ala Gly Asp Met Ala Leu Val
        120                 125                 130

tgg gca gaa gac atg ctg cag gat tcc ggt ttg agt gca gag gca ttg 547
Trp Ala Glu Asp Met Leu Gln Asp Ser Gly Leu Ser Ala Glu Ala Leu
    135                 140                 145

gcc cgc acg agg gat gct tgg cgt ggc atg cgt act gag gtt att ggc 595
Ala Arg Thr Arg Asp Ala Trp Arg Gly Met Arg Thr Glu Val Ile Gly
150                 155                 160                 165

ggc cag ctg ctt gat att tat cta gag tcg cac gcc aac gag tcg gtg 643
Gly Gln Leu Leu Asp Ile Tyr Leu Glu Ser His Ala Asn Glu Ser Val
                170                 175                 180

gag ctt gcg gat tct gtc aac cgc ttc aaa acg gcc gct tac acg att 691
Glu Leu Ala Asp Ser Val Asn Arg Phe Lys Thr Ala Ala Tyr Thr Ile
            185                 190                 195

gcg cgc cca ttg cac ctg ggc gcc tcc att gct ggc ggt tcg ccg cag 739
Ala Arg Pro Leu His Leu Gly Ala Ser Ile Ala Gly Gly Ser Pro Gln
        200                 205                 210

ctt atc gac gcg ctc ctc cac tac ggc cac gac atc ggc att gca ttc 787
Leu Ile Asp Ala Leu Leu His Tyr Gly His Asp Ile Gly Ile Ala Phe
    215                 220                 225

cag ttg agg gat gat ctg ctt ggt gtg ttt ggg gat ccc gct atc acc 835
Gln Leu Arg Asp Asp Leu Leu Gly Val Phe Gly Asp Pro Ala Ile Thr
230                 235                 240                 245

ggc aaa cca gct gga gac gat atc cgt gaa ggc aag cgc act gtt ctt 883
Gly Lys Pro Ala Gly Asp Asp Ile Arg Glu Gly Lys Arg Thr Val Leu
                250                 255                 260

ctt gcg ctc gct cta caa cgc gct gat aag caa tct cct gaa gct gca 931
Leu Ala Leu Ala Leu Gln Arg Ala Asp Lys Gln Ser Pro Glu Ala Ala
            265                 270                 275

acg gcc att cgc gca ggt gtt gga aag gtg act tca cca gaa gat att 979
Thr Ala Ile Arg Ala Gly Val Gly Lys Val Thr Ser Pro Glu Asp Ile
        280                 285                 290

gct gtc att aca gag cat att cga gct act ggt gct gaa gaa gaa gtt 1027
Ala Val Ile Thr Glu His Ile Arg Ala Thr Gly Ala Glu Glu Glu Val
    295                 300                 305

gag cag cga att tcc cag ctg act gaa tcc ggt ttg gct cac ctc gat 1075
Glu Gln Arg Ile Ser Gln Leu Thr Glu Ser Gly Leu Ala His Leu Asp
310                 315                 320                 325

gat gta gac atc ccc gat gag gtg cgc gca cag ttg cgg gca ctg gct     1123
Asp Val Asp Ile Pro Asp Glu Val Arg Ala Gln Leu Arg Ala Leu Ala
                330                 335                 340

atc cgc tca acc gaa cgt cgg atg tagtagacgc gtatgacact ttt          1170
Ile Arg Ser Thr Glu Arg Arg Met
            345

<210> 248
<211> 349
<212> PRT
<213> Corynebacterium glutamicum

<400> 248
Val Arg Asp Arg Leu Thr Gln Phe Leu Asp Ala Gln Glu Leu Thr Ile
  1               5                  10                  15

Ala Asp Ile Gly Ala Pro Val Thr Asp Ala Val Ala His Leu Arg Ser
             20                  25                  30

Phe Val Leu Asn Gly Gly Lys Arg Ile Arg Pro Leu Tyr Ala Trp Ala
         35                  40                  45

Gly Phe Leu Ala Ala Gln Gly His Lys Asn Ser Ser Glu Lys Leu Glu
     50                  55                  60

Ser Val Leu Asp Ala Ala Ala Ser Leu Glu Phe Ile Gln Ala Cys Ala
 65                  70                  75                  80

Leu Ile His Asp Asp Ile Ile Asp Ser Ser Asp Thr Arg Arg Gly Ala
                 85                  90                  95

Pro Thr Val His Arg Ala Val Glu Ala Asp His Arg Ala Asn Asn Phe
            100                 105                 110

Glu Gly Asp Pro Glu His Phe Gly Val Ser Val Ser Ile Leu Ala Gly
        115                 120                 125

Asp Met Ala Leu Val Trp Ala Glu Asp Met Leu Gln Asp Ser Gly Leu
    130                 135                 140

Ser Ala Glu Ala Leu Ala Arg Thr Arg Asp Ala Trp Arg Gly Met Arg
145                 150                 155                 160

Thr Glu Val Ile Gly Gly Gln Leu Leu Asp Ile Tyr Leu Glu Ser His
                165                 170                 175

Ala Asn Glu Ser Val Glu Leu Ala Asp Ser Val Asn Arg Phe Lys Thr
            180                 185                 190

Ala Ala Tyr Thr Ile Ala Arg Pro Leu His Leu Gly Ala Ser Ile Ala
        195                 200                 205

Gly Gly Ser Pro Gln Leu Ile Asp Ala Leu Leu His Tyr Gly His Asp
    210                 215                 220

Ile Gly Ile Ala Phe Gln Leu Arg Asp Asp Leu Leu Gly Val Phe Gly
225                 230                 235                 240

Asp Pro Ala Ile Thr Gly Lys Pro Ala Gly Asp Asp Ile Arg Glu Gly

245                 250                 255

Lys Arg Thr Val Leu Leu Ala Leu Ala Leu Gln Arg Ala Asp Lys Gln
            260                 265                 270

Ser Pro Glu Ala Ala Thr Ala Ile Arg Ala Gly Val Gly Lys Val Thr
        275                 280                 285

Ser Pro Glu Asp Ile Ala Val Ile Thr Glu His Ile Arg Ala Thr Gly
    290                 295                 300

Ala Glu Glu Glu Val Glu Gln Arg Ile Ser Gln Leu Thr Glu Ser Gly
305                 310                 315                 320

Leu Ala His Leu Asp Asp Val Asp Ile Pro Asp Glu Val Arg Ala Gln
                325                 330                 335

Leu Arg Ala Leu Ala Ile Arg Ser Thr Glu Arg Arg Met
            340                 345

<210> 249
<211> 852
<212> DNA
<213> Corynebacterium glutamicum

<220>
<221> CDS
<222> (101)..(829)
<223> RXA01067

<400> 249
ggctttaagg ccgaggtgcc tggctgaact gcagcccctc cagaccctga gatcaggcta 60

aatggcaaac acaaatagtt tgcatggcag aatagcttag gtg agt gaa ttc caa    115
                                            Val Ser Glu Phe Gln
                                            1               5

gta ccc gaa atc cct gcc caa ttc cta ccc aag cat att gcg ctt gtc    163
Val Pro Glu Ile Pro Ala Gln Phe Leu Pro Lys His Ile Ala Leu Val
                10                  15                  20

atg gat gga aat gga cgc tgg gct acc gag cgt ggc atg aag cgc acc    211
Met Asp Gly Asn Gly Arg Trp Ala Thr Glu Arg Gly Met Lys Arg Thr
            25                  30                  35

gaa ggc cat aag cgt ggc gag gca gtc ctg ctt gat gtt gtt gat gca    259
Glu Gly His Lys Arg Gly Glu Ala Val Leu Leu Asp Val Val Asp Ala
        40                  45                  50

tgc att gaa ctt ggt gtt ccg tac ctt tct gct tat gcc ttc tct act    307
Cys Ile Glu Leu Gly Val Pro Tyr Leu Ser Ala Tyr Ala Phe Ser Thr
    55                  60                  65

gaa aac tgg cgt cgt tcc acc gat gag gtc cgt ttc ctc atg gga ttc    355
Glu Asn Trp Arg Arg Ser Thr Asp Glu Val Arg Phe Leu Met Gly Phe
70                  75                  80                  85

aac cga gat gtg ctg cga cga caa cgc gat gac cta cat gaa aag ggc    403
Asn Arg Asp Val Leu Arg Arg Gln Arg Asp Asp Leu His Glu Lys Gly
                90                  95                  100

gtt cgt gtg cgt tgg gtt ggc cgt cgt ccc cgc ctg tgg cgt tcg gtt     451
Val Arg Val Arg Trp Val Gly Arg Arg Pro Arg Leu Trp Arg Ser Val
            105                 110                 115

atc cgt gag ctg gaa act gcg gaa gag cta acc aaa gac aac acc acc     499
Ile Arg Glu Leu Glu Thr Ala Glu Glu Leu Thr Lys Asp Asn Thr Thr
        120                 125                 130

atg acc ttg gcc atg tgt gtg aac tat ggt gga cgc gcc gaa atc atc     547
Met Thr Leu Ala Met Cys Val Asn Tyr Gly Gly Arg Ala Glu Ile Ile
    135                 140                 145

gac gca gcc cgc gac atc gcc cgc ctt gca gct gaa ggc aaa ctg cgc     595
Asp Ala Ala Arg Asp Ile Ala Arg Leu Ala Ala Glu Gly Lys Leu Arg
150                 155                 160                 165

ccg gaa caa atc act gag aag acc ttc ccg aac ttc ctc gac gaa cct     643
Pro Glu Gln Ile Thr Glu Lys Thr Phe Pro Asn Phe Leu Asp Glu Pro
                170                 175                 180

gac atg cca gac gtc gac ctg ttc ctg cgc cca tcc ggt gag aag cgc     691
Asp Met Pro Asp Val Asp Leu Phe Leu Arg Pro Ser Gly Glu Lys Arg
            185                 190                 195

acg tca aac ttc ctg ctg tgg cag tct gcc tac gcg gaa atg gtc tac     739
Thr Ser Asn Phe Leu Leu Trp Gln Ser Ala Tyr Ala Glu Met Val Tyr
        200                 205                 210

caa gac aag ctg ttc cct gat ttc acg cag caa gat ctg tac gac gcg     787
Gln Asp Lys Leu Phe Pro Asp Phe Thr Gln Gln Asp Leu Tyr Asp Ala
    215                 220                 225

gtc ctg gaa tac gcc aag cgg gat cgc aga ttc gga agc gca             829
Val Leu Glu Tyr Ala Lys Arg Asp Arg Arg Phe Gly Ser Ala
230                 235                 240

taatgcccac caaccagccg acg                                           852


<210> 250
<211> 243
<212> PRT
<213> Corynebacterium glutamicum

<400> 250
Val Ser Glu Phe Gln Val Pro Glu Ile Pro Ala Gln Phe Leu Pro Lys
1               5                   10                  15

His Ile Ala Leu Val Met Asp Gly Asn Gly Arg Trp Ala Thr Glu Arg
            20                  25                  30

Gly Met Lys Arg Thr Glu Gly His Lys Arg Gly Glu Ala Val Leu Leu
        35                  40                  45

Asp Val Val Asp Ala Cys Ile Glu Leu Gly Val Pro Tyr Leu Ser Ala
    50                  55                  60

Tyr Ala Phe Ser Thr Glu Asn Trp Arg Arg Ser Thr Asp Glu Val Arg
65                  70                  75                  80

Phe Leu Met Gly Phe Asn Arg Asp Val Leu Arg Arg Gln Arg Asp Asp
                85                  90                  95

Leu His Glu Lys Gly Val Arg Val Arg Trp Val Gly Arg Arg Pro Arg
            100                 105                 110

Leu Trp Arg Ser Val Ile Arg Glu Leu Glu Thr Ala Glu Glu Leu Thr
        115                 120                 125

Lys Asp Asn Thr Thr Met Thr Leu Ala Met Cys Val Asn Tyr Gly Gly
    130                 135                 140

Arg Ala Glu Ile Ile Asp Ala Ala Arg Asp Ile Ala Arg Leu Ala Ala
145                 150                 155                 160

Glu Gly Lys Leu Arg Pro Glu Gln Ile Thr Glu Lys Thr Phe Pro Asn
                165                 170                 175

Phe Leu Asp Glu Pro Asp Met Pro Asp Val Asp Leu Phe Leu Arg Pro
            180                 185                 190

Ser Gly Glu Lys Arg Thr Ser Asn Phe Leu Leu Trp Gln Ser Ala Tyr
        195                 200                 205

Ala Glu Met Val Tyr Gln Asp Lys Leu Phe Pro Asp Phe Thr Gln Gln
    210                 215                 220

Asp Leu Tyr Asp Ala Val Leu Glu Tyr Ala Lys Arg Asp Arg Arg Phe
225                 230                 235                 240

Gly Ser Ala

<210> 251
<211> 564
<212> DNA
<213> Corynebacterium glutamicum

<220>
<221> CDS
<222> (101)..(541)
<223> RXA01269

<400> 251
gattatctct gcgccgattc agctggcaat agcagcagtt gtattgagag ctcatggtcg 60

tccgattcta tttcgtcaac cacgacctgg gaaagacggt gtg gta ttt gag atg   115
                                          Val Val Phe Glu Met
                                          1               5

att aag ttt cga acc atg ctt gaa cca gat gaa aaa cat gta act gat   163
Ile Lys Phe Arg Thr Met Leu Glu Pro Asp Glu Lys His Val Thr Asp
                10                  15                  20

gaa cag cgt cta act aaa gtt gga aag ctt ctg cgg gaa acg agt tta   211
Glu Gln Arg Leu Thr Lys Val Gly Lys Leu Leu Arg Glu Thr Ser Leu
            25                  30                  35

gat gag tta cct aca ctc tgg aat gta ttt aaa ggt gat atg agc ctt   259
Asp Glu Leu Pro Thr Leu Trp Asn Val Phe Lys Gly Asp Met Ser Leu

40                  45                  50

gta ggg cct cga cct ttg ctt gtt agc tat ctg gaa cat tac tct tct     307
Val Gly Pro Arg Pro Leu Leu Val Ser Tyr Leu Glu His Tyr Ser Ser
    55                  60                  65

gaa caa gct cga cgc cat gaa gtt cgt cct ggg att act ggt ttg gct     355
Glu Gln Ala Arg Arg His Glu Val Arg Pro Gly Ile Thr Gly Leu Ala
70                  75                  80                  85

cag gtg aat ggc cgt aat caa act act tgg gat gaa cga ctt aag ttg     403
Gln Val Asn Gly Arg Asn Gln Thr Thr Trp Asp Glu Arg Leu Lys Leu
                90                  95                  100

gat gtc gaa tat gtg gat cgc tgt agt ttg aaa cta gat ttc aaa ata     451
Asp Val Glu Tyr Val Asp Arg Cys Ser Leu Lys Leu Asp Phe Lys Ile
            105                 110                 115

tta atc gcc act gta aaa aca gtt ctt tct aaa aag ggc att agt aat     499
Leu Ile Ala Thr Val Lys Thr Val Leu Ser Lys Lys Gly Ile Ser Asn
        120                 125                 130

gaa ggt cat gtc acg atg cca tcc ttc att gaa gaa aga aaa             541
Glu Gly His Val Thr Met Pro Ser Phe Ile Glu Glu Arg Lys
    135                 140                 145

tagcaggtaa aaattttact ttc                                           564


<210> 252
<211> 147
<212> PRT
<213> Corynebacterium glutamicum

<400> 252
Val Val Phe Glu Met Ile Lys Phe Arg Thr Met Leu Glu Pro Asp Glu
1               5                   10                  15

Lys His Val Thr Asp Glu Gln Arg Leu Thr Lys Val Gly Lys Leu Leu
            20                  25                  30

Arg Glu Thr Ser Leu Asp Glu Leu Pro Thr Leu Trp Asn Val Phe Lys
        35                  40                  45

Gly Asp Met Ser Leu Val Gly Pro Arg Pro Leu Leu Val Ser Tyr Leu
    50                  55                  60

Glu His Tyr Ser Ser Glu Gln Ala Arg Arg His Glu Val Arg Pro Gly
65                  70                  75                  80

Ile Thr Gly Leu Ala Gln Val Asn Gly Arg Asn Gln Thr Thr Trp Asp
                85                  90                  95

Glu Arg Leu Lys Leu Asp Val Glu Tyr Val Asp Arg Cys Ser Leu Lys
            100                 105                 110

Leu Asp Phe Lys Ile Leu Ile Ala Thr Val Lys Thr Val Leu Ser Lys
        115                 120                 125

Lys Gly Ile Ser Asn Glu Gly His Val Thr Met Pro Ser Phe Ile Glu
    130                 135                 140

Glu Arg Lys
145

<210> 253
<211> 554
<212> DNA
<213> Corynebacterium glutamicum

<220>
<221> CDS
<222> (1)..(531)
<223> RXA01205

<400> 253
gta tcg gcc tat ccg ccg gcc atc atc gca gcg gct ctc gta gga att 48
Val Ser Ala Tyr Pro Pro Ala Ile Ile Ala Ala Ala Leu Val Gly Ile
1               5                   10                  15

tgc gcg gga gtt ttg ccc cat aat ttt gaa ccc tcg cga ata ttt atg 96
Cys Ala Gly Val Leu Pro His Asn Phe Glu Pro Ser Arg Ile Phe Met
            20                  25                  30

ggc gat tcc ggc tcc atg ctc atc ggc ctg ctg ttg gct gca gca tcg 144
Gly Asp Ser Gly Ser Met Leu Ile Gly Leu Leu Leu Ala Ala Ala Ser
        35                  40                  45

acc tca gcg tca gga aaa atc aac atg agc ctg tat ggc gca gct gat 192
Thr Ser Ala Ser Gly Lys Ile Asn Met Ser Leu Tyr Gly Ala Ala Asp
    50                  55                  60

ttt atc gca ttg atc tca ccc atc atc gtt gtt ctc gcc gcc gtg gcc 240
Phe Ile Ala Leu Ile Ser Pro Ile Ile Val Val Leu Ala Ala Val Ala
65                  70                  75                  80

atc cca ctg ctc gac ctc gtg atg gca gtg gtt agg cgc gtg ggc agg 288
Ile Pro Leu Leu Asp Leu Val Met Ala Val Val Arg Arg Val Gly Arg
                85                  90                  95

gga gca tca ccc ttt tcc ccg gac aaa atg cat ctg cac cac cga ctg 336
Gly Ala Ser Pro Phe Ser Pro Asp Lys Met His Leu His His Arg Leu
            100                 105                 110

ctg tcc atc gga cac acc cat agg cgc gtg gtc cta gtg ctc tac acc 384
Leu Ser Ile Gly His Thr His Arg Arg Val Val Leu Val Leu Tyr Thr
        115                 120                 125

tgg gcg agc gcc gtg gca ttc ggc gca gtg agc ttc tcc gtc gtt ccg 432
Trp Ala Ser Ala Val Ala Phe Gly Ala Val Ser Phe Ser Val Val Pro
    130                 135                 140

cca ctg ttt gcc acc gga tcg agc atc tgt ggc atc ctc atc gcc gtc 480
Pro Leu Phe Ala Thr Gly Ser Ser Ile Cys Gly Ile Leu Ile Ala Val
145                 150                 155                 160

gct gtc aca gcc gtg cca gtg atg aaa agc cgg cga gcc gcc aaa ctt 528
Ala Val Thr Ala Val Pro Val Met Lys Ser Arg Arg Ala Ala Lys Leu
                165                 170                 175

gat taagtgattg tcactttgga ttg 554

Asp

<210> 254
<211> 177
<212> PRT
<213> Corynebacterium glutamicum

<400> 254
Val Ser Ala Tyr Pro Pro Ala Ile Ile Ala Ala Ala Leu Val Gly Ile
1               5                   10                  15

Cys Ala Gly Val Leu Pro His Asn Phe Glu Pro Ser Arg Ile Phe Met
            20                  25                  30

Gly Asp Ser Gly Ser Met Leu Ile Gly Leu Leu Leu Ala Ala Ala Ser
        35                  40                  45

Thr Ser Ala Ser Gly Lys Ile Asn Met Ser Leu Tyr Gly Ala Ala Asp
    50                  55                  60

Phe Ile Ala Leu Ile Ser Pro Ile Ile Val Val Leu Ala Ala Val Ala
65                  70                  75                  80

Ile Pro Leu Leu Asp Leu Val Met Ala Val Val Arg Arg Val Gly Arg
                85                  90                  95

Gly Ala Ser Pro Phe Ser Pro Asp Lys Met His Leu His His Arg Leu
            100                 105                 110

Leu Ser Ile Gly His Thr His Arg Arg Val Val Leu Val Leu Tyr Thr
        115                 120                 125

Trp Ala Ser Ala Val Ala Phe Gly Ala Val Ser Phe Ser Val Val Pro
    130                 135                 140

Pro Leu Phe Ala Thr Gly Ser Ser Ile Cys Gly Ile Leu Ile Ala Val
145                 150                 155                 160

Ala Val Thr Ala Val Pro Val Met Lys Ser Arg Arg Ala Ala Lys Leu
                165                 170                 175

Asp

<210> 255
<211> 882
<212> DNA
<213> Corynebacterium glutamicum

<220>
<221> CDS
<222> (101)..(859)
<223> RXA01576

<400> 255
atcggtgatc tttcgcgatt gatacgtgcg cgattgcgtg cccgccagct gctcaagatc 60

aatggatttc atgacaatca agttatcatc actggggtcg gtg gca cct aac agg   115

Val Ala Pro Asn Arg
1               5

aaa atc agc cat cat gca cta ggc tct att cct atc atg gat gca tca     163
Lys Ile Ser His His Ala Leu Gly Ser Ile Pro Ile Met Asp Ala Ser
                10                  15                  20

aaa aac agc gac ttt aaa gac acc tgg cta gtc gta cct tgt tat aac     211
Lys Asn Ser Asp Phe Lys Asp Thr Trp Leu Val Val Pro Cys Tyr Asn
            25                  30                  35

gag gca aca gtt atc cgg gaa gtt tta gag aac gca ctc aaa aca ttc     259
Glu Ala Thr Val Ile Arg Glu Val Leu Glu Asn Ala Leu Lys Thr Phe
        40                  45                  50

cct aat att gtt gcg gtc aac gat ggc tcc ccg gac aac tcc gcg gaa     307
Pro Asn Ile Val Ala Val Asn Asp Gly Ser Pro Asp Asn Ser Ala Glu
    55                  60                  65

gaa atc cat gcg gcc ggc gca cac ctg gtc aac cac ccc gtg aac ctc     355
Glu Ile His Ala Ala Gly Ala His Leu Val Asn His Pro Val Asn Leu
70                  75                  80                  85

gga caa ggt gcc gcg atc caa acc ggc atc gaa tac gcc cgc aag caa     403
Gly Gln Gly Ala Ala Ile Gln Thr Gly Ile Glu Tyr Ala Arg Lys Gln
                90                  95                  100

ccc ggt gca aaa tac ttt gta act ttc gac gcc gac ggc caa cac caa     451
Pro Gly Ala Lys Tyr Phe Val Thr Phe Asp Ala Asp Gly Gln His Gln
            105                 110                 115

gtg aaa gac gtt atc cgc atg gtg gag cga ctg cgt gcc gag gac gtg     499
Val Lys Asp Val Ile Arg Met Val Glu Arg Leu Arg Ala Glu Asp Val
        120                 125                 130

gac att atc gtc ggc acg cgt ttt gga cgc cca cgc caa gcc gat gac     547
Asp Ile Ile Val Gly Thr Arg Phe Gly Arg Pro Arg Gln Ala Asp Asp
    135                 140                 145

cag gtg cca cta atc aag cgc ctt gtg ctg cgc acc gtg gtc ctg ctg     595
Gln Val Pro Leu Ile Lys Arg Leu Val Leu Arg Thr Val Val Leu Leu
150                 155                 160                 165

tca ccg aaa acc cgc cga ctt ggg ctc acc gac gcc cac aac ggc ctg     643
Ser Pro Lys Thr Arg Arg Leu Gly Leu Thr Asp Ala His Asn Gly Leu
                170                 175                 180

cgc gta ttc aac caa aaa gtg gcg cag gaa atg aac atc cgc atg aac     691
Arg Val Phe Asn Gln Lys Val Ala Gln Glu Met Asn Ile Arg Met Asn
            185                 190                 195

ggc atg tcg cat gca tcc gaa atc gtc gac caa atc gat gaa cgc ggc     739
Gly Met Ser His Ala Ser Glu Ile Val Asp Gln Ile Asp Glu Arg Gly
        200                 205                 210

tgg cgc att tca gaa gaa ccc gta gac atc ctc tac acc gaa tac tcc     787
Trp Arg Ile Ser Glu Glu Pro Val Asp Ile Leu Tyr Thr Glu Tyr Ser
    215                 220                 225

atg agc aag ggg caa tcc ctg ctc aac ggc gta aac atc ctg gcc gac     835
Met Ser Lys Gly Gln Ser Leu Leu Asn Gly Val Asn Ile Leu Ala Asp

230                 235                 240                 245

ggc ttc cta gcg agg aga ctc cca tgacgcagac caccacccaa atc          882
Gly Phe Leu Ala Arg Arg Leu Pro
                250

<210> 256
<211> 253
<212> PRT
<213> Corynebacterium glutamicum

<400> 256
Val Ala Pro Asn Arg Lys Ile Ser His His Ala Leu Gly Ser Ile Pro
1               5                   10                  15

Ile Met Asp Ala Ser Lys Asn Ser Asp Phe Lys Asp Thr Trp Leu Val
            20                  25                  30

Val Pro Cys Tyr Asn Glu Ala Thr Val Ile Arg Glu Val Leu Glu Asn
        35                  40                  45

Ala Leu Lys Thr Phe Pro Asn Ile Val Ala Val Asn Asp Gly Ser Pro
    50                  55                  60

Asp Asn Ser Ala Glu Glu Ile His Ala Ala Gly Ala His Leu Val Asn
65                  70                  75                  80

His Pro Val Asn Leu Gly Gln Gly Ala Ala Ile Gln Thr Gly Ile Glu
                85                  90                  95

Tyr Ala Arg Lys Gln Pro Gly Ala Lys Tyr Phe Val Thr Phe Asp Ala
            100                 105                 110

Asp Gly Gln His Gln Val Lys Asp Val Ile Arg Met Val Glu Arg Leu
        115                 120                 125

Arg Ala Glu Asp Val Asp Ile Ile Val Gly Thr Arg Phe Gly Arg Pro
    130                 135                 140

Arg Gln Ala Asp Asp Gln Val Pro Leu Ile Lys Arg Leu Val Leu Arg
145                 150                 155                 160

Thr Val Val Leu Leu Ser Pro Lys Thr Arg Arg Leu Gly Leu Thr Asp
                165                 170                 175

Ala His Asn Gly Leu Arg Val Phe Asn Gln Lys Val Ala Gln Glu Met
            180                 185                 190

Asn Ile Arg Met Asn Gly Met Ser His Ala Ser Glu Ile Val Asp Gln
        195                 200                 205

Ile Asp Glu Arg Gly Trp Arg Ile Ser Glu Glu Pro Val Asp Ile Leu
    210                 215                 220

Tyr Thr Glu Tyr Ser Met Ser Lys Gly Gln Ser Leu Leu Asn Gly Val
225                 230                 235                 240

Asn Ile Leu Ala Asp Gly Phe Leu Ala Arg Arg Leu Pro
                245                 250

<210> 257
<211> 1173
<212> DNA
<213> Corynebacterium glutamicum

<220>
<221> CDS
<222> (101)..(1150)
<223> RXN02309

<400> 257
gtgccttccg tcgactacgg ttaaacaaaa agctttttgt ccatttcact ggattcaccg 60

aaagaatgaa tccacactcg atcaccaaag gtagcgatga atg agt agc ggc cga 115
Met Ser Ser Gly Arg
1               5

acc gtt cca acc cgt tcc cac ggg ctc gga aaa gaa ggt gta tcc acc 163
Thr Val Pro Thr Arg Ser His Gly Leu Gly Lys Glu Gly Val Ser Thr
                10                  15                  20

aca gga gca tct cag gtc gag ttt ggt gat ccc gag cta acg gcc agg 211
Thr Gly Ala Ser Gln Val Glu Phe Gly Asp Pro Glu Leu Thr Ala Arg
            25                  30                  35

atc aat gac gcc atg gtg cag gta gaa gaa ctc ctg cac act gaa cta 259
Ile Asn Asp Ala Met Val Gln Val Glu Glu Leu Leu His Thr Glu Leu
        40                  45                  50

tcg tcc ggg gaa gac ttc ctc gtc gat atc gtc atg cac cta aca cga 307
Ser Ser Gly Glu Asp Phe Leu Val Asp Ile Val Met His Leu Thr Arg
    55                  60                  65

gcc ggc ggc aaa cga ttc cgc ccc atg ttt gca ctg ctg gcc tcc gag 355
Ala Gly Gly Lys Arg Phe Arg Pro Met Phe Ala Leu Leu Ala Ser Glu
70                  75                  80                  85

ttc ggt gaa aaa cca ctc tcc gaa aac gtc atc aaa gcc gcc gtt gtc 403
Phe Gly Glu Lys Pro Leu Ser Glu Asn Val Ile Lys Ala Ala Val Val
                90                  95                  100

gta gag atc acc cac ctg gcc acc ctg tac cac gac gat gtc atg gac 451
Val Glu Ile Thr His Leu Ala Thr Leu Tyr His Asp Asp Val Met Asp
            105                 110                 115

gag gca tcc atg cgc cgc ggc gtc cca agt gct aac gcg cga tgg gac 499
Glu Ala Ser Met Arg Arg Gly Val Pro Ser Ala Asn Ala Arg Trp Asp
        120                 125                 130

aac tcc gta gcc atc ctc gca ggc gac atc ctc cta gca cat gca tca 547
Asn Ser Val Ala Ile Leu Ala Gly Asp Ile Leu Leu Ala His Ala Ser
    135                 140                 145

ggt ctg atg agt cag ctg ggt acc gac aca gtc gcc cac ttt gcc gaa 595
Gly Leu Met Ser Gln Leu Gly Thr Asp Thr Val Ala His Phe Ala Glu
150                 155                 160                 165

aca ttc ggc gaa cta gtc acc ggc caa atg cgc gaa aca gtc ggg cca 643
Thr Phe Gly Glu Leu Val Thr Gly Gln Met Arg Glu Thr Val Gly Pro
                170                 175                 180

cgc gac acc gac ccg atc gag cac tac acc aac gta atc cgt gaa aaa    691
Arg Asp Thr Asp Pro Ile Glu His Tyr Thr Asn Val Ile Arg Glu Lys
            185                 190                 195

act ggt gtc ctc atc gcc tcc gca ggc tat ttg gga gcc atg cac gca    739
Thr Gly Val Leu Ile Ala Ser Ala Gly Tyr Leu Gly Ala Met His Ala
        200                 205                 210

ggc gcc gca cct gaa cac atc gac gcc ctg aag aac ttc ggc gca gcc    787
Gly Ala Ala Pro Glu His Ile Asp Ala Leu Lys Asn Phe Gly Ala Ala
    215                 220                 225

gtc ggc atg atc ttc caa atc gtc gac gac atc atc gac atc ttc tcg    835
Val Gly Met Ile Phe Gln Ile Val Asp Asp Ile Ile Asp Ile Phe Ser
230                 235                 240                 245

gaa acc cac gaa tcc gga aaa acg ccc ggc acc gac ctc cgc gaa ggt    883
Glu Thr His Glu Ser Gly Lys Thr Pro Gly Thr Asp Leu Arg Glu Gly
                250                 255                 260

gta ttc acc ctc cca gtg ctc tac gca ctc cgt gaa gac acc ccc gtc    931
Val Phe Thr Leu Pro Val Leu Tyr Ala Leu Arg Glu Asp Thr Pro Val
            265                 270                 275

ggc gca gaa ctc cgc gac atc ctc acc ggc cct cta gaa gac gac gag    979
Gly Ala Glu Leu Arg Asp Ile Leu Thr Gly Pro Leu Glu Asp Asp Glu
        280                 285                 290

acc gtc aac cac gtc ctc gag ctc ctc tcc caa tcc ggc gga cgc caa    1027
Thr Val Asn His Val Leu Glu Leu Leu Ser Gln Ser Gly Gly Arg Gln
    295                 300                 305

gca gcc ctc gac gag gtc tac cgc tac atg gac atc gcc aac gca gaa    1075
Ala Ala Leu Asp Glu Val Tyr Arg Tyr Met Asp Ile Ala Asn Ala Glu
310                 315                 320                 325

ctc gac cgc ctc cca gac agc acc gtc aag gaa gcc ctc cgc aac ctt    1123
Leu Asp Arg Leu Pro Asp Ser Thr Val Lys Glu Ala Leu Arg Asn Leu
                330                 335                 340

gca acc ttc aca gtc aag cgc gtc gga taaccccgta atccaccact          1170
Ala Thr Phe Thr Val Lys Arg Val Gly
            345                 350

taa                                                                1173

<210> 258
<211> 350
<212> PRT
<213> Corynebacterium glutamicum

<400> 258
Met Ser Ser Gly Arg Thr Val Pro Thr Arg Ser His Gly Leu Gly Lys
1               5                   10                  15

Glu Gly Val Ser Thr Thr Gly Ala Ser Gln Val Glu Phe Gly Asp Pro
            20                  25                  30

Glu Leu Thr Ala Arg Ile Asn Asp Ala Met Val Gln Val Glu Glu Leu

        35                  40                  45

Leu His Thr Glu Leu Ser Ser Gly Glu Asp Phe Leu Val Asp Ile Val
    50                  55                  60

Met His Leu Thr Arg Ala Gly Gly Lys Arg Phe Arg Pro Met Phe Ala
65                  70                  75                  80

Leu Leu Ala Ser Glu Phe Gly Glu Lys Pro Leu Ser Glu Asn Val Ile
                85                  90                  95

Lys Ala Ala Val Val Val Glu Ile Thr His Leu Ala Thr Leu Tyr His
            100                 105                 110

Asp Asp Val Met Asp Glu Ala Ser Met Arg Arg Gly Val Pro Ser Ala
        115                 120                 125

Asn Ala Arg Trp Asp Asn Ser Val Ala Ile Leu Ala Gly Asp Ile Leu
    130                 135                 140

Leu Ala His Ala Ser Gly Leu Met Ser Gln Leu Gly Thr Asp Thr Val
145                 150                 155                 160

Ala His Phe Ala Glu Thr Phe Gly Glu Leu Val Thr Gly Gln Met Arg
                165                 170                 175

Glu Thr Val Gly Pro Arg Asp Thr Asp Pro Ile Glu His Tyr Thr Asn
            180                 185                 190

Val Ile Arg Glu Lys Thr Gly Val Leu Ile Ala Ser Ala Gly Tyr Leu
        195                 200                 205

Gly Ala Met His Ala Gly Ala Ala Pro Glu His Ile Asp Ala Leu Lys
    210                 215                 220

Asn Phe Gly Ala Ala Val Gly Met Ile Phe Gln Ile Val Asp Asp Ile
225                 230                 235                 240

Ile Asp Ile Phe Ser Glu Thr His Glu Ser Gly Lys Thr Pro Gly Thr
                245                 250                 255

Asp Leu Arg Glu Gly Val Phe Thr Leu Pro Val Leu Tyr Ala Leu Arg
            260                 265                 270

Glu Asp Thr Pro Val Gly Ala Glu Leu Arg Asp Ile Leu Thr Gly Pro
        275                 280                 285

Leu Glu Asp Asp Glu Thr Val Asn His Val Leu Glu Leu Leu Ser Gln
    290                 295                 300

Ser Gly Gly Arg Gln Ala Ala Leu Asp Glu Val Tyr Arg Tyr Met Asp
305                 310                 315                 320

Ile Ala Asn Ala Glu Leu Asp Arg Leu Pro Asp Ser Thr Val Lys Glu
                325                 330                 335

Ala Leu Arg Asn Leu Ala Thr Phe Thr Val Lys Arg Val Gly
            340                 345                 350

<210> 259

<211> 1075
<212> DNA
<213> Corynebacterium glutamicum

<220>
<221> CDS
<222> (101)..(1075)
<223> FRXA02309

<400> 259
gtgccttccg tcgactacgg ttaaacaaaa agctttttgt ccatttcact ggattcaccg 60

aaagaatgaa tccacactcg atcaccaaag gtagcgatga atg agt agc ggc cga 115
Met Ser Ser Gly Arg
1               5

acc gtt cca acc cgt tcc cac ggg ctc gga aaa gaa ggt gta tcc acc 163
Thr Val Pro Thr Arg Ser His Gly Leu Gly Lys Glu Gly Val Ser Thr
                10                  15                  20

aca gga gca tct cag gtc gag ttt ggt gat ccc gag cta acg gcc agg 211
Thr Gly Ala Ser Gln Val Glu Phe Gly Asp Pro Glu Leu Thr Ala Arg
            25                  30                  35

atc aat gac gcc atg gtg cag gta gaa gaa ctc ctg cac act gaa cta 259
Ile Asn Asp Ala Met Val Gln Val Glu Glu Leu Leu His Thr Glu Leu
        40                  45                  50

tcg tcc ggg gaa gac ttc ctc gtc gat atc gtc atg cac cta aca cga 307
Ser Ser Gly Glu Asp Phe Leu Val Asp Ile Val Met His Leu Thr Arg
    55                  60                  65

gcc ggc ggc aaa cga ttc cgc ccc atg ttt gca ctg ctg gcc tcc gag 355
Ala Gly Gly Lys Arg Phe Arg Pro Met Phe Ala Leu Leu Ala Ser Glu
70                  75                  80                  85

ttc ggt gaa aaa cca ctc tcc gaa aac gtc atc aaa gcc gcc gtt gtc 403
Phe Gly Glu Lys Pro Leu Ser Glu Asn Val Ile Lys Ala Ala Val Val
                90                  95                  100

gta gag atc acc cac ctg gcc acc ctg tac cac gac gat gtc atg gac 451
Val Glu Ile Thr His Leu Ala Thr Leu Tyr His Asp Asp Val Met Asp
            105                 110                 115

gag gca tcc atg cgc cgc ggc gtc cca agt gct aac gcg cga tgg gac 499
Glu Ala Ser Met Arg Arg Gly Val Pro Ser Ala Asn Ala Arg Trp Asp
        120                 125                 130

aac tcc gta gcc atc ctc gca ggc gac atc ctc cta gca cat gca tca 547
Asn Ser Val Ala Ile Leu Ala Gly Asp Ile Leu Leu Ala His Ala Ser
    135                 140                 145

ggt ctg atg agt cag ctg ggt acc gac aca gtc gcc cac ttt gcc gaa 595
Gly Leu Met Ser Gln Leu Gly Thr Asp Thr Val Ala His Phe Ala Glu
150                 155                 160                 165

aca ttc ggc gaa cta gtc acc ggc caa atg cgc gaa aca gtc ggg cca 643
Thr Phe Gly Glu Leu Val Thr Gly Gln Met Arg Glu Thr Val Gly Pro
                170                 175                 180

cgc gac acc gac ccg atc gag cac tac acc aac gta atc cgt gaa aaa 691

Arg Asp Thr Asp Pro Ile Glu His Tyr Thr Asn Val Ile Arg Glu Lys
            185                 190                 195

act ggt gtc ctc atc gcc tcc gca ggc tat ttg gga gcc atg cac gca     739
Thr Gly Val Leu Ile Ala Ser Ala Gly Tyr Leu Gly Ala Met His Ala
        200                 205                 210

ggc gcc gca cct gaa cac atc gac gcc ctg aag aac ttc ggc gca gcc     787
Gly Ala Ala Pro Glu His Ile Asp Ala Leu Lys Asn Phe Gly Ala Ala
    215                 220                 225

gtc ggc atg atc ttc caa atc gtc gac gac atc atc gac atc ttc tcg     835
Val Gly Met Ile Phe Gln Ile Val Asp Asp Ile Ile Asp Ile Phe Ser
230                 235                 240                 245

gaa acc cac gaa tcc gga aaa acg ccc ggc acc gac ctc cgc gaa ggt     883
Glu Thr His Glu Ser Gly Lys Thr Pro Gly Thr Asp Leu Arg Glu Gly
                250                 255                 260

gta ttc acc ctc cca gtg ctc tac gca ctc cgt gaa gac acc ccc gtc     931
Val Phe Thr Leu Pro Val Leu Tyr Ala Leu Arg Glu Asp Thr Pro Val
            265                 270                 275

ggc gca gaa ctc cgc gac atc ctc acc ggc cct cta gaa gac gac gag     979
Gly Ala Glu Leu Arg Asp Ile Leu Thr Gly Pro Leu Glu Asp Asp Glu
        280                 285                 290

acc gtc aac cac gtc ctc gag ctc ctc tcc caa tcc ggc gga cgc caa     1027
Thr Val Asn His Val Leu Glu Leu Leu Ser Gln Ser Gly Gly Arg Gln
    295                 300                 305

gca gcc ctc gac gag gtc tac cgc tac atg gac atc gcc aac gca gaa     1075
Ala Ala Leu Asp Glu Val Tyr Arg Tyr Met Asp Ile Ala Asn Ala Glu
310                 315                 320                 325

<210> 260
<211> 325
<212> PRT
<213> Corynebacterium glutamicum

<400> 260
Met Ser Ser Gly Arg Thr Val Pro Thr Arg Ser His Gly Leu Gly Lys
  1               5                  10                  15

Glu Gly Val Ser Thr Thr Gly Ala Ser Gln Val Glu Phe Gly Asp Pro
             20                  25                  30

Glu Leu Thr Ala Arg Ile Asn Asp Ala Met Val Gln Val Glu Glu Leu
         35                  40                  45

Leu His Thr Glu Leu Ser Ser Gly Glu Asp Phe Leu Val Asp Ile Val
     50                  55                  60

Met His Leu Thr Arg Ala Gly Gly Lys Arg Phe Arg Pro Met Phe Ala
 65                  70                  75                  80

Leu Leu Ala Ser Glu Phe Gly Glu Lys Pro Leu Ser Glu Asn Val Ile
                 85                  90                  95

Lys Ala Ala Val Val Val Glu Ile Thr His Leu Ala Thr Leu Tyr His

                100                 105                 110

Asp Asp Val Met Asp Glu Ala Ser Met Arg Arg Gly Val Pro Ser Ala
        115                 120                 125

Asn Ala Arg Trp Asp Asn Ser Val Ala Ile Leu Ala Gly Asp Ile Leu
    130                 135                 140

Leu Ala His Ala Ser Gly Leu Met Ser Gln Leu Gly Thr Asp Thr Val
145                 150                 155                 160

Ala His Phe Ala Glu Thr Phe Gly Glu Leu Val Thr Gly Gln Met Arg
                165                 170                 175

Glu Thr Val Gly Pro Arg Asp Thr Asp Pro Ile Glu His Tyr Thr Asn
            180                 185                 190

Val Ile Arg Glu Lys Thr Gly Val Leu Ile Ala Ser Ala Gly Tyr Leu
        195                 200                 205

Gly Ala Met His Ala Gly Ala Ala Pro Glu His Ile Asp Ala Leu Lys
    210                 215                 220

Asn Phe Gly Ala Ala Val Gly Met Ile Phe Gln Ile Val Asp Asp Ile
225                 230                 235                 240

Ile Asp Ile Phe Ser Glu Thr His Glu Ser Gly Lys Thr Pro Gly Thr
                245                 250                 255

Asp Leu Arg Glu Gly Val Phe Thr Leu Pro Val Leu Tyr Ala Leu Arg
            260                 265                 270

Glu Asp Thr Pro Val Gly Ala Glu Leu Arg Asp Ile Leu Thr Gly Pro
        275                 280                 285

Leu Glu Asp Asp Glu Thr Val Asn His Val Leu Glu Leu Leu Ser Gln
    290                 295                 300

Ser Gly Gly Arg Gln Ala Ala Leu Asp Glu Val Tyr Arg Tyr Met Asp
305                 310                 315                 320

Ile Ala Asn Ala Glu
                325

<210> 261
<211> 1767
<212> DNA
<213> Corynebacterium glutamicum

<220>
<221> CDS
<222> (101)..(1744)
<223> RXN00477

<400> 261
tgcgggagcg aatcagagtt ccacttcata tcaaactctc tacactcgct agagccacga 60

tgaaaggtct atctatgagc atctacagaa agaattcgtg atg aag gtc tcg act   115
                                            Met Lys Val Ser Thr
                                            1               5

aaa act cca cgc tcc tca ggt acc gcc gta gtc ata ggc gca ggt gtt 163
Lys Thr Pro Arg Ser Ser Gly Thr Ala Val Val Ile Gly Ala Gly Val
                10                  15                  20

gct ggt tta gcc act tct gca ctt tta gca cgt gat ggc tgg caa gta 211
Ala Gly Leu Ala Thr Ser Ala Leu Leu Ala Arg Asp Gly Trp Gln Val
            25                  30                  35

act gtt ttg gaa aaa aat act gat gtc ggt ggc cga gct gga tcg ctt 259
Thr Val Leu Glu Lys Asn Thr Asp Val Gly Gly Arg Ala Gly Ser Leu
        40                  45                  50

gaa ata tca ggc ttt cct ggc ttt cga tgg gat acc gga cct tct tgg 307
Glu Ile Ser Gly Phe Pro Gly Phe Arg Trp Asp Thr Gly Pro Ser Trp
    55                  60                  65

tac ctc atg ccc gag gcc ttt gac cat ttc ttc gca ctt ttt ggt gca 355
Tyr Leu Met Pro Glu Ala Phe Asp His Phe Phe Ala Leu Phe Gly Ala
70                  75                  80                  85

tgt act tct gat tat ctc gat ttg gta gaa tta acg cct ggt tat cga 403
Cys Thr Ser Asp Tyr Leu Asp Leu Val Glu Leu Thr Pro Gly Tyr Arg
                90                  95                  100

gtt ttt tct ggc aca cat gac gct gtc gat gtc ccc act ggg cgt gaa 451
Val Phe Ser Gly Thr His Asp Ala Val Asp Val Pro Thr Gly Arg Glu
            105                 110                 115

gaa gca att gcg cta ttc gaa tcc atc gaa ccc ggc gcg ggt gca aaa 499
Glu Ala Ile Ala Leu Phe Glu Ser Ile Glu Pro Gly Ala Gly Ala Lys
        120                 125                 130

cta gga aat tat ctt gat agc gcg gca gac gcc tat gac att gcc att 547
Leu Gly Asn Tyr Leu Asp Ser Ala Ala Asp Ala Tyr Asp Ile Ala Ile
    135                 140                 145

gat aga ttc ctt tat aat aat ttc tcc acg tta ggc ccg ctg ctt cac 595
Asp Arg Phe Leu Tyr Asn Asn Phe Ser Thr Leu Gly Pro Leu Leu His
150                 155                 160                 165

cgg gat gta ctg acc cga gct ggc cga ctg ttt tct cta ctg acc cgt 643
Arg Asp Val Leu Thr Arg Ala Gly Arg Leu Phe Ser Leu Leu Thr Arg
                170                 175                 180

tct tta caa aag tac gta aat agt caa ttc agt agc ccg gtg ttg cgc 691
Ser Leu Gln Lys Tyr Val Asn Ser Gln Phe Ser Ser Pro Val Leu Arg
            185                 190                 195

cag atc cta acc tat cca gca gtc ttc ctg tct tcc cga ccc act act 739
Gln Ile Leu Thr Tyr Pro Ala Val Phe Leu Ser Ser Arg Pro Thr Thr
        200                 205                 210

acc cca tcg atg tac cac ttg atg agt cat acc gat ttg gtg cag gga 787
Thr Pro Ser Met Tyr His Leu Met Ser His Thr Asp Leu Val Gln Gly
    215                 220                 225

gtg aaa tac cct ata ggt ggt ttt act gca gtg gtt aac gct ctg cat 835
Val Lys Tyr Pro Ile Gly Gly Phe Thr Ala Val Val Asn Ala Leu His
230                 235                 240                 245

cag tta gcg ctg gaa aac ggg gtt gag ttt caa ctc gat tct gag gtc 883
Gln Leu Ala Leu Glu Asn Gly Val Glu Phe Gln Leu Asp Ser Glu Val
                250                 255                 260

att tcc atc aac act gct tca tcg agg ggc aac aca agc gcc aca ggt 931
Ile Ser Ile Asn Thr Ala Ser Ser Arg Gly Asn Thr Ser Ala Thr Gly
            265                 270                 275

gtg agc ttg ctt cac aac aga aaa gtg caa aat cta gat gcg gat ctt 979
Val Ser Leu Leu His Asn Arg Lys Val Gln Asn Leu Asp Ala Asp Leu
        280                 285                 290

gtg gtt tca gca ggc gac cta cac cat aca gaa aat aat ctg ctt ccc 1027
Val Val Ser Ala Gly Asp Leu His His Thr Glu Asn Asn Leu Leu Pro
    295                 300                 305

cgg gaa ctt cga acc tat ccc gaa cga tat tgg tcc aat cgc aat cct 1075
Arg Glu Leu Arg Thr Tyr Pro Glu Arg Tyr Trp Ser Asn Arg Asn Pro
310                 315                 320                 325

gga att gga gcg gta tta atc ctc ctg ggc gta aaa gga gag tta ccc 1123
Gly Ile Gly Ala Val Leu Ile Leu Leu Gly Val Lys Gly Glu Leu Pro
                330                 335                 340

cag ctc gac cat cac aac ctt ttc ttc agt gaa gat tgg aca gat gat 1171
Gln Leu Asp His His Asn Leu Phe Phe Ser Glu Asp Trp Thr Asp Asp
            345                 350                 355

ttt gct gta gtt ttc gac ggg cct caa ctt acc cgc ccc cac aat gca 1219
Phe Ala Val Val Phe Asp Gly Pro Gln Leu Thr Arg Pro His Asn Ala
        360                 365                 370

tca aat tcc att tat gtc tcc aag cct tca acg tcc gaa gac ggc gtt 1267
Ser Asn Ser Ile Tyr Val Ser Lys Pro Ser Thr Ser Glu Asp Gly Val
    375                 380                 385

gca cct gct gga tac gaa aac ctt ttt gtt tta att ccg acc aag gcc 1315
Ala Pro Ala Gly Tyr Glu Asn Leu Phe Val Leu Ile Pro Thr Lys Ala
390                 395                 400                 405

tct agc agc atc ggc cac ggt gat gcg tat atg cag tcg gct tca gca 1363
Ser Ser Ser Ile Gly His Gly Asp Ala Tyr Met Gln Ser Ala Ser Ala
                410                 415                 420

tcc gtg gaa aca atc gcg tca cat gca atc aat caa att gct acg caa 1411
Ser Val Glu Thr Ile Ala Ser His Ala Ile Asn Gln Ile Ala Thr Gln
            425                 430                 435

gcc ggc atc cct gac ctc act gac cga att gtg gtc aaa cgc acc att 1459
Ala Gly Ile Pro Asp Leu Thr Asp Arg Ile Val Val Lys Arg Thr Ile
        440                 445                 450

ggc cct gcg gat ttt gag cac cgc tac cat tca tgg gta ggc agt gcg 1507
Gly Pro Ala Asp Phe Glu His Arg Tyr His Ser Trp Val Gly Ser Ala
    455                 460                 465

ctg ggt cca gca cat acc ctc aga cag tcc gct ttc tta aga ggg cgc 1555
Leu Gly Pro Ala His Thr Leu Arg Gln Ser Ala Phe Leu Arg Gly Arg
470                 475                 480                 485

aat agc tcc cgc aag gtc aat aac ctc ttc tat tcc ggt gcc acc acc 1603

Asn Ser Ser Arg Lys Val Asn Asn Leu Phe Tyr Ser Gly Ala Thr Thr
                490                 495                 500

gtc ccg ggt gta gga ata ccc atg tgt tta att tct gcc gag aat att    1651
Val Pro Gly Val Gly Ile Pro Met Cys Leu Ile Ser Ala Glu Asn Ile
            505                 510                 515

att aag cgt tta cat gcc gat acc agt gca gga cca ctg ccc gaa cca    1699
Ile Lys Arg Leu His Ala Asp Thr Ser Ala Gly Pro Leu Pro Glu Pro
        520                 525                 530

ttg ccg cct aaa acg aca cca tct caa aag acc tca tac gat cat        1744
Leu Pro Pro Lys Thr Thr Pro Ser Gln Lys Thr Ser Tyr Asp His
    535                 540                 545

taaattttga tccctatcat cga                                          1767

<210> 262
<211> 548
<212> PRT
<213> Corynebacterium glutamicum

<400> 262
Met Lys Val Ser Thr Lys Thr Pro Arg Ser Ser Gly Thr Ala Val Val
1               5                   10                  15

Ile Gly Ala Gly Val Ala Gly Leu Ala Thr Ser Ala Leu Leu Ala Arg
            20                  25                  30

Asp Gly Trp Gln Val Thr Val Leu Glu Lys Asn Thr Asp Val Gly Gly
        35                  40                  45

Arg Ala Gly Ser Leu Glu Ile Ser Gly Phe Pro Gly Phe Arg Trp Asp
    50                  55                  60

Thr Gly Pro Ser Trp Tyr Leu Met Pro Glu Ala Phe Asp His Phe Phe
65                  70                  75                  80

Ala Leu Phe Gly Ala Cys Thr Ser Asp Tyr Leu Asp Leu Val Glu Leu
                85                  90                  95

Thr Pro Gly Tyr Arg Val Phe Ser Gly Thr His Asp Ala Val Asp Val
            100                 105                 110

Pro Thr Gly Arg Glu Glu Ala Ile Ala Leu Phe Glu Ser Ile Glu Pro
        115                 120                 125

Gly Ala Gly Ala Lys Leu Gly Asn Tyr Leu Asp Ser Ala Ala Asp Ala
    130                 135                 140

Tyr Asp Ile Ala Ile Asp Arg Phe Leu Tyr Asn Asn Phe Ser Thr Leu
145                 150                 155                 160

Gly Pro Leu Leu His Arg Asp Val Leu Thr Arg Ala Gly Arg Leu Phe
                165                 170                 175

Ser Leu Leu Thr Arg Ser Leu Gln Lys Tyr Val Asn Ser Gln Phe Ser
            180                 185                 190

Ser Pro Val Leu Arg Gln Ile Leu Thr Tyr Pro Ala Val Phe Leu Ser

        195                 200                 205

Ser Arg Pro Thr Thr Thr Pro Ser Met Tyr His Leu Met Ser His Thr
    210                 215                 220

Asp Leu Val Gln Gly Val Lys Tyr Pro Ile Gly Gly Phe Thr Ala Val
225                 230                 235                 240

Val Asn Ala Leu His Gln Leu Ala Leu Glu Asn Gly Val Glu Phe Gln
                245                 250                 255

Leu Asp Ser Glu Val Ile Ser Ile Asn Thr Ala Ser Ser Arg Gly Asn
            260                 265                 270

Thr Ser Ala Thr Gly Val Ser Leu Leu His Asn Arg Lys Val Gln Asn
        275                 280                 285

Leu Asp Ala Asp Leu Val Val Ser Ala Gly Asp Leu His His Thr Glu
    290                 295                 300

Asn Asn Leu Leu Pro Arg Glu Leu Arg Thr Tyr Pro Glu Arg Tyr Trp
305                 310                 315                 320

Ser Asn Arg Asn Pro Gly Ile Gly Ala Val Leu Ile Leu Leu Gly Val
                325                 330                 335

Lys Gly Glu Leu Pro Gln Leu Asp His His Asn Leu Phe Phe Ser Glu
            340                 345                 350

Asp Trp Thr Asp Asp Phe Ala Val Val Phe Asp Gly Pro Gln Leu Thr
        355                 360                 365

Arg Pro His Asn Ala Ser Asn Ser Ile Tyr Val Ser Lys Pro Ser Thr
    370                 375                 380

Ser Glu Asp Gly Val Ala Pro Ala Gly Tyr Glu Asn Leu Phe Val Leu
385                 390                 395                 400

Ile Pro Thr Lys Ala Ser Ser Ser Ile Gly His Gly Asp Ala Tyr Met
                405                 410                 415

Gln Ser Ala Ser Ala Ser Val Glu Thr Ile Ala Ser His Ala Ile Asn
            420                 425                 430

Gln Ile Ala Thr Gln Ala Gly Ile Pro Asp Leu Thr Asp Arg Ile Val
        435                 440                 445

Val Lys Arg Thr Ile Gly Pro Ala Asp Phe Glu His Arg Tyr His Ser
    450                 455                 460

Trp Val Gly Ser Ala Leu Gly Pro Ala His Thr Leu Arg Gln Ser Ala
465                 470                 475                 480

Phe Leu Arg Gly Arg Asn Ser Ser Arg Lys Val Asn Asn Leu Phe Tyr
                485                 490                 495

Ser Gly Ala Thr Thr Val Pro Gly Val Gly Ile Pro Met Cys Leu Ile
            500                 505                 510

Ser Ala Glu Asn Ile Ile Lys Arg Leu His Ala Asp Thr Ser Ala Gly
        515                 520                 525

Pro Leu Pro Glu Pro Leu Pro Pro Lys Thr Thr Pro Ser Gln Lys Thr
    530                 535                 540

Ser Tyr Asp His
545

<210> 263
<211> 1767
<212> DNA
<213> Corynebacterium glutamicum

<220>
<221> CDS
<222> (101)..(1744)
<223> FRXA00477

<400> 263
tgcgggagcg aatcagagtt ccacttcata tcaaactctc tacactcgct agagccacga 60

tgaaaggtct atctatgagc atctacagaa agaattcgtg atg aag gtc tcg act 115
Met Lys Val Ser Thr
1               5

aaa act cca cgc tcc tca ggt acc gcc gta gtc ata ggc gca ggt gtt 163
Lys Thr Pro Arg Ser Ser Gly Thr Ala Val Val Ile Gly Ala Gly Val
                10                  15                  20

gct ggt tta gcc act tct gca ctt tta gca cgt gat ggc tgg caa gta 211
Ala Gly Leu Ala Thr Ser Ala Leu Leu Ala Arg Asp Gly Trp Gln Val
            25                  30                  35

act gtt ttg gaa aaa aat act gat gtc ggt ggc cga gct gga tcg ctt 259
Thr Val Leu Glu Lys Asn Thr Asp Val Gly Gly Arg Ala Gly Ser Leu
        40                  45                  50

gaa ata tca ggc ttt cct ggc ttt cga tgg gat acc gga cct tct tgg 307
Glu Ile Ser Gly Phe Pro Gly Phe Arg Trp Asp Thr Gly Pro Ser Trp
    55                  60                  65

tac ctc atg ccc gag gcc ttt gac cat ttc ttc gca ctt ttt ggt gca 355
Tyr Leu Met Pro Glu Ala Phe Asp His Phe Phe Ala Leu Phe Gly Ala
70                  75                  80                  85

tgt act tct gat tat ctc gat ttg gta gaa tta acg cct ggt tat cga 403
Cys Thr Ser Asp Tyr Leu Asp Leu Val Glu Leu Thr Pro Gly Tyr Arg
                90                  95                  100

gtt ttt tct ggc aca cat gac gct gtc gat gtc ccc act ggg cgt gaa 451
Val Phe Ser Gly Thr His Asp Ala Val Asp Val Pro Thr Gly Arg Glu
            105                 110                 115

gaa gca att gcg cta ttc gaa tcc atc gaa ccc ggc gcg ggt gca aaa 499
Glu Ala Ile Ala Leu Phe Glu Ser Ile Glu Pro Gly Ala Gly Ala Lys
        120                 125                 130

cta gga aat tat ctt gat agc gcg gca gac gcc tat gac att gcc att 547
Leu Gly Asn Tyr Leu Asp Ser Ala Ala Asp Ala Tyr Asp Ile Ala Ile
    135                 140                 145

gat aga ttc ctt tat aat aat ttc tcc acg tta ggc ccg ctg ctt cac 595
Asp Arg Phe Leu Tyr Asn Asn Phe Ser Thr Leu Gly Pro Leu Leu His
150                 155                 160                 165

cgg gat gta ctg acc cga gct ggc cga ctg ttt tct cta ctg acc cgt 643
Arg Asp Val Leu Thr Arg Ala Gly Arg Leu Phe Ser Leu Leu Thr Arg
                170                 175                 180

tct tta caa aag tac gta aat agt caa ttc agt agc ccg gtg ttg cgc 691
Ser Leu Gln Lys Tyr Val Asn Ser Gln Phe Ser Ser Pro Val Leu Arg
            185                 190                 195

cag atc cta acc tat cca gca gtc ttc ctg tct tcc cga ccc act act 739
Gln Ile Leu Thr Tyr Pro Ala Val Phe Leu Ser Ser Arg Pro Thr Thr
        200                 205                 210

acc cca tcg atg tac cac ttg atg agt cat acc gat ttg gtg cag gga 787
Thr Pro Ser Met Tyr His Leu Met Ser His Thr Asp Leu Val Gln Gly
    215                 220                 225

gtg aaa tac cct ata ggt ggt ttt act gca gtg gtt aac gct ctg cat 835
Val Lys Tyr Pro Ile Gly Gly Phe Thr Ala Val Val Asn Ala Leu His
230                 235                 240                 245

cag tta gcg ctg gaa aac ggg gtt gag ttt caa ctc gat tct gag gtc 883
Gln Leu Ala Leu Glu Asn Gly Val Glu Phe Gln Leu Asp Ser Glu Val
                250                 255                 260

att tcc atc aac act gct tca tcg agg ggc aac aca agc gcc aca ggt 931
Ile Ser Ile Asn Thr Ala Ser Ser Arg Gly Asn Thr Ser Ala Thr Gly
            265                 270                 275

gtg agc ttg ctt cac aac aga aaa gtg caa aat cta gat gcg gat ctt 979
Val Ser Leu Leu His Asn Arg Lys Val Gln Asn Leu Asp Ala Asp Leu
        280                 285                 290

gtg gtt tca gca ggc gac cta cac cat aca gaa aat aat ctg ctt ccc 1027
Val Val Ser Ala Gly Asp Leu His His Thr Glu Asn Asn Leu Leu Pro
    295                 300                 305

cgg gaa ctt cga acc tat ccc gaa cga tat tgg tcc aat cgc aat cct 1075
Arg Glu Leu Arg Thr Tyr Pro Glu Arg Tyr Trp Ser Asn Arg Asn Pro
310                 315                 320                 325

gga att gga gcg gta tta atc ctc ctg ggc gta aaa gga gag tta ccc 1123
Gly Ile Gly Ala Val Leu Ile Leu Leu Gly Val Lys Gly Glu Leu Pro
                330                 335                 340

cag ctc gac cat cac aac ctt ttc ttc agt gaa gat tgg aca gat gat 1171
Gln Leu Asp His His Asn Leu Phe Phe Ser Glu Asp Trp Thr Asp Asp
            345                 350                 355

ttt gct gta gtt ttc gac ggg cct caa ctt acc cgc ccc cac aat gca 1219
Phe Ala Val Val Phe Asp Gly Pro Gln Leu Thr Arg Pro His Asn Ala
        360                 365                 370

tca aat tcc att tat gtc tcc aag cct tca acg tcc gaa gac ggc gtt 1267
Ser Asn Ser Ile Tyr Val Ser Lys Pro Ser Thr Ser Glu Asp Gly Val
    375                 380                 385

gca cct gct gga tac gaa aac ctt ttt gtt tta att ccg acc aag gcc 1315

Ala Pro Ala Gly Tyr Glu Asn Leu Phe Val Leu Ile Pro Thr Lys Ala
390                 395                 400                 405

tct agc agc atc ggc cac ggt gat gcg tat atg cag tcg gct tca gca    1363
Ser Ser Ser Ile Gly His Gly Asp Ala Tyr Met Gln Ser Ala Ser Ala
                410                 415                 420

tcc gtg gaa aca atc gcg tca cat gca atc aat caa att gct acg caa    1411
Ser Val Glu Thr Ile Ala Ser His Ala Ile Asn Gln Ile Ala Thr Gln
            425                 430                 435

gcc ggc atc cct gac ctc act gac cga att gtg gtc aaa cgc acc att    1459
Ala Gly Ile Pro Asp Leu Thr Asp Arg Ile Val Val Lys Arg Thr Ile
        440                 445                 450

ggc cct gcg gat ttt gag cac cgc tac cat tca tgg gta ggc agt gcg    1507
Gly Pro Ala Asp Phe Glu His Arg Tyr His Ser Trp Val Gly Ser Ala
    455                 460                 465

ctg ggt cca gca cat acc ctc aga cag tcc gct ttc tta aga ggg cgc    1555
Leu Gly Pro Ala His Thr Leu Arg Gln Ser Ala Phe Leu Arg Gly Arg
470                 475                 480                 485

aat agc tcc cgc aag gtc aat aac ctc ttc tat tcc ggt gcc acc acc    1603
Asn Ser Ser Arg Lys Val Asn Asn Leu Phe Tyr Ser Gly Ala Thr Thr
                490                 495                 500

gtc ccg ggt gta gga ata ccc atg tgt tta att tct gcc gag aat att    1651
Val Pro Gly Val Gly Ile Pro Met Cys Leu Ile Ser Ala Glu Asn Ile
            505                 510                 515

att aag cgt tta cat gcc gat acc agt gca gga cca ctg ccc gaa cca    1699
Ile Lys Arg Leu His Ala Asp Thr Ser Ala Gly Pro Leu Pro Glu Pro
        520                 525                 530

ttg ccg cct aaa acg aca cca tct caa aag acc tca tac gat cat        1744
Leu Pro Pro Lys Thr Thr Pro Ser Gln Lys Thr Ser Tyr Asp His
    535                 540                 545

taaattttga tccctatcat cga                                          1767

<210> 264
<211> 548
<212> PRT
<213> Corynebacterium glutamicum

<400> 264
Met Lys Val Ser Thr Lys Thr Pro Arg Ser Ser Gly Thr Ala Val Val
1               5                   10                  15

Ile Gly Ala Gly Val Ala Gly Leu Ala Thr Ser Ala Leu Leu Ala Arg
            20                  25                  30

Asp Gly Trp Gln Val Thr Val Leu Glu Lys Asn Thr Asp Val Gly Gly
        35                  40                  45

Arg Ala Gly Ser Leu Glu Ile Ser Gly Phe Pro Gly Phe Arg Trp Asp
    50                  55                  60

Thr Gly Pro Ser Trp Tyr Leu Met Pro Glu Ala Phe Asp His Phe Phe

65                  70                  75                  80

Ala Leu Phe Gly Ala Cys Thr Ser Asp Tyr Leu Asp Leu Val Glu Leu
                85                  90                  95

Thr Pro Gly Tyr Arg Val Phe Ser Gly Thr His Asp Ala Val Asp Val
            100                 105                 110

Pro Thr Gly Arg Glu Glu Ala Ile Ala Leu Phe Glu Ser Ile Glu Pro
        115                 120                 125

Gly Ala Gly Ala Lys Leu Gly Asn Tyr Leu Asp Ser Ala Ala Asp Ala
    130                 135                 140

Tyr Asp Ile Ala Ile Asp Arg Phe Leu Tyr Asn Asn Phe Ser Thr Leu
145                 150                 155                 160

Gly Pro Leu Leu His Arg Asp Val Leu Thr Arg Ala Gly Arg Leu Phe
                165                 170                 175

Ser Leu Leu Thr Arg Ser Leu Gln Lys Tyr Val Asn Ser Gln Phe Ser
            180                 185                 190

Ser Pro Val Leu Arg Gln Ile Leu Thr Tyr Pro Ala Val Phe Leu Ser
        195                 200                 205

Ser Arg Pro Thr Thr Thr Pro Ser Met Tyr His Leu Met Ser His Thr
    210                 215                 220

Asp Leu Val Gln Gly Val Lys Tyr Pro Ile Gly Gly Phe Thr Ala Val
225                 230                 235                 240

Val Asn Ala Leu His Gln Leu Ala Leu Glu Asn Gly Val Glu Phe Gln
                245                 250                 255

Leu Asp Ser Glu Val Ile Ser Ile Asn Thr Ala Ser Ser Arg Gly Asn
            260                 265                 270

Thr Ser Ala Thr Gly Val Ser Leu Leu His Asn Arg Lys Val Gln Asn
        275                 280                 285

Leu Asp Ala Asp Leu Val Val Ser Ala Gly Asp Leu His His Thr Glu
    290                 295                 300

Asn Asn Leu Leu Pro Arg Glu Leu Arg Thr Tyr Pro Glu Arg Tyr Trp
305                 310                 315                 320

Ser Asn Arg Asn Pro Gly Ile Gly Ala Val Leu Ile Leu Leu Gly Val
                325                 330                 335

Lys Gly Glu Leu Pro Gln Leu Asp His His Asn Leu Phe Phe Ser Glu
            340                 345                 350

Asp Trp Thr Asp Asp Phe Ala Val Val Phe Asp Gly Pro Gln Leu Thr
        355                 360                 365

Arg Pro His Asn Ala Ser Asn Ser Ile Tyr Val Ser Lys Pro Ser Thr
    370                 375                 380

Ser Glu Asp Gly Val Ala Pro Ala Gly Tyr Glu Asn Leu Phe Val Leu
385                 390                 395                 400

Ile Pro Thr Lys Ala Ser Ser Ser Ile Gly His Gly Asp Ala Tyr Met
                405                 410                 415

Gln Ser Ala Ser Ala Ser Val Glu Thr Ile Ala Ser His Ala Ile Asn
            420                 425                 430

Gln Ile Ala Thr Gln Ala Gly Ile Pro Asp Leu Thr Asp Arg Ile Val
        435                 440                 445

Val Lys Arg Thr Ile Gly Pro Ala Asp Phe Glu His Arg Tyr His Ser
    450                 455                 460

Trp Val Gly Ser Ala Leu Gly Pro Ala His Thr Leu Arg Gln Ser Ala
465                 470                 475                 480

Phe Leu Arg Gly Arg Asn Ser Ser Arg Lys Val Asn Asn Leu Phe Tyr
                485                 490                 495

Ser Gly Ala Thr Thr Val Pro Gly Val Gly Ile Pro Met Cys Leu Ile
            500                 505                 510

Ser Ala Glu Asn Ile Ile Lys Arg Leu His Ala Asp Thr Ser Ala Gly
        515                 520                 525

Pro Leu Pro Glu Pro Leu Pro Pro Lys Thr Thr Pro Ser Gln Lys Thr
    530                 535                 540

Ser Tyr Asp His
545

<210> 265
<211> 954
<212> DNA
<213> Corynebacterium glutamicum

<220>
<221> CDS
<222> (101)..(931)
<223> RXA00478

<400> 265
accagaagca gcctcagcta tgacacacca aaattcgcct ctcttcctta aaagtgcact 60

gagactttac aatcgggcct cattcaaggc ttcacataaa gtg atc gaa gaa tat 115
Val Ile Glu Glu Tyr
1               5

tcc acg agc ttc agt ctg tct acg tgg ttg cta tcc cca cgc ata cga 163
Ser Thr Ser Phe Ser Leu Ser Thr Trp Leu Leu Ser Pro Arg Ile Arg
                10                  15                  20

aat gac ata cga aat ctc tat gca gta gtt cgt atc gcc gat gag att 211
Asn Asp Ile Arg Asn Leu Tyr Ala Val Val Arg Ile Ala Asp Glu Ile
            25                  30                  35

gtc gac ggc act gca cat gcc gct ggt tgc tca act gcc aaa atc gaa 259
Val Asp Gly Thr Ala His Ala Ala Gly Cys Ser Thr Ala Lys Ile Glu
        40                  45                  50

gag att ctc gat gcc tat gaa att gcg gtt ctt gca gca cca caa caa 307
Glu Ile Leu Asp Ala Tyr Glu Ile Ala Val Leu Ala Ala Pro Gln Gln
    55                  60                  65

cgc ttc aac aca gat ctt gtt tta caa gct tat ggt gaa act gcc cga 355
Arg Phe Asn Thr Asp Leu Val Leu Gln Ala Tyr Gly Glu Thr Ala Arg
70                  75                  80                  85

cgc tgt gat ttc gaa caa gag cat gta ata gcc ttc ttt gca tca atg 403
Arg Cys Asp Phe Glu Gln Glu His Val Ile Ala Phe Phe Ala Ser Met
                90                  95                  100

cgt aag gac ctc aaa gct aat aca cac gac cca gat agc ttc aca acg 451
Arg Lys Asp Leu Lys Ala Asn Thr His Asp Pro Asp Ser Phe Thr Thr
            105                 110                 115

tat gtc tat ggc tcc gcg gaa gtt ata ggc ctg ctt tgt ctc agc gtt 499
Tyr Val Tyr Gly Ser Ala Glu Val Ile Gly Leu Leu Cys Leu Ser Val
        120                 125                 130

ttc aac caa ggt aga acg att agc aaa aaa cgg cta gag att atg caa 547
Phe Asn Gln Gly Arg Thr Ile Ser Lys Lys Arg Leu Glu Ile Met Gln
    135                 140                 145

aac gga gcc cgc tca ttg gga gcg gca ttc cag aaa att aac ttt ctc 595
Asn Gly Ala Arg Ser Leu Gly Ala Ala Phe Gln Lys Ile Asn Phe Leu
150                 155                 160                 165

cgt gac ttg gca gaa gat cag caa aat ttg ggc cga ttt tat ttc ccc 643
Arg Asp Leu Ala Glu Asp Gln Gln Asn Leu Gly Arg Phe Tyr Phe Pro
                170                 175                 180

aaa acc agc caa gga act ctt act aaa gaa caa aaa gaa gat ctc atc 691
Lys Thr Ser Gln Gly Thr Leu Thr Lys Glu Gln Lys Glu Asp Leu Ile
            185                 190                 195

gct gat atc cgt caa gac cta gca att gcc cac gat gca ttt cca gaa 739
Ala Asp Ile Arg Gln Asp Leu Ala Ile Ala His Asp Ala Phe Pro Glu
        200                 205                 210

ata cca gtg cag gct cgc atc gga gtg atc tct gct tat ttg ctc ttt 787
Ile Pro Val Gln Ala Arg Ile Gly Val Ile Ser Ala Tyr Leu Leu Phe
    215                 220                 225

caa aaa ctc act gac cga att gag gct act cct acc gcc gat tta ttg 835
Gln Lys Leu Thr Asp Arg Ile Glu Ala Thr Pro Thr Ala Asp Leu Leu
230                 235                 240                 245

cgg gag cga atc aga gtt cca ctt cat atc aaa ctc tct aca ctc gct 883
Arg Glu Arg Ile Arg Val Pro Leu His Ile Lys Leu Ser Thr Leu Ala
                250                 255                 260

aga gcc acg atg aaa ggt cta tct atg agc atc tac aga aag aat tcg 931
Arg Ala Thr Met Lys Gly Leu Ser Met Ser Ile Tyr Arg Lys Asn Ser
            265                 270                 275

tgatgaaggt ctcgactaaa act 954

<210> 266
<211> 277

<212> PRT
<213> Corynebacterium glutamicum

<400> 266
Val Ile Glu Glu Tyr Ser Thr Ser Phe Ser Leu Ser Thr Trp Leu Leu
1               5                   10                  15

Ser Pro Arg Ile Arg Asn Asp Ile Arg Asn Leu Tyr Ala Val Val Arg
            20                  25                  30

Ile Ala Asp Glu Ile Val Asp Gly Thr Ala His Ala Ala Gly Cys Ser
        35                  40                  45

Thr Ala Lys Ile Glu Glu Ile Leu Asp Ala Tyr Glu Ile Ala Val Leu
    50                  55                  60

Ala Ala Pro Gln Gln Arg Phe Asn Thr Asp Leu Val Leu Gln Ala Tyr
65                  70                  75                  80

Gly Glu Thr Ala Arg Arg Cys Asp Phe Glu Gln Glu His Val Ile Ala
                85                  90                  95

Phe Phe Ala Ser Met Arg Lys Asp Leu Lys Ala Asn Thr His Asp Pro
            100                 105                 110

Asp Ser Phe Thr Thr Tyr Val Tyr Gly Ser Ala Glu Val Ile Gly Leu
        115                 120                 125

Leu Cys Leu Ser Val Phe Asn Gln Gly Arg Thr Ile Ser Lys Lys Arg
    130                 135                 140

Leu Glu Ile Met Gln Asn Gly Ala Arg Ser Leu Gly Ala Ala Phe Gln
145                 150                 155                 160

Lys Ile Asn Phe Leu Arg Asp Leu Ala Glu Asp Gln Gln Asn Leu Gly
                165                 170                 175

Arg Phe Tyr Phe Pro Lys Thr Ser Gln Gly Thr Leu Thr Lys Glu Gln
            180                 185                 190

Lys Glu Asp Leu Ile Ala Asp Ile Arg Gln Asp Leu Ala Ile Ala His
        195                 200                 205

Asp Ala Phe Pro Glu Ile Pro Val Gln Ala Arg Ile Gly Val Ile Ser
    210                 215                 220

Ala Tyr Leu Leu Phe Gln Lys Leu Thr Asp Arg Ile Glu Ala Thr Pro
225                 230                 235                 240

Thr Ala Asp Leu Leu Arg Glu Arg Ile Arg Val Pro Leu His Ile Lys
                245                 250                 255

Leu Ser Thr Leu Ala Arg Ala Thr Met Lys Gly Leu Ser Met Ser Ile
            260                 265                 270

Tyr Arg Lys Asn Ser
        275


<210> 267
<211> 1056

<212> DNA
<213> Corynebacterium glutamicum

<220>
<221> CDS
<222> (101)..(1033)
<223> RXA01291

<400> 267
aacccgcagc gggtggagcg gggctgaggg agacgtcgag aagcgtccct ttcggttgtc 60

tggatttctc gcggtaaagt ttcctgtgaa ggaggcgagt gtg gct gat cag caa 115
Val Ala Asp Gln Gln
1               5

gat ttt ttg ggc cgt ttt gat gcg atg agc tca aag gcg acg gcc acg 163
Asp Phe Leu Gly Arg Phe Asp Ala Met Ser Ser Lys Ala Thr Ala Thr
                10                  15                  20

gta atc gcc cat tat tcc tcc agt ttt act ctg gca tcg aag ctg ttg 211
Val Ile Ala His Tyr Ser Ser Ser Phe Thr Leu Ala Ser Lys Leu Leu
            25                  30                  35

tcg ccg aaa att cgt cgc gat att gag gca ttg tac gca atg gtg cga 259
Ser Pro Lys Ile Arg Arg Asp Ile Glu Ala Leu Tyr Ala Met Val Arg
        40                  45                  50

gtg gcc gat gag gtt gtg gac ggc gct gct gct gcc gcg ggg tgt gcg 307
Val Ala Asp Glu Val Val Asp Gly Ala Ala Ala Ala Ala Gly Cys Ala
    55                  60                  65

ccg gac gcc gtt gcg gag att ttg gac aat tac gaa cgc cag gtt ctg 355
Pro Asp Ala Val Ala Glu Ile Leu Asp Asn Tyr Glu Arg Gln Val Leu
70                  75                  80                  85

ctc agt ttg tcc gtt cct ttt cat acc gat cca gtg atc cat gcg ttt 403
Leu Ser Leu Ser Val Pro Phe His Thr Asp Pro Val Ile His Ala Phe
                90                  95                  100

ggc aat acc gcc cgc aaa tgt ggt ttt gag cag gct cac atc gtg gcg 451
Gly Asn Thr Ala Arg Lys Cys Gly Phe Glu Gln Ala His Ile Val Ala
            105                 110                 115

ttt ttt gat tcc atg cgc cgc gat ctc tcc caa acc tcc tat gat ccg 499
Phe Phe Asp Ser Met Arg Arg Asp Leu Ser Gln Thr Ser Tyr Asp Pro
        120                 125                 130

act cag ttg gat gag tac att tac ggc tcc gct gag gtc atc ggg ttg 547
Thr Gln Leu Asp Glu Tyr Ile Tyr Gly Ser Ala Glu Val Ile Gly Leu
    135                 140                 145

atg tgt ttg aaa att ttc ctc caa gat tcc act gcc agc ccg cag gat 595
Met Cys Leu Lys Ile Phe Leu Gln Asp Ser Thr Ala Ser Pro Gln Asp
150                 155                 160                 165

cgg gcc acg atg gaa cac ggc gcc agg cgt ttg ggt gcg gca ttt caa 643
Arg Ala Thr Met Glu His Gly Ala Arg Arg Leu Gly Ala Ala Phe Gln
                170                 175                 180

aag gta aat ttc ctg cga gat cta gct gaa gac cgc gaa ggc cta ggc 691
Lys Val Asn Phe Leu Arg Asp Leu Ala Glu Asp Arg Glu Gly Leu Gly

185                 190                 195

cgt tct tat ttg ccg gta ttc acc gaa gaa atg cgc gat gag atc gtt     739
Arg Ser Tyr Leu Pro Val Phe Thr Glu Glu Met Arg Asp Glu Ile Val
        200                 205                 210

act gat atc cga gag gat ttg gat gcc gcc cgg ctg agc att ccg ctg     787
Thr Asp Ile Arg Glu Asp Leu Asp Ala Ala Arg Leu Ser Ile Pro Leu
    215                 220                 225

ctg cca ttt ggc gcg cga acc ggt gtg cgc gcc gcg acc gat ctc tac     835
Leu Pro Phe Gly Ala Arg Thr Gly Val Arg Ala Ala Thr Asp Leu Tyr
230                 235                 240                 245

ggt tgc ctc gtg gac aac ctg gaa tcc gcg tcc ctc gaa gac tta aaa     883
Gly Cys Leu Val Asp Asn Leu Glu Ser Ala Ser Leu Glu Asp Leu Lys
                250                 255                 260

aac ggg cgg gat ttt gtg ccg tct tta aaa aag cca gcc tgg caa cca     931
Asn Gly Arg Asp Phe Val Pro Ser Leu Lys Lys Pro Ala Trp Gln Pro
            265                 270                 275

aag caa tgt gga aag aag tgt ttc aaa aat gac aaa agc agt ggt cat     979
Lys Gln Cys Gly Lys Lys Cys Phe Lys Asn Asp Lys Ser Ser Gly His
        280                 285                 290

cgg cgg ggg act agc agg act agc cac cac cgc act gct ctt acg cga     1027
Arg Arg Gly Thr Ser Arg Thr Ser His His Arg Thr Ala Leu Thr Arg
    295                 300                 305

agg ata tgaagtccac ctcgtcgaac aaa                                   1056
Arg Ile
310

<210> 268
<211> 311
<212> PRT
<213> Corynebacterium glutamicum

<400> 268
Val Ala Asp Gln Gln Asp Phe Leu Gly Arg Phe Asp Ala Met Ser Ser
1               5                   10                  15

Lys Ala Thr Ala Thr Val Ile Ala His Tyr Ser Ser Ser Phe Thr Leu
            20                  25                  30

Ala Ser Lys Leu Leu Ser Pro Lys Ile Arg Arg Asp Ile Glu Ala Leu
        35                  40                  45

Tyr Ala Met Val Arg Val Ala Asp Glu Val Val Asp Gly Ala Ala Ala
    50                  55                  60

Ala Ala Gly Cys Ala Pro Asp Ala Val Ala Glu Ile Leu Asp Asn Tyr
65                  70                  75                  80

Glu Arg Gln Val Leu Leu Ser Leu Ser Val Pro Phe His Thr Asp Pro
                85                  90                  95

Val Ile His Ala Phe Gly Asn Thr Ala Arg Lys Cys Gly Phe Glu Gln
            100                 105                 110

Ala His Ile Val Ala Phe Phe Asp Ser Met Arg Arg Asp Leu Ser Gln
        115                 120                 125

Thr Ser Tyr Asp Pro Thr Gln Leu Asp Glu Tyr Ile Tyr Gly Ser Ala
    130                 135                 140

Glu Val Ile Gly Leu Met Cys Leu Lys Ile Phe Leu Gln Asp Ser Thr
145                 150                 155                 160

Ala Ser Pro Gln Asp Arg Ala Thr Met Glu His Gly Ala Arg Arg Leu
                165                 170                 175

Gly Ala Ala Phe Gln Lys Val Asn Phe Leu Arg Asp Leu Ala Glu Asp
            180                 185                 190

Arg Glu Gly Leu Gly Arg Ser Tyr Leu Pro Val Phe Thr Glu Glu Met
        195                 200                 205

Arg Asp Glu Ile Val Thr Asp Ile Arg Glu Asp Leu Asp Ala Ala Arg
    210                 215                 220

Leu Ser Ile Pro Leu Leu Pro Phe Gly Ala Arg Thr Gly Val Arg Ala
225                 230                 235                 240

Ala Thr Asp Leu Tyr Gly Cys Leu Val Asp Asn Leu Glu Ser Ala Ser
                245                 250                 255

Leu Glu Asp Leu Lys Asn Gly Arg Asp Phe Val Pro Ser Leu Lys Lys
            260                 265                 270

Pro Ala Trp Gln Pro Lys Gln Cys Gly Lys Lys Cys Phe Lys Asn Asp
        275                 280                 285

Lys Ser Ser Gly His Arg Arg Gly Thr Ser Arg Thr Ser His His Arg
    290                 295                 300

Thr Ala Leu Thr Arg Arg Ile
305                 310

<210> 269
<211> 1239
<212> DNA
<213> Corynebacterium glutamicum

<220>
<221> CDS
<222> (101)..(1216)
<223> RXA00480

<400> 269
tttatggacc caaattcaca ctttctgtac ttcatcaaaa taaagcgcca tatacaacga 60

ttgggaattt ttgcgagata actggccgtg tgatactcga atg gac aat ggc atg 115
                                           Met Asp Asn Gly Met
                                           1               5

aca atc acc aca gaa cat tca act cat cct gat ctt gat ttc aat gat 163
Thr Ile Thr Thr Glu His Ser Thr His Pro Asp Leu Asp Phe Asn Asp
                10                  15                  20

gag att tat cgg gaa cta aac cgc atc tgc gct tcg cta tct caa cag 211
Glu Ile Tyr Arg Glu Leu Asn Arg Ile Cys Ala Ser Leu Ser Gln Gln
            25                  30                  35

tgc agc aca tat caa cca gag ttc cgt acc tgc cta gat gct gct ttc 259
Cys Ser Thr Tyr Gln Pro Glu Phe Arg Thr Cys Leu Asp Ala Ala Phe
        40                  45                  50

caa gct ttg cga ggt ggc aag tta atc cgc cct cga atg cta ctg ggg 307
Gln Ala Leu Arg Gly Gly Lys Leu Ile Arg Pro Arg Met Leu Leu Gly
    55                  60                  65

cta tac aac acg ctt gta gac gat gac att gag gtc aaa ctc aac acc 355
Leu Tyr Asn Thr Leu Val Asp Asp Asp Ile Glu Val Lys Leu Asn Thr
70                  75                  80                  85

gtt tta cag gta gca gtg gct tta gaa cta ctg cat ttt tcc ctt ttg 403
Val Leu Gln Val Ala Val Ala Leu Glu Leu Leu His Phe Ser Leu Leu
                90                  95                  100

gtt cat gac gat gtt att gac gga gac ctc tat cgc cga ggc aaa ctt 451
Val His Asp Asp Val Ile Asp Gly Asp Leu Tyr Arg Arg Gly Lys Leu
            105                 110                 115

aat ttt att ggg cag att ctc atg cat cgc aca cct gaa agt ttt gca 499
Asn Phe Ile Gly Gln Ile Leu Met His Arg Thr Pro Glu Ser Phe Ala
        120                 125                 130

caa atc cag cgc gat cca gag cat cta gat tgg gca caa tct aat gga 547
Gln Ile Gln Arg Asp Pro Glu His Leu Asp Trp Ala Gln Ser Asn Gly
    135                 140                 145

ctg ctt atg gga aat ctt ttt ctt gct gcc acc cat caa atc ttc gcg 595
Leu Leu Met Gly Asn Leu Phe Leu Ala Ala Thr His Gln Ile Phe Ala
150                 155                 160                 165

cgc ctt gac ctt cca cat cac caa cgg gtt cga ctt tta gat tta ctc 643
Arg Leu Asp Leu Pro His His Gln Arg Val Arg Leu Leu Asp Leu Leu
                170                 175                 180

aac cac acg ata aat gac act att gtg ggt gag ttt ctt gat gtg gga 691
Asn His Thr Ile Asn Asp Thr Ile Val Gly Glu Phe Leu Asp Val Gly
            185                 190                 195

tta agc agc aaa gcc atc agc ccc aat atg gac att gct cta gaa atg 739
Leu Ser Ser Lys Ala Ile Ser Pro Asn Met Asp Ile Ala Leu Glu Met
        200                 205                 210

agt cgg cta aaa aca gcc aca tac act ttt gaa ctt cca atg aga gca 787
Ser Arg Leu Lys Thr Ala Thr Tyr Thr Phe Glu Leu Pro Met Arg Ala
    215                 220                 225

gcg gca att ctc gcg gaa cta cct cag gag att gaa aca aag ata ggt 835
Ala Ala Ile Leu Ala Glu Leu Pro Gln Glu Ile Glu Thr Lys Ile Gly
230                 235                 240                 245

gag ata ggc aca aac ttg ggc atc gct tat caa ttg cag gac gat tac 883
Glu Ile Gly Thr Asn Leu Gly Ile Ala Tyr Gln Leu Gln Asp Asp Tyr
                250                 255                 260

tta tct act ttt ggt gac gca gcc gaa cac ggc aaa gat gcc ttt tct     931
Leu Ser Thr Phe Gly Asp Ala Ala Glu His Gly Lys Asp Ala Phe Ser
            265                 270                 275

gac ctt cga gaa gga aaa gaa act aca att atc gcc ttc gct cga gat     979
Asp Leu Arg Glu Gly Lys Glu Thr Thr Ile Ile Ala Phe Ala Arg Asp
        280                 285                 290

act gct aaa tgg act gat att caa gac aac ttc ggc tcc gca gat ctg     1027
Thr Ala Lys Trp Thr Asp Ile Gln Asp Asn Phe Gly Ser Ala Asp Leu
    295                 300                 305

agc acc tct cag gca gag cga att caa cat ctt ctc ata cag tgt gga     1075
Ser Thr Ser Gln Ala Glu Arg Ile Gln His Leu Leu Ile Gln Cys Gly
310                 315                 320                 325

gca aag aat cac tcc ttg aat gcc atc tcc gac cac tta aat atc tgc     1123
Ala Lys Asn His Ser Leu Asn Ala Ile Ser Asp His Leu Asn Ile Cys
                330                 335                 340

cgt tcg atg atc aaa aca cta agc ccc cag gta gat ccc aag gct caa     1171
Arg Ser Met Ile Lys Thr Leu Ser Pro Gln Val Asp Pro Lys Ala Gln
            345                 350                 355

aat tta tta ctt aaa caa gtt gag caa cta gcc agc cgc aaa tct         1216
Asn Leu Leu Leu Lys Gln Val Glu Gln Leu Ala Ser Arg Lys Ser
        360                 365                 370

tagaactaac ctttacgcct tta                                           1239


<210> 270
<211> 372
<212> PRT
<213> Corynebacterium glutamicum

<400> 270
Met Asp Asn Gly Met Thr Ile Thr Thr Glu His Ser Thr His Pro Asp
1               5                   10                  15

Leu Asp Phe Asn Asp Glu Ile Tyr Arg Glu Leu Asn Arg Ile Cys Ala
            20                  25                  30

Ser Leu Ser Gln Gln Cys Ser Thr Tyr Gln Pro Glu Phe Arg Thr Cys
        35                  40                  45

Leu Asp Ala Ala Phe Gln Ala Leu Arg Gly Gly Lys Leu Ile Arg Pro
    50                  55                  60

Arg Met Leu Leu Gly Leu Tyr Asn Thr Leu Val Asp Asp Asp Ile Glu
65                  70                  75                  80

Val Lys Leu Asn Thr Val Leu Gln Val Ala Val Ala Leu Glu Leu Leu
                85                  90                  95

His Phe Ser Leu Leu Val His Asp Asp Val Ile Asp Gly Asp Leu Tyr
            100                 105                 110

Arg Arg Gly Lys Leu Asn Phe Ile Gly Gln Ile Leu Met His Arg Thr
        115                 120                 125

Pro Glu Ser Phe Ala Gln Ile Gln Arg Asp Pro Glu His Leu Asp Trp
    130                 135                 140

Ala Gln Ser Asn Gly Leu Leu Met Gly Asn Leu Phe Leu Ala Ala Thr
145                 150                 155                 160

His Gln Ile Phe Ala Arg Leu Asp Leu Pro His His Gln Arg Val Arg
                165                 170                 175

Leu Leu Asp Leu Leu Asn His Thr Ile Asn Asp Thr Ile Val Gly Glu
            180                 185                 190

Phe Leu Asp Val Gly Leu Ser Ser Lys Ala Ile Ser Pro Asn Met Asp
        195                 200                 205

Ile Ala Leu Glu Met Ser Arg Leu Lys Thr Ala Thr Tyr Thr Phe Glu
    210                 215                 220

Leu Pro Met Arg Ala Ala Ala Ile Leu Ala Glu Leu Pro Gln Glu Ile
225                 230                 235                 240

Glu Thr Lys Ile Gly Glu Ile Gly Thr Asn Leu Gly Ile Ala Tyr Gln
                245                 250                 255

Leu Gln Asp Asp Tyr Leu Ser Thr Phe Gly Asp Ala Ala Glu His Gly
            260                 265                 270

Lys Asp Ala Phe Ser Asp Leu Arg Glu Gly Lys Glu Thr Thr Ile Ile
        275                 280                 285

Ala Phe Ala Arg Asp Thr Ala Lys Trp Thr Asp Ile Gln Asp Asn Phe
    290                 295                 300

Gly Ser Ala Asp Leu Ser Thr Ser Gln Ala Glu Arg Ile Gln His Leu
305                 310                 315                 320

Leu Ile Gln Cys Gly Ala Lys Asn His Ser Leu Asn Ala Ile Ser Asp
                325                 330                 335

His Leu Asn Ile Cys Arg Ser Met Ile Lys Thr Leu Ser Pro Gln Val
            340                 345                 350

Asp Pro Lys Ala Gln Asn Leu Leu Leu Lys Gln Val Glu Gln Leu Ala
        355                 360                 365

Ser Arg Lys Ser
    370

<210> 271
<211> 1056
<212> DNA
<213> Corynebacterium glutamicum

<220>
<221> CDS
<222> (101)..(1033)
<223> RXS01879

<400> 271
ctttgcgggc cgctgatatt gatccaacgc ttcgtggcga aaagcttgat gtcactgact 60

atgtgcgcct agctggggtg ttgcagcaaa aggatgagaa gtg aaa att acc gct 115
Val Lys Ile Thr Ala
1               5

aag gcg tgg gcg aaa acc aac ctg cat tta ggt gtg gga ccg gct cac 163
Lys Ala Trp Ala Lys Thr Asn Leu His Leu Gly Val Gly Pro Ala His
                10                  15                  20

gac gat gga ttt cac gag ctc atg acg gtg ttt caa acc att gat ctg 211
Asp Asp Gly Phe His Glu Leu Met Thr Val Phe Gln Thr Ile Asp Leu
            25                  30                  35

ttt gac acc gtc acc tta acc acc ctc gat gag gag ttg gtg gag gag 259
Phe Asp Thr Val Thr Leu Thr Thr Leu Asp Glu Glu Leu Val Glu Glu
        40                  45                  50

ggg agc gtc gtc aag caa tta tct gtg acc ggt gcc cgt ggc gtg cct 307
Gly Ser Val Val Lys Gln Leu Ser Val Thr Gly Ala Arg Gly Val Pro
    55                  60                  65

gag gac gcc agc aat ctt gcg tgg cgc gct gtg gat gcg ttg gtt aag 355
Glu Asp Ala Ser Asn Leu Ala Trp Arg Ala Val Asp Ala Leu Val Lys
70                  75                  80                  85

cgg cgc gcg gaa aag acg ccg ctg tct gca gtt tcg ctg cat att tcc 403
Arg Arg Ala Glu Lys Thr Pro Leu Ser Ala Val Ser Leu His Ile Ser
                90                  95                  100

aag ggg att ccg gtg gct ggc ggc atg gct ggc ggc tct gcg gat gcg 451
Lys Gly Ile Pro Val Ala Gly Gly Met Ala Gly Gly Ser Ala Asp Ala
            105                 110                 115

gct gcg aca ctg cgc gca gtg gat gcc tgg att ggg cct ttc ggc gag 499
Ala Ala Thr Leu Arg Ala Val Asp Ala Trp Ile Gly Pro Phe Gly Glu
        120                 125                 130

gac aca ttg ctg gag gtt gcc gcg gag ctc ggc tca gat gtg ccg ttt 547
Asp Thr Leu Leu Glu Val Ala Ala Glu Leu Gly Ser Asp Val Pro Phe
    135                 140                 145

tgc ctg ctt ggt ggc acc atg cgc ggt acc ggt cgc ggc gag cag ctg 595
Cys Leu Leu Gly Gly Thr Met Arg Gly Thr Gly Arg Gly Glu Gln Leu
150                 155                 160                 165

gta gat atg ttg acg cgc ggc aag cta cat tgg gtg gtg gcc gcg atg 643
Val Asp Met Leu Thr Arg Gly Lys Leu His Trp Val Val Ala Ala Met
                170                 175                 180

gcg cat ggc ctg tcc acg cct gag gta ttc aaa aag cat gat gag ctg 691
Ala His Gly Leu Ser Thr Pro Glu Val Phe Lys Lys His Asp Glu Leu
            185                 190                 195

aat ccg gaa tcg cat atg gat atc agc gac ctc agc gcc gca ctt ctc 739
Asn Pro Glu Ser His Met Asp Ile Ser Asp Leu Ser Ala Ala Leu Leu
        200                 205                 210

acc ggc aac acc gcc gag gtg ggg cag tgg ctg cac aat gat ctg acc 787
Thr Gly Asn Thr Ala Glu Val Gly Gln Trp Leu His Asn Asp Leu Thr
    215                 220                 225

agc gcc gca ctc agt ttg cgc cct gaa ctg cgc agc gtc ctc caa gaa     835
Ser Ala Ala Leu Ser Leu Arg Pro Glu Leu Arg Ser Val Leu Gln Glu
230                 235                 240                 245

ggc atc cgc tcc ggc gcg cat gca gga att gtc tcc ggc tcc ggc ccg     883
Gly Ile Arg Ser Gly Ala His Ala Gly Ile Val Ser Gly Ser Gly Pro
                250                 255                 260

acc acg gta ttc ttg tgc gaa tcg gag cac aaa gcg caa gac gtt aaa     931
Thr Thr Val Phe Leu Cys Glu Ser Glu His Lys Ala Gln Asp Val Lys
            265                 270                 275

gag gcg cta atc gac gcc ggc cag gtg tac gct gct tac acc gcc acc     979
Glu Ala Leu Ile Asp Ala Gly Gln Val Tyr Ala Ala Tyr Thr Ala Thr
        280                 285                 290

ggc cct gcg gcc tca acc gcc gac cag cgc ggc gca cac att ttg act     1027
Gly Pro Ala Ala Ser Thr Ala Asp Gln Arg Gly Ala His Ile Leu Thr
    295                 300                 305

gtt tca taataaagac aaacttaagt atc                                   1056
Val Ser
310


<210> 272
<211> 311
<212> PRT
<213> Corynebacterium glutamicum

<400> 272
Val Lys Ile Thr Ala Lys Ala Trp Ala Lys Thr Asn Leu His Leu Gly
1               5                   10                  15

Val Gly Pro Ala His Asp Asp Gly Phe His Glu Leu Met Thr Val Phe
            20                  25                  30

Gln Thr Ile Asp Leu Phe Asp Thr Val Thr Leu Thr Thr Leu Asp Glu
        35                  40                  45

Glu Leu Val Glu Glu Gly Ser Val Val Lys Gln Leu Ser Val Thr Gly
    50                  55                  60

Ala Arg Gly Val Pro Glu Asp Ala Ser Asn Leu Ala Trp Arg Ala Val
65                  70                  75                  80

Asp Ala Leu Val Lys Arg Arg Ala Glu Lys Thr Pro Leu Ser Ala Val
                85                  90                  95

Ser Leu His Ile Ser Lys Gly Ile Pro Val Ala Gly Gly Met Ala Gly
            100                 105                 110

Gly Ser Ala Asp Ala Ala Ala Thr Leu Arg Ala Val Asp Ala Trp Ile
        115                 120                 125

Gly Pro Phe Gly Glu Asp Thr Leu Leu Glu Val Ala Ala Glu Leu Gly
    130                 135                 140

Ser Asp Val Pro Phe Cys Leu Leu Gly Gly Thr Met Arg Gly Thr Gly
145                 150                 155                 160

Arg Gly Glu Gln Leu Val Asp Met Leu Thr Arg Gly Lys Leu His Trp
                165                 170                 175

Val Val Ala Ala Met Ala His Gly Leu Ser Thr Pro Glu Val Phe Lys
            180                 185                 190

Lys His Asp Glu Leu Asn Pro Glu Ser His Met Asp Ile Ser Asp Leu
        195                 200                 205

Ser Ala Ala Leu Leu Thr Gly Asn Thr Ala Glu Val Gly Gln Trp Leu
    210                 215                 220

His Asn Asp Leu Thr Ser Ala Ala Leu Ser Leu Arg Pro Glu Leu Arg
225                 230                 235                 240

Ser Val Leu Gln Glu Gly Ile Arg Ser Gly Ala His Ala Gly Ile Val
                245                 250                 255

Ser Gly Ser Gly Pro Thr Thr Val Phe Leu Cys Glu Ser Glu His Lys
            260                 265                 270

Ala Gln Asp Val Lys Glu Ala Leu Ile Asp Ala Gly Gln Val Tyr Ala
        275                 280                 285

Ala Tyr Thr Ala Thr Gly Pro Ala Ala Ser Thr Ala Asp Gln Arg Gly
    290                 295                 300

Ala His Ile Leu Thr Val Ser
305                 310

<210> 273
<211> 891
<212> DNA
<213> Corynebacterium glutamicum

<220>
<221> CDS
<222> (101)..(868)
<223> RXS02023

<400> 273
gatgtggcag caattttgaa gcagtacctg agcgagtaac cgcattcggg gttatcgtgg 60

gacttccgaa atgtaactag agactagagg aggaaacacg atg gct cct aaa caa   115
                                           Met Ala Pro Lys Gln
                                           1               5

act ccc agc cca gag aag aat cga aac ctg gtg gga cca gtt ctg caa   163
Thr Pro Ser Pro Glu Lys Asn Arg Asn Leu Val Gly Pro Val Leu Gln
                10                  15                  20

cgt cgg cag aca gag ggt act ttt gat caa cgc ttg cta gaa atg cgc   211
Arg Arg Gln Thr Glu Gly Thr Phe Asp Gln Arg Leu Leu Glu Met Arg
            25                  30                  35

gct gat cac aat tgg aag cac gcc gat cca tgg cgt gta ctg cgt att   259
Ala Asp His Asn Trp Lys His Ala Asp Pro Trp Arg Val Leu Arg Ile
        40                  45                  50

cag tct gag ttt gtg gcg ggt ttt gat gcc ctc cac gag atg cca aag   307

Gln Ser Glu Phe Val Ala Gly Phe Asp Ala Leu His Glu Met Pro Lys
     55                  60                  65

gcc gta acc gtc ttt ggt tcc gca cgc att aaa gag gat cac ccg tac     355
Ala Val Thr Val Phe Gly Ser Ala Arg Ile Lys Glu Asp His Pro Tyr
 70                  75                  80                  85

tac aag gcg ggt gta gaa ctt ggt gaa aag ctc gtt gca gcg gac tac     403
Tyr Lys Ala Gly Val Glu Leu Gly Glu Lys Leu Val Ala Ala Asp Tyr
                 90                  95                 100

gca gtt gtc acc ggt ggc ggt cca ggt ctg atg gaa gcc ccc aat aag     451
Ala Val Val Thr Gly Gly Gly Pro Gly Leu Met Glu Ala Pro Asn Lys
            105                 110                 115

ggg gca agc gag gcc aat ggt tta tca gtt ggt ctg ggc att gag ttg     499
Gly Ala Ser Glu Ala Asn Gly Leu Ser Val Gly Leu Gly Ile Glu Leu
        120                 125                 130

cca cat gaa cag cat ctg aac cct tat gtg gat ttg ggt ctg aac ttc     547
Pro His Glu Gln His Leu Asn Pro Tyr Val Asp Leu Gly Leu Asn Phe
    135                 140                 145

cgg tac ttc ttc gca cgc aag acc atg ttc ctg aaa tac tcc cag gct     595
Arg Tyr Phe Phe Ala Arg Lys Thr Met Phe Leu Lys Tyr Ser Gln Ala
150                 155                 160                 165

ttt gtg tgt ctg cct ggt ggt ttc ggc acg ctc gat gag ctt ttc gag     643
Phe Val Cys Leu Pro Gly Gly Phe Gly Thr Leu Asp Glu Leu Phe Glu
                170                 175                 180

gtc ctc tgc atg gta caa acc ggc aag gta ccc aac ttt ccc atc gtg     691
Val Leu Cys Met Val Gln Thr Gly Lys Val Pro Asn Phe Pro Ile Val
            185                 190                 195

ctg atc ggc act gag ttc tgg gca ggt ttg gtg gat tgg atc cgt cac     739
Leu Ile Gly Thr Glu Phe Trp Ala Gly Leu Val Asp Trp Ile Arg His
        200                 205                 210

cgc ctg gta gag gaa ggc atg atc gat gag aag gat gtt gac cgg atg     787
Arg Leu Val Glu Glu Gly Met Ile Asp Glu Lys Asp Val Asp Arg Met
    215                 220                 225

ttg gtc act gat gac ctg gat cag gcc gtc aaa ttc atc gtc gat gca     835
Leu Val Thr Asp Asp Leu Asp Gln Ala Val Lys Phe Ile Val Asp Ala
230                 235                 240                 245

cac gct gga ttg gac gta gcg cgt ctc cac aat taagcagtgg ctacattagg 888
His Ala Gly Leu Asp Val Ala Arg Leu His Asn
                250                 255

tgt                                                                 891

<210> 274
<211> 256
<212> PRT
<213> Corynebacterium glutamicum

<400> 274
Met Ala Pro Lys Gln Thr Pro Ser Pro Glu Lys Asn Arg Asn Leu Val

1               5                   10                  15

Gly Pro Val Leu Gln Arg Arg Gln Thr Glu Gly Thr Phe Asp Gln Arg
            20                  25                  30

Leu Leu Glu Met Arg Ala Asp His Asn Trp Lys His Ala Asp Pro Trp
        35                  40                  45

Arg Val Leu Arg Ile Gln Ser Glu Phe Val Ala Gly Phe Asp Ala Leu
    50                  55                  60

His Glu Met Pro Lys Ala Val Thr Val Phe Gly Ser Ala Arg Ile Lys
65                  70                  75                  80

Glu Asp His Pro Tyr Tyr Lys Ala Gly Val Glu Leu Gly Glu Lys Leu
                85                  90                  95

Val Ala Ala Asp Tyr Ala Val Val Thr Gly Gly Gly Pro Gly Leu Met
            100                 105                 110

Glu Ala Pro Asn Lys Gly Ala Ser Glu Ala Asn Gly Leu Ser Val Gly
        115                 120                 125

Leu Gly Ile Glu Leu Pro His Glu Gln His Leu Asn Pro Tyr Val Asp
    130                 135                 140

Leu Gly Leu Asn Phe Arg Tyr Phe Phe Ala Arg Lys Thr Met Phe Leu
145                 150                 155                 160

Lys Tyr Ser Gln Ala Phe Val Cys Leu Pro Gly Gly Phe Gly Thr Leu
                165                 170                 175

Asp Glu Leu Phe Glu Val Leu Cys Met Val Gln Thr Gly Lys Val Pro
            180                 185                 190

Asn Phe Pro Ile Val Leu Ile Gly Thr Glu Phe Trp Ala Gly Leu Val
        195                 200                 205

Asp Trp Ile Arg His Arg Leu Val Glu Glu Gly Met Ile Asp Glu Lys
    210                 215                 220

Asp Val Asp Arg Met Leu Val Thr Asp Asp Leu Asp Gln Ala Val Lys
225                 230                 235                 240

Phe Ile Val Asp Ala His Ala Gly Leu Asp Val Ala Arg Leu His Asn
                245                 250                 255

<210> 275
<211> 1242
<212> DNA
<213> Corynebacterium glutamicum

<220>
<221> CDS
<222> (101)..(1219)
<223> RXS00948

<400> 275
acaccctcca aatgatctcg taaaacagta ttgaatttag gtacgactct aatcgtacct 60

tgccctcaag ccaagctagt tgtacgatca aactcgttgt atg gca aac gtc gta 115
Met Ala Asn Val Val
1               5

cta gtc gat cga atg gag cct ttg gtg tcc aag ctg ttt acc cca att 163
Leu Val Asp Arg Met Glu Pro Leu Val Ser Lys Leu Phe Thr Pro Ile
                10                  15                  20

caa atc cgc gac atc acc atc ccc aac cgc gtg tgg atg tca ccg atg 211
Gln Ile Arg Asp Ile Thr Ile Pro Asn Arg Val Trp Met Ser Pro Met
            25                  30                  35

tgc acc tac tct gca gcc acc ggt tca ggt ctt ccc acc gat ttt cac 259
Cys Thr Tyr Ser Ala Ala Thr Gly Ser Gly Leu Pro Thr Asp Phe His
        40                  45                  50

cag gct cat tac gca gct cgc gca gca ggt ggt gtc gga tta gtc atg 307
Gln Ala His Tyr Ala Ala Arg Ala Ala Gly Gly Val Gly Leu Val Met
    55                  60                  65

gtt gaa gca act gga gtg aac ccc gta gct ccc atc tcc cca gtc gac 355
Val Glu Ala Thr Gly Val Asn Pro Val Ala Pro Ile Ser Pro Val Asp
70                  75                  80                  85

ctt gga ctt tgg agc cat gac caa att gaa cca ttc tcc cga gtg aca 403
Leu Gly Leu Trp Ser His Asp Gln Ile Glu Pro Phe Ser Arg Val Thr
                90                  95                  100

gca gct att cgc gcc ggt ggg gca gta ccg gcc gtt caa tta gcc cat 451
Ala Ala Ile Arg Ala Gly Gly Ala Val Pro Ala Val Gln Leu Ala His
            105                 110                 115

gct ggc cgc aag gca tcc acc gat gct ccg tgg aat ggt ggc gga tat 499
Ala Gly Arg Lys Ala Ser Thr Asp Ala Pro Trp Asn Gly Gly Gly Tyr
        120                 125                 130

gtt gga cca gaa acc aat gga tgg gag act gtc ggc ccc agc cct ctg 547
Val Gly Pro Glu Thr Asn Gly Trp Glu Thr Val Gly Pro Ser Pro Leu
    135                 140                 145

gca ttc cca ggt ttg cct gct ccg cgc gag ctg acg gtt tca gaa atc 595
Ala Phe Pro Gly Leu Pro Ala Pro Arg Glu Leu Thr Val Ser Glu Ile
150                 155                 160                 165

caa gag gtt gtg cag cag ttc gct ggc gcc gcc gtt cgt gcc gat cag 643
Gln Glu Val Val Gln Gln Phe Ala Gly Ala Ala Val Arg Ala Asp Gln
                170                 175                 180

gct ggt ttt gat gtc gtg gaa att cac gca gca cac ggc tac ctt ttg 691
Ala Gly Phe Asp Val Val Glu Ile His Ala Ala His Gly Tyr Leu Leu
            185                 190                 195

cat aac ttc ctt tct ccg atc tcc aac aag cgc acc gat tca tac ggc 739
His Asn Phe Leu Ser Pro Ile Ser Asn Lys Arg Thr Asp Ser Tyr Gly
        200                 205                 210

gga tct tta gaa aac cgc gct cgc atc gtg ctc gaa gtc att gat gca 787
Gly Ser Leu Glu Asn Arg Ala Arg Ile Val Leu Glu Val Ile Asp Ala

215                 220                 225

atc cgc gca gtg tgg cca gag gaa aag cct gta ttc atg cgc att tcc     835
Ile Arg Ala Val Trp Pro Glu Glu Lys Pro Val Phe Met Arg Ile Ser
230                 235                 240                 245

acc acc gac tgg gtg gag gaa aac cca cag gat gat cgc gag tcc tgg     883
Thr Thr Asp Trp Val Glu Glu Asn Pro Gln Asp Asp Arg Glu Ser Trp
                250                 255                 260

acg ctg agc caa agc agg cag ctg gct ttg tgg gca tcc gag cac gga     931
Thr Leu Ser Gln Ser Arg Gln Leu Ala Leu Trp Ala Ser Glu His Gly
            265                 270                 275

gtt gat ttg atc gat gcc tct tct ggt ggc ctc gac atc gtc ccc att     979
Val Asp Leu Ile Asp Ala Ser Ser Gly Gly Leu Asp Ile Val Pro Ile
        280                 285                 290

ccg cat gac cgc gat tac caa acc gcg aag gcc gca gat ctt cac gca     1027
Pro His Asp Arg Asp Tyr Gln Thr Ala Lys Ala Ala Asp Leu His Ala
    295                 300                 305

agt acc gga gtg aca gtc gct gct gtg ggg cgc att gat gac gcc caa     1075
Ser Thr Gly Val Thr Val Ala Ala Val Gly Arg Ile Asp Asp Ala Gln
310                 315                 320                 325

act gcg cac aat ttg gtt gat tct ggc gat gtc aat gca gtt ttc ctc     1123
Thr Ala His Asn Leu Val Asp Ser Gly Asp Val Asn Ala Val Phe Leu
                330                 335                 340

ggc cgt cca ctg ctc aag gat cct tcc tgg gca aac caa gca gcc ctc     1171
Gly Arg Pro Leu Leu Lys Asp Pro Ser Trp Ala Asn Gln Ala Ala Leu
            345                 350                 355

gca cta ggt gcg gaa ccc agg tat gtt cac caa tac gac tac gta ctt     1219
Ala Leu Gly Ala Glu Pro Arg Tyr Val His Gln Tyr Asp Tyr Val Leu
        360                 365                 370

taaaggagag ttgacatgaa ggt                                           1242


<210> 276
<211> 373
<212> PRT
<213> Corynebacterium glutamicum

<400> 276
Met Ala Asn Val Val Leu Val Asp Arg Met Glu Pro Leu Val Ser Lys
1               5                   10                  15

Leu Phe Thr Pro Ile Gln Ile Arg Asp Ile Thr Ile Pro Asn Arg Val
            20                  25                  30

Trp Met Ser Pro Met Cys Thr Tyr Ser Ala Ala Thr Gly Ser Gly Leu
        35                  40                  45

Pro Thr Asp Phe His Gln Ala His Tyr Ala Ala Arg Ala Ala Gly Gly
    50                  55                  60

Val Gly Leu Val Met Val Glu Ala Thr Gly Val Asn Pro Val Ala Pro
65                  70                  75                  80

Ile Ser Pro Val Asp Leu Gly Leu Trp Ser His Asp Gln Ile Glu Pro
                85                  90                  95

Phe Ser Arg Val Thr Ala Ala Ile Arg Ala Gly Gly Ala Val Pro Ala
            100                 105                 110

Val Gln Leu Ala His Ala Gly Arg Lys Ala Ser Thr Asp Ala Pro Trp
        115                 120                 125

Asn Gly Gly Gly Tyr Val Gly Pro Glu Thr Asn Gly Trp Glu Thr Val
    130                 135                 140

Gly Pro Ser Pro Leu Ala Phe Pro Gly Leu Pro Ala Pro Arg Glu Leu
145                 150                 155                 160

Thr Val Ser Glu Ile Gln Glu Val Val Gln Gln Phe Ala Gly Ala Ala
                165                 170                 175

Val Arg Ala Asp Gln Ala Gly Phe Asp Val Val Glu Ile His Ala Ala
            180                 185                 190

His Gly Tyr Leu Leu His Asn Phe Leu Ser Pro Ile Ser Asn Lys Arg
        195                 200                 205

Thr Asp Ser Tyr Gly Gly Ser Leu Glu Asn Arg Ala Arg Ile Val Leu
    210                 215                 220

Glu Val Ile Asp Ala Ile Arg Ala Val Trp Pro Glu Glu Lys Pro Val
225                 230                 235                 240

Phe Met Arg Ile Ser Thr Thr Asp Trp Val Glu Glu Asn Pro Gln Asp
                245                 250                 255

Asp Arg Glu Ser Trp Thr Leu Ser Gln Ser Arg Gln Leu Ala Leu Trp
            260                 265                 270

Ala Ser Glu His Gly Val Asp Leu Ile Asp Ala Ser Ser Gly Gly Leu
        275                 280                 285

Asp Ile Val Pro Ile Pro His Asp Arg Asp Tyr Gln Thr Ala Lys Ala
    290                 295                 300

Ala Asp Leu His Ala Ser Thr Gly Val Thr Val Ala Ala Val Gly Arg
305                 310                 315                 320

Ile Asp Asp Ala Gln Thr Ala His Asn Leu Val Asp Ser Gly Asp Val
                325                 330                 335

Asn Ala Val Phe Leu Gly Arg Pro Leu Leu Lys Asp Pro Ser Trp Ala
            340                 345                 350

Asn Gln Ala Ala Leu Ala Leu Gly Ala Glu Pro Arg Tyr Val His Gln
        355                 360                 365

Tyr Asp Tyr Val Leu
    370

<210> 277
<211> 1026

<212> DNA
<213> Corynebacterium glutamicum

<220>
<221> CDS
<222> (101)..(1003)
<223> RXS02228

<400> 277
acagaagcgt caactgctca acttctcgac agcgacgaca cacacggcgt gggacgctac 60

gttgctcgct ggacttttta acttaaggga gatctagatc gtg gta aca ccg atc 115
Val Val Thr Pro Ile
1               5

gca gtg gtt gga ccc act gca tct gga aaa tca gct ttg gga att gct 163
Ala Val Val Gly Pro Thr Ala Ser Gly Lys Ser Ala Leu Gly Ile Ala
                10                  15                  20

cta gcc cac aag ctt gac ggt gaa gta gtc aat gtg gat tcc atg cag 211
Leu Ala His Lys Leu Asp Gly Glu Val Val Asn Val Asp Ser Met Gln
            25                  30                  35

ctg tac aaa ggc atg gac atc ggc acg gca aag ctg act gtc gaa gaa 259
Leu Tyr Lys Gly Met Asp Ile Gly Thr Ala Lys Leu Thr Val Glu Glu
        40                  45                  50

cgc gaa ggc att gcg cat cat cag ctc gat gtc tgg gac gtt acc gaa 307
Arg Glu Gly Ile Ala His His Gln Leu Asp Val Trp Asp Val Thr Glu
    55                  60                  65

act gcg tca gtg gcg agg ttt caa tcc gac gcc gtt gcc gat gtg gaa 355
Thr Ala Ser Val Ala Arg Phe Gln Ser Asp Ala Val Ala Asp Val Glu
70                  75                  80                  85

gat att atg tcc cgt ggc aaa acc ccc atc ttg gtt ggc ggc tcc atg 403
Asp Ile Met Ser Arg Gly Lys Thr Pro Ile Leu Val Gly Gly Ser Met
                90                  95                  100

ttg tac gtc caa tct ttg gtc gat gat tgg caa ttc cca cct acc gac 451
Leu Tyr Val Gln Ser Leu Val Asp Asp Trp Gln Phe Pro Pro Thr Asp
            105                 110                 115

agc gct gtt cgc gca cgc ttt gag gcc cgc ttg gca gac atc ggt gtc 499
Ser Ala Val Arg Ala Arg Phe Glu Ala Arg Leu Ala Asp Ile Gly Val
        120                 125                 130

gaa gca cta cac gct gaa ctt act cag ctt gac cca gaa gca gca gcc 547
Glu Ala Leu His Ala Glu Leu Thr Gln Leu Asp Pro Glu Ala Ala Ala
    135                 140                 145

gtc atc gaa agc aat gat ccc cga cgc acc gtc cga gca tta gaa gtc 595
Val Ile Glu Ser Asn Asp Pro Arg Arg Thr Val Arg Ala Leu Glu Val
150                 155                 160                 165

att gaa cta acc ggc cag ccc ttc caa gca agc caa ccg ccc aaa gac 643
Ile Glu Leu Thr Gly Gln Pro Phe Gln Ala Ser Gln Pro Pro Lys Asp
                170                 175                 180

gcg cca cct cgc tgg gga act cga atc att ggc ctg aaa acc act cca 691
Ala Pro Pro Arg Trp Gly Thr Arg Ile Ile Gly Leu Lys Thr Thr Pro

185                 190                 195

gaa tgg cta aat cca cgc atc gag cag cgc acc gcc agg atg ttt gaa     739
Glu Trp Leu Asn Pro Arg Ile Glu Gln Arg Thr Ala Arg Met Phe Glu
        200                 205                 210

caa ggc ttt gtc gcc gaa gtg gaa cac ctt gtg cag caa gga ctc atc     787
Gln Gly Phe Val Ala Glu Val Glu His Leu Val Gln Gln Gly Leu Ile
    215                 220                 225

gct gac tcc acc gcg gga cga gca atc ggc tac tcc caa gta ctg gca     835
Ala Asp Ser Thr Ala Gly Arg Ala Ile Gly Tyr Ser Gln Val Leu Ala
230                 235                 240                 245

gcc atg gca ggg gag atg acc tgg gaa gac gcc ttc gaa cgc acg gtc     883
Ala Met Ala Gly Glu Met Thr Trp Glu Asp Ala Phe Glu Arg Thr Val
                250                 255                 260

acc gga acc aga cgc tat gtc agg cgc caa cgc agc tgg ttc aac aga     931
Thr Gly Thr Arg Arg Tyr Val Arg Arg Gln Arg Ser Trp Phe Asn Arg
            265                 270                 275

gac cac cgc gtg tcc tgg gtc gac gcc tct ggc gat ccc acc gca caa     979
Asp His Arg Val Ser Trp Val Asp Ala Ser Gly Asp Pro Thr Ala Gln
        280                 285                 290

gcc ttg gag att ttg ggt cta caa tagcgagggt gaatttgacc atc          1026
Ala Leu Glu Ile Leu Gly Leu Gln
    295                 300


<210> 278
<211> 301
<212> PRT
<213> Corynebacterium glutamicum

<400> 278
Val Val Thr Pro Ile Ala Val Val Gly Pro Thr Ala Ser Gly Lys Ser
1               5                   10                  15

Ala Leu Gly Ile Ala Leu Ala His Lys Leu Asp Gly Glu Val Val Asn
            20                  25                  30

Val Asp Ser Met Gln Leu Tyr Lys Gly Met Asp Ile Gly Thr Ala Lys
        35                  40                  45

Leu Thr Val Glu Glu Arg Glu Gly Ile Ala His His Gln Leu Asp Val
    50                  55                  60

Trp Asp Val Thr Glu Thr Ala Ser Val Ala Arg Phe Gln Ser Asp Ala
65                  70                  75                  80

Val Ala Asp Val Glu Asp Ile Met Ser Arg Gly Lys Thr Pro Ile Leu
                85                  90                  95

Val Gly Gly Ser Met Leu Tyr Val Gln Ser Leu Val Asp Asp Trp Gln
            100                 105                 110

Phe Pro Pro Thr Asp Ser Ala Val Arg Ala Arg Phe Glu Ala Arg Leu
        115                 120                 125

Ala Asp Ile Gly Val Glu Ala Leu His Ala Glu Leu Thr Gln Leu Asp
    130                 135                 140

Pro Glu Ala Ala Ala Val Ile Glu Ser Asn Asp Pro Arg Arg Thr Val
145                 150                 155                 160

Arg Ala Leu Glu Val Ile Glu Leu Thr Gly Gln Pro Phe Gln Ala Ser
                165                 170                 175

Gln Pro Pro Lys Asp Ala Pro Pro Arg Trp Gly Thr Arg Ile Ile Gly
            180                 185                 190

Leu Lys Thr Thr Pro Glu Trp Leu Asn Pro Arg Ile Glu Gln Arg Thr
        195                 200                 205

Ala Arg Met Phe Glu Gln Gly Phe Val Ala Glu Val Glu His Leu Val
    210                 215                 220

Gln Gln Gly Leu Ile Ala Asp Ser Thr Ala Gly Arg Ala Ile Gly Tyr
225                 230                 235                 240

Ser Gln Val Leu Ala Ala Met Ala Gly Glu Met Thr Trp Glu Asp Ala
                245                 250                 255

Phe Glu Arg Thr Val Thr Gly Thr Arg Arg Tyr Val Arg Arg Gln Arg
            260                 265                 270

Ser Trp Phe Asn Arg Asp His Arg Val Ser Trp Val Asp Ala Ser Gly
        275                 280                 285

Asp Pro Thr Ala Gln Ala Leu Glu Ile Leu Gly Leu Gln
    290                 295                 300


<210> 279
<211> 954
<212> DNA
<213> Corynebacterium glutamicum

<220>
<221> CDS
<222> (101)..(931)
<223> RXC01971

<400> 279
aggtcttgtt tatttcggct actgattcag tagctgcgct ccgataggat tcttagtttt 60

cagttcagta tctttgagcc acggctagaa tgtgaatcct atg tct aag aag aag   115
                                           Met Ser Lys Lys Lys
                                           1               5

cct cgc ccc att ccg gtt cct gcc caa ttt atc cct ggt ctc att gat   163
Pro Arg Pro Ile Pro Val Pro Ala Gln Phe Ile Pro Gly Leu Ile Asp
                10                  15                  20

gcg cat aca cat ttg gca tcg tgt gga gga gat ctt gca ggg ttg gtg   211
Ala His Thr His Leu Ala Ser Cys Gly Gly Asp Leu Ala Gly Leu Val
            25                  30                  35

gaa agg gcc aag gag gcg ggc gtc gaa aag ctt tgt acc gtc ggt gat   259
Glu Arg Ala Lys Glu Ala Gly Val Glu Lys Leu Cys Thr Val Gly Asp

40                  45                  50

ggt ttg gct gag gcc gag ctt gcg ctg gag gcc gcg caa cag ttt ggc     307
Gly Leu Ala Glu Ala Glu Leu Ala Leu Glu Ala Ala Gln Gln Phe Gly
    55                  60                  65

aat gtg ttt gct gcg tgt gcg att cat ccg acg aag gct gat cag ttg     355
Asn Val Phe Ala Ala Cys Ala Ile His Pro Thr Lys Ala Asp Gln Leu
70                  75                  80                  85

gat ggg gct gcg cgt gcg cgg ctg acg cag atg gcg gcg gat ccg aat     403
Asp Gly Ala Ala Arg Ala Arg Leu Thr Gln Met Ala Ala Asp Pro Asn
                90                  95                  100

tgt gtg gcc att ggt gag act ggt ttg gat tcg tat tgg atc aag cac     451
Cys Val Ala Ile Gly Glu Thr Gly Leu Asp Ser Tyr Trp Ile Lys His
            105                 110                 115

gat cca gag gac acg gcg gcg ttg gat gtg caa gag gag gcg ctg cgc     499
Asp Pro Glu Asp Thr Ala Ala Leu Asp Val Gln Glu Glu Ala Leu Arg
        120                 125                 130

tgg cat att gat ttg gca att agt gcg gat aag ccg ttg atg att cac     547
Trp His Ile Asp Leu Ala Ile Ser Ala Asp Lys Pro Leu Met Ile His
    135                 140                 145

aat cgt gag gcg gat gct gat ttg atg cga gtg ttg gcg gat gct cca     595
Asn Arg Glu Ala Asp Ala Asp Leu Met Arg Val Leu Ala Asp Ala Pro
150                 155                 160                 165

cct cca aaa gat acg att ctg cat tgt ttt tct tcg ccg ttg gac gtg     643
Pro Pro Lys Asp Thr Ile Leu His Cys Phe Ser Ser Pro Leu Asp Val
                170                 175                 180

gcg aag gaa gcg ttg gat cgt gga tat gtg ttg agt ttt gcg ggc aat     691
Ala Lys Glu Ala Leu Asp Arg Gly Tyr Val Leu Ser Phe Ala Gly Asn
            185                 190                 195

gtg acg ttt aag cgt aat gag gag ttg cgg gag gct gct cgt att gcg     739
Val Thr Phe Lys Arg Asn Glu Glu Leu Arg Glu Ala Ala Arg Ile Ala
        200                 205                 210

ccg att tcc cag att ttg att gaa acc gat gcg ccg tat atg acg ccg     787
Pro Ile Ser Gln Ile Leu Ile Glu Thr Asp Ala Pro Tyr Met Thr Pro
    215                 220                 225

gag ccg ttt cgg ggg agt agg aat gag ccg tcg ttg att ggt cat acg     835
Glu Pro Phe Arg Gly Ser Arg Asn Glu Pro Ser Leu Ile Gly His Thr
230                 235                 240                 245

gcg cta tgc att gcg gag gtt cgg ggg atg gct gtg gag gat gtt gcg     883
Ala Leu Cys Ile Ala Glu Val Arg Gly Met Ala Val Glu Asp Val Ala
                250                 255                 260

gcg gct ttg aat gag aat ttt gat cgc gtt tat ggg gtc aca aat cta     931
Ala Ala Leu Asn Glu Asn Phe Asp Arg Val Tyr Gly Val Thr Asn Leu
            265                 270                 275

taacgtgagg tagctcacag tca                                           954

<210> 280
<211> 277
<212> PRT
<213> Corynebacterium glutamicum

<400> 280
Met Ser Lys Lys Lys Pro Arg Pro Ile Pro Val Pro Ala Gln Phe Ile
1               5                   10                  15

Pro Gly Leu Ile Asp Ala His Thr His Leu Ala Ser Cys Gly Gly Asp
            20                  25                  30

Leu Ala Gly Leu Val Glu Arg Ala Lys Glu Ala Gly Val Glu Lys Leu
        35                  40                  45

Cys Thr Val Gly Asp Gly Leu Ala Glu Ala Glu Leu Ala Leu Glu Ala
    50                  55                  60

Ala Gln Gln Phe Gly Asn Val Phe Ala Ala Cys Ala Ile His Pro Thr
65                  70                  75                  80

Lys Ala Asp Gln Leu Asp Gly Ala Ala Arg Ala Arg Leu Thr Gln Met
                85                  90                  95

Ala Ala Asp Pro Asn Cys Val Ala Ile Gly Glu Thr Gly Leu Asp Ser
            100                 105                 110

Tyr Trp Ile Lys His Asp Pro Glu Asp Thr Ala Ala Leu Asp Val Gln
        115                 120                 125

Glu Glu Ala Leu Arg Trp His Ile Asp Leu Ala Ile Ser Ala Asp Lys
    130                 135                 140

Pro Leu Met Ile His Asn Arg Glu Ala Asp Ala Asp Leu Met Arg Val
145                 150                 155                 160

Leu Ala Asp Ala Pro Pro Pro Lys Asp Thr Ile Leu His Cys Phe Ser
                165                 170                 175

Ser Pro Leu Asp Val Ala Lys Glu Ala Leu Asp Arg Gly Tyr Val Leu
            180                 185                 190

Ser Phe Ala Gly Asn Val Thr Phe Lys Arg Asn Glu Glu Leu Arg Glu
        195                 200                 205

Ala Ala Arg Ile Ala Pro Ile Ser Gln Ile Leu Ile Glu Thr Asp Ala
    210                 215                 220

Pro Tyr Met Thr Pro Glu Pro Phe Arg Gly Ser Arg Asn Glu Pro Ser
225                 230                 235                 240

Leu Ile Gly His Thr Ala Leu Cys Ile Ala Glu Val Arg Gly Met Ala
                245                 250                 255

Val Glu Asp Val Ala Ala Ala Leu Asn Glu Asn Phe Asp Arg Val Tyr
            260                 265                 270

Gly Val Thr Asn Leu
        275

<210> 281
<211> 1650
<212> DNA
<213> Corynebacterium glutamicum

<220>
<221> CDS
<222> (101)..(1627)
<223> RXC02697

<400> 281
tttggctcac ctcgatgatg tagacatccc cgatgaggtg cgcgcacagt tgcgggcact 60

ggctatccgc tcaaccgaac gtcggatgta gtagacgcgt atg aca ctt ttt caa 115
Met Thr Leu Phe Gln
1               5

cgt tta acc aac cct gta gtg ctc ggc ggc cta gca ggt gtt ttg ctt 163
Arg Leu Thr Asn Pro Val Val Leu Gly Gly Leu Ala Gly Val Leu Leu
                10                  15                  20

ctg ctc ggc tct ttc ggt ggc ggt gcc att cgg tac cgt ggc gga gtg 211
Leu Leu Gly Ser Phe Gly Gly Gly Ala Ile Arg Tyr Arg Gly Gly Val
            25                  30                  35

ctc gat gcg ttg ggg ctt aac ttc ctt gct ttt ggc cac gcg cag ggt 259
Leu Asp Ala Leu Gly Leu Asn Phe Leu Ala Phe Gly His Ala Gln Gly
        40                  45                  50

att tcc aat acc gtg ttg tgg gtt ggg cag ctg ctg ctg att ggc gcg 307
Ile Ser Asn Thr Val Leu Trp Val Gly Gln Leu Leu Leu Ile Gly Ala
    55                  60                  65

tgg gtt cac ctt gga cgt cgg ttg ttc aag aaa aaa gtc gct gat gac 355
Trp Val His Leu Gly Arg Arg Leu Phe Lys Lys Lys Val Ala Asp Asp
70                  75                  80                  85

acc gca gac gct gct gac tta ggt ctt gta aag cgc acg ttg tat gcc 403
Thr Ala Asp Ala Ala Asp Leu Gly Leu Val Lys Arg Thr Leu Tyr Ala
                90                  95                  100

atg gtg gtg ccc ctc att ttt gcg gca cca atg atg tcg cgt gat gtt 451
Met Val Val Pro Leu Ile Phe Ala Ala Pro Met Met Ser Arg Asp Val
            105                 110                 115

tat tcc tat ctc atg cag ggc gcg atg ctg cgt gat ggc ttc gat ccc 499
Tyr Ser Tyr Leu Met Gln Gly Ala Met Leu Arg Asp Gly Phe Asp Pro
        120                 125                 130

tac act gag ggc gct gcg gta aac cct ggc ccc atg ttg ctt gag gtc 547
Tyr Thr Glu Gly Ala Ala Val Asn Pro Gly Pro Met Leu Leu Glu Val
    135                 140                 145

tct cat gat tgg cgc aac acc acg acg ccg tat ggt cca cta cac ctg 595
Ser His Asp Trp Arg Asn Thr Thr Thr Pro Tyr Gly Pro Leu His Leu
150                 155                 160                 165

tgg att gga gac atg atc acc acg gtt gtg ggc gat aat gtc acc ttg 643
Trp Ile Gly Asp Met Ile Thr Thr Val Val Gly Asp Asn Val Thr Leu
                170                 175                 180

ggc gtc gtc gct tac aag atc ttg tcg atc att ggc ctt gct gtg aca 691
Gly Val Val Ala Tyr Lys Ile Leu Ser Ile Ile Gly Leu Ala Val Thr
            185                 190                 195

ggc tgg agc att gtc cgc att gca caa cat ttt gga gcc aac cca gca 739
Gly Trp Ser Ile Val Arg Ile Ala Gln His Phe Gly Ala Asn Pro Ala
        200                 205                 210

att gca ttg tgg att ggt gtg gcc aat cct gtg atg atc atc cac atg 787
Ile Ala Leu Trp Ile Gly Val Ala Asn Pro Val Met Ile Ile His Met
    215                 220                 225

atc ggc ggc atg cac aat gaa tcc ctc atg gtg gga ttg gtc agc gtc 835
Ile Gly Gly Met His Asn Glu Ser Leu Met Val Gly Leu Val Ser Val
230                 235                 240                 245

ggc ttg ttg cta gca ctg aag aag cgt ttc gtg gca ggt gtg gca ctc 883
Gly Leu Leu Leu Ala Leu Lys Lys Arg Phe Val Ala Gly Val Ala Leu
                250                 255                 260

att gca gtg gct gtg tcg ctg aaa gct aca gcg gcg att gca ctt cct 931
Ile Ala Val Ala Val Ser Leu Lys Ala Thr Ala Ala Ile Ala Leu Pro
            265                 270                 275

ttt gtg gtg tgg atc ggc atg cat cat ttc gca gga ttc tta gcc acc 979
Phe Val Val Trp Ile Gly Met His His Phe Ala Gly Phe Leu Ala Thr
        280                 285                 290

aaa aag ggc aaa gac tcc cct acc ctt aag caa cag gtc ccc gcg ttc 1027
Lys Lys Gly Lys Asp Ser Pro Thr Leu Lys Gln Gln Val Pro Ala Phe
    295                 300                 305

ttt gcc act gga gct gca ggt gtt gct gtc act ggt gtt gtt gtc agt 1075
Phe Ala Thr Gly Ala Ala Gly Val Ala Val Thr Gly Val Val Val Ser
310                 315                 320                 325

gcg atc act tgg gcg tct ggc gct tcg tgg ggc tgg atc agt gag atc 1123
Ala Ile Thr Trp Ala Ser Gly Ala Ser Trp Gly Trp Ile Ser Glu Ile
                330                 335                 340

agt ggc aac agc aag gta atc aac ccg ctg gct ttc cct tct ttg gtg 1171
Ser Gly Asn Ser Lys Val Ile Asn Pro Leu Ala Phe Pro Ser Leu Val
            345                 350                 355

gcc agt gtg atc acc atg gtg gct gaa gtg ttc gtt gac gat ttc gac 1219
Ala Ser Val Ile Thr Met Val Ala Glu Val Phe Val Asp Asp Phe Asp
        360                 365                 370

tac aac gca gtg gtt aat gtt gtg cgc tca atc tcc atg ctg atc atg 1267
Tyr Asn Ala Val Val Asn Val Val Arg Ser Ile Ser Met Leu Ile Met
    375                 380                 385

ctt ggc ggg ttg gtc gta tgt tgg tgg ctg ttc cgc cag aac gaa cgc 1315
Leu Gly Gly Leu Val Val Cys Trp Trp Leu Phe Arg Gln Asn Glu Arg
390                 395                 400                 405

agg gcg gtc act ggt aca gca gcg gct tat gcc gtg gct ttt gtg ttc 1363
Arg Ala Val Thr Gly Thr Ala Ala Ala Tyr Ala Val Ala Phe Val Phe
                410                 415                 420

aat tct gtg acc ttg ccg tgg tac tac gcc agc ttg atc tct ttg ctc 1411

Asn Ser Val Thr Leu Pro Trp Tyr Tyr Ala Ser Leu Ile Ser Leu Leu
            425                 430                 435

ggc aca ttt aaa cca ccg atg tgg ttg att cgc ttc gca gcg ggt gct    1459
Gly Thr Phe Lys Pro Pro Met Trp Leu Ile Arg Phe Ala Ala Gly Ala
        440                 445                 450

tcg gtg ttt atc gcg ctg atg ttt acc gga agt gga aac cac cag ctg    1507
Ser Val Phe Ile Ala Leu Met Phe Thr Gly Ser Gly Asn His Gln Leu
    455                 460                 465

tac aac atc gtt acg gtg atc atc gca gca att atc gcg tgg ctt gcc    1555
Tyr Asn Ile Val Thr Val Ile Ile Ala Ala Ile Ile Ala Trp Leu Ala
470                 475                 480                 485

acc gtg gtg atc ttt gat gac act gac cct gca aca acg gcc acg gag    1603
Thr Val Val Ile Phe Asp Asp Thr Asp Pro Ala Thr Thr Ala Thr Glu
                490                 495                 500

aaa ccc tcc ccg cat acc gtt tcc tagttgcata aggtaaaccg cca          1650
Lys Pro Ser Pro His Thr Val Ser
            505

<210> 282
<211> 509
<212> PRT
<213> Corynebacterium glutamicum

<400> 282
Met Thr Leu Phe Gln Arg Leu Thr Asn Pro Val Val Leu Gly Gly Leu
  1               5                  10                  15

Ala Gly Val Leu Leu Leu Leu Gly Ser Phe Gly Gly Gly Ala Ile Arg
             20                  25                  30

Tyr Arg Gly Gly Val Leu Asp Ala Leu Gly Leu Asn Phe Leu Ala Phe
         35                  40                  45

Gly His Ala Gln Gly Ile Ser Asn Thr Val Leu Trp Val Gly Gln Leu
     50                  55                  60

Leu Leu Ile Gly Ala Trp Val His Leu Gly Arg Arg Leu Phe Lys Lys
 65                  70                  75                  80

Lys Val Ala Asp Asp Thr Ala Asp Ala Ala Asp Leu Gly Leu Val Lys
                 85                  90                  95

Arg Thr Leu Tyr Ala Met Val Val Pro Leu Ile Phe Ala Ala Pro Met
            100                 105                 110

Met Ser Arg Asp Val Tyr Ser Tyr Leu Met Gln Gly Ala Met Leu Arg
        115                 120                 125

Asp Gly Phe Asp Pro Tyr Thr Glu Gly Ala Ala Val Asn Pro Gly Pro
    130                 135                 140

Met Leu Leu Glu Val Ser His Asp Trp Arg Asn Thr Thr Thr Pro Tyr
145                 150                 155                 160

Gly Pro Leu His Leu Trp Ile Gly Asp Met Ile Thr Thr Val Val Gly

                165                 170                 175

Asp Asn Val Thr Leu Gly Val Val Ala Tyr Lys Ile Leu Ser Ile Ile
            180                 185                 190

Gly Leu Ala Val Thr Gly Trp Ser Ile Val Arg Ile Ala Gln His Phe
        195                 200                 205

Gly Ala Asn Pro Ala Ile Ala Leu Trp Ile Gly Val Ala Asn Pro Val
    210                 215                 220

Met Ile Ile His Met Ile Gly Gly Met His Asn Glu Ser Leu Met Val
225                 230                 235                 240

Gly Leu Val Ser Val Gly Leu Leu Leu Ala Leu Lys Lys Arg Phe Val
                245                 250                 255

Ala Gly Val Ala Leu Ile Ala Val Ala Val Ser Leu Lys Ala Thr Ala
            260                 265                 270

Ala Ile Ala Leu Pro Phe Val Val Trp Ile Gly Met His His Phe Ala
        275                 280                 285

Gly Phe Leu Ala Thr Lys Lys Gly Lys Asp Ser Pro Thr Leu Lys Gln
    290                 295                 300

Gln Val Pro Ala Phe Phe Ala Thr Gly Ala Ala Gly Val Ala Val Thr
305                 310                 315                 320

Gly Val Val Val Ser Ala Ile Thr Trp Ala Ser Gly Ala Ser Trp Gly
                325                 330                 335

Trp Ile Ser Glu Ile Ser Gly Asn Ser Lys Val Ile Asn Pro Leu Ala
            340                 345                 350

Phe Pro Ser Leu Val Ala Ser Val Ile Thr Met Val Ala Glu Val Phe
        355                 360                 365

Val Asp Asp Phe Asp Tyr Asn Ala Val Val Asn Val Val Arg Ser Ile
    370                 375                 380

Ser Met Leu Ile Met Leu Gly Gly Leu Val Val Cys Trp Trp Leu Phe
385                 390                 395                 400

Arg Gln Asn Glu Arg Arg Ala Val Thr Gly Thr Ala Ala Ala Tyr Ala
                405                 410                 415

Val Ala Phe Val Phe Asn Ser Val Thr Leu Pro Trp Tyr Tyr Ala Ser
            420                 425                 430

Leu Ile Ser Leu Leu Gly Thr Phe Lys Pro Pro Met Trp Leu Ile Arg
        435                 440                 445

Phe Ala Ala Gly Ala Ser Val Phe Ile Ala Leu Met Phe Thr Gly Ser
    450                 455                 460

Gly Asn His Gln Leu Tyr Asn Ile Val Thr Val Ile Ile Ala Ala Ile
465                 470                 475                 480

Ile Ala Trp Leu Ala Thr Val Val Ile Phe Asp Asp Thr Asp Pro Ala
                485                 490                 495

Thr Thr Ala Thr Glu Lys Pro Ser Pro His Thr Val Ser
            500                 505

<210> 283
<211> 1298
<212> DNA
<213> Corynebacterium glutamicum

<220>
<221> CDS
<222> (1)..(1275)
<223> RXN01946

<400> 283
atc cgc aag tac tcc agg ctc gag gaa caa ttc cag tcg ctc ggc ggc      48
Ile Arg Lys Tyr Ser Arg Leu Glu Glu Gln Phe Gln Ser Leu Gly Gly
  1               5                  10                  15

tac gaa gct gac gcc gaa gca gcc cag atc tgc gac aac ctc ggc ctc      96
Tyr Glu Ala Asp Ala Glu Ala Ala Gln Ile Cys Asp Asn Leu Gly Leu
             20                  25                  30

gag gca cgc atc ctc gac cag cag ctt aaa acc ctg tcc ggc ggc cag     144
Glu Ala Arg Ile Leu Asp Gln Gln Leu Lys Thr Leu Ser Gly Gly Gln
         35                  40                  45

cgc cgc cgc gtc gag ttg gcg cag atc ctc ttc gcc gcc acc aac ggc     192
Arg Arg Arg Val Glu Leu Ala Gln Ile Leu Phe Ala Ala Thr Asn Gly
     50                  55                  60

tcc ggc aaa tca aaa acc aca ttg ctt ctc gac gag ccc acc aac cac     240
Ser Gly Lys Ser Lys Thr Thr Leu Leu Leu Asp Glu Pro Thr Asn His
 65                  70                  75                  80

ttg gac gca gac tcg atc acc tgg ctc cgt gac ttc ctg gcg aag cac     288
Leu Asp Ala Asp Ser Ile Thr Trp Leu Arg Asp Phe Leu Ala Lys His
                 85                  90                  95

gaa ggt gga ctg atc atg att tcg cac gac gtc gaa ctg ctt ggc gcc     336
Glu Gly Gly Leu Ile Met Ile Ser His Asp Val Glu Leu Leu Gly Ala
            100                 105                 110

gta tgt aac aag att tgg tac ctc gac gca gta cgc agc gaa gcc gat     384
Val Cys Asn Lys Ile Trp Tyr Leu Asp Ala Val Arg Ser Glu Ala Asp
        115                 120                 125

gtc tac aac atg ggc ttt agc aaa tac gtc gat gca cgt gca ctc gat     432
Val Tyr Asn Met Gly Phe Ser Lys Tyr Val Asp Ala Arg Ala Leu Asp
    130                 135                 140

gaa gca cgc cga cgc cgt gag cgc gca aac gcc gaa aag aag gcc gga     480
Glu Ala Arg Arg Arg Arg Glu Arg Ala Asn Ala Glu Lys Lys Ala Gly
145                 150                 155                 160

gcc ctc aag gac cag gct gca cgc ctc ggc gcg aaa gca acc aag gct     528
Ala Leu Lys Asp Gln Ala Ala Arg Leu Gly Ala Lys Ala Thr Lys Ala
                165                 170                 175

gcc gca gct aag cag atg atc gcc cgt gcg gaa cga atg atc gac aac     576

Ala Ala Ala Lys Gln Met Ile Ala Arg Ala Glu Arg Met Ile Asp Asn
            180                 185                 190

ctc gac gaa atc cgc gta gct gac cgc gcc gcc aac atc gtt ttc cca     624
Leu Asp Glu Ile Arg Val Ala Asp Arg Ala Ala Asn Ile Val Phe Pro
        195                 200                 205

gaa cca gca ccc tgt gga aaa acc cca ctc aac gcc aag ggc ctg acc     672
Glu Pro Ala Pro Cys Gly Lys Thr Pro Leu Asn Ala Lys Gly Leu Thr
    210                 215                 220

aag atg tac ggc tcc ctc gaa gtc ttc gcc ggc gtc gac cta gcc atc     720
Lys Met Tyr Gly Ser Leu Glu Val Phe Ala Gly Val Asp Leu Ala Ile
225                 230                 235                 240

gac aaa ggc tcc cgc gta gtc gtc ctc gga ttc aac ggt gca ggt aaa     768
Asp Lys Gly Ser Arg Val Val Val Leu Gly Phe Asn Gly Ala Gly Lys
                245                 250                 255

acc acc ctg ctc aaa ctc ctc gcc ggt gtg gaa cgc acc gac ggc gaa     816
Thr Thr Leu Leu Lys Leu Leu Ala Gly Val Glu Arg Thr Asp Gly Glu
            260                 265                 270

ggc ggc atc gtc acc gga tac ggc ctc aaa atc ggc tac ttc gcc cag     864
Gly Gly Ile Val Thr Gly Tyr Gly Leu Lys Ile Gly Tyr Phe Ala Gln
        275                 280                 285

gaa cac gac acc atc gac ccc gac aaa tcc gtc tgg caa aac acc atc     912
Glu His Asp Thr Ile Asp Pro Asp Lys Ser Val Trp Gln Asn Thr Ile
    290                 295                 300

gaa gcc tgc gcc gac gcc gac caa caa agc ctc cgc agc ctc ctc gga     960
Glu Ala Cys Ala Asp Ala Asp Gln Gln Ser Leu Arg Ser Leu Leu Gly
305                 310                 315                 320

tcc ttc atg ttc tcc ggc gaa caa ctc gac caa cca gca gga aca ctc    1008
Ser Phe Met Phe Ser Gly Glu Gln Leu Asp Gln Pro Ala Gly Thr Leu
                325                 330                 335

tcc ggc ggt gaa aaa acc cgc ctc gca ctg gcc acc ctc gtg tcc tcc    1056
Ser Gly Gly Glu Lys Thr Arg Leu Ala Leu Ala Thr Leu Val Ser Ser
            340                 345                 350

cgc gca aac gtc ctg ctt ctc gac gag ccc acc aac aac ctt gac ccg    1104
Arg Ala Asn Val Leu Leu Leu Asp Glu Pro Thr Asn Asn Leu Asp Pro
        355                 360                 365

atc tcc cgc gaa cag gtc ctc gac gca ctg cgc acc tac acc ggc gca    1152
Ile Ser Arg Glu Gln Val Leu Asp Ala Leu Arg Thr Tyr Thr Gly Ala
    370                 375                 380

gtc gtc ctg gtt acc cac gac ccg ggt gca gtc aag gcc ctt gag cca    1200
Val Val Leu Val Thr His Asp Pro Gly Ala Val Lys Ala Leu Glu Pro
385                 390                 395                 400

gaa cgc gtc atc gtg ctt cct gat ggc acc gag gat ctt tgg aat gat    1248
Glu Arg Val Ile Val Leu Pro Asp Gly Thr Glu Asp Leu Trp Asn Asp
                405                 410                 415

cag tac atg gaa atc gtg gaa ttg gcg taggttctaa ggctgtttat          1295
Gln Tyr Met Glu Ile Val Glu Leu Ala

                420                 425

gct                                                                    1298


<210> 284
<211> 425
<212> PRT
<213> Corynebacterium glutamicum

<400> 284
Ile Arg Lys Tyr Ser Arg Leu Glu Glu Gln Phe Gln Ser Leu Gly Gly
1               5                   10                  15

Tyr Glu Ala Asp Ala Glu Ala Ala Gln Ile Cys Asp Asn Leu Gly Leu
            20                  25                  30

Glu Ala Arg Ile Leu Asp Gln Gln Leu Lys Thr Leu Ser Gly Gly Gln
        35                  40                  45

Arg Arg Arg Val Glu Leu Ala Gln Ile Leu Phe Ala Ala Thr Asn Gly
    50                  55                  60

Ser Gly Lys Ser Lys Thr Thr Leu Leu Leu Asp Glu Pro Thr Asn His
65                  70                  75                  80

Leu Asp Ala Asp Ser Ile Thr Trp Leu Arg Asp Phe Leu Ala Lys His
                85                  90                  95

Glu Gly Gly Leu Ile Met Ile Ser His Asp Val Glu Leu Leu Gly Ala
            100                 105                 110

Val Cys Asn Lys Ile Trp Tyr Leu Asp Ala Val Arg Ser Glu Ala Asp
        115                 120                 125

Val Tyr Asn Met Gly Phe Ser Lys Tyr Val Asp Ala Arg Ala Leu Asp
    130                 135                 140

Glu Ala Arg Arg Arg Arg Glu Arg Ala Asn Ala Glu Lys Lys Ala Gly
145                 150                 155                 160

Ala Leu Lys Asp Gln Ala Ala Arg Leu Gly Ala Lys Ala Thr Lys Ala
                165                 170                 175

Ala Ala Ala Lys Gln Met Ile Ala Arg Ala Glu Arg Met Ile Asp Asn
            180                 185                 190

Leu Asp Glu Ile Arg Val Ala Asp Arg Ala Ala Asn Ile Val Phe Pro
        195                 200                 205

Glu Pro Ala Pro Cys Gly Lys Thr Pro Leu Asn Ala Lys Gly Leu Thr
    210                 215                 220

Lys Met Tyr Gly Ser Leu Glu Val Phe Ala Gly Val Asp Leu Ala Ile
225                 230                 235                 240

Asp Lys Gly Ser Arg Val Val Val Leu Gly Phe Asn Gly Ala Gly Lys
                245                 250                 255

Thr Thr Leu Leu Lys Leu Leu Ala Gly Val Glu Arg Thr Asp Gly Glu
            260                 265                 270

Gly Gly Ile Val Thr Gly Tyr Gly Leu Lys Ile Gly Tyr Phe Ala Gln
        275                 280                 285

Glu His Asp Thr Ile Asp Pro Asp Lys Ser Val Trp Gln Asn Thr Ile
    290                 295                 300

Glu Ala Cys Ala Asp Ala Asp Gln Gln Ser Leu Arg Ser Leu Leu Gly
305                 310                 315                 320

Ser Phe Met Phe Ser Gly Glu Gln Leu Asp Gln Pro Ala Gly Thr Leu
                325                 330                 335

Ser Gly Gly Glu Lys Thr Arg Leu Ala Leu Ala Thr Leu Val Ser Ser
            340                 345                 350

Arg Ala Asn Val Leu Leu Leu Asp Glu Pro Thr Asn Asn Leu Asp Pro
        355                 360                 365

Ile Ser Arg Glu Gln Val Leu Asp Ala Leu Arg Thr Tyr Thr Gly Ala
    370                 375                 380

Val Val Leu Val Thr His Asp Pro Gly Ala Val Lys Ala Leu Glu Pro
385                 390                 395                 400

Glu Arg Val Ile Val Leu Pro Asp Gly Thr Glu Asp Leu Trp Asn Asp
                405                 410                 415

Gln Tyr Met Glu Ile Val Glu Leu Ala
            420                 425


<210> 285
<211> 1298
<212> DNA
<213> Corynebacterium glutamicum

<220>
<221> CDS
<222> (1)..(1275)
<223> FRXA01946

<400> 285
atc cgc aag tac tcc agg ctc gag gaa caa ttc cag tcg ctc ggc ggc      48
Ile Arg Lys Tyr Ser Arg Leu Glu Glu Gln Phe Gln Ser Leu Gly Gly
  1               5                  10                  15

tac gaa gct gac gcc gaa gca gcc cag atc tgc gac aac ctc ggc ctc      96
Tyr Glu Ala Asp Ala Glu Ala Ala Gln Ile Cys Asp Asn Leu Gly Leu
             20                  25                  30

gag gca cgc atc ctc gac cag cag ctt aaa acc ctg tcc ggc ggc cag     144
Glu Ala Arg Ile Leu Asp Gln Gln Leu Lys Thr Leu Ser Gly Gly Gln
         35                  40                  45

cgc cgc cgc gtc gag ttg gcg cag atc ctc ttc gcc gcc acc aac ggc     192
Arg Arg Arg Val Glu Leu Ala Gln Ile Leu Phe Ala Ala Thr Asn Gly
     50                  55                  60

tcc ggc aaa tca aaa acc aca ttg ctt ctc gac gag ccc acc aac cac     240
Ser Gly Lys Ser Lys Thr Thr Leu Leu Leu Asp Glu Pro Thr Asn His

65                  70                  75                  80

ttg gac gca gac tcg atc acc tgg ctc cgt gac ttc ctg gcg aag cac     288
Leu Asp Ala Asp Ser Ile Thr Trp Leu Arg Asp Phe Leu Ala Lys His
                85                  90                  95

gaa ggt gga ctg atc atg att tcg cac gac gtc gaa ctg ctt ggc gcc     336
Glu Gly Gly Leu Ile Met Ile Ser His Asp Val Glu Leu Leu Gly Ala
            100                 105                 110

gta tgt aac aag att tgg tac ctc gac gca gta cgc agc gaa gcc gat     384
Val Cys Asn Lys Ile Trp Tyr Leu Asp Ala Val Arg Ser Glu Ala Asp
        115                 120                 125

gtc tac aac atg ggc ttt agc aaa tac gtc gat gca cgt gca ctc gat     432
Val Tyr Asn Met Gly Phe Ser Lys Tyr Val Asp Ala Arg Ala Leu Asp
    130                 135                 140

gaa gca cgc cga cgc cgt gag cgc gca aac gcc gaa aag aag gcc gga     480
Glu Ala Arg Arg Arg Arg Glu Arg Ala Asn Ala Glu Lys Lys Ala Gly
145                 150                 155                 160

gcc ctc aag gac cag gct gca cgc ctc ggc gcg aaa gca acc aag gct     528
Ala Leu Lys Asp Gln Ala Ala Arg Leu Gly Ala Lys Ala Thr Lys Ala
                165                 170                 175

gcc gca gct aag cag atg atc gcc cgt gcg gaa cga atg atc gac aac     576
Ala Ala Ala Lys Gln Met Ile Ala Arg Ala Glu Arg Met Ile Asp Asn
            180                 185                 190

ctc gac gaa atc cgc gta gct gac cgc gcc gcc aac atc gtt ttc cca     624
Leu Asp Glu Ile Arg Val Ala Asp Arg Ala Ala Asn Ile Val Phe Pro
        195                 200                 205

gaa cca gca ccc tgt gga aaa acc cca ctc aac gcc aag ggc ctg acc     672
Glu Pro Ala Pro Cys Gly Lys Thr Pro Leu Asn Ala Lys Gly Leu Thr
    210                 215                 220

aag atg tac ggc tcc ctc gaa gtc ttc gcc ggc gtc gac cta gcc atc     720
Lys Met Tyr Gly Ser Leu Glu Val Phe Ala Gly Val Asp Leu Ala Ile
225                 230                 235                 240

gac aaa ggc tcc cgc gta gtc gtc ctc gga ttc aac ggt gca ggt aaa     768
Asp Lys Gly Ser Arg Val Val Val Leu Gly Phe Asn Gly Ala Gly Lys
                245                 250                 255

acc acc ctg ctc aaa ctc ctc gcc ggt gtg gaa cgc acc gac ggc gaa     816
Thr Thr Leu Leu Lys Leu Leu Ala Gly Val Glu Arg Thr Asp Gly Glu
            260                 265                 270

ggc ggc atc gtc acc gga tac ggc ctc aaa atc ggc tac ttc gcc cag     864
Gly Gly Ile Val Thr Gly Tyr Gly Leu Lys Ile Gly Tyr Phe Ala Gln
        275                 280                 285

gaa cac gac acc atc gac ccc gac aaa tcc gtc tgg caa aac acc atc     912
Glu His Asp Thr Ile Asp Pro Asp Lys Ser Val Trp Gln Asn Thr Ile
    290                 295                 300

gaa gcc tgc gcc gac gcc gac caa caa agc ctc cgc agc ctc ctc gga     960
Glu Ala Cys Ala Asp Ala Asp Gln Gln Ser Leu Arg Ser Leu Leu Gly
305                 310                 315                 320

tcc ttc atg ttc tcc ggc gaa caa ctc gac caa cca gca gga aca ctc 1008
Ser Phe Met Phe Ser Gly Glu Gln Leu Asp Gln Pro Ala Gly Thr Leu
                325                 330                 335

tcc ggc ggt gaa aaa acc cgc ctc gca ctg gcc acc ctc gtg tcc tcc 1056
Ser Gly Gly Glu Lys Thr Arg Leu Ala Leu Ala Thr Leu Val Ser Ser
            340                 345                 350

cgc gca aac gtc ctg ctt ctc gac gag ccc acc aac aac ctt gac ccg 1104
Arg Ala Asn Val Leu Leu Leu Asp Glu Pro Thr Asn Asn Leu Asp Pro
        355                 360                 365

atc tcc cgc gaa cag gtc ctc gac gca ctg cgc acc tac acc ggc gca 1152
Ile Ser Arg Glu Gln Val Leu Asp Ala Leu Arg Thr Tyr Thr Gly Ala
    370                 375                 380

gtc gtc ctg gtt acc cac gac ccg ggt gca gtc aag gcc ctt gag cca 1200
Val Val Leu Val Thr His Asp Pro Gly Ala Val Lys Ala Leu Glu Pro
385                 390                 395                 400

gaa cgc gtc atc gtg ctt cct gat ggc acc gag gat ctt tgg aat gat 1248
Glu Arg Val Ile Val Leu Pro Asp Gly Thr Glu Asp Leu Trp Asn Asp
                405                 410                 415

cag tac atg gaa atc gtg gaa ttg gcg taggttctaa ggctgtttat 1295
Gln Tyr Met Glu Ile Val Glu Leu Ala
            420                 425

gct 1298

<210> 286
<211> 425
<212> PRT
<213> Corynebacterium glutamicum

<400> 286
Ile Arg Lys Tyr Ser Arg Leu Glu Glu Gln Phe Gln Ser Leu Gly Gly
1               5                   10                  15

Tyr Glu Ala Asp Ala Glu Ala Ala Gln Ile Cys Asp Asn Leu Gly Leu
            20                  25                  30

Glu Ala Arg Ile Leu Asp Gln Gln Leu Lys Thr Leu Ser Gly Gly Gln
        35                  40                  45

Arg Arg Arg Val Glu Leu Ala Gln Ile Leu Phe Ala Ala Thr Asn Gly
    50                  55                  60

Ser Gly Lys Ser Lys Thr Thr Leu Leu Leu Asp Glu Pro Thr Asn His
65                  70                  75                  80

Leu Asp Ala Asp Ser Ile Thr Trp Leu Arg Asp Phe Leu Ala Lys His
                85                  90                  95

Glu Gly Gly Leu Ile Met Ile Ser His Asp Val Glu Leu Leu Gly Ala
            100                 105                 110

Val Cys Asn Lys Ile Trp Tyr Leu Asp Ala Val Arg Ser Glu Ala Asp
        115                 120                 125

Val Tyr Asn Met Gly Phe Ser Lys Tyr Val Asp Ala Arg Ala Leu Asp
    130                 135                 140

Glu Ala Arg Arg Arg Arg Glu Arg Ala Asn Ala Glu Lys Lys Ala Gly
145                 150                 155                 160

Ala Leu Lys Asp Gln Ala Ala Arg Leu Gly Ala Lys Ala Thr Lys Ala
                165                 170                 175

Ala Ala Ala Lys Gln Met Ile Ala Arg Ala Glu Arg Met Ile Asp Asn
            180                 185                 190

Leu Asp Glu Ile Arg Val Ala Asp Arg Ala Ala Asn Ile Val Phe Pro
        195                 200                 205

Glu Pro Ala Pro Cys Gly Lys Thr Pro Leu Asn Ala Lys Gly Leu Thr
    210                 215                 220

Lys Met Tyr Gly Ser Leu Glu Val Phe Ala Gly Val Asp Leu Ala Ile
225                 230                 235                 240

Asp Lys Gly Ser Arg Val Val Val Leu Gly Phe Asn Gly Ala Gly Lys
                245                 250                 255

Thr Thr Leu Leu Lys Leu Leu Ala Gly Val Glu Arg Thr Asp Gly Glu
            260                 265                 270

Gly Gly Ile Val Thr Gly Tyr Gly Leu Lys Ile Gly Tyr Phe Ala Gln
        275                 280                 285

Glu His Asp Thr Ile Asp Pro Asp Lys Ser Val Trp Gln Asn Thr Ile
    290                 295                 300

Glu Ala Cys Ala Asp Ala Asp Gln Gln Ser Leu Arg Ser Leu Leu Gly
305                 310                 315                 320

Ser Phe Met Phe Ser Gly Glu Gln Leu Asp Gln Pro Ala Gly Thr Leu
                325                 330                 335

Ser Gly Gly Glu Lys Thr Arg Leu Ala Leu Ala Thr Leu Val Ser Ser
            340                 345                 350

Arg Ala Asn Val Leu Leu Leu Asp Glu Pro Thr Asn Asn Leu Asp Pro
        355                 360                 365

Ile Ser Arg Glu Gln Val Leu Asp Ala Leu Arg Thr Tyr Thr Gly Ala
    370                 375                 380

Val Val Leu Val Thr His Asp Pro Gly Ala Val Lys Ala Leu Glu Pro
385                 390                 395                 400

Glu Arg Val Ile Val Leu Pro Asp Gly Thr Glu Asp Leu Trp Asn Asp
                405                 410                 415

Gln Tyr Met Glu Ile Val Glu Leu Ala
            420                 425

<210> 287
<211> 1812

<212> DNA
<213> Corynebacterium glutamicum

<220>
<221> CDS
<222> (101)..(1789)
<223> RXN00164

<400> 287
ctgctttgcg ggaggttatg aaatgagtgg ggagacgtcg aaaagcatgc gctttccgtt 60

ggccagcctg ccgcaagtgc ggcgcgaggt ggcccggcag gtg ggt cgt att ccg 115
Val Gly Arg Ile Pro
1               5

cgg gcg aag tgg tgg ttt tta ggc gcg ctg gtg ttg ctg agt gcg ggc 163
Arg Ala Lys Trp Trp Phe Leu Gly Ala Leu Val Leu Leu Ser Ala Gly
                10                  15                  20

gct tat gcg tcg gtg ctg gtg ccg cag gtg ctg ggg cgg att gtg gat 211
Ala Tyr Ala Ser Val Leu Val Pro Gln Val Leu Gly Arg Ile Val Asp
            25                  30                  35

ctg gtg tcc gat ggc gcg cag atg cgt gat ttt gtt gag ctc agt gtg 259
Leu Val Ser Asp Gly Ala Gln Met Arg Asp Phe Val Glu Leu Ser Val
        40                  45                  50

att ctc att gcg gtg gca att gcc ggc gcg gtg ctc agt gcg tgc ggg 307
Ile Leu Ile Ala Val Ala Ile Ala Gly Ala Val Leu Ser Ala Cys Gly
    55                  60                  65

ttc tat gtg gtg tcg cgg att tct gag aag att atc gcc aat ttg agg 355
Phe Tyr Val Val Ser Arg Ile Ser Glu Lys Ile Ile Ala Asn Leu Arg
70                  75                  80                  85

gaa gat atg gtg ggc acc gcg ctt ggg ttg ccc acg cac cag gtg gaa 403
Glu Asp Met Val Gly Thr Ala Leu Gly Leu Pro Thr His Gln Val Glu
                90                  95                  100

gat gcg ggc tct ggc gat ttg gtg agc cgc tcc acc gat gat gtc tcc 451
Asp Ala Gly Ser Gly Asp Leu Val Ser Arg Ser Thr Asp Asp Val Ser
            105                 110                 115

gag cta tcc gca gcg gtg aca gag acc gtc ccg att tta agt tcc tca 499
Glu Leu Ser Ala Ala Val Thr Glu Thr Val Pro Ile Leu Ser Ser Ser
        120                 125                 130

ctg ttt acc att gcc gcg acg atc att gcg ctg ttt tct ttg gac tgg 547
Leu Phe Thr Ile Ala Ala Thr Ile Ile Ala Leu Phe Ser Leu Asp Trp
    135                 140                 145

caa ttt gtg ctc att cct gtc gtg gtg gcg ccg gtg tac tac ttc gcg 595
Gln Phe Val Leu Ile Pro Val Val Val Ala Pro Val Tyr Tyr Phe Ala
150                 155                 160                 165

tcc aag cac tat ttg agc aag gcg ccg gat cgg tat gcg gca gaa cgc 643
Ser Lys His Tyr Leu Ser Lys Ala Pro Asp Arg Tyr Ala Ala Glu Arg
                170                 175                 180

gcg gcg atg gcg gag cgt gcg cga aag gta ctt gag gct att cgc ggg 691
Ala Ala Met Ala Glu Arg Ala Arg Lys Val Leu Glu Ala Ile Arg Gly

            185                 190                 195

cgt gca act gtg cgg gcg tat tcc atg gaa gat gcc atg cat aat cag 739
Arg Ala Thr Val Arg Ala Tyr Ser Met Glu Asp Ala Met His Asn Gln
        200                 205                 210

att gat cag gcg tcg tgg tct gtg gtg gtc aag ggt att cgt gcg cgc 787
Ile Asp Gln Ala Ser Trp Ser Val Val Val Lys Gly Ile Arg Ala Arg
    215                 220                 225

acc acc atg ttg att ttg aac atg tgg atg ctg ttt gcg gaa ttc ctc 835
Thr Thr Met Leu Ile Leu Asn Met Trp Met Leu Phe Ala Glu Phe Leu
230                 235                 240                 245

atg ctc gcg gtc gcg ttg gtg atc ggc tac aag ctg gtc att gat aat 883
Met Leu Ala Val Ala Leu Val Ile Gly Tyr Lys Leu Val Ile Asp Asn
                250                 255                 260

gcg ctg acg atc ggc gcg gtt acc ggt gcc gtg ctg atg att att cgt 931
Ala Leu Thr Ile Gly Ala Val Thr Gly Ala Val Leu Met Ile Ile Arg
            265                 270                 275

ctg cgt ggc ccg atg aat atg ttc atg cgc gtg ctc gac acc att caa 979
Leu Arg Gly Pro Met Asn Met Phe Met Arg Val Leu Asp Thr Ile Gln
        280                 285                 290

tcc ggc tat gcg tcg ctg gcg cgc atc gtg gga gtt gtt gcg gat ccg 1027
Ser Gly Tyr Ala Ser Leu Ala Arg Ile Val Gly Val Val Ala Asp Pro
    295                 300                 305

ccg att cct gtg ccc gac agc ggt gtg aaa gca cct cag ggc aaa gtg 1075
Pro Ile Pro Val Pro Asp Ser Gly Val Lys Ala Pro Gln Gly Lys Val
310                 315                 320                 325

gaa ttg cgc aac gtc agc ttt agc tat ggc gat tcc tgg gcg gtg aaa 1123
Glu Leu Arg Asn Val Ser Phe Ser Tyr Gly Asp Ser Trp Ala Val Lys
                330                 335                 340

gac atc gac atc acg atc aat tcc ggc gaa act gtc gcg ctc gtg ggc 1171
Asp Ile Asp Ile Thr Ile Asn Ser Gly Glu Thr Val Ala Leu Val Gly
            345                 350                 355

gca tct ggc gca ggt aag acg acg gtc gcc gcc ttg ctg gcg ggc ttg 1219
Ala Ser Gly Ala Gly Lys Thr Thr Val Ala Ala Leu Leu Ala Gly Leu
        360                 365                 370

cgg gtg cca gat caa ggg caa gtg ctt gtc gac gac ttc ccc gtc tct 1267
Arg Val Pro Asp Gln Gly Gln Val Leu Val Asp Asp Phe Pro Val Ser
    375                 380                 385

cac ctc tct gac cgc gag cgt atc gcc cgc ttg gcc atg gtc agc cag 1315
His Leu Ser Asp Arg Glu Arg Ile Ala Arg Leu Ala Met Val Ser Gln
390                 395                 400                 405

gag gtt cat gtt ttc tcc ggc acg ctg cgc cag gat ctc acc ttg gct 1363
Glu Val His Val Phe Ser Gly Thr Leu Arg Gln Asp Leu Thr Leu Ala
                410                 415                 420

aaa cca gat gcc tcc gat gag gaa tta gcg cat gct ctt ggg caa gtt 1411
Lys Pro Asp Ala Ser Asp Glu Glu Leu Ala His Ala Leu Gly Gln Val
            425                 430                 435

aat gcc ctt gac tgg ttg gag agt ctt cca gaa gga ctg gac acg gtc 1459
Asn Ala Leu Asp Trp Leu Glu Ser Leu Pro Glu Gly Leu Asp Thr Val
        440                 445                 450

gtt ggt gcg cga gga atc cag cta gaa cca gtg gtg gct cag cag ttg 1507
Val Gly Ala Arg Gly Ile Gln Leu Glu Pro Val Val Ala Gln Gln Leu
    455                 460                 465

gcg ttg gcc cgg gtg ttg ttg ctc aat ccg gcg atc gtc atc atg gat 1555
Ala Leu Ala Arg Val Leu Leu Leu Asn Pro Ala Ile Val Ile Met Asp
470                 475                 480                 485

gaa gcc acg gca gaa gca gga tcg gcg ggt gcc agc gca ctg gaa gag 1603
Glu Ala Thr Ala Glu Ala Gly Ser Ala Gly Ala Ser Ala Leu Glu Glu
                490                 495                 500

gct gca gat gca gtg agc aag aac cgt tcc gca ttg gtg gtg gcg cac 1651
Ala Ala Asp Ala Val Ser Lys Asn Arg Ser Ala Leu Val Val Ala His
            505                 510                 515

cgg ttg gat cag gca tcg cgg gct gat cag att ctg gtg atg gat aag 1699
Arg Leu Asp Gln Ala Ser Arg Ala Asp Gln Ile Leu Val Met Asp Lys
        520                 525                 530

ggg gag gtt gtg gaa tcc ggt act cac cag gag tta ttg gat cac ggg 1747
Gly Glu Val Val Glu Ser Gly Thr His Gln Glu Leu Leu Asp His Gly
    535                 540                 545

ggt att tat cag cgt ctg tgg act gcg tgg agt gtc gga aga 1789
Gly Ile Tyr Gln Arg Leu Trp Thr Ala Trp Ser Val Gly Arg
550                 555                 560

tagttgactg ttcaatgcgt tga 1812

<210> 288
<211> 563
<212> PRT
<213> Corynebacterium glutamicum

<400> 288
Val Gly Arg Ile Pro Arg Ala Lys Trp Trp Phe Leu Gly Ala Leu Val
1               5                   10                  15

Leu Leu Ser Ala Gly Ala Tyr Ala Ser Val Leu Val Pro Gln Val Leu
            20                  25                  30

Gly Arg Ile Val Asp Leu Val Ser Asp Gly Ala Gln Met Arg Asp Phe
        35                  40                  45

Val Glu Leu Ser Val Ile Leu Ile Ala Val Ala Ile Ala Gly Ala Val
    50                  55                  60

Leu Ser Ala Cys Gly Phe Tyr Val Val Ser Arg Ile Ser Glu Lys Ile
65                  70                  75                  80

Ile Ala Asn Leu Arg Glu Asp Met Val Gly Thr Ala Leu Gly Leu Pro
                85                  90                  95

Thr His Gln Val Glu Asp Ala Gly Ser Gly Asp Leu Val Ser Arg Ser

            100                 105                 110

Thr Asp Asp Val Ser Glu Leu Ser Ala Ala Val Thr Glu Thr Val Pro
        115                 120                 125

Ile Leu Ser Ser Ser Leu Phe Thr Ile Ala Ala Thr Ile Ile Ala Leu
    130                 135                 140

Phe Ser Leu Asp Trp Gln Phe Val Leu Ile Pro Val Val Val Ala Pro
145                 150                 155                 160

Val Tyr Tyr Phe Ala Ser Lys His Tyr Leu Ser Lys Ala Pro Asp Arg
                165                 170                 175

Tyr Ala Ala Glu Arg Ala Ala Met Ala Glu Arg Ala Arg Lys Val Leu
            180                 185                 190

Glu Ala Ile Arg Gly Arg Ala Thr Val Arg Ala Tyr Ser Met Glu Asp
        195                 200                 205

Ala Met His Asn Gln Ile Asp Gln Ala Ser Trp Ser Val Val Val Lys
    210                 215                 220

Gly Ile Arg Ala Arg Thr Thr Met Leu Ile Leu Asn Met Trp Met Leu
225                 230                 235                 240

Phe Ala Glu Phe Leu Met Leu Ala Val Ala Leu Val Ile Gly Tyr Lys
                245                 250                 255

Leu Val Ile Asp Asn Ala Leu Thr Ile Gly Ala Val Thr Gly Ala Val
            260                 265                 270

Leu Met Ile Ile Arg Leu Arg Gly Pro Met Asn Met Phe Met Arg Val
        275                 280                 285

Leu Asp Thr Ile Gln Ser Gly Tyr Ala Ser Leu Ala Arg Ile Val Gly
    290                 295                 300

Val Val Ala Asp Pro Pro Ile Pro Val Pro Asp Ser Gly Val Lys Ala
305                 310                 315                 320

Pro Gln Gly Lys Val Glu Leu Arg Asn Val Ser Phe Ser Tyr Gly Asp
                325                 330                 335

Ser Trp Ala Val Lys Asp Ile Asp Ile Thr Ile Asn Ser Gly Glu Thr
            340                 345                 350

Val Ala Leu Val Gly Ala Ser Gly Ala Gly Lys Thr Thr Val Ala Ala
        355                 360                 365

Leu Leu Ala Gly Leu Arg Val Pro Asp Gln Gly Gln Val Leu Val Asp
    370                 375                 380

Asp Phe Pro Val Ser His Leu Ser Asp Arg Glu Arg Ile Ala Arg Leu
385                 390                 395                 400

Ala Met Val Ser Gln Glu Val His Val Phe Ser Gly Thr Leu Arg Gln
                405                 410                 415

Asp Leu Thr Leu Ala Lys Pro Asp Ala Ser Asp Glu Glu Leu Ala His
            420                 425                 430

Ala Leu Gly Gln Val Asn Ala Leu Asp Trp Leu Glu Ser Leu Pro Glu
        435                 440                 445

Gly Leu Asp Thr Val Val Gly Ala Arg Gly Ile Gln Leu Glu Pro Val
    450                 455                 460

Val Ala Gln Gln Leu Ala Leu Ala Arg Val Leu Leu Leu Asn Pro Ala
465                 470                 475                 480

Ile Val Ile Met Asp Glu Ala Thr Ala Glu Ala Gly Ser Ala Gly Ala
                485                 490                 495

Ser Ala Leu Glu Glu Ala Ala Asp Ala Val Ser Lys Asn Arg Ser Ala
            500                 505                 510

Leu Val Val Ala His Arg Leu Asp Gln Ala Ser Arg Ala Asp Gln Ile
        515                 520                 525

Leu Val Met Asp Lys Gly Glu Val Val Glu Ser Gly Thr His Gln Glu
    530                 535                 540

Leu Leu Asp His Gly Gly Ile Tyr Gln Arg Leu Trp Thr Ala Trp Ser
545                 550                 555                 560

Val Gly Arg

<210> 289
<211> 1812
<212> DNA
<213> Corynebacterium glutamicum

<220>
<221> CDS
<222> (101)..(1789)
<223> FRXA00164

<400> 289
ctgctttgcg ggaggttatg aaatgagtgg ggagacgtcg aaaagcatgc gctttccgtt 60

ggccagcctg ccgcaagtgc ggcgcgaggt ggcccggcag gtg ggt cgt att ccg   115
                                          Val Gly Arg Ile Pro
                                          1               5

cgg gcg aag tgg tgg ttt tta ggc gcg ctg gtg ttg ctg agt gcg ggc   163
Arg Ala Lys Trp Trp Phe Leu Gly Ala Leu Val Leu Leu Ser Ala Gly
                10                  15                  20

gct tat gcg tcg gtg ctg gtg ccg cag gtg ctg ggg cgg att gtg gat   211
Ala Tyr Ala Ser Val Leu Val Pro Gln Val Leu Gly Arg Ile Val Asp
            25                  30                  35

ctg gtg tcc gat ggc gcg cag atg cgt gat ttt gtt gag ctc agt gtg   259
Leu Val Ser Asp Gly Ala Gln Met Arg Asp Phe Val Glu Leu Ser Val
        40                  45                  50

att ctc att gcg gtg gca att gcc ggc gcg gtg ctc agt gcg tgc ggg   307
Ile Leu Ile Ala Val Ala Ile Ala Gly Ala Val Leu Ser Ala Cys Gly
    55                  60                  65

ttc tat gtg gtg tcg cgg att tct gag aag att atc gcc aat ttg agg 355
Phe Tyr Val Val Ser Arg Ile Ser Glu Lys Ile Ile Ala Asn Leu Arg
70                  75                  80                  85

gaa gat atg gtg ggc acc gcg ctt ggg ttg ccc acg cac cag gtg gaa 403
Glu Asp Met Val Gly Thr Ala Leu Gly Leu Pro Thr His Gln Val Glu
                90                  95                  100

gat gcg ggc tct ggc gat ttg gtg agc cgc tcc acc gat gat gtc tcc 451
Asp Ala Gly Ser Gly Asp Leu Val Ser Arg Ser Thr Asp Asp Val Ser
            105                 110                 115

gag cta tcc gca gcg gtg aca gag acc gtc ccg att tta agt tcc tca 499
Glu Leu Ser Ala Ala Val Thr Glu Thr Val Pro Ile Leu Ser Ser Ser
        120                 125                 130

ctg ttt acc att gcc gcg acg atc att gcg ctg ttt tct ttg gac tgg 547
Leu Phe Thr Ile Ala Ala Thr Ile Ile Ala Leu Phe Ser Leu Asp Trp
    135                 140                 145

caa ttt gtg ctc att cct gtc gtg gtg gcg ccg gtg tac tac ttc gcg 595
Gln Phe Val Leu Ile Pro Val Val Val Ala Pro Val Tyr Tyr Phe Ala
150                 155                 160                 165

tcc aag cac tat ttg agc aag gcg ccg gat cgg tat gcg gca gaa cgc 643
Ser Lys His Tyr Leu Ser Lys Ala Pro Asp Arg Tyr Ala Ala Glu Arg
                170                 175                 180

gcg gcg atg gcg gag cgt gcg cga aag gta ctt gag gct att cgc ggg 691
Ala Ala Met Ala Glu Arg Ala Arg Lys Val Leu Glu Ala Ile Arg Gly
            185                 190                 195

cgt gca act gtg cgg gcg tat tcc atg gaa gat gcc atg cat aat cag 739
Arg Ala Thr Val Arg Ala Tyr Ser Met Glu Asp Ala Met His Asn Gln
        200                 205                 210

att gat cag gcg tcg tgg tct gtg gtg gtc aag ggt att cgt gcg cgc 787
Ile Asp Gln Ala Ser Trp Ser Val Val Val Lys Gly Ile Arg Ala Arg
    215                 220                 225

acc acc atg ttg att ttg aac atg tgg atg ctg ttt gcg gaa ttc ctc 835
Thr Thr Met Leu Ile Leu Asn Met Trp Met Leu Phe Ala Glu Phe Leu
230                 235                 240                 245

atg ctc gcg gtc gcg ttg gtg atc ggc tac aag ctg gtc att gat aat 883
Met Leu Ala Val Ala Leu Val Ile Gly Tyr Lys Leu Val Ile Asp Asn
                250                 255                 260

gcg ctg acg atc ggc gcg gtt acc ggt gcc gtg ctg atg att att cgt 931
Ala Leu Thr Ile Gly Ala Val Thr Gly Ala Val Leu Met Ile Ile Arg
            265                 270                 275

ctg cgt ggc ccg atg aat atg ttc atg cgc gtg ctc gac acc att caa 979
Leu Arg Gly Pro Met Asn Met Phe Met Arg Val Leu Asp Thr Ile Gln
        280                 285                 290

tcc ggc tat gcg tcg ctg gcg cgc atc gtg gga gtt gtt gcg gat ccg 1027
Ser Gly Tyr Ala Ser Leu Ala Arg Ile Val Gly Val Val Ala Asp Pro
    295                 300                 305

ccg att cct gtg ccc gac agc ggt gtg aaa gca cct cag ggc aaa gtg 1075
Pro Ile Pro Val Pro Asp Ser Gly Val Lys Ala Pro Gln Gly Lys Val
310                 315                 320                 325

gaa ttg cgc aac gtc agc ttt agc tat ggc gat tcc tgg gcg gtg aaa 1123
Glu Leu Arg Asn Val Ser Phe Ser Tyr Gly Asp Ser Trp Ala Val Lys
                330                 335                 340

gac atc gac atc acg atc aat tcc ggc gaa act gtc gcg ctc gtg ggc 1171
Asp Ile Asp Ile Thr Ile Asn Ser Gly Glu Thr Val Ala Leu Val Gly
            345                 350                 355

gca tct ggc gca ggt aag acg acg gtc gcc gcc ttg ctg gcg ggc ttg 1219
Ala Ser Gly Ala Gly Lys Thr Thr Val Ala Ala Leu Leu Ala Gly Leu
        360                 365                 370

cgg gtg cca gat caa ggg caa gtg ctt gtc gac gac ttc ccc gtc tct 1267
Arg Val Pro Asp Gln Gly Gln Val Leu Val Asp Asp Phe Pro Val Ser
    375                 380                 385

cac ctc tct gac cgc gag cgt atc gcc cgc ttg gcc atg gtc agc cag 1315
His Leu Ser Asp Arg Glu Arg Ile Ala Arg Leu Ala Met Val Ser Gln
390                 395                 400                 405

gag gtt cat gtt ttc tcc ggc acg ctg cgc cag gat ctc acc ttg gct 1363
Glu Val His Val Phe Ser Gly Thr Leu Arg Gln Asp Leu Thr Leu Ala
                410                 415                 420

aaa cca gat gcc tcc gat gag gaa tta gcg cat gct ctt ggg caa gtt 1411
Lys Pro Asp Ala Ser Asp Glu Glu Leu Ala His Ala Leu Gly Gln Val
            425                 430                 435

aat gcc ctt gac tgg ttg gag agt ctt cca gaa gga ctg gac acg gtc 1459
Asn Ala Leu Asp Trp Leu Glu Ser Leu Pro Glu Gly Leu Asp Thr Val
        440                 445                 450

gtt ggt gcg cga gga atc cag cta gaa cca gtg gtg gct cag cag ttg 1507
Val Gly Ala Arg Gly Ile Gln Leu Glu Pro Val Val Ala Gln Gln Leu
    455                 460                 465

gcg ttg gcc cgg gtg ttg ttg ctc aat ccg gcg atc gtc atc atg gat 1555
Ala Leu Ala Arg Val Leu Leu Leu Asn Pro Ala Ile Val Ile Met Asp
470                 475                 480                 485

gaa gcc acg gca gaa gca gga tcg gcg ggt gcc agc gca ctg gaa gag 1603
Glu Ala Thr Ala Glu Ala Gly Ser Ala Gly Ala Ser Ala Leu Glu Glu
                490                 495                 500

gct gca gat gca gtg agc aag aac cgt tcc gca ttg gtg gtg gcg cac 1651
Ala Ala Asp Ala Val Ser Lys Asn Arg Ser Ala Leu Val Val Ala His
            505                 510                 515

cgg ttg gat cag gca tcg cgg gct gat cag att ctg gtg atg gat aag 1699
Arg Leu Asp Gln Ala Ser Arg Ala Asp Gln Ile Leu Val Met Asp Lys
        520                 525                 530

ggg gag gtt gtg gaa tcc ggt act cac cag gag tta ttg gat cac ggg 1747
Gly Glu Val Val Glu Ser Gly Thr His Gln Glu Leu Leu Asp His Gly
    535                 540                 545

ggt att tat cag cgt ctg tgg act gcg tgg agt gtc gga aga 1789

Gly Ile Tyr Gln Arg Leu Trp Thr Ala Trp Ser Val Gly Arg
550                 555                 560

tagttgactg ttcaatgcgt tga                                              1812

<210> 290
<211> 563
<212> PRT
<213> Corynebacterium glutamicum

<400> 290
Val Gly Arg Ile Pro Arg Ala Lys Trp Trp Phe Leu Gly Ala Leu Val
1               5                   10                  15

Leu Leu Ser Ala Gly Ala Tyr Ala Ser Val Leu Val Pro Gln Val Leu
            20                  25                  30

Gly Arg Ile Val Asp Leu Val Ser Asp Gly Ala Gln Met Arg Asp Phe
        35                  40                  45

Val Glu Leu Ser Val Ile Leu Ile Ala Val Ala Ile Ala Gly Ala Val
    50                  55                  60

Leu Ser Ala Cys Gly Phe Tyr Val Val Ser Arg Ile Ser Glu Lys Ile
65                  70                  75                  80

Ile Ala Asn Leu Arg Glu Asp Met Val Gly Thr Ala Leu Gly Leu Pro
                85                  90                  95

Thr His Gln Val Glu Asp Ala Gly Ser Gly Asp Leu Val Ser Arg Ser
            100                 105                 110

Thr Asp Asp Val Ser Glu Leu Ser Ala Ala Val Thr Glu Thr Val Pro
        115                 120                 125

Ile Leu Ser Ser Ser Leu Phe Thr Ile Ala Ala Thr Ile Ile Ala Leu
    130                 135                 140

Phe Ser Leu Asp Trp Gln Phe Val Leu Ile Pro Val Val Val Ala Pro
145                 150                 155                 160

Val Tyr Tyr Phe Ala Ser Lys His Tyr Leu Ser Lys Ala Pro Asp Arg
                165                 170                 175

Tyr Ala Ala Glu Arg Ala Ala Met Ala Glu Arg Ala Arg Lys Val Leu
            180                 185                 190

Glu Ala Ile Arg Gly Arg Ala Thr Val Arg Ala Tyr Ser Met Glu Asp
        195                 200                 205

Ala Met His Asn Gln Ile Asp Gln Ala Ser Trp Ser Val Val Val Lys
    210                 215                 220

Gly Ile Arg Ala Arg Thr Thr Met Leu Ile Leu Asn Met Trp Met Leu
225                 230                 235                 240

Phe Ala Glu Phe Leu Met Leu Ala Val Ala Leu Val Ile Gly Tyr Lys
                245                 250                 255

Leu Val Ile Asp Asn Ala Leu Thr Ile Gly Ala Val Thr Gly Ala Val

            260                 265                 270

Leu Met Ile Ile Arg Leu Arg Gly Pro Met Asn Met Phe Met Arg Val
        275                 280                 285

Leu Asp Thr Ile Gln Ser Gly Tyr Ala Ser Leu Ala Arg Ile Val Gly
    290                 295                 300

Val Val Ala Asp Pro Pro Ile Pro Val Pro Asp Ser Gly Val Lys Ala
305                 310                 315                 320

Pro Gln Gly Lys Val Glu Leu Arg Asn Val Ser Phe Ser Tyr Gly Asp
                325                 330                 335

Ser Trp Ala Val Lys Asp Ile Asp Ile Thr Ile Asn Ser Gly Glu Thr
            340                 345                 350

Val Ala Leu Val Gly Ala Ser Gly Ala Gly Lys Thr Thr Val Ala Ala
        355                 360                 365

Leu Leu Ala Gly Leu Arg Val Pro Asp Gln Gly Gln Val Leu Val Asp
    370                 375                 380

Asp Phe Pro Val Ser His Leu Ser Asp Arg Glu Arg Ile Ala Arg Leu
385                 390                 395                 400

Ala Met Val Ser Gln Glu Val His Val Phe Ser Gly Thr Leu Arg Gln
                405                 410                 415

Asp Leu Thr Leu Ala Lys Pro Asp Ala Ser Asp Glu Glu Leu Ala His
            420                 425                 430

Ala Leu Gly Gln Val Asn Ala Leu Asp Trp Leu Glu Ser Leu Pro Glu
        435                 440                 445

Gly Leu Asp Thr Val Val Gly Ala Arg Gly Ile Gln Leu Glu Pro Val
    450                 455                 460

Val Ala Gln Gln Leu Ala Leu Ala Arg Val Leu Leu Leu Asn Pro Ala
465                 470                 475                 480

Ile Val Ile Met Asp Glu Ala Thr Ala Glu Ala Gly Ser Ala Gly Ala
                485                 490                 495

Ser Ala Leu Glu Glu Ala Ala Asp Ala Val Ser Lys Asn Arg Ser Ala
            500                 505                 510

Leu Val Val Ala His Arg Leu Asp Gln Ala Ser Arg Ala Asp Gln Ile
        515                 520                 525

Leu Val Met Asp Lys Gly Glu Val Val Glu Ser Gly Thr His Gln Glu
    530                 535                 540

Leu Leu Asp His Gly Gly Ile Tyr Gln Arg Leu Trp Thr Ala Trp Ser
545                 550                 555                 560

Val Gly Arg

<210> 291

<211> 1140
<212> DNA
<213> Corynebacterium glutamicum

<220>
<221> CDS
<222> (101)..(1117)
<223> RXN00243

<400> 291
cactgcgcca gatttttgat gccgacactg tggcaggtgt gcgcgctgag tacgaaaaat 60

ttaacaaagc agcccatgat ggaaatgaag aggaacagaa gtg acc agc gaa caa 115
Val Thr Ser Glu Gln
1               5

gct tta gat cct atc cac cca ggt cag ttc cgt ctt tct cgg att cag 163
Ala Leu Asp Pro Ile His Pro Gly Gln Phe Arg Leu Ser Arg Ile Gln
                10                  15                  20

ttg atc aac tgg gga acc ttc cac gga acg gtg gac att cct gtg acc 211
Leu Ile Asn Trp Gly Thr Phe His Gly Thr Val Asp Ile Pro Val Thr
            25                  30                  35

agg gaa gga atc tta gtt acc ggt ggt tcg gga tca gga aaa tcc acg 259
Arg Glu Gly Ile Leu Val Thr Gly Gly Ser Gly Ser Gly Lys Ser Thr
        40                  45                  50

ctg att gat gcg atc acg gcg gta ttg ctt ccg caa gga aag ctg agg 307
Leu Ile Asp Ala Ile Thr Ala Val Leu Leu Pro Gln Gly Lys Leu Arg
    55                  60                  65

ttt aac tct gcc gca cag gct aat act ccg cgg aat aag gga cgc agt 355
Phe Asn Ser Ala Ala Gln Ala Asn Thr Pro Arg Asn Lys Gly Arg Ser
70                  75                  80                  85

ttg gtt acc tat atc cgt ggc gct tgg cgt gcg cag gag gat ccg ctg 403
Leu Val Thr Tyr Ile Arg Gly Ala Trp Arg Ala Gln Glu Asp Pro Leu
                90                  95                  100

cag gat cag att gtc tcc acg tac cta cgt ccc cgc gca acc tat tcg 451
Gln Asp Gln Ile Val Ser Thr Tyr Leu Arg Pro Arg Ala Thr Tyr Ser
            105                 110                 115

ctg gtt gga ttg act tat tcc aac ggt gaa ggc gtc gag cac acc ttg 499
Leu Val Gly Leu Thr Tyr Ser Asn Gly Glu Gly Val Glu His Thr Leu
        120                 125                 130

gtg gct att ttc tat ctg aaa tcg gga cac aat tta acc tcc gat att 547
Val Ala Ile Phe Tyr Leu Lys Ser Gly His Asn Leu Thr Ser Asp Ile
    135                 140                 145

tct tca tat tat ggt gtg ttt ccc gtt gat caa gac atc aat gcg ctg 595
Ser Ser Tyr Tyr Gly Val Phe Pro Val Asp Gln Asp Ile Asn Ala Leu
150                 155                 160                 165

ctg gat ttc ctg aaa gag ggc atc gat aaa cgc cag atc aga gct gct 643
Leu Asp Phe Leu Lys Glu Gly Ile Asp Lys Arg Gln Ile Arg Ala Ala
                170                 175                 180

ttc aag gaa gcc atc ttt agc gag cag cat tct gta ttc tcc ggc agg 691

Phe Lys Glu Ala Ile Phe Ser Glu Gln His Ser Val Phe Ser Gly Arg
            185                 190                 195

ttt aga agc cgt ttg ggg atc tcc agt gag gaa gct ttg ctg ttg ttg     739
Phe Arg Ser Arg Leu Gly Ile Ser Ser Glu Glu Ala Leu Leu Leu Leu
        200                 205                 210

cac cgc gcg cag tcg gcg aaa gat ctt caa agc ttg gat gat cta ttt     787
His Arg Ala Gln Ser Ala Lys Asp Leu Gln Ser Leu Asp Asp Leu Phe
    215                 220                 225

cgg gat tac atg ctg gtg gaa ccg gat acg ttc agc att gcc aaa act     835
Arg Asp Tyr Met Leu Val Glu Pro Asp Thr Phe Ser Ile Ala Lys Thr
230                 235                 240                 245

gcc gtg gaa caa ttc caa gac ctt gaa ggt gct tat gag cag gtc gaa     883
Ala Val Glu Gln Phe Gln Asp Leu Glu Gly Ala Tyr Glu Gln Val Glu
                250                 255                 260

gat att aaa cgg cag atc cac acc ctg gat cct ttg gtg cag ctg aag     931
Asp Ile Lys Arg Gln Ile His Thr Leu Asp Pro Leu Val Gln Leu Lys
            265                 270                 275

aat cgg cga gag aaa gcg caa cag tcc aaa gat cat gcc aat gca ctg     979
Asn Arg Arg Glu Lys Ala Gln Gln Ser Lys Asp His Ala Asn Ala Leu
        280                 285                 290

aag aag gcg ctg ccg act gtc ggg aat cgc att aag aag gaa gag caa     1027
Lys Lys Ala Leu Pro Thr Val Gly Asn Arg Ile Lys Lys Glu Glu Gln
    295                 300                 305

gaa ccg ctg gtt cga caa ttt act gtc gag caa acg cag cga agt cga     1075
Glu Pro Leu Val Arg Gln Phe Thr Val Glu Gln Thr Gln Arg Ser Arg
310                 315                 320                 325

agg tgg agt ccg cca aaa ttg aga cag atc gtg ccc gcg aaa             1117
Arg Trp Ser Pro Pro Lys Leu Arg Gln Ile Val Pro Ala Lys
                330                 335

tgaaaaccct cgcgcacgac aac                                           1140


<210> 292
<211> 339
<212> PRT
<213> Corynebacterium glutamicum

<400> 292
Val Thr Ser Glu Gln Ala Leu Asp Pro Ile His Pro Gly Gln Phe Arg
  1               5                  10                  15

Leu Ser Arg Ile Gln Leu Ile Asn Trp Gly Thr Phe His Gly Thr Val
             20                  25                  30

Asp Ile Pro Val Thr Arg Glu Gly Ile Leu Val Thr Gly Gly Ser Gly
         35                  40                  45

Ser Gly Lys Ser Thr Leu Ile Asp Ala Ile Thr Ala Val Leu Leu Pro
     50                  55                  60

Gln Gly Lys Leu Arg Phe Asn Ser Ala Ala Gln Ala Asn Thr Pro Arg

65                  70                  75                  80

Asn Lys Gly Arg Ser Leu Val Thr Tyr Ile Arg Gly Ala Trp Arg Ala
                85                  90                  95

Gln Glu Asp Pro Leu Gln Asp Gln Ile Val Ser Thr Tyr Leu Arg Pro
            100                 105                 110

Arg Ala Thr Tyr Ser Leu Val Gly Leu Thr Tyr Ser Asn Gly Glu Gly
        115                 120                 125

Val Glu His Thr Leu Val Ala Ile Phe Tyr Leu Lys Ser Gly His Asn
    130                 135                 140

Leu Thr Ser Asp Ile Ser Ser Tyr Tyr Gly Val Phe Pro Val Asp Gln
145                 150                 155                 160

Asp Ile Asn Ala Leu Leu Asp Phe Leu Lys Glu Gly Ile Asp Lys Arg
                165                 170                 175

Gln Ile Arg Ala Ala Phe Lys Glu Ala Ile Phe Ser Glu Gln His Ser
            180                 185                 190

Val Phe Ser Gly Arg Phe Arg Ser Arg Leu Gly Ile Ser Ser Glu Glu
        195                 200                 205

Ala Leu Leu Leu Leu His Arg Ala Gln Ser Ala Lys Asp Leu Gln Ser
    210                 215                 220

Leu Asp Asp Leu Phe Arg Asp Tyr Met Leu Val Glu Pro Asp Thr Phe
225                 230                 235                 240

Ser Ile Ala Lys Thr Ala Val Glu Gln Phe Gln Asp Leu Glu Gly Ala
                245                 250                 255

Tyr Glu Gln Val Glu Asp Ile Lys Arg Gln Ile His Thr Leu Asp Pro
            260                 265                 270

Leu Val Gln Leu Lys Asn Arg Arg Glu Lys Ala Gln Gln Ser Lys Asp
        275                 280                 285

His Ala Asn Ala Leu Lys Lys Ala Leu Pro Thr Val Gly Asn Arg Ile
    290                 295                 300

Lys Lys Glu Glu Gln Glu Pro Leu Val Arg Gln Phe Thr Val Glu Gln
305                 310                 315                 320

Thr Gln Arg Ser Arg Arg Trp Ser Pro Pro Lys Leu Arg Gln Ile Val
                325                 330                 335

Pro Ala Lys

<210> 293
<211> 1027
<212> DNA
<213> Corynebacterium glutamicum

<220>
<221> CDS

<222> (101)..(1027)
<223> FRXA00243

<400> 293
cactgcgcca gatttttgat gccgacactg tggcaggtgt gcgcgctgag tacgaaaaat 60

ttaacaaagc agcccatgat ggaaatgaag aggaacagaa gtg acc agc gaa caa 115
Val Thr Ser Glu Gln
1               5

gct tta gat cct atc cac cca ggt cag ttc cgt ctt tct cgg att cag 163
Ala Leu Asp Pro Ile His Pro Gly Gln Phe Arg Leu Ser Arg Ile Gln
                10                  15                  20

ttg atc aac tgg gga acc ttc cac gga acg gtg gac att cct gtg acc 211
Leu Ile Asn Trp Gly Thr Phe His Gly Thr Val Asp Ile Pro Val Thr
            25                  30                  35

agg gaa gga atc tta gtt acc ggt ggt tcg gga tca gga aaa tcc acg 259
Arg Glu Gly Ile Leu Val Thr Gly Gly Ser Gly Ser Gly Lys Ser Thr
        40                  45                  50

ctg att gat gcg atc acg gcg gta ttg ctt ccg caa gga aag ctg agg 307
Leu Ile Asp Ala Ile Thr Ala Val Leu Leu Pro Gln Gly Lys Leu Arg
    55                  60                  65

ttt aac tct gcc gca cag gct aat act ccg cgg aat aag gga cgc agt 355
Phe Asn Ser Ala Ala Gln Ala Asn Thr Pro Arg Asn Lys Gly Arg Ser
70                  75                  80                  85

ttg gtt acc tat atc cgt ggc gct tgg cgt gcg cag gag gat ccg ctg 403
Leu Val Thr Tyr Ile Arg Gly Ala Trp Arg Ala Gln Glu Asp Pro Leu
                90                  95                  100

cag gat cag att gtc tcc acg tac cta cgt ccc cgc gca acc tat tcg 451
Gln Asp Gln Ile Val Ser Thr Tyr Leu Arg Pro Arg Ala Thr Tyr Ser
            105                 110                 115

ctg gtt gga ttg act tat tcc aac ggt gaa ggc gtc gag cac acc ttg 499
Leu Val Gly Leu Thr Tyr Ser Asn Gly Glu Gly Val Glu His Thr Leu
        120                 125                 130

gtg gct att ttc tat ctg aaa tcg gga cac aat tta acc tcc gat att 547
Val Ala Ile Phe Tyr Leu Lys Ser Gly His Asn Leu Thr Ser Asp Ile
    135                 140                 145

tct tca tat tat ggt gtg ttt ccc gtt gat caa gac atc aat gcg ctg 595
Ser Ser Tyr Tyr Gly Val Phe Pro Val Asp Gln Asp Ile Asn Ala Leu
150                 155                 160                 165

ctg gat ttc ctg aaa gag ggc atc gat aaa cgc cag atc aga gct gct 643
Leu Asp Phe Leu Lys Glu Gly Ile Asp Lys Arg Gln Ile Arg Ala Ala
                170                 175                 180

ttc aag gaa gcc atc ttt agc gag cag cat tct gta ttc tcc ggc agg 691
Phe Lys Glu Ala Ile Phe Ser Glu Gln His Ser Val Phe Ser Gly Arg
            185                 190                 195

ttt aga agc cgt ttg ggg atc tcc agt gag gaa gct ttg ctg ttg ttg 739
Phe Arg Ser Arg Leu Gly Ile Ser Ser Glu Glu Ala Leu Leu Leu Leu
        200                 205                 210

cac cgc gcg cag tcg gcg aaa gat ctt caa agc ttg gat gat cta ttt 787
His Arg Ala Gln Ser Ala Lys Asp Leu Gln Ser Leu Asp Asp Leu Phe
    215                 220                 225

cgg gat tac atg ctg gtg gaa ccg gat acg ttc agc att gcc aaa act 835
Arg Asp Tyr Met Leu Val Glu Pro Asp Thr Phe Ser Ile Ala Lys Thr
230                 235                 240                 245

gcc gtg gaa caa ttc caa gac ctt gaa ggt gct tat gag cag gtc gaa 883
Ala Val Glu Gln Phe Gln Asp Leu Glu Gly Ala Tyr Glu Gln Val Glu
                250                 255                 260

gat att aaa cgg cag atc cac acc ctg gat cct ttg gtg cag ctg aag 931
Asp Ile Lys Arg Gln Ile His Thr Leu Asp Pro Leu Val Gln Leu Lys
            265                 270                 275

aat cgg cga gag aaa gcg caa cag tcc aaa gat cat gcc aat gca ctg 979
Asn Arg Arg Glu Lys Ala Gln Gln Ser Lys Asp His Ala Asn Ala Leu
        280                 285                 290

aag aag gcg ctg ccg act gtc ggg aat cgc att aag aag gaa gag caa 1027
Lys Lys Ala Leu Pro Thr Val Gly Asn Arg Ile Lys Lys Glu Glu Gln
    295                 300                 305

<210> 294
<211> 309
<212> PRT
<213> Corynebacterium glutamicum

<400> 294
Val Thr Ser Glu Gln Ala Leu Asp Pro Ile His Pro Gly Gln Phe Arg
1               5                   10                  15

Leu Ser Arg Ile Gln Leu Ile Asn Trp Gly Thr Phe His Gly Thr Val
            20                  25                  30

Asp Ile Pro Val Thr Arg Glu Gly Ile Leu Val Thr Gly Gly Ser Gly
        35                  40                  45

Ser Gly Lys Ser Thr Leu Ile Asp Ala Ile Thr Ala Val Leu Leu Pro
    50                  55                  60

Gln Gly Lys Leu Arg Phe Asn Ser Ala Ala Gln Ala Asn Thr Pro Arg
65                  70                  75                  80

Asn Lys Gly Arg Ser Leu Val Thr Tyr Ile Arg Gly Ala Trp Arg Ala
                85                  90                  95

Gln Glu Asp Pro Leu Gln Asp Gln Ile Val Ser Thr Tyr Leu Arg Pro
            100                 105                 110

Arg Ala Thr Tyr Ser Leu Val Gly Leu Thr Tyr Ser Asn Gly Glu Gly
        115                 120                 125

Val Glu His Thr Leu Val Ala Ile Phe Tyr Leu Lys Ser Gly His Asn
    130                 135                 140

Leu Thr Ser Asp Ile Ser Ser Tyr Tyr Gly Val Phe Pro Val Asp Gln
145                 150                 155                 160

Asp Ile Asn Ala Leu Leu Asp Phe Leu Lys Glu Gly Ile Asp Lys Arg
                165                 170                 175

Gln Ile Arg Ala Ala Phe Lys Glu Ala Ile Phe Ser Glu Gln His Ser
            180                 185                 190

Val Phe Ser Gly Arg Phe Arg Ser Arg Leu Gly Ile Ser Ser Glu Glu
        195                 200                 205

Ala Leu Leu Leu Leu His Arg Ala Gln Ser Ala Lys Asp Leu Gln Ser
    210                 215                 220

Leu Asp Asp Leu Phe Arg Asp Tyr Met Leu Val Glu Pro Asp Thr Phe
225                 230                 235                 240

Ser Ile Ala Lys Thr Ala Val Glu Gln Phe Gln Asp Leu Glu Gly Ala
                245                 250                 255

Tyr Glu Gln Val Glu Asp Ile Lys Arg Gln Ile His Thr Leu Asp Pro
            260                 265                 270

Leu Val Gln Leu Lys Asn Arg Arg Glu Lys Ala Gln Gln Ser Lys Asp
        275                 280                 285

His Ala Asn Ala Leu Lys Lys Ala Leu Pro Thr Val Gly Asn Arg Ile
    290                 295                 300

Lys Lys Glu Glu Gln
305

<210> 295
<211> 2325
<212> DNA
<213> Corynebacterium glutamicum

<220>
<221> CDS
<222> (101)..(2302)
<223> RXA00259

<400> 295
ggccttatta acatacggcg gttctagcac acagcgatgg atgatgtgtc ccaccgatat 60

tgctgcatgt gccaagattt agcacggtac agtgctagaa atg agc gga ctg ttt 115
                                            Met Ser Gly Leu Phe
                                            1               5

acc cca ttt tca gat gcg gca aaa aac aac acg gta aaa act gat gga 163
Thr Pro Phe Ser Asp Ala Ala Lys Asn Asn Thr Val Lys Thr Asp Gly
                10                  15                  20

gat tca gta tct ggt cga gac ttg cct att act aag atc tct gag gat 211
Asp Ser Val Ser Gly Arg Asp Leu Pro Ile Thr Lys Ile Ser Glu Asp
            25                  30                  35

cgt ttc gag cgt tct gcg tat tca gcc cag ctg gca aat ata atc tgc 259
Arg Phe Glu Arg Ser Ala Tyr Ser Ala Gln Leu Ala Asn Ile Ile Cys
        40                  45                  50

gat gtg gca cct tgg gga gcg agc act gtt ttc agt ctt act ggt cag 307
Asp Val Ala Pro Trp Gly Ala Ser Thr Val Phe Ser Leu Thr Gly Gln
    55                  60                  65

tgg ggc agt ggt aag aca tct ctt gtt aat ttg att cgc tcg gaa gaa 355
Trp Gly Ser Gly Lys Thr Ser Leu Val Asn Leu Ile Arg Ser Glu Glu
70                  75                  80                  85

tct cta tcg aac gaa aaa tgg aca atc gtt gat ttc aac ccg tgg gtg 403
Ser Leu Ser Asn Glu Lys Trp Thr Ile Val Asp Phe Asn Pro Trp Val
                90                  95                  100

gcc tct gac ccg caa tct ttg att gag gag ttt tac cga gta atc gtt 451
Ala Ser Asp Pro Gln Ser Leu Ile Glu Glu Phe Tyr Arg Val Ile Val
            105                 110                 115

ggg acg gta cct gat gat aag acc ggc caa aag atc aaa act gtt ctg 499
Gly Thr Val Pro Asp Asp Lys Thr Gly Gln Lys Ile Lys Thr Val Leu
        120                 125                 130

cag aaa acc ttt agc acg att ggg tca att gca ggt ggg gtc gga ggg 547
Gln Lys Thr Phe Ser Thr Ile Gly Ser Ile Ala Gly Gly Val Gly Gly
    135                 140                 145

ttt ggt gtc cta gaa gca ctt gcg ctc tca aaa gga gta gat gct gca 595
Phe Gly Val Leu Glu Ala Leu Ala Leu Ser Lys Gly Val Asp Ala Ala
150                 155                 160                 165

aac gct gta tat aag aca tgg aaa cag gag caa gat tcg tgg cca acg 643
Asn Ala Val Tyr Lys Thr Trp Lys Gln Glu Gln Asp Ser Trp Pro Thr
                170                 175                 180

ctg tat aca cgt gct gcg aac cat ttt aaa gat ctg aac aag cga att 691
Leu Tyr Thr Arg Ala Ala Asn His Phe Lys Asp Leu Asn Lys Arg Ile
            185                 190                 195

ctc att gtc gtc gat gat att gat cgc ctc cat act gat gaa ttg gcg 739
Leu Ile Val Val Asp Asp Ile Asp Arg Leu His Thr Asp Glu Leu Ala
        200                 205                 210

ctg tta atg aaa gta ata cgc ttg ctt gga cga ttc ccg cag gtg aat 787
Leu Leu Met Lys Val Ile Arg Leu Leu Gly Arg Phe Pro Gln Val Asn
    215                 220                 225

tat ctt ttg gtt tat gaa gaa gaa tca ctg tta acg acg cta gcc aga 835
Tyr Leu Leu Val Tyr Glu Glu Glu Ser Leu Leu Thr Thr Leu Ala Arg
230                 235                 240                 245

tcg aca gct gta ggt ggt agc gaa gat gat gct ttg cgt ttc atg gag 883
Ser Thr Ala Val Gly Gly Ser Glu Asp Asp Ala Leu Arg Phe Met Glu
                250                 255                 260

aaa atc gtg cag tat cct ttc gat gtt ccg cct ctg aca tca ttt caa 931
Lys Ile Val Gln Tyr Pro Phe Asp Val Pro Pro Leu Thr Ser Phe Gln
            265                 270                 275

ata gag aaa gag ctc agt gca tta ttt gac aag ctt ttc cag ggt gtt 979
Ile Glu Lys Glu Leu Ser Ala Leu Phe Asp Lys Leu Phe Gln Gly Val
        280                 285                 290

tcg cta tcg ggt gat cct gaa gac ttt gca cta gtg aag tcg aga atg 1027

Ser Leu Ser Gly Asp Pro Glu Asp Phe Ala Leu Val Lys Ser Arg Met
    295                 300                 305

ttc gat gtc tgg gaa aag act ctg gtc acg ccg agg ctg ttg cac cgt    1075
Phe Asp Val Trp Glu Lys Thr Leu Val Thr Pro Arg Leu Leu His Arg
310                 315                 320                 325

ttt gct gct cta cta acc aac tgg act cgg ata tat gga tca ggt gaa    1123
Phe Ala Ala Leu Leu Thr Asn Trp Thr Arg Ile Tyr Gly Ser Gly Glu
                330                 335                 340

gtt aac ggc gtt gat ctc aca ata ctt gcg acc att cga att gtt ttt    1171
Val Asn Gly Val Asp Leu Thr Ile Leu Ala Thr Ile Arg Ile Val Phe
            345                 350                 355

ccg tct gtg tat aaa cgt ctt tct cga gcg aag gaa gta ttg ctt caa    1219
Pro Ser Val Tyr Lys Arg Leu Ser Arg Ala Lys Glu Val Leu Leu Gln
        360                 365                 370

gga ggt cga acg aca ggc tcg cag aaa ccc ggt tgg gaa aag caa tta    1267
Gly Gly Arg Thr Thr Gly Ser Gln Lys Pro Gly Trp Glu Lys Gln Leu
    375                 380                 385

tgt gag ggg atg aac aac gag cag atg gat ctt tta aag acc atg ctt    1315
Cys Glu Gly Met Asn Asn Glu Gln Met Asp Leu Leu Lys Thr Met Leu
390                 395                 400                 405

ttg ttc ctt ttc cca cgt ctt tcg gat cac cct agt acg aga atg cat    1363
Leu Phe Leu Phe Pro Arg Leu Ser Asp His Pro Ser Thr Arg Met His
                410                 415                 420

cgt gag agg ggg atc tcg acg gaa gtt tat ttt gac acg tac ctc atg    1411
Arg Glu Arg Gly Ile Ser Thr Glu Val Tyr Phe Asp Thr Tyr Leu Met
            425                 430                 435

ttt caa aga cct gga cat gtc ata agt gat gaa cag ttg gat aag tat    1459
Phe Gln Arg Pro Gly His Val Ile Ser Asp Glu Gln Leu Asp Lys Tyr
        440                 445                 450

cta tct aat gcg gac gat gct atg ggt ttc gtc gat tta att aac tcc    1507
Leu Ser Asn Ala Asp Asp Ala Met Gly Phe Val Asp Leu Ile Asn Ser
    455                 460                 465

gat gac aat gac atg gtg gca tca gtg atg aaa aag ctt cct cta gca    1555
Asp Asp Asn Asp Met Val Ala Ser Val Met Lys Lys Leu Pro Leu Ala
470                 475                 480                 485

att gat cga ctt gat gga gag ggt gtt agg cac atg gca gtt gag gtg    1603
Ile Asp Arg Leu Asp Gly Glu Gly Val Arg His Met Ala Val Glu Val
                490                 495                 500

tta ttc acc gct gct aat ggt atg cat gat aaa ggt cgt caa gtg cgt    1651
Leu Phe Thr Ala Ala Asn Gly Met His Asp Lys Gly Arg Gln Val Arg
            505                 510                 515

atg agc ggc ata ttc agt gac ctg tat tcc cat gcg tgc tcg att ctt    1699
Met Ser Gly Ile Phe Ser Asp Leu Tyr Ser His Ala Cys Ser Ile Leu
        520                 525                 530

ggt gca ttg cct caa tta cca gtg gaa caa ctc tat gag aaa ttc ttt    1747
Gly Ala Leu Pro Gln Leu Pro Val Glu Gln Leu Tyr Glu Lys Phe Phe

535                 540                 545

tct gag atg acg ctt aat gag gct gct ttc tgg tta aac cag gtg ggg     1795
Ser Glu Met Thr Leu Asn Glu Ala Ala Phe Trp Leu Asn Gln Val Gly
550                 555                 560                 565

gaa agg gct aga gcc tgt ggt aat gat gta agt ggc ctt gag ctt ttt     1843
Glu Arg Ala Arg Ala Cys Gly Asn Asp Val Ser Gly Leu Glu Leu Phe
                570                 575                 580

cgt aaa gtt aat ata aag acc gaa gct aga att tta agt gta ttg aag     1891
Arg Lys Val Asn Ile Lys Thr Glu Ala Arg Ile Leu Ser Val Leu Lys
            585                 590                 595

aat cag gac ccc tca gat tgg gat tta ggt cca tat tcg ctt ggt att     1939
Asn Gln Asp Pro Ser Asp Trp Asp Leu Gly Pro Tyr Ser Leu Gly Ile
        600                 605                 610

ttg gcg aaa agc tcg aat ttt tct tca gtg ctg aag tct ctg caa agt     1987
Leu Ala Lys Ser Ser Asn Phe Ser Ser Val Leu Lys Ser Leu Gln Ser
    615                 620                 625

ggt ata gag gaa cat cag ttt gat gtg ata gat att gga gtg ctt ttc     2035
Gly Ile Glu Glu His Gln Phe Asp Val Ile Asp Ile Gly Val Leu Phe
630                 635                 640                 645

tta acg act gtg tat tct tcg cga cag gga cca agc ggt ggt gca tgg     2083
Leu Thr Thr Val Tyr Ser Ser Arg Gln Gly Pro Ser Gly Gly Ala Trp
                650                 655                 660

ata gat tct ttt cag cat agt ctg ttt tca cgg tac gta cct gat tct     2131
Ile Asp Ser Phe Gln His Ser Leu Phe Ser Arg Tyr Val Pro Asp Ser
            665                 670                 675

cta cgg gct ata acc aag tct gaa gta gat gta gaa cta ggt aag ata     2179
Leu Arg Ala Ile Thr Lys Ser Glu Val Asp Val Glu Leu Gly Lys Ile
        680                 685                 690

cag ttc acg gat ttt agc tgg gaa ggg aag cga aaa gtt gtc gca tat     2227
Gln Phe Thr Asp Phe Ser Trp Glu Gly Lys Arg Lys Val Val Ala Tyr
    695                 700                 705

gca ctg gag act gga aga agt gat ttc act cga gaa cga tta ggg ggc     2275
Ala Leu Glu Thr Gly Arg Ser Asp Phe Thr Arg Glu Arg Leu Gly Gly
710                 715                 720                 725

tac agt atc gca gat tct ata gtc gat tgatgaggct gaggtcatga           2322
Tyr Ser Ile Ala Asp Ser Ile Val Asp
                730

ctt                                                                 2325


<210> 296
<211> 734
<212> PRT
<213> Corynebacterium glutamicum

<400> 296
Met Ser Gly Leu Phe Thr Pro Phe Ser Asp Ala Ala Lys Asn Asn Thr
  1               5                  10                  15

Val Lys Thr Asp Gly Asp Ser Val Ser Gly Arg Asp Leu Pro Ile Thr
            20                  25                  30

Lys Ile Ser Glu Asp Arg Phe Glu Arg Ser Ala Tyr Ser Ala Gln Leu
        35                  40                  45

Ala Asn Ile Ile Cys Asp Val Ala Pro Trp Gly Ala Ser Thr Val Phe
    50                  55                  60

Ser Leu Thr Gly Gln Trp Gly Ser Gly Lys Thr Ser Leu Val Asn Leu
65                  70                  75                  80

Ile Arg Ser Glu Glu Ser Leu Ser Asn Glu Lys Trp Thr Ile Val Asp
                85                  90                  95

Phe Asn Pro Trp Val Ala Ser Asp Pro Gln Ser Leu Ile Glu Glu Phe
            100                 105                 110

Tyr Arg Val Ile Val Gly Thr Val Pro Asp Asp Lys Thr Gly Gln Lys
        115                 120                 125

Ile Lys Thr Val Leu Gln Lys Thr Phe Ser Thr Ile Gly Ser Ile Ala
    130                 135                 140

Gly Gly Val Gly Gly Phe Gly Val Leu Glu Ala Leu Ala Leu Ser Lys
145                 150                 155                 160

Gly Val Asp Ala Ala Asn Ala Val Tyr Lys Thr Trp Lys Gln Glu Gln
                165                 170                 175

Asp Ser Trp Pro Thr Leu Tyr Thr Arg Ala Ala Asn His Phe Lys Asp
            180                 185                 190

Leu Asn Lys Arg Ile Leu Ile Val Val Asp Asp Ile Asp Arg Leu His
        195                 200                 205

Thr Asp Glu Leu Ala Leu Leu Met Lys Val Ile Arg Leu Leu Gly Arg
    210                 215                 220

Phe Pro Gln Val Asn Tyr Leu Leu Val Tyr Glu Glu Glu Ser Leu Leu
225                 230                 235                 240

Thr Thr Leu Ala Arg Ser Thr Ala Val Gly Gly Ser Glu Asp Asp Ala
                245                 250                 255

Leu Arg Phe Met Glu Lys Ile Val Gln Tyr Pro Phe Asp Val Pro Pro
            260                 265                 270

Leu Thr Ser Phe Gln Ile Glu Lys Glu Leu Ser Ala Leu Phe Asp Lys
        275                 280                 285

Leu Phe Gln Gly Val Ser Leu Ser Gly Asp Pro Glu Asp Phe Ala Leu
    290                 295                 300

Val Lys Ser Arg Met Phe Asp Val Trp Glu Lys Thr Leu Val Thr Pro
305                 310                 315                 320

Arg Leu Leu His Arg Phe Ala Ala Leu Leu Thr Asn Trp Thr Arg Ile
                325                 330                 335

Tyr Gly Ser Gly Glu Val Asn Gly Val Asp Leu Thr Ile Leu Ala Thr
            340                 345                 350

Ile Arg Ile Val Phe Pro Ser Val Tyr Lys Arg Leu Ser Arg Ala Lys
        355                 360                 365

Glu Val Leu Leu Gln Gly Gly Arg Thr Thr Gly Ser Gln Lys Pro Gly
    370                 375                 380

Trp Glu Lys Gln Leu Cys Glu Gly Met Asn Asn Glu Gln Met Asp Leu
385                 390                 395                 400

Leu Lys Thr Met Leu Leu Phe Leu Phe Pro Arg Leu Ser Asp His Pro
                405                 410                 415

Ser Thr Arg Met His Arg Glu Arg Gly Ile Ser Thr Glu Val Tyr Phe
            420                 425                 430

Asp Thr Tyr Leu Met Phe Gln Arg Pro Gly His Val Ile Ser Asp Glu
        435                 440                 445

Gln Leu Asp Lys Tyr Leu Ser Asn Ala Asp Asp Ala Met Gly Phe Val
    450                 455                 460

Asp Leu Ile Asn Ser Asp Asp Asn Asp Met Val Ala Ser Val Met Lys
465                 470                 475                 480

Lys Leu Pro Leu Ala Ile Asp Arg Leu Asp Gly Glu Gly Val Arg His
                485                 490                 495

Met Ala Val Glu Val Leu Phe Thr Ala Ala Asn Gly Met His Asp Lys
            500                 505                 510

Gly Arg Gln Val Arg Met Ser Gly Ile Phe Ser Asp Leu Tyr Ser His
        515                 520                 525

Ala Cys Ser Ile Leu Gly Ala Leu Pro Gln Leu Pro Val Glu Gln Leu
    530                 535                 540

Tyr Glu Lys Phe Phe Ser Glu Met Thr Leu Asn Glu Ala Ala Phe Trp
545                 550                 555                 560

Leu Asn Gln Val Gly Glu Arg Ala Arg Ala Cys Gly Asn Asp Val Ser
                565                 570                 575

Gly Leu Glu Leu Phe Arg Lys Val Asn Ile Lys Thr Glu Ala Arg Ile
            580                 585                 590

Leu Ser Val Leu Lys Asn Gln Asp Pro Ser Asp Trp Asp Leu Gly Pro
        595                 600                 605

Tyr Ser Leu Gly Ile Leu Ala Lys Ser Ser Asn Phe Ser Ser Val Leu
    610                 615                 620

Lys Ser Leu Gln Ser Gly Ile Glu Glu His Gln Phe Asp Val Ile Asp
625                 630                 635                 640

Ile Gly Val Leu Phe Leu Thr Thr Val Tyr Ser Ser Arg Gln Gly Pro
                645                 650                 655

Ser Gly Gly Ala Trp Ile Asp Ser Phe Gln His Ser Leu Phe Ser Arg

660                 665                 670

Tyr Val Pro Asp Ser Leu Arg Ala Ile Thr Lys Ser Glu Val Asp Val
        675                 680                 685

Glu Leu Gly Lys Ile Gln Phe Thr Asp Phe Ser Trp Glu Gly Lys Arg
    690                 695                 700

Lys Val Val Ala Tyr Ala Leu Glu Thr Gly Arg Ser Asp Phe Thr Arg
705                 710                 715                 720

Glu Arg Leu Gly Gly Tyr Ser Ile Ala Asp Ser Ile Val Asp
                725                 730

<210> 297
<211> 789
<212> DNA
<213> Corynebacterium glutamicum

<220>
<221> CDS
<222> (101)..(766)
<223> RXN00410

<400> 297
gtgttgatgc gttagtccac ccacgcagct acgccccaaa ggaataatct tgaaccctgc 60

cacagataac gctccgccgg tcctttcagc ccaagatctc atg atg atc tat gga   115
                                           Met Met Ile Tyr Gly
                                           1               5

aaa gga tca aca gaa gtt cgg gct ctc gat ggc att tct gta cag att   163
Lys Gly Ser Thr Glu Val Arg Ala Leu Asp Gly Ile Ser Val Gln Ile
                10                  15                  20

cag tcc gac aaa tgg acc tcc atc atg ggg caa tca ggc tct ggc aaa   211
Gln Ser Asp Lys Trp Thr Ser Ile Met Gly Gln Ser Gly Ser Gly Lys
            25                  30                  35

aca act ctg ttg cag tgc ctt tcc gga ttg gcg cag cca acc tca ggc   259
Thr Thr Leu Leu Gln Cys Leu Ser Gly Leu Ala Gln Pro Thr Ser Gly
        40                  45                  50

aga gtg aca ctg aac aaa aac aac atc acg ttg agc tcc ctg tca gaa   307
Arg Val Thr Leu Asn Lys Asn Asn Ile Thr Leu Ser Ser Leu Ser Glu
    55                  60                  65

aat aag cgt gcc aag ctg cgt cgc acg cac atc agc atg gtg ttt cag   355
Asn Lys Arg Ala Lys Leu Arg Arg Thr His Ile Ser Met Val Phe Gln
70                  75                  80                  85

gat ttc aac ttg gtg cct att ttg tcg gtg aag gac aat att ttg ctg   403
Asp Phe Asn Leu Val Pro Ile Leu Ser Val Lys Asp Asn Ile Leu Leu
                90                  95                  100

ccg ttg cgt ctt gcg cat cgc agg gtg gat aag cag tgg ttt gaa cac   451
Pro Leu Arg Leu Ala His Arg Arg Val Asp Lys Gln Trp Phe Glu His
            105                 110                 115

atc acc agt gtg ttg aag att gat aat cgt atg cgc cat ttg cct ggg   499

Ile Thr Ser Val Leu Lys Ile Asp Asn Arg Met Arg His Leu Pro Gly
        120                 125                 130

gag ctg tct ggc ggt cag caa caa cgc gcc gcg att gcc cgg gcg ttg     547
Glu Leu Ser Gly Gly Gln Gln Gln Arg Ala Ala Ile Ala Arg Ala Leu
    135                 140                 145

atg tct agg ccc gat att gtc att gcg gat gag cca aca gga agt ttg     595
Met Ser Arg Pro Asp Ile Val Ile Ala Asp Glu Pro Thr Gly Ser Leu
150                 155                 160                 165

gat tcc gtc acc agc gat gca gtg ttg aat ttg ttc cgc agc att gtt     643
Asp Ser Val Thr Ser Asp Ala Val Leu Asn Leu Phe Arg Ser Ile Val
                170                 175                 180

gat gat ttt ggg cag tca ctt gtg ttt gtc acc cac gat aaa gat gct     691
Asp Asp Phe Gly Gln Ser Leu Val Phe Val Thr His Asp Lys Asp Ala
            185                 190                 195

gct cac cgt ggt gac gtg ttg atc aca atg cgt gat ggc aag atc atc     739
Ala His Arg Gly Asp Val Leu Ile Thr Met Arg Asp Gly Lys Ile Ile
        200                 205                 210

gat acg gca gat ttg cgg gtg ggg cgt taatgttcag gcttgctttc           786
Asp Thr Ala Asp Leu Arg Val Gly Arg
    215                 220

gct                                                                 789


<210> 298
<211> 222
<212> PRT
<213> Corynebacterium glutamicum

<400> 298
Met Met Ile Tyr Gly Lys Gly Ser Thr Glu Val Arg Ala Leu Asp Gly
1               5                   10                  15

Ile Ser Val Gln Ile Gln Ser Asp Lys Trp Thr Ser Ile Met Gly Gln
            20                  25                  30

Ser Gly Ser Gly Lys Thr Thr Leu Leu Gln Cys Leu Ser Gly Leu Ala
        35                  40                  45

Gln Pro Thr Ser Gly Arg Val Thr Leu Asn Lys Asn Asn Ile Thr Leu
    50                  55                  60

Ser Ser Leu Ser Glu Asn Lys Arg Ala Lys Leu Arg Arg Thr His Ile
65                  70                  75                  80

Ser Met Val Phe Gln Asp Phe Asn Leu Val Pro Ile Leu Ser Val Lys
                85                  90                  95

Asp Asn Ile Leu Leu Pro Leu Arg Leu Ala His Arg Arg Val Asp Lys
            100                 105                 110

Gln Trp Phe Glu His Ile Thr Ser Val Leu Lys Ile Asp Asn Arg Met
        115                 120                 125

Arg His Leu Pro Gly Glu Leu Ser Gly Gly Gln Gln Gln Arg Ala Ala

130                 135                 140

Ile Ala Arg Ala Leu Met Ser Arg Pro Asp Ile Val Ile Ala Asp Glu
145                 150                 155                 160

Pro Thr Gly Ser Leu Asp Ser Val Thr Ser Asp Ala Val Leu Asn Leu
                165                 170                 175

Phe Arg Ser Ile Val Asp Asp Phe Gly Gln Ser Leu Val Phe Val Thr
            180                 185                 190

His Asp Lys Asp Ala Ala His Arg Gly Asp Val Leu Ile Thr Met Arg
        195                 200                 205

Asp Gly Lys Ile Ile Asp Thr Ala Asp Leu Arg Val Gly Arg
    210                 215                 220


<210> 299
<211> 789
<212> DNA
<213> Corynebacterium glutamicum

<220>
<221> CDS
<222> (101)..(766)
<223> FRXA00410

<400> 299
gtgttgatgc gttagtccac ccacgcagct acgccccaaa ggaataatct tgaaccctgc 60

cacagataac gctccgccgg tcctttcagc ccaagatctc atg atg atc tat gga    115
                                          Met Met Ile Tyr Gly
                                          1               5

aaa gga tca aca gaa gtt cgg gct ctc gat ggc att tct gta cag att    163
Lys Gly Ser Thr Glu Val Arg Ala Leu Asp Gly Ile Ser Val Gln Ile
                10                  15                  20

cag tcc gac aaa tgg acc tcc atc atg ggg caa tca ggc tct ggc aaa    211
Gln Ser Asp Lys Trp Thr Ser Ile Met Gly Gln Ser Gly Ser Gly Lys
            25                  30                  35

aca act ctg ttg cag tgc ctt tcc gga ttg gcg cag cca acc tca ggc    259
Thr Thr Leu Leu Gln Cys Leu Ser Gly Leu Ala Gln Pro Thr Ser Gly
        40                  45                  50

aga gtg aca ctg aac aaa aac aac atc acg ttg agc tcc ctg tca gaa    307
Arg Val Thr Leu Asn Lys Asn Asn Ile Thr Leu Ser Ser Leu Ser Glu
    55                  60                  65

aat aag cgt gcc aag ctg cgt cgc acg cac atc agc atg gtg ttt cag    355
Asn Lys Arg Ala Lys Leu Arg Arg Thr His Ile Ser Met Val Phe Gln
70                  75                  80                  85

gat ttc aac ttg gtg cct att ttg tcg gtg aag gac aat att ttg ctg    403
Asp Phe Asn Leu Val Pro Ile Leu Ser Val Lys Asp Asn Ile Leu Leu
                90                  95                  100

ccg ttg cgt ctt gcg cat cgc agg gtg gat aag cag tgg ttt gaa cac    451
Pro Leu Arg Leu Ala His Arg Arg Val Asp Lys Gln Trp Phe Glu His

105                 110                 115

atc acc agt gtg ttg aag att gat aat cgt atg cgc cat ttg cct ggg     499
Ile Thr Ser Val Leu Lys Ile Asp Asn Arg Met Arg His Leu Pro Gly
        120                 125                 130

gag ctg tct ggc ggt cag caa caa cgc gcc gcg att gcc cgg gcg ttg     547
Glu Leu Ser Gly Gly Gln Gln Gln Arg Ala Ala Ile Ala Arg Ala Leu
    135                 140                 145

atg tct agg ccc gat att gtc att gcg gat gag cca aca gga agt ttg     595
Met Ser Arg Pro Asp Ile Val Ile Ala Asp Glu Pro Thr Gly Ser Leu
150                 155                 160                 165

gat tcc gtc acc agc gat gca gtg ttg aat ttg ttc cgc agc att gtt     643
Asp Ser Val Thr Ser Asp Ala Val Leu Asn Leu Phe Arg Ser Ile Val
                170                 175                 180

gat gat ttt ggg cag tca ctt gtg ttt gtc acc cac gat aaa gat gct     691
Asp Asp Phe Gly Gln Ser Leu Val Phe Val Thr His Asp Lys Asp Ala
            185                 190                 195

gct cac cgt ggt gac gtg ttg atc aca atg cgt gat ggc aag atc atc     739
Ala His Arg Gly Asp Val Leu Ile Thr Met Arg Asp Gly Lys Ile Ile
        200                 205                 210

gat acg gca gat ttg cgg gtg ggg cgt taatgttcag gcttgctttc           786
Asp Thr Ala Asp Leu Arg Val Gly Arg
    215                 220

gct                                                                 789

<210> 300
<211> 222
<212> PRT
<213> Corynebacterium glutamicum

<400> 300
Met Met Ile Tyr Gly Lys Gly Ser Thr Glu Val Arg Ala Leu Asp Gly
  1               5                  10                  15

Ile Ser Val Gln Ile Gln Ser Asp Lys Trp Thr Ser Ile Met Gly Gln
             20                  25                  30

Ser Gly Ser Gly Lys Thr Thr Leu Leu Gln Cys Leu Ser Gly Leu Ala
         35                  40                  45

Gln Pro Thr Ser Gly Arg Val Thr Leu Asn Lys Asn Asn Ile Thr Leu
     50                  55                  60

Ser Ser Leu Ser Glu Asn Lys Arg Ala Lys Leu Arg Arg Thr His Ile
 65                  70                  75                  80

Ser Met Val Phe Gln Asp Phe Asn Leu Val Pro Ile Leu Ser Val Lys
                 85                  90                  95

Asp Asn Ile Leu Leu Pro Leu Arg Leu Ala His Arg Arg Val Asp Lys
            100                 105                 110

Gln Trp Phe Glu His Ile Thr Ser Val Leu Lys Ile Asp Asn Arg Met

115                 120                 125

Arg His Leu Pro Gly Glu Leu Ser Gly Gly Gln Gln Gln Arg Ala Ala
    130                 135                 140

Ile Ala Arg Ala Leu Met Ser Arg Pro Asp Ile Val Ile Ala Asp Glu
145                 150                 155                 160

Pro Thr Gly Ser Leu Asp Ser Val Thr Ser Asp Ala Val Leu Asn Leu
                165                 170                 175

Phe Arg Ser Ile Val Asp Asp Phe Gly Gln Ser Leu Val Phe Val Thr
            180                 185                 190

His Asp Lys Asp Ala Ala His Arg Gly Asp Val Leu Ile Thr Met Arg
        195                 200                 205

Asp Gly Lys Ile Ile Asp Thr Ala Asp Leu Arg Val Gly Arg
    210                 215                 220

<210> 301
<211> 1500
<212> DNA
<213> Corynebacterium glutamicum

<220>
<221> CDS
<222> (101)..(1477)
<223> RXN00456

<400> 301
ctcaccaacc cggagatcgt cacagcggtg ctaacggatc atgcctagct tatggcgtgc 60

tcgtcgcaga cttttgctca ttgccctagg tgtacttggt gtg ctg cag gca ctg   115
                                          Val Leu Gln Ala Leu
                                          1               5

ctg gcg atc atg gtg tcg ttg agc gta gcc gcc ata ctt gag gga aac   163
Leu Ala Ile Met Val Ser Leu Ser Val Ala Ala Ile Leu Glu Gly Asn
                10                  15                  20

cga gca ctt gtt gga ttg ctg ctt gct acc acg ttg ggt ttg ggg gtg   211
Arg Ala Leu Val Gly Leu Leu Leu Ala Thr Thr Leu Gly Leu Gly Val
            25                  30                  35

gcg cag tgg att caa aaa gta gtg gca gaa gat cta ggc cag cat tat   259
Ala Gln Trp Ile Gln Lys Val Val Ala Glu Asp Leu Gly Gln His Tyr
        40                  45                  50

gtg cat gag gtg cgt cgt gaa ttg gtg ggt gct gcg ctg gtg cct gga   307
Val His Glu Val Arg Arg Glu Leu Val Gly Ala Ala Leu Val Pro Gly
    55                  60                  65

aat acg gcc tcg ttg ggc gtg act gtc acc cga gcc agc aat gat ctc   355
Asn Thr Ala Ser Leu Gly Val Thr Val Thr Arg Ala Ser Asn Asp Leu
70                  75                  80                  85

acc gcg gtg cgc aat tgg gtg gct ttg ggc att gtt ccg atg gtc acc   403
Thr Ala Val Arg Asn Trp Val Ala Leu Gly Ile Val Pro Met Val Thr
                90                  95                  100

ggg ctg ccg ttg att gcg att gtg ctg gtg gcg ttg ttt atc caa gat 451
Gly Leu Pro Leu Ile Ala Ile Val Leu Val Ala Leu Phe Ile Gln Asp
            105                 110                 115

ctc cgc aca ggc gtg gct gtt act gtg cca ctg ctc atg tgt gta gcc 499
Leu Arg Thr Gly Val Ala Val Thr Val Pro Leu Leu Met Cys Val Ala
        120                 125                 130

gtg ctg ccg gtg gtg gcg cgg tgg act ttg aaa aga gca cgt gaa cta 547
Val Leu Pro Val Val Ala Arg Trp Thr Leu Lys Arg Ala Arg Glu Leu
    135                 140                 145

cgc aaa aaa cgt gga cgc atg gct gcg cgg atc gca gat tct gtc atg 595
Arg Lys Lys Arg Gly Arg Met Ala Ala Arg Ile Ala Asp Ser Val Met
150                 155                 160                 165

gct gga gaa tta ctg cac gca aca gga gca ata gac cgt gag ctc aat 643
Ala Gly Glu Leu Leu His Ala Thr Gly Ala Ile Asp Arg Glu Leu Asn
                170                 175                 180

gca gtc acc cga gat tcc gac cga gtg gtg ata gct gct gta aga cgt 691
Ala Val Thr Arg Asp Ser Asp Arg Val Val Ile Ala Ala Val Arg Arg
            185                 190                 195

tcc tgg gcc acc ggt ttt agc cgc gca ttg atg gcc atg gca gcc tcg 739
Ser Trp Ala Thr Gly Phe Ser Arg Ala Leu Met Ala Met Ala Ala Ser
        200                 205                 210

ctt ggc act gtc agc att gtg att tct ggc cac ctg gaa gta agt gag 787
Leu Gly Thr Val Ser Ile Val Ile Ser Gly His Leu Glu Val Ser Glu
    215                 220                 225

gtt gcg gga ata atg atg ctt ctt ggc gtt ctt gcc act cca gtt gca 835
Val Ala Gly Ile Met Met Leu Leu Gly Val Leu Ala Thr Pro Val Ala
230                 235                 240                 245

gaa ctt ggc cgc gtg gtg gaa tat cgc caa aat tat aaa gcc gcg aca 883
Glu Leu Gly Arg Val Val Glu Tyr Arg Gln Asn Tyr Lys Ala Ala Thr
                250                 255                 260

cgc atc ctg att cca ctt ctg caa cga ggc tca gaa ttt aaa cac tcc 931
Arg Ile Leu Ile Pro Leu Leu Gln Arg Gly Ser Glu Phe Lys His Ser
            265                 270                 275

caa caa aaa cta ccc ggg ttg caa gca aca gaa gga atc ccc ggt gtc 979
Gln Gln Lys Leu Pro Gly Leu Gln Ala Thr Glu Gly Ile Pro Gly Val
        280                 285                 290

tat gtc aaa ggt att tcc gcc ctt cct gga gaa cgg atc tac ctc cac 1027
Tyr Val Lys Gly Ile Ser Ala Leu Pro Gly Glu Arg Ile Tyr Leu His
    295                 300                 305

ggc tct gca gat gcg acg aga aaa tgg gtc acc tcg ttg tct gca atg 1075
Gly Ser Ala Asp Ala Thr Arg Lys Trp Val Thr Ser Leu Ser Ala Met
310                 315                 320                 325

gag gaa ggc aca gat gta ata gtc aac ggt caa agg ctt tcg cag ctt 1123
Glu Glu Gly Thr Asp Val Ile Val Asn Gly Gln Arg Leu Ser Gln Leu
                330                 335                 340

cct ttg aaa caa cga cgc gcc ctc atc gga atc gcc tca gca cac cac 1171
Pro Leu Lys Gln Arg Arg Ala Leu Ile Gly Ile Ala Ser Ala His His
            345                 350                 355

cac tta agc cgt ggt tca gta tcg cgc ctg gtt ggt ttg cga gtg ccg 1219
His Leu Ser Arg Gly Ser Val Ser Arg Leu Val Gly Leu Arg Val Pro
        360                 365                 370

gat gcc acc gtg gaa gaa att gag caa gca ctg gaa caa gtt ggt ctg 1267
Asp Ala Thr Val Glu Glu Ile Glu Gln Ala Leu Glu Gln Val Gly Leu
    375                 380                 385

aac aac acc ggg aaa caa cgc ttg aaa aac ggc gga cac ccc tgg agt 1315
Asn Asn Thr Gly Lys Gln Arg Leu Lys Asn Gly Gly His Pro Trp Ser
390                 395                 400                 405

act tcg cag atc aac aaa ctg aaa att gcc agc gcc acc ctt cga acc 1363
Thr Ser Gln Ile Asn Lys Leu Lys Ile Ala Ser Ala Thr Leu Arg Thr
                410                 415                 420

cca ccg ctt ttg gta ctt gaa ggc atc acc cct gaa aac ctc ctc aac 1411
Pro Pro Leu Leu Val Leu Glu Gly Ile Thr Pro Glu Asn Leu Leu Asn
            425                 430                 435

tat ccc gga gtg atc atc tcc acc gtt cag gag aac cca tcc gaa aca 1459
Tyr Pro Gly Val Ile Ile Ser Thr Val Gln Glu Asn Pro Ser Glu Thr
        440                 445                 450

tgg cgg caa gtg aac atc taatctagaa acatggcagg acg 1500
Trp Arg Gln Val Asn Ile
    455

<210> 302
<211> 459
<212> PRT
<213> Corynebacterium glutamicum

<400> 302
Val Leu Gln Ala Leu Leu Ala Ile Met Val Ser Leu Ser Val Ala Ala
1               5                   10                  15

Ile Leu Glu Gly Asn Arg Ala Leu Val Gly Leu Leu Leu Ala Thr Thr
            20                  25                  30

Leu Gly Leu Gly Val Ala Gln Trp Ile Gln Lys Val Val Ala Glu Asp
        35                  40                  45

Leu Gly Gln His Tyr Val His Glu Val Arg Arg Glu Leu Val Gly Ala
    50                  55                  60

Ala Leu Val Pro Gly Asn Thr Ala Ser Leu Gly Val Thr Val Thr Arg
65                  70                  75                  80

Ala Ser Asn Asp Leu Thr Ala Val Arg Asn Trp Val Ala Leu Gly Ile
                85                  90                  95

Val Pro Met Val Thr Gly Leu Pro Leu Ile Ala Ile Val Leu Val Ala
            100                 105                 110

Leu Phe Ile Gln Asp Leu Arg Thr Gly Val Ala Val Thr Val Pro Leu

115                 120                 125

Leu Met Cys Val Ala Val Leu Pro Val Val Ala Arg Trp Thr Leu Lys
    130                 135                 140

Arg Ala Arg Glu Leu Arg Lys Lys Arg Gly Arg Met Ala Ala Arg Ile
145                 150                 155                 160

Ala Asp Ser Val Met Ala Gly Glu Leu Leu His Ala Thr Gly Ala Ile
                165                 170                 175

Asp Arg Glu Leu Asn Ala Val Thr Arg Asp Ser Asp Arg Val Val Ile
            180                 185                 190

Ala Ala Val Arg Arg Ser Trp Ala Thr Gly Phe Ser Arg Ala Leu Met
        195                 200                 205

Ala Met Ala Ala Ser Leu Gly Thr Val Ser Ile Val Ile Ser Gly His
    210                 215                 220

Leu Glu Val Ser Glu Val Ala Gly Ile Met Met Leu Leu Gly Val Leu
225                 230                 235                 240

Ala Thr Pro Val Ala Glu Leu Gly Arg Val Val Glu Tyr Arg Gln Asn
                245                 250                 255

Tyr Lys Ala Ala Thr Arg Ile Leu Ile Pro Leu Leu Gln Arg Gly Ser
            260                 265                 270

Glu Phe Lys His Ser Gln Gln Lys Leu Pro Gly Leu Gln Ala Thr Glu
        275                 280                 285

Gly Ile Pro Gly Val Tyr Val Lys Gly Ile Ser Ala Leu Pro Gly Glu
    290                 295                 300

Arg Ile Tyr Leu His Gly Ser Ala Asp Ala Thr Arg Lys Trp Val Thr
305                 310                 315                 320

Ser Leu Ser Ala Met Glu Glu Gly Thr Asp Val Ile Val Asn Gly Gln
                325                 330                 335

Arg Leu Ser Gln Leu Pro Leu Lys Gln Arg Arg Ala Leu Ile Gly Ile
            340                 345                 350

Ala Ser Ala His His His Leu Ser Arg Gly Ser Val Ser Arg Leu Val
        355                 360                 365

Gly Leu Arg Val Pro Asp Ala Thr Val Glu Glu Ile Glu Gln Ala Leu
    370                 375                 380

Glu Gln Val Gly Leu Asn Asn Thr Gly Lys Gln Arg Leu Lys Asn Gly
385                 390                 395                 400

Gly His Pro Trp Ser Thr Ser Gln Ile Asn Lys Leu Lys Ile Ala Ser
                405                 410                 415

Ala Thr Leu Arg Thr Pro Pro Leu Leu Val Leu Glu Gly Ile Thr Pro
            420                 425                 430

Glu Asn Leu Leu Asn Tyr Pro Gly Val Ile Ile Ser Thr Val Gln Glu
        435                 440                 445

Asn Pro Ser Glu Thr Trp Arg Gln Val Asn Ile
    450                 455

<210> 303
<211> 412
<212> DNA
<213> Corynebacterium glutamicum

<220>
<221> CDS
<222> (101)..(412)
<223> FRXA00456

<400> 303
ctcaccaacc cggagatcgt cacagcggtg ctaacggatc atgcctagct tatggcgtgc 60

tcgtcgcaga cttttgctca ttgccctagg tgtacttggt gtg ctg cag gca ctg   115
                                           Val Leu Gln Ala Leu
                                            1               5

ctg gcg atc atg gtg tcg ttg agc gta gcc gcc ata ctt gag gga aac   163
Leu Ala Ile Met Val Ser Leu Ser Val Ala Ala Ile Leu Glu Gly Asn
                10                  15                  20

cga gca ctt gtt gga ttg ctg ctt gct acc acg ttg ggt ttg ggg gtg   211
Arg Ala Leu Val Gly Leu Leu Leu Ala Thr Thr Leu Gly Leu Gly Val
            25                  30                  35

gcg cag tgg att caa aaa gta gtg gca gaa gat cta ggc cag cat tat   259
Ala Gln Trp Ile Gln Lys Val Val Ala Glu Asp Leu Gly Gln His Tyr
        40                  45                  50

gtg cat gag gtg cgt cgt gaa ttg gtg ggt gct gcg ctg gtg cct gga   307
Val His Glu Val Arg Arg Glu Leu Val Gly Ala Ala Leu Val Pro Gly
    55                  60                  65

aat acg gcc tcg ttg ggc gtg act gtc acc cga gcc agc aat gat ctc   355
Asn Thr Ala Ser Leu Gly Val Thr Val Thr Arg Ala Ser Asn Asp Leu
70                  75                  80                  85

acc gcg gtg cgc aat tgg gtg gct ttg ggc att gtt ccg atg gtc acc   403
Thr Ala Val Arg Asn Trp Val Ala Leu Gly Ile Val Pro Met Val Thr
                90                  95                  100

ggg ctg ccg                                                       412
Gly Leu Pro

<210> 304
<211> 104
<212> PRT
<213> Corynebacterium glutamicum

<400> 304
Val Leu Gln Ala Leu Leu Ala Ile Met Val Ser Leu Ser Val Ala Ala
1               5                   10                  15

Ile Leu Glu Gly Asn Arg Ala Leu Val Gly Leu Leu Leu Ala Thr Thr

                    20                          25                          30

Leu Gly Leu Gly Val Ala Gln Trp Ile Gln Lys Val Val Ala Glu Asp
            35                          40                          45

Leu Gly Gln His Tyr Val His Glu Val Arg Arg Glu Leu Val Gly Ala
        50                          55                          60

Ala Leu Val Pro Gly Asn Thr Ala Ser Leu Gly Val Thr Val Thr Arg
65                          70                          75                          80

Ala Ser Asn Asp Leu Thr Ala Val Arg Asn Trp Val Ala Leu Gly Ile
                        85                          90                          95

Val Pro Met Val Thr Gly Leu Pro
                100

<210> 305
<211> 1040
<212> DNA
<213> Corynebacterium glutamicum

<220>
<221> CDS
<222> (31)..(1017)
<223> FRXA00459

<400> 305
aggcgtggct gttactgtgc cactgctcat gtg tgt acc cgt gct gcc ggt ggt    54
                                 Val Cys Thr Arg Ala Ala Gly Gly
                                   1               5

ggc gcg gtg act ttg aaa aga gca cgt gaa cta cgc aaa aaa cgt gga    102
Gly Ala Val Thr Leu Lys Arg Ala Arg Glu Leu Arg Lys Lys Arg Gly
    10                  15                  20

cgc atg gct gcg cgg atc gca gat tct gtc atg gct gga gaa tta ctg    150
Arg Met Ala Ala Arg Ile Ala Asp Ser Val Met Ala Gly Glu Leu Leu
25                  30                  35                  40

cac gca aca gga gca ata gac cgt gag ctc aat gca gtc acc cga gat    198
His Ala Thr Gly Ala Ile Asp Arg Glu Leu Asn Ala Val Thr Arg Asp
                45                  50                  55

tcc gac cga gtg gtg ata gct gct gta aga cgt tcc tgg gcc acc ggt    246
Ser Asp Arg Val Val Ile Ala Ala Val Arg Arg Ser Trp Ala Thr Gly
            60                  65                  70

ttt agc cgc gca ttg atg gcc atg gca gcc tcg ctt ggc act gtc agc    294
Phe Ser Arg Ala Leu Met Ala Met Ala Ala Ser Leu Gly Thr Val Ser
        75                  80                  85

att gtg att tct ggc cac ctg gaa gta agt gag gtt gcg gga ata atg    342
Ile Val Ile Ser Gly His Leu Glu Val Ser Glu Val Ala Gly Ile Met
    90                  95                  100

atg ctt ctt ggc gtt ctt gcc act cca gtt gca gaa ctt ggc cgc gtg    390
Met Leu Leu Gly Val Leu Ala Thr Pro Val Ala Glu Leu Gly Arg Val
105                 110                 115                 120

gtg gaa tat cgc caa aat tat aaa gcc gcg aca cgc atc ctg att cca     438
Val Glu Tyr Arg Gln Asn Tyr Lys Ala Ala Thr Arg Ile Leu Ile Pro
                125                 130                 135

ctt ctg caa cga ggc tca gaa ttt aaa cac tcc caa caa aaa cta ccc     486
Leu Leu Gln Arg Gly Ser Glu Phe Lys His Ser Gln Gln Lys Leu Pro
            140                 145                 150

ggg ttg caa gca aca gaa gga atc ccc ggt gtc tat gtc aaa ggt att     534
Gly Leu Gln Ala Thr Glu Gly Ile Pro Gly Val Tyr Val Lys Gly Ile
        155                 160                 165

tcc gcc ctt cct gga gaa cgg atc tac ctc cac ggc tct gca gat gcg     582
Ser Ala Leu Pro Gly Glu Arg Ile Tyr Leu His Gly Ser Ala Asp Ala
    170                 175                 180

acg aga aaa tgg gtc acc tcg ttg tct gca atg gag gaa ggc aca gat     630
Thr Arg Lys Trp Val Thr Ser Leu Ser Ala Met Glu Glu Gly Thr Asp
185                 190                 195                 200

gta ata gtc aac ggt caa agg ctt tcg cag ctt cct ttg aaa caa cga     678
Val Ile Val Asn Gly Gln Arg Leu Ser Gln Leu Pro Leu Lys Gln Arg
                205                 210                 215

cgc gcc ctc atc gga atc gcc tca gca cac cac cac tta agc cgt ggt     726
Arg Ala Leu Ile Gly Ile Ala Ser Ala His His His Leu Ser Arg Gly
            220                 225                 230

tca gta tcg cgc ctg gtt ggt ttg cga gtg ccg gat gcc acc gtg gaa     774
Ser Val Ser Arg Leu Val Gly Leu Arg Val Pro Asp Ala Thr Val Glu
        235                 240                 245

gaa att gag caa gca ctg gaa caa gtt ggt ctg aac aac acc ggg aaa     822
Glu Ile Glu Gln Ala Leu Glu Gln Val Gly Leu Asn Asn Thr Gly Lys
    250                 255                 260

caa cgc ttg aaa aac ggc gga cac ccc tgg agt act tcg cag atc aac     870
Gln Arg Leu Lys Asn Gly Gly His Pro Trp Ser Thr Ser Gln Ile Asn
265                 270                 275                 280

aaa ctg aaa att gcc agc gcc acc ctt cga acc cca ccg ctt ttg gta     918
Lys Leu Lys Ile Ala Ser Ala Thr Leu Arg Thr Pro Pro Leu Leu Val
                285                 290                 295

ctt gaa ggc atc acc cct gaa aac ctc ctc aac tat ccc gga gtg atc     966
Leu Glu Gly Ile Thr Pro Glu Asn Leu Leu Asn Tyr Pro Gly Val Ile
            300                 305                 310

atc tcc acc gtt cag gag aac cca tcc gaa aca tgg cgg caa gtg aac     1014
Ile Ser Thr Val Gln Glu Asn Pro Ser Glu Thr Trp Arg Gln Val Asn
        315                 320                 325

atc taatctagaa acatggcagg acg                                       1040
Ile

<210> 306
<211> 329
<212> PRT
<213> Corynebacterium glutamicum

<400> 306
Val Cys Thr Arg Ala Ala Gly Gly Gly Ala Val Thr Leu Lys Arg Ala
1               5                   10                  15

Arg Glu Leu Arg Lys Lys Arg Gly Arg Met Ala Ala Arg Ile Ala Asp
            20                  25                  30

Ser Val Met Ala Gly Glu Leu Leu His Ala Thr Gly Ala Ile Asp Arg
        35                  40                  45

Glu Leu Asn Ala Val Thr Arg Asp Ser Asp Arg Val Val Ile Ala Ala
    50                  55                  60

Val Arg Arg Ser Trp Ala Thr Gly Phe Ser Arg Ala Leu Met Ala Met
65                  70                  75                  80

Ala Ala Ser Leu Gly Thr Val Ser Ile Val Ile Ser Gly His Leu Glu
                85                  90                  95

Val Ser Glu Val Ala Gly Ile Met Met Leu Leu Gly Val Leu Ala Thr
            100                 105                 110

Pro Val Ala Glu Leu Gly Arg Val Val Glu Tyr Arg Gln Asn Tyr Lys
        115                 120                 125

Ala Ala Thr Arg Ile Leu Ile Pro Leu Leu Gln Arg Gly Ser Glu Phe
    130                 135                 140

Lys His Ser Gln Gln Lys Leu Pro Gly Leu Gln Ala Thr Glu Gly Ile
145                 150                 155                 160

Pro Gly Val Tyr Val Lys Gly Ile Ser Ala Leu Pro Gly Glu Arg Ile
                165                 170                 175

Tyr Leu His Gly Ser Ala Asp Ala Thr Arg Lys Trp Val Thr Ser Leu
            180                 185                 190

Ser Ala Met Glu Glu Gly Thr Asp Val Ile Val Asn Gly Gln Arg Leu
        195                 200                 205

Ser Gln Leu Pro Leu Lys Gln Arg Arg Ala Leu Ile Gly Ile Ala Ser
    210                 215                 220

Ala His His His Leu Ser Arg Gly Ser Val Ser Arg Leu Val Gly Leu
225                 230                 235                 240

Arg Val Pro Asp Ala Thr Val Glu Glu Ile Glu Gln Ala Leu Glu Gln
                245                 250                 255

Val Gly Leu Asn Asn Thr Gly Lys Gln Arg Leu Lys Asn Gly Gly His
            260                 265                 270

Pro Trp Ser Thr Ser Gln Ile Asn Lys Leu Lys Ile Ala Ser Ala Thr
        275                 280                 285

Leu Arg Thr Pro Pro Leu Leu Val Leu Glu Gly Ile Thr Pro Glu Asn
    290                 295                 300

Leu Leu Asn Tyr Pro Gly Val Ile Ile Ser Thr Val Gln Glu Asn Pro
305                 310                 315                 320

Ser Glu Thr Trp Arg Gln Val Asn Ile
                325

<210> 307
<211> 771
<212> DNA
<213> Corynebacterium glutamicum

<220>
<221> CDS
<222> (101)..(748)
<223> RXN01604

<400> 307
ctctcatgtt gttgtcctct agttgacagc gggggtggtg gtggtcctaa aatagcctac 60

gataactgat agtgtttcct ccacttacgg aagaagatac atg aat acc cct ctt   115
                                           Met Asn Thr Pro Leu
                                           1               5

ttg aga agc tct ggg ctc tcc atc cgc gac aca ccc ttc gcc gat gtt   163
Leu Arg Ser Ser Gly Leu Ser Ile Arg Asp Thr Pro Phe Ala Asp Val
                10                  15                  20

gag ata gct cca gac agc gga ctc act ttg ctg agc acc ggg cgc gaa   211
Glu Ile Ala Pro Asp Ser Gly Leu Thr Leu Leu Ser Thr Gly Arg Glu
            25                  30                  35

tcc caa tcc agt tcc ttt tct ttg gta ctt tcc ggc cgc atg cgc gcc   259
Ser Gln Ser Ser Ser Phe Ser Leu Val Leu Ser Gly Arg Met Arg Ala
        40                  45                  50

tcc acc gga acc atc gaa tta aac ggc gaa ccc atc aag gca acc aag   307
Ser Thr Gly Thr Ile Glu Leu Asn Gly Glu Pro Ile Lys Ala Thr Lys
    55                  60                  65

ctg gcc aag cat gtg gct ttg gcg ggc atc cct gaa atc gat tca ctc   355
Leu Ala Lys His Val Ala Leu Ala Gly Ile Pro Glu Ile Asp Ser Leu
70                  75                  80                  85

gag cga ctt gtc act gtg cgc acc gtt gtc cgt gaa caa ctc gcc tgg   403
Glu Arg Leu Val Thr Val Arg Thr Val Val Arg Glu Gln Leu Ala Trp
                90                  95                  100

tca agc cct tgg tac ctg atg gtg ccc agg gat att agt gat tcg gga   451
Ser Ser Pro Trp Tyr Leu Met Val Pro Arg Asp Ile Ser Asp Ser Gly
            105                 110                 115

cgg tgg gtt gac gtc gaa aag cat ctt ggc ctg aac ctg aac cct aaa   499
Arg Trp Val Asp Val Glu Lys His Leu Gly Leu Asn Leu Asn Pro Lys
        120                 125                 130

acc tta atc ggc gac ctc agc gtg ctc gag cgt ttt aag ctg cgc atc   547
Thr Leu Ile Gly Asp Leu Ser Val Leu Glu Arg Phe Lys Leu Arg Ile
    135                 140                 145

gcg ctg gcg ctg ctg gcg cgg cca gag gcg caa ctg ttg gtc gtg gat   595
Ala Leu Ala Leu Leu Ala Arg Pro Glu Ala Gln Leu Leu Val Val Asp
150                 155                 160                 165

gat ccc gat caa gtg cgc agc atg gaa ttg cgt gcg gag gtg ttg cac 643
Asp Pro Asp Gln Val Arg Ser Met Glu Leu Arg Ala Glu Val Leu His
                170                 175                 180

gca ttg aaa ggc gtt gca gag gat ctc cct gtg gtc gtg gta tcc acc 691
Ala Leu Lys Gly Val Ala Glu Asp Leu Pro Val Val Val Val Ser Thr
            185                 190                 195

aac cca gat ttt gat tcc ttg gcc gat acc gct ttg acc att acg ggg 739
Asn Pro Asp Phe Asp Ser Leu Ala Asp Thr Ala Leu Thr Ile Thr Gly
        200                 205                 210

gct gga aac taatggcatt tttacacttt ggc 771
Ala Gly Asn
    215

<210> 308
<211> 216
<212> PRT
<213> Corynebacterium glutamicum

<400> 308
Met Asn Thr Pro Leu Leu Arg Ser Ser Gly Leu Ser Ile Arg Asp Thr
1               5                   10                  15

Pro Phe Ala Asp Val Glu Ile Ala Pro Asp Ser Gly Leu Thr Leu Leu
            20                  25                  30

Ser Thr Gly Arg Glu Ser Gln Ser Ser Ser Phe Ser Leu Val Leu Ser
        35                  40                  45

Gly Arg Met Arg Ala Ser Thr Gly Thr Ile Glu Leu Asn Gly Glu Pro
    50                  55                  60

Ile Lys Ala Thr Lys Leu Ala Lys His Val Ala Leu Ala Gly Ile Pro
65                  70                  75                  80

Glu Ile Asp Ser Leu Glu Arg Leu Val Thr Val Arg Thr Val Val Arg
                85                  90                  95

Glu Gln Leu Ala Trp Ser Ser Pro Trp Tyr Leu Met Val Pro Arg Asp
            100                 105                 110

Ile Ser Asp Ser Gly Arg Trp Val Asp Val Glu Lys His Leu Gly Leu
        115                 120                 125

Asn Leu Asn Pro Lys Thr Leu Ile Gly Asp Leu Ser Val Leu Glu Arg
    130                 135                 140

Phe Lys Leu Arg Ile Ala Leu Ala Leu Leu Ala Arg Pro Glu Ala Gln
145                 150                 155                 160

Leu Leu Val Val Asp Asp Pro Asp Gln Val Arg Ser Met Glu Leu Arg
                165                 170                 175

Ala Glu Val Leu His Ala Leu Lys Gly Val Ala Glu Asp Leu Pro Val
            180                 185                 190

Val Val Val Ser Thr Asn Pro Asp Phe Asp Ser Leu Ala Asp Thr Ala

195                 200                 205

Leu Thr Ile Thr Gly Ala Gly Asn
    210                 215

<210> 309
<211> 629
<212> DNA
<213> Corynebacterium glutamicum

<220>
<221> CDS
<222> (1)..(606)
<223> FRXA01604

<400> 309
gac aca ccc ttc gcc gat gtt gag ata gct cca gac agc gga ctc act      48
Asp Thr Pro Phe Ala Asp Val Glu Ile Ala Pro Asp Ser Gly Leu Thr
  1               5                  10                  15

ttg ctg agc acc ggg cgc gaa tcc caa tcc agt tcc ttt tct ttg gta      96
Leu Leu Ser Thr Gly Arg Glu Ser Gln Ser Ser Ser Phe Ser Leu Val
             20                  25                  30

ctt tcc ggc cgc atg cgc gcc tcc acc gga acc atc gaa tta aac ggc     144
Leu Ser Gly Arg Met Arg Ala Ser Thr Gly Thr Ile Glu Leu Asn Gly
         35                  40                  45

gaa ccc atc aag gca acc aag ctg gcc aag cat gtg gct ttg gcg ggc     192
Glu Pro Ile Lys Ala Thr Lys Leu Ala Lys His Val Ala Leu Ala Gly
     50                  55                  60

atc cct gaa atc gat tca ctc gag cga ctt gtc act gtg cgc acc gtt     240
Ile Pro Glu Ile Asp Ser Leu Glu Arg Leu Val Thr Val Arg Thr Val
 65                  70                  75                  80

gtc cgt gaa caa ctc gcc tgg tca agc cct tgg tac ctg atg gtg ccc     288
Val Arg Glu Gln Leu Ala Trp Ser Ser Pro Trp Tyr Leu Met Val Pro
                 85                  90                  95

agg gat att agt gat tcg gga cgg tgg gtt gac gtc gaa aag cat ctt     336
Arg Asp Ile Ser Asp Ser Gly Arg Trp Val Asp Val Glu Lys His Leu
            100                 105                 110

ggc ctg aac ctg aac cct aaa acc tta atc ggc gac ctc agc gtg ctc     384
Gly Leu Asn Leu Asn Pro Lys Thr Leu Ile Gly Asp Leu Ser Val Leu
        115                 120                 125

gag cgt ttt aag ctg cgc atc gcg ctg gcg ctg ctg gcg cgg cca gag     432
Glu Arg Phe Lys Leu Arg Ile Ala Leu Ala Leu Leu Ala Arg Pro Glu
    130                 135                 140

gcg caa ctg ttg gtc gtg gat gat ccc gat caa gtg cgc agc atg gaa     480
Ala Gln Leu Leu Val Val Asp Asp Pro Asp Gln Val Arg Ser Met Glu
145                 150                 155                 160

ttg cgt gcg gag gtg ttg cac gca ttg aaa ggc gtt gca gag gat ctc     528
Leu Arg Ala Glu Val Leu His Ala Leu Lys Gly Val Ala Glu Asp Leu
                165                 170                 175

cct gtg gtc gtg gta tcc acc aac cca gat ttt gat tcc ttg gcc gat     576
Pro Val Val Val Val Ser Thr Asn Pro Asp Phe Asp Ser Leu Ala Asp
            180                 185                 190

acc gct ttg acc att acg ggg gct gga aac taatggcatt tttacacttt     626
Thr Ala Leu Thr Ile Thr Gly Ala Gly Asn
        195                 200

ggc                                                               629

<210> 310
<211> 202
<212> PRT
<213> Corynebacterium glutamicum

<400> 310
Asp Thr Pro Phe Ala Asp Val Glu Ile Ala Pro Asp Ser Gly Leu Thr
  1               5                  10                  15

Leu Leu Ser Thr Gly Arg Glu Ser Gln Ser Ser Ser Phe Ser Leu Val
             20                  25                  30

Leu Ser Gly Arg Met Arg Ala Ser Thr Gly Thr Ile Glu Leu Asn Gly
         35                  40                  45

Glu Pro Ile Lys Ala Thr Lys Leu Ala Lys His Val Ala Leu Ala Gly
     50                  55                  60

Ile Pro Glu Ile Asp Ser Leu Glu Arg Leu Val Thr Val Arg Thr Val
 65                  70                  75                  80

Val Arg Glu Gln Leu Ala Trp Ser Ser Pro Trp Tyr Leu Met Val Pro
                 85                  90                  95

Arg Asp Ile Ser Asp Ser Gly Arg Trp Val Asp Val Glu Lys His Leu
            100                 105                 110

Gly Leu Asn Leu Asn Pro Lys Thr Leu Ile Gly Asp Leu Ser Val Leu
        115                 120                 125

Glu Arg Phe Lys Leu Arg Ile Ala Leu Ala Leu Leu Ala Arg Pro Glu
    130                 135                 140

Ala Gln Leu Leu Val Val Asp Asp Pro Asp Gln Val Arg Ser Met Glu
145                 150                 155                 160

Leu Arg Ala Glu Val Leu His Ala Leu Lys Gly Val Ala Glu Asp Leu
                165                 170                 175

Pro Val Val Val Val Ser Thr Asn Pro Asp Phe Asp Ser Leu Ala Asp
            180                 185                 190

Thr Ala Leu Thr Ile Thr Gly Ala Gly Asn
        195                 200

<210> 311
<211> 2262
<212> DNA
<213> Corynebacterium glutamicum

<220>
<221> CDS
<222> (101)..(2239)
<223> RXN02547

<400> 311
ggggccgtcg ataagcgaag aacaattagc actgcgcgcg ctggtctgac gcattggtta 60

aggggctggc ggtccggcgc cggaatcggc ggaggagctt ttg gag ctc aac aac 115
Leu Glu Leu Asn Asn
1               5

gct gcg cgg ctg acc gtg gat gag tat ccg gcg gcg agg gaa gcg ctt 163
Ala Ala Arg Leu Thr Val Asp Glu Tyr Pro Ala Ala Arg Glu Ala Leu
                10                  15                  20

gaa tct gca ggt cag agg aat gta gag gac cga acc cgt gcg gtt gat 211
Glu Ser Ala Gly Gln Arg Asn Val Glu Asp Arg Thr Arg Ala Val Asp
            25                  30                  35

gag ttc aaa gcg gcg gat caa gag ctg tct tct ttg agt aaa ggc agc 259
Glu Phe Lys Ala Ala Asp Gln Glu Leu Ser Ser Leu Ser Lys Gly Ser
        40                  45                  50

agt aat att gag tac cgt ttg ctg cag gtg cgg gaa aat ttg tgt cag 307
Ser Asn Ile Glu Tyr Arg Leu Leu Gln Val Arg Glu Asn Leu Cys Gln
    55                  60                  65

gat ttg ggc gtg agc ccg cgg gat atg ccc ttt gcc ggt gag ctg att 355
Asp Leu Gly Val Ser Pro Arg Asp Met Pro Phe Ala Gly Glu Leu Ile
70                  75                  80                  85

gat ccg aat aat gcg gaa tgg gaa ccc gtt gtg cag cgc att ttg ggt 403
Asp Pro Asn Asn Ala Glu Trp Glu Pro Val Val Gln Arg Ile Leu Gly
                90                  95                  100

ggt ttt gct gcg gaa atg ttg gtt cct cat ggg ttg ttg cca cgg gtt 451
Gly Phe Ala Ala Glu Met Leu Val Pro His Gly Leu Leu Pro Arg Val
            105                 110                 115

cgg gat tgg gta aat gcc aaa cat ttg gca gcg ctg ctg aaa ttc aac 499
Arg Asp Trp Val Asn Ala Lys His Leu Ala Ala Leu Leu Lys Phe Asn
        120                 125                 130

ggc gtg gtg aca acg ggg gag tac aaa acc tcg cgt ttt ccg gcg gat 547
Gly Val Val Thr Thr Gly Glu Tyr Lys Thr Ser Arg Phe Pro Ala Asp
    135                 140                 145

tcc ctg atc cga aaa gtt gat gtt gtg gag tcg ccg ttt cgc gat tgg 595
Ser Leu Ile Arg Lys Val Asp Val Val Glu Ser Pro Phe Arg Asp Trp
150                 155                 160                 165

gta aat caa gaa tta ggc aag cgt ttt aat att cgg tgc gtg cgc act 643
Val Asn Gln Glu Leu Gly Lys Arg Phe Asn Ile Arg Cys Val Arg Thr
                170                 175                 180

cct gag gaa ttg tcg gcg ctg ggg cca cgc gat cag ggc gtg acc att 691
Pro Glu Glu Leu Ser Ala Leu Gly Pro Arg Asp Gln Gly Val Thr Ile
            185                 190                 195

ttg ggt gtg cga aaa ttt gcg cag cag aca ggc gat ccg acg acg cgt 739
Leu Gly Val Arg Lys Phe Ala Gln Gln Thr Gly Asp Pro Thr Thr Arg
        200                 205                 210

tgg gaa aaa gat gat cgc cga aag ctg ggg gat cgt tcc aca tac cgt 787
Trp Glu Lys Asp Asp Arg Arg Lys Leu Gly Asp Arg Ser Thr Tyr Arg
    215                 220                 225

ttg ggt tcc acc aat gat gcc aag gtg gaa acg ctt cgg gaa acc gtg 835
Leu Gly Ser Thr Asn Asp Ala Lys Val Glu Thr Leu Arg Glu Thr Val
230                 235                 240                 245

aaa gct ggc aaa gca gtt gtg cag gca gct gat aat cgc att gct gca 883
Lys Ala Gly Lys Ala Val Val Gln Ala Ala Asp Asn Arg Ile Ala Ala
                250                 255                 260

aac cgc gct gag ctg cgg gaa ctt gaa cgg cag tat caa gct tcg caa 931
Asn Arg Ala Glu Leu Arg Glu Leu Glu Arg Gln Tyr Gln Ala Ser Gln
            265                 270                 275

gaa att ttg aaa gtg tcg tgg gct cag att gat gtg gaa tca gcc gac 979
Glu Ile Leu Lys Val Ser Trp Ala Gln Ile Asp Val Glu Ser Ala Asp
        280                 285                 290

gcg gcg att gct gag ctg gac cga ttg ctg gaa gag ctg aac aac act 1027
Ala Ala Ile Ala Glu Leu Asp Arg Leu Leu Glu Glu Leu Asn Asn Thr
    295                 300                 305

cca gag gcc acc gag ctt tcc gcg cgg cat gag gcg gcg aag cag acg 1075
Pro Glu Ala Thr Glu Leu Ser Ala Arg His Glu Ala Ala Lys Gln Thr
310                 315                 320                 325

ctc gcg agg gtt tct gac ttg ctt gtc gca gct cag agt gag gaa acc 1123
Leu Ala Arg Val Ser Asp Leu Leu Val Ala Ala Gln Ser Glu Glu Thr
                330                 335                 340

gtg gcg tcg atg aac ctg aaa cgc gcc gaa act gaa ttg aaa cgg ctc 1171
Val Ala Ser Met Asn Leu Lys Arg Ala Glu Thr Glu Leu Lys Arg Leu
            345                 350                 355

gaa agc ctg ccg gtt gcg gag gtt tct gaa gaa atc gcg cgg gaa gtg 1219
Glu Ser Leu Pro Val Ala Glu Val Ser Glu Glu Ile Ala Arg Glu Val
        360                 365                 370

gag aaa cta ttt ctt gcc aac acc cgc cgg gtt cac gcc gcc aac gtg 1267
Glu Lys Leu Phe Leu Ala Asn Thr Arg Arg Val His Ala Ala Asn Val
    375                 380                 385

gat gag cag acc att gcg ctg cgc gag gat ctg gac aaa caa atc gat 1315
Asp Glu Gln Thr Ile Ala Leu Arg Glu Asp Leu Asp Lys Gln Ile Asp
390                 395                 400                 405

gcc aat gag gca gaa ctt cga cgt tgt gaa aac caa att gtt ggc att 1363
Ala Asn Glu Ala Glu Leu Arg Arg Cys Glu Asn Gln Ile Val Gly Ile
                410                 415                 420

ttg cgc agc tat att gaa acg tgg cct gcg aac cgc gct gac tta caa 1411
Leu Arg Ser Tyr Ile Glu Thr Trp Pro Ala Asn Arg Ala Asp Leu Gln
            425                 430                 435

gcc gaa cct gag ttt gtt ggt gag gcc atc aac cgc ctc ggc gag ctt 1459

Ala Glu Pro Glu Phe Val Gly Glu Ala Ile Asn Arg Leu Gly Glu Leu
        440                 445                 450

cgc agc gat cgt ttg gca gaa ttc acg gcc aaa ttc cta ggg ctc atg 1507
Arg Ser Asp Arg Leu Ala Glu Phe Thr Ala Lys Phe Leu Gly Leu Met
    455                 460                 465

aac gag atg tcc acc cga aac ctc ggc caa atc tcg cgg cgt cta cgt 1555
Asn Glu Met Ser Thr Arg Asn Leu Gly Gln Ile Ser Arg Arg Leu Arg
470                 475                 480                 485

gat gcg cgc cgg gaa atc gag gag cgc atc gag ccg atc aac gcc tcc 1603
Asp Ala Arg Arg Glu Ile Glu Glu Arg Ile Glu Pro Ile Asn Ala Ser
                490                 495                 500

ttg gcg cag tcg gaa ttc aac gaa ggt cgc ttc ctg cac atc gac atc 1651
Leu Ala Gln Ser Glu Phe Asn Glu Gly Arg Phe Leu His Ile Asp Ile
            505                 510                 515

cgt gat caa agt ggt ccg att gtg agg gaa ttc cag cag aaa ctt gat 1699
Arg Asp Gln Ser Gly Pro Ile Val Arg Glu Phe Gln Gln Lys Leu Asp
        520                 525                 530

gcc gct acc agc ggt gac ctg gga acc agt acc gag aaa caa gcc ttc 1747
Ala Ala Thr Ser Gly Asp Leu Gly Thr Ser Thr Glu Lys Gln Ala Phe
    535                 540                 545

gcc cgt tat gcg ctg atc gct gaa atc att tcc aaa ctc gcc tcc cac 1795
Ala Arg Tyr Ala Leu Ile Ala Glu Ile Ile Ser Lys Leu Ala Ser His
550                 555                 560                 565

gac tcc gcc gac gcc cgc tgg cgc aac acc gtt cta gac acc cgc cgc 1843
Asp Ser Ala Asp Ala Arg Trp Arg Asn Thr Val Leu Asp Thr Arg Arg
                570                 575                 580

cac gtt cgc ttc atc ggc ctc gag cgc gat tcc gac ggc gca acc gtc 1891
His Val Arg Phe Ile Gly Leu Glu Arg Asp Ser Asp Gly Ala Thr Val
            585                 590                 595

aac acc tac gtc gac tcc gca tca ctt tca ggc gga caa gcc cag aag 1939
Asn Thr Tyr Val Asp Ser Ala Ser Leu Ser Gly Gly Gln Ala Gln Lys
        600                 605                 610

ctg gtg ttt ttc tgc ctc gcc gct gcc ttg cgc tac cag cta gcc gaa 1987
Leu Val Phe Phe Cys Leu Ala Ala Ala Leu Arg Tyr Gln Leu Ala Glu
    615                 620                 625

ccc ggc gcc cat tat ccc acc tac gcc acc gtc att ctg gac gaa gcc 2035
Pro Gly Ala His Tyr Pro Thr Tyr Ala Thr Val Ile Leu Asp Glu Ala
630                 635                 640                 645

ttc gac cgc gcc gac ccc gcc ttc acc cgc caa acc atg aac gtc ttc 2083
Phe Asp Arg Ala Asp Pro Ala Phe Thr Arg Gln Thr Met Asn Val Phe
                650                 655                 660

cac agc ttc ggc ttc cac atg gtg ctc gcg acc ccg ctg aaa ctt atc 2131
His Ser Phe Gly Phe His Met Val Leu Ala Thr Pro Leu Lys Leu Ile
            665                 670                 675

caa acc ctc ggc gat tat gtc ggc tcc acc atc gtg gtc agc tac acc 2179
Gln Thr Leu Gly Asp Tyr Val Gly Ser Thr Ile Val Val Ser Tyr Thr

680                 685                 690

gaa aaa cca aac gcc cag ggc gca att cag ggc aat tcc agt ttc tct    2227
Glu Lys Pro Asn Ala Gln Gly Ala Ile Gln Gly Asn Ser Ser Phe Ser
    695                 700                 705

agg atc gag aaa taacatgcca ttgtttatcg acg                          2262
Arg Ile Glu Lys
710

<210> 312
<211> 713
<212> PRT
<213> Corynebacterium glutamicum

<400> 312
Leu Glu Leu Asn Asn Ala Ala Arg Leu Thr Val Asp Glu Tyr Pro Ala
1               5                   10                  15

Ala Arg Glu Ala Leu Glu Ser Ala Gly Gln Arg Asn Val Glu Asp Arg
            20                  25                  30

Thr Arg Ala Val Asp Glu Phe Lys Ala Ala Asp Gln Glu Leu Ser Ser
        35                  40                  45

Leu Ser Lys Gly Ser Ser Asn Ile Glu Tyr Arg Leu Leu Gln Val Arg
    50                  55                  60

Glu Asn Leu Cys Gln Asp Leu Gly Val Ser Pro Arg Asp Met Pro Phe
65                  70                  75                  80

Ala Gly Glu Leu Ile Asp Pro Asn Asn Ala Glu Trp Glu Pro Val Val
                85                  90                  95

Gln Arg Ile Leu Gly Gly Phe Ala Ala Glu Met Leu Val Pro His Gly
            100                 105                 110

Leu Leu Pro Arg Val Arg Asp Trp Val Asn Ala Lys His Leu Ala Ala
        115                 120                 125

Leu Leu Lys Phe Asn Gly Val Val Thr Thr Gly Glu Tyr Lys Thr Ser
    130                 135                 140

Arg Phe Pro Ala Asp Ser Leu Ile Arg Lys Val Asp Val Val Glu Ser
145                 150                 155                 160

Pro Phe Arg Asp Trp Val Asn Gln Glu Leu Gly Lys Arg Phe Asn Ile
                165                 170                 175

Arg Cys Val Arg Thr Pro Glu Glu Leu Ser Ala Leu Gly Pro Arg Asp
            180                 185                 190

Gln Gly Val Thr Ile Leu Gly Val Arg Lys Phe Ala Gln Gln Thr Gly
        195                 200                 205

Asp Pro Thr Thr Arg Trp Glu Lys Asp Asp Arg Arg Lys Leu Gly Asp
    210                 215                 220

Arg Ser Thr Tyr Arg Leu Gly Ser Thr Asn Asp Ala Lys Val Glu Thr
225                 230                 235                 240

Leu Arg Glu Thr Val Lys Ala Gly Lys Ala Val Val Gln Ala Ala Asp
                245                 250                 255

Asn Arg Ile Ala Ala Asn Arg Ala Glu Leu Arg Glu Leu Glu Arg Gln
            260                 265                 270

Tyr Gln Ala Ser Gln Glu Ile Leu Lys Val Ser Trp Ala Gln Ile Asp
        275                 280                 285

Val Glu Ser Ala Asp Ala Ala Ile Ala Glu Leu Asp Arg Leu Leu Glu
    290                 295                 300

Glu Leu Asn Asn Thr Pro Glu Ala Thr Glu Leu Ser Ala Arg His Glu
305                 310                 315                 320

Ala Ala Lys Gln Thr Leu Ala Arg Val Ser Asp Leu Leu Val Ala Ala
                325                 330                 335

Gln Ser Glu Glu Thr Val Ala Ser Met Asn Leu Lys Arg Ala Glu Thr
            340                 345                 350

Glu Leu Lys Arg Leu Glu Ser Leu Pro Val Ala Glu Val Ser Glu Glu
        355                 360                 365

Ile Ala Arg Glu Val Glu Lys Leu Phe Leu Ala Asn Thr Arg Arg Val
    370                 375                 380

His Ala Ala Asn Val Asp Glu Gln Thr Ile Ala Leu Arg Glu Asp Leu
385                 390                 395                 400

Asp Lys Gln Ile Asp Ala Asn Glu Ala Glu Leu Arg Arg Cys Glu Asn
                405                 410                 415

Gln Ile Val Gly Ile Leu Arg Ser Tyr Ile Glu Thr Trp Pro Ala Asn
            420                 425                 430

Arg Ala Asp Leu Gln Ala Glu Pro Glu Phe Val Gly Glu Ala Ile Asn
        435                 440                 445

Arg Leu Gly Glu Leu Arg Ser Asp Arg Leu Ala Glu Phe Thr Ala Lys
    450                 455                 460

Phe Leu Gly Leu Met Asn Glu Met Ser Thr Arg Asn Leu Gly Gln Ile
465                 470                 475                 480

Ser Arg Arg Leu Arg Asp Ala Arg Arg Glu Ile Glu Glu Arg Ile Glu
                485                 490                 495

Pro Ile Asn Ala Ser Leu Ala Gln Ser Glu Phe Asn Glu Gly Arg Phe
            500                 505                 510

Leu His Ile Asp Ile Arg Asp Gln Ser Gly Pro Ile Val Arg Glu Phe
        515                 520                 525

Gln Gln Lys Leu Asp Ala Ala Thr Ser Gly Asp Leu Gly Thr Ser Thr
    530                 535                 540

Glu Lys Gln Ala Phe Ala Arg Tyr Ala Leu Ile Ala Glu Ile Ile Ser
545                 550                 555                 560

Lys Leu Ala Ser His Asp Ser Ala Asp Ala Arg Trp Arg Asn Thr Val
                565                 570                 575

Leu Asp Thr Arg Arg His Val Arg Phe Ile Gly Leu Glu Arg Asp Ser
            580                 585                 590

Asp Gly Ala Thr Val Asn Thr Tyr Val Asp Ser Ala Ser Leu Ser Gly
        595                 600                 605

Gly Gln Ala Gln Lys Leu Val Phe Phe Cys Leu Ala Ala Ala Leu Arg
    610                 615                 620

Tyr Gln Leu Ala Glu Pro Gly Ala His Tyr Pro Thr Tyr Ala Thr Val
625                 630                 635                 640

Ile Leu Asp Glu Ala Phe Asp Arg Ala Asp Pro Ala Phe Thr Arg Gln
                645                 650                 655

Thr Met Asn Val Phe His Ser Phe Gly Phe His Met Val Leu Ala Thr
            660                 665                 670

Pro Leu Lys Leu Ile Gln Thr Leu Gly Asp Tyr Val Gly Ser Thr Ile
        675                 680                 685

Val Val Ser Tyr Thr Glu Lys Pro Asn Ala Gln Gly Ala Ile Gln Gly
    690                 695                 700

Asn Ser Ser Phe Ser Arg Ile Glu Lys
705                 710


<210> 313
<211> 2147
<212> DNA
<213> Corynebacterium glutamicum

<220>
<221> CDS
<222> (1)..(2124)
<223> FRXA02547

<400> 313
gct gcg cgg ctg acc gtg gat gag tat ccg gcg gcg agg gaa gcg ctt      48
Ala Ala Arg Leu Thr Val Asp Glu Tyr Pro Ala Ala Arg Glu Ala Leu
  1               5                  10                  15

gaa tct gca ggt cag agg aat gta gag gac cga acc cgt gcg gtt gat      96
Glu Ser Ala Gly Gln Arg Asn Val Glu Asp Arg Thr Arg Ala Val Asp
             20                  25                  30

gag ttc aaa gcg gcg gat caa gag ctg tct tct ttg agt aaa ggc agc     144
Glu Phe Lys Ala Ala Asp Gln Glu Leu Ser Ser Leu Ser Lys Gly Ser
         35                  40                  45

agt aat att gag tac cgt ttg ctg cag gtg cgg gaa aat ttg tgt cag     192
Ser Asn Ile Glu Tyr Arg Leu Leu Gln Val Arg Glu Asn Leu Cys Gln
     50                  55                  60

gat ttg ggc gtg agc ccg cgg gat atg ccc ttt gcc ggt gag ctg att     240
Asp Leu Gly Val Ser Pro Arg Asp Met Pro Phe Ala Gly Glu Leu Ile
 65                  70                  75                  80

gat ccg aat aat gcg gaa tgg gaa ccc gtt gtg cag cgc att ttg ggt 288
Asp Pro Asn Asn Ala Glu Trp Glu Pro Val Val Gln Arg Ile Leu Gly
                85                  90                  95

ggt ttt gct gcg gaa atg ttg gtt cct cat ggg ttg ttg cca cgg gtt 336
Gly Phe Ala Ala Glu Met Leu Val Pro His Gly Leu Leu Pro Arg Val
            100                 105                 110

cgg gat tgg gta aat gcc aaa cat ttg gca gcg ctg ctg aaa ttc aac 384
Arg Asp Trp Val Asn Ala Lys His Leu Ala Ala Leu Leu Lys Phe Asn
        115                 120                 125

ggc gtg gtg aca acg ggg gag tac aaa acc tcg cgt ttt ccg gcg gat 432
Gly Val Val Thr Thr Gly Glu Tyr Lys Thr Ser Arg Phe Pro Ala Asp
    130                 135                 140

tcc ctg atc cga aaa gtt gat gtt gtg gag tcg ccg ttt cgc gat tgg 480
Ser Leu Ile Arg Lys Val Asp Val Val Glu Ser Pro Phe Arg Asp Trp
145                 150                 155                 160

gta aat caa gaa tta ggc aag cgt ttt aat att cgg tgc gtg cgc act 528
Val Asn Gln Glu Leu Gly Lys Arg Phe Asn Ile Arg Cys Val Arg Thr
                165                 170                 175

cct gag gaa ttg tcg gcg ctg ggg cca cgc gat cag ggc gtg acc att 576
Pro Glu Glu Leu Ser Ala Leu Gly Pro Arg Asp Gln Gly Val Thr Ile
            180                 185                 190

ttg ggt gtg cga aaa ttt gcg cag cag aca ggc gat ccg acg acg cgt 624
Leu Gly Val Arg Lys Phe Ala Gln Gln Thr Gly Asp Pro Thr Thr Arg
        195                 200                 205

tgg gaa aaa gat gat cgc cga aag ctg ggg gat cgt tcc aca tac cgt 672
Trp Glu Lys Asp Asp Arg Arg Lys Leu Gly Asp Arg Ser Thr Tyr Arg
    210                 215                 220

ttg ggt tcc acc aat gat gcc aag gtg gaa acg ctt cgg gaa acc gtg 720
Leu Gly Ser Thr Asn Asp Ala Lys Val Glu Thr Leu Arg Glu Thr Val
225                 230                 235                 240

aaa gct ggc aaa gca gtt gtg cag gca gct gat aat cgc att gct gca 768
Lys Ala Gly Lys Ala Val Val Gln Ala Ala Asp Asn Arg Ile Ala Ala
                245                 250                 255

aac cgc gct gag ctg cgg gaa ctt gaa cgg cag tat caa gct tcg caa 816
Asn Arg Ala Glu Leu Arg Glu Leu Glu Arg Gln Tyr Gln Ala Ser Gln
            260                 265                 270

gaa att ttg aaa gtg tcg tgg gct cag att gat gtg gaa tca gcc gac 864
Glu Ile Leu Lys Val Ser Trp Ala Gln Ile Asp Val Glu Ser Ala Asp
        275                 280                 285

gcg gcg att gct gag ctg gac cga ttg ctg gaa gag ctg aac aac act 912
Ala Ala Ile Ala Glu Leu Asp Arg Leu Leu Glu Glu Leu Asn Asn Thr
    290                 295                 300

cca gag gcc acc gag ctt tcc gcg cgg cat gag gcg gcg aag cag acg 960
Pro Glu Ala Thr Glu Leu Ser Ala Arg His Glu Ala Ala Lys Gln Thr
305                 310                 315                 320

ctc gcg agg gtt tct gac ttg ctt gtc gca gct cag agt gag gaa acc 1008
Leu Ala Arg Val Ser Asp Leu Leu Val Ala Ala Gln Ser Glu Glu Thr
                325                 330                 335

gtg gcg tcg atg aac ctg aaa cgc gcc gaa act gaa ttg aaa cgg ctc 1056
Val Ala Ser Met Asn Leu Lys Arg Ala Glu Thr Glu Leu Lys Arg Leu
            340                 345                 350

gaa agc ctg ccg gtt gcg gag gtt tct gaa gaa atc gcg cgg gaa gtg 1104
Glu Ser Leu Pro Val Ala Glu Val Ser Glu Glu Ile Ala Arg Glu Val
        355                 360                 365

gag aaa cta ttt ctt gcc aac acc cgc cgg gtt cac gcc gcc aac gtg 1152
Glu Lys Leu Phe Leu Ala Asn Thr Arg Arg Val His Ala Ala Asn Val
    370                 375                 380

gat gag cag acc att gcg ctg cgc gag gat ctg gac aaa caa atc gat 1200
Asp Glu Gln Thr Ile Ala Leu Arg Glu Asp Leu Asp Lys Gln Ile Asp
385                 390                 395                 400

gcc aat gag gca gaa ctt cga cgt tgt gaa aac caa att gtt ggc att 1248
Ala Asn Glu Ala Glu Leu Arg Arg Cys Glu Asn Gln Ile Val Gly Ile
                405                 410                 415

ttg cgc agc tat att gaa acg tgg cct gcg aac cgc gct gac tta caa 1296
Leu Arg Ser Tyr Ile Glu Thr Trp Pro Ala Asn Arg Ala Asp Leu Gln
            420                 425                 430

gcc gaa cct gag ttt gtt ggt gag gcc atc aac cgc ctc ggc gag ctt 1344
Ala Glu Pro Glu Phe Val Gly Glu Ala Ile Asn Arg Leu Gly Glu Leu
        435                 440                 445

cgc agc gat cgt ttg gca gaa ttc acg gcc aaa ttc cta ggg ctc atg 1392
Arg Ser Asp Arg Leu Ala Glu Phe Thr Ala Lys Phe Leu Gly Leu Met
    450                 455                 460

aac gag atg tcc acc cga aac ctc ggc caa atc tcg cgg cgt cta cgt 1440
Asn Glu Met Ser Thr Arg Asn Leu Gly Gln Ile Ser Arg Arg Leu Arg
465                 470                 475                 480

gat gcg cgc cgg gaa atc gag gag cgc atc gag ccg atc aac gcc tcc 1488
Asp Ala Arg Arg Glu Ile Glu Glu Arg Ile Glu Pro Ile Asn Ala Ser
                485                 490                 495

ttg gcg cag tcg gaa ttc aac gaa ggt cgc ttc ctg cac atc gac atc 1536
Leu Ala Gln Ser Glu Phe Asn Glu Gly Arg Phe Leu His Ile Asp Ile
            500                 505                 510

cgt gat caa agt ggt ccg att gtg agg gaa ttc cag cag aaa ctt gat 1584
Arg Asp Gln Ser Gly Pro Ile Val Arg Glu Phe Gln Gln Lys Leu Asp
        515                 520                 525

gcc gct acc agc ggt gac ctg gga acc agt acc gag aaa caa gcc ttc 1632
Ala Ala Thr Ser Gly Asp Leu Gly Thr Ser Thr Glu Lys Gln Ala Phe
    530                 535                 540

gcc cgt tat gcg ctg atc gct gaa atc att tcc aaa ctc gcc tcc cac 1680
Ala Arg Tyr Ala Leu Ile Ala Glu Ile Ile Ser Lys Leu Ala Ser His
545                 550                 555                 560

gac tcc gcc gac gcc cgc tgg cgc aac acc gtt cta gac acc cgc cgc 1728

Asp Ser Ala Asp Ala Arg Trp Arg Asn Thr Val Leu Asp Thr Arg Arg
                565                 570                 575

cac gtt cgc ttc atc ggc ctc gag cgc gat tcc gac ggc gca acc gtc     1776
His Val Arg Phe Ile Gly Leu Glu Arg Asp Ser Asp Gly Ala Thr Val
            580                 585                 590

aac acc tac gtc gac tcc gca tca ctt tca ggc gga caa gcc cag aag     1824
Asn Thr Tyr Val Asp Ser Ala Ser Leu Ser Gly Gly Gln Ala Gln Lys
        595                 600                 605

ctg gtg ttt ttc tgc ctc gcc gct gcc ttg cgc tac cag cta gcc gaa     1872
Leu Val Phe Phe Cys Leu Ala Ala Ala Leu Arg Tyr Gln Leu Ala Glu
    610                 615                 620

ccc ggc gcc cat tat ccc acc tac gcc acc gtc att ctg gac gaa gcc     1920
Pro Gly Ala His Tyr Pro Thr Tyr Ala Thr Val Ile Leu Asp Glu Ala
625                 630                 635                 640

ttc gac cgc gcc gac ccc gcc ttc acc cgc caa acc atg aac gtc ttc     1968
Phe Asp Arg Ala Asp Pro Ala Phe Thr Arg Gln Thr Met Asn Val Phe
                645                 650                 655

cac agc ttc ggc ttc cac atg gtg ctc gcg acc ccg ctg aaa ctt atc     2016
His Ser Phe Gly Phe His Met Val Leu Ala Thr Pro Leu Lys Leu Ile
            660                 665                 670

caa acc ctc ggc gat tat gtc ggc tcc acc atc gtg gtc agc tac acc     2064
Gln Thr Leu Gly Asp Tyr Val Gly Ser Thr Ile Val Val Ser Tyr Thr
        675                 680                 685

gaa aaa cca aac gcc cag ggc gca att cag ggc aat tcc agt ttc tct     2112
Glu Lys Pro Asn Ala Gln Gly Ala Ile Gln Gly Asn Ser Ser Phe Ser
    690                 695                 700

agg atc gag aaa taacatgcca ttgtttatcg acg                           2147
Arg Ile Glu Lys
705


<210> 314
<211> 708
<212> PRT
<213> Corynebacterium glutamicum

<400> 314
Ala Ala Arg Leu Thr Val Asp Glu Tyr Pro Ala Ala Arg Glu Ala Leu
  1               5                  10                  15

Glu Ser Ala Gly Gln Arg Asn Val Glu Asp Arg Thr Arg Ala Val Asp
             20                  25                  30

Glu Phe Lys Ala Ala Asp Gln Glu Leu Ser Ser Leu Ser Lys Gly Ser
         35                  40                  45

Ser Asn Ile Glu Tyr Arg Leu Leu Gln Val Arg Glu Asn Leu Cys Gln
     50                  55                  60

Asp Leu Gly Val Ser Pro Arg Asp Met Pro Phe Ala Gly Glu Leu Ile
 65                  70                  75                  80

Asp Pro Asn Asn Ala Glu Trp Glu Pro Val Val Gln Arg Ile Leu Gly
                85                  90                  95

Gly Phe Ala Ala Glu Met Leu Val Pro His Gly Leu Leu Pro Arg Val
            100                 105                 110

Arg Asp Trp Val Asn Ala Lys His Leu Ala Ala Leu Leu Lys Phe Asn
        115                 120                 125

Gly Val Val Thr Thr Gly Glu Tyr Lys Thr Ser Arg Phe Pro Ala Asp
    130                 135                 140

Ser Leu Ile Arg Lys Val Asp Val Val Glu Ser Pro Phe Arg Asp Trp
145                 150                 155                 160

Val Asn Gln Glu Leu Gly Lys Arg Phe Asn Ile Arg Cys Val Arg Thr
                165                 170                 175

Pro Glu Glu Leu Ser Ala Leu Gly Pro Arg Asp Gln Gly Val Thr Ile
            180                 185                 190

Leu Gly Val Arg Lys Phe Ala Gln Gln Thr Gly Asp Pro Thr Thr Arg
        195                 200                 205

Trp Glu Lys Asp Asp Arg Arg Lys Leu Gly Asp Arg Ser Thr Tyr Arg
    210                 215                 220

Leu Gly Ser Thr Asn Asp Ala Lys Val Glu Thr Leu Arg Glu Thr Val
225                 230                 235                 240

Lys Ala Gly Lys Ala Val Val Gln Ala Ala Asp Asn Arg Ile Ala Ala
                245                 250                 255

Asn Arg Ala Glu Leu Arg Glu Leu Glu Arg Gln Tyr Gln Ala Ser Gln
            260                 265                 270

Glu Ile Leu Lys Val Ser Trp Ala Gln Ile Asp Val Glu Ser Ala Asp
        275                 280                 285

Ala Ala Ile Ala Glu Leu Asp Arg Leu Leu Glu Glu Leu Asn Asn Thr
    290                 295                 300

Pro Glu Ala Thr Glu Leu Ser Ala Arg His Glu Ala Ala Lys Gln Thr
305                 310                 315                 320

Leu Ala Arg Val Ser Asp Leu Leu Val Ala Ala Gln Ser Glu Glu Thr
                325                 330                 335

Val Ala Ser Met Asn Leu Lys Arg Ala Glu Thr Glu Leu Lys Arg Leu
            340                 345                 350

Glu Ser Leu Pro Val Ala Glu Val Ser Glu Glu Ile Ala Arg Glu Val
        355                 360                 365

Glu Lys Leu Phe Leu Ala Asn Thr Arg Arg Val His Ala Ala Asn Val
    370                 375                 380

Asp Glu Gln Thr Ile Ala Leu Arg Glu Asp Leu Asp Lys Gln Ile Asp
385                 390                 395                 400

Ala Asn Glu Ala Glu Leu Arg Arg Cys Glu Asn Gln Ile Val Gly Ile

405                 410                 415

Leu Arg Ser Tyr Ile Glu Thr Trp Pro Ala Asn Arg Ala Asp Leu Gln
            420                 425                 430

Ala Glu Pro Glu Phe Val Gly Glu Ala Ile Asn Arg Leu Gly Glu Leu
        435                 440                 445

Arg Ser Asp Arg Leu Ala Glu Phe Thr Ala Lys Phe Leu Gly Leu Met
    450                 455                 460

Asn Glu Met Ser Thr Arg Asn Leu Gly Gln Ile Ser Arg Arg Leu Arg
465                 470                 475                 480

Asp Ala Arg Arg Glu Ile Glu Glu Arg Ile Glu Pro Ile Asn Ala Ser
                485                 490                 495

Leu Ala Gln Ser Glu Phe Asn Glu Gly Arg Phe Leu His Ile Asp Ile
            500                 505                 510

Arg Asp Gln Ser Gly Pro Ile Val Arg Glu Phe Gln Gln Lys Leu Asp
        515                 520                 525

Ala Ala Thr Ser Gly Asp Leu Gly Thr Ser Thr Glu Lys Gln Ala Phe
    530                 535                 540

Ala Arg Tyr Ala Leu Ile Ala Glu Ile Ile Ser Lys Leu Ala Ser His
545                 550                 555                 560

Asp Ser Ala Asp Ala Arg Trp Arg Asn Thr Val Leu Asp Thr Arg Arg
                565                 570                 575

His Val Arg Phe Ile Gly Leu Glu Arg Asp Ser Asp Gly Ala Thr Val
            580                 585                 590

Asn Thr Tyr Val Asp Ser Ala Ser Leu Ser Gly Gly Gln Ala Gln Lys
        595                 600                 605

Leu Val Phe Phe Cys Leu Ala Ala Ala Leu Arg Tyr Gln Leu Ala Glu
    610                 615                 620

Pro Gly Ala His Tyr Pro Thr Tyr Ala Thr Val Ile Leu Asp Glu Ala
625                 630                 635                 640

Phe Asp Arg Ala Asp Pro Ala Phe Thr Arg Gln Thr Met Asn Val Phe
                645                 650                 655

His Ser Phe Gly Phe His Met Val Leu Ala Thr Pro Leu Lys Leu Ile
            660                 665                 670

Gln Thr Leu Gly Asp Tyr Val Gly Ser Thr Ile Val Val Ser Tyr Thr
        675                 680                 685

Glu Lys Pro Asn Ala Gln Gly Ala Ile Gln Gly Asn Ser Ser Phe Ser
    690                 695                 700

Arg Ile Glu Lys
705

<210> 315

<211> 1152
<212> DNA
<213> Corynebacterium glutamicum

<220>
<221> CDS
<222> (101)..(1129)
<223> RXN02571

<400> 315
tggacaggcc ggggccgcgt acggtgttgg ttgaggtggt ggaggggcgc gtcgaaaagc 60

attgtcgctg gttgttgccg cttttggcag tcgggatggc gtg gtg gct cta act 115
Val Val Ala Leu Thr
1               5

caa atc gtc gga ccg tcc ggc tcc ggg ctc acg cgg gaa ttg gaa aaa 163
Gln Ile Val Gly Pro Ser Gly Ser Gly Leu Thr Arg Glu Leu Glu Lys
                10                  15                  20

cgc tac cgg gaa acg ccc gga gcg gtg atg ctg acc gcc gac ccg cgc 211
Arg Tyr Arg Glu Thr Pro Gly Ala Val Met Leu Thr Ala Asp Pro Arg
            25                  30                  35

gcg cat atc acc tac ctg cgc gcg aca gtc gcc gag gag ctg gcc ttt 259
Ala His Ile Thr Tyr Leu Arg Ala Thr Val Ala Glu Glu Leu Ala Phe
        40                  45                  50

ggg ctg gaa caa cgc ggc atc gta ccc gcg cag atg tgg gag cgc gtc 307
Gly Leu Glu Gln Arg Gly Ile Val Pro Ala Gln Met Trp Glu Arg Val
    55                  60                  65

cga aac atc ggg ctc ggc ctc gag aat ctg cta gac cgc gca ccc gcg 355
Arg Asn Ile Gly Leu Gly Leu Glu Asn Leu Leu Asp Arg Ala Pro Ala
70                  75                  80                  85

caa ctt tcc ggc ggg caa aca cgg cgg ctg gcg atc ggc acc gtc gcc 403
Gln Leu Ser Gly Gly Gln Thr Arg Arg Leu Ala Ile Gly Thr Val Ala
                90                  95                  100

atc tta gag gcg cca acg atg ctt ctc gac gac ccc ctc tcc ggt ctt 451
Ile Leu Glu Ala Pro Thr Met Leu Leu Asp Asp Pro Leu Ser Gly Leu
            105                 110                 115

gat acc tcc tcg cga gcc caa ctc atc aca atg ttg gaa tca tat gag 499
Asp Thr Ser Ser Arg Ala Gln Leu Ile Thr Met Leu Glu Ser Tyr Glu
        120                 125                 130

ggc gat gtc atc gtc gct gcg cac aag cgg tgg ctc gac gcg ccg act 547
Gly Asp Val Ile Val Ala Ala His Lys Arg Trp Leu Asp Ala Pro Thr
    135                 140                 145

gtg tac tta ggg gat ttg gag gag ctg tcc ctg cct gcg cgg gtg gaa 595
Val Tyr Leu Gly Asp Leu Glu Glu Leu Ser Leu Pro Ala Arg Val Glu
150                 155                 160                 165

ttt tcc ggt cca tcg cga acg ttt tca gcg att aca gga acc cgc gga 643
Phe Ser Gly Pro Ser Arg Thr Phe Ser Ala Ile Thr Gly Thr Arg Gly
                170                 175                 180

caa caa cgc cga cgc tgg tgg caa ttc aac gaa tcc caa cca cag ttt 691

Gln Gln Arg Arg Arg Trp Trp Gln Phe Asn Glu Ser Gln Pro Gln Phe
            185                 190                 195

cag atc ggc ccc ctg gat att act gtt tct gca ggt caa gtg ctg tgg 739
Gln Ile Gly Pro Leu Asp Ile Thr Val Ser Ala Gly Gln Val Leu Trp
        200                 205                 210

ttg cag ggt ccc aat ggt tca ggg aag tcc aca ctc ctg cgt ggt ctt 787
Leu Gln Gly Pro Asn Gly Ser Gly Lys Ser Thr Leu Leu Arg Gly Leu
    215                 220                 225

gcc aat gaa ccc ggc act gaa ttg atg ctg caa aac cct agc gat caa 835
Ala Asn Glu Pro Gly Thr Glu Leu Met Leu Gln Asn Pro Ser Asp Gln
230                 235                 240                 245

gtc att gac tcc act gtt gct aat tgg gtg cca ggc agt aac agt gaa 883
Val Ile Asp Ser Thr Val Ala Asn Trp Val Pro Gly Ser Asn Ser Glu
                250                 255                 260

gaa cat ccg ctg gat tta tcg caa cgc gaa ctc cgc ctt gcc caa tgc 931
Glu His Pro Leu Asp Leu Ser Gln Arg Glu Leu Arg Leu Ala Gln Cys
            265                 270                 275

gac gca gcc ctg ggt aat aac ccg gaa gtt ttg ctt gct gat gaa ccc 979
Asp Ala Ala Leu Gly Asn Asn Pro Glu Val Leu Leu Ala Asp Glu Pro
        280                 285                 290

gac gtc ggc ctt gat gtc ggc ggt cga aac gcc atc cac cag cgc ttt 1027
Asp Val Gly Leu Asp Val Gly Gly Arg Asn Ala Ile His Gln Arg Phe
    295                 300                 305

gcg gat ttc tta ggg aat ggg gga gcg ctg atc ctg acc tgc cat gat 1075
Ala Asp Phe Leu Gly Asn Gly Gly Ala Leu Ile Leu Thr Cys His Asp
310                 315                 320                 325

gaa acc ttc gtg gca gag gta gct gaa tac gcg ata gtg aag gaa atg 1123
Glu Thr Phe Val Ala Glu Val Ala Glu Tyr Ala Ile Val Lys Glu Met
                330                 335                 340

ggg ctc taggtttctt tggaccaaac cac 1152
Gly Leu

<210> 316
<211> 343
<212> PRT
<213> Corynebacterium glutamicum

<400> 316
Val Val Ala Leu Thr Gln Ile Val Gly Pro Ser Gly Ser Gly Leu Thr
1               5                   10                  15

Arg Glu Leu Glu Lys Arg Tyr Arg Glu Thr Pro Gly Ala Val Met Leu
            20                  25                  30

Thr Ala Asp Pro Arg Ala His Ile Thr Tyr Leu Arg Ala Thr Val Ala
        35                  40                  45

Glu Glu Leu Ala Phe Gly Leu Glu Gln Arg Gly Ile Val Pro Ala Gln
    50                  55                  60

Met Trp Glu Arg Val Arg Asn Ile Gly Leu Gly Leu Glu Asn Leu Leu
65                  70                  75                  80

Asp Arg Ala Pro Ala Gln Leu Ser Gly Gly Gln Thr Arg Arg Leu Ala
                85                  90                  95

Ile Gly Thr Val Ala Ile Leu Glu Ala Pro Thr Met Leu Leu Asp Asp
            100                 105                 110

Pro Leu Ser Gly Leu Asp Thr Ser Ser Arg Ala Gln Leu Ile Thr Met
        115                 120                 125

Leu Glu Ser Tyr Glu Gly Asp Val Ile Val Ala Ala His Lys Arg Trp
    130                 135                 140

Leu Asp Ala Pro Thr Val Tyr Leu Gly Asp Leu Glu Glu Leu Ser Leu
145                 150                 155                 160

Pro Ala Arg Val Glu Phe Ser Gly Pro Ser Arg Thr Phe Ser Ala Ile
                165                 170                 175

Thr Gly Thr Arg Gly Gln Gln Arg Arg Arg Trp Trp Gln Phe Asn Glu
            180                 185                 190

Ser Gln Pro Gln Phe Gln Ile Gly Pro Leu Asp Ile Thr Val Ser Ala
        195                 200                 205

Gly Gln Val Leu Trp Leu Gln Gly Pro Asn Gly Ser Gly Lys Ser Thr
    210                 215                 220

Leu Leu Arg Gly Leu Ala Asn Glu Pro Gly Thr Glu Leu Met Leu Gln
225                 230                 235                 240

Asn Pro Ser Asp Gln Val Ile Asp Ser Thr Val Ala Asn Trp Val Pro
                245                 250                 255

Gly Ser Asn Ser Glu Glu His Pro Leu Asp Leu Ser Gln Arg Glu Leu
            260                 265                 270

Arg Leu Ala Gln Cys Asp Ala Ala Leu Gly Asn Asn Pro Glu Val Leu
        275                 280                 285

Leu Ala Asp Glu Pro Asp Val Gly Leu Asp Val Gly Gly Arg Asn Ala
    290                 295                 300

Ile His Gln Arg Phe Ala Asp Phe Leu Gly Asn Gly Gly Ala Leu Ile
305                 310                 315                 320

Leu Thr Cys His Asp Glu Thr Phe Val Ala Glu Val Ala Glu Tyr Ala
                325                 330                 335

Ile Val Lys Glu Met Gly Leu
            340

<210> 317
<211> 1152
<212> DNA
<213> Corynebacterium glutamicum

<220>
<221> CDS
<222> (101)..(1129)
<223> FRXA02571

<400> 317
tggacaggcc ggggccgcgt acggtgttgg ttgaggtggt ggaggggcgc gtcgaaaagc 60

attgtcgctg gttgttgccg cttttggcag tcgggatggc gtg gtg gct cta act 115
Val Val Ala Leu Thr
1               5

caa atc gtc gga ccg tcc ggc tcc ggg ctc acg cgg gaa ttg gaa aaa 163
Gln Ile Val Gly Pro Ser Gly Ser Gly Leu Thr Arg Glu Leu Glu Lys
                10                  15                  20

cgc tac cgg gaa acg ccc gga gcg gtg atg ctg acc gcc gac ccg cgc 211
Arg Tyr Arg Glu Thr Pro Gly Ala Val Met Leu Thr Ala Asp Pro Arg
            25                  30                  35

gcg cat atc acc tac ctg cgc gcg aca gtc gcc gag gag ctg gcc ttt 259
Ala His Ile Thr Tyr Leu Arg Ala Thr Val Ala Glu Glu Leu Ala Phe
        40                  45                  50

ggg ctg gaa caa cgc ggc atc gta ccc gcg cag atg tgg gag cgc gtc 307
Gly Leu Glu Gln Arg Gly Ile Val Pro Ala Gln Met Trp Glu Arg Val
    55                  60                  65

cga aac atc ggg ctc ggc ctc gag aat ctg cta gac cgc gca ccc gcg 355
Arg Asn Ile Gly Leu Gly Leu Glu Asn Leu Leu Asp Arg Ala Pro Ala
70                  75                  80                  85

caa ctt tcc ggc ggg caa aca cgg cgg ctg gcg atc ggc acc gtc gcc 403
Gln Leu Ser Gly Gly Gln Thr Arg Arg Leu Ala Ile Gly Thr Val Ala
                90                  95                  100

atc tta gag gcg cca acg atg ctt ctc gac gac ccc ctc tcc ggt ctt 451
Ile Leu Glu Ala Pro Thr Met Leu Leu Asp Asp Pro Leu Ser Gly Leu
            105                 110                 115

gat acc tcc tcg cga gcc caa ctc atc aca atg ttg gaa tca tat gag 499
Asp Thr Ser Ser Arg Ala Gln Leu Ile Thr Met Leu Glu Ser Tyr Glu
        120                 125                 130

ggc gat gtc atc gtc gct gcg cac aag cgg tgg ctc gac gcg ccg act 547
Gly Asp Val Ile Val Ala Ala His Lys Arg Trp Leu Asp Ala Pro Thr
    135                 140                 145

gtg tac tta ggg gat ttg gag gag ctg tcc ctg cct gcg cgg gtg gaa 595
Val Tyr Leu Gly Asp Leu Glu Glu Leu Ser Leu Pro Ala Arg Val Glu
150                 155                 160                 165

ttt tcc ggt cca tcg cga acg ttt tca gcg att aca gga acc cgc gga 643
Phe Ser Gly Pro Ser Arg Thr Phe Ser Ala Ile Thr Gly Thr Arg Gly
                170                 175                 180

caa caa cgc cga cgc tgg tgg caa ttc aac gaa tcc caa cca cag ttt 691
Gln Gln Arg Arg Arg Trp Trp Gln Phe Asn Glu Ser Gln Pro Gln Phe
            185                 190                 195

cag atc ggc ccc ctg gat att act gtt tct gca ggt caa gtg ctg tgg 739

Gln Ile Gly Pro Leu Asp Ile Thr Val Ser Ala Gly Gln Val Leu Trp
        200                 205                 210

ttg cag ggt ccc aat ggt tca ggg aag tcc aca ctc ctg cgt ggt ctt     787
Leu Gln Gly Pro Asn Gly Ser Gly Lys Ser Thr Leu Leu Arg Gly Leu
    215                 220                 225

gcc aat gaa ccc ggc act gaa ttg atg ctg caa aac cct agc gat caa     835
Ala Asn Glu Pro Gly Thr Glu Leu Met Leu Gln Asn Pro Ser Asp Gln
230                 235                 240                 245

gtc att gac tcc act gtt gct aat tgg gtg cca ggc agt aac agt gaa     883
Val Ile Asp Ser Thr Val Ala Asn Trp Val Pro Gly Ser Asn Ser Glu
                250                 255                 260

gaa cat ccg ctg gat tta tcg caa cgc gaa ctc cgc ctt gcc caa tgc     931
Glu His Pro Leu Asp Leu Ser Gln Arg Glu Leu Arg Leu Ala Gln Cys
            265                 270                 275

gac gca gcc ctg ggt aat aac ccg gaa gtt ttg ctt gct gat gaa ccc     979
Asp Ala Ala Leu Gly Asn Asn Pro Glu Val Leu Leu Ala Asp Glu Pro
        280                 285                 290

gac gtc ggc ctt gat gtc ggc ggt cga aac gcc atc cac cag cgc ttt     1027
Asp Val Gly Leu Asp Val Gly Gly Arg Asn Ala Ile His Gln Arg Phe
    295                 300                 305

gcg gat ttc tta ggg aat ggg gga gcg ctg atc ctg acc tgc cat gat     1075
Ala Asp Phe Leu Gly Asn Gly Gly Ala Leu Ile Leu Thr Cys His Asp
310                 315                 320                 325

gaa acc ttc gtg gca gag gta gct gaa tac gcg ata gtg aag gaa atg     1123
Glu Thr Phe Val Ala Glu Val Ala Glu Tyr Ala Ile Val Lys Glu Met
                330                 335                 340

ggg ctc taggtttctt tggaccaaac cac                                   1152
Gly Leu

<210> 318
<211> 343
<212> PRT
<213> Corynebacterium glutamicum

<400> 318
Val Val Ala Leu Thr Gln Ile Val Gly Pro Ser Gly Ser Gly Leu Thr
1               5                   10                  15

Arg Glu Leu Glu Lys Arg Tyr Arg Glu Thr Pro Gly Ala Val Met Leu
            20                  25                  30

Thr Ala Asp Pro Arg Ala His Ile Thr Tyr Leu Arg Ala Thr Val Ala
        35                  40                  45

Glu Glu Leu Ala Phe Gly Leu Glu Gln Arg Gly Ile Val Pro Ala Gln
    50                  55                  60

Met Trp Glu Arg Val Arg Asn Ile Gly Leu Gly Leu Glu Asn Leu Leu
65                  70                  75                  80

Asp Arg Ala Pro Ala Gln Leu Ser Gly Gly Gln Thr Arg Arg Leu Ala
                85                  90                  95

Ile Gly Thr Val Ala Ile Leu Glu Ala Pro Thr Met Leu Leu Asp Asp
            100                 105                 110

Pro Leu Ser Gly Leu Asp Thr Ser Ser Arg Ala Gln Leu Ile Thr Met
        115                 120                 125

Leu Glu Ser Tyr Glu Gly Asp Val Ile Val Ala Ala His Lys Arg Trp
    130                 135                 140

Leu Asp Ala Pro Thr Val Tyr Leu Gly Asp Leu Glu Glu Leu Ser Leu
145                 150                 155                 160

Pro Ala Arg Val Glu Phe Ser Gly Pro Ser Arg Thr Phe Ser Ala Ile
                165                 170                 175

Thr Gly Thr Arg Gly Gln Gln Arg Arg Arg Trp Trp Gln Phe Asn Glu
            180                 185                 190

Ser Gln Pro Gln Phe Gln Ile Gly Pro Leu Asp Ile Thr Val Ser Ala
        195                 200                 205

Gly Gln Val Leu Trp Leu Gln Gly Pro Asn Gly Ser Gly Lys Ser Thr
    210                 215                 220

Leu Leu Arg Gly Leu Ala Asn Glu Pro Gly Thr Glu Leu Met Leu Gln
225                 230                 235                 240

Asn Pro Ser Asp Gln Val Ile Asp Ser Thr Val Ala Asn Trp Val Pro
                245                 250                 255

Gly Ser Asn Ser Glu Glu His Pro Leu Asp Leu Ser Gln Arg Glu Leu
            260                 265                 270

Arg Leu Ala Gln Cys Asp Ala Ala Leu Gly Asn Asn Pro Glu Val Leu
        275                 280                 285

Leu Ala Asp Glu Pro Asp Val Gly Leu Asp Val Gly Gly Arg Asn Ala
    290                 295                 300

Ile His Gln Arg Phe Ala Asp Phe Leu Gly Asn Gly Gly Ala Leu Ile
305                 310                 315                 320

Leu Thr Cys His Asp Glu Thr Phe Val Ala Glu Val Ala Glu Tyr Ala
                325                 330                 335

Ile Val Lys Glu Met Gly Leu
            340

<210> 319
<211> 1746
<212> DNA
<213> Corynebacterium glutamicum

<220>
<221> CDS
<222> (101)..(1723)
<223> RXN02074

<400> 319
cgggggaagg ccgtgtcgca tgctcgggct agccttggat ctcaagaaga attcgactgg 60

tttaaagtct gggctttaag tgcagaaagg ttgtggattg atg cgc tcc ctg ctt 115
Met Arg Ser Leu Leu
1               5

cgt gat atc cct gcg gtg ggt tgg cta atc acc gcg acg att gtt gtg 163
Arg Asp Ile Pro Ala Val Gly Trp Leu Ile Thr Ala Thr Ile Val Val
                10                  15                  20

cgc acg ctc gtt gtt gcg ctg gtc atc gtt ggg atc ggc ttg ctt atc 211
Arg Thr Leu Val Val Ala Leu Val Ile Val Gly Ile Gly Leu Leu Ile
            25                  30                  35

gac gtc ccc tcg ccc gct cat tca gcc atg ttg tgg tgg gtt ctg gca 259
Asp Val Pro Ser Pro Ala His Ser Ala Met Leu Trp Trp Val Leu Ala
        40                  45                  50

ggt gcc acg gca gca gct gcg ctg ctg tgc gcg gaa gcg gtg ctc ccc 307
Gly Ala Thr Ala Ala Ala Ala Leu Leu Cys Ala Glu Ala Val Leu Pro
    55                  60                  65

caa cgt att cgt gca cga gtt gaa cga tcc tgg cgg cgg cag ttg gct 355
Gln Arg Ile Arg Ala Arg Val Glu Arg Ser Trp Arg Arg Gln Leu Ala
70                  75                  80                  85

gct aaa aat ctg gag ctg aat tcc agt tcg tca gat gat gcc cag ttg 403
Ala Lys Asn Leu Glu Leu Asn Ser Ser Ser Ser Asp Asp Ala Gln Leu
                90                  95                  100

atc aca ctg gca act gaa gcc acc tca aaa gca tcc act tac aca gtg 451
Ile Thr Leu Ala Thr Glu Ala Thr Ser Lys Ala Ser Thr Tyr Thr Val
            105                 110                 115

atg ttt ctg ggg cct tac ttt gca gta ttt ttg gcc cca ctg aca gtt 499
Met Phe Leu Gly Pro Tyr Phe Ala Val Phe Leu Ala Pro Leu Thr Val
        120                 125                 130

att gcc gtt gtc ggc gcg gct att tcc tgg ccg att gcg ggg ata ctg 547
Ile Ala Val Val Gly Ala Ala Ile Ser Trp Pro Ile Ala Gly Ile Leu
    135                 140                 145

tgc ctc ggg ttg tgc gtg ata cct ttc gtt att tct tgg gca cag cgc 595
Cys Leu Gly Leu Cys Val Ile Pro Phe Val Ile Ser Trp Ala Gln Arg
150                 155                 160                 165

atg ttg aaa ggc gct ggc gcg gga tac ggg cga gca tct ggg cag ttg 643
Met Leu Lys Gly Ala Gly Ala Gly Tyr Gly Arg Ala Ser Gly Gln Leu
                170                 175                 180

gca ggc gtg ttt ttg gaa tcg gtg cgc aca cta ggc acc acg atg atg 691
Ala Gly Val Phe Leu Glu Ser Val Arg Thr Leu Gly Thr Thr Met Met
            185                 190                 195

ctg aat gcc gct ggg cag cgc agg cag atc atc aca cag cgc gca gag 739
Leu Asn Ala Ala Gly Gln Arg Arg Gln Ile Ile Thr Gln Arg Ala Glu
        200                 205                 210

aat atg cgc tcc caa gtg atg tca ttg ctg tac cga aat cag ttg atg 787

Asn Met Arg Ser Gln Val Met Ser Leu Leu Tyr Arg Asn Gln Leu Met
    215                 220                 225

att ctg gtg acc gac ggc gtg ttt gga gtt gcc acc aca atg gtt gct     835
Ile Leu Val Thr Asp Gly Val Phe Gly Val Ala Thr Thr Met Val Ala
230                 235                 240                 245

gcg gtg ttt gcc att gga gga ttc ttt tca ggc tct ctt act ctc ggc     883
Ala Val Phe Ala Ile Gly Gly Phe Phe Ser Gly Ser Leu Thr Leu Gly
                250                 255                 260

caa gct gta gca ctc gta ttg ctg gcc agg ctg ctt att gat ccc atc     931
Gln Ala Val Ala Leu Val Leu Leu Ala Arg Leu Leu Ile Asp Pro Ile
            265                 270                 275

aac cgc atg ggt cgc acg ttt tac acc ggc atg gca ggc aaa ccc tcg     979
Asn Arg Met Gly Arg Thr Phe Tyr Thr Gly Met Ala Gly Lys Pro Ser
        280                 285                 290

ctg atc gcc att gaa aaa gcc ctc gcg aca acc ttt act gat cag cca     1027
Leu Ile Ala Ile Glu Lys Ala Leu Ala Thr Thr Phe Thr Asp Gln Pro
    295                 300                 305

act caa cag gga cag cgc cac gat ggg gat ctg gtg gtc aac aac ttg     1075
Thr Gln Gln Gly Gln Arg His Asp Gly Asp Leu Val Val Asn Asn Leu
310                 315                 320                 325

aag atc gcc cgc gat cac agg gac att gtg cac ggt atc tct ttc agc     1123
Lys Ile Ala Arg Asp His Arg Asp Ile Val His Gly Ile Ser Phe Ser
                330                 335                 340

att ccc cgc ggt tcc cac atc gcg gtg gta ggt ccc agt ggc gct ggt     1171
Ile Pro Arg Gly Ser His Ile Ala Val Val Gly Pro Ser Gly Ala Gly
            345                 350                 355

aaa tcc tct gtg gct cta gcg ttg tcc gga ctt tta gag ttt gat ggt     1219
Lys Ser Ser Val Ala Leu Ala Leu Ser Gly Leu Leu Glu Phe Asp Gly
        360                 365                 370

gcg att tcc ctc ggc ggc cac aac tgt gag atg tta gat ctt cgc gcc     1267
Ala Ile Ser Leu Gly Gly His Asn Cys Glu Met Leu Asp Leu Arg Ala
    375                 380                 385

tca gtc agt ttc gtg ccc caa tcc ccc acg ctg ttt agc gga agc atc     1315
Ser Val Ser Phe Val Pro Gln Ser Pro Thr Leu Phe Ser Gly Ser Ile
390                 395                 400                 405

aaa agc aat atc gat ctg gcg cgc acg ggt gtt gat tct gat cac atc     1363
Lys Ser Asn Ile Asp Leu Ala Arg Thr Gly Val Asp Ser Asp His Ile
                410                 415                 420

cac gca gca ctt tta ggc gaa gaa ctc ccc gcg gac ctc aaa gtc ggt     1411
His Ala Ala Leu Leu Gly Glu Glu Leu Pro Ala Asp Leu Lys Val Gly
            425                 430                 435

gaa acc ggc aaa ggt gtc tcc ggc ggc caa gca gca cgc att tcc att     1459
Glu Thr Gly Lys Gly Val Ser Gly Gly Gln Ala Ala Arg Ile Ser Ile
        440                 445                 450

gcc cga ggt tta gta aag aat gct gcc gtg att gtt ctc gac gag gcg     1507
Ala Arg Gly Leu Val Lys Asn Ala Ala Val Ile Val Leu Asp Glu Ala

455                    460                    465

acc gca caa ctc gac tac acc aac gcc cgc cag gtt cga cat ctt gcc    1555
Thr Ala Gln Leu Asp Tyr Thr Asn Ala Arg Gln Val Arg His Leu Ala
470                 475                 480                 485

aaa tcc ctt gag tgc acg ttg gtt gag atc acc cac cgc cca tca gaa    1603
Lys Ser Leu Glu Cys Thr Leu Val Glu Ile Thr His Arg Pro Ser Glu
                490                 495                 500

gcc ctc gat gca gac ttc atc att gtt tta gag gat ggc caa ttg acc    1651
Ala Leu Asp Ala Asp Phe Ile Ile Val Leu Glu Asp Gly Gln Leu Thr
            505                 510                 515

atg atg gat aca ccc agc aac gtt tcc cag cac aat gcg ttt ttc cgc    1699
Met Met Asp Thr Pro Ser Asn Val Ser Gln His Asn Ala Phe Phe Arg
        520                 525                 530

acc gct gtg atg gag gaa gaa caa tgatttcccg acttctccaa ttg          1746
Thr Ala Val Met Glu Glu Glu Gln
    535                 540


<210> 320
<211> 541
<212> PRT
<213> Corynebacterium glutamicum

<400> 320
Met Arg Ser Leu Leu Arg Asp Ile Pro Ala Val Gly Trp Leu Ile Thr
1               5                   10                  15

Ala Thr Ile Val Val Arg Thr Leu Val Val Ala Leu Val Ile Val Gly
            20                  25                  30

Ile Gly Leu Leu Ile Asp Val Pro Ser Pro Ala His Ser Ala Met Leu
        35                  40                  45

Trp Trp Val Leu Ala Gly Ala Thr Ala Ala Ala Ala Leu Leu Cys Ala
    50                  55                  60

Glu Ala Val Leu Pro Gln Arg Ile Arg Ala Arg Val Glu Arg Ser Trp
65                  70                  75                  80

Arg Arg Gln Leu Ala Ala Lys Asn Leu Glu Leu Asn Ser Ser Ser Ser
                85                  90                  95

Asp Asp Ala Gln Leu Ile Thr Leu Ala Thr Glu Ala Thr Ser Lys Ala
            100                 105                 110

Ser Thr Tyr Thr Val Met Phe Leu Gly Pro Tyr Phe Ala Val Phe Leu
        115                 120                 125

Ala Pro Leu Thr Val Ile Ala Val Val Gly Ala Ala Ile Ser Trp Pro
    130                 135                 140

Ile Ala Gly Ile Leu Cys Leu Gly Leu Cys Val Ile Pro Phe Val Ile
145                 150                 155                 160

Ser Trp Ala Gln Arg Met Leu Lys Gly Ala Gly Ala Gly Tyr Gly Arg
                165                 170                 175

Ala Ser Gly Gln Leu Ala Gly Val Phe Leu Glu Ser Val Arg Thr Leu
            180                 185                 190

Gly Thr Thr Met Met Leu Asn Ala Ala Gly Gln Arg Arg Gln Ile Ile
        195                 200                 205

Thr Gln Arg Ala Glu Asn Met Arg Ser Gln Val Met Ser Leu Leu Tyr
    210                 215                 220

Arg Asn Gln Leu Met Ile Leu Val Thr Asp Gly Val Phe Gly Val Ala
225                 230                 235                 240

Thr Thr Met Val Ala Ala Val Phe Ala Ile Gly Gly Phe Phe Ser Gly
                245                 250                 255

Ser Leu Thr Leu Gly Gln Ala Val Ala Leu Val Leu Leu Ala Arg Leu
            260                 265                 270

Leu Ile Asp Pro Ile Asn Arg Met Gly Arg Thr Phe Tyr Thr Gly Met
        275                 280                 285

Ala Gly Lys Pro Ser Leu Ile Ala Ile Glu Lys Ala Leu Ala Thr Thr
    290                 295                 300

Phe Thr Asp Gln Pro Thr Gln Gln Gly Gln Arg His Asp Gly Asp Leu
305                 310                 315                 320

Val Val Asn Asn Leu Lys Ile Ala Arg Asp His Arg Asp Ile Val His
                325                 330                 335

Gly Ile Ser Phe Ser Ile Pro Arg Gly Ser His Ile Ala Val Val Gly
            340                 345                 350

Pro Ser Gly Ala Gly Lys Ser Ser Val Ala Leu Ala Leu Ser Gly Leu
        355                 360                 365

Leu Glu Phe Asp Gly Ala Ile Ser Leu Gly Gly His Asn Cys Glu Met
    370                 375                 380

Leu Asp Leu Arg Ala Ser Val Ser Phe Val Pro Gln Ser Pro Thr Leu
385                 390                 395                 400

Phe Ser Gly Ser Ile Lys Ser Asn Ile Asp Leu Ala Arg Thr Gly Val
                405                 410                 415

Asp Ser Asp His Ile His Ala Ala Leu Leu Gly Glu Glu Leu Pro Ala
            420                 425                 430

Asp Leu Lys Val Gly Glu Thr Gly Lys Gly Val Ser Gly Gly Gln Ala
        435                 440                 445

Ala Arg Ile Ser Ile Ala Arg Gly Leu Val Lys Asn Ala Ala Val Ile
    450                 455                 460

Val Leu Asp Glu Ala Thr Ala Gln Leu Asp Tyr Thr Asn Ala Arg Gln
465                 470                 475                 480

Val Arg His Leu Ala Lys Ser Leu Glu Cys Thr Leu Val Glu Ile Thr
                485                 490                 495

His Arg Pro Ser Glu Ala Leu Asp Ala Asp Phe Ile Ile Val Leu Glu
            500                 505                 510

Asp Gly Gln Leu Thr Met Met Asp Thr Pro Ser Asn Val Ser Gln His
        515                 520                 525

Asn Ala Phe Phe Arg Thr Ala Val Met Glu Glu Glu Gln
    530                 535                 540


<210> 321
<211> 1746
<212> DNA
<213> Corynebacterium glutamicum

<220>
<221> CDS
<222> (101)..(1723)
<223> FRXA02074

<400> 321
cgggggaagg ccgtgtcgca tgctcgggct agccttggat ctcaagaaga attcgactgg 60

tttaaagtct gggctttaag tgcagaaagg ttgtggattg atg cgc tcc ctg ctt   115
                                           Met Arg Ser Leu Leu
                                             1               5

cgt gat atc cct gcg gtg ggt tgg cta atc acc gcg acg att gtt gtg   163
Arg Asp Ile Pro Ala Val Gly Trp Leu Ile Thr Ala Thr Ile Val Val
                 10                  15                  20

cgc acg ctc gtt gtt gcg ctg gtc atc gtt ggg atc ggc ttg ctt atc   211
Arg Thr Leu Val Val Ala Leu Val Ile Val Gly Ile Gly Leu Leu Ile
             25                  30                  35

gac gtc ccc tcg ccc gct cat tca gcc atg ttg tgg tgg gtt ctg gca   259
Asp Val Pro Ser Pro Ala His Ser Ala Met Leu Trp Trp Val Leu Ala
         40                  45                  50

ggt gcc acg gca gca gct gcg ctg ctg tgc gcg gaa gcg gtg ctc ccc   307
Gly Ala Thr Ala Ala Ala Ala Leu Leu Cys Ala Glu Ala Val Leu Pro
     55                  60                  65

caa cgt att cgt gca cga gtt gaa cga tcc tgg cgg cgg cag ttg gct   355
Gln Arg Ile Arg Ala Arg Val Glu Arg Ser Trp Arg Arg Gln Leu Ala
 70                  75                  80                  85

gct aaa aat ctg gag ctg aat tcc agt tcg tca gat gat gcc cag ttg   403
Ala Lys Asn Leu Glu Leu Asn Ser Ser Ser Ser Asp Asp Ala Gln Leu
                 90                  95                 100

atc aca ctg gca act gaa gcc acc tca aaa gca tcc act tac aca gtg   451
Ile Thr Leu Ala Thr Glu Ala Thr Ser Lys Ala Ser Thr Tyr Thr Val
            105                 110                 115

atg ttt ctg ggg cct tac ttt gca gta ttt ttg gcc cca ctg aca gtt   499
Met Phe Leu Gly Pro Tyr Phe Ala Val Phe Leu Ala Pro Leu Thr Val
        120                 125                 130

att gcc gtt gtc ggc gcg gct att tcc tgg ccg att gcg ggg ata ctg   547
Ile Ala Val Val Gly Ala Ala Ile Ser Trp Pro Ile Ala Gly Ile Leu

135                 140                 145

tgc ctc ggg ttg tgc gtg ata cct ttc gtt att tct tgg gca cag cgc    595
Cys Leu Gly Leu Cys Val Ile Pro Phe Val Ile Ser Trp Ala Gln Arg
150                 155                 160                 165

atg ttg aaa ggc gct ggc gcg gga tac ggg cga gca tct ggg cag ttg    643
Met Leu Lys Gly Ala Gly Ala Gly Tyr Gly Arg Ala Ser Gly Gln Leu
                170                 175                 180

gca ggc gtg ttt ttg gaa tcg gtg cgc aca cta ggc acc acg atg atg    691
Ala Gly Val Phe Leu Glu Ser Val Arg Thr Leu Gly Thr Thr Met Met
            185                 190                 195

ctg aat gcc gct ggg cag cgc agg cag atc atc aca cag cgc gca gag    739
Leu Asn Ala Ala Gly Gln Arg Arg Gln Ile Ile Thr Gln Arg Ala Glu
        200                 205                 210

aat atg cgc tcc caa gtg atg tca ttg ctg tac cga aat cag ttg atg    787
Asn Met Arg Ser Gln Val Met Ser Leu Leu Tyr Arg Asn Gln Leu Met
    215                 220                 225

att ctg gtg acc gac ggc gtg ttt gga gtt gcc acc aca atg gtt gct    835
Ile Leu Val Thr Asp Gly Val Phe Gly Val Ala Thr Thr Met Val Ala
230                 235                 240                 245

gcg gtg ttt gcc att gga gga ttc ttt tca ggc tct ctt act ctc ggc    883
Ala Val Phe Ala Ile Gly Gly Phe Phe Ser Gly Ser Leu Thr Leu Gly
                250                 255                 260

caa gct gta gca ctc gta ttg ctg gcc agg ctg ctt att gat ccc atc    931
Gln Ala Val Ala Leu Val Leu Leu Ala Arg Leu Leu Ile Asp Pro Ile
            265                 270                 275

aac cgc atg ggt cgc acg ttt tac acc ggc atg gca ggc aaa ccc tcg    979
Asn Arg Met Gly Arg Thr Phe Tyr Thr Gly Met Ala Gly Lys Pro Ser
        280                 285                 290

ctg atc gcc att gaa aaa gcc ctc gcg aca acc ttt act gat cag cca    1027
Leu Ile Ala Ile Glu Lys Ala Leu Ala Thr Thr Phe Thr Asp Gln Pro
    295                 300                 305

act caa cag gga cag cgc cac gat ggg gat ctg gtg gtc aac aac ttg    1075
Thr Gln Gln Gly Gln Arg His Asp Gly Asp Leu Val Val Asn Asn Leu
310                 315                 320                 325

aag atc gcc cgc gat cac agg gac att gtg cac ggt atc tct ttc agc    1123
Lys Ile Ala Arg Asp His Arg Asp Ile Val His Gly Ile Ser Phe Ser
                330                 335                 340

att ccc cgc ggt tcc cac atc gcg gtg gta ggt ccc agt ggc gct ggt    1171
Ile Pro Arg Gly Ser His Ile Ala Val Val Gly Pro Ser Gly Ala Gly
            345                 350                 355

aaa tcc tct gtg gct cta gcg ttg tcc gga ctt tta gag ttt gat ggt    1219
Lys Ser Ser Val Ala Leu Ala Leu Ser Gly Leu Leu Glu Phe Asp Gly
        360                 365                 370

gcg att tcc ctc ggc ggc cac aac tgt gag atg tta gat ctt cgc gcc    1267
Ala Ile Ser Leu Gly Gly His Asn Cys Glu Met Leu Asp Leu Arg Ala
    375                 380                 385

tca gtc agt ttc gtg ccc caa tcc ccc acg ctg ttt agc gga agc atc     1315
Ser Val Ser Phe Val Pro Gln Ser Pro Thr Leu Phe Ser Gly Ser Ile
390                 395                 400                 405

aaa agc aat atc gat ctg gcg cgc acg ggt gtt gat tct gat cac atc     1363
Lys Ser Asn Ile Asp Leu Ala Arg Thr Gly Val Asp Ser Asp His Ile
                410                 415                 420

cac gca gca ctt tta ggc gaa gaa ctc ccc gcg gac ctc aaa gtc ggt     1411
His Ala Ala Leu Leu Gly Glu Glu Leu Pro Ala Asp Leu Lys Val Gly
            425                 430                 435

gaa acc ggc aaa ggt gtc tcc ggc ggc caa gca gca cgc att tcc att     1459
Glu Thr Gly Lys Gly Val Ser Gly Gly Gln Ala Ala Arg Ile Ser Ile
        440                 445                 450

gcc cga ggt tta gta aag aat gct gcc gtg att gtt ctc gac gag gcg     1507
Ala Arg Gly Leu Val Lys Asn Ala Ala Val Ile Val Leu Asp Glu Ala
    455                 460                 465

acc gca caa ctc gac tac acc aac gcc cgc cag gtt cga cat ctt gcc     1555
Thr Ala Gln Leu Asp Tyr Thr Asn Ala Arg Gln Val Arg His Leu Ala
470                 475                 480                 485

aaa tcc ctt gag tgc acg ttg gtt gag atc acc cac cgc cca tca gaa     1603
Lys Ser Leu Glu Cys Thr Leu Val Glu Ile Thr His Arg Pro Ser Glu
                490                 495                 500

gcc ctc gat gca gac ttc atc att gtt tta gag gat ggc caa ttg acc     1651
Ala Leu Asp Ala Asp Phe Ile Ile Val Leu Glu Asp Gly Gln Leu Thr
            505                 510                 515

atg atg gat aca ccc agc aac gtt tcc cag cac aat gcg ttt ttc cgc     1699
Met Met Asp Thr Pro Ser Asn Val Ser Gln His Asn Ala Phe Phe Arg
        520                 525                 530

acc gct gtg atg gag gaa gaa caa tgatttcccg acttctccaa ttg           1746
Thr Ala Val Met Glu Glu Glu Gln
    535                 540


<210> 322
<211> 541
<212> PRT
<213> Corynebacterium glutamicum

<400> 322
Met Arg Ser Leu Leu Arg Asp Ile Pro Ala Val Gly Trp Leu Ile Thr
1               5                   10                  15

Ala Thr Ile Val Val Arg Thr Leu Val Val Ala Leu Val Ile Val Gly
            20                  25                  30

Ile Gly Leu Leu Ile Asp Val Pro Ser Pro Ala His Ser Ala Met Leu
        35                  40                  45

Trp Trp Val Leu Ala Gly Ala Thr Ala Ala Ala Ala Leu Leu Cys Ala
    50                  55                  60

Glu Ala Val Leu Pro Gln Arg Ile Arg Ala Arg Val Glu Arg Ser Trp

65                  70                  75                  80

Arg Arg Gln Leu Ala Ala Lys Asn Leu Glu Leu Asn Ser Ser Ser Ser
                85                  90                  95

Asp Asp Ala Gln Leu Ile Thr Leu Ala Thr Glu Ala Thr Ser Lys Ala
            100                 105                 110

Ser Thr Tyr Thr Val Met Phe Leu Gly Pro Tyr Phe Ala Val Phe Leu
        115                 120                 125

Ala Pro Leu Thr Val Ile Ala Val Val Gly Ala Ala Ile Ser Trp Pro
    130                 135                 140

Ile Ala Gly Ile Leu Cys Leu Gly Leu Cys Val Ile Pro Phe Val Ile
145                 150                 155                 160

Ser Trp Ala Gln Arg Met Leu Lys Gly Ala Gly Ala Gly Tyr Gly Arg
                165                 170                 175

Ala Ser Gly Gln Leu Ala Gly Val Phe Leu Glu Ser Val Arg Thr Leu
            180                 185                 190

Gly Thr Thr Met Met Leu Asn Ala Ala Gly Gln Arg Arg Gln Ile Ile
        195                 200                 205

Thr Gln Arg Ala Glu Asn Met Arg Ser Gln Val Met Ser Leu Leu Tyr
    210                 215                 220

Arg Asn Gln Leu Met Ile Leu Val Thr Asp Gly Val Phe Gly Val Ala
225                 230                 235                 240

Thr Thr Met Val Ala Ala Val Phe Ala Ile Gly Gly Phe Phe Ser Gly
                245                 250                 255

Ser Leu Thr Leu Gly Gln Ala Val Ala Leu Val Leu Leu Ala Arg Leu
            260                 265                 270

Leu Ile Asp Pro Ile Asn Arg Met Gly Arg Thr Phe Tyr Thr Gly Met
        275                 280                 285

Ala Gly Lys Pro Ser Leu Ile Ala Ile Glu Lys Ala Leu Ala Thr Thr
    290                 295                 300

Phe Thr Asp Gln Pro Thr Gln Gln Gly Gln Arg His Asp Gly Asp Leu
305                 310                 315                 320

Val Val Asn Asn Leu Lys Ile Ala Arg Asp His Arg Asp Ile Val His
                325                 330                 335

Gly Ile Ser Phe Ser Ile Pro Arg Gly Ser His Ile Ala Val Val Gly
            340                 345                 350

Pro Ser Gly Ala Gly Lys Ser Ser Val Ala Leu Ala Leu Ser Gly Leu
        355                 360                 365

Leu Glu Phe Asp Gly Ala Ile Ser Leu Gly Gly His Asn Cys Glu Met
    370                 375                 380

Leu Asp Leu Arg Ala Ser Val Ser Phe Val Pro Gln Ser Pro Thr Leu
385                 390                 395                 400

Phe Ser Gly Ser Ile Lys Ser Asn Ile Asp Leu Ala Arg Thr Gly Val
                405                 410                 415

Asp Ser Asp His Ile His Ala Ala Leu Leu Gly Glu Glu Leu Pro Ala
            420                 425                 430

Asp Leu Lys Val Gly Glu Thr Gly Lys Gly Val Ser Gly Gly Gln Ala
        435                 440                 445

Ala Arg Ile Ser Ile Ala Arg Gly Leu Val Lys Asn Ala Ala Val Ile
    450                 455                 460

Val Leu Asp Glu Ala Thr Ala Gln Leu Asp Tyr Thr Asn Ala Arg Gln
465                 470                 475                 480

Val Arg His Leu Ala Lys Ser Leu Glu Cys Thr Leu Val Glu Ile Thr
                485                 490                 495

His Arg Pro Ser Glu Ala Leu Asp Ala Asp Phe Ile Ile Val Leu Glu
            500                 505                 510

Asp Gly Gln Leu Thr Met Met Asp Thr Pro Ser Asn Val Ser Gln His
        515                 520                 525

Asn Ala Phe Phe Arg Thr Ala Val Met Glu Glu Glu Gln
    530                 535                 540


<210> 323
<211> 1527
<212> DNA
<213> Corynebacterium glutamicum

<220>
<221> CDS
<222> (101)..(1504)
<223> RXA02095

<400> 323
ctctcttggt cctctcccca cccattttta agtactcaag acccttccaa cagaaaggat 60

tactccccca acaggctcaa aaatactgaa aggctcacgc atg aaa act gag caa    115
                                            Met Lys Thr Glu Gln
                                            1               5

tcc caa aaa gca caa tta gcc cct aag aaa gca cct gaa aag cca caa    163
Ser Gln Lys Ala Gln Leu Ala Pro Lys Lys Ala Pro Glu Lys Pro Gln
                10                  15                  20

cgc atc cgc caa ctt att tcc gtg gcg tgg cag cga cct tgg ctc acc    211
Arg Ile Arg Gln Leu Ile Ser Val Ala Trp Gln Arg Pro Trp Leu Thr
            25                  30                  35

tca ttc acc gta atc agc gct tta gct gca acg ttg ttt gaa ctt aca    259
Ser Phe Thr Val Ile Ser Ala Leu Ala Ala Thr Leu Phe Glu Leu Thr
        40                  45                  50

ctt cct ctt ttg acc ggt ggc gcc atc gat atc gcg ctc gga aat acc    307
Leu Pro Leu Leu Thr Gly Gly Ala Ile Asp Ile Ala Leu Gly Asn Thr
    55                  60                  65

gga gat act tta acc act gac ctg ctg gac cgg ttc act ccg agt gga 355
Gly Asp Thr Leu Thr Thr Asp Leu Leu Asp Arg Phe Thr Pro Ser Gly
70                  75                  80                  85

tta agc gtg ttg acc agc gtc att gcc ctt atc gtg ctt ctc gcg ttg 403
Leu Ser Val Leu Thr Ser Val Ile Ala Leu Ile Val Leu Leu Ala Leu
                90                  95                  100

ctt cgc tat gcc agt caa ttt gga cgg cga tac acc gca ggc aag ctc 451
Leu Arg Tyr Ala Ser Gln Phe Gly Arg Arg Tyr Thr Ala Gly Lys Leu
            105                 110                 115

agc atg ggg gta cag cat gat gtc cgg ctt aaa acg atg cgc tca ttg 499
Ser Met Gly Val Gln His Asp Val Arg Leu Lys Thr Met Arg Ser Leu
        120                 125                 130

cag aac ctc gat ggg cca ggt cag gac tct att cgc aca ggc caa gta 547
Gln Asn Leu Asp Gly Pro Gly Gln Asp Ser Ile Arg Thr Gly Gln Val
    135                 140                 145

gtc agt cgg tcc att tcg gat atc aac atg gtg caa agc ctt gtg gcg 595
Val Ser Arg Ser Ile Ser Asp Ile Asn Met Val Gln Ser Leu Val Ala
150                 155                 160                 165

atg ttg ccg atg ttg atc gga aat gtg gtc aag ctt gtg ctc act ttg 643
Met Leu Pro Met Leu Ile Gly Asn Val Val Lys Leu Val Leu Thr Leu
                170                 175                 180

gtg atc atg ctg gct att tcc ccg ccg ctg acc atc atc gct gca gtg 691
Val Ile Met Leu Ala Ile Ser Pro Pro Leu Thr Ile Ile Ala Ala Val
            185                 190                 195

ttg gtg cct ttg ctg ttg tgg gcc gtg gcc tat tcg cga aaa gcg ctt 739
Leu Val Pro Leu Leu Leu Trp Ala Val Ala Tyr Ser Arg Lys Ala Leu
        200                 205                 210

ttt gcg tcc acg tgg tcg gcc cag caa aag gct gcg gat ctg acc act 787
Phe Ala Ser Thr Trp Ser Ala Gln Gln Lys Ala Ala Asp Leu Thr Thr
    215                 220                 225

cat gtg gaa gaa act gtc acg ggt atc cgc gtg gtc aag gca ttt gcg 835
His Val Glu Glu Thr Val Thr Gly Ile Arg Val Val Lys Ala Phe Ala
230                 235                 240                 245

cag gaa gac cgc gag acc gac aaa ttg gat ctc acc gca cgt gag tta 883
Gln Glu Asp Arg Glu Thr Asp Lys Leu Asp Leu Thr Ala Arg Glu Leu
                250                 255                 260

ttt gcc cag cgc atg cgc act gca cgt ctg acg gca aag ttc atc ccc 931
Phe Ala Gln Arg Met Arg Thr Ala Arg Leu Thr Ala Lys Phe Ile Pro
            265                 270                 275

atg gtt gag cag ctt ccg cag ctt gct ttg gtg gtc aac att gtt ggc 979
Met Val Glu Gln Leu Pro Gln Leu Ala Leu Val Val Asn Ile Val Gly
        280                 285                 290

ggt ggc tat ttg gcc atg act ggt cac atc acg gtg ggc acg ttt gtg 1027
Gly Gly Tyr Leu Ala Met Thr Gly His Ile Thr Val Gly Thr Phe Val
    295                 300                 305

gcg ttt tct tcc tat ctc act agc ttg tcg gcg gtg gct agg tcc ctg     1075
Ala Phe Ser Ser Tyr Leu Thr Ser Leu Ser Ala Val Ala Arg Ser Leu
310                 315                 320                 325

tcg ggc atg ctc atg cgc gtg cag ttg gcg ctg tct tct gtg gag cgc     1123
Ser Gly Met Leu Met Arg Val Gln Leu Ala Leu Ser Ser Val Glu Arg
                330                 335                 340

atc ttt gaa gtc att gat ctt cag cct gaa cgc acc gat cct gca cac     1171
Ile Phe Glu Val Ile Asp Leu Gln Pro Glu Arg Thr Asp Pro Ala His
            345                 350                 355

ccc ctg tca ctt ccc gac act ccc ctg ggt ctg tcg ttc aac aac gta     1219
Pro Leu Ser Leu Pro Asp Thr Pro Leu Gly Leu Ser Phe Asn Asn Val
        360                 365                 370

gat ttc cgt ggg att ctc aac ggt ttt gag ctg ggt gtt cag gcc ggt     1267
Asp Phe Arg Gly Ile Leu Asn Gly Phe Glu Leu Gly Val Gln Ala Gly
    375                 380                 385

gaa acc gtt gtg ttg gtg ggc cct cca ggt tca ggc aag acc atg gct     1315
Glu Thr Val Val Leu Val Gly Pro Pro Gly Ser Gly Lys Thr Met Ala
390                 395                 400                 405

gtg cag ctt gct gga aac ttt tat caa cca gac agc ggc cac atc gcc     1363
Val Gln Leu Ala Gly Asn Phe Tyr Gln Pro Asp Ser Gly His Ile Ala
                410                 415                 420

ttt gat agc aac ggc cat cgc act cgc ttc gac gac ctc acc cac agc     1411
Phe Asp Ser Asn Gly His Arg Thr Arg Phe Asp Asp Leu Thr His Ser
            425                 430                 435

gat atc cgc agg aat ctc atc gcg gtt ttt gat gag ccg ttc ttg tac     1459
Asp Ile Arg Arg Asn Leu Ile Ala Val Phe Asp Glu Pro Phe Leu Tyr
        440                 445                 450

tcc tcc tcc ata ccg cga gaa cat ctc gat ggg ttt gga tgt cag         1504
Ser Ser Ser Ile Pro Arg Glu His Leu Asp Gly Phe Gly Cys Gln
    455                 460                 465

tgatgagcag atcgaacacg cag                                           1527


<210> 324
<211> 468
<212> PRT
<213> Corynebacterium glutamicum

<400> 324
Met Lys Thr Glu Gln Ser Gln Lys Ala Gln Leu Ala Pro Lys Lys Ala
1               5                   10                  15

Pro Glu Lys Pro Gln Arg Ile Arg Gln Leu Ile Ser Val Ala Trp Gln
            20                  25                  30

Arg Pro Trp Leu Thr Ser Phe Thr Val Ile Ser Ala Leu Ala Ala Thr
        35                  40                  45

Leu Phe Glu Leu Thr Leu Pro Leu Leu Thr Gly Gly Ala Ile Asp Ile
    50                  55                  60

Ala Leu Gly Asn Thr Gly Asp Thr Leu Thr Thr Asp Leu Leu Asp Arg
65                  70                  75                  80

Phe Thr Pro Ser Gly Leu Ser Val Leu Thr Ser Val Ile Ala Leu Ile
                85                  90                  95

Val Leu Leu Ala Leu Leu Arg Tyr Ala Ser Gln Phe Gly Arg Arg Tyr
            100                 105                 110

Thr Ala Gly Lys Leu Ser Met Gly Val Gln His Asp Val Arg Leu Lys
        115                 120                 125

Thr Met Arg Ser Leu Gln Asn Leu Asp Gly Pro Gly Gln Asp Ser Ile
    130                 135                 140

Arg Thr Gly Gln Val Val Ser Arg Ser Ile Ser Asp Ile Asn Met Val
145                 150                 155                 160

Gln Ser Leu Val Ala Met Leu Pro Met Leu Ile Gly Asn Val Val Lys
                165                 170                 175

Leu Val Leu Thr Leu Val Ile Met Leu Ala Ile Ser Pro Pro Leu Thr
            180                 185                 190

Ile Ile Ala Ala Val Leu Val Pro Leu Leu Leu Trp Ala Val Ala Tyr
        195                 200                 205

Ser Arg Lys Ala Leu Phe Ala Ser Thr Trp Ser Ala Gln Gln Lys Ala
    210                 215                 220

Ala Asp Leu Thr Thr His Val Glu Glu Thr Val Thr Gly Ile Arg Val
225                 230                 235                 240

Val Lys Ala Phe Ala Gln Glu Asp Arg Glu Thr Asp Lys Leu Asp Leu
                245                 250                 255

Thr Ala Arg Glu Leu Phe Ala Gln Arg Met Arg Thr Ala Arg Leu Thr
            260                 265                 270

Ala Lys Phe Ile Pro Met Val Glu Gln Leu Pro Gln Leu Ala Leu Val
        275                 280                 285

Val Asn Ile Val Gly Gly Gly Tyr Leu Ala Met Thr Gly His Ile Thr
    290                 295                 300

Val Gly Thr Phe Val Ala Phe Ser Ser Tyr Leu Thr Ser Leu Ser Ala
305                 310                 315                 320

Val Ala Arg Ser Leu Ser Gly Met Leu Met Arg Val Gln Leu Ala Leu
                325                 330                 335

Ser Ser Val Glu Arg Ile Phe Glu Val Ile Asp Leu Gln Pro Glu Arg
            340                 345                 350

Thr Asp Pro Ala His Pro Leu Ser Leu Pro Asp Thr Pro Leu Gly Leu
        355                 360                 365

Ser Phe Asn Asn Val Asp Phe Arg Gly Ile Leu Asn Gly Phe Glu Leu
    370                 375                 380

Gly Val Gln Ala Gly Glu Thr Val Val Leu Val Gly Pro Pro Gly Ser

385                 390                 395                 400

Gly Lys Thr Met Ala Val Gln Leu Ala Gly Asn Phe Tyr Gln Pro Asp
                405                 410                 415

Ser Gly His Ile Ala Phe Asp Ser Asn Gly His Arg Thr Arg Phe Asp
            420                 425                 430

Asp Leu Thr His Ser Asp Ile Arg Arg Asn Leu Ile Ala Val Phe Asp
        435                 440                 445

Glu Pro Phe Leu Tyr Ser Ser Ser Ile Pro Arg Glu His Leu Asp Gly
    450                 455                 460

Phe Gly Cys Gln
465

<210> 325
<211> 905
<212> DNA
<213> Corynebacterium glutamicum

<220>
<221> CDS
<222> (1)..(882)
<223> RXA02225

<400> 325
caa act gag gag cgc ttt ggc gca gcg gct gat gaa gcc ttg gca atc      48
Gln Thr Glu Glu Arg Phe Gly Ala Ala Ala Asp Glu Ala Leu Ala Ile
  1               5                  10                  15

atg ttg aag gaa gct cgt ctg cag tcg ttg ctg act ttt gtg cgc caa      96
Met Leu Lys Glu Ala Arg Leu Gln Ser Leu Leu Thr Phe Val Arg Gln
             20                  25                  30

ctt gtc cca gcg gtg ttt tct gtg ggt ctt ttg gct tat gcg tca ctg     144
Leu Val Pro Ala Val Phe Ser Val Gly Leu Leu Ala Tyr Ala Ser Leu
         35                  40                  45

ttg gct ttt gac ggt gac ata act ggt ggt gag atg atc tcg gtg acg     192
Leu Ala Phe Asp Gly Asp Ile Thr Gly Gly Glu Met Ile Ser Val Thr
     50                  55                  60

ttg ctg gtg cca cct tcg ttg act gtg ttg ggt gtg tcg ctt ggc atg     240
Leu Leu Val Pro Pro Ser Leu Thr Val Leu Gly Val Ser Leu Gly Met
 65                  70                  75                  80

atg aca gag att tgg gct agg gga cag gct tcg aca aaa agg gtc caa     288
Met Thr Glu Ile Trp Ala Arg Gly Gln Ala Ser Thr Lys Arg Val Gln
                 85                  90                  95

aac tta gtc act gaa ctg gat aag gcg gcc gct gag cca cga cct cag     336
Asn Leu Val Thr Glu Leu Asp Lys Ala Ala Ala Glu Pro Arg Pro Gln
            100                 105                 110

cct gcc acc ttt gaa ttt gaa gag ggg atc acg gtg tgg gat cct tcg     384
Pro Ala Thr Phe Glu Phe Glu Glu Gly Ile Thr Val Trp Asp Pro Ser
        115                 120                 125

aca cct gag gca cgc gat gtg att gat cgg gag ttg gag gcg ctt cag     432
Thr Pro Glu Ala Arg Asp Val Ile Asp Arg Glu Leu Glu Ala Leu Gln
    130                 135                 140

gtt cgc gaa gat gtc att gtg gct cct cac cgc gtc agc gtg ttt gaa     480
Val Arg Glu Asp Val Ile Val Ala Pro His Arg Val Ser Val Phe Glu
145                 150                 155                 160

ggt gtg ctg aag gat aat ttg aat ccg atg ggc act atc gca ccg gag     528
Gly Val Leu Lys Asp Asn Leu Asn Pro Met Gly Thr Ile Ala Pro Glu
                165                 170                 175

atg ctg cgc gct gct ctt cat gct gca agt tgt gag gac atc ttg agc     576
Met Leu Arg Ala Ala Leu His Ala Ala Ser Cys Glu Asp Ile Leu Ser
            180                 185                 190

cga ttg ggt gct gat ctg aac atg ccg ggg gag ttt gag ctt cca gat     624
Arg Leu Gly Ala Asp Leu Asn Met Pro Gly Glu Phe Glu Leu Pro Asp
        195                 200                 205

acc ttg atc ggc gag gcc gga ttg aat ctc tcc ggt ggc caa cgc cag     672
Thr Leu Ile Gly Glu Ala Gly Leu Asn Leu Ser Gly Gly Gln Arg Gln
    210                 215                 220

agg att gct ttg gca cga ttc ttg gct gtt gat cct gag gtg ctc att     720
Arg Ile Ala Leu Ala Arg Phe Leu Ala Val Asp Pro Glu Val Leu Ile
225                 230                 235                 240

ttg gat gaa ccg acc acg ggg ttg gat gcg gtg acc ctg gat gaa gtg     768
Leu Asp Glu Pro Thr Thr Gly Leu Asp Ala Val Thr Leu Asp Glu Val
                245                 250                 255

gca cat cgc gtc gaa aag ctt cgt cga ggc cgg aaa acc gtc gtc att     816
Ala His Arg Val Glu Lys Leu Arg Arg Gly Arg Lys Thr Val Val Ile
            260                 265                 270

acg tcg aac ccg acg tgg cac ggc gtc gca aag cag atg caa tct gat     864
Thr Ser Asn Pro Thr Trp His Gly Val Ala Lys Gln Met Gln Ser Asp
        275                 280                 285

ttt tcg gaa ggg gtg aag tagatggcgc agcatgagcg cgt                   905
Phe Ser Glu Gly Val Lys
    290


<210> 326
<211> 294
<212> PRT
<213> Corynebacterium glutamicum

<400> 326
Gln Thr Glu Glu Arg Phe Gly Ala Ala Ala Asp Glu Ala Leu Ala Ile
1               5                   10                  15

Met Leu Lys Glu Ala Arg Leu Gln Ser Leu Leu Thr Phe Val Arg Gln
            20                  25                  30

Leu Val Pro Ala Val Phe Ser Val Gly Leu Leu Ala Tyr Ala Ser Leu
        35                  40                  45

Leu Ala Phe Asp Gly Asp Ile Thr Gly Gly Glu Met Ile Ser Val Thr

50                  55                  60

Leu Leu Val Pro Pro Ser Leu Thr Val Leu Gly Val Ser Leu Gly Met
65                  70                  75                  80

Met Thr Glu Ile Trp Ala Arg Gly Gln Ala Ser Thr Lys Arg Val Gln
                85                  90                  95

Asn Leu Val Thr Glu Leu Asp Lys Ala Ala Ala Glu Pro Arg Pro Gln
            100                 105                 110

Pro Ala Thr Phe Glu Phe Glu Glu Gly Ile Thr Val Trp Asp Pro Ser
        115                 120                 125

Thr Pro Glu Ala Arg Asp Val Ile Asp Arg Glu Leu Glu Ala Leu Gln
    130                 135                 140

Val Arg Glu Asp Val Ile Val Ala Pro His Arg Val Ser Val Phe Glu
145                 150                 155                 160

Gly Val Leu Lys Asp Asn Leu Asn Pro Met Gly Thr Ile Ala Pro Glu
                165                 170                 175

Met Leu Arg Ala Ala Leu His Ala Ala Ser Cys Glu Asp Ile Leu Ser
            180                 185                 190

Arg Leu Gly Ala Asp Leu Asn Met Pro Gly Glu Phe Glu Leu Pro Asp
        195                 200                 205

Thr Leu Ile Gly Glu Ala Gly Leu Asn Leu Ser Gly Gly Gln Arg Gln
    210                 215                 220

Arg Ile Ala Leu Ala Arg Phe Leu Ala Val Asp Pro Glu Val Leu Ile
225                 230                 235                 240

Leu Asp Glu Pro Thr Thr Gly Leu Asp Ala Val Thr Leu Asp Glu Val
                245                 250                 255

Ala His Arg Val Glu Lys Leu Arg Arg Gly Arg Lys Thr Val Val Ile
            260                 265                 270

Thr Ser Asn Pro Thr Trp His Gly Val Ala Lys Gln Met Gln Ser Asp
        275                 280                 285

Phe Ser Glu Gly Val Lys
    290

<210> 327
<211> 1050
<212> DNA
<213> Corynebacterium glutamicum

<220>
<221> CDS
<222> (101)..(1027)
<223> RXA02253

<400> 327
tgaggacatt tcccaagtga aaggcttcgg cccgaaactt gcggaggctg tctatgaagg 60

tcttcacgcg tcaaaataag tagatcgcta ggatgtaacc atg att caa tcc act 115
Met Ile Gln Ser Thr
1               5

ggg gtc acg cac act gat aag tct gca caa gaa aat cct gtg aag tac 163
Gly Val Thr His Thr Asp Lys Ser Ala Gln Glu Asn Pro Val Lys Tyr
                10                  15                  20

agg gac aat ttc aca cct gtc atc atc acc ggt atg tca ggc gca ggt 211
Arg Asp Asn Phe Thr Pro Val Ile Ile Thr Gly Met Ser Gly Ala Gly
            25                  30                  35

ctg agc aca gca gcc cga gtc ctc gaa gac ttg ggt tgg tat gtg gcg 259
Leu Ser Thr Ala Ala Arg Val Leu Glu Asp Leu Gly Trp Tyr Val Ala
        40                  45                  50

cac aat att cca cca cag atc atc ctg gaa tta att gat atg tgc gct 307
His Asn Ile Pro Pro Gln Ile Ile Leu Glu Leu Ile Asp Met Cys Ala
    55                  60                  65

cgg gaa gat tct ccc gtc gac aaa gtt gca gtc gtg tgc gat gtg cgc 355
Arg Glu Asp Ser Pro Val Asp Lys Val Ala Val Val Cys Asp Val Arg
70                  75                  80                  85

tcc cgt gaa ttc cgc gga agc ctc acc cag gtt gtt tca gag ctg cgt 403
Ser Arg Glu Phe Arg Gly Ser Leu Thr Gln Val Val Ser Glu Leu Arg
                90                  95                  100

gat aag cag ctc gat ccc acg gtg tta ttt ctg gaa gca cgc gat gag 451
Asp Lys Gln Leu Asp Pro Thr Val Leu Phe Leu Glu Ala Arg Asp Glu
            105                 110                 115

gtg ctg atc aag cga ttc gat aat gtg cgc cgc acc cat cct ttg cag 499
Val Leu Ile Lys Arg Phe Asp Asn Val Arg Arg Thr His Pro Leu Gln
        120                 125                 130

ggc agc caa acc ctt cag gtg ggt att gaa cgt gaa cga acc gtg ctg 547
Gly Ser Gln Thr Leu Gln Val Gly Ile Glu Arg Glu Arg Thr Val Leu
    135                 140                 145

tct cct gtg aag gaa gac gct tca gtg gtc atc gat acc tcg gat ctg 595
Ser Pro Val Lys Glu Asp Ala Ser Val Val Ile Asp Thr Ser Asp Leu
150                 155                 160                 165

tcc gtg cat gat ttg cgc cgc gcc atc gaa tcc tcg ttt agg aca atc 643
Ser Val His Asp Leu Arg Arg Ala Ile Glu Ser Ser Phe Arg Thr Ile
                170                 175                 180

gcc acg cgc acc cag cac gtc acc att gaa tca ttc ggt ttc aaa cac 691
Ala Thr Arg Thr Gln His Val Thr Ile Glu Ser Phe Gly Phe Lys His
            185                 190                 195

ggc tca cca cgc gac gcc gac ttt gtt gtg gac gtg cgt ttc ttg ccg 739
Gly Ser Pro Arg Asp Ala Asp Phe Val Val Asp Val Arg Phe Leu Pro
        200                 205                 210

aac cca ttc tgg gtt cca gag ctg cgc cca ttt agg gga gtg gac aag 787
Asn Pro Phe Trp Val Pro Glu Leu Arg Pro Phe Arg Gly Val Asp Lys
    215                 220                 225

cca gta tct gac tat gtg ctc tcc caa aaa ggc gca gaa gaa ttt ttg 835

Pro Val Ser Asp Tyr Val Leu Ser Gln Lys Gly Ala Glu Glu Phe Leu
230                 235                 240                 245

aac aac ttt gtg gac atg ctc aaa gac atg ctt ccg gga tac cgc cac     883
Asn Asn Phe Val Asp Met Leu Lys Asp Met Leu Pro Gly Tyr Arg His
                250                 255                 260

gaa gga aaa aac ttc atc aca atc ggt gtc ggc tgc acc ggt gga cac     931
Glu Gly Lys Asn Phe Ile Thr Ile Gly Val Gly Cys Thr Gly Gly His
            265                 270                 275

cac aga tca gtt gcg gtg tct gaa gaa cta gcc aaa aga atc gca gat     979
His Arg Ser Val Ala Val Ser Glu Glu Leu Ala Lys Arg Ile Ala Asp
        280                 285                 290

cag acc acg ctc gac gtg tct gta gta cac cgc gat att aac cgc cac     1027
Gln Thr Thr Leu Asp Val Ser Val Val His Arg Asp Ile Asn Arg His
    295                 300                 305

taggaaaggg gccaactaat tga                                           1050

<210> 328
<211> 309
<212> PRT
<213> Corynebacterium glutamicum

<400> 328
Met Ile Gln Ser Thr Gly Val Thr His Thr Asp Lys Ser Ala Gln Glu
1               5                   10                  15

Asn Pro Val Lys Tyr Arg Asp Asn Phe Thr Pro Val Ile Ile Thr Gly
            20                  25                  30

Met Ser Gly Ala Gly Leu Ser Thr Ala Ala Arg Val Leu Glu Asp Leu
        35                  40                  45

Gly Trp Tyr Val Ala His Asn Ile Pro Pro Gln Ile Ile Leu Glu Leu
    50                  55                  60

Ile Asp Met Cys Ala Arg Glu Asp Ser Pro Val Asp Lys Val Ala Val
65                  70                  75                  80

Val Cys Asp Val Arg Ser Arg Glu Phe Arg Gly Ser Leu Thr Gln Val
                85                  90                  95

Val Ser Glu Leu Arg Asp Lys Gln Leu Asp Pro Thr Val Leu Phe Leu
            100                 105                 110

Glu Ala Arg Asp Glu Val Leu Ile Lys Arg Phe Asp Asn Val Arg Arg
        115                 120                 125

Thr His Pro Leu Gln Gly Ser Gln Thr Leu Gln Val Gly Ile Glu Arg
    130                 135                 140

Glu Arg Thr Val Leu Ser Pro Val Lys Glu Asp Ala Ser Val Val Ile
145                 150                 155                 160

Asp Thr Ser Asp Leu Ser Val His Asp Leu Arg Arg Ala Ile Glu Ser
                165                 170                 175

Ser Phe Arg Thr Ile Ala Thr Arg Thr Gln His Val Thr Ile Glu Ser
            180                 185                 190

Phe Gly Phe Lys His Gly Ser Pro Arg Asp Ala Asp Phe Val Val Asp
        195                 200                 205

Val Arg Phe Leu Pro Asn Pro Phe Trp Val Pro Glu Leu Arg Pro Phe
    210                 215                 220

Arg Gly Val Asp Lys Pro Val Ser Asp Tyr Val Leu Ser Gln Lys Gly
225                 230                 235                 240

Ala Glu Glu Phe Leu Asn Asn Phe Val Asp Met Leu Lys Asp Met Leu
                245                 250                 255

Pro Gly Tyr Arg His Glu Gly Lys Asn Phe Ile Thr Ile Gly Val Gly
            260                 265                 270

Cys Thr Gly Gly His His Arg Ser Val Ala Val Ser Glu Glu Leu Ala
        275                 280                 285

Lys Arg Ile Ala Asp Gln Thr Thr Leu Asp Val Ser Val Val His Arg
    290                 295                 300

Asp Ile Asn Arg His
305


<210> 329
<211> 558
<212> DNA
<213> Corynebacterium glutamicum

<220>
<221> CDS
<222> (101)..(535)
<223> RXN01881

<400> 329
accggccctg cggcctcaac cgccgaccag cgcggcgcac acattttgac tgtttcataa 60

taaagacaaa cttaagtatc ggagtcgaag aaaaaccaca atg gcc aat ctg att   115
                                           Met Ala Asn Leu Ile
                                           1               5

aat ctc gag aac gtc tcc aaa acc tgg gga tta aaa acg ctt ctc gac   163
Asn Leu Glu Asn Val Ser Lys Thr Trp Gly Leu Lys Thr Leu Leu Asp
                10                  15                  20

ggt gtc tcc tta ggt gtt caa acc ggc gac cgc att ggc gtc gtc ggc   211
Gly Val Ser Leu Gly Val Gln Thr Gly Asp Arg Ile Gly Val Val Gly
            25                  30                  35

ctc aat ggt ggc gga aaa acc acc ctg ctg gaa gta ctt act ggc atc   259
Leu Asn Gly Gly Gly Lys Thr Thr Leu Leu Glu Val Leu Thr Gly Ile
        40                  45                  50

gaa aag ccg gat cag ggc cgt gtg tct cac aac tct gac ctg cgc atg   307
Glu Lys Pro Asp Gln Gly Arg Val Ser His Asn Ser Asp Leu Arg Met
    55                  60                  65

gct gtg gtg acg cag cgt gct gaa ctc aat gat gac gac acc gtc gct     355
Ala Val Val Thr Gln Arg Ala Glu Leu Asn Asp Asp Asp Thr Val Ala
 70                  75                  80                  85

gac gtg gtg ctt gga cct ttg ggt ttg gaa gtt ttc gaa tgg gca tca     403
Asp Val Val Leu Gly Pro Leu Gly Leu Glu Val Phe Glu Trp Ala Ser
                 90                  95                 100

aac gcc acg gtg cgc gac gtc ctc ggt ggc ttg ggc att gtc gat ctt     451
Asn Ala Thr Val Arg Asp Val Leu Gly Gly Leu Gly Ile Val Asp Leu
            105                 110                 115

ggc ctt gac acc aag gtg ggc aaa cct ttt ccg gtg ggc gaa gcg ccg     499
Gly Leu Asp Thr Lys Val Gly Lys Pro Phe Pro Val Gly Glu Ala Pro
        120                 125                 130

acg cac caa cct ggc cgc cgc gct ggt tcg cga cct tgacctgatc          545
Thr His Gln Pro Gly Arg Arg Ala Gly Ser Arg Pro
    135                 140                 145

gtgctcgacg agc                                                      558


<210> 330
<211> 145
<212> PRT
<213> Corynebacterium glutamicum

<400> 330
Met Ala Asn Leu Ile Asn Leu Glu Asn Val Ser Lys Thr Trp Gly Leu
  1               5                  10                  15

Lys Thr Leu Leu Asp Gly Val Ser Leu Gly Val Gln Thr Gly Asp Arg
             20                  25                  30

Ile Gly Val Val Gly Leu Asn Gly Gly Gly Lys Thr Thr Leu Leu Glu
         35                  40                  45

Val Leu Thr Gly Ile Glu Lys Pro Asp Gln Gly Arg Val Ser His Asn
     50                  55                  60

Ser Asp Leu Arg Met Ala Val Val Thr Gln Arg Ala Glu Leu Asn Asp
 65                  70                  75                  80

Asp Asp Thr Val Ala Asp Val Val Leu Gly Pro Leu Gly Leu Glu Val
                 85                  90                  95

Phe Glu Trp Ala Ser Asn Ala Thr Val Arg Asp Val Leu Gly Gly Leu
            100                 105                 110

Gly Ile Val Asp Leu Gly Leu Asp Thr Lys Val Gly Lys Pro Phe Pro
        115                 120                 125

Val Gly Glu Ala Pro Thr His Gln Pro Gly Arg Arg Ala Gly Ser Arg
    130                 135                 140

Pro
145


<210> 331

<211> 564
<212> DNA
<213> Corynebacterium glutamicum

<220>
<221> CDS
<222> (101)..(541)
<223> FRXA01881

<400> 331
accggccctg cggcctcaac cgccgaccag cgcggcgcac acattttgac tgtttcataa 60

taaagacaaa cttaagtatc ggagtcgaag aaaaaccaca atg gcc aat ctg att 115
Met Ala Asn Leu Ile
1               5

aat ctc gag aac gtc tcc aaa acc tgg gga tta aaa acg ctt ctc gac 163
Asn Leu Glu Asn Val Ser Lys Thr Trp Gly Leu Lys Thr Leu Leu Asp
                10                  15                  20

ggt gtc tcc tta ggt gtt caa acc ggc gac cgc att ggc gtc gtc ggc 211
Gly Val Ser Leu Gly Val Gln Thr Gly Asp Arg Ile Gly Val Val Gly
            25                  30                  35

ctc aat ggt ggc gga aaa acc acc ctg ctg gaa gta ctt act ggc atc 259
Leu Asn Gly Gly Gly Lys Thr Thr Leu Leu Glu Val Leu Thr Gly Ile
        40                  45                  50

gaa aag ccg gat cag ggc cgt gtg tct cac aac tct gac ctg cgc atg 307
Glu Lys Pro Asp Gln Gly Arg Val Ser His Asn Ser Asp Leu Arg Met
    55                  60                  65

gct gtg gtg acg cag cgt gct gaa ctc aat gat gac gac acc gtc gct 355
Ala Val Val Thr Gln Arg Ala Glu Leu Asn Asp Asp Asp Thr Val Ala
70                  75                  80                  85

gac gtg gtg ctt gga cct ttg ggt ttg gaa gtt ttc gaa tgg gca tca 403
Asp Val Val Leu Gly Pro Leu Gly Leu Glu Val Phe Glu Trp Ala Ser
                90                  95                  100

aac gcc acg gtg cgc gac gtc ctc ggt ggc ttg ggc att gtc gat ctt 451
Asn Ala Thr Val Arg Asp Val Leu Gly Gly Leu Gly Ile Val Asp Leu
            105                 110                 115

ggc ctt gac acc aag gtg ggg caa acc ttt tcc ggt ggg cga agc gcc 499
Gly Leu Asp Thr Lys Val Gly Gln Thr Phe Ser Gly Gly Arg Ser Ala
        120                 125                 130

gac gca cca acc tgg ccg ccg cgc tgg ttc gcg acc ttg acc 541
Asp Ala Pro Thr Trp Pro Pro Arg Trp Phe Ala Thr Leu Thr
    135                 140                 145

tgatcgtgct cgacgagccc acc 564


<210> 332
<211> 147
<212> PRT
<213> Corynebacterium glutamicum

<400> 332

Met Ala Asn Leu Ile Asn Leu Glu Asn Val Ser Lys Thr Trp Gly Leu
1               5                   10                  15

Lys Thr Leu Leu Asp Gly Val Ser Leu Gly Val Gln Thr Gly Asp Arg
            20                  25                  30

Ile Gly Val Val Gly Leu Asn Gly Gly Gly Lys Thr Thr Leu Leu Glu
        35                  40                  45

Val Leu Thr Gly Ile Glu Lys Pro Asp Gln Gly Arg Val Ser His Asn
    50                  55                  60

Ser Asp Leu Arg Met Ala Val Val Thr Gln Arg Ala Glu Leu Asn Asp
65                  70                  75                  80

Asp Asp Thr Val Ala Asp Val Val Leu Gly Pro Leu Gly Leu Glu Val
                85                  90                  95

Phe Glu Trp Ala Ser Asn Ala Thr Val Arg Asp Val Leu Gly Gly Leu
            100                 105                 110

Gly Ile Val Asp Leu Gly Leu Asp Thr Lys Val Gly Gln Thr Phe Ser
        115                 120                 125

Gly Gly Arg Ser Ala Asp Ala Pro Thr Trp Pro Pro Arg Trp Phe Ala
    130                 135                 140

Thr Leu Thr
145

<210> 333
<211> 813
<212> DNA
<213> Corynebacterium glutamicum

<220>
<221> CDS
<222> (101)..(790)
<223> RXA00526

<400> 333
ggtggagcag gcggcggctc cttttagtcc tgcggcccct tttgaccctg cagcccctgc 60

cgtttctgcc aagcaaaccg tgggccaggt gatttagcct atg agc ctc atc gaa   115
                                           Met Ser Leu Ile Glu
                                           1               5

atg cga aat att gtc aag acc tac aac att gga tct gaa ggt gaa ctc   163
Met Arg Asn Ile Val Lys Thr Tyr Asn Ile Gly Ser Glu Gly Glu Leu
                10                  15                  20

acc gtg ttg cac ggt gtg gat ttc cat gtg gac cgt ggc gaa ttc gtg   211
Thr Val Leu His Gly Val Asp Phe His Val Asp Arg Gly Glu Phe Val
            25                  30                  35

tcg gtt gtg ggt acg tcc ggc tca ggt aaa tca acg atg atg aac atc   259
Ser Val Val Gly Thr Ser Gly Ser Gly Lys Ser Thr Met Met Asn Ile
        40                  45                  50

att ggg ttg ttg gat aag cca act gat ggc acg tac acc ttg gat ggc   307

Ile Gly Leu Leu Asp Lys Pro Thr Asp Gly Thr Tyr Thr Leu Asp Gly
    55                  60                  65

gtg gat gtg ttg gat atc agc gat gat gct ttg gcg agc cac cgc gct     355
Val Asp Val Leu Asp Ile Ser Asp Asp Ala Leu Ala Ser His Arg Ala
 70                  75                  80                  85

aaa tcg att ggt ttt gtg ttt cag aac ttc aat ctg att ggc cgg atc     403
Lys Ser Ile Gly Phe Val Phe Gln Asn Phe Asn Leu Ile Gly Arg Ile
                 90                  95                 100

gat gcg ttg aag aat gtg gaa atg ccc atg atg tat gcg ggc att ccg     451
Asp Ala Leu Lys Asn Val Glu Met Pro Met Met Tyr Ala Gly Ile Pro
            105                 110                 115

gct aag cag cgg aga agt cgt gcg gtt gaa tta ttg gaa atg gtc ggg     499
Ala Lys Gln Arg Arg Ser Arg Ala Val Glu Leu Leu Glu Met Val Gly
        120                 125                 130

atg ggt gag cgt ctc aac cat gag ccc aat gag ctt tcg ggt ggt cag     547
Met Gly Glu Arg Leu Asn His Glu Pro Asn Glu Leu Ser Gly Gly Gln
    135                 140                 145

aag cag cgc gtg gcc att gct cgc gcg ttg gcg aac gat cct gag atc     595
Lys Gln Arg Val Ala Ile Ala Arg Ala Leu Ala Asn Asp Pro Glu Ile
150                 155                 160                 165

att ctt gct gat gaa cca act ggt gcg ttg gat tct gca acg ggc cgg     643
Ile Leu Ala Asp Glu Pro Thr Gly Ala Leu Asp Ser Ala Thr Gly Arg
                170                 175                 180

atg gtg atg gat att ttc cac cag ctc aac aag gag cag ggc aaa acc     691
Met Val Met Asp Ile Phe His Gln Leu Asn Lys Glu Gln Gly Lys Thr
            185                 190                 195

atc gtg ttt att act cac aac cct gag ctt gct gat gaa tct gat cgg     739
Ile Val Phe Ile Thr His Asn Pro Glu Leu Ala Asp Glu Ser Asp Arg
        200                 205                 210

gtg gtc acc atg gtt gac ggg cgc atc att ggg tct gag gtg aaa cac     787
Val Val Thr Met Val Asp Gly Arg Ile Ile Gly Ser Glu Val Lys His
    215                 220                 225

tca tgagccttgc agaatcaatt ctt                                       813
Ser
230


<210> 334
<211> 230
<212> PRT
<213> Corynebacterium glutamicum

<400> 334
Met Ser Leu Ile Glu Met Arg Asn Ile Val Lys Thr Tyr Asn Ile Gly
  1               5                  10                  15

Ser Glu Gly Glu Leu Thr Val Leu His Gly Val Asp Phe His Val Asp
             20                  25                  30

Arg Gly Glu Phe Val Ser Val Val Gly Thr Ser Gly Ser Gly Lys Ser

35                  40                  45

Thr Met Met Asn Ile Ile Gly Leu Leu Asp Lys Pro Thr Asp Gly Thr
    50                  55                  60

Tyr Thr Leu Asp Gly Val Asp Val Leu Asp Ile Ser Asp Asp Ala Leu
65                  70                  75                  80

Ala Ser His Arg Ala Lys Ser Ile Gly Phe Val Phe Gln Asn Phe Asn
                85                  90                  95

Leu Ile Gly Arg Ile Asp Ala Leu Lys Asn Val Glu Met Pro Met Met
            100                 105                 110

Tyr Ala Gly Ile Pro Ala Lys Gln Arg Arg Ser Arg Ala Val Glu Leu
        115                 120                 125

Leu Glu Met Val Gly Met Gly Glu Arg Leu Asn His Glu Pro Asn Glu
    130                 135                 140

Leu Ser Gly Gly Gln Lys Gln Arg Val Ala Ile Ala Arg Ala Leu Ala
145                 150                 155                 160

Asn Asp Pro Glu Ile Ile Leu Ala Asp Glu Pro Thr Gly Ala Leu Asp
                165                 170                 175

Ser Ala Thr Gly Arg Met Val Met Asp Ile Phe His Gln Leu Asn Lys
            180                 185                 190

Glu Gln Gly Lys Thr Ile Val Phe Ile Thr His Asn Pro Glu Leu Ala
        195                 200                 205

Asp Glu Ser Asp Arg Val Val Thr Met Val Asp Gly Arg Ile Ile Gly
    210                 215                 220

Ser Glu Val Lys His Ser
225                 230


<210> 335
<211> 1008
<212> DNA
<213> Corynebacterium glutamicum

<220>
<221> CDS
<222> (101)..(985)
<223> RXN00733

<400> 335
acggcgaggt tgtcggtatt ggaacgcaca cgaatttgct gaacacgtgc ggtacctacc 60

gtgaaattgt tgaatcccaa gagactgcgc aggcgcaatc atg agt aat act gca   115
                                            Met Ser Asn Thr Ala
                                            1               5

ggc ccc cgc ggg cgt tcc cat cag gca gac gcc gcg ccg aat caa aag   163
Gly Pro Arg Gly Arg Ser His Gln Ala Asp Ala Ala Pro Asn Gln Lys
                10                  15                  20

gca cag aat ttc gga cca tct gcc aaa agg ctt ttc gga att cta ggc   211

Ala Gln Asn Phe Gly Pro Ser Ala Lys Arg Leu Phe Gly Ile Leu Gly
            25                  30                  35

cat gac cgt aac acc tta att ttt gtt atc ttc cta gcc gtc ctg agc     259
His Asp Arg Asn Thr Leu Ile Phe Val Ile Phe Leu Ala Val Leu Ser
        40                  45                  50

gtt gga ctt acc gtc ttg ggc cca tgg ttg ctg ggt aaa gcc acc aac     307
Val Gly Leu Thr Val Leu Gly Pro Trp Leu Leu Gly Lys Ala Thr Asn
    55                  60                  65

gtg gtg ttt gaa gga ttc cta tct aag cgc atg ccg gct ggt gcg tca     355
Val Val Phe Glu Gly Phe Leu Ser Lys Arg Met Pro Ala Gly Ala Ser
70                  75                  80                  85

aag gaa gat atc atc gcg cag ttg cag gct gca ggt aaa cat aat cag     403
Lys Glu Asp Ile Ile Ala Gln Leu Gln Ala Ala Gly Lys His Asn Gln
                90                  95                  100

gct tcc atg atg gaa gac atg aac ctt gtt cca ggc tca ggc att gat     451
Ala Ser Met Met Glu Asp Met Asn Leu Val Pro Gly Ser Gly Ile Asp
            105                 110                 115

ttt gaa aaa tta gcc atg atc ctc gga ctg gtg atc ggt gct tat ctc     499
Phe Glu Lys Leu Ala Met Ile Leu Gly Leu Val Ile Gly Ala Tyr Leu
        120                 125                 130

atc ggt agc ctg ttg tcg ttg ttc cag gcg cgg atg ctc aac cgc atc     547
Ile Gly Ser Leu Leu Ser Leu Phe Gln Ala Arg Met Leu Asn Arg Ile
    135                 140                 145

gtg caa agt gcc atg cac cgg ctg cgc atg gag gtg gag gaa aaa atc     595
Val Gln Ser Ala Met His Arg Leu Arg Met Glu Val Glu Glu Lys Ile
150                 155                 160                 165

cac cgc cta ccg ctg agc tat ttc gat tcc atc aaa cgt ggt gat ctg     643
His Arg Leu Pro Leu Ser Tyr Phe Asp Ser Ile Lys Arg Gly Asp Leu
                170                 175                 180

ctt agc cgt gtg acc aac gat gtg gat aat atc ggt caa tcc ctg caa     691
Leu Ser Arg Val Thr Asn Asp Val Asp Asn Ile Gly Gln Ser Leu Gln
            185                 190                 195

caa acc ttg tca cag gcg atc act tcc cta ctg acc gtc atc ggt gtg     739
Gln Thr Leu Ser Gln Ala Ile Thr Ser Leu Leu Thr Val Ile Gly Val
        200                 205                 210

ttg gtg atg atg ttt atc atc tcc cca ctg ctc gca ctc gtg gcg ctg     787
Leu Val Met Met Phe Ile Ile Ser Pro Leu Leu Ala Leu Val Ala Leu
    215                 220                 225

gta tcc att ccg gtc acc atc gtg gtc act gtg gtg gtt gcg agc cgt     835
Val Ser Ile Pro Val Thr Ile Val Val Thr Val Val Val Ala Ser Arg
230                 235                 240                 245

tcc cag aaa ctc ttt gcg gaa cag tgg aag cag acc ggt att ttg aat     883
Ser Gln Lys Leu Phe Ala Glu Gln Trp Lys Gln Thr Gly Ile Leu Asn
                250                 255                 260

gcg cgc ctg gag gaa acc tac tct ggc cac gcc gtg gtt aag gtt ttc     931
Ala Arg Leu Glu Glu Thr Tyr Ser Gly His Ala Val Val Lys Val Phe

            265                 270                 275

gga cac caa aag gat gtt caa gaa gca ttc gag gaa gaa aat caa gct 979
Gly His Gln Lys Asp Val Gln Glu Ala Phe Glu Glu Glu Asn Gln Ala
        280                 285                 290

tgt gta taaggccagc tttggtgccc agt 1008
Cys Val
    295

<210> 336
<211> 295
<212> PRT
<213> Corynebacterium glutamicum

<400> 336
Met Ser Asn Thr Ala Gly Pro Arg Gly Arg Ser His Gln Ala Asp Ala
1               5                   10                  15

Ala Pro Asn Gln Lys Ala Gln Asn Phe Gly Pro Ser Ala Lys Arg Leu
            20                  25                  30

Phe Gly Ile Leu Gly His Asp Arg Asn Thr Leu Ile Phe Val Ile Phe
        35                  40                  45

Leu Ala Val Leu Ser Val Gly Leu Thr Val Leu Gly Pro Trp Leu Leu
    50                  55                  60

Gly Lys Ala Thr Asn Val Val Phe Glu Gly Phe Leu Ser Lys Arg Met
65                  70                  75                  80

Pro Ala Gly Ala Ser Lys Glu Asp Ile Ile Ala Gln Leu Gln Ala Ala
                85                  90                  95

Gly Lys His Asn Gln Ala Ser Met Met Glu Asp Met Asn Leu Val Pro
            100                 105                 110

Gly Ser Gly Ile Asp Phe Glu Lys Leu Ala Met Ile Leu Gly Leu Val
        115                 120                 125

Ile Gly Ala Tyr Leu Ile Gly Ser Leu Leu Ser Leu Phe Gln Ala Arg
    130                 135                 140

Met Leu Asn Arg Ile Val Gln Ser Ala Met His Arg Leu Arg Met Glu
145                 150                 155                 160

Val Glu Glu Lys Ile His Arg Leu Pro Leu Ser Tyr Phe Asp Ser Ile
                165                 170                 175

Lys Arg Gly Asp Leu Leu Ser Arg Val Thr Asn Asp Val Asp Asn Ile
            180                 185                 190

Gly Gln Ser Leu Gln Gln Thr Leu Ser Gln Ala Ile Thr Ser Leu Leu
        195                 200                 205

Thr Val Ile Gly Val Leu Val Met Met Phe Ile Ile Ser Pro Leu Leu
    210                 215                 220

Ala Leu Val Ala Leu Val Ser Ile Pro Val Thr Ile Val Val Thr Val
225                 230                 235                 240

Val Val Ala Ser Arg Ser Gln Lys Leu Phe Ala Glu Gln Trp Lys Gln
                245                 250                 255

Thr Gly Ile Leu Asn Ala Arg Leu Glu Glu Thr Tyr Ser Gly His Ala
            260                 265                 270

Val Val Lys Val Phe Gly His Gln Lys Asp Val Gln Glu Ala Phe Glu
        275                 280                 285

Glu Glu Asn Gln Ala Cys Val
    290                 295

<210> 337
<211> 508
<212> DNA
<213> Corynebacterium glutamicum

<220>
<221> CDS
<222> (101)..(508)
<223> FRXA00733

<400> 337
acggcgaggt tgtcggtatt ggaacgcaca cgaatttgct gaacacgtgc ggtacctacc 60

gtgaaattgt tgaatcccaa gagactgcgc aggcgcaatc atg agt aat act gca    115
                                            Met Ser Asn Thr Ala
                                            1               5

ggc ccc cgc ggg cgt tcc cat cag gca gac gcc gcg ccg aat caa aag    163
Gly Pro Arg Gly Arg Ser His Gln Ala Asp Ala Ala Pro Asn Gln Lys
                10                  15                  20

gca cag aat ttc gga cca tct gcc aaa agg ctt ttc gga att cta ggc    211
Ala Gln Asn Phe Gly Pro Ser Ala Lys Arg Leu Phe Gly Ile Leu Gly
            25                  30                  35

cat gac cgt aac acc tta att ttt gtt atc ttc cta gcc gtc ctg agc    259
His Asp Arg Asn Thr Leu Ile Phe Val Ile Phe Leu Ala Val Leu Ser
        40                  45                  50

gtt gga ctt acc gtc ttg ggc cca tgg ttg ctg ggt aaa gcc acc aac    307
Val Gly Leu Thr Val Leu Gly Pro Trp Leu Leu Gly Lys Ala Thr Asn
    55                  60                  65

gtg gtg ttt gaa gga ttc cta tct aag cgc atg ccg gct ggt gcg tca    355
Val Val Phe Glu Gly Phe Leu Ser Lys Arg Met Pro Ala Gly Ala Ser
70                  75                  80                  85

aag gaa gat atc atc gcg cag ttg cag gct gca ggt aaa cat aat cag    403
Lys Glu Asp Ile Ile Ala Gln Leu Gln Ala Ala Gly Lys His Asn Gln
                90                  95                  100

gct tcc atg atg gaa gac atg aac ctt gtt cca ggc tca ggc att gat    451
Ala Ser Met Met Glu Asp Met Asn Leu Val Pro Gly Ser Gly Ile Asp
            105                 110                 115

ttt gaa aaa tta gcc atg atc ctc gga ctg gtg atc ggt gct tat ctc    499
Phe Glu Lys Leu Ala Met Ile Leu Gly Leu Val Ile Gly Ala Tyr Leu

120                 125                 130

att cgt agc                                                       508
Ile Arg Ser
    135


<210> 338
<211> 136
<212> PRT
<213> Corynebacterium glutamicum

<400> 338
Met Ser Asn Thr Ala Gly Pro Arg Gly Arg Ser His Gln Ala Asp Ala
  1               5                  10                  15

Ala Pro Asn Gln Lys Ala Gln Asn Phe Gly Pro Ser Ala Lys Arg Leu
             20                  25                  30

Phe Gly Ile Leu Gly His Asp Arg Asn Thr Leu Ile Phe Val Ile Phe
         35                  40                  45

Leu Ala Val Leu Ser Val Gly Leu Thr Val Leu Gly Pro Trp Leu Leu
     50                  55                  60

Gly Lys Ala Thr Asn Val Val Phe Glu Gly Phe Leu Ser Lys Arg Met
 65                  70                  75                  80

Pro Ala Gly Ala Ser Lys Glu Asp Ile Ile Ala Gln Leu Gln Ala Ala
                 85                  90                  95

Gly Lys His Asn Gln Ala Ser Met Met Glu Asp Met Asn Leu Val Pro
            100                 105                 110

Gly Ser Gly Ile Asp Phe Glu Lys Leu Ala Met Ile Leu Gly Leu Val
        115                 120                 125

Ile Gly Ala Tyr Leu Ile Arg Ser
    130                 135


<210> 339
<211> 692
<212> DNA
<213> Corynebacterium glutamicum

<220>
<221> CDS
<222> (1)..(669)
<223> RXA00735

<400> 339
ggt ccc acc ggt gcg ggc aag acc aca ttg gtg aat ctg atc atg cgt    48
Gly Pro Thr Gly Ala Gly Lys Thr Thr Leu Val Asn Leu Ile Met Arg
  1               5                  10                  15

ttc tac gac atc aac agc ggt tcc atc act ctt ggt gaa aca gca caa    96
Phe Tyr Asp Ile Asn Ser Gly Ser Ile Thr Leu Gly Glu Thr Ala Gln
             20                  25                  30

gac gcc gtg gat atc cgc acc atg gct aga gaa gat ctg cga tca cga   144

Asp Ala Val Asp Ile Arg Thr Met Ala Arg Glu Asp Leu Arg Ser Arg
        35                  40                  45

acc ggc atg gtg ttg cag gat acg tgg ctg ttt gcc gga acc atc agg     192
Thr Gly Met Val Leu Gln Asp Thr Trp Leu Phe Ala Gly Thr Ile Arg
    50                  55                  60

gat aac att ctt tac ggt aga cct gaa gca act gag gaa gaa atg ctt     240
Asp Asn Ile Leu Tyr Gly Arg Pro Glu Ala Thr Glu Glu Glu Met Leu
65                  70                  75                  80

gct gcg tcc aag gcc gcc tac gtg gat cgt ttt gtc cgt tcc ctg cca     288
Ala Ala Ser Lys Ala Ala Tyr Val Asp Arg Phe Val Arg Ser Leu Pro
                85                  90                  95

gaa ggc tac gac acc gta ctt gat gat gaa gcc atg aac cta tcg gtg     336
Glu Gly Tyr Asp Thr Val Leu Asp Asp Glu Ala Met Asn Leu Ser Val
            100                 105                 110

ggt gaa cgc cag ctg atc acc atc gcg cgt gca ttc ttg gct aat ccc     384
Gly Glu Arg Gln Leu Ile Thr Ile Ala Arg Ala Phe Leu Ala Asn Pro
        115                 120                 125

cga ctg ctg att ctg gat gaa gcc acc tca tcg gtg gat acg cgt acc     432
Arg Leu Leu Ile Leu Asp Glu Ala Thr Ser Ser Val Asp Thr Arg Thr
    130                 135                 140

gaa ttg ttg att cag cgc gcc atg tcc aag ctg cgc caa gac cgc acc     480
Glu Leu Leu Ile Gln Arg Ala Met Ser Lys Leu Arg Gln Asp Arg Thr
145                 150                 155                 160

gcc ttc gtc atc gcg cac cgg ttg tcc acg att cgt gat gcc aac ctg     528
Ala Phe Val Ile Ala His Arg Leu Ser Thr Ile Arg Asp Ala Asn Leu
                165                 170                 175

att ttg atg atg aaa gac ggc gag atc gtg gag cag ggc aat cac cgt     576
Ile Leu Met Met Lys Asp Gly Glu Ile Val Glu Gln Gly Asn His Arg
            180                 185                 190

gag ttg atg gcc ctg gag ggc gca tat tgg gag ttg tat aac tcc caa     624
Glu Leu Met Ala Leu Glu Gly Ala Tyr Trp Glu Leu Tyr Asn Ser Gln
        195                 200                 205

ttc aac gcc ccc gcg aaa gaa gaa tta cag gct gac gga gat cac         669
Phe Asn Ala Pro Ala Lys Glu Glu Leu Gln Ala Asp Gly Asp His
    210                 215                 220

tgatgatttc ttcttaggct ttc                                           692


<210> 340
<211> 223
<212> PRT
<213> Corynebacterium glutamicum

<400> 340
Gly Pro Thr Gly Ala Gly Lys Thr Thr Leu Val Asn Leu Ile Met Arg
1               5                   10                  15

Phe Tyr Asp Ile Asn Ser Gly Ser Ile Thr Leu Gly Glu Thr Ala Gln
            20                  25                  30

Asp Ala Val Asp Ile Arg Thr Met Ala Arg Glu Asp Leu Arg Ser Arg
        35                  40                  45

Thr Gly Met Val Leu Gln Asp Thr Trp Leu Phe Ala Gly Thr Ile Arg
    50                  55                  60

Asp Asn Ile Leu Tyr Gly Arg Pro Glu Ala Thr Glu Glu Glu Met Leu
65                  70                  75                  80

Ala Ala Ser Lys Ala Ala Tyr Val Asp Arg Phe Val Arg Ser Leu Pro
                85                  90                  95

Glu Gly Tyr Asp Thr Val Leu Asp Asp Glu Ala Met Asn Leu Ser Val
            100                 105                 110

Gly Glu Arg Gln Leu Ile Thr Ile Ala Arg Ala Phe Leu Ala Asn Pro
        115                 120                 125

Arg Leu Leu Ile Leu Asp Glu Ala Thr Ser Ser Val Asp Thr Arg Thr
    130                 135                 140

Glu Leu Leu Ile Gln Arg Ala Met Ser Lys Leu Arg Gln Asp Arg Thr
145                 150                 155                 160

Ala Phe Val Ile Ala His Arg Leu Ser Thr Ile Arg Asp Ala Asn Leu
                165                 170                 175

Ile Leu Met Met Lys Asp Gly Glu Ile Val Glu Gln Gly Asn His Arg
            180                 185                 190

Glu Leu Met Ala Leu Glu Gly Ala Tyr Trp Glu Leu Tyr Asn Ser Gln
        195                 200                 205

Phe Asn Ala Pro Ala Lys Glu Glu Leu Gln Ala Asp Gly Asp His
    210                 215                 220


<210> 341
<211> 1986
<212> DNA
<213> Corynebacterium glutamicum

<220>
<221> CDS
<222> (101)..(1963)
<223> RXA00878

<400> 341
cgagattagg tccgcttcag ttgtggtggc tccgaatctg atgaacaatg atcattccta 60

attcatttac atctttatca aagagagcca ccacctacta atg cga ctt ctt ggt   115
                                           Met Arg Leu Leu Gly
                                           1               5

cga att tta aaa acc acg tct gcg ctt tgg ccc tac tat ctc gga att   163
Arg Ile Leu Lys Thr Thr Ser Ala Leu Trp Pro Tyr Tyr Leu Gly Ile
                10                  15                  20

atc gtc gta tcc att gtg atc gcg gcg ttg tcg ctg ctg tcg ccg ttt   211
Ile Val Val Ser Ile Val Ile Ala Ala Leu Ser Leu Leu Ser Pro Phe

            25                  30                  35

att ctc cgc gaa gca aca gat tcc att gtt tct gca gta acc gga tct 259
Ile Leu Arg Glu Ala Thr Asp Ser Ile Val Ser Ala Val Thr Gly Ser
        40                  45                  50

aac acc gtc gat gca gtt act cgc act att att ttc tta gct tta gcc 307
Asn Thr Val Asp Ala Val Thr Arg Thr Ile Ile Phe Leu Ala Leu Ala
    55                  60                  65

ctg ttt gtc gca agc ttc ctc aat acg gtg atg acc aac atc ggt ggc 355
Leu Phe Val Ala Ser Phe Leu Asn Thr Val Met Thr Asn Ile Gly Gly
70                  75                  80                  85

tac atc ggt gat gtc atg gca tct cgt atg cgc cag att ctg gcc acg 403
Tyr Ile Gly Asp Val Met Ala Ser Arg Met Arg Gln Ile Leu Ala Thr
                90                  95                  100

cgc tat tac gca aag ctg ttg gcg ctg cct cag aag tat ttt gat aat 451
Arg Tyr Tyr Ala Lys Leu Leu Ala Leu Pro Gln Lys Tyr Phe Asp Asn
            105                 110                 115

cag gtc acc ggc acc atc atc gcc cgc ctt gat cga tca atc aac ggc 499
Gln Val Thr Gly Thr Ile Ile Ala Arg Leu Asp Arg Ser Ile Asn Gly
        120                 125                 130

atc acg cag ttc atg cag agc ttc tcc aac aac ttc ttc ccc atg ctc 547
Ile Thr Gln Phe Met Gln Ser Phe Ser Asn Asn Phe Phe Pro Met Leu
    135                 140                 145

atc acc atg gtg gca gtg ctg att att tcc gcg att ttc tac tgg cct 595
Ile Thr Met Val Ala Val Leu Ile Ile Ser Ala Ile Phe Tyr Trp Pro
150                 155                 160                 165

ctg gca att ctg ctg gcc atg ttg ttc ccg att tac atg tgg ctg acg 643
Leu Ala Ile Leu Leu Ala Met Leu Phe Pro Ile Tyr Met Trp Leu Thr
                170                 175                 180

gcg ttg aca tcg aaa cgc tgg cag aaa tat gag ggc gag aaa aac cat 691
Ala Leu Thr Ser Lys Arg Trp Gln Lys Tyr Glu Gly Glu Lys Asn His
            185                 190                 195

gaa atc gac gtg gct aac ggc cgc ttc gct gag gtt gtc ggc cag gtc 739
Glu Ile Asp Val Ala Asn Gly Arg Phe Ala Glu Val Val Gly Gln Val
        200                 205                 210

aag gtt gtt aaa tca ttc gtc gca gag acc cgc gag ctg gct gat ttc 787
Lys Val Val Lys Ser Phe Val Ala Glu Thr Arg Glu Leu Ala Asp Phe
    215                 220                 225

ggt ggg cgt tac ggc aaa aca gta gcg att acc cgg ccg caa tcc ggt 835
Gly Gly Arg Tyr Gly Lys Thr Val Ala Ile Thr Arg Pro Gln Ser Gly
230                 235                 240                 245

tgg tgg cac cgc atg gat act ctc cgt ggc gcg gca cta aat atc atc 883
Trp Trp His Arg Met Asp Thr Leu Arg Gly Ala Ala Leu Asn Ile Ile
                250                 255                 260

ttc ctg gcc att cac ctg ctg att ttc tac cgc acc ttg cac ggc cat 931
Phe Leu Ala Ile His Leu Leu Ile Phe Tyr Arg Thr Leu His Gly His
            265                 270                 275

ttc acc atc ggc gac atg gtc atg ctc atc cag ctt gtc acc atg gcg 979
Phe Thr Ile Gly Asp Met Val Met Leu Ile Gln Leu Val Thr Met Ala
        280                 285                 290

cag caa ccg gtg tac atg atg agc tac atc gtc gac tcc gcg cag cgc 1027
Gln Gln Pro Val Tyr Met Met Ser Tyr Ile Val Asp Ser Ala Gln Arg
    295                 300                 305

gcc atc gcc ggc tcc cgc gac tac ttc gag gtc atg gcg cag cag gtc 1075
Ala Ile Ala Gly Ser Arg Asp Tyr Phe Glu Val Met Ala Gln Gln Val
310                 315                 320                 325

gag ccc acc gcc aat aag gag ctt gtc gac gcc acc ctc gcc tca gac 1123
Glu Pro Thr Ala Asn Lys Glu Leu Val Asp Ala Thr Leu Ala Ser Asp
                330                 335                 340

act cca cgc atc agt gtg ggc acg ccc gcc gcg ctg ccc gct gga gaa 1171
Thr Pro Arg Ile Ser Val Gly Thr Pro Ala Ala Leu Pro Ala Gly Glu
            345                 350                 355

cca gcg atg gaa ttc aaa aac gtc acc ttc gcc tac gaa gaa ggc aag 1219
Pro Ala Met Glu Phe Lys Asn Val Thr Phe Ala Tyr Glu Glu Gly Lys
        360                 365                 370

ccg gtt att tcc gac gtg tcc att acc gcc cgc cac ggc gag cgc atc 1267
Pro Val Ile Ser Asp Val Ser Ile Thr Ala Arg His Gly Glu Arg Ile
    375                 380                 385

gcg ttg gtc ggt gaa tcc ggc ggc ggt aaa tcc acc ctg gtc aac ctt 1315
Ala Leu Val Gly Glu Ser Gly Gly Gly Lys Ser Thr Leu Val Asn Leu
390                 395                 400                 405

ctg tta ggt ctg tac aaa cca aac agc ggc agc ctt gca gta tgt ggc 1363
Leu Leu Gly Leu Tyr Lys Pro Asn Ser Gly Ser Leu Ala Val Cys Gly
                410                 415                 420

gtg gat gtt aaa gat ctg act tcc gag gaa ctt cgc gca tcc gtg ggt 1411
Val Asp Val Lys Asp Leu Thr Ser Glu Glu Leu Arg Ala Ser Val Gly
            425                 430                 435

gtg gtc ttc cag gac gcc agc ttg ttc tct gga tct att gca gaa aac 1459
Val Val Phe Gln Asp Ala Ser Leu Phe Ser Gly Ser Ile Ala Glu Asn
        440                 445                 450

atc gcc tac ggt cgc cca ggt gcc acc cgc gaa gag atc atc gaa gtg 1507
Ile Ala Tyr Gly Arg Pro Gly Ala Thr Arg Glu Glu Ile Ile Glu Val
    455                 460                 465

gct aag aaa gcc aac gca cat gag ttc att tcc gcc ttc cct gaa gga 1555
Ala Lys Lys Ala Asn Ala His Glu Phe Ile Ser Ala Phe Pro Glu Gly
470                 475                 480                 485

tat gaa acc gtc gtc ggt gaa cgc gga ctc aaa ctt tct ggt ggc cag 1603
Tyr Glu Thr Val Val Gly Glu Arg Gly Leu Lys Leu Ser Gly Gly Gln
                490                 495                 500

aag cag cgc gtc tct gtg gca cgg gcc atg ctt aaa gat gcc cca ctt 1651
Lys Gln Arg Val Ser Val Ala Arg Ala Met Leu Lys Asp Ala Pro Leu
            505                 510                 515

ctt gtt ctc gat gaa gcc acc tct gca ctg gat acc aag tct gag cag    1699
Leu Val Leu Asp Glu Ala Thr Ser Ala Leu Asp Thr Lys Ser Glu Gln
        520                 525                 530

gca gtc caa gcc ggt ttg gaa cag ctg atg gaa aac cgc acc acc tta    1747
Ala Val Gln Ala Gly Leu Glu Gln Leu Met Glu Asn Arg Thr Thr Leu
    535                 540                 545

atg atc gcc cac cgc ctg tcc acc atc gca ggc gtc gat acc atc gtg    1795
Met Ile Ala His Arg Leu Ser Thr Ile Ala Gly Val Asp Thr Ile Val
550                 555                 560                 565

acc atc caa aac gga cgg gtt gaa gag gtc gga tct cct acc gag ctc    1843
Thr Ile Gln Asn Gly Arg Val Glu Glu Val Gly Ser Pro Thr Glu Leu
                570                 575                 580

gca gtc tca ggc ggt atc tat tcc gaa ctg ctg cgc ctg acc aac tcc    1891
Ala Val Ser Gly Gly Ile Tyr Ser Glu Leu Leu Arg Leu Thr Asn Ser
            585                 590                 595

aca gca gaa gcc gac cgg gag cgt ctg cgc gcc ttt ggt ttc act ggc    1939
Thr Ala Glu Ala Asp Arg Glu Arg Leu Arg Ala Phe Gly Phe Thr Gly
        600                 605                 610

gat gca cca gct gaa gaa gag gac tagccccgcg aaagaacaat ccc          1986
Asp Ala Pro Ala Glu Glu Glu Asp
    615                 620


<210> 342
<211> 621
<212> PRT
<213> Corynebacterium glutamicum

<400> 342
Met Arg Leu Leu Gly Arg Ile Leu Lys Thr Thr Ser Ala Leu Trp Pro
1               5                   10                  15

Tyr Tyr Leu Gly Ile Ile Val Val Ser Ile Val Ile Ala Ala Leu Ser
            20                  25                  30

Leu Leu Ser Pro Phe Ile Leu Arg Glu Ala Thr Asp Ser Ile Val Ser
        35                  40                  45

Ala Val Thr Gly Ser Asn Thr Val Asp Ala Val Thr Arg Thr Ile Ile
    50                  55                  60

Phe Leu Ala Leu Ala Leu Phe Val Ala Ser Phe Leu Asn Thr Val Met
65                  70                  75                  80

Thr Asn Ile Gly Gly Tyr Ile Gly Asp Val Met Ala Ser Arg Met Arg
                85                  90                  95

Gln Ile Leu Ala Thr Arg Tyr Tyr Ala Lys Leu Leu Ala Leu Pro Gln
            100                 105                 110

Lys Tyr Phe Asp Asn Gln Val Thr Gly Thr Ile Ile Ala Arg Leu Asp
        115                 120                 125

Arg Ser Ile Asn Gly Ile Thr Gln Phe Met Gln Ser Phe Ser Asn Asn
    130                 135                 140

Phe Phe Pro Met Leu Ile Thr Met Val Ala Val Leu Ile Ile Ser Ala
145                 150                 155                 160

Ile Phe Tyr Trp Pro Leu Ala Ile Leu Leu Ala Met Leu Phe Pro Ile
                165                 170                 175

Tyr Met Trp Leu Thr Ala Leu Thr Ser Lys Arg Trp Gln Lys Tyr Glu
            180                 185                 190

Gly Glu Lys Asn His Glu Ile Asp Val Ala Asn Gly Arg Phe Ala Glu
        195                 200                 205

Val Val Gly Gln Val Lys Val Val Lys Ser Phe Val Ala Glu Thr Arg
    210                 215                 220

Glu Leu Ala Asp Phe Gly Gly Arg Tyr Gly Lys Thr Val Ala Ile Thr
225                 230                 235                 240

Arg Pro Gln Ser Gly Trp Trp His Arg Met Asp Thr Leu Arg Gly Ala
                245                 250                 255

Ala Leu Asn Ile Ile Phe Leu Ala Ile His Leu Leu Ile Phe Tyr Arg
            260                 265                 270

Thr Leu His Gly His Phe Thr Ile Gly Asp Met Val Met Leu Ile Gln
        275                 280                 285

Leu Val Thr Met Ala Gln Gln Pro Val Tyr Met Met Ser Tyr Ile Val
    290                 295                 300

Asp Ser Ala Gln Arg Ala Ile Ala Gly Ser Arg Asp Tyr Phe Glu Val
305                 310                 315                 320

Met Ala Gln Gln Val Glu Pro Thr Ala Asn Lys Glu Leu Val Asp Ala
                325                 330                 335

Thr Leu Ala Ser Asp Thr Pro Arg Ile Ser Val Gly Thr Pro Ala Ala
            340                 345                 350

Leu Pro Ala Gly Glu Pro Ala Met Glu Phe Lys Asn Val Thr Phe Ala
        355                 360                 365

Tyr Glu Glu Gly Lys Pro Val Ile Ser Asp Val Ser Ile Thr Ala Arg
    370                 375                 380

His Gly Glu Arg Ile Ala Leu Val Gly Glu Ser Gly Gly Gly Lys Ser
385                 390                 395                 400

Thr Leu Val Asn Leu Leu Leu Gly Leu Tyr Lys Pro Asn Ser Gly Ser
                405                 410                 415

Leu Ala Val Cys Gly Val Asp Val Lys Asp Leu Thr Ser Glu Glu Leu
            420                 425                 430

Arg Ala Ser Val Gly Val Val Phe Gln Asp Ala Ser Leu Phe Ser Gly
        435                 440                 445

Ser Ile Ala Glu Asn Ile Ala Tyr Gly Arg Pro Gly Ala Thr Arg Glu
    450                 455                 460

Glu Ile Ile Glu Val Ala Lys Lys Ala Asn Ala His Glu Phe Ile Ser
465                 470                 475                 480

Ala Phe Pro Glu Gly Tyr Glu Thr Val Val Gly Glu Arg Gly Leu Lys
                485                 490                 495

Leu Ser Gly Gly Gln Lys Gln Arg Val Ser Val Ala Arg Ala Met Leu
            500                 505                 510

Lys Asp Ala Pro Leu Leu Val Leu Asp Glu Ala Thr Ser Ala Leu Asp
        515                 520                 525

Thr Lys Ser Glu Gln Ala Val Gln Ala Gly Leu Glu Gln Leu Met Glu
    530                 535                 540

Asn Arg Thr Thr Leu Met Ile Ala His Arg Leu Ser Thr Ile Ala Gly
545                 550                 555                 560

Val Asp Thr Ile Val Thr Ile Gln Asn Gly Arg Val Glu Glu Val Gly
                565                 570                 575

Ser Pro Thr Glu Leu Ala Val Ser Gly Gly Ile Tyr Ser Glu Leu Leu
            580                 585                 590

Arg Leu Thr Asn Ser Thr Ala Glu Ala Asp Arg Glu Arg Leu Arg Ala
        595                 600                 605

Phe Gly Phe Thr Gly Asp Ala Pro Ala Glu Glu Glu Asp
    610                 615                 620


<210> 343
<211> 1713
<212> DNA
<213> Corynebacterium glutamicum

<220>
<221> CDS
<222> (101)..(1690)
<223> RXN01191

<400> 343
cgctgctttc acgcaactga aaccgcaccg gatcaagtta tttggggttg ttctttgtgg 60

cgtgttggtg gccgtcgcgg ggttggtagg gccctgggcg gtg ggt gga ctc gtc   115
                                           Val Gly Gly Leu Val
                                           1               5

gat aag ctc ctt gca acc ccg agc atg cgc gac gtt gta gtg ttc gcg   163
Asp Lys Leu Leu Ala Thr Pro Ser Met Arg Asp Val Val Val Phe Ala
                10                  15                  20

ctg ctt atc gtg gct ggc ggc gtt gtt tcg agc ctg ggc acg tgg tgg   211
Leu Leu Ile Val Ala Gly Gly Val Val Ser Ser Leu Gly Thr Trp Trp
            25                  30                  35

ggc agc gcg ctg atg gcg cgc gcg ttg gag ccg gcg atc gcg ggg ctg   259
Gly Ser Ala Leu Met Ala Arg Ala Leu Glu Pro Ala Ile Ala Gly Leu
        40                  45                  50

cgc gag gat gtg ttg cgc gcg gcg gtg agt ttg gat gcg aac acg att   307

Arg Glu Asp Val Leu Arg Ala Ala Val Ser Leu Asp Ala Asn Thr Ile
    55                  60                  65

gaa acg gcg ggg cgc ggc gac gtg att tcg cgt atc gcg gat gat tcg 355
Glu Thr Ala Gly Arg Gly Asp Val Ile Ser Arg Ile Ala Asp Asp Ser
70                  75                  80                  85

cgg gag gtg tcc act gcg gcg agc acc gtg gtg ccg ctg atg gtg cag 403
Arg Glu Val Ser Thr Ala Ala Ser Thr Val Val Pro Leu Met Val Gln
                90                  95                  100

gcg ggc ttt acc gtg gtg att tcc gcg ttt ggc atg gcg gcg gtt gat 451
Ala Gly Phe Thr Val Val Ile Ser Ala Phe Gly Met Ala Ala Val Asp
            105                 110                 115

tgg cgc ctc ggc ctt gtc ggt ttg gtc gcg atc ccg ctg tat tgg acc 499
Trp Arg Leu Gly Leu Val Gly Leu Val Ala Ile Pro Leu Tyr Trp Thr
        120                 125                 130

acg ttg cgc gtc tat tta ccc cgc tca ggt ccg ctt tat acg cgt gag 547
Thr Leu Arg Val Tyr Leu Pro Arg Ser Gly Pro Leu Tyr Thr Arg Glu
    135                 140                 145

cgc gag gcc ttt ggg gtg cgc acg cag cgg ctt gtc ggc gca gtc gaa 595
Arg Glu Ala Phe Gly Val Arg Thr Gln Arg Leu Val Gly Ala Val Glu
150                 155                 160                 165

ggc gcg gaa acc ttg cgc gct ttc cgc gca gaa gat aca gaa tta aag 643
Gly Ala Glu Thr Leu Arg Ala Phe Arg Ala Glu Asp Thr Glu Leu Lys
                170                 175                 180

cgt atc gac gca gcc tcc ggc gaa gcc cgc gac att tcc att tct gtt 691
Arg Ile Asp Ala Ala Ser Gly Glu Ala Arg Asp Ile Ser Ile Ser Val
            185                 190                 195

ttc agg ttc ctc aca tgg gca ttt tcc cgc aac aac cgc gcg gaa tgc 739
Phe Arg Phe Leu Thr Trp Ala Phe Ser Arg Asn Asn Arg Ala Glu Cys
        200                 205                 210

atc acc ctc gtg ctc atc ttg ggc acc ggc ttt tac ctg gtc aac atc 787
Ile Thr Leu Val Leu Ile Leu Gly Thr Gly Phe Tyr Leu Val Asn Ile
    215                 220                 225

gat ctg gtc acc gtc ggc gca gtc tca acc gcc gca ctg atc ttc cac 835
Asp Leu Val Thr Val Gly Ala Val Ser Thr Ala Ala Leu Ile Phe His
230                 235                 240                 245

cga ctc ttc ggt cca atc ggc acg ctc gtg ggc atg ttc tcc gac atc 883
Arg Leu Phe Gly Pro Ile Gly Thr Leu Val Gly Met Phe Ser Asp Ile
                250                 255                 260

caa tcc gcc agc gca tcg ctg atc cgc atg gtg ggc gtt att aac gcg 931
Gln Ser Ala Ser Ala Ser Leu Ile Arg Met Val Gly Val Ile Asn Ala
            265                 270                 275

gca tcg aac cag gtc agc ggc acc tcg ccg gcg tct gcc agc acc gct 979
Ala Ser Asn Gln Val Ser Gly Thr Ser Pro Ala Ser Ala Ser Thr Ala
        280                 285                 290

tta acg ctt ttc gac gtc tcc cac cac tat cac act gca ccc gtc atc 1027
Leu Thr Leu Phe Asp Val Ser His His Tyr His Thr Ala Pro Val Ile

295                 300                 305

aag aat gca tcc gtg cag ctg gaa cca ggg gaa cac atc gcc att gtg     1075
Lys Asn Ala Ser Val Gln Leu Glu Pro Gly Glu His Ile Ala Ile Val
310                 315                 320                 325

ggt gcg acc ggc gct ggt aaa agc acg ctc gcc ctc att gcg gca ggc     1123
Gly Ala Thr Gly Ala Gly Lys Ser Thr Leu Ala Leu Ile Ala Ala Gly
                330                 335                 340

ctg ctc agc cca act tcc ggg cag gtg gct ctc ggc gga tcg agt ttt     1171
Leu Leu Ser Pro Thr Ser Gly Gln Val Ala Leu Gly Gly Ser Ser Phe
            345                 350                 355

tct aac gtc gaa ccg gaa gca ttg cgc cag aag atc gcg atg gtc agc     1219
Ser Asn Val Glu Pro Glu Ala Leu Arg Gln Lys Ile Ala Met Val Ser
        360                 365                 370

caa gaa atc cac tgc ttc cga gga tct gtt tta gat aat ctt cgt atc     1267
Gln Glu Ile His Cys Phe Arg Gly Ser Val Leu Asp Asn Leu Arg Ile
    375                 380                 385

gca cgc ccc gaa gcc acc gat gcg gac atc cac gcc gtt ctc gcc gat     1315
Ala Arg Pro Glu Ala Thr Asp Ala Asp Ile His Ala Val Leu Ala Asp
390                 395                 400                 405

att ggt gat tcc tgg ttg gag cgc tta ccg caa ggc ata gac acc atc     1363
Ile Gly Asp Ser Trp Leu Glu Arg Leu Pro Gln Gly Ile Asp Thr Ile
                410                 415                 420

gtg ggt gat ggc gct ttc cgt tta acc tct gtg gaa aac cag atc atg     1411
Val Gly Asp Gly Ala Phe Arg Leu Thr Ser Val Glu Asn Gln Ile Met
            425                 430                 435

gcg ctt gct cgc gta cat ttg gcc gac cta gca atc gtc atc ctt gat     1459
Ala Leu Ala Arg Val His Leu Ala Asp Leu Ala Ile Val Ile Leu Asp
        440                 445                 450

gaa gca acg gct gaa tca ggc tct gat cat gca aaa cag ctt gaa gat     1507
Glu Ala Thr Ala Glu Ser Gly Ser Asp His Ala Lys Gln Leu Glu Asp
    455                 460                 465

gca gcc ctt aaa gtc act gaa aac aga tca gcc atc atc gtg gct cac     1555
Ala Ala Leu Lys Val Thr Glu Asn Arg Ser Ala Ile Ile Val Ala His
470                 475                 480                 485

cgc ctc aac caa gcg aaa acc gcc gat cgc atc atc gtc atg gac tcc     1603
Arg Leu Asn Gln Ala Lys Thr Ala Asp Arg Ile Ile Val Met Asp Ser
                490                 495                 500

gga gaa atc ata gaa tct gga acc cat gaa gag ctt cga gcg atc ggc     1651
Gly Glu Ile Ile Glu Ser Gly Thr His Glu Glu Leu Arg Ala Ile Gly
            505                 510                 515

ggc cga tat gaa caa ctg tgg act gcg tgg tct gcg cgc taattagcca     1700
Gly Arg Tyr Glu Gln Leu Trp Thr Ala Trp Ser Ala Arg
        520                 525                 530

cccaagacca cgc                                                      1713

<210> 344
<211> 530
<212> PRT
<213> Corynebacterium glutamicum

<400> 344
Val Gly Gly Leu Val Asp Lys Leu Leu Ala Thr Pro Ser Met Arg Asp
1               5                   10                  15

Val Val Val Phe Ala Leu Leu Ile Val Ala Gly Gly Val Val Ser Ser
            20                  25                  30

Leu Gly Thr Trp Trp Gly Ser Ala Leu Met Ala Arg Ala Leu Glu Pro
        35                  40                  45

Ala Ile Ala Gly Leu Arg Glu Asp Val Leu Arg Ala Ala Val Ser Leu
    50                  55                  60

Asp Ala Asn Thr Ile Glu Thr Ala Gly Arg Gly Asp Val Ile Ser Arg
65                  70                  75                  80

Ile Ala Asp Asp Ser Arg Glu Val Ser Thr Ala Ala Ser Thr Val Val
                85                  90                  95

Pro Leu Met Val Gln Ala Gly Phe Thr Val Val Ile Ser Ala Phe Gly
            100                 105                 110

Met Ala Ala Val Asp Trp Arg Leu Gly Leu Val Gly Leu Val Ala Ile
        115                 120                 125

Pro Leu Tyr Trp Thr Thr Leu Arg Val Tyr Leu Pro Arg Ser Gly Pro
    130                 135                 140

Leu Tyr Thr Arg Glu Arg Glu Ala Phe Gly Val Arg Thr Gln Arg Leu
145                 150                 155                 160

Val Gly Ala Val Glu Gly Ala Glu Thr Leu Arg Ala Phe Arg Ala Glu
                165                 170                 175

Asp Thr Glu Leu Lys Arg Ile Asp Ala Ala Ser Gly Glu Ala Arg Asp
            180                 185                 190

Ile Ser Ile Ser Val Phe Arg Phe Leu Thr Trp Ala Phe Ser Arg Asn
        195                 200                 205

Asn Arg Ala Glu Cys Ile Thr Leu Val Leu Ile Leu Gly Thr Gly Phe
    210                 215                 220

Tyr Leu Val Asn Ile Asp Leu Val Thr Val Gly Ala Val Ser Thr Ala
225                 230                 235                 240

Ala Leu Ile Phe His Arg Leu Phe Gly Pro Ile Gly Thr Leu Val Gly
                245                 250                 255

Met Phe Ser Asp Ile Gln Ser Ala Ser Ala Ser Leu Ile Arg Met Val
            260                 265                 270

Gly Val Ile Asn Ala Ala Ser Asn Gln Val Ser Gly Thr Ser Pro Ala
        275                 280                 285

Ser Ala Ser Thr Ala Leu Thr Leu Phe Asp Val Ser His His Tyr His

290                 295                 300

Thr Ala Pro Val Ile Lys Asn Ala Ser Val Gln Leu Glu Pro Gly Glu
305                 310                 315                 320

His Ile Ala Ile Val Gly Ala Thr Gly Ala Gly Lys Ser Thr Leu Ala
                325                 330                 335

Leu Ile Ala Ala Gly Leu Leu Ser Pro Thr Ser Gly Gln Val Ala Leu
            340                 345                 350

Gly Gly Ser Ser Phe Ser Asn Val Glu Pro Glu Ala Leu Arg Gln Lys
        355                 360                 365

Ile Ala Met Val Ser Gln Glu Ile His Cys Phe Arg Gly Ser Val Leu
    370                 375                 380

Asp Asn Leu Arg Ile Ala Arg Pro Glu Ala Thr Asp Ala Asp Ile His
385                 390                 395                 400

Ala Val Leu Ala Asp Ile Gly Asp Ser Trp Leu Glu Arg Leu Pro Gln
                405                 410                 415

Gly Ile Asp Thr Ile Val Gly Asp Gly Ala Phe Arg Leu Thr Ser Val
            420                 425                 430

Glu Asn Gln Ile Met Ala Leu Ala Arg Val His Leu Ala Asp Leu Ala
        435                 440                 445

Ile Val Ile Leu Asp Glu Ala Thr Ala Glu Ser Gly Ser Asp His Ala
    450                 455                 460

Lys Gln Leu Glu Asp Ala Ala Leu Lys Val Thr Glu Asn Arg Ser Ala
465                 470                 475                 480

Ile Ile Val Ala His Arg Leu Asn Gln Ala Lys Thr Ala Asp Arg Ile
                485                 490                 495

Ile Val Met Asp Ser Gly Glu Ile Ile Glu Ser Gly Thr His Glu Glu
            500                 505                 510

Leu Arg Ala Ile Gly Gly Arg Tyr Glu Gln Leu Trp Thr Ala Trp Ser
        515                 520                 525

Ala Arg
    530


<210> 345
<211> 1430
<212> DNA
<213> Corynebacterium glutamicum

<220>
<221> CDS
<222> (1)..(1407)
<223> FRXA01191

<400> 345
gtg agt ttg gat gcg aac acg att gaa acg gcg ggg cgc ggc gac gtg      48
Val Ser Leu Asp Ala Asn Thr Ile Glu Thr Ala Gly Arg Gly Asp Val

1                 5                   10                  15

att tcg cgt atc gcg gat gat tcg cgg gag gtg tcc act gcg gcg agc    96
Ile Ser Arg Ile Ala Asp Asp Ser Arg Glu Val Ser Thr Ala Ala Ser
            20                  25                  30

acc gtg gtg ccg ctg atg gtg cag gcg ggc ttt acc gtg gtg att tcc    144
Thr Val Val Pro Leu Met Val Gln Ala Gly Phe Thr Val Val Ile Ser
        35                  40                  45

gcg ttt ggc atg gcg gcg gtt gat tgg cgc ctc ggc ctt gtc ggt ttg    192
Ala Phe Gly Met Ala Ala Val Asp Trp Arg Leu Gly Leu Val Gly Leu
    50                  55                  60

gtc gcg atc ccg ctg tat tgg acc acg ttg cgc gtc tat tta ccc cgc    240
Val Ala Ile Pro Leu Tyr Trp Thr Thr Leu Arg Val Tyr Leu Pro Arg
65                  70                  75                  80

tca ggt ccg ctt tat acg cgt gag cgc gag gcc ttt ggg gtg cgc acg    288
Ser Gly Pro Leu Tyr Thr Arg Glu Arg Glu Ala Phe Gly Val Arg Thr
                85                  90                  95

cag cgg ctt gtc ggc gca gtc gaa ggc gcg gaa acc ttg cgc gct ttc    336
Gln Arg Leu Val Gly Ala Val Glu Gly Ala Glu Thr Leu Arg Ala Phe
            100                 105                 110

cgc gca gaa gat aca gaa tta aag cgt atc gac gca gcc tcc ggc gaa    384
Arg Ala Glu Asp Thr Glu Leu Lys Arg Ile Asp Ala Ala Ser Gly Glu
        115                 120                 125

gcc cgc gac att tcc att tct gtt ttc agg ttc ctc aca tgg gca ttt    432
Ala Arg Asp Ile Ser Ile Ser Val Phe Arg Phe Leu Thr Trp Ala Phe
    130                 135                 140

tcc cgc aac aac cgc gcg gaa tgc atc acc ctc gtg ctc atc ttg ggc    480
Ser Arg Asn Asn Arg Ala Glu Cys Ile Thr Leu Val Leu Ile Leu Gly
145                 150                 155                 160

acc ggc ttt tac ctg gtc aac atc gat ctg gtc acc gtc ggc gca gtc    528
Thr Gly Phe Tyr Leu Val Asn Ile Asp Leu Val Thr Val Gly Ala Val
                165                 170                 175

tca acc gcc gca ctg atc ttc cac cga ctc ttc ggt cca atc ggc acg    576
Ser Thr Ala Ala Leu Ile Phe His Arg Leu Phe Gly Pro Ile Gly Thr
            180                 185                 190

ctc gtg ggc atg ttc tcc gac atc caa tcc gcc agc gca tcg ctg atc    624
Leu Val Gly Met Phe Ser Asp Ile Gln Ser Ala Ser Ala Ser Leu Ile
        195                 200                 205

cgc atg gtg ggc gtt att aac gcg gca tcg aac cag gtc agc ggc acc    672
Arg Met Val Gly Val Ile Asn Ala Ala Ser Asn Gln Val Ser Gly Thr
    210                 215                 220

tcg ccg gcg tct gcc agc acc gct tta acg ctt ttc gac gtc tcc cac    720
Ser Pro Ala Ser Ala Ser Thr Ala Leu Thr Leu Phe Asp Val Ser His
225                 230                 235                 240

cac tat cac act gca ccc gtc atc aag aat gca tcc gtg cag ctg gaa    768
His Tyr His Thr Ala Pro Val Ile Lys Asn Ala Ser Val Gln Leu Glu
                245                 250                 255

cca ggg gaa cac atc gcc att gtg ggt gcg acc ggc gct ggt aaa agc     816
Pro Gly Glu His Ile Ala Ile Val Gly Ala Thr Gly Ala Gly Lys Ser
            260                 265                 270

acg ctc gcc ctc att gcg gca ggc ctg ctc agc cca act tcc ggg cag     864
Thr Leu Ala Leu Ile Ala Ala Gly Leu Leu Ser Pro Thr Ser Gly Gln
        275                 280                 285

gtg gct ctc ggc gga tcg agt ttt tct aac gtc gaa ccg gaa gca ttg     912
Val Ala Leu Gly Gly Ser Ser Phe Ser Asn Val Glu Pro Glu Ala Leu
    290                 295                 300

cgc cag aag atc gcg atg gtc agc caa gaa atc cac tgc ttc cga gga     960
Arg Gln Lys Ile Ala Met Val Ser Gln Glu Ile His Cys Phe Arg Gly
305                 310                 315                 320

tct gtt tta gat aat ctt cgt atc gca cgc ccc gaa gcc acc gat gcg    1008
Ser Val Leu Asp Asn Leu Arg Ile Ala Arg Pro Glu Ala Thr Asp Ala
                325                 330                 335

gac atc cac gcc gtt ctc gcc gat att ggt gat tcc tgg ttg gag cgc    1056
Asp Ile His Ala Val Leu Ala Asp Ile Gly Asp Ser Trp Leu Glu Arg
            340                 345                 350

tta ccg caa ggc ata gac acc atc gtg ggt gat ggc gct ttc cgt tta    1104
Leu Pro Gln Gly Ile Asp Thr Ile Val Gly Asp Gly Ala Phe Arg Leu
        355                 360                 365

acc tct gtg gaa aac cag atc atg gcg ctt gct cgc gta cat ttg gcc    1152
Thr Ser Val Glu Asn Gln Ile Met Ala Leu Ala Arg Val His Leu Ala
    370                 375                 380

gac cta gca atc gtc atc ctt gat gaa gca acg gct gaa tca ggc tct    1200
Asp Leu Ala Ile Val Ile Leu Asp Glu Ala Thr Ala Glu Ser Gly Ser
385                 390                 395                 400

gat cat gca aaa cag ctt gaa gat gca gcc ctt aaa gtc act gaa aac    1248
Asp His Ala Lys Gln Leu Glu Asp Ala Ala Leu Lys Val Thr Glu Asn
                405                 410                 415

aga tca gcc atc atc gtg gct cac cgc ctc aac caa gcg aaa acc gcc    1296
Arg Ser Ala Ile Ile Val Ala His Arg Leu Asn Gln Ala Lys Thr Ala
            420                 425                 430

gat cgc atc atc gtc atg gac tcc gga gaa atc ata gaa tct gga acc    1344
Asp Arg Ile Ile Val Met Asp Ser Gly Glu Ile Ile Glu Ser Gly Thr
        435                 440                 445

cat gaa gag ctt cga gcg atc ggc ggt cga tat gaa caa ctg tgg act    1392
His Glu Glu Leu Arg Ala Ile Gly Gly Arg Tyr Glu Gln Leu Trp Thr
    450                 455                 460

gcg tgg tct gcg cgc taattagcca cccaagacca cgc                      1430
Ala Trp Ser Ala Arg
465

<210> 346
<211> 469
<212> PRT

<213> Corynebacterium glutamicum

<400> 346
Val Ser Leu Asp Ala Asn Thr Ile Glu Thr Ala Gly Arg Gly Asp Val
1               5                   10                  15

Ile Ser Arg Ile Ala Asp Asp Ser Arg Glu Val Ser Thr Ala Ala Ser
            20                  25                  30

Thr Val Val Pro Leu Met Val Gln Ala Gly Phe Thr Val Val Ile Ser
        35                  40                  45

Ala Phe Gly Met Ala Ala Val Asp Trp Arg Leu Gly Leu Val Gly Leu
    50                  55                  60

Val Ala Ile Pro Leu Tyr Trp Thr Thr Leu Arg Val Tyr Leu Pro Arg
65                  70                  75                  80

Ser Gly Pro Leu Tyr Thr Arg Glu Arg Glu Ala Phe Gly Val Arg Thr
                85                  90                  95

Gln Arg Leu Val Gly Ala Val Glu Gly Ala Glu Thr Leu Arg Ala Phe
            100                 105                 110

Arg Ala Glu Asp Thr Glu Leu Lys Arg Ile Asp Ala Ala Ser Gly Glu
        115                 120                 125

Ala Arg Asp Ile Ser Ile Ser Val Phe Arg Phe Leu Thr Trp Ala Phe
    130                 135                 140

Ser Arg Asn Asn Arg Ala Glu Cys Ile Thr Leu Val Leu Ile Leu Gly
145                 150                 155                 160

Thr Gly Phe Tyr Leu Val Asn Ile Asp Leu Val Thr Val Gly Ala Val
                165                 170                 175

Ser Thr Ala Ala Leu Ile Phe His Arg Leu Phe Gly Pro Ile Gly Thr
            180                 185                 190

Leu Val Gly Met Phe Ser Asp Ile Gln Ser Ala Ser Ala Ser Leu Ile
        195                 200                 205

Arg Met Val Gly Val Ile Asn Ala Ala Ser Asn Gln Val Ser Gly Thr
    210                 215                 220

Ser Pro Ala Ser Ala Ser Thr Ala Leu Thr Leu Phe Asp Val Ser His
225                 230                 235                 240

His Tyr His Thr Ala Pro Val Ile Lys Asn Ala Ser Val Gln Leu Glu
                245                 250                 255

Pro Gly Glu His Ile Ala Ile Val Gly Ala Thr Gly Ala Gly Lys Ser
            260                 265                 270

Thr Leu Ala Leu Ile Ala Ala Gly Leu Leu Ser Pro Thr Ser Gly Gln
        275                 280                 285

Val Ala Leu Gly Gly Ser Ser Phe Ser Asn Val Glu Pro Glu Ala Leu
    290                 295                 300

Arg Gln Lys Ile Ala Met Val Ser Gln Glu Ile His Cys Phe Arg Gly

305                 310                 315                 320

Ser Val Leu Asp Asn Leu Arg Ile Ala Arg Pro Glu Ala Thr Asp Ala
                325                 330                 335

Asp Ile His Ala Val Leu Ala Asp Ile Gly Asp Ser Trp Leu Glu Arg
            340                 345                 350

Leu Pro Gln Gly Ile Asp Thr Ile Val Gly Asp Gly Ala Phe Arg Leu
        355                 360                 365

Thr Ser Val Glu Asn Gln Ile Met Ala Leu Ala Arg Val His Leu Ala
    370                 375                 380

Asp Leu Ala Ile Val Ile Leu Asp Glu Ala Thr Ala Glu Ser Gly Ser
385                 390                 395                 400

Asp His Ala Lys Gln Leu Glu Asp Ala Ala Leu Lys Val Thr Glu Asn
                405                 410                 415

Arg Ser Ala Ile Ile Val Ala His Arg Leu Asn Gln Ala Lys Thr Ala
            420                 425                 430

Asp Arg Ile Ile Val Met Asp Ser Gly Glu Ile Ile Glu Ser Gly Thr
        435                 440                 445

His Glu Glu Leu Arg Ala Ile Gly Gly Arg Tyr Glu Gln Leu Trp Thr
    450                 455                 460

Ala Trp Ser Ala Arg
465

<210> 347
<211> 1047
<212> DNA
<213> Corynebacterium glutamicum

<220>
<221> CDS
<222> (101)..(1024)
<223> RXN01212

<400> 347
tttagaagcc acatgacata tgtcatgaaa attatgtgca aagtgcagta atactcctga 60

catatggctc taccagcgcc aatgcgaagt aggaagaatt atg cct atg aca acg   115
                                            Met Pro Met Thr Thr
                                            1               5

aca cca gca atc gac gta aca gac ctc gtg aga acc tac ggc gac tac   163
Thr Pro Ala Ile Asp Val Thr Asp Leu Val Arg Thr Tyr Gly Asp Tyr
                10                  15                  20

acc gca gtc aag ggc ctg aat ttc cat gta cag cgc ggt gaa gta ttt   211
Thr Ala Val Lys Gly Leu Asn Phe His Val Gln Arg Gly Glu Val Phe
            25                  30                  35

ggt ctg ctc ggc acc aac ggg gcc ggc aaa acc tcc acc ttg gaa gtc   259
Gly Leu Leu Gly Thr Asn Gly Ala Gly Lys Thr Ser Thr Leu Glu Val
        40                  45                  50

atc gaa gga ctt tcc gca ccc agc tcc ggc acc gtg cgc atc tcc ggg 307
Ile Glu Gly Leu Ser Ala Pro Ser Ser Gly Thr Val Arg Ile Ser Gly
    55                  60                  65

ctt gac ccc gtt gcc gac cgc gcg atc ctg cgc ccc gag ctc ggc atc 355
Leu Asp Pro Val Ala Asp Arg Ala Ile Leu Arg Pro Glu Leu Gly Ile
70                  75                  80                  85

atg ctg caa tca ggc ggc ctg cca tca cag ctc acc gtc gcc gaa acc 403
Met Leu Gln Ser Gly Gly Leu Pro Ser Gln Leu Thr Val Ala Glu Thr
                90                  95                  100

atg gac atg tgg cac ggc acc tgc acg tat ccg cgc gcc att aaa gat 451
Met Asp Met Trp His Gly Thr Cys Thr Tyr Pro Arg Ala Ile Lys Asp
            105                 110                 115

gtg ctt gcc gac gtc gac ctc cta cac cgc gaa aac gtc aag gtc ggc 499
Val Leu Ala Asp Val Asp Leu Leu His Arg Glu Asn Val Lys Val Gly
        120                 125                 130

gcg ctt tcc gga ggc gaa caa cga cgc ctt gat ttg gcc tgc gca ctg 547
Ala Leu Ser Gly Gly Glu Gln Arg Arg Leu Asp Leu Ala Cys Ala Leu
    135                 140                 145

ctt ggc gac ccc tca att ttg ttc ctc gac gaa ccc acc acc ggc ctc 595
Leu Gly Asp Pro Ser Ile Leu Phe Leu Asp Glu Pro Thr Thr Gly Leu
150                 155                 160                 165

gac cca gaa tct agg cgc cac acc tgg caa ctc ctg ctg gac ctg aaa 643
Asp Pro Glu Ser Arg Arg His Thr Trp Gln Leu Leu Leu Asp Leu Lys
                170                 175                 180

cag cgc ggc gtc acc atg atg ctg acc acc cac tac ctg gag gaa gcc 691
Gln Arg Gly Val Thr Met Met Leu Thr Thr His Tyr Leu Glu Glu Ala
            185                 190                 195

gaa ttc ctc tgc gac cgg att gcc atc atg aac gcc ggt gag atc gca 739
Glu Phe Leu Cys Asp Arg Ile Ala Ile Met Asn Ala Gly Glu Ile Ala
        200                 205                 210

gtg gaa ggc acc ttg gat gaa ctg gtg gcc cgc gag aag tcg atc atc 787
Val Glu Gly Thr Leu Asp Glu Leu Val Ala Arg Glu Lys Ser Ile Ile
    215                 220                 225

agt ttc gtg ctg cgt ggc ggg cag gtg gag ttg ccg gtc ttg agt ggg 835
Ser Phe Val Leu Arg Gly Gly Gln Val Glu Leu Pro Val Leu Ser Gly
230                 235                 240                 245

gct gaa atc atc cgc gac aac aac cac gtc cgc atc gcc acc acc acc 883
Ala Glu Ile Ile Arg Asp Asn Asn His Val Arg Ile Ala Thr Thr Thr
                250                 255                 260

ctg cag cag cac acc tta gaa ata ctt acc tgg gct gca gag acc ggg 931
Leu Gln Gln His Thr Leu Glu Ile Leu Thr Trp Ala Ala Glu Thr Gly
            265                 270                 275

atc gcg ctg gaa ggc ttc gct gca aaa ccc gcc acc ttg gaa tcc gta 979
Ile Ala Leu Glu Gly Phe Ala Ala Lys Pro Ala Thr Leu Glu Ser Val
        280                 285                 290

ttc atg gac atc gcc tca ctc gag aac acc tcg ctg caa acc gcc          1024
Phe Met Asp Ile Ala Ser Leu Glu Asn Thr Ser Leu Gln Thr Ala
    295                 300                 305

tagaatcttt aaggagacca caa                                            1047

<210> 348
<211> 308
<212> PRT
<213> Corynebacterium glutamicum

<400> 348
Met Pro Met Thr Thr Thr Pro Ala Ile Asp Val Thr Asp Leu Val Arg
  1               5                  10                  15

Thr Tyr Gly Asp Tyr Thr Ala Val Lys Gly Leu Asn Phe His Val Gln
             20                  25                  30

Arg Gly Glu Val Phe Gly Leu Leu Gly Thr Asn Gly Ala Gly Lys Thr
         35                  40                  45

Ser Thr Leu Glu Val Ile Glu Gly Leu Ser Ala Pro Ser Ser Gly Thr
     50                  55                  60

Val Arg Ile Ser Gly Leu Asp Pro Val Ala Asp Arg Ala Ile Leu Arg
 65                  70                  75                  80

Pro Glu Leu Gly Ile Met Leu Gln Ser Gly Gly Leu Pro Ser Gln Leu
                 85                  90                  95

Thr Val Ala Glu Thr Met Asp Met Trp His Gly Thr Cys Thr Tyr Pro
            100                 105                 110

Arg Ala Ile Lys Asp Val Leu Ala Asp Val Asp Leu Leu His Arg Glu
        115                 120                 125

Asn Val Lys Val Gly Ala Leu Ser Gly Gly Glu Gln Arg Arg Leu Asp
    130                 135                 140

Leu Ala Cys Ala Leu Leu Gly Asp Pro Ser Ile Leu Phe Leu Asp Glu
145                 150                 155                 160

Pro Thr Thr Gly Leu Asp Pro Glu Ser Arg Arg His Thr Trp Gln Leu
                165                 170                 175

Leu Leu Asp Leu Lys Gln Arg Gly Val Thr Met Met Leu Thr Thr His
            180                 185                 190

Tyr Leu Glu Glu Ala Glu Phe Leu Cys Asp Arg Ile Ala Ile Met Asn
        195                 200                 205

Ala Gly Glu Ile Ala Val Glu Gly Thr Leu Asp Glu Leu Val Ala Arg
    210                 215                 220

Glu Lys Ser Ile Ile Ser Phe Val Leu Arg Gly Gly Gln Val Glu Leu
225                 230                 235                 240

Pro Val Leu Ser Gly Ala Glu Ile Ile Arg Asp Asn Asn His Val Arg
                245                 250                 255

Ile Ala Thr Thr Thr Leu Gln Gln His Thr Leu Glu Ile Leu Thr Trp
            260                 265                 270

Ala Ala Glu Thr Gly Ile Ala Leu Glu Gly Phe Ala Ala Lys Pro Ala
        275                 280                 285

Thr Leu Glu Ser Val Phe Met Asp Ile Ala Ser Leu Glu Asn Thr Ser
    290                 295                 300

Leu Gln Thr Ala
305


<210> 349
<211> 813
<212> DNA
<213> Corynebacterium glutamicum

<220>
<221> CDS
<222> (1)..(813)
<223> FRXA01212

<400> 349
   ggc ctg aat ttc cat gta cag cgc ggt gaa gta ttt ggt ctg ctc ggc      48
   Gly Leu Asn Phe His Val Gln Arg Gly Glu Val Phe Gly Leu Leu Gly
     1               5                  10                  15
   acc aac ggg gcc ggc aaa acc tcc acc ttg gaa gtc atc gaa gga ctt      96
   Thr Asn Gly Ala Gly Lys Thr Ser Thr Leu Glu Val Ile Glu Gly Leu
                20                  25                  30
   tcc gca ccc agc tcc ggc acc gtg cgc atc tcc ggg ctt gac ccc gtt     144
   Ser Ala Pro Ser Ser Gly Thr Val Arg Ile Ser Gly Leu Asp Pro Val
            35                  40                  45
   gcc gac cgc gcg atc ctg cgc ccc gag ctc ggc atc atg ctg caa tca     192
   Ala Asp Arg Ala Ile Leu Arg Pro Glu Leu Gly Ile Met Leu Gln Ser
        50                  55                  60
   ggc ggc ctg cca tca cag ctc acc gtc gcc gaa acc atg gac atg tgg     240
   Gly Gly Leu Pro Ser Gln Leu Thr Val Ala Glu Thr Met Asp Met Trp
    65                  70                  75                  80
   cac ggc acc tgc acg tat ccg cgc gcc att aaa gat gtg ctt gcc gac     288
   His Gly Thr Cys Thr Tyr Pro Arg Ala Ile Lys Asp Val Leu Ala Asp
                    85                  90                  95
   gtc gac ctc cta cac cgc gaa aac gtc aag gtc ggc gcg ctt tcc gga     336
   Val Asp Leu Leu His Arg Glu Asn Val Lys Val Gly Ala Leu Ser Gly
               100                 105                 110
   ggc gaa caa cga cgc ctt gat ttg gcc tgc gca ctg ctt ggc gac ccc     384
   Gly Glu Gln Arg Arg Leu Asp Leu Ala Cys Ala Leu Leu Gly Asp Pro
           115                 120                 125
   tca att ttg ttc ctc gac gaa ccc acc acc ggc ctc gac cca gaa tct     432
   Ser Ile Leu Phe Leu Asp Glu Pro Thr Thr Gly Leu Asp Pro Glu Ser
       130                 135                 140
   agg cgc cac acc tgg caa ctc ctg ctg gac ctg aaa cag cgc ggc gtc     480
   Arg Arg His Thr Trp Gln Leu Leu Leu Asp Leu Lys Gln Arg Gly Val
   145                 150                 155                 160
   acc atg atg ctg acc acc cac tac ctg gag gaa gcc gaa ttc ctc tgc     528
   Thr Met Met Leu Thr Thr His Tyr Leu Glu Glu Ala Glu Phe Leu Cys
                   165                 170                 175
   gac cgg att gcc atc atg aac gcc ggt gag atc gca gtg gaa ggc acc     576
   Asp Arg Ile Ala Ile Met Asn Ala Gly Glu Ile Ala Val Glu Gly Thr
               180                 185                 190
   ttg gat gaa ctg gtg gcc cgc gag aag tcg atc atc agt ttc gtg ctg     624
   Leu Asp Glu Leu Val Ala Arg Glu Lys Ser Ile Ile Ser Phe Val Leu
           195                 200                 205
   cgt ggc ggg cag gtg gag ttg ccg gtc ttg agt ggg gct gaa atc atc     672
   Arg Gly Gly Gln Val Glu Leu Pro Val Leu Ser Gly Ala Glu Ile Ile
       210                 215                 220
   cgc gac aac aac cac gtc cgc atc gcc acc acc acc ctg cag cag cac     720
   Arg Asp Asn Asn His Val Arg Ile Ala Thr Thr Thr Leu Gln Gln His
   225                 230                 235                 240
   acc tta gaa ata ctt acc tgg gct gca gag acc ggg atc gcg ctg gaa     768
   Thr Leu Glu Ile Leu Thr Trp Ala Ala Glu Thr Gly Ile Ala Leu Glu
                   245                 250                 255
   ggc ttc gct gca aaa ccc gcc acc ttg gaa tcc gta ttc atg gac         813
   Gly Phe Ala Ala Lys Pro Ala Thr Leu Glu Ser Val Phe Met Asp
               260                 265                 270

<210> 350
<211> 271
<212> PRT
<213> Corynebacterium glutamicum

<400> 350
Gly Leu Asn Phe His Val Gln Arg Gly Glu Val Phe Gly Leu Leu Gly
  1               5                  10                  15

Thr Asn Gly Ala Gly Lys Thr Ser Thr Leu Glu Val Ile Glu Gly Leu
             20                  25                  30

Ser Ala Pro Ser Ser Gly Thr Val Arg Ile Ser Gly Leu Asp Pro Val
         35                  40                  45

Ala Asp Arg Ala Ile Leu Arg Pro Glu Leu Gly Ile Met Leu Gln Ser
     50                  55                  60

Gly Gly Leu Pro Ser Gln Leu Thr Val Ala Glu Thr Met Asp Met Trp
 65                  70                  75                  80

His Gly Thr Cys Thr Tyr Pro Arg Ala Ile Lys Asp Val Leu Ala Asp
                 85                  90                  95

Val Asp Leu Leu His Arg Glu Asn Val Lys Val Gly Ala Leu Ser Gly
            100                 105                 110

Gly Glu Gln Arg Arg Leu Asp Leu Ala Cys Ala Leu Leu Gly Asp Pro
        115                 120                 125

Ser Ile Leu Phe Leu Asp Glu Pro Thr Thr Gly Leu Asp Pro Glu Ser
    130                 135                 140

Arg Arg His Thr Trp Gln Leu Leu Leu Asp Leu Lys Gln Arg Gly Val
145                 150                 155                 160

Thr Met Met Leu Thr Thr His Tyr Leu Glu Glu Ala Glu Phe Leu Cys
                165                 170                 175

Asp Arg Ile Ala Ile Met Asn Ala Gly Glu Ile Ala Val Glu Gly Thr
            180                 185                 190

Leu Asp Glu Leu Val Ala Arg Glu Lys Ser Ile Ile Ser Phe Val Leu
        195                 200                 205

Arg Gly Gly Gln Val Glu Leu Pro Val Leu Ser Gly Ala Glu Ile Ile
    210                 215                 220

Arg Asp Asn Asn His Val Arg Ile Ala Thr Thr Thr Leu Gln Gln His
225                 230                 235                 240

Thr Leu Glu Ile Leu Thr Trp Ala Ala Glu Thr Gly Ile Ala Leu Glu
                245                 250                 255

Gly Phe Ala Ala Lys Pro Ala Thr Leu Glu Ser Val Phe Met Asp
            260                 265                 270


<210> 351
<211> 999
<212> DNA
<213> Corynebacterium glutamicum

<220>
<221> CDS
<222> (101)..(976)
<223> RXA02749

<400> 351
caacctagac ttcggtaaga agtaactttg ctttagttgg tcggcgcatc actttcccta 60

agcgatgcgc cgattacttg tttttgctac aaatttaact atg tca ccc atc cta   115
                                           Met Ser Pro Ile Leu
                                           1               5

aaa gtg cgg gac ctc gtc aaa cgc tat ggc gac acc gtt gcg gtt gac   163
Lys Val Arg Asp Leu Val Lys Arg Tyr Gly Asp Thr Val Ala Val Asp
                10                  15                  20

ggt tta aat ttt gat gtt tca caa ggg gaa att ttt gcc ttt cta ggg   211
Gly Leu Asn Phe Asp Val Ser Gln Gly Glu Ile Phe Ala Phe Leu Gly
            25                  30                  35

gag aac ggc gca gga aaa aca acc acg att tca tgc ctg att ggc att   259
Glu Asn Gly Ala Gly Lys Thr Thr Thr Ile Ser Cys Leu Ile Gly Ile
        40                  45                  50

gat caa gcc acc tct ggg gag atc gaa ctg cag ggt ggc caa gta gat   307
Asp Gln Ala Thr Ser Gly Glu Ile Glu Leu Gln Gly Gly Gln Val Asp
    55                  60                  65

tct gaa aaa ctt gga gtg gtg ttt caa caa tcc gtc tta gac cct ttg 355
Ser Glu Lys Leu Gly Val Val Phe Gln Gln Ser Val Leu Asp Pro Leu
70                  75                  80                  85

ctg agt gcc aaa gaa aac ttg gaa aca cgc gga cag ctg tac cca ggg 403
Leu Ser Ala Lys Glu Asn Leu Glu Thr Arg Gly Gln Leu Tyr Pro Gly
                90                  95                  100

gtg ggg aag cag cgg gtt gca cag ctc att gag caa atc ggg atg gaa 451
Val Gly Lys Gln Arg Val Ala Gln Leu Ile Glu Gln Ile Gly Met Glu
            105                 110                 115

ggg ttt gcg gac cgc cga tac gga gtg ttg tcg ggc ggt gaa aaa cgt 499
Gly Phe Ala Asp Arg Arg Tyr Gly Val Leu Ser Gly Gly Glu Lys Arg
        120                 125                 130

cgc acc gac atc gca cga gct tta ctg cac agc cca gac att ctt ttt 547
Arg Thr Asp Ile Ala Arg Ala Leu Leu His Ser Pro Asp Ile Leu Phe
    135                 140                 145

ctt gat gaa ccc aca gca ggc ctc gac ccc aga tca cga cgc caa gtt 595
Leu Asp Glu Pro Thr Ala Gly Leu Asp Pro Arg Ser Arg Arg Gln Val
150                 155                 160                 165

tgg gac acc atc aat tcc ctg cgt aac gat gtg ggc ctc act gtc ttt 643
Trp Asp Thr Ile Asn Ser Leu Arg Asn Asp Val Gly Leu Thr Val Phe
                170                 175                 180

ttg acc act cac tac atg gaa gaa aca gaa ctg gct gat tca gtt cta 691
Leu Thr Thr His Tyr Met Glu Glu Thr Glu Leu Ala Asp Ser Val Leu
            185                 190                 195

atc att gac cgt ggc aaa gag gtc gca tca gga acc ccg atg gaa ctg 739
Ile Ile Asp Arg Gly Lys Glu Val Ala Ser Gly Thr Pro Met Glu Leu
        200                 205                 210

cgc gcc cgt tac acc aca aca gaa ttg act ctt aga aca aac gac cct 787
Arg Ala Arg Tyr Thr Thr Thr Glu Leu Thr Leu Arg Thr Asn Asp Pro
    215                 220                 225

act cat tcg ggt aaa gag ttg gcc cac ttg agc cca gaa atc gac ggt 835
Thr His Ser Gly Lys Glu Leu Ala His Leu Ser Pro Glu Ile Asp Gly
230                 235                 240                 245

gac cga ctg cgg atc aag ttg gaa aat ggg ctc gaa gct gcg cgc ctg 883
Asp Arg Leu Arg Ile Lys Leu Glu Asn Gly Leu Glu Ala Ala Arg Leu
                250                 255                 260

gca aca gaa cta gat ggg gtt ctc gac gta gag atc cgc cac ggt tcc 931
Ala Thr Glu Leu Asp Gly Val Leu Asp Val Glu Ile Arg His Gly Ser
            265                 270                 275

atg gac gat gta ttt cta gca gtt aca gct gaa cgg aaa cga tca 976
Met Asp Asp Val Phe Leu Ala Val Thr Ala Glu Arg Lys Arg Ser
        280                 285                 290

tgattacagt tctgacacgc aga 999

<210> 352

<211> 292
<212> PRT
<213> Corynebacterium glutamicum

<400> 352
Met Ser Pro Ile Leu Lys Val Arg Asp Leu Val Lys Arg Tyr Gly Asp
1               5                   10                  15

Thr Val Ala Val Asp Gly Leu Asn Phe Asp Val Ser Gln Gly Glu Ile
            20                  25                  30

Phe Ala Phe Leu Gly Glu Asn Gly Ala Gly Lys Thr Thr Thr Ile Ser
        35                  40                  45

Cys Leu Ile Gly Ile Asp Gln Ala Thr Ser Gly Glu Ile Glu Leu Gln
    50                  55                  60

Gly Gly Gln Val Asp Ser Glu Lys Leu Gly Val Val Phe Gln Gln Ser
65                  70                  75                  80

Val Leu Asp Pro Leu Leu Ser Ala Lys Glu Asn Leu Glu Thr Arg Gly
                85                  90                  95

Gln Leu Tyr Pro Gly Val Gly Lys Gln Arg Val Ala Gln Leu Ile Glu
            100                 105                 110

Gln Ile Gly Met Glu Gly Phe Ala Asp Arg Arg Tyr Gly Val Leu Ser
        115                 120                 125

Gly Gly Glu Lys Arg Arg Thr Asp Ile Ala Arg Ala Leu Leu His Ser
    130                 135                 140

Pro Asp Ile Leu Phe Leu Asp Glu Pro Thr Ala Gly Leu Asp Pro Arg
145                 150                 155                 160

Ser Arg Arg Gln Val Trp Asp Thr Ile Asn Ser Leu Arg Asn Asp Val
                165                 170                 175

Gly Leu Thr Val Phe Leu Thr Thr His Tyr Met Glu Glu Thr Glu Leu
            180                 185                 190

Ala Asp Ser Val Leu Ile Ile Asp Arg Gly Lys Glu Val Ala Ser Gly
        195                 200                 205

Thr Pro Met Glu Leu Arg Ala Arg Tyr Thr Thr Thr Glu Leu Thr Leu
    210                 215                 220

Arg Thr Asn Asp Pro Thr His Ser Gly Lys Glu Leu Ala His Leu Ser
225                 230                 235                 240

Pro Glu Ile Asp Gly Asp Arg Leu Arg Ile Lys Leu Glu Asn Gly Leu
                245                 250                 255

Glu Ala Ala Arg Leu Ala Thr Glu Leu Asp Gly Val Leu Asp Val Glu
            260                 265                 270

Ile Arg His Gly Ser Met Asp Asp Val Phe Leu Ala Val Thr Ala Glu
        275                 280                 285

Arg Lys Arg Ser
    290

<210> 353
<211> 1920
<212> DNA
<213> Corynebacterium glutamicum

<220>
<221> CDS
<222> (101)..(1897)
<223> RXA02224

<400> 353
gcttcgtcga ggccggaaaa ccgtcgtcat tacgtcgaac ccgacgtggc acggcgtcgc 60

aaagcagatg caatctgatt tttcggaagg ggtgaagtag atg gcg cag cat gag 115
Met Ala Gln His Glu
1               5

cgc gtt gcg gat gcg ctg cag ccg gcg tcg ttg gcg gag tcg tgg cgt 163
Arg Val Ala Asp Ala Leu Gln Pro Ala Ser Leu Ala Glu Ser Trp Arg
                10                  15                  20

gag ctg aaa acg atg cct tcg ggg ccc aag gcc tgg tgg tat gtg agt 211
Glu Leu Lys Thr Met Pro Ser Gly Pro Lys Ala Trp Trp Tyr Val Ser
            25                  30                  35

ttc gtg gtt att agc gtg gtc acg gtc gtg gcg atg gtc ggc acg tcc 259
Phe Val Val Ile Ser Val Val Thr Val Val Ala Met Val Gly Thr Ser
        40                  45                  50

aac ttg ttg ggc tat tcc gtt gat ctg atc aat ggg cag tcg ttg ccg 307
Asn Leu Leu Gly Tyr Ser Val Asp Leu Ile Asn Gly Gln Ser Leu Pro
    55                  60                  65

ctg atc ggt tca gga tcg acc gca atg atc tgg ttg ctt ggt ttg gtg 355
Leu Ile Gly Ser Gly Ser Thr Ala Met Ile Trp Leu Leu Gly Leu Val
70                  75                  80                  85

ggc gct gga att tta gca gaa act gcc ggt cgc gcg ctg ctg caa ttg 403
Gly Ala Gly Ile Leu Ala Glu Thr Ala Gly Arg Ala Leu Leu Gln Leu
                90                  95                  100

gtg atc aac acc ttg gca cgt cgc ctg tcg gtg gat ctg cgg aaa gct 451
Val Ile Asn Thr Leu Ala Arg Arg Leu Ser Val Asp Leu Arg Lys Ala
            105                 110                 115

gcg ctg tct tcg gcg ttg cgt gca ccg gtt cct gat gtc atg gaa ttg 499
Ala Leu Ser Ser Ala Leu Arg Ala Pro Val Pro Asp Val Met Glu Leu
        120                 125                 130

ggc acg gga aac gtg att agc cgc ctg acg caa gac atc gat aac act 547
Gly Thr Gly Asn Val Ile Ser Arg Leu Thr Gln Asp Ile Asp Asn Thr
    135                 140                 145

gtg cgc atc gtc ggc atg gta ggt gtg cgt ttg gtg atc acc att ttg 595
Val Arg Ile Val Gly Met Val Gly Val Arg Leu Val Ile Thr Ile Leu
150                 155                 160                 165

att ctg ccc agc tcc ttg ttc gcg ttg atg acc att cac tgg acc ttt 643
Ile Leu Pro Ser Ser Leu Phe Ala Leu Met Thr Ile His Trp Thr Phe

                170                 175                 180

gtg atc ctg ttc atc gca gtg att gtg gtg ctg att ccc agc ggt cgg 691
Val Ile Leu Phe Ile Ala Val Ile Val Val Leu Ile Pro Ser Gly Arg
            185                 190                 195

aaa gcc gtg cga gct att cct tcg gca aca aat att gtg tcc agt acg 739
Lys Ala Val Arg Ala Ile Pro Ser Ala Thr Asn Ile Val Ser Ser Thr
        200                 205                 210

gag gcg cgt cga aac aat ctg ctc ctc gat acg atc cgt ggc att gaa 787
Glu Ala Arg Arg Asn Asn Leu Leu Leu Asp Thr Ile Arg Gly Ile Glu
    215                 220                 225

aca ctg cgt gtg ctc aag ctc ggt gcg tgg ggt gtg cag cgg atg cgc 835
Thr Leu Arg Val Leu Lys Leu Gly Ala Trp Gly Val Gln Arg Met Arg
230                 235                 240                 245

caa gcg tcg tgg act gcg gtg caa gca aca gct gat cgc gcg ccg att 883
Gln Ala Ser Trp Thr Ala Val Gln Ala Thr Ala Asp Arg Ala Pro Ile
                250                 255                 260

ttc act cgt ctg ctc gcc ctt ggt tcg att gct tat ggc ctg ctg cta 931
Phe Thr Arg Leu Leu Ala Leu Gly Ser Ile Ala Tyr Gly Leu Leu Leu
            265                 270                 275

att ggc gtg ttt ggg ctc agt gcg ttt tgg gtt gcc cag gat gcg atg 979
Ile Gly Val Phe Gly Leu Ser Ala Phe Trp Val Ala Gln Asp Ala Met
        280                 285                 290

agc att gga gcg gca acg gca gca gtt ttc gtg gtt gtg cgc atg gaa 1027
Ser Ile Gly Ala Ala Thr Ala Ala Val Phe Val Val Val Arg Met Glu
    295                 300                 305

att cac gtg ttc aac gtg ctg ttc ttc gca tcg gaa att cag agt gcg 1075
Ile His Val Phe Asn Val Leu Phe Phe Ala Ser Glu Ile Gln Ser Ala
310                 315                 320                 325

tct act tct ctt ggt cgc gcg gtg tcc ctt gcc cag atg gct cgt cgc 1123
Ser Thr Ser Leu Gly Arg Ala Val Ser Leu Ala Gln Met Ala Arg Arg
                330                 335                 340

acc gaa cag ctg tct gag tct gcc gat tgc aca gaa cca ccc tcc gtg 1171
Thr Glu Gln Leu Ser Glu Ser Ala Asp Cys Thr Glu Pro Pro Ser Val
            345                 350                 355

act gtg cag gac gtg acg ttt aaa tat ccc ggc ggc gtg gcc att ttg 1219
Thr Val Gln Asp Val Thr Phe Lys Tyr Pro Gly Gly Val Ala Ile Leu
        360                 365                 370

gag gat ttc aat ctg gtc ttg gaa gca gga aca acc aca gcg ctg gtc 1267
Glu Asp Phe Asn Leu Val Leu Glu Ala Gly Thr Thr Thr Ala Leu Val
    375                 380                 385

ggt act tct ggt gcg gga aaa tcc acg ctc gcg ggc gtc att gcg ggg 1315
Gly Thr Ser Gly Ala Gly Lys Ser Thr Leu Ala Gly Val Ile Ala Gly
390                 395                 400                 405

ctg cag cgc cct gat tcc ggc gcc gtt ttg gtc ggg ggc atc aac acc 1363
Leu Gln Arg Pro Asp Ser Gly Ala Val Leu Val Gly Gly Ile Asn Thr
                410                 415                 420

gcc acc gtc acc gac acg tgg act acc cgc cag gtt gcg ctg atc agc 1411
Ala Thr Val Thr Asp Thr Trp Thr Thr Arg Gln Val Ala Leu Ile Ser
            425                 430                 435

cag gaa gtc cac ctt ttc gca ggc act ctg gca gag gat ctt cgc atg 1459
Gln Glu Val His Leu Phe Ala Gly Thr Leu Ala Glu Asp Leu Arg Met
        440                 445                 450

gcc aat gcg cac gcc acc gac gcg cag ctc cac gca gcg ctc gag tcg 1507
Ala Asn Ala His Ala Thr Asp Ala Gln Leu His Ala Ala Leu Glu Ser
    455                 460                 465

gtc ggg ctc ggg caa atg aca act gct ttt cga cgt ttc ttt cca tcc 1555
Val Gly Leu Gly Gln Met Thr Thr Ala Phe Arg Arg Phe Phe Pro Ser
470                 475                 480                 485

gga tta gac acc aaa att ggc gcc ggc gca gaa gaa ctc acc cct gaa 1603
Gly Leu Asp Thr Lys Ile Gly Ala Gly Ala Glu Glu Leu Thr Pro Glu
                490                 495                 500

atc caa cag caa atc tct ctt gcc cgc atc gtg ctc cgc aat cca cct 1651
Ile Gln Gln Gln Ile Ser Leu Ala Arg Ile Val Leu Arg Asn Pro Pro
            505                 510                 515

gtg ttg atc atg gat gaa gcc acc agt gaa gcc ggc agc gat gat gcc 1699
Val Leu Ile Met Asp Glu Ala Thr Ser Glu Ala Gly Ser Asp Asp Ala
        520                 525                 530

cgc atg ttg gaa aaa gcc gcc aca gaa atc gca cga aac cgc acc acc 1747
Arg Met Leu Glu Lys Ala Ala Thr Glu Ile Ala Arg Asn Arg Thr Thr
    535                 540                 545

ttg gtt gtt gcg cac cgc ctt gac caa gca gtt gtc gca gat cgc atc 1795
Leu Val Val Ala His Arg Leu Asp Gln Ala Val Val Ala Asp Arg Ile
550                 555                 560                 565

atc gtg atg gaa caa ggc aca atc acc gaa gac ggc act cac cag gaa 1843
Ile Val Met Glu Gln Gly Thr Ile Thr Glu Asp Gly Thr His Gln Glu
                570                 575                 580

tta ctt gct ttt gag ggc cgc tac gcg cag ctg tat caa cga tgg agt 1891
Leu Leu Ala Phe Glu Gly Arg Tyr Ala Gln Leu Tyr Gln Arg Trp Ser
            585                 590                 595

gct caa tagttcaaat ccaccacaaa ctc 1920
Ala Gln

<210> 354
<211> 599
<212> PRT
<213> Corynebacterium glutamicum

<400> 354
Met Ala Gln His Glu Arg Val Ala Asp Ala Leu Gln Pro Ala Ser Leu
1               5                   10                  15

Ala Glu Ser Trp Arg Glu Leu Lys Thr Met Pro Ser Gly Pro Lys Ala
            20                  25                  30

Trp Trp Tyr Val Ser Phe Val Val Ile Ser Val Val Thr Val Val Ala
        35                  40                  45

Met Val Gly Thr Ser Asn Leu Leu Gly Tyr Ser Val Asp Leu Ile Asn
    50                  55                  60

Gly Gln Ser Leu Pro Leu Ile Gly Ser Gly Ser Thr Ala Met Ile Trp
65                  70                  75                  80

Leu Leu Gly Leu Val Gly Ala Gly Ile Leu Ala Glu Thr Ala Gly Arg
                85                  90                  95

Ala Leu Leu Gln Leu Val Ile Asn Thr Leu Ala Arg Arg Leu Ser Val
            100                 105                 110

Asp Leu Arg Lys Ala Ala Leu Ser Ser Ala Leu Arg Ala Pro Val Pro
        115                 120                 125

Asp Val Met Glu Leu Gly Thr Gly Asn Val Ile Ser Arg Leu Thr Gln
    130                 135                 140

Asp Ile Asp Asn Thr Val Arg Ile Val Gly Met Val Gly Val Arg Leu
145                 150                 155                 160

Val Ile Thr Ile Leu Ile Leu Pro Ser Ser Leu Phe Ala Leu Met Thr
                165                 170                 175

Ile His Trp Thr Phe Val Ile Leu Phe Ile Ala Val Ile Val Val Leu
            180                 185                 190

Ile Pro Ser Gly Arg Lys Ala Val Arg Ala Ile Pro Ser Ala Thr Asn
        195                 200                 205

Ile Val Ser Ser Thr Glu Ala Arg Arg Asn Asn Leu Leu Leu Asp Thr
    210                 215                 220

Ile Arg Gly Ile Glu Thr Leu Arg Val Leu Lys Leu Gly Ala Trp Gly
225                 230                 235                 240

Val Gln Arg Met Arg Gln Ala Ser Trp Thr Ala Val Gln Ala Thr Ala
                245                 250                 255

Asp Arg Ala Pro Ile Phe Thr Arg Leu Leu Ala Leu Gly Ser Ile Ala
            260                 265                 270

Tyr Gly Leu Leu Leu Ile Gly Val Phe Gly Leu Ser Ala Phe Trp Val
        275                 280                 285

Ala Gln Asp Ala Met Ser Ile Gly Ala Ala Thr Ala Ala Val Phe Val
    290                 295                 300

Val Val Arg Met Glu Ile His Val Phe Asn Val Leu Phe Phe Ala Ser
305                 310                 315                 320

Glu Ile Gln Ser Ala Ser Thr Ser Leu Gly Arg Ala Val Ser Leu Ala
                325                 330                 335

Gln Met Ala Arg Arg Thr Glu Gln Leu Ser Glu Ser Ala Asp Cys Thr
            340                 345                 350

Glu Pro Pro Ser Val Thr Val Gln Asp Val Thr Phe Lys Tyr Pro Gly
        355                 360                 365

Gly Val Ala Ile Leu Glu Asp Phe Asn Leu Val Leu Glu Ala Gly Thr
    370                 375                 380

Thr Thr Ala Leu Val Gly Thr Ser Gly Ala Gly Lys Ser Thr Leu Ala
385                 390                 395                 400

Gly Val Ile Ala Gly Leu Gln Arg Pro Asp Ser Gly Ala Val Leu Val
                405                 410                 415

Gly Gly Ile Asn Thr Ala Thr Val Thr Asp Thr Trp Thr Thr Arg Gln
            420                 425                 430

Val Ala Leu Ile Ser Gln Glu Val His Leu Phe Ala Gly Thr Leu Ala
        435                 440                 445

Glu Asp Leu Arg Met Ala Asn Ala His Ala Thr Asp Ala Gln Leu His
    450                 455                 460

Ala Ala Leu Glu Ser Val Gly Leu Gly Gln Met Thr Thr Ala Phe Arg
465                 470                 475                 480

Arg Phe Phe Pro Ser Gly Leu Asp Thr Lys Ile Gly Ala Gly Ala Glu
                485                 490                 495

Glu Leu Thr Pro Glu Ile Gln Gln Gln Ile Ser Leu Ala Arg Ile Val
            500                 505                 510

Leu Arg Asn Pro Pro Val Leu Ile Met Asp Glu Ala Thr Ser Glu Ala
        515                 520                 525

Gly Ser Asp Asp Ala Arg Met Leu Glu Lys Ala Ala Thr Glu Ile Ala
    530                 535                 540

Arg Asn Arg Thr Thr Leu Val Val Ala His Arg Leu Asp Gln Ala Val
545                 550                 555                 560

Val Ala Asp Arg Ile Ile Val Met Glu Gln Gly Thr Ile Thr Glu Asp
                565                 570                 575

Gly Thr His Gln Glu Leu Leu Ala Phe Glu Gly Arg Tyr Ala Gln Leu
            580                 585                 590

Tyr Gln Arg Trp Ser Ala Gln
        595

<210> 355
<211> 1653
<212> DNA
<213> Corynebacterium glutamicum

<220>
<221> CDS
<222> (101)..(1630)
<223> RXN01602

<400> 355
tgcaggccac atgcctccca gtgccgtctc tgcacgttga ttttcccctg ccacgactgg 60

tcgcagggcg actttctagc acttttaaag gaatttttta atg gct aaa acc cat 115
Met Ala Lys Thr His
1               5

att cgg tta cag gac ctt tcc ctg tca tac acc tca acc ccg tta att 163
Ile Arg Leu Gln Asp Leu Ser Leu Ser Tyr Thr Ser Thr Pro Leu Ile
                10                  15                  20

acg aag ctc aat atc act gtt tct tct gga cag tgc gca gtg att gtt 211
Thr Lys Leu Asn Ile Thr Val Ser Ser Gly Gln Cys Ala Val Ile Val
            25                  30                  35

ggt gag aat ggt cga ggt aaa acc aca ctt ctg cga gca ctg gct cga 259
Gly Glu Asn Gly Arg Gly Lys Thr Thr Leu Leu Arg Ala Leu Ala Arg
        40                  45                  50

gaa ttc ccg cca tct gca ggt gag att ctc act cat ggc acg gta gca 307
Glu Phe Pro Pro Ser Ala Gly Glu Ile Leu Thr His Gly Thr Val Ala
    55                  60                  65

att gct cat caa cac atg cct gca ggt gat ctg tcc gtc gga gag atc 355
Ile Ala His Gln His Met Pro Ala Gly Asp Leu Ser Val Gly Glu Ile
70                  75                  80                  85

tgt gat gag gca att cgt gat tca aag aat gct ctc gaa gag ctt gag 403
Cys Asp Glu Ala Ile Arg Asp Ser Lys Asn Ala Leu Glu Glu Leu Glu
                90                  95                  100

aga gct gga gct cta ctt gag aca aac act gcg cac gca ctt gat gga 451
Arg Ala Gly Ala Leu Leu Glu Thr Asn Thr Ala His Ala Leu Asp Gly
            105                 110                 115

tat caa caa gcc ctt gat gcc gct gaa gtg ctt gac gca tgg aac gct 499
Tyr Gln Gln Ala Leu Asp Ala Ala Glu Val Leu Asp Ala Trp Asn Ala
        120                 125                 130

gaa cat cga tta gaa aaa gct ctg cgc agc ttt ggc gcg atc acc gat 547
Glu His Arg Leu Glu Lys Ala Leu Arg Ser Phe Gly Ala Ile Thr Asp
    135                 140                 145

aga tcc cgt gca ctc agt gag cta tcg atc ggg caa agg tat cgg gta 595
Arg Ser Arg Ala Leu Ser Glu Leu Ser Ile Gly Gln Arg Tyr Arg Val
150                 155                 160                 165

cgg ctg gcc tgc ctc atc ggt ggc gat gct gat att ttg ctt ctc gat 643
Arg Leu Ala Cys Leu Ile Gly Gly Asp Ala Asp Ile Leu Leu Leu Asp
                170                 175                 180

gaa ccc acc aat cat ctt gac cgg ggc gcg ctt aac tat ctc acc gaa 691
Glu Pro Thr Asn His Leu Asp Arg Gly Ala Leu Asn Tyr Leu Thr Glu
            185                 190                 195

gcc ata acc tcc cac aaa ggt gtg gta ctt gtt gtt tct cat gat caa 739
Ala Ile Thr Ser His Lys Gly Val Val Leu Val Val Ser His Asp Gln
        200                 205                 210

gca ctg atc aaa gat gtc gcg gat ttc atc atc gat att gat tca acc 787
Ala Leu Ile Lys Asp Val Ala Asp Phe Ile Ile Asp Ile Asp Ser Thr
    215                 220                 225

cca gac ggc cta cca cgg atc tat cat gag ggt ttt gat tct tat cga    835
Pro Asp Gly Leu Pro Arg Ile Tyr His Glu Gly Phe Asp Ser Tyr Arg
230                 235                 240                 245

cgc caa agg agt gcg ctt ctt gaa act tgg agg cag gat tat gcc gct    883
Arg Gln Arg Ser Ala Leu Leu Glu Thr Trp Arg Gln Asp Tyr Ala Ala
                250                 255                 260

gca caa act gtg caa cag caa ttg cag gag gat cta gag cac gca cgc    931
Ala Gln Thr Val Gln Gln Gln Leu Gln Glu Asp Leu Glu His Ala Arg
            265                 270                 275

cag cgg gtg aat tct tcg tgg aaa cct cca aaa gga acg gga aaa cac    979
Gln Arg Val Asn Ser Ser Trp Lys Pro Pro Lys Gly Thr Gly Lys His
        280                 285                 290

act cgc gca tct cgg gct ccc gga gtg gtg cag gcc tta aag cga gca    1027
Thr Arg Ala Ser Arg Ala Pro Gly Val Val Gln Ala Leu Lys Arg Ala
    295                 300                 305

cag gat gcg ttg gat agc aaa gcg ttg gac gtt ccc ccg gct ccg gcc    1075
Gln Asp Ala Leu Asp Ser Lys Ala Leu Asp Val Pro Pro Ala Pro Ala
310                 315                 320                 325

cca ttg ctt ctg cct acc ttg aaa gtg cga cca gat aaa ccc atg gtg    1123
Pro Leu Leu Leu Pro Thr Leu Lys Val Arg Pro Asp Lys Pro Met Val
                330                 335                 340

gac ttt tcg gac ctt ttt gta ccc cac cgc ttg cgt ctg cca ggc tca    1171
Asp Phe Ser Asp Leu Phe Val Pro His Arg Leu Arg Leu Pro Gly Ser
            345                 350                 355

cat tca gtg gta tca ggt gac aaa ata gtg atc act ggt gac aac ggc    1219
His Ser Val Val Ser Gly Asp Lys Ile Val Ile Thr Gly Asp Asn Gly
        360                 365                 370

gct ggc aaa tca acg ctc atc gaa gtc ttg tct ggg gtt ttg act ccg    1267
Ala Gly Lys Ser Thr Leu Ile Glu Val Leu Ser Gly Val Leu Thr Pro
    375                 380                 385

gca agt ggt tcg gtt gca aac cat gcc cga act ggg gtt ctc ggc caa    1315
Ala Ser Gly Ser Val Ala Asn His Ala Arg Thr Gly Val Leu Gly Gln
390                 395                 400                 405

gaa tca ctt gtc ggc gag gtg cca tca ata gca cga gat cac gca gtt    1363
Glu Ser Leu Val Gly Glu Val Pro Ser Ile Ala Arg Asp His Ala Val
                410                 415                 420

aag tgg gga ctt tta agt gtt gag gag agc cga ttt gcc cta cag gaa    1411
Lys Trp Gly Leu Leu Ser Val Glu Glu Ser Arg Phe Ala Leu Gln Glu
            425                 430                 435

ttc tca att ggt caa cgc aga aga cta gat ttg gcc atg tcg tta gct    1459
Phe Ser Ile Gly Gln Arg Arg Arg Leu Asp Leu Ala Met Ser Leu Ala
        440                 445                 450

ggc aat cct gaa ctg ttg ctt ctc gat gaa cct tcg aac cat ctg tct    1507
Gly Asn Pro Glu Leu Leu Leu Leu Asp Glu Pro Ser Asn His Leu Ser
    455                 460                 465

atg cac ttg gtt tcc gca ctt aca gag tgg ctg gac acg acc gcg gct    1555

Met His Leu Val Ser Ala Leu Thr Glu Trp Leu Asp Thr Thr Ala Ala
470                 475                 480                 485

gca gtg atc atg gta acg cat gat cga cag cta ctc cgc gat acg gct     1603
Ala Val Ile Met Val Thr His Asp Arg Gln Leu Leu Arg Asp Thr Ala
                490                 495                 500

cat tgg agg cac atc gag ttg aaa tct taagaattcg caagggcttt           1650
His Trp Arg His Ile Glu Leu Lys Ser  .
            505                 510

cac                                                                 1653

<210> 356
<211> 510
<212> PRT
<213> Corynebacterium glutamicum

<400> 356
Met Ala Lys Thr His Ile Arg Leu Gln Asp Leu Ser Leu Ser Tyr Thr
  1               5                  10                  15

Ser Thr Pro Leu Ile Thr Lys Leu Asn Ile Thr Val Ser Ser Gly Gln
             20                  25                  30

Cys Ala Val Ile Val Gly Glu Asn Gly Arg Gly Lys Thr Thr Leu Leu
         35                  40                  45

Arg Ala Leu Ala Arg Glu Phe Pro Pro Ser Ala Gly Glu Ile Leu Thr
     50                  55                  60

His Gly Thr Val Ala Ile Ala His Gln His Met Pro Ala Gly Asp Leu
 65                  70                  75                  80

Ser Val Gly Glu Ile Cys Asp Glu Ala Ile Arg Asp Ser Lys Asn Ala
                 85                  90                  95

Leu Glu Glu Leu Glu Arg Ala Gly Ala Leu Leu Glu Thr Asn Thr Ala
            100                 105                 110

His Ala Leu Asp Gly Tyr Gln Gln Ala Leu Asp Ala Ala Glu Val Leu
        115                 120                 125

Asp Ala Trp Asn Ala Glu His Arg Leu Glu Lys Ala Leu Arg Ser Phe
    130                 135                 140

Gly Ala Ile Thr Asp Arg Ser Arg Ala Leu Ser Glu Leu Ser Ile Gly
145                 150                 155                 160

Gln Arg Tyr Arg Val Arg Leu Ala Cys Leu Ile Gly Gly Asp Ala Asp
                165                 170                 175

Ile Leu Leu Leu Asp Glu Pro Thr Asn His Leu Asp Arg Gly Ala Leu
            180                 185                 190

Asn Tyr Leu Thr Glu Ala Ile Thr Ser His Lys Gly Val Val Leu Val
        195                 200                 205

Val Ser His Asp Gln Ala Leu Ile Lys Asp Val Ala Asp Phe Ile Ile
    210                 215                 220

Asp Ile Asp Ser Thr Pro Asp Gly Leu Pro Arg Ile Tyr His Glu Gly
225                 230                 235                 240

Phe Asp Ser Tyr Arg Arg Gln Arg Ser Ala Leu Leu Glu Thr Trp Arg
                245                 250                 255

Gln Asp Tyr Ala Ala Ala Gln Thr Val Gln Gln Gln Leu Gln Glu Asp
            260                 265                 270

Leu Glu His Ala Arg Gln Arg Val Asn Ser Ser Trp Lys Pro Pro Lys
        275                 280                 285

Gly Thr Gly Lys His Thr Arg Ala Ser Arg Ala Pro Gly Val Val Gln
    290                 295                 300

Ala Leu Lys Arg Ala Gln Asp Ala Leu Asp Ser Lys Ala Leu Asp Val
305                 310                 315                 320

Pro Pro Ala Pro Ala Pro Leu Leu Leu Pro Thr Leu Lys Val Arg Pro
                325                 330                 335

Asp Lys Pro Met Val Asp Phe Ser Asp Leu Phe Val Pro His Arg Leu
            340                 345                 350

Arg Leu Pro Gly Ser His Ser Val Val Ser Gly Asp Lys Ile Val Ile
        355                 360                 365

Thr Gly Asp Asn Gly Ala Gly Lys Ser Thr Leu Ile Glu Val Leu Ser
    370                 375                 380

Gly Val Leu Thr Pro Ala Ser Gly Ser Val Ala Asn His Ala Arg Thr
385                 390                 395                 400

Gly Val Leu Gly Gln Glu Ser Leu Val Gly Glu Val Pro Ser Ile Ala
                405                 410                 415

Arg Asp His Ala Val Lys Trp Gly Leu Leu Ser Val Glu Glu Ser Arg
            420                 425                 430

Phe Ala Leu Gln Glu Phe Ser Ile Gly Gln Arg Arg Arg Leu Asp Leu
        435                 440                 445

Ala Met Ser Leu Ala Gly Asn Pro Glu Leu Leu Leu Leu Asp Glu Pro
    450                 455                 460

Ser Asn His Leu Ser Met His Leu Val Ser Ala Leu Thr Glu Trp Leu
465                 470                 475                 480

Asp Thr Thr Ala Ala Ala Val Ile Met Val Thr His Asp Arg Gln Leu
                485                 490                 495

Leu Arg Asp Thr Ala His Trp Arg His Ile Glu Leu Lys Ser
            500                 505                 510

<210> 357
<211> 879
<212> DNA
<213> Corynebacterium glutamicum

<220>
<221> CDS
<222> (101)..(856)
<223> RXN02515

<400> 357
gtggctaagc acagttactt ggccaagctg ggcggcagaa aaaccggccc agctaatact 60

tcagtttaaa attcgcttca accctgaaag attgtgacag atg agc act ctt gaa 115
Met Ser Thr Leu Glu
1               5

atc cgt aac ctg cac gca cag gtc ctg ccg tcc gat gag tcc gct gag 163
Ile Arg Asn Leu His Ala Gln Val Leu Pro Ser Asp Glu Ser Ala Glu
                10                  15                  20

cct aag gaa atc ctc aag ggc gtc aac ctc acc atc aac tct ggt gag 211
Pro Lys Glu Ile Leu Lys Gly Val Asn Leu Thr Ile Asn Ser Gly Glu
            25                  30                  35

atc cac gcc atc atg ggc cct aac ggt tcc ggc aag tcc act ctt gct 259
Ile His Ala Ile Met Gly Pro Asn Gly Ser Gly Lys Ser Thr Leu Ala
        40                  45                  50

tac acc ctt ggt gga cac cca cgc tac gag gta acc gca ggc gag gtc 307
Tyr Thr Leu Gly Gly His Pro Arg Tyr Glu Val Thr Ala Gly Glu Val
    55                  60                  65

ctc ctc gac ggc gag aac atc ctg gag atg gaa gtt gat gag cgt gca 355
Leu Leu Asp Gly Glu Asn Ile Leu Glu Met Glu Val Asp Glu Arg Ala
70                  75                  80                  85

cgc gct ggt ctc ttc ctg gcc atg cag tat cca act gaa atc cct ggc 403
Arg Ala Gly Leu Phe Leu Ala Met Gln Tyr Pro Thr Glu Ile Pro Gly
                90                  95                  100

gtt tcc gtt gct aac ttc ctg cgt tcc gca gcg acc gca atc cgc ggc 451
Val Ser Val Ala Asn Phe Leu Arg Ser Ala Ala Thr Ala Ile Arg Gly
            105                 110                 115

gag gct cct aag ctt cgc gag tgg gtt aag gaa gtc cgc acc gct cag 499
Glu Ala Pro Lys Leu Arg Glu Trp Val Lys Glu Val Arg Thr Ala Gln
        120                 125                 130

gaa gct ctg gca att gac cct gag ttc tcc aac cgc tca gtc aac gaa 547
Glu Ala Leu Ala Ile Asp Pro Glu Phe Ser Asn Arg Ser Val Asn Glu
    135                 140                 145

ggt ttc tcc ggt ggc gag aag aag cgc cac gag gtt ctg cag ctt gat 595
Gly Phe Ser Gly Gly Glu Lys Lys Arg His Glu Val Leu Gln Leu Asp
150                 155                 160                 165

ctg ctg aag cca aag ttc gcg atc atg gat gag acc gac tcc ggc ctt 643
Leu Leu Lys Pro Lys Phe Ala Ile Met Asp Glu Thr Asp Ser Gly Leu
                170                 175                 180

gac gtg gat gca ctg cgc att gtt tcc gag ggc atc aac tcc tac aag 691
Asp Val Asp Ala Leu Arg Ile Val Ser Glu Gly Ile Asn Ser Tyr Lys
            185                 190                 195

cag gag acc gaa ggt ggc atc ttg atg atc acc cac tac aag cgc atc 739

Gln Glu Thr Glu Gly Gly Ile Leu Met Ile Thr His Tyr Lys Arg Ile
        200                 205                 210

ctc aac tac gtt aag cct gac ttc att cac gtt ttc gcg aat ggc cag 787
Leu Asn Tyr Val Lys Pro Asp Phe Ile His Val Phe Ala Asn Gly Gln
    215                 220                 225

att gtg acc acc ggt ggc gct gag ctt gct gac aag ctc gag gct gac 835
Ile Val Thr Thr Gly Gly Ala Glu Leu Ala Asp Lys Leu Glu Ala Asp
230                 235                 240                 245

ggc tac gac cag ttc atc aag taacatgtcc gatttcctca atg 879
Gly Tyr Asp Gln Phe Ile Lys
                250

<210> 358
<211> 252
<212> PRT
<213> Corynebacterium glutamicum

<400> 358
Met Ser Thr Leu Glu Ile Arg Asn Leu His Ala Gln Val Leu Pro Ser
1               5                   10                  15

Asp Glu Ser Ala Glu Pro Lys Glu Ile Leu Lys Gly Val Asn Leu Thr
            20                  25                  30

Ile Asn Ser Gly Glu Ile His Ala Ile Met Gly Pro Asn Gly Ser Gly
        35                  40                  45

Lys Ser Thr Leu Ala Tyr Thr Leu Gly Gly His Pro Arg Tyr Glu Val
    50                  55                  60

Thr Ala Gly Glu Val Leu Leu Asp Gly Glu Asn Ile Leu Glu Met Glu
65                  70                  75                  80

Val Asp Glu Arg Ala Arg Ala Gly Leu Phe Leu Ala Met Gln Tyr Pro
                85                  90                  95

Thr Glu Ile Pro Gly Val Ser Val Ala Asn Phe Leu Arg Ser Ala Ala
            100                 105                 110

Thr Ala Ile Arg Gly Glu Ala Pro Lys Leu Arg Glu Trp Val Lys Glu
        115                 120                 125

Val Arg Thr Ala Gln Glu Ala Leu Ala Ile Asp Pro Glu Phe Ser Asn
    130                 135                 140

Arg Ser Val Asn Glu Gly Phe Ser Gly Gly Glu Lys Lys Arg His Glu
145                 150                 155                 160

Val Leu Gln Leu Asp Leu Leu Lys Pro Lys Phe Ala Ile Met Asp Glu
                165                 170                 175

Thr Asp Ser Gly Leu Asp Val Asp Ala Leu Arg Ile Val Ser Glu Gly
            180                 185                 190

Ile Asn Ser Tyr Lys Gln Glu Thr Glu Gly Gly Ile Leu Met Ile Thr
        195                 200                 205

His Tyr Lys Arg Ile Leu Asn Tyr Val Lys Pro Asp Phe Ile His Val
    210                 215                 220

Phe Ala Asn Gly Gln Ile Val Thr Thr Gly Gly Ala Glu Leu Ala Asp
225                 230                 235                 240

Lys Leu Glu Ala Asp Gly Tyr Asp Gln Phe Ile Lys
                245                 250


<210> 359
<211> 1386
<212> DNA
<213> Corynebacterium glutamicum

<220>
<221> CDS
<222> (101)..(1363)
<223> RXN00525

<400> 359
ccatcgtgtt tattactcac aaccctgagc ttgctgatga atctgatcgg gtggtcacca 60

tggttgacgg gcgcatcatt gggtctgagg tgaaacactc atg agc ctt gca gaa   115
                                           Met Ser Leu Ala Glu
                                             1               5

tca att ctt ttg gcg ctc acc agc ctg aga agc aac aag atg cgt gca   163
Ser Ile Leu Leu Ala Leu Thr Ser Leu Arg Ser Asn Lys Met Arg Ala
                10                  15                  20

ttg ttg acg ctg tta gga gtc atc att ggt atc gca tca gtc atc gga   211
Leu Leu Thr Leu Leu Gly Val Ile Ile Gly Ile Ala Ser Val Ile Gly
            25                  30                  35

att ttg acc att ggt aaa gcc ctg cag gat caa act ttg aat agt ttg   259
Ile Leu Thr Ile Gly Lys Ala Leu Gln Asp Gln Thr Leu Asn Ser Leu
        40                  45                  50

gaa agc ttg ggc gcg aat gat ctg tcg gcg cag gtg gag gaa cgc ccc   307
Glu Ser Leu Gly Ala Asn Asp Leu Ser Ala Gln Val Glu Glu Arg Pro
    55                  60                  65

gac gaa gat tcc ccc gaa ccc gat atg ttc gct ttt tct ggg gct gca   355
Asp Glu Asp Ser Pro Glu Pro Asp Met Phe Ala Phe Ser Gly Ala Ala
70                  75                  80                  85

aac tct agt ggc aat ctg att ccg gaa gaa aca gtt gat acg ctg cgc   403
Asn Ser Ser Gly Asn Leu Ile Pro Glu Glu Thr Val Asp Thr Leu Arg
                90                  95                  100

gat cgt ttc gca ggc agc atc acg gga atc agc gtt ggc gga atg ggt   451
Asp Arg Phe Ala Gly Ser Ile Thr Gly Ile Ser Val Gly Gly Met Gly
            105                 110                 115

acg caa ggc act ctc atc ggc gac acc gca gat ctt aaa tcc gat ctc   499
Thr Gln Gly Thr Leu Ile Gly Asp Thr Ala Asp Leu Lys Ser Asp Leu
        120                 125                 130

ctc ggc gtc aac gag gat tat atg tgg atg aat ggc gtc gaa atg aac   547
Leu Gly Val Asn Glu Asp Tyr Met Trp Met Asn Gly Val Glu Met Asn

135                 140                 145

tac ggc cgc gcc atc acg caa gac gat gtt gcc gct cag cgc ccc gtt     595
Tyr Gly Arg Ala Ile Thr Gln Asp Asp Val Ala Ala Gln Arg Pro Val
150                 155                 160                 165

gcg gtc atc gcc cca gac acc ttt aat acg ctt ttc gac gca aac ccc     643
Ala Val Ile Ala Pro Asp Thr Phe Asn Thr Leu Phe Asp Ala Asn Pro
                170                 175                 180

aac ctc gct ctg ggg tcc gaa gta gct ttt gaa ctc aac ggt caa gag     691
Asn Leu Ala Leu Gly Ser Glu Val Ala Phe Glu Leu Asn Gly Gln Glu
            185                 190                 195

aca ttt ttg cgg gtt atc ggt gtg tat aaa gaa gcc gca gca ggt gga     739
Thr Phe Leu Arg Val Ile Gly Val Tyr Lys Glu Ala Ala Ala Gly Gly
        200                 205                 210

ctt gtg gga agc aat cca acc gtc cac acc tac acc cca tat acg gtg     787
Leu Val Gly Ser Asn Pro Thr Val His Thr Tyr Thr Pro Tyr Thr Val
    215                 220                 225

gcc aat gac atc acc cac acg gaa gat gga ttg aac acg tta agt atc     835
Ala Asn Asp Ile Thr His Thr Glu Asp Gly Leu Asn Thr Leu Ser Ile
230                 235                 240                 245

cgt gca gct cag ggc gta gac cag gat tca ctt aag ggt tca ctg caa     883
Arg Ala Ala Gln Gly Val Asp Gln Asp Ser Leu Lys Gly Ser Leu Gln
                250                 255                 260

acc tac ttc gac gcg ctg tac gcc aac aat gac tcg cac cac gtt gcc     931
Thr Tyr Phe Asp Ala Leu Tyr Ala Asn Asn Asp Ser His His Val Ala
            265                 270                 275

atg ttg gac ttc cgt aaa cag atc gaa gag ttc aac acc att ctc ggc     979
Met Leu Asp Phe Arg Lys Gln Ile Glu Glu Phe Asn Thr Ile Leu Gly
        280                 285                 290

gca atg agt ttg ggt atc tca gcc atc ggc gga att tcc ttg ctt gtc     1027
Ala Met Ser Leu Gly Ile Ser Ala Ile Gly Gly Ile Ser Leu Leu Val
    295                 300                 305

ggt ggc atc gga gtg atg aac att atg ttg gtg tct gtc acc gag cga     1075
Gly Gly Ile Gly Val Met Asn Ile Met Leu Val Ser Val Thr Glu Arg
310                 315                 320                 325

acc cgc gaa atc ggt gtc cga aaa gcc ctc ggc gct cgt cga cgt gac     1123
Thr Arg Glu Ile Gly Val Arg Lys Ala Leu Gly Ala Arg Arg Arg Asp
                330                 335                 340

att cgc ctg caa ttc gtc gtt gaa gcc atg atc att tgt ttc atc ggt     1171
Ile Arg Leu Gln Phe Val Val Glu Ala Met Ile Ile Cys Phe Ile Gly
            345                 350                 355

ggc atc ctc ggc gtg ctt ttg ggc ggc att ttg gga ttg atc atg tcc     1219
Gly Ile Leu Gly Val Leu Leu Gly Gly Ile Leu Gly Leu Ile Met Ser
        360                 365                 370

agc gct att ggc tac att tcc ttg cca cca ctg agt gga atc gtg atc     1267
Ser Ala Ile Gly Tyr Ile Ser Leu Pro Pro Leu Ser Gly Ile Val Ile
    375                 380                 385

gcc ttg gta ttt tcc atg gct atc ggc ctg ttt ttc ggc tac tac ccc 1315
Ala Leu Val Phe Ser Met Ala Ile Gly Leu Phe Phe Gly Tyr Tyr Pro
390                 395                 400                 405

gcc aac aag gca gca aag ctc gat cca att gac gcc ttg cgt tat gag 1363
Ala Asn Lys Ala Ala Lys Leu Asp Pro Ile Asp Ala Leu Arg Tyr Glu
                410                 415                 420

taaaagcctc gtttttaagg tag 1386

<210> 360
<211> 421
<212> PRT
<213> Corynebacterium glutamicum

<400> 360
Met Ser Leu Ala Glu Ser Ile Leu Leu Ala Leu Thr Ser Leu Arg Ser
1               5                   10                  15

Asn Lys Met Arg Ala Leu Leu Thr Leu Leu Gly Val Ile Ile Gly Ile
            20                  25                  30

Ala Ser Val Ile Gly Ile Leu Thr Ile Gly Lys Ala Leu Gln Asp Gln
        35                  40                  45

Thr Leu Asn Ser Leu Glu Ser Leu Gly Ala Asn Asp Leu Ser Ala Gln
    50                  55                  60

Val Glu Glu Arg Pro Asp Glu Asp Ser Pro Glu Pro Asp Met Phe Ala
65                  70                  75                  80

Phe Ser Gly Ala Ala Asn Ser Ser Gly Asn Leu Ile Pro Glu Glu Thr
                85                  90                  95

Val Asp Thr Leu Arg Asp Arg Phe Ala Gly Ser Ile Thr Gly Ile Ser
            100                 105                 110

Val Gly Gly Met Gly Thr Gln Gly Thr Leu Ile Gly Asp Thr Ala Asp
        115                 120                 125

Leu Lys Ser Asp Leu Leu Gly Val Asn Glu Asp Tyr Met Trp Met Asn
    130                 135                 140

Gly Val Glu Met Asn Tyr Gly Arg Ala Ile Thr Gln Asp Asp Val Ala
145                 150                 155                 160

Ala Gln Arg Pro Val Ala Val Ile Ala Pro Asp Thr Phe Asn Thr Leu
                165                 170                 175

Phe Asp Ala Asn Pro Asn Leu Ala Leu Gly Ser Glu Val Ala Phe Glu
            180                 185                 190

Leu Asn Gly Gln Glu Thr Phe Leu Arg Val Ile Gly Val Tyr Lys Glu
        195                 200                 205

Ala Ala Ala Gly Gly Leu Val Gly Ser Asn Pro Thr Val His Thr Tyr
    210                 215                 220

Thr Pro Tyr Thr Val Ala Asn Asp Ile Thr His Thr Glu Asp Gly Leu

225                 230                 235                 240

Asn Thr Leu Ser Ile Arg Ala Ala Gln Gly Val Asp Gln Asp Ser Leu
                245                 250                 255

Lys Gly Ser Leu Gln Thr Tyr Phe Asp Ala Leu Tyr Ala Asn Asn Asp
            260                 265                 270

Ser His His Val Ala Met Leu Asp Phe Arg Lys Gln Ile Glu Glu Phe
        275                 280                 285

Asn Thr Ile Leu Gly Ala Met Ser Leu Gly Ile Ser Ala Ile Gly Gly
    290                 295                 300

Ile Ser Leu Leu Val Gly Gly Ile Gly Val Met Asn Ile Met Leu Val
305                 310                 315                 320

Ser Val Thr Glu Arg Thr Arg Glu Ile Gly Val Arg Lys Ala Leu Gly
                325                 330                 335

Ala Arg Arg Arg Asp Ile Arg Leu Gln Phe Val Val Glu Ala Met Ile
            340                 345                 350

Ile Cys Phe Ile Gly Gly Ile Leu Gly Val Leu Leu Gly Gly Ile Leu
        355                 360                 365

Gly Leu Ile Met Ser Ser Ala Ile Gly Tyr Ile Ser Leu Pro Pro Leu
    370                 375                 380

Ser Gly Ile Val Ile Ala Leu Val Phe Ser Met Ala Ile Gly Leu Phe
385                 390                 395                 400

Phe Gly Tyr Tyr Pro Ala Asn Lys Ala Ala Lys Leu Asp Pro Ile Asp
                405                 410                 415

Ala Leu Arg Tyr Glu
            420


<210> 361
<211> 1815
<212> DNA
<213> Corynebacterium glutamicum

<220>
<221> CDS
<222> (101)..(1792)
<223> RXN02096

<400> 361
   cgcttcgacg acctcaccca cagcgatatc cgcaggaatc tcatcgcggt ttttgatgag 60
   ccgttcttgt actcctcctc cataccgcga gaacatctcg atg ggt ttg gat gtc    115
                                               Met Gly Leu Asp Val
                                               1               5
   agt gat gag cag atc gaa cac gca gcc agg ctt gcc cag gct cat gat    163
   Ser Asp Glu Gln Ile Glu His Ala Ala Arg Leu Ala Gln Ala His Asp
                   10                  15                  20
   ttt atc gat cgc ctt cca aac aaa tac gag gaa gtc att ggc gaa cgc    211
   Phe Ile Asp Arg Leu Pro Asn Lys Tyr Glu Glu Val Ile Gly Glu Arg
   25 30 35
   ggc ctg acg ctt tct ggt ggt caa cgc caa cgc atc gcc ctc gca cgg 259
   Gly Leu Thr Leu Ser Gly Gly Gln Arg Gln Arg Ile Ala Leu Ala Arg
   40 45 50
   gct ttc ctg gcg cat ccc aaa gtg ttg gtg ctt gat gat gcc acc tct 307
   Ala Phe Leu Ala His Pro Lys Val Leu Val Leu Asp Asp Ala Thr Ser
   55 60 65
   gcc att gat gcc tcc act gag gac cgc att ttc cag gcc ttg cgc gaa 355
   Ala Ile Asp Ala Ser Thr Glu Asp Arg Ile Phe Gln Ala Leu Arg Glu
   70 75 80 85
   gaa ctg cac gat gtc acc att ttg atc atc gcg cac cgc cac tcc act 403
   Glu Leu His Asp Val Thr Ile Leu Ile Ile Ala His Arg His Ser Thr
   90 95 100
   ttg gag ctc ggc gat cgg gtt ggt ctg gtc gaa gat gga cgg gta aca 451
   Leu Glu Leu Gly Asp Arg Val Gly Leu Val Glu Asp Gly Arg Val Thr
   105 110 115
   gca ctg gga ccg ttg agt gag atg cgt gat cac gct cgt ttc tcg cat 499
   Ala Leu Gly Pro Leu Ser Glu Met Arg Asp His Ala Arg Phe Ser His
   120 125 130
   ctg atg gct ctt gat ttc cag gat tct cac gat ccg gaa ttc acc ctc 547
   Leu Met Ala Leu Asp Phe Gln Asp Ser His Asp Pro Glu Phe Thr Leu
   135 140 145
   gac aac ggt tca cta ccc agc caa gag caa ttg tgg ccg gag gtc tcc 595
   Asp Asn Gly Ser Leu Pro Ser Gln Glu Gln Leu Trp Pro Glu Val Ser
   150 155 160 165
   aca gaa aag cag tac aag att ctt gcg cct gcc cct ggt cga ggc cgt 643
   Thr Glu Lys Gln Tyr Lys Ile Leu Ala Pro Ala Pro Gly Arg Gly Arg
   170 175 180
   ggc atg tcc atg cca gca acc cct gag ctg ctc gcc cag att gag gcg 691
   Gly Met Ser Met Pro Ala Thr Pro Glu Leu Leu Ala Gln Ile Glu Ala
   185 190 195
   ctg cca gca gca acg gaa gaa aca cga gtt gat gcc ggg agg cta cgc 739
   Leu Pro Ala Ala Thr Glu Glu Thr Arg Val Asp Ala Gly Arg Leu Arg
   200 205 210
   acc agt acc tcc ggt ttc aaa ttg ctc agt tta ttc aag cag gtc cgt 787
   Thr Ser Thr Ser Gly Phe Lys Leu Leu Ser Leu Phe Lys Gln Val Arg
   215 220 225
   tgg ctc gtc gtc gcg gtc atc gcg ttg ttg ctg gtg ggc gta gcc gcc 835
   Trp Leu Val Val Ala Val Ile Ala Leu Leu Leu Val Gly Val Ala Ala
   230 235 240 245
   gat cta gca ttt cca aca ctg atg cgc gca gcc atc gac aac ggt gtg 883
   Asp Leu Ala Phe Pro Thr Leu Met Arg Ala Ala Ile Asp Asn Gly Val
   250 255 260
   caa gca caa agc acc tcc acg ttg tgg tgg atc gcc atc gca ggc agc 931
   Gln Ala Gln Ser Thr Ser Thr Leu Trp Trp Ile Ala Ile Ala Gly Ser
   265 270 275
   gta gta gtc ctt ctg tcc tgg gcc gcc gcc gcg atc aac acg att atc 979
   Val Val Val Leu Leu Ser Trp Ala Ala Ala Ala Ile Asn Thr Ile Ile
   280 285 290
   acg gca cgc acc ggt gaa cgg ctg ctt tac ggc ttg cgt ctg cgc tca 1027
   Thr Ala Arg Thr Gly Glu Arg Leu Leu Tyr Gly Leu Arg Leu Arg Ser
   295 300 305
   ttt gtg cat cta ttg cgc ctg tcc atg agc tat ttc gaa cgc acc atg 1075
   Phe Val His Leu Leu Arg Leu Ser Met Ser Tyr Phe Glu Arg Thr Met
   310 315 320 325
   tcc ggc cgc atc atg acg cgc atg acc acc gac atc gac aac ctc tcg 1123
   Ser Gly Arg Ile Met Thr Arg Met Thr Thr Asp Ile Asp Asn Leu Ser
   330 335 340
   tcc ttc ctc caa tca ggt ctg gcg caa aca gtt gtc tct gtg ggc acg 1171
   Ser Phe Leu Gln Ser Gly Leu Ala Gln Thr Val Val Ser Val Gly Thr
   345 350 355
   ctc atc ggt gtg gtc acc atg ctc gcc atc acc gac gca caa cta gca 1219
   Leu Ile Gly Val Val Thr Met Leu Ala Ile Thr Asp Ala Gln Leu Ala
   360 365 370
   ctc gtt gcg ctg tcc gtg gtg ccg atc atc atc gtg ctc act ctc att 1267
   Leu Val Ala Leu Ser Val Val Pro Ile Ile Ile Val Leu Thr Leu Ile
   375 380 385
   ttc cga cgc atc agc tcc agg ctg tac acc gct tca cgc gag caa gcc 1315
   Phe Arg Arg Ile Ser Ser Arg Leu Tyr Thr Ala Ser Arg Glu Gln Ala
   390 395 400 405
   agc cag gtc aac gcg gta ttc cac gag tcc atc gcc ggt tta cgc acc 1363
   Ser Gln Val Asn Ala Val Phe His Glu Ser Ile Ala Gly Leu Arg Thr
   410 415 420
   gcg cag atg cac cgc atg gaa gac caa gtc ttt gac aat tat gcg ggc 1411
   Ala Gln Met His Arg Met Glu Asp Gln Val Phe Asp Asn Tyr Ala Gly
   425 430 435
   gaa gca gag gaa ttc cga cgc ctg cgt gtg aaa tcc cag acg gcc atc 1459
   Glu Ala Glu Glu Phe Arg Arg Leu Arg Val Lys Ser Gln Thr Ala Ile
   440 445 450
   gcc atc tac ttc ccc ggc ctt ggc gcg ctc tct gaa atc gcc cag gca 1507
   Ala Ile Tyr Phe Pro Gly Leu Gly Ala Leu Ser Glu Ile Ala Gln Ala
   455 460 465
   ctc gtc ctc ggt ttc ggc gca ctg caa gta acg cgc ggc gac atc tcc 1555
   Leu Val Leu Gly Phe Gly Ala Leu Gln Val Thr Arg Gly Asp Ile Ser
   470 475 480 485
   acc ggc gta ctc gtg gca ttc gtg ctg tac atg ggc ctg atg ttc ggc 1603
   Thr Gly Val Leu Val Ala Phe Val Leu Tyr Met Gly Leu Met Phe Gly
   490 495 500
   ccc atc caa caa cta agc caa atc ttc gac tcc tac caa caa gcc gcc 1651
   Pro Ile Gln Gln Leu Ser Gln Ile Phe Asp Ser Tyr Gln Gln Ala Ala
   505 510 515
   gtc ggc ttc cgt cgc atc acc gaa ctg ctc gca acg cag ccc agc gtc     1699
   Val Gly Phe Arg Arg Ile Thr Glu Leu Leu Ala Thr Gln Pro Ser Val
           520                 525                 530
   cag atc tgg gca cca aca ggc acg cta ggc agg ctg cca cgc agc ctt     1747
   Gln Ile Trp Ala Pro Thr Gly Thr Leu Gly Arg Leu Pro Arg Ser Leu
       535                 540                 545
   tat tgc ttg acg acg tca cct tcg gct att cag acg atc cga tcc         1792
   Tyr Cys Leu Thr Thr Ser Pro Ser Ala Ile Gln Thr Ile Arg Ser
   550                 555                 560
   tagacaacgt caccgtccag atc                                           1815

<210> 362
<211> 564
<212> PRT
<213> Corynebacterium glutamicum

<400> 362
Met Gly Leu Asp Val Ser Asp Glu Gln Ile Glu His Ala Ala Arg Leu
  1               5                  10                  15

Ala Gln Ala His Asp Phe Ile Asp Arg Leu Pro Asn Lys Tyr Glu Glu
             20                  25                  30

Val Ile Gly Glu Arg Gly Leu Thr Leu Ser Gly Gly Gln Arg Gln Arg
         35                  40                  45

Ile Ala Leu Ala Arg Ala Phe Leu Ala His Pro Lys Val Leu Val Leu
     50                  55                  60

Asp Asp Ala Thr Ser Ala Ile Asp Ala Ser Thr Glu Asp Arg Ile Phe
 65                  70                  75                  80

Gln Ala Leu Arg Glu Glu Leu His Asp Val Thr Ile Leu Ile Ile Ala
                 85                  90                  95

His Arg His Ser Thr Leu Glu Leu Gly Asp Arg Val Gly Leu Val Glu
            100                 105                 110

Asp Gly Arg Val Thr Ala Leu Gly Pro Leu Ser Glu Met Arg Asp His
        115                 120                 125

Ala Arg Phe Ser His Leu Met Ala Leu Asp Phe Gln Asp Ser His Asp
    130                 135                 140

Pro Glu Phe Thr Leu Asp Asn Gly Ser Leu Pro Ser Gln Glu Gln Leu
145                 150                 155                 160

Trp Pro Glu Val Ser Thr Glu Lys Gln Tyr Lys Ile Leu Ala Pro Ala
                165                 170                 175

Pro Gly Arg Gly Arg Gly Met Ser Met Pro Ala Thr Pro Glu Leu Leu
            180                 185                 190

Ala Gln Ile Glu Ala Leu Pro Ala Ala Thr Glu Glu Thr Arg Val Asp
        195                 200                 205

Ala Gly Arg Leu Arg Thr Ser Thr Ser Gly Phe Lys Leu Leu Ser Leu
    210                 215                 220

Phe Lys Gln Val Arg Trp Leu Val Val Ala Val Ile Ala Leu Leu Leu
225                 230                 235                 240

Val Gly Val Ala Ala Asp Leu Ala Phe Pro Thr Leu Met Arg Ala Ala
                245                 250                 255

Ile Asp Asn Gly Val Gln Ala Gln Ser Thr Ser Thr Leu Trp Trp Ile
            260                 265                 270

Ala Ile Ala Gly Ser Val Val Val Leu Leu Ser Trp Ala Ala Ala Ala
        275                 280                 285

Ile Asn Thr Ile Ile Thr Ala Arg Thr Gly Glu Arg Leu Leu Tyr Gly
    290                 295                 300

Leu Arg Leu Arg Ser Phe Val His Leu Leu Arg Leu Ser Met Ser Tyr
305                 310                 315                 320

Phe Glu Arg Thr Met Ser Gly Arg Ile Met Thr Arg Met Thr Thr Asp
                325                 330                 335

Ile Asp Asn Leu Ser Ser Phe Leu Gln Ser Gly Leu Ala Gln Thr Val
            340                 345                 350

Val Ser Val Gly Thr Leu Ile Gly Val Val Thr Met Leu Ala Ile Thr
        355                 360                 365

Asp Ala Gln Leu Ala Leu Val Ala Leu Ser Val Val Pro Ile Ile Ile
    370                 375                 380

Val Leu Thr Leu Ile Phe Arg Arg Ile Ser Ser Arg Leu Tyr Thr Ala
385                 390                 395                 400

Ser Arg Glu Gln Ala Ser Gln Val Asn Ala Val Phe His Glu Ser Ile
                405                 410                 415

Ala Gly Leu Arg Thr Ala Gln Met His Arg Met Glu Asp Gln Val Phe
            420                 425                 430

Asp Asn Tyr Ala Gly Glu Ala Glu Glu Phe Arg Arg Leu Arg Val Lys
        435                 440                 445

Ser Gln Thr Ala Ile Ala Ile Tyr Phe Pro Gly Leu Gly Ala Leu Ser
    450                 455                 460

Glu Ile Ala Gln Ala Leu Val Leu Gly Phe Gly Ala Leu Gln Val Thr
465                 470                 475                 480

Arg Gly Asp Ile Ser Thr Gly Val Leu Val Ala Phe Val Leu Tyr Met
                485                 490                 495

Gly Leu Met Phe Gly Pro Ile Gln Gln Leu Ser Gln Ile Phe Asp Ser
            500                 505                 510

Tyr Gln Gln Ala Ala Val Gly Phe Arg Arg Ile Thr Glu Leu Leu Ala
        515                 520                 525

Thr Gln Pro Ser Val Gln Ile Trp Ala Pro Thr Gly Thr Leu Gly Arg

530                 535                 540

Leu Pro Arg Ser Leu Tyr Cys Leu Thr Thr Ser Pro Ser Ala Ile Gln
545                 550                 555                 560

Thr Ile Arg Ser

<210> 363
<211> 1203
<212> DNA
<213> Corynebacterium glutamicum

<220>
<221> CDS
<222> (101)..(1180)
<223> RXN00412

<400> 363
cttttgacga acaccacgtc gcgtacgctt cctcggggcg ttaaactatt tgtcttccag 60

cttttgtccc ccgacttttg tacgaatcga ggacaccgtc gtg tca cac acc gcg   115
                                          Val Ser His Thr Ala
                                          1               5

tcc aca ccg acg cca gag gaa tac tcc gcg cag caa ccc agc acc cag   163
Ser Thr Pro Thr Pro Glu Glu Tyr Ser Ala Gln Gln Pro Ser Thr Gln
                10                  15                  20

ggc act cgc gtt gag ttc cgc ggc ata acc aaa gtc ttt agc aac aat   211
Gly Thr Arg Val Glu Phe Arg Gly Ile Thr Lys Val Phe Ser Asn Asn
            25                  30                  35

aaa tct gct aaa acc acc gcg ctt gat aat gtc act ctc acc gta gaa   259
Lys Ser Ala Lys Thr Thr Ala Leu Asp Asn Val Thr Leu Thr Val Glu
        40                  45                  50

ccc ggt gag gta atc ggc atc atc ggt tac tct ggc gcc ggc aag tcc   307
Pro Gly Glu Val Ile Gly Ile Ile Gly Tyr Ser Gly Ala Gly Lys Ser
    55                  60                  65

act ctt gtc cgc ctc atc aat ggc ctt gac tcc ccc acg agc ggt tcg   355
Thr Leu Val Arg Leu Ile Asn Gly Leu Asp Ser Pro Thr Ser Gly Ser
70                  75                  80                  85

ttg ctg ctc aac ggc acc gac atc gtc gga atg ccc gag tct aag ctg   403
Leu Leu Leu Asn Gly Thr Asp Ile Val Gly Met Pro Glu Ser Lys Leu
                90                  95                  100

cgt aaa ctg cgc agt aat atc ggc atg att ttc cag cag ttc aac ctg   451
Arg Lys Leu Arg Ser Asn Ile Gly Met Ile Phe Gln Gln Phe Asn Leu
            105                 110                 115

ttc cag tcg cgt act gcg gct gga aat gtg gag tac ccg ctg gaa gtt   499
Phe Gln Ser Arg Thr Ala Ala Gly Asn Val Glu Tyr Pro Leu Glu Val
        120                 125                 130

gcc aag atg gac aag gca gct cgt aaa gct cgc gtg caa gaa atg ctc   547
Ala Lys Met Asp Lys Ala Ala Arg Lys Ala Arg Val Gln Glu Met Leu
    135                 140                 145

gag ttc gtc ggc ctg ggc gac aaa ggc aaa aac tac ccc gag cag ctg     595
Glu Phe Val Gly Leu Gly Asp Lys Gly Lys Asn Tyr Pro Glu Gln Leu
150                 155                 160                 165

tcg ggc ggc cag aag cag cgc gtc ggc att gcc cgt gca ctg gcc acc     643
Ser Gly Gly Gln Lys Gln Arg Val Gly Ile Ala Arg Ala Leu Ala Thr
                170                 175                 180

aat cca acg ctt ttg ctt gcc gac gaa gcc acc tcc gct ttg gac cca     691
Asn Pro Thr Leu Leu Leu Ala Asp Glu Ala Thr Ser Ala Leu Asp Pro
            185                 190                 195

gaa acc acc cat gaa gtt ctg gag ctg ctg cgc aag gta aac cgc gaa     739
Glu Thr Thr His Glu Val Leu Glu Leu Leu Arg Lys Val Asn Arg Glu
        200                 205                 210

ctg ggc atc acc atc gtt gtg atc acc cac gaa atg gaa gtt gtg cgt     787
Leu Gly Ile Thr Ile Val Val Ile Thr His Glu Met Glu Val Val Arg
    215                 220                 225

tcc atc gca gac aag gtt gct gtg atg gaa tcc ggc aaa gtt gtg gaa     835
Ser Ile Ala Asp Lys Val Ala Val Met Glu Ser Gly Lys Val Val Glu
230                 235                 240                 245

tac ggc agc gtc tac gag gtg ttc tcc aat cca caa aca cag gtt gct     883
Tyr Gly Ser Val Tyr Glu Val Phe Ser Asn Pro Gln Thr Gln Val Ala
                250                 255                 260

caa aag ttc gtg gcc acc gcg ctg cgt aac acc cca gac caa gtg gaa     931
Gln Lys Phe Val Ala Thr Ala Leu Arg Asn Thr Pro Asp Gln Val Glu
            265                 270                 275

tcg gaa gat ctg ctt agc cat gag gga cgt ctg ttc acc att gat ctg     979
Ser Glu Asp Leu Leu Ser His Glu Gly Arg Leu Phe Thr Ile Asp Leu
        280                 285                 290

act gaa acg tcc ggc ttc ttt gca gca acc gct cgt gct gcc gaa caa     1027
Thr Glu Thr Ser Gly Phe Phe Ala Ala Thr Ala Arg Ala Ala Glu Gln
    295                 300                 305

ggt gct ttt gtc aac atc gtt cac ggt ggc gtg acc acc ttg caa cgc     1075
Gly Ala Phe Val Asn Ile Val His Gly Gly Val Thr Thr Leu Gln Arg
310                 315                 320                 325

caa tca ttt ggc aaa atg act gtt cga ctc acc ggc aac acc gct gcg     1123
Gln Ser Phe Gly Lys Met Thr Val Arg Leu Thr Gly Asn Thr Ala Ala
                330                 335                 340

att gaa gag ttc tat caa acc ttg acc aag acc acg acc atc aag gag     1171
Ile Glu Glu Phe Tyr Gln Thr Leu Thr Lys Thr Thr Thr Ile Lys Glu
            345                 350                 355

atc acc cga tgaacgagat gatcctcgca gct                               1203
Ile Thr Arg
        360

<210> 364
<211> 360
<212> PRT

<213> Corynebacterium glutamicum

<400> 364
Val Ser His Thr Ala Ser Thr Pro Thr Pro Glu Glu Tyr Ser Ala Gln
1               5                   10                  15

Gln Pro Ser Thr Gln Gly Thr Arg Val Glu Phe Arg Gly Ile Thr Lys
            20                  25                  30

Val Phe Ser Asn Asn Lys Ser Ala Lys Thr Thr Ala Leu Asp Asn Val
        35                  40                  45

Thr Leu Thr Val Glu Pro Gly Glu Val Ile Gly Ile Ile Gly Tyr Ser
    50                  55                  60

Gly Ala Gly Lys Ser Thr Leu Val Arg Leu Ile Asn Gly Leu Asp Ser
65                  70                  75                  80

Pro Thr Ser Gly Ser Leu Leu Leu Asn Gly Thr Asp Ile Val Gly Met
                85                  90                  95

Pro Glu Ser Lys Leu Arg Lys Leu Arg Ser Asn Ile Gly Met Ile Phe
            100                 105                 110

Gln Gln Phe Asn Leu Phe Gln Ser Arg Thr Ala Ala Gly Asn Val Glu
        115                 120                 125

Tyr Pro Leu Glu Val Ala Lys Met Asp Lys Ala Ala Arg Lys Ala Arg
    130                 135                 140

Val Gln Glu Met Leu Glu Phe Val Gly Leu Gly Asp Lys Gly Lys Asn
145                 150                 155                 160

Tyr Pro Glu Gln Leu Ser Gly Gly Gln Lys Gln Arg Val Gly Ile Ala
                165                 170                 175

Arg Ala Leu Ala Thr Asn Pro Thr Leu Leu Leu Ala Asp Glu Ala Thr
            180                 185                 190

Ser Ala Leu Asp Pro Glu Thr Thr His Glu Val Leu Glu Leu Leu Arg
        195                 200                 205

Lys Val Asn Arg Glu Leu Gly Ile Thr Ile Val Val Ile Thr His Glu
    210                 215                 220

Met Glu Val Val Arg Ser Ile Ala Asp Lys Val Ala Val Met Glu Ser
225                 230                 235                 240

Gly Lys Val Val Glu Tyr Gly Ser Val Tyr Glu Val Phe Ser Asn Pro
                245                 250                 255

Gln Thr Gln Val Ala Gln Lys Phe Val Ala Thr Ala Leu Arg Asn Thr
            260                 265                 270

Pro Asp Gln Val Glu Ser Glu Asp Leu Leu Ser His Glu Gly Arg Leu
        275                 280                 285

Phe Thr Ile Asp Leu Thr Glu Thr Ser Gly Phe Phe Ala Ala Thr Ala
    290                 295                 300

Arg Ala Ala Glu Gln Gly Ala Phe Val Asn Ile Val His Gly Gly Val

305                 310                 315                 320

Thr Thr Leu Gln Arg Gln Ser Phe Gly Lys Met Thr Val Arg Leu Thr
                325                 330                 335

Gly Asn Thr Ala Ala Ile Glu Glu Phe Tyr Gln Thr Leu Thr Lys Thr
            340                 345                 350

Thr Thr Ile Lys Glu Ile Thr Arg
        355                 360


<210> 365
<211> 798
<212> DNA
<213> Corynebacterium glutamicum

<220>
<221> CDS
<222> (101)..(775)
<223> RXN00411

<400> 365
catttggcaa aatgactgtt cgactcaccg gcaacaccgc tgcgattgaa gagttctatc 60

aaaccttgac caagaccacg accatcaagg agatcacccg atg aac gag atg atc    115
                                           Met Asn Glu Met Ile
                                            1               5

ctc gca gct gac tgg aac cgg cta gga ccc acc ttc caa aca gcc atc    163
Leu Ala Ala Asp Trp Asn Arg Leu Gly Pro Thr Phe Gln Thr Ala Ile
                10                  15                  20

att gac acc ctg ttg atg gtc atc atc acc atg gtg gtg gct ggc tta    211
Ile Asp Thr Leu Leu Met Val Ile Ile Thr Met Val Val Ala Gly Leu
            25                  30                  35

ctg ggt ctt gtc gtc ggc ctg ctg ctt tac acc acc cgc gct ggt gga    259
Leu Gly Leu Val Val Gly Leu Leu Leu Tyr Thr Thr Arg Ala Gly Gly
        40                  45                  50

atc ttg aag aac aag gtc atc tac acc att ttg aat gtg ctg gtg aac    307
Ile Leu Lys Asn Lys Val Ile Tyr Thr Ile Leu Asn Val Leu Val Asn
    55                  60                  65

ttt gtt cga ccc atc cca ttc att att ttg atc gcc gcc atc aag cca    355
Phe Val Arg Pro Ile Pro Phe Ile Ile Leu Ile Ala Ala Ile Lys Pro
70                  75                  80                  85

cta acg gtc gcc gtc atg ggc acc tcc atc ggc cga gat gcc ggc atc    403
Leu Thr Val Ala Val Met Gly Thr Ser Ile Gly Arg Asp Ala Gly Ile
                90                  95                  100

ttc gtc atg gtt gtc gca gcg att ttc tct gtg gct cga atc gtg gag    451
Phe Val Met Val Val Ala Ala Ile Phe Ser Val Ala Arg Ile Val Glu
            105                 110                 115

caa aac ttg gtc tcc att gat cct ggt gtc atc gag gca gct cgc tcc    499
Gln Asn Leu Val Ser Ile Asp Pro Gly Val Ile Glu Ala Ala Arg Ser
        120                 125                 130

atg ggt gcg tcc ccg atg cgc atc atc gcc acc gtg atc att cca gaa     547
Met Gly Ala Ser Pro Met Arg Ile Ile Ala Thr Val Ile Ile Pro Glu
    135                 140                 145

gca ctt gga cca ttg gtt ctg ggt tac acc ttc ctg ttc atc gcg atc     595
Ala Leu Gly Pro Leu Val Leu Gly Tyr Thr Phe Leu Phe Ile Ala Ile
150                 155                 160                 165

gtc gat atg tcc gca atg gtc ggc tac atc ggt ggc ggt ggt ctt ggt     643
Val Asp Met Ser Ala Met Val Gly Tyr Ile Gly Gly Gly Gly Leu Gly
                170                 175                 180

gac ttc gcc att gtt tac ggc tac cgc gcc ttc gac aac gaa gtt atg     691
Asp Phe Ala Ile Val Tyr Gly Tyr Arg Ala Phe Asp Asn Glu Val Met
            185                 190                 195

tac gtt gcc gtc ctg gtt atc gtc atc atc gtg cag gca gcc cag ctt     739
Tyr Val Ala Val Leu Val Ile Val Ile Ile Val Gln Ala Ala Gln Leu
        200                 205                 210

ctg ggc aat tgg ctg tcc aag aag atc atg cgc cgc taaacctctt          785
Leu Gly Asn Trp Leu Ser Lys Lys Ile Met Arg Arg
    215                 220                 225

gcatagaaaa acc                                                      798


<210> 366
<211> 225
<212> PRT
<213> Corynebacterium glutamicum

<400> 366
Met Asn Glu Met Ile Leu Ala Ala Asp Trp Asn Arg Leu Gly Pro Thr
1               5                   10                  15

Phe Gln Thr Ala Ile Ile Asp Thr Leu Leu Met Val Ile Ile Thr Met
            20                  25                  30

Val Val Ala Gly Leu Leu Gly Leu Val Val Gly Leu Leu Leu Tyr Thr
        35                  40                  45

Thr Arg Ala Gly Gly Ile Leu Lys Asn Lys Val Ile Tyr Thr Ile Leu
    50                  55                  60

Asn Val Leu Val Asn Phe Val Arg Pro Ile Pro Phe Ile Ile Leu Ile
65                  70                  75                  80

Ala Ala Ile Lys Pro Leu Thr Val Ala Val Met Gly Thr Ser Ile Gly
                85                  90                  95

Arg Asp Ala Gly Ile Phe Val Met Val Val Ala Ala Ile Phe Ser Val
            100                 105                 110

Ala Arg Ile Val Glu Gln Asn Leu Val Ser Ile Asp Pro Gly Val Ile
        115                 120                 125

Glu Ala Ala Arg Ser Met Gly Ala Ser Pro Met Arg Ile Ile Ala Thr
    130                 135                 140

Val Ile Ile Pro Glu Ala Leu Gly Pro Leu Val Leu Gly Tyr Thr Phe

145                 150                 155                 160

Leu Phe Ile Ala Ile Val Asp Met Ser Ala Met Val Gly Tyr Ile Gly
                165                 170                 175

Gly Gly Gly Leu Gly Asp Phe Ala Ile Val Tyr Gly Tyr Arg Ala Phe
            180                 185                 190

Asp Asn Glu Val Met Tyr Val Ala Val Leu Val Ile Val Ile Ile Val
        195                 200                 205

Gln Ala Ala Gln Leu Leu Gly Asn Trp Leu Ser Lys Lys Ile Met Arg
    210                 215                 220

Arg
225


<210> 367
<211> 852
<212> DNA
<213> Corynebacterium glutamicum

<220>
<221> CDS
<222> (101)..(829)
<223> RXN02614

<400> 367
tcattgtata cgccaccctc ggtctgctgt ctgaagcgct gatcagagct tgggaacgtc 60

acaccttccg ctaccgaaac gcataagaaa gttgctcgcc atg act gcc aca ttg   115
                                            Met Thr Ala Thr Leu
                                            1               5

tca ctc aaa ccc gca gcc act gtc cgt gga ttg cgc aaa tca tac gga   163
Ser Leu Lys Pro Ala Ala Thr Val Arg Gly Leu Arg Lys Ser Tyr Gly
                10                  15                  20

act aaa gaa gtc ctc caa gga atc gac ctc acc atc aac tgc ggc gaa   211
Thr Lys Glu Val Leu Gln Gly Ile Asp Leu Thr Ile Asn Cys Gly Glu
            25                  30                  35

gta acc gcg ctg atc gga cgc tca ggt tca gga aaa tcc acc atc ctg   259
Val Thr Ala Leu Ile Gly Arg Ser Gly Ser Gly Lys Ser Thr Ile Leu
        40                  45                  50

cgc gtg ttg gcg ggc cta tct aaa gag cat tcc ggc tct gta gaa att   307
Arg Val Leu Ala Gly Leu Ser Lys Glu His Ser Gly Ser Val Glu Ile
    55                  60                  65

tcc gga aac ccg gcc gtt gcc ttc caa gag cct cgc ctg ttg ccg tgg   355
Ser Gly Asn Pro Ala Val Ala Phe Gln Glu Pro Arg Leu Leu Pro Trp
70                  75                  80                  85

aaa acg gtg ctc gat aat gtg acc ttt ggc ctc aac cgc act gat att   403
Lys Thr Val Leu Asp Asn Val Thr Phe Gly Leu Asn Arg Thr Asp Ile
                90                  95                  100

tcc tgg tca gaa gca caa gaa cgc gcc tcg gca ctg ctt gca gaa gtc   451
Ser Trp Ser Glu Ala Gln Glu Arg Ala Ser Ala Leu Leu Ala Glu Val

            105                 110                 115

aaa ctt ccc gac tcc gac gcc gcc tgg ccc ctc acg ctc tcc ggc ggc 499
Lys Leu Pro Asp Ser Asp Ala Ala Trp Pro Leu Thr Leu Ser Gly Gly
        120                 125                 130

caa gcc cag cgc gtc tcc ctt gcg cga gcg ctc atc tcc gag cca gag 547
Gln Ala Gln Arg Val Ser Leu Ala Arg Ala Leu Ile Ser Glu Pro Glu
    135                 140                 145

ctt ttg ctt ctc gac gaa ccc ttc ggc gcc ctc gat gct ctg aca aga 595
Leu Leu Leu Leu Asp Glu Pro Phe Gly Ala Leu Asp Ala Leu Thr Arg
150                 155                 160                 165

ctg aca gcc caa gac ctg ctg ctc aaa acc gtg aac acc cga aac ttg 643
Leu Thr Ala Gln Asp Leu Leu Leu Lys Thr Val Asn Thr Arg Asn Leu
                170                 175                 180

gga gtt ctg ctg gtc acc cat gat gtt tcc gag gcc atc gcc ctg gcc 691
Gly Val Leu Leu Val Thr His Asp Val Ser Glu Ala Ile Ala Leu Ala
            185                 190                 195

gac cac gtc ctt ctt ctt gac gac ggc gcc atc aca cac agt ttg act 739
Asp His Val Leu Leu Leu Asp Asp Gly Ala Ile Thr His Ser Leu Thr
        200                 205                 210

gta gat atc ccc ggc gat cgc cgc acc cac ccc tcc ttt gcc tcc tac 787
Val Asp Ile Pro Gly Asp Arg Arg Thr His Pro Ser Phe Ala Ser Tyr
    215                 220                 225

acc gct caa ctc ctt gag tgg ctc gaa atc acc aca cct gcc 829
Thr Ala Gln Leu Leu Glu Trp Leu Glu Ile Thr Thr Pro Ala
230                 235                 240

tagaaagaaa tcatgaaatt taa 852

<210> 368
<211> 243
<212> PRT
<213> Corynebacterium glutamicum

<400> 368
Met Thr Ala Thr Leu Ser Leu Lys Pro Ala Ala Thr Val Arg Gly Leu
1               5                   10                  15

Arg Lys Ser Tyr Gly Thr Lys Glu Val Leu Gln Gly Ile Asp Leu Thr
            20                  25                  30

Ile Asn Cys Gly Glu Val Thr Ala Leu Ile Gly Arg Ser Gly Ser Gly
        35                  40                  45

Lys Ser Thr Ile Leu Arg Val Leu Ala Gly Leu Ser Lys Glu His Ser
    50                  55                  60

Gly Ser Val Glu Ile Ser Gly Asn Pro Ala Val Ala Phe Gln Glu Pro
65                  70                  75                  80

Arg Leu Leu Pro Trp Lys Thr Val Leu Asp Asn Val Thr Phe Gly Leu
                85                  90                  95

Asn Arg Thr Asp Ile Ser Trp Ser Glu Ala Gln Glu Arg Ala Ser Ala
            100                 105                 110

Leu Leu Ala Glu Val Lys Leu Pro Asp Ser Asp Ala Ala Trp Pro Leu
        115                 120                 125

Thr Leu Ser Gly Gly Gln Ala Gln Arg Val Ser Leu Ala Arg Ala Leu
    130                 135                 140

Ile Ser Glu Pro Glu Leu Leu Leu Leu Asp Glu Pro Phe Gly Ala Leu
145                 150                 155                 160

Asp Ala Leu Thr Arg Leu Thr Ala Gln Asp Leu Leu Leu Lys Thr Val
                165                 170                 175

Asn Thr Arg Asn Leu Gly Val Leu Leu Val Thr His Asp Val Ser Glu
            180                 185                 190

Ala Ile Ala Leu Ala Asp His Val Leu Leu Leu Asp Asp Gly Ala Ile
        195                 200                 205

Thr His Ser Leu Thr Val Asp Ile Pro Gly Asp Arg Arg Thr His Pro
    210                 215                 220

Ser Phe Ala Ser Tyr Thr Ala Gln Leu Leu Glu Trp Leu Glu Ile Thr
225                 230                 235                 240

Thr Pro Ala

<210> 369
<211> 1080
<212> DNA
<213> Corynebacterium glutamicum

<220>
<221> CDS
<222> (101)..(1057)
<223> RXN02613

<400> 369
agatatcccc ggcgatcgcc gcacccaccc ctcctttgcc tcctacaccg ctcaactcct 60

tgagtggctc gaaatcacca cacctgccta gaaagaaatc atg aaa ttt aag aaa   115
                                            Met Lys Phe Lys Lys
                                            1               5

atc gcc ctc gtt ctc gcc ttc ggt cta ggc ctt gca tcc tgc tca tca   163
Ile Ala Leu Val Leu Ala Phe Gly Leu Gly Leu Ala Ser Cys Ser Ser
                10                  15                  20

gct tct ggc gat ccc gcc acc aac gcc gat gga tcc atc gat ctg agc   211
Ala Ser Gly Asp Pro Ala Thr Asn Ala Asp Gly Ser Ile Asp Leu Ser
            25                  30                  35

aaa gta acc ctt aac atc ggt gat caa atc gcc gga aca gaa caa gtg   259
Lys Val Thr Leu Asn Ile Gly Asp Gln Ile Ala Gly Thr Glu Gln Val
        40                  45                  50

ctc caa gct tca ggg gag cta gat gat gtc cct tat aaa atc gaa tgg   307

Leu Gln Ala Ser Gly Glu Leu Asp Asp Val Pro Tyr Lys Ile Glu Trp
    55                  60                  65

tca tca ttt acc tct gga cca ccc caa atc gaa gca tta aac gca ggt 355
Ser Ser Phe Thr Ser Gly Pro Pro Gln Ile Glu Ala Leu Asn Ala Gly
70                  75                  80                  85

caa att gat ttc gcg atc acc gga aac acc cca ccg atc atc ggc ggc 403
Gln Ile Asp Phe Ala Ile Thr Gly Asn Thr Pro Pro Ile Ile Gly Gly
                90                  95                  100

ccc acc aac acc aaa gtg gtc tcc gcc tac aac aac gat gct tta ggt 451
Pro Thr Asn Thr Lys Val Val Ser Ala Tyr Asn Asn Asp Ala Leu Gly
            105                 110                 115

gat gtc atc ttg gtc gcc ccg gat tct tca ata acc tcg gtg gct gac 499
Asp Val Ile Leu Val Ala Pro Asp Ser Ser Ile Thr Ser Val Ala Asp
        120                 125                 130

ctt gct gga aag aaa gtg gct gtc gcc cgc gga tcc agc gcc cac gga 547
Leu Ala Gly Lys Lys Val Ala Val Ala Arg Gly Ser Ser Ala His Gly
    135                 140                 145

cac ctc atc caa caa cta gaa aaa gca ggc gtg agc gtt gac gac gta 595
His Leu Ile Gln Gln Leu Glu Lys Ala Gly Val Ser Val Asp Asp Val
150                 155                 160                 165

gaa atc aac ctc ctc caa ccc tcc gac gcc aaa gcc gct ttc caa aac 643
Glu Ile Asn Leu Leu Gln Pro Ser Asp Ala Lys Ala Ala Phe Gln Asn
                170                 175                 180

ggc cag gta gat gcg tgg gca gtg tgg gat ccc tac agc tca cag gcg 691
Gly Gln Val Asp Ala Trp Ala Val Trp Asp Pro Tyr Ser Ser Gln Ala
            185                 190                 195

gaa ctg gaa gga gct caa gtt ttg gtc agg gga gcg gga ctg gtc agt 739
Glu Leu Glu Gly Ala Gln Val Leu Val Arg Gly Ala Gly Leu Val Ser
        200                 205                 210

ggg cat gga ttt ggt gtc gca agt gat gaa gcg ctc gat gac ccc gca 787
Gly His Gly Phe Gly Val Ala Ser Asp Glu Ala Leu Asp Asp Pro Ala
    215                 220                 225

aag gaa gcc gcc ttg gca gat ttc ctc gat cgc gtg gcc gac tct tat 835
Lys Glu Ala Ala Leu Ala Asp Phe Leu Asp Arg Val Ala Asp Ser Tyr
230                 235                 240                 245

gaa tgg gct gaa gac aac acc gat gaa tgg gcg acg att ttc agc caa 883
Glu Trp Ala Glu Asp Asn Thr Asp Glu Trp Ala Thr Ile Phe Ser Gln
                250                 255                 260

gaa tcc ggc ttt gat ccg gag gcc tct caa ctg aac acc cgc agc ctg 931
Glu Ser Gly Phe Asp Pro Glu Ala Ser Gln Leu Asn Thr Arg Ser Leu
            265                 270                 275

cgc cat cag gtg ccg ctc gac gag tcc gtc aac acc tat cag aac gcg 979
Arg His Gln Val Pro Leu Asp Glu Ser Val Asn Thr Tyr Gln Asn Ala
        280                 285                 290

ctt atc gac gct ttc gtc tcc gcg ggt ctc gtt gag gac ttt aat ttc 1027
Leu Ile Asp Ala Phe Val Ser Ala Gly Leu Val Glu Asp Phe Asn Phe

295                 300                 305

gag gac acc gta gac acc cga ttt gag ggc taagtatgtc tgagtatggc     1077
Glu Asp Thr Val Asp Thr Arg Phe Glu Gly
310                 315

aaa                                                               1080

<210> 370
<211> 319
<212> PRT
<213> Corynebacterium glutamicum

<400> 370
Met Lys Phe Lys Lys Ile Ala Leu Val Leu Ala Phe Gly Leu Gly Leu
1               5                   10                  15

Ala Ser Cys Ser Ser Ala Ser Gly Asp Pro Ala Thr Asn Ala Asp Gly
            20                  25                  30

Ser Ile Asp Leu Ser Lys Val Thr Leu Asn Ile Gly Asp Gln Ile Ala
        35                  40                  45

Gly Thr Glu Gln Val Leu Gln Ala Ser Gly Glu Leu Asp Asp Val Pro
    50                  55                  60

Tyr Lys Ile Glu Trp Ser Ser Phe Thr Ser Gly Pro Pro Gln Ile Glu
65                  70                  75                  80

Ala Leu Asn Ala Gly Gln Ile Asp Phe Ala Ile Thr Gly Asn Thr Pro
                85                  90                  95

Pro Ile Ile Gly Gly Pro Thr Asn Thr Lys Val Val Ser Ala Tyr Asn
            100                 105                 110

Asn Asp Ala Leu Gly Asp Val Ile Leu Val Ala Pro Asp Ser Ser Ile
        115                 120                 125

Thr Ser Val Ala Asp Leu Ala Gly Lys Lys Val Ala Val Ala Arg Gly
    130                 135                 140

Ser Ser Ala His Gly His Leu Ile Gln Gln Leu Glu Lys Ala Gly Val
145                 150                 155                 160

Ser Val Asp Asp Val Glu Ile Asn Leu Leu Gln Pro Ser Asp Ala Lys
                165                 170                 175

Ala Ala Phe Gln Asn Gly Gln Val Asp Ala Trp Ala Val Trp Asp Pro
            180                 185                 190

Tyr Ser Ser Gln Ala Glu Leu Glu Gly Ala Gln Val Leu Val Arg Gly
        195                 200                 205

Ala Gly Leu Val Ser Gly His Gly Phe Gly Val Ala Ser Asp Glu Ala
    210                 215                 220

Leu Asp Asp Pro Ala Lys Glu Ala Ala Leu Ala Asp Phe Leu Asp Arg
225                 230                 235                 240

Val Ala Asp Ser Tyr Glu Trp Ala Glu Asp Asn Thr Asp Glu Trp Ala

245                 250                 255

Thr Ile Phe Ser Gln Glu Ser Gly Phe Asp Pro Glu Ala Ser Gln Leu
            260                 265                 270

Asn Thr Arg Ser Leu Arg His Gln Val Pro Leu Asp Glu Ser Val Asn
        275                 280                 285

Thr Tyr Gln Asn Ala Leu Ile Asp Ala Phe Val Ser Ala Gly Leu Val
    290                 295                 300

Glu Asp Phe Asn Phe Glu Asp Thr Val Asp Thr Arg Phe Glu Gly
305                 310                 315

<210> 371
<211> 1698
<212> DNA
<213> Corynebacterium glutamicum

<220>
<221> CDS
<222> (101)..(1675)
<223> RXN00368

<400> 371
ttcttccaaa acgcaatgaa ctagttttcc tatgcagtta tctccttcaa atacgtcgac 60

gcagcgcttt cacatctctg cgttgacgtc tttgtctttc atg cgt ctg ggt gtg   115
                                            Met Arg Leu Gly Val
                                            1               5

tgg ctg att gtc gcg ggg ttg ttt atc act ccg ttg gcg ctg gtg gtg   163
Trp Leu Ile Val Ala Gly Leu Phe Ile Thr Pro Leu Ala Leu Val Val
                10                  15                  20

ggc ttg gcg ttg gga ggc aat cag ttt cct gct ctg tgg gat tcc gga   211
Gly Leu Ala Leu Gly Gly Asn Gln Phe Pro Ala Leu Trp Asp Ser Gly
            25                  30                  35

ttg ggc aaa gcc cta tgg aat tcc gcc tat aca aca gtg ctt tct gcg   259
Leu Gly Lys Ala Leu Trp Asn Ser Ala Tyr Thr Thr Val Leu Ser Ala
        40                  45                  50

gtg ggc gcg acc att atc ggc acg atc atg gct ctc acg ctg gac cga   307
Val Gly Ala Thr Ile Ile Gly Thr Ile Met Ala Leu Thr Leu Asp Arg
    55                  60                  65

act gat gtt ttc ggg cgc acc gcg ttg cgg tta ttt ttg tta tcc ccg   355
Thr Asp Val Phe Gly Arg Thr Ala Leu Arg Leu Phe Leu Leu Ser Pro
70                  75                  80                  85

ctg ttg atc cct ccg ttt att ggg gct att gcg tgg ttg cag ctg ttc   403
Leu Leu Ile Pro Pro Phe Ile Gly Ala Ile Ala Trp Leu Gln Leu Phe
                90                  95                  100

ggg aag aac cag ggc atc aac cgg ttt ttc ggc acg gaa gtg tgg gat   451
Gly Lys Asn Gln Gly Ile Asn Arg Phe Phe Gly Thr Glu Val Trp Asp
            105                 110                 115

att tac ggc gct gat ggt gtg aca ttt ttg ttg att gtg cac tcc tat   499

Ile Tyr Gly Ala Asp Gly Val Thr Phe Leu Leu Ile Val His Ser Tyr
        120                 125                 130

ccc act gtg tac atc att gtt tcg gca gct ctg agg caa ctt cct agt     547
Pro Thr Val Tyr Ile Ile Val Ser Ala Ala Leu Arg Gln Leu Pro Ser
    135                 140                 145

gat ttg gag caa gct gca cgg atc gcg ggg gcg gat act ttt acg gtg     595
Asp Leu Glu Gln Ala Ala Arg Ile Ala Gly Ala Asp Thr Phe Thr Val
150                 155                 160                 165

ttg cgc acc atc aca ctc cca ctg ctc aaa cct gca ttg ttg tcg gcg     643
Leu Arg Thr Ile Thr Leu Pro Leu Leu Lys Pro Ala Leu Leu Ser Ala
                170                 175                 180

ttt act ctt acc aca gtg gcg aac ctc gcc gac ttt ggc att cca gct     691
Phe Thr Leu Thr Thr Val Ala Asn Leu Ala Asp Phe Gly Ile Pro Ala
            185                 190                 195

ctg ttg gga tcg cca gcg cgt ttt gaa acc tta gcc acc atg att tat     739
Leu Leu Gly Ser Pro Ala Arg Phe Glu Thr Leu Ala Thr Met Ile Tyr
        200                 205                 210

cgc ttc atg gaa tcc ggc acc gtg agc aat cca ttg cag gtg gta tcc     787
Arg Phe Met Glu Ser Gly Thr Val Ser Asn Pro Leu Gln Val Val Ser
    215                 220                 225

acc att ggc atc gtg ttg ttg ttc ctg gga atc gca gca gta acc gcg     835
Thr Ile Gly Ile Val Leu Leu Phe Leu Gly Ile Ala Ala Val Thr Ala
230                 235                 240                 245

gat tat ctg gtg tct ttg tac gcg gca tca aag ttg caa gac gca gga     883
Asp Tyr Leu Val Ser Leu Tyr Ala Ala Ser Lys Leu Gln Asp Ala Gly
                250                 255                 260

aca ccg cat cgc ttt act ctc aac aaa tca cga atc cca gtc agc gtg     931
Thr Pro His Arg Phe Thr Leu Asn Lys Ser Arg Ile Pro Val Ser Val
            265                 270                 275

atc acg tgg atc atc gcg ttg atc atc acc gcc gcc ccg ctg ctg ggt     979
Ile Thr Trp Ile Ile Ala Leu Ile Ile Thr Ala Ala Pro Leu Leu Gly
        280                 285                 290

ctg gca tac aga gca tta ctg cct gcc cca ggt gtg ccg ttc aac cta     1027
Leu Ala Tyr Arg Ala Leu Leu Pro Ala Pro Gly Val Pro Phe Asn Leu
    295                 300                 305

gac aac atc acg ctc aac aac ttt gaa gca gca ctg agc aat cca cga     1075
Asp Asn Ile Thr Leu Asn Asn Phe Glu Ala Ala Leu Ser Asn Pro Arg
310                 315                 320                 325

gta atc gaa gga ttc agc aac tcc ctc atg tta tcc ctg ggt gca gcc     1123
Val Ile Glu Gly Phe Ser Asn Ser Leu Met Leu Ser Leu Gly Ala Ala
                330                 335                 340

cta atc tgt ggg gtg ctg gga tgg ctg atc gga gtg ctc atc acc cga     1171
Leu Ile Cys Gly Val Leu Gly Trp Leu Ile Gly Val Leu Ile Thr Arg
            345                 350                 355

acc cag cat ttc gcc aac gta ccg ttg aca ctc act gtg ctg ctt ccc     1219
Thr Gln His Phe Ala Asn Val Pro Leu Thr Leu Thr Val Leu Leu Pro

360                 365                 370

acc gca ctg ccg ggc atg atc atc ggc gtc ggc tgg ctc att ttg ggc     1267
Thr Ala Leu Pro Gly Met Ile Ile Gly Val Gly Trp Leu Ile Leu Gly
    375                 380                 385

aga tac acc gga att tac aac aca cct tgg gtg att ttg ggt gca tat     1315
Arg Tyr Thr Gly Ile Tyr Asn Thr Pro Trp Val Ile Leu Gly Ala Tyr
390                 395                 400                 405

gtg tgt gct ttt acc gcg ctg gtt gtc caa gct gta cgc gga cca ctc     1363
Val Cys Ala Phe Thr Ala Leu Val Val Gln Ala Val Arg Gly Pro Leu
                410                 415                 420

agt caa gca ccc gaa gca atc gaa gaa gcc gca cga atc agc ggc gca     1411
Ser Gln Ala Pro Glu Ala Ile Glu Glu Ala Ala Arg Ile Ser Gly Ala
            425                 430                 435

ggc aga tta cga tcc atc atg gac acc acc gga gcg atg gca att ccc     1459
Gly Arg Leu Arg Ser Ile Met Asp Thr Thr Gly Ala Met Ala Ile Pro
        440                 445                 450

gca gct ttc gcc ggc gca gtg ctg gtt gcg gta act gcg gtt cga gag     1507
Ala Ala Phe Ala Gly Ala Val Leu Val Ala Val Thr Ala Val Arg Glu
    455                 460                 465

tta acc gtg tcc att ttg ctc atc gcg ccg ggc acc acc acc ctg ggt     1555
Leu Thr Val Ser Ile Leu Leu Ile Ala Pro Gly Thr Thr Thr Leu Gly
470                 475                 480                 485

gtg cag gtg ttc aat ttg cag cag gcg gga aat tac aat cag gca tcg     1603
Val Gln Val Phe Asn Leu Gln Gln Ala Gly Asn Tyr Asn Gln Ala Ser
                490                 495                 500

gcg ttg tcg ttg atg ttt gcg att atc ggt atc gtg gcg ctc gcg ttg     1651
Ala Leu Ser Leu Met Phe Ala Ile Ile Gly Ile Val Ala Leu Ala Leu
            505                 510                 515

acg gtg cgc agc cag aag gag ttt taggtgtcat cgatcaaatt gcg           1698
Thr Val Arg Ser Gln Lys Glu Phe
        520                 525


<210> 372
<211> 525
<212> PRT
<213> Corynebacterium glutamicum

<400> 372
Met Arg Leu Gly Val Trp Leu Ile Val Ala Gly Leu Phe Ile Thr Pro
  1               5                  10                  15

Leu Ala Leu Val Val Gly Leu Ala Leu Gly Gly Asn Gln Phe Pro Ala
             20                  25                  30

Leu Trp Asp Ser Gly Leu Gly Lys Ala Leu Trp Asn Ser Ala Tyr Thr
         35                  40                  45

Thr Val Leu Ser Ala Val Gly Ala Thr Ile Ile Gly Thr Ile Met Ala
     50                  55                  60

Leu Thr Leu Asp Arg Thr Asp Val Phe Gly Arg Thr Ala Leu Arg Leu
65                  70                  75                  80

Phe Leu Leu Ser Pro Leu Leu Ile Pro Pro Phe Ile Gly Ala Ile Ala
                85                  90                  95

Trp Leu Gln Leu Phe Gly Lys Asn Gln Gly Ile Asn Arg Phe Phe Gly
            100                 105                 110

Thr Glu Val Trp Asp Ile Tyr Gly Ala Asp Gly Val Thr Phe Leu Leu
        115                 120                 125

Ile Val His Ser Tyr Pro Thr Val Tyr Ile Ile Val Ser Ala Ala Leu
    130                 135                 140

Arg Gln Leu Pro Ser Asp Leu Glu Gln Ala Ala Arg Ile Ala Gly Ala
145                 150                 155                 160

Asp Thr Phe Thr Val Leu Arg Thr Ile Thr Leu Pro Leu Leu Lys Pro
                165                 170                 175

Ala Leu Leu Ser Ala Phe Thr Leu Thr Thr Val Ala Asn Leu Ala Asp
            180                 185                 190

Phe Gly Ile Pro Ala Leu Leu Gly Ser Pro Ala Arg Phe Glu Thr Leu
        195                 200                 205

Ala Thr Met Ile Tyr Arg Phe Met Glu Ser Gly Thr Val Ser Asn Pro
    210                 215                 220

Leu Gln Val Val Ser Thr Ile Gly Ile Val Leu Leu Phe Leu Gly Ile
225                 230                 235                 240

Ala Ala Val Thr Ala Asp Tyr Leu Val Ser Leu Tyr Ala Ala Ser Lys
                245                 250                 255

Leu Gln Asp Ala Gly Thr Pro His Arg Phe Thr Leu Asn Lys Ser Arg
            260                 265                 270

Ile Pro Val Ser Val Ile Thr Trp Ile Ile Ala Leu Ile Ile Thr Ala
        275                 280                 285

Ala Pro Leu Leu Gly Leu Ala Tyr Arg Ala Leu Leu Pro Ala Pro Gly
    290                 295                 300

Val Pro Phe Asn Leu Asp Asn Ile Thr Leu Asn Asn Phe Glu Ala Ala
305                 310                 315                 320

Leu Ser Asn Pro Arg Val Ile Glu Gly Phe Ser Asn Ser Leu Met Leu
                325                 330                 335

Ser Leu Gly Ala Ala Leu Ile Cys Gly Val Leu Gly Trp Leu Ile Gly
            340                 345                 350

Val Leu Ile Thr Arg Thr Gln His Phe Ala Asn Val Pro Leu Thr Leu
        355                 360                 365

Thr Val Leu Leu Pro Thr Ala Leu Pro Gly Met Ile Ile Gly Val Gly
    370                 375                 380

Trp Leu Ile Leu Gly Arg Tyr Thr Gly Ile Tyr Asn Thr Pro Trp Val

385                 390                 395                 400

Ile Leu Gly Ala Tyr Val Cys Ala Phe Thr Ala Leu Val Val Gln Ala
                405                 410                 415

Val Arg Gly Pro Leu Ser Gln Ala Pro Glu Ala Ile Glu Glu Ala Ala
            420                 425                 430

Arg Ile Ser Gly Ala Gly Arg Leu Arg Ser Ile Met Asp Thr Thr Gly
        435                 440                 445

Ala Met Ala Ile Pro Ala Ala Phe Ala Gly Ala Val Leu Val Ala Val
    450                 455                 460

Thr Ala Val Arg Glu Leu Thr Val Ser Ile Leu Leu Ile Ala Pro Gly
465                 470                 475                 480

Thr Thr Thr Leu Gly Val Gln Val Phe Asn Leu Gln Gln Ala Gly Asn
                485                 490                 495

Tyr Asn Gln Ala Ser Ala Leu Ser Leu Met Phe Ala Ile Ile Gly Ile
            500                 505                 510

Val Ala Leu Ala Leu Thr Val Arg Ser Gln Lys Glu Phe
        515                 520                 525


<210> 373
<211> 602
<212> DNA
<213> Corynebacterium glutamicum

<220>
<221> CDS
<222> (1)..(579)
<223> FRXA00368

<400> 373
tcc ctc atg tta tcc ctg ggt gca gcc cta atc tgt ggg gtg ctg gga      48
Ser Leu Met Leu Ser Leu Gly Ala Ala Leu Ile Cys Gly Val Leu Gly
  1               5                  10                  15

tgg ctg atc gga gtg ctc atc acc cga acc cag cat ttc gcc aac gta      96
Trp Leu Ile Gly Val Leu Ile Thr Arg Thr Gln His Phe Ala Asn Val
             20                  25                  30

ccg ttg aca ctc act gtg ctg ctt ccc acc gca ctg ccg ggc atg atc     144
Pro Leu Thr Leu Thr Val Leu Leu Pro Thr Ala Leu Pro Gly Met Ile
         35                  40                  45

atc ggc gtc ggc tgg ctc att ttg ggc aga tac acc gga att tac aac     192
Ile Gly Val Gly Trp Leu Ile Leu Gly Arg Tyr Thr Gly Ile Tyr Asn
     50                  55                  60

aca cct tgg gtg att ttg ggt gca tat gtg tgt gct ttt acc gcg ctg     240
Thr Pro Trp Val Ile Leu Gly Ala Tyr Val Cys Ala Phe Thr Ala Leu
 65                  70                  75                  80

gtt gtc caa gct gta cgc gga cca ctc agt caa gca ccc gaa gca atc     288
Val Val Gln Ala Val Arg Gly Pro Leu Ser Gln Ala Pro Glu Ala Ile
                 85                  90                  95

gaa gaa gcc gca cga atc agc ggc gca ggc aga tta cga tcc atc atg     336
Glu Glu Ala Ala Arg Ile Ser Gly Ala Gly Arg Leu Arg Ser Ile Met
            100                 105                 110

gac acc acc gga gcg atg gca att ccc gca gct ttc gcc ggc gca gtg     384
Asp Thr Thr Gly Ala Met Ala Ile Pro Ala Ala Phe Ala Gly Ala Val
        115                 120                 125

ctg gtt gcg gta act gcg gtt cga gag tta acc gtg tcc att ttg ctc     432
Leu Val Ala Val Thr Ala Val Arg Glu Leu Thr Val Ser Ile Leu Leu
    130                 135                 140

atc gcg ccg ggc acc acc acc ctg ggt gtg cag gtg ttc aat ttg cag     480
Ile Ala Pro Gly Thr Thr Thr Leu Gly Val Gln Val Phe Asn Leu Gln
145                 150                 155                 160

cag gcg gga aat tac aat cag gca tcg gcg ttg tcg ttg atg ttt gcg     528
Gln Ala Gly Asn Tyr Asn Gln Ala Ser Ala Leu Ser Leu Met Phe Ala
                165                 170                 175

att atc ggt atc gtg gcg ctc gcg ttg acg gtg cgc agc cag aag gag     576
Ile Ile Gly Ile Val Ala Leu Ala Leu Thr Val Arg Ser Gln Lys Glu
            180                 185                 190

ttt taggtgtcat cgatcaaatt gcg                                       602
Phe

<210> 374
<211> 193
<212> PRT
<213> Corynebacterium glutamicum

<400> 374
Ser Leu Met Leu Ser Leu Gly Ala Ala Leu Ile Cys Gly Val Leu Gly
  1               5                  10                  15

Trp Leu Ile Gly Val Leu Ile Thr Arg Thr Gln His Phe Ala Asn Val
             20                  25                  30

Pro Leu Thr Leu Thr Val Leu Leu Pro Thr Ala Leu Pro Gly Met Ile
         35                  40                  45

Ile Gly Val Gly Trp Leu Ile Leu Gly Arg Tyr Thr Gly Ile Tyr Asn
     50                  55                  60

Thr Pro Trp Val Ile Leu Gly Ala Tyr Val Cys Ala Phe Thr Ala Leu
 65                  70                  75                  80

Val Val Gln Ala Val Arg Gly Pro Leu Ser Gln Ala Pro Glu Ala Ile
                 85                  90                  95

Glu Glu Ala Ala Arg Ile Ser Gly Ala Gly Arg Leu Arg Ser Ile Met
            100                 105                 110

Asp Thr Thr Gly Ala Met Ala Ile Pro Ala Ala Phe Ala Gly Ala Val
        115                 120                 125

Leu Val Ala Val Thr Ala Val Arg Glu Leu Thr Val Ser Ile Leu Leu

130                 135                 140

Ile Ala Pro Gly Thr Thr Thr Leu Gly Val Gln Val Phe Asn Leu Gln
145                 150                 155                 160

Gln Ala Gly Asn Tyr Asn Gln Ala Ser Ala Leu Ser Leu Met Phe Ala
                165                 170                 175

Ile Ile Gly Ile Val Ala Leu Ala Leu Thr Val Arg Ser Gln Lys Glu
            180                 185                 190

Phe

<210> 375
<211> 798
<212> DNA
<213> Corynebacterium glutamicum

<220>
<221> CDS
<222> (1)..(798)
<223> FRXA00370

<400> 375
ggc aaa gcc cta tgg aat tcc gcc tat aca aca gtg ctt tct gcg gtg      48
Gly Lys Ala Leu Trp Asn Ser Ala Tyr Thr Thr Val Leu Ser Ala Val
  1               5                  10                  15

ggc gcg acc att atc ggc acg atc atg gct ctc acg ctg gac cga act      96
Gly Ala Thr Ile Ile Gly Thr Ile Met Ala Leu Thr Leu Asp Arg Thr
             20                  25                  30

gat gtt ttc ggg cgc acc gcg ttg cgg tta ttt ttg tta tcc ccg ctg     144
Asp Val Phe Gly Arg Thr Ala Leu Arg Leu Phe Leu Leu Ser Pro Leu
         35                  40                  45

ttg atc cct ccg ttt att ggg gct att gcg tgg ttg cag ctg ttc ggg     192
Leu Ile Pro Pro Phe Ile Gly Ala Ile Ala Trp Leu Gln Leu Phe Gly
     50                  55                  60

aag aac cag ggc atc aac cgg ttt ttc ggc acg gaa gtg tgg gat att     240
Lys Asn Gln Gly Ile Asn Arg Phe Phe Gly Thr Glu Val Trp Asp Ile
 65                  70                  75                  80

tac ggc gct gat ggt gtg aca ttt ttg ttg att gtg cac tcc tat ccc     288
Tyr Gly Ala Asp Gly Val Thr Phe Leu Leu Ile Val His Ser Tyr Pro
                 85                  90                  95

act gtg tac atc att gtt tcg gca gct ctg agg caa ctt cct agt gat     336
Thr Val Tyr Ile Ile Val Ser Ala Ala Leu Arg Gln Leu Pro Ser Asp
            100                 105                 110

ttg gag caa gct gca cgg atc gcg ggg gcg gat act ttt acg gtg ttg     384
Leu Glu Gln Ala Ala Arg Ile Ala Gly Ala Asp Thr Phe Thr Val Leu
        115                 120                 125

cgc acc atc aca ctc cca ctg ctc aaa cct gca ttg ttg tcg gcg ttt     432
Arg Thr Ile Thr Leu Pro Leu Leu Lys Pro Ala Leu Leu Ser Ala Phe
    130                 135                 140

act ctt acc aca gtg gcg aac ctc gcc gac ttt ggc att cca gct ctg     480
Thr Leu Thr Thr Val Ala Asn Leu Ala Asp Phe Gly Ile Pro Ala Leu
145                 150                 155                 160

ttg gga tcg cca gcg cgt ttt gaa acc tta gcc acc atg att tat cgc     528
Leu Gly Ser Pro Ala Arg Phe Glu Thr Leu Ala Thr Met Ile Tyr Arg
                165                 170                 175

ttc atg gaa tcc ggc acc gtg agc aat cca ttg cag gtg gta tcc acc     576
Phe Met Glu Ser Gly Thr Val Ser Asn Pro Leu Gln Val Val Ser Thr
            180                 185                 190

att ggc atc gtg ttg ttg ttc ctg gga atc gca gca gta acc gcg gat     624
Ile Gly Ile Val Leu Leu Phe Leu Gly Ile Ala Ala Val Thr Ala Asp
        195                 200                 205

tat ctg gtg tct ttg tac gcg gca tca aag ttg caa gac gca gga aca     672
Tyr Leu Val Ser Leu Tyr Ala Ala Ser Lys Leu Gln Asp Ala Gly Thr
    210                 215                 220

ccg cat cgc ttt act ctc aac aaa tca cga atc cca gtc agc gtg atc     720
Pro His Arg Phe Thr Leu Asn Lys Ser Arg Ile Pro Val Ser Val Ile
225                 230                 235                 240

acg tgg atc atc gcg ttg atc atc acc gcc gcc ccg ctg ctg ggt ctg     768
Thr Trp Ile Ile Ala Leu Ile Ile Thr Ala Ala Pro Leu Leu Gly Leu
                245                 250                 255

gca tac aga gca tta ctg cct gcc cca ggt                             798
Ala Tyr Arg Ala Leu Leu Pro Ala Pro Gly
            260                 265


<210> 376
<211> 266
<212> PRT
<213> Corynebacterium glutamicum

<400> 376
Gly Lys Ala Leu Trp Asn Ser Ala Tyr Thr Thr Val Leu Ser Ala Val
1               5                   10                  15

Gly Ala Thr Ile Ile Gly Thr Ile Met Ala Leu Thr Leu Asp Arg Thr
            20                  25                  30

Asp Val Phe Gly Arg Thr Ala Leu Arg Leu Phe Leu Leu Ser Pro Leu
        35                  40                  45

Leu Ile Pro Pro Phe Ile Gly Ala Ile Ala Trp Leu Gln Leu Phe Gly
    50                  55                  60

Lys Asn Gln Gly Ile Asn Arg Phe Phe Gly Thr Glu Val Trp Asp Ile
65                  70                  75                  80

Tyr Gly Ala Asp Gly Val Thr Phe Leu Leu Ile Val His Ser Tyr Pro
                85                  90                  95

Thr Val Tyr Ile Ile Val Ser Ala Ala Leu Arg Gln Leu Pro Ser Asp
            100                 105                 110

Leu Glu Gln Ala Ala Arg Ile Ala Gly Ala Asp Thr Phe Thr Val Leu
        115                 120                 125

Arg Thr Ile Thr Leu Pro Leu Leu Lys Pro Ala Leu Leu Ser Ala Phe
    130                 135                 140

Thr Leu Thr Thr Val Ala Asn Leu Ala Asp Phe Gly Ile Pro Ala Leu
145                 150                 155                 160

Leu Gly Ser Pro Ala Arg Phe Glu Thr Leu Ala Thr Met Ile Tyr Arg
                165                 170                 175

Phe Met Glu Ser Gly Thr Val Ser Asn Pro Leu Gln Val Val Ser Thr
            180                 185                 190

Ile Gly Ile Val Leu Leu Phe Leu Gly Ile Ala Ala Val Thr Ala Asp
        195                 200                 205

Tyr Leu Val Ser Leu Tyr Ala Ala Ser Lys Leu Gln Asp Ala Gly Thr
    210                 215                 220

Pro His Arg Phe Thr Leu Asn Lys Ser Arg Ile Pro Val Ser Val Ile
225                 230                 235                 240

Thr Trp Ile Ile Ala Leu Ile Ile Thr Ala Ala Pro Leu Leu Gly Leu
                245                 250                 255

Ala Tyr Arg Ala Leu Leu Pro Ala Pro Gly
            260                 265

<210> 377
<211> 749
<212> DNA
<213> Corynebacterium glutamicum

<220>
<221> CDS
<222> (1)..(726)
<223> RXN01285

<400> 377
ctc aac gtc acc atc ccc gac aac acc ttc acc gcc atc atc ggc ccc      48
Leu Asn Val Thr Ile Pro Asp Asn Thr Phe Thr Ala Ile Ile Gly Pro
  1               5                  10                  15

aac ggc tgc ggc aaa tcc acc ctg ctc cgc ggt ttc tcc cgc gtg ctc      96
Asn Gly Cys Gly Lys Ser Thr Leu Leu Arg Gly Phe Ser Arg Val Leu
             20                  25                  30

aat ccg cag cac ggc aaa gtg ctt ctc gac ggt cgg caa ctc gat tca     144
Asn Pro Gln His Gly Lys Val Leu Leu Asp Gly Arg Gln Leu Asp Ser
         35                  40                  45

ttc aag cct aaa gag atc gcc cga gaa cta ggc ctg ctg cca cag acc     192
Phe Lys Pro Lys Glu Ile Ala Arg Glu Leu Gly Leu Leu Pro Gln Thr
     50                  55                  60

tcc atc gcc cca gaa ggc atc cgg gtt tac gat ctc atc gcg cgc ggg     240
Ser Ile Ala Pro Glu Gly Ile Arg Val Tyr Asp Leu Ile Ala Arg Gly
 65                  70                  75                  80

cgc gct ccc tac caa agc ctc ata caa caa tgg cgc acc tcc gac gaa     288
Arg Ala Pro Tyr Gln Ser Leu Ile Gln Gln Trp Arg Thr Ser Asp Glu
                85                  90                  95

gac gcc gtc gcg caa gcg ctc gcc tcc acg aat ctc acc gaa ctt gca     336
Asp Ala Val Ala Gln Ala Leu Ala Ser Thr Asn Leu Thr Glu Leu Ala
            100                 105                 110

gct cgc ctc gtc gat gaa ctc tcc ggt ggc cag cgc caa cga gtg tgg     384
Ala Arg Leu Val Asp Glu Leu Ser Gly Gly Gln Arg Gln Arg Val Trp
        115                 120                 125

gtg gcc atg ttg ctc gcc cag caa aca ccg atc atg ctt ctc gac gag     432
Val Ala Met Leu Leu Ala Gln Gln Thr Pro Ile Met Leu Leu Asp Glu
    130                 135                 140

ccc acc acc ttc ctc gac atc gcc cac caa tac gaa ctc ttg gaa ttg     480
Pro Thr Thr Phe Leu Asp Ile Ala His Gln Tyr Glu Leu Leu Glu Leu
145                 150                 155                 160

ctg cgc gca ttc aac gag gcc ggg aaa act gtg gtc act gtg ctt cac     528
Leu Arg Ala Phe Asn Glu Ala Gly Lys Thr Val Val Thr Val Leu His
                165                 170                 175

gat ctc aac caa gcc gcc cgc tac gcc gac cac ctc atc gtg atg aaa     576
Asp Leu Asn Gln Ala Ala Arg Tyr Ala Asp His Leu Ile Val Met Lys
            180                 185                 190

gat ggg cac gta cat gcc acg ggc aca ccg gag gaa gtc tta act gcc     624
Asp Gly His Val His Ala Thr Gly Thr Pro Glu Glu Val Leu Thr Ala
        195                 200                 205

gag atg gtt caa gga gtt ttt ggc ctg ccc tgc atc atc tcc cca gac     672
Glu Met Val Gln Gly Val Phe Gly Leu Pro Cys Ile Ile Ser Pro Asp
    210                 215                 220

ccc gtc aca gga acc ccc acc gtc gtt ccc ctc agt cgg tct cgc gca     720
Pro Val Thr Gly Thr Pro Thr Val Val Pro Leu Ser Arg Ser Arg Ala
225                 230                 235                 240

gga gct taagtagcta cccctccaac gga                                   749
Gly Ala

<210> 378
<211> 242
<212> PRT
<213> Corynebacterium glutamicum

<400> 378
Leu Asn Val Thr Ile Pro Asp Asn Thr Phe Thr Ala Ile Ile Gly Pro
  1               5                  10                  15

Asn Gly Cys Gly Lys Ser Thr Leu Leu Arg Gly Phe Ser Arg Val Leu
             20                  25                  30

Asn Pro Gln His Gly Lys Val Leu Leu Asp Gly Arg Gln Leu Asp Ser
         35                  40                  45

Phe Lys Pro Lys Glu Ile Ala Arg Glu Leu Gly Leu Leu Pro Gln Thr
    50                  55                  60

Ser Ile Ala Pro Glu Gly Ile Arg Val Tyr Asp Leu Ile Ala Arg Gly
65                  70                  75                  80

Arg Ala Pro Tyr Gln Ser Leu Ile Gln Gln Trp Arg Thr Ser Asp Glu
                85                  90                  95

Asp Ala Val Ala Gln Ala Leu Ala Ser Thr Asn Leu Thr Glu Leu Ala
            100                 105                 110

Ala Arg Leu Val Asp Glu Leu Ser Gly Gly Gln Arg Gln Arg Val Trp
        115                 120                 125

Val Ala Met Leu Leu Ala Gln Gln Thr Pro Ile Met Leu Leu Asp Glu
    130                 135                 140

Pro Thr Thr Phe Leu Asp Ile Ala His Gln Tyr Glu Leu Leu Glu Leu
145                 150                 155                 160

Leu Arg Ala Phe Asn Glu Ala Gly Lys Thr Val Val Thr Val Leu His
                165                 170                 175

Asp Leu Asn Gln Ala Ala Arg Tyr Ala Asp His Leu Ile Val Met Lys
            180                 185                 190

Asp Gly His Val His Ala Thr Gly Thr Pro Glu Glu Val Leu Thr Ala
        195                 200                 205

Glu Met Val Gln Gly Val Phe Gly Leu Pro Cys Ile Ile Ser Pro Asp
    210                 215                 220

Pro Val Thr Gly Thr Pro Thr Val Val Pro Leu Ser Arg Ser Arg Ala
225                 230                 235                 240

Gly Ala

<210> 379
<211> 1149
<212> DNA
<213> Corynebacterium glutamicum

<220>
<221> CDS
<222> (101)..(1126)
<223> RXN00523

<400> 379
tggtgactcg tccgagtgaa attgccgtgg gcatcatcat gccgatcatt ggtgcgccac 60

tgtttatttg gattattcgt cgtcagaaag tcaaagagct atg agc ctt agc cat   115
                                           Met Ser Leu Ser His
                                           1               5

caa ctc aag cgc cag cgc gca tcg cgc aac tcc cgc agg tgg ctg att   163
Gln Leu Lys Arg Gln Arg Ala Ser Arg Asn Ser Arg Arg Trp Leu Ile
                10                  15                  20

gtt gcg gca ttg ggc gtc gtc acg ctt ggt att ttt gct ttt tct ttg 211
Val Ala Ala Leu Gly Val Val Thr Leu Gly Ile Phe Ala Phe Ser Leu
            25                  30                  35

atg tgg ggc gag gtg ttt tat ggc cct gct cag gtg ctg aaa gtg ttg 259
Met Trp Gly Glu Val Phe Tyr Gly Pro Ala Gln Val Leu Lys Val Leu
        40                  45                  50

tct gga cag cag gtt ccc ggc gcg agt tat tcc gtt ggc gtg ttg cgt 307
Ser Gly Gln Gln Val Pro Gly Ala Ser Tyr Ser Val Gly Val Leu Arg
    55                  60                  65

ttg ccg cgc gcg gtg atg ggt ttg act gcg ggt ttg gcg ttt ggc gcg 355
Leu Pro Arg Ala Val Met Gly Leu Thr Ala Gly Leu Ala Phe Gly Ala
70                  75                  80                  85

gcg ggc gtg att ttt cag acg gtg ttg cgt aat cag ttg gcg tcg ccg 403
Ala Gly Val Ile Phe Gln Thr Val Leu Arg Asn Gln Leu Ala Ser Pro
                90                  95                  100

gat att atc ggc att tct tct ggc gcg tcg gcg gcg ggc gta att tgc 451
Asp Ile Ile Gly Ile Ser Ser Gly Ala Ser Ala Ala Gly Val Ile Cys
            105                 110                 115

att gtg ttt ttc ggg atg tcg cag tct gca gtg tcg gcg att tct ttg 499
Ile Val Phe Phe Gly Met Ser Gln Ser Ala Val Ser Ala Ile Ser Leu
        120                 125                 130

tgt gcg tcc ttg gct gtg gcg ttg ttg att tat ctg gtg gcg tat cgc 547
Cys Ala Ser Leu Ala Val Ala Leu Leu Ile Tyr Leu Val Ala Tyr Arg
    135                 140                 145

ggt ggt ttt tcg gcc acg cgt ctg att ctt acc ggc att ggt att gct 595
Gly Gly Phe Ser Ala Thr Arg Leu Ile Leu Thr Gly Ile Gly Ile Ala
150                 155                 160                 165

gcg atg ctg aat tca tta gtg tcg tat tcg ctg tcc aag gct gat tct 643
Ala Met Leu Asn Ser Leu Val Ser Tyr Ser Leu Ser Lys Ala Asp Ser
                170                 175                 180

tgg gat ctg ccg acc gcg acg cgc tgg ctt acc ggc tcg ctc aat ggt 691
Trp Asp Leu Pro Thr Ala Thr Arg Trp Leu Thr Gly Ser Leu Asn Gly
            185                 190                 195

gcg acg tgg gat cgt gcg atg ccg ctg att gtc acc act gtg gta ctc 739
Ala Thr Trp Asp Arg Ala Met Pro Leu Ile Val Thr Thr Val Val Leu
        200                 205                 210

att ccg ctg ctg gtg gct aat gcg cgc aat gtg gat ctt atg cgt ttg 787
Ile Pro Leu Leu Val Ala Asn Ala Arg Asn Val Asp Leu Met Arg Leu
    215                 220                 225

ggc aat gat tcc gcg gtg ggt ttg ggc gtt gct act aat cgc acg cgc 835
Gly Asn Asp Ser Ala Val Gly Leu Gly Val Ala Thr Asn Arg Thr Arg
230                 235                 240                 245

gtc att gcg att att gcc gct gtt gcg ctc atc gcc gtt gct acc gct 883
Val Ile Ala Ile Ile Ala Ala Val Ala Leu Ile Ala Val Ala Thr Ala
                250                 255                 260

gca tgc ggc ccg atc gca ttc gtg gcg ttt gtg tct ggc ccc att gcc 931

Ala Cys Gly Pro Ile Ala Phe Val Ala Phe Val Ser Gly Pro Ile Ala
            265                 270                 275

gcg cgc att tta ggc tcc ggc gga tcg ctc atc atc ccc tcc gca ctc      979
Ala Arg Ile Leu Gly Ser Gly Gly Ser Leu Ile Ile Pro Ser Ala Leu
        280                 285                 290

atc ggc ggg ttg atc gtg ctc atc gcc gac cta att ggc caa tac ttc      1027
Ile Gly Gly Leu Ile Val Leu Ile Ala Asp Leu Ile Gly Gln Tyr Phe
    295                 300                 305

ctc ggc acc cgc tac ccc gtc gga gtt gtc acc ggc gca ttc ggc gcc      1075
Leu Gly Thr Arg Tyr Pro Val Gly Val Val Thr Gly Ala Phe Gly Ala
310                 315                 320                 325

cca ttc ctt atc tat tta ctc att cgt tcc aac cgc gcg gga gta acc      1123
Pro Phe Leu Ile Tyr Leu Leu Ile Arg Ser Asn Arg Ala Gly Val Thr
                330                 335                 340

ctg tgaccaccaa ccatcaacta tcc                                        1149
Leu

<210> 380
<211> 342
<212> PRT
<213> Corynebacterium glutamicum

<400> 380
Met Ser Leu Ser His Gln Leu Lys Arg Gln Arg Ala Ser Arg Asn Ser
  1               5                  10                  15

Arg Arg Trp Leu Ile Val Ala Ala Leu Gly Val Val Thr Leu Gly Ile
             20                  25                  30

Phe Ala Phe Ser Leu Met Trp Gly Glu Val Phe Tyr Gly Pro Ala Gln
         35                  40                  45

Val Leu Lys Val Leu Ser Gly Gln Gln Val Pro Gly Ala Ser Tyr Ser
     50                  55                  60

Val Gly Val Leu Arg Leu Pro Arg Ala Val Met Gly Leu Thr Ala Gly
 65                  70                  75                  80

Leu Ala Phe Gly Ala Ala Gly Val Ile Phe Gln Thr Val Leu Arg Asn
                 85                  90                  95

Gln Leu Ala Ser Pro Asp Ile Ile Gly Ile Ser Ser Gly Ala Ser Ala
            100                 105                 110

Ala Gly Val Ile Cys Ile Val Phe Phe Gly Met Ser Gln Ser Ala Val
        115                 120                 125

Ser Ala Ile Ser Leu Cys Ala Ser Leu Ala Val Ala Leu Leu Ile Tyr
    130                 135                 140

Leu Val Ala Tyr Arg Gly Gly Phe Ser Ala Thr Arg Leu Ile Leu Thr
145                 150                 155                 160

Gly Ile Gly Ile Ala Ala Met Leu Asn Ser Leu Val Ser Tyr Ser Leu

                165                 170                 175

Ser Lys Ala Asp Ser Trp Asp Leu Pro Thr Ala Thr Arg Trp Leu Thr
            180                 185                 190

Gly Ser Leu Asn Gly Ala Thr Trp Asp Arg Ala Met Pro Leu Ile Val
        195                 200                 205

Thr Thr Val Val Leu Ile Pro Leu Leu Val Ala Asn Ala Arg Asn Val
    210                 215                 220

Asp Leu Met Arg Leu Gly Asn Asp Ser Ala Val Gly Leu Gly Val Ala
225                 230                 235                 240

Thr Asn Arg Thr Arg Val Ile Ala Ile Ile Ala Ala Val Ala Leu Ile
                245                 250                 255

Ala Val Ala Thr Ala Ala Cys Gly Pro Ile Ala Phe Val Ala Phe Val
            260                 265                 270

Ser Gly Pro Ile Ala Ala Arg Ile Leu Gly Ser Gly Gly Ser Leu Ile
        275                 280                 285

Ile Pro Ser Ala Leu Ile Gly Gly Leu Ile Val Leu Ile Ala Asp Leu
    290                 295                 300

Ile Gly Gln Tyr Phe Leu Gly Thr Arg Tyr Pro Val Gly Val Val Thr
305                 310                 315                 320

Gly Ala Phe Gly Ala Pro Phe Leu Ile Tyr Leu Leu Ile Arg Ser Asn
                325                 330                 335

Arg Ala Gly Val Thr Leu
            340

<210> 381
<211> 621
<212> DNA
<213> Corynebacterium glutamicum

<220>
<221> CDS
<222> (101)..(598)
<223> RXN01142

<400> 381
ctccccatcc accggcacag tcagcgcagg caacgaagaa attaaaggac caggacctga 60

ccgaggcatg gttttccaag accacgccct cctgccctga ttg acc gca cgc ggc 115
Leu Thr Ala Arg Gly
1               5

aac atc gac ttc ggg ctc cgc tcc gcg cgc ccc tcc ttg agc aaa acc 163
Asn Ile Asp Phe Gly Leu Arg Ser Ala Arg Pro Ser Leu Ser Lys Thr
                10                  15                  20

gaa cgc gcc gac atc acc cgc acc cac ctc gaa caa gta ggc ctc acc 211
Glu Arg Ala Asp Ile Thr Arg Thr His Leu Glu Gln Val Gly Leu Thr
            25                  30                  35

gac gcc gcc gaa cgg cgc ccc gcc cgc ctc tcc ggc ggc atg caa cag     259
Asp Ala Ala Glu Arg Arg Pro Ala Arg Leu Ser Gly Gly Met Gln Gln
        40                  45                  50

cga gtc ggc atc gca cgc gcc ttc gcc atc gac cca cca atc atg ctt     307
Arg Val Gly Ile Ala Arg Ala Phe Ala Ile Asp Pro Pro Ile Met Leu
    55                  60                  65

ctc gac gaa ccc ttc ggc gcc ctc gac gcc ctc acc cgc cgc gaa ctc     355
Leu Asp Glu Pro Phe Gly Ala Leu Asp Ala Leu Thr Arg Arg Glu Leu
70                  75                  80                  85

cag ctc caa cta ctc aac att tgg gaa gcc tcc cgc cgc acc gtc gtc     403
Gln Leu Gln Leu Leu Asn Ile Trp Glu Ala Ser Arg Arg Thr Val Val
                90                  95                  100

atg gtc acc cac gac gtc gac gag gcc atc ctg ctc tcc gac cga gtt     451
Met Val Thr His Asp Val Asp Glu Ala Ile Leu Leu Ser Asp Arg Val
            105                 110                 115

ctc gtg atg tcc aag agc ccc gaa gcc acc atc atc acc gat att cca     499
Leu Val Met Ser Lys Ser Pro Glu Ala Thr Ile Ile Thr Asp Ile Pro
        120                 125                 130

gtg aat ctt ccc cgc ccc aga cac gag ctg agt gaa gac gct tct gtt     547
Val Asn Leu Pro Arg Pro Arg His Glu Leu Ser Glu Asp Ala Ser Val
    135                 140                 145

gaa gcc gag acc aca gcc ctg cgt aag cgg atg ctg cat ctg ctg gag     595
Glu Ala Glu Thr Thr Ala Leu Arg Lys Arg Met Leu His Leu Leu Glu
150                 155                 160                 165

cac tagtttctaa cacgtctttt aaa                                       621
His

<210> 382
<211> 166
<212> PRT
<213> Corynebacterium glutamicum

<400> 382
Leu Thr Ala Arg Gly Asn Ile Asp Phe Gly Leu Arg Ser Ala Arg Pro
1               5                   10                  15

Ser Leu Ser Lys Thr Glu Arg Ala Asp Ile Thr Arg Thr His Leu Glu
            20                  25                  30

Gln Val Gly Leu Thr Asp Ala Ala Glu Arg Arg Pro Ala Arg Leu Ser
        35                  40                  45

Gly Gly Met Gln Gln Arg Val Gly Ile Ala Arg Ala Phe Ala Ile Asp
    50                  55                  60

Pro Pro Ile Met Leu Leu Asp Glu Pro Phe Gly Ala Leu Asp Ala Leu
65                  70                  75                  80

Thr Arg Arg Glu Leu Gln Leu Gln Leu Leu Asn Ile Trp Glu Ala Ser
                85                  90                  95

Arg Arg Thr Val Val Met Val Thr His Asp Val Asp Glu Ala Ile Leu
            100                 105                 110

Leu Ser Asp Arg Val Leu Val Met Ser Lys Ser Pro Glu Ala Thr Ile
        115                 120                 125

Ile Thr Asp Ile Pro Val Asn Leu Pro Arg Pro Arg His Glu Leu Ser
    130                 135                 140

Glu Asp Ala Ser Val Glu Ala Glu Thr Thr Ala Leu Arg Lys Arg Met
145                 150                 155                 160

Leu His Leu Leu Glu His
                165

<210> 383
<211> 948
<212> DNA
<213> Corynebacterium glutamicum

<220>
<221> CDS
<222> (101)..(925)
<223> RXN01141

<400> 383
   aaagaacact cggtatggca cctgatttaa ggatgctgca atcgtgacac atatcctctt 60
   cgacagcagg cgttttctgc aactgggcgc ttttgcgtcc ttg agc acc gca ttg 115
                                              Leu Ser Thr Ala Leu
                                                1               5
   gcc gga gcg gcc cgc tac gtg acg tcg aca agc aat aat gaa cct gcg   163
   Ala Gly Ala Ala Arg Tyr Val Thr Ser Thr Ser Asn Asn Glu Pro Ala
                   10                  15                  20
   gat aac act ccc ctg acc att ggc tac gtg cct att gcg ggc tcg gcg   211
   Asp Asn Thr Pro Leu Thr Ile Gly Tyr Val Pro Ile Ala Gly Ser Ala
               25                  30                  35
   ccg att gct atc gca gat gcg cta ggg ctg ttt aag aaa cac ggc gtg   259
   Pro Ile Ala Ile Ala Asp Ala Leu Gly Leu Phe Lys Lys His Gly Val
           40                  45                  50
   aat gtc acg ttg aag aag tac tca ggc tgg tcc gac ctg tgg acc gcc   307
   Asn Val Thr Leu Lys Lys Tyr Ser Gly Trp Ser Asp Leu Trp Thr Ala
       55                  60                  65
   tat gca aca gag cag ctt gat gtt gcg cac atg ctg tcg ccg atg act   355
   Tyr Ala Thr Glu Gln Leu Asp Val Ala His Met Leu Ser Pro Met Thr
   70                  75                  80                  85
   gtg gcg att aat gct gga gtg acc aac gcg tcg cgc ccg acg gag ctg   403
   Val Ala Ile Asn Ala Gly Val Thr Asn Ala Ser Arg Pro Thr Glu Leu
                   90                  95                  100
   tcg ttt acc cag aac acc aat ggg caa gca att acc ttg gcg tca aag   451
   Ser Phe Thr Gln Asn Thr Asn Gly Gln Ala Ile Thr Leu Ala Ser Lys
               105                 110                 115
   cac tat ggt tcc gtc aat tca gcg gcg gat ctt aaa ggc atg gtg ctg     499
   His Tyr Gly Ser Val Asn Ser Ala Ala Asp Leu Lys Gly Met Val Leu
           120                 125                 130
   gga att cct ttt gaa tat tca gtc cat gcg ctg ctc ctg cgc gat tat     547
   Gly Ile Pro Phe Glu Tyr Ser Val His Ala Leu Leu Leu Arg Asp Tyr
       135                 140                 145
   ctc gtc tca aac gca gtt gat ccc atc gcc gat ctt gag ctt cgc ctg     595
   Leu Val Ser Asn Ala Val Asp Pro Ile Ala Asp Leu Glu Leu Arg Leu
   150                 155                 160                 165
   ctc cga cct gcc gat atg gtc gca caa ttg aca gtt gag ggc atc gat     643
   Leu Arg Pro Ala Asp Met Val Ala Gln Leu Thr Val Glu Gly Ile Asp
                   170                 175                 180
   gga ttc att ggg cct ggg ccg ttt aat gaa cgc gcc atc agc aat ggc     691
   Gly Phe Ile Gly Pro Gly Pro Phe Asn Glu Arg Ala Ile Ser Asn Gly
               185                 190                 195
   tcc ggc cgg att tgg ctg ctg acc aaa caa ctg tgg gac aaa cat cca     739
   Ser Gly Arg Ile Trp Leu Leu Thr Lys Gln Leu Trp Asp Lys His Pro
           200                 205                 210
   tgc tgc gcc gtg gcg atg gcc aaa gag tgg aaa gct gaa cac ccc acg     787
   Cys Cys Ala Val Ala Met Ala Lys Glu Trp Lys Ala Glu His Pro Thr
       215                 220                 225
   gcg gct cag ggt gtg ctt aat gcg ctg gag gaa gcc tcc gca att ttg     835
   Ala Ala Gln Gly Val Leu Asn Ala Leu Glu Glu Ala Ser Ala Ile Leu
   230                 235                 240                 245
   agc aat ccg gca caa ttt gat tcc tcg gca cgc acg ctg tcg cag gaa     883
   Ser Asn Pro Ala Gln Phe Asp Ser Ser Ala Arg Thr Leu Ser Gln Glu
                   250                 255                 260
   aaa tac ctc aac cag cct gcc acg ttg ctg gat gga ccg tcg             925
   Lys Tyr Leu Asn Gln Pro Ala Thr Leu Leu Asp Gly Pro Ser
               265                 270                 275
   taatcatcgg catcaccggc tta                                           948


<210> 384
<211> 275
<212> PRT
<213> Corynebacterium glutamicum

<400> 384
Leu Ser Thr Ala Leu Ala Gly Ala Ala Arg Tyr Val Thr Ser Thr Ser
 1               5                  10                  15

Asn Asn Glu Pro Ala Asp Asn Thr Pro Leu Thr Ile Gly Tyr Val Pro
            20                  25                  30

Ile Ala Gly Ser Ala Pro Ile Ala Ile Ala Asp Ala Leu Gly Leu Phe
        35                  40                  45

Lys Lys His Gly Val Asn Val Thr Leu Lys Lys Tyr Ser Gly Trp Ser
    50                  55                  60

Asp Leu Trp Thr Ala Tyr Ala Thr Glu Gln Leu Asp Val Ala His Met
65                  70                  75                  80

Leu Ser Pro Met Thr Val Ala Ile Asn Ala Gly Val Thr Asn Ala Ser
                85                  90                  95

Arg Pro Thr Glu Leu Ser Phe Thr Gln Asn Thr Asn Gly Gln Ala Ile
            100                 105                 110

Thr Leu Ala Ser Lys His Tyr Gly Ser Val Asn Ser Ala Ala Asp Leu
        115                 120                 125

Lys Gly Met Val Leu Gly Ile Pro Phe Glu Tyr Ser Val His Ala Leu
    130                 135                 140

Leu Leu Arg Asp Tyr Leu Val Ser Asn Ala Val Asp Pro Ile Ala Asp
145                 150                 155                 160

Leu Glu Leu Arg Leu Leu Arg Pro Ala Asp Met Val Ala Gln Leu Thr
                165                 170                 175

Val Glu Gly Ile Asp Gly Phe Ile Gly Pro Gly Pro Phe Asn Glu Arg
            180                 185                 190

Ala Ile Ser Asn Gly Ser Gly Arg Ile Trp Leu Leu Thr Lys Gln Leu
        195                 200                 205

Trp Asp Lys His Pro Cys Cys Ala Val Ala Met Ala Lys Glu Trp Lys
    210                 215                 220

Ala Glu His Pro Thr Ala Ala Gln Gly Val Leu Asn Ala Leu Glu Glu
225                 230                 235                 240

Ala Ser Ala Ile Leu Ser Asn Pro Ala Gln Phe Asp Ser Ser Ala Arg
                245                 250                 255

Thr Leu Ser Gln Glu Lys Tyr Leu Asn Gln Pro Ala Thr Leu Leu Asp
            260                 265                 270

Gly Pro Ser
        275


<210> 385
<211> 927
<212> DNA
<213> Corynebacterium glutamicum

<220>
<221> CDS
<222> (101)..(904)
<223> RXN01002

<400> 385
gactgctgat accgcacagg atgaaatcac tcgttacggc gagatcctga agaagttctc 60

caactaattt ccctgtttcc aatactcaag gtgtgcgcat atg aat tct gat gct    115
                                           Met Asn Ser Asp Ala
                                           1               5

tcg gct acc acc aac tcc tgg gct atc aac ttc gac cat gtg tcg gtg    163

Ser Ala Thr Thr Asn Ser Trp Ala Ile Asn Phe Asp His Val Ser Val
                10                  15                  20

acg tat ccc aat ggg acg aaa gcc ctc gat gat gtt tcc ctc acc atc     211
Thr Tyr Pro Asn Gly Thr Lys Ala Leu Asp Asp Val Ser Leu Thr Ile
            25                  30                  35

aat ccc ggt gag atg gtt gcc atc gtg ggt ctg tca gga tcg ggt aaa     259
Asn Pro Gly Glu Met Val Ala Ile Val Gly Leu Ser Gly Ser Gly Lys
        40                  45                  50

tcc acg ctg att cgc acg atc aac ggt ctt gtc cgc gct acg gaa ggc     307
Ser Thr Leu Ile Arg Thr Ile Asn Gly Leu Val Arg Ala Thr Glu Gly
    55                  60                  65

acc gtg acg gtg ggg ccg cat cag atc aac acc ttg aag ggg aaa gca     355
Thr Val Thr Val Gly Pro His Gln Ile Asn Thr Leu Lys Gly Lys Ala
70                  75                  80                  85

ctg cgt gat gcc cgt ggg cag atc ggc atg att ttc cag ggg ttc aac     403
Leu Arg Asp Ala Arg Gly Gln Ile Gly Met Ile Phe Gln Gly Phe Asn
                90                  95                  100

ctg tcg gaa cgc agc agt gtg ttc cag aat gtt ttg gtg ggc cgc ttc     451
Leu Ser Glu Arg Ser Ser Val Phe Gln Asn Val Leu Val Gly Arg Phe
            105                 110                 115

gcg cac aca gcg tgg tgg cgt aac ctc ctc ggg ttt ccc acg gag cac     499
Ala His Thr Ala Trp Trp Arg Asn Leu Leu Gly Phe Pro Thr Glu His
        120                 125                 130

gac aag cag att gct ttt cac gcg ttg gag tcc gtg ggc att ttg cac     547
Asp Lys Gln Ile Ala Phe His Ala Leu Glu Ser Val Gly Ile Leu His
    135                 140                 145

aaa gtg tgg acc cga gct ggt gct ttg tcg ggt gga cag aaa cag cgc     595
Lys Val Trp Thr Arg Ala Gly Ala Leu Ser Gly Gly Gln Lys Gln Arg
150                 155                 160                 165

gtt gct att gcg cgc gcc tta tcg caa gat ccg tct gtc atg ctg gca     643
Val Ala Ile Ala Arg Ala Leu Ser Gln Asp Pro Ser Val Met Leu Ala
                170                 175                 180

gat gag cct gtg gca agc ctt gat ccg cca acc gcg cat tcc gtg atg     691
Asp Glu Pro Val Ala Ser Leu Asp Pro Pro Thr Ala His Ser Val Met
            185                 190                 195

cgc gat cta gaa aac atc aac aac gtg gaa ggc ctc acc gtg ttg gtg     739
Arg Asp Leu Glu Asn Ile Asn Asn Val Glu Gly Leu Thr Val Leu Val
        200                 205                 210

aac ttg cac ttg att gat ttg gct cgt caa tac acc aca agg ctt gtg     787
Asn Leu His Leu Ile Asp Leu Ala Arg Gln Tyr Thr Thr Arg Leu Val
    215                 220                 225

ggt ttg cgt gcc ggc aag ctg gtc tat gac ggt cct atc tct gag gcc     835
Gly Leu Arg Ala Gly Lys Leu Val Tyr Asp Gly Pro Ile Ser Glu Ala
230                 235                 240                 245

acc gat aaa gac ttt gaa gct atc tat ggt cgc ccc atc cag gct aaa     883
Thr Asp Lys Asp Phe Glu Ala Ile Tyr Gly Arg Pro Ile Gln Ala Lys

250                 255                 260

gac ctg cta ggt gat cgc gca tgaccacgcc ttcttctaca ctt          927
Asp Leu Leu Gly Asp Arg Ala
            265

<210> 386
<211> 268
<212> PRT
<213> Corynebacterium glutamicum

<400> 386
Met Asn Ser Asp Ala Ser Ala Thr Thr Asn Ser Trp Ala Ile Asn Phe
  1               5                  10                  15

Asp His Val Ser Val Thr Tyr Pro Asn Gly Thr Lys Ala Leu Asp Asp
             20                  25                  30

Val Ser Leu Thr Ile Asn Pro Gly Glu Met Val Ala Ile Val Gly Leu
         35                  40                  45

Ser Gly Ser Gly Lys Ser Thr Leu Ile Arg Thr Ile Asn Gly Leu Val
     50                  55                  60

Arg Ala Thr Glu Gly Thr Val Thr Val Gly Pro His Gln Ile Asn Thr
 65                  70                  75                  80

Leu Lys Gly Lys Ala Leu Arg Asp Ala Arg Gly Gln Ile Gly Met Ile
                 85                  90                  95

Phe Gln Gly Phe Asn Leu Ser Glu Arg Ser Ser Val Phe Gln Asn Val
            100                 105                 110

Leu Val Gly Arg Phe Ala His Thr Ala Trp Trp Arg Asn Leu Leu Gly
        115                 120                 125

Phe Pro Thr Glu His Asp Lys Gln Ile Ala Phe His Ala Leu Glu Ser
    130                 135                 140

Val Gly Ile Leu His Lys Val Trp Thr Arg Ala Gly Ala Leu Ser Gly
145                 150                 155                 160

Gly Gln Lys Gln Arg Val Ala Ile Ala Arg Ala Leu Ser Gln Asp Pro
                165                 170                 175

Ser Val Met Leu Ala Asp Glu Pro Val Ala Ser Leu Asp Pro Pro Thr
            180                 185                 190

Ala His Ser Val Met Arg Asp Leu Glu Asn Ile Asn Asn Val Glu Gly
        195                 200                 205

Leu Thr Val Leu Val Asn Leu His Leu Ile Asp Leu Ala Arg Gln Tyr
    210                 215                 220

Thr Thr Arg Leu Val Gly Leu Arg Ala Gly Lys Leu Val Tyr Asp Gly
225                 230                 235                 240

Pro Ile Ser Glu Ala Thr Asp Lys Asp Phe Glu Ala Ile Tyr Gly Arg
                245                 250                 255

Pro Ile Gln Ala Lys Asp Leu Leu Gly Asp Arg Ala
            260                 265

<210> 387
<211> 969
<212> DNA
<213> Corynebacterium glutamicum

<220>
<221> CDS
<222> (101)..(946)
<223> RXN01000

<400> 387
ctttctatgc ctacgcggat gtttccgtga tcattctgga aatcctcatc gtggtgattg 60

tcattgaagt aatctccaac gcacttcgaa agaggctggt atg agc acc tta acc   115
                                           Met Ser Thr Leu Thr
                                           1               5

tct cac cgc aca gta ccg gcc ccc agc tct ccc ccg gcg cgc ccc aac   163
Ser His Arg Thr Val Pro Ala Pro Ser Ser Pro Pro Ala Arg Pro Asn
                10                  15                  20

aaa ctg gcg cgc aat atc gtt gca att gtc gct gcg ctg att gtc ctt   211
Lys Leu Ala Arg Asn Ile Val Ala Ile Val Ala Ala Leu Ile Val Leu
            25                  30                  35

ata gct acc ggc acg ctc aag atc gag tgg aat gag ctt ccg cag atg   259
Ile Ala Thr Gly Thr Leu Lys Ile Glu Trp Asn Glu Leu Pro Gln Met
        40                  45                  50

ccc gcg cag gtg tgg cat tac tta gag ctg atg ttt agc gat ccc gat   307
Pro Ala Gln Val Trp His Tyr Leu Glu Leu Met Phe Ser Asp Pro Asp
    55                  60                  65

tgg tcg aag ttt ggc cgc gcc gtc cag gaa atg tgg cgt tcc atc gcc   355
Trp Ser Lys Phe Gly Arg Ala Val Gln Glu Met Trp Arg Ser Ile Ala
70                  75                  80                  85

atg gcg tgg ttg ggt gcc att tta tgc gtg gtg gtc tct gtc cct ctg   403
Met Ala Trp Leu Gly Ala Ile Leu Cys Val Val Val Ser Val Pro Leu
                90                  95                  100

gga atg ttg gct gcc cgc ggg gtg gga cct tat tgg ctg cgt acc gtt   451
Gly Met Leu Ala Ala Arg Gly Val Gly Pro Tyr Trp Leu Arg Thr Val
            105                 110                 115

tta cgg ttc gtg ttc gcg gtg att cgt gcg ttc ccc gaa gtg gtt atc   499
Leu Arg Phe Val Phe Ala Val Ile Arg Ala Phe Pro Glu Val Val Ile
        120                 125                 130

gca att att ttg cta act gtc acc ggc cta act cct ttt act ggt gcg   547
Ala Ile Ile Leu Leu Thr Val Thr Gly Leu Thr Pro Phe Thr Gly Ala
    135                 140                 145

ctc gca ttg ggt atc tcc ggt att gga caa cag gca aag tgg acc tat   595
Leu Ala Leu Gly Ile Ser Gly Ile Gly Gln Gln Ala Lys Trp Thr Tyr
150                 155                 160                 165

gaa gcc att gag tcc act ccc acc ggc ccg tca gag gca gtg cgt gca     643
Glu Ala Ile Glu Ser Thr Pro Thr Gly Pro Ser Glu Ala Val Arg Ala
                170                 175                 180

gcg ggt gga act acg ccg gag gtt ctg cgg tgg gcg ttg tgg cca cag     691
Ala Gly Gly Thr Thr Pro Glu Val Leu Arg Trp Ala Leu Trp Pro Gln
            185                 190                 195

gtt gcg cca tcc att gca tct ttt gcc ctg tac cgc ttt gag atc aac     739
Val Ala Pro Ser Ile Ala Ser Phe Ala Leu Tyr Arg Phe Glu Ile Asn
        200                 205                 210

atc cgt acc tct gcg gta ttg ggc atc gtt ggt gca ggt ggt atc ggt     787
Ile Arg Thr Ser Ala Val Leu Gly Ile Val Gly Ala Gly Gly Ile Gly
    215                 220                 225

agt atg ctt gcc aat tac acc aac tac agg cag tgg gac acc gtg ggc     835
Ser Met Leu Ala Asn Tyr Thr Asn Tyr Arg Gln Trp Asp Thr Val Gly
230                 235                 240                 245

atg ctg ctc atc gtc gtg gtt gtc gca acg atg atc gtc gat ctc atc     883
Met Leu Leu Ile Val Val Val Val Ala Thr Met Ile Val Asp Leu Ile
                250                 255                 260

tcc ggc acc atc cgc cgc cgc atc atg aag ggg gct agt gac cgt gtc     931
Ser Gly Thr Ile Arg Arg Arg Ile Met Lys Gly Ala Ser Asp Arg Val
            265                 270                 275

gtg gca cca agc aac tgacgctcca ccaagcatcc gca                       969
Val Ala Pro Ser Asn
        280

<210> 388
<211> 282
<212> PRT
<213> Corynebacterium glutamicum

<400> 388
   Met Ser Thr Leu Thr Ser His Arg Thr Val Pro Ala Pro Ser Ser Pro
     1               5                  10                  15
   Pro Ala Arg Pro Asn Lys Leu Ala Arg Asn Ile Val Ala Ile Val Ala
               20                  25                  30
   Ala Leu Ile Val Leu Ile Ala Thr Gly Thr Leu Lys Ile Glu Trp Asn
           35                  40                  45
   Glu Leu Pro Gln Met Pro Ala Gln Val Trp His Tyr Leu Glu Leu Met
       50                  55                  60
   Phe Ser Asp Pro Asp Trp Ser Lys Phe Gly Arg Ala Val Gln Glu Met
    65                  70                  75                  80
   Trp Arg Ser Ile Ala Met Ala Trp Leu Gly Ala Ile Leu Cys Val Val
                   85                  90                  95
   Val Ser Val Pro Leu Gly Met Leu Ala Ala Arg Gly Val Gly Pro Tyr
               100                 105                 110
   Trp Leu Arg Thr Val Leu Arg Phe Val Phe Ala Val Ile Arg Ala Phe
   115 120 125
   Pro Glu Val Val Ile Ala Ile Ile Leu Leu Thr Val Thr Gly Leu Thr
   130 135 140
   Pro Phe Thr Gly Ala Leu Ala Leu Gly Ile Ser Gly Ile Gly Gln Gln
   145 150 155 160
   Ala Lys Trp Thr Tyr Glu Ala Ile Glu Ser Thr Pro Thr Gly Pro Ser
   165 170 175
   Glu Ala Val Arg Ala Ala Gly Gly Thr Thr Pro Glu Val Leu Arg Trp
   180 185 190
   Ala Leu Trp Pro Gln Val Ala Pro Ser Ile Ala Ser Phe Ala Leu Tyr
   195 200 205
   Arg Phe Glu Ile Asn Ile Arg Thr Ser Ala Val Leu Gly Ile Val Gly
   210 215 220
   Ala Gly Gly Ile Gly Ser Met Leu Ala Asn Tyr Thr Asn Tyr Arg Gln
   225 230 235 240
   Trp Asp Thr Val Gly Met Leu Leu Ile Val Val Val Val Ala Thr Met
   245 250 255
   Ile Val Asp Leu Ile Ser Gly Thr Ile Arg Arg Arg Ile Met Lys Gly
   260 265 270
   Ala Ser Asp Arg Val Val Ala Pro Ser Asn
   275 280

<210> 389
<211> 1173
<212> DNA
<213> Corynebacterium glutamicum

<220>
<221> CDS
<222> (101)..(1150)
<223> RXN01732

<400> 389
gattctatcg ctgatctccc tctccttgcc tgggtttgtt cccgggtctc ctcttcttaa 60

ctttcttgtc tcatgtcgct gaaaggtttt taaagatctc atg ttc aag ctc tct   115
                                           Met Phe Lys Leu Ser
                                           1               5

aag cca tcc aag tcc atg cgt gtt gct gtt tct acg ctt gcg atc tct   163
Lys Pro Ser Lys Ser Met Arg Val Ala Val Ser Thr Leu Ala Ile Ser
                10                  15                  20

acc ctt gct cta gtt ggt tgt tcc tct tcc gat gag tct tct tcc tca   211
Thr Leu Ala Leu Val Gly Cys Ser Ser Ser Asp Glu Ser Ser Ser Ser
            25                  30                  35

tca tct gcg tct tct tct tcg gat gct gca agc cag tgg cct gag tcc   259
Ser Ser Ala Ser Ser Ser Ser Asp Ala Ala Ser Gln Trp Pro Glu Ser
        40                  45                  50

att act ttg tct ctt gtt cct tct act gag ggt gag gat ttg gct gaa 307
Ile Thr Leu Ser Leu Val Pro Ser Thr Glu Gly Glu Asp Leu Ala Glu
    55                  60                  65

gcg ttg gct cct ttg act gat tac ctg tct gag aac ctt ggt att gag 355
Ala Leu Ala Pro Leu Thr Asp Tyr Leu Ser Glu Asn Leu Gly Ile Glu
70                  75                  80                  85

gtc aat ggt gtg gtg gcg tct gat tac gct gca acc gtt gag gct ttg 403
Val Asn Gly Val Val Ala Ser Asp Tyr Ala Ala Thr Val Glu Ala Leu
                90                  95                  100

ggt gct gat cag gct cag gtg atc atc act gat gcg ggt tcc ctg tat 451
Gly Ala Asp Gln Ala Gln Val Ile Ile Thr Asp Ala Gly Ser Leu Tyr
            105                 110                 115

aac gcg att gag cag tac gat gcg cag ctg att ctg cgt gat gtg cgt 499
Asn Ala Ile Glu Gln Tyr Asp Ala Gln Leu Ile Leu Arg Asp Val Arg
        120                 125                 130

ttc ggt gcc acc tcg tac tct gct gtg gcg tac acc aac aat cct gat 547
Phe Gly Ala Thr Ser Tyr Ser Ala Val Ala Tyr Thr Asn Asn Pro Asp
    135                 140                 145

aag tac tgc gac gat gcc cca gtg gct gcg tct tat gct gcg tcc gat 595
Lys Tyr Cys Asp Asp Ala Pro Val Ala Ala Ser Tyr Ala Ala Ser Asp
150                 155                 160                 165

gta gac atg ctt tac tgc aac ggt att gaa act gag ggc cag gct gct 643
Val Asp Met Leu Tyr Cys Asn Gly Ile Glu Thr Glu Gly Gln Ala Ala
                170                 175                 180

acc ggt gag ggc cca gca gct ctt gat gcg ctg gaa aag atc gag tcc 691
Thr Gly Glu Gly Pro Ala Ala Leu Asp Ala Leu Glu Lys Ile Glu Ser
            185                 190                 195

ggt gac aag gta gcg ctg cag gct gca acc tct cct gcg ggt tac cag 739
Gly Asp Lys Val Ala Leu Gln Ala Ala Thr Ser Pro Ala Gly Tyr Gln
        200                 205                 210

tac cct atc gtc gct atg cag gat ctg ggc atg gat acc gat tcc gct 787
Tyr Pro Ile Val Ala Met Gln Asp Leu Gly Met Asp Thr Asp Ser Ala
    215                 220                 225

ttt gtt cag gtt cca gta gag ggc aac aac aac gct gtg ctg tct gtc 835
Phe Val Gln Val Pro Val Glu Gly Asn Asn Asn Ala Val Leu Ser Val
230                 235                 240                 245

ctg aac ggt gac gcg gaa gtg tcc ttc ggt ttc tgg gat gcg cgt tcc 883
Leu Asn Gly Asp Ala Glu Val Ser Phe Gly Phe Trp Asp Ala Arg Ser
                250                 255                 260

acc gtg ctg tct gag gct cct aac gca gct gag gat gtc gta gcg ttt 931
Thr Val Leu Ser Glu Ala Pro Asn Ala Ala Glu Asp Val Val Ala Phe
            265                 270                 275

gcc tac acc gag atg atc cct aac ggt ggc gtc gca gcg tcc aag tcc 979
Ala Tyr Thr Glu Met Ile Pro Asn Gly Gly Val Ala Ala Ser Lys Ser
        280                 285                 290

ctt cca tcc gac ctg gtg gaa aag ctc acc gag ttg atg gat gat tac 1027
Leu Pro Ser Asp Leu Val Glu Lys Leu Thr Glu Leu Met Asp Asp Tyr
    295                 300                 305

gca gat tcc tcc gag gaa gcc aag gat gtc atg ttc gac atg gtt ggt 1075
Ala Asp Ser Ser Glu Glu Ala Lys Asp Val Met Phe Asp Met Val Gly
310                 315                 320                 325

cta tct gac tgg act gct gat acc gca cag gat gaa atc act cgt tac 1123
Leu Ser Asp Trp Thr Ala Asp Thr Ala Gln Asp Glu Ile Thr Arg Tyr
                330                 335                 340

ggc gag atc ctg aag aag ttc tcc aac taatttccct gtttccaata 1170
Gly Glu Ile Leu Lys Lys Phe Ser Asn
            345                 350

ctc 1173

<210> 390
<211> 350
<212> PRT
<213> Corynebacterium glutamicum

<400> 390
Met Phe Lys Leu Ser Lys Pro Ser Lys Ser Met Arg Val Ala Val Ser
1               5                   10                  15

Thr Leu Ala Ile Ser Thr Leu Ala Leu Val Gly Cys Ser Ser Ser Asp
            20                  25                  30

Glu Ser Ser Ser Ser Ser Ser Ala Ser Ser Ser Ser Asp Ala Ala Ser
        35                  40                  45

Gln Trp Pro Glu Ser Ile Thr Leu Ser Leu Val Pro Ser Thr Glu Gly
    50                  55                  60

Glu Asp Leu Ala Glu Ala Leu Ala Pro Leu Thr Asp Tyr Leu Ser Glu
65                  70                  75                  80

Asn Leu Gly Ile Glu Val Asn Gly Val Val Ala Ser Asp Tyr Ala Ala
                85                  90                  95

Thr Val Glu Ala Leu Gly Ala Asp Gln Ala Gln Val Ile Ile Thr Asp
            100                 105                 110

Ala Gly Ser Leu Tyr Asn Ala Ile Glu Gln Tyr Asp Ala Gln Leu Ile
        115                 120                 125

Leu Arg Asp Val Arg Phe Gly Ala Thr Ser Tyr Ser Ala Val Ala Tyr
    130                 135                 140

Thr Asn Asn Pro Asp Lys Tyr Cys Asp Asp Ala Pro Val Ala Ala Ser
145                 150                 155                 160

Tyr Ala Ala Ser Asp Val Asp Met Leu Tyr Cys Asn Gly Ile Glu Thr
                165                 170                 175

Glu Gly Gln Ala Ala Thr Gly Glu Gly Pro Ala Ala Leu Asp Ala Leu
            180                 185                 190

Glu Lys Ile Glu Ser Gly Asp Lys Val Ala Leu Gln Ala Ala Thr Ser
        195                 200                 205

Pro Ala Gly Tyr Gln Tyr Pro Ile Val Ala Met Gln Asp Leu Gly Met
    210                 215                 220

Asp Thr Asp Ser Ala Phe Val Gln Val Pro Val Glu Gly Asn Asn Asn
225                 230                 235                 240

Ala Val Leu Ser Val Leu Asn Gly Asp Ala Glu Val Ser Phe Gly Phe
                245                 250                 255

Trp Asp Ala Arg Ser Thr Val Leu Ser Glu Ala Pro Asn Ala Ala Glu
            260                 265                 270

Asp Val Val Ala Phe Ala Tyr Thr Glu Met Ile Pro Asn Gly Gly Val
        275                 280                 285

Ala Ala Ser Lys Ser Leu Pro Ser Asp Leu Val Glu Lys Leu Thr Glu
    290                 295                 300

Leu Met Asp Asp Tyr Ala Asp Ser Ser Glu Glu Ala Lys Asp Val Met
305                 310                 315                 320

Phe Asp Met Val Gly Leu Ser Asp Trp Thr Ala Asp Thr Ala Gln Asp
                325                 330                 335

Glu Ile Thr Arg Tyr Gly Glu Ile Leu Lys Lys Phe Ser Asn
            340                 345                 350

<210> 391
<211> 903
<212> DNA
<213> Corynebacterium glutamicum

<220>
<221> CDS
<222> (101)..(880)
<223> RXN03080

<400> 391
cttgcaaaca ggcgtggtgg tggcgttcat tggctcacca attttccttt atttactgct 60

cagcatgcgc aagcgacgcg gattggggct gtaaaaactc atg cct caa tta gtt 115
Met Pro Gln Leu Val
1               5

gaa att cgt gat ctc aac gtt gaa ttc ccc tct cgc cat gca gtg aaa 163
Glu Ile Arg Asp Leu Asn Val Glu Phe Pro Ser Arg His Ala Val Lys
                10                  15                  20

aac gtg tct ttt tct gca cct gct gga aaa gtc acc gca ctg att ggc 211
Asn Val Ser Phe Ser Ala Pro Ala Gly Lys Val Thr Ala Leu Ile Gly
            25                  30                  35

cca aat ggt gct ggt aaa agt act gcc ctt tcg gcg att gca gga ttg 259
Pro Asn Gly Ala Gly Lys Ser Thr Ala Leu Ser Ala Ile Ala Gly Leu
        40                  45                  50

gtt gaa tcc acc ggc gag gta atg gtt ggt ggg agt ggg gtt gcg tcg 307

Val Glu Ser Thr Gly Glu Val Met Val Gly Gly Ser Gly Val Ala Ser
    55                  60                  65

aaa agc gct aaa gcc cga gcc cgc ctg ctc tca ctc gtg ccg caa aac 355
Lys Ser Ala Lys Ala Arg Ala Arg Leu Leu Ser Leu Val Pro Gln Asn
70                  75                  80                  85

acc gag ttg cgc att ggt ttt agt gca cgc gac gtt gtc gcg atg ggc 403
Thr Glu Leu Arg Ile Gly Phe Ser Ala Arg Asp Val Val Ala Met Gly
                90                  95                  100

cgc tac ccg cat cgt ggc cgc ttc gcc gtg gag acc gac gca gat cga 451
Arg Tyr Pro His Arg Gly Arg Phe Ala Val Glu Thr Asp Ala Asp Arg
            105                 110                 115

cgc gcc acc gat gac gcc ctg cgc gcc atc aac gcg ctc gac atc gcc 499
Arg Ala Thr Asp Asp Ala Leu Arg Ala Ile Asn Ala Leu Asp Ile Ala
        120                 125                 130

gag cag ccc gtc aac gaa tta tcg ggc ggc cag cag cag ctc atc cac 547
Glu Gln Pro Val Asn Glu Leu Ser Gly Gly Gln Gln Gln Leu Ile His
    135                 140                 145

atc ggc cga gcg ctc gcc caa gac acc gcc gtc gtg ctt ctc gac gag 595
Ile Gly Arg Ala Leu Ala Gln Asp Thr Ala Val Val Leu Leu Asp Glu
150                 155                 160                 165

ccc gtc tcc gcc ctt gat cta cgg cac caa gtt gaa gtc ctt caa ctc 643
Pro Val Ser Ala Leu Asp Leu Arg His Gln Val Glu Val Leu Gln Leu
                170                 175                 180

ctg cgc gcc cga gct aat tcc ggc acc acc gtg atc gtc gtc ctt cac 691
Leu Arg Ala Arg Ala Asn Ser Gly Thr Thr Val Ile Val Val Leu His
            185                 190                 195

gat ctc aac cac gtt gcc cgt tgg tgc gac cat gca gtg ttg atg gcc 739
Asp Leu Asn His Val Ala Arg Trp Cys Asp His Ala Val Leu Met Ala
        200                 205                 210

gac ggc gaa gtt gtc tcc caa ggt gac atc cgc gag gtg ctc gaa cct 787
Asp Gly Glu Val Val Ser Gln Gly Asp Ile Arg Glu Val Leu Glu Pro
    215                 220                 225

gcc aca ctg tcc acc gtg tac gga ctg ccc att gcg gtg cgc gat gat 835
Ala Thr Leu Ser Thr Val Tyr Gly Leu Pro Ile Ala Val Arg Asp Asp
230                 235                 240                 245

ccc gaa acc agc tca ctt cgc gtg atc ccg cat cca aat ccc ttt 880
Pro Glu Thr Ser Ser Leu Arg Val Ile Pro His Pro Asn Pro Phe
                250                 255                 260

tgattgaaag tttgacttaa aaa 903

<210> 392
<211> 260
<212> PRT
<213> Corynebacterium glutamicum

<400> 392
Met Pro Gln Leu Val Glu Ile Arg Asp Leu Asn Val Glu Phe Pro Ser

1                   5                   10                  15

Arg His Ala Val Lys Asn Val Ser Phe Ser Ala Pro Ala Gly Lys Val
                20                  25                  30

Thr Ala Leu Ile Gly Pro Asn Gly Ala Gly Lys Ser Thr Ala Leu Ser
            35                  40                  45

Ala Ile Ala Gly Leu Val Glu Ser Thr Gly Glu Val Met Val Gly Gly
    50                  55                  60

Ser Gly Val Ala Ser Lys Ser Ala Lys Ala Arg Ala Arg Leu Leu Ser
65                  70                  75                  80

Leu Val Pro Gln Asn Thr Glu Leu Arg Ile Gly Phe Ser Ala Arg Asp
                85                  90                  95

Val Val Ala Met Gly Arg Tyr Pro His Arg Gly Arg Phe Ala Val Glu
            100                 105                 110

Thr Asp Ala Asp Arg Arg Ala Thr Asp Asp Ala Leu Arg Ala Ile Asn
        115                 120                 125

Ala Leu Asp Ile Ala Glu Gln Pro Val Asn Glu Leu Ser Gly Gly Gln
    130                 135                 140

Gln Gln Leu Ile His Ile Gly Arg Ala Leu Ala Gln Asp Thr Ala Val
145                 150                 155                 160

Val Leu Leu Asp Glu Pro Val Ser Ala Leu Asp Leu Arg His Gln Val
                165                 170                 175

Glu Val Leu Gln Leu Leu Arg Ala Arg Ala Asn Ser Gly Thr Thr Val
            180                 185                 190

Ile Val Val Leu His Asp Leu Asn His Val Ala Arg Trp Cys Asp His
        195                 200                 205

Ala Val Leu Met Ala Asp Gly Glu Val Val Ser Gln Gly Asp Ile Arg
    210                 215                 220

Glu Val Leu Glu Pro Ala Thr Leu Ser Thr Val Tyr Gly Leu Pro Ile
225                 230                 235                 240

Ala Val Arg Asp Asp Pro Glu Thr Ser Ser Leu Arg Val Ile Pro His
                245                 250                 255

Pro Asn Pro Phe
            260

<210> 393
<211> 582
<212> DNA
<213> Corynebacterium glutamicum

<220>
<221> CDS
<222> (101)..(559)
<223> RXN03081

<400> 393
acggactgcc cattgcggtg cgcgatgatc ccgaaaccag ctcacttcgc gtgatcccgc 60

atccaaatcc cttttgattg aaagtttgac ttaaaaaccc atg aaa aaa tca ctc 115
Met Lys Lys Ser Leu
1               5

atc gcc att gtt gcc agt gcg ctc gtg tta agc ggc tgc acc tct gat 163
Ile Ala Ile Val Ala Ser Ala Leu Val Leu Ser Gly Cys Thr Ser Asp
                10                  15                  20

tct tct gac tct tcc ggc act tcc gga act gtg gaa acc act tcg att 211
Ser Ser Asp Ser Ser Gly Thr Ser Gly Thr Val Glu Thr Thr Ser Ile
            25                  30                  35

aca acc agc gtt gcc gca gct gac ggc gca ttc cca cgc acc gtc aca 259
Thr Thr Ser Val Ala Ala Ala Asp Gly Ala Phe Pro Arg Thr Val Thr
        40                  45                  50

ctc gac gat tcc tcc atc acc tta gaa tcc aaa cca gag cgc atc gcc 307
Leu Asp Asp Ser Ser Ile Thr Leu Glu Ser Lys Pro Glu Arg Ile Ala
    55                  60                  65

gta ctc acc cca gag gca gca tcc ttg gtt ctc ccc atc aca ggc gcc 355
Val Leu Thr Pro Glu Ala Ala Ser Leu Val Leu Pro Ile Thr Gly Ala
70                  75                  80                  85

gac cgc gtc gtg atg acc gcc gaa atg gac acc gct gac gaa gaa acc 403
Asp Arg Val Val Met Thr Ala Glu Met Asp Thr Ala Asp Glu Glu Thr
                90                  95                  100

gca gct ctg gcc tcc caa gtg gaa tac caa gtc aaa aac ggt ggc agg 451
Ala Ala Leu Ala Ser Gln Val Glu Tyr Gln Val Lys Asn Gly Gly Arg
            105                 110                 115

ctc gac ccc gaa caa gtt gtc gcc ggc gac cca gat ttg gtg atc gtc 499
Leu Asp Pro Glu Gln Val Val Ala Gly Asp Pro Asp Leu Val Ile Val
        120                 125                 130

agt gcg cgt ttc gat acc gaa caa ggc acc atc gac att ttg gaa ggc 547
Ser Ala Arg Phe Asp Thr Glu Gln Gly Thr Ile Asp Ile Leu Glu Gly
    135                 140                 145

ctc aac gtc ccg tagttaactt cgattcagac gct 582
Leu Asn Val Pro
150

<210> 394
<211> 153
<212> PRT
<213> Corynebacterium glutamicum

<400> 394
Met Lys Lys Ser Leu Ile Ala Ile Val Ala Ser Ala Leu Val Leu Ser
1               5                   10                  15

Gly Cys Thr Ser Asp Ser Ser Asp Ser Ser Gly Thr Ser Gly Thr Val
            20                  25                  30

Glu Thr Thr Ser Ile Thr Thr Ser Val Ala Ala Ala Asp Gly Ala Phe

        35                  40                  45

Pro Arg Thr Val Thr Leu Asp Asp Ser Ser Ile Thr Leu Glu Ser Lys
    50                  55                  60

Pro Glu Arg Ile Ala Val Leu Thr Pro Glu Ala Ala Ser Leu Val Leu
65                  70                  75                  80

Pro Ile Thr Gly Ala Asp Arg Val Val Met Thr Ala Glu Met Asp Thr
                85                  90                  95

Ala Asp Glu Glu Thr Ala Ala Leu Ala Ser Gln Val Glu Tyr Gln Val
            100                 105                 110

Lys Asn Gly Gly Arg Leu Asp Pro Glu Gln Val Val Ala Gly Asp Pro
        115                 120                 125

Asp Leu Val Ile Val Ser Ala Arg Phe Asp Thr Glu Gln Gly Thr Ile
    130                 135                 140

Asp Ile Leu Glu Gly Leu Asn Val Pro
145                 150

<210> 395
<211> 444
<212> DNA
<213> Corynebacterium glutamicum

<220>
<221> CDS
<222> (101)..(421)
<223> RXN03082

<400> 395
cagaagcaat cgcagaaatc gatgcaaacc gcattcgaca ttcgacaagc ctgccacctc 60

ccccactgtg ctcactttga tgcaacgcgg accacgccaa atg gtc atg cca gaa 115
Met Val Met Pro Glu
1               5

tct gcc atg ctc acc ggc ctg atc cgc gaa gcc ggc ggc act cca gtg 163
Ser Ala Met Leu Thr Gly Leu Ile Arg Glu Ala Gly Gly Thr Pro Val
                10                  15                  20

gta gat tct ctc ggc gcg gta ggc acc atc act gca gac cca gaa caa 211
Val Asp Ser Leu Gly Ala Val Gly Thr Ile Thr Ala Asp Pro Glu Gln
            25                  30                  35

gtt gtt gcg atg gca cct gag atc atc atc att cag gac ttc caa ggt 259
Val Val Ala Met Ala Pro Glu Ile Ile Ile Ile Gln Asp Phe Gln Gly
        40                  45                  50

aaa ggc cga gag aac ttc gct aat ttc ctc tcc aac cca gcg cta gcc 307
Lys Gly Arg Glu Asn Phe Ala Asn Phe Leu Ser Asn Pro Ala Leu Ala
    55                  60                  65

aac gtt ccc gcc att gaa aac gac aag att ttc tac gcc gac act gtc 355
Asn Val Pro Ala Ile Glu Asn Asp Lys Ile Phe Tyr Ala Asp Thr Val
70                  75                  80                  85

acc act gga gtt act gca ggt acc gat atc acc act ggt ctg cag caa     403
Thr Thr Gly Val Thr Ala Gly Thr Asp Ile Thr Thr Gly Leu Gln Gln
                90                  95                  100

gtg gca gaa atg ctg agc tagttttgag atgttgaaac tag                   444
Val Ala Glu Met Leu Ser
            105

<210> 396
<211> 107
<212> PRT
<213> Corynebacterium glutamicum

<400> 396
Met Val Met Pro Glu Ser Ala Met Leu Thr Gly Leu Ile Arg Glu Ala
1               5                   10                  15

Gly Gly Thr Pro Val Val Asp Ser Leu Gly Ala Val Gly Thr Ile Thr
            20                  25                  30

Ala Asp Pro Glu Gln Val Val Ala Met Ala Pro Glu Ile Ile Ile Ile
        35                  40                  45

Gln Asp Phe Gln Gly Lys Gly Arg Glu Asn Phe Ala Asn Phe Leu Ser
    50                  55                  60

Asn Pro Ala Leu Ala Asn Val Pro Ala Ile Glu Asn Asp Lys Ile Phe
65                  70                  75                  80

Tyr Ala Asp Thr Val Thr Thr Gly Val Thr Ala Gly Thr Asp Ile Thr
                85                  90                  95

Thr Gly Leu Gln Gln Val Ala Glu Met Leu Ser
            100                 105

<210> 397
<211> 1479
<212> DNA
<213> Corynebacterium glutamicum

<220>
<221> CDS
<222> (101)..(1456)
<223> RXA02261

<400> 397
gaaatctata gaacgatagg taaaaactgg actaggttta tctatagcgg aatagaaaat 60

actccgctcg acagcatcac ttagctgaaa ggcctttaac atg gac ccc tca gat    115
                                            Met Asp Pro Ser Asp
                                            1               5

cta gcc tgg att ctc gca gct ttt gcg ttg gta agc ctg atg ttc ccc    163
Leu Ala Trp Ile Leu Ala Ala Phe Ala Leu Val Ser Leu Met Phe Pro
                10                  15                  20

gga ttg tcc ctg ctc tac ggc ggc atg ctg ggt ggg caa cac gtt ctt    211
Gly Leu Ser Leu Leu Tyr Gly Gly Met Leu Gly Gly Gln His Val Leu
            25                  30                  35

aac acg ttc atg atg gtt atg agc tca ctt gga atc atc agc ctt gtg 259
Asn Thr Phe Met Met Val Met Ser Ser Leu Gly Ile Ile Ser Leu Val
        40                  45                  50

tac atc att tat gga cac gga ctt gtc tta gga aac tcc atc ggt ggg 307
Tyr Ile Ile Tyr Gly His Gly Leu Val Leu Gly Asn Ser Ile Gly Gly
    55                  60                  65

tgg gga att atc gga aat ccc ctt gaa tac ttc ggc ttc cgc aac att 355
Trp Gly Ile Ile Gly Asn Pro Leu Glu Tyr Phe Gly Phe Arg Asn Ile
70                  75                  80                  85

atg gaa gat gac ggc acc gga gac ctc atg tgg gcc ggc ttc tac att 403
Met Glu Asp Asp Gly Thr Gly Asp Leu Met Trp Ala Gly Phe Tyr Ile
                90                  95                  100

ctg ttc gct gca atc tca ctc gca ctt gtt tca tct ggt gca gcg ggg 451
Leu Phe Ala Ala Ile Ser Leu Ala Leu Val Ser Ser Gly Ala Ala Gly
            105                 110                 115

cgc atg cgc ttt gga gcg tgg ctg gtc ttc ggt gtc ctg tgg ttc acc 499
Arg Met Arg Phe Gly Ala Trp Leu Val Phe Gly Val Leu Trp Phe Thr
        120                 125                 130

ttt gtg tac gcg cca ctg gca cac tgg gtt ttc gct atc gat gat cct 547
Phe Val Tyr Ala Pro Leu Ala His Trp Val Phe Ala Ile Asp Asp Pro
    135                 140                 145

gag tcc ggc tac gtg ggt ggc tgg atg aaa aat gtg ctt gag ttc cac 595
Glu Ser Gly Tyr Val Gly Gly Trp Met Lys Asn Val Leu Glu Phe His
150                 155                 160                 165

gac ttt gct ggt gga acg gca gtg cac atg aat gcg ggt gcg tct gga 643
Asp Phe Ala Gly Gly Thr Ala Val His Met Asn Ala Gly Ala Ser Gly
                170                 175                 180

ctc gcg ctg gca ata gtg ctg gga cgc cgc cac tcc atg gct gtg cgt 691
Leu Ala Leu Ala Ile Val Leu Gly Arg Arg His Ser Met Ala Val Arg
            185                 190                 195

cca cac aac ctt cca ctg att ttg att ggt gca gga ctg atc gtt gcg 739
Pro His Asn Leu Pro Leu Ile Leu Ile Gly Ala Gly Leu Ile Val Ala
        200                 205                 210

ggc tgg ttc gga ttc aat ggt ggt acc gca ggt ggt gcc aac ttc ctc 787
Gly Trp Phe Gly Phe Asn Gly Gly Thr Ala Gly Gly Ala Asn Phe Leu
    215                 220                 225

gca agc tac gtg gtc gtt acc tct ctc att gct gca gct ggc gga atg 835
Ala Ser Tyr Val Val Val Thr Ser Leu Ile Ala Ala Ala Gly Gly Met
230                 235                 240                 245

atg ggc ttc atg ctc gtt gaa cgt gtg ttc agc gga aaa ccc act ttc 883
Met Gly Phe Met Leu Val Glu Arg Val Phe Ser Gly Lys Pro Thr Phe
                250                 255                 260

ttt ggc tcg gca acc ggc aca atc gca ggc ctt gtg gct atc acc ccg 931
Phe Gly Ser Ala Thr Gly Thr Ile Ala Gly Leu Val Ala Ile Thr Pro
            265                 270                 275

gcc gcg gat gca gtg agc ccg ctc gga gca ttc gcc gtc gga gcg ctc     979
Ala Ala Asp Ala Val Ser Pro Leu Gly Ala Phe Ala Val Gly Ala Leu
        280                 285                 290

ggc gca gtt gtc tcc ttc tgg gca att agc tgg aag aag gga cac cga     1027
Gly Ala Val Val Ser Phe Trp Ala Ile Ser Trp Lys Lys Gly His Arg
    295                 300                 305

gtc gat gat tcc ttc gat gtg ttc gca gtc cac gga atg gcc ggc att     1075
Val Asp Asp Ser Phe Asp Val Phe Ala Val His Gly Met Ala Gly Ile
310                 315                 320                 325

gca ggt gca ctg ttt gtc atg ctc ttt ggc gat cca cta gca cca gcg     1123
Ala Gly Ala Leu Phe Val Met Leu Phe Gly Asp Pro Leu Ala Pro Ala
                330                 335                 340

gga gtt tcc gga gtc ttc ttc ggt ggc gaa ctc tcc ctg ctg tgg agg     1171
Gly Val Ser Gly Val Phe Phe Gly Gly Glu Leu Ser Leu Leu Trp Arg
            345                 350                 355

gaa cca ctg gcc atc atc gtg acc ctt aca tac gca ttc ggc gtg acc     1219
Glu Pro Leu Ala Ile Ile Val Thr Leu Thr Tyr Ala Phe Gly Val Thr
        360                 365                 370

tgg ttg att gcc acg atc ttg aac aag ttc atg act ctg cgc atc acc     1267
Trp Leu Ile Ala Thr Ile Leu Asn Lys Phe Met Thr Leu Arg Ile Thr
    375                 380                 385

tcc gaa gcc gaa tat gaa ggc att gac cgc gca gaa cac gca gaa tct     1315
Ser Glu Ala Glu Tyr Glu Gly Ile Asp Arg Ala Glu His Ala Glu Ser
390                 395                 400                 405

gcc tac cac ctc aat tcc aac gga att ggg atg gca acc cgc acc aat     1363
Ala Tyr His Leu Asn Ser Asn Gly Ile Gly Met Ala Thr Arg Thr Asn
                410                 415                 420

ttc gga cct gaa atc ccc gag gaa acc gtg ccc gac gcc gtg cag gtg     1411
Phe Gly Pro Glu Ile Pro Glu Glu Thr Val Pro Asp Ala Val Gln Val
            425                 430                 435

ggc gtc gat aag caa aaa atc gct gat act cga aag gcc tca aaa         1456
Gly Val Asp Lys Gln Lys Ile Ala Asp Thr Arg Lys Ala Ser Lys
        440                 445                 450

tgaccgcaac ctacaccact gaa                                           1479

<210> 398
<211> 452
<212> PRT
<213> Corynebacterium glutamicum

<400> 398
Met Asp Pro Ser Asp Leu Ala Trp Ile Leu Ala Ala Phe Ala Leu Val
1               5                   10                  15

Ser Leu Met Phe Pro Gly Leu Ser Leu Leu Tyr Gly Gly Met Leu Gly
            20                  25                  30

Gly Gln His Val Leu Asn Thr Phe Met Met Val Met Ser Ser Leu Gly
        35                  40                  45

Ile Ile Ser Leu Val Tyr Ile Ile Tyr Gly His Gly Leu Val Leu Gly
    50                  55                  60

Asn Ser Ile Gly Gly Trp Gly Ile Ile Gly Asn Pro Leu Glu Tyr Phe
65                  70                  75                  80

Gly Phe Arg Asn Ile Met Glu Asp Asp Gly Thr Gly Asp Leu Met Trp
                85                  90                  95

Ala Gly Phe Tyr Ile Leu Phe Ala Ala Ile Ser Leu Ala Leu Val Ser
            100                 105                 110

Ser Gly Ala Ala Gly Arg Met Arg Phe Gly Ala Trp Leu Val Phe Gly
        115                 120                 125

Val Leu Trp Phe Thr Phe Val Tyr Ala Pro Leu Ala His Trp Val Phe
    130                 135                 140

Ala Ile Asp Asp Pro Glu Ser Gly Tyr Val Gly Gly Trp Met Lys Asn
145                 150                 155                 160

Val Leu Glu Phe His Asp Phe Ala Gly Gly Thr Ala Val His Met Asn
                165                 170                 175

Ala Gly Ala Ser Gly Leu Ala Leu Ala Ile Val Leu Gly Arg Arg His
            180                 185                 190

Ser Met Ala Val Arg Pro His Asn Leu Pro Leu Ile Leu Ile Gly Ala
        195                 200                 205

Gly Leu Ile Val Ala Gly Trp Phe Gly Phe Asn Gly Gly Thr Ala Gly
    210                 215                 220

Gly Ala Asn Phe Leu Ala Ser Tyr Val Val Val Thr Ser Leu Ile Ala
225                 230                 235                 240

Ala Ala Gly Gly Met Met Gly Phe Met Leu Val Glu Arg Val Phe Ser
                245                 250                 255

Gly Lys Pro Thr Phe Phe Gly Ser Ala Thr Gly Thr Ile Ala Gly Leu
            260                 265                 270

Val Ala Ile Thr Pro Ala Ala Asp Ala Val Ser Pro Leu Gly Ala Phe
        275                 280                 285

Ala Val Gly Ala Leu Gly Ala Val Val Ser Phe Trp Ala Ile Ser Trp
    290                 295                 300

Lys Lys Gly His Arg Val Asp Asp Ser Phe Asp Val Phe Ala Val His
305                 310                 315                 320

Gly Met Ala Gly Ile Ala Gly Ala Leu Phe Val Met Leu Phe Gly Asp
                325                 330                 335

Pro Leu Ala Pro Ala Gly Val Ser Gly Val Phe Phe Gly Gly Glu Leu
            340                 345                 350

Ser Leu Leu Trp Arg Glu Pro Leu Ala Ile Ile Val Thr Leu Thr Tyr
        355                 360                 365

Ala Phe Gly Val Thr Trp Leu Ile Ala Thr Ile Leu Asn Lys Phe Met
    370                 375                 380

Thr Leu Arg Ile Thr Ser Glu Ala Glu Tyr Glu Gly Ile Asp Arg Ala
385                 390                 395                 400

Glu His Ala Glu Ser Ala Tyr His Leu Asn Ser Asn Gly Ile Gly Met
                405                 410                 415

Ala Thr Arg Thr Asn Phe Gly Pro Glu Ile Pro Glu Glu Thr Val Pro
            420                 425                 430

Asp Ala Val Gln Val Gly Val Asp Lys Gln Lys Ile Ala Asp Thr Arg
        435                 440                 445

Lys Ala Ser Lys
    450


<210> 399
<211> 1111
<212> DNA
<213> Corynebacterium glutamicum

<220>
<221> CDS
<222> (101)..(1111)
<223> RXA02020

<400> 399
ttggtcaatc aagcgtgaat ccggcttcca tgagccagtt gcccgcctca aagcttgacc 60

cattttcata accagtgcca tgtgggttta cggttgatac atg gct aaa tct aat   115
                                            Met Ala Lys Ser Asn
                                            1               5

gaa ggg ctg gga acc gga ctt cgg acc cgc cac ctc aca atg atg gga   163
Glu Gly Leu Gly Thr Gly Leu Arg Thr Arg His Leu Thr Met Met Gly
                10                  15                  20

ctc ggc tcc gca att ggt gcc gga ctg ttc ctc ggc acc ggc gtt ggt   211
Leu Gly Ser Ala Ile Gly Ala Gly Leu Phe Leu Gly Thr Gly Val Gly
            25                  30                  35

atc cgc gca gcc ggc ccc gca gtg ctc ctg gcg tac atc atc gcc gga   259
Ile Arg Ala Ala Gly Pro Ala Val Leu Leu Ala Tyr Ile Ile Ala Gly
        40                  45                  50

gcc atc gtt gtg ctt gtt atg caa atg ctc ggc gag atg gct gct gcc   307
Ala Ile Val Val Leu Val Met Gln Met Leu Gly Glu Met Ala Ala Ala
    55                  60                  65

cgt ccc gcc tcc gga tcg ttt tca cgt tac ggc gag gat gct ttc ggc   355
Arg Pro Ala Ser Gly Ser Phe Ser Arg Tyr Gly Glu Asp Ala Phe Gly
70                  75                  80                  85

cac tgg gct ggt ttc tcc ctc ggt tgg ttg tac tgg ttc atg ctg att   403
His Trp Ala Gly Phe Ser Leu Gly Trp Leu Tyr Trp Phe Met Leu Ile
                90                  95                  100

atg gtg atg ggc gcc gaa atg act ggc gct gct gcc atc atg ggt gca   451

Met Val Met Gly Ala Glu Met Thr Gly Ala Ala Ala Ile Met Gly Ala
            105                 110                 115

tgg ttc ggc gtc gaa ccg tgg att cct tcg ctt gtc tgc gtg gtc ttc     499
Trp Phe Gly Val Glu Pro Trp Ile Pro Ser Leu Val Cys Val Val Phe
        120                 125                 130

ttc gct gtg gtg aac ctc gtc gcg gtt cgc ggt ttc ggt gaa ttc gag     547
Phe Ala Val Val Asn Leu Val Ala Val Arg Gly Phe Gly Glu Phe Glu
    135                 140                 145

tac tgg ttc gca ttc att aag gtc gcg gtg atc atc gct ttc ctc atc     595
Tyr Trp Phe Ala Phe Ile Lys Val Ala Val Ile Ile Ala Phe Leu Ile
150                 155                 160                 165

att ggt att gct ctt att ttc gga tgg ctt ccc gga tcc acc ttt gtt     643
Ile Gly Ile Ala Leu Ile Phe Gly Trp Leu Pro Gly Ser Thr Phe Val
                170                 175                 180

gga acc tca aac ttc atc ggt gat cac gga ttc atg ccc aat ggt att     691
Gly Thr Ser Asn Phe Ile Gly Asp His Gly Phe Met Pro Asn Gly Ile
            185                 190                 195

tct ggt gtt gct gct ggt ttg ctc gcg gtg gct ttt gcc ttt ggt ggc     739
Ser Gly Val Ala Ala Gly Leu Leu Ala Val Ala Phe Ala Phe Gly Gly
        200                 205                 210

att gaa att gtc acc att gca gct gca gag tcc gat aag cca cgt gaa     787
Ile Glu Ile Val Thr Ile Ala Ala Ala Glu Ser Asp Lys Pro Arg Glu
    215                 220                 225

gct att tcc ctg gcg gtg cgt gcc gtg att tgg cgt att tca gtc ttt     835
Ala Ile Ser Leu Ala Val Arg Ala Val Ile Trp Arg Ile Ser Val Phe
230                 235                 240                 245

tac ttg ggc tct gtt ttg gtc atc act ttc ctc atg cct tat gag tcg     883
Tyr Leu Gly Ser Val Leu Val Ile Thr Phe Leu Met Pro Tyr Glu Ser
                250                 255                 260

atc aat ggt gcc gac acc gct gcg gaa tcc ccc ttc acc caa atc ctg     931
Ile Asn Gly Ala Asp Thr Ala Ala Glu Ser Pro Phe Thr Gln Ile Leu
            265                 270                 275

gcg atg gca aac atc cct ggc acg gtt ggt ttc atg gaa gcg atc atc     979
Ala Met Ala Asn Ile Pro Gly Thr Val Gly Phe Met Glu Ala Ile Ile
        280                 285                 290

gtt cta gca ctg ctt tcc gct ttc aac gcc caa atc tat gcc act tct     1027
Val Leu Ala Leu Leu Ser Ala Phe Asn Ala Gln Ile Tyr Ala Thr Ser
    295                 300                 305

cgt ttg gta ttt tcc atg gcg aat cga caa gac gct ccg cga gtt ttc     1075
Arg Leu Val Phe Ser Met Ala Asn Arg Gln Asp Ala Pro Arg Val Phe
310                 315                 320                 325

agt aag ctc agc acc agc cac gtc ccc acc aat gcg                     1111
Ser Lys Leu Ser Thr Ser His Val Pro Thr Asn Ala
                330                 335

<210> 400

<211> 337
<212> PRT
<213> Corynebacterium glutamicum

<400> 400
Met Ala Lys Ser Asn Glu Gly Leu Gly Thr Gly Leu Arg Thr Arg His
1               5                   10                  15

Leu Thr Met Met Gly Leu Gly Ser Ala Ile Gly Ala Gly Leu Phe Leu
            20                  25                  30

Gly Thr Gly Val Gly Ile Arg Ala Ala Gly Pro Ala Val Leu Leu Ala
        35                  40                  45

Tyr Ile Ile Ala Gly Ala Ile Val Val Leu Val Met Gln Met Leu Gly
    50                  55                  60

Glu Met Ala Ala Ala Arg Pro Ala Ser Gly Ser Phe Ser Arg Tyr Gly
65                  70                  75                  80

Glu Asp Ala Phe Gly His Trp Ala Gly Phe Ser Leu Gly Trp Leu Tyr
                85                  90                  95

Trp Phe Met Leu Ile Met Val Met Gly Ala Glu Met Thr Gly Ala Ala
            100                 105                 110

Ala Ile Met Gly Ala Trp Phe Gly Val Glu Pro Trp Ile Pro Ser Leu
        115                 120                 125

Val Cys Val Val Phe Phe Ala Val Val Asn Leu Val Ala Val Arg Gly
    130                 135                 140

Phe Gly Glu Phe Glu Tyr Trp Phe Ala Phe Ile Lys Val Ala Val Ile
145                 150                 155                 160

Ile Ala Phe Leu Ile Ile Gly Ile Ala Leu Ile Phe Gly Trp Leu Pro
                165                 170                 175

Gly Ser Thr Phe Val Gly Thr Ser Asn Phe Ile Gly Asp His Gly Phe
            180                 185                 190

Met Pro Asn Gly Ile Ser Gly Val Ala Ala Gly Leu Leu Ala Val Ala
        195                 200                 205

Phe Ala Phe Gly Gly Ile Glu Ile Val Thr Ile Ala Ala Ala Glu Ser
    210                 215                 220

Asp Lys Pro Arg Glu Ala Ile Ser Leu Ala Val Arg Ala Val Ile Trp
225                 230                 235                 240

Arg Ile Ser Val Phe Tyr Leu Gly Ser Val Leu Val Ile Thr Phe Leu
                245                 250                 255

Met Pro Tyr Glu Ser Ile Asn Gly Ala Asp Thr Ala Ala Glu Ser Pro
            260                 265                 270

Phe Thr Gln Ile Leu Ala Met Ala Asn Ile Pro Gly Thr Val Gly Phe
        275                 280                 285

Met Glu Ala Ile Ile Val Leu Ala Leu Leu Ser Ala Phe Asn Ala Gln
    290                 295                 300

Ile Tyr Ala Thr Ser Arg Leu Val Phe Ser Met Ala Asn Arg Gln Asp
305                 310                 315                 320

Ala Pro Arg Val Phe Ser Lys Leu Ser Thr Ser His Val Pro Thr Asn
                325                 330                 335

Ala

<210> 401
<211> 784
<212> DNA
<213> Corynebacterium glutamicum

<220>
<221> CDS
<222> (101)..(784)
<223> RXA00281

<400> 401
ggttgactct ttggctttga tgccagcatt cgccaaagcg tggccctctt taagaaagct 60

actgaacaca acagagtagg tttatgcgac actggtgcgc atg atc aac gtt gaa   115
                                           Met Ile Asn Val Glu
                                           1               5

ggc ctc acc aaa caa tat ggt cag gtc cgc gca gtc gat gat ctg agc   163
Gly Leu Thr Lys Gln Tyr Gly Gln Val Arg Ala Val Asp Asp Leu Ser
                10                  15                  20

ttc gaa gta aaa ccc gga ata gtc acc gga ttt ctc ggc ccc aac ggc   211
Phe Glu Val Lys Pro Gly Ile Val Thr Gly Phe Leu Gly Pro Asn Gly
            25                  30                  35

gcc gga aaa tcc acc acg atg cgg ctg atc ctt ggc tta gat aat cca   259
Ala Gly Lys Ser Thr Thr Met Arg Leu Ile Leu Gly Leu Asp Asn Pro
        40                  45                  50

act gca ggg cat gcc acg atc gaa gga caa ccc tac cga tcg ctc aaa   307
Thr Ala Gly His Ala Thr Ile Glu Gly Gln Pro Tyr Arg Ser Leu Lys
    55                  60                  65

aat ccc ctg acc aaa gtg gga gca ctg ctt gat gcc aaa gca aca cac   355
Asn Pro Leu Thr Lys Val Gly Ala Leu Leu Asp Ala Lys Ala Thr His
70                  75                  80                  85

cca aat aga aca gca gaa aac cac ctc aag tgg atc gcc cgt gca aat   403
Pro Asn Arg Thr Ala Glu Asn His Leu Lys Trp Ile Ala Arg Ala Asn
                90                  95                  100

ggg ctg tcc acc aaa aga gtc gat gaa gtt ctc acc ctc gtg gga ctg   451
Gly Leu Ser Thr Lys Arg Val Asp Glu Val Leu Thr Leu Val Gly Leu
            105                 110                 115

act ggt gtt ggg tca aag aag acc ggt ggg ttt tca cta ggc atg ggc   499
Thr Gly Val Gly Ser Lys Lys Thr Gly Gly Phe Ser Leu Gly Met Gly
        120                 125                 130

caa cgt cta gga ctt gct gca gca ttg ctc ggc gat ccg gaa tac tta   547

Gln Arg Leu Gly Leu Ala Ala Ala Leu Leu Gly Asp Pro Glu Tyr Leu
    135                 140                 145

att ctc gac gaa ccc gtc aac ggc ctt gac cca gaa ggc att cac tgg     595
Ile Leu Asp Glu Pro Val Asn Gly Leu Asp Pro Glu Gly Ile His Trp
150                 155                 160                 165

gtg cgc acc ttg ttg caa aac atc gcc aag cag ggc aga acc gtg ctc     643
Val Arg Thr Leu Leu Gln Asn Ile Ala Lys Gln Gly Arg Thr Val Leu
                170                 175                 180

gtg agt tcc cac ctg ctg tcc gag atg gcg caa act gcg gaa cat ttg     691
Val Ser Ser His Leu Leu Ser Glu Met Ala Gln Thr Ala Glu His Leu
            185                 190                 195

atc gtg att ggg cgt ggc aag ctg gtc gcc gat atg ccc atg cat gag     739
Ile Val Ile Gly Arg Gly Lys Leu Val Ala Asp Met Pro Met His Glu
        200                 205                 210

ttt gtg cgc tcc cat tcc gct tcc aca gtt gtg gtg cgg gca gca         784
Phe Val Arg Ser His Ser Ala Ser Thr Val Val Val Arg Ala Ala
    215                 220                 225


<210> 402
<211> 228
<212> PRT
<213> Corynebacterium glutamicum

<400> 402
Met Ile Asn Val Glu Gly Leu Thr Lys Gln Tyr Gly Gln Val Arg Ala
1               5                   10                  15

Val Asp Asp Leu Ser Phe Glu Val Lys Pro Gly Ile Val Thr Gly Phe
            20                  25                  30

Leu Gly Pro Asn Gly Ala Gly Lys Ser Thr Thr Met Arg Leu Ile Leu
        35                  40                  45

Gly Leu Asp Asn Pro Thr Ala Gly His Ala Thr Ile Glu Gly Gln Pro
    50                  55                  60

Tyr Arg Ser Leu Lys Asn Pro Leu Thr Lys Val Gly Ala Leu Leu Asp
65                  70                  75                  80

Ala Lys Ala Thr His Pro Asn Arg Thr Ala Glu Asn His Leu Lys Trp
                85                  90                  95

Ile Ala Arg Ala Asn Gly Leu Ser Thr Lys Arg Val Asp Glu Val Leu
            100                 105                 110

Thr Leu Val Gly Leu Thr Gly Val Gly Ser Lys Lys Thr Gly Gly Phe
        115                 120                 125

Ser Leu Gly Met Gly Gln Arg Leu Gly Leu Ala Ala Ala Leu Leu Gly
    130                 135                 140

Asp Pro Glu Tyr Leu Ile Leu Asp Glu Pro Val Asn Gly Leu Asp Pro
145                 150                 155                 160

Glu Gly Ile His Trp Val Arg Thr Leu Leu Gln Asn Ile Ala Lys Gln

165                 170                 175

Gly Arg Thr Val Leu Val Ser Ser His Leu Leu Ser Glu Met Ala Gln
            180                 185                 190

Thr Ala Glu His Leu Ile Val Ile Gly Arg Gly Lys Leu Val Ala Asp
        195                 200                 205

Met Pro Met His Glu Phe Val Arg Ser His Ser Ala Ser Thr Val Val
    210                 215                 220

Val Arg Ala Ala
225

<210> 403
<211> 852
<212> DNA
<213> Corynebacterium glutamicum

<220>
<221> CDS
<222> (1)..(852)
<223> RXN00570

<400> 403
acg aga cct cgg ccc caa gaa att ggc aac ggg ctt gtc gca ctg att      48
Thr Arg Pro Arg Pro Gln Glu Ile Gly Asn Gly Leu Val Ala Leu Ile
  1               5                  10                  15

ttc tcc gca tcc gga ccc atc gca gtg atc ctg gct gct gct gca gcg      96
Phe Ser Ala Ser Gly Pro Ile Ala Val Ile Leu Ala Ala Ala Ala Ala
             20                  25                  30

gga aac ctt tcg cct gat caa aca tct tca tgg atc ttc gga gca ttt     144
Gly Asn Leu Ser Pro Asp Gln Thr Ser Ser Trp Ile Phe Gly Ala Phe
         35                  40                  45

tta ggc aac gga ctg ctc acg ctg tgg ctt acc tat atg tac cgc agc     192
Leu Gly Asn Gly Leu Leu Thr Leu Trp Leu Thr Tyr Met Tyr Arg Ser
     50                  55                  60

ccg cag gca tac ttc tgg acg att ccc gga acc gtc atc gtg ggc gac     240
Pro Gln Ala Tyr Phe Trp Thr Ile Pro Gly Thr Val Ile Val Gly Asp
 65                  70                  75                  80

tca ctt acc cac tta agt ttc gct gaa gtt atc ggc gca tac ctt gtt     288
Ser Leu Thr His Leu Ser Phe Ala Glu Val Ile Gly Ala Tyr Leu Val
                 85                  90                  95

acc ggc gtt gtg gtg ttt gcg ctc gga tgg acc ggt ctc atc gga cgg     336
Thr Gly Val Val Val Phe Ala Leu Gly Trp Thr Gly Leu Ile Gly Arg
            100                 105                 110

atc atg gcg gta ctg cca cca acc atc gtg atg gcc atg gtc gca ggc     384
Ile Met Ala Val Leu Pro Pro Thr Ile Val Met Ala Met Val Ala Gly
        115                 120                 125

att ttc ctc cgc ttc gga ctc gac ctc atc gac gcc agc gtg acc gac     432
Ile Phe Leu Arg Phe Gly Leu Asp Leu Ile Asp Ala Ser Val Thr Asp
    130                 135                 140

ccg ctc att gca ctt ccc atg gtc ata gtt ttt gtg gca ttg agc atg     480
Pro Leu Ile Ala Leu Pro Met Val Ile Val Phe Val Ala Leu Ser Met
145                 150                 155                 160

agt ccc cgc ttg gca agc atc gcc cca ccc gtt gca gta gcc gca gta     528
Ser Pro Arg Leu Ala Ser Ile Ala Pro Pro Val Ala Val Ala Ala Val
                165                 170                 175

gtg gga acc atc gtt gcc atc gca tcc ggc aaa cta gcg tcc gga att     576
Val Gly Thr Ile Val Ala Ile Ala Ser Gly Lys Leu Ala Ser Gly Ile
            180                 185                 190

cta gac aac gga att atc tcc cgc ccc gtc ttt acc gcc cca gaa ttt     624
Leu Asp Asn Gly Ile Ile Ser Arg Pro Val Phe Thr Ala Pro Glu Phe
        195                 200                 205

tcc ttc gcc gcc atc atg gaa ctc gtt gtt ccc ttg gcg atc acc gta     672
Ser Phe Ala Ala Ile Met Glu Leu Val Val Pro Leu Ala Ile Thr Val
    210                 215                 220

gtc att gtc caa aac ggc caa ggc gtc gca gtg ctt aaa gca gca ggt     720
Val Ile Val Gln Asn Gly Gln Gly Val Ala Val Leu Lys Ala Ala Gly
225                 230                 235                 240

cac cgc ccc gga gta aac ctt gcc gcc gcg gcc tcc gga ctg tgg tcc     768
His Arg Pro Gly Val Asn Leu Ala Ala Ala Ala Ser Gly Leu Trp Ser
                245                 250                 255

cta ccc atg gcg ttg atc ggc aac atc acc acc tgc ctc acc ggc ccc     816
Leu Pro Met Ala Leu Ile Gly Asn Ile Thr Thr Cys Leu Thr Gly Pro
            260                 265                 270

acc aac gcg ctg atc gtc gcc gga gca aaa tca cac                     852
Thr Asn Ala Leu Ile Val Ala Gly Ala Lys Ser His
        275                 280


<210> 404
<211> 284
<212> PRT
<213> Corynebacterium glutamicum

<400> 404
Thr Arg Pro Arg Pro Gln Glu Ile Gly Asn Gly Leu Val Ala Leu Ile
  1               5                  10                  15

Phe Ser Ala Ser Gly Pro Ile Ala Val Ile Leu Ala Ala Ala Ala Ala
             20                  25                  30

Gly Asn Leu Ser Pro Asp Gln Thr Ser Ser Trp Ile Phe Gly Ala Phe
         35                  40                  45

Leu Gly Asn Gly Leu Leu Thr Leu Trp Leu Thr Tyr Met Tyr Arg Ser
     50                  55                  60

Pro Gln Ala Tyr Phe Trp Thr Ile Pro Gly Thr Val Ile Val Gly Asp
 65                  70                  75                  80

Ser Leu Thr His Leu Ser Phe Ala Glu Val Ile Gly Ala Tyr Leu Val
                 85                  90                  95

Thr Gly Val Val Val Phe Ala Leu Gly Trp Thr Gly Leu Ile Gly Arg
            100                 105                 110

Ile Met Ala Val Leu Pro Pro Thr Ile Val Met Ala Met Val Ala Gly
        115                 120                 125

Ile Phe Leu Arg Phe Gly Leu Asp Leu Ile Asp Ala Ser Val Thr Asp
    130                 135                 140

Pro Leu Ile Ala Leu Pro Met Val Ile Val Phe Val Ala Leu Ser Met
145                 150                 155                 160

Ser Pro Arg Leu Ala Ser Ile Ala Pro Pro Val Ala Val Ala Ala Val
                165                 170                 175

Val Gly Thr Ile Val Ala Ile Ala Ser Gly Lys Leu Ala Ser Gly Ile
            180                 185                 190

Leu Asp Asn Gly Ile Ile Ser Arg Pro Val Phe Thr Ala Pro Glu Phe
        195                 200                 205

Ser Phe Ala Ala Ile Met Glu Leu Val Val Pro Leu Ala Ile Thr Val
    210                 215                 220

Val Ile Val Gln Asn Gly Gln Gly Val Ala Val Leu Lys Ala Ala Gly
225                 230                 235                 240

His Arg Pro Gly Val Asn Leu Ala Ala Ala Ala Ser Gly Leu Trp Ser
                245                 250                 255

Leu Pro Met Ala Leu Ile Gly Asn Ile Thr Thr Cys Leu Thr Gly Pro
            260                 265                 270

Thr Asn Ala Leu Ile Val Ala Gly Ala Lys Ser His
        275                 280

<210> 405
<211> 498
<212> DNA
<213> Corynebacterium glutamicum

<220>
<221> CDS
<222> (1)..(498)
<223> FRXA00570

<400> 405
cca acc atc gtg atg gcc atg gtc gca ggc att ttc ctc cgc ttc gga 48
Pro Thr Ile Val Met Ala Met Val Ala Gly Ile Phe Leu Arg Phe Gly
1               5                   10                  15

ctc gac ctc atc gac gcc agc gtg acc gac ccg ctc att gca ctt ccc 96
Leu Asp Leu Ile Asp Ala Ser Val Thr Asp Pro Leu Ile Ala Leu Pro
            20                  25                  30

atg gtc ata gtt ttt gtg gca ttg agc atg agt ccc cgc ttg gca agc 144
Met Val Ile Val Phe Val Ala Leu Ser Met Ser Pro Arg Leu Ala Ser
        35                  40                  45

atc gcc cca ccc gtt gca gta gcc gca gta gtg gga acc atc gtt gcc     192
Ile Ala Pro Pro Val Ala Val Ala Ala Val Val Gly Thr Ile Val Ala
     50                  55                  60

atc gca tcc ggc aaa cta gcg tcc gga att cta gac aac gga att atc     240
Ile Ala Ser Gly Lys Leu Ala Ser Gly Ile Leu Asp Asn Gly Ile Ile
 65                  70                  75                  80

tcc cgc ccc gtc ttt acc gcc cca gaa ttt tcc ttc gcc gcc atc atg     288
Ser Arg Pro Val Phe Thr Ala Pro Glu Phe Ser Phe Ala Ala Ile Met
                 85                  90                  95

gaa ctc gtt gtt ccc ttg gcg atc acc gta gtc att gtc caa aac ggc     336
Glu Leu Val Val Pro Leu Ala Ile Thr Val Val Ile Val Gln Asn Gly
            100                 105                 110

caa ggc gtc gca gtg ctt aaa gca gca ggt cac cgc ccc gga gta aac     384
Gln Gly Val Ala Val Leu Lys Ala Ala Gly His Arg Pro Gly Val Asn
        115                 120                 125

ctt gcc gcc gcg gcc tcc gga ctg tgg tcc cta ccc atg gcg ttg atc     432
Leu Ala Ala Ala Ala Ser Gly Leu Trp Ser Leu Pro Met Ala Leu Ile
    130                 135                 140

ggc aac atc acc acc tgc ctc acc ggc ccc acc aac gcg ctg atc gtc     480
Gly Asn Ile Thr Thr Cys Leu Thr Gly Pro Thr Asn Ala Leu Ile Val
145                 150                 155                 160

gcc gga gca aaa tca cac                                             498
Ala Gly Ala Lys Ser His
                165

<210> 406
<211> 166
<212> PRT
<213> Corynebacterium glutamicum

<400> 406
Pro Thr Ile Val Met Ala Met Val Ala Gly Ile Phe Leu Arg Phe Gly
  1               5                  10                  15

Leu Asp Leu Ile Asp Ala Ser Val Thr Asp Pro Leu Ile Ala Leu Pro
             20                  25                  30

Met Val Ile Val Phe Val Ala Leu Ser Met Ser Pro Arg Leu Ala Ser
         35                  40                  45

Ile Ala Pro Pro Val Ala Val Ala Ala Val Val Gly Thr Ile Val Ala
     50                  55                  60

Ile Ala Ser Gly Lys Leu Ala Ser Gly Ile Leu Asp Asn Gly Ile Ile
 65                  70                  75                  80

Ser Arg Pro Val Phe Thr Ala Pro Glu Phe Ser Phe Ala Ala Ile Met
                 85                  90                  95

Glu Leu Val Val Pro Leu Ala Ile Thr Val Val Ile Val Gln Asn Gly
            100                 105                 110

Gln Gly Val Ala Val Leu Lys Ala Ala Gly His Arg Pro Gly Val Asn

115                 120                 125

Leu Ala Ala Ala Ala Ser Gly Leu Trp Ser Leu Pro Met Ala Leu Ile
    130                 135                 140

Gly Asn Ile Thr Thr Cys Leu Thr Gly Pro Thr Asn Ala Leu Ile Val
145                 150                 155                 160

Ala Gly Ala Lys Ser His
                165


<210> 407
<211> 1280
<212> DNA
<213> Corynebacterium glutamicum

<220>
<221> CDS
<222> (1)..(1257)
<223> RXN00571

<400> 407
aca ctt gtt cca cag gtt tac gaa ata gtg atc tac ggc gct gtg ctg      48
Thr Leu Val Pro Gln Val Tyr Glu Ile Val Ile Tyr Gly Ala Val Leu
  1               5                  10                  15

tca gct gta cat gaa gat cca acc cag atc ggt gcg ctc agc cca gca      96
Ser Ala Val His Glu Asp Pro Thr Gln Ile Gly Ala Leu Ser Pro Ala
             20                  25                  30

gtc gcc ggc acc ctt ggt tcc tac gcc atg atc ggc gtg atg atc ggt     144
Val Ala Gly Thr Leu Gly Ser Tyr Ala Met Ile Gly Val Met Ile Gly
         35                  40                  45

gct cta tct gca ggt gcc gtt ggt gac cgc ctt ggt cgt cgc aaa gtt     192
Ala Leu Ser Ala Gly Ala Val Gly Asp Arg Leu Gly Arg Arg Lys Val
     50                  55                  60

atg ctc acc gca atc gtc tgg ttc tct gtg ggc atg gcg ctg acc gcg     240
Met Leu Thr Ala Ile Val Trp Phe Ser Val Gly Met Ala Leu Thr Ala
 65                  70                  75                  80

ttc gcg tcc tcg att gcg ctg ttc ggt ttc ttg cgc ttc ctc acc gga     288
Phe Ala Ser Ser Ile Ala Leu Phe Gly Phe Leu Arg Phe Leu Thr Gly
                 85                  90                  95

ctt ggc gtg ggc atg atc gtt gca acc ggc ggc gca atc atc gcg gag     336
Leu Gly Val Gly Met Ile Val Ala Thr Gly Gly Ala Ile Ile Ala Glu
            100                 105                 110

ttc gct cca gcg aat agg cgc aac ttg ttc aac gca atc gtg tac tcc     384
Phe Ala Pro Ala Asn Arg Arg Asn Leu Phe Asn Ala Ile Val Tyr Ser
        115                 120                 125

ggt gtc cca gcc ggt ggc gtg ctg gct tct atc ctt gca ctg ctc ttt     432
Gly Val Pro Ala Gly Gly Val Leu Ala Ser Ile Leu Ala Leu Leu Phe
    130                 135                 140

gaa gat gtc atc ggc tgg cgc gga ctc ttc ctc atc ggt gga tcc cca     480
Glu Asp Val Ile Gly Trp Arg Gly Leu Phe Leu Ile Gly Gly Ser Pro

145                 150                 155                 160

cta ctg ttc ctc ctg cca ctt gca tac ttc ttc ctc cca gag tcc ccg     528
Leu Leu Phe Leu Leu Pro Leu Ala Tyr Phe Phe Leu Pro Glu Ser Pro
                165                 170                 175

cgc tgg ctc acc tcc cgc ggc cgt gct gcg gac gcc aaa gcc ctc tgc     576
Arg Trp Leu Thr Ser Arg Gly Arg Ala Ala Asp Ala Lys Ala Leu Cys
            180                 185                 190

gca cgc tat ggg ctg ccg acg gag gaa ttt gtc gtc gaa aag cag cag     624
Ala Arg Tyr Gly Leu Pro Thr Glu Glu Phe Val Val Glu Lys Gln Gln
        195                 200                 205

gaa aca aag ggc acc gga ttc gct gga att ttc tcc tcc aag tac ctc     672
Glu Thr Lys Gly Thr Gly Phe Ala Gly Ile Phe Ser Ser Lys Tyr Leu
    210                 215                 220

atg ggc acc att ctc atc ggc gca atg agc ttc atc ggg ctg ctt tcg     720
Met Gly Thr Ile Leu Ile Gly Ala Met Ser Phe Ile Gly Leu Leu Ser
225                 230                 235                 240

acc tac ggc ctg aac acc tgg ttg cca aag atc atg gaa tcc aac ggc     768
Thr Tyr Gly Leu Asn Thr Trp Leu Pro Lys Ile Met Glu Ser Asn Gly
                245                 250                 255

gca acc tca cat gat tcc ctg tac tcc ctg ctg ttc ctc aac ggc ggc     816
Ala Thr Ser His Asp Ser Leu Tyr Ser Leu Leu Phe Leu Asn Gly Gly
            260                 265                 270

gca gtg ttc ggt ggc ctc atc gca tcc tgg ttc gct gac cgc atc ggc     864
Ala Val Phe Gly Gly Leu Ile Ala Ser Trp Phe Ala Asp Arg Ile Gly
        275                 280                 285

gcg aag acc gtg atc acc tcc acc ttc gct ctc gcc gcg atc tgc ctc     912
Ala Lys Thr Val Ile Thr Ser Thr Phe Ala Leu Ala Ala Ile Cys Leu
    290                 295                 300

gga gtc ctg cca aac atc tcc tcc tgg cca atg atg tac acc gca atc     960
Gly Val Leu Pro Asn Ile Ser Ser Trp Pro Met Met Tyr Thr Ala Ile
305                 310                 315                 320

gca ttc gca ggc gtc ggc gtc ctg ggc acc cag gtt ctc acc tac ggc     1008
Ala Phe Ala Gly Val Gly Val Leu Gly Thr Gln Val Leu Thr Tyr Gly
                325                 330                 335

ctg acc tcg aac ttc ttc gga acc gaa tgc cgc gca gcg gga gtt gca     1056
Leu Thr Ser Asn Phe Phe Gly Thr Glu Cys Arg Ala Ala Gly Val Ala
            340                 345                 350

tgg tgt gca gga ttc ggc cga ctc ggc gga atc gtc gga cca gca atc     1104
Trp Cys Ala Gly Phe Gly Arg Leu Gly Gly Ile Val Gly Pro Ala Ile
        355                 360                 365

ggt ggc ctg atc atc ggc gca gga ttc gga cca agc tcc gca ttc ctc     1152
Gly Gly Leu Ile Ile Gly Ala Gly Phe Gly Pro Ser Ser Ala Phe Leu
    370                 375                 380

atc ttc gca gca gct gcc gca atc ggc gcg gtc tgc acc ttg ctg atc     1200
Ile Phe Ala Ala Ala Ala Ala Ile Gly Ala Val Cys Thr Leu Leu Ile
385                 390                 395                 400

ccg cgc tcc cca gca gaa gta gag gtc aag gtc gcg cag gaa cca ctt    1248
Pro Arg Ser Pro Ala Glu Val Glu Val Lys Val Ala Gln Glu Pro Leu
            405                 410                 415

gca cgt gtc taaccccaat taattcgaaa caa                              1280
Ala Arg Val

<210> 408
<211> 419
<212> PRT
<213> Corynebacterium glutamicum

<400> 408
Thr Leu Val Pro Gln Val Tyr Glu Ile Val Ile Tyr Gly Ala Val Leu
  1               5                  10                  15

Ser Ala Val His Glu Asp Pro Thr Gln Ile Gly Ala Leu Ser Pro Ala
             20                  25                  30

Val Ala Gly Thr Leu Gly Ser Tyr Ala Met Ile Gly Val Met Ile Gly
         35                  40                  45

Ala Leu Ser Ala Gly Ala Val Gly Asp Arg Leu Gly Arg Arg Lys Val
     50                  55                  60

Met Leu Thr Ala Ile Val Trp Phe Ser Val Gly Met Ala Leu Thr Ala
 65                  70                  75                  80

Phe Ala Ser Ser Ile Ala Leu Phe Gly Phe Leu Arg Phe Leu Thr Gly
                 85                  90                  95

Leu Gly Val Gly Met Ile Val Ala Thr Gly Gly Ala Ile Ile Ala Glu
            100                 105                 110

Phe Ala Pro Ala Asn Arg Arg Asn Leu Phe Asn Ala Ile Val Tyr Ser
        115                 120                 125

Gly Val Pro Ala Gly Gly Val Leu Ala Ser Ile Leu Ala Leu Leu Phe
    130                 135                 140

Glu Asp Val Ile Gly Trp Arg Gly Leu Phe Leu Ile Gly Gly Ser Pro
145                 150                 155                 160

Leu Leu Phe Leu Leu Pro Leu Ala Tyr Phe Phe Leu Pro Glu Ser Pro
                165                 170                 175

Arg Trp Leu Thr Ser Arg Gly Arg Ala Ala Asp Ala Lys Ala Leu Cys
            180                 185                 190

Ala Arg Tyr Gly Leu Pro Thr Glu Glu Phe Val Val Glu Lys Gln Gln
        195                 200                 205

Glu Thr Lys Gly Thr Gly Phe Ala Gly Ile Phe Ser Ser Lys Tyr Leu
    210                 215                 220

Met Gly Thr Ile Leu Ile Gly Ala Met Ser Phe Ile Gly Leu Leu Ser
225                 230                 235                 240

Thr Tyr Gly Leu Asn Thr Trp Leu Pro Lys Ile Met Glu Ser Asn Gly
                245                 250                 255

Ala Thr Ser His Asp Ser Leu Tyr Ser Leu Leu Phe Leu Asn Gly Gly
            260                 265                 270

Ala Val Phe Gly Gly Leu Ile Ala Ser Trp Phe Ala Asp Arg Ile Gly
        275                 280                 285

Ala Lys Thr Val Ile Thr Ser Thr Phe Ala Leu Ala Ala Ile Cys Leu
    290                 295                 300

Gly Val Leu Pro Asn Ile Ser Ser Trp Pro Met Met Tyr Thr Ala Ile
305                 310                 315                 320

Ala Phe Ala Gly Val Gly Val Leu Gly Thr Gln Val Leu Thr Tyr Gly
                325                 330                 335

Leu Thr Ser Asn Phe Phe Gly Thr Glu Cys Arg Ala Ala Gly Val Ala
            340                 345                 350

Trp Cys Ala Gly Phe Gly Arg Leu Gly Gly Ile Val Gly Pro Ala Ile
        355                 360                 365

Gly Gly Leu Ile Ile Gly Ala Gly Phe Gly Pro Ser Ser Ala Phe Leu
    370                 375                 380

Ile Phe Ala Ala Ala Ala Ala Ile Gly Ala Val Cys Thr Leu Leu Ile
385                 390                 395                 400

Pro Arg Ser Pro Ala Glu Val Glu Val Lys Val Ala Gln Glu Pro Leu
                405                 410                 415

Ala Arg Val

<210> 409
<211> 1208
<212> DNA
<213> Corynebacterium glutamicum

<220>
<221> CDS
<222> (1)..(1185)
<223> FRXA00571

<400> 409
cag atc ggt gcg ctc agc cca gca gtc gcc ggc acc ctt ggt tcc tac      48
Gln Ile Gly Ala Leu Ser Pro Ala Val Ala Gly Thr Leu Gly Ser Tyr
  1               5                  10                  15

gcc atg atc ggc gtg atg atc ggt gct cta tct gca ggt gcc gtt ggt      96
Ala Met Ile Gly Val Met Ile Gly Ala Leu Ser Ala Gly Ala Val Gly
             20                  25                  30

gac cgc ctt ggt cgt cgc aaa gtt atg ctc acc gca atc gtc tgg ttc     144
Asp Arg Leu Gly Arg Arg Lys Val Met Leu Thr Ala Ile Val Trp Phe
         35                  40                  45

tct gtg ggc atg gcg ctg acc gcg ttc gcg tcc tcg att gcg ctg ttc     192

Ser Val Gly Met Ala Leu Thr Ala Phe Ala Ser Ser Ile Ala Leu Phe
    50                  55                  60

ggt ttc ttg cgc ttc ctc acc gga ctt ggc gtg ggc atg atc gtt gca 240
Gly Phe Leu Arg Phe Leu Thr Gly Leu Gly Val Gly Met Ile Val Ala
65                  70                  75                  80

acc ggc ggc gca atc atc gcg gag ttc gct cca gcg aat agg cgc aac 288
Thr Gly Gly Ala Ile Ile Ala Glu Phe Ala Pro Ala Asn Arg Arg Asn
                85                  90                  95

ttg ttc aac gca atc gtg tac tcc ggt gtc cca gcc ggt ggc gtg ctg 336
Leu Phe Asn Ala Ile Val Tyr Ser Gly Val Pro Ala Gly Gly Val Leu
            100                 105                 110

gct tct atc ctt gca ctg ctc ttt gaa gat gtc atc ggc tgg cgc gga 384
Ala Ser Ile Leu Ala Leu Leu Phe Glu Asp Val Ile Gly Trp Arg Gly
        115                 120                 125

ctc ttc ctc atc ggt gga tcc cca cta ctg ttc ctc ctg cca ctt gca 432
Leu Phe Leu Ile Gly Gly Ser Pro Leu Leu Phe Leu Leu Pro Leu Ala
    130                 135                 140

tac ttc ttc ctc cca gag tcc ccg cgc tgg ctc acc tcc cgc ggc cgt 480
Tyr Phe Phe Leu Pro Glu Ser Pro Arg Trp Leu Thr Ser Arg Gly Arg
145                 150                 155                 160

gct gcg gac gcc aaa gcc ctc tgc gca cgc tat ggg ctg ccg acg gag 528
Ala Ala Asp Ala Lys Ala Leu Cys Ala Arg Tyr Gly Leu Pro Thr Glu
                165                 170                 175

gaa ttt gtc gtc gaa aag cag cag gaa aca aag ggc acc gga ttc gct 576
Glu Phe Val Val Glu Lys Gln Gln Glu Thr Lys Gly Thr Gly Phe Ala
            180                 185                 190

gga att ttc tcc tcc aag tac ctc atg ggc acc att ctc atc ggc gca 624
Gly Ile Phe Ser Ser Lys Tyr Leu Met Gly Thr Ile Leu Ile Gly Ala
        195                 200                 205

atg agc ttc atc ggg ctg ctt tcg acc tac ggc ctg aac acc tgg ttg 672
Met Ser Phe Ile Gly Leu Leu Ser Thr Tyr Gly Leu Asn Thr Trp Leu
    210                 215                 220

cca aag atc atg gaa tcc aac ggc gca acc tca cat gat tcc ctg tac 720
Pro Lys Ile Met Glu Ser Asn Gly Ala Thr Ser His Asp Ser Leu Tyr
225                 230                 235                 240

tcc ctg ctg ttc ctc aac ggc ggc gca gtg ttc ggt ggc ctc atc gca 768
Ser Leu Leu Phe Leu Asn Gly Gly Ala Val Phe Gly Gly Leu Ile Ala
                245                 250                 255

tcc tgg ttc gct gac cgc atc ggc gcg aag acc gtg atc acc tcc acc 816
Ser Trp Phe Ala Asp Arg Ile Gly Ala Lys Thr Val Ile Thr Ser Thr
            260                 265                 270

ttc gct ctc gcc gcg atc tgc ctc gga gtc ctg cca aac atc tcc tcc 864
Phe Ala Leu Ala Ala Ile Cys Leu Gly Val Leu Pro Asn Ile Ser Ser
        275                 280                 285

tgg cca atg atg tac acc gca atc gca ttc gca ggc gtc ggc gtc ctg 912
Trp Pro Met Met Tyr Thr Ala Ile Ala Phe Ala Gly Val Gly Val Leu

290                 295                 300

ggc acc cag gtt ctc acc tac ggc ctg acc tcg aac ttc ttc gga acc     960
Gly Thr Gln Val Leu Thr Tyr Gly Leu Thr Ser Asn Phe Phe Gly Thr
305                 310                 315                 320

gaa tgc cgc gca gcg gga gtt gca tgg tgt gca gga ttc ggc cga ctc     1008
Glu Cys Arg Ala Ala Gly Val Ala Trp Cys Ala Gly Phe Gly Arg Leu
                325                 330                 335

ggc gga atc gtc gga cca gca atc ggt ggc ctg atc atc ggc gca gga     1056
Gly Gly Ile Val Gly Pro Ala Ile Gly Gly Leu Ile Ile Gly Ala Gly
            340                 345                 350

ttc gga cca agc tcc gca ttc ctc atc ttc gca gca gct gcc gca atc     1104
Phe Gly Pro Ser Ser Ala Phe Leu Ile Phe Ala Ala Ala Ala Ala Ile
        355                 360                 365

ggc gcg gtc tgc acc ttg ctg atc ccg cgc tcc cca gca gaa gta gag     1152
Gly Ala Val Cys Thr Leu Leu Ile Pro Arg Ser Pro Ala Glu Val Glu
    370                 375                 380

gtc aag gtc gcg cag gaa cca ctt gca cgt gtc taaccccaat taattcgaaa 1205
Val Lys Val Ala Gln Glu Pro Leu Ala Arg Val
385                 390                 395

caa                                                                 1208

<210> 410
<211> 395
<212> PRT
<213> Corynebacterium glutamicum

<400> 410
Gln Ile Gly Ala Leu Ser Pro Ala Val Ala Gly Thr Leu Gly Ser Tyr
 1               5                  10                  15

Ala Met Ile Gly Val Met Ile Gly Ala Leu Ser Ala Gly Ala Val Gly
            20                  25                  30

Asp Arg Leu Gly Arg Arg Lys Val Met Leu Thr Ala Ile Val Trp Phe
        35                  40                  45

Ser Val Gly Met Ala Leu Thr Ala Phe Ala Ser Ser Ile Ala Leu Phe
    50                  55                  60

Gly Phe Leu Arg Phe Leu Thr Gly Leu Gly Val Gly Met Ile Val Ala
65                  70                  75                  80

Thr Gly Gly Ala Ile Ile Ala Glu Phe Ala Pro Ala Asn Arg Arg Asn
                85                  90                  95

Leu Phe Asn Ala Ile Val Tyr Ser Gly Val Pro Ala Gly Gly Val Leu
            100                 105                 110

Ala Ser Ile Leu Ala Leu Leu Phe Glu Asp Val Ile Gly Trp Arg Gly
        115                 120                 125

Leu Phe Leu Ile Gly Gly Ser Pro Leu Leu Phe Leu Leu Pro Leu Ala
    130                 135                 140

Tyr Phe Phe Leu Pro Glu Ser Pro Arg Trp Leu Thr Ser Arg Gly Arg
145                 150                 155                 160

Ala Ala Asp Ala Lys Ala Leu Cys Ala Arg Tyr Gly Leu Pro Thr Glu
                165                 170                 175

Glu Phe Val Val Glu Lys Gln Gln Glu Thr Lys Gly Thr Gly Phe Ala
            180                 185                 190

Gly Ile Phe Ser Ser Lys Tyr Leu Met Gly Thr Ile Leu Ile Gly Ala
        195                 200                 205

Met Ser Phe Ile Gly Leu Leu Ser Thr Tyr Gly Leu Asn Thr Trp Leu
    210                 215                 220

Pro Lys Ile Met Glu Ser Asn Gly Ala Thr Ser His Asp Ser Leu Tyr
225                 230                 235                 240

Ser Leu Leu Phe Leu Asn Gly Gly Ala Val Phe Gly Gly Leu Ile Ala
                245                 250                 255

Ser Trp Phe Ala Asp Arg Ile Gly Ala Lys Thr Val Ile Thr Ser Thr
            260                 265                 270

Phe Ala Leu Ala Ala Ile Cys Leu Gly Val Leu Pro Asn Ile Ser Ser
        275                 280                 285

Trp Pro Met Met Tyr Thr Ala Ile Ala Phe Ala Gly Val Gly Val Leu
    290                 295                 300

Gly Thr Gln Val Leu Thr Tyr Gly Leu Thr Ser Asn Phe Phe Gly Thr
305                 310                 315                 320

Glu Cys Arg Ala Ala Gly Val Ala Trp Cys Ala Gly Phe Gly Arg Leu
                325                 330                 335

Gly Gly Ile Val Gly Pro Ala Ile Gly Gly Leu Ile Ile Gly Ala Gly
            340                 345                 350

Phe Gly Pro Ser Ser Ala Phe Leu Ile Phe Ala Ala Ala Ala Ala Ile
        355                 360                 365

Gly Ala Val Cys Thr Leu Leu Ile Pro Arg Ser Pro Ala Glu Val Glu
    370                 375                 380

Val Lys Val Ala Gln Glu Pro Leu Ala Arg Val
385                 390                 395

<210> 411
<211> 689
<212> DNA
<213> Corynebacterium glutamicum

<220>
<221> CDS
<222> (1)..(666)
<223> RXA00962

<400> 411

gat aag gcc atc aag gcg gat cac gac att aga gaa ggc cac gat gag     48
Asp Lys Ala Ile Lys Ala Asp His Asp Ile Arg Glu Gly His Asp Glu
1               5                   10                  15

cca gca ggt ttc aag gat ctg ctt gtc gat cgc tac cgc tgg atc tcc     96
Pro Ala Gly Phe Lys Asp Leu Leu Val Asp Arg Tyr Arg Trp Ile Ser
            20                  25                  30

att tgg ttc gcg ctc gcc aca ttt gtc acc ctg ctc gcg tgg tac gga     144
Ile Trp Phe Ala Leu Ala Thr Phe Val Thr Leu Leu Ala Trp Tyr Gly
        35                  40                  45

ctg ggc aca tgg ttg cct cgc ctc atg gaa act gca ggt tat gag ttc     192
Leu Gly Thr Trp Leu Pro Arg Leu Met Glu Thr Ala Gly Tyr Glu Phe
    50                  55                  60

ggc cat gca ttg atg ttc acc ctg gct ctg aac ctc ggt gca gtg atc     240
Gly His Ala Leu Met Phe Thr Leu Ala Leu Asn Leu Gly Ala Val Ile
65                  70                  75                  80

gga tcc gtg gtt act gcg tgg gcc ggc gat cgc ttc ggg cca atc cgt     288
Gly Ser Val Val Thr Ala Trp Ala Gly Asp Arg Phe Gly Pro Ile Arg
                85                  90                  95

tcc ggt gtc atc gct gca ggt atc gcc ggt att gca ctg ctc ctg ctg     336
Ser Gly Val Ile Ala Ala Gly Ile Ala Gly Ile Ala Leu Leu Leu Leu
            100                 105                 110

ctc act tac ccg cct gtc acc gcg gtt tat gtc att ctc att ttg gct     384
Leu Thr Tyr Pro Pro Val Thr Ala Val Tyr Val Ile Leu Ile Leu Ala
        115                 120                 125

ggc gtg ggc acc cac ggc act cag atc ctc atc att gca gct gtc gcc     432
Gly Val Gly Thr His Gly Thr Gln Ile Leu Ile Ile Ala Ala Val Ala
    130                 135                 140

aac ttc tac cca agc aac ctg cgt ggc aca gca ctg ggc tgg gcg cta     480
Asn Phe Tyr Pro Ser Asn Leu Arg Gly Thr Ala Leu Gly Trp Ala Leu
145                 150                 155                 160

ggt gta ggt cgt att ggt gct gtt gtg gcc ccg cag ctc gct ggc ctg     528
Gly Val Gly Arg Ile Gly Ala Val Val Ala Pro Gln Leu Ala Gly Leu
                165                 170                 175

ctg ctg gca tgg aac ttg ggc gtg aac tcc aac ttc atc atg ttc ggc     576
Leu Leu Ala Trp Asn Leu Gly Val Asn Ser Asn Phe Ile Met Phe Gly
            180                 185                 190

acc gct gcg ctg ctc tct gcg ctg gct ctc agc gtg ttg ctg cgc ctg     624
Thr Ala Ala Leu Leu Ser Ala Leu Ala Leu Ser Val Leu Leu Arg Leu
        195                 200                 205

cag aaa acc tac agc gtc acc cac aaa gtc gaa atc caa ggc             666
Gln Lys Thr Tyr Ser Val Thr His Lys Val Glu Ile Gln Gly
    210                 215                 220

taaccccctt tttcaactca cag                                           689

<210> 412
<211> 222

<212> PRT
<213> Corynebacterium glutamicum

<400> 412
Asp Lys Ala Ile Lys Ala Asp His Asp Ile Arg Glu Gly His Asp Glu
1               5                   10                  15

Pro Ala Gly Phe Lys Asp Leu Leu Val Asp Arg Tyr Arg Trp Ile Ser
            20                  25                  30

Ile Trp Phe Ala Leu Ala Thr Phe Val Thr Leu Leu Ala Trp Tyr Gly
        35                  40                  45

Leu Gly Thr Trp Leu Pro Arg Leu Met Glu Thr Ala Gly Tyr Glu Phe
    50                  55                  60

Gly His Ala Leu Met Phe Thr Leu Ala Leu Asn Leu Gly Ala Val Ile
65                  70                  75                  80

Gly Ser Val Val Thr Ala Trp Ala Gly Asp Arg Phe Gly Pro Ile Arg
                85                  90                  95

Ser Gly Val Ile Ala Ala Gly Ile Ala Gly Ile Ala Leu Leu Leu Leu
            100                 105                 110

Leu Thr Tyr Pro Pro Val Thr Ala Val Tyr Val Ile Leu Ile Leu Ala
        115                 120                 125

Gly Val Gly Thr His Gly Thr Gln Ile Leu Ile Ile Ala Ala Val Ala
    130                 135                 140

Asn Phe Tyr Pro Ser Asn Leu Arg Gly Thr Ala Leu Gly Trp Ala Leu
145                 150                 155                 160

Gly Val Gly Arg Ile Gly Ala Val Val Ala Pro Gln Leu Ala Gly Leu
                165                 170                 175

Leu Leu Ala Trp Asn Leu Gly Val Asn Ser Asn Phe Ile Met Phe Gly
            180                 185                 190

Thr Ala Ala Leu Leu Ser Ala Leu Ala Leu Ser Val Leu Leu Arg Leu
        195                 200                 205

Gln Lys Thr Tyr Ser Val Thr His Lys Val Glu Ile Gln Gly
    210                 215                 220


<210> 413
<211> 484
<212> DNA
<213> Corynebacterium glutamicum

<220>
<221> CDS
<222> (101)..(484)
<223> RXA02811

<400> 413
cataatcact ctttccagat agcggaatcc ctttgattgt aacttggctc acctcgatta 60

tgtgtttatg acatcacacg caccagaatc gggaggactt gtg aca gag tca act   115

Val Thr Glu Ser Thr
1               5

ctc gga gca tcg aat agc tcc caa aca att gaa aat aaa ggc ctc acc     163
Leu Gly Ala Ser Asn Ser Ser Gln Thr Ile Glu Asn Lys Gly Leu Thr
                10                  15                  20

atc ttg ggc atc agc ggc cga cgc ttg gct gcg gtg ctc att ggc tgg     211
Ile Leu Gly Ile Ser Gly Arg Arg Leu Ala Ala Val Leu Ile Gly Trp
            25                  30                  35

ttt ttt gtc att ttc gac ggc tac gac ctc att gtg tac ggc acc gtc     259
Phe Phe Val Ile Phe Asp Gly Tyr Asp Leu Ile Val Tyr Gly Thr Val
        40                  45                  50

caa tcg gcc ctg gct aag gag tgg aac tta agc tct gca acg ctg ggc     307
Gln Ser Ala Leu Ala Lys Glu Trp Asn Leu Ser Ser Ala Thr Leu Gly
    55                  60                  65

acc atc ggc tcc acc gcg ttc ttt ggc atg gcg atc ggc gct gtg ttc     355
Thr Ile Gly Ser Thr Ala Phe Phe Gly Met Ala Ile Gly Ala Val Phe
70                  75                  80                  85

att ggt cga ctg tca gac cgc gtg ggc cga aaa gca gcg gtg att gga     403
Ile Gly Arg Leu Ser Asp Arg Val Gly Arg Lys Ala Ala Val Ile Gly
                90                  95                  100

tcc gtg ctg att ctc tct gtc ttc acc atg ctg tgt gca ttt gct cca     451
Ser Val Leu Ile Leu Ser Val Phe Thr Met Leu Cys Ala Phe Ala Pro
            105                 110                 115

aac cca tgg gtg ttc ggc gct ttc cgt ttc atc                         484
Asn Pro Trp Val Phe Gly Ala Phe Arg Phe Ile
        120                 125


<210> 414
<211> 128
<212> PRT
<213> Corynebacterium glutamicum

<400> 414
Val Thr Glu Ser Thr Leu Gly Ala Ser Asn Ser Ser Gln Thr Ile Glu
1               5                   10                  15

Asn Lys Gly Leu Thr Ile Leu Gly Ile Ser Gly Arg Arg Leu Ala Ala
            20                  25                  30

Val Leu Ile Gly Trp Phe Phe Val Ile Phe Asp Gly Tyr Asp Leu Ile
        35                  40                  45

Val Tyr Gly Thr Val Gln Ser Ala Leu Ala Lys Glu Trp Asn Leu Ser
    50                  55                  60

Ser Ala Thr Leu Gly Thr Ile Gly Ser Thr Ala Phe Phe Gly Met Ala
65                  70                  75                  80

Ile Gly Ala Val Phe Ile Gly Arg Leu Ser Asp Arg Val Gly Arg Lys
                85                  90                  95

Ala Ala Val Ile Gly Ser Val Leu Ile Leu Ser Val Phe Thr Met Leu

            100                 105                 110

Cys Ala Phe Ala Pro Asn Pro Trp Val Phe Gly Ala Phe Arg Phe Ile
        115                 120                 125

<210> 415
<211> 1197
<212> DNA
<213> Corynebacterium glutamicum

<220>
<221> CDS
<222> (1)..(1197)
<223> RXA02115

<400> 415
acc cgc gca acc aaa agt gtc gga aca gtt ctc gca ctc ctg tgg ttc 48
Thr Arg Ala Thr Lys Ser Val Gly Thr Val Leu Ala Leu Leu Trp Phe
1               5                   10                  15

gca att gtc ctc gac ggc ttt gac cta gtc gtc ctg ggt gca aca atc 96
Ala Ile Val Leu Asp Gly Phe Asp Leu Val Val Leu Gly Ala Thr Ile
            20                  25                  30

ccg tcc atg ctg gag gat ccc gcg tgg gat ctc act gct gga cag gcc 144
Pro Ser Met Leu Glu Asp Pro Ala Trp Asp Leu Thr Ala Gly Gln Ala
        35                  40                  45

aca cag att tcc acc atc ggc ctc gtc ggc atg acc att ggc gca ctg 192
Thr Gln Ile Ser Thr Ile Gly Leu Val Gly Met Thr Ile Gly Ala Leu
    50                  55                  60

acc atc ggt ttc tta acg gac cgt ctg ggt cga cgc cgt gtc atg ctg 240
Thr Ile Gly Phe Leu Thr Asp Arg Leu Gly Arg Arg Arg Val Met Leu
65                  70                  75                  80

ttc tct gtg gca gta ttt tct gta ttc act ctc ctg ctg gca ttc acc 288
Phe Ser Val Ala Val Phe Ser Val Phe Thr Leu Leu Leu Ala Phe Thr
                85                  90                  95

acc aac gtc cag ctc ttc agc ctg tgg cgt ttc ctc gca ggt gtt ggc 336
Thr Asn Val Gln Leu Phe Ser Leu Trp Arg Phe Leu Ala Gly Val Gly
            100                 105                 110

ctt ggt gga gca ctc ccc acc gca att gcc atg gtg acc gag ttc cgc 384
Leu Gly Gly Ala Leu Pro Thr Ala Ile Ala Met Val Thr Glu Phe Arg
        115                 120                 125

ccc ggc acc aaa gcg ggc tct gca tcc acc acc ttg atg acc ggc tac 432
Pro Gly Thr Lys Ala Gly Ser Ala Ser Thr Thr Leu Met Thr Gly Tyr
    130                 135                 140

cac gtc ggc gca gta gca acc gcc ttc ctt ggc ctc ttc ctt atc gac 480
His Val Gly Ala Val Ala Thr Ala Phe Leu Gly Leu Phe Leu Ile Asp
145                 150                 155                 160

ggc ttt ggt tgg cac tcc atg ttc atc gca ggt gcc gta cca gga ctg 528

Gly Phe Gly Trp His Ser Met Phe Ile Ala Gly Ala Val Pro Gly Leu
                165                 170                 175

atc ctg cta cca ctg ctg tac ttc ttc ctt cca gaa tcc ccg cag tac      576
Ile Leu Leu Pro Leu Leu Tyr Phe Phe Leu Pro Glu Ser Pro Gln Tyr
            180                 185                 190

ctc aaa atc tcc ggc aag ttg gat gag gcg cag gca gtt gca gca tct      624
Leu Lys Ile Ser Gly Lys Leu Asp Glu Ala Gln Ala Val Ala Ala Ser
        195                 200                 205

tat gga ctt tcc ctg gat gat gat ctt gat cgc gaa cac gaa gaa gaa      672
Tyr Gly Leu Ser Leu Asp Asp Asp Leu Asp Arg Glu His Glu Glu Glu
    210                 215                 220

ctc ggc gag tcc tcc tca ctt tcc tcc ctg ttc aag ccc tcg ttc cgc      720
Leu Gly Glu Ser Ser Ser Leu Ser Ser Leu Phe Lys Pro Ser Phe Arg
225                 230                 235                 240

cgc aac acc ctg gcg att tgg ggc acc tca ttc atg gga ctc ctc ctg      768
Arg Asn Thr Leu Ala Ile Trp Gly Thr Ser Phe Met Gly Leu Leu Leu
                245                 250                 255

gtc tac ggc ctg aac aca tgg ctg cca caa atc atg cgc caa gca gac      816
Val Tyr Gly Leu Asn Thr Trp Leu Pro Gln Ile Met Arg Gln Ala Asp
            260                 265                 270

tac gac atg ggt aac tcg ctt gga ttc ctc atg gtg ctc aac atc ggc      864
Tyr Asp Met Gly Asn Ser Leu Gly Phe Leu Met Val Leu Asn Ile Gly
        275                 280                 285

gca gtg atc ggc ctt tat att gca ggg cga att gcc gat aag aac tcc      912
Ala Val Ile Gly Leu Tyr Ile Ala Gly Arg Ile Ala Asp Lys Asn Ser
    290                 295                 300

cct cgc aaa aca gca ctc gta tgg ttc gtg ttc tct gca ttt tcc ctc      960
Pro Arg Lys Thr Ala Leu Val Trp Phe Val Phe Ser Ala Phe Ser Leu
305                 310                 315                 320

gcg ttg ctt gct gtc cgg atg cca ctg atc ggt ctg tat ggc atc gtg     1008
Ala Leu Leu Ala Val Arg Met Pro Leu Ile Gly Leu Tyr Gly Ile Val
                325                 330                 335

ctg ctc acc ggc atc ttt gtg ttc agc tcc cag gta ctc atc tac gcc     1056
Leu Leu Thr Gly Ile Phe Val Phe Ser Ser Gln Val Leu Ile Tyr Ala
            340                 345                 350

ttc gtt ggt gag aat cac cct gcc aag atg cgc gca acc gcc atg gga     1104
Phe Val Gly Glu Asn His Pro Ala Lys Met Arg Ala Thr Ala Met Gly
        355                 360                 365

ttc tcc gca gga att ggt cgc ctc ggc gcc atc tct ggc ccg ttg ctt     1152
Phe Ser Ala Gly Ile Gly Arg Leu Gly Ala Ile Ser Gly Pro Leu Leu
    370                 375                 380

ggt ggt ctg ctt gtc agt gcc aac ctt gct tac cca tgg ggc ttc         1197
Gly Gly Leu Leu Val Ser Ala Asn Leu Ala Tyr Pro Trp Gly Phe
385                 390                 395

<210> 416

<211> 399
<212> PRT
<213> Corynebacterium glutamicum

<400> 416
Thr Arg Ala Thr Lys Ser Val Gly Thr Val Leu Ala Leu Leu Trp Phe
1               5                   10                  15

Ala Ile Val Leu Asp Gly Phe Asp Leu Val Val Leu Gly Ala Thr Ile
            20                  25                  30

Pro Ser Met Leu Glu Asp Pro Ala Trp Asp Leu Thr Ala Gly Gln Ala
        35                  40                  45

Thr Gln Ile Ser Thr Ile Gly Leu Val Gly Met Thr Ile Gly Ala Leu
    50                  55                  60

Thr Ile Gly Phe Leu Thr Asp Arg Leu Gly Arg Arg Arg Val Met Leu
65                  70                  75                  80

Phe Ser Val Ala Val Phe Ser Val Phe Thr Leu Leu Leu Ala Phe Thr
                85                  90                  95

Thr Asn Val Gln Leu Phe Ser Leu Trp Arg Phe Leu Ala Gly Val Gly
            100                 105                 110

Leu Gly Gly Ala Leu Pro Thr Ala Ile Ala Met Val Thr Glu Phe Arg
        115                 120                 125

Pro Gly Thr Lys Ala Gly Ser Ala Ser Thr Thr Leu Met Thr Gly Tyr
    130                 135                 140

His Val Gly Ala Val Ala Thr Ala Phe Leu Gly Leu Phe Leu Ile Asp
145                 150                 155                 160

Gly Phe Gly Trp His Ser Met Phe Ile Ala Gly Ala Val Pro Gly Leu
                165                 170                 175

Ile Leu Leu Pro Leu Leu Tyr Phe Phe Leu Pro Glu Ser Pro Gln Tyr
            180                 185                 190

Leu Lys Ile Ser Gly Lys Leu Asp Glu Ala Gln Ala Val Ala Ala Ser
        195                 200                 205

Tyr Gly Leu Ser Leu Asp Asp Asp Leu Asp Arg Glu His Glu Glu Glu
    210                 215                 220

Leu Gly Glu Ser Ser Ser Leu Ser Ser Leu Phe Lys Pro Ser Phe Arg
225                 230                 235                 240

Arg Asn Thr Leu Ala Ile Trp Gly Thr Ser Phe Met Gly Leu Leu Leu
                245                 250                 255

Val Tyr Gly Leu Asn Thr Trp Leu Pro Gln Ile Met Arg Gln Ala Asp
            260                 265                 270

Tyr Asp Met Gly Asn Ser Leu Gly Phe Leu Met Val Leu Asn Ile Gly
        275                 280                 285

Ala Val Ile Gly Leu Tyr Ile Ala Gly Arg Ile Ala Asp Lys Asn Ser
    290                 295                 300

Pro Arg Lys Thr Ala Leu Val Trp Phe Val Phe Ser Ala Phe Ser Leu
305                 310                 315                 320

Ala Leu Leu Ala Val Arg Met Pro Leu Ile Gly Leu Tyr Gly Ile Val
                325                 330                 335

Leu Leu Thr Gly Ile Phe Val Phe Ser Ser Gln Val Leu Ile Tyr Ala
            340                 345                 350

Phe Val Gly Glu Asn His Pro Ala Lys Met Arg Ala Thr Ala Met Gly
        355                 360                 365

Phe Ser Ala Gly Ile Gly Arg Leu Gly Ala Ile Ser Gly Pro Leu Leu
    370                 375                 380

Gly Gly Leu Leu Val Ser Ala Asn Leu Ala Tyr Pro Trp Gly Phe
385                 390                 395


<210> 417
<211> 1288
<212> DNA
<213> Corynebacterium glutamicum

<220>
<221> CDS
<222> (101)..(1288)
<223> RXN00590

<400> 417
tctaaactca ctctcaactc accaagattg ttcaacaatc tgcgattggt gtgcaatcta 60

ccccaatcat tttgaaagcc cccacgaaag gagcgcgaca atg gcc gac aac aaa   115
                                           Met Ala Asp Asn Lys
                                           1               5

aat gcc gat gac agc cag cta gtc tca gcc agc act gga acc cct ggg   163
Asn Ala Asp Asp Ser Gln Leu Val Ser Ala Ser Thr Gly Thr Pro Gly
                10                  15                  20

cct ggc gac att gca aaa gcc aat gcg cca tcc ctc aag caa gct gca   211
Pro Gly Asp Ile Ala Lys Ala Asn Ala Pro Ser Leu Lys Gln Ala Ala
            25                  30                  35

gta acc gcc tct ggc cga agc gct ctg atg ggt gcc atc ttc ctc atg   259
Val Thr Ala Ser Gly Arg Ser Ala Leu Met Gly Ala Ile Phe Leu Met
        40                  45                  50

gca act tct gcc atc ggc cca ggg ttc ctc acc caa acc gct gtc ttc   307
Ala Thr Ser Ala Ile Gly Pro Gly Phe Leu Thr Gln Thr Ala Val Phe
    55                  60                  65

acc aac cag ctc ggc gca gct ttc gca ttt gcg atc ctg gtg tcg atc   355
Thr Asn Gln Leu Gly Ala Ala Phe Ala Phe Ala Ile Leu Val Ser Ile
70                  75                  80                  85

ctc att gac atc gcg gtg cag ctg aat gtg tgg cgc atc atc ggc gtc   403
Leu Ile Asp Ile Ala Val Gln Leu Asn Val Trp Arg Ile Ile Gly Val
                90                  95                  100

tct gaa atg cgc gcc caa gaa ctc ggc aac acg gtt atc cca ggt ttt 451
Ser Glu Met Arg Ala Gln Glu Leu Gly Asn Thr Val Ile Pro Gly Phe
            105                 110                 115

ggt tgg gtg ctg gcc gta ctg gtc tgt att ggc ggc gta gta ttc aac 499
Gly Trp Val Leu Ala Val Leu Val Cys Ile Gly Gly Val Val Phe Asn
        120                 125                 130

atc ggc aac atc gcc ggt ggt ggc ctt ggg ctt aac gcg ctg ctt ggc 547
Ile Gly Asn Ile Ala Gly Gly Gly Leu Gly Leu Asn Ala Leu Leu Gly
    135                 140                 145

tgg gac gtc aaa gtt ggt ggc gtg atc acc gcg gcc atc gcg att gcg 595
Trp Asp Val Lys Val Gly Gly Val Ile Thr Ala Ala Ile Ala Ile Ala
150                 155                 160                 165

atc ttc ttg ttc aag cga ctt ggt gct gct ctg gac aaa ttc ctc gtg 643
Ile Phe Leu Phe Lys Arg Leu Gly Ala Ala Leu Asp Lys Phe Leu Val
                170                 175                 180

gtc ctc ggc gtc gtg atg atc ctg ctc acc gtc tac gtg gct ttc gtc 691
Val Leu Gly Val Val Met Ile Leu Leu Thr Val Tyr Val Ala Phe Val
            185                 190                 195

tcc caa cct cca gtt ggc tcg gcg ctg aag aat gca gta ctt cct gac 739
Ser Gln Pro Pro Val Gly Ser Ala Leu Lys Asn Ala Val Leu Pro Asp
        200                 205                 210

acc atc gac tgg ctt gtc atc acc aca ctt gtg ggt gga acc gtc ggt 787
Thr Ile Asp Trp Leu Val Ile Thr Thr Leu Val Gly Gly Thr Val Gly
    215                 220                 225

gga tac atc act tac gct ggc gca cac cgc atg ctg gac tcc gga cga 835
Gly Tyr Ile Thr Tyr Ala Gly Ala His Arg Met Leu Asp Ser Gly Arg
230                 235                 240                 245

acc ggc ccc aac aac gtc aaa gct gtt tcc aat tcc tct atc acc ggc 883
Thr Gly Pro Asn Asn Val Lys Ala Val Ser Asn Ser Ser Ile Thr Gly
                250                 255                 260

atc ctg atc act ggc ctc atg cgc gtg gtg ctc ttc ctc gcg gtt ctc 931
Ile Leu Ile Thr Gly Leu Met Arg Val Val Leu Phe Leu Ala Val Leu
            265                 270                 275

ggt gtt gtc gca ggt ggc gtc acc cta tcc acc acg ggc aac cca gcc 979
Gly Val Val Ala Gly Gly Val Thr Leu Ser Thr Thr Gly Asn Pro Ala
        280                 285                 290

gcg gaa gca ttc cag cac gct gca ggc gat atc gga cta cgc atc ttc 1027
Ala Glu Ala Phe Gln His Ala Ala Gly Asp Ile Gly Leu Arg Ile Phe
    295                 300                 305

ggc gcc gtg ctg tgg gca gcg tcc att tcc tca gtc atc ggc gcc agc 1075
Gly Ala Val Leu Trp Ala Ala Ser Ile Ser Ser Val Ile Gly Ala Ser
310                 315                 320                 325

tac acc tct gca acc ttc ctg gtg gaa aac aag cca gag aag aag cgt 1123
Tyr Thr Ser Ala Thr Phe Leu Val Glu Asn Lys Pro Glu Lys Lys Arg
                330                 335                 340

ctg caa aac tgg gtg acc atc atc ttc atc ctg att tct tgc tcc gtg 1171

Leu Gln Asn Trp Val Thr Ile Ile Phe Ile Leu Ile Ser Cys Ser Val
            345                 350                 355

ttc atc atg ctc ggc acg gca cca gca atc ctc ttg gtc ttc gcc gga    1219
Phe Ile Met Leu Gly Thr Ala Pro Ala Ile Leu Leu Val Phe Ala Gly
        360                 365                 370

gca ttc aac ggt ttg gtc ctc ccc gta ggc ttt acc ctg atg atc tac    1267
Ala Phe Asn Gly Leu Val Leu Pro Val Gly Phe Thr Leu Met Ile Tyr
    375                 380                 385

gta gcg atc ttc cgc caa aaa                                        1288
Val Ala Ile Phe Arg Gln Lys
390                 395

<210> 418
<211> 396
<212> PRT
<213> Corynebacterium glutamicum

<400> 418
Met Ala Asp Asn Lys Asn Ala Asp Asp Ser Gln Leu Val Ser Ala Ser
  1               5                  10                  15

Thr Gly Thr Pro Gly Pro Gly Asp Ile Ala Lys Ala Asn Ala Pro Ser
             20                  25                  30

Leu Lys Gln Ala Ala Val Thr Ala Ser Gly Arg Ser Ala Leu Met Gly
         35                  40                  45

Ala Ile Phe Leu Met Ala Thr Ser Ala Ile Gly Pro Gly Phe Leu Thr
     50                  55                  60

Gln Thr Ala Val Phe Thr Asn Gln Leu Gly Ala Ala Phe Ala Phe Ala
 65                  70                  75                  80

Ile Leu Val Ser Ile Leu Ile Asp Ile Ala Val Gln Leu Asn Val Trp
                 85                  90                  95

Arg Ile Ile Gly Val Ser Glu Met Arg Ala Gln Glu Leu Gly Asn Thr
            100                 105                 110

Val Ile Pro Gly Phe Gly Trp Val Leu Ala Val Leu Val Cys Ile Gly
        115                 120                 125

Gly Val Val Phe Asn Ile Gly Asn Ile Ala Gly Gly Gly Leu Gly Leu
    130                 135                 140

Asn Ala Leu Leu Gly Trp Asp Val Lys Val Gly Gly Val Ile Thr Ala
145                 150                 155                 160

Ala Ile Ala Ile Ala Ile Phe Leu Phe Lys Arg Leu Gly Ala Ala Leu
                165                 170                 175

Asp Lys Phe Leu Val Val Leu Gly Val Val Met Ile Leu Leu Thr Val
            180                 185                 190

Tyr Val Ala Phe Val Ser Gln Pro Pro Val Gly Ser Ala Leu Lys Asn
        195                 200                 205

Ala Val Leu Pro Asp Thr Ile Asp Trp Leu Val Ile Thr Thr Leu Val
    210                 215                 220

Gly Gly Thr Val Gly Gly Tyr Ile Thr Tyr Ala Gly Ala His Arg Met
225                 230                 235                 240

Leu Asp Ser Gly Arg Thr Gly Pro Asn Asn Val Lys Ala Val Ser Asn
                245                 250                 255

Ser Ser Ile Thr Gly Ile Leu Ile Thr Gly Leu Met Arg Val Val Leu
            260                 265                 270

Phe Leu Ala Val Leu Gly Val Val Ala Gly Gly Val Thr Leu Ser Thr
        275                 280                 285

Thr Gly Asn Pro Ala Ala Glu Ala Phe Gln His Ala Ala Gly Asp Ile
    290                 295                 300

Gly Leu Arg Ile Phe Gly Ala Val Leu Trp Ala Ala Ser Ile Ser Ser
305                 310                 315                 320

Val Ile Gly Ala Ser Tyr Thr Ser Ala Thr Phe Leu Val Glu Asn Lys
                325                 330                 335

Pro Glu Lys Lys Arg Leu Gln Asn Trp Val Thr Ile Ile Phe Ile Leu
            340                 345                 350

Ile Ser Cys Ser Val Phe Ile Met Leu Gly Thr Ala Pro Ala Ile Leu
        355                 360                 365

Leu Val Phe Ala Gly Ala Phe Asn Gly Leu Val Leu Pro Val Gly Phe
    370                 375                 380

Thr Leu Met Ile Tyr Val Ala Ile Phe Arg Gln Lys
385                 390                 395

<210> 419
<211> 487
<212> DNA
<213> Corynebacterium glutamicum

<220>
<221> CDS
<222> (101)..(487)
<223> FRXA00590

<400> 419
tacgctggcg cacaccgcat gctggactcc ggacgaaccg gccccaacac gtcaaagctg 60

tttccaattc tctatcaccg gcatcctgat cactggcctc atg cgc gtg gtg ctc   115
                                          Met Arg Val Val Leu
                                          1               5

ttc ctc gcg gtt ctc ggt gtt gtc gca ggt ggc gtc acc cta tcc acc   163
Phe Leu Ala Val Leu Gly Val Val Ala Gly Gly Val Thr Leu Ser Thr
                10                  15                  20

acg ggc aac cca gcc gcg gaa gca ttc cag cac gct gca ggc gat atc   211
Thr Gly Asn Pro Ala Ala Glu Ala Phe Gln His Ala Ala Gly Asp Ile
            25                  30                  35

gga cta cgc atc ttc ggc gcc gtg ctg tgg gca gcg tcc att tcc tca     259
Gly Leu Arg Ile Phe Gly Ala Val Leu Trp Ala Ala Ser Ile Ser Ser
        40                  45                  50

gtc atc ggc gcc agc tac acc tct gca acc ttc ctg gtg gaa aac aag     307
Val Ile Gly Ala Ser Tyr Thr Ser Ala Thr Phe Leu Val Glu Asn Lys
    55                  60                  65

cca gag aag aag cgt ctg caa aac tgg gtg acc atc atc ttc atc ctg     355
Pro Glu Lys Lys Arg Leu Gln Asn Trp Val Thr Ile Ile Phe Ile Leu
70                  75                  80                  85

att tct tgc tcc gtg ttc atc atg ctc ggc acg gca cca gca atc ctc     403
Ile Ser Cys Ser Val Phe Ile Met Leu Gly Thr Ala Pro Ala Ile Leu
                90                  95                  100

ttg gtc ttc gcc gga gca ttc aac ggt ttg gtc ctc ccc gta ggc ttt     451
Leu Val Phe Ala Gly Ala Phe Asn Gly Leu Val Leu Pro Val Gly Phe
            105                 110                 115

acc ctg atg atc tac gta gcg atc ttc cgc caa aaa                     487
Thr Leu Met Ile Tyr Val Ala Ile Phe Arg Gln Lys
        120                 125


<210> 420
<211> 129
<212> PRT
<213> Corynebacterium glutamicum

<400> 420
Met Arg Val Val Leu Phe Leu Ala Val Leu Gly Val Val Ala Gly Gly
1               5                   10                  15

Val Thr Leu Ser Thr Thr Gly Asn Pro Ala Ala Glu Ala Phe Gln His
            20                  25                  30

Ala Ala Gly Asp Ile Gly Leu Arg Ile Phe Gly Ala Val Leu Trp Ala
        35                  40                  45

Ala Ser Ile Ser Ser Val Ile Gly Ala Ser Tyr Thr Ser Ala Thr Phe
    50                  55                  60

Leu Val Glu Asn Lys Pro Glu Lys Lys Arg Leu Gln Asn Trp Val Thr
65                  70                  75                  80

Ile Ile Phe Ile Leu Ile Ser Cys Ser Val Phe Ile Met Leu Gly Thr
                85                  90                  95

Ala Pro Ala Ile Leu Leu Val Phe Ala Gly Ala Phe Asn Gly Leu Val
            100                 105                 110

Leu Pro Val Gly Phe Thr Leu Met Ile Tyr Val Ala Ile Phe Arg Gln
        115                 120                 125

Lys


<210> 421

<211> 490
<212> DNA
<213> Corynebacterium glutamicum

<220>
<221> CDS
<222> (101)..(490)
<223> FRXA01538

<400> 421
tctaaactca ctctcaactc accaagattg ttcaacaatc tgcgattggt gtgcaatcta 60

ccccaatcat tttgaaagcc cccacgaaag gagcgcgaca atg gcc gac aac aaa 115
Met Ala Asp Asn Lys
1               5

aat gcc gat gac agc cag cta gtc tca gcc agc act gga acc cct ggg 163
Asn Ala Asp Asp Ser Gln Leu Val Ser Ala Ser Thr Gly Thr Pro Gly
                10                  15                  20

cct ggc gac att gca aaa gcc aat gcg cca tcc ctc aag caa gct gca 211
Pro Gly Asp Ile Ala Lys Ala Asn Ala Pro Ser Leu Lys Gln Ala Ala
            25                  30                  35

gta acc gcc tct ggc cga agc gct ctg atg ggt gcc atc ttc ctc atg 259
Val Thr Ala Ser Gly Arg Ser Ala Leu Met Gly Ala Ile Phe Leu Met
        40                  45                  50

gca act tct gcc atc ggc cca ggg ttc ctc acc caa acc gct gtc ttc 307
Ala Thr Ser Ala Ile Gly Pro Gly Phe Leu Thr Gln Thr Ala Val Phe
    55                  60                  65

acc aac cag ctc ggc gca gct ttc gca ttt gcg atc ctg gtg tcg atc 355
Thr Asn Gln Leu Gly Ala Ala Phe Ala Phe Ala Ile Leu Val Ser Ile
70                  75                  80                  85

ctc att gac atc gcg gtg cag ctg aat gtg tgg cgc atc atc ggc gtc 403
Leu Ile Asp Ile Ala Val Gln Leu Asn Val Trp Arg Ile Ile Gly Val
                90                  95                  100

tct gaa atg cgc gcc caa gaa ctc ggc aac acg gtt atc cca ggt ttt 451
Ser Glu Met Arg Ala Gln Glu Leu Gly Asn Thr Val Ile Pro Gly Phe
            105                 110                 115

ggt tgg gtg ctg gcc gta ctg gtc tgt att ggc ggc gta 490
Gly Trp Val Leu Ala Val Leu Val Cys Ile Gly Gly Val
        120                 125                 130

<210> 422
<211> 130
<212> PRT
<213> Corynebacterium glutamicum

<400> 422
Met Ala Asp Asn Lys Asn Ala Asp Asp Ser Gln Leu Val Ser Ala Ser
1               5                   10                  15

Thr Gly Thr Pro Gly Pro Gly Asp Ile Ala Lys Ala Asn Ala Pro Ser
            20                  25                  30

Leu Lys Gln Ala Ala Val Thr Ala Ser Gly Arg Ser Ala Leu Met Gly
        35                  40                  45

Ala Ile Phe Leu Met Ala Thr Ser Ala Ile Gly Pro Gly Phe Leu Thr
    50                  55                  60

Gln Thr Ala Val Phe Thr Asn Gln Leu Gly Ala Ala Phe Ala Phe Ala
65                  70                  75                  80

Ile Leu Val Ser Ile Leu Ile Asp Ile Ala Val Gln Leu Asn Val Trp
                85                  90                  95

Arg Ile Ile Gly Val Ser Glu Met Arg Ala Gln Glu Leu Gly Asn Thr
            100                 105                 110

Val Ile Pro Gly Phe Gly Trp Val Leu Ala Val Leu Val Cys Ile Gly
        115                 120                 125

Gly Val
    130


<210> 423
<211> 1401
<212> DNA
<213> Corynebacterium glutamicum

<220>
<221> CDS
<222> (101)..(1378)
<223> RXA01727

<400> 423
agcttttggt ggtttcacca cctgcgctcg tcttaatttt gcgtgttcca gagagaccct 60

tgaggagggg ctgcgccgta tcgccagcgt gttgtaaata atg agt aaa aag tct   115
                                           Met Ser Lys Lys Ser
                                           1               5

gtc ctg att act tct ttg atg ctg ttt tcc atg ttc ttc gga gct gga   163
Val Leu Ile Thr Ser Leu Met Leu Phe Ser Met Phe Phe Gly Ala Gly
                10                  15                  20

aac ctc atc ttc ccg ccg atg ctt gga ttg tcg gca gga acc aac tat   211
Asn Leu Ile Phe Pro Pro Met Leu Gly Leu Ser Ala Gly Thr Asn Tyr
            25                  30                  35

cta cca gct atc tta gga ttt cta gca acg agt gtt ctg ctc ccg gtg   259
Leu Pro Ala Ile Leu Gly Phe Leu Ala Thr Ser Val Leu Leu Pro Val
        40                  45                  50

ctg gcg att atc gcg gtg gtg ttg tcg gga gaa aat gtc aag gac atg   307
Leu Ala Ile Ile Ala Val Val Leu Ser Gly Glu Asn Val Lys Asp Met
    55                  60                  65

gct tct cgt ggc ggt aag atc ttt ggc ctg gtg ttt cct att gct gcc   355
Ala Ser Arg Gly Gly Lys Ile Phe Gly Leu Val Phe Pro Ile Ala Ala
70                  75                  80                  85

tat ttg tct atc ggc gcg ttt tac gcg ctg ccg agg act ggg gcg gtg   403
Tyr Leu Ser Ile Gly Ala Phe Tyr Ala Leu Pro Arg Thr Gly Ala Val

90                  95                  100

agc tat tcg acg gcg gtt ggc gtc gat aat gcg ctt tat tcg ggc ttg     451
Ser Tyr Ser Thr Ala Val Gly Val Asp Asn Ala Leu Tyr Ser Gly Leu
            105                 110                 115

ttt aac ttt gtg ttt ttt gcg gtg gca ctg gcg ttg tcg tgg aat ccg     499
Phe Asn Phe Val Phe Phe Ala Val Ala Leu Ala Leu Ser Trp Asn Pro
        120                 125                 130

aat ggc att gca gac aag ttg ggt aag tgg ctc acg cca gcg ttg ctc     547
Asn Gly Ile Ala Asp Lys Leu Gly Lys Trp Leu Thr Pro Ala Leu Leu
    135                 140                 145

acg ttg att gtg gtg ctg gtg gtg ttg tcg gta gcc aag ttg gat ggc     595
Thr Leu Ile Val Val Leu Val Val Leu Ser Val Ala Lys Leu Asp Gly
150                 155                 160                 165

acg cca ggt gag cca agt agt gcg tat gcg cag cag cct gcg ggg gcg     643
Thr Pro Gly Glu Pro Ser Ser Ala Tyr Ala Gln Gln Pro Ala Gly Ala
                170                 175                 180

ggt ttg ctt gag ggc tac atg acg atg gat gcg att gct gcg ttg gcg     691
Gly Leu Leu Glu Gly Tyr Met Thr Met Asp Ala Ile Ala Ala Leu Ala
            185                 190                 195

ttt ggc atc gtg gtg att tct gcg ttc aag tac caa aag gtt aac aag     739
Phe Gly Ile Val Val Ile Ser Ala Phe Lys Tyr Gln Lys Val Asn Lys
        200                 205                 210

gtc cgc acg gca act gtc gtg tcg gcg ttc att gcc gga att ttg ttg     787
Val Arg Thr Ala Thr Val Val Ser Ala Phe Ile Ala Gly Ile Leu Leu
    215                 220                 225

gcg ctg gtt tat ctt ggt ttg ggc tca atc ggt caa gta gta aac ggt     835
Ala Leu Val Tyr Leu Gly Leu Gly Ser Ile Gly Gln Val Val Asn Gly
230                 235                 240                 245

gag ttc gct gat ggc acc gca att ttg aac tac gct gca ctg tcc acg     883
Glu Phe Ala Asp Gly Thr Ala Ile Leu Asn Tyr Ala Ala Leu Ser Thr
                250                 255                 260

atg ggt cag gct ggt cgc atc atg ttc gtg gct att ttg atc ctt gca     931
Met Gly Gln Ala Gly Arg Ile Met Phe Val Ala Ile Leu Ile Leu Ala
            265                 270                 275

tgt atg acc acc gca gtt ggt ctg atc agt gcg acg tct gag ttt ttc     979
Cys Met Thr Thr Ala Val Gly Leu Ile Ser Ala Thr Ser Glu Phe Phe
        280                 285                 290

aat tcg ctg ctg cca ggt gtc aag tac cac gtc tgg gcc act gtt ttc     1027
Asn Ser Leu Leu Pro Gly Val Lys Tyr His Val Trp Ala Thr Val Phe
    295                 300                 305

gcg ctg att tcc ttt ggc gtt gcc acc atg ggt ttg gat acg gtg ttg     1075
Ala Leu Ile Ser Phe Gly Val Ala Thr Met Gly Leu Asp Thr Val Leu
310                 315                 320                 325

gcc gtt gcg gct cca gtg att agt ttc att tac cca tcg gcc atc acc     1123
Ala Val Ala Ala Pro Val Ile Ser Phe Ile Tyr Pro Ser Ala Ile Thr
                330                 335                 340

ttg gtg ttc ttg tcg ctc atc gag ccc ctg ctg ttc cgt ctc aag tgg 1171
Leu Val Phe Leu Ser Leu Ile Glu Pro Leu Leu Phe Arg Leu Lys Trp
            345                 350                 355

acc tac cta ttc ggc att tgg act gca gtt gtg tgg gcg ctg ttc atg 1219
Thr Tyr Leu Phe Gly Ile Trp Thr Ala Val Val Trp Ala Leu Phe Met
        360                 365                 370

tct atc cct gcg ctg aat cca ttc atc gaa tgg gcg ccg ctg cac agc 1267
Ser Ile Pro Ala Leu Asn Pro Phe Ile Glu Trp Ala Pro Leu His Ser
    375                 380                 385

atg tct ttg ggt tgg gtt gtc cca gtt ctc gtg gcc tct gcc atc ggt 1315
Met Ser Leu Gly Trp Val Val Pro Val Leu Val Ala Ser Ala Ile Gly
390                 395                 400                 405

ttg gct att gat tgg aac aag aaa ggt gcc cag tct gtt gca aag aag 1363
Leu Ala Ile Asp Trp Asn Lys Lys Gly Ala Gln Ser Val Ala Lys Lys
                410                 415                 420

gaa tcc att tcc gtc taatcgctaa ttgcgaggag tct 1401
Glu Ser Ile Ser Val
            425

<210> 424
<211> 426
<212> PRT
<213> Corynebacterium glutamicum

<400> 424
Met Ser Lys Lys Ser Val Leu Ile Thr Ser Leu Met Leu Phe Ser Met
1               5                   10                  15

Phe Phe Gly Ala Gly Asn Leu Ile Phe Pro Pro Met Leu Gly Leu Ser
            20                  25                  30

Ala Gly Thr Asn Tyr Leu Pro Ala Ile Leu Gly Phe Leu Ala Thr Ser
        35                  40                  45

Val Leu Leu Pro Val Leu Ala Ile Ile Ala Val Val Leu Ser Gly Glu
    50                  55                  60

Asn Val Lys Asp Met Ala Ser Arg Gly Gly Lys Ile Phe Gly Leu Val
65                  70                  75                  80

Phe Pro Ile Ala Ala Tyr Leu Ser Ile Gly Ala Phe Tyr Ala Leu Pro
                85                  90                  95

Arg Thr Gly Ala Val Ser Tyr Ser Thr Ala Val Gly Val Asp Asn Ala
            100                 105                 110

Leu Tyr Ser Gly Leu Phe Asn Phe Val Phe Phe Ala Val Ala Leu Ala
        115                 120                 125

Leu Ser Trp Asn Pro Asn Gly Ile Ala Asp Lys Leu Gly Lys Trp Leu
    130                 135                 140

Thr Pro Ala Leu Leu Thr Leu Ile Val Val Leu Val Val Leu Ser Val
145                 150                 155                 160

Ala Lys Leu Asp Gly Thr Pro Gly Glu Pro Ser Ser Ala Tyr Ala Gln
                165                 170                 175

Gln Pro Ala Gly Ala Gly Leu Leu Glu Gly Tyr Met Thr Met Asp Ala
            180                 185                 190

Ile Ala Ala Leu Ala Phe Gly Ile Val Val Ile Ser Ala Phe Lys Tyr
        195                 200                 205

Gln Lys Val Asn Lys Val Arg Thr Ala Thr Val Val Ser Ala Phe Ile
    210                 215                 220

Ala Gly Ile Leu Leu Ala Leu Val Tyr Leu Gly Leu Gly Ser Ile Gly
225                 230                 235                 240

Gln Val Val Asn Gly Glu Phe Ala Asp Gly Thr Ala Ile Leu Asn Tyr
                245                 250                 255

Ala Ala Leu Ser Thr Met Gly Gln Ala Gly Arg Ile Met Phe Val Ala
            260                 265                 270

Ile Leu Ile Leu Ala Cys Met Thr Thr Ala Val Gly Leu Ile Ser Ala
        275                 280                 285

Thr Ser Glu Phe Phe Asn Ser Leu Leu Pro Gly Val Lys Tyr His Val
    290                 295                 300

Trp Ala Thr Val Phe Ala Leu Ile Ser Phe Gly Val Ala Thr Met Gly
305                 310                 315                 320

Leu Asp Thr Val Leu Ala Val Ala Ala Pro Val Ile Ser Phe Ile Tyr
                325                 330                 335

Pro Ser Ala Ile Thr Leu Val Phe Leu Ser Leu Ile Glu Pro Leu Leu
            340                 345                 350

Phe Arg Leu Lys Trp Thr Tyr Leu Phe Gly Ile Trp Thr Ala Val Val
        355                 360                 365

Trp Ala Leu Phe Met Ser Ile Pro Ala Leu Asn Pro Phe Ile Glu Trp
    370                 375                 380

Ala Pro Leu His Ser Met Ser Leu Gly Trp Val Val Pro Val Leu Val
385                 390                 395                 400

Ala Ser Ala Ile Gly Leu Ala Ile Asp Trp Asn Lys Lys Gly Ala Gln
                405                 410                 415

Ser Val Ala Lys Lys Glu Ser Ile Ser Val
            420                 425

<210> 425
<211> 1461
<212> DNA
<213> Corynebacterium glutamicum

<220>
<221> CDS
<222> (101)..(1438)

<223> RXA00623

<400> 425
tttaagcttc gtcggcaacg atcacaccac gttaaatttc gaccatcaat gacggtggcc 60

gtttttggc gaatccccaa aaaggtcgaa gaggaagttc atg gat tca aac aca 115
Met Asp Ser Asn Thr
1               5

gaa tct tca agt gtt gag gtc aaa aac gaa cac att aaa gtt caa aag 163
Glu Ser Ser Ser Val Glu Val Lys Asn Glu His Ile Lys Val Gln Lys
                10                  15                  20

ccg ccg aag aag gac cgc act cac tgg ctc tac att gcg gtc att atc 211
Pro Pro Lys Lys Asp Arg Thr His Trp Leu Tyr Ile Ala Val Ile Ile
            25                  30                  35

gca ttg att ggc ggt att acc cta ggc ctg att tca ccg gag ttg ggc 259
Ala Leu Ile Gly Gly Ile Thr Leu Gly Leu Ile Ser Pro Glu Leu Gly
        40                  45                  50

aaa gaa ttc aag att ttg ggc acc atg ttt gtg tcc ttg atc aag atg 307
Lys Glu Phe Lys Ile Leu Gly Thr Met Phe Val Ser Leu Ile Lys Met
    55                  60                  65

att atc gct cca gtt att ttc tgc acc atc gtc atc gga atc ggt tca 355
Ile Ile Ala Pro Val Ile Phe Cys Thr Ile Val Ile Gly Ile Gly Ser
70                  75                  80                  85

gtc aag gca gcg gca aca gtc gga cgc gct ggt ggc atc gcc ctt gcg 403
Val Lys Ala Ala Ala Thr Val Gly Arg Ala Gly Gly Ile Ala Leu Ala
                90                  95                  100

tac ttc atc acg atg tcc aca ttc gca ctc gca gtt ggc ctg cta gtc 451
Tyr Phe Ile Thr Met Ser Thr Phe Ala Leu Ala Val Gly Leu Leu Val
            105                 110                 115

ggt aac ttc atc cag cca ggt agc gga ctg aac atc tca gtt gat gaa 499
Gly Asn Phe Ile Gln Pro Gly Ser Gly Leu Asn Ile Ser Val Asp Glu
        120                 125                 130

gaa tct tca ttc gca tcc aca gag agc agc cct gaa gga ctc ttg gga 547
Glu Ser Ser Phe Ala Ser Thr Glu Ser Ser Pro Glu Gly Leu Leu Gly
    135                 140                 145

ttc atc cac tcg atc atc cct gaa acg ttc ttc tct gca ttt act gat 595
Phe Ile His Ser Ile Ile Pro Glu Thr Phe Phe Ser Ala Phe Thr Asp
150                 155                 160                 165

ggt tcg gtg ctg cag gta ctg ttc atc gcc atc ctc gtg ggc ttt gca 643
Gly Ser Val Leu Gln Val Leu Phe Ile Ala Ile Leu Val Gly Phe Ala
                170                 175                 180

gct cag tcg atg ggt gaa aag gga cag ccc atc ctt gat ttc gta tcc 691
Ala Gln Ser Met Gly Glu Lys Gly Gln Pro Ile Leu Asp Phe Val Ser
            185                 190                 195

cat ctg cag aag ctc atc ttc aag att ttg aac tgg att ctg tgg ctc 739
His Leu Gln Lys Leu Ile Phe Lys Ile Leu Asn Trp Ile Leu Trp Leu
        200                 205                 210

gcc cca gtc ggt gca ttc ggt gca atg gcc ggc gtc gtt ggc gaa aca     787
Ala Pro Val Gly Ala Phe Gly Ala Met Ala Gly Val Val Gly Glu Thr
    215                 220                 225

ggc ttt gat gcc gtt gtt cag ctc ggt att ttg atc ctc gcc ttt tac     835
Gly Phe Asp Ala Val Val Gln Leu Gly Ile Leu Ile Leu Ala Phe Tyr
230                 235                 240                 245

gtc acc tgc gtg atc ttc atc ttt ggc gtg ctg ggc gcc gta ctg aag     883
Val Thr Cys Val Ile Phe Ile Phe Gly Val Leu Gly Ala Val Leu Lys
                250                 255                 260

gtg ttc acc ggc gtg aat atc ttc aag ctg gtc aag tac ctt gcc aag     931
Val Phe Thr Gly Val Asn Ile Phe Lys Leu Val Lys Tyr Leu Ala Lys
            265                 270                 275

gaa ttc ctg ctg atc ttt gct acc tca tcc tct gaa tct gcc ttg cca     979
Glu Phe Leu Leu Ile Phe Ala Thr Ser Ser Ser Glu Ser Ala Leu Pro
        280                 285                 290

aac ctc atg cgc aag atg gaa cac atc ggt gtg gct aaa cca acc gtc     1027
Asn Leu Met Arg Lys Met Glu His Ile Gly Val Ala Lys Pro Thr Val
    295                 300                 305

gga atc gtg gtc cca acc ggc tat tcc ttc aac ttg gac ggc acc gca     1075
Gly Ile Val Val Pro Thr Gly Tyr Ser Phe Asn Leu Asp Gly Thr Ala
310                 315                 320                 325

att tac ctc acc atg gca tct atc ttc att gcc gac gcg atg aat atg     1123
Ile Tyr Leu Thr Met Ala Ser Ile Phe Ile Ala Asp Ala Met Asn Met
                330                 335                 340

ccg atg agc ctc ggc gag cag gtc ggt ctg ctt gtc ttc atg atc atc     1171
Pro Met Ser Leu Gly Glu Gln Val Gly Leu Leu Val Phe Met Ile Ile
            345                 350                 355

gca tcc aag ggc gct gct ggt gtc tcg ggt gcc ggt att gca acg ttg     1219
Ala Ser Lys Gly Ala Ala Gly Val Ser Gly Ala Gly Ile Ala Thr Leu
        360                 365                 370

gct gcc gga ttg tct tca cac cgc cca gaa ctt ctg cac ggc gtt gac     1267
Ala Ala Gly Leu Ser Ser His Arg Pro Glu Leu Leu His Gly Val Asp
    375                 380                 385

gtg att gtg ggc atc gat aaa ttc atg tct gaa gcc cgc gca cta acc     1315
Val Ile Val Gly Ile Asp Lys Phe Met Ser Glu Ala Arg Ala Leu Thr
390                 395                 400                 405

aac ttc gcc gga aac tcc gtg gca aca ctg ctg gtc ggc aag tgg act     1363
Asn Phe Ala Gly Asn Ser Val Ala Thr Leu Leu Val Gly Lys Trp Thr
                410                 415                 420

ggc acc gtg gac atg aac caa gtc cat gac gtt ttg aat gga aaa tct     1411
Gly Thr Val Asp Met Asn Gln Val His Asp Val Leu Asn Gly Lys Ser
            425                 430                 435

cca ttt gtg gag tta gaa gaa gac cac tagttttcaa caggacgaca           1458
Pro Phe Val Glu Leu Glu Glu Asp His
        440                 445

acg                                                                 1461

<210> 426
<211> 446
<212> PRT
<213> Corynebacterium glutamicum

<400> 426
Met Asp Ser Asn Thr Glu Ser Ser Ser Val Glu Val Lys Asn Glu His
1               5                   10                  15

Ile Lys Val Gln Lys Pro Pro Lys Lys Asp Arg Thr His Trp Leu Tyr
            20                  25                  30

Ile Ala Val Ile Ile Ala Leu Ile Gly Gly Ile Thr Leu Gly Leu Ile
        35                  40                  45

Ser Pro Glu Leu Gly Lys Glu Phe Lys Ile Leu Gly Thr Met Phe Val
    50                  55                  60

Ser Leu Ile Lys Met Ile Ile Ala Pro Val Ile Phe Cys Thr Ile Val
65                  70                  75                  80

Ile Gly Ile Gly Ser Val Lys Ala Ala Ala Thr Val Gly Arg Ala Gly
                85                  90                  95

Gly Ile Ala Leu Ala Tyr Phe Ile Thr Met Ser Thr Phe Ala Leu Ala
            100                 105                 110

Val Gly Leu Leu Val Gly Asn Phe Ile Gln Pro Gly Ser Gly Leu Asn
        115                 120                 125

Ile Ser Val Asp Glu Glu Ser Ser Phe Ala Ser Thr Glu Ser Ser Pro
    130                 135                 140

Glu Gly Leu Leu Gly Phe Ile His Ser Ile Ile Pro Glu Thr Phe Phe
145                 150                 155                 160

Ser Ala Phe Thr Asp Gly Ser Val Leu Gln Val Leu Phe Ile Ala Ile
                165                 170                 175

Leu Val Gly Phe Ala Ala Gln Ser Met Gly Glu Lys Gly Gln Pro Ile
            180                 185                 190

Leu Asp Phe Val Ser His Leu Gln Lys Leu Ile Phe Lys Ile Leu Asn
        195                 200                 205

Trp Ile Leu Trp Leu Ala Pro Val Gly Ala Phe Gly Ala Met Ala Gly
    210                 215                 220

Val Val Gly Glu Thr Gly Phe Asp Ala Val Val Gln Leu Gly Ile Leu
225                 230                 235                 240

Ile Leu Ala Phe Tyr Val Thr Cys Val Ile Phe Ile Phe Gly Val Leu
                245                 250                 255

Gly Ala Val Leu Lys Val Phe Thr Gly Val Asn Ile Phe Lys Leu Val
            260                 265                 270

Lys Tyr Leu Ala Lys Glu Phe Leu Leu Ile Phe Ala Thr Ser Ser Ser
        275                 280                 285

Glu Ser Ala Leu Pro Asn Leu Met Arg Lys Met Glu His Ile Gly Val
    290                 295                 300

Ala Lys Pro Thr Val Gly Ile Val Val Pro Thr Gly Tyr Ser Phe Asn
305                 310                 315                 320

Leu Asp Gly Thr Ala Ile Tyr Leu Thr Met Ala Ser Ile Phe Ile Ala
                325                 330                 335

Asp Ala Met Asn Met Pro Met Ser Leu Gly Glu Gln Val Gly Leu Leu
            340                 345                 350

Val Phe Met Ile Ile Ala Ser Lys Gly Ala Ala Gly Val Ser Gly Ala
        355                 360                 365

Gly Ile Ala Thr Leu Ala Ala Gly Leu Ser Ser His Arg Pro Glu Leu
    370                 375                 380

Leu His Gly Val Asp Val Ile Val Gly Ile Asp Lys Phe Met Ser Glu
385                 390                 395                 400

Ala Arg Ala Leu Thr Asn Phe Ala Gly Asn Ser Val Ala Thr Leu Leu
                405                 410                 415

Val Gly Lys Trp Thr Gly Thr Val Asp Met Asn Gln Val His Asp Val
            420                 425                 430

Leu Asn Gly Lys Ser Pro Phe Val Glu Leu Glu Glu Asp His
        435                 440                 445

<210> 427
<211> 620
<212> DNA
<213> Corynebacterium glutamicum

<220>
<221> CDS
<222> (55)..(597)
<223> RXA01584

<400> 427
ccaccccctt ccccgctacg cgccataccg gcttgggcgg gcatcggatc actagtg     57
                                                          Val
                                                            1

gtg ttt ttg ggc gca ctt ctc ggc gcc gta atc atg ggc ggg ttt tat    105
Val Phe Leu Gly Ala Leu Leu Gly Ala Val Ile Met Gly Gly Phe Tyr
            5                   10                  15

cca gca ttc att caa gcc gga tcc aca gtg ttc ggc ggc ggc cac gtg    153
Pro Ala Phe Ile Gln Ala Gly Ser Thr Val Phe Gly Gly Gly His Val
        20                  25                  30

gtt ttg cca ctg ctg gaa aag ctc gtt gtt gcg ccc ggt ttt att aaa    201
Val Leu Pro Leu Leu Glu Lys Leu Val Val Ala Pro Gly Phe Ile Lys
    35                  40                  45

gaa acc gac ttc cta tcc ggc tac tcc gca gcg cag gca gtg cct ggc    249
Glu Thr Asp Phe Leu Ser Gly Tyr Ser Ala Ala Gln Ala Val Pro Gly

50                  55                  60                  65

ccc atg ttc agc ttc gcc agc tac ctc ggc gca atc tac ggt ggc atc 297
Pro Met Phe Ser Phe Ala Ser Tyr Leu Gly Ala Ile Tyr Gly Gly Ile
                70                  75                  80

ggt ggt gca gtg ctg gcc agc ctg gcg atc ttc ttc ccc gcc gca ctc 345
Gly Gly Ala Val Leu Ala Ser Leu Ala Ile Phe Phe Pro Ala Ala Leu
            85                  90                  95

ttg agc atc agc gga atg tac ttt tgg gga cgc tgg cga aaa gca ccg 393
Leu Ser Ile Ser Gly Met Tyr Phe Trp Gly Arg Trp Arg Lys Ala Pro
        100                 105                 110

cgc atc caa gca gca gtc acc ggc atc aac gcc ggt gtg gtg ggg ctt 441
Arg Ile Gln Ala Ala Val Thr Gly Ile Asn Ala Gly Val Val Gly Leu
    115                 120                 125

ttg ggc gca gcg ctc tac gat ccc gta ttc acc cac ggc atc acc agc 489
Leu Gly Ala Ala Leu Tyr Asp Pro Val Phe Thr His Gly Ile Thr Ser
130                 135                 140                 145

gtt tcc gca tta gct atc gca acg gtg tgt tgg ctg ggg cta gcc cac 537
Val Ser Ala Leu Ala Ile Ala Thr Val Cys Trp Leu Gly Leu Ala His
                150                 155                 160

tgg aaa att ccg ccg tgg gcc atc gcc gcg ggt gcg gcc ctt gca ggc 585
Trp Lys Ile Pro Pro Trp Ala Ile Ala Ala Gly Ala Ala Leu Ala Gly
165- 170 175

tgg gtc ttg ctt tagaaaacgc tcagacccaa acc 620
Trp Val Leu Leu
180

<210> 428
<211> 181
<212> PRT
<213> Corynebacterium glutamicum

<400> 428
   Val Val Phe Leu Gly Ala Leu Leu Gly Ala Val Ile Met Gly Gly Phe
     1               5                  10                  15
   Tyr Pro Ala Phe Ile Gln Ala Gly Ser Thr Val Phe Gly Gly Gly His
                20                  25                  30
   Val Val Leu Pro Leu Leu Glu Lys Leu Val Val Ala Pro Gly Phe Ile
            35                  40                  45
   Lys Glu Thr Asp Phe Leu Ser Gly Tyr Ser Ala Ala Gln Ala Val Pro
        50                  55                  60
   Gly Pro Met Phe Ser Phe Ala Ser Tyr Leu Gly Ala Ile Tyr Gly Gly
    65                  70                  75                  80
   Ile Gly Gly Ala Val Leu Ala Ser Leu Ala Ile Phe Phe Pro Ala Ala
                    85                  90                  95
   Leu Leu Ser Ile Ser Gly Met Tyr Phe Trp Gly Arg Trp Arg Lys Ala
               100                 105                 110
   Pro Arg Ile Gln Ala Ala Val Thr Gly Ile Asn Ala Gly Val Val Gly
         115                 120                 125
   Leu Leu Gly Ala Ala Leu Tyr Asp Pro Val Phe Thr His Gly Ile Thr
       130                 135                 140
   Ser Val Ser Ala Leu Ala Ile Ala Thr Val Cys Trp Leu Gly Leu Ala
   145                 150                 155                 160
   His Trp Lys Ile Pro Pro Trp Ala Ile Ala Ala Gly Ala Ala Leu Ala
                   165                 170                 175
   Gly Trp Val Leu Leu
               180

<210> 429
<211> 813
<212> DNA
<213> Corynebacterium glutamicum

<220>
<221> CDS
<222> (101)..(790)
<223> RXA00852

<400> 429
agatcaccgt catggtcata atcgccgctg gagttcatgc agcattccct gacatccgta 60

agaaatagtt ccaacccact tttcctcaga attgcagttc atg ccc gag atc att   115
                                          Met Pro Glu Ile Ile
                                          1               5

ttt gac aac act gaa gta cgc tac gat gac tcg ctc att tta gag ccc   163
Phe Asp Asn Thr Glu Val Arg Tyr Asp Asp Ser Leu Ile Leu Glu Pro
                10                  15                  20

cta tcg tta aaa ctg aca gaa caa cgc att ggc atc atc ggg gct aac   211
Leu Ser Leu Lys Leu Thr Glu Gln Arg Ile Gly Ile Ile Gly Ala Asn
            25                  30                  35

ggc ggt gga aaa tcc acg ctc atc aga atg atc aat ggt ctc ggc gaa   259
Gly Gly Gly Lys Ser Thr Leu Ile Arg Met Ile Asn Gly Leu Gly Glu
        40                  45                  50

cca acc aca ggg cgt gtt cta gtt gat ggc ctt gac gtc tcg cat tcc   307
Pro Thr Thr Gly Arg Val Leu Val Asp Gly Leu Asp Val Ser His Ser
    55                  60                  65

gga cgg gaa gtt cgc aag aag gtt gga ttt gtc ttc tct gac gct gaa   355
Gly Arg Glu Val Arg Lys Lys Val Gly Phe Val Phe Ser Asp Ala Glu
70                  75                  80                  85

aac cag atc gtg atg cca act gtg cgt gag gat att gcc ttc tcg ctt   403
Asn Gln Ile Val Met Pro Thr Val Arg Glu Asp Ile Ala Phe Ser Leu
                90                  95                  100

cgc cgg cac aaa atg cca cgc gct gaa aag gcg caa cgt gtc gac gag   451
Arg Arg His Lys Met Pro Arg Ala Glu Lys Ala Gln Arg Val Asp Glu
            105                 110                 115

atg atg gcg cga ttc aac ttg agc gag cat gca gat caa tca ccg cac     499
Met Met Ala Arg Phe Asn Leu Ser Glu His Ala Asp Gln Ser Pro His
        120                 125                 130

acc cta tcc ggt ggt caa aag cag ttg tta gcg ctg gct gca gta ctg     547
Thr Leu Ser Gly Gly Gln Lys Gln Leu Leu Ala Leu Ala Ala Val Leu
    135                 140                 145

att ttg gag cca gaa gtg atc atc gct gat gag ccc act acc ctg ctg     595
Ile Leu Glu Pro Glu Val Ile Ile Ala Asp Glu Pro Thr Thr Leu Leu
150                 155                 160                 165

gat ctg cgc aat agg ctg atg atc aaa gac gtg ttc aat aaa ctc gag     643
Asp Leu Arg Asn Arg Leu Met Ile Lys Asp Val Phe Asn Lys Leu Glu
                170                 175                 180

cag caa tta atc gtt gtc agc cat gat tta gat ttc ctc agc gat ttt     691
Gln Gln Leu Ile Val Val Ser His Asp Leu Asp Phe Leu Ser Asp Phe
            185                 190                 195

gag cgg gtc att tgc atc aat gat cat aaa atc gct gct gat ggc cct     739
Glu Arg Val Ile Cys Ile Asn Asp His Lys Ile Ala Ala Asp Gly Pro
        200                 205                 210

ccg caa aag tcc att gac ctg tac gta tcg ctt atg gcg gaa cct gcg     787
Pro Gln Lys Ser Ile Asp Leu Tyr Val Ser Leu Met Ala Glu Pro Ala
    215                 220                 225

aaa tgaacagtat tcctttaggt ttt                                       813
Lys
230

<210> 430
<211> 230
<212> PRT
<213> Corynebacterium glutamicum

<400> 430
Met Pro Glu Ile Ile Phe Asp Asn Thr Glu Val Arg Tyr Asp Asp Ser
1               5                   10                  15

Leu Ile Leu Glu Pro Leu Ser Leu Lys Leu Thr Glu Gln Arg Ile Gly
            20                  25                  30

Ile Ile Gly Ala Asn Gly Gly Gly Lys Ser Thr Leu Ile Arg Met Ile
        35                  40                  45

Asn Gly Leu Gly Glu Pro Thr Thr Gly Arg Val Leu Val Asp Gly Leu
    50                  55                  60

Asp Val Ser His Ser Gly Arg Glu Val Arg Lys Lys Val Gly Phe Val
65                  70                  75                  80

Phe Ser Asp Ala Glu Asn Gln Ile Val Met Pro Thr Val Arg Glu Asp
                85                  90                  95

Ile Ala Phe Ser Leu Arg Arg His Lys Met Pro Arg Ala Glu Lys Ala
            100                 105                 110

Gln Arg Val Asp Glu Met Met Ala Arg Phe Asn Leu Ser Glu His Ala
        115                 120                 125

Asp Gln Ser Pro His Thr Leu Ser Gly Gly Gln Lys Gln Leu Leu Ala
    130                 135                 140

Leu Ala Ala Val Leu Ile Leu Glu Pro Glu Val Ile Ile Ala Asp Glu
145                 150                 155                 160

Pro Thr Thr Leu Leu Asp Leu Arg Asn Arg Leu Met Ile Lys Asp Val
                165                 170                 175

Phe Asn Lys Leu Glu Gln Gln Leu Ile Val Val Ser His Asp Leu Asp
            180                 185                 190

Phe Leu Ser Asp Phe Glu Arg Val Ile Cys Ile Asn Asp His Lys Ile
        195                 200                 205

Ala Ala Asp Gly Pro Pro Gln Lys Ser Ile Asp Leu Tyr Val Ser Leu
    210                 215                 220

Met Ala Glu Pro Ala Lys
225                 230

<210> 431
<211> 1269
<212> DNA
<213> Corynebacterium glutamicum

<220>
<221> CDS
<222> (101)..(1246)
<223> RXA00690

<400> 431
atggatcatt ggactcacac tggctgtcat ttccttggtt tcagcgtctg ctgtggcggc 60

gtgggcgatt cgtcgttcag aggtccaggg ttaaagcttc gtg aaa tgg att gag   115
                                            Val Lys Trp Ile Glu
                                            1               5

cga tat gtg ctg tcc cgg cgg atg gtt cat ccc tgg gcg tgg tgg gtg   163
Arg Tyr Val Leu Ser Arg Arg Met Val His Pro Trp Ala Trp Trp Val
                10                  15                  20

tgg gcg ttg ggt att gct ggt tgt gcc agc atg acc aac aat cct tat   211
Trp Ala Leu Gly Ile Ala Gly Cys Ala Ser Met Thr Asn Asn Pro Tyr
            25                  30                  35

att ttg gcg ctc act ttt gcc acg ttg tgt ttt gtg gtg ttt aac cgt   259
Ile Leu Ala Leu Thr Phe Ala Thr Leu Cys Phe Val Val Phe Asn Arg
        40                  45                  50

cgt ggg tca tcg ccg tgg tcg cgt gct ttc ccg atc tat ttg atg atc   307
Arg Gly Ser Ser Pro Trp Ser Arg Ala Phe Pro Ile Tyr Leu Met Ile
    55                  60                  65

gcg ggt tgg ctc gtg gtg tac cgg ttg gtc atg cac att gtg gtg gga   355
Ala Gly Trp Leu Val Val Tyr Arg Leu Val Met His Ile Val Val Gly
70                  75                  80                  85

gca aaa att ggc acc att gaa ctg ttt cgg atc ccg ccg gtg cag ttg 403
Ala Lys Ile Gly Thr Ile Glu Leu Phe Arg Ile Pro Pro Val Gln Leu
                90                  95                  100

ccg gaa tgg gct gcg ggt atc cac gtg ttt ggc acg gtg tat ctc gag 451
Pro Glu Trp Ala Ala Gly Ile His Val Phe Gly Thr Val Tyr Leu Glu
            105                 110                 115

ggt ctg atc atc gcg acg acg caa ggc tta acg ctt gga acg atg atc 499
Gly Leu Ile Ile Ala Thr Thr Gln Gly Leu Thr Leu Gly Thr Met Ile
        120                 125                 130

gtg gcg gtg ggt gct gcg aac tct ttg gcg gat ccc aag aag ctg ctc 547
Val Ala Val Gly Ala Ala Asn Ser Leu Ala Asp Pro Lys Lys Leu Leu
    135                 140                 145

aaa tcg ttg cct ggc gca ttg ggc gaa ctg gga act gcg gtg gtc atc 595
Lys Ser Leu Pro Gly Ala Leu Gly Glu Leu Gly Thr Ala Val Val Ile
150                 155                 160                 165

ggt att tcc att gca cct cag atg gct gag tcg gcg ttc cgc att aat 643
Gly Ile Ser Ile Ala Pro Gln Met Ala Glu Ser Ala Phe Arg Ile Asn
                170                 175                 180

cgt gca cga acc ttg cgt ggt gat gat gcc aaa ggt gtt cgt ggt ttc 691
Arg Ala Arg Thr Leu Arg Gly Asp Asp Ala Lys Gly Val Arg Gly Phe
            185                 190                 195

gcg cgg att ttg atg ccg gtt ttc cag gac act ttg gat agg tct ttg 739
Ala Arg Ile Leu Met Pro Val Phe Gln Asp Thr Leu Asp Arg Ser Leu
        200                 205                 210

gcc ctg gct aat tcc atg gat gcc cgt ggt tat ggc agg cag gct cat 787
Ala Leu Ala Asn Ser Met Asp Ala Arg Gly Tyr Gly Arg Gln Ala His
    215                 220                 225

gta tcc aaa ttc caa cag cgt gtg acc tct att ttt ggt gca ttc gga 835
Val Ser Lys Phe Gln Gln Arg Val Thr Ser Ile Phe Gly Ala Phe Gly
230                 235                 240                 245

ata ctc ggc gtg acc gtt ggt ctg ttt gtg gtc tta gat gca tca tca 883
Ile Leu Gly Val Thr Val Gly Leu Phe Val Val Leu Asp Ala Ser Ser
                250                 255                 260

ccg atg ttc gtt gcc gtt ccg gtg ttt att acc ggc gtg ggc ttc ttg 931
Pro Met Phe Val Ala Val Pro Val Phe Ile Thr Gly Val Gly Phe Leu
            265                 270                 275

atc att tcg ttg gtc gtt gct tca cat aga aaa aca tcc acc act ttt 979
Ile Ile Ser Leu Val Val Ala Ser His Arg Lys Thr Ser Thr Thr Phe
        280                 285                 290

gat cag ttg cct tgg ggt gct gcg gaa tgg ctt gtg tgc atc aca ggt 1027
Asp Gln Leu Pro Trp Gly Ala Ala Glu Trp Leu Val Cys Ile Thr Gly
    295                 300                 305

gtg att ccg ctg ctc atg gct gcg ctg aca cga tac ctt gat cca ggt 1075
Val Ile Pro Leu Leu Met Ala Ala Leu Thr Arg Tyr Leu Asp Pro Gly
310                 315                 320                 325

tcc atg atc acc acc tgg gtt cct ttg cat atg cca gac acc gtt ccg    1123
Ser Met Ile Thr Thr Trp Val Pro Leu His Met Pro Asp Thr Val Pro
                330                 335                 340

ttg ctc gtt gtg gca gga ctt gtt gtg gcg acg atg cca gga ttc ttg    1171
Leu Leu Val Val Ala Gly Leu Val Val Ala Thr Met Pro Gly Phe Leu
            345                 350                 355

acg ccc cgc ttg ccg aag aac aaa gtg agg gtc aag cgt cga aaa gca    1219
Thr Pro Arg Leu Pro Lys Asn Lys Val Arg Val Lys Arg Arg Lys Ala
        360                 365                 370

ata aat agc cca gaa agg gcc gaa gtt taatgagtgc tccttttagc          1266
Ile Asn Ser Pro Glu Arg Ala Glu Val
    375                 380

gcg                                                                1269

<210> 432
<211> 382
<212> PRT
<213> Corynebacterium glutamicum

<400> 432
Val Lys Trp Ile Glu Arg Tyr Val Leu Ser Arg Arg Met Val His Pro
  1               5                  10                  15

Trp Ala Trp Trp Val Trp Ala Leu Gly Ile Ala Gly Cys Ala Ser Met
             20                  25                  30

Thr Asn Asn Pro Tyr Ile Leu Ala Leu Thr Phe Ala Thr Leu Cys Phe
         35                  40                  45

Val Val Phe Asn Arg Arg Gly Ser Ser Pro Trp Ser Arg Ala Phe Pro
     50                  55                  60

Ile Tyr Leu Met Ile Ala Gly Trp Leu Val Val Tyr Arg Leu Val Met
 65                  70                  75                  80

His Ile Val Val Gly Ala Lys Ile Gly Thr Ile Glu Leu Phe Arg Ile
                 85                  90                  95

Pro Pro Val Gln Leu Pro Glu Trp Ala Ala Gly Ile His Val Phe Gly
            100                 105                 110

Thr Val Tyr Leu Glu Gly Leu Ile Ile Ala Thr Thr Gln Gly Leu Thr
        115                 120                 125

Leu Gly Thr Met Ile Val Ala Val Gly Ala Ala Asn Ser Leu Ala Asp
    130                 135                 140

Pro Lys Lys Leu Leu Lys Ser Leu Pro Gly Ala Leu Gly Glu Leu Gly
145                 150                 155                 160

Thr Ala Val Val Ile Gly Ile Ser Ile Ala Pro Gln Met Ala Glu Ser
                165                 170                 175

Ala Phe Arg Ile Asn Arg Ala Arg Thr Leu Arg Gly Asp Asp Ala Lys
            180                 185                 190

Gly Val Arg Gly Phe Ala Arg Ile Leu Met Pro Val Phe Gln Asp Thr
        195                 200                 205

Leu Asp Arg Ser Leu Ala Leu Ala Asn Ser Met Asp Ala Arg Gly Tyr
    210                 215                 220

Gly Arg Gln Ala His Val Ser Lys Phe Gln Gln Arg Val Thr Ser Ile
225                 230                 235                 240

Phe Gly Ala Phe Gly Ile Leu Gly Val Thr Val Gly Leu Phe Val Val
                245                 250                 255

Leu Asp Ala Ser Ser Pro Met Phe Val Ala Val Pro Val Phe Ile Thr
            260                 265                 270

Gly Val Gly Phe Leu Ile Ile Ser Leu Val Val Ala Ser His Arg Lys
        275                 280                 285

Thr Ser Thr Thr Phe Asp Gln Leu Pro Trp Gly Ala Ala Glu Trp Leu
    290                 295                 300

Val Cys Ile Thr Gly Val Ile Pro Leu Leu Met Ala Ala Leu Thr Arg
305                 310                 315                 320

Tyr Leu Asp Pro Gly Ser Met Ile Thr Thr Trp Val Pro Leu His Met
                325                 330                 335

Pro Asp Thr Val Pro Leu Leu Val Val Ala Gly Leu Val Val Ala Thr
            340                 345                 350

Met Pro Gly Phe Leu Thr Pro Arg Leu Pro Lys Asn Lys Val Arg Val
        355                 360                 365

Lys Arg Arg Lys Ala Ile Asn Ser Pro Glu Arg Ala Glu Val
    370                 375                 380

<210> 433
<211> 876
<212> DNA
<213> Corynebacterium glutamicum

<220>
<221> CDS
<222> (101)..(853)
<223> RXA00827

<400> 433
tgcgtgaatt aacagacaac ggaatcagca ttgtgtcagt aacccatgat cctgatttca 60

tcgcagcgct gggcgatcac cacattgagg tgagcgcgaa gtg aac ctg ctg atc 115
Val Asn Leu Leu Ile
1               5

aaa att aat ccc gtc acc cgc atc atc gcg ttg atg gta ctg acc acg 163
Lys Ile Asn Pro Val Thr Arg Ile Ile Ala Leu Met Val Leu Thr Thr
                10                  15                  20

ccg ttg ctg ctg agt ttg gat gtg atg tcg gca gcg atc gcg ctg gtg 211
Pro Leu Leu Leu Ser Leu Asp Val Met Ser Ala Ala Ile Ala Leu Val
            25                  30                  35

gca acc att att ctg gca cca ttt gcc ggc gtg acc tgg aag atg ctg 259
Ala Thr Ile Ile Leu Ala Pro Phe Ala Gly Val Thr Trp Lys Met Leu
        40                  45                  50

ctg aaa cgt ggc tgg atg ctg ttc ctc atg gca ccg gtg gct gca tta 307
Leu Lys Arg Gly Trp Met Leu Phe Leu Met Ala Pro Val Ala Ala Leu
    55                  60                  65

tcc atg gcg ctt tat ggc agg ccg gat gga aaa gag tac ttt agc ttc 355
Ser Met Ala Leu Tyr Gly Arg Pro Asp Gly Lys Glu Tyr Phe Ser Phe
70                  75                  80                  85

ctg ctc att cac gtc act gat aat tca ctg gct ttg gct gct gcc att 403
Leu Leu Ile His Val Thr Asp Asn Ser Leu Ala Leu Ala Ala Ala Ile
                90                  95                  100

ggg ctg cgt gtt ctg gcg att ggt ctg ccc gtt gtg gtg ctg att gct 451
Gly Leu Arg Val Leu Ala Ile Gly Leu Pro Val Val Val Leu Ile Ala
            105                 110                 115

cgc att gat ccc acc gac ctg ggc gat ggt ttg gcg cag ctg ctc aaa 499
Arg Ile Asp Pro Thr Asp Leu Gly Asp Gly Leu Ala Gln Leu Leu Lys
        120                 125                 130

ctg cct gaa agg ttt gtc atc ggt gct gtg gca gga agc cga ctg atg 547
Leu Pro Glu Arg Phe Val Ile Gly Ala Val Ala Gly Ser Arg Leu Met
    135                 140                 145

acg ctt ttt cgg gaa gat tgg tac tcc atg tcc agg gca agg cgt gcc 595
Thr Leu Phe Arg Glu Asp Trp Tyr Ser Met Ser Arg Ala Arg Arg Ala
150                 155                 160                 165

cgc gga att gct gat cag ggc aag atc aag cac ttt ttc acc atg act 643
Arg Gly Ile Ala Asp Gln Gly Lys Ile Lys His Phe Phe Thr Met Thr
                170                 175                 180

ttt ggt ttg ttg gtg ctc tcg ctt cgc cgt gga tcc aag ctt gca acg 691
Phe Gly Leu Leu Val Leu Ser Leu Arg Arg Gly Ser Lys Leu Ala Thr
            185                 190                 195

gcg atg gaa gca cgc ggt ttt ggt cgc acg act ggc cgc acc tgg gca 739
Ala Met Glu Ala Arg Gly Phe Gly Arg Thr Thr Gly Arg Thr Trp Ala
        200                 205                 210

agg gaa tcc acc gtc ggc gcg cgc gat ctg gtg ctc atc ttg gtg tgt 787
Arg Glu Ser Thr Val Gly Ala Arg Asp Leu Val Leu Ile Leu Val Cys
    215                 220                 225

gct gcc att tcc gcg atc gct cta acc gtg tcc att cag act ggt ttc 835
Ala Ala Ile Ser Ala Ile Ala Leu Thr Val Ser Ile Gln Thr Gly Phe
230                 235                 240                 245

ttt aag ttc ttg ggc aca tgatcacagt tttaattgat gga 876
Phe Lys Phe Leu Gly Thr
                250

<210> 434
<211> 251
<212> PRT

<213> Corynebacterium glutamicum

<400> 434
Val Asn Leu Leu Ile Lys Ile Asn Pro Val Thr Arg Ile Ile Ala Leu
1               5                   10                  15

Met Val Leu Thr Thr Pro Leu Leu Leu Ser Leu Asp Val Met Ser Ala
            20                  25                  30

Ala Ile Ala Leu Val Ala Thr Ile Ile Leu Ala Pro Phe Ala Gly Val
        35                  40                  45

Thr Trp Lys Met Leu Leu Lys Arg Gly Trp Met Leu Phe Leu Met Ala
    50                  55                  60

Pro Val Ala Ala Leu Ser Met Ala Leu Tyr Gly Arg Pro Asp Gly Lys
65                  70                  75                  80

Glu Tyr Phe Ser Phe Leu Leu Ile His Val Thr Asp Asn Ser Leu Ala
                85                  90                  95

Leu Ala Ala Ala Ile Gly Leu Arg Val Leu Ala Ile Gly Leu Pro Val
            100                 105                 110

Val Val Leu Ile Ala Arg Ile Asp Pro Thr Asp Leu Gly Asp Gly Leu
        115                 120                 125

Ala Gln Leu Leu Lys Leu Pro Glu Arg Phe Val Ile Gly Ala Val Ala
    130                 135                 140

Gly Ser Arg Leu Met Thr Leu Phe Arg Glu Asp Trp Tyr Ser Met Ser
145                 150                 155                 160

Arg Ala Arg Arg Ala Arg Gly Ile Ala Asp Gln Gly Lys Ile Lys His
                165                 170                 175

Phe Phe Thr Met Thr Phe Gly Leu Leu Val Leu Ser Leu Arg Arg Gly
            180                 185                 190

Ser Lys Leu Ala Thr Ala Met Glu Ala Arg Gly Phe Gly Arg Thr Thr
        195                 200                 205

Gly Arg Thr Trp Ala Arg Glu Ser Thr Val Gly Ala Arg Asp Leu Val
    210                 215                 220

Leu Ile Leu Val Cys Ala Ala Ile Ser Ala Ile Ala Leu Thr Val Ser
225                 230                 235                 240

Ile Gln Thr Gly Phe Phe Lys Phe Leu Gly Thr
                245                 250


<210> 435
<211> 732
<212> DNA
<213> Corynebacterium glutamicum

<220>
<221> CDS
<222> (101)..(709)
<223> RXA00851

<400> 435
ttgagcgggt catttgcatc aatgatcata aaatcgctgc tgatggccct ccgcaaaagt 60

ccattgacct gtacgtatcg cttatggcgg aacctgcgaa atg aac agt att cct 115
Met Asn Ser Ile Pro
1               5

tta ggt ttt tac gtc gat aag caa tct gtt gtt cat tct ttt cct gct 163
Leu Gly Phe Tyr Val Asp Lys Gln Ser Val Val His Ser Phe Pro Ala
                10                  15                  20

ttg tgg aaa ttc cca ctt ctg ctg ttt ttc atc atc ggc ggc tcc atc 211
Leu Trp Lys Phe Pro Leu Leu Leu Phe Phe Ile Ile Gly Gly Ser Ile
            25                  30                  35

gcg gct tct acc ccg gtt cat ggg ttg att ttg gtg ggg att gca gtg 259
Ala Ala Ser Thr Pro Val His Gly Leu Ile Leu Val Gly Ile Ala Val
        40                  45                  50

gtg ttt tac gtg ctg gcg aag att ccg ctg aag gtc gcg tgg gag cag 307
Val Phe Tyr Val Leu Ala Lys Ile Pro Leu Lys Val Ala Trp Glu Gln
    55                  60                  65

ttg tgg cca gtg ctg ccg att ttg atc atg ctc ggt gcg ttt cag tgg 355
Leu Trp Pro Val Leu Pro Ile Leu Ile Met Leu Gly Ala Phe Gln Trp
70                  75                  80                  85

tgg cag cgc ggc ttt gat ttc gcg gca acc aca gtg ctc acg ctg ttt 403
Trp Gln Arg Gly Phe Asp Phe Ala Ala Thr Thr Val Leu Thr Leu Phe
                90                  95                  100

tcc gcg gtg atg gcc gcc atg ttg ttg acg ttg acc acg cgg ttg gaa 451
Ser Ala Val Met Ala Ala Met Leu Leu Thr Leu Thr Thr Arg Leu Glu
            105                 110                 115

gcg ctc atg aat gca gtt gag cgg atg ttg cag cct ttt gca cgt ttt 499
Ala Leu Met Asn Ala Val Glu Arg Met Leu Gln Pro Phe Ala Arg Phe
        120                 125                 130

ggc ctg cca gta gag acg atc acc ttg gct att tct ctc acg att cgg 547
Gly Leu Pro Val Glu Thr Ile Thr Leu Ala Ile Ser Leu Thr Ile Arg
    135                 140                 145

ctc att ccg ctg caa tta gcc acg gtg aag gaa gtc ctc gat gcc cgt 595
Leu Ile Pro Leu Gln Leu Ala Thr Val Lys Glu Val Leu Asp Ala Arg
150                 155                 160                 165

aaa gct cgt ggt gcc ggt ttt tct atc gcc gcg ttt ggc acg cct gtg 643
Lys Ala Arg Gly Ala Gly Phe Ser Ile Ala Ala Phe Gly Thr Pro Val
                170                 175                 180

atc atc aga tca ata aag agg gcc cgc aat atc ggc gat gct ctt ctc 691
Ile Ile Arg Ser Ile Lys Arg Ala Arg Asn Ile Gly Asp Ala Leu Leu
            185                 190                 195

gca cgt ggt gcc ggc gat taatttcttt caacacatag caa 732
Ala Arg Gly Ala Gly Asp
        200

<210> 436
<211> 203
<212> PRT
<213> Corynebacterium glutamicum

<400> 436
Met Asn Ser Ile Pro Leu Gly Phe Tyr Val Asp Lys Gln Ser Val Val
1               5                   10                  15

His Ser Phe Pro Ala Leu Trp Lys Phe Pro Leu Leu Leu Phe Phe Ile
            20                  25                  30

Ile Gly Gly Ser Ile Ala Ala Ser Thr Pro Val His Gly Leu Ile Leu
        35                  40                  45

Val Gly Ile Ala Val Val Phe Tyr Val Leu Ala Lys Ile Pro Leu Lys
    50                  55                  60

Val Ala Trp Glu Gln Leu Trp Pro Val Leu Pro Ile Leu Ile Met Leu
65                  70                  75                  80

Gly Ala Phe Gln Trp Trp Gln Arg Gly Phe Asp Phe Ala Ala Thr Thr
                85                  90                  95

Val Leu Thr Leu Phe Ser Ala Val Met Ala Ala Met Leu Leu Thr Leu
            100                 105                 110

Thr Thr Arg Leu Glu Ala Leu Met Asn Ala Val Glu Arg Met Leu Gln
        115                 120                 125

Pro Phe Ala Arg Phe Gly Leu Pro Val Glu Thr Ile Thr Leu Ala Ile
    130                 135                 140

Ser Leu Thr Ile Arg Leu Ile Pro Leu Gln Leu Ala Thr Val Lys Glu
145                 150                 155                 160

Val Leu Asp Ala Arg Lys Ala Arg Gly Ala Gly Phe Ser Ile Ala Ala
                165                 170                 175

Phe Gly Thr Pro Val Ile Ile Arg Ser Ile Lys Arg Ala Arg Asn Ile
            180                 185                 190

Gly Asp Ala Leu Leu Ala Arg Gly Ala Gly Asp
        195                 200

<210> 437
<211> 725
<212> DNA
<213> Corynebacterium glutamicum

<220>
<221> CDS
<222> (3)..(695)
<223> RXS03220

<400> 437
ccatg ggc tta agg gaa att ttg tcc agc aag tgg ctt gtg cgc atc ctc 50
  Met Gly Leu Arg Glu Ile Leu Ser Ser Lys Trp Leu Val Arg Ile Leu
    1               5                   10                  15

ctg gta ggt atc gga ttg ggt gtc gca cag cag ctg acc ggc atc aac 98
Leu Val Gly Ile Gly Leu Gly Val Ala Gln Gln Leu Thr Gly Ile Asn
            20                  25                  30

tcc atc atg tac tac ggc cag gtt gtt ctc att gag gct ggt ttc tcc 146
Ser Ile Met Tyr Tyr Gly Gln Val Val Leu Ile Glu Ala Gly Phe Ser
        35                  40                  45

gag aat gca gct ctg atc gcc aac gtg gcg cca gga gtg atc gca gtt 194
Glu Asn Ala Ala Leu Ile Ala Asn Val Ala Pro Gly Val Ile Ala Val
    50                  55                  60

gtc ggt gca ttc atc gca ctg tgg atg atg gat ggt atc aac cgc cgt 242
Val Gly Ala Phe Ile Ala Leu Trp Met Met Asp Gly Ile Asn Arg Arg
65                  70                  75                  80

acc acc ctc att acc ggt tat tct ctc acc acc att agc cac gta ttg 290
Thr Thr Leu Ile Thr Gly Tyr Ser Leu Thr Thr Ile Ser His Val Leu
                85                  90                  95

atc ggt atc gca tcc gta gca ttc cca gtc ggc gat cct ctt cgc ccc 338
Ile Gly Ile Ala Ser Val Ala Phe Pro Val Gly Asp Pro Leu Arg Pro
            100                 105                 110

tac gtt atc ttg act ctg gtt gtg gtc ttc gtg gga tcc atg cag acc 386
Tyr Val Ile Leu Thr Leu Val Val Val Phe Val Gly Ser Met Gln Thr
        115                 120                 125

ttc ctc aac gta gct acc tgg gtt atg ctc tct gag ctc ttc ccg ctg 434
Phe Leu Asn Val Ala Thr Trp Val Met Leu Ser Glu Leu Phe Pro Leu
    130                 135                 140

gca atg cgc ggt ttc gca atc ggt atc tca gtg ttc ttc ctc tgg atc 482
Ala Met Arg Gly Phe Ala Ile Gly Ile Ser Val Phe Phe Leu Trp Ile
145                 150                 155                 160

gca aac gcg ttc ctc gga ttg ttc ttc cca acc atc atg gaa gca gta 530
Ala Asn Ala Phe Leu Gly Leu Phe Phe Pro Thr Ile Met Glu Ala Val
                165                 170                 175

gga cta acc gga acc ttc ttc atg ttc gcc gga atc ggt gtg gtt gcc 578
Gly Leu Thr Gly Thr Phe Phe Met Phe Ala Gly Ile Gly Val Val Ala
            180                 185                 190

ttg atc ttc atc tac acc cag gtt cct gaa act cgt gga cgt acc ttg 626
Leu Ile Phe Ile Tyr Thr Gln Val Pro Glu Thr Arg Gly Arg Thr Leu
        195                 200                 205

gag gag att gat gag gat gtt act tcc ggt gtc att ttc aac aag gac 674
Glu Glu Ile Asp Glu Asp Val Thr Ser Gly Val Ile Phe Asn Lys Asp
    210                 215                 220

atc cga aaa gga aag gtg cac taaaaaccca gacactgcat agataacacg 725
Ile Arg Lys Gly Lys Val His
225                 230

<210> 438
<211> 231
<212> PRT
<213> Corynebacterium glutamicum

<400> 438
Met Gly Leu Arg Glu Ile Leu Ser Ser Lys Trp Leu Val Arg Ile Leu
1               5                   10                  15

Leu Val Gly Ile Gly Leu Gly Val Ala Gln Gln Leu Thr Gly Ile Asn
            20                  25                  30

Ser Ile Met Tyr Tyr Gly Gln Val Val Leu Ile Glu Ala Gly Phe Ser
        35                  40                  45

Glu Asn Ala Ala Leu Ile Ala Asn Val Ala Pro Gly Val Ile Ala Val
    50                  55                  60

Val Gly Ala Phe Ile Ala Leu Trp Met Met Asp Gly Ile Asn Arg Arg
65                  70                  75                  80

Thr Thr Leu Ile Thr Gly Tyr Ser Leu Thr Thr Ile Ser His Val Leu
                85                  90                  95

Ile Gly Ile Ala Ser Val Ala Phe Pro Val Gly Asp Pro Leu Arg Pro
            100                 105                 110

Tyr Val Ile Leu Thr Leu Val Val Val Phe Val Gly Ser Met Gln Thr
        115                 120                 125

Phe Leu Asn Val Ala Thr Trp Val Met Leu Ser Glu Leu Phe Pro Leu
    130                 135                 140

Ala Met Arg Gly Phe Ala Ile Gly Ile Ser Val Phe Phe Leu Trp Ile
145                 150                 155                 160

Ala Asn Ala Phe Leu Gly Leu Phe Phe Pro Thr Ile Met Glu Ala Val
                165                 170                 175

Gly Leu Thr Gly Thr Phe Phe Met Phe Ala Gly Ile Gly Val Val Ala
            180                 185                 190

Leu Ile Phe Ile Tyr Thr Gln Val Pro Glu Thr Arg Gly Arg Thr Leu
        195                 200                 205

Glu Glu Ile Asp Glu Asp Val Thr Ser Gly Val Ile Phe Asn Lys Asp
    210                 215                 220

Ile Arg Lys Gly Lys Val His
225                 230


<210> 439
<211> 408
<212> DNA
<213> Corynebacterium glutamicum

<220>
<221> CDS
<222> (101)..(385)
<223> FRXA02762

<400> 439
ttcccagtcg gcgatcctct tcgcccctac gttatcttga ctctggttgt ggtcttcgtg 60

ggatccatgc agaccttcct caacggtagc tacctgggtt atg ctc tct gag ctc 115
Met Leu Ser Glu Leu
1               5

ttc ccg ctg gca atg cgc ggt ttc gca atc ggt atc tca gtg ttc ttc 163
Phe Pro Leu Ala Met Arg Gly Phe Ala Ile Gly Ile Ser Val Phe Phe
                10                  15                  20

ctc tgg atc gca aac gcg ttc ctc gga ttg ttc ttc cca acc atc atg 211
Leu Trp Ile Ala Asn Ala Phe Leu Gly Leu Phe Phe Pro Thr Ile Met
            25                  30                  35

gaa gca gta gga cta acc gga acc ttc ttc atg ttc gcc gga atc ggt 259
Glu Ala Val Gly Leu Thr Gly Thr Phe Phe Met Phe Ala Gly Ile Gly
        40                  45                  50

gtg gtt gcc ttg atc ttc atc tac acc cag gtt cct gaa act cgt gga 307
Val Val Ala Leu Ile Phe Ile Tyr Thr Gln Val Pro Glu Thr Arg Gly
    55                  60                  65

cgt acc ttg gag gag att gat gag gat gtt act tcc ggt gtc att ttc 355
Arg Thr Leu Glu Glu Ile Asp Glu Asp Val Thr Ser Gly Val Ile Phe
70                  75                  80                  85

aac aag gac atc cga aaa gga aag gtg cac taaaaaccca gacactgcat 405
Asn Lys Asp Ile Arg Lys Gly Lys Val His
                90                  95

aga 408

<210> 440
<211> 95
<212> PRT
<213> Corynebacterium glutamicum

<400> 440
Met Leu Ser Glu Leu Phe Pro Leu Ala Met Arg Gly Phe Ala Ile Gly
1               5                   10                  15

Ile Ser Val Phe Phe Leu Trp Ile Ala Asn Ala Phe Leu Gly Leu Phe
            20                  25                  30

Phe Pro Thr Ile Met Glu Ala Val Gly Leu Thr Gly Thr Phe Phe Met
        35                  40                  45

Phe Ala Gly Ile Gly Val Val Ala Leu Ile Phe Ile Tyr Thr Gln Val
    50                  55                  60

Pro Glu Thr Arg Gly Arg Thr Leu Glu Glu Ile Asp Glu Asp Val Thr
65                  70                  75                  80

Ser Gly Val Ile Phe Asn Lys Asp Ile Arg Lys Gly Lys Val His
                85                  90                  95

<210> 441
<211> 789
<212> DNA
<213> Corynebacterium glutamicum

<220>
<221> CDS
<222> (101)..(766)
<223> RXN00092

<400> 441
cgaaaccaga aacacccagc ggctcctcgt cgaaaagcga atctttgaac tagaagccca 60

ggcacgttgg ctcgaccgaa ttgaagcatt ggagcagtaa atg aca aac acg cct 115
Met Thr Asn Thr Pro
1               5

ttc ccc ctt gaa ctt caa aac atc tcc tgc gcc ttc gga gaa ggc cca 163
Phe Pro Leu Glu Leu Gln Asn Ile Ser Cys Ala Phe Gly Glu Gly Pro
                10                  15                  20

cgc cac gtc tcc gcg ctc aac aac gtc tcg ctg gca gtc aat ccc ggc 211
Arg His Val Ser Ala Leu Asn Asn Val Ser Leu Ala Val Asn Pro Gly
            25                  30                  35

gaa ctc gtt gcc atc atg ggc ccg tcc ggc tca gga aaa tcc acc ttg 259
Glu Leu Val Ala Ile Met Gly Pro Ser Gly Ser Gly Lys Ser Thr Leu
        40                  45                  50

ctc aac gtc gcc ggc ctc ctg cag cgc gca acc tct ggc cat gtg ctt 307
Leu Asn Val Ala Gly Leu Leu Gln Arg Ala Thr Ser Gly His Val Leu
    55                  60                  65

atc gac ggt gcc agc gcc tca gac ctc aac gcc aaa cgc gca gct gaa 355
Ile Asp Gly Ala Ser Ala Ser Asp Leu Asn Ala Lys Arg Ala Ala Glu
70                  75                  80                  85

acc agg cgt cgc cac atc gga gtt att ttc caa aac tac aac ctg gtc 403
Thr Arg Arg Arg His Ile Gly Val Ile Phe Gln Asn Tyr Asn Leu Val
                90                  95                  100

ccc acc ctc acc gtc gga gaa aac gtc ggt ctg ccc cta gaa ctc gac 451
Pro Thr Leu Thr Val Gly Glu Asn Val Gly Leu Pro Leu Glu Leu Asp
            105                 110                 115

ggc aaa acc gac cgc cag gca gta gca atc gca ctc gcg gaa gtc ggc 499
Gly Lys Thr Asp Arg Gln Ala Val Ala Ile Ala Leu Ala Glu Val Gly
        120                 125                 130

ctc gaa ggc ttc gac gac cgc ttc ccc gaa gag atc tcc ggc ggc caa 547
Leu Glu Gly Phe Asp Asp Arg Phe Pro Glu Glu Ile Ser Gly Gly Gln
    135                 140                 145

gcc cag cgc gtc gcc atc gcg aga gcc ctc atc ggc ccc cga aaa atc 595
Ala Gln Arg Val Ala Ile Ala Arg Ala Leu Ile Gly Pro Arg Lys Ile
150                 155                 160                 165

ttg ctt gcc gac gaa ccc acc ggc gcc ctc gac acc tcc acc ggc gac 643
Leu Leu Ala Asp Glu Pro Thr Gly Ala Leu Asp Thr Ser Thr Gly Asp
                170                 175                 180

gca gtc ctc cgc gtc ctc cgc caa aga atc gat tcc ggt gcc gca ggc 691
Ala Val Leu Arg Val Leu Arg Gln Arg Ile Asp Ser Gly Ala Ala Gly
            185                 190                 195

ctc ctt gtc acc cac gaa ccc cgc ttc gcc gcg tgg gca gac cga aca 739

Leu Leu Val Thr His Glu Pro Arg Phe Ala Ala Trp Ala Asp Arg Thr
        200                 205                 210

atc atg ctt agg gat ggt gaa atc cag tgaccacact tctagcagca        786
Ile Met Leu Arg Asp Gly Glu Ile Gln
    215                 220

acc                                                              789


<210> 442
<211> 222
<212> PRT
<213> Corynebacterium glutamicum

<400> 442
Met Thr Asn Thr Pro Phe Pro Leu Glu Leu Gln Asn Ile Ser Cys Ala
 1               5                  10                  15

Phe Gly Glu Gly Pro Arg His Val Ser Ala Leu Asn Asn Val Ser Leu
            20                  25                  30

Ala Val Asn Pro Gly Glu Leu Val Ala Ile Met Gly Pro Ser Gly Ser
        35                  40                  45

Gly Lys Ser Thr Leu Leu Asn Val Ala Gly Leu Leu Gln Arg Ala Thr
    50                  55                  60

Ser Gly His Val Leu Ile Asp Gly Ala Ser Ala Ser Asp Leu Asn Ala
65                  70                  75                  80

Lys Arg Ala Ala Glu Thr Arg Arg Arg His Ile Gly Val Ile Phe Gln
                85                  90                  95

Asn Tyr Asn Leu Val Pro Thr Leu Thr Val Gly Glu Asn Val Gly Leu
            100                 105                 110

Pro Leu Glu Leu Asp Gly Lys Thr Asp Arg Gln Ala Val Ala Ile Ala
        115                 120                 125

Leu Ala Glu Val Gly Leu Glu Gly Phe Asp Asp Arg Phe Pro Glu Glu
    130                 135                 140

Ile Ser Gly Gly Gln Ala Gln Arg Val Ala Ile Ala Arg Ala Leu Ile
145                 150                 155                 160

Gly Pro Arg Lys Ile Leu Leu Ala Asp Glu Pro Thr Gly Ala Leu Asp
                165                 170                 175

Thr Ser Thr Gly Asp Ala Val Leu Arg Val Leu Arg Gln Arg Ile Asp
            180                 185                 190

Ser Gly Ala Ala Gly Leu Leu Val Thr His Glu Pro Arg Phe Ala Ala
        195                 200                 205

Trp Ala Asp Arg Thr Ile Met Leu Arg Asp Gly Glu Ile Gln
    210                 215                 220


<210> 443
<211> 227

<212> DNA
<213> Corynebacterium glutamicum

<220>
<221> CDS
<222> (1)..(204)
<223> FRXA00092

<400> 443
atc gcg aga gcc ctc atc ggc ccc cga aaa atc ttg ctt gcc gac gaa     48
Ile Ala Arg Ala Leu Ile Gly Pro Arg Lys Ile Leu Leu Ala Asp Glu
1               5                   10                  15

ccc acc ggc gcc ctc gac acc tcc acc ggc gac gca gtc ctc cgc gtc     96
Pro Thr Gly Ala Leu Asp Thr Ser Thr Gly Asp Ala Val Leu Arg Val
            20                  25                  30

ctc cgc caa aga atc gat tcc ggt gcc gca ggc ctc ctt gtc acc cac     144
Leu Arg Gln Arg Ile Asp Ser Gly Ala Ala Gly Leu Leu Val Thr His
        35                  40                  45

gaa ccc cgc ttc gcc gcg tgg gca gac cga aca atc atg ctt agg gat     192
Glu Pro Arg Phe Ala Ala Trp Ala Asp Arg Thr Ile Met Leu Arg Asp
    50                  55                  60

ggt gaa atc cag tgaccacact tctagcagca acc                           227
Gly Glu Ile Gln
65


<210> 444
<211> 68
<212> PRT
<213> Corynebacterium glutamicum

<400> 444
Ile Ala Arg Ala Leu Ile Gly Pro Arg Lys Ile Leu Leu Ala Asp Glu
1               5                   10                  15

Pro Thr Gly Ala Leu Asp Thr Ser Thr Gly Asp Ala Val Leu Arg Val
            20                  25                  30

Leu Arg Gln Arg Ile Asp Ser Gly Ala Ala Gly Leu Leu Val Thr His
        35                  40                  45

Glu Pro Arg Phe Ala Ala Trp Ala Asp Arg Thr Ile Met Leu Arg Asp
    50                  55                  60

Gly Glu Ile Gln
65


<210> 445
<211> 975
<212> DNA
<213> Corynebacterium glutamicum

<220>
<221> CDS
<222> (101)..(952)
<223> RXN03060

<400> 445
tatgtccttt ggctccggta ccctggcagg agtacctggg ctgtttttct aaaatggcct 60

gacgttttca agattgaatt taaggaaagc atcgtagttc atg agt aac cct gcc 115
                                           Met Ser Asn Pro Ala
                                           1               5

gcg agc aca cct gcc aac aat tcg gac gat gtt gcg aag gag aat tgg 163
Ala Ser Thr Pro Ala Asn Asn Ser Asp Asp Val Ala Lys Glu Asn Trp
            10                  15                  20

gac tct tct ttt acg ccg aag act gac att gac tct tcc cag cct gtc 211
Asp Ser Ser Phe Thr Pro Lys Thr Asp Ile Asp Ser Ser Gln Pro Val
        25                  30                  35

aat aac tcg act ggt gaa gcc gct gcg cgc gca gtg aac ctg tac aag 259
Asn Asn Ser Thr Gly Glu Ala Ala Ala Arg Ala Val Asn Leu Tyr Lys
    40                  45                  50

gcg tat ggc cag ggt gat acc act gtc acc gcg ttg gat cac gtc aac 307
Ala Tyr Gly Gln Gly Asp Thr Thr Val Thr Ala Leu Asp His Val Asn
    55                  60                  65

gtg gag ttt gag aag aac aag ttc act gcc atc atg ggt cct tct ggc 355
Val Glu Phe Glu Lys Asn Lys Phe Thr Ala Ile Met Gly Pro Ser Gly
70                  75                  80                  85

tcg ggt aag tcc acg ttg atg cac tgc atg gct ggt ctg gat gct gcg 403
Ser Gly Lys Ser Thr Leu Met His Cys Met Ala Gly Leu Asp Ala Ala
            90                  95                  100

act ggt ggt tcg gca ttc att ggt gat acg gat ctg tcg cgg ttg aag 451
Thr Gly Gly Ser Ala Phe Ile Gly Asp Thr Asp Leu Ser Arg Leu Lys
        105                 110                 115

gac aaa gag atg acc tct ttg cgt cgt gat cgt ttg gga ttc att ttc 499
Asp Lys Glu Met Thr Ser Leu Arg Arg Asp Arg Leu Gly Phe Ile Phe
        120                 125                 130

cag tcg ttc aac ttg gtt cct act ctg acg gcg tcg gag aac att acg 547
Gln Ser Phe Asn Leu Val Pro Thr Leu Thr Ala Ser Glu Asn Ile Thr
    135                 140                 145

ctg cct acc gat atc gcg ggc cgc aag att gat cag tcg tgg ttc gat 595
Leu Pro Thr Asp Ile Ala Gly Arg Lys Ile Asp Gln Ser Trp Phe Asp
150                 155                 160                 165

gag att acc tct cgt ctg ggt ctg act gag cgc ctt aag cac cgt cct 643
Glu Ile Thr Ser Arg Leu Gly Leu Thr Glu Arg Leu Lys His Arg Pro
            170                 175                 180

gca gag ctc tct ggt ggt cag cag cag cgt gtg gcg tgt gct cgt gcg 691
Ala Glu Leu Ser Gly Gly Gln Gln Gln Arg Val Ala Cys Ala Arg Ala
        185                 190                 195

ttg gtg tct cgt ccg gag atc att ttc ggc gac gag cca acc ggt aac 739
Leu Val Ser Arg Pro Glu Ile Ile Phe Gly Asp Glu Pro Thr Gly Asn
        200                 205                 210

ttg gat tcg aac tct tct agg gaa gtg ctg gat atc ctg cgc acc gca 787

Leu Asp Ser Asn Ser Ser Arg Glu Val Leu Asp Ile Leu Arg Thr Ala
    215                 220                 225

gtt gat cag gat gat cag acc gtt gtg atc gtt acc cac gat gcc aag     835
Val Asp Gln Asp Asp Gln Thr Val Val Ile Val Thr His Asp Ala Lys
230                 235                 240                 245

gcg gcg tcc tat gca gat cgt gtc att ttc ttg gcg gac ggt cgt atc     883
Ala Ala Ser Tyr Ala Asp Arg Val Ile Phe Leu Ala Asp Gly Arg Ile
                250                 255                 260

gtg aac cag ttg ttt gat ccc acc atc gag gaa atc ttg gcc acg atg     931
Val Asn Gln Leu Phe Asp Pro Thr Ile Glu Glu Ile Leu Ala Thr Met
            265                 270                 275

aac gga att gag gat att gcc taatgaattc cggttccaca atg               975
Asn Gly Ile Glu Asp Ile Ala
        280

<210> 446
<211> 284
<212> PRT
<213> Corynebacterium glutamicum

<400> 446
Met Ser Asn Pro Ala Ala Ser Thr Pro Ala Asn Asn Ser Asp Asp Val
  1               5                  10                  15

Ala Lys Glu Asn Trp Asp Ser Ser Phe Thr Pro Lys Thr Asp Ile Asp
             20                  25                  30

Ser Ser Gln Pro Val Asn Asn Ser Thr Gly Glu Ala Ala Ala Arg Ala
         35                  40                  45

Val Asn Leu Tyr Lys Ala Tyr Gly Gln Gly Asp Thr Thr Val Thr Ala
     50                  55                  60

Leu Asp His Val Asn Val Glu Phe Glu Lys Asn Lys Phe Thr Ala Ile
 65                  70                  75                  80

Met Gly Pro Ser Gly Ser Gly Lys Ser Thr Leu Met His Cys Met Ala
                 85                  90                  95

Gly Leu Asp Ala Ala Thr Gly Gly Ser Ala Phe Ile Gly Asp Thr Asp
            100                 105                 110

Leu Ser Arg Leu Lys Asp Lys Glu Met Thr Ser Leu Arg Arg Asp Arg
        115                 120                 125

Leu Gly Phe Ile Phe Gln Ser Phe Asn Leu Val Pro Thr Leu Thr Ala
    130                 135                 140

Ser Glu Asn Ile Thr Leu Pro Thr Asp Ile Ala Gly Arg Lys Ile Asp
145                 150                 155                 160

Gln Ser Trp Phe Asp Glu Ile Thr Ser Arg Leu Gly Leu Thr Glu Arg
                165                 170                 175

Leu Lys His Arg Pro Ala Glu Leu Ser Gly Gly Gln Gln Gln Arg Val
            180                 185                 190

Ala Cys Ala Arg Ala Leu Val Ser Arg Pro Glu Ile Ile Phe Gly Asp
        195                 200                 205

Glu Pro Thr Gly Asn Leu Asp Ser Asn Ser Ser Arg Glu Val Leu Asp
    210                 215                 220

Ile Leu Arg Thr Ala Val Asp Gln Asp Asp Gln Thr Val Val Ile Val
225                 230                 235                 240

Thr His Asp Ala Lys Ala Ala Ser Tyr Ala Asp Arg Val Ile Phe Leu
                245                 250                 255

Ala Asp Gly Arg Ile Val Asn Gln Leu Phe Asp Pro Thr Ile Glu Glu
            260                 265                 270

Ile Leu Ala Thr Met Asn Gly Ile Glu Asp Ile Ala
        275                 280


<210> 447
<211> 538
<212> DNA
<213> Corynebacterium glutamicum

<220>
<221> CDS
<222> (101)..(538)
<223> FRXA02618

<400> 447
cgaaaccaga aacacccagc ggctcctcgt cgaaaagcga atctttgaac tagaagccca 60

ggcacgttgg ctcgaccgaa ttgaagcatt ggagcagtaa atg aca aac acg cct   115
                                            Met Thr Asn Thr Pro
                                            1               5

ttc ccc ctt gaa ctt caa aac atc tcc tgc gcc ttc gga gaa ggc cca   163
Phe Pro Leu Glu Leu Gln Asn Ile Ser Cys Ala Phe Gly Glu Gly Pro
                10                  15                  20

cgc cac gtc tcc gcg ctc aac aac gtc tcg ctg gca gtc aat ccc ggc   211
Arg His Val Ser Ala Leu Asn Asn Val Ser Leu Ala Val Asn Pro Gly
            25                  30                  35

gaa ctc gtt gcc atc atg ggc ccg tcc ggc tca gga aaa tcc acc ttg   259
Glu Leu Val Ala Ile Met Gly Pro Ser Gly Ser Gly Lys Ser Thr Leu
        40                  45                  50

ctc aac gtc gcc ggc ctc ctg cag cgc gca acc tct ggc cat gtg ctt   307
Leu Asn Val Ala Gly Leu Leu Gln Arg Ala Thr Ser Gly His Val Leu
    55                  60                  65

atc gac ggt gcc agc gcc tca gac ctc aac gcc aaa cgc gca gct gaa   355
Ile Asp Gly Ala Ser Ala Ser Asp Leu Asn Ala Lys Arg Ala Ala Glu
70                  75                  80                  85

acc agg cgt cgc cac atc gga gtt att ttc caa aac tac aac ctg gtc   403
Thr Arg Arg Arg His Ile Gly Val Ile Phe Gln Asn Tyr Asn Leu Val
                90                  95                  100

ccc acc ctc acc gtc gga gaa aac atc ggt ctg ccc cta gaa ctc gac     451
Pro Thr Leu Thr Val Gly Glu Asn Ile Gly Leu Pro Leu Glu Leu Asp
            105                 110                 115

ggc aaa acc gac cgc cag gca gta gca atc gca ctc gcg gaa gtc ggc     499
Gly Lys Thr Asp Arg Gln Ala Val Ala Ile Ala Leu Ala Glu Val Gly
        120                 125                 130

ctc aag ggc tct acg acc gct ttt ccc gag aga tct ctg                 538
Leu Lys Gly Ser Thr Thr Ala Phe Pro Glu Arg Ser Leu
    135                 140                 145

<210> 448
<211> 146
<212> PRT
<213> Corynebacterium glutamicum

<400> 448
Met Thr Asn Thr Pro Phe Pro Leu Glu Leu Gln Asn Ile Ser Cys Ala
  1               5                  10                  15

Phe Gly Glu Gly Pro Arg His Val Ser Ala Leu Asn Asn Val Ser Leu
             20                  25                  30

Ala Val Asn Pro Gly Glu Leu Val Ala Ile Met Gly Pro Ser Gly Ser
         35                  40                  45

Gly Lys Ser Thr Leu Leu Asn Val Ala Gly Leu Leu Gln Arg Ala Thr
     50                  55                  60

Ser Gly His Val Leu Ile Asp Gly Ala Ser Ala Ser Asp Leu Asn Ala
 65                  70                  75                  80

Lys Arg Ala Ala Glu Thr Arg Arg Arg His Ile Gly Val Ile Phe Gln
                 85                  90                  95

Asn Tyr Asn Leu Val Pro Thr Leu Thr Val Gly Glu Asn Ile Gly Leu
            100                 105                 110

Pro Leu Glu Leu Asp Gly Lys Thr Asp Arg Gln Ala Val Ala Ile Ala
        115                 120                 125

Leu Ala Glu Val Gly Leu Lys Gly Ser Thr Thr Ala Phe Pro Glu Arg
    130                 135                 140

Ser Leu
145

<210> 449
<211> 975
<212> DNA
<213> Corynebacterium glutamicum

<220>
<221> CDS
<222> (101)..(952)
<223> FRXA02900

<400> 449

tatgtccttt ggctccggta ccctggcagg agtacctggg ctgtttttct aaaatggcct 60

gacgttttca agattgaatt taaggaaagc atcgtagttc atg agt aac cct gcc 115
Met Ser Asn Pro Ala
1               5

gcg agc aca cct gcc aac aat tcg gac gat gtt gcg aag gag aat tgg 163
Ala Ser Thr Pro Ala Asn Asn Ser Asp Asp Val Ala Lys Glu Asn Trp
                10                  15                  20

gac tct tct ttt acg ccg aag act gac att gac tct tcc cag cct gtc 211
Asp Ser Ser Phe Thr Pro Lys Thr Asp Ile Asp Ser Ser Gln Pro Val
            25                  30                  35

aat aac tcg act ggt gaa gcc gct gcg cgc gca gtg aac ctg tac aag 259
Asn Asn Ser Thr Gly Glu Ala Ala Ala Arg Ala Val Asn Leu Tyr Lys
        40                  45                  50

gcg tat ggc cag ggt gat acc act gtc acc gcg ttg gat cac gtc aac 307
Ala Tyr Gly Gln Gly Asp Thr Thr Val Thr Ala Leu Asp His Val Asn
    55                  60                  65

gtg gag ttt gag aag aac aag ttc act gcc atc atg ggt cct tct ggc 355
Val Glu Phe Glu Lys Asn Lys Phe Thr Ala Ile Met Gly Pro Ser Gly
70                  75                  80                  85

tcg ggt aag tcc acg ttg atg cac tgc atg gct ggt ctg gat gct gcg 403
Ser Gly Lys Ser Thr Leu Met His Cys Met Ala Gly Leu Asp Ala Ala
                90                  95                  100

act ggt ggt tcg gca ttc att ggt gat acg gat ctg tcg cgg ttg aag 451
Thr Gly Gly Ser Ala Phe Ile Gly Asp Thr Asp Leu Ser Arg Leu Lys
            105                 110                 115

gac aaa gag atg acc tct ttg cgt cgt gat cgt ttg gga ttc att ttc 499
Asp Lys Glu Met Thr Ser Leu Arg Arg Asp Arg Leu Gly Phe Ile Phe
        120                 125                 130

cag tcg ttc aac ttg gtt cct act ctg acg gcg tcg gag aac att acg 547
Gln Ser Phe Asn Leu Val Pro Thr Leu Thr Ala Ser Glu Asn Ile Thr
    135                 140                 145

ctg cct acc gat atc gcg ggc cgc aag att gat cag tcg tgg ttc gat 595
Leu Pro Thr Asp Ile Ala Gly Arg Lys Ile Asp Gln Ser Trp Phe Asp
150                 155                 160                 165

gag att acc tct cgt ctg ggt ctg act gag cgc ctt aag cac cgt cct 643
Glu Ile Thr Ser Arg Leu Gly Leu Thr Glu Arg Leu Lys His Arg Pro
                170                 175                 180

gca gag ctc tct ggt ggt cag cag cag cgt gtg gcg tgt gct cgt gcg 691
Ala Glu Leu Ser Gly Gly Gln Gln Gln Arg Val Ala Cys Ala Arg Ala
            185                 190                 195

ttg gtg tct cgt ccg gag atc att ttc ggc gac gag cca acc ggt aac 739
Leu Val Ser Arg Pro Glu Ile Ile Phe Gly Asp Glu Pro Thr Gly Asn
        200                 205                 210

ttg gat tcg aac tct tct agg gaa gtg ctg gat atc ctg cgc acc gca 787
Leu Asp Ser Asn Ser Ser Arg Glu Val Leu Asp Ile Leu Arg Thr Ala
    215                 220                 225

gtt gat cag gat gat cag acc gtt gtg atc gtt acc cac gat gcc aag     835
Val Asp Gln Asp Asp Gln Thr Val Val Ile Val Thr His Asp Ala Lys
230                 235                 240                 245

gcg gcg tcc tat gca gat cgt gtc att ttc ttg gcg gac ggt cgt atc     883
Ala Ala Ser Tyr Ala Asp Arg Val Ile Phe Leu Ala Asp Gly Arg Ile
                250                 255                 260

gtg aac cag ttg ttt gat ccc acc atc gag gaa atc ttg gcc acg atg     931
Val Asn Gln Leu Phe Asp Pro Thr Ile Glu Glu Ile Leu Ala Thr Met
            265                 270                 275

aac gga att gag gat att gcc taatgaattc cggttccaca atg               975
Asn Gly Ile Glu Asp Ile Ala
        280

<210> 450
<211> 284
<212> PRT
<213> Corynebacterium glutamicum

<400> 450
Met Ser Asn Pro Ala Ala Ser Thr Pro Ala Asn Asn Ser Asp Asp Val
  1               5                  10                  15

Ala Lys Glu Asn Trp Asp Ser Ser Phe Thr Pro Lys Thr Asp Ile Asp
             20                  25                  30

Ser Ser Gln Pro Val Asn Asn Ser Thr Gly Glu Ala Ala Ala Arg Ala
         35                  40                  45

Val Asn Leu Tyr Lys Ala Tyr Gly Gln Gly Asp Thr Thr Val Thr Ala
     50                  55                  60

Leu Asp His Val Asn Val Glu Phe Glu Lys Asn Lys Phe Thr Ala Ile
 65                  70                  75                  80

Met Gly Pro Ser Gly Ser Gly Lys Ser Thr Leu Met His Cys Met Ala
                 85                  90                  95

Gly Leu Asp Ala Ala Thr Gly Gly Ser Ala Phe Ile Gly Asp Thr Asp
            100                 105                 110

Leu Ser Arg Leu Lys Asp Lys Glu Met Thr Ser Leu Arg Arg Asp Arg
        115                 120                 125

Leu Gly Phe Ile Phe Gln Ser Phe Asn Leu Val Pro Thr Leu Thr Ala
    130                 135                 140

Ser Glu Asn Ile Thr Leu Pro Thr Asp Ile Ala Gly Arg Lys Ile Asp
145                 150                 155                 160

Gln Ser Trp Phe Asp Glu Ile Thr Ser Arg Leu Gly Leu Thr Glu Arg
                165                 170                 175

Leu Lys His Arg Pro Ala Glu Leu Ser Gly Gly Gln Gln Gln Arg Val
            180                 185                 190

Ala Cys Ala Arg Ala Leu Val Ser Arg Pro Glu Ile Ile Phe Gly Asp

        195                 200                 205

Glu Pro Thr Gly Asn Leu Asp Ser Asn Ser Ser Arg Glu Val Leu Asp
    210                 215                 220

Ile Leu Arg Thr Ala Val Asp Gln Asp Asp Gln Thr Val Val Ile Val
225                 230                 235                 240

Thr His Asp Ala Lys Ala Ala Ser Tyr Ala Asp Arg Val Ile Phe Leu
                245                 250                 255

Ala Asp Gly Arg Ile Val Asn Gln Leu Phe Asp Pro Thr Ile Glu Glu
            260                 265                 270

Ile Leu Ala Thr Met Asn Gly Ile Glu Asp Ile Ala
        275                 280

<210> 451
<211> 1452
<212> DNA
<213> Corynebacterium glutamicum

<220>
<221> CDS
<222> (1)..(1452)
<223> RXS03212

<400> 451
gcc tca ctt aac tgg tca gtc atc gtt cca gcc cta gtc att gtc cta 48
Ala Ser Leu Asn Trp Ser Val Ile Val Pro Ala Leu Val Ile Val Leu
1               5                   10                  15

gcg aca gtg gtg tgg ggt atc gga ttc aaa gat agc ttt acc aac ttt 96
Ala Thr Val Val Trp Gly Ile Gly Phe Lys Asp Ser Phe Thr Asn Phe
            20                  25                  30

gct agt tct gcg ttg tca gca gta gtt gac aat ctc ggc tgg gcc ttc 144
Ala Ser Ser Ala Leu Ser Ala Val Val Asp Asn Leu Gly Trp Ala Phe
        35                  40                  45

att ttg ttt ggc aca gtc ttt gta ttt ttt atc gtt gtt atc gcc gct 192
Ile Leu Phe Gly Thr Val Phe Val Phe Phe Ile Val Val Ile Ala Ala
    50                  55                  60

agt aaa ttc ggc acg att cgc tta ggc cgc att gat gaa gca cca gag 240
Ser Lys Phe Gly Thr Ile Arg Leu Gly Arg Ile Asp Glu Ala Pro Glu
65                  70                  75                  80

ttt cgc acg gtg tca tgg att tcc atg atg ttt gct gca ggt atg ggt 288
Phe Arg Thr Val Ser Trp Ile Ser Met Met Phe Ala Ala Gly Met Gly
                85                  90                  95

att ggt ttg atg ttc tac gga acc aca gaa cct tta acc ttc tac cgc 336
Ile Gly Leu Met Phe Tyr Gly Thr Thr Glu Pro Leu Thr Phe Tyr Arg
            100                 105                 110

aat ggt gta cct gga cat gat gaa cac aat gtt ggc gtt gct atg tcc 384
Asn Gly Val Pro Gly His Asp Glu His Asn Val Gly Val Ala Met Ser
        115                 120                 125

acg aca atg ttc cac tgg acc ttg cat cca tgg gct atc tac gca att    432
Thr Thr Met Phe His Trp Thr Leu His Pro Trp Ala Ile Tyr Ala Ile
    130                 135                 140

gtg ggc cta gcc att gcc tat tcg acc ttc cga gtg ggc cgt aaa cag    480
Val Gly Leu Ala Ile Ala Tyr Ser Thr Phe Arg Val Gly Arg Lys Gln
145                 150                 155                 160

ctt cta agc tct gca ttc gtg cca ctc att ggt gaa aaa ggt gca gaa    528
Leu Leu Ser Ser Ala Phe Val Pro Leu Ile Gly Glu Lys Gly Ala Glu
                165                 170                 175

gga tgg ttg ggc aag ctc atc gac atc ctg gcg att atc gcc acc gta    576
Gly Trp Leu Gly Lys Leu Ile Asp Ile Leu Ala Ile Ile Ala Thr Val
            180                 185                 190

ttc ggc acg gca tgt tcc ctt ggc ctt ggt gcc ctg cag att ggt gca    624
Phe Gly Thr Ala Cys Ser Leu Gly Leu Gly Ala Leu Gln Ile Gly Ala
        195                 200                 205

ggc ctg tcc gca gca aac atc att gaa gat cca agc gat tgg acc atc    672
Gly Leu Ser Ala Ala Asn Ile Ile Glu Asp Pro Ser Asp Trp Thr Ile
    210                 215                 220

gtt ggc att gtt tct gtt ttg acc ctg gca ttt atc ttc tcc gct att    720
Val Gly Ile Val Ser Val Leu Thr Leu Ala Phe Ile Phe Ser Ala Ile
225                 230                 235                 240

tct ggt gtg ggc aag gga atc cag tac ctc tcc aac gcc aac atg gtt    768
Ser Gly Val Gly Lys Gly Ile Gln Tyr Leu Ser Asn Ala Asn Met Val
                245                 250                 255

ctg gca gct ctg ctc gcg att ttc gtg ttc gtt gtc gga cca acc gtg    816
Leu Ala Ala Leu Leu Ala Ile Phe Val Phe Val Val Gly Pro Thr Val
            260                 265                 270

tcg att ttg aac ctg ctg cca ggt tct att ggc aac tac ctg tcc aac    864
Ser Ile Leu Asn Leu Leu Pro Gly Ser Ile Gly Asn Tyr Leu Ser Asn
        275                 280                 285

ttc ttt caa atg gca ggc cgc act gcc atg agt gcc gac ggc aca gca    912
Phe Phe Gln Met Ala Gly Arg Thr Ala Met Ser Ala Asp Gly Thr Ala
    290                 295                 300

ggt gag tgg cta ggt agc tgg acc atc ttc tac tgg gca tgg tgg atc    960
Gly Glu Trp Leu Gly Ser Trp Thr Ile Phe Tyr Trp Ala Trp Trp Ile
305                 310                 315                 320

tct tgg tca cca ttc gta gga atg ttc ttg gca cgt att tcc cgt ggc    1008
Ser Trp Ser Pro Phe Val Gly Met Phe Leu Ala Arg Ile Ser Arg Gly
                325                 330                 335

cgc tcc atc cgt gag ttc atc ctg ggc gtg ttg ctc gtc cca gca ggt    1056
Arg Ser Ile Arg Glu Phe Ile Leu Gly Val Leu Leu Val Pro Ala Gly
            340                 345                 350

gtg tcc acc gta tgg ttc tcc att ttt ggt ggc act gcg att gtc ttc    1104
Val Ser Thr Val Trp Phe Ser Ile Phe Gly Gly Thr Ala Ile Val Phe
        355                 360                 365

gaa caa aat ggg gaa tcc att tgg ggt gat ggt gca gca gaa gag cag    1152

Glu Gln Asn Gly Glu Ser Ile Trp Gly Asp Gly Ala Ala Glu Glu Gln
    370                 375                 380

ctt ttt gga ttg ctt cat gca ctt cca ggt ggg caa atc atg ggc atc     1200
Leu Phe Gly Leu Leu His Ala Leu Pro Gly Gly Gln Ile Met Gly Ile
385                 390                 395                 400

atc gcc atg att ttg ctg ggt act ttc ttc att acc tct gct gac tct     1248
Ile Ala Met Ile Leu Leu Gly Thr Phe Phe Ile Thr Ser Ala Asp Ser
                405                 410                 415

gct tcc acc gtc atg ggc acc atg agt cag cac ggc cag ctg gaa gcc     1296
Ala Ser Thr Val Met Gly Thr Met Ser Gln His Gly Gln Leu Glu Ala
            420                 425                 430

aac aag tgg gtg aca gct gcc tgg ggt gtt gct acc gca gct att gga     1344
Asn Lys Trp Val Thr Ala Ala Trp Gly Val Ala Thr Ala Ala Ile Gly
        435                 440                 445

cta acg cta ttg ctt tct ggt ggt gac aat gcc ttg agc aac ttg caa     1392
Leu Thr Leu Leu Leu Ser Gly Gly Asp Asn Ala Leu Ser Asn Leu Gln
    450                 455                 460

aac gtc acc atc gtg gct gca aca cca ttc ttg ttt gtg gtt att gga     1440
Asn Val Thr Ile Val Ala Ala Thr Pro Phe Leu Phe Val Val Ile Gly
465                 470                 475                 480

ttg atg ttt gcg                                                     1452
Leu Met Phe Ala

<210> 452
<211> 484
<212> PRT
<213> Corynebacterium glutamicum

<400> 452
Ala Ser Leu Asn Trp Ser Val Ile Val Pro Ala Leu Val Ile Val Leu
  1               5                  10                  15

Ala Thr Val Val Trp Gly Ile Gly Phe Lys Asp Ser Phe Thr Asn Phe
             20                  25                  30

Ala Ser Ser Ala Leu Ser Ala Val Val Asp Asn Leu Gly Trp Ala Phe
         35                  40                  45

Ile Leu Phe Gly Thr Val Phe Val Phe Phe Ile Val Val Ile Ala Ala
     50                  55                  60

Ser Lys Phe Gly Thr Ile Arg Leu Gly Arg Ile Asp Glu Ala Pro Glu
 65                  70                  75                  80

Phe Arg Thr Val Ser Trp Ile Ser Met Met Phe Ala Ala Gly Met Gly
                 85                  90                  95

Ile Gly Leu Met Phe Tyr Gly Thr Thr Glu Pro Leu Thr Phe Tyr Arg
            100                 105                 110

Asn Gly Val Pro Gly His Asp Glu His Asn Val Gly Val Ala Met Ser
        115                 120                 125

Thr Thr Met Phe His Trp Thr Leu His Pro Trp Ala Ile Tyr Ala Ile
    130                 135                 140

Val Gly Leu Ala Ile Ala Tyr Ser Thr Phe Arg Val Gly Arg Lys Gln
145                 150                 155                 160

Leu Leu Ser Ser Ala Phe Val Pro Leu Ile Gly Glu Lys Gly Ala Glu
                165                 170                 175

Gly Trp Leu Gly Lys Leu Ile Asp Ile Leu Ala Ile Ile Ala Thr Val
            180                 185                 190

Phe Gly Thr Ala Cys Ser Leu Gly Leu Gly Ala Leu Gln Ile Gly Ala
        195                 200                 205

Gly Leu Ser Ala Ala Asn Ile Ile Glu Asp Pro Ser Asp Trp Thr Ile
    210                 215                 220

Val Gly Ile Val Ser Val Leu Thr Leu Ala Phe Ile Phe Ser Ala Ile
225                 230                 235                 240

Ser Gly Val Gly Lys Gly Ile Gln Tyr Leu Ser Asn Ala Asn Met Val
                245                 250                 255

Leu Ala Ala Leu Leu Ala Ile Phe Val Phe Val Val Gly Pro Thr Val
            260                 265                 270

Ser Ile Leu Asn Leu Leu Pro Gly Ser Ile Gly Asn Tyr Leu Ser Asn
        275                 280                 285

Phe Phe Gln Met Ala Gly Arg Thr Ala Met Ser Ala Asp Gly Thr Ala
    290                 295                 300

Gly Glu Trp Leu Gly Ser Trp Thr Ile Phe Tyr Trp Ala Trp Trp Ile
305                 310                 315                 320

Ser Trp Ser Pro Phe Val Gly Met Phe Leu Ala Arg Ile Ser Arg Gly
                325                 330                 335

Arg Ser Ile Arg Glu Phe Ile Leu Gly Val Leu Leu Val Pro Ala Gly
            340                 345                 350

Val Ser Thr Val Trp Phe Ser Ile Phe Gly Gly Thr Ala Ile Val Phe
        355                 360                 365

Glu Gln Asn Gly Glu Ser Ile Trp Gly Asp Gly Ala Ala Glu Glu Gln
    370                 375                 380

Leu Phe Gly Leu Leu His Ala Leu Pro Gly Gly Gln Ile Met Gly Ile
385                 390                 395                 400

Ile Ala Met Ile Leu Leu Gly Thr Phe Phe Ile Thr Ser Ala Asp Ser
                405                 410                 415

Ala Ser Thr Val Met Gly Thr Met Ser Gln His Gly Gln Leu Glu Ala
            420                 425                 430

Asn Lys Trp Val Thr Ala Ala Trp Gly Val Ala Thr Ala Ala Ile Gly
        435                 440                 445

Leu Thr Leu Leu Leu Ser Gly Gly Asp Asn Ala Leu Ser Asn Leu Gln
    450                 455                 460

Asn Val Thr Ile Val Ala Ala Thr Pro Phe Leu Phe Val Val Ile Gly
465                 470                 475                 480

Leu Met Phe Ala

<210> 453
<211> 945
<212> DNA
<213> Corynebacterium glutamicum

<220>
<221> CDS
<222> (1)..(945)
<223> FRXA01591

<400> 453
gcc tca ctt aac tgg tca gtc atc gtt cca gcc cta gtc att gtc cta      48
Ala Ser Leu Asn Trp Ser Val Ile Val Pro Ala Leu Val Ile Val Leu
  1               5                  10                  15

gcg aca gtg gtg tgg ggt atc gga ttc aaa gat agc ttt acc acc ttt      96
Ala Thr Val Val Trp Gly Ile Gly Phe Lys Asp Ser Phe Thr Thr Phe
             20                  25                  30

gct agt tct gcg ttg tca gca gta gtt gac aat ctc ggc tgg gcc ttc     144
Ala Ser Ser Ala Leu Ser Ala Val Val Asp Asn Leu Gly Trp Ala Phe
         35                  40                  45

att ttg ttt ggc aca gtc ttt gta ttt ttt atc gtt gtt atc gcc gct     192
Ile Leu Phe Gly Thr Val Phe Val Phe Phe Ile Val Val Ile Ala Ala
     50                  55                  60

agt aaa ttc ggc acg att cgc tta ggc cgc att gat gaa gca cca gag     240
Ser Lys Phe Gly Thr Ile Arg Leu Gly Arg Ile Asp Glu Ala Pro Glu
 65                  70                  75                  80

ttt cgc acg gtg tca tgg att tcc atg atg ttt gct gca ggt atg ggt     288
Phe Arg Thr Val Ser Trp Ile Ser Met Met Phe Ala Ala Gly Met Gly
                 85                  90                  95

att ggt ttg atg ttc tac gga acc aca gaa cct tta acc ttc tac cgc     336
Ile Gly Leu Met Phe Tyr Gly Thr Thr Glu Pro Leu Thr Phe Tyr Arg
            100                 105                 110

aat ggt gta cct gga cat gat gaa cac aat gtt ggc gtt gct atg tcc     384
Asn Gly Val Pro Gly His Asp Glu His Asn Val Gly Val Ala Met Ser
        115                 120                 125

acg aca atg ttc cac tgg acc ttg cat cca tgg gct atc tac gca att     432
Thr Thr Met Phe His Trp Thr Leu His Pro Trp Ala Ile Tyr Ala Ile
    130                 135                 140

gtg ggc cta gcc att gcc tat tcg acc ttc cga gtg ggc cgt aaa cag     480
Val Gly Leu Ala Ile Ala Tyr Ser Thr Phe Arg Val Gly Arg Lys Gln
145                 150                 155                 160

ctt cta agc tct gca ttc gtg cca ctc att ggt gaa aaa ggt gca gaa     528
Leu Leu Ser Ser Ala Phe Val Pro Leu Ile Gly Glu Lys Gly Ala Glu
                165                 170                 175

gga tgg ttg ggc aag ctc atc gac atc ctg gcg att atc gcc acc gta     576
Gly Trp Leu Gly Lys Leu Ile Asp Ile Leu Ala Ile Ile Ala Thr Val
            180                 185                 190

ttc ggc acg gca tgt tcc ctt ggc ctt ggt gcc ctg cag att ggt gca     624
Phe Gly Thr Ala Cys Ser Leu Gly Leu Gly Ala Leu Gln Ile Gly Ala
        195                 200                 205

ggc ctg tcc gca gca aac atc att gaa gat cca agc gat tgg acc atc     672
Gly Leu Ser Ala Ala Asn Ile Ile Glu Asp Pro Ser Asp Trp Thr Ile
    210                 215                 220

gtt ggc att gtt tct gtt ttg acc ctg gca ttt atc ttc tcc gct att     720
Val Gly Ile Val Ser Val Leu Thr Leu Ala Phe Ile Phe Ser Ala Ile
225                 230                 235                 240

tct ggt gtg ggc aag gga atc cag tac ctc tcc aac gcc aac atg gtt     768
Ser Gly Val Gly Lys Gly Ile Gln Tyr Leu Ser Asn Ala Asn Met Val
                245                 250                 255

ctg gca gct ctg ctc gcg att ttc gtg ttc gtt gtc gga cca acc gtg     816
Leu Ala Ala Leu Leu Ala Ile Phe Val Phe Val Val Gly Pro Thr Val
            260                 265                 270

tcg att ttg aac ctg ctg cca ggt tct att gtg aac tac ctg tcc aac     864
Ser Ile Leu Asn Leu Leu Pro Gly Ser Ile Val Asn Tyr Leu Ser Asn
        275                 280                 285

ttc ttt caa atg gta ggc cgc act gcc atg agt gcc gac ggc aca cca     912
Phe Phe Gln Met Val Gly Arg Thr Ala Met Ser Ala Asp Gly Thr Pro
    290                 295                 300

ggt gag tgg ctt ggt ggc tgc acc atc ttc tac                         945
Gly Glu Trp Leu Gly Gly Cys Thr Ile Phe Tyr
305                 310                 315


<210> 454
<211> 315
<212> PRT
<213> Corynebacterium glutamicum

<400> 454
Ala Ser Leu Asn Trp Ser Val Ile Val Pro Ala Leu Val Ile Val Leu
1               5                   10                  15

Ala Thr Val Val Trp Gly Ile Gly Phe Lys Asp Ser Phe Thr Thr Phe
            20                  25                  30

Ala Ser Ser Ala Leu Ser Ala Val Val Asp Asn Leu Gly Trp Ala Phe
        35                  40                  45

Ile Leu Phe Gly Thr Val Phe Val Phe Phe Ile Val Val Ile Ala Ala
    50                  55                  60

Ser Lys Phe Gly Thr Ile Arg Leu Gly Arg Ile Asp Glu Ala Pro Glu
65                  70                  75                  80

Phe Arg Thr Val Ser Trp Ile Ser Met Met Phe Ala Ala Gly Met Gly
                85                  90                  95

Ile Gly Leu Met Phe Tyr Gly Thr Thr Glu Pro Leu Thr Phe Tyr Arg
            100                 105                 110

Asn Gly Val Pro Gly His Asp Glu His Asn Val Gly Val Ala Met Ser
        115                 120                 125

Thr Thr Met Phe His Trp Thr Leu His Pro Trp Ala Ile Tyr Ala Ile
    130                 135                 140

Val Gly Leu Ala Ile Ala Tyr Ser Thr Phe Arg Val Gly Arg Lys Gln
145                 150                 155                 160

Leu Leu Ser Ser Ala Phe Val Pro Leu Ile Gly Glu Lys Gly Ala Glu
                165                 170                 175

Gly Trp Leu Gly Lys Leu Ile Asp Ile Leu Ala Ile Ile Ala Thr Val
            180                 185                 190

Phe Gly Thr Ala Cys Ser Leu Gly Leu Gly Ala Leu Gln Ile Gly Ala
        195                 200                 205

Gly Leu Ser Ala Ala Asn Ile Ile Glu Asp Pro Ser Asp Trp Thr Ile
    210                 215                 220

Val Gly Ile Val Ser Val Leu Thr Leu Ala Phe Ile Phe Ser Ala Ile
225                 230                 235                 240

Ser Gly Val Gly Lys Gly Ile Gln Tyr Leu Ser Asn Ala Asn Met Val
                245                 250                 255

Leu Ala Ala Leu Leu Ala Ile Phe Val Phe Val Val Gly Pro Thr Val
            260                 265                 270

Ser Ile Leu Asn Leu Leu Pro Gly Ser Ile Val Asn Tyr Leu Ser Asn
        275                 280                 285

Phe Phe Gln Met Val Gly Arg Thr Ala Met Ser Ala Asp Gly Thr Pro
    290                 295                 300

Gly Glu Trp Leu Gly Gly Cys Thr Ile Phe Tyr
305                 310                 315

<210> 455
<211> 292
<212> DNA
<213> Corynebacterium glutamicum

<220>
<221> CDS
<222> (101)..(292)
<223> RXN00201

<400> 455
acgtcgcgga cttcaagtag tcggcgatga aaaagtccgt ttactaaacc ccgatctgtg 60

ttacgccatc gcgcggctcg ggcacaccga tacctgggca gtg gca gat tgc gga   115

Val Ala Asp Cys Gly
1               5

tta ccc atc cca gaa cac gta gag atc atc gat ttg gca ctc gtg ttt     163
Leu Pro Ile Pro Glu His Val Glu Ile Ile Asp Leu Ala Leu Val Phe
                10                  15                  20

ggg atc ccc acc ttt gaa caa gta ctg aat gct ctc aag ccg gaa gta     211
Gly Ile Pro Thr Phe Glu Gln Val Leu Asn Ala Leu Lys Pro Glu Val
            25                  30                  35

gtt gtg gaa ggc gcg gtg att gcc gaa ggg gca ccc caa cgt atc cgc     259
Val Val Glu Gly Ala Val Ile Ala Glu Gly Ala Pro Gln Arg Ile Arg
        40                  45                  50

gaa atg gtg gat acg gat gtg gaa gta tgt gcg                         292
Glu Met Val Asp Thr Asp Val Glu Val Cys Ala
    55                  60

<210> 456
<211> 64
<212> PRT
<213> Corynebacterium glutamicum

<400> 456
Val Ala Asp Cys Gly Leu Pro Ile Pro Glu His Val Glu Ile Ile Asp
1               5                   10                  15

Leu Ala Leu Val Phe Gly Ile Pro Thr Phe Glu Gln Val Leu Asn Ala
            20                  25                  30

Leu Lys Pro Glu Val Val Val Glu Gly Ala Val Ile Ala Glu Gly Ala
        35                  40                  45

Pro Gln Arg Ile Arg Glu Met Val Asp Thr Asp Val Glu Val Cys Ala
    50                  55                  60

<210> 457
<211> 286
<212> DNA
<213> Corynebacterium glutamicum

<220>
<221> CDS
<222> (101)..(286)
<223> FRXA00201

<400> 457
acgtcgcgga cttcaagtag tcggcgatga aaaagtccgt ttactaaacc ccgatctgtg 60

ttacgccatc gcgcggctcg ggcacaccga tacctgggca gtg gca gat tgc gga     115
                                           Val Ala Asp Cys Gly
                                           1               5

tta ccc atc cca gaa cac gta gag atc atc gat ttg gca ctc gtg ttt     163
Leu Pro Ile Pro Glu His Val Glu Ile Ile Asp Leu Ala Leu Val Phe

10                  15                  20

ggg atc ccc acc ttt gaa caa gta ctg aat gct ctc aag ccg gaa gta     211
Gly Ile Pro Thr Phe Glu Gln Val Leu Asn Ala Leu Lys Pro Glu Val
            25                  30                  35

gtt gtg gaa ggc gcg gtg att gcc gaa ggg gca ccc caa cgt atc cgc     259
Val Val Glu Gly Ala Val Ile Ala Glu Gly Ala Pro Gln Arg Ile Arg
        40                  45                  50

gaa atg gtg gat acg gat gtg gaa gtt                                 286
Glu Met Val Asp Thr Asp Val Glu Val
    55                  60


<210> 458
<211> 62
<212> PRT
<213> Corynebacterium glutamicum

<400> 458
Val Ala Asp Cys Gly Leu Pro Ile Pro Glu His Val Glu Ile Ile Asp
  1               5                  10                  15

Leu Ala Leu Val Phe Gly Ile Pro Thr Phe Glu Gln Val Leu Asn Ala
            20                  25                  30

Leu Lys Pro Glu Val Val Val Glu Gly Ala Val Ile Ala Glu Gly Ala
        35                  40                  45

Pro Gln Arg Ile Arg Glu Met Val Asp Thr Asp Val Glu Val
    50                  55                  60


<210> 459
<211> 849
<212> DNA
<213> Corynebacterium glutamicum

<220>
<221> CDS
<222> (101)..(826)
<223> RXA01221

<400> 459
tccagggtgc gatcctggtt ctcgtgatcg tgttgctgcc gggcggaatt gcttcaatta 60

aactttccgc gctcaaaaat aaggccagga aggccacctc atg agc ctt aaa atc     115
                                            Met Ser Leu Lys Ile
                                              1               5

acc aac ctc aaa gtc gct ttc ggg tcg ttc atc gcc gtg aat gag att     163
Thr Asn Leu Lys Val Ala Phe Gly Ser Phe Ile Ala Val Asn Glu Ile
                 10                  15                  20

agt ttt cag gtg ctg ccc ggt cac gtc cac ttc ctc atc ggt gcc aac     211
Ser Phe Gln Val Leu Pro Gly His Val His Phe Leu Ile Gly Ala Asn
             25                  30                  35

ggt gca ggt aaa acc acc tgc att gac gcg atc agc gga ttg gcg ccg     259
Gly Ala Gly Lys Thr Thr Cys Ile Asp Ala Ile Ser Gly Leu Ala Pro

40                    45                    50

ggg cag gga tca gtg cag ttg gat ggc act gag att ctg gga acc cct     307
Gly Gln Gly Ser Val Gln Leu Asp Gly Thr Glu Ile Leu Gly Thr Pro
    55                  60                  65

gtg cac cgc att gct cgg atg ggt gtg ggg cga acg ttt cag acc gcc     355
Val His Arg Ile Ala Arg Met Gly Val Gly Arg Thr Phe Gln Thr Ala
70                  75                  80                  85

agc gtg ttt gaa gaa ttg tct gtg ttg cag aat ctg gat att gcg tgc     403
Ser Val Phe Glu Glu Leu Ser Val Leu Gln Asn Leu Asp Ile Ala Cys
                90                  95                  100

ggg att cat cgt ccg ttg cgg gcg ctt ctc ggg gtg cgt cat cgg att     451
Gly Ile His Arg Pro Leu Arg Ala Leu Leu Gly Val Arg His Arg Ile
            105                 110                 115

gat ccc cga att gaa cac gcc ctg gag gtc acg ggt ctt gct gat ctg     499
Asp Pro Arg Ile Glu His Ala Leu Glu Val Thr Gly Leu Ala Asp Leu
        120                 125                 130

gtg aat gct cag gcg gga acc ttg tcg cat ggg cag aaa cag tgg ctg     547
Val Asn Ala Gln Ala Gly Thr Leu Ser His Gly Gln Lys Gln Trp Leu
    135                 140                 145

gaa att gca atg ttg ctg gtg cag gat gcg cag gtg ctc atg ctg gat     595
Glu Ile Ala Met Leu Leu Val Gln Asp Ala Gln Val Leu Met Leu Asp
150                 155                 160                 165

gag ccg gtg gcg ggc atg agt gag gag gag cgt gtc gca acg ggt gag     643
Glu Pro Val Ala Gly Met Ser Glu Glu Glu Arg Val Ala Thr Gly Glu
                170                 175                 180

ctt ttg cag agg gtt gcg cgg gga cgg gtg gtg ttg gtg gtg gag cac     691
Leu Leu Gln Arg Val Ala Arg Gly Arg Val Val Leu Val Val Glu His
            185                 190                 195

gat atg gag ttc atg cgt cgt ttt gcc act cgc gtc act gtg atg aat     739
Asp Met Glu Phe Met Arg Arg Phe Ala Thr Arg Val Thr Val Met Asn
        200                 205                 210

cgc ggc acg atc ttg tgt gag ggg tcg gtc gat gag att cag gcg aat     787
Arg Gly Thr Ile Leu Cys Glu Gly Ser Val Asp Glu Ile Gln Ala Asn
    215                 220                 225

ccg gat gtg cag tcc att tat tta ggt acg gca ggg aag tgagttagtc     836
Pro Asp Val Gln Ser Ile Tyr Leu Gly Thr Ala Gly Lys
230                 235                 240

atgttggaaa tca                                                      849


<210> 460
<211> 242
<212> PRT
<213> Corynebacterium glutamicum

<400> 460
Met Ser Leu Lys Ile Thr Asn Leu Lys Val Ala Phe Gly Ser Phe Ile
1               5                   10                  15

Ala Val Asn Glu Ile Ser Phe Gln Val Leu Pro Gly His Val His Phe
            20                  25                  30

Leu Ile Gly Ala Asn Gly Ala Gly Lys Thr Thr Cys Ile Asp Ala Ile
        35                  40                  45

Ser Gly Leu Ala Pro Gly Gln Gly Ser Val Gln Leu Asp Gly Thr Glu
    50                  55                  60

Ile Leu Gly Thr Pro Val His Arg Ile Ala Arg Met Gly Val Gly Arg
65                  70                  75                  80

Thr Phe Gln Thr Ala Ser Val Phe Glu Glu Leu Ser Val Leu Gln Asn
                85                  90                  95

Leu Asp Ile Ala Cys Gly Ile His Arg Pro Leu Arg Ala Leu Leu Gly
            100                 105                 110

Val Arg His Arg Ile Asp Pro Arg Ile Glu His Ala Leu Glu Val Thr
        115                 120                 125

Gly Leu Ala Asp Leu Val Asn Ala Gln Ala Gly Thr Leu Ser His Gly
    130                 135                 140

Gln Lys Gln Trp Leu Glu Ile Ala Met Leu Leu Val Gln Asp Ala Gln
145                 150                 155                 160

Val Leu Met Leu Asp Glu Pro Val Ala Gly Met Ser Glu Glu Glu Arg
                165                 170                 175

Val Ala Thr Gly Glu Leu Leu Gln Arg Val Ala Arg Gly Arg Val Val
            180                 185                 190

Leu Val Val Glu His Asp Met Glu Phe Met Arg Arg Phe Ala Thr Arg
        195                 200                 205

Val Thr Val Met Asn Arg Gly Thr Ile Leu Cys Glu Gly Ser Val Asp
    210                 215                 220

Glu Ile Gln Ala Asn Pro Asp Val Gln Ser Ile Tyr Leu Gly Thr Ala
225                 230                 235                 240

Gly Lys

<210> 461
<211> 822
<212> DNA
<213> Corynebacterium glutamicum

<220>
<221> CDS
<222> (101)..(799)
<223> RXA01222

<400> 461
aatcgcggca cgatcttgtg tgaggggtcg gtcgatgaga ttcaggcgaa tccggatgtg 60

cagtccattt atttaggtac ggcagggaag tgagttagtc atg ttg gaa atc act   115

Met Leu Glu Ile Thr
1               5

aat ttg tgt gca ggt tat ggc cgc acg cag gta ctt cat tct ctt tca     163
Asn Leu Cys Ala Gly Tyr Gly Arg Thr Gln Val Leu His Ser Leu Ser
                10                  15                  20

atc tcc acg agc agc aac ggc atc ctg tcg atc ctc ggc cac aat ggc     211
Ile Ser Thr Ser Ser Asn Gly Ile Leu Ser Ile Leu Gly His Asn Gly
            25                  30                  35

gct ggt aaa tcc acc ttg ctg cga acc gcg gtg ggg ttg att aag ccg     259
Ala Gly Lys Ser Thr Leu Leu Arg Thr Ala Val Gly Leu Ile Lys Pro
        40                  45                  50

act tcc gga gag gtc aaa ctt ttc ggc cag gat gtc acc tcg ttg tcc     307
Thr Ser Gly Glu Val Lys Leu Phe Gly Gln Asp Val Thr Ser Leu Ser
    55                  60                  65

acg cat gag cga gta aag cgc gga atg gct tat gtg ccg cag ggc cag     355
Thr His Glu Arg Val Lys Arg Gly Met Ala Tyr Val Pro Gln Gly Gln
70                  75                  80                  85

cag tct ttt acg cag ctt agt tgc atg gaa aat ttg cag gtg gta gcg     403
Gln Ser Phe Thr Gln Leu Ser Cys Met Glu Asn Leu Gln Val Val Ala
                90                  95                  100

gat ctg cag gga cgt gtg ggc aag gca cgc atc gcg gag gcg ctt gat     451
Asp Leu Gln Gly Arg Val Gly Lys Ala Arg Ile Ala Glu Ala Leu Asp
            105                 110                 115

cgc ttt ccg gcg ctg acc cag gtg ctg gac cgc caa gcc ggc ctg ttg     499
Arg Phe Pro Ala Leu Thr Gln Val Leu Asp Arg Gln Ala Gly Leu Leu
        120                 125                 130

tcg ggt ggt cag cgt cag cag ctt gcc atc gcc cgc gcg ctg atc acg     547
Ser Gly Gly Gln Arg Gln Gln Leu Ala Ile Ala Arg Ala Leu Ile Thr
    135                 140                 145

gcg cca aag ctt ttg ctt ctc gac gaa ccc acc gag ggt att cag cct     595
Ala Pro Lys Leu Leu Leu Leu Asp Glu Pro Thr Glu Gly Ile Gln Pro
150                 155                 160                 165

tcg gtg gtt gct gaa att cag cag acc atc atc gat ttg gct aag gac     643
Ser Val Val Ala Glu Ile Gln Gln Thr Ile Ile Asp Leu Ala Lys Asp
                170                 175                 180

ggc atg agc att gtc ctg gtg gag caa aac att ggt ttt gca ttg gat     691
Gly Met Ser Ile Val Leu Val Glu Gln Asn Ile Gly Phe Ala Leu Asp
            185                 190                 195

gct gca aca agc tac gcc att gtg gcg cgt ggt cag gtc gtg gaa tcg     739
Ala Ala Thr Ser Tyr Ala Ile Val Ala Arg Gly Gln Val Val Glu Ser
        200                 205                 210

gga caa ggc gct gaa acc acc gca gag aag cag act aaa gtg cgg gaa     787
Gly Gln Gly Ala Glu Thr Thr Ala Glu Lys Gln Thr Lys Val Arg Glu
    215                 220                 225

tct cta gca atc tagcggctgt ggatagcgtt ttg                           822
Ser Leu Ala Ile

230

<210> 462
<211> 233
<212> PRT
<213> Corynebacterium glutamicum

<400> 462
Met Leu Glu Ile Thr Asn Leu Cys Ala Gly Tyr Gly Arg Thr Gln Val
1               5                   10                  15

Leu His Ser Leu Ser Ile Ser Thr Ser Ser Asn Gly Ile Leu Ser Ile
            20                  25                  30

Leu Gly His Asn Gly Ala Gly Lys Ser Thr Leu Leu Arg Thr Ala Val
        35                  40                  45

Gly Leu Ile Lys Pro Thr Ser Gly Glu Val Lys Leu Phe Gly Gln Asp
    50                  55                  60

Val Thr Ser Leu Ser Thr His Glu Arg Val Lys Arg Gly Met Ala Tyr
65                  70                  75                  80

Val Pro Gln Gly Gln Gln Ser Phe Thr Gln Leu Ser Cys Met Glu Asn
                85                  90                  95

Leu Gln Val Val Ala Asp Leu Gln Gly Arg Val Gly Lys Ala Arg Ile
            100                 105                 110

Ala Glu Ala Leu Asp Arg Phe Pro Ala Leu Thr Gln Val Leu Asp Arg
        115                 120                 125

Gln Ala Gly Leu Leu Ser Gly Gly Gln Arg Gln Gln Leu Ala Ile Ala
    130                 135                 140

Arg Ala Leu Ile Thr Ala Pro Lys Leu Leu Leu Leu Asp Glu Pro Thr
145                 150                 155                 160

Glu Gly Ile Gln Pro Ser Val Val Ala Glu Ile Gln Gln Thr Ile Ile
                165                 170                 175

Asp Leu Ala Lys Asp Gly Met Ser Ile Val Leu Val Glu Gln Asn Ile
            180                 185                 190

Gly Phe Ala Leu Asp Ala Ala Thr Ser Tyr Ala Ile Val Ala Arg Gly
        195                 200                 205

Gln Val Val Glu Ser Gly Gln Gly Ala Glu Thr Thr Ala Glu Lys Gln
    210                 215                 220

Thr Lys Val Arg Glu Ser Leu Ala Ile
225                 230


<210> 463
<211> 1005
<212> DNA
<213> Corynebacterium glutamicum

<220>

<221> CDS
<222> (101)..(982)
<223> RXA01219

<400> 463
   caccatttgg gaaaccgatt ccccagttga tccggaccca tacttgtctt cctatgactg 60
   ggccaagacc accgctgcga cttcctaaga gataaaaatc atg gac att tta ctc 115
   Met Asp Ile Leu Leu
   1 5
   aat cag ctc gta gcc ggg ctt tca gtt gga tcg gtc ctt cta ttg gtc 163
   Asn Gln Leu Val Ala Gly Leu Ser Val Gly Ser Val Leu Leu Leu Val
   10 15 20
   gca gtg gga ttg tca ctg acc ttt gga cag atg ggc gtt att aat atg 211
   Ala Val Gly Leu Ser Leu Thr Phe Gly Gln Met Gly Val Ile Asn Met
   25 30 35
   gcg cac gga gag ttc atc atg gtc ggc gca tac acc gca tat gtg gtg 259
   Ala His Gly Glu Phe Ile Met Val Gly Ala Tyr Thr Ala Tyr Val Val
   40 45 50
   cag ctg gtc gtc ggt tct gcc ggt tta tcc cta ctg atc agc att ccg 307
   Gln Leu Val Val Gly Ser Ala Gly Leu Ser Leu Leu Ile Ser Ile Pro
   55 60 65
   ctg gcc ttt att atc ggt ggg ctt ttc gga gtt ctc ctc gaa caa ttc 355
   Leu Ala Phe Ile Ile Gly Gly Leu Phe Gly Val Leu Leu Glu Gln Phe
   70 75 80 85
   ctg ctg aag tat ctt tat cac agg cca cta gac acg ctg ctg gcc aca 403
   Leu Leu Lys Tyr Leu Tyr His Arg Pro Leu Asp Thr Leu Leu Ala Thr
   90 95 100
   ttc ggt gtc ggt ttg atc ctt cag cag ctg gcc cga aac att ttc gga 451
   Phe Gly Val Gly Leu Ile Leu Gln Gln Leu Ala Arg Asn Ile Phe Gly
   105 110 115
   gct ccc gca gtg gat gtc agg gca ccg gaa ttt ctc cgc gga aac gtc 499
   Ala Pro Ala Val Asp Val Arg Ala Pro Glu Phe Leu Arg Gly Asn Val
   120 125 130
   gaa gtt cta ggc gtc ttg gtg ccg acc gcg cga cta ttc atc ctg gcg 547
   Glu Val Leu Gly Val Leu Val Pro Thr Ala Arg Leu Phe Ile Leu Ala
   135 140 145
   ctg gcc atc gca tca gtg act gca cta gct gtg ttc cta aat cgc act 595
   Leu Ala Ile Ala Ser Val Thr Ala Leu Ala Val Phe Leu Asn Arg Thr
   150 155 160 165
   gcc tgg ggc cga cgc atc cgc gcc gtg gtt ctg aac cgc gac ctc gcg 643
   Ala Trp Gly Arg Arg Ile Arg Ala Val Val Leu Asn Arg Asp Leu Ala
   170 175 180
   gaa acc gca ggt att gat acc cga gct act gac cga atg acg ttc ttt 691
   Glu Thr Ala Gly Ile Asp Thr Arg Ala Thr Asp Arg Met Thr Phe Phe
   185 190 195
   gtg ggc tcc ggt ctt gcc gga atc gcc ggg gta gct atc aca ttg att 739
   Val Gly Ser Gly Leu Ala Gly Ile Ala Gly Val Ala Ile Thr Leu Ile
   200                 205                 210
   ggc gcg acc ggc ccc acc atc ggt cag aac tac atc gtg gat gcc ttc     787
   Gly Ala Thr Gly Pro Thr Ile Gly Gln Asn Tyr Ile Val Asp Ala Phe
       215                 220                 225
   ctt gtt gtt gcc gcc ggt ggc atc ggc cgg gtg aag ggc gct gtg atc     835
   Leu Val Val Ala Ala Gly Gly Ile Gly Arg Val Lys Gly Ala Val Ile
   230                 235                 240                 245
   atg gct ttc gtg ctg gga att act caa gca ttc gtg gaa tat acg aca     883
   Met Ala Phe Val Leu Gly Ile Thr Gln Ala Phe Val Glu Tyr Thr Thr
                   250                 255                 260
   ggt gcg agc atc gcg aag ttc atc gta ctc atc gct gtt gtt gcc ttc     931
   Gly Ala Ser Ile Ala Lys Phe Ile Val Leu Ile Ala Val Val Ala Phe
               265                 270                 275
   ctg cag ttt agg cct caa gga ctc ttc caa acc caa act agg agc ctc     979
   Leu Gln Phe Arg Pro Gln Gly Leu Phe Gln Thr Gln Thr Arg Ser Leu
           280                 285                 290
   gta tgagcactca actcaagctg aag                                       1005
   Val

<210> 464
<211> 294
<212> PRT
<213> Corynebacterium glutamicum

<400> 464
Met Asp Ile Leu Leu Asn Gln Leu Val Ala Gly Leu Ser Val Gly Ser
  1               5                  10                  15

Val Leu Leu Leu Val Ala Val Gly Leu Ser Leu Thr Phe Gly Gln Met
             20                  25                  30

Gly Val Ile Asn Met Ala His Gly Glu Phe Ile Met Val Gly Ala Tyr
         35                  40                  45

Thr Ala Tyr Val Val Gln Leu Val Val Gly Ser Ala Gly Leu Ser Leu
     50                  55                  60

Leu Ile Ser Ile Pro Leu Ala Phe Ile Ile Gly Gly Leu Phe Gly Val
 65                  70                  75                  80

Leu Leu Glu Gln Phe Leu Leu Lys Tyr Leu Tyr His Arg Pro Leu Asp
                 85                  90                  95

Thr Leu Leu Ala Thr Phe Gly Val Gly Leu Ile Leu Gln Gln Leu Ala
            100                 105                 110

Arg Asn Ile Phe Gly Ala Pro Ala Val Asp Val Arg Ala Pro Glu Phe
        115                 120                 125

Leu Arg Gly Asn Val Glu Val Leu Gly Val Leu Val Pro Thr Ala Arg
    130                 135                 140

Leu Phe Ile Leu Ala Leu Ala Ile Ala Ser Val Thr Ala Leu Ala Val

145                 150                 155                 160

Phe Leu Asn Arg Thr Ala Trp Gly Arg Arg Ile Arg Ala Val Val Leu
                165                 170                 175

Asn Arg Asp Leu Ala Glu Thr Ala Gly Ile Asp Thr Arg Ala Thr Asp
            180                 185                 190

Arg Met Thr Phe Phe Val Gly Ser Gly Leu Ala Gly Ile Ala Gly Val
        195                 200                 205

Ala Ile Thr Leu Ile Gly Ala Thr Gly Pro Thr Ile Gly Gln Asn Tyr
    210                 215                 220

Ile Val Asp Ala Phe Leu Val Val Ala Ala Gly Gly Ile Gly Arg Val
225                 230                 235                 240

Lys Gly Ala Val Ile Met Ala Phe Val Leu Gly Ile Thr Gln Ala Phe
                245                 250                 255

Val Glu Tyr Thr Thr Gly Ala Ser Ile Ala Lys Phe Ile Val Leu Ile
            260                 265                 270

Ala Val Val Ala Phe Leu Gln Phe Arg Pro Gln Gly Leu Phe Gln Thr
        275                 280                 285

Gln Thr Arg Ser Leu Val
    290

<210> 465
<211> 1200
<212> DNA
<213> Corynebacterium glutamicum

<220>
<221> CDS
<222> (101)..(1177)
<223> RXA01220

<400> 465
caggtgcgag catcgcgaag ttcatcgtac tcatcgctgt tgttgccttc ctgcagttta 60

ggcctcaagg actcttccaa acccaaacta ggagcctcgt atg agc act caa ctc    115
                                           Met Ser Thr Gln Leu
                                           1               5

aag ctg aag aag ccc gca aag aag aaa act acg ccg aaa ctt agc gtc    163
Lys Leu Lys Lys Pro Ala Lys Lys Lys Thr Thr Pro Lys Leu Ser Val
                10                  15                  20

gta aat gct ccc acg ctg cgc act gcg gcg ttg ggc ctg gcc gcg ctc    211
Val Asn Ala Pro Thr Leu Arg Thr Ala Ala Leu Gly Leu Ala Ala Leu
            25                  30                  35

gct gcg gta ttg ctg tgc gcc ccg ctc ttt tta tcc aca ttc cag ctg    259
Ala Ala Val Leu Leu Cys Ala Pro Leu Phe Leu Ser Thr Phe Gln Leu
        40                  45                  50

acg ttg atg tcg cgc ttg gtg tgt tat gcg atc gtc gca gtc ggc atc    307
Thr Leu Met Ser Arg Leu Val Cys Tyr Ala Ile Val Ala Val Gly Ile

    55                  60                  65

ggg ctg gcg tgg ggc aga ggc ggc atg ctc acg ctg ggg caa ggc gtg 355
Gly Leu Ala Trp Gly Arg Gly Gly Met Leu Thr Leu Gly Gln Gly Val
70                  75                  80                  85

ttc ttt gga atc ggc gcg tac atc atg gcc atg cac atg ctg tac agc 403
Phe Phe Gly Ile Gly Ala Tyr Ile Met Ala Met His Met Leu Tyr Ser
                90                  95                  100

gat tcg cag att ttt ggg acc aca gtt ccg caa tgg tgg tcc att ttt 451
Asp Ser Gln Ile Phe Gly Thr Thr Val Pro Gln Trp Trp Ser Ile Phe
            105                 110                 115

gcc aac ccg gca gtc gca ctc atc gca gtt gtg gcg ctt ccc ggc atc 499
Ala Asn Pro Ala Val Ala Leu Ile Ala Val Val Ala Leu Pro Gly Ile
        120                 125                 130

gtg gct ttt gtg ctc ggc ttc tcc att ttc aaa cga cgc atc aaa ggc 547
Val Ala Phe Val Leu Gly Phe Ser Ile Phe Lys Arg Arg Ile Lys Gly
    135                 140                 145

gcc tac ttt gcc atc gtg aac caa gcg ctc gcc gca gct gtt gtg gtg 595
Ala Tyr Phe Ala Ile Val Asn Gln Ala Leu Ala Ala Ala Val Val Val
150                 155                 160                 165

ttg ctg gtc gga caa caa gat tcc ctt gga ggt tcc aat ggt ctt tcc 643
Leu Leu Val Gly Gln Gln Asp Ser Leu Gly Gly Ser Asn Gly Leu Ser
                170                 175                 180

gga ttt cga tcg ttc atg ggt ttt gcc gtc tac gac ccc atc aac cgc 691
Gly Phe Arg Ser Phe Met Gly Phe Ala Val Tyr Asp Pro Ile Asn Arg
            185                 190                 195

atc atg ttt tac ttc acc gca gtg gga gtt ctc ttg gct ttg gtg gct 739
Ile Met Phe Tyr Phe Thr Ala Val Gly Val Leu Leu Ala Leu Val Ala
        200                 205                 210

atc tca tat tgg ctc atg cgc agc cgc tat gga gaa ctg ctc gtg gcc 787
Ile Ser Tyr Trp Leu Met Arg Ser Arg Tyr Gly Glu Leu Leu Val Ala
    215                 220                 225

acc aga gat gca gaa gaa cgc gtc cga ttc ctc gga tat gat ccc gca 835
Thr Arg Asp Ala Glu Glu Arg Val Arg Phe Leu Gly Tyr Asp Pro Ala
230                 235                 240                 245

ttg atc aaa acc gcc gca tat gtc att gct gcg atg att gcc gga atc 883
Leu Ile Lys Thr Ala Ala Tyr Val Ile Ala Ala Met Ile Ala Gly Ile
                250                 255                 260

gcc gga gcg ctg ttc gtg ccg atc gtg ggc atc att tca ccc gca gaa 931
Ala Gly Ala Leu Phe Val Pro Ile Val Gly Ile Ile Ser Pro Ala Glu
            265                 270                 275

atc ggc gtg gtg cca tca atc gtg ttc gtg atc gcc gtc gcc gct ggt 979
Ile Gly Val Val Pro Ser Ile Val Phe Val Ile Ala Val Ala Ala Gly
        280                 285                 290

ggc agg gca tcc cta ttc ggt ccc gta gtt ggc gcg ctg gtg ctg ggc 1027
Gly Arg Ala Ser Leu Phe Gly Pro Val Val Gly Ala Leu Val Leu Gly
    295                 300                 305

tgg gtg gaa tcc aca ctt gct caa act ttc ccc agc atg tgg tcc tat    1075
Trp Val Glu Ser Thr Leu Ala Gln Thr Phe Pro Ser Met Trp Ser Tyr
310                 315                 320                 325

ttc cag ggt gcg atc ctg gtt ctc gtg atc gtg ttg ctg ccg ggc gga    1123
Phe Gln Gly Ala Ile Leu Val Leu Val Ile Val Leu Leu Pro Gly Gly
                330                 335                 340

att gct tca att aaa ctt tcc gcg ctc aaa aat aag gcc agg aag gcc    1171
Ile Ala Ser Ile Lys Leu Ser Ala Leu Lys Asn Lys Ala Arg Lys Ala
            345                 350                 355

acc tca tgagccttaa aatcaccaac ctc                                  1200
Thr Ser

<210> 466
<211> 359
<212> PRT
<213> Corynebacterium glutamicum

<400> 466
Met Ser Thr Gln Leu Lys Leu Lys Lys Pro Ala Lys Lys Lys Thr Thr
  1               5                  10                  15

Pro Lys Leu Ser Val Val Asn Ala Pro Thr Leu Arg Thr Ala Ala Leu
             20                  25                  30

Gly Leu Ala Ala Leu Ala Ala Val Leu Leu Cys Ala Pro Leu Phe Leu
         35                  40                  45

Ser Thr Phe Gln Leu Thr Leu Met Ser Arg Leu Val Cys Tyr Ala Ile
     50                  55                  60

Val Ala Val Gly Ile Gly Leu Ala Trp Gly Arg Gly Gly Met Leu Thr
 65                  70                  75                  80

Leu Gly Gln Gly Val Phe Phe Gly Ile Gly Ala Tyr Ile Met Ala Met
                 85                  90                  95

His Met Leu Tyr Ser Asp Ser Gln Ile Phe Gly Thr Thr Val Pro Gln
            100                 105                 110

Trp Trp Ser Ile Phe Ala Asn Pro Ala Val Ala Leu Ile Ala Val Val
        115                 120                 125

Ala Leu Pro Gly Ile Val Ala Phe Val Leu Gly Phe Ser Ile Phe Lys
    130                 135                 140

Arg Arg Ile Lys Gly Ala Tyr Phe Ala Ile Val Asn Gln Ala Leu Ala
145                 150                 155                 160

Ala Ala Val Val Val Leu Leu Val Gly Gln Gln Asp Ser Leu Gly Gly
                165                 170                 175

Ser Asn Gly Leu Ser Gly Phe Arg Ser Phe Met Gly Phe Ala Val Tyr
            180                 185                 190

Asp Pro Ile Asn Arg Ile Met Phe Tyr Phe Thr Ala Val Gly Val Leu

195                 200                 205

Leu Ala Leu Val Ala Ile Ser Tyr Trp Leu Met Arg Ser Arg Tyr Gly
    210                 215                 220

Glu Leu Leu Val Ala Thr Arg Asp Ala Glu Glu Arg Val Arg Phe Leu
225                 230                 235                 240

Gly Tyr Asp Pro Ala Leu Ile Lys Thr Ala Ala Tyr Val Ile Ala Ala
                245                 250                 255

Met Ile Ala Gly Ile Ala Gly Ala Leu Phe Val Pro Ile Val Gly Ile
            260                 265                 270

Ile Ser Pro Ala Glu Ile Gly Val Val Pro Ser Ile Val Phe Val Ile
        275                 280                 285

Ala Val Ala Ala Gly Gly Arg Ala Ser Leu Phe Gly Pro Val Val Gly
    290                 295                 300

Ala Leu Val Leu Gly Trp Val Glu Ser Thr Leu Ala Gln Thr Phe Pro
305                 310                 315                 320

Ser Met Trp Ser Tyr Phe Gln Gly Ala Ile Leu Val Leu Val Ile Val
                325                 330                 335

Leu Leu Pro Gly Gly Ile Ala Ser Ile Lys Leu Ser Ala Leu Lys Asn
            340                 345                 350

Lys Ala Arg Lys Ala Thr Ser
        355

<210> 467
<211> 876
<212> DNA
<213> Corynebacterium glutamicum

<220>
<221> CDS
<222> (101)..(853)
<223> RXA00091

<400> 467
tcatgaacca cgtgttccgc gcacaaggcg tcgtgtccat cattattgag atggtcggcg 60

gtaccgtctt cctcatcgtc atcctcagaa agggcagact gtg att acg tta act   115
                                            Val Ile Thr Leu Thr
                                            1               5

aat gtc cgc aag gaa tac tcc agc gac gtt gcc atc ggc ccc gtc aac   163
Asn Val Arg Lys Glu Tyr Ser Ser Asp Val Ala Ile Gly Pro Val Asn
                10                  15                  20

ctt gag atc cca gcc ggc ggc atc acc gcg ttg gtc ggc cca aac ggt   211
Leu Glu Ile Pro Ala Gly Gly Ile Thr Ala Leu Val Gly Pro Asn Gly
            25                  30                  35

gca ggc aag tca aca ctg ctc acc atg atc ggt cga ctc ctc ggc atc   259
Ala Gly Lys Ser Thr Leu Leu Thr Met Ile Gly Arg Leu Leu Gly Ile
        40                  45                  50

gat gaa ggc aac atc acc gta gcc tcc tac gat gtc acc tca acc gca     307
Asp Glu Gly Asn Ile Thr Val Ala Ser Tyr Asp Val Thr Ser Thr Ala
     55                  60                  65

tcc aaa gat ctg gcc aag atc atc tcc atc ctg cgc cag gaa aac cac     355
Ser Lys Asp Leu Ala Lys Ile Ile Ser Ile Leu Arg Gln Glu Asn His
 70                  75                  80                  85

ttt gtt acc aag ctg acc gtg cgc cag ctc gta ggt ttc gga cgc ttc     403
Phe Val Thr Lys Leu Thr Val Arg Gln Leu Val Gly Phe Gly Arg Phe
                 90                  95                 100

cca tat agc aag ggc cgg ctg acg gaa gaa gac gag gga atc atc tcc     451
Pro Tyr Ser Lys Gly Arg Leu Thr Glu Glu Asp Glu Gly Ile Ile Ser
            105                 110                 115

cgc tac atc gac ttc ttc aat ctc acc gaa ctc gaa gac cgc tac ctc     499
Arg Tyr Ile Asp Phe Phe Asn Leu Thr Glu Leu Glu Asp Arg Tyr Leu
        120                 125                 130

gac cag ctt tcc ggc ggc cag cgc cag cgc gcc tat gtc gcc atg gtg     547
Asp Gln Leu Ser Gly Gly Gln Arg Gln Arg Ala Tyr Val Ala Met Val
    135                 140                 145

ctg tgc caa gag acg gac tac gtg ctt ctc gac gaa ccc ctc aac aat     595
Leu Cys Gln Glu Thr Asp Tyr Val Leu Leu Asp Glu Pro Leu Asn Asn
150                 155                 160                 165

ctt gat atc gca cac tcg gtg gaa atg atg aaa cac ctc gag aat gct     643
Leu Asp Ile Ala His Ser Val Glu Met Met Lys His Leu Glu Asn Ala
                170                 175                 180

gca gcc caa ttt ggc cgc acc atc atc gtg gtt ctt cac gac atc aac     691
Ala Ala Gln Phe Gly Arg Thr Ile Ile Val Val Leu His Asp Ile Asn
            185                 190                 195

ttc gcc gcg cgc tac gcc gat tac atc gtg gcc gta aag cac gga atg     739
Phe Ala Ala Arg Tyr Ala Asp Tyr Ile Val Ala Val Lys His Gly Met
        200                 205                 210

atc gaa aaa gag gga aca cct gaa cag atc atg aaa aac gag atc ctt     787
Ile Glu Lys Glu Gly Thr Pro Glu Gln Ile Met Lys Asn Glu Ile Leu
    215                 220                 225

tca gaa atc ttc aat aca gag atc gaa gtt att gaa gga cca cac ggc     835
Ser Glu Ile Phe Asn Thr Glu Ile Glu Val Ile Glu Gly Pro His Gly
230                 235                 240                 245

aag att gct tgc tac cac taaatgaaga gaaataagcc gac                   876
Lys Ile Ala Cys Tyr His
                250

<210> 468
<211> 251
<212> PRT
<213> Corynebacterium glutamicum

<400> 468
Val Ile Thr Leu Thr Asn Val Arg Lys Glu Tyr Ser Ser Asp Val Ala

1               5                   10                  15

Ile Gly Pro Val Asn Leu Glu Ile Pro Ala Gly Gly Ile Thr Ala Leu
            20                  25                  30

Val Gly Pro Asn Gly Ala Gly Lys Ser Thr Leu Leu Thr Met Ile Gly
        35                  40                  45

Arg Leu Leu Gly Ile Asp Glu Gly Asn Ile Thr Val Ala Ser Tyr Asp
    50                  55                  60

Val Thr Ser Thr Ala Ser Lys Asp Leu Ala Lys Ile Ile Ser Ile Leu
65                  70                  75                  80

Arg Gln Glu Asn His Phe Val Thr Lys Leu Thr Val Arg Gln Leu Val
                85                  90                  95

Gly Phe Gly Arg Phe Pro Tyr Ser Lys Gly Arg Leu Thr Glu Glu Asp
            100                 105                 110

Glu Gly Ile Ile Ser Arg Tyr Ile Asp Phe Phe Asn Leu Thr Glu Leu
        115                 120                 125

Glu Asp Arg Tyr Leu Asp Gln Leu Ser Gly Gly Gln Arg Gln Arg Ala
    130                 135                 140

Tyr Val Ala Met Val Leu Cys Gln Glu Thr Asp Tyr Val Leu Leu Asp
145                 150                 155                 160

Glu Pro Leu Asn Asn Leu Asp Ile Ala His Ser Val Glu Met Met Lys
                165                 170                 175

His Leu Glu Asn Ala Ala Ala Gln Phe Gly Arg Thr Ile Ile Val Val
            180                 185                 190

Leu His Asp Ile Asn Phe Ala Ala Arg Tyr Ala Asp Tyr Ile Val Ala
        195                 200                 205

Val Lys His Gly Met Ile Glu Lys Glu Gly Thr Pro Glu Gln Ile Met
    210                 215                 220

Lys Asn Glu Ile Leu Ser Glu Ile Phe Asn Thr Glu Ile Glu Val Ile
225                 230                 235                 240

Glu Gly Pro His Gly Lys Ile Ala Cys Tyr His
                245                 250

<210> 469
<211> 692
<212> DNA
<213> Corynebacterium glutamicum

<220>
<221> CDS
<222> (79)..(669)
<223> RXA00228

<400> 469
ccgcatggca tccgccgaaa gaattcccct ccggaggaat ggcacgcctc atgggagaac 60

gcatcggcaa aaactgacatg cgc gac ctc cgc tcc atg atc ggc gtc agc     111
                      Met Arg Asp Leu Arg Ser Met Ile Gly Val Ser
                      1               5                   10

tcc tcc gca tta ggc aac cga atc ccc tcc gaa gaa aaa gtc tct gac     159
Ser Ser Ala Leu Gly Asn Arg Ile Pro Ser Glu Glu Lys Val Ser Asp
            15                  20                  25

cta gtc atc tcc gca ggc tac gca atc ctc ggc cgc tgg cgc gaa gac     207
Leu Val Ile Ser Ala Gly Tyr Ala Ile Leu Gly Arg Trp Arg Glu Asp
        30                  35                  40

tac gac gaa atg gac ttc gga caa gcc acc gaa atc ctc gaa caa gtc     255
Tyr Asp Glu Met Asp Phe Gly Gln Ala Thr Glu Ile Leu Glu Gln Val
    45                  50                  55

gga gcc atg cac cta gcc gac cgc acc tgg gga acc ctc tcc gaa ggc     303
Gly Ala Met His Leu Ala Asp Arg Thr Trp Gly Thr Leu Ser Glu Gly
60                  65                  70                  75

gaa cgc aaa cga gtc ctg gtc gca cgc gca ctc atg acc aac ccg gaa     351
Glu Arg Lys Arg Val Leu Val Ala Arg Ala Leu Met Thr Asn Pro Glu
                80                  85                  90

ctc ctc atc ctt gac gaa cca acc gca gga atg gac ctc ggc gga cgc     399
Leu Leu Ile Leu Asp Glu Pro Thr Ala Gly Met Asp Leu Gly Gly Arg
            95                  100                 105

gaa gac ctc gtc ggc tac ctc gga gaa ctc gcc atg gac cca gac gca     447
Glu Asp Leu Val Gly Tyr Leu Gly Glu Leu Ala Met Asp Pro Asp Ala
        110                 115                 120

cct gcc atc gtc atg atc acc cac cac gtc gaa gaa atc ccc gcc gga     495
Pro Ala Ile Val Met Ile Thr His His Val Glu Glu Ile Pro Ala Gly
    125                 130                 135

ttc acc cac gca atg ctc ctc gac gaa ggt gaa atc gta gcc caa ggc     543
Phe Thr His Ala Met Leu Leu Asp Glu Gly Glu Ile Val Ala Gln Gly
140                 145                 150                 155

ctg atc aac acc gtc atg aca aac gag aac cta tcc aaa gca ttc cac     591
Leu Ile Asn Thr Val Met Thr Asn Glu Asn Leu Ser Lys Ala Phe His
                160                 165                 170

cag cca atc caa gta gac cgc atc ggg gaa cgc tac ttt gcc cgc cgt     639
Gln Pro Ile Gln Val Asp Arg Ile Gly Glu Arg Tyr Phe Ala Arg Arg
            175                 180                 185

gtg aga acc gcc agg agt cat agg gct cag taggtttttt ggagttgtgg     689
Val Arg Thr Ala Arg Ser His Arg Ala Gln
        190                 195

gcc                                                                692

<210> 470
<211> 197
<212> PRT
<213> Corynebacterium glutamicum

<400> 470

Met Arg Asp Leu Arg Ser Met Ile Gly Val Ser Ser Ser Ala Leu Gly
1               5                   10                  15

Asn Arg Ile Pro Ser Glu Glu Lys Val Ser Asp Leu Val Ile Ser Ala
            20                  25                  30

Gly Tyr Ala Ile Leu Gly Arg Trp Arg Glu Asp Tyr Asp Glu Met Asp
        35                  40                  45

Phe Gly Gln Ala Thr Glu Ile Leu Glu Gln Val Gly Ala Met His Leu
    50                  55                  60

Ala Asp Arg Thr Trp Gly Thr Leu Ser Glu Gly Glu Arg Lys Arg Val
65                  70                  75                  80

Leu Val Ala Arg Ala Leu Met Thr Asn Pro Glu Leu Leu Ile Leu Asp
                85                  90                  95

Glu Pro Thr Ala Gly Met Asp Leu Gly Gly Arg Glu Asp Leu Val Gly
            100                 105                 110

Tyr Leu Gly Glu Leu Ala Met Asp Pro Asp Ala Pro Ala Ile Val Met
        115                 120                 125

Ile Thr His His Val Glu Glu Ile Pro Ala Gly Phe Thr His Ala Met
    130                 135                 140

Leu Leu Asp Glu Gly Glu Ile Val Ala Gln Gly Leu Ile Asn Thr Val
145                 150                 155                 160

Met Thr Asn Glu Asn Leu Ser Lys Ala Phe His Gln Pro Ile Gln Val
                165                 170                 175

Asp Arg Ile Gly Glu Arg Tyr Phe Ala Arg Arg Val Arg Thr Ala Arg
            180                 185                 190

Ser His Arg Ala Gln
        195


<210> 471
<211> 813
<212> DNA
<213> Corynebacterium glutamicum

<220>
<221> CDS
<222> (101)..(790)
<223> RXA00346

<400> 471
atctatgaga cccctcaaaa caccgagaat ttcctcgatg cattcaccaa ggcagttgat 60

gatctcaccg ctgccactaa ccaggtttag aattatttaa atg ctg ttg act ttc    115
                                            Met Leu Leu Thr Phe
                                            1               5

aat gat gct gcg gtg gat ccc ctc tgg agg ggc ctg aat tta gag ctc    163
Asn Asp Ala Ala Val Asp Pro Leu Trp Arg Gly Leu Asn Leu Glu Leu
                10                  15                  20

cga cag ggg gaa ttt ctt gcg gtt tta ggc ccc aac ggc gtg gga aaa 211
Arg Gln Gly Glu Phe Leu Ala Val Leu Gly Pro Asn Gly Val Gly Lys
            25                  30                  35

tcc acg ctc atc ggt acg att ttg ggc acc cga aaa ctc acc cac ggt 259
Ser Thr Leu Ile Gly Thr Ile Leu Gly Thr Arg Lys Leu Thr His Gly
        40                  45                  50

tcg gtt aaa act gat gcc cgg gtg ggt tat att ccg caa caa cga att 307
Ser Val Lys Thr Asp Ala Arg Val Gly Tyr Ile Pro Gln Gln Arg Ile
    55                  60                  65

ttc gat gtc ccg ttg cgt gcc cgc gat atg gtt tcg ctg tcc gcg gcg 355
Phe Asp Val Pro Leu Arg Ala Arg Asp Met Val Ser Leu Ser Ala Ala
70                  75                  80                  85

cat ggc gtg gtt tcc aaa agg gga ccc gcg aag ggt gac gtc gat aag 403
His Gly Val Val Ser Lys Arg Gly Pro Ala Lys Gly Asp Val Asp Lys
                90                  95                  100

ctt ctt gcc cgc gtg ggc gct tcc gga atc gcc gat cga cgc gtc ggc 451
Leu Leu Ala Arg Val Gly Ala Ser Gly Ile Ala Asp Arg Arg Val Gly
            105                 110                 115

gag ctc tcc ggc ggg cag cag cag ctc gtc cgc caa gcc cag gcc ctt 499
Glu Leu Ser Gly Gly Gln Gln Gln Leu Val Arg Gln Ala Gln Ala Leu
        120                 125                 130

gcc acg cgc ccg caa tta ttg ctt gcc gac gaa ccc ctc ctc agc ctt 547
Ala Thr Arg Pro Gln Leu Leu Leu Ala Asp Glu Pro Leu Leu Ser Leu
    135                 140                 145

gac ccc ggc gtc gcg cag cgc acg gtg tcc cta ttt ggt gaa ttg aag 595
Asp Pro Gly Val Ala Gln Arg Thr Val Ser Leu Phe Gly Glu Leu Lys
150                 155                 160                 165

gcc gaa ggc gtc ggc gtt gtt gtg gtc acc cac gat gtc aat cca cta 643
Ala Glu Gly Val Gly Val Val Val Val Thr His Asp Val Asn Pro Leu
                170                 175                 180

atg ggc ctg gta gat cgc att ttg tac ctc gcc ccc aac ggc cac acc 691
Met Gly Leu Val Asp Arg Ile Leu Tyr Leu Ala Pro Asn Gly His Thr
            185                 190                 195

atc ggc acg gtt ggc gat gtc atg cag tcc gaa aaa ctc agc gaa ctc 739
Ile Gly Thr Val Gly Asp Val Met Gln Ser Glu Lys Leu Ser Glu Leu
        200                 205                 210

tac aac gca ccc gtc acg gtg gct cgc atc aac gac aga atc gtg gtg 787
Tyr Asn Ala Pro Val Thr Val Ala Arg Ile Asn Asp Arg Ile Val Val
    215                 220                 225

gtt taagtggatc tatccacctg gct 813
Val
230

<210> 472
<211> 230
<212> PRT
<213> Corynebacterium glutamicum

<400> 472
Met Leu Leu Thr Phe Asn Asp Ala Ala Val Asp Pro Leu Trp Arg Gly
1               5                   10                  15

Leu Asn Leu Glu Leu Arg Gln Gly Glu Phe Leu Ala Val Leu Gly Pro
            20                  25                  30

Asn Gly Val Gly Lys Ser Thr Leu Ile Gly Thr Ile Leu Gly Thr Arg
        35                  40                  45

Lys Leu Thr His Gly Ser Val Lys Thr Asp Ala Arg Val Gly Tyr Ile
    50                  55                  60

Pro Gln Gln Arg Ile Phe Asp Val Pro Leu Arg Ala Arg Asp Met Val
65                  70                  75                  80

Ser Leu Ser Ala Ala His Gly Val Val Ser Lys Arg Gly Pro Ala Lys
                85                  90                  95

Gly Asp Val Asp Lys Leu Leu Ala Arg Val Gly Ala Ser Gly Ile Ala
            100                 105                 110

Asp Arg Arg Val Gly Glu Leu Ser Gly Gly Gln Gln Gln Leu Val Arg
        115                 120                 125

Gln Ala Gln Ala Leu Ala Thr Arg Pro Gln Leu Leu Leu Ala Asp Glu
    130                 135                 140

Pro Leu Leu Ser Leu Asp Pro Gly Val Ala Gln Arg Thr Val Ser Leu
145                 150                 155                 160

Phe Gly Glu Leu Lys Ala Glu Gly Val Gly Val Val Val Val Thr His
                165                 170                 175

Asp Val Asn Pro Leu Met Gly Leu Val Asp Arg Ile Leu Tyr Leu Ala
            180                 185                 190

Pro Asn Gly His Thr Ile Gly Thr Val Gly Asp Val Met Gln Ser Glu
        195                 200                 205

Lys Leu Ser Glu Leu Tyr Asn Ala Pro Val Thr Val Ala Arg Ile Asn
    210                 215                 220

Asp Arg Ile Val Val Val
225                 230


<210> 473
<211> 433
<212> DNA
<213> Corynebacterium glutamicum

<220>
<221> CDS
<222> (101)..(433)
<223> RXA00524

<400> 473
tcctcggcac ccgctacccc gtcggagttg tcaccggcgc attcggcgcc ccattcctta 60

tctatttact cattcgttcc aaccgcgcgg gagtaaccct gtg acc acc aac cat 115
Val Thr Thr Asn His
1               5

caa cta tcc gcc gaa gaa att tcc ctg gcg tac ggc gag cgc acc atc 163
Gln Leu Ser Ala Glu Glu Ile Ser Leu Ala Tyr Gly Glu Arg Thr Ile
                10                  15                  20

atc gat tcg ctc agc gtc gac atc gtc ccc ggc aaa atc acc tcc atc 211
Ile Asp Ser Leu Ser Val Asp Ile Val Pro Gly Lys Ile Thr Ser Ile
            25                  30                  35

gtc ggc ccc aac gga tgc ggc aaa tca acg ctg ctg cgc gcc ttt gcg 259
Val Gly Pro Asn Gly Cys Gly Lys Ser Thr Leu Leu Arg Ala Phe Ala
        40                  45                  50

cgc ctc ctt aaa cct agc gcc ggg caa gcg ctt atc gac gcc cac ccc 307
Arg Leu Leu Lys Pro Ser Ala Gly Gln Ala Leu Ile Asp Ala His Pro
    55                  60                  65

ctt cct tca ctg cca ggc aaa gaa cta gct cgc atg ctc ggg ctg tta 355
Leu Pro Ser Leu Pro Gly Lys Glu Leu Ala Arg Met Leu Gly Leu Leu
70                  75                  80                  85

ccg caa tcc ccc acc gga cct gaa ggc atc gtc gtc gcc gac ctc gtg 403
Pro Gln Ser Pro Thr Gly Pro Glu Gly Ile Val Val Ala Asp Leu Val
                90                  95                  100

ggc cgc ggc cgc cac ccc cac caa gga ctc 433
Gly Arg Gly Arg His Pro His Gln Gly Leu
            105                 110

<210> 474
<211> 111
<212> PRT
<213> Corynebacterium glutamicum

<400> 474
Val Thr Thr Asn His Gln Leu Ser Ala Glu Glu Ile Ser Leu Ala Tyr
1               5                   10                  15

Gly Glu Arg Thr Ile Ile Asp Ser Leu Ser Val Asp Ile Val Pro Gly
            20                  25                  30

Lys Ile Thr Ser Ile Val Gly Pro Asn Gly Cys Gly Lys Ser Thr Leu
        35                  40                  45

Leu Arg Ala Phe Ala Arg Leu Leu Lys Pro Ser Ala Gly Gln Ala Leu
    50                  55                  60

Ile Asp Ala His Pro Leu Pro Ser Leu Pro Gly Lys Glu Leu Ala Arg
65                  70                  75                  80

Met Leu Gly Leu Leu Pro Gln Ser Pro Thr Gly Pro Glu Gly Ile Val
                85                  90                  95

Val Ala Asp Leu Val Gly Arg Gly Arg His Pro His Gln Gly Leu
            100                 105                 110

<210> 475
<211> 900
<212> DNA
<213> Corynebacterium glutamicum

<220>
<221> CDS
<222> (101)..(877)
<223> RXA01823

<400> 475
gcaacacgtt gatactgagc tgcacacgcc cgttggcctg gtgaccagtt tgctgggcgg 60

ggtgtatttg atgtggcttt tgagccgaaa ggaggcataa atg ctg caa gcg cat 115
Met Leu Gln Ala His
1               5

gat ctc acg ctg agt tac ggc ggg cga aat att gta gaa ggg ctc agt 163
Asp Leu Thr Leu Ser Tyr Gly Gly Arg Asn Ile Val Glu Gly Leu Ser
                10                  15                  20

ctg gac ctt ccg gaa aga ggc ctc agc atc atc att ggc ccc aac gga 211
Leu Asp Leu Pro Glu Arg Gly Leu Ser Ile Ile Ile Gly Pro Asn Gly
            25                  30                  35

tgc ggg aaa tca acc gtt ttg aaa gcg ttg ggc aga ctg ctg aaa cca 259
Cys Gly Lys Ser Thr Val Leu Lys Ala Leu Gly Arg Leu Leu Lys Pro
        40                  45                  50

caa ttg ggg aag atc acg cta ggt ggg cga gat att tcc agc atg ggc 307
Gln Leu Gly Lys Ile Thr Leu Gly Gly Arg Asp Ile Ser Ser Met Gly
    55                  60                  65

acc aag cat gtg gcg aaa cac atc ggg gtg ctt ccg caa ccc cca tat 355
Thr Lys His Val Ala Lys His Ile Gly Val Leu Pro Gln Pro Pro Tyr
70                  75                  80                  85

gcg ccc gat ggg gtg agc gtc acg gag ctg gtc agc cgc ggg cgg tat 403
Ala Pro Asp Gly Val Ser Val Thr Glu Leu Val Ser Arg Gly Arg Tyr
                90                  95                  100

ccg cac caa cat ctg ctg tcg caa tgg tcg aaa gac gat gaa gcc att 451
Pro His Gln His Leu Leu Ser Gln Trp Ser Lys Asp Asp Glu Ala Ile
            105                 110                 115

gtg gcg cgc tcg ctg gcg gaa gtc ggc atg cac acc cat gct gag cat 499
Val Ala Arg Ser Leu Ala Glu Val Gly Met His Thr His Ala Glu His
        120                 125                 130

tta gtg tcg gaa ctt tca ggc ggc cag cgc caa cgg gcg tgg atc gcc 547
Leu Val Ser Glu Leu Ser Gly Gly Gln Arg Gln Arg Ala Trp Ile Ala
    135                 140                 145

atg gcg ctc gct cag gaa aca gac att ttg ctt ctc gac gag ccc acc 595
Met Ala Leu Ala Gln Glu Thr Asp Ile Leu Leu Leu Asp Glu Pro Thr
150                 155                 160                 165

acg ttc ctc gac gta gcc cac caa ata tcc gtc ctc gat tta tgc tcc 643
Thr Phe Leu Asp Val Ala His Gln Ile Ser Val Leu Asp Leu Cys Ser
                170                 175                 180

gac ctg cac caa cgt ggt cgc acc ctg gcc att gtt cta cac gat ctg 691
Asp Leu His Gln Arg Gly Arg Thr Leu Ala Ile Val Leu His Asp Leu
            185                 190                 195

aac atg gct gca cgg tat gcc acc cac atc atc gcc atg cgg gac ggc 739
Asn Met Ala Ala Arg Tyr Ala Thr His Ile Ile Ala Met Arg Asp Gly
        200                 205                 210

acc atc atc gac caa gga aaa ccc gaa gaa ata ctc act aaa gcg ctg 787
Thr Ile Ile Asp Gln Gly Lys Pro Glu Glu Ile Leu Thr Lys Ala Leu
    215                 220                 225

ctc aaa gaa gtt ttt gac ctc gac gca ctc atc ctc aaa gac ccc aac 835
Leu Lys Glu Val Phe Asp Leu Asp Ala Leu Ile Leu Lys Asp Pro Asn
230                 235                 240                 245

aac ggc cga cca ctc atc gtg ccc aca gac agg aga aac tca 877
Asn Gly Arg Pro Leu Ile Val Pro Thr Asp Arg Arg Asn Ser
                250                 255

tgaaagaaac cgacaaccta ctg 900

<210> 476
<211> 259
<212> PRT
<213> Corynebacterium glutamicum

<400> 476
Met Leu Gln Ala His Asp Leu Thr Leu Ser Tyr Gly Gly Arg Asn Ile
1               5                   10                  15

Val Glu Gly Leu Ser Leu Asp Leu Pro Glu Arg Gly Leu Ser Ile Ile
            20                  25                  30

Ile Gly Pro Asn Gly Cys Gly Lys Ser Thr Val Leu Lys Ala Leu Gly
        35                  40                  45

Arg Leu Leu Lys Pro Gln Leu Gly Lys Ile Thr Leu Gly Gly Arg Asp
    50                  55                  60

Ile Ser Ser Met Gly Thr Lys His Val Ala Lys His Ile Gly Val Leu
65                  70                  75                  80

Pro Gln Pro Pro Tyr Ala Pro Asp Gly Val Ser Val Thr Glu Leu Val
                85                  90                  95

Ser Arg Gly Arg Tyr Pro His Gln His Leu Leu Ser Gln Trp Ser Lys
            100                 105                 110

Asp Asp Glu Ala Ile Val Ala Arg Ser Leu Ala Glu Val Gly Met His
        115                 120                 125

Thr His Ala Glu His Leu Val Ser Glu Leu Ser Gly Gly Gln Arg Gln
    130                 135                 140

Arg Ala Trp Ile Ala Met Ala Leu Ala Gln Glu Thr Asp Ile Leu Leu
145                 150                 155                 160

Leu Asp Glu Pro Thr Thr Phe Leu Asp Val Ala His Gln Ile Ser Val
                165                 170                 175

Leu Asp Leu Cys Ser Asp Leu His Gln Arg Gly Arg Thr Leu Ala Ile
        180                 185                 190

Val Leu His Asp Leu Asn Met Ala Ala Arg Tyr Ala Thr His Ile Ile
    195                 200                 205

Ala Met Arg Asp Gly Thr Ile Ile Asp Gln Gly Lys Pro Glu Glu Ile
210                 215                 220

Leu Thr Lys Ala Leu Leu Lys Glu Val Phe Asp Leu Asp Ala Leu Ile
225                 230                 235                 240

Leu Lys Asp Pro Asn Asn Gly Arg Pro Leu Ile Val Pro Thr Asp Arg
                245                 250                 255

Arg Asn Ser

<210> 477
<211> 906
<212> DNA
<213> Corynebacterium glutamicum

<220>
<221> CDS
<222> (101)..(883)
<223> RXA02767

<400> 477
gggaaaacgt ttcctcgccg cccacagaat cataaaaatt ttctgaggtt gtcatgggta 60

ccagtctaag ccctggcctt acgccagtaa ggtgttaccc atg cgc gaa cta gca   115
                                            Met Arg Glu Leu Ala
                                            1               5

ctc aac atg gcc ggc gtc acc gtg cgg cgc ggc gag aaa ttg ctt ctc   163
Leu Asn Met Ala Gly Val Thr Val Arg Arg Gly Glu Lys Leu Leu Leu
                10                  15                  20

gac gat atc tcc ctc tca att ccg caa ggg tcg cac tgg gcc gta ctt   211
Asp Asp Ile Ser Leu Ser Ile Pro Gln Gly Ser His Trp Ala Val Leu
            25                  30                  35

ggt cca aat ggc gcc ggt aaa acc acc atg ctg aag atc gca gcc acc   259
Gly Pro Asn Gly Ala Gly Lys Thr Thr Met Leu Lys Ile Ala Ala Thr
        40                  45                  50

ttg ctg tac cca tcg gaa ggc acc gtg gac atc ctg ggg cat cgc ttt   307
Leu Leu Tyr Pro Ser Glu Gly Thr Val Asp Ile Leu Gly His Arg Phe
    55                  60                  65

ggt cgg gtg gat act cgt gag ctg cgg aaa aca atc ggc ctg gtg gac   355
Gly Arg Val Asp Thr Arg Glu Leu Arg Lys Thr Ile Gly Leu Val Asp
70                  75                  80                  85

ccg aag caa aga ttt acc aac ctg ccg gcc cac gaa att gtg ctg tcg   403
Pro Lys Gln Arg Phe Thr Asn Leu Pro Ala His Glu Ile Val Leu Ser
                90                  95                  100

ggg tta acc gcc tcc aac ggg ttg ttg cca cgg tgg tcg gct tcg gct     451
Gly Leu Thr Ala Ser Asn Gly Leu Leu Pro Arg Trp Ser Ala Ser Ala
            105                 110                 115

tcg gag ttg gag cgg tgc gct ttg atg ttg gag ttg gtg ggc atg aca     499
Ser Glu Leu Glu Arg Cys Ala Leu Met Leu Glu Leu Val Gly Met Thr
        120                 125                 130

gcg cgt gcc gat cgt tac tgg gcc gat atg agc cag ggc gaa aaa gcc     547
Ala Arg Ala Asp Arg Tyr Trp Ala Asp Met Ser Gln Gly Glu Lys Ala
    135                 140                 145

cgc acc ctg att gct cgt gcg ctg att atc tca ccg acc cta ctg ctg     595
Arg Thr Leu Ile Ala Arg Ala Leu Ile Ile Ser Pro Thr Leu Leu Leu
150                 155                 160                 165

ctt gat gaa ccc acc acc ggc ctt gac ctg ccc gga cgt gaa act ttg     643
Leu Asp Glu Pro Thr Thr Gly Leu Asp Leu Pro Gly Arg Glu Thr Leu
                170                 175                 180

ctc agt gtg att gat ggt ttg cga gcc gct ctt cct ggt ctg acg aca     691
Leu Ser Val Ile Asp Gly Leu Arg Ala Ala Leu Pro Gly Leu Thr Thr
            185                 190                 195

gtg atg atc acc cac cac gtc gaa gag atc gcc gcc tcc acg aca gat     739
Val Met Ile Thr His His Val Glu Glu Ile Ala Ala Ser Thr Thr Asp
        200                 205                 210

atc ctc atg atc aag gac gcc cgc ata ctg gct tcg ggg act gtt tca     787
Ile Leu Met Ile Lys Asp Ala Arg Ile Leu Ala Ser Gly Thr Val Ser
    215                 220                 225

gaa gtg atg act cct gaa aat ttg ggc gcg ctg tat gac atg tcg gtg     835
Glu Val Met Thr Pro Glu Asn Leu Gly Ala Leu Tyr Asp Met Ser Val
230                 235                 240                 245

tcg ttg gaa act gtg cgc agc cgg tgg ttc gcg ttc gat gct ctg cat     883
Ser Leu Glu Thr Val Arg Ser Arg Trp Phe Ala Phe Asp Ala Leu His
                250                 255                 260

taaaaggggc tagttttaca caa                                           906

<210> 478
<211> 261
<212> PRT
<213> Corynebacterium glutamicum

<400> 478
Met Arg Glu Leu Ala Leu Asn Met Ala Gly Val Thr Val Arg Arg Gly
  1               5                  10                  15

Glu Lys Leu Leu Leu Asp Asp Ile Ser Leu Ser Ile Pro Gln Gly Ser
             20                  25                  30

His Trp Ala Val Leu Gly Pro Asn Gly Ala Gly Lys Thr Thr Met Leu
         35                  40                  45

Lys Ile Ala Ala Thr Leu Leu Tyr Pro Ser Glu Gly Thr Val Asp Ile
     50                  55                  60

Leu Gly His Arg Phe Gly Arg Val Asp Thr Arg Glu Leu Arg Lys Thr
65                  70                  75                  80

Ile Gly Leu Val Asp Pro Lys Gln Arg Phe Thr Asn Leu Pro Ala His
                85                  90                  95

Glu Ile Val Leu Ser Gly Leu Thr Ala Ser Asn Gly Leu Leu Pro Arg
            100                 105                 110

Trp Ser Ala Ser Ala Ser Glu Leu Glu Arg Cys Ala Leu Met Leu Glu
        115                 120                 125

Leu Val Gly Met Thr Ala Arg Ala Asp Arg Tyr Trp Ala Asp Met Ser
    130                 135                 140

Gln Gly Glu Lys Ala Arg Thr Leu Ile Ala Arg Ala Leu Ile Ile Ser
145                 150                 155                 160

Pro Thr Leu Leu Leu Leu Asp Glu Pro Thr Thr Gly Leu Asp Leu Pro
                165                 170                 175

Gly Arg Glu Thr Leu Leu Ser Val Ile Asp Gly Leu Arg Ala Ala Leu
            180                 185                 190

Pro Gly Leu Thr Thr Val Met Ile Thr His His Val Glu Glu Ile Ala
        195                 200                 205

Ala Ser Thr Thr Asp Ile Leu Met Ile Lys Asp Ala Arg Ile Leu Ala
    210                 215                 220

Ser Gly Thr Val Ser Glu Val Met Thr Pro Glu Asn Leu Gly Ala Leu
225                 230                 235                 240

Tyr Asp Met Ser Val Ser Leu Glu Thr Val Arg Ser Arg Trp Phe Ala
                245                 250                 255

Phe Asp Ala Leu His
            260

<210> 479
<211> 876
<212> DNA
<213> Corynebacterium glutamicum

<220>
<221> CDS
<222> (101)..(853)
<223> RXA02792

<400> 479
   agaacaaagt gctgcccata ctcatgaact ttgccgaacc cccaaccccg ctggccgtcg 60
   atggcctaga aaaaatcatc gactttgtgg aaacccaccc atg atc gag gcc aca   115
                                               Met Ile Glu Ala Thr
                                               1               5
   cac cta cgc cac agt ttt ggc gac aac atc gtc atc gat gac gtc acc   163
   His Leu Arg His Ser Phe Gly Asp Asn Ile Val Ile Asp Asp Val Thr
                   10                  15                  20
   ctc cat cta cct gca cac ggc acc gtc agc ttg gtc ggc ccc aac ggc 211
   Leu His Leu Pro Ala His Gly Thr Val Ser Leu Val Gly Pro Asn Gly
   25 30 35
   agt ggc aaa acc acc ctg ctg cgt gca cta tac gga gca ctg caa cca 259
   Ser Gly Lys Thr Thr Leu Leu Arg Ala Leu Tyr Gly Ala Leu Gln Pro
   40 45 50
   aat gaa ggg cac atc cac gta gat ggc gtt cca cta ata agc ttg cac 307
   Asn Glu Gly His Ile His Val Asp Gly Val Pro Leu Ile Ser Leu His
   55 60 65
   cgc aaa gac atc gca aaa acc atg gcc gta gtc atc caa gaa cat gac 355
   Arg Lys Asp Ile Ala Lys Thr Met Ala Val Val Ile Gln Glu His Asp
   70 75 80 85
   tcc gac ctc ccc atg acc gtc gct gac ctg gtc tta cta ggc aga ctc 403
   Ser Asp Leu Pro Met Thr Val Ala Asp Leu Val Leu Leu Gly Arg Leu
   90 95 100
   ccc cac caa aag atg ttt gcc ggc aac tct caa gct gat cag tta ctc 451
   Pro His Gln Lys Met Phe Ala Gly Asn Ser Gln Ala Asp Gln Leu Leu
   105 110 115
   gtt aag gaa gca ctc acc cga gtc ggc gcc att cac ctg gcc gac cga 499
   Val Lys Glu Ala Leu Thr Arg Val Gly Ala Ile His Leu Ala Asp Arg
   120 125 130
   caa ttc ggc gca ctt tca ggc ggt gaa cgc caa cgc gtc ctc atc gca 547
   Gln Phe Gly Ala Leu Ser Gly Gly Glu Arg Gln Arg Val Leu Ile Ala
   135 140 145
   cga gca ctc gta caa aac gcc aca cac att ctg ctc gac gaa ccc acc 595
   Arg Ala Leu Val Gln Asn Ala Thr His Ile Leu Leu Asp Glu Pro Thr
   150 155 160 165
   aac cac ctc gac atc cgc tac cag cac gaa gtc ctt cac ctc gtc cgc 643
   Asn His Leu Asp Ile Arg Tyr Gln His Glu Val Leu His Leu Val Arg
   170 175 180
   gaa ctc agc tca agt tcc atc atc gtc ctc cac gac ctc aac ctc gca 691
   Glu Leu Ser Ser Ser Ser Ile Ile Val Leu His Asp Leu Asn Leu Ala
   185 190 195
   ggt gcc tac agc gac cac atc atc ctc ctt gac caa gga cgt gtg gtt 739
   Gly Ala Tyr Ser Asp His Ile Ile Leu Leu Asp Gln Gly Arg Val Val
   200 205 210
   act caa gga acg ccc tca gag gta ttg acc cca gag cat ttg gaa cct 787
   Thr Gln Gly Thr Pro Ser Glu Val Leu Thr Pro Glu His Leu Glu Pro
   215 220 225
   gtg tat ggc gtt cgt gtt gag cgc ttt gac cta ggc gat gaa gtc cac 835
   Val Tyr Gly Val Arg Val Glu Arg Phe Asp Leu Gly Asp Glu Val His
   230 235 240 245
   ctt cgg ttc aag cgt cac taggagtttt gttgaggagg tgg 876
   Leu Arg Phe Lys Arg His
   250

<210> 480
<211> 251
<212> PRT
<213> Corynebacterium glutamicum

<400> 480
Met Ile Glu Ala Thr His Leu Arg His Ser Phe Gly Asp Asn Ile Val
1               5                   10                  15

Ile Asp Asp Val Thr Leu His Leu Pro Ala His Gly Thr Val Ser Leu
            20                  25                  30

Val Gly Pro Asn Gly Ser Gly Lys Thr Thr Leu Leu Arg Ala Leu Tyr
        35                  40                  45

Gly Ala Leu Gln Pro Asn Glu Gly His Ile His Val Asp Gly Val Pro
    50                  55                  60

Leu Ile Ser Leu His Arg Lys Asp Ile Ala Lys Thr Met Ala Val Val
65                  70                  75                  80

Ile Gln Glu His Asp Ser Asp Leu Pro Met Thr Val Ala Asp Leu Val
                85                  90                  95

Leu Leu Gly Arg Leu Pro His Gln Lys Met Phe Ala Gly Asn Ser Gln
            100                 105                 110

Ala Asp Gln Leu Leu Val Lys Glu Ala Leu Thr Arg Val Gly Ala Ile
        115                 120                 125

His Leu Ala Asp Arg Gln Phe Gly Ala Leu Ser Gly Gly Glu Arg Gln
    130                 135                 140

Arg Val Leu Ile Ala Arg Ala Leu Val Gln Asn Ala Thr His Ile Leu
145                 150                 155                 160

Leu Asp Glu Pro Thr Asn His Leu Asp Ile Arg Tyr Gln His Glu Val
                165                 170                 175

Leu His Leu Val Arg Glu Leu Ser Ser Ser Ile Ile Val Leu His
            180                 185                 190

Asp Leu Asn Leu Ala Gly Ala Tyr Ser Asp His Ile Ile Leu Leu Asp
        195                 200                 205

Gln Gly Arg Val Val Thr Gln Gly Thr Pro Ser Glu Val Leu Thr Pro
    210                 215                 220

Glu His Leu Glu Pro Val Tyr Gly Val Arg Val Glu Arg Phe Asp Leu
225                 230                 235                 240

Gly Asp Glu Val His Leu Arg Phe Lys Arg His
                245                 250


<210> 481
<211> 1161
<212> DNA
<213> Corynebacterium glutamicum

<220>

<221> CDS
<222> (101)..(1138)
<223> RXN02929

<400> 481
   caggtgggga cctcgcgtat cccagctggt ggaagaaatc gcagcgcagt tgaatcagct 60
   tgcttcttct gaagctgtgc cggccgctgc ttaagttttc gtg ctg aag aga att 115
   Val Leu Lys Arg Ile
   1 5
   ttc ctc aac ccc tgg gtg gct acc gcg ttg tcg gta gtc att ttg ggg 163
   Phe Leu Asn Pro Trp Val Ala Thr Ala Leu Ser Val Val Ile Leu Gly
   10 15 20
   ttt gtg gtg ctg ttt tca ggt ttt agc ggt gtt att gat tta agc ccc 211
   Phe Val Val Leu Phe Ser Gly Phe Ser Gly Val Ile Asp Leu Ser Pro
   25 30 35
   aca gca gtg att aga cat ttg agt ggg cag gac acg ctc acc cct cga 259
   Thr Ala Val Ile Arg His Leu Ser Gly Gln Asp Thr Leu Thr Pro Arg
   40 45 50
   gat cag gcc atc ttc ttt gat atc cgg ctg cct cga att atc gct ggt 307
   Asp Gln Ala Ile Phe Phe Asp Ile Arg Leu Pro Arg Ile Ile Ala Gly
   55 60 65
   gtc att gtc gga gca acg ctg gct att tct ggt gct tct tac caa gcg 355
   Val Ile Val Gly Ala Thr Leu Ala Ile Ser Gly Ala Ser Tyr Gln Ala
   70 75 80 85
   gta ttt aga aac ccg ctg gct gat cct tat ttg ttg ggt gtg tcc gca 403
   Val Phe Arg Asn Pro Leu Ala Asp Pro Tyr Leu Leu Gly Val Ser Ala
   90 95 100
   ggt tct ggc ctt ggt gtc acg gca gtg att gtt ggc ggt acc gtg ctg 451
   Gly Ser Gly Leu Gly Val Thr Ala Val Ile Val Gly Gly Thr Val Leu
   105 110 115
   gga ttt tct gca ccg agc atc ggc gtg att ggt gca gca ttt gta ggt 499
   Gly Phe Ser Ala Pro Ser Ile Gly Val Ile Gly Ala Ala Phe Val Gly
   120 125 130
   ggt gtt gcc gca gta ctt gcc acg ctg atg gtg agt cgg gga gta gga 547
   Gly Val Ala Ala Val Leu Ala Thr Leu Met Val Ser Arg Gly Val Gly
   135 140 145
   cag gga tca tca acc acc gtg gtt att ttg gcg ggc gtg gcg gtt gct 595
   Gln Gly Ser Ser Thr Thr Val Val Ile Leu Ala Gly Val Ala Val Ala
   150 155 160 165
   gct ttt gcc agt tcc atc cag acc tat att cag caa cga cac atc gat 643
   Ala Phe Ala Ser Ser Ile Gln Thr Tyr Ile Gln Gln Arg His Ile Asp
   170 175 180
   acg gtg gcg cgc gta tat gtg tgg atg ttg ggc aac ctc aat gtc acc 691
   Thr Val Ala Arg Val Tyr Val Trp Met Leu Gly Asn Leu Asn Val Thr
   185 190 195
   aac tgg atg tcg atc ttc atc gtg gct gtg gtg gcg gga cta tgc gcg 739
   Asn Trp Met Ser Ile Phe Ile Val Ala Val Val Ala Gly Leu Cys Ala
   200                 205                 210
   gcc gtg atc atg tcc tgc gcc agg ttg tta gac gtg atg gct gtt ggt     787
   Ala Val Ile Met Ser Cys Ala Arg Leu Leu Asp Val Met Ala Val Gly
       215                 220                 225
   gat gtg gaa gcc cgc aca ttg ggc gtc gat cca ggc ctc gta cgc att     835
   Asp Val Glu Ala Arg Thr Leu Gly Val Asp Pro Gly Leu Val Arg Ile
   230                 235                 240                 245
   ggc att gtc atc gtg gca acc ctt ggt aca gct gca gtg gta tcc att     883
   Gly Ile Val Ile Val Ala Thr Leu Gly Thr Ala Ala Val Val Ser Ile
                   250                 255                 260
   tcc ggt ctc atc ggg ttt gtg ggc atc att gtt ccg cac gcc ctg cgc     931
   Ser Gly Leu Ile Gly Phe Val Gly Ile Ile Val Pro His Ala Leu Arg
               265                 270                 275
   cta att gtt ggc ccg ggg cat cgg att tta ctg cca ctg tct ttc gta     979
   Leu Ile Val Gly Pro Gly His Arg Ile Leu Leu Pro Leu Ser Phe Val
           280                 285                 290
   tgg ggt gcc att ttc ctc gtg ttg gca gat acc gca ggg cga aca ttg     1027
   Trp Gly Ala Ile Phe Leu Val Leu Ala Asp Thr Ala Gly Arg Thr Leu
       295                 300                 305
   atg gct cct cag gaa ctt ccc gtg ggt gtg gtg aca gct gca ctc ggc     1075
   Met Ala Pro Gln Glu Leu Pro Val Gly Val Val Thr Ala Ala Leu Gly
   310                 315                 320                 325
   gca ccg ttc ttc tta ttt att ttg cgc aga acc agc aga caa cga gtt     1123
   Ala Pro Phe Phe Leu Phe Ile Leu Arg Arg Thr Ser Arg Gln Arg Val
                   330                 335                 340
   cca aaa agg agt gct taagtggcga tcattgaatg cga                       1161
   Pro Lys Arg Ser Ala
               345


<210> 482
<211> 346
<212> PRT
<213> Corynebacterium glutamicum

<400> 482
Val Leu Lys Arg Ile Phe Leu Asn Pro Trp Val Ala Thr Ala Leu Ser
  1               5                  10                  15

Val Val Ile Leu Gly Phe Val Val Leu Phe Ser Gly Phe Ser Gly Val
             20                  25                  30

Ile Asp Leu Ser Pro Thr Ala Val Ile Arg His Leu Ser Gly Gln Asp
         35                  40                  45

Thr Leu Thr Pro Arg Asp Gln Ala Ile Phe Phe Asp Ile Arg Leu Pro
     50                  55                  60

Arg Ile Ile Ala Gly Val Ile Val Gly Ala Thr Leu Ala Ile Ser Gly
 65                  70                  75                  80

Ala Ser Tyr Gln Ala Val Phe Arg Asn Pro Leu Ala Asp Pro Tyr Leu

85                  90                  95

Leu Gly Val Ser Ala Gly Ser Gly Leu Gly Val Thr Ala Val Ile Val
            100                 105                 110

Gly Gly Thr Val Leu Gly Phe Ser Ala Pro Ser Ile Gly Val Ile Gly
        115                 120                 125

Ala Ala Phe Val Gly Gly Val Ala Ala Val Leu Ala Thr Leu Met Val
    130                 135                 140

Ser Arg Gly Val Gly Gln Gly Ser Ser Thr Thr Val Val Ile Leu Ala
145                 150                 155                 160

Gly Val Ala Val Ala Ala Phe Ala Ser Ser Ile Gln Thr Tyr Ile Gln
                165                 170                 175

Gln Arg His Ile Asp Thr Val Ala Arg Val Tyr Val Trp Met Leu Gly
            180                 185                 190

Asn Leu Asn Val Thr Asn Trp Met Ser Ile Phe Ile Val Ala Val Val
        195                 200                 205

Ala Gly Leu Cys Ala Ala Val Ile Met Ser Cys Ala Arg Leu Leu Asp
    210                 215                 220

Val Met Ala Val Gly Asp Val Glu Ala Arg Thr Leu Gly Val Asp Pro
225                 230                 235                 240

Gly Leu Val Arg Ile Gly Ile Val Ile Val Ala Thr Leu Gly Thr Ala
                245                 250                 255

Ala Val Val Ser Ile Ser Gly Leu Ile Gly Phe Val Gly Ile Ile Val
            260                 265                 270

Pro His Ala Leu Arg Leu Ile Val Gly Pro Gly His Arg Ile Leu Leu
        275                 280                 285

Pro Leu Ser Phe Val Trp Gly Ala Ile Phe Leu Val Leu Ala Asp Thr
    290                 295                 300

Ala Gly Arg Thr Leu Met Ala Pro Gln Glu Leu Pro Val Gly Val Val
305                 310                 315                 320

Thr Ala Ala Leu Gly Ala Pro Phe Phe Leu Phe Ile Leu Arg Arg Thr
                325                 330                 335

Ser Arg Gln Arg Val Pro Lys Arg Ser Ala
            340                 345

<210> 483
<211> 1095
<212> DNA
<213> Corynebacterium glutamicum

<220>
<221> CDS
<222> (101)..(1072)
<223> FRXA01235

<400> 483
ttctgaagct gtgccggccg ctgcttaagt tttcgtgctg aagagaattt tcctcaaccc 60

ctgggtggct accgcgttgt cggtagtcat tttggggttt gtg gtg ctg ttt tca 115
Val Val Leu Phe Ser
1               5

ggt ttt agc ggt gtt att gat tta agc ccc aca gca gtg att aga cat 163
Gly Phe Ser Gly Val Ile Asp Leu Ser Pro Thr Ala Val Ile Arg His
                10                  15                  20

ttg agt ggg cag gac acg ctc acc cct cga gat cag gcc atc ttc ttt 211
Leu Ser Gly Gln Asp Thr Leu Thr Pro Arg Asp Gln Ala Ile Phe Phe
            25                  30                  35

gat atc cgg ctg cct cga att atc gct ggt gtc att gtc gga gca acg 259
Asp Ile Arg Leu Pro Arg Ile Ile Ala Gly Val Ile Val Gly Ala Thr
        40                  45                  50

ctg gct att tct ggt gct gct tac caa gcg gta ttt aga aac ccg ctg 307
Leu Ala Ile Ser Gly Ala Ala Tyr Gln Ala Val Phe Arg Asn Pro Leu
    55                  60                  65

gct gat cct tat ttg ttg ggt gtg tcc gca ggt tct ggc ctt ggt gtc 355
Ala Asp Pro Tyr Leu Leu Gly Val Ser Ala Gly Ser Gly Leu Gly Val
70                  75                  80                  85

acg gca gtg att gtt ggc ggt acc gtg ctg gga ttt tct gca ccg agc 403
Thr Ala Val Ile Val Gly Gly Thr Val Leu Gly Phe Ser Ala Pro Ser
                90                  95                  100

atc ggc gtg att ggt gca gca ttt gta ggt ggt gtt gcc gca gta ctt 451
Ile Gly Val Ile Gly Ala Ala Phe Val Gly Gly Val Ala Ala Val Leu
            105                 110                 115

gcc acg ctg atg gtg agt cgg gga gta gga cag gga tca tca acc acc 499
Ala Thr Leu Met Val Ser Arg Gly Val Gly Gln Gly Ser Ser Thr Thr
        120                 125                 130

gtg gtt att ttg gcg ggc gtg gcg gtt gct gct ttt gcc agt tcc atc 547
Val Val Ile Leu Ala Gly Val Ala Val Ala Ala Phe Ala Ser Ser Ile
    135                 140                 145

cag acc tat att cag caa cga cac atc gat acg gtg gcg cgc gta tat 595
Gln Thr Tyr Ile Gln Gln Arg His Ile Asp Thr Val Ala Arg Val Tyr
150                 155                 160                 165

gtg tgg atg ttg ggc aac ctc aat gtc acc aac tgg atg tcg atc ttc 643
Val Trp Met Leu Gly Asn Leu Asn Val Thr Asn Trp Met Ser Ile Phe
                170                 175                 180

atc gtg gct gtg gtg gcg gga cta tgc gcg gcc gtg atc atg tcc tgc 691
Ile Val Ala Val Val Ala Gly Leu Cys Ala Ala Val Ile Met Ser Cys
            185                 190                 195

gcc agg ttg tta gac gtg atg gct gtt ggt gat gtg gaa gcc cgc aca 739
Ala Arg Leu Leu Asp Val Met Ala Val Gly Asp Val Glu Ala Arg Thr
        200                 205                 210

ttg ggc gtc gat cca ggc ctc gta cgc att ggc att gtc atc gtg gca 787
Leu Gly Val Asp Pro Gly Leu Val Arg Ile Gly Ile Val Ile Val Ala

215                 220                 225

acc ctt ggt aca gct gca gtg gta tcc att tcc ggt ctc atc ggg ttt      835
Thr Leu Gly Thr Ala Ala Val Val Ser Ile Ser Gly Leu Ile Gly Phe
230                 235                 240                 245

gtg ggc atc att gtt ccg cac gcc ctg cgc cta att gtt ggc ccg ggg      883
Val Gly Ile Ile Val Pro His Ala Leu Arg Leu Ile Val Gly Pro Gly
                250                 255                 260

cat cgg att tta ctg cca ctg tct ttc gta tgg ggt gcc att ttc ctc      931
His Arg Ile Leu Leu Pro Leu Ser Phe Val Trp Gly Ala Ile Phe Leu
            265                 270                 275

gtg ttg gca gat acc gca ggg cga aca ttg atg gct cct cag gaa ctt      979
Val Leu Ala Asp Thr Ala Gly Arg Thr Leu Met Ala Pro Gln Glu Leu
        280                 285                 290

ccc gtg ggt gtg gtg aca gct gca ctc ggc gca ccg ttc ttc tta ttt      1027
Pro Val Gly Val Val Thr Ala Ala Leu Gly Ala Pro Phe Phe Leu Phe
    295                 300                 305

att ttg cgc aga acc agc aga caa cga gtt cca aaa agg agt gct          1072
Ile Leu Arg Arg Thr Ser Arg Gln Arg Val Pro Lys Arg Ser Ala
310                 315                 320

taagtggcga tcattgaatg cga                                            1095

<210> 484
<211> 324
<212> PRT
<213> Corynebacterium glutamicum

<400> 484
Val Val Leu Phe Ser Gly Phe Ser Gly Val Ile Asp Leu Ser Pro Thr
1               5                   10                  15

Ala Val Ile Arg His Leu Ser Gly Gln Asp Thr Leu Thr Pro Arg Asp
            20                  25                  30

Gln Ala Ile Phe Phe Asp Ile Arg Leu Pro Arg Ile Ile Ala Gly Val
        35                  40                  45

Ile Val Gly Ala Thr Leu Ala Ile Ser Gly Ala Ala Tyr Gln Ala Val
    50                  55                  60

Phe Arg Asn Pro Leu Ala Asp Pro Tyr Leu Leu Gly Val Ser Ala Gly
65                  70                  75                  80

Ser Gly Leu Gly Val Thr Ala Val Ile Val Gly Gly Thr Val Leu Gly
                85                  90                  95

Phe Ser Ala Pro Ser Ile Gly Val Ile Gly Ala Ala Phe Val Gly Gly
            100                 105                 110

Val Ala Ala Val Leu Ala Thr Leu Met Val Ser Arg Gly Val Gly Gln
        115                 120                 125

Gly Ser Ser Thr Thr Val Val Ile Leu Ala Gly Val Ala Val Ala Ala
    130                 135                 140

Phe Ala Ser Ser Ile Gln Thr Tyr Ile Gln Gln Arg His Ile Asp Thr
145                 150                 155                 160

Val Ala Arg Val Tyr Val Trp Met Leu Gly Asn Leu Asn Val Thr Asn
                165                 170                 175

Trp Met Ser Ile Phe Ile Val Ala Val Val Ala Gly Leu Cys Ala Ala
            180                 185                 190

Val Ile Met Ser Cys Ala Arg Leu Leu Asp Val Met Ala Val Gly Asp
        195                 200                 205

Val Glu Ala Arg Thr Leu Gly Val Asp Pro Gly Leu Val Arg Ile Gly
    210                 215                 220

Ile Val Ile Val Ala Thr Leu Gly Thr Ala Ala Val Val Ser Ile Ser
225                 230                 235                 240

Gly Leu Ile Gly Phe Val Gly Ile Ile Val Pro His Ala Leu Arg Leu
                245                 250                 255

Ile Val Gly Pro Gly His Arg Ile Leu Leu Pro Leu Ser Phe Val Trp
            260                 265                 270

Gly Ala Ile Phe Leu Val Leu Ala Asp Thr Ala Gly Arg Thr Leu Met
        275                 280                 285

Ala Pro Gln Glu Leu Pro Val Gly Val Val Thr Ala Ala Leu Gly Ala
    290                 295                 300

Pro Phe Phe Leu Phe Ile Leu Arg Arg Thr Ser Arg Gln Arg Val Pro
305                 310                 315                 320

Lys Arg Ser Ala

<210> 485
<211> 1197
<212> DNA
<213> Corynebacterium glutamicum

<220>
<221> CDS
<222> (101)..(1174)
<223> RXN02794

<400> 485
gcgcccactc atcggcgagc ttcaggagat gaggttgatg ctccattgat aatttctttc 60

gctaatagtc aaatgatcat ttgagtgtta gtgttttctc atg ctt ctt tcc gcc   115
                                          Met Leu Leu Ser Ala
                                          1               5

cgc aca cac acg agt ttc caa gaa ctt gga ctc aat gct agt cgg cgc   163
Arg Thr His Thr Ser Phe Gln Glu Leu Gly Leu Asn Ala Ser Arg Arg
                10                  15                  20

aaa gca atc aac tgg aca ctg gca ctc act gtg gtg cta att gcc tcc   211
Lys Ala Ile Asn Trp Thr Leu Ala Leu Thr Val Val Leu Ile Ala Ser

25                  30                  35

atg ttt gtt ggc gtg ctc atc ggt gca tcc ggg acc tca gtg ttt tcc    259
Met Phe Val Gly Val Leu Ile Gly Ala Ser Gly Thr Ser Val Phe Ser
        40                  45                  50

acg tgg acc gta att agc cac cat ctt ttt ggc act gag cta ggt ggc    307
Thr Trp Thr Val Ile Ser His His Leu Phe Gly Thr Glu Leu Gly Gly
    55                  60                  65

tcc gac act gcc gac gcc atc att tgg tac atc cgc acc cca cgc gtc    355
Ser Asp Thr Ala Asp Ala Ile Ile Trp Tyr Ile Arg Thr Pro Arg Val
70                  75                  80                  85

ttg ctc gct gcc att gtg ggc gca ggc ctt gcc ctg gca ggt gcc atc    403
Leu Leu Ala Ala Ile Val Gly Ala Gly Leu Ala Leu Ala Gly Ala Ile
                90                  95                  100

atg caa gta ctg gtc cga aac atg ctg gca gac ccc tat atc ctc ggg    451
Met Gln Val Leu Val Arg Asn Met Leu Ala Asp Pro Tyr Ile Leu Gly
            105                 110                 115

gtg aac tca ggt gcc agt tgc ggt gcg gcc gct gcc tta ctg ttc gga    499
Val Asn Ser Gly Ala Ser Cys Gly Ala Ala Ala Ala Leu Leu Phe Gly
        120                 125                 130

gtg ggc gct gga ttt ggc gat tac gcc ctc caa ggc agc gcg ttt ctc    547
Val Gly Ala Gly Phe Gly Asp Tyr Ala Leu Gln Gly Ser Ala Phe Leu
    135                 140                 145

ggc gca atg gca gct tcc gga ttg atc ttc ttc gtg gcg cgc gca gcg    595
Gly Ala Met Ala Ala Ser Gly Leu Ile Phe Phe Val Ala Arg Ala Ala
150                 155                 160                 165

ggg cgc atc tcc tcg acc cgc ttg ttg atg tcc ggc gta gcg atc gga    643
Gly Arg Ile Ser Ser Thr Arg Leu Leu Met Ser Gly Val Ala Ile Gly
                170                 175                 180

tac atg ctc tct gcg gca aca agc ttt ctc atc ttc tcc tcc gac tcc    691
Tyr Met Leu Ser Ala Ala Thr Ser Phe Leu Ile Phe Ser Ser Asp Ser
            185                 190                 195

gcc gaa ggc agc cgc tcc gtg ttg ttc tgg ctg ctt gga tcc tta gga    739
Ala Glu Gly Ser Arg Ser Val Leu Phe Trp Leu Leu Gly Ser Leu Gly
        200                 205                 210

ctt gcc gca tgg aat ggg ccg atg gcg atc atc ttc ctc atc gtg ggc    787
Leu Ala Ala Trp Asn Gly Pro Met Ala Ile Ile Phe Leu Ile Val Gly
    215                 220                 225

att gcc ctg gcg ttg ctc atg gtg ttg ggt ccg caa ttg gat gcc tta    835
Ile Ala Leu Ala Leu Leu Met Val Leu Gly Pro Gln Leu Asp Ala Leu
230                 235                 240                 245

aac tcc ggc gat gaa acc gca ctt acc ttg gga gta tcc cct gat cgc    883
Asn Ser Gly Asp Glu Thr Ala Leu Thr Leu Gly Val Ser Pro Asp Arg
                250                 255                 260

ctc cgc att ctc ctc ctg gtg atc acc tgc ctg ctg gtg gga tcc atg    931
Leu Arg Ile Leu Leu Leu Val Ile Thr Cys Leu Leu Val Gly Ser Met
            265                 270                 275

gtt gcc atg gcc ggc agc atc gga ttc atc ggc ctt gtc atc ccc cac 979
Val Ala Met Ala Gly Ser Ile Gly Phe Ile Gly Leu Val Ile Pro His
        280                 285                 290

ctg gcc agg cgt ttt gtt agt gga aaa cac cga ctc atg ctg cct gta 1027
Leu Ala Arg Arg Phe Val Ser Gly Lys His Arg Leu Met Leu Pro Val
    295                 300                 305

tcc gcg ttg atg ggc gca att ttg ctc atc tgg gct gat atc gcc gcc 1075
Ser Ala Leu Met Gly Ala Ile Leu Leu Ile Trp Ala Asp Ile Ala Ala
310                 315                 320                 325

cgc acc ctg ctt gcg ccc caa gag att ccc atc ggc atc atc acc gca 1123
Arg Thr Leu Leu Ala Pro Gln Glu Ile Pro Ile Gly Ile Ile Thr Ala
                330                 335                 340

ctc atc gga gca ccc ttc ctc ctg att ctg gtt cgc cgg atg cac acc 1171
Leu Ile Gly Ala Pro Phe Leu Leu Ile Leu Val Arg Arg Met His Thr
            345                 350                 355

tac tgattttttaa ggaattatgc gta 1197
Tyr

<210> 486
<211> 358
<212> PRT
<213> Corynebacterium glutamicum

<400> 486
Met Leu Leu Ser Ala Arg Thr His Thr Ser Phe Gln Glu Leu Gly Leu
1               5                   10                  15

Asn Ala Ser Arg Arg Lys Ala Ile Asn Trp Thr Leu Ala Leu Thr Val
            20                  25                  30

Val Leu Ile Ala Ser Met Phe Val Gly Val Leu Ile Gly Ala Ser Gly
        35                  40                  45

Thr Ser Val Phe Ser Thr Trp Thr Val Ile Ser His His Leu Phe Gly
    50                  55                  60

Thr Glu Leu Gly Gly Ser Asp Thr Ala Asp Ala Ile Ile Trp Tyr Ile
65                  70                  75                  80

Arg Thr Pro Arg Val Leu Leu Ala Ala Ile Val Gly Ala Gly Leu Ala
                85                  90                  95

Leu Ala Gly Ala Ile Met Gln Val Leu Val Arg Asn Met Leu Ala Asp
            100                 105                 110

Pro Tyr Ile Leu Gly Val Asn Ser Gly Ala Ser Cys Gly Ala Ala Ala
        115                 120                 125

Ala Leu Leu Phe Gly Val Gly Ala Gly Phe Gly Asp Tyr Ala Leu Gln
    130                 135                 140

Gly Ser Ala Phe Leu Gly Ala Met Ala Ala Ser Gly Leu Ile Phe Phe
145                 150                 155                 160

Val Ala Arg Ala Ala Gly Arg Ile Ser Ser Thr Arg Leu Leu Met Ser
                165                 170                 175

Gly Val Ala Ile Gly Tyr Met Leu Ser Ala Ala Thr Ser Phe Leu Ile
            180                 185                 190

Phe Ser Ser Asp Ser Ala Glu Gly Ser Arg Ser Val Leu Phe Trp Leu
        195                 200                 205

Leu Gly Ser Leu Gly Leu Ala Ala Trp Asn Gly Pro Met Ala Ile Ile
    210                 215                 220

Phe Leu Ile Val Gly Ile Ala Leu Ala Leu Leu Met Val Leu Gly Pro
225                 230                 235                 240

Gln Leu Asp Ala Leu Asn Ser Gly Asp Glu Thr Ala Leu Thr Leu Gly
                245                 250                 255

Val Ser Pro Asp Arg Leu Arg Ile Leu Leu Leu Val Ile Thr Cys Leu
            260                 265                 270

Leu Val Gly Ser Met Val Ala Met Ala Gly Ser Ile Gly Phe Ile Gly
        275                 280                 285

Leu Val Ile Pro His Leu Ala Arg Arg Phe Val Ser Gly Lys His Arg
    290                 295                 300

Leu Met Leu Pro Val Ser Ala Leu Met Gly Ala Ile Leu Leu Ile Trp
305                 310                 315                 320

Ala Asp Ile Ala Ala Arg Thr Leu Leu Ala Pro Gln Glu Ile Pro Ile
                325                 330                 335

Gly Ile Ile Thr Ala Leu Ile Gly Ala Pro Phe Leu Leu Ile Leu Val
            340                 345                 350

Arg Arg Met His Thr Tyr
        355

<210> 487
<211> 364
<212> DNA
<213> Corynebacterium glutamicum

<220>
<221> CDS
<222> (101)..(364)
<223> FRXA01419

<400> 487
gcgcccactc atcggcgagc ttcaggagat gaggttgatg ctccattgat aatttctttc 60

gctaatagtc aaatgatcat ttgagtgtta gtgttttctc atg ctt ctt tcc gcc   115
                                           Met Leu Leu Ser Ala
                                           1               5

cgc aca cac acg agt ttc caa gaa ctt gga ctc aat gct agt cgg cgc   163
Arg Thr His Thr Ser Phe Gln Glu Leu Gly Leu Asn Ala Ser Arg Arg
                10                  15                  20

aaa gca atc aac tgg aca ctg gca ctc act gtg gtg cta att gcc tcc    211
Lys Ala Ile Asn Trp Thr Leu Ala Leu Thr Val Val Leu Ile Ala Ser
            25                  30                  35

atg ttt gtt ggc gtg ctc atc ggt gca tcc ggg acc tca gtg ttt tcc    259
Met Phe Val Gly Val Leu Ile Gly Ala Ser Gly Thr Ser Val Phe Ser
        40                  45                  50

acg tgg acc gta att agc cac cat ctt ttt ggc act gag cta ggt ggc    307
Thr Trp Thr Val Ile Ser His His Leu Phe Gly Thr Glu Leu Gly Gly
    55                  60                  65

tcc gac act gcc gac gcc atc att tgg tac atc cgc acc cca cgc gtc    355
Ser Asp Thr Ala Asp Ala Ile Ile Trp Tyr Ile Arg Thr Pro Arg Val
70                  75                  80                  85

ttg ctc gct                                                        364
Leu Leu Ala

<210> 488
<211> 88
<212> PRT
<213> Corynebacterium glutamicum

<400> 488
Met Leu Leu Ser Ala Arg Thr His Thr Ser Phe Gln Glu Leu Gly Leu
1               5                   10                  15

Asn Ala Ser Arg Arg Lys Ala Ile Asn Trp Thr Leu Ala Leu Thr Val
            20                  25                  30

Val Leu Ile Ala Ser Met Phe Val Gly Val Leu Ile Gly Ala Ser Gly
        35                  40                  45

Thr Ser Val Phe Ser Thr Trp Thr Val Ile Ser His His Leu Phe Gly
    50                  55                  60

Thr Glu Leu Gly Gly Ser Asp Thr Ala Asp Ala Ile Ile Trp Tyr Ile
65                  70                  75                  80

Arg Thr Pro Arg Val Leu Leu Ala
                85

<210> 489
<211> 744
<212> DNA
<213> Corynebacterium glutamicum

<220>
<221> CDS
<222> (101)..(721)
<223> FRXA02794

<400> 489
tgaactcagg tgccagttgc ggtgcggccg ctgccttact gttcggagtg ggcgctggat 60

ttggcgatta cgccctccaa gcagcgcgtt tctcggcgca atg gca gct tcc gga    115

Met Ala Ala Ser Gly
1               5

ttg atc ttc ttc gtg gcg cgc gca gcg ggg cgc atc tcc tcg acc cgc     163
Leu Ile Phe Phe Val Ala Arg Ala Ala Gly Arg Ile Ser Ser Thr Arg
                10                  15                  20

ttg ttg atg tcc ggc gta gcg atc gga tac atg ctc tct gcg gca aca     211
Leu Leu Met Ser Gly Val Ala Ile Gly Tyr Met Leu Ser Ala Ala Thr
            25                  30                  35

agc ttt ctc atc ttc tcc tcc gac tcc gcc gaa ggc agc cgc tcc gtg     259
Ser Phe Leu Ile Phe Ser Ser Asp Ser Ala Glu Gly Ser Arg Ser Val
        40                  45                  50

ttg ttc tgg ctg ctt gga tcc tta gga ctt gcc gca tgg aat ggg ccg     307
Leu Phe Trp Leu Leu Gly Ser Leu Gly Leu Ala Ala Trp Asn Gly Pro
    55                  60                  65

atg gcg atc atc ttc ctc atc gtg ggc att gcc ctg gcg ttg ctc atg     355
Met Ala Ile Ile Phe Leu Ile Val Gly Ile Ala Leu Ala Leu Leu Met
70                  75                  80                  85

gtg ttg ggt ccg caa ttg gat gcc tta aac tcc ggc gat gaa acc gca     403
Val Leu Gly Pro Gln Leu Asp Ala Leu Asn Ser Gly Asp Glu Thr Ala
                90                  95                  100

ctt acc ttg gga gta tcc cct gat cgc ctc cgc att ctc ctc ctg gtg     451
Leu Thr Leu Gly Val Ser Pro Asp Arg Leu Arg Ile Leu Leu Leu Val
            105                 110                 115

atc acc tgc ctg ctg gtg gga tcc atg gtt gcc atg gcc ggc agc atc     499
Ile Thr Cys Leu Leu Val Gly Ser Met Val Ala Met Ala Gly Ser Ile
        120                 125                 130

gga ttc atc ggc ctt gtc atc ccc cac ctg gcc agg cgt ttt gtt agt     547
Gly Phe Ile Gly Leu Val Ile Pro His Leu Ala Arg Arg Phe Val Ser
    135                 140                 145

gga aaa cac cga ctc atg ctg cct gta tcc gcg ttg atg ggc gca att     595
Gly Lys His Arg Leu Met Leu Pro Val Ser Ala Leu Met Gly Ala Ile
150                 155                 160                 165

ttg ctc atc tgg gct gat atc gcc gcc cgc acc ctg ctt gcg ccc caa     643
Leu Leu Ile Trp Ala Asp Ile Ala Ala Arg Thr Leu Leu Ala Pro Gln
                170                 175                 180

gag att ccc atc ggc atc atc acc gca ctc atc gga gca ccc ttc ctc     691
Glu Ile Pro Ile Gly Ile Ile Thr Ala Leu Ile Gly Ala Pro Phe Leu
            185                 190                 195

ctg att ctg gtt cgc cgg atg cac acc tac tgatttttaa ggaattatgc     741
Leu Ile Leu Val Arg Arg Met His Thr Tyr
        200                 205

gta                                                                 744

<210> 490
<211> 207
<212> PRT

<213> Corynebacterium glutamicum

<400> 490
Met Ala Ala Ser Gly Leu Ile Phe Phe Val Ala Arg Ala Ala Gly Arg
1               5                   10                  15

Ile Ser Ser Thr Arg Leu Leu Met Ser Gly Val Ala Ile Gly Tyr Met
            20                  25                  30

Leu Ser Ala Ala Thr Ser Phe Leu Ile Phe Ser Ser Asp Ser Ala Glu
        35                  40                  45

Gly Ser Arg Ser Val Leu Phe Trp Leu Leu Gly Ser Leu Gly Leu Ala
    50                  55                  60

Ala Trp Asn Gly Pro Met Ala Ile Ile Phe Leu Ile Val Gly Ile Ala
65                  70                  75                  80

Leu Ala Leu Leu Met Val Leu Gly Pro Gln Leu Asp Ala Leu Asn Ser
                85                  90                  95

Gly Asp Glu Thr Ala Leu Thr Leu Gly Val Ser Pro Asp Arg Leu Arg
            100                 105                 110

Ile Leu Leu Leu Val Ile Thr Cys Leu Leu Val Gly Ser Met Val Ala
        115                 120                 125

Met Ala Gly Ser Ile Gly Phe Ile Gly Leu Val Ile Pro His Leu Ala
    130                 135                 140

Arg Arg Phe Val Ser Gly Lys His Arg Leu Met Leu Pro Val Ser Ala
145                 150                 155                 160

Leu Met Gly Ala Ile Leu Leu Ile Trp Ala Asp Ile Ala Ala Arg Thr
                165                 170                 175

Leu Leu Ala Pro Gln Glu Ile Pro Ile Gly Ile Ile Thr Ala Leu Ile
            180                 185                 190

Gly Ala Pro Phe Leu Leu Ile Leu Val Arg Arg Met His Thr Tyr
        195                 200                 205


<210> 491
<211> 1140
<212> DNA
<213> Corynebacterium glutamicum

<220>
<221> CDS
<222> (101)..(1117)
<223> RXN03079

<400> 491
gagcggggtt gctatcggcc gaaagtttag ggttttgttc aatctgttgg ttagtattgc 60

ttgggtaaac aagtcataac aattttcatt aagggtcgtt ttg tcg cgc aca ggt   115
                                           Leu Ser Arg Thr Gly
                                           1               5

gtt tcg aaa aaa cca aag ctc acc gct cct gtt gtc atc atc ggc acc   163

Val Ser Lys Lys Pro Lys Leu Thr Ala Pro Val Val Ile Ile Gly Thr
                10                  15                  20

ctc gtc ttg ttg atc atc gcc ttc acc gct tcc ctc atg ctg ggt ccc     211
Leu Val Leu Leu Ile Ile Ala Phe Thr Ala Ser Leu Met Leu Gly Pro
            25                  30                  35

gtg acg gtt cca ttg aat gag ctt gca acc aac ccc gtt gtc acc gat     259
Val Thr Val Pro Leu Asn Glu Leu Ala Thr Asn Pro Val Val Thr Asp
        40                  45                  50

atc cgt gca cca cgc att atc atc gca gca ttg gtg ggt gcg gcg ctg     307
Ile Arg Ala Pro Arg Ile Ile Ile Ala Ala Leu Val Gly Ala Ala Leu
    55                  60                  65

gct gtc tcc ggt gcg atc atg cag acg gtg ttt cac aac ccg ttg gcg     355
Ala Val Ser Gly Ala Ile Met Gln Thr Val Phe His Asn Pro Leu Ala
70                  75                  80                  85

gat ccc ggc att gtg ggt gtg tcc tcc ggt gca gct gtt gca gct gtc     403
Asp Pro Gly Ile Val Gly Val Ser Ser Gly Ala Ala Val Ala Ala Val
                90                  95                  100

ttg gcg att gtc acc ggt gcg agt ttc ttt ggc caa tgg acc gtt cct     451
Leu Ala Ile Val Thr Gly Ala Ser Phe Phe Gly Gln Trp Thr Val Pro
            105                 110                 115

ttt gcg gcc ttc gtg ggc gca ttg gtc acg gtg gct gtg gta tat ttg     499
Phe Ala Ala Phe Val Gly Ala Leu Val Thr Val Ala Val Val Tyr Leu
        120                 125                 130

atc gct agt tcc cgc gcg atg gat ggc cgt ggc gca gat ccg gcc acg     547
Ile Ala Ser Ser Arg Ala Met Asp Gly Arg Gly Ala Asp Pro Ala Thr
    135                 140                 145

ttg gta ctg gtc ggc atg gct atc act gcc ttt ttg ggt gct gtt att     595
Leu Val Leu Val Gly Met Ala Ile Thr Ala Phe Leu Gly Ala Val Ile
150                 155                 160                 165

tcc agc gcc act gcg aac gca cca caa gat tct gag ctt cga tcc gtg     643
Ser Ser Ala Thr Ala Asn Ala Pro Gln Asp Ser Glu Leu Arg Ser Val
                170                 175                 180

acg ttt tgg ctc aac ggc gat ctg gta tct cgg acg tgg gaa cat gtg     691
Thr Phe Trp Leu Asn Gly Asp Leu Val Ser Arg Thr Trp Glu His Val
            185                 190                 195

ggc gtt gca ata atc ccc att atc gtt ggg ttg att cta gct atc ggc     739
Gly Val Ala Ile Ile Pro Ile Ile Val Gly Leu Ile Leu Ala Ile Gly
        200                 205                 210

ggt tcc cgc gat ctg aac ttg ttg ctg ctg ggt gat tcc aca gcg caa     787
Gly Ser Arg Asp Leu Asn Leu Leu Leu Leu Gly Asp Ser Thr Ala Gln
    215                 220                 225

aca tct gga ctc aac gtc aac cgc gca cgc atc att ttg cta gca ctt     835
Thr Ser Gly Leu Asn Val Asn Arg Ala Arg Ile Ile Leu Leu Ala Leu
230                 235                 240                 245

gcg gca ctg ctc acc gcc aca gct gtt gcg gtc tcc ggc acc att acg     883
Ala Ala Leu Leu Thr Ala Thr Ala Val Ala Val Ser Gly Thr Ile Thr

                250                 255                 260

ttt gtt gga ttg gta gta ccc cac ctg gtg cgc att gtt tta ggt gcc 931
Phe Val Gly Leu Val Val Pro His Leu Val Arg Ile Val Leu Gly Ala
            265                 270                 275

gat cac cga gcg tta ctc ccg gcc gcc gcg att ttg ggc gcc acg ttt 979
Asp His Arg Ala Leu Leu Pro Ala Ala Ala Ile Leu Gly Ala Thr Phe
        280                 285                 290

gtt atc gtt tcc gac act gtt gcc cgc atg atc ttc tcc ccc atc gtc 1027
Val Ile Val Ser Asp Thr Val Ala Arg Met Ile Phe Ser Pro Ile Val
    295                 300                 305

ttg caa aca ggc gtg gtg gtg gcg ttc att ggc tca cca att ttc ctt 1075
Leu Gln Thr Gly Val Val Val Ala Phe Ile Gly Ser Pro Ile Phe Leu
310                 315                 320                 325

tat tta ctg ctc agc atg cgc aag cga cgc gga ttg ggg ctg 1117
Tyr Leu Leu Leu Ser Met Arg Lys Arg Arg Gly Leu Gly Leu
                330                 335

taaaaactca tgcctcaatt agt 1140

<210> 492
<211> 339
<212> PRT
<213> Corynebacterium glutamicum

<400> 492
Leu Ser Arg Thr Gly Val Ser Lys Lys Pro Lys Leu Thr Ala Pro Val
1               5                   10                  15

Val Ile Ile Gly Thr Leu Val Leu Leu Ile Ile Ala Phe Thr Ala Ser
            20                  25                  30

Leu Met Leu Gly Pro Val Thr Val Pro Leu Asn Glu Leu Ala Thr Asn
        35                  40                  45

Pro Val Val Thr Asp Ile Arg Ala Pro Arg Ile Ile Ile Ala Ala Leu
    50                  55                  60

Val Gly Ala Ala Leu Ala Val Ser Gly Ala Ile Met Gln Thr Val Phe
65                  70                  75                  80

His Asn Pro Leu Ala Asp Pro Gly Ile Val Gly Val Ser Ser Gly Ala
                85                  90                  95

Ala Val Ala Ala Val Leu Ala Ile Val Thr Gly Ala Ser Phe Phe Gly
            100                 105                 110

Gln Trp Thr Val Pro Phe Ala Ala Phe Val Gly Ala Leu Val Thr Val
        115                 120                 125

Ala Val Val Tyr Leu Ile Ala Ser Ser Arg Ala Met Asp Gly Arg Gly
    130                 135                 140

Ala Asp Pro Ala Thr Leu Val Leu Val Gly Met Ala Ile Thr Ala Phe
145                 150                 155                 160

Leu Gly Ala Val Ile Ser Ser Ala Thr Ala Asn Ala Pro Gln Asp Ser
                165                 170                 175

Glu Leu Arg Ser Val Thr Phe Trp Leu Asn Gly Asp Leu Val Ser Arg
            180                 185                 190

Thr Trp Glu His Val Gly Val Ala Ile Ile Pro Ile Ile Val Gly Leu
        195                 200                 205

Ile Leu Ala Ile Gly Gly Ser Arg Asp Leu Asn Leu Leu Leu Leu Gly
    210                 215                 220

Asp Ser Thr Ala Gln Thr Ser Gly Leu Asn Val Asn Arg Ala Arg Ile
225                 230                 235                 240

Ile Leu Leu Ala Leu Ala Ala Leu Leu Thr Ala Thr Ala Val Ala Val
                245                 250                 255

Ser Gly Thr Ile Thr Phe Val Gly Leu Val Val Pro His Leu Val Arg
            260                 265                 270

Ile Val Leu Gly Ala Asp His Arg Ala Leu Leu Pro Ala Ala Ala Ile
        275                 280                 285

Leu Gly Ala Thr Phe Val Ile Val Ser Asp Thr Val Ala Arg Met Ile
    290                 295                 300

Phe Ser Pro Ile Val Leu Gln Thr Gly Val Val Val Ala Phe Ile Gly
305                 310                 315                 320

Ser Pro Ile Phe Leu Tyr Leu Leu Leu Ser Met Arg Lys Arg Arg Gly
                325                 330                 335

Leu Gly Leu

<210> 493
<211> 1140
<212> DNA
<213> Corynebacterium glutamicum

<220>
<221> CDS
<222> (101)..(1117)
<223> FRXA02865

<400> 493
gagcggggtt gctatcggcc gaaagtttag ggttttgttc aatctgttgg ttagtattgc 60

ttgggtaaac aagtcataac aattttcatt aagggtcgtt ttg tcg cgc aca ggt   115
                                            Leu Ser Arg Thr Gly
                                            1               5

gtt tcg aaa aaa cca aag ctc acc gct cct gtt gtc atc atc ggc acc   163
Val Ser Lys Lys Pro Lys Leu Thr Ala Pro Val Val Ile Ile Gly Thr
                10                  15                  20

ctc gtc ttg ttg atc atc gcc ttc acc gct tcc ctc atg ctg ggt ccc   211
Leu Val Leu Leu Ile Ile Ala Phe Thr Ala Ser Leu Met Leu Gly Pro
            25                  30                  35

gtg acg gtt cca ttg aat gag ctt gca acc aac ccc gtt gtc acc gat 259
Val Thr Val Pro Leu Asn Glu Leu Ala Thr Asn Pro Val Val Thr Asp
        40                  45                  50

atc cgt gca cca cgc att atc atc gca gca ttg gtg ggt gcg gcg ctg 307
Ile Arg Ala Pro Arg Ile Ile Ile Ala Ala Leu Val Gly Ala Ala Leu
    55                  60                  65

gct gtc tcc ggt gcg atc atg cag acg gtg ttt cac aac ccg ttg gcg 355
Ala Val Ser Gly Ala Ile Met Gln Thr Val Phe His Asn Pro Leu Ala
70                  75                  80                  85

gat ccc ggc att gtg ggt gtg tcc tcc ggt gca gct gtt gca gct gtc 403
Asp Pro Gly Ile Val Gly Val Ser Ser Gly Ala Ala Val Ala Ala Val
                90                  95                  100

ttg gcg att gtc acc ggt gcg agt ttc ttt ggc caa tgg acc gtt cct 451
Leu Ala Ile Val Thr Gly Ala Ser Phe Phe Gly Gln Trp Thr Val Pro
            105                 110                 115

ttt gcg gcc ttc gtg ggc gca ttg gtc acg gtg gct gtg gta tat ttg 499
Phe Ala Ala Phe Val Gly Ala Leu Val Thr Val Ala Val Val Tyr Leu
        120                 125                 130

atc gct agt tcc cgc gcg atg gat ggc cgt ggc gca gat ccg gcc acg 547
Ile Ala Ser Ser Arg Ala Met Asp Gly Arg Gly Ala Asp Pro Ala Thr
    135                 140                 145

ttg gta ctg gtc ggc atg gct atc act gcc ttt ttg ggt gct gtt att 595
Leu Val Leu Val Gly Met Ala Ile Thr Ala Phe Leu Gly Ala Val Ile
150                 155                 160                 165

tcc agc gcc act gcg aac gca cca caa gat tct gag ctt cga tcc gtg 643
Ser Ser Ala Thr Ala Asn Ala Pro Gln Asp Ser Glu Leu Arg Ser Val
                170                 175                 180

acg ttt tgg ctc aac ggc gat ctg gta tct cgg acg tgg gaa cat gtg 691
Thr Phe Trp Leu Asn Gly Asp Leu Val Ser Arg Thr Trp Glu His Val
            185                 190                 195

ggc gtt gca ata atc ccc att atc gtt ggg ttg att cta gct atc ggc 739
Gly Val Ala Ile Ile Pro Ile Ile Val Gly Leu Ile Leu Ala Ile Gly
        200                 205                 210

ggt tcc cgc gat ctg aac ttg ttg ctg ctg ggt gat tcc aca gcg caa 787
Gly Ser Arg Asp Leu Asn Leu Leu Leu Leu Gly Asp Ser Thr Ala Gln
    215                 220                 225

aca tct gga ctc aac gtc aac cgc gca cgc atc att ttg cta gca ctt 835
Thr Ser Gly Leu Asn Val Asn Arg Ala Arg Ile Ile Leu Leu Ala Leu
230                 235                 240                 245

gcg gca ctg ctc acc gcc aca gct gtt gcg gtc tcc ggc acc att acg 883
Ala Ala Leu Leu Thr Ala Thr Ala Val Ala Val Ser Gly Thr Ile Thr
                250                 255                 260

ttt gtt gga ttg gta gta ccc cac ctg gtg cgc att gtt tta ggt gcc 931
Phe Val Gly Leu Val Val Pro His Leu Val Arg Ile Val Leu Gly Ala
            265                 270                 275

gat cac cga gcg tta ctc ccg gcc gcc gcg att ttg ggc gcc acg ttt     979
Asp His Arg Ala Leu Leu Pro Ala Ala Ala Ile Leu Gly Ala Thr Phe
        280                 285                 290

gtt atc gtt tcc gac act gtt gcc cgc atg atc ttc tcc ccc atc gtc     1027
Val Ile Val Ser Asp Thr Val Ala Arg Met Ile Phe Ser Pro Ile Val
    295                 300                 305

ttg caa aca ggc gtg gtg gtg gcg ttc att ggc tca cca att ttc ctt     1075
Leu Gln Thr Gly Val Val Val Ala Phe Ile Gly Ser Pro Ile Phe Leu
310                 315                 320                 325

tat tta ctg ctc agc atg cgc aag cga cgc gga ttg ggg ctg             1117
Tyr Leu Leu Leu Ser Met Arg Lys Arg Arg Gly Leu Gly Leu
                330                 335

taaaaactca tgcctcaatt agt                                           1140

<210> 494
<211> 339
<212> PRT
<213> Corynebacterium glutamicum

<400> 494
Leu Ser Arg Thr Gly Val Ser Lys Lys Pro Lys Leu Thr Ala Pro Val
1               5                   10                  15

Val Ile Ile Gly Thr Leu Val Leu Leu Ile Ile Ala Phe Thr Ala Ser
            20                  25                  30

Leu Met Leu Gly Pro Val Thr Val Pro Leu Asn Glu Leu Ala Thr Asn
        35                  40                  45

Pro Val Val Thr Asp Ile Arg Ala Pro Arg Ile Ile Ile Ala Ala Leu
    50                  55                  60

Val Gly Ala Ala Leu Ala Val Ser Gly Ala Ile Met Gln Thr Val Phe
65                  70                  75                  80

His Asn Pro Leu Ala Asp Pro Gly Ile Val Gly Val Ser Ser Gly Ala
                85                  90                  95

Ala Val Ala Ala Val Leu Ala Ile Val Thr Gly Ala Ser Phe Phe Gly
            100                 105                 110

Gln Trp Thr Val Pro Phe Ala Ala Phe Val Gly Ala Leu Val Thr Val
        115                 120                 125

Ala Val Val Tyr Leu Ile Ala Ser Ser Arg Ala Met Asp Gly Arg Gly
    130                 135                 140

Ala Asp Pro Ala Thr Leu Val Leu Val Gly Met Ala Ile Thr Ala Phe
145                 150                 155                 160

Leu Gly Ala Val Ile Ser Ser Ala Thr Ala Asn Ala Pro Gln Asp Ser
                165                 170                 175

Glu Leu Arg Ser Val Thr Phe Trp Leu Asn Gly Asp Leu Val Ser Arg
            180                 185                 190

Thr Trp Glu His Val Gly Val Ala Ile Ile Pro Ile Ile Val Gly Leu
        195                 200                 205

Ile Leu Ala Ile Gly Gly Ser Arg Asp Leu Asn Leu Leu Leu Leu Gly
    210                 215                 220

Asp Ser Thr Ala Gln Thr Ser Gly Leu Asn Val Asn Arg Ala Arg Ile
225                 230                 235                 240

Ile Leu Leu Ala Leu Ala Ala Leu Leu Thr Ala Thr Ala Val Ala Val
                245                 250                 255

Ser Gly Thr Ile Thr Phe Val Gly Leu Val Val Pro His Leu Val Arg
            260                 265                 270

Ile Val Leu Gly Ala Asp His Arg Ala Leu Leu Pro Ala Ala Ala Ile
        275                 280                 285

Leu Gly Ala Thr Phe Val Ile Val Ser Asp Thr Val Ala Arg Met Ile
    290                 295                 300

Phe Ser Pro Ile Val Leu Gln Thr Gly Val Val Val Ala Phe Ile Gly
305                 310                 315                 320

Ser Pro Ile Phe Leu Tyr Leu Leu Leu Ser Met Arg Lys Arg Arg Gly
                325                 330                 335

Leu Gly Leu

<210> 495
<211> 1695
<212> DNA
<213> Corynebacterium glutamicum

<220>
<221> CDS
<222> (101)..(1672)
<223> RXA00181

<400> 495
gtatattcag acacccacgt cgattagtgt tgtagttcac agtgcttatt ttttatttgt 60

atctttgcac gtttgtcccc tacccaaaag gagaaacctc atg agc gat aac acc   115
                                            Met Ser Asp Asn Thr
                                            1               5

tgg ttc atc ata gcc atc gtt atc tat atg ttg gtg atg gtg ctc atc   163
Trp Phe Ile Ile Ala Ile Val Ile Tyr Met Leu Val Met Val Leu Ile
                10                  15                  20

ggc tat tgg agt tac cgc aag aca gaa aaa tac gac gac tac atg ctc   211
Gly Tyr Trp Ser Tyr Arg Lys Thr Glu Lys Tyr Asp Asp Tyr Met Leu
            25                  30                  35

gcc ggc cgc ggg ctc aac cct ttt gtt gcc gca atg tcc gca ggt gcc   259
Ala Gly Arg Gly Leu Asn Pro Phe Val Ala Ala Met Ser Ala Gly Ala
        40                  45                  50

tca gat atg tca ggt tgg ctg ctc atg ggt ctg ccc ggc gcg ctg ttt   307

Ser Asp Met Ser Gly Trp Leu Leu Met Gly Leu Pro Gly Ala Leu Phe
    55                  60                  65

gtc acc ggc atg tcc gag ttg tgg atc gca gtc gga ctc acc att ggt    355
Val Thr Gly Met Ser Glu Leu Trp Ile Ala Val Gly Leu Thr Ile Gly
 70                  75                  80                  85

gca tgg gcg aac tgg atg tgg gtg gca cct cgt ctt cgt tct tat tcc    403
Ala Trp Ala Asn Trp Met Trp Val Ala Pro Arg Leu Arg Ser Tyr Ser
                 90                  95                 100

gaa atc tcg gcc aac tca atc acc ctg cct tca ttc ttt gag aac cga    451
Glu Ile Ser Ala Asn Ser Ile Thr Leu Pro Ser Phe Phe Glu Asn Arg
            105                 110                 115

ctt cgc gat aaa tct cgc gcg ctt cgc atc att gca gca cta att atc    499
Leu Arg Asp Lys Ser Arg Ala Leu Arg Ile Ile Ala Ala Leu Ile Ile
        120                 125                 130

att gtg ttc ttc acc ttc tac att tct tca ggc atg gtt gct ggt gga    547
Ile Val Phe Phe Thr Phe Tyr Ile Ser Ser Gly Met Val Ala Gly Gly
    135                 140                 145

gtg tat tgg gag tcc acg ttt ggt gga gat tat cta ctc ggt atg gcc    595
Val Tyr Trp Glu Ser Thr Phe Gly Gly Asp Tyr Leu Leu Gly Met Ala
150                 155                 160                 165

att gtc gca ggt gtg aca gtg ctg tat acc ttc att gga gga ttc ctt    643
Ile Val Ala Gly Val Thr Val Leu Tyr Thr Phe Ile Gly Gly Phe Leu
                170                 175                 180

gct gtg tct tat aca gac gca gtt cag ggg act atc atg ttc ttc tcg    691
Ala Val Ser Tyr Thr Asp Ala Val Gln Gly Thr Ile Met Phe Phe Ser
            185                 190                 195

ttg atc atc gtg cca gtc atg gca tat ttc gca ctg gcg aac ccc atg    739
Leu Ile Ile Val Pro Val Met Ala Tyr Phe Ala Leu Ala Asn Pro Met
        200                 205                 210

gat att tgg agc ttt gca aac tct aat gat tac ggc ccg cac acc gat    787
Asp Ile Trp Ser Phe Ala Asn Ser Asn Asp Tyr Gly Pro His Thr Asp
    215                 220                 225

gga att ggc aat ccc acc tac ttc tcc atg atc agt ggc att tct gca    835
Gly Ile Gly Asn Pro Thr Tyr Phe Ser Met Ile Ser Gly Ile Ser Ala
230                 235                 240                 245

gca gca atc att ggt aac tta ggt tgg ggt ctt gga tac ttc ggc cag    883
Ala Ala Ile Ile Gly Asn Leu Gly Trp Gly Leu Gly Tyr Phe Gly Gln
                250                 255                 260

cca cac att gtg gtt cgt ttc atg gca ctt cgc aca cca gct gaa gca    931
Pro His Ile Val Val Arg Phe Met Ala Leu Arg Thr Pro Ala Glu Ala
            265                 270                 275

aag caa ggt cgt cgc atc ggt att tcc tgg atg ata atc tgc ctg att    979
Lys Gln Gly Arg Arg Ile Gly Ile Ser Trp Met Ile Ile Cys Leu Ile
        280                 285                 290

ggt gca acc ttt acc gca att atc tcc aca gtt ttc ttt gca caa aac    1027
Gly Ala Thr Phe Thr Ala Ile Ile Ser Thr Val Phe Phe Ala Gln Asn

295                 300                 305

ccc gac gcc aac atc act gac aca agg gct tat gag tcc atc ttc cta     1075
Pro Asp Ala Asn Ile Thr Asp Thr Arg Ala Tyr Glu Ser Ile Phe Leu
310                 315                 320                 325

gat ctt gcc cgg atg ctg ttc cac cca ttg atc gct gga ctt att ctg     1123
Asp Leu Ala Arg Met Leu Phe His Pro Leu Ile Ala Gly Leu Ile Leu
                330                 335                 340

act gct gtt ctc gcg gcc atc atg tct acc atg tcg tca cag ctg ctg     1171
Thr Ala Val Leu Ala Ala Ile Met Ser Thr Met Ser Ser Gln Leu Leu
            345                 350                 355

gtc acc gca tct tcc ctg att gag gac ctg ctg aag gta gtt aaa aag     1219
Val Thr Ala Ser Ser Leu Ile Glu Asp Leu Leu Lys Val Val Lys Lys
        360                 365                 370

gac tcg ctg agc gaa cgc acc ctg atc atg ctg tct cgt gcc aca gtc     1267
Asp Ser Leu Ser Glu Arg Thr Leu Ile Met Leu Ser Arg Ala Thr Val
    375                 380                 385

atc att ctg gcg atc att gca gca gcc atg gct att aac ccg tct gat     1315
Ile Ile Leu Ala Ile Ile Ala Ala Ala Met Ala Ile Asn Pro Ser Asp
390                 395                 400                 405

tcc atc ctc gga ttg gtg gga ttt gcg tgg gca gga ttc ggc tct gca     1363
Ser Ile Leu Gly Leu Val Gly Phe Ala Trp Ala Gly Phe Gly Ser Ala
                410                 415                 420

ttt ggt ccg atc ata ctt gct agc ctt tat tgg aag cgt ctc aac gcc     1411
Phe Gly Pro Ile Ile Leu Ala Ser Leu Tyr Trp Lys Arg Leu Asn Ala
            425                 430                 435

gcc ggc gct atc tcc ggc atg att act ggt gct att gtc tcc atc gcg     1459
Ala Gly Ala Ile Ser Gly Met Ile Thr Gly Ala Ile Val Ser Ile Ala
        440                 445                 450

tgg ggt atg tcc cca cta agc gat acg ttg tat gaa atc atc cca ggt     1507
Trp Gly Met Ser Pro Leu Ser Asp Thr Leu Tyr Glu Ile Ile Pro Gly
    455                 460                 465

ttc gcc ttg gca acc atc gtg atg gtc gtt gtt tcc ctc ctg aca aag     1555
Phe Ala Leu Ala Thr Ile Val Met Val Val Val Ser Leu Leu Thr Lys
470                 475                 480                 485

gaa ccc tcc gaa gaa atc ctc aac gag ttt gaa acc gcc aag gat ctt     1603
Glu Pro Ser Glu Glu Ile Leu Asn Glu Phe Glu Thr Ala Lys Asp Leu
                490                 495                 500

gct gcc gct gtg gaa tca aac gag gat gtt gat ttc gct gac gca gct     1651
Ala Ala Ala Val Glu Ser Asn Glu Asp Val Asp Phe Ala Asp Ala Ala
            505                 510                 515

cag aag ctt tcg aaa gaa agt taagccttag agggaaccaa acg               1695
Gln Lys Leu Ser Lys Glu Ser
        520

<210> 496
<211> 524

<212> PRT
<213> Corynebacterium glutamicum

<400> 496
Met Ser Asp Asn Thr Trp Phe Ile Ile Ala Ile Val Ile Tyr Met Leu
1               5                   10                  15

Val Met Val Leu Ile Gly Tyr Trp Ser Tyr Arg Lys Thr Glu Lys Tyr
            20                  25                  30

Asp Asp Tyr Met Leu Ala Gly Arg Gly Leu Asn Pro Phe Val Ala Ala
        35                  40                  45

Met Ser Ala Gly Ala Ser Asp Met Ser Gly Trp Leu Leu Met Gly Leu
    50                  55                  60

Pro Gly Ala Leu Phe Val Thr Gly Met Ser Glu Leu Trp Ile Ala Val
65                  70                  75                  80

Gly Leu Thr Ile Gly Ala Trp Ala Asn Trp Met Trp Val Ala Pro Arg
                85                  90                  95

Leu Arg Ser Tyr Ser Glu Ile Ser Ala Asn Ser Ile Thr Leu Pro Ser
            100                 105                 110

Phe Phe Glu Asn Arg Leu Arg Asp Lys Ser Arg Ala Leu Arg Ile Ile
        115                 120                 125

Ala Ala Leu Ile Ile Ile Val Phe Phe Thr Phe Tyr Ile Ser Ser Gly
    130                 135                 140

Met Val Ala Gly Gly Val Tyr Trp Glu Ser Thr Phe Gly Gly Asp Tyr
145                 150                 155                 160

Leu Leu Gly Met Ala Ile Val Ala Gly Val Thr Val Leu Tyr Thr Phe
                165                 170                 175

Ile Gly Gly Phe Leu Ala Val Ser Tyr Thr Asp Ala Val Gln Gly Thr
            180                 185                 190

Ile Met Phe Phe Ser Leu Ile Ile Val Pro Val Met Ala Tyr Phe Ala
        195                 200                 205

Leu Ala Asn Pro Met Asp Ile Trp Ser Phe Ala Asn Ser Asn Asp Tyr
    210                 215                 220

Gly Pro His Thr Asp Gly Ile Gly Asn Pro Thr Tyr Phe Ser Met Ile
225                 230                 235                 240

Ser Gly Ile Ser Ala Ala Ala Ile Ile Gly Asn Leu Gly Trp Gly Leu
                245                 250                 255

Gly Tyr Phe Gly Gln Pro His Ile Val Val Arg Phe Met Ala Leu Arg
            260                 265                 270

Thr Pro Ala Glu Ala Lys Gln Gly Arg Arg Ile Gly Ile Ser Trp Met
        275                 280                 285

Ile Ile Cys Leu Ile Gly Ala Thr Phe Thr Ala Ile Ile Ser Thr Val
    290                 295                 300

Phe Phe Ala Gln Asn Pro Asp Ala Asn Ile Thr Asp Thr Arg Ala Tyr
305                 310                 315                 320

Glu Ser Ile Phe Leu Asp Leu Ala Arg Met Leu Phe His Pro Leu Ile
                325                 330                 335

Ala Gly Leu Ile Leu Thr Ala Val Leu Ala Ala Ile Met Ser Thr Met
            340                 345                 350

Ser Ser Gln Leu Leu Val Thr Ala Ser Ser Leu Ile Glu Asp Leu Leu
        355                 360                 365

Lys Val Val Lys Lys Asp Ser Leu Ser Glu Arg Thr Leu Ile Met Leu
    370                 375                 380

Ser Arg Ala Thr Val Ile Ile Leu Ala Ile Ile Ala Ala Ala Met Ala
385                 390                 395                 400

Ile Asn Pro Ser Asp Ser Ile Leu Gly Leu Val Gly Phe Ala Trp Ala
                405                 410                 415

Gly Phe Gly Ser Ala Phe Gly Pro Ile Ile Leu Ala Ser Leu Tyr Trp
            420                 425                 430

Lys Arg Leu Asn Ala Ala Gly Ala Ile Ser Gly Met Ile Thr Gly Ala
        435                 440                 445

Ile Val Ser Ile Ala Trp Gly Met Ser Pro Leu Ser Asp Thr Leu Tyr
    450                 455                 460

Glu Ile Ile Pro Gly Phe Ala Leu Ala Thr Ile Val Met Val Val Val
465                 470                 475                 480

Ser Leu Leu Thr Lys Glu Pro Ser Glu Glu Ile Leu Asn Glu Phe Glu
                485                 490                 495

Thr Ala Lys Asp Leu Ala Ala Ala Val Glu Ser Asn Glu Asp Val Asp
            500                 505                 510

Phe Ala Asp Ala Ala Gln Lys Leu Ser Lys Glu Ser
        515                 520

<210> 497
<211> 1476
<212> DNA
<213> Corynebacterium glutamicum

<220>
<221> CDS
<222> (101)..(1453)
<223> RXA00591

<400> 497
gcaacgccac aggcaaaatc caaaagaaca tcctgcgaga cttcaccatc cccgtttcat 60

aaacccccca acgtcacttt gaaaacactt ggagaacgca atg act aca tct tcc   115
                                           Met Thr Thr Ser Ser
                                           1               5

aca gct tcg ccg atc gct gaa tta caa aac ctc agc ccg aag caa aga   163

Thr Ala Ser Pro Ile Ala Glu Leu Gln Asn Leu Ser Pro Lys Gln Arg
                10                  15                  20

aaa tca gaa tcc cgg cgc gcg att ata tcc agc tac ttg ggc tcc acg     211
Lys Ser Glu Ser Arg Arg Ala Ile Ile Ser Ser Tyr Leu Gly Ser Thr
            25                  30                  35

atc gag ttc tat gac ttc ctg tta tat gcc gca gcc tcc gcg acg gta     259
Ile Glu Phe Tyr Asp Phe Leu Leu Tyr Ala Ala Ala Ser Ala Thr Val
        40                  45                  50

ttc ccc gca gtg ttc ttt acc aat ctc gat ccg ctg gct gga acc att     307
Phe Pro Ala Val Phe Phe Thr Asn Leu Asp Pro Leu Ala Gly Thr Ile
    55                  60                  65

gcc gcc tac gga acc ttc gcc gca ggt tat tta gct agg cca ctt ggt     355
Ala Ala Tyr Gly Thr Phe Ala Ala Gly Tyr Leu Ala Arg Pro Leu Gly
70                  75                  80                  85

gga gca atc ttc gga cac ttc ggt gac cgt ctt gga cgc aag aag atg     403
Gly Ala Ile Phe Gly His Phe Gly Asp Arg Leu Gly Arg Lys Lys Met
                90                  95                  100

ctt gtg ttg tcc atg ctc atc atg ggt gtg gca tcc aca tgc atc ggc     451
Leu Val Leu Ser Met Leu Ile Met Gly Val Ala Ser Thr Cys Ile Gly
            105                 110                 115

ctt gtc cca agc gct gaa atg atc gga agc atg ggc gcg gta atc ctg     499
Leu Val Pro Ser Ala Glu Met Ile Gly Ser Met Gly Ala Val Ile Leu
        120                 125                 130

atc atg ctg cgc atc tgc caa ggc atc gct gtc ggt ggc gaa tgg ggc     547
Ile Met Leu Arg Ile Cys Gln Gly Ile Ala Val Gly Gly Glu Trp Gly
    135                 140                 145

gga gct gcc ctc atg gcg ctc gag cac tca gat tcc aag aag cgc gga     595
Gly Ala Ala Leu Met Ala Leu Glu His Ser Asp Ser Lys Lys Arg Gly
150                 155                 160                 165

ttc gcc gcc tcc ttc acc aac gct ggt gca cca acc ggc gct gca ctg     643
Phe Ala Ala Ser Phe Thr Asn Ala Gly Ala Pro Thr Gly Ala Ala Leu
                170                 175                 180

gga acc ttc gcg ctc ggt acc gca tct gct gtt ctc acc cag gag caa     691
Gly Thr Phe Ala Leu Gly Thr Ala Ser Ala Val Leu Thr Gln Glu Gln
            185                 190                 195

ttc ctt tca tgg ggt tgg cgc atc cca ttc ctg ctc tct ttc gtt ctg     739
Phe Leu Ser Trp Gly Trp Arg Ile Pro Phe Leu Leu Ser Phe Val Leu
        200                 205                 210

ctg att gtc ggc ttg gtt atc cgc gcg aaa gtt agc gaa tca cca ctg     787
Leu Ile Val Gly Leu Val Ile Arg Ala Lys Val Ser Glu Ser Pro Leu
    215                 220                 225

ttc gca gct gca gcg gcc gcc gaa aaa gcc aag cca acc gaa cgt aaa     835
Phe Ala Ala Ala Ala Ala Ala Glu Lys Ala Lys Pro Thr Glu Arg Lys
230                 235                 240                 245

gtc ccc ctc ttg cag gtt ctt cgc cga cca aag gca ctg atc ttg acc     883
Val Pro Leu Leu Gln Val Leu Arg Arg Pro Lys Ala Leu Ile Leu Thr

                250                 255                 260

atg ctt ggc ggc gca tca gga ttc gga ctt caa gtt ctc ttg tcc acc 931
Met Leu Gly Gly Ala Ser Gly Phe Gly Leu Gln Val Leu Leu Ser Thr
            265                 270                 275

ttc tcc atc agc tac gca aca caa tcc ggc atc gaa aga tcc agc gtc 979
Phe Ser Ile Ser Tyr Ala Thr Gln Ser Gly Ile Glu Arg Ser Ser Val
        280                 285                 290

ctc tac gcc ttc gca gtc gcc tca gtg ttc tct gtc ttc ttt gtg atc 1027
Leu Tyr Ala Phe Ala Val Ala Ser Val Phe Ser Val Phe Phe Val Ile
    295                 300                 305

ctc ttc ggt cgc gta tcc gac ctc ttc gga cgc cga ccc gtc atg atc 1075
Leu Phe Gly Arg Val Ser Asp Leu Phe Gly Arg Arg Pro Val Met Ile
310                 315                 320                 325

atc gcg ctc gta ctg ttc gtg gcc tac ctg ccg gca ttc ttc cgg atg 1123
Ile Ala Leu Val Leu Phe Val Ala Tyr Leu Pro Ala Phe Phe Arg Met
                330                 335                 340

ctc acc tca gac aac tgg ttc atc ctg ctc tcg gca ttc acc atc gcg 1171
Leu Thr Ser Asp Asn Trp Phe Ile Leu Leu Ser Ala Phe Thr Ile Ala
            345                 350                 355

ctc gca ctc cac gcc atg ctt tac ggc cca cta gca gcg ttt atc tcc 1219
Leu Ala Leu His Ala Met Leu Tyr Gly Pro Leu Ala Ala Phe Ile Ser
        360                 365                 370

gaa caa ttc gga acc tcc gcg cgc tac aca ggc gca tcc ctg ggt tac 1267
Glu Gln Phe Gly Thr Ser Ala Arg Tyr Thr Gly Ala Ser Leu Gly Tyr
    375                 380                 385

cag ttg gcc acg ctc atc ggt gca gga ttc acc cca acc atc ctg gct 1315
Gln Leu Ala Thr Leu Ile Gly Ala Gly Phe Thr Pro Thr Ile Leu Ala
390                 395                 400                 405

agc ctc tac gcg gga cca ggc ggc gga acc tct gtc acc cca gtc atc 1363
Ser Leu Tyr Ala Gly Pro Gly Gly Gly Thr Ser Val Thr Pro Val Ile
                410                 415                 420

gtc ttc ctc gca acg atg tcc cta gtg tcc atc atc gcc atc gca atc 1411
Val Phe Leu Ala Thr Met Ser Leu Val Ser Ile Ile Ala Ile Ala Ile
            425                 430                 435

acc aga gaa tca aaa gac cac gat ctt tct act tac gaa cac 1453
Thr Arg Glu Ser Lys Asp His Asp Leu Ser Thr Tyr Glu His
        440                 445                 450

taaggggacg atagggtcaa ttt 1476

<210> 498
<211> 451
<212> PRT
<213> Corynebacterium glutamicum

<400> 498
Met Thr Thr Ser Ser Thr Ala Ser Pro Ile Ala Glu Leu Gln Asn Leu
1               5                   10                  15

Ser Pro Lys Gln Arg Lys Ser Glu Ser Arg Arg Ala Ile Ile Ser Ser
            20                  25                  30

Tyr Leu Gly Ser Thr Ile Glu Phe Tyr Asp Phe Leu Leu Tyr Ala Ala
        35                  40                  45

Ala Ser Ala Thr Val Phe Pro Ala Val Phe Phe Thr Asn Leu Asp Pro
    50                  55                  60

Leu Ala Gly Thr Ile Ala Ala Tyr Gly Thr Phe Ala Ala Gly Tyr Leu
65                  70                  75                  80

Ala Arg Pro Leu Gly Gly Ala Ile Phe Gly His Phe Gly Asp Arg Leu
                85                  90                  95

Gly Arg Lys Lys Met Leu Val Leu Ser Met Leu Ile Met Gly Val Ala
            100                 105                 110

Ser Thr Cys Ile Gly Leu Val Pro Ser Ala Glu Met Ile Gly Ser Met
        115                 120                 125

Gly Ala Val Ile Leu Ile Met Leu Arg Ile Cys Gln Gly Ile Ala Val
    130                 135                 140

Gly Gly Glu Trp Gly Gly Ala Ala Leu Met Ala Leu Glu His Ser Asp
145                 150                 155                 160

Ser Lys Lys Arg Gly Phe Ala Ala Ser Phe Thr Asn Ala Gly Ala Pro
                165                 170                 175

Thr Gly Ala Ala Leu Gly Thr Phe Ala Leu Gly Thr Ala Ser Ala Val
            180                 185                 190

Leu Thr Gln Glu Gln Phe Leu Ser Trp Gly Trp Arg Ile Pro Phe Leu
        195                 200                 205

Leu Ser Phe Val Leu Leu Ile Val Gly Leu Val Ile Arg Ala Lys Val
    210                 215                 220

Ser Glu Ser Pro Leu Phe Ala Ala Ala Ala Ala Ala Glu Lys Ala Lys
225                 230                 235                 240

Pro Thr Glu Arg Lys Val Pro Leu Leu Gln Val Leu Arg Arg Pro Lys
                245                 250                 255

Ala Leu Ile Leu Thr Met Leu Gly Gly Ala Ser Gly Phe Gly Leu Gln
            260                 265                 270

Val Leu Leu Ser Thr Phe Ser Ile Ser Tyr Ala Thr Gln Ser Gly Ile
        275                 280                 285

Glu Arg Ser Ser Val Leu Tyr Ala Phe Ala Val Ala Ser Val Phe Ser
    290                 295                 300

Val Phe Phe Val Ile Leu Phe Gly Arg Val Ser Asp Leu Phe Gly Arg
305                 310                 315                 320

Arg Pro Val Met Ile Ile Ala Leu Val Leu Phe Val Ala Tyr Leu Pro
                325                 330                 335

Ala Phe Phe Arg Met Leu Thr Ser Asp Asn Trp Phe Ile Leu Leu Ser
            340                 345                 350

Ala Phe Thr Ile Ala Leu Ala Leu His Ala Met Leu Tyr Gly Pro Leu
        355                 360                 365

Ala Ala Phe Ile Ser Glu Gln Phe Gly Thr Ser Ala Arg Tyr Thr Gly
    370                 375                 380

Ala Ser Leu Gly Tyr Gln Leu Ala Thr Leu Ile Gly Ala Gly Phe Thr
385                 390                 395                 400

Pro Thr Ile Leu Ala Ser Leu Tyr Ala Gly Pro Gly Gly Gly Thr Ser
                405                 410                 415

Val Thr Pro Val Ile Val Phe Leu Ala Thr Met Ser Leu Val Ser Ile
            420                 425                 430

Ile Ala Ile Ala Ile Thr Arg Glu Ser Lys Asp His Asp Leu Ser Thr
        435                 440                 445

Tyr Glu His
    450

<210> 499
<211> 1635
<212> DNA
<213> Corynebacterium glutamicum

<220>
<221> CDS
<222> (101)..(1612)
<223> RXA01629

<400> 499
caggaagaag ttaacaccgc ccaggggtgc gttggatgat gatcatctac aaacaaacat 60

tccgttatgc actcataaga tatgacgaga ggttttactc gtg agc ccg att cgc   115
                                           Val Ser Pro Ile Arg
                                           1               5

tca aaa aag aaa atc aag aac gaa cca aga cta aca gtc gat gac gtc   163
Ser Lys Lys Lys Ile Lys Asn Glu Pro Arg Leu Thr Val Asp Asp Val
                10                  15                  20

aac gtt gtt ccc cca aag aag atc cgt ccg gcc att aaa ggc act gtg   211
Asn Val Val Pro Pro Lys Lys Ile Arg Pro Ala Ile Lys Gly Thr Val
            25                  30                  35

gtg ggt aac ttc atg gag tgg tac gac ttc gga att tat ggt tat ttg   259
Val Gly Asn Phe Met Glu Trp Tyr Asp Phe Gly Ile Tyr Gly Tyr Leu
        40                  45                  50

acg gtc acg atg acc gca gta ttt acc caa ggc ctg ccg caa gaa tgg   307
Thr Val Thr Met Thr Ala Val Phe Thr Gln Gly Leu Pro Gln Glu Trp
    55                  60                  65

cag ttg ttg gcc gtg atg ttt ggt ttc gcg gtg tct tat tta gtt cgc   355
Gln Leu Leu Ala Val Met Phe Gly Phe Ala Val Ser Tyr Leu Val Arg
70                  75                  80                  85

ccg ctt ggt ggc ctg gtt ctg ggt ccg ctt ggc gat aag gtt ggc cgc 403
Pro Leu Gly Gly Leu Val Leu Gly Pro Leu Gly Asp Lys Val Gly Arg
                90                  95                  100

cag aag gtt ttg tac gta acc atg gcc atg atg gcc gtg tct acg gcg 451
Gln Lys Val Leu Tyr Val Thr Met Ala Met Met Ala Val Ser Thr Ala
            105                 110                 115

ctg att ggc ttg ctg ccg acg gcg gcg tcg ata ggc gct tgg gct ttg 499
Leu Ile Gly Leu Leu Pro Thr Ala Ala Ser Ile Gly Ala Trp Ala Leu
        120                 125                 130

gtg ctt ttg tac ctg ttg aag atg gtg cag gga ttt tcg act ggt ggt 547
Val Leu Leu Tyr Leu Leu Lys Met Val Gln Gly Phe Ser Thr Gly Gly
    135                 140                 145

gaa tat gct ggt gcg acc acc tat gtt gct gag ttt gcg ccg gat cgt 595
Glu Tyr Ala Gly Ala Thr Thr Tyr Val Ala Glu Phe Ala Pro Asp Arg
150                 155                 160                 165

cga cga ggt ttc ttc gga gct ttc cta gat atg ggt tcc tac ctg ggc 643
Arg Arg Gly Phe Phe Gly Ala Phe Leu Asp Met Gly Ser Tyr Leu Gly
                170                 175                 180

ttt gcc gcg ggt gct tcg gtc gtg gcg att act acc tgg gtg acc act 691
Phe Ala Ala Gly Ala Ser Val Val Ala Ile Thr Thr Trp Val Thr Thr
            185                 190                 195

cac ttc tat ggc gca act gcc atg gag gat ttc ggt tgg cgt att cct 739
His Phe Tyr Gly Ala Thr Ala Met Glu Asp Phe Gly Trp Arg Ile Pro
        200                 205                 210

ttc ctg act gcg atc ccg ctg ggc atc att gcg gtg tac ctg cgt act 787
Phe Leu Thr Ala Ile Pro Leu Gly Ile Ile Ala Val Tyr Leu Arg Thr
    215                 220                 225

cgt atc cct gag acc cct gcg ttt gag aac aac caa gac gag cca aat 835
Arg Ile Pro Glu Thr Pro Ala Phe Glu Asn Asn Gln Asp Glu Pro Asn
230                 235                 240                 245

gca gtt gtt gaa aag gac act gaa gat cct tat gca cgc ctg ggc ctg 883
Ala Val Val Glu Lys Asp Thr Glu Asp Pro Tyr Ala Arg Leu Gly Leu
                250                 255                 260

gct ggt gtt atc cgc cac cac tgg cgt cca ctg ctg att ggt att gcg 931
Ala Gly Val Ile Arg His His Trp Arg Pro Leu Leu Ile Gly Ile Ala
            265                 270                 275

att gtg gca gcg acc aac acc gcc ggt tac gcg cta acc agt tac atg 979
Ile Val Ala Ala Thr Asn Thr Ala Gly Tyr Ala Leu Thr Ser Tyr Met
        280                 285                 290

cct gtg tat cta gag gag cag atc ggt ctg cac tcc gca tcc gct gcc 1027
Pro Val Tyr Leu Glu Glu Gln Ile Gly Leu His Ser Ala Ser Ala Ala
    295                 300                 305

gct gtg acc gtg ccg att ctg gtt gtt atg tcc ctg ctg ctg cca ttt 1075
Ala Val Thr Val Pro Ile Leu Val Val Met Ser Leu Leu Leu Pro Phe
310                 315                 320                 325

gtt ggt atg tgg agt gac cgc gtg ggc cgc aag cct gtc tac gcg act 1123
Val Gly Met Trp Ser Asp Arg Val Gly Arg Lys Pro Val Tyr Ala Thr
                330                 335                 340

gct gtt gcg gca acg ctg atc ttg atg gtt cct gcc ttc ttg atc atg 1171
Ala Val Ala Ala Thr Leu Ile Leu Met Val Pro Ala Phe Leu Ile Met
            345                 350                 355

aac acc gga acc atc ggc gcc gta ctg att gca ctg tcc atg gtt gct 1219
Asn Thr Gly Thr Ile Gly Ala Val Leu Ile Ala Leu Ser Met Val Ala
        360                 365                 370

att cca acc ggt ttg tat gtg gca ctg tcc gca tct gcc ctg cca gcg 1267
Ile Pro Thr Gly Leu Tyr Val Ala Leu Ser Ala Ser Ala Leu Pro Ala
    375                 380                 385

ctg ttc cca acc gcg tca cga ttc tct gga atg ggt att tcc tac aac 1315
Leu Phe Pro Thr Ala Ser Arg Phe Ser Gly Met Gly Ile Ser Tyr Asn
390                 395                 400                 405

att tcc gtg tcg ttg ttc ggt ggt aca acc ccg ctg atc acc cag ttc 1363
Ile Ser Val Ser Leu Phe Gly Gly Thr Thr Pro Leu Ile Thr Gln Phe
                410                 415                 420

ctg ctg cag aag act ggc ctg gat atc gtt cca gcg ctc tac atc atg 1411
Leu Leu Gln Lys Thr Gly Leu Asp Ile Val Pro Ala Leu Tyr Ile Met
            425                 430                 435

ttc ttc tct gca atc gca ggt gtg gcg ctg ctg ttc atg acc gag tct 1459
Phe Phe Ser Ala Ile Ala Gly Val Ala Leu Leu Phe Met Thr Glu Ser
        440                 445                 450

tca cag aag cca ctg ctt ggc tca ttc cca acc gtg gaa acc aag tct 1507
Ser Gln Lys Pro Leu Leu Gly Ser Phe Pro Thr Val Glu Thr Lys Ser
    455                 460                 465

gag gcc gtg gag atc gtg aag aac cag gac gag gat cca aat att gat 1555
Glu Ala Val Glu Ile Val Lys Asn Gln Asp Glu Asp Pro Asn Ile Asp
470                 475                 480                 485

ctt tcc cat atg ccg ttt cct gat gag gaa aac gta ggt gct gaa aag 1603
Leu Ser His Met Pro Phe Pro Asp Glu Glu Asn Val Gly Ala Glu Lys
                490                 495                 500

caa aac gca taaccgtaaa gcccgctgca agg 1635
Gln Asn Ala

<210> 500
<211> 504
<212> PRT
<213> Corynebacterium glutamicum

<400> 500
Val Ser Pro Ile Arg Ser Lys Lys Lys Ile Lys Asn Glu Pro Arg Leu
1               5                   10                  15

Thr Val Asp Asp Val Asn Val Val Pro Pro Lys Lys Ile Arg Pro Ala
            20                  25                  30

Ile Lys Gly Thr Val Val Gly Asn Phe Met Glu Trp Tyr Asp Phe Gly
        35                  40                  45

Ile Tyr Gly Tyr Leu Thr Val Thr Met Thr Ala Val Phe Thr Gln Gly
    50                  55                  60

Leu Pro Gln Glu Trp Gln Leu Leu Ala Val Met Phe Gly Phe Ala Val
65                  70                  75                  80

Ser Tyr Leu Val Arg Pro Leu Gly Gly Leu Val Leu Gly Pro Leu Gly
                85                  90                  95

Asp Lys Val Gly Arg Gln Lys Val Leu Tyr Val Thr Met Ala Met Met
            100                 105                 110

Ala Val Ser Thr Ala Leu Ile Gly Leu Leu Pro Thr Ala Ala Ser Ile
        115                 120                 125

Gly Ala Trp Ala Leu Val Leu Leu Tyr Leu Leu Lys Met Val Gln Gly
    130                 135                 140

Phe Ser Thr Gly Gly Glu Tyr Ala Gly Ala Thr Thr Tyr Val Ala Glu
145                 150                 155                 160

Phe Ala Pro Asp Arg Arg Arg Gly Phe Phe Gly Ala Phe Leu Asp Met
                165                 170                 175

Gly Ser Tyr Leu Gly Phe Ala Ala Gly Ala Ser Val Val Ala Ile Thr
            180                 185                 190

Thr Trp Val Thr Thr His Phe Tyr Gly Ala Thr Ala Met Glu Asp Phe
        195                 200                 205

Gly Trp Arg Ile Pro Phe Leu Thr Ala Ile Pro Leu Gly Ile Ile Ala
    210                 215                 220

Val Tyr Leu Arg Thr Arg Ile Pro Glu Thr Pro Ala Phe Glu Asn Asn
225                 230                 235                 240

Gln Asp Glu Pro Asn Ala Val Val Glu Lys Asp Thr Glu Asp Pro Tyr
                245                 250                 255

Ala Arg Leu Gly Leu Ala Gly Val Ile Arg His His Trp Arg Pro Leu
            260                 265                 270

Leu Ile Gly Ile Ala Ile Val Ala Ala Thr Asn Thr Ala Gly Tyr Ala
        275                 280                 285

Leu Thr Ser Tyr Met Pro Val Tyr Leu Glu Glu Gln Ile Gly Leu His
    290                 295                 300

Ser Ala Ser Ala Ala Ala Val Thr Val Pro Ile Leu Val Val Met Ser
305                 310                 315                 320

Leu Leu Leu Pro Phe Val Gly Met Trp Ser Asp Arg Val Gly Arg Lys
                325                 330                 335

Pro Val Tyr Ala Thr Ala Val Ala Ala Thr Leu Ile Leu Met Val Pro
            340                 345                 350

Ala Phe Leu Ile Met Asn Thr Gly Thr Ile Gly Ala Val Leu Ile Ala

355                 360                 365

Leu Ser Met Val Ala Ile Pro Thr Gly Leu Tyr Val Ala Leu Ser Ala
    370                 375                 380

Ser Ala Leu Pro Ala Leu Phe Pro Thr Ala Ser Arg Phe Ser Gly Met
385                 390                 395                 400

Gly Ile Ser Tyr Asn Ile Ser Val Ser Leu Phe Gly Gly Thr Thr Pro
                405                 410                 415

Leu Ile Thr Gln Phe Leu Leu Gln Lys Thr Gly Leu Asp Ile Val Pro
            420                 425                 430

Ala Leu Tyr Ile Met Phe Phe Ser Ala Ile Ala Gly Val Ala Leu Leu
        435                 440                 445

Phe Met Thr Glu Ser Ser Gln Lys Pro Leu Leu Gly Ser Phe Pro Thr
    450                 455                 460

Val Glu Thr Lys Ser Glu Ala Val Glu Ile Val Lys Asn Gln Asp Glu
465                 470                 475                 480

Asp Pro Asn Ile Asp Leu Ser His Met Pro Phe Pro Asp Glu Glu Asn
                485                 490                 495

Val Gly Ala Glu Lys Gln Asn Ala
            500

<210> 501
<211> 1509
<212> DNA
<213> Corynebacterium glutamicum

<220>
<221> CDS
<222> (101)..(1486)
<223> RXA02030

<400> 501
gcgcctaaca tctcattgta tgggattgac tctaataatc tcctgtggaa acatcgcaag 60

ggtaattatt ggaatctatt attgtgtgag gcgataactc gtg acc aca act gat    115
                                           Val Thr Thr Thr Asp
                                           1               5

cac tcc acg gag ttg aat cct tct gat cca ggt ggg cag acg gca acc    163
His Ser Thr Glu Leu Asn Pro Ser Asp Pro Gly Gly Gln Thr Ala Thr
                10                  15                  20

cta gtg att gac aag aaa act aaa cgt cgg gtt gca gca gcc tcc acc    211
Leu Val Ile Asp Lys Lys Thr Lys Arg Arg Val Ala Ala Ala Ser Thr
            25                  30                  35

atc ggc aca acc atc gag ttc tac gat ttt tat gcg tac gct gcg gca    259
Ile Gly Thr Thr Ile Glu Phe Tyr Asp Phe Tyr Ala Tyr Ala Ala Ala
        40                  45                  50

gct gtc gtt gtt ttc cca agt ctg ttt ttc cct gcc aat gac aac cca    307
Ala Val Val Val Phe Pro Ser Leu Phe Phe Pro Ala Asn Asp Asn Pro

    55                  60                  65

acc gtg aac ctg ttg gca tcg ttt gcc acc ttt ggt ctt gcg ttc gtt 355
Thr Val Asn Leu Leu Ala Ser Phe Ala Thr Phe Gly Leu Ala Phe Val
70                  75                  80                  85

gct cgt cca ctc ggt tca atc att ttc ggg cac ttc ggt gac cgt gtg 403
Ala Arg Pro Leu Gly Ser Ile Ile Phe Gly His Phe Gly Asp Arg Val
                90                  95                  100

gga cgc aaa gct acc ttg atc ggt tca ctg ctg acc atg ggc att gcc 451
Gly Arg Lys Ala Thr Leu Ile Gly Ser Leu Leu Thr Met Gly Ile Ala
            105                 110                 115

acc atc ttg atc ggc ctg ctg cct act tat ggt cag gtc gga atc att 499
Thr Ile Leu Ile Gly Leu Leu Pro Thr Tyr Gly Gln Val Gly Ile Ile
        120                 125                 130

gca ccg gcg ctg ttg gcg ctc atg cgt ttc tgc cag ggc ctg ggc ctt 547
Ala Pro Ala Leu Leu Ala Leu Met Arg Phe Cys Gln Gly Leu Gly Leu
    135                 140                 145

ggc ggt gaa tgg tct ggc gct gcg ttg ctg gct ggt gaa aac gcg gaa 595
Gly Gly Glu Trp Ser Gly Ala Ala Leu Leu Ala Gly Glu Asn Ala Glu
150                 155                 160                 165

aac act cac cgc gct cgt gct gca atg tgg cca cag ctg ggt gca ccg 643
Asn Thr His Arg Ala Arg Ala Ala Met Trp Pro Gln Leu Gly Ala Pro
                170                 175                 180

ttt ggt ttc ttc ttg gcc aat ggt ttc atg ctc att ctg gtc ggt gtg 691
Phe Gly Phe Phe Leu Ala Asn Gly Phe Met Leu Ile Leu Val Gly Val
            185                 190                 195

ctt gcc cat cag gac ggc gat ctt cac ggc gcg ttc atg acc tgg ggt 739
Leu Ala His Gln Asp Gly Asp Leu His Gly Ala Phe Met Thr Trp Gly
        200                 205                 210

tgg cgt ctg cca ttc ctg tct tcg gcg atc atg atc gca gtt ggc ctg 787
Trp Arg Leu Pro Phe Leu Ser Ser Ala Ile Met Ile Ala Val Gly Leu
    215                 220                 225

tgg gtg cgt ttc tca ctg gag gaa act cca gtg ttc aag cag gct gtt 835
Trp Val Arg Phe Ser Leu Glu Glu Thr Pro Val Phe Lys Gln Ala Val
230                 235                 240                 245

gat cag ggc aag aag gtg aag tct ccg ctc aag gag ctg ttc aag act 883
Asp Gln Gly Lys Lys Val Lys Ser Pro Leu Lys Glu Leu Phe Lys Thr
                250                 255                 260

tcc cct ggc cct gtt gtg cag gct act ttg atc atg ctg tcc act tat 931
Ser Pro Gly Pro Val Val Gln Ala Thr Leu Ile Met Leu Ser Thr Tyr
            265                 270                 275

acc ttg ttc tac ctg gtt acc acg tgg att ctg tcg tat ggc atc ggt 979
Thr Leu Phe Tyr Leu Val Thr Thr Trp Ile Leu Ser Tyr Gly Ile Gly
        280                 285                 290

aat cgc agc acc gga aac ggc ctg tct att ccg tac ttt gag ttc ttg 1027
Asn Arg Ser Thr Gly Asn Gly Leu Ser Ile Pro Tyr Phe Glu Phe Leu
    295                 300                 305

cag ttg cag ctg gcc acc atc gtg ttc ttc gcc atc atg atc cct gtg 1075
Gln Leu Gln Leu Ala Thr Ile Val Phe Phe Ala Ile Met Ile Pro Val
310                 315                 320                 325

tca ggc tgg ttg gct gat gtg tgg ggt cgt aaa aac acc ctg acc ttg 1123
Ser Gly Trp Leu Ala Asp Val Trp Gly Arg Lys Asn Thr Leu Thr Leu
                330                 335                 340

gct tct gtg ctg ctt ctc ggc ttt ggt ctg acg ttt aat ctg ctt ctc 1171
Ala Ser Val Leu Leu Leu Gly Phe Gly Leu Thr Phe Asn Leu Leu Leu
            345                 350                 355

gat cca gag acc gcc acc aag acc acc gtg ttc atc ttc ttg ttc gtg 1219
Asp Pro Glu Thr Ala Thr Lys Thr Thr Val Phe Ile Phe Leu Phe Val
        360                 365                 370

ggc atg agc atc atg ggt ctg atc ttt gga ccc atg tcg gca att ttg 1267
Gly Met Ser Ile Met Gly Leu Ile Phe Gly Pro Met Ser Ala Ile Leu
    375                 380                 385

ccg gag ctc ttc ccc acc aac gtc cgc tac acg ggc tcc gga atc gcc 1315
Pro Glu Leu Phe Pro Thr Asn Val Arg Tyr Thr Gly Ser Gly Ile Ala
390                 395                 400                 405

tac aat gtc tcc tcg atc ctc ggt gca gct att gca ccg ttc atc gca 1363
Tyr Asn Val Ser Ser Ile Leu Gly Ala Ala Ile Ala Pro Phe Ile Ala
                410                 415                 420

acg tgg ctg gtg tcc gag ttt tca gtg gcg tac gtc ggc tat tac ctc 1411
Thr Trp Leu Val Ser Glu Phe Ser Val Ala Tyr Val Gly Tyr Tyr Leu
            425                 430                 435

atc atc gtc acc gca att acc ttt gtt gcg gtg ctg acg atg aag gaa 1459
Ile Ile Val Thr Ala Ile Thr Phe Val Ala Val Leu Thr Met Lys Glu
        440                 445                 450

aac aaa aac cac gac ctc cga gag gtc taaaagattt ttgcttatcg 1506
Asn Lys Asn His Asp Leu Arg Glu Val
    455                 460

acg 1509

<210> 502
<211> 462
<212> PRT
<213> Corynebacterium glutamicum

<400> 502
Val Thr Thr Thr Asp His Ser Thr Glu Leu Asn Pro Ser Asp Pro Gly
1               5                   10                  15

Gly Gln Thr Ala Thr Leu Val Ile Asp Lys Lys Thr Lys Arg Arg Val
            20                  25                  30

Ala Ala Ala Ser Thr Ile Gly Thr Thr Ile Glu Phe Tyr Asp Phe Tyr
        35                  40                  45

Ala Tyr Ala Ala Ala Ala Val Val Val Phe Pro Ser Leu Phe Phe Pro
    50                  55                  60

Ala Asn Asp Asn Pro Thr Val Asn Leu Leu Ala Ser Phe Ala Thr Phe
65                  70                  75                  80

Gly Leu Ala Phe Val Ala Arg Pro Leu Gly Ser Ile Ile Phe Gly His
                85                  90                  95

Phe Gly Asp Arg Val Gly Arg Lys Ala Thr Leu Ile Gly Ser Leu Leu
            100                 105                 110

Thr Met Gly Ile Ala Thr Ile Leu Ile Gly Leu Leu Pro Thr Tyr Gly
        115                 120                 125

Gln Val Gly Ile Ile Ala Pro Ala Leu Leu Ala Leu Met Arg Phe Cys
    130                 135                 140

Gln Gly Leu Gly Leu Gly Gly Glu Trp Ser Gly Ala Ala Leu Leu Ala
145                 150                 155                 160

Gly Glu Asn Ala Glu Asn Thr His Arg Ala Arg Ala Ala Met Trp Pro
                165                 170                 175

Gln Leu Gly Ala Pro Phe Gly Phe Phe Leu Ala Asn Gly Phe Met Leu
            180                 185                 190

Ile Leu Val Gly Val Leu Ala His Gln Asp Gly Asp Leu His Gly Ala
        195                 200                 205

Phe Met Thr Trp Gly Trp Arg Leu Pro Phe Leu Ser Ser Ala Ile Met
    210                 215                 220

Ile Ala Val Gly Leu Trp Val Arg Phe Ser Leu Glu Glu Thr Pro Val
225                 230                 235                 240

Phe Lys Gln Ala Val Asp Gln Gly Lys Lys Val Lys Ser Pro Leu Lys
                245                 250                 255

Glu Leu Phe Lys Thr Ser Pro Gly Pro Val Val Gln Ala Thr Leu Ile
            260                 265                 270

Met Leu Ser Thr Tyr Thr Leu Phe Tyr Leu Val Thr Thr Trp Ile Leu
        275                 280                 285

Ser Tyr Gly Ile Gly Asn Arg Ser Thr Gly Asn Gly Leu Ser Ile Pro
    290                 295                 300

Tyr Phe Glu Phe Leu Gln Leu Gln Leu Ala Thr Ile Val Phe Phe Ala
305                 310                 315                 320

Ile Met Ile Pro Val Ser Gly Trp Leu Ala Asp Val Trp Gly Arg Lys
                325                 330                 335

Asn Thr Leu Thr Leu Ala Ser Val Leu Leu Leu Gly Phe Gly Leu Thr
            340                 345                 350

Phe Asn Leu Leu Leu Asp Pro Glu Thr Ala Thr Lys Thr Thr Val Phe
        355                 360                 365

Ile Phe Leu Phe Val Gly Met Ser Ile Met Gly Leu Ile Phe Gly Pro
    370                 375                 380

Met Ser Ala Ile Leu Pro Glu Leu Phe Pro Thr Asn Val Arg Tyr Thr
385                 390                 395                 400

Gly Ser Gly Ile Ala Tyr Asn Val Ser Ser Ile Leu Gly Ala Ala Ile
                405                 410                 415

Ala Pro Phe Ile Ala Thr Trp Leu Val Ser Glu Phe Ser Val Ala Tyr
            420                 425                 430

Val Gly Tyr Tyr Leu Ile Ile Val Thr Ala Ile Thr Phe Val Ala Val
        435                 440                 445

Leu Thr Met Lys Glu Asn Lys Asn His Asp Leu Arg Glu Val
    450                 455                 460

<210> 503
<211> 870
<212> DNA
<213> Corynebacterium glutamicum

<220>
<221> CDS
<222> (101)..(847)
<223> RXA00186

<400> 503
agaagcccag cattttgttt agctcacctc taaagctcct gatctggata cgaggactcc 60

tgcggaaaac cgtgtggcta gtgaagctac ccggcatctc atg ggg gag aag act    115
                                            Met Gly Glu Lys Thr
                                            1               5

tct gtc gcg tat gtt cac gat gtt ctg atc aag ggc gtg cca gtg cgg    163
Ser Val Ala Tyr Val His Asp Val Leu Ile Lys Gly Val Pro Val Arg
                10                  15                  20

ata tac aat ccg cac ccc aac gac ggg ccg ctt cct gtc ttt atc tac    211
Ile Tyr Asn Pro His Pro Asn Asp Gly Pro Leu Pro Val Phe Ile Tyr
            25                  30                  35

ttc cat ggt ggc gga tgg gtt ttg ggg gac ctt gaa tca gtg gat tcc    259
Phe His Gly Gly Gly Trp Val Leu Gly Asp Leu Glu Ser Val Asp Ser
        40                  45                  50

acc gtg cgc gat att gcc gtt gct tca ggc gcc ctg tgt atc agt gtg    307
Thr Val Arg Asp Ile Ala Val Ala Ser Gly Ala Leu Cys Ile Ser Val
    55                  60                  65

gat tat cgc cgt gca ccg gaa cat cct ttt ctc gct gca ttg gat gat    355
Asp Tyr Arg Arg Ala Pro Glu His Pro Phe Leu Ala Ala Leu Asp Asp
70                  75                  80                  85

tgc caa gtg gtc acg gaa gct gtc ctc aat ggt gaa ctc gcc agt gct    403
Cys Gln Val Val Thr Glu Ala Val Leu Asn Gly Glu Leu Ala Ser Ala
                90                  95                  100

aac cag cat cta gtg gca gtc ggc ggg gac agc gcc ggt ggc aat att    451
Asn Gln His Leu Val Ala Val Gly Gly Asp Ser Ala Gly Gly Asn Ile
            105                 110                 115

gcg gcg gtc atc gct caa cag ctg cgc gat cag atc acc cac caa gtt     499
Ala Ala Val Ile Ala Gln Gln Leu Arg Asp Gln Ile Thr His Gln Val
        120                 125                 130

ctt gtg tat ccg gtg atg gac gta atc ttg ctg gtg att tgt tct atc     547
Leu Val Tyr Pro Val Met Asp Val Ile Leu Leu Val Ile Cys Ser Ile
    135                 140                 145

att acc act ggt ttt gcg ctg ctc atg cat cca aag agc aag gac aag     595
Ile Thr Thr Gly Phe Ala Leu Leu Met His Pro Lys Ser Lys Asp Lys
150                 155                 160                 165

acg gag acg att tct gat gaa ttc ctc gca gag atc caa gcg gga agt     643
Thr Glu Thr Ile Ser Asp Glu Phe Leu Ala Glu Ile Gln Ala Gly Ser
                170                 175                 180

gaa aag atc tcc att ctg cgt tcg acc cct gct gaa aaa gcc aat gcg     691
Glu Lys Ile Ser Ile Leu Arg Ser Thr Pro Ala Glu Lys Ala Asn Ala
            185                 190                 195

agc cgt tgg atc atg tac ttc gtt ggc gga att ggc ctg ctc tac agc     739
Ser Arg Trp Ile Met Tyr Phe Val Gly Gly Ile Gly Leu Leu Tyr Ser
        200                 205                 210

gtg ttc agc ctg tgg aca ggt ggc gta acc gga ctg acg ttg aac tcc     787
Val Phe Ser Leu Trp Thr Gly Gly Val Thr Gly Leu Thr Leu Asn Ser
    215                 220                 225

ttc aac ttc ctg ttc ctg tca ctc ggc atg gtg ctc acc gct aat tac     835
Phe Asn Phe Leu Phe Leu Ser Leu Gly Met Val Leu Thr Ala Asn Tyr
230                 235                 240                 245

ggg cca gag tat taagcaaagc ttattcgcga agg                           870
Gly Pro Glu Tyr

<210> 504
<211> 249
<212> PRT
<213> Corynebacterium glutamicum

<400> 504
Met Gly Glu Lys Thr Ser Val Ala Tyr Val His Asp Val Leu Ile Lys
  1               5                  10                  15

Gly Val Pro Val Arg Ile Tyr Asn Pro His Pro Asn Asp Gly Pro Leu
             20                  25                  30

Pro Val Phe Ile Tyr Phe His Gly Gly Gly Trp Val Leu Gly Asp Leu
         35                  40                  45

Glu Ser Val Asp Ser Thr Val Arg Asp Ile Ala Val Ala Ser Gly Ala
     50                  55                  60

Leu Cys Ile Ser Val Asp Tyr Arg Arg Ala Pro Glu His Pro Phe Leu
 65                  70                  75                  80

Ala Ala Leu Asp Asp Cys Gln Val Val Thr Glu Ala Val Leu Asn Gly
                 85                  90                  95

Glu Leu Ala Ser Ala Asn Gln His Leu Val Ala Val Gly Gly Asp Ser
            100                 105                 110

Ala Gly Gly Asn Ile Ala Ala Val Ile Ala Gln Gln Leu Arg Asp Gln
        115                 120                 125

Ile Thr His Gln Val Leu Val Tyr Pro Val Met Asp Val Ile Leu Leu
    130                 135                 140

Val Ile Cys Ser Ile Ile Thr Thr Gly Phe Ala Leu Leu Met His Pro
145                 150                 155                 160

Lys Ser Lys Asp Lys Thr Glu Thr Ile Ser Asp Glu Phe Leu Ala Glu
                165                 170                 175

Ile Gln Ala Gly Ser Glu Lys Ile Ser Ile Leu Arg Ser Thr Pro Ala
            180                 185                 190

Glu Lys Ala Asn Ala Ser Arg Trp Ile Met Tyr Phe Val Gly Gly Ile
        195                 200                 205

Gly Leu Leu Tyr Ser Val Phe Ser Leu Trp Thr Gly Gly Val Thr Gly
    210                 215                 220

Leu Thr Leu Asn Ser Phe Asn Phe Leu Phe Leu Ser Leu Gly Met Val
225                 230                 235                 240

Leu Thr Ala Asn Tyr Gly Pro Glu Tyr
                245

<210> 505
<211> 468
<212> DNA
<213> Corynebacterium glutamicum

<220>
<221> CDS
<222> (101)..(451)
<223> RXA00187

<400> 505
   gcttattcgc gaaggcatcc aaggaacgtg gggattcatt cttcaattcc cgttctacgc 60
   aggcattttc gggttgattt ccttcaccgg tctgggtgtt gtg atc tcc gga ttt 115
                                               Val Ile Ser Gly Phe
                                               1               5
   ttc acc tcg att tcc acg gca acc acg tgg cct gtc atc gcg ttc ctc   163
   Phe Thr Ser Ile Ser Thr Ala Thr Thr Trp Pro Val Ile Ala Phe Leu
                   10                  15                  20
   tac tct gga ctg ctc aac atc gcg gtg cct tca ggt ggc tcg aaa ttc   211
   Tyr Ser Gly Leu Leu Asn Ile Ala Val Pro Ser Gly Gly Ser Lys Phe
               25                  30                  35
   atc atc gaa gcc ccg tac atc atc cca acc cca gtg gat ctt ggc gct   259
   Ile Ile Glu Ala Pro Tyr Ile Ile Pro Thr Pro Val Asp Leu Gly Ala
           40                  45                  50
   gac atg ggc ctt gtc ctg cag gct tat caa atg agt gat ggc gcg acc   307
   Asp Met Gly Leu Val Leu Gln Ala Tyr Gln Met Ser Asp Gly Ala Thr
        55                  60                  65
   aac ctg ctc att ccg ttc ttc gcg ctg cca tat ttg gcc aat ttc aag     355
   Asn Leu Leu Ile Pro Phe Phe Ala Leu Pro Tyr Leu Ala Asn Phe Lys
    70                  75                  80                  85
   atc aaa ttc agc caa gtg gtg ggc tat aca gtt ccg cct gtc ctc gtt     403
   Ile Lys Phe Ser Gln Val Val Gly Tyr Thr Val Pro Pro Val Leu Val
                    90                  95                 100
   gtt atc gcc gtg atc tgc att tac ctg ttc ctg cga gca tca atc att     451
   Val Ile Ala Val Ile Cys Ile Tyr Leu Phe Leu Arg Ala Ser Ile Ile
               105                 110                 115
   taaaaagatg cttctcg                                                  468

<210> 506
<211> 117
<212> PRT
<213> Corynebacterium glutamicum

<400> 506
Val Ile Ser Gly Phe Phe Thr Ser Ile Ser Thr Ala Thr Thr Trp Pro
  1               5                  10                  15

Val Ile Ala Phe Leu Tyr Ser Gly Leu Leu Asn Ile Ala Val Pro Ser
             20                  25                  30

Gly Gly Ser Lys Phe Ile Ile Glu Ala Pro Tyr Ile Ile Pro Thr Pro
         35                  40                  45

Val Asp Leu Gly Ala Asp Met Gly Leu Val Leu Gln Ala Tyr Gln Met
     50                  55                  60

Ser Asp Gly Ala Thr Asn Leu Leu Ile Pro Phe Phe Ala Leu Pro Tyr
 65                  70                  75                  80

Leu Ala Asn Phe Lys Ile Lys Phe Ser Gln Val Val Gly Tyr Thr Val
                 85                  90                  95

Pro Pro Val Leu Val Val Ile Ala Val Ile Cys Ile Tyr Leu Phe Leu
            100                 105                 110

Arg Ala Ser Ile Ile
        115

<210> 507
<211> 1329
<212> DNA
<213> Corynebacterium glutamicum

<220>
<221> CDS
<222> (101)..(1306)
<223> RXA01667

<400> 507
tctcggcgtt gatgatcatc cgcaatatca tgccgccgca agattccgct tttccagact 60

ttgctcattc cttcctctat tactgctcgc catcatcggc atg att ttg ggg cct 115
Met Ile Leu Gly Pro
1               5

aag gtg ttg ggt ctg att ggt tgg tcg gat cat ctt tcc act tac act 163
Lys Val Leu Gly Leu Ile Gly Trp Ser Asp His Leu Ser Thr Tyr Thr
                10                  15                  20

acg gtg ctc att gcg att gtg ttt gct tcg atg ccg tat tcg atg aag 211
Thr Val Leu Ile Ala Ile Val Phe Ala Ser Met Pro Tyr Ser Met Lys
            25                  30                  35

ttt gac cgt ggc gtg cgt act ggc atg aag acg atg tgg gcg tat tcg 259
Phe Asp Arg Gly Val Arg Thr Gly Met Lys Thr Met Trp Ala Tyr Ser
        40                  45                  50

act gcg atg ttt gtg ggg cag tgg ggt ctg ttc att ttg ctg ggt ctg 307
Thr Ala Met Phe Val Gly Gln Trp Gly Leu Phe Ile Leu Leu Gly Leu
    55                  60                  65

ttt ttg ttc caa ccg gtg tgg ggt acc gat gag tgg ttc ggc atg atg 355
Phe Leu Phe Gln Pro Val Trp Gly Thr Asp Glu Trp Phe Gly Met Met
70                  75                  80                  85

ttg ccg gtt ggt ttt gtt ggt ggt ttt ggt act gct gct gcg gtg ggt 403
Leu Pro Val Gly Phe Val Gly Gly Phe Gly Thr Ala Ala Ala Val Gly
                90                  95                  100

act gcg ttg gag tct tcg ggt gcg gag gct gcg atg tct ttg ggc ttt 451
Thr Ala Leu Glu Ser Ser Gly Ala Glu Ala Ala Met Ser Leu Gly Phe
            105                 110                 115

acc tct gcg acg gtg ggt act ttt gct gcg att gtt ggt gga att att 499
Thr Ser Ala Thr Val Gly Thr Phe Ala Ala Ile Val Gly Gly Ile Ile
        120                 125                 130

ttc aca acg tgg ggt att aag aag ggc aag act gcg gcg atg cct gcg 547
Phe Thr Thr Trp Gly Ile Lys Lys Gly Lys Thr Ala Ala Met Pro Ala
    135                 140                 145

cag ttg ccg tgg gat ttg cgt tcg ggt tat atc gat aag ctg agt gat 595
Gln Leu Pro Trp Asp Leu Arg Ser Gly Tyr Ile Asp Lys Leu Ser Asp
150                 155                 160                 165

cgt ccg tcg att ggt aag gcg agt acg aat ccg tct gcg att gag cct 643
Arg Pro Ser Ile Gly Lys Ala Ser Thr Asn Pro Ser Ala Ile Glu Pro
                170                 175                 180

ttg gcg ctg cat act ggc att att ttg ttg act gtt gcg gtt gcg tat 691
Leu Ala Leu His Thr Gly Ile Ile Leu Leu Thr Val Ala Val Ala Tyr
            185                 190                 195

tcc att aat cag tgg ttg ggt agc atg ttc cca acc gtg cag att ccg 739
Ser Ile Asn Gln Trp Leu Gly Ser Met Phe Pro Thr Val Gln Ile Pro
        200                 205                 210

ttg ttt gcg atg tca ttt gtg gtc ggc att gtg ggc atg gga att atg 787
Leu Phe Ala Met Ser Phe Val Val Gly Ile Val Gly Met Gly Ile Met
    215                 220                 225

cgt ttg ttg aag aag cct gag tat ttg gat cgc gac acc gtg aat tcc     835
Arg Leu Leu Lys Lys Pro Glu Tyr Leu Asp Arg Asp Thr Val Asn Ser
230                 235                 240                 245

gtg tcg ggc gcg gcg acg gat tac ctc att gcg ttt ggt att gcc tcg     883
Val Ser Gly Ala Ala Thr Asp Tyr Leu Ile Ala Phe Gly Ile Ala Ser
                250                 255                 260

att gct cct gct gcg att gct gat tac tgg gtt cct ctt gtg gtg ctg     931
Ile Ala Pro Ala Ala Ile Ala Asp Tyr Trp Val Pro Leu Val Val Leu
            265                 270                 275

ttt gtt ttg ggc acc atc aac tgt tgt ttc ttc ttt ttc tgg gtt gcg     979
Phe Val Leu Gly Thr Ile Asn Cys Cys Phe Phe Phe Phe Trp Val Ala
        280                 285                 290

cct cgt ttc ttt ggc gag aag tgg ctg gag cgc gcg atc ttc ggt tgg     1027
Pro Arg Phe Phe Gly Glu Lys Trp Leu Glu Arg Ala Ile Phe Gly Trp
    295                 300                 305

ggt tgg gct act gcc gcg gtt gct acc ggt atc gcg ttg ctg aag atc     1075
Gly Trp Ala Thr Ala Ala Val Ala Thr Gly Ile Ala Leu Leu Lys Ile
310                 315                 320                 325

gtg gat ccg aag ttg aag tcg gga gcg ctt aat gag tac ggc gtg gct     1123
Val Asp Pro Lys Leu Lys Ser Gly Ala Leu Asn Glu Tyr Gly Val Ala
                330                 335                 340

tat atc ggt ttt gct cca ttt gaa atc ggc atg acc atc atc gcg ccg     1171
Tyr Ile Gly Phe Ala Pro Phe Glu Ile Gly Met Thr Ile Ile Ala Pro
            345                 350                 355

atc gcg gtg ctc gca ggc ttt acc atg ggg ttg ggt tgg gcg tcg ttg     1219
Ile Ala Val Leu Ala Gly Phe Thr Met Gly Leu Gly Trp Ala Ser Leu
        360                 365                 370

att gtt gcg atc gtg att ttt ggc ctc gcg tgg ggt ctg aag tgg ttg     1267
Ile Val Ala Ile Val Ile Phe Gly Leu Ala Trp Gly Leu Lys Trp Leu
    375                 380                 385

ccg gag cgc gga cat gtc cgc ggc gag ggt aag ccg caa taaaggttgg     1316
Pro Glu Arg Gly His Val Arg Gly Glu Gly Lys Pro Gln
390                 395                 400

aagcgccggg tct                                                      1329


<210> 508
<211> 402
<212> PRT
<213> Corynebacterium glutamicum

<400> 508
Met Ile Leu Gly Pro Lys Val Leu Gly Leu Ile Gly Trp Ser Asp His
  1               5                  10                  15

Leu Ser Thr Tyr Thr Thr Val Leu Ile Ala Ile Val Phe Ala Ser Met
             20                  25                  30

Pro Tyr Ser Met Lys Phe Asp Arg Gly Val Arg Thr Gly Met Lys Thr
         35                  40                  45

Met Trp Ala Tyr Ser Thr Ala Met Phe Val Gly Gln Trp Gly Leu Phe
    50                  55                  60

Ile Leu Leu Gly Leu Phe Leu Phe Gln Pro Val Trp Gly Thr Asp Glu
65                  70                  75                  80

Trp Phe Gly Met Met Leu Pro Val Gly Phe Val Gly Gly Phe Gly Thr
                85                  90                  95

Ala Ala Ala Val Gly Thr Ala Leu Glu Ser Ser Gly Ala Glu Ala Ala
            100                 105                 110

Met Ser Leu Gly Phe Thr Ser Ala Thr Val Gly Thr Phe Ala Ala Ile
        115                 120                 125

Val Gly Gly Ile Ile Phe Thr Thr Trp Gly Ile Lys Lys Gly Lys Thr
    130                 135                 140

Ala Ala Met Pro Ala Gln Leu Pro Trp Asp Leu Arg Ser Gly Tyr Ile
145                 150                 155                 160

Asp Lys Leu Ser Asp Arg Pro Ser Ile Gly Lys Ala Ser Thr Asn Pro
                165                 170                 175

Ser Ala Ile Glu Pro Leu Ala Leu His Thr Gly Ile Ile Leu Leu Thr
            180                 185                 190

Val Ala Val Ala Tyr Ser Ile Asn Gln Trp Leu Gly Ser Met Phe Pro
        195                 200                 205

Thr Val Gln Ile Pro Leu Phe Ala Met Ser Phe Val Val Gly Ile Val
    210                 215                 220

Gly Met Gly Ile Met Arg Leu Leu Lys Lys Pro Glu Tyr Leu Asp Arg
225                 230                 235                 240

Asp Thr Val Asn Ser Val Ser Gly Ala Ala Thr Asp Tyr Leu Ile Ala
                245                 250                 255

Phe Gly Ile Ala Ser Ile Ala Pro Ala Ala Ile Ala Asp Tyr Trp Val
            260                 265                 270

Pro Leu Val Val Leu Phe Val Leu Gly Thr Ile Asn Cys Cys Phe Phe
        275                 280                 285

Phe Phe Trp Val Ala Pro Arg Phe Phe Gly Glu Lys Trp Leu Glu Arg
    290                 295                 300

Ala Ile Phe Gly Trp Gly Trp Ala Thr Ala Ala Val Ala Thr Gly Ile
305                 310                 315                 320

Ala Leu Leu Lys Ile Val Asp Pro Lys Leu Lys Ser Gly Ala Leu Asn
                325                 330                 335

Glu Tyr Gly Val Ala Tyr Ile Gly Phe Ala Pro Phe Glu Ile Gly Met
            340                 345                 350

Thr Ile Ile Ala Pro Ile Ala Val Leu Ala Gly Phe Thr Met Gly Leu
        355                 360                 365

Gly Trp Ala Ser Leu Ile Val Ala Ile Val Ile Phe Gly Leu Ala Trp
    370                 375                 380

Gly Leu Lys Trp Leu Pro Glu Arg Gly His Val Arg Gly Glu Gly Lys
385                 390                 395                 400

Pro Gln

<210> 509
<211> 1776
<212> DNA
<213> Corynebacterium glutamicum

<220>
<221> CDS
<222> (101)..(1753)
<223> RXA02171

<400> 509
aaccacattc gtcattactt acatctatgt catgtttgcg aacaagaact tggagcctcg 60

tcaggctgct attcgccaga agatggaagg ttaatcagat atg aat tcc act att   115
                                           Met Asn Ser Thr Ile
                                             1               5

ctc ctt gca caa gac gct gtt tct gag ggc gtc ggt aat ccg att ctt   163
Leu Leu Ala Gln Asp Ala Val Ser Glu Gly Val Gly Asn Pro Ile Leu
                10                  15                  20

aac atc agt gtc ttc gtc gtc ttc att att gtg acg atg acc gtg gtg   211
Asn Ile Ser Val Phe Val Val Phe Ile Ile Val Thr Met Thr Val Val
            25                  30                  35

ctt cgc gtg ggc aag agc acc agc gaa tcc acc gac ttc tac acc ggt   259
Leu Arg Val Gly Lys Ser Thr Ser Glu Ser Thr Asp Phe Tyr Thr Gly
        40                  45                  50

ggt gct tcc ttc tcc gga acc cag aac ggt ctg gct atc gca ggt gac   307
Gly Ala Ser Phe Ser Gly Thr Gln Asn Gly Leu Ala Ile Ala Gly Asp
    55                  60                  65

tac ctg tct gca gcg tcc ttc ctc gga atc gtt ggt gca att tca ctc   355
Tyr Leu Ser Ala Ala Ser Phe Leu Gly Ile Val Gly Ala Ile Ser Leu
 70                  75                  80                  85

aac ggt tac gac gga ttc ctt tac tcc atc ggc ttc ttc gtc gca tgg   403
Asn Gly Tyr Asp Gly Phe Leu Tyr Ser Ile Gly Phe Phe Val Ala Trp
                90                  95                 100

ctt gtt gca ctg ctg ctc gtg gca gag cca ctt cgt aac gtg ggc cgc   451
Leu Val Ala Leu Leu Leu Val Ala Glu Pro Leu Arg Asn Val Gly Arg
            105                 110                 115

ttc acc atg gct gac gtg ctg tcc ttc cga ctg cgt cag aaa cca gtc   499
Phe Thr Met Ala Asp Val Leu Ser Phe Arg Leu Arg Gln Lys Pro Val
        120                 125                 130

cgc gtc gct gcg gcc tgc ggt acc ctc gcg gtt acc ctc ttt tac ttg   547
Arg Val Ala Ala Ala Cys Gly Thr Leu Ala Val Thr Leu Phe Tyr Leu

    135                 140                 145

atc gct cag atg gct ggt gca ggt tcg ctt gtg tcc gtt ctg ctg gac 595
Ile Ala Gln Met Ala Gly Ala Gly Ser Leu Val Ser Val Leu Leu Asp
150                 155                 160                 165

atc cac gag ttc aag tgg cag gca gtt gtt gtc ggt atc gtt ggc att 643
Ile His Glu Phe Lys Trp Gln Ala Val Val Val Gly Ile Val Gly Ile
                170                 175                 180

gtc atg atc gcc tac gtt ctt ctt ggc ggt atg aag ggc acc aca tac 691
Val Met Ile Ala Tyr Val Leu Leu Gly Gly Met Lys Gly Thr Thr Tyr
            185                 190                 195

gtt cag atg att aag gca gtt ctg ctg gtc ggt ggc gtt gcc att atg 739
Val Gln Met Ile Lys Ala Val Leu Leu Val Gly Gly Val Ala Ile Met
        200                 205                 210

acc gtt ctg acc ttc gtc aag gtg tct ggt ggc ctg acc acc ctt tta 787
Thr Val Leu Thr Phe Val Lys Val Ser Gly Gly Leu Thr Thr Leu Leu
    215                 220                 225

aat gac gct gtt gag aag cac gcc gct tca gat tac gct gcc acc aag 835
Asn Asp Ala Val Glu Lys His Ala Ala Ser Asp Tyr Ala Ala Thr Lys
230                 235                 240                 245

ggg tac gat cca acc cag atc ctg gag cct ggt ctg cag tac ggt gca 883
Gly Tyr Asp Pro Thr Gln Ile Leu Glu Pro Gly Leu Gln Tyr Gly Ala
                250                 255                 260

act ctg acc act cag ctg gac ttc att tcc ttg gct ctc gct ctg tgt 931
Thr Leu Thr Thr Gln Leu Asp Phe Ile Ser Leu Ala Leu Ala Leu Cys
            265                 270                 275

ctt gga acc gct ggt ctg cca cac gtt ctg atg cgc ttc tac acc gtt 979
Leu Gly Thr Ala Gly Leu Pro His Val Leu Met Arg Phe Tyr Thr Val
        280                 285                 290

cct acc gcc aag gaa gca cgt aag tct gtg acc tgg gct atc gtc ctc 1027
Pro Thr Ala Lys Glu Ala Arg Lys Ser Val Thr Trp Ala Ile Val Leu
    295                 300                 305

att ggt gcg ttc tac ctg atg acc ctg gtc ctt ggt tac ggc gct gcg 1075
Ile Gly Ala Phe Tyr Leu Met Thr Leu Val Leu Gly Tyr Gly Ala Ala
310                 315                 320                 325

gca ctg gtc ggt cca gac cgc gtc att gcc gca cca ggt gct gct aat 1123
Ala Leu Val Gly Pro Asp Arg Val Ile Ala Ala Pro Gly Ala Ala Asn
                330                 335                 340

gct gct gct cct ctg ctg gcc ttc gag ctt ggt ggt tcc atc ttc atg 1171
Ala Ala Ala Pro Leu Leu Ala Phe Glu Leu Gly Gly Ser Ile Phe Met
            345                 350                 355

gcg ctg att tcc gca gtt gcg ttc gct acc gtt ctc gcc gtg gtc gca 1219
Ala Leu Ile Ser Ala Val Ala Phe Ala Thr Val Leu Ala Val Val Ala
        360                 365                 370

ggt ctt gca att acc gca tcc gct gct gtt ggt cac gac atc tac aac 1267
Gly Leu Ala Ile Thr Ala Ser Ala Ala Val Gly His Asp Ile Tyr Asn
    375                 380                 385

gct gtt atc cgc aac ggt cag tcc acc gaa gcg gag cag gtc cga gta    1315
Ala Val Ile Arg Asn Gly Gln Ser Thr Glu Ala Glu Gln Val Arg Val
390                 395                 400                 405

tcc cgc atc acc gtt gtc gtc att ggc ctg att tcc att gtc ctg gga    1363
Ser Arg Ile Thr Val Val Val Ile Gly Leu Ile Ser Ile Val Leu Gly
                410                 415                 420

att ctt gca atg acc cag aac gtt gcg ttc ctc gtg gcc ctg gcc ttc    1411
Ile Leu Ala Met Thr Gln Asn Val Ala Phe Leu Val Ala Leu Ala Phe
            425                 430                 435

gca gtt gca gca tcc gct aac ctg cca acc atc ctg tac tcc ctg tac    1459
Ala Val Ala Ala Ser Ala Asn Leu Pro Thr Ile Leu Tyr Ser Leu Tyr
        440                 445                 450

tgg aag aag ttc aac acc acc ggc gct gtg gcc gct atc tac acc ggt    1507
Trp Lys Lys Phe Asn Thr Thr Gly Ala Val Ala Ala Ile Tyr Thr Gly
    455                 460                 465

ctc atc tcc gcg ctg ctg ctg atc ttc ctg tcc cca gca gtc tcc ggt    1555
Leu Ile Ser Ala Leu Leu Leu Ile Phe Leu Ser Pro Ala Val Ser Gly
470                 475                 480                 485

aat gac agc gca atg gtt cca ggt gca gac tgg gca atc ttc cca ctg    1603
Asn Asp Ser Ala Met Val Pro Gly Ala Asp Trp Ala Ile Phe Pro Leu
                490                 495                 500

aag aac cca ggc ctc gtc tcc atc cca ctg gca ttc atc gct ggt tgg    1651
Lys Asn Pro Gly Leu Val Ser Ile Pro Leu Ala Phe Ile Ala Gly Trp
            505                 510                 515

atc ggc act ttg gtt ggc aag cca gac aac atg gat gat ctt gct gcc    1699
Ile Gly Thr Leu Val Gly Lys Pro Asp Asn Met Asp Asp Leu Ala Ala
        520                 525                 530

gaa atg gaa gtt cgt tcc ctc acc ggt gtc ggt gtt gaa aag gct gtt    1747
Glu Met Glu Val Arg Ser Leu Thr Gly Val Gly Val Glu Lys Ala Val
    535                 540                 545

gat cac taaatctagt ttctgaagtt att                                  1776
Asp His
550


<210> 510
<211> 551
<212> PRT
<213> Corynebacterium glutamicum

<400> 510
Met Asn Ser Thr Ile Leu Leu Ala Gln Asp Ala Val Ser Glu Gly Val
1               5                   10                  15

Gly Asn Pro Ile Leu Asn Ile Ser Val Phe Val Val Phe Ile Ile Val
            20                  25                  30

Thr Met Thr Val Val Leu Arg Val Gly Lys Ser Thr Ser Glu Ser Thr
        35                  40                  45

Asp Phe Tyr Thr Gly Gly Ala Ser Phe Ser Gly Thr Gln Asn Gly Leu
    50                  55                  60

Ala Ile Ala Gly Asp Tyr Leu Ser Ala Ala Ser Phe Leu Gly Ile Val
65                  70                  75                  80

Gly Ala Ile Ser Leu Asn Gly Tyr Asp Gly Phe Leu Tyr Ser Ile Gly
                85                  90                  95

Phe Phe Val Ala Trp Leu Val Ala Leu Leu Leu Val Ala Glu Pro Leu
            100                 105                 110

Arg Asn Val Gly Arg Phe Thr Met Ala Asp Val Leu Ser Phe Arg Leu
        115                 120                 125

Arg Gln Lys Pro Val Arg Val Ala Ala Ala Cys Gly Thr Leu Ala Val
    130                 135                 140

Thr Leu Phe Tyr Leu Ile Ala Gln Met Ala Gly Ala Gly Ser Leu Val
145                 150                 155                 160

Ser Val Leu Leu Asp Ile His Glu Phe Lys Trp Gln Ala Val Val Val
                165                 170                 175

Gly Ile Val Gly Ile Val Met Ile Ala Tyr Val Leu Leu Gly Gly Met
            180                 185                 190

Lys Gly Thr Thr Tyr Val Gln Met Ile Lys Ala Val Leu Leu Val Gly
        195                 200                 205

Gly Val Ala Ile Met Thr Val Leu Thr Phe Val Lys Val Ser Gly Gly
    210                 215                 220

Leu Thr Thr Leu Leu Asn Asp Ala Val Glu Lys His Ala Ala Ser Asp
225                 230                 235                 240

Tyr Ala Ala Thr Lys Gly Tyr Asp Pro Thr Gln Ile Leu Glu Pro Gly
                245                 250                 255

Leu Gln Tyr Gly Ala Thr Leu Thr Thr Gln Leu Asp Phe Ile Ser Leu
            260                 265                 270

Ala Leu Ala Leu Cys Leu Gly Thr Ala Gly Leu Pro His Val Leu Met
        275                 280                 285

Arg Phe Tyr Thr Val Pro Thr Ala Lys Glu Ala Arg Lys Ser Val Thr
    290                 295                 300

Trp Ala Ile Val Leu Ile Gly Ala Phe Tyr Leu Met Thr Leu Val Leu
305                 310                 315                 320

Gly Tyr Gly Ala Ala Ala Leu Val Gly Pro Asp Arg Val Ile Ala Ala
                325                 330                 335

Pro Gly Ala Ala Asn Ala Ala Ala Pro Leu Leu Ala Phe Glu Leu Gly
            340                 345                 350

Gly Ser Ile Phe Met Ala Leu Ile Ser Ala Val Ala Phe Ala Thr Val
        355                 360                 365

Leu Ala Val Val Ala Gly Leu Ala Ile Thr Ala Ser Ala Ala Val Gly

370                 375                 380

His Asp Ile Tyr Asn Ala Val Ile Arg Asn Gly Gln Ser Thr Glu Ala
385                 390                 395                 400

Glu Gln Val Arg Val Ser Arg Ile Thr Val Val Val Ile Gly Leu Ile
                405                 410                 415

Ser Ile Val Leu Gly Ile Leu Ala Met Thr Gln Asn Val Ala Phe Leu
            420                 425                 430

Val Ala Leu Ala Phe Ala Val Ala Ala Ser Ala Asn Leu Pro Thr Ile
        435                 440                 445

Leu Tyr Ser Leu Tyr Trp Lys Lys Phe Asn Thr Thr Gly Ala Val Ala
    450                 455                 460

Ala Ile Tyr Thr Gly Leu Ile Ser Ala Leu Leu Leu Ile Phe Leu Ser
465                 470                 475                 480

Pro Ala Val Ser Gly Asn Asp Ser Ala Met Val Pro Gly Ala Asp Trp
                485                 490                 495

Ala Ile Phe Pro Leu Lys Asn Pro Gly Leu Val Ser Ile Pro Leu Ala
            500                 505                 510

Phe Ile Ala Gly Trp Ile Gly Thr Leu Val Gly Lys Pro Asp Asn Met
        515                 520                 525

Asp Asp Leu Ala Ala Glu Met Glu Val Arg Ser Leu Thr Gly Val Gly
    530                 535                 540

Val Glu Lys Ala Val Asp His
545                 550

<210> 511
<211> 1333
<212> DNA
<213> Corynebacterium glutamicum

<220>
<221> CDS
<222> (101)..(1333)
<223> RXA00902

<400> 511
ttgttattgg tttgttcact cccatgcaat gtacgatcgg ttctaacgtc tacggcaatt 60

gacacgtcaa tttaattcag gaaaggggtg gggagaattt atg tct agt ggt ttt   115
                                           Met Ser Ser Gly Phe
                                           1               5

gaa tat gtt caa cat cct cga cga gcc ctc cct ccg cct ata cct gaa   163
Glu Tyr Val Gln His Pro Arg Arg Ala Leu Pro Pro Pro Ile Pro Glu
                10                  15                  20

cgg aaa ggt cct gcc gca gca ttc ctg ccg gga act ttc cat ccc att   211
Arg Lys Gly Pro Ala Ala Ala Phe Leu Pro Gly Thr Phe His Pro Ile
            25                  30                  35

aat cca aag aat att gca gcg agc cac gat cag gtg ctt cta tct ggt 259
Asn Pro Lys Asn Ile Ala Ala Ser His Asp Gln Val Leu Leu Ser Gly
        40                  45                  50

tgg ggc aaa ttt gtg cgt tgg ctc ttg gta ttg ttg tcc att ttg gtc 307
Trp Gly Lys Phe Val Arg Trp Leu Leu Val Leu Leu Ser Ile Leu Val
    55                  60                  65

atc atc att ggc atc aac ctc atc ttg gac ggt gtc tac gga ttt ggt 355
Ile Ile Ile Gly Ile Asn Leu Ile Leu Asp Gly Val Tyr Gly Phe Gly
70                  75                  80                  85

act ttt tca acc acc cag atg tac caa gtt gcg aaa gat cca ctc att 403
Thr Phe Ser Thr Thr Gln Met Tyr Gln Val Ala Lys Asp Pro Leu Ile
                90                  95                  100

ggt gtg ctg atc ggt atc ttg gct acg gcc ttg gtg caa tca tca acc 451
Gly Val Leu Ile Gly Ile Leu Ala Thr Ala Leu Val Gln Ser Ser Thr
            105                 110                 115

acc acc aca acg ttg acg gtg act gca gtt ggt acg ggc att gtg tcg 499
Thr Thr Thr Thr Leu Thr Val Thr Ala Val Gly Thr Gly Ile Val Ser
        120                 125                 130

gtg cct gtg gcg att ccg atc att ctt ggc gca aat atc ggt acg acg 547
Val Pro Val Ala Ile Pro Ile Ile Leu Gly Ala Asn Ile Gly Thr Thr
    135                 140                 145

atc acc gcg atg ctc gtt gcg ttt tct tat gtg ggt gaa cgc agg gag 595
Ile Thr Ala Met Leu Val Ala Phe Ser Tyr Val Gly Glu Arg Arg Glu
150                 155                 160                 165

ttt aag cga gct ttt acg gtt gcc gcc atg cat gtg tgg ttt aac gtg 643
Phe Lys Arg Ala Phe Thr Val Ala Ala Met His Val Trp Phe Asn Val
                170                 175                 180

ctc gtc att ctt gtt cta ttt gtt gtg gaa ttg ctc ttt cat cca ttc 691
Leu Val Ile Leu Val Leu Phe Val Val Glu Leu Leu Phe His Pro Phe
            185                 190                 195

cgc aca att agt ggt gcg atc gca acg gag atc aca ctg aca act ggt 739
Arg Thr Ile Ser Gly Ala Ile Ala Thr Glu Ile Thr Leu Thr Thr Gly
        200                 205                 210

ggc tct ttg cct acc agt ggt gtg atg acc aag att ttt gat ccc cca 787
Gly Ser Leu Pro Thr Ser Gly Val Met Thr Lys Ile Phe Asp Pro Pro
    215                 220                 225

acc caa ctt ctg ggt atg aat ggt ctt atc ggt tcg atc ggc aat cct 835
Thr Gln Leu Leu Gly Met Asn Gly Leu Ile Gly Ser Ile Gly Asn Pro
230                 235                 240                 245

agt att tcg gcg att gta tgt ctt gtg gtg ggc acc att ctt att ctg 883
Ser Ile Ser Ala Ile Val Cys Leu Val Val Gly Thr Ile Leu Ile Leu
                250                 255                 260

att tcg gtg cgt gcc atg agt tct cag atc cga acc att acg gca gcg 931
Ile Ser Val Arg Ala Met Ser Ser Gln Ile Arg Thr Ile Thr Ala Ala
            265                 270                 275

acc gta acc tca att atg gac aag gtg atc aat cca gag aac agc ccc 979

Thr Val Thr Ser Ile Met Asp Lys Val Ile Asn Pro Glu Asn Ser Pro
        280                 285                 290

aag gcg acg att ctt tcc aat ttc tgg agc ttc att ctt gga gtt ttg 1027
Lys Ala Thr Ile Leu Ser Asn Phe Trp Ser Phe Ile Leu Gly Val Leu
    295                 300                 305

ttc acg ctc atg gtc act gcc tcg tca gtg acc gtg gct tcc atg cag 1075
Phe Thr Leu Met Val Thr Ala Ser Ser Val Thr Val Ala Ser Met Gln
310                 315                 320                 325

cca gtg gct gcc tct ggt gtc gtt aag caa aag cca ttg ctg ggc gtc 1123
Pro Val Ala Ala Ser Gly Val Val Lys Gln Lys Pro Leu Leu Gly Val
                330                 335                 340

att ttg ggt gcc aac gtg ggc acc acg gtg acc gca atg ttt gct act 1171
Ile Leu Gly Ala Asn Val Gly Thr Thr Val Thr Ala Met Phe Ala Thr
            345                 350                 355

ttc gcg att gtc agc gat cag ggt gag ttc gct att cag gct gcg ttg 1219
Phe Ala Ile Val Ser Asp Gln Gly Glu Phe Ala Ile Gln Ala Ala Leu
        360                 365                 370

atc cac ctc att gtg aac ttc acc ggc gca tta cta gtg ctg tgt att 1267
Ile His Leu Ile Val Asn Phe Thr Gly Ala Leu Leu Val Leu Cys Ile
    375                 380                 385

ccg cag ctt gcc aat gtg att att cac ttg gcc gag aaa act gcg aac 1315
Pro Gln Leu Ala Asn Val Ile Ile His Leu Ala Glu Lys Thr Ala Asn
390                 395                 400                 405

ctc act gcc cgc agt tac 1333
Leu Thr Ala Arg Ser Tyr
                410

<210> 512
<211> 411
<212> PRT
<213> Corynebacterium glutamicum

<400> 512
Met Ser Ser Gly Phe Glu Tyr Val Gln His Pro Arg Arg Ala Leu Pro
1               5                   10                  15

Pro Pro Ile Pro Glu Arg Lys Gly Pro Ala Ala Ala Phe Leu Pro Gly
            20                  25                  30

Thr Phe His Pro Ile Asn Pro Lys Asn Ile Ala Ala Ser His Asp Gln
        35                  40                  45

Val Leu Leu Ser Gly Trp Gly Lys Phe Val Arg Trp Leu Leu Val Leu
    50                  55                  60

Leu Ser Ile Leu Val Ile Ile Ile Gly Ile Asn Leu Ile Leu Asp Gly
65                  70                  75                  80

Val Tyr Gly Phe Gly Thr Phe Ser Thr Thr Gln Met Tyr Gln Val Ala
                85                  90                  95

Lys Asp Pro Leu Ile Gly Val Leu Ile Gly Ile Leu Ala Thr Ala Leu

            100                 105                 110

Val Gln Ser Ser Thr Thr Thr Thr Thr Leu Thr Val Thr Ala Val Gly
        115                 120                 125

Thr Gly Ile Val Ser Val Pro Val Ala Ile Pro Ile Ile Leu Gly Ala
    130                 135                 140

Asn Ile Gly Thr Thr Ile Thr Ala Met Leu Val Ala Phe Ser Tyr Val
145                 150                 155                 160

Gly Glu Arg Arg Glu Phe Lys Arg Ala Phe Thr Val Ala Ala Met His
                165                 170                 175

Val Trp Phe Asn Val Leu Val Ile Leu Val Leu Phe Val Val Glu Leu
            180                 185                 190

Leu Phe His Pro Phe Arg Thr Ile Ser Gly Ala Ile Ala Thr Glu Ile
        195                 200                 205

Thr Leu Thr Thr Gly Gly Ser Leu Pro Thr Ser Gly Val Met Thr Lys
    210                 215                 220

Ile Phe Asp Pro Pro Thr Gln Leu Leu Gly Met Asn Gly Leu Ile Gly
225                 230                 235                 240

Ser Ile Gly Asn Pro Ser Ile Ser Ala Ile Val Cys Leu Val Val Gly
                245                 250                 255

Thr Ile Leu Ile Leu Ile Ser Val Arg Ala Met Ser Ser Gln Ile Arg
            260                 265                 270

Thr Ile Thr Ala Ala Thr Val Thr Ser Ile Met Asp Lys Val Ile Asn
        275                 280                 285

Pro Glu Asn Ser Pro Lys Ala Thr Ile Leu Ser Asn Phe Trp Ser Phe
    290                 295                 300

Ile Leu Gly Val Leu Phe Thr Leu Met Val Thr Ala Ser Ser Val Thr
305                 310                 315                 320

Val Ala Ser Met Gln Pro Val Ala Ala Ser Gly Val Val Lys Gln Lys
                325                 330                 335

Pro Leu Leu Gly Val Ile Leu Gly Ala Asn Val Gly Thr Thr Val Thr
            340                 345                 350

Ala Met Phe Ala Thr Phe Ala Ile Val Ser Asp Gln Gly Glu Phe Ala
        355                 360                 365

Ile Gln Ala Ala Leu Ile His Leu Ile Val Asn Phe Thr Gly Ala Leu
    370                 375                 380

Leu Val Leu Cys Ile Pro Gln Leu Ala Asn Val Ile Ile His Leu Ala
385                 390                 395                 400

Glu Lys Thr Ala Asn Leu Thr Ala Arg Ser Tyr
                405                 410

<210> 513

<211> 1440
<212> DNA
<213> Corynebacterium glutamicum

<220>
<221> CDS
<222> (101)..(1417)
<223> RXA00941

<400> 513
agccggggtg gccgtttgcg tgtacgggtt ttgcttatcg acgcccactt ctgattttaa 60

aggcaaatat tttgtgtcac agcgagtaat cttttcgggc atg aac cta acc cgg 115
Met Asn Leu Thr Arg
1               5

aat gac agg ctc gat cga ctg ccc gta act tcc aaa cat aaa aag att 163
Asn Asp Arg Leu Asp Arg Leu Pro Val Thr Ser Lys His Lys Lys Ile
                10                  15                  20

ctt ggc ggc tca ggt atc ggc tgg gca ttg gat gcc atg gat gtc gga 211
Leu Gly Gly Ser Gly Ile Gly Trp Ala Leu Asp Ala Met Asp Val Gly
            25                  30                  35

ctg atc tct ttc gtc atg gct gcg ttg gcc act cat tgg ggc cta tcc 259
Leu Ile Ser Phe Val Met Ala Ala Leu Ala Thr His Trp Gly Leu Ser
        40                  45                  50

cct act gaa act tcc ctg ctc gga tcc atc ggt ttc gtt ggc atg gcc 307
Pro Thr Glu Thr Ser Leu Leu Gly Ser Ile Gly Phe Val Gly Met Ala
    55                  60                  65

atc ggc gct tcg ctg ggc ggt ttg ctg gcg gac aag ttg ggg cgt cga 355
Ile Gly Ala Ser Leu Gly Gly Leu Leu Ala Asp Lys Leu Gly Arg Arg
70                  75                  80                  85

caa gtt ttt gcg ttg tct ttg cta gtt tat ggc gtt gcc act ggc gcg 403
Gln Val Phe Ala Leu Ser Leu Leu Val Tyr Gly Val Ala Thr Gly Ala
                90                  95                  100

tcg gcg ctt tca gtg tcg ctg gca atg ttg atg gcg ctg cgc ttc gtc 451
Ser Ala Leu Ser Val Ser Leu Ala Met Leu Met Ala Leu Arg Phe Val
            105                 110                 115

gtt gga ctt ggc ctg ggc gct gaa ctc ccc gtt gca tcc act ctg att 499
Val Gly Leu Gly Leu Gly Ala Glu Leu Pro Val Ala Ser Thr Leu Ile
        120                 125                 130

tcc gag ttt tct cca cga aaa gtt cgt ggc cgc atg gtc gtt atc ctg 547
Ser Glu Phe Ser Pro Arg Lys Val Arg Gly Arg Met Val Val Ile Leu
    135                 140                 145

gag gcg ttt tgg gcg ctg ggc tgg atc atg gct gca atc gtc gga acc 595
Glu Ala Phe Trp Ala Leu Gly Trp Ile Met Ala Ala Ile Val Gly Thr
150                 155                 160                 165

ttt gtc gtg gca gga tcc gat aat ggt tgg cgt tgg gcg tta gct ctt 643
Phe Val Val Ala Gly Ser Asp Asn Gly Trp Arg Trp Ala Leu Ala Leu
                170                 175                 180

ggc tgt gtc cct gca att tac gcg gtg tat gtc cgt ctc gga ctt cca 691

Gly Cys Val Pro Ala Ile Tyr Ala Val Tyr Val Arg Leu Gly Leu Pro
            185                 190                 195

gaa tca gta cgt ttc ctg gag aag aaa ggc cgg cac gac gaa gca gaa 739
Glu Ser Val Arg Phe Leu Glu Lys Lys Gly Arg His Asp Glu Ala Glu
        200                 205                 210

gcc atc gtt gtt tcc ttc gaa gaa gct gcc gct gcc gaa ggt aaa gct 787
Ala Ile Val Val Ser Phe Glu Glu Ala Ala Ala Ala Glu Gly Lys Ala
    215                 220                 225

gcc gat gcc acc acc gct gtg gtt cat gac aac gct gcc gag ggt tcc 835
Ala Asp Ala Thr Thr Ala Val Val His Asp Asn Ala Ala Glu Gly Ser
230                 235                 240                 245

gta tcc atc tgg tca gct gct ttg cgc aag cgc acc gtc gcg ctg tgg 883
Val Ser Ile Trp Ser Ala Ala Leu Arg Lys Arg Thr Val Ala Leu Trp
                250                 255                 260

atc gtg tgg ttc tgc atc aac ttg tcc tac tac gga gcc ttc att tgg 931
Ile Val Trp Phe Cys Ile Asn Leu Ser Tyr Tyr Gly Ala Phe Ile Trp
            265                 270                 275

att cca tcg ctg ctg gtt gcc gac ggt ttc acc ctc gtg aag tct ttc 979
Ile Pro Ser Leu Leu Val Ala Asp Gly Phe Thr Leu Val Lys Ser Phe
        280                 285                 290

caa ttc act ctg atc atc acc ttg gct cag ctt cca ggc tat gcg gtt 1027
Gln Phe Thr Leu Ile Ile Thr Leu Ala Gln Leu Pro Gly Tyr Ala Val
    295                 300                 305

gca gcg tgg ttg att gaa aag tgg ggc cgt cgc agc aca ttg gcc acg 1075
Ala Ala Trp Leu Ile Glu Lys Trp Gly Arg Arg Ser Thr Leu Ala Thr
310                 315                 320                 325

ttc ctg gtt ggt tct gct atc tct gca gcg ctc tac ggc ttg gca aat 1123
Phe Leu Val Gly Ser Ala Ile Ser Ala Ala Leu Tyr Gly Leu Ala Asn
                330                 335                 340

gtg gag tgg cag atc ctg gtc gca ggt tgt tta cta tcc ttc ttc aac 1171
Val Glu Trp Gln Ile Leu Val Ala Gly Cys Leu Leu Ser Phe Phe Asn
            345                 350                 355

ctg ggc gca tgg ggc gca ctg tat gcc att ggg ccg gag ctt tat ccc 1219
Leu Gly Ala Trp Gly Ala Leu Tyr Ala Ile Gly Pro Glu Leu Tyr Pro
        360                 365                 370

act aat gtc cgt gga act gga acg ggt gct gcc gcg gga ttc ggg cgc 1267
Thr Asn Val Arg Gly Thr Gly Thr Gly Ala Ala Ala Gly Phe Gly Arg
    375                 380                 385

att gct tcc atc atc gct ccg ctc att gtt ccg cca gtg att gct ttt 1315
Ile Ala Ser Ile Ile Ala Pro Leu Ile Val Pro Pro Val Ile Ala Phe
390                 395                 400                 405

ggt gga cca att gct ttg ttc gct ctc ttt gcc acc gcg ttt gcg atc 1363
Gly Gly Pro Ile Ala Leu Phe Ala Leu Phe Ala Thr Ala Phe Ala Ile
                410                 415                 420

gca gcg att gcg gcg ttt acg ctg cct gag cag aag ggt aag tct ctc 1411
Ala Ala Ile Ala Ala Phe Thr Leu Pro Glu Gln Lys Gly Lys Ser Leu

            425                 430                 435

gct gat tagtgagatc cttccaccag ttt 1440
Ala Asp

<210> 514
<211> 439
<212> PRT
<213> Corynebacterium glutamicum

<400> 514
Met Asn Leu Thr Arg Asn Asp Arg Leu Asp Arg Leu Pro Val Thr Ser
1               5                   10                  15

Lys His Lys Lys Ile Leu Gly Gly Ser Gly Ile Gly Trp Ala Leu Asp
            20                  25                  30

Ala Met Asp Val Gly Leu Ile Ser Phe Val Met Ala Ala Leu Ala Thr
        35                  40                  45

His Trp Gly Leu Ser Pro Thr Glu Thr Ser Leu Leu Gly Ser Ile Gly
    50                  55                  60

Phe Val Gly Met Ala Ile Gly Ala Ser Leu Gly Gly Leu Leu Ala Asp
65                  70                  75                  80

Lys Leu Gly Arg Arg Gln Val Phe Ala Leu Ser Leu Leu Val Tyr Gly
                85                  90                  95

Val Ala Thr Gly Ala Ser Ala Leu Ser Val Ser Leu Ala Met Leu Met
            100                 105                 110

Ala Leu Arg Phe Val Val Gly Leu Gly Leu Gly Ala Glu Leu Pro Val
        115                 120                 125

Ala Ser Thr Leu Ile Ser Glu Phe Ser Pro Arg Lys Val Arg Gly Arg
    130                 135                 140

Met Val Val Ile Leu Glu Ala Phe Trp Ala Leu Gly Trp Ile Met Ala
145                 150                 155                 160

Ala Ile Val Gly Thr Phe Val Val Ala Gly Ser Asp Asn Gly Trp Arg
                165                 170                 175

Trp Ala Leu Ala Leu Gly Cys Val Pro Ala Ile Tyr Ala Val Tyr Val
            180                 185                 190

Arg Leu Gly Leu Pro Glu Ser Val Arg Phe Leu Glu Lys Lys Gly Arg
        195                 200                 205

His Asp Glu Ala Glu Ala Ile Val Val Ser Phe Glu Glu Ala Ala Ala
    210                 215                 220

Ala Glu Gly Lys Ala Ala Asp Ala Thr Thr Ala Val Val His Asp Asn
225                 230                 235                 240

Ala Ala Glu Gly Ser Val Ser Ile Trp Ser Ala Ala Leu Arg Lys Arg
                245                 250                 255

Thr Val Ala Leu Trp Ile Val Trp Phe Cys Ile Asn Leu Ser Tyr Tyr
            260                 265                 270

Gly Ala Phe Ile Trp Ile Pro Ser Leu Leu Val Ala Asp Gly Phe Thr
        275                 280                 285

Leu Val Lys Ser Phe Gln Phe Thr Leu Ile Ile Thr Leu Ala Gln Leu
    290                 295                 300

Pro Gly Tyr Ala Val Ala Ala Trp Leu Ile Glu Lys Trp Gly Arg Arg
305                 310                 315                 320

Ser Thr Leu Ala Thr Phe Leu Val Gly Ser Ala Ile Ser Ala Ala Leu
                325                 330                 335

Tyr Gly Leu Ala Asn Val Glu Trp Gln Ile Leu Val Ala Gly Cys Leu
            340                 345                 350

Leu Ser Phe Phe Asn Leu Gly Ala Trp Gly Ala Leu Tyr Ala Ile Gly
        355                 360                 365

Pro Glu Leu Tyr Pro Thr Asn Val Arg Gly Thr Gly Thr Gly Ala Ala
    370                 375                 380

Ala Gly Phe Gly Arg Ile Ala Ser Ile Ile Ala Pro Leu Ile Val Pro
385                 390                 395                 400

Pro Val Ile Ala Phe Gly Gly Pro Ile Ala Leu Phe Ala Leu Phe Ala
                405                 410                 415

Thr Ala Phe Ala Ile Ala Ala Ile Ala Ala Phe Thr Leu Pro Glu Gln
            420                 425                 430

Lys Gly Lys Ser Leu Ala Asp
        435

<210> 515
<211> 1704
<212> DNA
<213> Corynebacterium glutamicum

<220>
<221> CDS
<222> (101)..(1681)
<223> RXN00449

<400> 515
tcgttggctt acatggtcat tgatgaccgg gctgaatatg tgagaaaatc catcccttct 60

ttaagcaagg gagtgaatta cagaaaagga ttgttcagca atg agc aca cct gac 115
Met Ser Thr Pro Asp
1               5

att aaa gaa ggc tcg gca gaa tca ccg ggc gaa gta atg gtc gtt gga 163
Ile Lys Glu Gly Ser Ala Glu Ser Pro Gly Glu Val Met Val Val Gly
                10                  15                  20

gac agg cga gag tgg cgt cga caa gca acc ggc atc att gcc ggc ctc 211
Asp Arg Arg Glu Trp Arg Arg Gln Ala Thr Gly Ile Ile Ala Gly Leu
            25                  30                  35

gtc tta gcc gcc ctg gtc tat ctt ctc ttc ccc tcg aac tcc gtg gaa 259
Val Leu Ala Ala Leu Val Tyr Leu Leu Phe Pro Ser Asn Ser Val Glu
        40                  45                  50

acc gtc atg caa tcc agt ggc gtc gat cca gaa act gaa tac acc aac 307
Thr Val Met Gln Ser Ser Gly Val Asp Pro Glu Thr Glu Tyr Thr Asn
    55                  60                  65

aac gcg atg cgt ctt act gct gca gtc aca atc ttg atg gca gtg tgg 355
Asn Ala Met Arg Leu Thr Ala Ala Val Thr Ile Leu Met Ala Val Trp
70                  75                  80                  85

tgg atg aca gaa gca atc cca cta gca gca acc gca ctt atc ccg ttg 403
Trp Met Thr Glu Ala Ile Pro Leu Ala Ala Thr Ala Leu Ile Pro Leu
                90                  95                  100

gtt gca ttc cct gct ttc cag gtt gtg gac ttt ggg aag gca aca gct 451
Val Ala Phe Pro Ala Phe Gln Val Val Asp Phe Gly Lys Ala Thr Ala
            105                 110                 115

ccg tat gcc aac cct acg agc ttc ctc ttc ttg ggc ggc ttt ctt atg 499
Pro Tyr Ala Asn Pro Thr Ser Phe Leu Phe Leu Gly Gly Phe Leu Met
        120                 125                 130

gca ctt ggc ctg cag aaa tgg aac cta cac cga cgt atg gct cta gcg 547
Ala Leu Gly Leu Gln Lys Trp Asn Leu His Arg Arg Met Ala Leu Ala
    135                 140                 145

gtc gtg cta gct gtt ggt act aag cca aag caa ttg gtc ttg ggt ttt 595
Val Val Leu Ala Val Gly Thr Lys Pro Lys Gln Leu Val Leu Gly Phe
150                 155                 160                 165

atg gtg gca act gga ttt ttg tcc atg tgg gtg tct aac act gca acg 643
Met Val Ala Thr Gly Phe Leu Ser Met Trp Val Ser Asn Thr Ala Thr
                170                 175                 180

gcc gtg gtt atg tta ccg atc ggt atg tcg gta ctg gca ctg acc gct 691
Ala Val Val Met Leu Pro Ile Gly Met Ser Val Leu Ala Leu Thr Ala
            185                 190                 195

gag act gtg ggc gga atg aaa aac caa aag aaa ttc gcc act gga ctc 739
Glu Thr Val Gly Gly Met Lys Asn Gln Lys Lys Phe Ala Thr Gly Leu
        200                 205                 210

atg ctg tcc att gct tat tct gct tcc atc ggt tca ctc ggc acc tta 787
Met Leu Ser Ile Ala Tyr Ser Ala Ser Ile Gly Ser Leu Gly Thr Leu
    215                 220                 225

att ggc acg cca ccc aat gcc ttg ctt gct gcg tat atg tct gaa tcg 835
Ile Gly Thr Pro Pro Asn Ala Leu Leu Ala Ala Tyr Met Ser Glu Ser
230                 235                 240                 245

cat gat atc cac atc gga ttt ggt cag tgg atg att ctt ggt gta cca 883
His Asp Ile His Ile Gly Phe Gly Gln Trp Met Ile Leu Gly Val Pro
                250                 255                 260

att gct gtc gtc ttc acc atc atc gcg tgg ctt gtg ttg acc acc gtg 931
Ile Ala Val Val Phe Thr Ile Ile Ala Trp Leu Val Leu Thr Thr Val
            265                 270                 275

ttc aag cca gaa atg aaa gaa atc cct ggc gga cgt gaa ctg atc aaa 979
Phe Lys Pro Glu Met Lys Glu Ile Pro Gly Gly Arg Glu Leu Ile Lys
        280                 285                 290

cgt gaa atc gct gaa atg ggg ccg tgg act gca cct cag gtc aca gtg 1027
Arg Glu Ile Ala Glu Met Gly Pro Trp Thr Ala Pro Gln Val Thr Val
    295                 300                 305

ggt gtt att ttt gcg gca gct gca ctg gct tgg gtc ttc att cca tta 1075
Gly Val Ile Phe Ala Ala Ala Ala Leu Ala Trp Val Phe Ile Pro Leu
310                 315                 320                 325

act cta gat tgg acc ggt tcc cag ctc tct atc aat gac tcc ctc att 1123
Thr Leu Asp Trp Thr Gly Ser Gln Leu Ser Ile Asn Asp Ser Leu Ile
                330                 335                 340

ggc atc gct gcc ggc ctg ctg atg ttt atc gtt ccc gct aac ttt aaa 1171
Gly Ile Ala Ala Gly Leu Leu Met Phe Ile Val Pro Ala Asn Phe Lys
            345                 350                 355

acc ggc gaa cgc att ctt gat tgg cgt act gca ggc gaa ctt cca tgg 1219
Thr Gly Glu Arg Ile Leu Asp Trp Arg Thr Ala Gly Glu Leu Pro Trp
        360                 365                 370

gat gtt ctc ttg ctt ttt ggt ggc ggg ctt tca ctt tct gcg atg ttt 1267
Asp Val Leu Leu Leu Phe Gly Gly Gly Leu Ser Leu Ser Ala Met Phe
    375                 380                 385

acc agc acg gga ctt tcc cta tgg atc ggt gaa cta gct aag gga ctt 1315
Thr Ser Thr Gly Leu Ser Leu Trp Ile Gly Glu Leu Ala Lys Gly Leu
390                 395                 400                 405

gat gcc ctt cca atc ttc att ctc atc ttc gcc att gct gtc ctg gtg 1363
Asp Ala Leu Pro Ile Phe Ile Leu Ile Phe Ala Ile Ala Val Leu Val
                410                 415                 420

ttg ttc ctg acc gag ttc acc tcc aac acc gca aca gcg gca acc ttc 1411
Leu Phe Leu Thr Glu Phe Thr Ser Asn Thr Ala Thr Ala Ala Thr Phe
            425                 430                 435

ctg cca atc atg ggt ggc gtc gcc gta ggt atc gga ctg acc gca ggt 1459
Leu Pro Ile Met Gly Gly Val Ala Val Gly Ile Gly Leu Thr Ala Gly
        440                 445                 450

ggc gag cag aat gtt ctg ctg ctg acc atc cca gtc gca ctg tcc gca 1507
Gly Glu Gln Asn Val Leu Leu Leu Thr Ile Pro Val Ala Leu Ser Ala
    455                 460                 465

acc tgt gcg ttc atg ctt cca gtg gca acg cct cca aac gcg att gca 1555
Thr Cys Ala Phe Met Leu Pro Val Ala Thr Pro Pro Asn Ala Ile Ala
470                 475                 480                 485

ttc ggc tcc ggc tac att aag atc ggc gaa atg gtc aag ggt ggt ctg 1603
Phe Gly Ser Gly Tyr Ile Lys Ile Gly Glu Met Val Lys Gly Gly Leu
                490                 495                 500

tgg ctg aac atc atc gca gtc atc ctc att acg att ttc acc tac ttc 1651
Trp Leu Asn Ile Ile Ala Val Ile Leu Ile Thr Ile Phe Thr Tyr Phe
            505                 510                 515

gta gcg atc cca ctc ttt ggc atc atg ctt taaaagttaa caggcccgca 1701

Val Ala Ile Pro Leu Phe Gly Ile Met Leu
        520                 525

gtc                                                                    1704

<210> 516
<211> 527
<212> PRT
<213> Corynebacterium glutamicum

<400> 516
Met Ser Thr Pro Asp Ile Lys Glu Gly Ser Ala Glu Ser Pro Gly Glu
  1               5                  10                  15

Val Met Val Val Gly Asp Arg Arg Glu Trp Arg Arg Gln Ala Thr Gly
             20                  25                  30

Ile Ile Ala Gly Leu Val Leu Ala Ala Leu Val Tyr Leu Leu Phe Pro
         35                  40                  45

Ser Asn Ser Val Glu Thr Val Met Gln Ser Ser Gly Val Asp Pro Glu
     50                  55                  60

Thr Glu Tyr Thr Asn Asn Ala Met Arg Leu Thr Ala Ala Val Thr Ile
 65                  70                  75                  80

Leu Met Ala Val Trp Trp Met Thr Glu Ala Ile Pro Leu Ala Ala Thr
                 85                  90                  95

Ala Leu Ile Pro Leu Val Ala Phe Pro Ala Phe Gln Val Val Asp Phe
            100                 105                 110

Gly Lys Ala Thr Ala Pro Tyr Ala Asn Pro Thr Ser Phe Leu Phe Leu
        115                 120                 125

Gly Gly Phe Leu Met Ala Leu Gly Leu Gln Lys Trp Asn Leu His Arg
    130                 135                 140

Arg Met Ala Leu Ala Val Val Leu Ala Val Gly Thr Lys Pro Lys Gln
145                 150                 155                 160

Leu Val Leu Gly Phe Met Val Ala Thr Gly Phe Leu Ser Met Trp Val
                165                 170                 175

Ser Asn Thr Ala Thr Ala Val Val Met Leu Pro Ile Gly Met Ser Val
            180                 185                 190

Leu Ala Leu Thr Ala Glu Thr Val Gly Gly Met Lys Asn Gln Lys Lys
        195                 200                 205

Phe Ala Thr Gly Leu Met Leu Ser Ile Ala Tyr Ser Ala Ser Ile Gly
    210                 215                 220

Ser Leu Gly Thr Leu Ile Gly Thr Pro Pro Asn Ala Leu Leu Ala Ala
225                 230                 235                 240

Tyr Met Ser Glu Ser His Asp Ile His Ile Gly Phe Gly Gln Trp Met
                245                 250                 255

Ile Leu Gly Val Pro Ile Ala Val Val Phe Thr Ile Ile Ala Trp Leu

            260                 265                 270

Val Leu Thr Thr Val Phe Lys Pro Glu Met Lys Glu Ile Pro Gly Gly
        275                 280                 285

Arg Glu Leu Ile Lys Arg Glu Ile Ala Glu Met Gly Pro Trp Thr Ala
    290                 295                 300

Pro Gln Val Thr Val Gly Val Ile Phe Ala Ala Ala Ala Leu Ala Trp
305                 310                 315                 320

Val Phe Ile Pro Leu Thr Leu Asp Trp Thr Gly Ser Gln Leu Ser Ile
                325                 330                 335

Asn Asp Ser Leu Ile Gly Ile Ala Ala Gly Leu Leu Met Phe Ile Val
            340                 345                 350

Pro Ala Asn Phe Lys Thr Gly Glu Arg Ile Leu Asp Trp Arg Thr Ala
        355                 360                 365

Gly Glu Leu Pro Trp Asp Val Leu Leu Leu Phe Gly Gly Gly Leu Ser
    370                 375                 380

Leu Ser Ala Met Phe Thr Ser Thr Gly Leu Ser Leu Trp Ile Gly Glu
385                 390                 395                 400

Leu Ala Lys Gly Leu Asp Ala Leu Pro Ile Phe Ile Leu Ile Phe Ala
                405                 410                 415

Ile Ala Val Leu Val Leu Phe Leu Thr Glu Phe Thr Ser Asn Thr Ala
            420                 425                 430

Thr Ala Ala Thr Phe Leu Pro Ile Met Gly Gly Val Ala Val Gly Ile
        435                 440                 445

Gly Leu Thr Ala Gly Gly Glu Gln Asn Val Leu Leu Leu Thr Ile Pro
    450                 455                 460

Val Ala Leu Ser Ala Thr Cys Ala Phe Met Leu Pro Val Ala Thr Pro
465                 470                 475                 480

Pro Asn Ala Ile Ala Phe Gly Ser Gly Tyr Ile Lys Ile Gly Glu Met
                485                 490                 495

Val Lys Gly Gly Leu Trp Leu Asn Ile Ile Ala Val Ile Leu Ile Thr
            500                 505                 510

Ile Phe Thr Tyr Phe Val Ala Ile Pro Leu Phe Gly Ile Met Leu
        515                 520                 525

<210> 517
<211> 1028
<212> DNA
<213> Corynebacterium glutamicum

<220>
<221> CDS
<222> (1)..(1005)
<223> FRXA00449

<400> 517
ctg gca ctg acc gct gag act gtg ggc gga atg aaa aac caa aag aaa      48
Leu Ala Leu Thr Ala Glu Thr Val Gly Gly Met Lys Asn Gln Lys Lys
1               5                   10                  15

ttc gcc act gga ctc atg ctg tcc att gct tat tct gct tcc atc ggt      96
Phe Ala Thr Gly Leu Met Leu Ser Ile Ala Tyr Ser Ala Ser Ile Gly
            20                  25                  30

tca ctc ggc acc tta att ggc acg cca ccc aat gcc ttg ctt gct gcg      144
Ser Leu Gly Thr Leu Ile Gly Thr Pro Pro Asn Ala Leu Leu Ala Ala
        35                  40                  45

tat atg tct gaa tcg cat gat atc cac atc gga ttt ggt cag tgg atg      192
Tyr Met Ser Glu Ser His Asp Ile His Ile Gly Phe Gly Gln Trp Met
    50                  55                  60

att ctt ggt gta cca att gct gtc gtc ttc acc atc atc gcg tgg ctt      240
Ile Leu Gly Val Pro Ile Ala Val Val Phe Thr Ile Ile Ala Trp Leu
65                  70                  75                  80

gtg ttg acc acc gtg ttc aag cca gaa atg aaa gaa atc cct ggc gga      288
Val Leu Thr Thr Val Phe Lys Pro Glu Met Lys Glu Ile Pro Gly Gly
                85                  90                  95

cgt gaa ctg atc aaa cgt gaa atc gct gaa atg ggg ccg tgg act gca      336
Arg Glu Leu Ile Lys Arg Glu Ile Ala Glu Met Gly Pro Trp Thr Ala
            100                 105                 110

cct cag gtc aca gtg ggt gtt att ttt gcg gca gct gca ctg gct tgg      384
Pro Gln Val Thr Val Gly Val Ile Phe Ala Ala Ala Ala Leu Ala Trp
        115                 120                 125

gtc ttc att cca tta act cta gat tgg acc ggt tcc cag ctc tct atc      432
Val Phe Ile Pro Leu Thr Leu Asp Trp Thr Gly Ser Gln Leu Ser Ile
    130                 135                 140

aat gac tcc ctc att ggc atc gct gcc ggc ctg ctg atg ttt atc gtt      480
Asn Asp Ser Leu Ile Gly Ile Ala Ala Gly Leu Leu Met Phe Ile Val
145                 150                 155                 160

ccc gct aac ttt aaa acc ggc gaa cgc att ctt gat tgg cgt act gca      528
Pro Ala Asn Phe Lys Thr Gly Glu Arg Ile Leu Asp Trp Arg Thr Ala
                165                 170                 175

ggc gaa ctt cca tgg gat gtt ctc ttg ctt ttt ggt ggc ggg ctt tca      576
Gly Glu Leu Pro Trp Asp Val Leu Leu Leu Phe Gly Gly Gly Leu Ser
            180                 185                 190

ctt tct gcg atg ttt acc agc acg gga ctt tcc cta tgg atc ggt gaa      624
Leu Ser Ala Met Phe Thr Ser Thr Gly Leu Ser Leu Trp Ile Gly Glu
        195                 200                 205

cta gct aag gga ctt gat gcc ctt cca atc ttc att ctc atc ttc gcc      672
Leu Ala Lys Gly Leu Asp Ala Leu Pro Ile Phe Ile Leu Ile Phe Ala
    210                 215                 220

att gct gtc ctg gtg ttg ttc ctg acc gag ttc acc tcc aac acc gca      720
Ile Ala Val Leu Val Leu Phe Leu Thr Glu Phe Thr Ser Asn Thr Ala
225                 230                 235                 240

aca gcg gca acc ttc ctg cca atc atg ggt ggc gtc gcc gta ggt atc     768
Thr Ala Ala Thr Phe Leu Pro Ile Met Gly Gly Val Ala Val Gly Ile
                245                 250                 255

gga ctg acc gca ggt ggc gag cag aat gtt ctg ctg ctg acc atc cca     816
Gly Leu Thr Ala Gly Gly Glu Gln Asn Val Leu Leu Leu Thr Ile Pro
            260                 265                 270

gtc gca ctg tcc gca acc tgt gcg ttc atg ctt cca gtg gca acg cct     864
Val Ala Leu Ser Ala Thr Cys Ala Phe Met Leu Pro Val Ala Thr Pro
        275                 280                 285

cca aac gcg att gca ttc ggc tcc ggc tac att aag atc ggc gaa atg     912
Pro Asn Ala Ile Ala Phe Gly Ser Gly Tyr Ile Lys Ile Gly Glu Met
    290                 295                 300

gtc aag ggt ggt ctg tgg ctg aac atc atc gca gtc atc ctc att acg     960
Val Lys Gly Gly Leu Trp Leu Asn Ile Ile Ala Val Ile Leu Ile Thr
305                 310                 315                 320

att ttc acc tac ttc gta gcg atc cca ctc ttt ggc atc atg ctt         1005
Ile Phe Thr Tyr Phe Val Ala Ile Pro Leu Phe Gly Ile Met Leu
                325                 330                 335

taaaagttaa caggcccgca gtc                                           1028


<210> 518
<211> 335
<212> PRT
<213> Corynebacterium glutamicum

<400> 518
Leu Ala Leu Thr Ala Glu Thr Val Gly Gly Met Lys Asn Gln Lys Lys
1               5                   10                  15

Phe Ala Thr Gly Leu Met Leu Ser Ile Ala Tyr Ser Ala Ser Ile Gly
            20                  25                  30

Ser Leu Gly Thr Leu Ile Gly Thr Pro Pro Asn Ala Leu Leu Ala Ala
        35                  40                  45

Tyr Met Ser Glu Ser His Asp Ile His Ile Gly Phe Gly Gln Trp Met
    50                  55                  60

Ile Leu Gly Val Pro Ile Ala Val Val Phe Thr Ile Ile Ala Trp Leu
65                  70                  75                  80

Val Leu Thr Thr Val Phe Lys Pro Glu Met Lys Glu Ile Pro Gly Gly
                85                  90                  95

Arg Glu Leu Ile Lys Arg Glu Ile Ala Glu Met Gly Pro Trp Thr Ala
            100                 105                 110

Pro Gln Val Thr Val Gly Val Ile Phe Ala Ala Ala Ala Leu Ala Trp
        115                 120                 125

Val Phe Ile Pro Leu Thr Leu Asp Trp Thr Gly Ser Gln Leu Ser Ile
    130                 135                 140

Asn Asp Ser Leu Ile Gly Ile Ala Ala Gly Leu Leu Met Phe Ile Val

145                 150                 155                 160

Pro Ala Asn Phe Lys Thr Gly Glu Arg Ile Leu Asp Trp Arg Thr Ala
                165                 170                 175

Gly Glu Leu Pro Trp Asp Val Leu Leu Leu Phe Gly Gly Gly Leu Ser
            180                 185                 190

Leu Ser Ala Met Phe Thr Ser Thr Gly Leu Ser Leu Trp Ile Gly Glu
        195                 200                 205

Leu Ala Lys Gly Leu Asp Ala Leu Pro Ile Phe Ile Leu Ile Phe Ala
    210                 215                 220

Ile Ala Val Leu Val Leu Phe Leu Thr Glu Phe Thr Ser Asn Thr Ala
225                 230                 235                 240

Thr Ala Ala Thr Phe Leu Pro Ile Met Gly Gly Val Ala Val Gly Ile
                245                 250                 255

Gly Leu Thr Ala Gly Gly Glu Gln Asn Val Leu Leu Leu Thr Ile Pro
            260                 265                 270

Val Ala Leu Ser Ala Thr Cys Ala Phe Met Leu Pro Val Ala Thr Pro
        275                 280                 285

Pro Asn Ala Ile Ala Phe Gly Ser Gly Tyr Ile Lys Ile Gly Glu Met
    290                 295                 300

Val Lys Gly Gly Leu Trp Leu Asn Ile Ile Ala Val Ile Leu Ile Thr
305                 310                 315                 320

Ile Phe Thr Tyr Phe Val Ala Ile Pro Leu Phe Gly Ile Met Leu
                325                 330                 335


<210> 519
<211> 448
<212> DNA
<213> Corynebacterium glutamicum

<220>
<221> CDS
<222> (101)..(448)
<223> FRXA01755

<400> 519
tcgttggctt acatggtcat tgatgaccgg gctgaatatg tgagaaaatc catcccttct 60

ttaagcaagg gagtgaatta cagaaaagga ttgttcagca atg agc aca cct gac    115
                                           Met Ser Thr Pro Asp
                                           1               5

att aaa gaa ggc tcg gca gaa tca ccg ggc gaa gta atg gtc gtt gga    163
Ile Lys Glu Gly Ser Ala Glu Ser Pro Gly Glu Val Met Val Val Gly
                10                  15                  20

gac agg cga gag tgg cgt cga caa gca acc ggc atc att gcc ggc ctc    211
Asp Arg Arg Glu Trp Arg Arg Gln Ala Thr Gly Ile Ile Ala Gly Leu
            25                  30                  35

gtc tta gcc gcc ctg gtc tat ctt ctc ttc ccc tcg aac tcc gtg gaa     259
Val Leu Ala Ala Leu Val Tyr Leu Leu Phe Pro Ser Asn Ser Val Glu
        40                  45                  50

acc gtc atg caa tcc agt ggc gtc gat cca gaa act gaa tac acc aac     307
Thr Val Met Gln Ser Ser Gly Val Asp Pro Glu Thr Glu Tyr Thr Asn
    55                  60                  65

aac gcg atg cgt ctt act gct gca gtc aca atc ttg atg gca gtg tgg     355
Asn Ala Met Arg Leu Thr Ala Ala Val Thr Ile Leu Met Ala Val Trp
70                  75                  80                  85

tgg atg aca gaa gca atc cca cta gca gca acc gca ctt atc ccg ttg     403
Trp Met Thr Glu Ala Ile Pro Leu Ala Ala Thr Ala Leu Ile Pro Leu
                90                  95                  100

gtt gca ttc cct gct ttc cag gtt gtg gac ttt ggg aag gca gca         448
Val Ala Phe Pro Ala Phe Gln Val Val Asp Phe Gly Lys Ala Ala
            105                 110                 115

<210> 520
<211> 116
<212> PRT
<213> Corynebacterium glutamicum

<400> 520
Met Ser Thr Pro Asp Ile Lys Glu Gly Ser Ala Glu Ser Pro Gly Glu
  1               5                  10                  15

Val Met Val Val Gly Asp Arg Arg Glu Trp Arg Arg Gln Ala Thr Gly
             20                  25                  30

Ile Ile Ala Gly Leu Val Leu Ala Ala Leu Val Tyr Leu Leu Phe Pro
         35                  40                  45

Ser Asn Ser Val Glu Thr Val Met Gln Ser Ser Gly Val Asp Pro Glu
     50                  55                  60

Thr Glu Tyr Thr Asn Asn Ala Met Arg Leu Thr Ala Ala Val Thr Ile
 65                  70                  75                  80

Leu Met Ala Val Trp Trp Met Thr Glu Ala Ile Pro Leu Ala Ala Thr
                 85                  90                  95

Ala Leu Ile Pro Leu Val Ala Phe Pro Ala Phe Gln Val Val Asp Phe
            100                 105                 110

Gly Lys Ala Ala
        115

<210> 521
<211> 912
<212> DNA
<213> Corynebacterium glutamicum

<220>
<221> CDS
<222> (101)..(889)
<223> RXA00269

<400> 521
tgcgatcttg gtggttgtcg ccatgctgct gccgaggtgg cgtgcgaagt tctccaaggc 60

accgaagcct aagcaaccag tagcagtgga ggcttaagac atg tta tcc atc aac 115
Met Leu Ser Ile Asn
1               5

gga att tct aag acg ttc ttc ccc ggc act gtg aat gag cgc cgc gcg 163
Gly Ile Ser Lys Thr Phe Phe Pro Gly Thr Val Asn Glu Arg Arg Ala
                10                  15                  20

ttg cag cag ctc aaa ctc gat atg gct gag ggc gat ttt gtc acc gtc 211
Leu Gln Gln Leu Lys Leu Asp Met Ala Glu Gly Asp Phe Val Thr Val
            25                  30                  35

atc ggt tct aac ggt gcg ggt aaa tcc acg ctg ctc aac gct gtt tct 259
Ile Gly Ser Asn Gly Ala Gly Lys Ser Thr Leu Leu Asn Ala Val Ser
        40                  45                  50

ggc cgt ttg ctt gtt gat tcc ggc gag att tcc atc gac ggc aac aag 307
Gly Arg Leu Leu Val Asp Ser Gly Glu Ile Ser Ile Asp Gly Asn Lys
    55                  60                  65

gta aac aag atg tca gag cac aag cgt gcc cgc tac atc ggc cgc gtt 355
Val Asn Lys Met Ser Glu His Lys Arg Ala Arg Tyr Ile Gly Arg Val
70                  75                  80                  85

ttc cag gat cct ctg gcc ggc acc gcg ccg aat ctc acc att gaa gag 403
Phe Gln Asp Pro Leu Ala Gly Thr Ala Pro Asn Leu Thr Ile Glu Glu
                90                  95                  100

aat ctg gcc atc gcg ttg ctg cgc ggc aag cgc cgt gga ttg ggc ttt 451
Asn Leu Ala Ile Ala Leu Leu Arg Gly Lys Arg Arg Gly Leu Gly Phe
            105                 110                 115

gca ctg acc tcg aag cgc cgt gag caa ttc aag cag gaa ctt gag cgc 499
Ala Leu Thr Ser Lys Arg Arg Glu Gln Phe Lys Gln Glu Leu Glu Arg
        120                 125                 130

ctt gag ctg ggt ctg gaa aac agg ctc act gcc aaa gtt ggt ttg ctc 547
Leu Glu Leu Gly Leu Glu Asn Arg Leu Thr Ala Lys Val Gly Leu Leu
    135                 140                 145

tct ggc ggt cag cgt cag gca ttg tcc ctg ctg atg gct ggt ttt act 595
Ser Gly Gly Gln Arg Gln Ala Leu Ser Leu Leu Met Ala Gly Phe Thr
150                 155                 160                 165

caa cct aaa atc atg ctg ttg gat gag cac acc gca gcg ctt gat cca 643
Gln Pro Lys Ile Met Leu Leu Asp Glu His Thr Ala Ala Leu Asp Pro
                170                 175                 180

cag cgt gca gag ctt gtg acc acc ttg acc gaa aag atc gtg gca gat 691
Gln Arg Ala Glu Leu Val Thr Thr Leu Thr Glu Lys Ile Val Ala Asp
            185                 190                 195

gga aat ctg act acg ctt atg gtc acg cac aac atg gaa cag gca att 739
Gly Asn Leu Thr Thr Leu Met Val Thr His Asn Met Glu Gln Ala Ile
        200                 205                 210

cgc ctg ggc aat cgc ctg atc atg atg cat gaa ggc cag att gtc tac 787

Arg Leu Gly Asn Arg Leu Ile Met Met His Glu Gly Gln Ile Val Tyr
    215                 220                 225

cag gca gat cag gct acc aag tcg aag ttg act gtg cgc gat ttg ctg     835
Gln Ala Asp Gln Ala Thr Lys Ser Lys Leu Thr Val Arg Asp Leu Leu
230                 235                 240                 245

cag gag ttc gcc aac atc aag ggc gca aca ttg tct gac aag gcg ttc     883
Gln Glu Phe Ala Asn Ile Lys Gly Ala Thr Leu Ser Asp Lys Ala Phe
                250                 255                 260

ctc ggc taaaaagagc ttgctttacg acg                                   912
Leu Gly

<210> 522
<211> 263
<212> PRT
<213> Corynebacterium glutamicum

<400> 522
Met Leu Ser Ile Asn Gly Ile Ser Lys Thr Phe Phe Pro Gly Thr Val
  1               5                  10                  15

Asn Glu Arg Arg Ala Leu Gln Gln Leu Lys Leu Asp Met Ala Glu Gly
             20                  25                  30

Asp Phe Val Thr Val Ile Gly Ser Asn Gly Ala Gly Lys Ser Thr Leu
         35                  40                  45

Leu Asn Ala Val Ser Gly Arg Leu Leu Val Asp Ser Gly Glu Ile Ser
     50                  55                  60

Ile Asp Gly Asn Lys Val Asn Lys Met Ser Glu His Lys Arg Ala Arg
 65                  70                  75                  80

Tyr Ile Gly Arg Val Phe Gln Asp Pro Leu Ala Gly Thr Ala Pro Asn
                 85                  90                  95

Leu Thr Ile Glu Glu Asn Leu Ala Ile Ala Leu Leu Arg Gly Lys Arg
            100                 105                 110

Arg Gly Leu Gly Phe Ala Leu Thr Ser Lys Arg Arg Glu Gln Phe Lys
        115                 120                 125

Gln Glu Leu Glu Arg Leu Glu Leu Gly Leu Glu Asn Arg Leu Thr Ala
    130                 135                 140

Lys Val Gly Leu Leu Ser Gly Gly Gln Arg Gln Ala Leu Ser Leu Leu
145                 150                 155                 160

Met Ala Gly Phe Thr Gln Pro Lys Ile Met Leu Leu Asp Glu His Thr
                165                 170                 175

Ala Ala Leu Asp Pro Gln Arg Ala Glu Leu Val Thr Thr Leu Thr Glu
            180                 185                 190

Lys Ile Val Ala Asp Gly Asn Leu Thr Thr Leu Met Val Thr His Asn
        195                 200                 205

Met Glu Gln Ala Ile Arg Leu Gly Asn Arg Leu Ile Met Met His Glu
    210                 215                 220

Gly Gln Ile Val Tyr Gln Ala Asp Gln Ala Thr Lys Ser Lys Leu Thr
225                 230                 235                 240

Val Arg Asp Leu Leu Gln Glu Phe Ala Asn Ile Lys Gly Ala Thr Leu
                245                 250                 255

Ser Asp Lys Ala Phe Leu Gly
            260

<210> 523
<211> 817
<212> DNA
<213> Corynebacterium glutamicum

<220>
<221> CDS
<222> (101)..(817)
<223> RXA00369

<400> 523
ggcgggaaat tacaatcagg catcggcgtt gtcgttgatg tttgcgatta tcggtatcgt 60

ggcgctcgcg ttgacggtgc gcagccagaa ggagttttag gtg tca tcg atc aaa   115
                                           Val Ser Ser Ile Lys
                                           1               5

ttg cgc gat tta agc gtg agc ttc cgc gac gga acc ttc ggg ctg caa   163
Leu Arg Asp Leu Ser Val Ser Phe Arg Asp Gly Thr Phe Gly Leu Gln
                10                  15                  20

gat atc aat ttg aaa att gag ccg gaa gaa ttt gtg gtg ctc atc ggg   211
Asp Ile Asn Leu Lys Ile Glu Pro Glu Glu Phe Val Val Leu Ile Gly
            25                  30                  35

ccg tcg ggg tcc ggt aaa acc acc atg ttg ggc acc atc gcg ggg ttt   259
Pro Ser Gly Ser Gly Lys Thr Thr Met Leu Gly Thr Ile Ala Gly Phe
        40                  45                  50

gtg gag cca agt tcc ggc agt gtg ctc atc gcc ggc gaa gaa atg acg   307
Val Glu Pro Ser Ser Gly Ser Val Leu Ile Ala Gly Glu Glu Met Thr
    55                  60                  65

cat gtc ccg ccg gag cgc cgt cgc atg ggc atg gtg ttt cag cag cat   355
His Val Pro Pro Glu Arg Arg Arg Met Gly Met Val Phe Gln Gln His
70                  75                  80                  85

gcg gtg tgg ccg cat atg tcg gtc gcc aag aat gtg gga tac ccg ctg   403
Ala Val Trp Pro His Met Ser Val Ala Lys Asn Val Gly Tyr Pro Leu
                90                  95                  100

gcg cga agt ggt cag aag ggg gcg tcg ata agc aaa cgc gtg gag cgc   451
Ala Arg Ser Gly Gln Lys Gly Ala Ser Ile Ser Lys Arg Val Glu Arg
            105                 110                 115

acg ctc gcg ctg gtg ggg ctt gag ggg ttc ggc agt cgc aga ccg gcc   499
Thr Leu Ala Leu Val Gly Leu Glu Gly Phe Gly Ser Arg Arg Pro Ala
        120                 125                 130

agc ctg tct ggt ggt caa cgt cag cgg gtg gcg ctt gcg cgc gcc atc     547
Ser Leu Ser Gly Gly Gln Arg Gln Arg Val Ala Leu Ala Arg Ala Ile
    135                 140                 145

atc gcc gac ccc acc gtg ctg ctt ctc gac gag gcc ctc tcc gcc ctc     595
Ile Ala Asp Pro Thr Val Leu Leu Leu Asp Glu Ala Leu Ser Ala Leu
150                 155                 160                 165

gac gaa ccg ctg cga gac gct tta cga cgc gaa ctc gta tct ttg acc     643
Asp Glu Pro Leu Arg Asp Ala Leu Arg Arg Glu Leu Val Ser Leu Thr
                170                 175                 180

cgg cgc gaa ggc ctc act act gtg cac gtg acg cat gac cgc gcc gaa     691
Arg Arg Glu Gly Leu Thr Thr Val His Val Thr His Asp Arg Ala Glu
            185                 190                 195

gcg atc tcc atc gct gac cgc atc gtc gta ctc ggc aac ggt cga atc     739
Ala Ile Ser Ile Ala Asp Arg Ile Val Val Leu Gly Asn Gly Arg Ile
        200                 205                 210

caa cag gta gcc acc cct act gag ctc ctt tcc gcc ccc gct act gcc     787
Gln Gln Val Ala Thr Pro Thr Glu Leu Leu Ser Ala Pro Ala Thr Ala
    215                 220                 225

gat gtt gcc cga ttc atc gtc gac gcc acc                             817
Asp Val Ala Arg Phe Ile Val Asp Ala Thr
230                 235


<210> 524
<211> 239
<212> PRT
<213> Corynebacterium glutamicum

<400> 524
Val Ser Ser Ile Lys Leu Arg Asp Leu Ser Val Ser Phe Arg Asp Gly
  1               5                  10                  15

Thr Phe Gly Leu Gln Asp Ile Asn Leu Lys Ile Glu Pro Glu Glu Phe
             20                  25                  30

Val Val Leu Ile Gly Pro Ser Gly Ser Gly Lys Thr Thr Met Leu Gly
         35                  40                  45

Thr Ile Ala Gly Phe Val Glu Pro Ser Ser Gly Ser Val Leu Ile Ala
     50                  55                  60

Gly Glu Glu Met Thr His Val Pro Pro Glu Arg Arg Arg Met Gly Met
 65                  70                  75                  80

Val Phe Gln Gln His Ala Val Trp Pro His Met Ser Val Ala Lys Asn
                 85                  90                  95

Val Gly Tyr Pro Leu Ala Arg Ser Gly Gln Lys Gly Ala Ser Ile Ser
            100                 105                 110

Lys Arg Val Glu Arg Thr Leu Ala Leu Val Gly Leu Glu Gly Phe Gly
        115                 120                 125

Ser Arg Arg Pro Ala Ser Leu Ser Gly Gly Gln Arg Gln Arg Val Ala

130                 135                 140

Leu Ala Arg Ala Ile Ile Ala Asp Pro Thr Val Leu Leu Leu Asp Glu
145                 150                 155                 160

Ala Leu Ser Ala Leu Asp Glu Pro Leu Arg Asp Ala Leu Arg Arg Glu
                165                 170                 175

Leu Val Ser Leu Thr Arg Arg Glu Gly Leu Thr Thr Val His Val Thr
            180                 185                 190

His Asp Arg Ala Glu Ala Ile Ser Ile Ala Asp Arg Ile Val Val Leu
        195                 200                 205

Gly Asn Gly Arg Ile Gln Gln Val Ala Thr Pro Thr Glu Leu Leu Ser
    210                 215                 220

Ala Pro Ala Thr Ala Asp Val Ala Arg Phe Ile Val Asp Ala Thr
225                 230                 235


<210> 525
<211> 1653
<212> DNA
<213> Corynebacterium glutamicum

<220>
<221> CDS
<222> (101)..(1630)
<223> RXA02073

<400> 525
tcattgtttt agaggatggc caattgacca tgatggatac acccagcaac gtttcccagc 60

acaatgcgtt tttccgcacc gctgtgatgg aggaagaaca atg att tcc cga ctt   115
                                           Met Ile Ser Arg Leu
                                           1               5

ctc caa ttg gct aag aaa gta tgg ccg gaa ctt gga gcc tcc acg ctc   163
Leu Gln Leu Ala Lys Lys Val Trp Pro Glu Leu Gly Ala Ser Thr Leu
                10                  15                  20

ctg cga ctg ctc aat cag cta ctc acc gca gca ctc att gtg ttc ccc   211
Leu Arg Leu Leu Asn Gln Leu Leu Thr Ala Ala Leu Ile Val Phe Pro
            25                  30                  35

gcc tgg gtg cta agc cgc aaa cca gac atc tcc ctg ctc gcc gtc gcc   259
Ala Trp Val Leu Ser Arg Lys Pro Asp Ile Ser Leu Leu Ala Val Ala
        40                  45                  50

atc atc atg gcg ctc atc gcg ctg aca gca gct gtc tgt cgc tgg ggt   307
Ile Ile Met Ala Leu Ile Ala Leu Thr Ala Ala Val Cys Arg Trp Gly
    55                  60                  65

gag cag gta tgc ggc cac cgc gct gcc ttt ggg ctg ctt gcc cac atg   355
Glu Gln Val Cys Gly His Arg Ala Ala Phe Gly Leu Leu Ala His Met
70                  75                  80                  85

cgc gtt atg ctt tac gac gcc ctc gtc cac aaa ggt tcc ccc tcg ccg   403
Arg Val Met Leu Tyr Asp Ala Leu Val His Lys Gly Ser Pro Ser Pro
                90                  95                  100

atc cac ggc agc ggt tcg atc atg tct gtt gcc acc cgt gac att aac 451
Ile His Gly Ser Gly Ser Ile Met Ser Val Ala Thr Arg Asp Ile Asn
            105                 110                 115

tcc atc gaa gta ttc ttc gcg cac acc att ggg cct aca gtc acc gca 499
Ser Ile Glu Val Phe Phe Ala His Thr Ile Gly Pro Thr Val Thr Ala
        120                 125                 130

gtg ctg ctc agt gcg gga ggc gtg atc acg ctg gca acg ctc gat ccc 547
Val Leu Leu Ser Ala Gly Gly Val Ile Thr Leu Ala Thr Leu Asp Pro
    135                 140                 145

gtt gct ggt cta att ggt tta ctc ggt gtc ctc atc gcg tgg ttg atc 595
Val Ala Gly Leu Ile Gly Leu Leu Gly Val Leu Ile Ala Trp Leu Ile
150                 155                 160                 165

ccc ttg att gga aaa caa tcc tca agc agt gaa gcc aca tca cgt gga 643
Pro Leu Ile Gly Lys Gln Ser Ser Ser Ser Glu Ala Thr Ser Arg Gly
                170                 175                 180

cac atc gcc cag cac ctc acc gaa gat gcc gcc ggc agg ctt gaa atc 691
His Ile Ala Gln His Leu Thr Glu Asp Ala Ala Gly Arg Leu Glu Ile
            185                 190                 195

aac tca cat gga gcg caa gcc aca cgg tta aat gcg ctc gag gtg aaa 739
Asn Ser His Gly Ala Gln Ala Thr Arg Leu Asn Ala Leu Glu Val Lys
        200                 205                 210

gag caa caa ctg gaa caa gtt gtg acc cga cag ggc ttg atc gtc ggt 787
Glu Gln Gln Leu Glu Gln Val Val Thr Arg Gln Gly Leu Ile Val Gly
    215                 220                 225

atc cgt cag ggc gca gca ctt tta tgg ccg tgg ata tca gct gtg ttg 835
Ile Arg Gln Gly Ala Ala Leu Leu Trp Pro Trp Ile Ser Ala Val Leu
230                 235                 240                 245

ttg gtt gct ctg gtt cct cat gtg ggc att gtt gca gcc gcg att atc 883
Leu Val Ala Leu Val Pro His Val Gly Ile Val Ala Ala Ala Ile Ile
                250                 255                 260

ctg ggc atc tcc cct gcg ttg gat gca gtt gag gga ttt gct cgc acc 931
Leu Gly Ile Ser Pro Ala Leu Asp Ala Val Glu Gly Phe Ala Arg Thr
            265                 270                 275

atg cct acc gcg tta aac agt gcg cag cgg tat ttc cag atc atc gat 979
Met Pro Thr Ala Leu Asn Ser Ala Gln Arg Tyr Phe Gln Ile Ile Asp
        280                 285                 290

gcc cct gtt gct atc gct gaa cct gac gag ccg aag cct ttg ccc aaa 1027
Ala Pro Val Ala Ile Ala Glu Pro Asp Glu Pro Lys Pro Leu Pro Lys
    295                 300                 305

ggc ccg ctt aag ctg cga att tct aga gtt cca gtc agc gca aag ggc 1075
Gly Pro Leu Lys Leu Arg Ile Ser Arg Val Pro Val Ser Ala Lys Gly
310                 315                 320                 325

acc gtg tct tta gag gtt gca gct ggt gaa cac atc ggc atc atc gga 1123
Thr Val Ser Leu Glu Val Ala Ala Gly Glu His Ile Gly Ile Ile Gly
                330                 335                 340

tcc agc ggt agt gga aaa tcc act ttg gcc aaa ctc atc ctc aag ctg 1171
Ser Ser Gly Ser Gly Lys Ser Thr Leu Ala Lys Leu Ile Leu Lys Leu
            345                 350                 355

gcg caa cta cgg tct gga acc atc acc atc ggt ggt gtt gat atc gca 1219
Ala Gln Leu Arg Ser Gly Thr Ile Thr Ile Gly Gly Val Asp Ile Ala
        360                 365                 370

gag gtt tca tcg gcg gag ctt cgc aaa tcc gtc acg ctg gtt gag cag 1267
Glu Val Ser Ser Ala Glu Leu Arg Lys Ser Val Thr Leu Val Glu Gln
    375                 380                 385

aaa tct gtg ctg ttt aga gca agc gtg ctg gag aat tta cgg atg ggc 1315
Lys Ser Val Leu Phe Arg Ala Ser Val Leu Glu Asn Leu Arg Met Gly
390                 395                 400                 405

aat cca gag ctg tct gaa gat gaa gca agg gaa gcc ttg agg ttg gcg 1363
Asn Pro Glu Leu Ser Glu Asp Glu Ala Arg Glu Ala Leu Arg Leu Ala
                410                 415                 420

tcg ata agc gaa ctg cct tta gat gct gac gcc ctg cgc cta tct ggc 1411
Ser Ile Ser Glu Leu Pro Leu Asp Ala Asp Ala Leu Arg Leu Ser Gly
            425                 430                 435

gga cag caa caa cgg ctc tgc ctg gcg cgt gct ttg gca cgc acc cct 1459
Gly Gln Gln Gln Arg Leu Cys Leu Ala Arg Ala Leu Ala Arg Thr Pro
        440                 445                 450

cag gta ctg att gtc gat gaa gcc acc agc cac caa gat gcg ctc aat 1507
Gln Val Leu Ile Val Asp Glu Ala Thr Ser His Gln Asp Ala Leu Asn
    455                 460                 465

caa gcg gat ctt tcc cag act ctg gcc acg ctt aaa gac acc acg gtg 1555
Gln Ala Asp Leu Ser Gln Thr Leu Ala Thr Leu Lys Asp Thr Thr Val
470                 475                 480                 485

atc atc att gcg cac cgc aca gct gca tta acc cat gtg gat cgg ata 1603
Ile Ile Ile Ala His Arg Thr Ala Ala Leu Thr His Val Asp Arg Ile
                490                 495                 500

att gac ctg gaa gaa atc aaa aat ccc tgatccttaa cgcggatcag 1650
Ile Asp Leu Glu Glu Ile Lys Asn Pro
            505                 510

ggg 1653

<210> 526
<211> 510
<212> PRT
<213> Corynebacterium glutamicum

<400> 526
Met Ile Ser Arg Leu Leu Gln Leu Ala Lys Lys Val Trp Pro Glu Leu
1               5                   10                  15

Gly Ala Ser Thr Leu Leu Arg Leu Leu Asn Gln Leu Leu Thr Ala Ala
            20                  25                  30

Leu Ile Val Phe Pro Ala Trp Val Leu Ser Arg Lys Pro Asp Ile Ser
        35                  40                  45

Leu Leu Ala Val Ala Ile Ile Met Ala Leu Ile Ala Leu Thr Ala Ala
    50                  55                  60

Val Cys Arg Trp Gly Glu Gln Val Cys Gly His Arg Ala Ala Phe Gly
65                  70                  75                  80

Leu Leu Ala His Met Arg Val Met Leu Tyr Asp Ala Leu Val His Lys
                85                  90                  95

Gly Ser Pro Ser Pro Ile His Gly Ser Gly Ser Ile Met Ser Val Ala
            100                 105                 110

Thr Arg Asp Ile Asn Ser Ile Glu Val Phe Phe Ala His Thr Ile Gly
        115                 120                 125

Pro Thr Val Thr Ala Val Leu Leu Ser Ala Gly Gly Val Ile Thr Leu
    130                 135                 140

Ala Thr Leu Asp Pro Val Ala Gly Leu Ile Gly Leu Leu Gly Val Leu
145                 150                 155                 160

Ile Ala Trp Leu Ile Pro Leu Ile Gly Lys Gln Ser Ser Ser Ser Glu
                165                 170                 175

Ala Thr Ser Arg Gly His Ile Ala Gln His Leu Thr Glu Asp Ala Ala
            180                 185                 190

Gly Arg Leu Glu Ile Asn Ser His Gly Ala Gln Ala Thr Arg Leu Asn
        195                 200                 205

Ala Leu Glu Val Lys Glu Gln Gln Leu Glu Gln Val Val Thr Arg Gln
    210                 215                 220

Gly Leu Ile Val Gly Ile Arg Gln Gly Ala Ala Leu Leu Trp Pro Trp
225                 230                 235                 240

Ile Ser Ala Val Leu Leu Val Ala Leu Val Pro His Val Gly Ile Val
                245                 250                 255

Ala Ala Ala Ile Ile Leu Gly Ile Ser Pro Ala Leu Asp Ala Val Glu
            260                 265                 270

Gly Phe Ala Arg Thr Met Pro Thr Ala Leu Asn Ser Ala Gln Arg Tyr
        275                 280                 285

Phe Gln Ile Ile Asp Ala Pro Val Ala Ile Ala Glu Pro Asp Glu Pro
    290                 295                 300

Lys Pro Leu Pro Lys Gly Pro Leu Lys Leu Arg Ile Ser Arg Val Pro
305                 310                 315                 320

Val Ser Ala Lys Gly Thr Val Ser Leu Glu Val Ala Ala Gly Glu His
                325                 330                 335

Ile Gly Ile Ile Gly Ser Ser Gly Ser Gly Lys Ser Thr Leu Ala Lys
            340                 345                 350

Leu Ile Leu Lys Leu Ala Gln Leu Arg Ser Gly Thr Ile Thr Ile Gly
        355                 360                 365

Gly Val Asp Ile Ala Glu Val Ser Ser Ala Glu Leu Arg Lys Ser Val
    370                 375                 380

Thr Leu Val Glu Gln Lys Ser Val Leu Phe Arg Ala Ser Val Leu Glu
385                 390                 395                 400

Asn Leu Arg Met Gly Asn Pro Glu Leu Ser Glu Asp Glu Ala Arg Glu
                405                 410                 415

Ala Leu Arg Leu Ala Ser Ile Ser Glu Leu Pro Leu Asp Ala Asp Ala
            420                 425                 430

Leu Arg Leu Ser Gly Gly Gln Gln Gln Arg Leu Cys Leu Ala Arg Ala
        435                 440                 445

Leu Ala Arg Thr Pro Gln Val Leu Ile Val Asp Glu Ala Thr Ser His
    450                 455                 460

Gln Asp Ala Leu Asn Gln Ala Asp Leu Ser Gln Thr Leu Ala Thr Leu
465                 470                 475                 480

Lys Asp Thr Thr Val Ile Ile Ile Ala His Arg Thr Ala Ala Leu Thr
                485                 490                 495

His Val Asp Arg Ile Ile Asp Leu Glu Glu Ile Lys Asn Pro
            500                 505                 510

<210> 527
<211> 1142
<212> DNA
<213> Corynebacterium glutamicum

<220>
<221> CDS
<222> (1)..(1119)
<223> RXA01399

<400> 527
att ctc agc ccc gca acc gca ctt gtc ctg gcg att gga ttg att gct 48
Ile Leu Ser Pro Ala Thr Ala Leu Val Leu Ala Ile Gly Leu Ile Ala
1               5                   10                  15

gct gca att atc cct ccc ctg ctt gct gct cgc gga gtt aaa aca gcc 96
Ala Ala Ile Ile Pro Pro Leu Leu Ala Ala Arg Gly Val Lys Thr Ala
            20                  25                  30

gaa gcc cgg cgc gct gaa tcc agc gaa gcc tac ttg agt tcc ttg gat 144
Glu Ala Arg Arg Ala Glu Ser Ser Glu Ala Tyr Leu Ser Ser Leu Asp
        35                  40                  45

cag gtg ctg tcc aac cag gcg gcg ctt cgt gtt cgt ggt gaa atg ccg 192
Gln Val Leu Ser Asn Gln Ala Ala Leu Arg Val Arg Gly Glu Met Pro
    50                  55                  60

gcc gct ctg tcc aag gcg gat gtg gct gcg cgt tcc tat tct tct tca 240
Ala Ala Leu Ser Lys Ala Asp Val Ala Ala Arg Ser Tyr Ser Ser Ser
65                  70                  75                  80

ctg gag gca ggc gcg aaa gac act gcc att ggc gca gcg agt tcc ctg 288
Leu Glu Ala Gly Ala Lys Asp Thr Ala Ile Gly Ala Ala Ser Ser Leu

85                  90                  95

tgg att cac ggt ttc act gtc att ggt gtg ctc atg gtt tcc gcg tca     336
Trp Ile His Gly Phe Thr Val Ile Gly Val Leu Met Val Ser Ala Ser
            100                 105                 110

ctg tat gca gat gga agc cat tca ccg cag tgg ttt ggt gtg ttg gtg     384
Leu Tyr Ala Asp Gly Ser His Ser Pro Gln Trp Phe Gly Val Leu Val
        115                 120                 125

ctg ctt tca ctc gca gct ttc gag gct gtc tct gtt ctc ccc gat gct     432
Leu Leu Ser Leu Ala Ala Phe Glu Ala Val Ser Val Leu Pro Asp Ala
    130                 135                 140

gcg att gct cgt acc cgc gcc gca gat gcc acc agg agg ctt gcg gaa     480
Ala Ile Ala Arg Thr Arg Ala Ala Asp Ala Thr Arg Arg Leu Ala Glu
145                 150                 155                 160

atc tcg gcg ctg cca gaa tct gtc tct ctt gag ctt cgc acg gcc tct     528
Ile Ser Ala Leu Pro Glu Ser Val Ser Leu Glu Leu Arg Thr Ala Ser
                165                 170                 175

gac cag ccc gta tta cgc gcc gag aat cta gtt tat gga tgg gac agc     576
Asp Gln Pro Val Leu Arg Ala Glu Asn Leu Val Tyr Gly Trp Asp Ser
            180                 185                 190

gac cta ggc acg agc aac ctg gat ctc acc ttt ggt tca cga cat gaa     624
Asp Leu Gly Thr Ser Asn Leu Asp Leu Thr Phe Gly Ser Arg His Glu
        195                 200                 205

atc atc gca ccc tct gga act ggc aaa acg acc ctg ctg ctc aca ctt     672
Ile Ile Ala Pro Ser Gly Thr Gly Lys Thr Thr Leu Leu Leu Thr Leu
    210                 215                 220

gcg ggg ctg ttg gaa cct cgt gga ggc caa gtg ctt atc gac ggc acc     720
Ala Gly Leu Leu Glu Pro Arg Gly Gly Gln Val Leu Ile Asp Gly Thr
225                 230                 235                 240

aat cct tcc gag ttg aaa aac gcc gtg ctg ttc agt cca gaa gat gcc     768
Asn Pro Ser Glu Leu Lys Asn Ala Val Leu Phe Ser Pro Glu Asp Ala
                245                 250                 255

cac att ttt gcc acc act gtc cga gat aac tta gca ctc gga gca ccg     816
His Ile Phe Ala Thr Thr Val Arg Asp Asn Leu Ala Leu Gly Ala Pro
            260                 265                 270

gaa gca acc gac gcg gaa atg aca tcg atc ctg gaa cat gtt ggt ttg     864
Glu Ala Thr Asp Ala Glu Met Thr Ser Ile Leu Glu His Val Gly Leu
        275                 280                 285

tca gag tgg gtt caa ggt tta ccc gat ggt ctt ggc act gtc ctt gat     912
Ser Glu Trp Val Gln Gly Leu Pro Asp Gly Leu Gly Thr Val Leu Asp
    290                 295                 300

tca ggt gcc gat agt ctc tcg gga ggt cag cgc cgc cgc ctg ctc ctt     960
Ser Gly Ala Asp Ser Leu Ser Gly Gly Gln Arg Arg Arg Leu Leu Leu
305                 310                 315                 320

gcc cgc gta cta cta agt gat gca cca att ctg ctt ttg gat gaa ccc    1008
Ala Arg Val Leu Leu Ser Asp Ala Pro Ile Leu Leu Leu Asp Glu Pro
                325                 330                 335

acc gag cac ctc gac act gca ggc tcc tct gaa atc ttg tct atg ctg     1056
Thr Glu His Leu Asp Thr Ala Gly Ser Ser Glu Ile Leu Ser Met Leu
            340                 345                 350

gcc tcc gat gaa ctc cct ggt aaa aga gct agg aga acc gta gtg att     1104
Ala Ser Asp Glu Leu Pro Gly Lys Arg Ala Arg Arg Thr Val Val Ile
        355                 360                 365

gtg agg cat gtg agg taattttgcc ccgctgtctt ctg                       1142
Val Arg His Val Arg
    370

<210> 528
<211> 373
<212> PRT
<213> Corynebacterium glutamicum

<400> 528
Ile Leu Ser Pro Ala Thr Ala Leu Val Leu Ala Ile Gly Leu Ile Ala
 1               5                  10                  15

Ala Ala Ile Ile Pro Pro Leu Leu Ala Ala Arg Gly Val Lys Thr Ala
            20                  25                  30

Glu Ala Arg Arg Ala Glu Ser Ser Glu Ala Tyr Leu Ser Ser Leu Asp
        35                  40                  45

Gln Val Leu Ser Asn Gln Ala Ala Leu Arg Val Arg Gly Glu Met Pro
    50                  55                  60

Ala Ala Leu Ser Lys Ala Asp Val Ala Ala Arg Ser Tyr Ser Ser Ser
65                  70                  75                  80

Leu Glu Ala Gly Ala Lys Asp Thr Ala Ile Gly Ala Ala Ser Ser Leu
                85                  90                  95

Trp Ile His Gly Phe Thr Val Ile Gly Val Leu Met Val Ser Ala Ser
            100                 105                 110

Leu Tyr Ala Asp Gly Ser His Ser Pro Gln Trp Phe Gly Val Leu Val
        115                 120                 125

Leu Leu Ser Leu Ala Ala Phe Glu Ala Val Ser Val Leu Pro Asp Ala
    130                 135                 140

Ala Ile Ala Arg Thr Arg Ala Ala Asp Ala Thr Arg Arg Leu Ala Glu
145                 150                 155                 160

Ile Ser Ala Leu Pro Glu Ser Val Ser Leu Glu Leu Arg Thr Ala Ser
                165                 170                 175

Asp Gln Pro Val Leu Arg Ala Glu Asn Leu Val Tyr Gly Trp Asp Ser
            180                 185                 190

Asp Leu Gly Thr Ser Asn Leu Asp Leu Thr Phe Gly Ser Arg His Glu
        195                 200                 205

Ile Ile Ala Pro Ser Gly Thr Gly Lys Thr Thr Leu Leu Leu Thr Leu
    210                 215                 220

Ala Gly Leu Leu Glu Pro Arg Gly Gly Gln Val Leu Ile Asp Gly Thr
225                 230                 235                 240

Asn Pro Ser Glu Leu Lys Asn Ala Val Leu Phe Ser Pro Glu Asp Ala
                245                 250                 255

His Ile Phe Ala Thr Thr Val Arg Asp Asn Leu Ala Leu Gly Ala Pro
            260                 265                 270

Glu Ala Thr Asp Ala Glu Met Thr Ser Ile Leu Glu His Val Gly Leu
        275                 280                 285

Ser Glu Trp Val Gln Gly Leu Pro Asp Gly Leu Gly Thr Val Leu Asp
    290                 295                 300

Ser Gly Ala Asp Ser Leu Ser Gly Gly Gln Arg Arg Arg Leu Leu Leu
305                 310                 315                 320

Ala Arg Val Leu Leu Ser Asp Ala Pro Ile Leu Leu Leu Asp Glu Pro
                325                 330                 335

Thr Glu His Leu Asp Thr Ala Gly Ser Ser Glu Ile Leu Ser Met Leu
            340                 345                 350

Ala Ser Asp Glu Leu Pro Gly Lys Arg Ala Arg Arg Thr Val Val Ile
        355                 360                 365

Val Arg His Val Arg
    370

<210> 529
<211> 1368
<212> DNA
<213> Corynebacterium glutamicum

<220>
<221> CDS
<222> (101)..(1345)
<223> RXA01339

<400> 529
gagaaggcgg aaccgaagga atcgaagaat atctctccgt gcgttacctc gctttgccgt 60

gacacatgag ctgtccggtg aataacccga aggaaaaaac atg act acc gaa tca    115
                                           Met Thr Thr Glu Ser
                                           1               5

ata gtt gcg cac aat gct gca ggt aca gca cct cag aat gtg tcc tct    163
Ile Val Ala His Asn Ala Ala Gly Thr Ala Pro Gln Asn Val Ser Ser
                10                  15                  20

gct aag aag aag tac ctc agc gtt gcc caa ggc gtt gcc ctt atc tac    211
Ala Lys Lys Lys Tyr Leu Ser Val Ala Gln Gly Val Ala Leu Ile Tyr
            25                  30                  35

gga acc aac atc ggc gcc ggt gtg ctc agc ctc cca tac gct gcc cgc    259
Gly Thr Asn Ile Gly Ala Gly Val Leu Ser Leu Pro Tyr Ala Ala Arg
        40                  45                  50

aac ggt ggt ttc ctc gcc ctg gtt gtt gcc ctg ctc att gcg gga aca 307
Asn Gly Gly Phe Leu Ala Leu Val Val Ala Leu Leu Ile Ala Gly Thr
    55                  60                  65

ctg acc acc atc tcc atg ctc tac atc gcc gaa gta tcc ctg cgc acc 355
Leu Thr Thr Ile Ser Met Leu Tyr Ile Ala Glu Val Ser Leu Arg Thr
70                  75                  80                  85

aag aag cca ctg cag ctt tcc ggc ctg gca gaa aaa tac ctg ggg cag 403
Lys Lys Pro Leu Gln Leu Ser Gly Leu Ala Glu Lys Tyr Leu Gly Gln
                90                  95                  100

tgg ggc cgc tgg ctg gtg ttc att gcc att gtg gtc aac agc gtg ggt 451
Trp Gly Arg Trp Leu Val Phe Ile Ala Ile Val Val Asn Ser Val Gly
            105                 110                 115

gca ctg att gcc tac gca tca gga tcc ggc att ttg ata ggc aac ctc 499
Ala Leu Ile Ala Tyr Ala Ser Gly Ser Gly Ile Leu Ile Gly Asn Leu
        120                 125                 130

acc ggc ctg cca cca atc gtg ggc acc ctt gga ttc ttt gtt tta ggc 547
Thr Gly Leu Pro Pro Ile Val Gly Thr Leu Gly Phe Phe Val Leu Gly
    135                 140                 145

acc ttg atc atg tgg aaa ggc ctg cac acg gca agc ttc gtg gag gca 595
Thr Leu Ile Met Trp Lys Gly Leu His Thr Ala Ser Phe Val Glu Ala
150                 155                 160                 165

ttg atc acc act ggc atg gca acg atc atc atc gtg ctg tgt gga tgg 643
Leu Ile Thr Thr Gly Met Ala Thr Ile Ile Ile Val Leu Cys Gly Trp
                170                 175                 180

aca gtg ctt ggc cct gga att tcc gca gac aac ctg atc gtg ttc cac 691
Thr Val Leu Gly Pro Gly Ile Ser Ala Asp Asn Leu Ile Val Phe His
            185                 190                 195

cca ttc ttc atc gtt ccg atc atg aac ctc gcg gtc ttt acc ttc ctt 739
Pro Phe Phe Ile Val Pro Ile Met Asn Leu Ala Val Phe Thr Phe Leu
        200                 205                 210

gcc caa tat gtg gtt cca gaa atc gca cga gga gtt aac cct gcc acc 787
Ala Gln Tyr Val Val Pro Glu Ile Ala Arg Gly Val Asn Pro Ala Thr
    215                 220                 225

ccg aag gca gtg cca cgc gcg atc atc atc ggc atg gtc gca act ggt 835
Pro Lys Ala Val Pro Arg Ala Ile Ile Ile Gly Met Val Ala Thr Gly
230                 235                 240                 245

gtc acc ctg gca gct gta cca ttc gcg gca cta ggg ctt ctg ggc aca 883
Val Thr Leu Ala Ala Val Pro Phe Ala Ala Leu Gly Leu Leu Gly Thr
                250                 255                 260

ggc gtc agt gaa gtt gtc acc atc tcc tgg ggc gaa gca ctc gcc cca 931
Gly Val Ser Glu Val Val Thr Ile Ser Trp Gly Glu Ala Leu Ala Pro
            265                 270                 275

gtg gcc tat tac atg gcc aat gcc ttc gca cta ttg gcc atg ttc act 979
Val Ala Tyr Tyr Met Ala Asn Ala Phe Ala Leu Leu Ala Met Phe Thr
        280                 285                 290

tca ttc atc gcc att gga ttc acc gcg atg cgc aac gta cta gat att 1027

Ser Phe Ile Ala Ile Gly Phe Thr Ala Met Arg Asn Val Leu Asp Ile
    295                 300                 305

ggc cac tgg cca caa cac gga tgg cag cga tcc gtt gct gtc gga cta    1075
Gly His Trp Pro Gln His Gly Trp Gln Arg Ser Val Ala Val Gly Leu
310                 315                 320                 325

act gtt ctt cca cca ctg gca att tca ctt gcg gga tta ggc gga ttc    1123
Thr Val Leu Pro Pro Leu Ala Ile Ser Leu Ala Gly Leu Gly Gly Phe
                330                 335                 340

gtg gca gca ctg agc tac gcc gga gga ttt gcc gga gca atc atg tcc    1171
Val Ala Ala Leu Ser Tyr Ala Gly Gly Phe Ala Gly Ala Ile Met Ser
            345                 350                 355

atc atc ccc gtg ctg ttg ctg cgc aac tcc cgc aag agt ggc gac caa    1219
Ile Ile Pro Val Leu Leu Leu Arg Asn Ser Arg Lys Ser Gly Asp Gln
        360                 365                 370

gaa cca gtg tgg aaa gcc acc tgg caa gcg cac ccc atc ttc cag atc    1267
Glu Pro Val Trp Lys Ala Thr Trp Gln Ala His Pro Ile Phe Gln Ile
    375                 380                 385

ctg ttg att gtg gtg tac tcc ctg gcg ttt gtg tac tcg gtt ctc gcg    1315
Leu Leu Ile Val Val Tyr Ser Leu Ala Phe Val Tyr Ser Val Leu Ala
390                 395                 400                 405

atc gtc gga tta atg cct gcg ggt tgg gca tagtcacttc ctgttgtggc      1365
Ile Val Gly Leu Met Pro Ala Gly Trp Ala
                410                 415

tgc                                                                1368

<210> 530
<211> 415
<212> PRT
<213> Corynebacterium glutamicum

<400> 530
Met Thr Thr Glu Ser Ile Val Ala His Asn Ala Ala Gly Thr Ala Pro
  1               5                  10                  15

Gln Asn Val Ser Ser Ala Lys Lys Lys Tyr Leu Ser Val Ala Gln Gly
             20                  25                  30

Val Ala Leu Ile Tyr Gly Thr Asn Ile Gly Ala Gly Val Leu Ser Leu
         35                  40                  45

Pro Tyr Ala Ala Arg Asn Gly Gly Phe Leu Ala Leu Val Val Ala Leu
     50                  55                  60

Leu Ile Ala Gly Thr Leu Thr Thr Ile Ser Met Leu Tyr Ile Ala Glu
 65                  70                  75                  80

Val Ser Leu Arg Thr Lys Lys Pro Leu Gln Leu Ser Gly Leu Ala Glu
                 85                  90                  95

Lys Tyr Leu Gly Gln Trp Gly Arg Trp Leu Val Phe Ile Ala Ile Val
            100                 105                 110

Val Asn Ser Val Gly Ala Leu Ile Ala Tyr Ala Ser Gly Ser Gly Ile
        115                 120                 125

Leu Ile Gly Asn Leu Thr Gly Leu Pro Pro Ile Val Gly Thr Leu Gly
    130                 135                 140

Phe Phe Val Leu Gly Thr Leu Ile Met Trp Lys Gly Leu His Thr Ala
145                 150                 155                 160

Ser Phe Val Glu Ala Leu Ile Thr Thr Gly Met Ala Thr Ile Ile Ile
                165                 170                 175

Val Leu Cys Gly Trp Thr Val Leu Gly Pro Gly Ile Ser Ala Asp Asn
            180                 185                 190

Leu Ile Val Phe His Pro Phe Phe Ile Val Pro Ile Met Asn Leu Ala
        195                 200                 205

Val Phe Thr Phe Leu Ala Gln Tyr Val Val Pro Glu Ile Ala Arg Gly
    210                 215                 220

Val Asn Pro Ala Thr Pro Lys Ala Val Pro Arg Ala Ile Ile Ile Gly
225                 230                 235                 240

Met Val Ala Thr Gly Val Thr Leu Ala Ala Val Pro Phe Ala Ala Leu
                245                 250                 255

Gly Leu Leu Gly Thr Gly Val Ser Glu Val Val Thr Ile Ser Trp Gly
            260                 265                 270

Glu Ala Leu Ala Pro Val Ala Tyr Tyr Met Ala Asn Ala Phe Ala Leu
        275                 280                 285

Leu Ala Met Phe Thr Ser Phe Ile Ala Ile Gly Phe Thr Ala Met Arg
    290                 295                 300

Asn Val Leu Asp Ile Gly His Trp Pro Gln His Gly Trp Gln Arg Ser
305                 310                 315                 320

Val Ala Val Gly Leu Thr Val Leu Pro Pro Leu Ala Ile Ser Leu Ala
                325                 330                 335

Gly Leu Gly Gly Phe Val Ala Ala Leu Ser Tyr Ala Gly Gly Phe Ala
            340                 345                 350

Gly Ala Ile Met Ser Ile Ile Pro Val Leu Leu Leu Arg Asn Ser Arg
        355                 360                 365

Lys Ser Gly Asp Gln Glu Pro Val Trp Lys Ala Thr Trp Gln Ala His
    370                 375                 380

Pro Ile Phe Gln Ile Leu Leu Ile Val Val Tyr Ser Leu Ala Phe Val
385                 390                 395                 400

Tyr Ser Val Leu Ala Ile Val Gly Leu Met Pro Ala Gly Trp Ala
                405                 410                 415

<210> 531
<211> 1452
<212> DNA

<213> Corynebacterium glutamicum

<220>
<221> CDS
<222> (101)..(1429)
<223> RXA02527

<400> 531
attggagcaa tcttttaatc aagttgggaa ttccagcgtt gattgtggtt gccatgtggt 60

ttgcccgcac cctgaagcac tcacggatca agcgtggcgg atg ttt ggc ctg ttc 115
Met Phe Gly Leu Phe
1               5

atc gcg acg att gtg gcg att att ctc aag cca atg cca atg ggt gcc 163
Ile Ala Thr Ile Val Ala Ile Ile Leu Lys Pro Met Pro Met Gly Ala
                10                  15                  20

gtg aca att atc ggc atg atc gcc gcg gtg ttg act ggt ttg gtg ccg 211
Val Thr Ile Ile Gly Met Ile Ala Ala Val Leu Thr Gly Leu Val Pro
            25                  30                  35

ttg acg gcg tct tct gat gat ccc ggc gcg gtg tat ggc ctt att ggt 259
Leu Thr Ala Ser Ser Asp Asp Pro Gly Ala Val Tyr Gly Leu Ile Gly
        40                  45                  50

ttc agt aac ggc acc att tgg ctg att gtg atg gcg ttc ctg att tcg 307
Phe Ser Asn Gly Thr Ile Trp Leu Ile Val Met Ala Phe Leu Ile Ser
    55                  60                  65

cgt gga ttc atc aag acg ggg ctt gga cgt cga ata gcg ttg ttc ttt 355
Arg Gly Phe Ile Lys Thr Gly Leu Gly Arg Arg Ile Ala Leu Phe Phe
70                  75                  80                  85

gtg tct aaa gtc ggc gga aaa atg ctg ggt gtg acc tat ggt ttg gcg 403
Val Ser Lys Val Gly Gly Lys Met Leu Gly Val Thr Tyr Gly Leu Ala
                90                  95                  100

ctc gct gat ttg gtg ttg gct cct gcg att cca tca gca act gcc cga 451
Leu Ala Asp Leu Val Leu Ala Pro Ala Ile Pro Ser Ala Thr Ala Arg
            105                 110                 115

ggt ggt ggc att atg gct ccg att atg aag tcg gtg gca ttg act tat 499
Gly Gly Gly Ile Met Ala Pro Ile Met Lys Ser Val Ala Leu Thr Tyr
        120                 125                 130

gat tcc act cct ggc cca act cgt cgc agg gct ggc gcg ttc ttg gcg 547
Asp Ser Thr Pro Gly Pro Thr Arg Arg Arg Ala Gly Ala Phe Leu Ala
    135                 140                 145

ctg aat gtg gga cag gta aat gcg att acg tgc gcg atg ttt cta act 595
Leu Asn Val Gly Gln Val Asn Ala Ile Thr Cys Ala Met Phe Leu Thr
150                 155                 160                 165

gca atg gca gga aac ccc ttg atc gcc tct ttg gct tcg cag atg gat 643
Ala Met Ala Gly Asn Pro Leu Ile Ala Ser Leu Ala Ser Gln Met Asp
                170                 175                 180

gtc aat atc acg tgg aca aac tgg gct gtg ggt gcg att gtg cct ggt 691
Val Asn Ile Thr Trp Thr Asn Trp Ala Val Gly Ala Ile Val Pro Gly
            185                 190                 195

ctg gtg gcg ctt att gtg gtg ccg tgg gtg gta tac aag atc tat cca 739
Leu Val Ala Leu Ile Val Val Pro Trp Val Val Tyr Lys Ile Tyr Pro
        200                 205                 210

cct gag ttg aag gac acc cct gag gtc aag aaa atg gct tct gat gag 787
Pro Glu Leu Lys Asp Thr Pro Glu Val Lys Lys Met Ala Ser Asp Glu
    215                 220                 225

ctc aag caa ttg ggt ggg ttt act tat ggt gag aag gtg ctg gcg gga 835
Leu Lys Gln Leu Gly Gly Phe Thr Tyr Gly Glu Lys Val Leu Ala Gly
230                 235                 240                 245

acc ttt gtt gtg ttg ctg ctg ctc tgg aca ggt ggc gat ttg gtc ttg 883
Thr Phe Val Val Leu Leu Leu Leu Trp Thr Gly Gly Asp Leu Val Leu
                250                 255                 260

gga atc tcg gca act acc acc gct ttc gtt ggc gtc atc atc ttg cta 931
Gly Ile Ser Ala Thr Thr Thr Ala Phe Val Gly Val Ile Ile Leu Leu
            265                 270                 275

gtg gct cac gtg ctg acg tgg gag gac atc att caa gaa aag act gcg 979
Val Ala His Val Leu Thr Trp Glu Asp Ile Ile Gln Glu Lys Thr Ala
        280                 285                 290

tgg gac acc atg gtg tgg ttc gcg gtg cta tac atg atg gca aca gcg 1027
Trp Asp Thr Met Val Trp Phe Ala Val Leu Tyr Met Met Ala Thr Ala
    295                 300                 305

tta tcg cag tac gga ttc atc gca tgg atc tct gag gta att gct tcc 1075
Leu Ser Gln Tyr Gly Phe Ile Ala Trp Ile Ser Glu Val Ile Ala Ser
310                 315                 320                 325

agt ttg ggt ggc atg aac tgg gtc gtt gct ttg gtt gtg ttg gtg ctg 1123
Ser Leu Gly Gly Met Asn Trp Val Val Ala Leu Val Val Leu Val Leu
                330                 335                 340

att tac ttc ttc agt cac tat ttc ttt gcc tcg gca aca gcg cat att 1171
Ile Tyr Phe Phe Ser His Tyr Phe Phe Ala Ser Ala Thr Ala His Ile
            345                 350                 355

tct gcg atg tac ttg gcc ttc ctg ggt gct gcg att gcg att ggt gca 1219
Ser Ala Met Tyr Leu Ala Phe Leu Gly Ala Ala Ile Ala Ile Gly Ala
        360                 365                 370

ccc ccg ttg atg gcg gcc ctg gtg ttg gcg tac acc tcc aat ttg ttc 1267
Pro Pro Leu Met Ala Ala Leu Val Leu Ala Tyr Thr Ser Asn Leu Phe
    375                 380                 385

tct tca ctc act cag tat tct ggt ggt cct tcg cca aca ttg ttt ggt 1315
Ser Ser Leu Thr Gln Tyr Ser Gly Gly Pro Ser Pro Thr Leu Phe Gly
390                 395                 400                 405

ttg aac tac atc acg gtg ggt gag tgg tgg cgg acc tcg gca att gct 1363
Leu Asn Tyr Ile Thr Val Gly Glu Trp Trp Arg Thr Ser Ala Ile Ala
                410                 415                 420

ggc gcg gta tcg att aca atc tgg ttg gtt atc ggt ggt ttg tgg atg 1411
Gly Ala Val Ser Ile Thr Ile Trp Leu Val Ile Gly Gly Leu Trp Met
            425                 430                 435

aat gtc atc gga ctc tgg taatcgaaaa ttaaaggtaa ggg 1452
Asn Val Ile Gly Leu Trp
        440

<210> 532
<211> 443
<212> PRT
<213> Corynebacterium glutamicum

<400> 532
Met Phe Gly Leu Phe Ile Ala Thr Ile Val Ala Ile Ile Leu Lys Pro
1               5                   10                  15

Met Pro Met Gly Ala Val Thr Ile Ile Gly Met Ile Ala Ala Val Leu
            20                  25                  30

Thr Gly Leu Val Pro Leu Thr Ala Ser Ser Asp Asp Pro Gly Ala Val
        35                  40                  45

Tyr Gly Leu Ile Gly Phe Ser Asn Gly Thr Ile Trp Leu Ile Val Met
    50                  55                  60

Ala Phe Leu Ile Ser Arg Gly Phe Ile Lys Thr Gly Leu Gly Arg Arg
65                  70                  75                  80

Ile Ala Leu Phe Phe Val Ser Lys Val Gly Gly Lys Met Leu Gly Val
                85                  90                  95

Thr Tyr Gly Leu Ala Leu Ala Asp Leu Val Leu Ala Pro Ala Ile Pro
            100                 105                 110

Ser Ala Thr Ala Arg Gly Gly Gly Ile Met Ala Pro Ile Met Lys Ser
        115                 120                 125

Val Ala Leu Thr Tyr Asp Ser Thr Pro Gly Pro Thr Arg Arg Arg Ala
    130                 135                 140

Gly Ala Phe Leu Ala Leu Asn Val Gly Gln Val Asn Ala Ile Thr Cys
145                 150                 155                 160

Ala Met Phe Leu Thr Ala Met Ala Gly Asn Pro Leu Ile Ala Ser Leu
                165                 170                 175

Ala Ser Gln Met Asp Val Asn Ile Thr Trp Thr Asn Trp Ala Val Gly
            180                 185                 190

Ala Ile Val Pro Gly Leu Val Ala Leu Ile Val Val Pro Trp Val Val
        195                 200                 205

Tyr Lys Ile Tyr Pro Pro Glu Leu Lys Asp Thr Pro Glu Val Lys Lys
    210                 215                 220

Met Ala Ser Asp Glu Leu Lys Gln Leu Gly Gly Phe Thr Tyr Gly Glu
225                 230                 235                 240

Lys Val Leu Ala Gly Thr Phe Val Val Leu Leu Leu Leu Trp Thr Gly
                245                 250                 255

Gly Asp Leu Val Leu Gly Ile Ser Ala Thr Thr Thr Ala Phe Val Gly
            260                 265                 270

Val Ile Ile Leu Leu Val Ala His Val Leu Thr Trp Glu Asp Ile Ile
        275                 280                 285

Gln Glu Lys Thr Ala Trp Asp Thr Met Val Trp Phe Ala Val Leu Tyr
    290                 295                 300

Met Met Ala Thr Ala Leu Ser Gln Tyr Gly Phe Ile Ala Trp Ile Ser
305                 310                 315                 320

Glu Val Ile Ala Ser Ser Leu Gly Gly Met Asn Trp Val Val Ala Leu
                325                 330                 335

Val Val Leu Val Leu Ile Tyr Phe Phe Ser His Tyr Phe Phe Ala Ser
            340                 345                 350

Ala Thr Ala His Ile Ser Ala Met Tyr Leu Ala Phe Leu Gly Ala Ala
        355                 360                 365

Ile Ala Ile Gly Ala Pro Pro Leu Met Ala Ala Leu Val Leu Ala Tyr
    370                 375                 380

Thr Ser Asn Leu Phe Ser Ser Leu Thr Gln Tyr Ser Gly Gly Pro Ser
385                 390                 395                 400

Pro Thr Leu Phe Gly Leu Asn Tyr Ile Thr Val Gly Glu Trp Trp Arg
                405                 410                 415

Thr Ser Ala Ile Ala Gly Ala Val Ser Ile Thr Ile Trp Leu Val Ile
            420                 425                 430

Gly Gly Leu Trp Met Asn Val Ile Gly Leu Trp
        435                 440


<210> 533
<211> 1968
<212> DNA
<213> Corynebacterium glutamicum

<220>
<221> CDS
<222> (101)..(1945)
<223> RXN00298

<400> 533
tttagacaag ttctggttaa aattcttcat gaaggtgaga atctgggaat ttctcggtac 60

tctttcagat tcgtagttat ccactgattg gaagaatgag atg agc tca aat ata   115
                                           Met Ser Ser Asn Ile
                                           1               5

gct atc acg acc gag cct gaa ggg aaa aat aaa aag ggt ctc aaa tca   163
Ala Ile Thr Thr Glu Pro Glu Gly Lys Asn Lys Lys Gly Leu Lys Ser
                10                  15                  20

gac ccg ttc att ttt tcc att tct gtc ggt ttt atc gtg gtg ttt gtc   211
Asp Pro Phe Ile Phe Ser Ile Ser Val Gly Phe Ile Val Val Phe Val
            25                  30                  35

atc gcc aca att gcg cta ggc gag aaa gct cga aca acc ttt tcc gcg   259

Ile Ala Thr Ile Ala Leu Gly Glu Lys Ala Arg Thr Thr Phe Ser Ala
        40                  45                  50

att gcc ggc tgg ctc tta gaa aat tta ggg tgg atg tat atc ggg ggt     307
Ile Ala Gly Trp Leu Leu Glu Asn Leu Gly Trp Met Tyr Ile Gly Gly
    55                  60                  65

gtc tcc ttg gtt ttc att ttc ctc atg ggt atc ttt gcg tcc cgg tat     355
Val Ser Leu Val Phe Ile Phe Leu Met Gly Ile Phe Ala Ser Arg Tyr
70                  75                  80                  85

ggc cgg gta aaa ctt ggt gat gac gat gat gac ccc gag cac acc cta     403
Gly Arg Val Lys Leu Gly Asp Asp Asp Asp Asp Pro Glu His Thr Leu
                90                  95                  100

atc gtg tgg ttc tgt atg ctt ttt gct ggc ggt gtc ggt gca gtc tta     451
Ile Val Trp Phe Cys Met Leu Phe Ala Gly Gly Val Gly Ala Val Leu
            105                 110                 115

atg ttt tgg ggt gtt gcc gaa ccg att aac cac gcg ttc aac gtg cca     499
Met Phe Trp Gly Val Ala Glu Pro Ile Asn His Ala Phe Asn Val Pro
        120                 125                 130

atg gct aat gaa gaa tcc atg agt gaa gcc gca att gtg cag gct ttt     547
Met Ala Asn Glu Glu Ser Met Ser Glu Ala Ala Ile Val Gln Ala Phe
    135                 140                 145

gct tat act ttc tat cac ttc ggt att cac atg tgg gta atc atg gca     595
Ala Tyr Thr Phe Tyr His Phe Gly Ile His Met Trp Val Ile Met Ala
150                 155                 160                 165

ctc cca gga tta tca ttg gga tac ttt att tac aaa cgt aag cta cct     643
Leu Pro Gly Leu Ser Leu Gly Tyr Phe Ile Tyr Lys Arg Lys Leu Pro
                170                 175                 180

ccc cgt cta tcc tct gtg ttt tct ccg atc ttg ggt aag cac att tat     691
Pro Arg Leu Ser Ser Val Phe Ser Pro Ile Leu Gly Lys His Ile Tyr
            185                 190                 195

tcc aca ccc ggc aag ctc atc gat gta ctg gcc atc gta ggc acc acg     739
Ser Thr Pro Gly Lys Leu Ile Asp Val Leu Ala Ile Val Gly Thr Thr
        200                 205                 210

ttt ggt att gct gtg tca gta ggt ctt ggt gtg ctg caa atc aat gca     787
Phe Gly Ile Ala Val Ser Val Gly Leu Gly Val Leu Gln Ile Asn Ala
    215                 220                 225

ggt atg aac aaa cta tgg agc acc ccg caa gta tcg tgg gtt cag ctt     835
Gly Met Asn Lys Leu Trp Ser Thr Pro Gln Val Ser Trp Val Gln Leu
230                 235                 240                 245

ttg atc atc ttg atc atc acc gcg gtt gca tgt att tcc gtt gct tcc     883
Leu Ile Ile Leu Ile Ile Thr Ala Val Ala Cys Ile Ser Val Ala Ser
                250                 255                 260

ggt ttg gat aag ggc att aag tta ctg tcc aac att aat att gca atg     931
Gly Leu Asp Lys Gly Ile Lys Leu Leu Ser Asn Ile Asn Ile Ala Met
            265                 270                 275

gcc gtt gcg ttg atg ttc ttc atc ttg ttc act ggt cca acc ctc aca     979
Ala Val Ala Leu Met Phe Phe Ile Leu Phe Thr Gly Pro Thr Leu Thr

        280                 285                 290

ttg ctg cgc ttt ctc gta gaa tcc ttc gga atc tat gca tcc tgg atg 1027
Leu Leu Arg Phe Leu Val Glu Ser Phe Gly Ile Tyr Ala Ser Trp Met
    295                 300                 305

cct aat ctg atg ttt tgg act gac tct ttc caa gat aac cca ggc tgg 1075
Pro Asn Leu Met Phe Trp Thr Asp Ser Phe Gln Asp Asn Pro Gly Trp
310                 315                 320                 325

cag ggc aaa tgg acg gtg ttc tat tgg gca tgg act att tgt tgg tcg 1123
Gln Gly Lys Trp Thr Val Phe Tyr Trp Ala Trp Thr Ile Cys Trp Ser
                330                 335                 340

cca tat gtc ggc atg ttc gtg gcg cgt att tcg cgt gga cgt acc gtc 1171
Pro Tyr Val Gly Met Phe Val Ala Arg Ile Ser Arg Gly Arg Thr Val
            345                 350                 355

cgt gaa ttt atc ggt ggg gtt cta gct ctg cca gcg atc ttt ggc gta 1219
Arg Glu Phe Ile Gly Gly Val Leu Ala Leu Pro Ala Ile Phe Gly Val
        360                 365                 370

gtt tgg ttc tct atc ttt ggt cgt gca ggc atc gaa gtg gaa ctg agt 1267
Val Trp Phe Ser Ile Phe Gly Arg Ala Gly Ile Glu Val Glu Leu Ser
    375                 380                 385

aac cca ggt ttc ttg acc cag cca act gtt gtt gaa ggt gac gtg cca 1315
Asn Pro Gly Phe Leu Thr Gln Pro Thr Val Val Glu Gly Asp Val Pro
390                 395                 400                 405

gca gcg ctt ttt aat gtg ctg caa gag tat ccg ctg act gga att gtc 1363
Ala Ala Leu Phe Asn Val Leu Gln Glu Tyr Pro Leu Thr Gly Ile Val
                410                 415                 420

tcc gcg ttt gca ctt gta att att gtg att ttc ttt atc acc tcc atc 1411
Ser Ala Phe Ala Leu Val Ile Ile Val Ile Phe Phe Ile Thr Ser Ile
            425                 430                 435

gat tcc gca gcg cta gtt aac gat atg ttc gct acc ggt gca gaa aat 1459
Asp Ser Ala Ala Leu Val Asn Asp Met Phe Ala Thr Gly Ala Glu Asn
        440                 445                 450

caa aca ccg act agt tac cgc gtg atg tgg gcc tgc acc att ggg gcg 1507
Gln Thr Pro Thr Ser Tyr Arg Val Met Trp Ala Cys Thr Ile Gly Ala
    455                 460                 465

gtc gca ggt tcc ttg ctg atc att tcc cca tcc tct ggt att gcc acg 1555
Val Ala Gly Ser Leu Leu Ile Ile Ser Pro Ser Ser Gly Ile Ala Thr
470                 475                 480                 485

ctg caa gaa gtg gtt atc atc gtg gct ttc cca ttc ttc ctc gtg caa 1603
Leu Gln Glu Val Val Ile Ile Val Ala Phe Pro Phe Phe Leu Val Gln
                490                 495                 500

ttt gtc atg atg ttt tct ttg ctt aaa ggc atg agt gaa gat gct gct 1651
Phe Val Met Met Phe Ser Leu Leu Lys Gly Met Ser Glu Asp Ala Ala
            505                 510                 515

gcg gtt cgt cgt gtg cag act cgt cag tgg gaa aag act gat aca cca 1699
Ala Val Arg Arg Val Gln Thr Arg Gln Trp Glu Lys Thr Asp Thr Pro
        520                 525                 530

gaa aaa ctt gaa gag cat tcg tcc caa cca gcc ccg ggc tat gat gac     1747
Glu Lys Leu Glu Glu His Ser Ser Gln Pro Ala Pro Gly Tyr Asp Asp
    535                 540                 545

gag ggc aac ccc ttg cca atg cct gcc ctc gaa cat gat gag gac ggt     1795
Glu Gly Asn Pro Leu Pro Met Pro Ala Leu Glu His Asp Glu Asp Gly
550                 555                 560                 565

aac att gtt atc cca ggc aac gta gtc att gaa ggt gat ctt ggg gta     1843
Asn Ile Val Ile Pro Gly Asn Val Val Ile Glu Gly Asp Leu Gly Val
                570                 575                 580

gtt ggt gat gtg gtc gac gat cct gag gaa gcc caa gag atg ggg tct     1891
Val Gly Asp Val Val Asp Asp Pro Glu Glu Ala Gln Glu Met Gly Ser
            585                 590                 595

cgt ttt aag atc gtc gag caa act cgg ccc cag tcc agg gac gaa tac     1939
Arg Phe Lys Ile Val Glu Gln Thr Arg Pro Gln Ser Arg Asp Glu Tyr
        600                 605                 610

gat att taaacgattg cttttcgacg cac                                   1968
Asp Ile
    615


<210> 534
<211> 615
<212> PRT
<213> Corynebacterium glutamicum

<400> 534
Met Ser Ser Asn Ile Ala Ile Thr Thr Glu Pro Glu Gly Lys Asn Lys
  1               5                  10                  15

Lys Gly Leu Lys Ser Asp Pro Phe Ile Phe Ser Ile Ser Val Gly Phe
             20                  25                  30

Ile Val Val Phe Val Ile Ala Thr Ile Ala Leu Gly Glu Lys Ala Arg
         35                  40                  45

Thr Thr Phe Ser Ala Ile Ala Gly Trp Leu Leu Glu Asn Leu Gly Trp
     50                  55                  60

Met Tyr Ile Gly Gly Val Ser Leu Val Phe Ile Phe Leu Met Gly Ile
 65                  70                  75                  80

Phe Ala Ser Arg Tyr Gly Arg Val Lys Leu Gly Asp Asp Asp Asp Asp
                 85                  90                  95

Pro Glu His Thr Leu Ile Val Trp Phe Cys Met Leu Phe Ala Gly Gly
            100                 105                 110

Val Gly Ala Val Leu Met Phe Trp Gly Val Ala Glu Pro Ile Asn His
        115                 120                 125

Ala Phe Asn Val Pro Met Ala Asn Glu Glu Ser Met Ser Glu Ala Ala
    130                 135                 140

Ile Val Gln Ala Phe Ala Tyr Thr Phe Tyr His Phe Gly Ile His Met
145                 150                 155                 160

Trp Val Ile Met Ala Leu Pro Gly Leu Ser Leu Gly Tyr Phe Ile Tyr
                165                 170                 175

Lys Arg Lys Leu Pro Pro Arg Leu Ser Ser Val Phe Ser Pro Ile Leu
            180                 185                 190

Gly Lys His Ile Tyr Ser Thr Pro Gly Lys Leu Ile Asp Val Leu Ala
        195                 200                 205

Ile Val Gly Thr Thr Phe Gly Ile Ala Val Ser Val Gly Leu Gly Val
    210                 215                 220

Leu Gln Ile Asn Ala Gly Met Asn Lys Leu Trp Ser Thr Pro Gln Val
225                 230                 235                 240

Ser Trp Val Gln Leu Leu Ile Ile Leu Ile Ile Thr Ala Val Ala Cys
                245                 250                 255

Ile Ser Val Ala Ser Gly Leu Asp Lys Gly Ile Lys Leu Leu Ser Asn
            260                 265                 270

Ile Asn Ile Ala Met Ala Val Ala Leu Met Phe Phe Ile Leu Phe Thr
        275                 280                 285

Gly Pro Thr Leu Thr Leu Leu Arg Phe Leu Val Glu Ser Phe Gly Ile
    290                 295                 300

Tyr Ala Ser Trp Met Pro Asn Leu Met Phe Trp Thr Asp Ser Phe Gln
305                 310                 315                 320

Asp Asn Pro Gly Trp Gln Gly Lys Trp Thr Val Phe Tyr Trp Ala Trp
                325                 330                 335

Thr Ile Cys Trp Ser Pro Tyr Val Gly Met Phe Val Ala Arg Ile Ser
            340                 345                 350

Arg Gly Arg Thr Val Arg Glu Phe Ile Gly Gly Val Leu Ala Leu Pro
        355                 360                 365

Ala Ile Phe Gly Val Val Trp Phe Ser Ile Phe Gly Arg Ala Gly Ile
    370                 375                 380

Glu Val Glu Leu Ser Asn Pro Gly Phe Leu Thr Gln Pro Thr Val Val
385                 390                 395                 400

Glu Gly Asp Val Pro Ala Ala Leu Phe Asn Val Leu Gln Glu Tyr Pro
                405                 410                 415

Leu Thr Gly Ile Val Ser Ala Phe Ala Leu Val Ile Ile Val Ile Phe
            420                 425                 430

Phe Ile Thr Ser Ile Asp Ser Ala Ala Leu Val Asn Asp Met Phe Ala
        435                 440                 445

Thr Gly Ala Glu Asn Gln Thr Pro Thr Ser Tyr Arg Val Met Trp Ala
    450                 455                 460

Cys Thr Ile Gly Ala Val Ala Gly Ser Leu Leu Ile Ile Ser Pro Ser
465                 470                 475                 480

Ser Gly Ile Ala Thr Leu Gln Glu Val Val Ile Ile Val Ala Phe Pro
                485                 490                 495

Phe Phe Leu Val Gln Phe Val Met Met Phe Ser Leu Leu Lys Gly Met
            500                 505                 510

Ser Glu Asp Ala Ala Ala Val Arg Arg Val Gln Thr Arg Gln Trp Glu
        515                 520                 525

Lys Thr Asp Thr Pro Glu Lys Leu Glu Glu His Ser Ser Gln Pro Ala
    530                 535                 540

Pro Gly Tyr Asp Asp Glu Gly Asn Pro Leu Pro Met Pro Ala Leu Glu
545                 550                 555                 560

His Asp Glu Asp Gly Asn Ile Val Ile Pro Gly Asn Val Val Ile Glu
                565                 570                 575

Gly Asp Leu Gly Val Val Gly Asp Val Val Asp Asp Pro Glu Glu Ala
            580                 585                 590

Gln Glu Met Gly Ser Arg Phe Lys Ile Val Glu Gln Thr Arg Pro Gln
        595                 600                 605

Ser Arg Asp Glu Tyr Asp Ile
    610                 615


<210> 535
<211> 1968
<212> DNA
<213> Corynebacterium glutamicum

<220>
<221> CDS
<222> (101)..(1945)
<223> FRXA00298

<400> 535
tttagacaag ttctggttaa aattcttcat gaaggtgaga atctgggaat ttctcggtac 60

tctttcagat tcgtagttat ccactgattg gaagaatgag atg agc tca aat ata    115
                                           Met Ser Ser Asn Ile
                                           1               5

gct atc acg acc gag cct gaa ggg aaa aat aaa aag ggt ctc aaa tca    163
Ala Ile Thr Thr Glu Pro Glu Gly Lys Asn Lys Lys Gly Leu Lys Ser
                10                  15                  20

gac ccg ttc att ttt tcc att tct gtc ggt ttt atc gtg gtg ttt gtc    211
Asp Pro Phe Ile Phe Ser Ile Ser Val Gly Phe Ile Val Val Phe Val
            25                  30                  35

atc gcc aca att gcg cta ggc gag aaa gct cga aca acc ttt tcc gcg    259
Ile Ala Thr Ile Ala Leu Gly Glu Lys Ala Arg Thr Thr Phe Ser Ala
        40                  45                  50

att gcc ggc tgg ctc tta gaa aat tta ggg tgg atg tat atc ggg ggt    307
Ile Ala Gly Trp Leu Leu Glu Asn Leu Gly Trp Met Tyr Ile Gly Gly
    55                  60                  65

gtc tcc ttg gtt ttc att ttc ctc atg ggt atc ttt gcg tcc cgg tat 355
Val Ser Leu Val Phe Ile Phe Leu Met Gly Ile Phe Ala Ser Arg Tyr
70                  75                  80                  85

ggc cgg gta aaa ctt ggt gat gac gat gat gac ccc gag cac acc cta 403
Gly Arg Val Lys Leu Gly Asp Asp Asp Asp Asp Pro Glu His Thr Leu
                90                  95                  100

atc gtg tgg ttc tgt atg ctt ttt gct ggc ggt gtc ggt gca gtc tta 451
Ile Val Trp Phe Cys Met Leu Phe Ala Gly Gly Val Gly Ala Val Leu
            105                 110                 115

atg ttt tgg ggt gtt gcc gaa ccg att aac cac gcg ttc aac gtg cca 499
Met Phe Trp Gly Val Ala Glu Pro Ile Asn His Ala Phe Asn Val Pro
        120                 125                 130

atg gct aat gaa gaa tcc atg agt gaa gcc gca att gtg cag gct ttt 547
Met Ala Asn Glu Glu Ser Met Ser Glu Ala Ala Ile Val Gln Ala Phe
    135                 140                 145

gct tat act ttc tat cac ttc ggt att cac atg tgg gta atc atg gca 595
Ala Tyr Thr Phe Tyr His Phe Gly Ile His Met Trp Val Ile Met Ala
150                 155                 160                 165

ctc cca gga tta tca ttg gga tac ttt att tac aaa cgt aag cta cct 643
Leu Pro Gly Leu Ser Leu Gly Tyr Phe Ile Tyr Lys Arg Lys Leu Pro
                170                 175                 180

ccc cgt cta tcc tct gtg ttt tct ccg atc ttg ggt aag cac att tat 691
Pro Arg Leu Ser Ser Val Phe Ser Pro Ile Leu Gly Lys His Ile Tyr
            185                 190                 195

tcc aca ccc ggc aag ctc atc gat gta ctg gcc atc gta ggc acc acg 739
Ser Thr Pro Gly Lys Leu Ile Asp Val Leu Ala Ile Val Gly Thr Thr
        200                 205                 210

ttt ggt att gct gtg tca gta ggt ctt ggt gtg ctg caa atc aat gca 787
Phe Gly Ile Ala Val Ser Val Gly Leu Gly Val Leu Gln Ile Asn Ala
    215                 220                 225

ggt atg aac aaa cta tgg agc acc ccg caa gta tcg tgg gtt cag ctt 835
Gly Met Asn Lys Leu Trp Ser Thr Pro Gln Val Ser Trp Val Gln Leu
230                 235                 240                 245

ttg atc atc ttg atc atc acc gcg gtt gca tgt att tcc gtt gct tcc 883
Leu Ile Ile Leu Ile Ile Thr Ala Val Ala Cys Ile Ser Val Ala Ser
                250                 255                 260

ggt ttg gat aag ggc att aag tta ctg tcc aac att aat att gca atg 931
Gly Leu Asp Lys Gly Ile Lys Leu Leu Ser Asn Ile Asn Ile Ala Met
            265                 270                 275

gcc gtt gcg ttg atg ttc ttc atc ttg ttc act ggt cca acc ctc aca 979
Ala Val Ala Leu Met Phe Phe Ile Leu Phe Thr Gly Pro Thr Leu Thr
        280                 285                 290

ttg ctg cgc ttt ctc gta gaa tcc ttc gga atc tat gca tcc tgg atg 1027
Leu Leu Arg Phe Leu Val Glu Ser Phe Gly Ile Tyr Ala Ser Trp Met
    295                 300                 305

cct aat ctg atg ttt tgg act gac tct ttc caa gat aac cca ggc tgg 1075

Pro Asn Leu Met Phe Trp Thr Asp Ser Phe Gln Asp Asn Pro Gly Trp
310                 315                 320                 325

cag ggc aaa tgg acg gtg ttc tat tgg gca tgg act att tgt tgg tcg 1123
Gln Gly Lys Trp Thr Val Phe Tyr Trp Ala Trp Thr Ile Cys Trp Ser
                330                 335                 340

cca tat gtc ggc atg ttc gtg gcg cgt att tcg cgt gga cgt acc gtc 1171
Pro Tyr Val Gly Met Phe Val Ala Arg Ile Ser Arg Gly Arg Thr Val
            345                 350                 355

cgt gaa ttt atc ggt ggg gtt cta gct ctg cca gcg atc ttt ggc gta 1219
Arg Glu Phe Ile Gly Gly Val Leu Ala Leu Pro Ala Ile Phe Gly Val
        360                 365                 370

gtt tgg ttc tct atc ttt ggt cgt gca ggc atc gaa gtg gaa ctg agt 1267
Val Trp Phe Ser Ile Phe Gly Arg Ala Gly Ile Glu Val Glu Leu Ser
    375                 380                 385

aac cca ggt ttc ttg acc cag cca act gtt gtt gaa ggt gac gtg cca 1315
Asn Pro Gly Phe Leu Thr Gln Pro Thr Val Val Glu Gly Asp Val Pro
390                 395                 400                 405

gca gcg ctt ttt aat gtg ctg caa gag tat ccg ctg act gga att gtc 1363
Ala Ala Leu Phe Asn Val Leu Gln Glu Tyr Pro Leu Thr Gly Ile Val
                410                 415                 420

tcc gcg ttt gca ctt gta att att gtg att ttc ttt atc acc tcc atc 1411
Ser Ala Phe Ala Leu Val Ile Ile Val Ile Phe Phe Ile Thr Ser Ile
            425                 430                 435

gat tcc gca gcg cta gtt aac gat atg ttc gct acc ggt gca gaa aat 1459
Asp Ser Ala Ala Leu Val Asn Asp Met Phe Ala Thr Gly Ala Glu Asn
        440                 445                 450

caa aca ccg act agt tac cgc gtg atg tgg gcc tgc acc att ggg gcg 1507
Gln Thr Pro Thr Ser Tyr Arg Val Met Trp Ala Cys Thr Ile Gly Ala
    455                 460                 465

gtc gca ggt tcc ttg ctg atc att tcc cca tcc tct ggt att gcc acg 1555
Val Ala Gly Ser Leu Leu Ile Ile Ser Pro Ser Ser Gly Ile Ala Thr
470                 475                 480                 485

ctg caa gaa gtg gtt atc atc gtg gct ttc cca ttc ttc ctc gtg caa 1603
Leu Gln Glu Val Val Ile Ile Val Ala Phe Pro Phe Phe Leu Val Gln
                490                 495                 500

ttt gtc atg atg ttt tct ttg ctt aaa ggc atg agt gaa gat gct gct 1651
Phe Val Met Met Phe Ser Leu Leu Lys Gly Met Ser Glu Asp Ala Ala
            505                 510                 515

gcg gtt cgt cgt gtg cag act cgt cag tgg gaa aag act gat aca cca 1699
Ala Val Arg Arg Val Gln Thr Arg Gln Trp Glu Lys Thr Asp Thr Pro
        520                 525                 530

gaa aaa ctt gaa gag cat tcg tcc caa cca gcc ccg ggc tat gat gac 1747
Glu Lys Leu Glu Glu His Ser Ser Gln Pro Ala Pro Gly Tyr Asp Asp
    535                 540                 545

gag ggc aac ccc ttg cca atg cct gcc ctc gaa cat gat gag gac ggt 1795
Glu Gly Asn Pro Leu Pro Met Pro Ala Leu Glu His Asp Glu Asp Gly

550                 555                 560                 565

aac att gtt atc cca ggc aac gta gtc att gaa ggt gat ctt ggg gta     1843
Asn Ile Val Ile Pro Gly Asn Val Val Ile Glu Gly Asp Leu Gly Val
                570                 575                 580

gtt ggt gat gtg gtc gac gat cct gag gaa gcc caa gag atg ggg tct     1891
Val Gly Asp Val Val Asp Asp Pro Glu Glu Ala Gln Glu Met Gly Ser
            585                 590                 595

cgt ttt aag atc gtc gag caa act cgg ccc cag tcc agg gac gaa tac     1939
Arg Phe Lys Ile Val Glu Gln Thr Arg Pro Gln Ser Arg Asp Glu Tyr
        600                 605                 610

gat att taaacgattg cttttcgacg cac                                   1968
Asp Ile
    615

<210> 536
<211> 615
<212> PRT
<213> Corynebacterium glutamicum

<400> 536
Met Ser Ser Asn Ile Ala Ile Thr Thr Glu Pro Glu Gly Lys Asn Lys
  1               5                  10                  15

Lys Gly Leu Lys Ser Asp Pro Phe Ile Phe Ser Ile Ser Val Gly Phe
             20                  25                  30

Ile Val Val Phe Val Ile Ala Thr Ile Ala Leu Gly Glu Lys Ala Arg
         35                  40                  45

Thr Thr Phe Ser Ala Ile Ala Gly Trp Leu Leu Glu Asn Leu Gly Trp
     50                  55                  60

Met Tyr Ile Gly Gly Val Ser Leu Val Phe Ile Phe Leu Met Gly Ile
 65                  70                  75                  80

Phe Ala Ser Arg Tyr Gly Arg Val Lys Leu Gly Asp Asp Asp Asp Asp
                 85                  90                  95

Pro Glu His Thr Leu Ile Val Trp Phe Cys Met Leu Phe Ala Gly Gly
            100                 105                 110

Val Gly Ala Val Leu Met Phe Trp Gly Val Ala Glu Pro Ile Asn His
        115                 120                 125

Ala Phe Asn Val Pro Met Ala Asn Glu Glu Ser Met Ser Glu Ala Ala
    130                 135                 140

Ile Val Gln Ala Phe Ala Tyr Thr Phe Tyr His Phe Gly Ile His Met
145                 150                 155                 160

Trp Val Ile Met Ala Leu Pro Gly Leu Ser Leu Gly Tyr Phe Ile Tyr
                165                 170                 175

Lys Arg Lys Leu Pro Pro Arg Leu Ser Ser Val Phe Ser Pro Ile Leu
            180                 185                 190

Gly Lys His Ile Tyr Ser Thr Pro Gly Lys Leu Ile Asp Val Leu Ala
        195                 200                 205

Ile Val Gly Thr Thr Phe Gly Ile Ala Val Ser Val Gly Leu Gly Val
    210                 215                 220

Leu Gln Ile Asn Ala Gly Met Asn Lys Leu Trp Ser Thr Pro Gln Val
225                 230                 235                 240

Ser Trp Val Gln Leu Leu Ile Ile Leu Ile Ile Thr Ala Val Ala Cys
                245                 250                 255

Ile Ser Val Ala Ser Gly Leu Asp Lys Gly Ile Lys Leu Leu Ser Asn
            260                 265                 270

Ile Asn Ile Ala Met Ala Val Ala Leu Met Phe Phe Ile Leu Phe Thr
        275                 280                 285

Gly Pro Thr Leu Thr Leu Leu Arg Phe Leu Val Glu Ser Phe Gly Ile
    290                 295                 300

Tyr Ala Ser Trp Met Pro Asn Leu Met Phe Trp Thr Asp Ser Phe Gln
305                 310                 315                 320

Asp Asn Pro Gly Trp Gln Gly Lys Trp Thr Val Phe Tyr Trp Ala Trp
                325                 330                 335

Thr Ile Cys Trp Ser Pro Tyr Val Gly Met Phe Val Ala Arg Ile Ser
            340                 345                 350

Arg Gly Arg Thr Val Arg Glu Phe Ile Gly Gly Val Leu Ala Leu Pro
        355                 360                 365

Ala Ile Phe Gly Val Val Trp Phe Ser Ile Phe Gly Arg Ala Gly Ile
    370                 375                 380

Glu Val Glu Leu Ser Asn Pro Gly Phe Leu Thr Gln Pro Thr Val Val
385                 390                 395                 400

Glu Gly Asp Val Pro Ala Ala Leu Phe Asn Val Leu Gln Glu Tyr Pro
                405                 410                 415

Leu Thr Gly Ile Val Ser Ala Phe Ala Leu Val Ile Ile Val Ile Phe
            420                 425                 430

Phe Ile Thr Ser Ile Asp Ser Ala Ala Leu Val Asn Asp Met Phe Ala
        435                 440                 445

Thr Gly Ala Glu Asn Gln Thr Pro Thr Ser Tyr Arg Val Met Trp Ala
    450                 455                 460

Cys Thr Ile Gly Ala Val Ala Gly Ser Leu Leu Ile Ile Ser Pro Ser
465                 470                 475                 480

Ser Gly Ile Ala Thr Leu Gln Glu Val Val Ile Ile Val Ala Phe Pro
                485                 490                 495

Phe Phe Leu Val Gln Phe Val Met Met Phe Ser Leu Leu Lys Gly Met
            500                 505                 510

Ser Glu Asp Ala Ala Ala Val Arg Arg Val Gln Thr Arg Gln Trp Glu

        515                 520                 525

Lys Thr Asp Thr Pro Glu Lys Leu Glu Glu His Ser Ser Gln Pro Ala
    530                 535                 540

Pro Gly Tyr Asp Asp Glu Gly Asn Pro Leu Pro Met Pro Ala Leu Glu
545                 550                 555                 560

His Asp Glu Asp Gly Asn Ile Val Ile Pro Gly Asn Val Val Ile Glu
                565                 570                 575

Gly Asp Leu Gly Val Val Gly Asp Val Val Asp Asp Pro Glu Glu Ala
            580                 585                 590

Gln Glu Met Gly Ser Arg Phe Lys Ile Val Glu Gln Thr Arg Pro Gln
        595                 600                 605

Ser Arg Asp Glu Tyr Asp Ile
    610                 615


<210> 537
<211> 576
<212> DNA
<213> Corynebacterium glutamicum

<220>
<221> CDS
<222> (101)..(553)
<223> RXA00596

<400> 537
ccgccaccga cggcctcttg aacaccgatg cataccaaca ggctgtgctc ggtgaaaatg 60

ccatcggagt gccaagccct agctaccagg gaggaaacta atg ctt aac gcc ctg   115
                                           Met Leu Asn Ala Leu
                                           1               5

aaa ttc atc cca tgg ctg atc ggc cag att ttc ctc tct ggc ttc agc   163
Lys Phe Ile Pro Trp Leu Ile Gly Gln Ile Phe Leu Ser Gly Phe Ser
                10                  15                  20

gtg atc acc gct gcg gta aaa aag gac acc ggc ttc aac ccc gtt gtt   211
Val Ile Thr Ala Ala Val Lys Lys Asp Thr Gly Phe Asn Pro Val Val
            25                  30                  35

atc cgc tac cca ctt cga gtg acc acg gac ttc cag atc gca gcc ctg   259
Ile Arg Tyr Pro Leu Arg Val Thr Thr Asp Phe Gln Ile Ala Ala Leu
        40                  45                  50

tca acg tgc atc acc gcg act cct tcc acc ctg tcc ctt ggc cta cgc   307
Ser Thr Cys Ile Thr Ala Thr Pro Ser Thr Leu Ser Leu Gly Leu Arg
    55                  60                  65

gaa ccc cgc aag ccc ggc gac ccc acc att ttg ctg atc caa gca gtg   355
Glu Pro Arg Lys Pro Gly Asp Pro Thr Ile Leu Leu Ile Gln Ala Val
70                  75                  80                  85

ttt ggt tcc gat cca gta gaa gtt ttt gaa tcc atc gcc gat atg gaa   403
Phe Gly Ser Asp Pro Val Glu Val Phe Glu Ser Ile Ala Asp Met Glu
                90                  95                  100

caa cgc ctc gtc cct tcg gtc gct tca att gac cac ggc gtc cca ggc     451
Gln Arg Leu Val Pro Ser Val Ala Ser Ile Asp His Gly Val Pro Gly
            105                 110                 115

caa ggc cct tac aag gag atc cgc ccc agc gat gct gag tgg cca agt     499
Gln Gly Pro Tyr Lys Glu Ile Arg Pro Ser Asp Ala Glu Trp Pro Ser
        120                 125                 130

cgc gag atc gct gac acc gcc caa aac acc gtc agc caa gac aag agg     547
Arg Glu Ile Ala Asp Thr Ala Gln Asn Thr Val Ser Gln Asp Lys Arg
    135                 140                 145

gag ttt taaaacaaca tgactgcttt tgg                                   576
Glu Phe
150


<210> 538
<211> 151
<212> PRT
<213> Corynebacterium glutamicum

<400> 538
Met Leu Asn Ala Leu Lys Phe Ile Pro Trp Leu Ile Gly Gln Ile Phe
  1               5                  10                  15

Leu Ser Gly Phe Ser Val Ile Thr Ala Ala Val Lys Lys Asp Thr Gly
             20                  25                  30

Phe Asn Pro Val Val Ile Arg Tyr Pro Leu Arg Val Thr Thr Asp Phe
         35                  40                  45

Gln Ile Ala Ala Leu Ser Thr Cys Ile Thr Ala Thr Pro Ser Thr Leu
     50                  55                  60

Ser Leu Gly Leu Arg Glu Pro Arg Lys Pro Gly Asp Pro Thr Ile Leu
 65                  70                  75                  80

Leu Ile Gln Ala Val Phe Gly Ser Asp Pro Val Glu Val Phe Glu Ser
                 85                  90                  95

Ile Ala Asp Met Glu Gln Arg Leu Val Pro Ser Val Ala Ser Ile Asp
            100                 105                 110

His Gly Val Pro Gly Gln Gly Pro Tyr Lys Glu Ile Arg Pro Ser Asp
        115                 120                 125

Ala Glu Trp Pro Ser Arg Glu Ile Ala Asp Thr Ala Gln Asn Thr Val
    130                 135                 140

Ser Gln Asp Lys Arg Glu Phe
145                 150


<210> 539
<211> 750
<212> DNA
<213> Corynebacterium glutamicum

<220>

<221> CDS
<222> (101)..(727)
<223> RXA02364

<400> 539
gcgaaagtat ctaaggtcgt ttgattggtt tactaccccc gaagtggggg ttattggcag 60

catcgagcac caaatgagcg tgatccggga tcaggatctg atg gag cca ctg ttc 115
Met Glu Pro Leu Phe
1               5

caa agt ctt gcg gaa tcg gac aat att tct gtc att ggt gga ttt acc 163
Gln Ser Leu Ala Glu Ser Asp Asn Ile Ser Val Ile Gly Gly Phe Thr
                10                  15                  20

caa ggc acc cga aat ctg tac acc act gat gcg ccg gtg aaa aga ccc 211
Gln Gly Thr Arg Asn Leu Tyr Thr Thr Asp Ala Pro Val Lys Arg Pro
            25                  30                  35

gct gac ctg gct gga aag aag att cgc gtc cag gaa tcc gcc atg cac 259
Ala Asp Leu Ala Gly Lys Lys Ile Arg Val Gln Glu Ser Ala Met His
        40                  45                  50

atc cgc atg att gaa ctc atg ggt ggc tcg gca acc ccg ttg act tac 307
Ile Arg Met Ile Glu Leu Met Gly Gly Ser Ala Thr Pro Leu Thr Tyr
    55                  60                  65

ggc gag gta tat acc gcg atg cag tct ggt gtg ctg gat ggc gcg gaa 355
Gly Glu Val Tyr Thr Ala Met Gln Ser Gly Val Leu Asp Gly Ala Glu
70                  75                  80                  85

aat aac gag atc agt tac gtt acc caa aac cac ttc gag gtt gct cgc 403
Asn Asn Glu Ile Ser Tyr Val Thr Gln Asn His Phe Glu Val Ala Arg
                90                  95                  100

tac aac agc aac acc aat cac ctt gtc ggt ttg gat tac atg gtc atg 451
Tyr Asn Ser Asn Thr Asn His Leu Val Gly Leu Asp Tyr Met Val Met
            105                 110                 115

cga cac gat ctg ctt gac gcc atg agc gag cca gac cgt gaa ttg ttc 499
Arg His Asp Leu Leu Asp Ala Met Ser Glu Pro Asp Arg Glu Leu Phe
        120                 125                 130

ctg gaa gaa tgg gac gcc gcg atg act gag cac acg gat ctg tgg aac 547
Leu Glu Glu Trp Asp Ala Ala Met Thr Glu His Thr Asp Leu Trp Asn
    135                 140                 145

aca gaa act gat gcc gtt att gaa aag gcg aaa gct ggg gga gcg gag 595
Thr Glu Thr Asp Ala Val Ile Glu Lys Ala Lys Ala Gly Gly Ala Glu
150                 155                 160                 165

ttc gtt gag gtg gat gcg caa gca ttt acc gat gca ctg gct ccc atc 643
Phe Val Glu Val Asp Ala Gln Ala Phe Thr Asp Ala Leu Ala Pro Ile
                170                 175                 180

aaa gac gaa ttc ttg acc tca gaa ttc cag cgt gaa ctc tac gaa gca 691
Lys Asp Glu Phe Leu Thr Ser Glu Phe Gln Arg Glu Leu Tyr Glu Ala
            185                 190                 195

gtg cgc gcc gct gat act tca gga ggt gcg gca tca tgatgaattt 737
Val Arg Ala Ala Asp Thr Ser Gly Gly Ala Ala Ser

200                 205

taagtccatc gtg                                                        750

<210> 540
<211> 209
<212> PRT
<213> Corynebacterium glutamicum

<400> 540
Met Glu Pro Leu Phe Gln Ser Leu Ala Glu Ser Asp Asn Ile Ser Val
1               5                   10                  15

Ile Gly Gly Phe Thr Gln Gly Thr Arg Asn Leu Tyr Thr Thr Asp Ala
            20                  25                  30

Pro Val Lys Arg Pro Ala Asp Leu Ala Gly Lys Lys Ile Arg Val Gln
        35                  40                  45

Glu Ser Ala Met His Ile Arg Met Ile Glu Leu Met Gly Gly Ser Ala
    50                  55                  60

Thr Pro Leu Thr Tyr Gly Glu Val Tyr Thr Ala Met Gln Ser Gly Val
65                  70                  75                  80

Leu Asp Gly Ala Glu Asn Asn Glu Ile Ser Tyr Val Thr Gln Asn His
                85                  90                  95

Phe Glu Val Ala Arg Tyr Asn Ser Asn Thr Asn His Leu Val Gly Leu
            100                 105                 110

Asp Tyr Met Val Met Arg His Asp Leu Leu Asp Ala Met Ser Glu Pro
        115                 120                 125

Asp Arg Glu Leu Phe Leu Glu Glu Trp Asp Ala Ala Met Thr Glu His
    130                 135                 140

Thr Asp Leu Trp Asn Thr Glu Thr Asp Ala Val Ile Glu Lys Ala Lys
145                 150                 155                 160

Ala Gly Gly Ala Glu Phe Val Glu Val Asp Ala Gln Ala Phe Thr Asp
                165                 170                 175

Ala Leu Ala Pro Ile Lys Asp Glu Phe Leu Thr Ser Glu Phe Gln Arg
            180                 185                 190

Glu Leu Tyr Glu Ala Val Arg Ala Ala Asp Thr Ser Gly Gly Ala Ala
        195                 200                 205

Ser

<210> 541
<211> 888
<212> DNA
<213> Corynebacterium glutamicum

<220>
<221> CDS

<222> (101)..(865)
<223> RXN01411

<400> 541
cttatcgacg tccccatccc cctcgccaat gcttcggcga ggggttctat ttattgtgtg 60

tgctagcctt ttcgcaatcg ttcagcccgc cccgacgtca atg ttg gga gtg ggc 115
Met Leu Gly Val Gly
1               5

tgg cgc att cca ttc ctg atg gcc gtg cca cta ggg ctt atc ggc tgg 163
Trp Arg Ile Pro Phe Leu Met Ala Val Pro Leu Gly Leu Ile Gly Trp
                10                  15                  20

tgg atc cgc acc ggt gcc cag gaa aat gta cgc ccc gca tcc gaa cgc 211
Trp Ile Arg Thr Gly Ala Gln Glu Asn Val Arg Pro Ala Ser Glu Arg
            25                  30                  35

ccc gaa gct cct att aag cag gca ttg cgt act gag tgg aag atg atg 259
Pro Glu Ala Pro Ile Lys Gln Ala Leu Arg Thr Glu Trp Lys Met Met
        40                  45                  50

ttg cgg gta ggt ggc ttt atc tct tgc acc ggt ctg agc ttc tac att 307
Leu Arg Val Gly Gly Phe Ile Ser Cys Thr Gly Leu Ser Phe Tyr Ile
    55                  60                  65

ttc acc acg tac atg acc act ttc ctg cgc agc acc gtc gga ctg gag 355
Phe Thr Thr Tyr Met Thr Thr Phe Leu Arg Ser Thr Val Gly Leu Glu
70                  75                  80                  85

ggc acg tta gtg ctg gct gga aac atc atc gct ctc agc atg gca gca 403
Gly Thr Leu Val Leu Ala Gly Asn Ile Ile Ala Leu Ser Met Ala Ala
                90                  95                  100

att gtg gcc cca ttt gtt ggc cgc gca att gat aaa ttc ccc cgc cgg 451
Ile Val Ala Pro Phe Val Gly Arg Ala Ile Asp Lys Phe Pro Arg Arg
            105                 110                 115

aac atc atg gct ttc gct acc tta agc aca gta att atg gcg atc ccg 499
Asn Ile Met Ala Phe Ala Thr Leu Ser Thr Val Ile Met Ala Ile Pro
        120                 125                 130

gcc tac atc att gca ggt caa ggt act ttg act gct tct ttg att gcg 547
Ala Tyr Ile Ile Ala Gly Gln Gly Thr Leu Thr Ala Ser Leu Ile Ala
    135                 140                 145

cag gta atg ctt gga atc ggc gcg gtt acc gct aac tgc gtt acc tca 595
Gln Val Met Leu Gly Ile Gly Ala Val Thr Ala Asn Cys Val Thr Ser
150                 155                 160                 165

gta atg atg gcc gag gtc ttc caa gag gtc acc cgc ggt act tcc gcc 643
Val Met Met Ala Glu Val Phe Gln Glu Val Thr Arg Gly Thr Ser Ala
                170                 175                 180

ggc att acc tac aac gtc act tac gca atc ttc ggc ggc tcg gct cca 691
Gly Ile Thr Tyr Asn Val Thr Tyr Ala Ile Phe Gly Gly Ser Ala Pro
            185                 190                 195

ttt atc tcc acc gca ttg gtc tcc tgg acc ggc agc ccg ctg gcc cct 739
Phe Ile Ser Thr Ala Leu Val Ser Trp Thr Gly Ser Pro Leu Ala Pro
        200                 205                 210

gcg gta tac atg atc atc att gcg ctc ttc gcc ttc acc gcg tcc cgc 787
Ala Val Tyr Met Ile Ile Ile Ala Leu Phe Ala Phe Thr Ala Ser Arg
    215                 220                 225

ttc att cct gaa acc tcc cca gtt ttt gtc acc gca acc ccg gcc att 835
Phe Ile Pro Glu Thr Ser Pro Val Phe Val Thr Ala Thr Pro Ala Ile
230                 235                 240                 245

aag gca cca aag gtg ctg gtc aac ccg ggt taaaccacgc ttttcgacga 885
Lys Ala Pro Lys Val Leu Val Asn Pro Gly
                250                 255

aaa 888

<210> 542
<211> 255
<212> PRT
<213> Corynebacterium glutamicum

<400> 542
Met Leu Gly Val Gly Trp Arg Ile Pro Phe Leu Met Ala Val Pro Leu
1               5                   10                  15

Gly Leu Ile Gly Trp Trp Ile Arg Thr Gly Ala Gln Glu Asn Val Arg
            20                  25                  30

Pro Ala Ser Glu Arg Pro Glu Ala Pro Ile Lys Gln Ala Leu Arg Thr
        35                  40                  45

Glu Trp Lys Met Met Leu Arg Val Gly Gly Phe Ile Ser Cys Thr Gly
    50                  55                  60

Leu Ser Phe Tyr Ile Phe Thr Thr Tyr Met Thr Thr Phe Leu Arg Ser
65                  70                  75                  80

Thr Val Gly Leu Glu Gly Thr Leu Val Leu Ala Gly Asn Ile Ile Ala
                85                  90                  95

Leu Ser Met Ala Ala Ile Val Ala Pro Phe Val Gly Arg Ala Ile Asp
            100                 105                 110

Lys Phe Pro Arg Arg Asn Ile Met Ala Phe Ala Thr Leu Ser Thr Val
        115                 120                 125

Ile Met Ala Ile Pro Ala Tyr Ile Ile Ala Gly Gln Gly Thr Leu Thr
    130                 135                 140

Ala Ser Leu Ile Ala Gln Val Met Leu Gly Ile Gly Ala Val Thr Ala
145                 150                 155                 160

Asn Cys Val Thr Ser Val Met Met Ala Glu Val Phe Gln Glu Val Thr
                165                 170                 175

Arg Gly Thr Ser Ala Gly Ile Thr Tyr Asn Val Thr Tyr Ala Ile Phe
            180                 185                 190

Gly Gly Ser Ala Pro Phe Ile Ser Thr Ala Leu Val Ser Trp Thr Gly
        195                 200                 205

Ser Pro Leu Ala Pro Ala Val Tyr Met Ile Ile Ile Ala Leu Phe Ala
    210                 215                 220

Phe Thr Ala Ser Arg Phe Ile Pro Glu Thr Ser Pro Val Phe Val Thr
225                 230                 235                 240

Ala Thr Pro Ala Ile Lys Ala Pro Lys Val Leu Val Asn Pro Gly
                245                 250                 255


<210> 543
<211> 1058
<212> DNA
<213> Corynebacterium glutamicum

<220>
<221> CDS
<222> (1)..(1035)
<223> RXN00960

<400> 543
   atg gct cgg cat tgt tgc agc aat cgc tac gcg tcc acc gtc ttc tcc      48
   Met Ala Arg His Cys Cys Ser Asn Arg Tyr Ala Ser Thr Val Phe Ser
     1               5                  10                  15
   ggt ctg atc gcc tac gga gca tcc caa gcg ctc tac cca tgg ctg ctg      96
   Gly Leu Ile Ala Tyr Gly Ala Ser Gln Ala Leu Tyr Pro Trp Leu Leu
                20                  25                  30
   aaa gac cac caa agc gtc acc gaa atc gac ctt gat gca ggt gcc ctc     144
   Lys Asp His Gln Ser Val Thr Glu Ile Asp Leu Asp Ala Gly Ala Leu
            35                  40                  45
   cag ccc tac ttc aac atc gag atg cca cca cca ttt gaa gtg atg acc     192
   Gln Pro Tyr Phe Asn Ile Glu Met Pro Pro Pro Phe Glu Val Met Thr
        50                  55                  60
   gca ctg ctg ctg gca ttc tgc ctc ggc ctg ggc atg gct gta att aaa     240
   Ala Leu Leu Leu Ala Phe Cys Leu Gly Leu Gly Met Ala Val Ile Lys
    65                  70                  75                  80
   tca gac acc ctg ttc aag gta acc cgc gaa ctc gag cgc gta gtc atg     288
   Ser Asp Thr Leu Phe Lys Val Thr Arg Glu Leu Glu Arg Val Val Met
                    85                  90                  95
   aag acc atc acc gcc ttt gtc atc cca ctg ctg cca ctc ttc atc ttc     336
   Lys Thr Ile Thr Ala Phe Val Ile Pro Leu Leu Pro Leu Phe Ile Phe
               100                 105                 110
   ggc atc ttc ctc ggc atg ggc atg aac ggt ggc ctc ctg gag atc atg     384
   Gly Ile Phe Leu Gly Met Gly Met Asn Gly Gly Leu Leu Glu Ile Met
           115                 120                 125
   tcc gcc ttt ggc aag gta ctg att ctc gcc gtc gtg gga acc ctg ctc     432
   Ser Ala Phe Gly Lys Val Leu Ile Leu Ala Val Val Gly Thr Leu Leu
       130                 135                 140
   ttc cta gcc atc cag ttc att atc gct ggt gca gta tcc aag aag aac     480
   Phe Leu Ala Ile Gln Phe Ile Ile Ala Gly Ala Val Ser Lys Lys Asn
   145                 150                 155                 160
   cca tgg aaa ctg ttc aaa aac atg ctc cct gca tac ttc act gca ctg     528
   Pro Trp Lys Leu Phe Lys Asn Met Leu Pro Ala Tyr Phe Thr Ala Leu
                   165                 170                 175
   ggc act tcc tct tca gcg gca acc atc cca gtg acc tac cag cag acc     576
   Gly Thr Ser Ser Ser Ala Ala Thr Ile Pro Val Thr Tyr Gln Gln Thr
               180                 185                 190
   ctg aaa aac gat gtt gat gtc aac gtc gca ggc ttt gtt gtc cca ctg     624
   Leu Lys Asn Asp Val Asp Val Asn Val Ala Gly Phe Val Val Pro Leu
           195                 200                 205
   tgc gcc acc atc cac cta gct gga tcg atg atg aag atc ggc ctc ttc     672
   Cys Ala Thr Ile His Leu Ala Gly Ser Met Met Lys Ile Gly Leu Phe
       210                 215                 220
   acc ttc gct gtt gtc ttc atg tac gac atg gaa gta ggc gtc ggc ctc     720
   Thr Phe Ala Val Val Phe Met Tyr Asp Met Glu Val Gly Val Gly Leu
   225                 230                 235                 240
   tcc atc gga ttc ctc ctc atg ctg ggc atc acc atg atc gcc gca cca     768
   Ser Ile Gly Phe Leu Leu Met Leu Gly Ile Thr Met Ile Ala Ala Pro
                   245                 250                 255
   ggc gtt ccc ggc gga gcc atc atg gca gca acc ggc atg ctg gcc tcc     816
   Gly Val Pro Gly Gly Ala Ile Met Ala Ala Thr Gly Met Leu Ala Ser
               260                 265                 270
   atg ctc gga ttc aac acc gaa caa gtc gcc ctc atg atc gcc gct tac     864
   Met Leu Gly Phe Asn Thr Glu Gln Val Ala Leu Met Ile Ala Ala Tyr
           275                 280                 285
   atc gcg att gac tcc ttc ggc acc gca gca aac gtc acc ggc gac ggc     912
   Ile Ala Ile Asp Ser Phe Gly Thr Ala Ala Asn Val Thr Gly Asp Gly
       290                 295                 300
   gca atc gca gtc atc gtg aac aaa ttc gcc aag ggc cag ctg cac acc     960
   Ala Ile Ala Val Ile Val Asn Lys Phe Ala Lys Gly Gln Leu His Thr
   305                 310                 315                 320
   act tcc cca gat gaa atc gaa gaa gac gac cgc gtt gcc ttc gac atc     1008
   Thr Ser Pro Asp Glu Ile Glu Glu Asp Asp Arg Val Ala Phe Asp Ile
                   325                 330                 335
   act cca tcg gat gtg gaa cat cac aag tagaaacccg cattttctgt 1055
   Thr Pro Ser Asp Val Glu His His Lys
   340 345
   agt                                                                 1058

<210> 544
<211> 345
<212> PRT
<213> Corynebacterium glutamicum

<400> 544
Met Ala Arg His Cys Cys Ser Asn Arg Tyr Ala Ser Thr Val Phe Ser
  1               5                  10                  15

Gly Leu Ile Ala Tyr Gly Ala Ser Gln Ala Leu Tyr Pro Trp Leu Leu

            20                  25                  30

Lys Asp His Gln Ser Val Thr Glu Ile Asp Leu Asp Ala Gly Ala Leu
        35                  40                  45

Gln Pro Tyr Phe Asn Ile Glu Met Pro Pro Pro Phe Glu Val Met Thr
    50                  55                  60

Ala Leu Leu Leu Ala Phe Cys Leu Gly Leu Gly Met Ala Val Ile Lys
65                  70                  75                  80

Ser Asp Thr Leu Phe Lys Val Thr Arg Glu Leu Glu Arg Val Val Met
                85                  90                  95

Lys Thr Ile Thr Ala Phe Val Ile Pro Leu Leu Pro Leu Phe Ile Phe
            100                 105                 110

Gly Ile Phe Leu Gly Met Gly Met Asn Gly Gly Leu Leu Glu Ile Met
        115                 120                 125

Ser Ala Phe Gly Lys Val Leu Ile Leu Ala Val Val Gly Thr Leu Leu
    130                 135                 140

Phe Leu Ala Ile Gln Phe Ile Ile Ala Gly Ala Val Ser Lys Lys Asn
145                 150                 155                 160

Pro Trp Lys Leu Phe Lys Asn Met Leu Pro Ala Tyr Phe Thr Ala Leu
                165                 170                 175

Gly Thr Ser Ser Ser Ala Ala Thr Ile Pro Val Thr Tyr Gln Gln Thr
            180                 185                 190

Leu Lys Asn Asp Val Asp Val Asn Val Ala Gly Phe Val Val Pro Leu
        195                 200                 205

Cys Ala Thr Ile His Leu Ala Gly Ser Met Met Lys Ile Gly Leu Phe
    210                 215                 220

Thr Phe Ala Val Val Phe Met Tyr Asp Met Glu Val Gly Val Gly Leu
225                 230                 235                 240

Ser Ile Gly Phe Leu Leu Met Leu Gly Ile Thr Met Ile Ala Ala Pro
                245                 250                 255

Gly Val Pro Gly Gly Ala Ile Met Ala Ala Thr Gly Met Leu Ala Ser
            260                 265                 270

Met Leu Gly Phe Asn Thr Glu Gln Val Ala Leu Met Ile Ala Ala Tyr
        275                 280                 285

Ile Ala Ile Asp Ser Phe Gly Thr Ala Ala Asn Val Thr Gly Asp Gly
    290                 295                 300

Ala Ile Ala Val Ile Val Asn Lys Phe Ala Lys Gly Gln Leu His Thr
305                 310                 315                 320

Thr Ser Pro Asp Glu Ile Glu Glu Asp Asp Arg Val Ala Phe Asp Ile
                325                 330                 335

Thr Pro Ser Asp Val Glu His His Lys
            340                 345

<210> 545
<211> 1118
<212> DNA
<213> Corynebacterium glutamicum

<220>
<221> CDS
<222> (1)..(1095)
<223> RXN02447

<400> 545
aca gta gtt ccg gtg tac ctc gct gaa ctc gca cca cta gaa atc cgc     48
Thr Val Val Pro Val Tyr Leu Ala Glu Leu Ala Pro Leu Glu Ile Arg
1               5                   10                  15

ggc tcc ctg acc ggc cga aac gag ctt gct atc gtc acc ggc cag ctg     96
Gly Ser Leu Thr Gly Arg Asn Glu Leu Ala Ile Val Thr Gly Gln Leu
            20                  25                  30

ctt gcc ttc gtg atc aac gcg ctt atc gcc gtc acc cta cac gga gtt     144
Leu Ala Phe Val Ile Asn Ala Leu Ile Ala Val Thr Leu His Gly Val
        35                  40                  45

att gat gga atc tgg cgc atc atg ttc gcc gtc tgt gcc ctc cct gcc     192
Ile Asp Gly Ile Trp Arg Ile Met Phe Ala Val Cys Ala Leu Pro Ala
    50                  55                  60

gtc gcc ctc ttc ctc ggc atg ctg cgg atg ccg gaa tca cca cgc tgg     240
Val Ala Leu Phe Leu Gly Met Leu Arg Met Pro Glu Ser Pro Arg Trp
65                  70                  75                  80

ctg gtc aac cag ggg cgt tac gac gac gcc cgc cgc gtc atg gag acc     288
Leu Val Asn Gln Gly Arg Tyr Asp Asp Ala Arg Arg Val Met Glu Thr
                85                  90                  95

gtc cgt acc cct gag cgt gcg aaa gcc gaa atg gat gaa atc atc gcg     336
Val Arg Thr Pro Glu Arg Ala Lys Ala Glu Met Asp Glu Ile Ile Ala
            100                 105                 110

gtg cac tct gaa aac aat gcg gca ctt cct ggt gtt aag cag tct tcg     384
Val His Ser Glu Asn Asn Ala Ala Leu Pro Gly Val Lys Gln Ser Ser
        115                 120                 125

ggc cag gct tca ggc cag gtt tct agc aag cac acc cac atg tcc atc     432
Gly Gln Ala Ser Gly Gln Val Ser Ser Lys His Thr His Met Ser Ile
    130                 135                 140

ggc gaa gtc ctc agc aac aaa tgg ctg gtt cgt ctg ctc atc gcc ggc     480
Gly Glu Val Leu Ser Asn Lys Trp Leu Val Arg Leu Leu Ile Ala Gly
145                 150                 155                 160

atc ggt gtt gca gtt gcc cag cag ctc acc ggc atc aac gcc atc atg     528
Ile Gly Val Ala Val Ala Gln Gln Leu Thr Gly Ile Asn Ala Ile Met
                165                 170                 175

tac tac gga acc cgc gtc ctc gag gaa tcc ggc atg agc gca gaa atg     576
Tyr Tyr Gly Thr Arg Val Leu Glu Glu Ser Gly Met Ser Ala Glu Met
            180                 185                 190

gct gtg gtt gcc aac att gct ttc ggt gcc gtt gcc gtc atc ggt gga     624
Ala Val Val Ala Asn Ile Ala Phe Gly Ala Val Ala Val Ile Gly Gly
        195                 200                 205

ctg atc gca ctg cgc aac atg gac cgc ctg gat cgc cgc acc acc ttc     672
Leu Ile Ala Leu Arg Asn Met Asp Arg Leu Asp Arg Arg Thr Thr Phe
    210                 215                 220

atc atc ggc ctg tca ctg acc acc acc ttc cac ctt ttg atc gca gct     720
Ile Ile Gly Leu Ser Leu Thr Thr Thr Phe His Leu Leu Ile Ala Ala
225                 230                 235                 240

gcc ggc act ctc ctt cca gaa ggt aac tcc att cga cca ttc gcc atc     768
Ala Gly Thr Leu Leu Pro Glu Gly Asn Ser Ile Arg Pro Phe Ala Ile
                245                 250                 255

atg atc ctt gtt gtt ggg ttc gtg ctc tcc atg cag act ttc ctc aac     816
Met Ile Leu Val Val Gly Phe Val Leu Ser Met Gln Thr Phe Leu Asn
            260                 265                 270

gtt gca gtg tgg gtg tgg ctg gcg gaa atc ttc cca gtc cga atg aag     864
Val Ala Val Trp Val Trp Leu Ala Glu Ile Phe Pro Val Arg Met Lys
        275                 280                 285

ggt atc ggc acc ggt att tcg gta ttc tgc ggt tgg ggc atc aat ggc     912
Gly Ile Gly Thr Gly Ile Ser Val Phe Cys Gly Trp Gly Ile Asn Gly
    290                 295                 300

gtc cta gcg ttg ttc ttc cca gca ctg gtc tcc ggc gtg ggt atc acc     960
Val Leu Ala Leu Phe Phe Pro Ala Leu Val Ser Gly Val Gly Ile Thr
305                 310                 315                 320

ttc tcc ttc ctt atc ttc gca gtc gtc gga gtc att gcc ctg gcg ttc     1008
Phe Ser Phe Leu Ile Phe Ala Val Val Gly Val Ile Ala Leu Ala Phe
                325                 330                 335

gtc acc aag ttt gtt cct gaa acc cgt ggc cgc tca ctt gaa gaa ctc     1056
Val Thr Lys Phe Val Pro Glu Thr Arg Gly Arg Ser Leu Glu Glu Leu
            340                 345                 350

gat cac gca gca ttc acc ggc cag atc ttc aag aag gct taaacccccct     1105
Asp His Ala Ala Phe Thr Gly Gln Ile Phe Lys Lys Ala
        355                 360                 365

ccgatctctt tgg                                                      1118

<210> 546
<211> 365
<212> PRT
<213> Corynebacterium glutamicum

<400> 546
Thr Val Val Pro Val Tyr Leu Ala Glu Leu Ala Pro Leu Glu Ile Arg
1               5                   10                  15

Gly Ser Leu Thr Gly Arg Asn Glu Leu Ala Ile Val Thr Gly Gln Leu
            20                  25                  30

Leu Ala Phe Val Ile Asn Ala Leu Ile Ala Val Thr Leu His Gly Val
        35                  40                  45

Ile Asp Gly Ile Trp Arg Ile Met Phe Ala Val Cys Ala Leu Pro Ala
    50                  55                  60

Val Ala Leu Phe Leu Gly Met Leu Arg Met Pro Glu Ser Pro Arg Trp
65                  70                  75                  80

Leu Val Asn Gln Gly Arg Tyr Asp Asp Ala Arg Arg Val Met Glu Thr
                85                  90                  95

Val Arg Thr Pro Glu Arg Ala Lys Ala Glu Met Asp Glu Ile Ile Ala
            100                 105                 110

Val His Ser Glu Asn Asn Ala Ala Leu Pro Gly Val Lys Gln Ser Ser
        115                 120                 125

Gly Gln Ala Ser Gly Gln Val Ser Ser Lys His Thr His Met Ser Ile
    130                 135                 140

Gly Glu Val Leu Ser Asn Lys Trp Leu Val Arg Leu Leu Ile Ala Gly
145                 150                 155                 160

Ile Gly Val Ala Val Ala Gln Gln Leu Thr Gly Ile Asn Ala Ile Met
                165                 170                 175

Tyr Tyr Gly Thr Arg Val Leu Glu Glu Ser Gly Met Ser Ala Glu Met
            180                 185                 190

Ala Val Val Ala Asn Ile Ala Phe Gly Ala Val Ala Val Ile Gly Gly
        195                 200                 205

Leu Ile Ala Leu Arg Asn Met Asp Arg Leu Asp Arg Arg Thr Thr Phe
    210                 215                 220

Ile Ile Gly Leu Ser Leu Thr Thr Thr Phe His Leu Leu Ile Ala Ala
225                 230                 235                 240

Ala Gly Thr Leu Leu Pro Glu Gly Asn Ser Ile Arg Pro Phe Ala Ile
                245                 250                 255

Met Ile Leu Val Val Gly Phe Val Leu Ser Met Gln Thr Phe Leu Asn
            260                 265                 270

Val Ala Val Trp Val Trp Leu Ala Glu Ile Phe Pro Val Arg Met Lys
        275                 280                 285

Gly Ile Gly Thr Gly Ile Ser Val Phe Cys Gly Trp Gly Ile Asn Gly
    290                 295                 300

Val Leu Ala Leu Phe Phe Pro Ala Leu Val Ser Gly Val Gly Ile Thr
305                 310                 315                 320

Phe Ser Phe Leu Ile Phe Ala Val Val Gly Val Ile Ala Leu Ala Phe
                325                 330                 335

Val Thr Lys Phe Val Pro Glu Thr Arg Gly Arg Ser Leu Glu Glu Leu
340 345 . 350

Asp His Ala Ala Phe Thr Gly Gln Ile Phe Lys Lys Ala
355 360 365

<210> 547
<211> 2013
<212> DNA
<213> Corynebacterium glutamicum

<220>
<221> CDS
<222> (101)..(1990)
<223> RXN02395

<400> 547
aaacaggaat atttaactcg acttcttaaa aaagctctat acgtaaatat cttgcccatc 60

cagcaccacc tcattgtggt gcacaatgga gaggacacac atg tcc acc aac tct 115
Met Ser Thr Asn Ser
1               5

ggc aat aac ttg cct gaa tcc caa gag tca ccc gag gaa cct cat tat 163
Gly Asn Asn Leu Pro Glu Ser Gln Glu Ser Pro Glu Glu Pro His Tyr
                10                  15                  20

cct cac gac acc cac cca ggc cta gtt cct ggc att tcg gtc gat gct 211
Pro His Asp Thr His Pro Gly Leu Val Pro Gly Ile Ser Val Asp Ala
            25                  30                  35

caa cgc aac aag ttc gga ctc gac aaa acc gtt ttc ggc gtc acc gca 259
Gln Arg Asn Lys Phe Gly Leu Asp Lys Thr Val Phe Gly Val Thr Ala
        40                  45                  50

gca tta att ctg gcg ttc atc gcc tgg ggc att tcc agc cct gat tcg 307
Ala Leu Ile Leu Ala Phe Ile Ala Trp Gly Ile Ser Ser Pro Asp Ser
    55                  60                  65

gtt tct tca gta tcg tcc acg atg ttc agt tgg gct atg acg aat act 355
Val Ser Ser Val Ser Ser Thr Met Phe Ser Trp Ala Met Thr Asn Thr
70                  75                  80                  85

ggt tgg ctc ctc aac ttt gtc atg ctg atc ggc att ggc acc atg ctc 403
Gly Trp Leu Leu Asn Phe Val Met Leu Ile Gly Ile Gly Thr Met Leu
                90                  95                  100

tac atc gct ttt tcg cgc tac ggc cgc atc aag ctg ggc acg gac gaa 451
Tyr Ile Ala Phe Ser Arg Tyr Gly Arg Ile Lys Leu Gly Thr Asp Glu
            105                 110                 115

gac gaa cca gaa ttc tct cgc ttt tcc tgg att gcg atg atg ttc ggc 499
Asp Glu Pro Glu Phe Ser Arg Phe Ser Trp Ile Ala Met Met Phe Gly
        120                 125                 130

gcc ggc atc ggt gtc ggt atc ttc ttc ttc ggt cct tcc gaa ccg ctg 547
Ala Gly Ile Gly Val Gly Ile Phe Phe Phe Gly Pro Ser Glu Pro Leu
    135                 140                 145

tgg cat tat ctc agt ccc cca cct cac acc gtg gaa gga tct aca cct 595
Trp His Tyr Leu Ser Pro Pro Pro His Thr Val Glu Gly Ser Thr Pro
150                 155                 160                 165

gag tcc ttg cac caa gca ctt gcg cag tcc cac ttc cac tgg ggc tta 643
Glu Ser Leu His Gln Ala Leu Ala Gln Ser His Phe His Trp Gly Leu
                170                 175                 180

tcc gcc tgg ggg ctg tat gcc ttg gtc ggc ggc gcg ttg gcg tac tcc 691
Ser Ala Trp Gly Leu Tyr Ala Leu Val Gly Gly Ala Leu Ala Tyr Ser
            185                 190                 195

agt tac cga cgc ggc cgc gta acc ttg atc agc tcc acc ttc cgg tca 739
Ser Tyr Arg Arg Gly Arg Val Thr Leu Ile Ser Ser Thr Phe Arg Ser
        200                 205                 210

ctt ttc ggt gag aaa acc gaa ggt atc gcg ggt cgc ctc atc gac atg 787
Leu Phe Gly Glu Lys Thr Glu Gly Ile Ala Gly Arg Leu Ile Asp Met
    215                 220                 225

atg gcg atc atc gcc acg ctg ttt gga acg gct gca acc ttg ggc ctt 835
Met Ala Ile Ile Ala Thr Leu Phe Gly Thr Ala Ala Thr Leu Gly Leu
230                 235                 240                 245

tca gca att cag gtt ggt cag ggc gtg cag att att tct ggc gct tcg 883
Ser Ala Ile Gln Val Gly Gln Gly Val Gln Ile Ile Ser Gly Ala Ser
                250                 255                 260

gaa atc acc aac aac atc ttg atc gcc atc atc gcg atc ttg acc att 931
Glu Ile Thr Asn Asn Ile Leu Ile Ala Ile Ile Ala Ile Leu Thr Ile
            265                 270                 275

ggc ttc atc atc tcc tca gta tct ggc gtg tcc aag ggc att cgt tat 979
Gly Phe Ile Ile Ser Ser Val Ser Gly Val Ser Lys Gly Ile Arg Tyr
        280                 285                 290

ctt tcc aac ctc aat att tcc ttg acg ctt gga ttg gtc ctg ttt gtg 1027
Leu Ser Asn Leu Asn Ile Ser Leu Thr Leu Gly Leu Val Leu Phe Val
    295                 300                 305

ttc atc acc ggc ccc acc ttg ttc ctg ctc aac ttg atc cca tcg agt 1075
Phe Ile Thr Gly Pro Thr Leu Phe Leu Leu Asn Leu Ile Pro Ser Ser
310                 315                 320                 325

gtg ttg gaa tat ggc agt gag ttc ttg tca atg gct ggc aag tct ttg 1123
Val Leu Glu Tyr Gly Ser Glu Phe Leu Ser Met Ala Gly Lys Ser Leu
                330                 335                 340

tcg tgg ggt gag gaa acc att gaa ttc caa gct ggt tgg acg gcg ttc 1171
Ser Trp Gly Glu Glu Thr Ile Glu Phe Gln Ala Gly Trp Thr Ala Phe
            345                 350                 355

tac tgg gca tgg tgg atc gca tgg act cca ttc gtg ggt atg ttc atc 1219
Tyr Trp Ala Trp Trp Ile Ala Trp Thr Pro Phe Val Gly Met Phe Ile
        360                 365                 370

gcg cgt att tct cgc ggc cgc acc ttg cgt gaa ttc gcg ctc atc acc 1267
Ala Arg Ile Ser Arg Gly Arg Thr Leu Arg Glu Phe Ala Leu Ile Thr
    375                 380                 385

atg gct atc ccc tcc ttc att ttg atc ctg gcg ttc acc att ttc ggt 1315
Met Ala Ile Pro Ser Phe Ile Leu Ile Leu Ala Phe Thr Ile Phe Gly
390                 395                 400                 405

gga act gcc atc acg atg aac cgc gag aac gta gat ggt ttt gac ggc 1363
Gly Thr Ala Ile Thr Met Asn Arg Glu Asn Val Asp Gly Phe Asp Gly
                410                 415                 420

agt tca tcc aag gaa cag gtg ctg ttt gat atg ttc agc aac ctt ccg     1411
Ser Ser Ser Lys Glu Gln Val Leu Phe Asp Met Phe Ser Asn Leu Pro
            425                 430                 435

ctg tac tcg atc aca ccg ttc att ttg atc ttt gtg ctg gca gta ttc     1459
Leu Tyr Ser Ile Thr Pro Phe Ile Leu Ile Phe Val Leu Ala Val Phe
        440                 445                 450

ttt gtt acc tct gcc gat tcc gcc tcc gtg gtg atg gga acg atg agc     1507
Phe Val Thr Ser Ala Asp Ser Ala Ser Val Val Met Gly Thr Met Ser
    455                 460                 465

tcc caa ggt aac cct gca cca aac aaa tta atc gtg gtg ttc tgg gga     1555
Ser Gln Gly Asn Pro Ala Pro Asn Lys Leu Ile Val Val Phe Trp Gly
470                 475                 480                 485

ctg tgc atg atg ggc atc gcg gtg gtc atg ctg ctt act ggt ggc gaa     1603
Leu Cys Met Met Gly Ile Ala Val Val Met Leu Leu Thr Gly Gly Glu
                490                 495                 500

tcc gcg ctg act ggt ctg cag aac ctc acc att ttg atc gcc att ccg     1651
Ser Ala Leu Thr Gly Leu Gln Asn Leu Thr Ile Leu Ile Ala Ile Pro
            505                 510                 515

ttt gcg ctg gtg ttg atc gtg atg gct att gcc ttt att aag gac tta     1699
Phe Ala Leu Val Leu Ile Val Met Ala Ile Ala Phe Ile Lys Asp Leu
        520                 525                 530

tcc aca gac cca gcc gct att cga caa cgc tat gca aag gca gcc atc     1747
Ser Thr Asp Pro Ala Ala Ile Arg Gln Arg Tyr Ala Lys Ala Ala Ile
    535                 540                 545

tct aac gcg gtg gtt cgt ggc ttg gaa gaa cac ggc gac gac ttc gag     1795
Ser Asn Ala Val Val Arg Gly Leu Glu Glu His Gly Asp Asp Phe Glu
550                 555                 560                 565

ctc tcc atc gaa cct gca gag gaa ggt cgt gga gcg ggt gct acc ttc     1843
Leu Ser Ile Glu Pro Ala Glu Glu Gly Arg Gly Ala Gly Ala Thr Phe
                570                 575                 580

gat tcc acc gct gat cac atc acc gac tgg tat cag cgc acc gac gaa     1891
Asp Ser Thr Ala Asp His Ile Thr Asp Trp Tyr Gln Arg Thr Asp Glu
            585                 590                 595

gaa ggc aat gat gtt gat tat gac ttc acc acc ggc aaa tgg gcc gat     1939
Glu Gly Asn Asp Val Asp Tyr Asp Phe Thr Thr Gly Lys Trp Ala Asp
        600                 605                 610

ggt tgg aca ccg gaa tcg acc gaa gaa ggc gaa gtg gac gcg aaa aag     1987
Gly Trp Thr Pro Glu Ser Thr Glu Glu Gly Glu Val Asp Ala Lys Lys
    615                 620                 625

gat taaaaataa cgactggctg gga                                        2013
Asp
630

<210> 548
<211> 630
<212> PRT
<213> Corynebacterium glutamicum

<400> 548
Met Ser Thr Asn Ser Gly Asn Asn Leu Pro Glu Ser Gln Glu Ser Pro
1               5                   10                  15

Glu Glu Pro His Tyr Pro His Asp Thr His Pro Gly Leu Val Pro Gly
            20                  25                  30

Ile Ser Val Asp Ala Gln Arg Asn Lys Phe Gly Leu Asp Lys Thr Val
        35                  40                  45

Phe Gly Val Thr Ala Ala Leu Ile Leu Ala Phe Ile Ala Trp Gly Ile
    50                  55                  60

Ser Ser Pro Asp Ser Val Ser Ser Val Ser Ser Thr Met Phe Ser Trp
65                  70                  75                  80

Ala Met Thr Asn Thr Gly Trp Leu Leu Asn Phe Val Met Leu Ile Gly
                85                  90                  95

Ile Gly Thr Met Leu Tyr Ile Ala Phe Ser Arg Tyr Gly Arg Ile Lys
            100                 105                 110

Leu Gly Thr Asp Glu Asp Glu Pro Glu Phe Ser Arg Phe Ser Trp Ile
        115                 120                 125

Ala Met Met Phe Gly Ala Gly Ile Gly Val Gly Ile Phe Phe Phe Gly
    130                 135                 140

Pro Ser Glu Pro Leu Trp His Tyr Leu Ser Pro Pro Pro His Thr Val
145                 150                 155                 160

Glu Gly Ser Thr Pro Glu Ser Leu His Gln Ala Leu Ala Gln Ser His
                165                 170                 175

Phe His Trp Gly Leu Ser Ala Trp Gly Leu Tyr Ala Leu Val Gly Gly
            180                 185                 190

Ala Leu Ala Tyr Ser Ser Tyr Arg Arg Gly Arg Val Thr Leu Ile Ser
        195                 200                 205

Ser Thr Phe Arg Ser Leu Phe Gly Glu Lys Thr Glu Gly Ile Ala Gly
    210                 215                 220

Arg Leu Ile Asp Met Met Ala Ile Ile Ala Thr Leu Phe Gly Thr Ala
225                 230                 235                 240

Ala Thr Leu Gly Leu Ser Ala Ile Gln Val Gly Gln Gly Val Gln Ile
                245                 250                 255

Ile Ser Gly Ala Ser Glu Ile Thr Asn Asn Ile Leu Ile Ala Ile Ile
            260                 265                 270

Ala Ile Leu Thr Ile Gly Phe Ile Ile Ser Ser Val Ser Gly Val Ser
        275                 280                 285

Lys Gly Ile Arg Tyr Leu Ser Asn Leu Asn Ile Ser Leu Thr Leu Gly
    290                 295                 300

Leu Val Leu Phe Val Phe Ile Thr Gly Pro Thr Leu Phe Leu Leu Asn
305                 310                 315                 320

Leu Ile Pro Ser Ser Val Leu Glu Tyr Gly Ser Glu Phe Leu Ser Met
                325                 330                 335

Ala Gly Lys Ser Leu Ser Trp Gly Glu Glu Thr Ile Glu Phe Gln Ala
            340                 345                 350

Gly Trp Thr Ala Phe Tyr Trp Ala Trp Trp Ile Ala Trp Thr Pro Phe
        355                 360                 365

Val Gly Met Phe Ile Ala Arg Ile Ser Arg Gly Arg Thr Leu Arg Glu
    370                 375                 380

Phe Ala Leu Ile Thr Met Ala Ile Pro Ser Phe Ile Leu Ile Leu Ala
385                 390                 395                 400

Phe Thr Ile Phe Gly Gly Thr Ala Ile Thr Met Asn Arg Glu Asn Val
                405                 410                 415

Asp Gly Phe Asp Gly Ser Ser Ser Lys Glu Gln Val Leu Phe Asp Met
            420                 425                 430

Phe Ser Asn Leu Pro Leu Tyr Ser Ile Thr Pro Phe Ile Leu Ile Phe
        435                 440                 445

Val Leu Ala Val Phe Phe Val Thr Ser Ala Asp Ser Ala Ser Val Val
    450                 455                 460

Met Gly Thr Met Ser Ser Gln Gly Asn Pro Ala Pro Asn Lys Leu Ile
465                 470                 475                 480

Val Val Phe Trp Gly Leu Cys Met Met Gly Ile Ala Val Val Met Leu
                485                 490                 495

Leu Thr Gly Gly Glu Ser Ala Leu Thr Gly Leu Gln Asn Leu Thr Ile
            500                 505                 510

Leu Ile Ala Ile Pro Phe Ala Leu Val Leu Ile Val Met Ala Ile Ala
        515                 520                 525

Phe Ile Lys Asp Leu Ser Thr Asp Pro Ala Ala Ile Arg Gln Arg Tyr
    530                 535                 540

Ala Lys Ala Ala Ile Ser Asn Ala Val Val Arg Gly Leu Glu Glu His
545                 550                 555                 560

Gly Asp Asp Phe Glu Leu Ser Ile Glu Pro Ala Glu Glu Gly Arg Gly
                565                 570                 575

Ala Gly Ala Thr Phe Asp Ser Thr Ala Asp His Ile Thr Asp Trp Tyr
            580                 585                 590

Gln Arg Thr Asp Glu Glu Gly Asn Asp Val Asp Tyr Asp Phe Thr Thr
        595                 600                 605

Gly Lys Trp Ala Asp Gly Trp Thr Pro Glu Ser Thr Glu Glu Gly Glu
    610                 615                 620

Val Asp Ala Lys Lys Asp
625                 630

<210> 549
<211> 2007
<212> DNA
<213> Corynebacterium glutamicum

<220>
<221> CDS
<222> (101)..(1984)
<223> RXN02348

<400> 549
aaagacccga gccgaagccc tggcctgcgc atacttcctt gtcaacgctc gctgggatta 60

ggtcttttct gagcgctagc atttctccac tcaaaggagc atg ctt aac cgc atg 115
Met Leu Asn Arg Met
1               5

aaa agt gcg cgg cca aaa tca gtc gct cca aaa tcc gga caa gct tta 163
Lys Ser Ala Arg Pro Lys Ser Val Ala Pro Lys Ser Gly Gln Ala Leu
                10                  15                  20

ctc act ctc ggt gcc cta ggt gtt gtg ttc ggc gac atc ggc acc agc 211
Leu Thr Leu Gly Ala Leu Gly Val Val Phe Gly Asp Ile Gly Thr Ser
            25                  30                  35

ccc ctg tac tca ctt cac act gca ttc agc atg cag cac aac aaa gtc 259
Pro Leu Tyr Ser Leu His Thr Ala Phe Ser Met Gln His Asn Lys Val
        40                  45                  50

gaa gtc act cag gaa aat gtg tac ggc atc atc tcc atg gtg ttg tgg 307
Glu Val Thr Gln Glu Asn Val Tyr Gly Ile Ile Ser Met Val Leu Trp
    55                  60                  65

acc atc act ttg atc gtc acc gtc aaa tac gtc atg ctg gtc acc cga 355
Thr Ile Thr Leu Ile Val Thr Val Lys Tyr Val Met Leu Val Thr Arg
70                  75                  80                  85

gct gac aac caa gga caa ggt ggc atc ctg gcg ctc gtt gct ttg ctg 403
Ala Asp Asn Gln Gly Gln Gly Gly Ile Leu Ala Leu Val Ala Leu Leu
                90                  95                  100

aaa aac cgt ggg cac tgg gga aaa ttc gtg gca gta gcc ggc atg ttg 451
Lys Asn Arg Gly His Trp Gly Lys Phe Val Ala Val Ala Gly Met Leu
            105                 110                 115

ggc gcc gca ttg ttt tat ggc gat gtg gtg atc acc ccg gcg atc tct 499
Gly Ala Ala Leu Phe Tyr Gly Asp Val Val Ile Thr Pro Ala Ile Ser
        120                 125                 130

gtt ctc agc gca aca gaa ggc ttg acg gtt atc tcc cca agc ttt gag 547
Val Leu Ser Ala Thr Glu Gly Leu Thr Val Ile Ser Pro Ser Phe Glu
    135                 140                 145

cgc ttc att ctg ccc gta tct ctc gca gtt ctg atc gct att ttt gca 595
Arg Phe Ile Leu Pro Val Ser Leu Ala Val Leu Ile Ala Ile Phe Ala
150                 155                 160                 165

atc caa ccg ctc ggt aca gaa aaa gtc ggc aaa gcc ttc ggc ccc atc 643
Ile Gln Pro Leu Gly Thr Glu Lys Val Gly Lys Ala Phe Gly Pro Ile
                170                 175                 180

atg ttg ctg tgg ttt gtc acc ctt gca gga ttg gga att ccg caa atc 691
Met Leu Leu Trp Phe Val Thr Leu Ala Gly Leu Gly Ile Pro Gln Ile
            185                 190                 195

atc ggg cac cca gaa atc ttg cag agc ttg tct cca cat tgg gcc ctg 739
Ile Gly His Pro Glu Ile Leu Gln Ser Leu Ser Pro His Trp Ala Leu
        200                 205                 210

cgc ttg att gtg gct gag cct ttc caa gca ttt gtg ctg ctt ggt gcc 787
Arg Leu Ile Val Ala Glu Pro Phe Gln Ala Phe Val Leu Leu Gly Ala
    215                 220                 225

gtt gtc ctg aca gta acg ggt gcg gaa gcg ctc tac gct gat atg ggc 835
Val Val Leu Thr Val Thr Gly Ala Glu Ala Leu Tyr Ala Asp Met Gly
230                 235                 240                 245

cat ttt ggg gcg agg cca atc aga gtg gcg tgg ttt tgc gtc gtc atg 883
His Phe Gly Ala Arg Pro Ile Arg Val Ala Trp Phe Cys Val Val Met
                250                 255                 260

cct gct tta atc ttg acg tat ttg ggg cag ggc gcc ttg gtg atc aac 931
Pro Ala Leu Ile Leu Thr Tyr Leu Gly Gln Gly Ala Leu Val Ile Asn
            265                 270                 275

cag cct gaa gcg gtg cgc aac ccc atg ttt tat ctc gcg ccg gaa ggt 979
Gln Pro Glu Ala Val Arg Asn Pro Met Phe Tyr Leu Ala Pro Glu Gly
        280                 285                 290

ctg cgg att ccg ttg gtt att ttg gcg acc atc gct acg gtg atc gca 1027
Leu Arg Ile Pro Leu Val Ile Leu Ala Thr Ile Ala Thr Val Ile Ala
    295                 300                 305

tcg cag gcc gtg att tct ggt gcg tat tca ttg acc aag cag gcc gtg 1075
Ser Gln Ala Val Ile Ser Gly Ala Tyr Ser Leu Thr Lys Gln Ala Val
310                 315                 320                 325

aat ttg aaa ctg ctg cca cgc atg gtg atc cgg cat acc tcc cgc aaa 1123
Asn Leu Lys Leu Leu Pro Arg Met Val Ile Arg His Thr Ser Arg Lys
                330                 335                 340

gag gaa ggc cag atc tat atg cca ctg gtt aat gga ttg ctg ttt gta 1171
Glu Glu Gly Gln Ile Tyr Met Pro Leu Val Asn Gly Leu Leu Phe Val
            345                 350                 355

tcc gtg atg gtt gtg gtg ctg gta ttc cga tcc tct gaa agc ctc gcc 1219
Ser Val Met Val Val Val Leu Val Phe Arg Ser Ser Glu Ser Leu Ala
        360                 365                 370

agc gcg tac gga ctt gca gtg acc gga acc ttg gtg ctg gtc agc gtc 1267
Ser Ala Tyr Gly Leu Ala Val Thr Gly Thr Leu Val Leu Val Ser Val
    375                 380                 385

ctg tat ctg atc tat gtt cac acc aca tgg tgg aaa aca gcg ctg ttc 1315
Leu Tyr Leu Ile Tyr Val His Thr Thr Trp Trp Lys Thr Ala Leu Phe
390                 395                 400                 405

att gtg ctc atc ggt att cca gaa gta ctt cta ttc gcc tcg aac acc 1363
Ile Val Leu Ile Gly Ile Pro Glu Val Leu Leu Phe Ala Ser Asn Thr
                410                 415                 420

acg aaa att cac gac ggt ggc tgg ctt cca cta ctt att gcg gcc gtg     1411
Thr Lys Ile His Asp Gly Gly Trp Leu Pro Leu Leu Ile Ala Ala Val
            425                 430                 435

ctc atc gtg gtg atg cgg acc tgg gag tgg gga agt gac cgc gtc aat     1459
Leu Ile Val Val Met Arg Thr Trp Glu Trp Gly Ser Asp Arg Val Asn
        440                 445                 450

cag gaa cgc gca gag ctg gaa ctt ccc atg gat aag ttc ttg gag aaa     1507
Gln Glu Arg Ala Glu Leu Glu Leu Pro Met Asp Lys Phe Leu Glu Lys
    455                 460                 465

ctc gat cag cca cac aat att ggt ctg cgt aaa gtt gcc gaa gtg gca     1555
Leu Asp Gln Pro His Asn Ile Gly Leu Arg Lys Val Ala Glu Val Ala
470                 475                 480                 485

gta ttt cca cat ggc acc agc gat act gtc ccg ttg tca ttg gtt cgc     1603
Val Phe Pro His Gly Thr Ser Asp Thr Val Pro Leu Ser Leu Val Arg
                490                 495                 500

tgc gtg aaa gac ctc aag ctt tta tac cga gag atc gtg atc gtt cga     1651
Cys Val Lys Asp Leu Lys Leu Leu Tyr Arg Glu Ile Val Ile Val Arg
            505                 510                 515

atc gtc caa gaa cac gtt ccg cac gtg cca cca gag gaa cgc gcg gaa     1699
Ile Val Gln Glu His Val Pro His Val Pro Pro Glu Glu Arg Ala Glu
        520                 525                 530

atg gaa gtg ctc cat cac gcc ccg atc aga gtc gtg cga gtt gat ctg     1747
Met Glu Val Leu His His Ala Pro Ile Arg Val Val Arg Val Asp Leu
    535                 540                 545

cac ctt ggt tat ttt gat gag cag aac ctg cct gag cat ctc cat gcc     1795
His Leu Gly Tyr Phe Asp Glu Gln Asn Leu Pro Glu His Leu His Ala
550                 555                 560                 565

att gac cca aca tgg gat aac gcc acc tac ttc ctg tct gcc ctg act     1843
Ile Asp Pro Thr Trp Asp Asn Ala Thr Tyr Phe Leu Ser Ala Leu Thr
                570                 575                 580

ctt cgg agc agg ttg cct gga aag att gct ggc tgg cgt gat cgt ttg     1891
Leu Arg Ser Arg Leu Pro Gly Lys Ile Ala Gly Trp Arg Asp Arg Leu
            585                 590                 595

tat ctt tcg atg gaa cgt aat cag gca tct cga act gag tct ttc aaa     1939
Tyr Leu Ser Met Glu Arg Asn Gln Ala Ser Arg Thr Glu Ser Phe Lys
        600                 605                 610

ttg caa cca agc aaa acc atc acg gtt gga aca gag ctg cac ctt         1984
Leu Gln Pro Ser Lys Thr Ile Thr Val Gly Thr Glu Leu His Leu
    615                 620                 625

taatcaggca gttgctggcc aac                                           2007

<210> 550
<211> 628
<212> PRT
<213> Corynebacterium glutamicum

<400> 550

Met Leu Asn Arg Met Lys Ser Ala Arg Pro Lys Ser Val Ala Pro Lys
1               5                   10                  15

Ser Gly Gln Ala Leu Leu Thr Leu Gly Ala Leu Gly Val Val Phe Gly
            20                  25                  30

Asp Ile Gly Thr Ser Pro Leu Tyr Ser Leu His Thr Ala Phe Ser Met
        35                  40                  45

Gln His Asn Lys Val Glu Val Thr Gln Glu Asn Val Tyr Gly Ile Ile
    50                  55                  60

Ser Met Val Leu Trp Thr Ile Thr Leu Ile Val Thr Val Lys Tyr Val
65                  70                  75                  80

Met Leu Val Thr Arg Ala Asp Asn Gln Gly Gln Gly Gly Ile Leu Ala
                85                  90                  95

Leu Val Ala Leu Leu Lys Asn Arg Gly His Trp Gly Lys Phe Val Ala
            100                 105                 110

Val Ala Gly Met Leu Gly Ala Ala Leu Phe Tyr Gly Asp Val Val Ile
        115                 120                 125

Thr Pro Ala Ile Ser Val Leu Ser Ala Thr Glu Gly Leu Thr Val Ile
    130                 135                 140

Ser Pro Ser Phe Glu Arg Phe Ile Leu Pro Val Ser Leu Ala Val Leu
145                 150                 155                 160

Ile Ala Ile Phe Ala Ile Gln Pro Leu Gly Thr Glu Lys Val Gly Lys
                165                 170                 175

Ala Phe Gly Pro Ile Met Leu Leu Trp Phe Val Thr Leu Ala Gly Leu
            180                 185                 190

Gly Ile Pro Gln Ile Ile Gly His Pro Glu Ile Leu Gln Ser Leu Ser
        195                 200                 205

Pro His Trp Ala Leu Arg Leu Ile Val Ala Glu Pro Phe Gln Ala Phe
    210                 215                 220

Val Leu Leu Gly Ala Val Val Leu Thr Val Thr Gly Ala Glu Ala Leu
225                 230                 235                 240

Tyr Ala Asp Met Gly His Phe Gly Ala Arg Pro Ile Arg Val Ala Trp
                245                 250                 255

Phe Cys Val Val Met Pro Ala Leu Ile Leu Thr Tyr Leu Gly Gln Gly
            260                 265                 270

Ala Leu Val Ile Asn Gln Pro Glu Ala Val Arg Asn Pro Met Phe Tyr
        275                 280                 285

Leu Ala Pro Glu Gly Leu Arg Ile Pro Leu Val Ile Leu Ala Thr Ile
    290                 295                 300

Ala Thr Val Ile Ala Ser Gln Ala Val Ile Ser Gly Ala Tyr Ser Leu
305                 310                 315                 320

Thr Lys Gln Ala Val Asn Leu Lys Leu Leu Pro Arg Met Val Ile Arg

325                 330                 335

His Thr Ser Arg Lys Glu Glu Gly Gln Ile Tyr Met Pro Leu Val Asn
            340                 345                 350

Gly Leu Leu Phe Val Ser Val Met Val Val Val Leu Val Phe Arg Ser
        355                 360                 365

Ser Glu Ser Leu Ala Ser Ala Tyr Gly Leu Ala Val Thr Gly Thr Leu
    370                 375                 380

Val Leu Val Ser Val Leu Tyr Leu Ile Tyr Val His Thr Thr Trp Trp
385                 390                 395                 400

Lys Thr Ala Leu Phe Ile Val Leu Ile Gly Ile Pro Glu Val Leu Leu
                405                 410                 415

Phe Ala Ser Asn Thr Thr Lys Ile His Asp Gly Gly Trp Leu Pro Leu
            420                 425                 430

Leu Ile Ala Ala Val Leu Ile Val Val Met Arg Thr Trp Glu Trp Gly
        435                 440                 445

Ser Asp Arg Val Asn Gln Glu Arg Ala Glu Leu Glu Leu Pro Met Asp
    450                 455                 460

Lys Phe Leu Glu Lys Leu Asp Gln Pro His Asn Ile Gly Leu Arg Lys
465                 470                 475                 480

Val Ala Glu Val Ala Val Phe Pro His Gly Thr Ser Asp Thr Val Pro
                485                 490                 495

Leu Ser Leu Val Arg Cys Val Lys Asp Leu Lys Leu Leu Tyr Arg Glu
            500                 505                 510

Ile Val Ile Val Arg Ile Val Gln Glu His Val Pro His Val Pro Pro
        515                 520                 525

Glu Glu Arg Ala Glu Met Glu Val Leu His His Ala Pro Ile Arg Val
    530                 535                 540

Val Arg Val Asp Leu His Leu Gly Tyr Phe Asp Glu Gln Asn Leu Pro
545                 550                 555                 560

Glu His Leu His Ala Ile Asp Pro Thr Trp Asp Asn Ala Thr Tyr Phe
                565                 570                 575

Leu Ser Ala Leu Thr Leu Arg Ser Arg Leu Pro Gly Lys Ile Ala Gly
            580                 585                 590

Trp Arg Asp Arg Leu Tyr Leu Ser Met Glu Arg Asn Gln Ala Ser Arg
        595                 600                 605

Thr Glu Ser Phe Lys Leu Gln Pro Ser Lys Thr Ile Thr Val Gly Thr
    610                 615                 620

Glu Leu His Leu
625

<210> 551

<211> 1035
<212> DNA
<213> Corynebacterium glutamicum

<220>
<221> CDS
<222> (101)..(1012)
<223> RXN00297

<400> 551
aacaaggcac cggaaaacaa gcaacgcacc ttgcagaggt ggtcttaagc atcttggagc 60

aaaacaacat ggcacaataa cgatcatgca acaggtgctc atg ggt ttc acg gtg 115
Met Gly Phe Thr Val
1               5

gtg ttc atc gtc atc gga ata ggt tgg att ctc ggt aga aga gac acc 163
Val Phe Ile Val Ile Gly Ile Gly Trp Ile Leu Gly Arg Arg Asp Thr
                10                  15                  20

ttg ggc aca cat gcc cag aaa cct ttg agc ctg ttt gtc tat tac gtg 211
Leu Gly Thr His Ala Gln Lys Pro Leu Ser Leu Phe Val Tyr Tyr Val
            25                  30                  35

gcc acc cca gcg ttg ttg ttt gat cgg gtc acc aag tca gat acc tcg 259
Ala Thr Pro Ala Leu Leu Phe Asp Arg Val Thr Lys Ser Asp Thr Ser
        40                  45                  50

acg att ttc tct ctg aac ttc gtg gtc att gca ctc tct gcg ttg atc 307
Thr Ile Phe Ser Leu Asn Phe Val Val Ile Ala Leu Ser Ala Leu Ile
    55                  60                  65

gta ggt ttc ctg ttt ttc cta ctc atg cgg ttc gtg atc aaa aga act 355
Val Gly Phe Leu Phe Phe Leu Leu Met Arg Phe Val Ile Lys Arg Thr
70                  75                  80                  85

gcc gca gta tcg gtg atc ggc atg cta gct gcg tcc tac gcc aac gcc 403
Ala Ala Val Ser Val Ile Gly Met Leu Ala Ala Ser Tyr Ala Asn Ala
                90                  95                  100

ggt aac ctg ggt atc cct ttg gca gcc tat att ttg gat gat ttc acc 451
Gly Asn Leu Gly Ile Pro Leu Ala Ala Tyr Ile Leu Asp Asp Phe Thr
            105                 110                 115

gtg gtg att ccc gtg att ttg ttc cag gtg gcg ttc tac gca ccg atc 499
Val Val Ile Pro Val Ile Leu Phe Gln Val Ala Phe Tyr Ala Pro Ile
        120                 125                 130

acc atg acc atc atg gag atg ctg acc aac aag aaa tcc acc aac ttg 547
Thr Met Thr Ile Met Glu Met Leu Thr Asn Lys Lys Ser Thr Asn Leu
    135                 140                 145

gtg cgc aac ctc ctg gtc acg cca cta acc aac acc atg gtg ctc gca 595
Val Arg Asn Leu Leu Val Thr Pro Leu Thr Asn Thr Met Val Leu Ala
150                 155                 160                 165

gcg att gcc ggt att gct gtg tct ttg act tcg atg agc gtg ccc gtg 643
Ala Ile Ala Gly Ile Ala Val Ser Leu Thr Ser Met Ser Val Pro Val
                170                 175                 180

gtg att gct cag cca gtg gaa atg ttg gcg aat gct tca gtg cca ctg 691

Val Ile Ala Gln Pro Val Glu Met Leu Ala Asn Ala Ser Val Pro Leu
            185                 190                 195

gct ttg gtg gtg ttc gga ctg tcc ttg tcc aag agc aag atc ctg gaa 739
Ala Leu Val Val Phe Gly Leu Ser Leu Ser Lys Ser Lys Ile Leu Glu
        200                 205                 210

aag ggg cag gta tcc agg cgc gat gtg ttt acg gcg gca ctg ttc aag 787
Lys Gly Gln Val Ser Arg Arg Asp Val Phe Thr Ala Ala Leu Phe Lys
    215                 220                 225

aat gtt ctg cac cca att gtt gcg gga ctt tta gcc tta gcc ttt ggt 835
Asn Val Leu His Pro Ile Val Ala Gly Leu Leu Ala Leu Ala Phe Gly
230                 235                 240                 245

atg gaa ggt act gcc ttg ttg tcg gcg gtg att ctc ggt gca ctg cca 883
Met Glu Gly Thr Ala Leu Leu Ser Ala Val Ile Leu Gly Ala Leu Pro
                250                 255                 260

aca gcg cag aat gtc tac acc tac gcg ttg cga ttt aga aca gct gaa 931
Thr Ala Gln Asn Val Tyr Thr Tyr Ala Leu Arg Phe Arg Thr Ala Glu
            265                 270                 275

tcg atg gcg aga gac aca ggg gtg gtc acc aca ctc att tcc ttc ccc 979
Ser Met Ala Arg Asp Thr Gly Val Val Thr Thr Leu Ile Ser Phe Pro
        280                 285                 290

gta ttg gtg gcg gtc tcc att atc ttt gga tct tagggttagc attagtggtc 1032
Val Leu Val Ala Val Ser Ile Ile Phe Gly Ser
    295                 300

atg 1035

<210> 552
<211> 304
<212> PRT
<213> Corynebacterium glutamicum

<400> 552
Met Gly Phe Thr Val Val Phe Ile Val Ile Gly Ile Gly Trp Ile Leu
1               5                   10                  15

Gly Arg Arg Asp Thr Leu Gly Thr His Ala Gln Lys Pro Leu Ser Leu
            20                  25                  30

Phe Val Tyr Tyr Val Ala Thr Pro Ala Leu Leu Phe Asp Arg Val Thr
        35                  40                  45

Lys Ser Asp Thr Ser Thr Ile Phe Ser Leu Asn Phe Val Val Ile Ala
    50                  55                  60

Leu Ser Ala Leu Ile Val Gly Phe Leu Phe Phe Leu Leu Met Arg Phe
65                  70                  75                  80

Val Ile Lys Arg Thr Ala Ala Val Ser Val Ile Gly Met Leu Ala Ala
                85                  90                  95

Ser Tyr Ala Asn Ala Gly Asn Leu Gly Ile Pro Leu Ala Ala Tyr Ile
            100                 105                 110

Leu Asp Asp Phe Thr Val Val Ile Pro Val Ile Leu Phe Gln Val Ala
        115                 120                 125

Phe Tyr Ala Pro Ile Thr Met Thr Ile Met Glu Met Leu Thr Asn Lys
    130                 135                 140

Lys Ser Thr Asn Leu Val Arg Asn Leu Leu Val Thr Pro Leu Thr Asn
145                 150                 155                 160

Thr Met Val Leu Ala Ala Ile Ala Gly Ile Ala Val Ser Leu Thr Ser
                165                 170                 175

Met Ser Val Pro Val Val Ile Ala Gln Pro Val Glu Met Leu Ala Asn
            180                 185                 190

Ala Ser Val Pro Leu Ala Leu Val Val Phe Gly Leu Ser Leu Ser Lys
        195                 200                 205

Ser Lys Ile Leu Glu Lys Gly Gln Val Ser Arg Arg Asp Val Phe Thr
    210                 215                 220

Ala Ala Leu Phe Lys Asn Val Leu His Pro Ile Val Ala Gly Leu Leu
225                 230                 235                 240

Ala Leu Ala Phe Gly Met Glu Gly Thr Ala Leu Leu Ser Ala Val Ile
                245                 250                 255

Leu Gly Ala Leu Pro Thr Ala Gln Asn Val Tyr Thr Tyr Ala Leu Arg
            260                 265                 270

Phe Arg Thr Ala Glu Ser Met Ala Arg Asp Thr Gly Val Val Thr Thr
        275                 280                 285

Leu Ile Ser Phe Pro Val Leu Val Ala Val Ser Ile Ile Phe Gly Ser
    290                 295                 300

<210> 553
<211> 353
<212> DNA
<213> Corynebacterium glutamicum

<220>
<221> CDS
<222> (101)..(343)
<223> RXN03103

<400> 553
atcttcagag tcacttcttt cagtgtcatt tttctcggcc ctaatccccc gctggagttc 60

aatcagcgat tgcaaccttt tagatatata aggagacaac atg tct gca aag cgt   115
                                           Met Ser Ala Lys Arg
                                           1               5

act ttt acc cgt atc ggt gcg att ctt gga gca act gca ctt gcc gga   163
Thr Phe Thr Arg Ile Gly Ala Ile Leu Gly Ala Thr Ala Leu Ala Gly
                10                  15                  20

gtt acc ctc acc gcc tgt ggt gat tca agc ggt ggc gac gga ttc ctc     211
Val Thr Leu Thr Ala Cys Gly Asp Ser Ser Gly Gly Asp Gly Phe Leu
            25                  30                  35

gca gcc att gaa aat ggt tct gtc aat gtc ggc acc aaa tac gat cag     259
Ala Ala Ile Glu Asn Gly Ser Val Asn Val Gly Thr Lys Tyr Asp Gln
        40                  45                  50

cct ggt ctt ggc ctc cgc aac cca gac aac tcc atg agc ggt ctc gac     307
Pro Gly Leu Gly Leu Arg Asn Pro Asp Asn Ser Met Ser Gly Leu Asp
    55                  60                  65

gtg gat gtt gct gaa tac gta att caa ctc cat cgc tgatgacaag          353
Val Asp Val Ala Glu Tyr Val Ile Gln Leu His Arg
70                  75                  80


<210> 554
<211> 81
<212> PRT
<213> Corynebacterium glutamicum

<400> 554
Met Ser Ala Lys Arg Thr Phe Thr Arg Ile Gly Ala Ile Leu Gly Ala
1               5                   10                  15

Thr Ala Leu Ala Gly Val Thr Leu Thr Ala Cys Gly Asp Ser Ser Gly
            20                  25                  30

Gly Asp Gly Phe Leu Ala Ala Ile Glu Asn Gly Ser Val Asn Val Gly
        35                  40                  45

Thr Lys Tyr Asp Gln Pro Gly Leu Gly Leu Arg Asn Pro Asp Asn Ser
    50                  55                  60

Met Ser Gly Leu Asp Val Asp Val Ala Glu Tyr Val Ile Gln Leu His
65                  70                  75                  80

Arg



<210> 555
<211> 695
<212> DNA
<213> Corynebacterium glutamicum

<220>
<221> CDS
<222> (1)..(672)
<223> RXN02993

<400> 555
gtt gct gaa tac gta gtc aac tcc atc gct gat gac aag ggc tgg gat     48
Val Ala Glu Tyr Val Val Asn Ser Ile Ala Asp Asp Lys Gly Trp Asp
1               5                   10                  15

cac ccc acc atc gaa tgg cgt gaa tcc cct tct gcg cag cgt gaa acc     96
His Pro Thr Ile Glu Trp Arg Glu Ser Pro Ser Ala Gln Arg Glu Thr
            20                  25                  30

ctc att caa aac ggt gag gta gac atg atc gca gca acc tac tcc atc     144
Leu Ile Gln Asn Gly Glu Val Asp Met Ile Ala Ala Thr Tyr Ser Ile
         35                  40                  45

aac gct ggc cgt tca gag tcc gtc aac ttc ggt ggc cca tac ctg ctt     192
Asn Ala Gly Arg Ser Glu Ser Val Asn Phe Gly Gly Pro Tyr Leu Leu
     50                  55                  60

acc cac cag gct ctg ctt gtt cgc caa gat gac gat cgc att gaa acc     240
Thr His Gln Ala Leu Leu Val Arg Gln Asp Asp Asp Arg Ile Glu Thr
 65                  70                  75                  80

ctc gag gac ttg gat aac ggt ttg atc ctg tgc tcc gtt tcc gga tcc     288
Leu Glu Asp Leu Asp Asn Gly Leu Ile Leu Cys Ser Val Ser Gly Ser
                 85                  90                  95

act cca gct cag aag gtc aag gat gtc ctc cca ggc gtt cag ctc caa     336
Thr Pro Ala Gln Lys Val Lys Asp Val Leu Pro Gly Val Gln Leu Gln
            100                 105                 110

gaa tac gac acc tac tct tcc tgt gtt gag gca ctg tcc cag ggc aac     384
Glu Tyr Asp Thr Tyr Ser Ser Cys Val Glu Ala Leu Ser Gln Gly Asn
        115                 120                 125

gtt gac gcc ctg acc act gac gcc acc atc ctc ttc ggc tac tcc cag     432
Val Asp Ala Leu Thr Thr Asp Ala Thr Ile Leu Phe Gly Tyr Ser Gln
    130                 135                 140

cag tac gaa ggc gac ttc cgc gtt gtg gaa atg gaa aag gac ggc gag     480
Gln Tyr Glu Gly Asp Phe Arg Val Val Glu Met Glu Lys Asp Gly Glu
145                 150                 155                 160

cca ttc acc gac gag tac tac ggc att ggc ctg aag aag gat gac cag     528
Pro Phe Thr Asp Glu Tyr Tyr Gly Ile Gly Leu Lys Lys Asp Asp Gln
                165                 170                 175

gaa ggc acc gac gct atc aac gcc gca ctt gag cgc atg tac gct gac     576
Glu Gly Thr Asp Ala Ile Asn Ala Ala Leu Glu Arg Met Tyr Ala Asp
            180                 185                 190

ggc acc ttc cag cga ctg ctc acc gag aac ctc ggt gaa gac tcc gtg     624
Gly Thr Phe Gln Arg Leu Leu Thr Glu Asn Leu Gly Glu Asp Ser Val
        195                 200                 205

gtt gtt gaa gaa ggc acc cca ggt gac ctc tcc ttc ctc gac gca agc     672
Val Val Glu Glu Gly Thr Pro Gly Asp Leu Ser Phe Leu Asp Ala Ser
    210                 215                 220

tagtgtgacg gcttttaaaa gcc                                           695

<210> 556
<211> 224
<212> PRT
<213> Corynebacterium glutamicum

<400> 556
Val Ala Glu Tyr Val Val Asn Ser Ile Ala Asp Asp Lys Gly Trp Asp
  1               5                  10                  15

His Pro Thr Ile Glu Trp Arg Glu Ser Pro Ser Ala Gln Arg Glu Thr

20                  25                  30

Leu Ile Gln Asn Gly Glu Val Asp Met Ile Ala Ala Thr Tyr Ser Ile
        35                  40                  45

Asn Ala Gly Arg Ser Glu Ser Val Asn Phe Gly Gly Pro Tyr Leu Leu
    50                  55                  60

Thr His Gln Ala Leu Leu Val Arg Gln Asp Asp Asp Arg Ile Glu Thr
65                  70                  75                  80

Leu Glu Asp Leu Asp Asn Gly Leu Ile Leu Cys Ser Val Ser Gly Ser
                85                  90                  95

Thr Pro Ala Gln Lys Val Lys Asp Val Leu Pro Gly Val Gln Leu Gln
            100                 105                 110

Glu Tyr Asp Thr Tyr Ser Ser Cys Val Glu Ala Leu Ser Gln Gly Asn
        115                 120                 125

Val Asp Ala Leu Thr Thr Asp Ala Thr Ile Leu Phe Gly Tyr Ser Gln
    130                 135                 140

Gln Tyr Glu Gly Asp Phe Arg Val Val Glu Met Glu Lys Asp Gly Glu
145                 150                 155                 160

Pro Phe Thr Asp Glu Tyr Tyr Gly Ile Gly Leu Lys Lys Asp Asp Gln
                165                 170                 175

Glu Gly Thr Asp Ala Ile Asn Ala Ala Leu Glu Arg Met Tyr Ala Asp
            180                 185                 190

Gly Thr Phe Gln Arg Leu Leu Thr Glu Asn Leu Gly Glu Asp Ser Val
        195                 200                 205

Val Val Glu Glu Gly Thr Pro Gly Asp Leu Ser Phe Leu Asp Ala Ser
    210                 215                 220

<210> 557
<211> 1590
<212> DNA
<213> Corynebacterium glutamicum

<220>
<221> CDS
<222> (101)..(1567)
<223> RXN00349

<400> 557
tgtgtacatc acaatggaat tcggggctag agtatctggt gaaccgtgca taaacgacct 60

gtgattggac tctttttcct tgcaaaatgt tttccagcgg atg ttg agt ttt gcg   115
                                           Met Leu Ser Phe Ala
                                           1               5

acc ctt cgt ggc cgc att tca aca gtt gac gct gca aaa gcc gca cct   163
Thr Leu Arg Gly Arg Ile Ser Thr Val Asp Ala Ala Lys Ala Ala Pro

10                  15                  20

ccg cca tcg cca cta gcc ccg att gat ctc act gac cat agt caa gtg    211
Pro Pro Ser Pro Leu Ala Pro Ile Asp Leu Thr Asp His Ser Gln Val
            25                  30                  35

gcc ggt gtg atg aat ttg gct gcg aga att ggc gat att ttg ctt tct    259
Ala Gly Val Met Asn Leu Ala Ala Arg Ile Gly Asp Ile Leu Leu Ser
        40                  45                  50

tca ggt acg tca aat agt gac acc aag gta caa gtt cga gca gtg acc    307
Ser Gly Thr Ser Asn Ser Asp Thr Lys Val Gln Val Arg Ala Val Thr
    55                  60                  65

tct gcg tac ggt ttg tac tac acg cac gtg gat atc acg ttg aat acg    355
Ser Ala Tyr Gly Leu Tyr Tyr Thr His Val Asp Ile Thr Leu Asn Thr
70                  75                  80                  85

atc acc atc ttc acc aac atc ggt gtg gag agg aag atg ccg gtc aac    403
Ile Thr Ile Phe Thr Asn Ile Gly Val Glu Arg Lys Met Pro Val Asn
                90                  95                  100

gtg ttt cat gtt gta ggc aag ttg gac acc aac ttc tcc aaa ctg tct    451
Val Phe His Val Val Gly Lys Leu Asp Thr Asn Phe Ser Lys Leu Ser
            105                 110                 115

gag gtt gac cgt ttg atc cgt tcc att cag gct ggt gcg acc ccg cct    499
Glu Val Asp Arg Leu Ile Arg Ser Ile Gln Ala Gly Ala Thr Pro Pro
        120                 125                 130

gag gtt gcc gag aaa atc ctg gac gag ttg gag caa tcc cct gcg tct    547
Glu Val Ala Glu Lys Ile Leu Asp Glu Leu Glu Gln Ser Pro Ala Ser
    135                 140                 145

tat ggt ttc cct gtt gcg ttg ctt ggc tgg gca atg atg ggt ggt gct    595
Tyr Gly Phe Pro Val Ala Leu Leu Gly Trp Ala Met Met Gly Gly Ala
150                 155                 160                 165

gtt gct gtg ctg ttg ggt ggt gga tgg cag gtt tcc cta att gct ttt    643
Val Ala Val Leu Leu Gly Gly Gly Trp Gln Val Ser Leu Ile Ala Phe
                170                 175                 180

att acc gcg ttc acg atc att gcc acg acg tca ttt ttg gga aag aag    691
Ile Thr Ala Phe Thr Ile Ile Ala Thr Thr Ser Phe Leu Gly Lys Lys
            185                 190                 195

ggt ttg cct act ttc ttc caa aat gtt gtt ggt ggt ttt att gcc acg    739
Gly Leu Pro Thr Phe Phe Gln Asn Val Val Gly Gly Phe Ile Ala Thr
        200                 205                 210

ctg cct gca tcg att gct tat tct ttg gcg ttg caa ttt ggt ctt gag    787
Leu Pro Ala Ser Ile Ala Tyr Ser Leu Ala Leu Gln Phe Gly Leu Glu
    215                 220                 225

atc aaa ccg agc cag atc atc gca tct gga att gtt gtg ctg ttg gca    835
Ile Lys Pro Ser Gln Ile Ile Ala Ser Gly Ile Val Val Leu Leu Ala
230                 235                 240                 245

ggt ttg aca ctc gtg caa tct ctg cag gac ggc atc acg ggc gct ccg    883
Gly Leu Thr Leu Val Gln Ser Leu Gln Asp Gly Ile Thr Gly Ala Pro
                250                 255                 260

gtg aca gca agt gca cga ttt ttc gaa aca ctc ctg ttt acc ggc ggc     931
Val Thr Ala Ser Ala Arg Phe Phe Glu Thr Leu Leu Phe Thr Gly Gly
            265                 270                 275

att gtt gct ggc gtg ggt ttg ggc att cag ctt tct gaa atc ttg cat     979
Ile Val Ala Gly Val Gly Leu Gly Ile Gln Leu Ser Glu Ile Leu His
        280                 285                 290

gtc atg ttg cct gcc atg gag tcc gct gca gca cct aat tat tcg tct    1027
Val Met Leu Pro Ala Met Glu Ser Ala Ala Ala Pro Asn Tyr Ser Ser
    295                 300                 305

aca ttc gcc cgc att atc gct ggt ggc gtc acc gca gcg gcc ttc gca    1075
Thr Phe Ala Arg Ile Ile Ala Gly Gly Val Thr Ala Ala Ala Phe Ala
310                 315                 320                 325

gtg ggt tgt tac gcg gag tgg tcc tcg gtg att att gcg ggg ctt act    1123
Val Gly Cys Tyr Ala Glu Trp Ser Ser Val Ile Ile Ala Gly Leu Thr
                330                 335                 340

gcg ctg atg ggt tct gcg ttt tat tac ctc ttc gtt gtt tat tta ggc    1171
Ala Leu Met Gly Ser Ala Phe Tyr Tyr Leu Phe Val Val Tyr Leu Gly
            345                 350                 355

ccc gtc tct gcc gct gcg att gct gca aca gca gtt ggt ttc act ggt    1219
Pro Val Ser Ala Ala Ala Ile Ala Ala Thr Ala Val Gly Phe Thr Gly
        360                 365                 370

ggt ttg ctt gcc cgt cga ttc ttg att cca ccg ttg att gtg gcg att    1267
Gly Leu Leu Ala Arg Arg Phe Leu Ile Pro Pro Leu Ile Val Ala Ile
    375                 380                 385

gcc ggc atc aca cca atg ctt cca ggt cta gca att tac cgc gga atg    1315
Ala Gly Ile Thr Pro Met Leu Pro Gly Leu Ala Ile Tyr Arg Gly Met
390                 395                 400                 405

tac gcc acc ctg aat gat caa aca ctc atg ggt ttc acc aac att gcg    1363
Tyr Ala Thr Leu Asn Asp Gln Thr Leu Met Gly Phe Thr Asn Ile Ala
                410                 415                 420

gtt gct tta gcc act gct tca tca ctt gcc gct ggc gtg gtt ttg ggt    1411
Val Ala Leu Ala Thr Ala Ser Ser Leu Ala Ala Gly Val Val Leu Gly
            425                 430                 435

gag tgg att gcc cgc agg cta cgt cgt cca cca cgc ttc aac cca tac    1459
Glu Trp Ile Ala Arg Arg Leu Arg Arg Pro Pro Arg Phe Asn Pro Tyr
        440                 445                 450

cgt gca ttt acc aag gcg aat gag ttc tcc ttc cag gag gaa gct gag    1507
Arg Ala Phe Thr Lys Ala Asn Glu Phe Ser Phe Gln Glu Glu Ala Glu
    455                 460                 465

cag aat cag cgc cgg cag aga aaa cgt cca aag act aat cag aga ttc    1555
Gln Asn Gln Arg Arg Gln Arg Lys Arg Pro Lys Thr Asn Gln Arg Phe
470                 475                 480                 485

ggt aat aaa agg taaaaatcaa cctgcttagg cgt                          1590
Gly Asn Lys Arg

<210> 558
<211> 489
<212> PRT
<213> Corynebacterium glutamicum

<400> 558
Met Leu Ser Phe Ala Thr Leu Arg Gly Arg Ile Ser Thr Val Asp Ala
1               5                   10                  15

Ala Lys Ala Ala Pro Pro Pro Ser Pro Leu Ala Pro Ile Asp Leu Thr
            20                  25                  30

Asp His Ser Gln Val Ala Gly Val Met Asn Leu Ala Ala Arg Ile Gly
        35                  40                  45

Asp Ile Leu Leu Ser Ser Gly Thr Ser Asn Ser Asp Thr Lys Val Gln
    50                  55                  60

Val Arg Ala Val Thr Ser Ala Tyr Gly Leu Tyr Tyr Thr His Val Asp
65                  70                  75                  80

Ile Thr Leu Asn Thr Ile Thr Ile Phe Thr Asn Ile Gly Val Glu Arg
                85                  90                  95

Lys Met Pro Val Asn Val Phe His Val Val Gly Lys Leu Asp Thr Asn
            100                 105                 110

Phe Ser Lys Leu Ser Glu Val Asp Arg Leu Ile Arg Ser Ile Gln Ala
        115                 120                 125

Gly Ala Thr Pro Pro Glu Val Ala Glu Lys Ile Leu Asp Glu Leu Glu
    130                 135                 140

Gln Ser Pro Ala Ser Tyr Gly Phe Pro Val Ala Leu Leu Gly Trp Ala
145                 150                 155                 160

Met Met Gly Gly Ala Val Ala Val Leu Leu Gly Gly Gly Trp Gln Val
                165                 170                 175

Ser Leu Ile Ala Phe Ile Thr Ala Phe Thr Ile Ile Ala Thr Thr Ser
            180                 185                 190

Phe Leu Gly Lys Lys Gly Leu Pro Thr Phe Phe Gln Asn Val Val Gly
        195                 200                 205

Gly Phe Ile Ala Thr Leu Pro Ala Ser Ile Ala Tyr Ser Leu Ala Leu
    210                 215                 220

Gln Phe Gly Leu Glu Ile Lys Pro Ser Gln Ile Ile Ala Ser Gly Ile
225                 230                 235                 240

Val Val Leu Leu Ala Gly Leu Thr Leu Val Gln Ser Leu Gln Asp Gly
                245                 250                 255

Ile Thr Gly Ala Pro Val Thr Ala Ser Ala Arg Phe Phe Glu Thr Leu
            260                 265                 270

Leu Phe Thr Gly Gly Ile Val Ala Gly Val Gly Leu Gly Ile Gln Leu
        275                 280                 285

Ser Glu Ile Leu His Val Met Leu Pro Ala Met Glu Ser Ala Ala Ala
    290                 295                 300

Pro Asn Tyr Ser Ser Thr Phe Ala Arg Ile Ile Ala Gly Gly Val Thr
305                 310                 315                 320

Ala Ala Ala Phe Ala Val Gly Cys Tyr Ala Glu Trp Ser Ser Val Ile
                325                 330                 335

Ile Ala Gly Leu Thr Ala Leu Met Gly Ser Ala Phe Tyr Tyr Leu Phe
            340                 345                 350

Val Val Tyr Leu Gly Pro Val Ser Ala Ala Ala Ile Ala Ala Thr Ala
        355                 360                 365

Val Gly Phe Thr Gly Gly Leu Leu Ala Arg Arg Phe Leu Ile Pro Pro
    370                 375                 380

Leu Ile Val Ala Ile Ala Gly Ile Thr Pro Met Leu Pro Gly Leu Ala
385                 390                 395                 400

Ile Tyr Arg Gly Met Tyr Ala Thr Leu Asn Asp Gln Thr Leu Met Gly
                405                 410                 415

Phe Thr Asn Ile Ala Val Ala Leu Ala Thr Ala Ser Ser Leu Ala Ala
            420                 425                 430

Gly Val Val Leu Gly Glu Trp Ile Ala Arg Arg Leu Arg Arg Pro Pro
        435                 440                 445

Arg Phe Asn Pro Tyr Arg Ala Phe Thr Lys Ala Asn Glu Phe Ser Phe
    450                 455                 460

Gln Glu Glu Ala Glu Gln Asn Gln Arg Arg Gln Arg Lys Arg Pro Lys
465                 470                 475                 480

Thr Asn Gln Arg Phe Gly Asn Lys Arg
                485

<210> 559
<211> 492
<212> DNA
<213> Corynebacterium glutamicum

<220>
<221> CDS
<222> (101)..(469)
<223> RXN03095

<400> 559
aacgcctcaa ttagtgccag accttgccga ccgcagacca aacttcacca tttcaaacca 60

tccctagcca caacaacggc agttgtgcaa tgatctgcgt atg aat gca gat aag   115
                                           Met Asn Ala Asp Lys
                                           1               5

aaa atg tgc gga atg aac ccg gat agc caa tac gtc gaa ctt gcc gtc   163
Lys Met Cys Gly Met Asn Pro Asp Ser Gln Tyr Val Glu Leu Ala Val
                10                  15                  20

gaa gtt ttc gga ctc ctc gcg gac gcc act cga gtt cgc atc atc ttg     211
Glu Val Phe Gly Leu Leu Ala Asp Ala Thr Arg Val Arg Ile Ile Leu
            25                  30                  35

gca ctt cga aac agt ggt gaa ctt tcc gta aac cac ctc gcg gac atc     259
Ala Leu Arg Asn Ser Gly Glu Leu Ser Val Asn His Leu Ala Asp Ile
        40                  45                  50

gtc gat aaa tcc ccc gca gca gtt tcc caa cac ctc gcc cgg ctg cgc     307
Val Asp Lys Ser Pro Ala Ala Val Ser Gln His Leu Ala Arg Leu Arg
    55                  60                  65

atg gcc cga atc gtg tcc acc cgt caa gaa ggt caa cga gtt ttc tac     355
Met Ala Arg Ile Val Ser Thr Arg Gln Glu Gly Gln Arg Val Phe Tyr
70                  75                  80                  85

aaa ctc acc aat gaa cac gca tca cag cta gtc tcc gac gct att ttt     403
Lys Leu Thr Asn Glu His Ala Ser Gln Leu Val Ser Asp Ala Ile Phe
                90                  95                  100

cag gcg gaa cac acc att gcg gac ggc cag act ccc cca cac cac cac     451
Gln Ala Glu His Thr Ile Ala Asp Gly Gln Thr Pro Pro His His His
            105                 110                 115

cga gaa cga gaa caa tca tgaccaccca cagtcaccaa gaa                   492
Arg Glu Arg Glu Gln Ser
        120

<210> 560
<211> 123
<212> PRT
<213> Corynebacterium glutamicum

<400> 560
Met Asn Ala Asp Lys Lys Met Cys Gly Met Asn Pro Asp Ser Gln Tyr
1               5                   10                  15

Val Glu Leu Ala Val Glu Val Phe Gly Leu Leu Ala Asp Ala Thr Arg
            20                  25                  30

Val Arg Ile Ile Leu Ala Leu Arg Asn Ser Gly Glu Leu Ser Val Asn
        35                  40                  45

His Leu Ala Asp Ile Val Asp Lys Ser Pro Ala Ala Val Ser Gln His
    50                  55                  60

Leu Ala Arg Leu Arg Met Ala Arg Ile Val Ser Thr Arg Gln Glu Gly
65                  70                  75                  80

Gln Arg Val Phe Tyr Lys Leu Thr Asn Glu His Ala Ser Gln Leu Val
                85                  90                  95

Ser Asp Ala Ile Phe Gln Ala Glu His Thr Ile Ala Asp Gly Gln Thr
            100                 105                 110

Pro Pro His His His Arg Glu Arg Glu Gln Ser
        115                 120

<210> 561

<211> 591
<212> DNA
<213> Corynebacterium glutamicum

<220>
<221> CDS
<222> (101)..(568)
<223> RXN03160

<400> 561
gagcatatgg tgcgcgcggc cgatatgctg atcaattcca accccgatcc gcacgcttaa 60

cttctgccaa aaagtcgttt tgaccataag ctaagcgatt gtg aat cga att gca 115
Val Asn Arg Ile Ala
1               5

gaa atc gca cgc agt ttc ggc gtg ctg ggc ttc agc gct ttc ggc ggc 163
Glu Ile Ala Arg Ser Phe Gly Val Leu Gly Phe Ser Ala Phe Gly Gly
                10                  15                  20

ccc acc gcg cac ctc gga tat ttc cgc acg gaa ttc gtg gag cgg cgg 211
Pro Thr Ala His Leu Gly Tyr Phe Arg Thr Glu Phe Val Glu Arg Arg
            25                  30                  35

cgc tgg ctg gat gat cgc caa tat tcc gag atc gta gcg ctc agc caa 259
Arg Trp Leu Asp Asp Arg Gln Tyr Ser Glu Ile Val Ala Leu Ser Gln
        40                  45                  50

cta ctt ccc gga cct gga tcg tcg cag gtc ggt atg atg ctg ggc tac 307
Leu Leu Pro Gly Pro Gly Ser Ser Gln Val Gly Met Met Leu Gly Tyr
    55                  60                  65

cac cgc gcc ggt ttt tcc ggc atg gcg atc gcc tgg ctc atg ttt acc 355
His Arg Ala Gly Phe Ser Gly Met Ala Ile Ala Trp Leu Met Phe Thr
70                  75                  80                  85

tgg ccc tca ttg gcg ctc atg gcg gcg ttc gcc ctg ctt ttt gat gcg 403
Trp Pro Ser Leu Ala Leu Met Ala Ala Phe Ala Leu Leu Phe Asp Ala
                90                  95                  100

acc tcc gcc agc tgg acg ctc ggc ctg ctc gcc gca gcg gtc gcc gtc 451
Thr Ser Ala Ser Trp Thr Leu Gly Leu Leu Ala Ala Ala Val Ala Val
            105                 110                 115

gtt ttc aaa cgc agt cac cgg gca tgg cgc ggt tca atg gct tcc acg 499
Val Phe Lys Arg Ser His Arg Ala Trp Arg Gly Ser Met Ala Ser Thr
        120                 125                 130

ccg ggg cgc cgg cca cca tcg ggg gtc ggc ctc ggg gca tcg cgg gtg 547
Pro Gly Arg Arg Pro Pro Ser Gly Val Gly Leu Gly Ala Ser Arg Val
    135                 140                 145

ctc ggc cct ccc caa cgg ggt taacacacct tggggttcaa tca 591
Leu Gly Pro Pro Gln Arg Gly
150                 155


<210> 562
<211> 156
<212> PRT
<213> Corynebacterium glutamicum

<400> 562
Val Asn Arg Ile Ala Glu Ile Ala Arg Ser Phe Gly Val Leu Gly Phe
1               5                   10                  15

Ser Ala Phe Gly Gly Pro Thr Ala His Leu Gly Tyr Phe Arg Thr Glu
            20                  25                  30

Phe Val Glu Arg Arg Arg Trp Leu Asp Asp Arg Gln Tyr Ser Glu Ile
        35                  40                  45

Val Ala Leu Ser Gln Leu Leu Pro Gly Pro Gly Ser Ser Gln Val Gly
    50                  55                  60

Met Met Leu Gly Tyr His Arg Ala Gly Phe Ser Gly Met Ala Ile Ala
65                  70                  75                  80

Trp Leu Met Phe Thr Trp Pro Ser Leu Ala Leu Met Ala Ala Phe Ala
                85                  90                  95

Leu Leu Phe Asp Ala Thr Ser Ala Ser Trp Thr Leu Gly Leu Leu Ala
            100                 105                 110

Ala Ala Val Ala Val Val Phe Lys Arg Ser His Arg Ala Trp Arg Gly
        115                 120                 125

Ser Met Ala Ser Thr Pro Gly Arg Arg Pro Pro Ser Gly Val Gly Leu
    130                 135                 140

Gly Ala Ser Arg Val Leu Gly Pro Pro Gln Arg Gly
145                 150                 155


<210> 563
<211> 645
<212> DNA
<213> Corynebacterium glutamicum

<220>
<221> CDS
<222> (101)..(622)
<223> RXN02955

<400> 563
atgcactggc tcccatcaaa gacgaattct tgacctcaga attccagcgt gaactctacg 60

aagcagtgcg cgccgctgat acttcaggag gtgcggcatc atg atg aat ttt aag   115
                                            Met Met Asn Phe Lys
                                            1               5

tcc atc gtg tgc gtc act gcc tgg cag gtg ttt agc cgc cag gtg ctg   163
Ser Ile Val Cys Val Thr Ala Trp Gln Val Phe Ser Arg Gln Val Leu
                10                  15                  20

cac agc cca tca acg tgg tct gaa gaa cta tcc aag ctg ttg ttc gtg   211
His Ser Pro Ser Thr Trp Ser Glu Glu Leu Ser Lys Leu Leu Phe Val
            25                  30                  35

tgg cta tct ttc gca ggt agt gcg ttc ctc ttt gga gag cgt gga cat   259
Trp Leu Ser Phe Ala Gly Ser Ala Phe Leu Phe Gly Glu Arg Gly His
        40                  45                  50

att gcg gtt gat ttc atc gcg cgc aaa ctg cct gtt tct gcg cag cgg     307
Ile Ala Val Asp Phe Ile Ala Arg Lys Leu Pro Val Ser Ala Gln Arg
     55                  60                  65

gtc ctg cag gtc att gtt cag ttg ttg att gtt gtt ttt gcg atc ctc     355
Val Leu Gln Val Ile Val Gln Leu Leu Ile Val Val Phe Ala Ile Leu
 70                  75                  80                  85

ggc atg atc tgg ggt ggc tac ttg gct gca tca atc gcg tgg aat cag     403
Gly Met Ile Trp Gly Gly Tyr Leu Ala Ala Ser Ile Ala Trp Asn Gln
                 90                  95                 100

cag ctc act gcg ctg cca ctt acc ttg gga tgg gtg tat gtt gtc atc     451
Gln Leu Thr Ala Leu Pro Leu Thr Leu Gly Trp Val Tyr Val Val Ile
            105                 110                 115

ccg atc gcg ggt gtg ttc atc gcg ttg ttc gcg atc atc gat ctc atc     499
Pro Ile Ala Gly Val Phe Ile Ala Leu Phe Ala Ile Ile Asp Leu Ile
        120                 125                 130

gaa gtg gcc aca ggc aag gaa gag cct tac ccc ctt gtt gat gaa tca     547
Glu Val Ala Thr Gly Lys Glu Glu Pro Tyr Pro Leu Val Asp Glu Ser
    135                 140                 145

gaa gaa cct cga gat ttg gac gag cta gag gcc caa agc gct ata gat     595
Glu Glu Pro Arg Asp Leu Asp Glu Leu Glu Ala Gln Ser Ala Ile Asp
150                 155                 160                 165

tct gca agt tca gcg gaa ggt agg aac taatatgttg tcgccagcag           642
Ser Ala Ser Ser Ala Glu Gly Arg Asn
                170

ctg                                                                 645


<210> 564
<211> 174
<212> PRT
<213> Corynebacterium glutamicum

<400> 564
Met Met Asn Phe Lys Ser Ile Val Cys Val Thr Ala Trp Gln Val Phe
  1               5                  10                  15

Ser Arg Gln Val Leu His Ser Pro Ser Thr Trp Ser Glu Glu Leu Ser
             20                  25                  30

Lys Leu Leu Phe Val Trp Leu Ser Phe Ala Gly Ser Ala Phe Leu Phe
         35                  40                  45

Gly Glu Arg Gly His Ile Ala Val Asp Phe Ile Ala Arg Lys Leu Pro
     50                  55                  60

Val Ser Ala Gln Arg Val Leu Gln Val Ile Val Gln Leu Leu Ile Val
 65                  70                  75                  80

Val Phe Ala Ile Leu Gly Met Ile Trp Gly Gly Tyr Leu Ala Ala Ser
                 85                  90                  95

Ile Ala Trp Asn Gln Gln Leu Thr Ala Leu Pro Leu Thr Leu Gly Trp

                100                 105                 110

Val Tyr Val Val Ile Pro Ile Ala Gly Val Phe Ile Ala Leu Phe Ala
        115                 120                 125

Ile Ile Asp Leu Ile Glu Val Ala Thr Gly Lys Glu Glu Pro Tyr Pro
    130                 135                 140

Leu Val Asp Glu Ser Glu Glu Pro Arg Asp Leu Asp Glu Leu Glu Ala
145                 150                 155                 160

Gln Ser Ala Ile Asp Ser Ala Ser Ser Ala Glu Gly Arg Asn
                165                 170

<210> 565
<211> 754
<212> DNA
<213> Corynebacterium glutamicum

<220>
<221> CDS
<222> (101)..(754)
<223> RXN03109

<400> 565
actggcgctt cccgatggtc aatcattggc ctttggtgcc caaactggcg agttgttgct 60

ccgcgcatcc cgcgaactgt atgtgcaggg cggcgagtag atg gtt gtg aag gag   115
                                           Met Val Val Lys Glu
                                           1               5

gtt gac gtc gaa aag caa aaa gct ggc cgg gtg cct ggt gct ata gct   163
Val Asp Val Glu Lys Gln Lys Ala Gly Arg Val Pro Gly Ala Ile Ala
                10                  15                  20

aag cgt cgg acc gtg cgg att gtg ctg ttt gtc gcg ctg ggc gcg atc   211
Lys Arg Arg Thr Val Arg Ile Val Leu Phe Val Ala Leu Gly Ala Ile
            25                  30                  35

gtg att gcg gcg tcg ctg tgg tcg att ctg gtc ggc caa tac acc atc   259
Val Ile Ala Ala Ser Leu Trp Ser Ile Leu Val Gly Gln Tyr Thr Ile
        40                  45                  50

ccg att cgc gat cta cct gcg att tta gcc tcc ggt ccg acc ggt gcg   307
Pro Ile Arg Asp Leu Pro Ala Ile Leu Ala Ser Gly Pro Thr Gly Ala
    55                  60                  65

cag acg atg gcg gaa caa gtc gtg tgg caa atc cgc atg ccg cgc atc   355
Gln Thr Met Ala Glu Gln Val Val Trp Gln Ile Arg Met Pro Arg Ile
70                  75                  80                  85

gtg ctg gga ctg ctc gtg ggt gcc gct ttg ggc gtg gcc ggc gcg ctg   403
Val Leu Gly Leu Leu Val Gly Ala Ala Leu Gly Val Ala Gly Ala Leu
                90                  95                  100

ttg cag gcg gtg ttt tcc aac ccg ctg gcg gaa ccg tcg atc atc ggc   451
Leu Gln Ala Val Phe Ser Asn Pro Leu Ala Glu Pro Ser Ile Ile Gly
            105                 110                 115

gtg acc tcc ggc gcg ggc gtg ggt gct gct gcg gtg atc gtg ttt aac   499

Val Thr Ser Gly Ala Gly Val Gly Ala Ala Ala Val Ile Val Phe Asn
        120                 125                 130

ctg aca ttt ttg ggc aca tcc acc gtc gca gtc ggc gcg ttt att acc     547
Leu Thr Phe Leu Gly Thr Ser Thr Val Ala Val Gly Ala Phe Ile Thr
    135                 140                 145

gcg gtg atc acc acg att ttg gta tat cag ctg gcc aga agc cgt gga     595
Ala Val Ile Thr Thr Ile Leu Val Tyr Gln Leu Ala Arg Ser Arg Gly
150                 155                 160                 165

cgt gtg caa gtg atc aac ctg atc ctg aca ggc ata gcc att aat gca     643
Arg Val Gln Val Ile Asn Leu Ile Leu Thr Gly Ile Ala Ile Asn Ala
                170                 175                 180

gta tcc ggc gcg ctg acc tca gtt ctt gat cta cat cgc gcc gac gag     691
Val Ser Gly Ala Leu Thr Ser Val Leu Asp Leu His Arg Ala Asp Glu
            185                 190                 195

ctc ccg cga aga aat att ttc tgc aga tgg gtt cct cac ggc tcg cat     739
Leu Pro Arg Arg Asn Ile Phe Cys Arg Trp Val Pro His Gly Ser His
        200                 205                 210

ggc gca cgt caa cgt                                                 754
Gly Ala Arg Gln Arg
    215


<210> 566
<211> 218
<212> PRT
<213> Corynebacterium glutamicum

<400> 566
Met Val Val Lys Glu Val Asp Val Glu Lys Gln Lys Ala Gly Arg Val
 1               5                  10                  15

Pro Gly Ala Ile Ala Lys Arg Arg Thr Val Arg Ile Val Leu Phe Val
            20                  25                  30

Ala Leu Gly Ala Ile Val Ile Ala Ala Ser Leu Trp Ser Ile Leu Val
        35                  40                  45

Gly Gln Tyr Thr Ile Pro Ile Arg Asp Leu Pro Ala Ile Leu Ala Ser
    50                  55                  60

Gly Pro Thr Gly Ala Gln Thr Met Ala Glu Gln Val Val Trp Gln Ile
65                  70                  75                  80

Arg Met Pro Arg Ile Val Leu Gly Leu Leu Val Gly Ala Ala Leu Gly
                85                  90                  95

Val Ala Gly Ala Leu Leu Gln Ala Val Phe Ser Asn Pro Leu Ala Glu
            100                 105                 110

Pro Ser Ile Ile Gly Val Thr Ser Gly Ala Gly Val Gly Ala Ala Ala
        115                 120                 125

Val Ile Val Phe Asn Leu Thr Phe Leu Gly Thr Ser Thr Val Ala Val
    130                 135                 140

Gly Ala Phe Ile Thr Ala Val Ile Thr Thr Ile Leu Val Tyr Gln Leu
145                 150                 155                 160

Ala Arg Ser Arg Gly Arg Val Gln Val Ile Asn Leu Ile Leu Thr Gly
                165                 170                 175

Ile Ala Ile Asn Ala Val Ser Gly Ala Leu Thr Ser Val Leu Asp Leu
            180                 185                 190

His Arg Ala Asp Glu Leu Pro Arg Arg Asn Ile Phe Cys Arg Trp Val
        195                 200                 205

Pro His Gly Ser His Gly Ala Arg Gln Arg
    210                 215


<210> 567
<211> 357
<212> DNA
<213> Corynebacterium glutamicum

<220>
<221> CDS
<222> (101)..(334)
<223> RXN02979

<400> 567
ctaggtcaag gaacttcact cggctagtcc ttagactcaa atgtgttcag acaaacactg 60

gcaccgtaag gcacgaaagt taccgaaagg actggttccc atg acc gcc cca aac   115
                                            Met Thr Ala Pro Asn
                                            1               5

act ctc aag caa aca act ctt cgc tct gat gag ttc tct tgc cca tcc   163
Thr Leu Lys Gln Thr Thr Leu Arg Ser Asp Glu Phe Ser Cys Pro Ser
                10                  15                  20

tgt gtc tcc aag att gaa aac aaa ttg aat gga ttg gat ggc gtc gac   211
Cys Val Ser Lys Ile Glu Asn Lys Leu Asn Gly Leu Asp Gly Val Asp
            25                  30                  35

aat gca gag gtg aag ttc tcc tcc gga aga atc ctt gtt gat cac gac   259
Asn Ala Glu Val Lys Phe Ser Ser Gly Arg Ile Leu Val Asp His Asp
        40                  45                  50

ccc agc aag gtc tct atc aag gat cta gtc gct gca gtc gca gag gtt   307
Pro Ser Lys Val Ser Ile Lys Asp Leu Val Ala Ala Val Ala Glu Val
    55                  60                  65

ggc tac acc gca aag cca tca gca atc taaaactctc agttagacca         354
Gly Tyr Thr Ala Lys Pro Ser Ala Ile
70                  75

tta                                                               357


<210> 568
<211> 78
<212> PRT
<213> Corynebacterium glutamicum

<400> 568
Met Thr Ala Pro Asn Thr Leu Lys Gln Thr Thr Leu Arg Ser Asp Glu
1               5                   10                  15

Phe Ser Cys Pro Ser Cys Val Ser Lys Ile Glu Asn Lys Leu Asn Gly
            20                  25                  30

Leu Asp Gly Val Asp Asn Ala Glu Val Lys Phe Ser Ser Gly Arg Ile
        35                  40                  45

Leu Val Asp His Asp Pro Ser Lys Val Ser Ile Lys Asp Leu Val Ala
    50                  55                  60

Ala Val Ala Glu Val Gly Tyr Thr Ala Lys Pro Ser Ala Ile
65                  70                  75


<210> 569
<211> 357
<212> DNA
<213> Corynebacterium glutamicum

<220>
<221> CDS
<222> (101)..(334)
<223> RXN02987

<400> 569
gttgtttgat ccaggtcaag gaattaaccc ggaaaggacc gtatctttaa aggtgcaagc 60

acaggaacat gacgataaaa gatgaaagga cctggttacg atg acc gcc ccc gcc    115
                                            Met Thr Ala Pro Ala
                                            1               5

acg ctg aag aac acc acc ttg cgc tct gat gag ttc acc tgt ccg agc    163
Thr Leu Lys Asn Thr Thr Leu Arg Ser Asp Glu Phe Thr Cys Pro Ser
                10                  15                  20

tgt gtc gcc aag atc gaa aac aag ctg aat ggt ttg gac ggc gtg gag    211
Cys Val Ala Lys Ile Glu Asn Lys Leu Asn Gly Leu Asp Gly Val Glu
            25                  30                  35

aat gcg gag gtg aag ttc tcc tcc gga cgc atc ctg atc acc cac gac    259
Asn Ala Glu Val Lys Phe Ser Ser Gly Arg Ile Leu Ile Thr His Asp
        40                  45                  50

cca cag aag gtc tcc gta cgt gac ctg gtc acc gcg gta gcc gag gtc    307
Pro Gln Lys Val Ser Val Arg Asp Leu Val Thr Ala Val Ala Glu Val
    55                  60                  65

ggt tac acc gcc aag ccg tcg gcg atc tgacgcactc ccgaccccac          354
Gly Tyr Thr Ala Lys Pro Ser Ala Ile
70                  75

aag                                                                357


<210> 570
<211> 78
<212> PRT
<213> Corynebacterium glutamicum

<400> 570
Met Thr Ala Pro Ala Thr Leu Lys Asn Thr Thr Leu Arg Ser Asp Glu
1               5                   10                  15

Phe Thr Cys Pro Ser Cys Val Ala Lys Ile Glu Asn Lys Leu Asn Gly
            20                  25                  30

Leu Asp Gly Val Glu Asn Ala Glu Val Lys Phe Ser Ser Gly Arg Ile
        35                  40                  45

Leu Ile Thr His Asp Pro Gln Lys Val Ser Val Arg Asp Leu Val Thr
    50                  55                  60

Ala Val Ala Glu Val Gly Tyr Thr Ala Lys Pro Ser Ala Ile
65                  70                  75


<210> 571
<211> 1041
<212> DNA
<213> Corynebacterium glutamicum

<220>
<221> CDS
<222> (101)..(1018)
<223> RXN03084

<400> 571
ccacccccac gtctaagttt tcccctattt acacacacct gaccgaagct gtaaggtttg 60

cctaatcttt ttcaatctaa agtcaggata ttcacagccc atg tca tct cgc aga    115
                                           Met Ser Ser Arg Arg
                                           1               5

aaa ctc tcc tcc gca ctg atc gtg ctt ctc gca gca gca cta cct ctt    163
Lys Leu Ser Ser Ala Leu Ile Val Leu Leu Ala Ala Ala Leu Pro Leu
                10                  15                  20

act gct tgt tcc tcc agc tca gaa gag gaa gca tcc acc agc tct gcg    211
Thr Ala Cys Ser Ser Ser Ser Glu Glu Glu Ala Ser Thr Ser Ser Ala
            25                  30                  35

act cgc gaa ttc aca gac gct cac gga aca acc gaa gtt ccc gaa aat    259
Thr Arg Glu Phe Thr Asp Ala His Gly Thr Thr Glu Val Pro Glu Asn
        40                  45                  50

cct cag cgc gtc gtt gtc ctc gag cca ctt gag cta gac acc gca atc    307
Pro Gln Arg Val Val Val Leu Glu Pro Leu Glu Leu Asp Thr Ala Ile
    55                  60                  65

gcc ctc gga atc acc cca gtg ggt gca gct gtc gcc aac aac gtc act    355
Ala Leu Gly Ile Thr Pro Val Gly Ala Ala Val Ala Asn Asn Val Thr
70                  75                  80                  85

ggt att cct gca tat ctc ggc gtc gat gga atc gag cct gtc ggc acc    403
Gly Ile Pro Ala Tyr Leu Gly Val Asp Gly Ile Glu Pro Val Gly Thr
                90                  95                  100

gtt tct gag cca aat atc gaa gcg atc gct gct ctc gag ccc gac ctg    451
Val Ser Glu Pro Asn Ile Glu Ala Ile Ala Ala Leu Glu Pro Asp Leu

            105                 110                 115

atc ctg ggc acc gat tcc cgc cac gcc gaa atc tac gac cgc ctc gaa 499
Ile Leu Gly Thr Asp Ser Arg His Ala Glu Ile Tyr Asp Arg Leu Glu
        120                 125                 130

tcc atc gcc cca acc gtg ttc atg aca acc cat gtt gat ccg tgg aaa 547
Ser Ile Ala Pro Thr Val Phe Met Thr Thr His Val Asp Pro Trp Lys
    135                 140                 145

gac aat gtc gtc ttc atc ggc gat gca ttg ggc aag aag cag gaa tcc 595
Asp Asn Val Val Phe Ile Gly Asp Ala Leu Gly Lys Lys Gln Glu Ser
150                 155                 160                 165

gag gat ctc atc caa ggc ttt aat gac aag tgc gaa gag atc aag tcc 643
Glu Asp Leu Ile Gln Gly Phe Asn Asp Lys Cys Glu Glu Ile Lys Ser
                170                 175                 180

gag cat gat gtc gaa ggt aag acc gtc aac atg att cgt ccc cgc gac 691
Glu His Asp Val Glu Gly Lys Thr Val Asn Met Ile Arg Pro Arg Asp
            185                 190                 195

gag caa acc atg agc cta tac ggc ccg acc tca ttt gcc ggc agc tct 739
Glu Gln Thr Met Ser Leu Tyr Gly Pro Thr Ser Phe Ala Gly Ser Ser
        200                 205                 210

ttg gag tgc gca gga ctc acc att cct gat cag gaa tgg aag gat gac 787
Leu Glu Cys Ala Gly Leu Thr Ile Pro Asp Gln Glu Trp Lys Asp Asp
    215                 220                 225

ctc cag gcc gac atc gct cct gag aac ttc atg ctc gcc acc gcc gac 835
Leu Gln Ala Asp Ile Ala Pro Glu Asn Phe Met Leu Ala Thr Ala Asp
230                 235                 240                 245

tac gtc ttt gtc acc gca act gat gtc acc gat gaa aat gag ctc ccc 883
Tyr Val Phe Val Thr Ala Thr Asp Val Thr Asp Glu Asn Glu Leu Pro
                250                 255                 260

gaa gta atc cga gaa aac cgc gaa cag ttc cca tca ctc acc ctt gtc 931
Glu Val Ile Arg Glu Asn Arg Glu Gln Phe Pro Ser Leu Thr Leu Val
            265                 270                 275

gat acc agc tac tgg gta tct ggc gtc ggt cca ctt ggc ggc agc aaa 979
Asp Thr Ser Tyr Trp Val Ser Gly Val Gly Pro Leu Gly Gly Ser Lys
        280                 285                 290

gtc ttg gaa gac atc gat gcc ttc ctc gac gca cag caa taatgtccac 1028
Val Leu Glu Asp Ile Asp Ala Phe Leu Asp Ala Gln Gln
    295                 300                 305

agctctcccc gat 1041

<210> 572
<211> 306
<212> PRT
<213> Corynebacterium glutamicum

<400> 572
Met Ser Ser Arg Arg Lys Leu Ser Ser Ala Leu Ile Val Leu Leu Ala
1               5                   10                  15

Ala Ala Leu Pro Leu Thr Ala Cys Ser Ser Ser Ser Glu Glu Glu Ala
            20                  25                  30

Ser Thr Ser Ser Ala Thr Arg Glu Phe Thr Asp Ala His Gly Thr Thr
        35                  40                  45

Glu Val Pro Glu Asn Pro Gln Arg Val Val Val Leu Glu Pro Leu Glu
    50                  55                  60

Leu Asp Thr Ala Ile Ala Leu Gly Ile Thr Pro Val Gly Ala Ala Val
65                  70                  75                  80

Ala Asn Asn Val Thr Gly Ile Pro Ala Tyr Leu Gly Val Asp Gly Ile
                85                  90                  95

Glu Pro Val Gly Thr Val Ser Glu Pro Asn Ile Glu Ala Ile Ala Ala
            100                 105                 110

Leu Glu Pro Asp Leu Ile Leu Gly Thr Asp Ser Arg His Ala Glu Ile
        115                 120                 125

Tyr Asp Arg Leu Glu Ser Ile Ala Pro Thr Val Phe Met Thr Thr His
    130                 135                 140

Val Asp Pro Trp Lys Asp Asn Val Val Phe Ile Gly Asp Ala Leu Gly
145                 150                 155                 160

Lys Lys Gln Glu Ser Glu Asp Leu Ile Gln Gly Phe Asn Asp Lys Cys
                165                 170                 175

Glu Glu Ile Lys Ser Glu His Asp Val Glu Gly Lys Thr Val Asn Met
            180                 185                 190

Ile Arg Pro Arg Asp Glu Gln Thr Met Ser Leu Tyr Gly Pro Thr Ser
        195                 200                 205

Phe Ala Gly Ser Ser Leu Glu Cys Ala Gly Leu Thr Ile Pro Asp Gln
    210                 215                 220

Glu Trp Lys Asp Asp Leu Gln Ala Asp Ile Ala Pro Glu Asn Phe Met
225                 230                 235                 240

Leu Ala Thr Ala Asp Tyr Val Phe Val Thr Ala Thr Asp Val Thr Asp
                245                 250                 255

Glu Asn Glu Leu Pro Glu Val Ile Arg Glu Asn Arg Glu Gln Phe Pro
            260                 265                 270

Ser Leu Thr Leu Val Asp Thr Ser Tyr Trp Val Ser Gly Val Gly Pro
        275                 280                 285

Leu Gly Gly Ser Lys Val Leu Glu Asp Ile Asp Ala Phe Leu Asp Ala
    290                 295                 300

Gln Gln
305

<210> 573
<211> 440

<212> DNA
<213> Corynebacterium glutamicum

<220>
<221> CDS
<222> (1)..(417)
<223> RXN03183

<400> 573
gaa gcc gaa gca acc gca ggc aaa ttc gaa gta cag ccc ctc gta ggt 48
Glu Ala Glu Ala Thr Ala Gly Lys Phe Glu Val Gln Pro Leu Val Gly
1               5                   10                  15

aaa gac ggc gtc ggc gta tcc acc ctt ggt ggc tac aac aac ggc atc 96
Lys Asp Gly Val Gly Val Ser Thr Leu Gly Gly Tyr Asn Asn Gly Ile
            20                  25                  30

aac gtc aac tcc gaa aac aag gca acc gcc cgc gac ttc atc gaa ttc 144
Asn Val Asn Ser Glu Asn Lys Ala Thr Ala Arg Asp Phe Ile Glu Phe
        35                  40                  45

atc atc aac gaa gag aac caa acc tgg ttc gcg gac aac tcc ttc cca 192
Ile Ile Asn Glu Glu Asn Gln Thr Trp Phe Ala Asp Asn Ser Phe Pro
    50                  55                  60

cca gtt ctg gca tcc atc tac gat gat gag tcc ctt gtt gag cag tac 240
Pro Val Leu Ala Ser Ile Tyr Asp Asp Glu Ser Leu Val Glu Gln Tyr
65                  70                  75                  80

cca tac ctg cca gca ctg aag gaa tcc ctg gaa aac gca gca cca cgc 288
Pro Tyr Leu Pro Ala Leu Lys Glu Ser Leu Glu Asn Ala Ala Pro Arg
                85                  90                  95

cca gtg tct cct ttc tac cca gcc atc tcc aag gca atc cag gac aac 336
Pro Val Ser Pro Phe Tyr Pro Ala Ile Ser Lys Ala Ile Gln Asp Asn
            100                 105                 110

gcc tac gca gcg ctt aac ggc aac gtc gac gtt gac cag gca acc acc 384
Ala Tyr Ala Ala Leu Asn Gly Asn Val Asp Val Asp Gln Ala Thr Thr
        115                 120                 125

gat atg aag gca gcg atc gaa aac gct tcc agc tagttcggta atttagttca 437
Asp Met Lys Ala Ala Ile Glu Asn Ala Ser Ser
    130                 135

ttc 440

<210> 574
<211> 139
<212> PRT
<213> Corynebacterium glutamicum

<400> 574
Glu Ala Glu Ala Thr Ala Gly Lys Phe Glu Val Gln Pro Leu Val Gly
1               5                   10                  15

Lys Asp Gly Val Gly Val Ser Thr Leu Gly Gly Tyr Asn Asn Gly Ile
            20                  25                  30

Asn Val Asn Ser Glu Asn Lys Ala Thr Ala Arg Asp Phe Ile Glu Phe

35                  40                  45

Ile Ile Asn Glu Glu Asn Gln Thr Trp Phe Ala Asp Asn Ser Phe Pro
    50                  55                  60

Pro Val Leu Ala Ser Ile Tyr Asp Asp Glu Ser Leu Val Glu Gln Tyr
65                  70                  75                  80

Pro Tyr Leu Pro Ala Leu Lys Glu Ser Leu Glu Asn Ala Ala Pro Arg
                85                  90                  95

Pro Val Ser Pro Phe Tyr Pro Ala Ile Ser Lys Ala Ile Gln Asp Asn
            100                 105                 110

Ala Tyr Ala Ala Leu Asn Gly Asn Val Asp Val Asp Gln Ala Thr Thr
        115                 120                 125

Asp Met Lys Ala Ala Ile Glu Asn Ala Ser Ser
    130                 135

<210> 575
<211> 1077
<212> DNA
<213> Corynebacterium glutamicum

<220>
<221> CDS
<222> (101)..(1054)
<223> RXN01139

<400> 575
accaccgtgg caggcttttt cagtggcaag aagattaatt ctccacccct tcattttcaa 60

taagctttca ataaggggag aagtgtgctt agctggggtc atg gaa agc cac gat    115
                                            Met Glu Ser His Asp
                                            1               5

ctt cag cag cgc agt tat gcg cac aat ccc gat ggc cac gac cac agc    163
Leu Gln Gln Arg Ser Tyr Ala His Asn Pro Asp Gly His Asp His Ser
                10                  15                  20

cat gac gga ctc gga cac tca cat gct ccc agc tcc ctc aag gct ctt    211
His Asp Gly Leu Gly His Ser His Ala Pro Ser Ser Leu Lys Ala Leu
            25                  30                  35

ttt gcg gtc atc att ttc acc tcg atc atc ttc cta gcg gaa cta atc    259
Phe Ala Val Ile Ile Phe Thr Ser Ile Ile Phe Leu Ala Glu Leu Ile
        40                  45                  50

gcc ggc ctt att tcc gga tct ttg gca ctg ctg gct gac gcc atg cac    307
Ala Gly Leu Ile Ser Gly Ser Leu Ala Leu Leu Ala Asp Ala Met His
    55                  60                  65

atg ctg tcc gac tcc act ggc ttg atc att gcg gct gtc gcc atg ctc    355
Met Leu Ser Asp Ser Thr Gly Leu Ile Ile Ala Ala Val Ala Met Leu
70                  75                  80                  85

att ggc cgt cgg gca cgc act tct cgt gcg acc tac gga tac aag cgt    403
Ile Gly Arg Arg Ala Arg Thr Ser Arg Ala Thr Tyr Gly Tyr Lys Arg
                90                  95                  100

gcg gaa gtc ttg gca gcg atg gtt aac gcc acc gtt gtt aca gca ctg     451
Ala Glu Val Leu Ala Ala Met Val Asn Ala Thr Val Val Thr Ala Leu
            105                 110                 115

tct gtg tgg atc gtc gtt gag gcc atc atg cgt ctg ggc aag gac ctg     499
Ser Val Trp Ile Val Val Glu Ala Ile Met Arg Leu Gly Lys Asp Leu
        120                 125                 130

gaa atc cag acc aac ctg atg ctc atc gtc gcg gtc att ggt ttt gtc     547
Glu Ile Gln Thr Asn Leu Met Leu Ile Val Ala Val Ile Gly Phe Val
    135                 140                 145

acc aac gga att tcc gcc ctg gtg ttg atg cgc cac caa gat ggc aat     595
Thr Asn Gly Ile Ser Ala Leu Val Leu Met Arg His Gln Asp Gly Asn
150                 155                 160                 165

atc aat atg cgt gga gca ttc ctt cac gtt ctc agt gac atg ctg ggt     643
Ile Asn Met Arg Gly Ala Phe Leu His Val Leu Ser Asp Met Leu Gly
                170                 175                 180

tcc gtt gcc gtc att att gcg ggc ctg gtg att cgc tac acg gga tgg     691
Ser Val Ala Val Ile Ile Ala Gly Leu Val Ile Arg Tyr Thr Gly Trp
            185                 190                 195

atg ccg gcc gat acc att gct tcg att gcg att gct gcg att att att     739
Met Pro Ala Asp Thr Ile Ala Ser Ile Ala Ile Ala Ala Ile Ile Ile
        200                 205                 210

cct cgc gca ttc agc ctc ctg aag gaa gct ctc aat atc ttg ttg gag     787
Pro Arg Ala Phe Ser Leu Leu Lys Glu Ala Leu Asn Ile Leu Leu Glu
    215                 220                 225

cgt gtt cct aca ggt gcg gag cct gca gag gtc gac gca gcc ctt cgt     835
Arg Val Pro Thr Gly Ala Glu Pro Ala Glu Val Asp Ala Ala Leu Arg
230                 235                 240                 245

aaa gtc cca ggt gtc agc gat gtg cat gat ctt cac att tgg agc att     883
Lys Val Pro Gly Val Ser Asp Val His Asp Leu His Ile Trp Ser Ile
                250                 255                 260

gac ggc aag gaa atc ctg gcc acg gtg cat ttg gtg gtg gat tcg tct     931
Asp Gly Lys Glu Ile Leu Ala Thr Val His Leu Val Val Asp Ser Ser
            265                 270                 275

aca aat cag ctg cat agt tgt ggc gtg ttg gat cgg gca gaa gcg gaa     979
Thr Asn Gln Leu His Ser Cys Gly Val Leu Asp Arg Ala Glu Ala Glu
        280                 285                 290

cta tcc aaa ctt ggg atc ttg cac tca aca att cag ctg gaa agc gca     1027
Leu Ser Lys Leu Gly Ile Leu His Ser Thr Ile Gln Leu Glu Ser Ala
    295                 300                 305

gat cac agt gat cat gaa agt gtg tgc tgatatagag tatgtcccat           1074
Asp His Ser Asp His Glu Ser Val Cys
310                 315

ggg                                                                 1077

<210> 576

<211> 318
<212> PRT
<213> Corynebacterium glutamicum

<400> 576
Met Glu Ser His Asp Leu Gln Gln Arg Ser Tyr Ala His Asn Pro Asp
1               5                   10                  15

Gly His Asp His Ser His Asp Gly Leu Gly His Ser His Ala Pro Ser
            20                  25                  30

Ser Leu Lys Ala Leu Phe Ala Val Ile Ile Phe Thr Ser Ile Ile Phe
        35                  40                  45

Leu Ala Glu Leu Ile Ala Gly Leu Ile Ser Gly Ser Leu Ala Leu Leu
    50                  55                  60

Ala Asp Ala Met His Met Leu Ser Asp Ser Thr Gly Leu Ile Ile Ala
65                  70                  75                  80

Ala Val Ala Met Leu Ile Gly Arg Arg Ala Arg Thr Ser Arg Ala Thr
                85                  90                  95

Tyr Gly Tyr Lys Arg Ala Glu Val Leu Ala Ala Met Val Asn Ala Thr
            100                 105                 110

Val Val Thr Ala Leu Ser Val Trp Ile Val Val Glu Ala Ile Met Arg
        115                 120                 125

Leu Gly Lys Asp Leu Glu Ile Gln Thr Asn Leu Met Leu Ile Val Ala
    130                 135                 140

Val Ile Gly Phe Val Thr Asn Gly Ile Ser Ala Leu Val Leu Met Arg
145                 150                 155                 160

His Gln Asp Gly Asn Ile Asn Met Arg Gly Ala Phe Leu His Val Leu
                165                 170                 175

Ser Asp Met Leu Gly Ser Val Ala Val Ile Ile Ala Gly Leu Val Ile
            180                 185                 190

Arg Tyr Thr Gly Trp Met Pro Ala Asp Thr Ile Ala Ser Ile Ala Ile
        195                 200                 205

Ala Ala Ile Ile Ile Pro Arg Ala Phe Ser Leu Leu Lys Glu Ala Leu
    210                 215                 220

Asn Ile Leu Leu Glu Arg Val Pro Thr Gly Ala Glu Pro Ala Glu Val
225                 230                 235                 240

Asp Ala Ala Leu Arg Lys Val Pro Gly Val Ser Asp Val His Asp Leu
                245                 250                 255

His Ile Trp Ser Ile Asp Gly Lys Glu Ile Leu Ala Thr Val His Leu
            260                 265                 270

Val Val Asp Ser Ser Thr Asn Gln Leu His Ser Cys Gly Val Leu Asp
        275                 280                 285

Arg Ala Glu Ala Glu Leu Ser Lys Leu Gly Ile Leu His Ser Thr Ile
    290                 295                 300

Gln Leu Glu Ser Ala Asp His Ser Asp His Glu Ser Val Cys
305                 310                 315

<210> 577
<211> 2733
<212> DNA
<213> Corynebacterium glutamicum

<220>
<221> CDS
<222> (101)..(2710)
<223> RXN00378

<400> 577
accgtgagcc ttatactgtg aggacattaa aagtgacacc tctttttcta tcttttacaa 60

cccaagaagg tttatcgtga gcacaccgga ttcttcctcg gtg gac aag gcc gta   115
                                          Val Asp Lys Ala Val
                                          1               5

aac act gct atc tct gac gcc aaa aca gcg gcg ctc aag gca ggt gtt   163
Asn Thr Ala Ile Ser Asp Ala Lys Thr Ala Ala Leu Lys Ala Gly Val
                10                  15                  20

gga ttg aac cga gcc acc gcc tca gaa gaa gag gaa gat tta agc tca   211
Gly Leu Asn Arg Ala Thr Ala Ser Glu Glu Glu Glu Asp Leu Ser Ser
            25                  30                  35

agc att aag gtt tct ttg gcc ttt gag ctc gag ggg tta agc aat gca   259
Ser Ile Lys Val Ser Leu Ala Phe Glu Leu Glu Gly Leu Ser Asn Ala
        40                  45                  50

cca tcg ttg atg gtg gtg gaa aaa gcc cta gag aag atc ccc ggt gta   307
Pro Ser Leu Met Val Val Glu Lys Ala Leu Glu Lys Ile Pro Gly Val
    55                  60                  65

tcc gcg gat ctg att tac cct tca caa act gca tgg att aca gca act   355
Ser Ala Asp Leu Ile Tyr Pro Ser Gln Thr Ala Trp Ile Thr Ala Thr
70                  75                  80                  85

gat cgg gta cat ccc gaa acc ctc att gag gtg ttt gag cag ttc ggc   403
Asp Arg Val His Pro Glu Thr Leu Ile Glu Val Phe Glu Gln Phe Gly
                90                  95                  100

atc aaa gca cac ctt tct aat tca tcg ctg ctg cgc agg cat caa cag   451
Ile Lys Ala His Leu Ser Asn Ser Ser Leu Leu Arg Arg His Gln Gln
            105                 110                 115

ctc agc gcg gaa gta aat agg gaa gca cgc ctt gat cgt tac cgc tcc   499
Leu Ser Ala Glu Val Asn Arg Glu Ala Arg Leu Asp Arg Tyr Arg Ser
        120                 125                 130

cga atg gat gcc aag cga atc tcg cct cgt gtg cga agg cat aac cga   547
Arg Met Asp Ala Lys Arg Ile Ser Pro Arg Val Arg Arg His Asn Arg
    135                 140                 145

caa gaa atg gta cat gcg gta cgc gct cgt gaa agt ggt tgg att aaa   595
Gln Glu Met Val His Ala Val Arg Ala Arg Glu Ser Gly Trp Ile Lys
150                 155                 160                 165

cgc agg aat cac acc acc tcg cag cat gaa gac cca atg tcg ggc gat 643
Arg Arg Asn His Thr Thr Ser Gln His Glu Asp Pro Met Ser Gly Asp
                170                 175                 180

gtg ctg ttc acc gcc cgc gca ctg att aca cct aag cgt ttg tgg gtg 691
Val Leu Phe Thr Ala Arg Ala Leu Ile Thr Pro Lys Arg Leu Trp Val
            185                 190                 195

tcg ttg ccg ttt gcg ctc atc gta ttg gcg tta tcg ttg aat cct tcg 739
Ser Leu Pro Phe Ala Leu Ile Val Leu Ala Leu Ser Leu Asn Pro Ser
        200                 205                 210

tgg cag ttt gat tat tgg cag tgg ttg tcc gct gtg ttg gct att cct 787
Trp Gln Phe Asp Tyr Trp Gln Trp Leu Ser Ala Val Leu Ala Ile Pro
    215                 220                 225

gtg gtg gtg tgg ggt gcc tgg ccg ttt cac cgc gct gca gca ggc ggt 835
Val Val Val Trp Gly Ala Trp Pro Phe His Arg Ala Ala Ala Gly Gly
230                 235                 240                 245

att cgt cga gga att tcc gct ctt gat gcg acc agc tca atc gct att 883
Ile Arg Arg Gly Ile Ser Ala Leu Asp Ala Thr Ser Ser Ile Ala Ile
                250                 255                 260

gct gct gca tac gcg tgg tct atc gcc atg ctg ttg ttt gaa acc cca 931
Ala Ala Ala Tyr Ala Trp Ser Ile Ala Met Leu Leu Phe Glu Thr Pro
            265                 270                 275

gga ggt aaa tcc tgg cgg tca tat ccg tcc tgg ttc gct ttt gac cac 979
Gly Gly Lys Ser Trp Arg Ser Tyr Pro Ser Trp Phe Ala Phe Asp His
        280                 285                 290

ggc acg ttg acc caa aac gag att tat ttt gat gtg gcc tgc gga atc 1027
Gly Thr Leu Thr Gln Asn Glu Ile Tyr Phe Asp Val Ala Cys Gly Ile
    295                 300                 305

acc gtg ttg ctt ctt gcc gga cgg ctg ctg aca agg cgt cga agc caa 1075
Thr Val Leu Leu Leu Ala Gly Arg Leu Leu Thr Arg Arg Arg Ser Gln
310                 315                 320                 325

tcc agt ttg tta gcg gaa ctt ggt cgc ctc caa atc gat cca cag cgc 1123
Ser Ser Leu Leu Ala Glu Leu Gly Arg Leu Gln Ile Asp Pro Gln Arg
                330                 335                 340

att gtc act gtg gtg cgt aaa cac cga ttg aag cgc gta gtc cag gaa 1171
Ile Val Thr Val Val Arg Lys His Arg Leu Lys Arg Val Val Gln Glu
            345                 350                 355

ctg aac att cca gtg cag gaa gtc cgt gtc aat gac gat gtg aaa gtt 1219
Leu Asn Ile Pro Val Gln Glu Val Arg Val Asn Asp Asp Val Lys Val
        360                 365                 370

cca cct aat acc acg atc cct gtg gat ggc act gtc atc ggt ggc ggt 1267
Pro Pro Asn Thr Thr Ile Pro Val Asp Gly Thr Val Ile Gly Gly Gly
    375                 380                 385

tcg cgg atc gca gct agc atc atc atg gga caa gac cag cgt gat gta 1315
Ser Arg Ile Ala Ala Ser Ile Ile Met Gly Gln Asp Gln Arg Asp Val
390                 395                 400                 405

aaa gta aat gac aaa gtt ttc gcc ggc agc ctc aac ctc gaa tcc gaa 1363
Lys Val Asn Asp Lys Val Phe Ala Gly Ser Leu Asn Leu Glu Ser Glu
                410                 415                 420

atc aag gtt cgt gtt att cgc act ggt cac cgc acc cgc atc gcc gcg 1411
Ile Lys Val Arg Val Ile Arg Thr Gly His Arg Thr Arg Ile Ala Ala
            425                 430                 435

gta cat agg tgg gtt aaa gaa gcg acg ttg aag gaa aac cgc cac aat 1459
Val His Arg Trp Val Lys Glu Ala Thr Leu Lys Glu Asn Arg His Asn
        440                 445                 450

agg gca gcg atc cgt tcg gcc ggt aac ctt gtg ccc atc acg ttc acc 1507
Arg Ala Ala Ile Arg Ser Ala Gly Asn Leu Val Pro Ile Thr Phe Thr
    455                 460                 465

ctt gct gtg gtg gac ttc tgt ctg tgg gca ctg atc tct gga aac atc 1555
Leu Ala Val Val Asp Phe Cys Leu Trp Ala Leu Ile Ser Gly Asn Ile
470                 475                 480                 485

aac gct gca ttt acc act acc ttg gct gtc ctt gcg tgc gtg gct ccg 1603
Asn Ala Ala Phe Thr Thr Thr Leu Ala Val Leu Ala Cys Val Ala Pro
                490                 495                 500

gtg gcc tta gcg ttg tct gct cca ctt gcc acg agg aat tcc atc gaa 1651
Val Ala Leu Ala Leu Ser Ala Pro Leu Ala Thr Arg Asn Ser Ile Glu
            505                 510                 515

gct gca gca cga cac ggt att ttg gtc cgc tct ggt gaa att ttc cga 1699
Ala Ala Ala Arg His Gly Ile Leu Val Arg Ser Gly Glu Ile Phe Arg
        520                 525                 530

gtt ctc gat gat gtg gat act gcc gta ttt aat cgt gtg ggc aca cta 1747
Val Leu Asp Asp Val Asp Thr Ala Val Phe Asn Arg Val Gly Thr Leu
    535                 540                 545

acc gat ggc gaa atg aca gtg gaa acc gtc aca gca gac aaa ggc gag 1795
Thr Asp Gly Glu Met Thr Val Glu Thr Val Thr Ala Asp Lys Gly Glu
550                 555                 560                 565

gac cca gaa cta gtg ctg cgt gtc gcc ggg gcg ttg gcc atg gaa tcc 1843
Asp Pro Glu Leu Val Leu Arg Val Ala Gly Ala Leu Ala Met Glu Ser
                570                 575                 580

cac cac gcg att tcc aaa gca ctg gtg aaa gca tcc cgt gaa gct cgt 1891
His His Ala Ile Ser Lys Ala Leu Val Lys Ala Ser Arg Glu Ala Arg
            585                 590                 595

gat acc ggc gcc ggt ggt gaa gat gtc cca cac tgg att gaa gta ggc 1939
Asp Thr Gly Ala Gly Gly Glu Asp Val Pro His Trp Ile Glu Val Gly
        600                 605                 610

aac gtg gaa atc acc gaa gcc ggc tca ttc caa gca acc atc gag ctg 1987
Asn Val Glu Ile Thr Glu Ala Gly Ser Phe Gln Ala Thr Ile Glu Leu
    615                 620                 625

cca ctg atc aaa cca tct ggc gaa aaa atc atg cgc acc aca gaa gca 2035
Pro Leu Ile Lys Pro Ser Gly Glu Lys Ile Met Arg Thr Thr Glu Ala
630                 635                 640                 645

ctc ctg tgg cga cca cga tcc atg aca gaa gtc cgt gag cac tta agc 2083

Leu Leu Trp Arg Pro Arg Ser Met Thr Glu Val Arg Glu His Leu Ser
                650                 655                 660

ccc cga cta gtg gca gca gca acc tca ggt ggc gca cca ctg atc gtg 2131
Pro Arg Leu Val Ala Ala Ala Thr Ser Gly Gly Ala Pro Leu Ile Val
            665                 670                 675

cga tgg aaa ggc aaa gac cgc gga gtt atc act cta agt gac cac gtg 2179
Arg Trp Lys Gly Lys Asp Arg Gly Val Ile Thr Leu Ser Asp His Val
        680                 685                 690

aga tca gat tcc tcc gat gcg att att gcg att gaa gaa caa ggc atc 2227
Arg Ser Asp Ser Ser Asp Ala Ile Ile Ala Ile Glu Glu Gln Gly Ile
    695                 700                 705

gag acc atg atg ctt tca cgt gat act tac ccg gtg gca cgt cga tac 2275
Glu Thr Met Met Leu Ser Arg Asp Thr Tyr Pro Val Ala Arg Arg Tyr
710                 715                 720                 725

gca gac agc tta ggc atc acc cac gtc ttg gcc ggc atc gcg ccg ggc 2323
Ala Asp Ser Leu Gly Ile Thr His Val Leu Ala Gly Ile Ala Pro Gly
                730                 735                 740

aag aaa gcc cag gtc gtc cgt gca gtc cac acc cgc gga tcc act gtc 2371
Lys Lys Ala Gln Val Val Arg Ala Val His Thr Arg Gly Ser Thr Val
            745                 750                 755

gcg atg atc ggc gat gaa tca gta atg gac tgt ttg aaa gtc gct gac 2419
Ala Met Ile Gly Asp Glu Ser Val Met Asp Cys Leu Lys Val Ala Asp
        760                 765                 770

gtg ggt gta ctg atg ggc gtc gat cgt ccc tca gat ctg cgt gat gat 2467
Val Gly Val Leu Met Gly Val Asp Arg Pro Ser Asp Leu Arg Asp Asp
    775                 780                 785

tcc gat gac ccg gca gct gac gtt gtg gtc atg cgc gaa gag gtc atg 2515
Ser Asp Asp Pro Ala Ala Asp Val Val Val Met Arg Glu Glu Val Met
790                 795                 800                 805

agc gtg ccg acg ctg ttt aaa ctg gct cga cgc tac gcc aag ttg gtc 2563
Ser Val Pro Thr Leu Phe Lys Leu Ala Arg Arg Tyr Ala Lys Leu Val
                810                 815                 820

aat ggc aat att gct ctg gcc tgg atc tat aac ggt gtt gcc atg gtg 2611
Asn Gly Asn Ile Ala Leu Ala Trp Ile Tyr Asn Gly Val Ala Met Val
            825                 830                 835

ctt gca gtg tct ggc ttg ctg cat cca atg gct gcg acc gtg gct atg 2659
Leu Ala Val Ser Gly Leu Leu His Pro Met Ala Ala Thr Val Ala Met
        840                 845                 850

ctg gcg tct tcg ctg ctt att gaa tgg cgc tcg ggc agg gcg cgc aag 2707
Leu Ala Ser Ser Leu Leu Ile Glu Trp Arg Ser Gly Arg Ala Arg Lys
    855                 860                 865

tac taaccagcaa ttcccaagcc caa 2733
Tyr
870

<210> 578

<211> 870
<212> PRT
<213> Corynebacterium glutamicum

<400> 578
Val Asp Lys Ala Val Asn Thr Ala Ile Ser Asp Ala Lys Thr Ala Ala
1               5                   10                  15

Leu Lys Ala Gly Val Gly Leu Asn Arg Ala Thr Ala Ser Glu Glu Glu
            20                  25                  30

Glu Asp Leu Ser Ser Ser Ile Lys Val Ser Leu Ala Phe Glu Leu Glu
        35                  40                  45

Gly Leu Ser Asn Ala Pro Ser Leu Met Val Val Glu Lys Ala Leu Glu
    50                  55                  60

Lys Ile Pro Gly Val Ser Ala Asp Leu Ile Tyr Pro Ser Gln Thr Ala
65                  70                  75                  80

Trp Ile Thr Ala Thr Asp Arg Val His Pro Glu Thr Leu Ile Glu Val
                85                  90                  95

Phe Glu Gln Phe Gly Ile Lys Ala His Leu Ser Asn Ser Ser Leu Leu
            100                 105                 110

Arg Arg His Gln Gln Leu Ser Ala Glu Val Asn Arg Glu Ala Arg Leu
        115                 120                 125

Asp Arg Tyr Arg Ser Arg Met Asp Ala Lys Arg Ile Ser Pro Arg Val
    130                 135                 140

Arg Arg His Asn Arg Gln Glu Met Val His Ala Val Arg Ala Arg Glu
145                 150                 155                 160

Ser Gly Trp Ile Lys Arg Arg Asn His Thr Thr Ser Gln His Glu Asp
                165                 170                 175

Pro Met Ser Gly Asp Val Leu Phe Thr Ala Arg Ala Leu Ile Thr Pro
            180                 185                 190

Lys Arg Leu Trp Val Ser Leu Pro Phe Ala Leu Ile Val Leu Ala Leu
        195                 200                 205

Ser Leu Asn Pro Ser Trp Gln Phe Asp Tyr Trp Gln Trp Leu Ser Ala
    210                 215                 220

Val Leu Ala Ile Pro Val Val Val Trp Gly Ala Trp Pro Phe His Arg
225                 230                 235                 240

Ala Ala Ala Gly Gly Ile Arg Arg Gly Ile Ser Ala Leu Asp Ala Thr
                245                 250                 255

Ser Ser Ile Ala Ile Ala Ala Ala Tyr Ala Trp Ser Ile Ala Met Leu
            260                 265                 270

Leu Phe Glu Thr Pro Gly Gly Lys Ser Trp Arg Ser Tyr Pro Ser Trp
        275                 280                 285

Phe Ala Phe Asp His Gly Thr Leu Thr Gln Asn Glu Ile Tyr Phe Asp
    290                 295                 300

Val Ala Cys Gly Ile Thr Val Leu Leu Leu Ala Gly Arg Leu Leu Thr
305                 310                 315                 320

Arg Arg Arg Ser Gln Ser Ser Leu Leu Ala Glu Leu Gly Arg Leu Gln
                325                 330                 335

Ile Asp Pro Gln Arg Ile Val Thr Val Val Arg Lys His Arg Leu Lys
            340                 345                 350

Arg Val Val Gln Glu Leu Asn Ile Pro Val Gln Glu Val Arg Val Asn
        355                 360                 365

Asp Asp Val Lys Val Pro Pro Asn Thr Thr Ile Pro Val Asp Gly Thr
    370                 375                 380

Val Ile Gly Gly Gly Ser Arg Ile Ala Ala Ser Ile Ile Met Gly Gln
385                 390                 395                 400

Asp Gln Arg Asp Val Lys Val Asn Asp Lys Val Phe Ala Gly Ser Leu
                405                 410                 415

Asn Leu Glu Ser Glu Ile Lys Val Arg Val Ile Arg Thr Gly His Arg
            420                 425                 430

Thr Arg Ile Ala Ala Val His Arg Trp Val Lys Glu Ala Thr Leu Lys
        435                 440                 445

Glu Asn Arg His Asn Arg Ala Ala Ile Arg Ser Ala Gly Asn Leu Val
    450                 455                 460

Pro Ile Thr Phe Thr Leu Ala Val Val Asp Phe Cys Leu Trp Ala Leu
465                 470                 475                 480

Ile Ser Gly Asn Ile Asn Ala Ala Phe Thr Thr Thr Leu Ala Val Leu
                485                 490                 495

Ala Cys Val Ala Pro Val Ala Leu Ala Leu Ser Ala Pro Leu Ala Thr
            500                 505                 510

Arg Asn Ser Ile Glu Ala Ala Ala Arg His Gly Ile Leu Val Arg Ser
        515                 520                 525

Gly Glu Ile Phe Arg Val Leu Asp Asp Val Asp Thr Ala Val Phe Asn
    530                 535                 540

Arg Val Gly Thr Leu Thr Asp Gly Glu Met Thr Val Glu Thr Val Thr
545                 550                 555                 560

Ala Asp Lys Gly Glu Asp Pro Glu Leu Val Leu Arg Val Ala Gly Ala
                565                 570                 575

Leu Ala Met Glu Ser His His Ala Ile Ser Lys Ala Leu Val Lys Ala
            580                 585                 590

Ser Arg Glu Ala Arg Asp Thr Gly Ala Gly Gly Glu Asp Val Pro His
        595                 600                 605

Trp Ile Glu Val Gly Asn Val Glu Ile Thr Glu Ala Gly Ser Phe Gln
    610                 615                 620

Ala Thr Ile Glu Leu Pro Leu Ile Lys Pro Ser Gly Glu Lys Ile Met
625                 630                 635                 640

Arg Thr Thr Glu Ala Leu Leu Trp Arg Pro Arg Ser Met Thr Glu Val
                645                 650                 655

Arg Glu His Leu Ser Pro Arg Leu Val Ala Ala Ala Thr Ser Gly Gly
            660                 665                 670

Ala Pro Leu Ile Val Arg Trp Lys Gly Lys Asp Arg Gly Val Ile Thr
        675                 680                 685

Leu Ser Asp His Val Arg Ser Asp Ser Ser Asp Ala Ile Ile Ala Ile
    690                 695                 700

Glu Glu Gln Gly Ile Glu Thr Met Met Leu Ser Arg Asp Thr Tyr Pro
705                 710                 715                 720

Val Ala Arg Arg Tyr Ala Asp Ser Leu Gly Ile Thr His Val Leu Ala
                725                 730                 735

Gly Ile Ala Pro Gly Lys Lys Ala Gln Val Val Arg Ala Val His Thr
            740                 745                 750

Arg Gly Ser Thr Val Ala Met Ile Gly Asp Glu Ser Val Met Asp Cys
        755                 760                 765

Leu Lys Val Ala Asp Val Gly Val Leu Met Gly Val Asp Arg Pro Ser
    770                 775                 780

Asp Leu Arg Asp Asp Ser Asp Asp Pro Ala Ala Asp Val Val Val Met
785                 790                 795                 800

Arg Glu Glu Val Met Ser Val Pro Thr Leu Phe Lys Leu Ala Arg Arg
                805                 810                 815

Tyr Ala Lys Leu Val Asn Gly Asn Ile Ala Leu Ala Trp Ile Tyr Asn
            820                 825                 830

Gly Val Ala Met Val Leu Ala Val Ser Gly Leu Leu His Pro Met Ala
        835                 840                 845

Ala Thr Val Ala Met Leu Ala Ser Ser Leu Leu Ile Glu Trp Arg Ser
    850                 855                 860

Gly Arg Ala Arg Lys Tyr
865                 870


<210> 579
<211> 1925
<212> DNA
<213> Corynebacterium glutamicum

<220>
<221> CDS
<222> (1)..(1902)
<223> RXN01338

<400> 579
aaa act tat acc cca aat ccc tgg atg tta ttc atc cgc tca ttt gat      48

Lys Thr Tyr Thr Pro Asn Pro Trp Met Leu Phe Ile Arg Ser Phe Asp
1               5                   10                  15

ggc atc atc act gtc gca gcc ctt gtt gcc atc gca ata cat ctc att     96
Gly Ile Ile Thr Val Ala Ala Leu Val Ala Ile Ala Ile His Leu Ile
            20                  25                  30

tta tgg ctg gct cta gat cta gat ggc ctt gct aaa aac tgg cct tta     144
Leu Trp Leu Ala Leu Asp Leu Asp Gly Leu Ala Lys Asn Trp Pro Leu
        35                  40                  45

ata gcc atc gtt atc gta ggt ggc att ccg ttg atg tgg gat gtg ctg     192
Ile Ala Ile Val Ile Val Gly Gly Ile Pro Leu Met Trp Asp Val Leu
    50                  55                  60

aaa tca gcc att aaa act cgc ggt ggc gcg gat act tta gca gca gtc     240
Lys Ser Ala Ile Lys Thr Arg Gly Gly Ala Asp Thr Leu Ala Ala Val
65                  70                  75                  80

tcc atc att act tct gtg ttg tta ggg gag tgg ttg gtt gcc gcg atc     288
Ser Ile Ile Thr Ser Val Leu Leu Gly Glu Trp Leu Val Ala Ala Ile
                85                  90                  95

atc gtg ctc atg ctc tct ggt ggt gaa gcg cta gaa gag gca gca tca     336
Ile Val Leu Met Leu Ser Gly Gly Glu Ala Leu Glu Glu Ala Ala Ser
            100                 105                 110

cgg cga gcc agt ggc acc ttg gac gca ctt gcc cgg cgc gca cca agt     384
Arg Arg Ala Ser Gly Thr Leu Asp Ala Leu Ala Arg Arg Ala Pro Ser
        115                 120                 125

aca gct cac cgc ctg ttg ggt gca acc att ctt gat gga acc gaa gag     432
Thr Ala His Arg Leu Leu Gly Ala Thr Ile Leu Asp Gly Thr Glu Glu
    130                 135                 140

atc gcc gtg gaa gag atc acg gtt ggt gat tta gtg gcg gtg ctc ccg     480
Ile Ala Val Glu Glu Ile Thr Val Gly Asp Leu Val Ala Val Leu Pro
145                 150                 155                 160

cat gaa ctt tgt ccc gtg gat ggt gaa atc gtg gca ggc cac ggc acc     528
His Glu Leu Cys Pro Val Asp Gly Glu Ile Val Ala Gly His Gly Thr
                165                 170                 175

atg gat gag tct tat ctc acg ggt gag ccc tat gtg gtg agt aaa tct     576
Met Asp Glu Ser Tyr Leu Thr Gly Glu Pro Tyr Val Val Ser Lys Ser
            180                 185                 190

aaa ggt tcg caa gca atg tcg ggt gca gtc aat ggt gat act ccg ctg     624
Lys Gly Ser Gln Ala Met Ser Gly Ala Val Asn Gly Asp Thr Pro Leu
        195                 200                 205

acg att gtt gcc aca aag ctt gcc cat gat tcc aga tac gcc caa att     672
Thr Ile Val Ala Thr Lys Leu Ala His Asp Ser Arg Tyr Ala Gln Ile
    210                 215                 220

gtt ggt gta ctc cat gaa gca gaa aac aac cgc cca gaa atg cgc agg     720
Val Gly Val Leu His Glu Ala Glu Asn Asn Arg Prc Glu Met Arg Arg
225                 230                 235                 240

atg gct gac cgt ctt ggc gcg tgg .at acg gtg att gca ctt gcc ctc     768
Met Ala Asp Arg Leu Gly Ala Trp Tyr Thr Val Ile Ala Leu Ala Leu

245 250 255

ggt ggt ctt ggc tgg att gtc tcc ggc gac cca gtg agg ttc ttg gct 816
Gly Gly Leu Gly Trp Ile Val Ser Gly Asp Pro Val Arg Phe Leu Ala
260 265 270

gtt gtc gtt gtc gcc acc cca tgt cca ttg ctc att gca gtg cca gtg 864
Val Val Val Val Ala Thr Pro Cys Pro Leu Leu Ile Ala Val Pro Val
275 280 285

gcg atc atc ggt gcg att tct ctt gcg gct cgt cgg ggc atc atc gtg 912
Ala Ile Ile Gly Ala Ile Ser Leu Ala Ala Arg Arg Gly Ile Ile Val
290 295 300

aag aac cct gga atg ctg gaa aac gct tca gga gta aag aca gtg atg 960
Lys Asn Pro Gly Met Leu Glu Asn Ala Ser Gly Val Lys Thr Val Met
305 310 315 320

ttc gat aag act gga acg ctc acc tat ggc agg cca gtg att act gat 1008
Phe Asp Lys Thr Gly Thr Leu Thr Tyr Gly Arg Pro Val Ile Thr Asp
325 330 335

atc cac act gct ccc gga gtt gag gaa gat aca gtc cta gct ttg gct 1056
Ile His Thr Ala Pro Gly Val Glu Glu Asp Thr Val Leu Ala Leu Ala
340 345 350

gct tca gta gag cgc tac tcc aga cac ccg ttg gct gac gcg att cgt 1104
Ala Ser Val Glu Arg Tyr Ser Arg His Pro Leu Ala Asp Ala Ile Arg
355 360 365

gag ggc gca aaa gcc agg gaa ctt cat ctg cct gat gta gtg gaa gta 1152
Glu Gly Ala Lys Ala Arg Glu Leu His Leu Pro Asp Val Val Glu Val
370 375 380

tcg gaa cgt cca gga cag gga cta acc ggc acg gtg ggc gag cac ctg 1200
Ser Glu Arg Pro Gly Gln Gly Leu Thr Gly Thr Val Gly Glu His Leu
385 390 395 400

gtt cga ata acc aat agg cgc agc aca cta gaa att gat cca gac agc 1248
Val Arg Ile Thr Asn Arg Arg Ser Thr Leu Glu Ile Asp Pro Asp Ser
405 410 415

aag aac tac att ccg gtg aca agt tcc ggc atg gaa tct gtg gtg ctt 1296
Lys Asn Tyr Ile Pro Val Thr Ser Ser Gly Met Glu Ser Val Val Leu
420 425 430

gtt gat gat aaa tat gca gca ctc att cgc ctc cgg gat gaa cct cgt 1344
Val Asp Asp Lys Tyr Ala Ala Leu Ile Arg Leu Arg Asp Glu Pro Arg
435 440 445

gca tct gcc agt gag ttc atc gcg cac ttg ccc aag aag cac aaa gtg 1392
Ala Ser Ala Ser Glu Phe Ile Ala His Leu Pro Lys Lys His Lys Val
450 455 460

gac aag ctc atg att atc tct ggt gat cgc gca tct gag gtt cgt tac 1440
Asp Lys Leu Met Ile Ile Ser Gly Asp Arg Ala Ser Glu Val Arg Tyr
465 470 475 480

ctt gcg gac aag gtt ggc att gat gag gta cac gca gag gcc tca ccg 1488
Leu Ala Asp Lys Val Gly Ile Asp Glu Val His Ala Glu Ala Ser Pro
485 490 495

gaa gac aag ctg aac att gtt aat cgg cat aat gag cac ggc gcc acc    1536
Glu Asp Lys Leu Asn Ile Val Asn Arg His Asn Glu His Gly Ala Thr
            500                 505                 510

atg ttc tta ggt gat gga atc aac gat gcg cca gcc atg gcc gtt gcc    1584
Met Phe Leu Gly Asp Gly Ile Asn Asp Ala Pro Ala Met Ala Val Ala
        515                 520                 525

acc gtt ggt gtc gcg atg gga gca gac tcc gat gtc acg tcc gaa gca    1632
Thr Val Gly Val Ala Met Gly Ala Asp Ser Asp Val Thr Ser Glu Ala
    530                 535                 540

gca gat gct gtg att ttg gat tct tcc ctg gaa cgt ctc gac gat ctg    1680
Ala Asp Ala Val Ile Leu Asp Ser Ser Leu Glu Arg Leu Asp Asp Leu
545                 550                 555                 560

ctc cac atc agt gca cgg atg cgt cga ata gcg ttg caa tct gcg ggc    1728
Leu His Ile Ser Ala Arg Met Arg Arg Ile Ala Leu Gln Ser Ala Gly
                565                 570                 575

ggt ggc atg gcg ttg agt gtc ata gga atg atc ctc gcg gta ttt gga    1776
Gly Gly Met Ala Leu Ser Val Ile Gly Met Ile Leu Ala Val Phe Gly
            580                 585                 590

ttc ttg acg cca ctg atg ggt gcg atc ttc caa gag gtc att gac gtg    1824
Phe Leu Thr Pro Leu Met Gly Ala Ile Phe Gln Glu Val Ile Asp Val
        595                 600                 605

ctg gct atc ctc aat tcc gct cgg gtc gca ctg cca cgc gga gcg att    1872
Leu Ala Ile Leu Asn Ser Ala Arg Val Ala Leu Pro Arg Gly Ala Ile
    610                 615                 620

agt gat ttt gat acg caa gaa aaa gtt tct tagcagggta acctaaatgt      1922
Ser Asp Phe Asp Thr Gln Glu Lys Val Ser
625                 630

cgt                                                                 1925


<210> 580
<211> 634
<212> PRT
<213> Corynebacterium glutamicum

<400> 580
Lys Thr Tyr Thr Pro Asn Pro Trp Met Leu Phe Ile Arg Ser Phe Asp
1               5                   10                  15

Gly Ile Ile Thr Val Ala Ala Leu Val Ala Ile Ala Ile His Leu Ile
            20                  25                  30

Leu Trp Leu Ala Leu Asp Leu Asp Gly Leu Ala Lys Asn Trp Pro Leu
        35                  40                  45

Ile Ala Ile Val Ile Val Gly Gly Ile Pro Leu Met Trp Asp Val Leu
    50                  55                  60

Lys Ser Ala Ile Lys Thr Arg Gly Gly Ala Asp Thr Leu Ala Ala Val
65                  70                  75                  80

Ser Ile Ile Thr Ser Val Leu Leu Gly Glu Trp Leu Val Ala Ala Ile
                85                  90                  95

Ile Val Leu Met Leu Ser Gly Gly Glu Ala Leu Glu Glu Ala Ala Ser
            100                 105                 110

Arg Arg Ala Ser Gly Thr Leu Asp Ala Leu Ala Arg Arg Ala Pro Ser
        115                 120                 125

Thr Ala His Arg Leu Leu Gly Ala Thr Ile Leu Asp Gly Thr Glu Glu
    130                 135                 140

Ile Ala Val Glu Glu Ile Thr Val Gly Asp Leu Val Ala Val Leu Pro
145                 150                 155                 160

His Glu Leu Cys Pro Val Asp Gly Glu Ile Val Ala Gly His Gly Thr
                165                 170                 175

Met Asp Glu Ser Tyr Leu Thr Gly Glu Pro Tyr Val Val Ser Lys Ser
            180                 185                 190

Lys Gly Ser Gln Ala Met Ser Gly Ala Val Asn Gly Asp Thr Pro Leu
        195                 200                 205

Thr Ile Val Ala Thr Lys Leu Ala His Asp Ser Arg Tyr Ala Gln Ile
    210                 215                 220

Val Gly Val Leu His Glu Ala Glu Asn Asn Arg Pro Glu Met Arg Arg
225                 230                 235                 240

Met Ala Asp Arg Leu Gly Ala Trp Tyr Thr Val Ile Ala Leu Ala Leu
                245                 250                 255

Gly Gly Leu Gly Trp Ile Val Ser Gly Asp Pro Val Arg Phe Leu Ala
            260                 265                 270

Val Val Val Val Ala Thr Pro Cys Pro Leu Leu Ile Ala Val Pro Val
        275                 280                 285

Ala Ile Ile Gly Ala Ile Ser Leu Ala Ala Arg Arg Gly Ile Ile Val
    290                 295                 300

Lys Asn Pro Gly Met Leu Glu Asn Ala Ser Gly Val Lys Thr Val Met
305                 310                 315                 320

Phe Asp Lys Thr Gly Thr Leu Thr Tyr Gly Arg Pro Val Ile Thr Asp
                325                 330                 335

Ile His Thr Ala Pro Gly Val Glu Glu Asp Thr Val Leu Ala Leu Ala
            340                 345                 350

Ala Ser Val Glu Arg Tyr Ser Arg His Pro Leu Ala Asp Ala Ile Arg
        355                 360                 365

Glu Gly Ala Lys Ala Arg Glu Leu His Leu Pro Asp Val Val Glu Val
    370                 375                 380

Ser Glu Arg Pro Gly Gln Gly Leu Thr Gly Thr Val Gly Glu His Leu
385                 390                 395                 400

Val Arg Ile Thr Asn Arg Arg Ser Thr Leu Glu Ile Asp Pro Asp Ser

405                 410                 415

Lys Asn Tyr Ile Pro Val Thr Ser Ser Gly Met Glu Ser Val Val Leu
            420                 425                 430

Val Asp Asp Lys Tyr Ala Ala Leu Ile Arg Leu Arg Asp Glu Pro Arg
        435                 440                 445

Ala Ser Ala Ser Glu Phe Ile Ala His Leu Pro Lys Lys His Lys Val
    450                 455                 460

Asp Lys Leu Met Ile Ile Ser Gly Asp Arg Ala Ser Glu Val Arg Tyr
465                 470                 475                 480

Leu Ala Asp Lys Val Gly Ile Asp Glu Val His Ala Glu Ala Ser Pro
                485                 490                 495

Glu Asp Lys Leu Asn Ile Val Asn Arg His Asn Glu His Gly Ala Thr
            500                 505                 510

Met Phe Leu Gly Asp Gly Ile Asn Asp Ala Pro Ala Met Ala Val Ala
        515                 520                 525

Thr Val Gly Val Ala Met Gly Ala Asp Ser Asp Val Thr Ser Glu Ala
    530                 535                 540

Ala Asp Ala Val Ile Leu Asp Ser Ser Leu Glu Arg Leu Asp Asp Leu
545                 550                 555                 560

Leu His Ile Ser Ala Arg Met Arg Arg Ile Ala Leu Gln Ser Ala Gly
                565                 570                 575

Gly Gly Met Ala Leu Ser Val Ile Gly Met Ile Leu Ala Val Phe Gly
            580                 585                 590

Phe Leu Thr Pro Leu Met Gly Ala Ile Phe Gln Glu Val Ile Asp Val
        595                 600                 605

Leu Ala Ile Leu Asn Ser Ala Arg Val Ala Leu Pro Arg Gly Ala Ile
    610                 615                 620

Ser Asp Phe Asp Thr Gln Glu Lys Val Ser
625                 630

<210> 581
<211> 1917
<212> DNA
<213> Corynebacterium glutamicum

<220>
<221> CDS
<222> (101)..(1894)
<223> RXN00980

<400> 581
agagagaaag ggagaaatca tgaaaacgtg gaagacctgg ggggtcgtcg gagcttcagg 60

cctcttgatt attttgtcgt ggttgagttc atcgagcccg atg ctg gca gat gca   115
                                            Met Leu Ala Asp Ala
                                            1               5

ttc atg atc gcg gct gca att gtt gca ggt tgg ccg atc gcg cag tct 163
Phe Met Ile Ala Ala Ala Ile Val Ala Gly Trp Pro Ile Ala Gln Ser
                10                  15                  20

gca tat caa gca ctt cgc att cga atg gtg tcg att gac tta ctg gtc 211
Ala Tyr Gln Ala Leu Arg Ile Arg Met Val Ser Ile Asp Leu Leu Val
            25                  30                  35

gtt gtg gct gcc gtt ggt gcc atg ttc atc aac aac tat tgg gag tct 259
Val Val Ala Ala Val Gly Ala Met Phe Ile Asn Asn Tyr Trp Glu Ser
        40                  45                  50

gcg gcg gtg acg ttc ctc ttt gcc ctt ggc aag gca ctg gaa cgc gcg 307
Ala Ala Val Thr Phe Leu Phe Ala Leu Gly Lys Ala Leu Glu Arg Ala
    55                  60                  65

aca atg aac cgc aca cga aaa gca cta tcg gat ctg gtg gat gca gct 355
Thr Met Asn Arg Thr Arg Lys Ala Leu Ser Asp Leu Val Asp Ala Ala
70                  75                  80                  85

cca gaa act gca aca agg ctc aac gcg gat gac tca aca gag gta gtt 403
Pro Glu Thr Ala Thr Arg Leu Asn Ala Asp Asp Ser Thr Glu Val Val
                90                  95                  100

gag ctg tgg gag ctt gag ccc ggt gac atc gtc ttg gta cgc aat ggc 451
Glu Leu Trp Glu Leu Glu Pro Gly Asp Ile Val Leu Val Arg Asn Gly
            105                 110                 115

gaa caa att ccc gtc gat gga aac gtg att gcg ggt gtc ggt gga att 499
Glu Gln Ile Pro Val Asp Gly Asn Val Ile Ala Gly Val Gly Gly Ile
        120                 125                 130

gat gaa tcc aac atc acg ggt gaa tca atg ccg gct gaa aag ggt caa 547
Asp Glu Ser Asn Ile Thr Gly Glu Ser Met Pro Ala Glu Lys Gly Gln
    135                 140                 145

ggc tct gat gtg tat gca gga acc tgg ctg cga tct ggt gtt ttg aga 595
Gly Ser Asp Val Tyr Ala Gly Thr Trp Leu Arg Ser Gly Val Leu Arg
150                 155                 160                 165

gtc gag gca aca gga att ggt tca gac tca act ttg gca aaa atc att 643
Val Glu Ala Thr Gly Ile Gly Ser Asp Ser Thr Leu Ala Lys Ile Ile
                170                 175                 180

cac cgc gtt gaa gac gcc cag gat gac aaa gcc cgc aca caa aca ttc 691
His Arg Val Glu Asp Ala Gln Asp Asp Lys Ala Arg Thr Gln Thr Phe
            185                 190                 195

tta gag aaa ttc tct aag tgg tac acc ccg ggc gtc atg atc gcc gcc 739
Leu Glu Lys Phe Ser Lys Trp Tyr Thr Pro Gly Val Met Ile Ala Ala
        200                 205                 210

gca gtg gtg gga ctt atc acc tgg gac gta gaa cta gca ctg acg ctc 787
Ala Val Val Gly Leu Ile Thr Trp Asp Val Glu Leu Ala Leu Thr Leu
    215                 220                 225

tta gtg atc ggc tgc ccc ggc gcg ttg gtt atc tcc atc ccg gtg tcc 835
Leu Val Ile Gly Cys Pro Gly Ala Leu Val Ile Ser Ile Pro Val Ser
230                 235                 240                 245

atc gtc gca ggc atc ggc cgt gct gca cgc gat ggc gtg ctg atc aag 883
Ile Val Ala Gly Ile Gly Arg Ala Ala Arg Asp Gly Val Leu Ile Lys
                250                 255                 260

ggt gga gaa tac cta gaa acc gcc gcg aaa gtc gac gtc gtt gtc gtg 931
Gly Gly Glu Tyr Leu Glu Thr Ala Ala Lys Val Asp Val Val Val Val
            265                 270                 275

gac aaa act gga acg ctg acc acc ggc cgc cca gaa ctc aca gac gta 979
Asp Lys Thr Gly Thr Leu Thr Thr Gly Arg Pro Glu Leu Thr Asp Val
        280                 285                 290

gaa gtc atc gag ccc gcc tac agc cag ggc gag gtg ctg gag ctc gcc 1027
Glu Val Ile Glu Pro Ala Tyr Ser Gln Gly Glu Val Leu Glu Leu Ala
    295                 300                 305

gcg cgc gcc gag acg gct tca gaa cat ccg ctt gcc gac gcc atc atc 1075
Ala Arg Ala Glu Thr Ala Ser Glu His Pro Leu Ala Asp Ala Ile Ile
310                 315                 320                 325

cgt ggt gcc cag gat cgg ggg ctg tcc aca aca ttg gtg gaa gca gct 1123
Arg Gly Ala Gln Asp Arg Gly Leu Ser Thr Thr Leu Val Glu Ala Ala
                330                 335                 340

gaa aac atc acc ggc cga ggc att atc gca aat gtt gat gga cag gca 1171
Glu Asn Ile Thr Gly Arg Gly Ile Ile Ala Asn Val Asp Gly Gln Ala
            345                 350                 355

gtt gct gtt gga tct gct gag tta ctt gat cat gaa cca gac tcg acc 1219
Val Ala Val Gly Ser Ala Glu Leu Leu Asp His Glu Pro Asp Ser Thr
        360                 365                 370

agg atc ctg gag cta aat gcc gaa gga aag acc gcg atg ttt gtc gga 1267
Arg Ile Leu Glu Leu Asn Ala Glu Gly Lys Thr Ala Met Phe Val Gly
    375                 380                 385

gtg aac gga cac gcc att gga atc gtg gcc gtc gcc gac gcc gtt cgt 1315
Val Asn Gly His Ala Ile Gly Ile Val Ala Val Ala Asp Ala Val Arg
390                 395                 400                 405

tca gat tct gcc tca gca atc gaa tcg ctg cat aag gcg ggc att caa 1363
Ser Asp Ser Ala Ser Ala Ile Glu Ser Leu His Lys Ala Gly Ile Gln
                410                 415                 420

gtt gtc atg gcg act ggc gac gct cac cgc gtt gca caa aac gtg gcc 1411
Val Val Met Ala Thr Gly Asp Ala His Arg Val Ala Gln Asn Val Ala
            425                 430                 435

tcc aag ctg gga gtg gat gaa gtc tac tca gag cta ctc cct gaa cag 1459
Ser Lys Leu Gly Val Asp Glu Val Tyr Ser Glu Leu Leu Pro Glu Gln
        440                 445                 450

aaa tta gaa ctg gtg cgt gat ctg caa gct gcc ggc aaa acg gtc gcg 1507
Lys Leu Glu Leu Val Arg Asp Leu Gln Ala Ala Gly Lys Thr Val Ala
    455                 460                 465

atg gtg ggt gac gga gtc aac gac acc cca gca ttg gca gct gct gat 1555
Met Val Gly Asp Gly Val Asn Asp Thr Pro Ala Leu Ala Ala Ala Asp
470                 475                 480                 485

atc gga gta gcg atg ggc gtg gca ggt tcc cct gca gcc att gaa acc 1603

Ile Gly Val Ala Met Gly Val Ala Gly Ser Pro Ala Ala Ile Glu Thr
                490                 495                 500

gct gat atc gca ctc atg gcg gat cgt ctc cca cgg ctg gca cat gca     1651
Ala Asp Ile Ala Leu Met Ala Asp Arg Leu Pro Arg Leu Ala His Ala
            505                 510                 515

gtg acc ttg gca aaa cgc acc gta aga acc atg cgc atc aat att ctg     1699
Val Thr Leu Ala Lys Arg Thr Val Arg Thr Met Arg Ile Asn Ile Leu
        520                 525                 530

att gcg ttg gct acc gtg atg gtg tta cta gct ggc gtc cta ttt ggc     1747
Ile Ala Leu Ala Thr Val Met Val Leu Leu Ala Gly Val Leu Phe Gly
    535                 540                 545

gga gtt acc atg tcg gtt ggc atg ctc gtt cac gaa gca agc gtg ctg     1795
Gly Val Thr Met Ser Val Gly Met Leu Val His Glu Ala Ser Val Leu
550                 555                 560                 565

ctt gtt atc agc atc gcc atg ctg ttg ctg cgt cca aca ctt aaa gaa     1843
Leu Val Ile Ser Ile Ala Met Leu Leu Leu Arg Pro Thr Leu Lys Glu
                570                 575                 580

gat gct gcg caa gca agt gat att aaa cgc tcg gaa ata caa cag atc     1891
Asp Ala Ala Gln Ala Ser Asp Ile Lys Arg Ser Glu Ile Gln Gln Ile
            585                 590                 595

gca taaccaatgg ctgggtactg atg                                       1917
Ala

<210> 582
<211> 598
<212> PRT
<213> Corynebacterium glutamicum

<400> 582
Met Leu Ala Asp Ala Phe Met Ile Ala Ala Ala Ile Val Ala Gly Trp
  1               5                  10                  15

Pro Ile Ala Gln Ser Ala Tyr Gln Ala Leu Arg Ile Arg Met Val Ser
             20                  25                  30

Ile Asp Leu Leu Val Val Val Ala Ala Val Gly Ala Met Phe Ile Asn
         35                  40                  45

Asn Tyr Trp Glu Ser Ala Ala Val Thr Phe Leu Phe Ala Leu Gly Lys
     50                  55                  60

Ala Leu Glu Arg Ala Thr Met Asn Arg Thr Arg Lys Ala Leu Ser Asp
 65                  70                  75                  80

Leu Val Asp Ala Ala Pro Glu Thr Ala Thr Arg Leu Asn Ala Asp Asp
                 85                  90                  95

Ser Thr Glu Val Val Glu Leu Trp Glu Leu Glu Pro Gly Asp Ile Val
            100                 105                 110

Leu Val Arg Asn Gly Glu Gln Ile Pro Val Asp Gly Asn Val Ile Ala
        115                 120                 125

Gly Val Gly Gly Ile Asp Glu Ser Asn Ile Thr Gly Glu Ser Met Pro
    130                 135                 140

Ala Glu Lys Gly Gln Gly Ser Asp Val Tyr Ala Gly Thr Trp Leu Arg
145                 150                 155                 160

Ser Gly Val Leu Arg Val Glu Ala Thr Gly Ile Gly Ser Asp Ser Thr
                165                 170                 175

Leu Ala Lys Ile Ile His Arg Val Glu Asp Ala Gln Asp Asp Lys Ala
            180                 185                 190

Arg Thr Gln Thr Phe Leu Glu Lys Phe Ser Lys Trp Tyr Thr Pro Gly
        195                 200                 205

Val Met Ile Ala Ala Ala Val Val Gly Leu Ile Thr Trp Asp Val Glu
    210                 215                 220

Leu Ala Leu Thr Leu Leu Val Ile Gly Cys Pro Gly Ala Leu Val Ile
225                 230                 235                 240

Ser Ile Pro Val Ser Ile Val Ala Gly Ile Gly Arg Ala Ala Arg Asp
                245                 250                 255

Gly Val Leu Ile Lys Gly Gly Glu Tyr Leu Glu Thr Ala Ala Lys Val
            260                 265                 270

Asp Val Val Val Val Asp Lys Thr Gly Thr Leu Thr Thr Gly Arg Pro
        275                 280                 285

Glu Leu Thr Asp Val Glu Val Ile Glu Pro Ala Tyr Ser Gln Gly Glu
    290                 295                 300

Val Leu Glu Leu Ala Ala Arg Ala Glu Thr Ala Ser Glu His Pro Leu
305                 310                 315                 320

Ala Asp Ala Ile Ile Arg Gly Ala Gln Asp Arg Gly Leu Ser Thr Thr
                325                 330                 335

Leu Val Glu Ala Ala Glu Asn Ile Thr Gly Arg Gly Ile Ile Ala Asn
            340                 345                 350

Val Asp Gly Gln Ala Val Ala Val Gly Ser Ala Glu Leu Leu Asp His
        355                 360                 365

Glu Pro Asp Ser Thr Arg Ile Leu Glu Leu Asn Ala Glu Gly Lys Thr
    370                 375                 380

Ala Met Phe Val Gly Val Asn Gly His Ala Ile Gly Ile Val Ala Val
385                 390                 395                 400

Ala Asp Ala Val Arg Ser Asp Ser Ala Ser Ala Ile Glu Ser Leu His
                405                 410                 415

Lys Ala Gly Ile Gln Val Val Met Ala Thr Gly Asp Ala His Arg Val
            420                 425                 430

Ala Gln Asn Val Ala Ser Lys Leu Gly Val Asp Glu Val Tyr Ser Glu
        435                 440                 445

Leu Leu Pro Glu Gln Lys Leu Glu Leu Val Arg Asp Leu Gln Ala Ala
    450                 455                 460

Gly Lys Thr Val Ala Met Val Gly Asp Gly Val Asn Asp Thr Pro Ala
465                 470                 475                 480

Leu Ala Ala Ala Asp Ile Gly Val Ala Met Gly Val Ala Gly Ser Pro
                485                 490                 495

Ala Ala Ile Glu Thr Ala Asp Ile Ala Leu Met Ala Asp Arg Leu Pro
            500                 505                 510

Arg Leu Ala His Ala Val Thr Leu Ala Lys Arg Thr Val Arg Thr Met
        515                 520                 525

Arg Ile Asn Ile Leu Ile Ala Leu Ala Thr Val Met Val Leu Leu Ala
    530                 535                 540

Gly Val Leu Phe Gly Gly Val Thr Met Ser Val Gly Met Leu Val His
545                 550                 555                 560

Glu Ala Ser Val Leu Leu Val Ile Ser Ile Ala Met Leu Leu Leu Arg
                565                 570                 575

Pro Thr Leu Lys Glu Asp Ala Ala Gln Ala Ser Asp Ile Lys Arg Ser
            580                 585                 590

Glu Ile Gln Gln Ile Ala
        595


<210> 583
<211> 1296
<212> DNA
<213> Corynebacterium glutamicum

<220>
<221> CDS
<222> (101)..(1273)
<223> RXN00099

<400> 583
ctctggtgaa gaggatgttg actcgggaga ttcttccact gattcactga ttaagtggta 60

ccgcgcaaat aggtagtcgc ttgcttatag ggtcaggggc gtg aag aat cct cgc   115
                                            Val Lys Asn Pro Arg
                                            1               5

ctc ata gca ctg gcc gct atc atc ctg acc tcg ttc aat ctg cga aca   163
Leu Ile Ala Leu Ala Ala Ile Ile Leu Thr Ser Phe Asn Leu Arg Thr
                10                  15                  20

gct att act gct tta gct ccg ctg gtt tct gag att cgg gat gat tta   211
Ala Ile Thr Ala Leu Ala Pro Leu Val Ser Glu Ile Arg Asp Asp Leu
            25                  30                  35

ggg gtt agt gct tct ctt att ggt gtg ttg ggc atg atc ccg act gct   259
Gly Val Ser Ala Ser Leu Ile Gly Val Leu Gly Met Ile Pro Thr Ala
        40                  45                  50

atg ttc gcg gat gct gcg ttt gcg ctt ccg tcg ttg aag agg aag ttc   307

Met Phe Ala Asp Ala Ala Phe Ala Leu Pro Ser Leu Lys Arg Lys Phe
    55                  60                  65

act act tcc caa ctg ttg atg ttt gcc atg ctg ttg act gct gcc ggt 355
Thr Thr Ser Gln Leu Leu Met Phe Ala Met Leu Leu Thr Ala Ala Gly
70                  75                  80                  85

cag att att cgt gtc gct gga cct gct tcg ctg ttg atg gtc ggt act 403
Gln Ile Ile Arg Val Ala Gly Pro Ala Ser Leu Leu Met Val Gly Thr
                90                  95                  100

gtg ttc gcg atg ttt gcg atc gga gtt acc aat gtg ttg ctt ccg att 451
Val Phe Ala Met Phe Ala Ile Gly Val Thr Asn Val Leu Leu Pro Ile
            105                 110                 115

gct gtt agg gag tat ttt ccg cgt cac gtc ggt gga atg tcg aca act 499
Ala Val Arg Glu Tyr Phe Pro Arg His Val Gly Gly Met Ser Thr Thr
        120                 125                 130

tat ctg gtg tcg ttc cag att gtt cag gca ctt gct ccg acg ctt gcc 547
Tyr Leu Val Ser Phe Gln Ile Val Gln Ala Leu Ala Pro Thr Leu Ala
    135                 140                 145

gtg ccg att tct cag tgg gct aca cat gtg ggg ttg acc ggt tgg agg 595
Val Pro Ile Ser Gln Trp Ala Thr His Val Gly Leu Thr Gly Trp Arg
150                 155                 160                 165

gtg tcg ctc ggt tcg tgg gcg ctg ctg ggg ttg gtt gcg gcg att tcg 643
Val Ser Leu Gly Ser Trp Ala Leu Leu Gly Leu Val Ala Ala Ile Ser
                170                 175                 180

tgg att ccg ctg ttg agt ttg cag ggt gcc agg gtt gtt gcg gcg ccg 691
Trp Ile Pro Leu Leu Ser Leu Gln Gly Ala Arg Val Val Ala Ala Pro
            185                 190                 195

tcg aag gtt tct ctt cct gtg tgg aag tct tcg gtt ggt gtg ggg ctc 739
Ser Lys Val Ser Leu Pro Val Trp Lys Ser Ser Val Gly Val Gly Leu
        200                 205                 210

ggg ttg atg ttt ggg ttt act tcg ttt gcg acg tat atc ctc atg ggt 787
Gly Leu Met Phe Gly Phe Thr Ser Phe Ala Thr Tyr Ile Leu Met Gly
    215                 220                 225

ttt atg ccg cag atg gta ggt gat cct cag ctc ggt gcg gtg ttg tta 835
Phe Met Pro Gln Met Val Gly Asp Pro Gln Leu Gly Ala Val Leu Leu
230                 235                 240                 245

ggc tgg tgg tca att ttg gga ttg ccg ctg aac att ctg gga ccg tgg 883
Gly Trp Trp Ser Ile Leu Gly Leu Pro Leu Asn Ile Leu Gly Pro Trp
                250                 255                 260

ttg gtg acg cgt ttc act aac tgc ttc ccg atg gtt gtt atc gcc agt 931
Leu Val Thr Arg Phe Thr Asn Cys Phe Pro Met Val Val Ile Ala Ser
            265                 270                 275

gtc atg ttt ctc atc ggt aat ggt ggg ttt tgt ttg gct ccg gat gtt 979
Val Met Phe Leu Ile Gly Asn Gly Gly Phe Cys Leu Ala Pro Asp Val
        280                 285                 290

gcg ccg tgg ttg tgg gcg acg ttg tct ggt ctt ggt ccc ctt gcg ttc 1027
Ala Pro Trp Leu Trp Ala Thr Leu Ser Gly Leu Gly Pro Leu Ala Phe

295                 300                 305

ccg atg gcg ttg acg ctc att aat att cgt gct gaa act agt gct ggt     1075
Pro Met Ala Leu Thr Leu Ile Asn Ile Arg Ala Glu Thr Ser Ala Gly
310                 315                 320                 325

gct tct gcg ttg agt tcc ttc ggg cag ggt ttg ggt tat acg att gcg     1123
Ala Ser Ala Leu Ser Ser Phe Gly Gln Gly Leu Gly Tyr Thr Ile Ala
                330                 335                 340

tgt ttc ggt ccc ttg ttg act ggt ttc att gtc gat gcg aca ggc agc     1171
Cys Phe Gly Pro Leu Leu Thr Gly Phe Ile Val Asp Ala Thr Gly Ser
            345                 350                 355

ttc cga aca atc ttt gtg ctt ttt gcg gtt gca aca ctc ttc gtt att     1219
Phe Arg Thr Ile Phe Val Leu Phe Ala Val Ala Thr Leu Phe Val Ile
        360                 365                 370

aga ggc ggt tac ttt gcg aca agg cag gtt tac gtc gaa aag ctt tta     1267
Arg Gly Gly Tyr Phe Ala Thr Arg Gln Val Tyr Val Glu Lys Leu Leu
    375                 380                 385

aat cgc taggatggcg ctatgccgca aag                                   1296
Asn Arg
390


<210> 584
<211> 391
<212> PRT
<213> Corynebacterium glutamicum

<400> 584
Val Lys Asn Pro Arg Leu Ile Ala Leu Ala Ala Ile Ile Leu Thr Ser
  1               5                  10                  15

Phe Asn Leu Arg Thr Ala Ile Thr Ala Leu Ala Pro Leu Val Ser Glu
             20                  25                  30

Ile Arg Asp Asp Leu Gly Val Ser Ala Ser Leu Ile Gly Val Leu Gly
         35                  40                  45

Met Ile Pro Thr Ala Met Phe Ala Asp Ala Ala Phe Ala Leu Pro Ser
     50                  55                  60

Leu Lys Arg Lys Phe Thr Thr Ser Gln Leu Leu Met Phe Ala Met Leu
 65                  70                  75                  80

Leu Thr Ala Ala Gly Gln Ile Ile Arg Val Ala Gly Pro Ala Ser Leu
                 85                  90                  95

Leu Met Val Gly Thr Val Phe Ala Met Phe Ala Ile Gly Val Thr Asn
            100                 105                 110

Val Leu Leu Pro Ile Ala Val Arg Glu Tyr Phe Pro Arg His Val Gly
        115                 120                 125

Gly Met Ser Thr Thr Tyr Leu Val Ser Phe Gln Ile Val Gln Ala Leu
    130                 135                 140

Ala Pro Thr Leu Ala Val Pro Ile Ser Gln Trp Ala Thr His Val Gly

145                 150                 155                 160

Leu Thr Gly Trp Arg Val Ser Leu Gly Ser Trp Ala Leu Leu Gly Leu
                165                 170                 175

Val Ala Ala Ile Ser Trp Ile Pro Leu Leu Ser Leu Gln Gly Ala Arg
            180                 185                 190

Val Val Ala Ala Pro Ser Lys Val Ser Leu Pro Val Trp Lys Ser Ser
        195                 200                 205

Val Gly Val Gly Leu Gly Leu Met Phe Gly Phe Thr Ser Phe Ala Thr
    210                 215                 220

Tyr Ile Leu Met Gly Phe Met Pro Gln Met Val Gly Asp Pro Gln Leu
225                 230                 235                 240

Gly Ala Val Leu Leu Gly Trp Trp Ser Ile Leu Gly Leu Pro Leu Asn
                245                 250                 255

Ile Leu Gly Pro Trp Leu Val Thr Arg Phe Thr Asn Cys Phe Pro Met
            260                 265                 270

Val Val Ile Ala Ser Val Met Phe Leu Ile Gly Asn Gly Gly Phe Cys
        275                 280                 285

Leu Ala Pro Asp Val Ala Pro Trp Leu Trp Ala Thr Leu Ser Gly Leu
    290                 295                 300

Gly Pro Leu Ala Phe Pro Met Ala Leu Thr Leu Ile Asn Ile Arg Ala
305                 310                 315                 320

Glu Thr Ser Ala Gly Ala Ser Ala Leu Ser Ser Phe Gly Gln Gly Leu
                325                 330                 335

Gly Tyr Thr Ile Ala Cys Phe Gly Pro Leu Leu Thr Gly Phe Ile Val
            340                 345                 350

Asp Ala Thr Gly Ser Phe Arg Thr Ile Phe Val Leu Phe Ala Val Ala
        355                 360                 365

Thr Leu Phe Val Ile Arg Gly Gly Tyr Phe Ala Thr Arg Gln Val Tyr
    370                 375                 380

Val Glu Lys Leu Leu Asn Arg
385                 390

<210> 585
<211> 387
<212> DNA
<213> Corynebacterium glutamicum

<220>
<221> CDS
<222> (101)..(364)
<223> RXN02662

<400> 585
ccaaggtgtc accctcaccg ttgccatcgc cttcatcatc gtcaatatcg ccgtggacct 60

gctctacgtc ctggtcaatc cacgtattag gagcatctag atg cgc cgt aaa cta 115
Met Arg Arg Lys Leu
1               5

acc acc aca tta gaa aac aag ccc ggt gca cga ctt ggt ggc ttc cgc 163
Thr Thr Thr Leu Glu Asn Lys Pro Gly Ala Arg Leu Gly Gly Phe Arg
                10                  15                  20

gca ctt gca cca act tca aaa atc gcg ctg gtt ttc ctt ctc ctg atc 211
Ala Leu Ala Pro Thr Ser Lys Ile Ala Leu Val Phe Leu Leu Leu Ile
            25                  30                  35

ttc ctc ctc gcg atc ttt gcc cca ctg att gct aaa tac gat cca ctg 259
Phe Leu Leu Ala Ile Phe Ala Pro Leu Ile Ala Lys Tyr Asp Pro Leu
        40                  45                  50

gcc tcc gga act cca gtc cag cct cca agc ggt gag cac tgg ttt ggt 307
Ala Ser Gly Thr Pro Val Gln Pro Pro Ser Gly Glu His Trp Phe Gly
    55                  60                  65

acc gac gcc atc ggc cgc gat att ttc tcc cgc gta gcc acg gcg cca 355
Thr Asp Ala Ile Gly Arg Asp Ile Phe Ser Arg Val Ala Thr Ala Pro
70                  75                  80                  85

gag cct ccc tgatcattgg tcttttcgct acg 387
Glu Pro Pro

<210> 586
<211> 88
<212> PRT
<213> Corynebacterium glutamicum

<400> 586
Met Arg Arg Lys Leu Thr Thr Thr Leu Glu Asn Lys Pro Gly Ala Arg
1               5                   10                  15

Leu Gly Gly Phe Arg Ala Leu Ala Pro Thr Ser Lys Ile Ala Leu Val
            20                  25                  30

Phe Leu Leu Leu Ile Phe Leu Leu Ala Ile Phe Ala Pro Leu Ile Ala
        35                  40                  45

Lys Tyr Asp Pro Leu Ala Ser Gly Thr Pro Val Gln Pro Pro Ser Gly
    50                  55                  60

Glu His Trp Phe Gly Thr Asp Ala Ile Gly Arg Asp Ile Phe Ser Arg
65                  70                  75                  80

Val Ala Thr Ala Pro Glu Pro Pro
                85

<210> 587
<211> 972
<212> DNA
<213> Corynebacterium glutamicum

<220>
<221> CDS

<222> (101)..(949)
<223> RXN02442

<400> 587
gccgtgatgt tgttgagcgc gatgtgattg ccgtatgtgc atgtgagatt ccggacgctg 60

agttctgcca ttccttaatg ataacggtta tcattttcaa atg aag ttt ttt act 115
Met Lys Phe Phe Thr
1               5

gac gcc ctc ata gtg cct ttt gac gtt tca ttc atc tcc cgc gcc ctg 163
Asp Ala Leu Ile Val Pro Phe Asp Val Ser Phe Ile Ser Arg Ala Leu
                10                  15                  20

gtc gcc gga tgc ctg gcc gca att tta tgc tca ctc att gga acg tgg 211
Val Ala Gly Cys Leu Ala Ala Ile Leu Cys Ser Leu Ile Gly Thr Trp
            25                  30                  35

gtt att ttg cgc agg cta acc ttt ttc ggc gac gct atg tcg cac ggc 259
Val Ile Leu Arg Arg Leu Thr Phe Phe Gly Asp Ala Met Ser His Gly
        40                  45                  50

ttg ctc ccc gga gta gcc acg gca tca cta ttg ggc gga aat ctc atg 307
Leu Leu Pro Gly Val Ala Thr Ala Ser Leu Leu Gly Gly Asn Leu Met
    55                  60                  65

ttc ggc gca gca atc agc gca tta atc atg tca gcc gga gtg gtg tgg 355
Phe Gly Ala Ala Ile Ser Ala Leu Ile Met Ser Ala Gly Val Val Trp
70                  75                  80                  85

acc agc aga aaa tcc agc ctc tcc caa gac gtc agc att ggc ctg caa 403
Thr Ser Arg Lys Ser Ser Leu Ser Gln Asp Val Ser Ile Gly Leu Gln
                90                  95                  100

ttt att acc atg ctt tcc ctc ggc gtg gtt att gtg tcc cac tcc gat 451
Phe Ile Thr Met Leu Ser Leu Gly Val Val Ile Val Ser His Ser Asp
            105                 110                 115

tcc cac gcc gta gac ctc acc agt ttc ctt ttt gga gac att ctt ggc 499
Ser His Ala Val Asp Leu Thr Ser Phe Leu Phe Gly Asp Ile Leu Gly
        120                 125                 130

gtg cga ccc tcg gat ata ttc atc atc gcc att gca aca gtg ttg ggt 547
Val Arg Pro Ser Asp Ile Phe Ile Ile Ala Ile Ala Thr Val Leu Gly
    135                 140                 145

gga ttg act att ttt ctc ttc cac cga cag ttc act gca ctc gct ttc 595
Gly Leu Thr Ile Phe Leu Phe His Arg Gln Phe Thr Ala Leu Ala Phe
150                 155                 160                 165

gac gag cgt aaa gct cac acc tta gga ctc aat ccc cgc ttt gca cac 643
Asp Glu Arg Lys Ala His Thr Leu Gly Leu Asn Pro Arg Phe Ala His
                170                 175                 180

cta ctc atg ctg gca ctg atc gca tta gct acg gtg gtg tcg ttt cag 691
Leu Leu Met Leu Ala Leu Ile Ala Leu Ala Thr Val Val Ser Phe Gln
            185                 190                 195

gtg gtg gga acg ctt tta gtg ttt gga ctt ctc att ggt ccg ccc gcc 739
Val Val Gly Thr Leu Leu Val Phe Gly Leu Leu Ile Gly Pro Pro Ala
        200                 205                 210

acg gct gca ctt tta gtg caa gac aaa gca agt att tca ctg atc atg 787
Thr Ala Ala Leu Leu Val Gln Asp Lys Ala Ser Ile Ser Leu Ile Met
    215                 220                 225

atc gtc gcg tcg ctt ctt gga tgc gcg gaa att tac ctc ggg ctt tta 835
Ile Val Ala Ser Leu Leu Gly Cys Ala Glu Ile Tyr Leu Gly Leu Leu
230                 235                 240                 245

atc agc tgg cac gca agc act gcc gcg gga gcc act atc act ttg tta 883
Ile Ser Trp His Ala Ser Thr Ala Ala Gly Ala Thr Ile Thr Leu Leu
                250                 255                 260

agt gct gcg ata ttt ttt gcc acc tta ttg aca aag agt gcc att agt 931
Ser Ala Ala Ile Phe Phe Ala Thr Leu Leu Thr Lys Ser Ala Ile Ser
            265                 270                 275

agg tta aac ttc acc gcg tgatactgaa agacattttc aat 972
Arg Leu Asn Phe Thr Ala
        280

<210> 588
<211> 283
<212> PRT
<213> Corynebacterium glutamicum

<400> 588
Met Lys Phe Phe Thr Asp Ala Leu Ile Val Pro Phe Asp Val Ser Phe
1               5                   10                  15

Ile Ser Arg Ala Leu Val Ala Gly Cys Leu Ala Ala Ile Leu Cys Ser
            20                  25                  30

Leu Ile Gly Thr Trp Val Ile Leu Arg Arg Leu Thr Phe Phe Gly Asp
        35                  40                  45

Ala Met Ser His Gly Leu Leu Pro Gly Val Ala Thr Ala Ser Leu Leu
    50                  55                  60

Gly Gly Asn Leu Met Phe Gly Ala Ala Ile Ser Ala Leu Ile Met Ser
65                  70                  75                  80

Ala Gly Val Val Trp Thr Ser Arg Lys Ser Ser Leu Ser Gln Asp Val
                85                  90                  95

Ser Ile Gly Leu Gln Phe Ile Thr Met Leu Ser Leu Gly Val Val Ile
            100                 105                 110

Val Ser His Ser Asp Ser His Ala Val Asp Leu Thr Ser Phe Leu Phe
        115                 120                 125

Gly Asp Ile Leu Gly Val Arg Pro Ser Asp Ile Phe Ile Ile Ala Ile
    130                 135                 140

Ala Thr Val Leu Gly Gly Leu Thr Ile Phe Leu Phe His Arg Gln Phe
145                 150                 155                 160

Thr Ala Leu Ala Phe Asp Glu Arg Lys Ala His Thr Leu Gly Leu Asn
                165                 170                 175

Pro Arg Phe Ala His Leu Leu Met Leu Ala Leu Ile Ala Leu Ala Thr
            180                 185                 190

Val Val Ser Phe Gln Val Val Gly Thr Leu Leu Val Phe Gly Leu Leu
        195                 200                 205

Ile Gly Pro Pro Ala Thr Ala Ala Leu Leu Val Gln Asp Lys Ala Ser
    210                 215                 220

Ile Ser Leu Ile Met Ile Val Ala Ser Leu Leu Gly Cys Ala Glu Ile
225                 230                 235                 240

Tyr Leu Gly Leu Leu Ile Ser Trp His Ala Ser Thr Ala Ala Gly Ala
                245                 250                 255

Thr Ile Thr Leu Leu Ser Ala Ala Ile Phe Phe Ala Thr Leu Leu Thr
            260                 265                 270

Lys Ser Ala Ile Ser Arg Leu Asn Phe Thr Ala
        275                 280

<210> 589
<211> 1077
<212> DNA
<213> Corynebacterium glutamicum

<220>
<221> CDS
<222> (101)..(1054)
<223> RXN02443

<400> 589
caagcactgc cgcgggagcc actatcactt tgttaagtgc tgcgatattt tttgccacct 60

tattgacaaa gagtgccatt agtaggttaa acttcaccgc gtg ata ctg aaa gac   115
                                            Val Ile Leu Lys Asp
                                            1               5

att ttc aat aat ggg gag ctc ttt ggg gct tcc tcc gcg aaa aat ttc   163
Ile Phe Asn Asn Gly Glu Leu Phe Gly Ala Ser Ser Ala Lys Asn Phe
                10                  15                  20

cga aaa cta cta gct gtt cca gcc gtt gcc gcc tca cta gct ttt ggt   211
Arg Lys Leu Leu Ala Val Pro Ala Val Ala Ala Ser Leu Ala Phe Gly
            25                  30                  35

atc acc gcc tgt tcc gct gta gat gac acc cct gac att gtg gtc acc   259
Ile Thr Ala Cys Ser Ala Val Asp Asp Thr Pro Asp Ile Val Val Thr
        40                  45                  50

acc aac atc ctg ggt gat gtt gta agc cat atc gtg gga gat tcc gca   307
Thr Asn Ile Leu Gly Asp Val Val Ser His Ile Val Gly Asp Ser Ala
    55                  60                  65

gat gtc caa gta ctc atg aaa ccc aac gca gat cca cat tcc ttc gga   355
Asp Val Gln Val Leu Met Lys Pro Asn Ala Asp Pro His Ser Phe Gly
70                  75                  80                  85

gtc tca gca caa gac gcc gct gcc atg gaa cat gcc gat ctc atc gtg   403
Val Ser Ala Gln Asp Ala Ala Ala Met Glu His Ala Asp Leu Ile Val

                90                  95                  100

gcc aat gga cta gga ctt gaa gag ggc ctt caa tcc aat gtg gac aat 451
Ala Asn Gly Leu Gly Leu Glu Glu Gly Leu Gln Ser Asn Val Asp Asn
            105                 110                 115

gcc aaa agc caa ggg gtt ccc gtc ttg gaa gtc ggc gaa cac atc gat 499
Ala Lys Ser Gln Gly Val Pro Val Leu Glu Val Gly Glu His Ile Asp
        120                 125                 130

gtc att gac tac tcc ccc ggc gtt cca gat cct cac ttt tgg aca gac 547
Val Ile Asp Tyr Ser Pro Gly Val Pro Asp Pro His Phe Trp Thr Asp
    135                 140                 145

ccg gcg cgc atg atc gcc gcc acg gaa gtt ata gaa gct gaa ctg atc 595
Pro Ala Arg Met Ile Ala Ala Thr Glu Val Ile Glu Ala Glu Leu Ile
150                 155                 160                 165

aaa gaa ctc gat cct tcc ctg act gaa tcg atc aca caa tca gcc cag 643
Lys Glu Leu Asp Pro Ser Leu Thr Glu Ser Ile Thr Gln Ser Ala Gln
                170                 175                 180

cac tac cgt gag gaa ctt gtt gcc ctt gat gag gaa gtc acc gaa ttg 691
His Tyr Arg Glu Glu Leu Val Ala Leu Asp Glu Glu Val Thr Glu Leu
            185                 190                 195

ctc agc ggc gtg gcc cca gaa aac cgc aag ctg gta acc aat cac aat 739
Leu Ser Gly Val Ala Pro Glu Asn Arg Lys Leu Val Thr Asn His Asn
        200                 205                 210

gtt ttt gga tac ctg gcc agc cgg ttt aac tac acc gtc att gac acc 787
Val Phe Gly Tyr Leu Ala Ser Arg Phe Asn Tyr Thr Val Ile Asp Thr
    215                 220                 225

atc atc cca ggt gga agc aca ttg gcg gcg cct tca gca tct gac ctc 835
Ile Ile Pro Gly Gly Ser Thr Leu Ala Ala Pro Ser Ala Ser Asp Leu
230                 235                 240                 245

aat gac atc tcc acc gcc atc gaa gac aac aat gtt ccc gca atc ttc 883
Asn Asp Ile Ser Thr Ala Ile Glu Asp Asn Asn Val Pro Ala Ile Phe
                250                 255                 260

acc gat acc tca agc cca caa cgg tta gct gaa gtg ttg gcc agc aac 931
Thr Asp Thr Ser Ser Pro Gln Arg Leu Ala Glu Val Leu Ala Ser Asn
            265                 270                 275

gct ggc att gat gtt caa gtg gtg tcc att ttc acg gaa tca ctc acc 979
Ala Gly Ile Asp Val Gln Val Val Ser Ile Phe Thr Glu Ser Leu Thr
        280                 285                 290

gat gca gat ggt gaa gca ccc acc tac atc agc atg caa aaa atc aat 1027
Asp Ala Asp Gly Glu Ala Pro Thr Tyr Ile Ser Met Gln Lys Ile Asn
    295                 300                 305

gcc gag cgc att gca agc act ttg tcc taaacagtcc taaacagtct 1074
Ala Glu Arg Ile Ala Ser Thr Leu Ser
310                 315

taa 1077

<210> 590
<211> 318
<212> PRT
<213> Corynebacterium glutamicum

<400> 590
Val Ile Leu Lys Asp Ile Phe Asn Asn Gly Glu Leu Phe Gly Ala Ser
1               5                   10                  15

Ser Ala Lys Asn Phe Arg Lys Leu Leu Ala Val Pro Ala Val Ala Ala
            20                  25                  30

Ser Leu Ala Phe Gly Ile Thr Ala Cys Ser Ala Val Asp Asp Thr Pro
        35                  40                  45

Asp Ile Val Val Thr Thr Asn Ile Leu Gly Asp Val Val Ser His Ile
    50                  55                  60

Val Gly Asp Ser Ala Asp Val Gln Val Leu Met Lys Pro Asn Ala Asp
65                  70                  75                  80

Pro His Ser Phe Gly Val Ser Ala Gln Asp Ala Ala Ala Met Glu His
                85                  90                  95

Ala Asp Leu Ile Val Ala Asn Gly Leu Gly Leu Glu Glu Gly Leu Gln
            100                 105                 110

Ser Asn Val Asp Asn Ala Lys Ser Gln Gly Val Pro Val Leu Glu Val
        115                 120                 125

Gly Glu His Ile Asp Val Ile Asp Tyr Ser Pro Gly Val Pro Asp Pro
    130                 135                 140

His Phe Trp Thr Asp Pro Ala Arg Met Ile Ala Ala Thr Glu Val Ile
145                 150                 155                 160

Glu Ala Glu Leu Ile Lys Glu Leu Asp Pro Ser Leu Thr Glu Ser Ile
                165                 170                 175

Thr Gln Ser Ala Gln His Tyr Arg Glu Glu Leu Val Ala Leu Asp Glu
            180                 185                 190

Glu Val Thr Glu Leu Leu Ser Gly Val Ala Pro Glu Asn Arg Lys Leu
        195                 200                 205

Val Thr Asn His Asn Val Phe Gly Tyr Leu Ala Ser Arg Phe Asn Tyr
    210                 215                 220

Thr Val Ile Asp Thr Ile Ile Pro Gly Gly Ser Thr Leu Ala Ala Pro
225                 230                 235                 240

Ser Ala Ser Asp Leu Asn Asp Ile Ser Thr Ala Ile Glu Asp Asn Asn
                245                 250                 255

Val Pro Ala Ile Phe Thr Asp Thr Ser Ser Pro Gln Arg Leu Ala Glu
            260                 265                 270

Val Leu Ala Ser Asn Ala Gly Ile Asp Val Gln Val Val Ser Ile Phe
        275                 280                 285

Thr Glu Ser Leu Thr Asp Ala Asp Gly Glu Ala Pro Thr Tyr Ile Ser

290                 295                 300

Met Gln Lys Ile Asn Ala Glu Arg Ile Ala Ser Thr Leu Ser
305                 310                 315

<210> 591
<211> 1323
<212> DNA
<213> Corynebacterium glutamicum

<220>
<221> CDS
<222> (101)..(1300)
<223> RXN00842

<400> 591
ccttgtcgcg gaggtaagcg agggtatttc tggatgtgga acaacgcgga ttatggaaaa 60

tcgtgacttt cataacgttg agcctactaa ggtttgttcc atg att att caa atc   115
                                           Met Ile Ile Gln Ile
                                           1               5

cta aga gtg gca ttt gcc ttc gtc ggc atc att gtt ggc gcc ggt ttc   163
Leu Arg Val Ala Phe Ala Phe Val Gly Ile Ile Val Gly Ala Gly Phe
                10                  15                  20

gca tca ggg caa gag gtc atg caa tat ttt gtg gcc ttc ggc ata gac   211
Ala Ser Gly Gln Glu Val Met Gln Tyr Phe Val Ala Phe Gly Ile Asp
            25                  30                  35

gga att tgg gga gtc att gtt tct gca gtg atc atg tcg gtg atg gcg   259
Gly Ile Trp Gly Val Ile Val Ser Ala Val Ile Met Ser Val Met Ala
        40                  45                  50

ttg atc att ttg cag ctc gga agc tat ttc aat gca ggt gaa cac ggt   307
Leu Ile Ile Leu Gln Leu Gly Ser Tyr Phe Asn Ala Gly Glu His Gly
    55                  60                  65

gaa gtg ttc cgc cga gta agt cac ccc gtt ttc tcc aaa att ttg gac   355
Glu Val Phe Arg Arg Val Ser His Pro Val Phe Ser Lys Ile Leu Asp
70                  75                  80                  85

atc ggc gtt gtg gtg acg ttg ttc tcc acc ggt ttc gtc atg ttt gca   403
Ile Gly Val Val Val Thr Leu Phe Ser Thr Gly Phe Val Met Phe Ala
                90                  95                  100

ggc gcg gga tca aat ctg aat cag cag tgg ggg ctt ccg ctc tgg atc   451
Gly Ala Gly Ser Asn Leu Asn Gln Gln Trp Gly Leu Pro Leu Trp Ile
            105                 110                 115

ggt tct gtg atc atg gtt ctt ctg gtg ctg gct gcg ggc atg ttg gac   499
Gly Ser Val Ile Met Val Leu Leu Val Leu Ala Ala Gly Met Leu Asp
        120                 125                 130

gtg gat aaa gta acc aca gtc att ggt gca att act ccg ttc atc atc   547
Val Asp Lys Val Thr Thr Val Ile Gly Ala Ile Thr Pro Phe Ile Ile
    135                 140                 145

att ttc atc act gcc gcc tcg atc tac acg ctg gta ggt aat ttc agc   595
Ile Phe Ile Thr Ala Ala Ser Ile Tyr Th[?] Leu Val Gly Asn Phe Ser

150                 155                 160                 165

tca gtg gag cag ctt gat tct gct gct tta gaa gtc ggc acg acg ttg     643
Ser Val Glu Gln Leu Asp Ser Ala Ala Leu Glu Val Gly Thr Thr Leu
                170                 175                 180

cct cac tgg gct gtt gca gcg gtg aac tat gtg gga ttc aac ctg atg     691
Pro His Trp Ala Val Ala Ala Val Asn Tyr Val Gly Phe Asn Leu Met
            185                 190                 195

gtt gcg gtg tcc atg gct gtg gtc att ggt gga tca atg ttt aac ccg     739
Val Ala Val Ser Met Ala Val Val Ile Gly Gly Ser Met Phe Asn Pro
        200                 205                 210

cgg gtc gca ggt cgg ggc ggt ttg ctg ggc gga ttg atc ctg gga ttc     787
Arg Val Ala Gly Arg Gly Gly Leu Leu Gly Gly Leu Ile Leu Gly Phe
    215                 220                 225

ttg atc atc atc agt gcg cta aca ctg ttc gcc acc gtg gaa gaa gtt     835
Leu Ile Ile Ile Ser Ala Leu Thr Leu Phe Ala Thr Val Glu Glu Val
230                 235                 240                 245

ggc caa gat gat atg cct atg ctg acg atc atc aac aat ttg aac ccg     883
Gly Gln Asp Asp Met Pro Met Leu Thr Ile Ile Asn Asn Leu Asn Pro
                250                 255                 260

ctg gct ggc caa gta atg gca gtg gtt atc tac gga atg atc ttc aac     931
Leu Ala Gly Gln Val Met Ala Val Val Ile Tyr Gly Met Ile Phe Asn
            265                 270                 275

acg gca ctg ggt atg ttc tac gca ttg ggc cgt cgt ctc act gcg aaa     979
Thr Ala Leu Gly Met Phe Tyr Ala Leu Gly Arg Arg Leu Thr Ala Lys
        280                 285                 290

aac cca cag cga ttc cgt ccg gtt tat gtg gtc aca gtg ctg att ggt     1027
Asn Pro Gln Arg Phe Arg Pro Val Tyr Val Val Thr Val Leu Ile Gly
    295                 300                 305

ttt gtg ttg agc ttt gtg gga ttc aag aac ttg gtg ggc tat gtg tac     1075
Phe Val Leu Ser Phe Val Gly Phe Lys Asn Leu Val Gly Tyr Val Tyr
310                 315                 320                 325

cca gtc ttg gga tac att ggc ctg ctg ctg att gca gtg atg atg gtg     1123
Pro Val Leu Gly Tyr Ile Gly Leu Leu Leu Ile Ala Val Met Met Val
                330                 335                 340

gcg tgg gtg agg gga cgc gta cgc atc tac aag gaa tcc gaa cgc cgc     1171
Ala Trp Val Arg Gly Arg Val Arg Ile Tyr Lys Glu Ser Glu Arg Arg
            345                 350                 355

atg cgg atc gca gac ttg ttg cag atc ggc cat gac gga gcg ttg agt     1219
Met Arg Ile Ala Asp Leu Leu Gln Ile Gly His Asp Gly Ala Leu Ser
        360                 365                 370

gga gca gag ctg gcg gtg ctc aac cag gaa atc caa gat tca aac ttg     1267
Gly Ala Glu Leu Ala Val Leu Asn Gln Glu Ile Gln Asp Ser Asn Leu
    375                 380                 385

gat gag gaa caa att aaa gca gcg gtt agg aag tagttactct gcagggacga 1320
Asp Glu Glu Gln Ile Lys Ala Ala Val Arg Lys
390                 395                 400

gct                                                                    1323

<210> 592
<211> 400
<212> PRT
<213> Corynebacterium glutamicum

<400> 592
Met Ile Ile Gln Ile Leu Arg Val Ala Phe Ala Phe Val Gly Ile Ile
1               5                   10                  15

Val Gly Ala Gly Phe Ala Ser Gly Gln Glu Val Met Gln Tyr Phe Val
            20                  25                  30

Ala Phe Gly Ile Asp Gly Ile Trp Gly Val Ile Val Ser Ala Val Ile
        35                  40                  45

Met Ser Val Met Ala Leu Ile Ile Leu Gln Leu Gly Ser Tyr Phe Asn
    50                  55                  60

Ala Gly Glu His Gly Glu Val Phe Arg Arg Val Ser His Pro Val Phe
65                  70                  75                  80

Ser Lys Ile Leu Asp Ile Gly Val Val Val Thr Leu Phe Ser Thr Gly
                85                  90                  95

Phe Val Met Phe Ala Gly Ala Gly Ser Asn Leu Asn Gln Gln Trp Gly
            100                 105                 110

Leu Pro Leu Trp Ile Gly Ser Val Ile Met Val Leu Leu Val Leu Ala
        115                 120                 125

Ala Gly Met Leu Asp Val Asp Lys Val Thr Thr Val Ile Gly Ala Ile
    130                 135                 140

Thr Pro Phe Ile Ile Ile Phe Ile Thr Ala Ala Ser Ile Tyr Thr Leu
145                 150                 155                 160

Val Gly Asn Phe Ser Ser Val Glu Gln Leu Asp Ser Ala Ala Leu Glu
                165                 170                 175

Val Gly Thr Thr Leu Pro His Trp Ala Val Ala Ala Val Asn Tyr Val
            180                 185                 190

Gly Phe Asn Leu Met Val Ala Val Ser Met Ala Val Val Ile Gly Gly
        195                 200                 205

Ser Met Phe Asn Pro Arg Val Ala Gly Arg Gly Gly Leu Leu Gly Gly
    210                 215                 220

Leu Ile Leu Gly Phe Leu Ile Ile Ile Ser Ala Leu Thr Leu Phe Ala
225                 230                 235                 240

Thr Val Glu Glu Val Gly Gln Asp Asp Met Pro Met Leu Thr Ile Ile
                245                 250                 255

Asn Asn Leu Asn Pro Leu Ala Gly Gln Val Met Ala Val Val Ile Tyr
            260                 265                 270

Gly Met Ile Phe Asn Thr Ala Leu Gly Met Phe Tyr Ala Leu Gly Arg
        275                 280                 285

Arg Leu Thr Ala Lys Asn Pro Gln Arg Phe Arg Pro Val Tyr Val Val
    290                 295                 300

Thr Val Leu Ile Gly Phe Val Leu Ser Phe Val Gly Phe Lys Asn Leu
305                 310                 315                 320

Val Gly Tyr Val Tyr Pro Val Leu Gly Tyr Ile Gly Leu Leu Leu Ile
                325                 330                 335

Ala Val Met Met Val Ala Trp Val Arg Gly Arg Val Arg Ile Tyr Lys
            340                 345                 350

Glu Ser Glu Arg Arg Met Arg Ile Ala Asp Leu Leu Gln Ile Gly His
        355                 360                 365

Asp Gly Ala Leu Ser Gly Ala Glu Leu Ala Val Leu Asn Gln Glu Ile
    370                 375                 380

Gln Asp Ser Asn Leu Asp Glu Glu Gln Ile Lys Ala Ala Val Arg Lys
385                 390                 395                 400

<210> 593
<211> 1323
<212> DNA
<213> Corynebacterium glutamicum

<220>
<221> CDS
<222> (101)..(1300)
<223> FRXA00842

<400> 593
ccttgtcgcg gaggtaagcg agggtatttc tggatgtgga acaacgcgga ttatggaaaa 60

tcgtgacttt cataacgttg agcctactaa ggtttgttcc atg att att caa atc   115
                                           Met Ile Ile Gln Ile
                                           1               5

cta aga gtg gca ttt gcc ttc gtc ggc atc att gtt ggc gcc ggt ttc   163
Leu Arg Val Ala Phe Ala Phe Val Gly Ile Ile Val Gly Ala Gly Phe
                10                  15                  20

gca tca ggg caa gag gtc atg caa tat ttt gtg gcc ttc ggc ata gac   211
Ala Ser Gly Gln Glu Val Met Gln Tyr Phe Val Ala Phe Gly Ile Asp
            25                  30                  35

gga att tgg gga gtc att gtt tct gca gtg atc atg tcg gtg atg gcg   259
Gly Ile Trp Gly Val Ile Val Ser Ala Val Ile Met Ser Val Met Ala
        40                  45                  50

ttg atc att ttg cag ctc gga agc tat ttc aat gca ggt gaa cac ggt   307
Leu Ile Ile Leu Gln Leu Gly Ser Tyr Phe Asn Ala Gly Glu His Gly
    55                  60                  65

gaa gtg ttc cgc cga gta agt cac ccc gtt ttc tcc aaa att ttg gac 355
Glu Val Phe Arg Arg Val Ser His Pro Val Phe Ser Lys Ile Leu Asp
70                  75                  80                  85

atc ggc gtt gtg gtg acg ttg ttc tcc acc ggt ttc gtc atg ttt gca 403
Ile Gly Val Val Val Thr Leu Phe Ser Thr Gly Phe Val Met Phe Ala
                90                  95                  100

ggc gcg gga tca aat ctg aat cag cag tgg ggg ctt ccg ctc tgg atc 451
Gly Ala Gly Ser Asn Leu Asn Gln Gln Trp Gly Leu Pro Leu Trp Ile
            105                 110                 115

ggt tct gtg atc atg gtt ctt ctg gtg ctg gct gcg ggc atg ttg gac 499
Gly Ser Val Ile Met Val Leu Leu Val Leu Ala Ala Gly Met Leu Asp
        120                 125                 130

gtg gat aaa gta acc aca gtc att ggt gca att act ccg ttc atc atc 547
Val Asp Lys Val Thr Thr Val Ile Gly Ala Ile Thr Pro Phe Ile Ile
    135                 140                 145

att ttc atc act gcc gcc tcg atc tac acg ctg gta ggt aat ttc agc 595
Ile Phe Ile Thr Ala Ala Ser Ile Tyr Thr Leu Val Gly Asn Phe Ser
150                 155                 160                 165

tca gtg gag cag ctt gat tct gct gct tta gaa gtc ggc acg acg ttg 643
Ser Val Glu Gln Leu Asp Ser Ala Ala Leu Glu Val Gly Thr Thr Leu
                170                 175                 180

cct cac tgg gct gtt gca gcg gtg aac tat gtg gga ttc aac ctg atg 691
Pro His Trp Ala Val Ala Ala Val Asn Tyr Val Gly Phe Asn Leu Met
            185                 190                 195

gtt gcg gtg tcc atg gct gtg gtc att ggt gga tca atg ttt aac ccg 739
Val Ala Val Ser Met Ala Val Val Ile Gly Gly Ser Met Phe Asn Pro
        200                 205                 210

cgg gtc gca ggt cgg ggc ggt ttg ctg ggc gga ttg atc ctg gga ttc 787
Arg Val Ala Gly Arg Gly Gly Leu Leu Gly Gly Leu Ile Leu Gly Phe
    215                 220                 225

ttg atc atc atc agt gcg cta aca ctg ttc gcc acc gtg gaa gaa gtt 835
Leu Ile Ile Ile Ser Ala Leu Thr Leu Phe Ala Thr Val Glu Glu Val
230                 235                 240                 245

ggc caa gat gat atg cct atg ctg acg atc atc aac aat ttg aac ccg 883
Gly Gln Asp Asp Met Pro Met Leu Thr Ile Ile Asn Asn Leu Asn Pro
                250                 255                 260

ctg gct ggc caa gta atg gca gtg gtt atc tac gga atg atc ttc aac 931
Leu Ala Gly Gln Val Met Ala Val Val Ile Tyr Gly Met Ile Phe Asn
            265                 270                 275

acg gca ctg ggt atg ttc tac gca ttg ggc cgt cgt ctc act gcg aaa 979
Thr Ala Leu Gly Met Phe Tyr Ala Leu Gly Arg Arg Leu Thr Ala Lys
        280                 285                 290

aac cca cag cga ttc cgt ccg gtt tat gtg gtc aca gtg ctg att ggt 1027
Asn Pro Gln Arg Phe Arg Pro Val Tyr Val Val Thr Val Leu Ile Gly
    295                 300                 305

ttt gtg ttg agc ttt gtg gga ttc aag aac ttg gtg ggc tat gtg tac 1075

Phe Val Leu Ser Phe Val Gly Phe Lys Asn Leu Val Gly Tyr Val Tyr
310                 315                 320                 325

cca gtc ttg gga tac att ggc ctg ctg ctg att gca gtg atg atg gtg     1123
Pro Val Leu Gly Tyr Ile Gly Leu Leu Leu Ile Ala Val Met Met Val
                330                 335                 340

gcg tgg gtg agg gga cgc gta cgc atc tac aag gaa tcc gaa cgc cgc     1171
Ala Trp Val Arg Gly Arg Val Arg Ile Tyr Lys Glu Ser Glu Arg Arg
            345                 350                 355

atg cgg atc gca gac ttg ttg cag atc ggc cat gac gga gcg ttg agt     1219
Met Arg Ile Ala Asp Leu Leu Gln Ile Gly His Asp Gly Ala Leu Ser
        360                 365                 370

gga gca gag ctg gcg gtg ctc aac cag gaa atc caa gat tca aac ttg     1267
Gly Ala Glu Leu Ala Val Leu Asn Gln Glu Ile Gln Asp Ser Asn Leu
    375                 380                 385

gat gag gaa caa att aaa gca gcg gtt agg aag tagttactct gcagggacga 1320
Asp Glu Glu Gln Ile Lys Ala Ala Val Arg Lys
390                 395                 400

gct                                                                 1323

<210> 594
<211> 400
<212> PRT
<213> Corynebacterium glutamicum

<400> 594
Met Ile Ile Gln Ile Leu Arg Val Ala Phe Ala Phe Val Gly Ile Ile
  1               5                  10                  15

Val Gly Ala Gly Phe Ala Ser Gly Gln Glu Val Met Gln Tyr Phe Val
             20                  25                  30

Ala Phe Gly Ile Asp Gly Ile Trp Gly Val Ile Val Ser Ala Val Ile
         35                  40                  45

Met Ser Val Met Ala Leu Ile Ile Leu Gln Leu Gly Ser Tyr Phe Asn
     50                  55                  60

Ala Gly Glu His Gly Glu Val Phe Arg Arg Val Ser His Pro Val Phe
 65                  70                  75                  80

Ser Lys Ile Leu Asp Ile Gly Val Val Val Thr Leu Phe Ser Thr Gly
                 85                  90                  95

Phe Val Met Phe Ala Gly Ala Gly Ser Asn Leu Asn Gln Gln Trp Gly
            100                 105                 110

Leu Pro Leu Trp Ile Gly Ser Val Ile Met Val Leu Leu Val Leu Ala
        115                 120                 125

Ala Gly Met Leu Asp Val Asp Lys Val Thr Thr Val Ile Gly Ala Ile
    130                 135                 140

Thr Pro Phe Ile Ile Ile Phe Ile Thr Ala Ala Ser Ile Tyr Thr Leu
145                 150                 155                 160

Val Gly Asn Phe Ser Ser Val Glu Gln Leu Asp Ser Ala Ala Leu Glu
                165                 170                 175

Val Gly Thr Thr Leu Pro His Trp Ala Val Ala Ala Val Asn Tyr Val
            180                 185                 190

Gly Phe Asn Leu Met Val Ala Val Ser Met Ala Val Val Ile Gly Gly
        195                 200                 205

Ser Met Phe Asn Pro Arg Val Ala Gly Arg Gly Gly Leu Leu Gly Gly
    210                 215                 220

Leu Ile Leu Gly Phe Leu Ile Ile Ile Ser Ala Leu Thr Leu Phe Ala
225                 230                 235                 240

Thr Val Glu Glu Val Gly Gln Asp Asp Met Pro Met Leu Thr Ile Ile
                245                 250                 255

Asn Asn Leu Asn Pro Leu Ala Gly Gln Val Met Ala Val Val Ile Tyr
            260                 265                 270

Gly Met Ile Phe Asn Thr Ala Leu Gly Met Phe Tyr Ala Leu Gly Arg
        275                 280                 285

Arg Leu Thr Ala Lys Asn Pro Gln Arg Phe Arg Pro Val Tyr Val Val
    290                 295                 300

Thr Val Leu Ile Gly Phe Val Leu Ser Phe Val Gly Phe Lys Asn Leu
305                 310                 315                 320

Val Gly Tyr Val Tyr Pro Val Leu Gly Tyr Ile Gly Leu Leu Leu Ile
                325                 330                 335

Ala Val Met Met Val Ala Trp Val Arg Gly Arg Val Arg Ile Tyr Lys
            340                 345                 350

Glu Ser Glu Arg Arg Met Arg Ile Ala Asp Leu Leu Gln Ile Gly His
        355                 360                 365

Asp Gly Ala Leu Ser Gly Ala Glu Leu Ala Val Leu Asn Gln Glu Ile
    370                 375                 380

Gln Asp Ser Asn Leu Asp Glu Glu Gln Ile Lys Ala Ala Val Arg Lys
385                 390                 395                 400

<210> 595
<211> 1173
<212> DNA
<213> Corynebacterium glutamicum

<220>
<221> CDS
<222> (101)..(1150)
<223> RXN00832

<400> 595

gagattgtgc taggttctga tgaggcttcg ggacgacccg aagaaatcta tgacagcctg 60

ggaacggccc agagttctta agaaagtttg actagagaac atg ccg ttt tct tgg 115
Met Pro Phe Ser Trp
1               5

cta aaa cca att gat tat gcc cgc atc ttt gtc ggc tgg gca tcg att 163
Leu Lys Pro Ile Asp Tyr Ala Arg Ile Phe Val Gly Trp Ala Ser Ile
                10                  15                  20

ttt atc atc ccc ctc atc aca ctg cca tca att att gag ttg gcg ctg 211
Phe Ile Ile Pro Leu Ile Thr Leu Pro Ser Ile Ile Glu Leu Ala Leu
            25                  30                  35

atc gtg gca gtc atc cta ttc tgc gca ttt ggc gtg gtg aag atg gcg 259
Ile Val Ala Val Ile Leu Phe Cys Ala Phe Gly Val Val Lys Met Ala
        40                  45                  50

gag cgt ttg gct cat att ttg ggt gat cct ttt gga tcg ttg atc ctt 307
Glu Arg Leu Ala His Ile Leu Gly Asp Pro Phe Gly Ser Leu Ile Leu
    55                  60                  65

acc ttg tcg atc gtg atc att gaa gtg att ttg atc tgt gcg gtg atg 355
Thr Leu Ser Ile Val Ile Ile Glu Val Ile Leu Ile Cys Ala Val Met
70                  75                  80                  85

ctg ggg cct gct gat tca acc act gct ggt cgg gat tcc gtg atg gca 403
Leu Gly Pro Ala Asp Ser Thr Thr Ala Gly Arg Asp Ser Val Met Ala
                90                  95                  100

gtg tcc atg atc atc atg ggt ttg gtc gtg gga ttg tgc cta ctc att 451
Val Ser Met Ile Ile Met Gly Leu Val Val Gly Leu Cys Leu Leu Ile
            105                 110                 115

ggt ggt tta agg cat gga agc atg cca cac aat ggg gtg gga act ccg 499
Gly Gly Leu Arg His Gly Ser Met Pro His Asn Gly Val Gly Thr Pro
        120                 125                 130

acc tac ttg gtg ctg atc gca act ttt tcc gta atc gcc ttt gcg gtt 547
Thr Tyr Leu Val Leu Ile Ala Thr Phe Ser Val Ile Ala Phe Ala Val
    135                 140                 145

cca gct ttc agg gga gaa tac tcc act ggg cag gca ctt gtt att tca 595
Pro Ala Phe Arg Gly Glu Tyr Ser Thr Gly Gln Ala Leu Val Ile Ser
150                 155                 160                 165

aca ctg aca gca gtg gtg tac ggg ttc ttc ctg ttt cgc caa atg ggt 643
Thr Leu Thr Ala Val Val Tyr Gly Phe Phe Leu Phe Arg Gln Met Gly
                170                 175                 180

gcc caa gct ggt gaa ttt caa gag gtc gag gtc gca gaa aag gca gac 691
Ala Gln Ala Gly Glu Phe Gln Glu Val Glu Val Ala Glu Lys Ala Asp
            185                 190                 195

gac gca gca aaa tgg gag gtc cca ttt aga ggc tta atc ttg att atc 739
Asp Ala Ala Lys Trp Glu Val Pro Phe Arg Gly Leu Ile Leu Ile Ile
        200                 205                 210

act gtg ctc ccc atc gtg ttg ctg tcc cat gac atg gcc acg gtg atg 787
Thr Val Leu Pro Ile Val Leu Leu Ser His Asp Met Ala Thr Val Met
    215                 220                 225

gat gaa gtc ctg gca agc ctt ggt gca ccc gta gca atg gct gga tta 835
Asp Glu Val Leu Ala Ser Leu Gly Ala Pro Val Ala Met Ala Gly Leu
230                 235                 240                 245

att att gcc acc att gtc ttc ttg cca gag acc atc acc tcc ttg aaa 883
Ile Ile Ala Thr Ile Val Phe Leu Pro Glu Thr Ile Thr Ser Leu Lys
                250                 255                 260

gct gcg tgg aca gga gag att cag cga gta agc aac ctc gcg cat gga 931
Ala Ala Trp Thr Gly Glu Ile Gln Arg Val Ser Asn Leu Ala His Gly
            265                 270                 275

gcc caa gta tca acg gtg ggg ctg aca atc cca gct gtt cta gtg atc 979
Ala Gln Val Ser Thr Val Gly Leu Thr Ile Pro Ala Val Leu Val Ile
        280                 285                 290

ggc gtg atc aca ggt caa gat gta gtt ttg ggg gag acc ccg atc aac 1027
Gly Val Ile Thr Gly Gln Asp Val Val Leu Gly Glu Thr Pro Ile Asn
    295                 300                 305

ttg ttg ctg ctg gga acc acc att gcg gtg aca gcc att gcg ttt agc 1075
Leu Leu Leu Leu Gly Thr Thr Ile Ala Val Thr Ala Ile Ala Phe Ser
310                 315                 320                 325

tcc aag aaa gtc agt gct gtg cat ggc tcg gtg ctg ctc atg ctt ttc 1123
Ser Lys Lys Val Ser Ala Val His Gly Ser Val Leu Leu Met Leu Phe
                330                 335                 340

ggt gtt tac atg atg agc atg ttc gcc tgatttaggt agcctggtgg 1170
Gly Val Tyr Met Met Ser Met Phe Ala
            345                 350

gaa 1173

<210> 596
<211> 350
<212> PRT
<213> Corynebacterium glutamicum

<400> 596
Met Pro Phe Ser Trp Leu Lys Pro Ile Asp Tyr Ala Arg Ile Phe Val
1               5                   10                  15

Gly Trp Ala Ser Ile Phe Ile Ile Pro Leu Ile Thr Leu Pro Ser Ile
            20                  25                  30

Ile Glu Leu Ala Leu Ile Val Ala Val Ile Leu Phe Cys Ala Phe Gly
        35                  40                  45

Val Val Lys Met Ala Glu Arg Leu Ala His Ile Leu Gly Asp Pro Phe
    50                  55                  60

Gly Ser Leu Ile Leu Thr Leu Ser Ile Val Ile Ile Glu Val Ile Leu
65                  70                  75                  80

Ile Cys Ala Val Met Leu Gly Pro Ala Asp Ser Thr Thr Ala Gly Arg
                85                  90                  95

Asp Ser Val Met Ala Val Ser Met Ile Ile Met Gly Leu Val Val Gly

100                 105                 110

Leu Cys Leu Leu Ile Gly Gly Leu Arg His Gly Ser Met Pro His Asn
        115                 120                 125

Gly Val Gly Thr Pro Thr Tyr Leu Val Leu Ile Ala Thr Phe Ser Val
    130                 135                 140

Ile Ala Phe Ala Val Pro Ala Phe Arg Gly Glu Tyr Ser Thr Gly Gln
145                 150                 155                 160

Ala Leu Val Ile Ser Thr Leu Thr Ala Val Val Tyr Gly Phe Phe Leu
                165                 170                 175

Phe Arg Gln Met Gly Ala Gln Ala Gly Glu Phe Gln Glu Val Glu Val
            180                 185                 190

Ala Glu Lys Ala Asp Asp Ala Ala Lys Trp Glu Val Pro Phe Arg Gly
        195                 200                 205

Leu Ile Leu Ile Ile Thr Val Leu Pro Ile Val Leu Leu Ser His Asp
    210                 215                 220

Met Ala Thr Val Met Asp Glu Val Leu Ala Ser Leu Gly Ala Pro Val
225                 230                 235                 240

Ala Met Ala Gly Leu Ile Ile Ala Thr Ile Val Phe Leu Pro Glu Thr
                245                 250                 255

Ile Thr Ser Leu Lys Ala Ala Trp Thr Gly Glu Ile Gln Arg Val Ser
            260                 265                 270

Asn Leu Ala His Gly Ala Gln Val Ser Thr Val Gly Leu Thr Ile Pro
        275                 280                 285

Ala Val Leu Val Ile Gly Val Ile Thr Gly Gln Asp Val Val Leu Gly
    290                 295                 300

Glu Thr Pro Ile Asn Leu Leu Leu Leu Gly Thr Thr Ile Ala Val Thr
305                 310                 315                 320

Ala Ile Ala Phe Ser Ser Lys Lys Val Ser Ala Val His Gly Ser Val
                325                 330                 335

Leu Leu Met Leu Phe Gly Val Tyr Met Met Ser Met Phe Ala
            340                 345                 350

<210> 597
<211> 1119
<212> DNA
<213> Corynebacterium glutamicum

<220>
<221> CDS
<222> (101)..(1096)
<223> RXN00466

<400> 597
tttaaaagcg cactaagagc tcgtcaattc tttaaaacaa gctgagaatg tgaataatag 60

gataggttaa cctgattcga ttagaaaacg gagatttgtc gtg caa tcc cgc ctg 115
Val Gln Ser Arg Leu
1               5

tcc aaa atc ctg cgc agt agc gtc gta ggc gtt gct gtc cta gcc ctg 163
Ser Lys Ile Leu Arg Ser Ser Val Val Gly Val Ala Val Leu Ala Leu
                10                  15                  20

tta gct ggg tgt tct aac aat gca gat gac acc gac gct gat tca aca 211
Leu Ala Gly Cys Ser Asn Asn Ala Asp Asp Thr Asp Ala Asp Ser Thr
            25                  30                  35

tcc acg gga aac tcc gct ttt cct gtt tcg att gaa cac gag ttc gga 259
Ser Thr Gly Asn Ser Ala Phe Pro Val Ser Ile Glu His Glu Phe Gly
        40                  45                  50

acc acc aca atc gat gat gta ccc gaa aga gtt gtc acc ctt ggc gtt 307
Thr Thr Thr Ile Asp Asp Val Pro Glu Arg Val Val Thr Leu Gly Val
    55                  60                  65

acc gac gcc gat att gtc ctc gca ttg ggg acc gtc cca gta ggc aac 355
Thr Asp Ala Asp Ile Val Leu Ala Leu Gly Thr Val Pro Val Gly Asn
70                  75                  80                  85

acc gga tac aaa ttc ttc gaa aac gga ttg gga ccg tgg act gat gag 403
Thr Gly Tyr Lys Phe Phe Glu Asn Gly Leu Gly Pro Trp Thr Asp Glu
                90                  95                  100

tta gtg gaa ggc aaa gaa tta aca ctg ctt gac tct gat tcc aca cca 451
Leu Val Glu Gly Lys Glu Leu Thr Leu Leu Asp Ser Asp Ser Thr Pro
            105                 110                 115

gat ctt gaa caa gta gca gcc ctg gag cca gac ctg att att gga gtc 499
Asp Leu Glu Gln Val Ala Ala Leu Glu Pro Asp Leu Ile Ile Gly Val
        120                 125                 130

tct gcg ggg ttt gac gac gtt gta tac gag caa cta tct gat atc gca 547
Ser Ala Gly Phe Asp Asp Val Val Tyr Glu Gln Leu Ser Asp Ile Ala
    135                 140                 145

ccg gtg gtc gcc cgt cca gcg gga aca gct gca tac gca gta gct cgc 595
Pro Val Val Ala Arg Pro Ala Gly Thr Ala Ala Tyr Ala Val Ala Arg
150                 155                 160                 165

gag gaa gct acc aac ctt gtt gcc cgt gcg atg ggg caa tca gaa aaa 643
Glu Glu Ala Thr Asn Leu Val Ala Arg Ala Met Gly Gln Ser Glu Lys
                170                 175                 180

gga caa gag ctc aat gag gaa aca gat gct ctg atc caa gct gcg cgt 691
Gly Gln Glu Leu Asn Glu Glu Thr Asp Ala Leu Ile Gln Ala Ala Arg
            185                 190                 195

gat gaa aat cct tct ttt gac ggt aaa aca gga acc gtc atc ttg cca 739
Asp Glu Asn Pro Ser Phe Asp Gly Lys Thr Gly Thr Val Ile Leu Pro
        200                 205                 210

tac cag ggt aaa tac ggt gcc tac ctg cca ggc gat gca cgg gga caa 787
Tyr Gln Gly Lys Tyr Gly Ala Tyr Leu Pro Gly Asp Ala Arg Gly Gln
    215                 220                 225

ttc ctc gat tca ctt ggc att tcg ctg ccg gaa gca gtt ctt tcg cga 835

Phe Leu Asp Ser Leu Gly Ile Ser Leu Pro Glu Ala Val Leu Ser Arg
230                 235                 240                 245

gac acc ggc gac agc ttc ttt gtc gat gtc ccc gct gaa agc gtc aaa     883
Asp Thr Gly Asp Ser Phe Phe Val Asp Val Pro Ala Glu Ser Val Lys
                250                 255                 260

gac gta gac ggt gat gtt ctc ctc gtg ctt tcc aac gac gaa aat ctg     931
Asp Val Asp Gly Asp Val Leu Leu Val Leu Ser Asn Asp Glu Asn Leu
            265                 270                 275

gat atc aca gca gag aat cca ctg ttt gaa aca ctc aac gtt gtg caa     979
Asp Ile Thr Ala Glu Asn Pro Leu Phe Glu Thr Leu Asn Val Val Gln
        280                 285                 290

aaa gac gca gta att gtg gca aca acc gaa gaa cgc ggg gcg att acc     1027
Lys Asp Ala Val Ile Val Ala Thr Thr Glu Glu Arg Gly Ala Ile Thr
    295                 300                 305

tac aac tca gtg ctg tct gtt cct ttt gcg ttg gaa cat ctc gca cca     1075
Tyr Asn Ser Val Leu Ser Val Pro Phe Ala Leu Glu His Leu Ala Pro
310                 315                 320                 325

cgt att gct gag gct ttg aag taaaactcaa ctactcgagc aca               1119
Arg Ile Ala Glu Ala Leu Lys
                330

<210> 598
<211> 332
<212> PRT
<213> Corynebacterium glutamicum

<400> 598
Val Gln Ser Arg Leu Ser Lys Ile Leu Arg Ser Ser Val Val Gly Val
1               5                   10                  15

Ala Val Leu Ala Leu Leu Ala Gly Cys Ser Asn Asn Ala Asp Asp Thr
            20                  25                  30

Asp Ala Asp Ser Thr Ser Thr Gly Asn Ser Ala Phe Pro Val Ser Ile
        35                  40                  45

Glu His Glu Phe Gly Thr Thr Thr Ile Asp Asp Val Pro Glu Arg Val
    50                  55                  60

Val Thr Leu Gly Val Thr Asp Ala Asp Ile Val Leu Ala Leu Gly Thr
65                  70                  75                  80

Val Pro Val Gly Asn Thr Gly Tyr Lys Phe Phe Glu Asn Gly Leu Gly
                85                  90                  95

Pro Trp Thr Asp Glu Leu Val Glu Gly Lys Glu Leu Thr Leu Leu Asp
            100                 105                 110

Ser Asp Ser Thr Pro Asp Leu Glu Gln Val Ala Ala Leu Glu Pro Asp
        115                 120                 125

Leu Ile Ile Gly Val Ser Ala Gly Phe Asp Asp Val Val Tyr Glu Gln
    130                 135                 140

Leu Ser Asp Ile Ala Pro Val Val Ala Arg Pro Ala Gly Thr Ala Ala
145                 150                 155                 160

Tyr Ala Val Ala Arg Glu Glu Ala Thr Asn Leu Val Ala Arg Ala Met
                165                 170                 175

Gly Gln Ser Glu Lys Gly Gln Glu Leu Asn Glu Glu Thr Asp Ala Leu
            180                 185                 190

Ile Gln Ala Ala Arg Asp Glu Asn Pro Ser Phe Asp Gly Lys Thr Gly
        195                 200                 205

Thr Val Ile Leu Pro Tyr Gln Gly Lys Tyr Gly Ala Tyr Leu Pro Gly
    210                 215                 220

Asp Ala Arg Gly Gln Phe Leu Asp Ser Leu Gly Ile Ser Leu Pro Glu
225                 230                 235                 240

Ala Val Leu Ser Arg Asp Thr Gly Asp Ser Phe Phe Val Asp Val Pro
                245                 250                 255

Ala Glu Ser Val Lys Asp Val Asp Gly Asp Val Leu Leu Val Leu Ser
            260                 265                 270

Asn Asp Glu Asn Leu Asp Ile Thr Ala Glu Asn Pro Leu Phe Glu Thr
        275                 280                 285

Leu Asn Val Val Gln Lys Asp Ala Val Ile Val Ala Thr Thr Glu Glu
    290                 295                 300

Arg Gly Ala Ile Thr Tyr Asn Ser Val Leu Ser Val Pro Phe Ala Leu
305                 310                 315                 320

Glu His Leu Ala Pro Arg Ile Ala Glu Ala Leu Lys
                325                 330


<210> 599
<211> 1395
<212> DNA
<213> Corynebacterium glutamicum

<220>
<221> CDS
<222> (101)..(1372)
<223> RXN01936

<400> 599
gcgcggtgac accacagccg ttgtcagcgg cgcttggtct gtggaggatc gccgaggtta 60

ctaacaaata ggcccaacaa agaggtctaa gctctacctg gtg agt ttc cga gat   115
                                           Val Ser Phe Arg Asp
                                           1               5

att ttc gct gac acc aga ccg ctg aaa gaa ccg gcc ttc aaa cgc ctc   163
Ile Phe Ala Asp Thr Arg Pro Leu Lys Glu Pro Ala Phe Lys Arg Leu
                10                  15                  20

tgg ctt ggc aat gtt gcc acc gtc att ggt gcc caa tta act gtt gtt   211
Trp Leu Gly Asn Val Ala Thr Val Ile Gly Ala Gln Leu Thr Val Val
            25                  30                  35

gcc gtt ccg gtg cag att tac caa atg act ggg tcc tcc ggc tat gtg 259
Ala Val Pro Val Gln Ile Tyr Gln Met Thr Gly Ser Ser Gly Tyr Val
        40                  45                  50

ggc ttg acc ggg ctt ttt ggc ctt att cct ttg gtt att ttt ggc ctt 307
Gly Leu Thr Gly Leu Phe Gly Leu Ile Pro Leu Val Ile Phe Gly Leu
    55                  60                  65

tat ggt gga tcc att gcg gat gct ttt gat aaa cgc atc gtg ctg atc 355
Tyr Gly Gly Ser Ile Ala Asp Ala Phe Asp Lys Arg Ile Val Leu Ile
70                  75                  80                  85

tgc acc acg atc ggc atg tgt gtc acc act gcc ggt ttt tgg gtg ctg 403
Cys Thr Thr Ile Gly Met Cys Val Thr Thr Ala Gly Phe Trp Val Leu
                90                  95                  100

acc att tta ggc aat gag aat att tgg ctc ctg tta ata aac ttt tct 451
Thr Ile Leu Gly Asn Glu Asn Ile Trp Leu Leu Leu Ile Asn Phe Ser
            105                 110                 115

tta cag cag gca ttt ttc gcg gtg aat caa ccc acc cga acg gcg atc 499
Leu Gln Gln Ala Phe Phe Ala Val Asn Gln Pro Thr Arg Thr Ala Ile
        120                 125                 130

ctt cga agt att ttg ccg att gat caa tta gcg tcg gca aca tca ctg 547
Leu Arg Ser Ile Leu Pro Ile Asp Gln Leu Ala Ser Ala Thr Ser Leu
    135                 140                 145

aat atg ctg ctc atg cag acc ggc gca atc gtt ggc ccg ctg atc gca 595
Asn Met Leu Leu Met Gln Thr Gly Ala Ile Val Gly Pro Leu Ile Ala
150                 155                 160                 165

ggt gcg ttg att ccg ctg atc ggt ttc ggg tgg ctg tat ttc ctt gat 643
Gly Ala Leu Ile Pro Leu Ile Gly Phe Gly Trp Leu Tyr Phe Leu Asp
                170                 175                 180

gtt gtc tcc atc atc ccc aca ctg tgg gct gta tgg tca ctg cct tcg 691
Val Val Ser Ile Ile Pro Thr Leu Trp Ala Val Trp Ser Leu Pro Ser
            185                 190                 195

atc aag cca tcc ggc aag gtg atg aag gct ggt ttc gcc agt gtg gtg 739
Ile Lys Pro Ser Gly Lys Val Met Lys Ala Gly Phe Ala Ser Val Val
        200                 205                 210

gat ggc ctg aag tat ttg gct ggc caa ccc gtg ttg ttg atg gtg atg 787
Asp Gly Leu Lys Tyr Leu Ala Gly Gln Pro Val Leu Leu Met Val Met
    215                 220                 225

gtg ctg gat ctt atc gcc atg att ttc ggc atg cca cgt gcg ctt tac 835
Val Leu Asp Leu Ile Ala Met Ile Phe Gly Met Pro Arg Ala Leu Tyr
230                 235                 240                 245

ccc gag atc gca gaa gtg aac ttc ggt ggg ggt gac gcc ggt gca acg 883
Pro Glu Ile Ala Glu Val Asn Phe Gly Gly Gly Asp Ala Gly Ala Thr
                250                 255                 260

atg ctg gcg ttc atg tac tca tcc atg gct gtt ggc gca gtt ctt ggc 931
Met Leu Ala Phe Met Tyr Ser Ser Met Ala Val Gly Ala Val Leu Gly
            265                 270                 275

ggc gtg ctg tct ggt tgg gtg gcc cgg att agc cgc cag ggt gtt gca 979
Gly Val Leu Ser Gly Trp Val Ala Arg Ile Ser Arg Gln Gly Val Ala
        280                 285                 290

gtt tat tgg tgc atc atc gcc tgg ggc gca gcc gtt gct ttg ggt ggt 1027
Val Tyr Trp Cys Ile Ile Ala Trp Gly Ala Ala Val Ala Leu Gly Gly
    295                 300                 305

gtg gca att gtt gtc agc ccc ggc gcg gtg act gcg tgg gcg tgg atg 1075
Val Ala Ile Val Val Ser Pro Gly Ala Val Thr Ala Trp Ala Trp Met
310                 315                 320                 325

ttc atc atc atg atg gtc att ggt ggc atg gct gac atg ttc agc tcg 1123
Phe Ile Ile Met Met Val Ile Gly Gly Met Ala Asp Met Phe Ser Ser
                330                 335                 340

gca gtt cga aac gct att ttg cag cag tct gct gcg gaa cat gtg cag 1171
Ala Val Arg Asn Ala Ile Leu Gln Gln Ser Ala Ala Glu His Val Gln
            345                 350                 355

ggc cga atc caa ggt gtg tgg atc atc gtc gtg gtg ggt gga cct cgt 1219
Gly Arg Ile Gln Gly Val Trp Ile Ile Val Val Val Gly Gly Pro Arg
        360                 365                 370

tta gct gac gtc ctt cac ggt tgg gcc gct gag ccc ctc ggc gca ggt 1267
Leu Ala Asp Val Leu His Gly Trp Ala Ala Glu Pro Leu Gly Ala Gly
    375                 380                 385

tgg acg gta tta tgg ggc gga gta gcg gtg gtt gta ctc act gca att 1315
Trp Thr Val Leu Trp Gly Gly Val Ala Val Val Val Leu Thr Ala Ile
390                 395                 400                 405

tgt atg gtg gcg gtg cct aaa ttc tgg aaa tac gag aaa cca aaa att 1363
Cys Met Val Ala Val Pro Lys Phe Trp Lys Tyr Glu Lys Pro Lys Ile
                410                 415                 420

acc ggc atc taaatactta tccatgccca ttt 1395
Thr Gly Ile

<210> 600
<211> 424
<212> PRT
<213> Corynebacterium glutamicum

<400> 600
Val Ser Phe Arg Asp Ile Phe Ala Asp Thr Arg Pro Leu Lys Glu Pro
1               5                   10                  15

Ala Phe Lys Arg Leu Trp Leu Gly Asn Val Ala Thr Val Ile Gly Ala
            20                  25                  30

Gln Leu Thr Val Val Ala Val Pro Val Gln Ile Tyr Gln Met Thr Gly
        35                  40                  45

Ser Ser Gly Tyr Val Gly Leu Thr Gly Leu Phe Gly Leu Ile Pro Leu
    50                  55                  60

Val Ile Phe Gly Leu Tyr Gly Gly Ser Ile Ala Asp Ala Phe Asp Lys
65                  70                  75                  80

Arg Ile Val Leu Ile Cys Thr Thr Ile Gly Met Cys Val Thr Thr Ala
                85                  90                  95

Gly Phe Trp Val Leu Thr Ile Leu Gly Asn Glu Asn Ile Trp Leu Leu
            100                 105                 110

Leu Ile Asn Phe Ser Leu Gln Gln Ala Phe Phe Ala Val Asn Gln Pro
        115                 120                 125

Thr Arg Thr Ala Ile Leu Arg Ser Ile Leu Pro Ile Asp Gln Leu Ala
    130                 135                 140

Ser Ala Thr Ser Leu Asn Met Leu Leu Met Gln Thr Gly Ala Ile Val
145                 150                 155                 160

Gly Pro Leu Ile Ala Gly Ala Leu Ile Pro Leu Ile Gly Phe Gly Trp
                165                 170                 175

Leu Tyr Phe Leu Asp Val Val Ser Ile Ile Pro Thr Leu Trp Ala Val
            180                 185                 190

Trp Ser Leu Pro Ser Ile Lys Pro Ser Gly Lys Val Met Lys Ala Gly
        195                 200                 205

Phe Ala Ser Val Val Asp Gly Leu Lys Tyr Leu Ala Gly Gln Pro Val
    210                 215                 220

Leu Leu Met Val Met Val Leu Asp Leu Ile Ala Met Ile Phe Gly Met
225                 230                 235                 240

Pro Arg Ala Leu Tyr Pro Glu Ile Ala Glu Val Asn Phe Gly Gly Gly
                245                 250                 255

Asp Ala Gly Ala Thr Met Leu Ala Phe Met Tyr Ser Ser Met Ala Val
            260                 265                 270

Gly Ala Val Leu Gly Gly Val Leu Ser Gly Trp Val Ala Arg Ile Ser
        275                 280                 285

Arg Gln Gly Val Ala Val Tyr Trp Cys Ile Ile Ala Trp Gly Ala Ala
    290                 295                 300

Val Ala Leu Gly Gly Val Ala Ile Val Val Ser Pro Gly Ala Val Thr
305                 310                 315                 320

Ala Trp Ala Trp Met Phe Ile Ile Met Met Val Ile Gly Gly Met Ala
                325                 330                 335

Asp Met Phe Ser Ser Ala Val Arg Asn Ala Ile Leu Gln Gln Ser Ala
            340                 345                 350

Ala Glu His Val Gln Gly Arg Ile Gln Gly Val Trp Ile Ile Val Val
        355                 360                 365

Val Gly Gly Pro Arg Leu Ala Asp Val Leu His Gly Trp Ala Ala Glu
    370                 375                 380

Pro Leu Gly Ala Gly Trp Thr Val Leu Trp Gly Gly Val Ala Val Val
385                 390                 395                 400

Val Leu Thr Ala Ile Cys Met Val Ala Val Pro Lys Phe Trp Lys Tyr
                405                 410                 415

Glu Lys Pro Lys Ile Thr Gly Ile
            420

<210> 601
<211> 1461
<212> DNA
<213> Corynebacterium glutamicum

<220>
<221> CDS
<222> (101)..(1438)
<223> RXN01995

<400> 601
ccgacgcaaa ggcatgcgcc tgcgtgtctc gagtagtctc ctccccttcc tcgtccccaa 60

cctcgaccat tacggtcgcc ctctcctaaa ggagcctggc atg gat atc cgc caa   115
                                           Met Asp Ile Arg Gln
                                           1               5

aca att aac gac aca gca atg tcg aga tat cag tgg ttc att gta ttt   163
Thr Ile Asn Asp Thr Ala Met Ser Arg Tyr Gln Trp Phe Ile Val Phe
                10                  15                  20

atc gca gtg ctg ctc aac gca ctg gac ggc ttt gat gtc ctc gcc atg   211
Ile Ala Val Leu Leu Asn Ala Leu Asp Gly Phe Asp Val Leu Ala Met
            25                  30                  35

tct ttt act gcg aat gca gtg acc gaa gaa ttt gga ctg agt ggc agc   259
Ser Phe Thr Ala Asn Ala Val Thr Glu Glu Phe Gly Leu Ser Gly Ser
        40                  45                  50

cag ctt ggt gtg ctg ctg agt tcc gcg ctg ttc ggc atg acc gct gga   307
Gln Leu Gly Val Leu Leu Ser Ser Ala Leu Phe Gly Met Thr Ala Gly
    55                  60                  65

tct ttg ctg ttc ggt ccg atc ggt gac cgt ttc ggc cgt aag aat gcc   355
Ser Leu Leu Phe Gly Pro Ile Gly Asp Arg Phe Gly Arg Lys Asn Ala
70                  75                  80                  85

ctg atg atc gcg ctg ctg ttc aac gtg gtg gga ttg gta ttg tcc gcc   403
Leu Met Ile Ala Leu Leu Phe Asn Val Val Gly Leu Val Leu Ser Ala
                90                  95                  100

acc gcg cag tcc gca ggc cag ttg ggc gtg tgg cgt ttg atc act ggt   451
Thr Ala Gln Ser Ala Gly Gln Leu Gly Val Trp Arg Leu Ile Thr Gly
            105                 110                 115

atc ggc atc ggc gga atc ctc gcc tgc atc aca gtg gtg atc agt gag   499
Ile Gly Ile Gly Gly Ile Leu Ala Cys Ile Thr Val Val Ile Ser Glu
        120                 125                 130

ttc tcc aac aac aaa aac cgc ggc atg gcc atg tcc atc tac gct gct   547
Phe Ser Asn Asn Lys Asn Arg Gly Met Ala Met Ser Ile Tyr Ala Ala
    135                 140                 145

ggt tac ggc atc ggc gcg tcc ttg ggc ggt ttc ggc gca gcg cag ctc   595

Gly Tyr Gly Ile Gly Ala Ser Leu Gly Gly Phe Gly Ala Ala Gln Leu
150                 155                 160                 165

atc cca aca ttt gga tgg cgc tcc gtg ttc gca gcc ggt gcg atc gca     643
Ile Pro Thr Phe Gly Trp Arg Ser Val Phe Ala Ala Gly Ala Ile Ala
                170                 175                 180

act ggt atc gcc acc atc gct act ttc ttc ttc ctg cca gaa tcc gtt     691
Thr Gly Ile Ala Thr Ile Ala Thr Phe Phe Phe Leu Pro Glu Ser Val
            185                 190                 195

gat tgg ctg agc act cgc cgc cct gcg ggc gct cgc gac aag atc aat     739
Asp Trp Leu Ser Thr Arg Arg Pro Ala Gly Ala Arg Asp Lys Ile Asn
        200                 205                 210

tac att gcg cgc cgc ctg ggc aaa gtc ggt acc ttt gag ctt cca ggc     787
Tyr Ile Ala Arg Arg Leu Gly Lys Val Gly Thr Phe Glu Leu Pro Gly
    215                 220                 225

gaa caa agc ttg tcg acg aaa aaa gcc ggt ctc caa tcg tat gca gtg     835
Glu Gln Ser Leu Ser Thr Lys Lys Ala Gly Leu Gln Ser Tyr Ala Val
230                 235                 240                 245

ctc gtt aac aaa gag aac cgt gga acc agc atc aag ctg tgg gtt gcg     883
Leu Val Asn Lys Glu Asn Arg Gly Thr Ser Ile Lys Leu Trp Val Ala
                250                 255                 260

ttc ggc atc gtg atg ttc ggc ttc tac ttc gcc aac act tgg acc ccg     931
Phe Gly Ile Val Met Phe Gly Phe Tyr Phe Ala Asn Thr Trp Thr Pro
            265                 270                 275

aag ctg ctc gtg gaa acc gga atg tca gaa cag cag ggc atc atc ggt     979
Lys Leu Leu Val Glu Thr Gly Met Ser Glu Gln Gln Gly Ile Ile Gly
        280                 285                 290

ggt ttg atg ttg tcc atg ggt gga gca ttc ggc tcc ctg ctc tac ggt     1027
Gly Leu Met Leu Ser Met Gly Gly Ala Phe Gly Ser Leu Leu Tyr Gly
    295                 300                 305

ttc ctc acc acc aag ttc agc tcc cga aac aca ctg atg acc ttc atg     1075
Phe Leu Thr Thr Lys Phe Ser Ser Arg Asn Thr Leu Met Thr Phe Met
310                 315                 320                 325

gtg ctg tcc ggc ctg acg ctg atc ctg ttc att tcc tcc acc tct gtt     1123
Val Leu Ser Gly Leu Thr Leu Ile Leu Phe Ile Ser Ser Thr Ser Val
                330                 335                 340

cca tcc atc gcg ttt gcc agc ggc gtt gtc gtg ggc atg ctg atc aat     1171
Pro Ser Ile Ala Phe Ala Ser Gly Val Val Val Gly Met Leu Ile Asn
            345                 350                 355

ggt tgt gtg gct ggt ctg tac acc ctg tcc cca cag ctg tac tcc gct     1219
Gly Cys Val Ala Gly Leu Tyr Thr Leu Ser Pro Gln Leu Tyr Ser Ala
        360                 365                 370

gaa gta cgc acc act ggt gtg ggc gct gcg att ggt atg ggt cgt gtc     1267
Glu Val Arg Thr Thr Gly Val Gly Ala Ala Ile Gly Met Gly Arg Val
    375                 380                 385

ggt gcg att tcc gcg cca ctg ctg gtg ggt ggc ctg ctg gat tct ggc     1315
Gly Ala Ile Ser Ala Pro Leu Leu Val Gly Gly Leu Leu Asp Ser Gly

390                 395                 400                 405

tgg tcc cca acg cag ctg tat gtt ggt gtg gca gtg att gtt att gcc     1363
Trp Ser Pro Thr Gln Leu Tyr Val Gly Val Ala Val Ile Val Ile Ala
                410                 415                 420

ggt gca acc gca ttg att ggg atg cgc act cag gcg gta gcc gtc gaa     1411
Gly Ala Thr Ala Leu Ile Gly Met Arg Thr Gln Ala Val Ala Val Glu
            425                 430                 435

aag cag cct gaa gcc cta gcg acc aaa tagggccgcg attcctagca           1458
Lys Gln Pro Glu Ala Leu Ala Thr Lys
        440                 445

tgc                                                                 1461


<210> 602
<211> 446
<212> PRT
<213> Corynebacterium glutamicum

<400> 602
Met Asp Ile Arg Gln Thr Ile Asn Asp Thr Ala Met Ser Arg Tyr Gln
  1               5                  10                  15

Trp Phe Ile Val Phe Ile Ala Val Leu Leu Asn Ala Leu Asp Gly Phe
             20                  25                  30

Asp Val Leu Ala Met Ser Phe Thr Ala Asn Ala Val Thr Glu Glu Phe
         35                  40                  45

Gly Leu Ser Gly Ser Gln Leu Gly Val Leu Leu Ser Ser Ala Leu Phe
     50                  55                  60

Gly Met Thr Ala Gly Ser Leu Leu Phe Gly Pro Ile Gly Asp Arg Phe
 65                  70                  75                  80

Gly Arg Lys Asn Ala Leu Met Ile Ala Leu Leu Phe Asn Val Val Gly
                 85                  90                  95

Leu Val Leu Ser Ala Thr Ala Gln Ser Ala Gly Gln Leu Gly Val Trp
            100                 105                 110

Arg Leu Ile Thr Gly Ile Gly Ile Gly Gly Ile Leu Ala Cys Ile Thr
        115                 120                 125

Val Val Ile Ser Glu Phe Ser Asn Asn Lys Asn Arg Gly Met Ala Met
    130                 135                 140

Ser Ile Tyr Ala Ala Gly Tyr Gly Ile Gly Ala Ser Leu Gly Gly Phe
145                 150                 155                 160

Gly Ala Ala Gln Leu Ile Pro Thr Phe Gly Trp Arg Ser Val Phe Ala
                165                 170                 175

Ala Gly Ala Ile Ala Thr Gly Ile Ala Thr Ile Ala Thr Phe Phe Phe
            180                 185                 190

Leu Pro Glu Ser Val Asp Trp Leu Ser Thr Arg Arg Pro Ala Gly Ala
        195                 200                 205

Arg Asp Lys Ile Asn Tyr Ile Ala Arg Arg Leu Gly Lys Val Gly Thr
    210                 215                 220

Phe Glu Leu Pro Gly Glu Gln Ser Leu Ser Thr Lys Lys Ala Gly Leu
225                 230                 235                 240

Gln Ser Tyr Ala Val Leu Val Asn Lys Glu Asn Arg Gly Thr Ser Ile
                245                 250                 255

Lys Leu Trp Val Ala Phe Gly Ile Val Met Phe Gly Phe Tyr Phe Ala
            260                 265                 270

Asn Thr Trp Thr Pro Lys Leu Leu Val Glu Thr Gly Met Ser Glu Gln
        275                 280                 285

Gln Gly Ile Ile Gly Gly Leu Met Leu Ser Met Gly Gly Ala Phe Gly
    290                 295                 300

Ser Leu Leu Tyr Gly Phe Leu Thr Thr Lys Phe Ser Ser Arg Asn Thr
305                 310                 315                 320

Leu Met Thr Phe Met Val Leu Ser Gly Leu Thr Leu Ile Leu Phe Ile
                325                 330                 335

Ser Ser Thr Ser Val Pro Ser Ile Ala Phe Ala Ser Gly Val Val Val
            340                 345                 350

Gly Met Leu Ile Asn Gly Cys Val Ala Gly Leu Tyr Thr Leu Ser Pro
        355                 360                 365

Gln Leu Tyr Ser Ala Glu Val Arg Thr Thr Gly Val Gly Ala Ala Ile
    370                 375                 380

Gly Met Gly Arg Val Gly Ala Ile Ser Ala Pro Leu Leu Val Gly Gly
385                 390                 395                 400

Leu Leu Asp Ser Gly Trp Ser Pro Thr Gln Leu Tyr Val Gly Val Ala
                405                 410                 415

Val Ile Val Ile Ala Gly Ala Thr Ala Leu Ile Gly Met Arg Thr Gln
            420                 425                 430

Ala Val Ala Val Glu Lys Gln Pro Glu Ala Leu Ala Thr Lys
        435                 440                 445

<210> 603
<211> 813
<212> DNA
<213> Corynebacterium glutamicum

<220>
<221> CDS
<222> (101)..(790)
<223> RXN00661

<400> 603
cggatgcaag agaaccgtgg tttcgctgat ttttggcgaa cccggaatta aggccccgag 60

gattacatgc ttttaaatcc tttgaaaagg ggacaagatc atg aat cct ata acc   115

Met Asn Pro Ile Thr
1               5

gaa tta tta gac gca aca cta tgg atc ggc gga gtt ccg att ctg tgg     163
Glu Leu Leu Asp Ala Thr Leu Trp Ile Gly Gly Val Pro Ile Leu Trp
            10                  15                  20

cgc gaa atc atc ggc aac gtt ttc gga tta ttt agc gcg tgg gca gga     211
Arg Glu Ile Ile Gly Asn Val Phe Gly Leu Phe Ser Ala Trp Ala Gly
        25                  30                  35

atg cga cgc atc gtg tgg gca tgg ccc atc ggc atc ata ggc aac gcg     259
Met Arg Arg Ile Val Trp Ala Trp Pro Ile Gly Ile Ile Gly Asn Ala
    40                  45                  50

ctg ctg ttc aca gta ttt atg ggc ggc ctt ttc cac act cca caa aac     307
Leu Leu Phe Thr Val Phe Met Gly Gly Leu Phe His Thr Pro Gln Asn
    55                  60                  65

ctc gat ctc tac ggc caa gcg ggt cgc cag atc atg ttc atc atc gtc     355
Leu Asp Leu Tyr Gly Gln Ala Gly Arg Gln Ile Met Phe Ile Ile Val
70                  75                  80                  85

agt ggt tat ggc tgg tac caa tgg tcg gcc gca aaa cgt cgc gca ctc     403
Ser Gly Tyr Gly Trp Tyr Gln Trp Ser Ala Ala Lys Arg Arg Ala Leu
            90                  95                  100

acc cca gaa aat gca gta gca gtg gtt cct cgc tgg gca agc acc aaa     451
Thr Pro Glu Asn Ala Val Ala Val Val Pro Arg Trp Ala Ser Thr Lys
            105                 110                 115

gaa cgc gcc ggc att gtg att gcg gcg gtt gtg gga aca ctc agc ttt     499
Glu Arg Ala Gly Ile Val Ile Ala Ala Val Val Gly Thr Leu Ser Phe
        120                 125                 130

gcc tgg att ttc caa gca ctc ggc tcc tgg ggg cca tgg gcc gac gcg     547
Ala Trp Ile Phe Gln Ala Leu Gly Ser Trp Gly Pro Trp Ala Asp Ala
    135                 140                 145

tgg att ttc gtc ggc tca atc ctg gct acc tac gga atg gct cgc gga     595
Trp Ile Phe Val Gly Ser Ile Leu Ala Thr Tyr Gly Met Ala Arg Gly
150                 155                 160                 165

tgg aca gag ttc tgg ctg atc tgg atc gcc gtc gac ata gtt ggc gtt     643
Trp Thr Glu Phe Trp Leu Ile Trp Ile Ala Val Asp Ile Val Gly Val
            170                 175                 180

cct cta ctt ttg act gct ggc tac tac cca tcc gcg gtg ctt tac ctg     691
Pro Leu Leu Leu Thr Ala Gly Tyr Tyr Pro Ser Ala Val Leu Tyr Leu
            185                 190                 195

gtg tac ggt gcg ttt gtc agc tgg gga ttt gtc gtg tgg ctg cgg gtg     739
Val Tyr Gly Ala Phe Val Ser Trp Gly Phe Val Val Trp Leu Arg Val
        200                 205                 210

caa aaa gca gac aag gct cgt gcg ctg gaa gct cag gag tct gtg aca     787
Gln Lys Ala Asp Lys Ala Arg Ala Leu Glu Ala Gln Glu Ser Val Thr
    215                 220                 225

gtc tgaaaagcgt ttactaaata gaa                                       813
Val

230

<210> 604
<211> 230
<212> PRT
<213> Corynebacterium glutamicum

<400> 604
Met Asn Pro Ile Thr Glu Leu Leu Asp Ala Thr Leu Trp Ile Gly Gly
1               5                   10                  15

Val Pro Ile Leu Trp Arg Glu Ile Ile Gly Asn Val Phe Gly Leu Phe
            20                  25                  30

Ser Ala Trp Ala Gly Met Arg Arg Ile Val Trp Ala Trp Pro Ile Gly
        35                  40                  45

Ile Ile Gly Asn Ala Leu Leu Phe Thr Val Phe Met Gly Gly Leu Phe
    50                  55                  60

His Thr Pro Gln Asn Leu Asp Leu Tyr Gly Gln Ala Gly Arg Gln Ile
65                  70                  75                  80

Met Phe Ile Ile Val Ser Gly Tyr Gly Trp Tyr Gln Trp Ser Ala Ala
                85                  90                  95

Lys Arg Arg Ala Leu Thr Pro Glu Asn Ala Val Ala Val Val Pro Arg
            100                 105                 110

Trp Ala Ser Thr Lys Glu Arg Ala Gly Ile Val Ile Ala Ala Val Val
        115                 120                 125

Gly Thr Leu Ser Phe Ala Trp Ile Phe Gln Ala Leu Gly Ser Trp Gly
    130                 135                 140

Pro Trp Ala Asp Ala Trp Ile Phe Val Gly Ser Ile Leu Ala Thr Tyr
145                 150                 155                 160

Gly Met Ala Arg Gly Trp Thr Glu Phe Trp Leu Ile Trp Ile Ala Val
                165                 170                 175

Asp Ile Val Gly Val Pro Leu Leu Leu Thr Ala Gly Tyr Tyr Pro Ser
            180                 185                 190

Ala Val Leu Tyr Leu Val Tyr Gly Ala Phe Val Ser Trp Gly Phe Val
        195                 200                 205

Val Trp Leu Arg Val Gln Lys Ala Asp Lys Ala Arg Ala Leu Glu Ala
    210                 215                 220

Gln Glu Ser Val Thr Val
225                 230


<210> 605
<211> 1332
<212> DNA
<213> Corynebacterium glutamicum

<220>

<221> CDS
<222> (101)..(1309)
<223> RXN02566

<400> 605
gactccggat gatcaagcag cgaaaccttt gtcaagaaca tagctcccca ccctagacaa 60

aagcccaaat aaatctgctc aataacctaa cctaaagtcc atg cac gcc tcg tcg 115
Met His Ala Ser Ser
1               5

ccc caa cct cac cct cag cgc acc cgt gtt ctc agc ggc ctg att ttc 163
Pro Gln Pro His Pro Gln Arg Thr Arg Val Leu Ser Gly Leu Ile Phe
                10                  15                  20

gcc caa atc atg gtt ggt gca tcc aat ggc gtg acg cta tcg atg gga 211
Ala Gln Ile Met Val Gly Ala Ser Asn Gly Val Thr Leu Ser Met Gly
            25                  30                  35

agt ttg ctg gca gca cac ttg gcg gga gct tcg tgg gga gga tca gcc 259
Ser Leu Leu Ala Ala His Leu Ala Gly Ala Ser Trp Gly Gly Ser Ala
        40                  45                  50

gcc aca ttg acc acg atc ggc gca gct atc ttt tcg att ccc ctt gcc 307
Ala Thr Leu Thr Thr Ile Gly Ala Ala Ile Phe Ser Ile Pro Leu Ala
    55                  60                  65

cgc atg gtc tcc aca tac gat cgc cga act tca ctc agc acg ggc atg 355
Arg Met Val Ser Thr Tyr Asp Arg Arg Thr Ser Leu Ser Thr Gly Met
70                  75                  80                  85

ttg ctt ggt tgc gtg ggc gca cta ctg gcg atc ctc ggc gca caa ttc 403
Leu Leu Gly Cys Val Gly Ala Leu Leu Ala Ile Leu Gly Ala Gln Phe
                90                  95                  100

ggc ttg ttt cca gta gta ctt ttg gca ttt ttg ttc ctc gga tcc atg 451
Gly Leu Phe Pro Val Val Leu Leu Ala Phe Leu Phe Leu Gly Ser Met
            105                 110                 115

tcg gcg gtt aac ctc caa gca cgt ttc gcc gca acc gac gtg gcc agt 499
Ser Ala Val Asn Leu Gln Ala Arg Phe Ala Ala Thr Asp Val Ala Ser
        120                 125                 130

gaa gaa acc cgc ggc cgc gac ctc tcg atc gtt gtg tgg tcc acc acc 547
Glu Glu Thr Arg Gly Arg Asp Leu Ser Ile Val Val Trp Ser Thr Thr
    135                 140                 145

atc ggc gca atc gcc gga cca aat tta ttt gaa cca agc gcc cga ttc 595
Ile Gly Ala Ile Ala Gly Pro Asn Leu Phe Glu Pro Ser Ala Arg Phe
150                 155                 160                 165

agc gaa acc ctg ggc ctc gaa caa cat gcc ggc gca tac ctg ctg tgt 643
Ser Glu Thr Leu Gly Leu Glu Gln His Ala Gly Ala Tyr Leu Leu Cys
                170                 175                 180

tta ttt ggc cag ctc atc gcc atc gca gtc tgg cga ttc acc ctc ccc 691
Leu Phe Gly Gln Leu Ile Ala Ile Ala Val Trp Arg Phe Thr Leu Pro
            185                 190                 195

aaa ggc ctc aaa ccc gaa gcc acc cca aat gca cca aca gaa aag aag 739
Lys Gly Leu Lys Pro Glu Ala Thr Pro Asn Ala Pro Thr Glu Lys Lys

200                 205                 210

cgc ctc acc ccg aaa gcc ctc caa gcc atc aca tcg gtt gca acc gca     787
Arg Leu Thr Pro Lys Ala Leu Gln Ala Ile Thr Ser Val Ala Thr Ala
    215                 220                 225

cac ttc tcc atg gtc ggt ctc atg tcc atg gcc gcc atc cac atg caa     835
His Phe Ser Met Val Gly Leu Met Ser Met Ala Ala Ile His Met Gln
230                 235                 240                 245

ggc cac ggc gcc agc ctc acc atc atc ggc ttc acc atc agt ttg cac     883
Gly His Gly Ala Ser Leu Thr Ile Ile Gly Phe Thr Ile Ser Leu His
                250                 255                 260

gtc gcc gga atg tac gca ctc tca cca gtg ttc ggc ctg ctc aca gac     931
Val Ala Gly Met Tyr Ala Leu Ser Pro Val Phe Gly Leu Leu Thr Asp
            265                 270                 275

aaa ctc ggc cgc aat gtc acc atc tat tcc ggc ttc gcc atg ctc gcc     979
Lys Leu Gly Arg Asn Val Thr Ile Tyr Ser Gly Phe Ala Met Leu Ala
        280                 285                 290

aca tcc gca gca ttt ctt atc att tgg ccc gaa cca cag tgg gcc atg     1027
Thr Ser Ala Ala Phe Leu Ile Ile Trp Pro Glu Pro Gln Trp Ala Met
    295                 300                 305

atc aca tcc atg atc ctg ctt ggg ctc ggc tgg aac tct gcc ctc gtc     1075
Ile Thr Ser Met Ile Leu Leu Gly Leu Gly Trp Asn Ser Ala Leu Val
310                 315                 320                 325

ggt tct tca aca ttg ctt gtc gac gcc acc ccc atc cac cac cgc acc     1123
Gly Ser Ser Thr Leu Leu Val Asp Ala Thr Pro Ile His His Arg Thr
                330                 335                 340

tac gcc cag ggg cgc agc gac cta acg atg aat ctt gcg gga gct tca     1171
Tyr Ala Gln Gly Arg Ser Asp Leu Thr Met Asn Leu Ala Gly Ala Ser
            345                 350                 355

ggc ggg ttg atc gcc gga ccg tta att gcc atg ggc gga atg ccc ttg     1219
Gly Gly Leu Ile Ala Gly Pro Leu Ile Ala Met Gly Gly Met Pro Leu
        360                 365                 370

ttg gca ggc gtc gtt ctt gca gtt gtg gcg ctt caa acg gtg ctt agt     1267
Leu Ala Gly Val Val Leu Ala Val Val Ala Leu Gln Thr Val Leu Ser
    375                 380                 385

ttc aga acc cgt tca att gaa aag act cct gct tca tgt ttt             1309
Phe Arg Thr Arg Ser Ile Glu Lys Thr Pro Ala Ser Cys Phe
390                 395                 400

tagcctagga attcacgcac gac                                           1332


<210> 606
<211> 403
<212> PRT
<213> Corynebacterium glutamicum

<400> 606
Met His Ala Ser Ser Pro Gln Pro His Pro Gln Arg Thr Arg Val Leu
  1               5                  10                  15

Ser Gly Leu Ile Phe Ala Gln Ile Met Val Gly Ala Ser Asn Gly Val
            20                  25                  30

Thr Leu Ser Met Gly Ser Leu Leu Ala Ala His Leu Ala Gly Ala Ser
        35                  40                  45

Trp Gly Gly Ser Ala Ala Thr Leu Thr Thr Ile Gly Ala Ala Ile Phe
    50                  55                  60

Ser Ile Pro Leu Ala Arg Met Val Ser Thr Tyr Asp Arg Arg Thr Ser
65                  70                  75                  80

Leu Ser Thr Gly Met Leu Leu Gly Cys Val Gly Ala Leu Leu Ala Ile
                85                  90                  95

Leu Gly Ala Gln Phe Gly Leu Phe Pro Val Val Leu Leu Ala Phe Leu
            100                 105                 110

Phe Leu Gly Ser Met Ser Ala Val Asn Leu Gln Ala Arg Phe Ala Ala
        115                 120                 125

Thr Asp Val Ala Ser Glu Glu Thr Arg Gly Arg Asp Leu Ser Ile Val
    130                 135                 140

Val Trp Ser Thr Thr Ile Gly Ala Ile Ala Gly Pro Asn Leu Phe Glu
145                 150                 155                 160

Pro Ser Ala Arg Phe Ser Glu Thr Leu Gly Leu Glu Gln His Ala Gly
                165                 170                 175

Ala Tyr Leu Leu Cys Leu Phe Gly Gln Leu Ile Ala Ile Ala Val Trp
            180                 185                 190

Arg Phe Thr Leu Pro Lys Gly Leu Lys Pro Glu Ala Thr Pro Asn Ala
        195                 200                 205

Pro Thr Glu Lys Lys Arg Leu Thr Pro Lys Ala Leu Gln Ala Ile Thr
    210                 215                 220

Ser Val Ala Thr Ala His Phe Ser Met Val Gly Leu Met Ser Met Ala
225                 230                 235                 240

Ala Ile His Met Gln Gly His Gly Ala Ser Leu Thr Ile Ile Gly Phe
                245                 250                 255

Thr Ile Ser Leu His Val Ala Gly Met Tyr Ala Leu Ser Pro Val Phe
            260                 265                 270

Gly Leu Leu Thr Asp Lys Leu Gly Arg Asn Val Thr Ile Tyr Ser Gly
        275                 280                 285

Phe Ala Met Leu Ala Thr Ser Ala Ala Phe Leu Ile Ile Trp Pro Glu
    290                 295                 300

Pro Gln Trp Ala Met Ile Thr Ser Met Ile Leu Leu Gly Leu Gly Trp
305                 310                 315                 320

Asn Ser Ala Leu Val Gly Ser Ser Thr Leu Leu Val Asp Ala Thr Pro
                325                 330                 335

Ile His His Arg Thr Tyr Ala Gln Gly Arg Ser Asp Leu Thr Met Asn
            340                 345                 350

Leu Ala Gly Ala Ser Gly Gly Leu Ile Ala Gly Pro Leu Ile Ala Met
        355                 360                 365

Gly Gly Met Pro Leu Leu Ala Gly Val Val Leu Ala Val Val Ala Leu
    370                 375                 380

Gln Thr Val Leu Ser Phe Arg Thr Arg Ser Ile Glu Lys Thr Pro Ala
385                 390                 395                 400

Ser Cys Phe

<210> 607
<211> 760
<212> DNA
<213> Corynebacterium glutamicum

<220>
<221> CDS
<222> (101)..(760)
<223> FRXA02561

<400> 607
atctgctcaa taacctaacc taaagtccat gcacgcctcg tcgccccaac ctcaccctca 60

gcgcacccgt gttctcagcg gcctgatttt cgcccaaatc atg gtt ggt gca tcc   115
                                           Met Val Gly Ala Ser
                                           1               5

aat ggc gtg acg cta tcg atg gga agt ttg ctg gca gca cac ttg gcg   163
Asn Gly Val Thr Leu Ser Met Gly Ser Leu Leu Ala Ala His Leu Ala
                10                  15                  20

gga gct tcg tgg gga gga tca gcc gcc aca ttg acc acg atc ggc gca   211
Gly Ala Ser Trp Gly Gly Ser Ala Ala Thr Leu Thr Thr Ile Gly Ala
            25                  30                  35

gct atc ttt tcg att ccc ctt gcc cgc atg gtc tcc aca tac gat cgc   259
Ala Ile Phe Ser Ile Pro Leu Ala Arg Met Val Ser Thr Tyr Asp Arg
        40                  45                  50

cga act tca ctc agc acg ggc atg ttg ctt ggt tgc gtg ggc gca cta   307
Arg Thr Ser Leu Ser Thr Gly Met Leu Leu Gly Cys Val Gly Ala Leu
    55                  60                  65

ctg gcg atc ctc ggc gca caa ttc ggc ttg ttt cca gta gta ctt ttg   355
Leu Ala Ile Leu Gly Ala Gln Phe Gly Leu Phe Pro Val Val Leu Leu
70                  75                  80                  85

gca ttt ttg ttc ctc gga tcc atg tcg gcg gtt aac ctc caa gca cgt   403
Ala Phe Leu Phe Leu Gly Ser Met Ser Ala Val Asn Leu Gln Ala Arg
                90                  95                  100

ttc gcc gca acc gac gtg gcc agt gaa gaa acc cgc ggc cgc gac ctc   451
Phe Ala Ala Thr Asp Val Ala Ser Glu Glu Thr Arg Gly Arg Asp Leu
            105                 110                 115

tcg atc gtt gtg tgg tcc acc acc atc ggc gca atc gcc gga cca aat     499
Ser Ile Val Val Trp Ser Thr Thr Ile Gly Ala Ile Ala Gly Pro Asn
        120                 125                 130

tta ttt gaa cca agc gcc cga ttc agc gaa acc ctg ggc ctc gaa caa     547
Leu Phe Glu Pro Ser Ala Arg Phe Ser Glu Thr Leu Gly Leu Glu Gln
    135                 140                 145

cat gcc ggc gca tac ctg ctg tgt tta ttt ggc cag ctc atc gcc atc     595
His Ala Gly Ala Tyr Leu Leu Cys Leu Phe Gly Gln Leu Ile Ala Ile
150                 155                 160                 165

gca gtc tgg cga ttc acc ctc ccc aaa ggc ctc aaa ccc gaa gcc acc     643
Ala Val Trp Arg Phe Thr Leu Pro Lys Gly Leu Lys Pro Glu Ala Thr
                170                 175                 180

cca aat gca cca aca gaa aag aag cgc ctc acc ccg aaa gcc ctc caa     691
Pro Asn Ala Pro Thr Glu Lys Lys Arg Leu Thr Pro Lys Ala Leu Gln
            185                 190                 195

gcc atc aca tcg gtt gca acc gca cac ttc tcc atg gtc ggt ctc atg     739
Ala Ile Thr Ser Val Ala Thr Ala His Phe Ser Met Val Gly Leu Met
        200                 205                 210

tcc atg gcc gcc atc cac atg                                         760
Ser Met Ala Ala Ile His Met
    215                 220


<210> 608
<211> 220
<212> PRT
<213> Corynebacterium glutamicum

<400> 608
Met Val Gly Ala Ser Asn Gly Val Thr Leu Ser Met Gly Ser Leu Leu
  1               5                  10                  15

Ala Ala His Leu Ala Gly Ala Ser Trp Gly Gly Ser Ala Ala Thr Leu
             20                  25                  30

Thr Thr Ile Gly Ala Ala Ile Phe Ser Ile Pro Leu Ala Arg Met Val
         35                  40                  45

Ser Thr Tyr Asp Arg Arg Thr Ser Leu Ser Thr Gly Met Leu Leu Gly
     50                  55                  60

Cys Val Gly Ala Leu Leu Ala Ile Leu Gly Ala Gln Phe Gly Leu Phe
 65                  70                  75                  80

Pro Val Val Leu Leu Ala Phe Leu Phe Leu Gly Ser Met Ser Ala Val
                 85                  90                  95

Asn Leu Gln Ala Arg Phe Ala Ala Thr Asp Val Ala Ser Glu Glu Thr
            100                 105                 110

Arg Gly Arg Asp Leu Ser Ile Val Val Trp Ser Thr Thr Ile Gly Ala
        115                 120                 125

Ile Ala Gly Pro Asn Leu Phe Glu Pro Ser Ala Arg Phe Ser Glu Thr
    130                 135                 140

Leu Gly Leu Glu Gln His Ala Gly Ala Tyr Leu Leu Cys Leu Phe Gly
145                 150                 155                 160

Gln Leu Ile Ala Ile Ala Val Trp Arg Phe Thr Leu Pro Lys Gly Leu
                165                 170                 175

Lys Pro Glu Ala Thr Pro Asn Ala Pro Thr Glu Lys Lys Arg Leu Thr
            180                 185                 190

Pro Lys Ala Leu Gln Ala Ile Thr Ser Val Ala Thr Ala His Phe Ser
        195                 200                 205

Met Val Gly Leu Met Ser Met Ala Ala Ile His Met
    210                 215                 220

<210> 609
<211> 461
<212> DNA
<213> Corynebacterium glutamicum

<220>
<221> CDS
<222> (1)..(438)
<223> FRXA02566

<400> 609
atc agt ttg cac gtc gcc gga atg tac gca ctc tca cca gtg ttc ggc      48
Ile Ser Leu His Val Ala Gly Met Tyr Ala Leu Ser Pro Val Phe Gly
  1               5                  10                  15

ctg ctc aca gac aaa ctc ggc cgc aat gtc acc atc tat tcc ggc ttc      96
Leu Leu Thr Asp Lys Leu Gly Arg Asn Val Thr Ile Tyr Ser Gly Phe
             20                  25                  30

gcc atg ctc gcc aca tcc gca gca ttt ctt atc att tgg ccc gaa cca     144
Ala Met Leu Ala Thr Ser Ala Ala Phe Leu Ile Ile Trp Pro Glu Pro
         35                  40                  45

cag tgg gcc atg atc aca tcc atg atc ctg ctt ggg ctc ggc tgg aac     192
Gln Trp Ala Met Ile Thr Ser Met Ile Leu Leu Gly Leu Gly Trp Asn
     50                  55                  60

tct gcc ctc gtc ggt tct tca aca ttg ctt gtc gac gcc acc ccc atc     240
Ser Ala Leu Val Gly Ser Ser Thr Leu Leu Val Asp Ala Thr Pro Ile
 65                  70                  75                  80

cac cac cgc acc tac gcc cag ggg cgc agc gac cta acg atg aat ctt     288
His His Arg Thr Tyr Ala Gln Gly Arg Ser Asp Leu Thr Met Asn Leu
                 85                  90                  95

gcg gga gct tca ggc ggg ttg atc gcc gga ccg tta att gcc atg ggc     336
Ala Gly Ala Ser Gly Gly Leu Ile Ala Gly Pro Leu Ile Ala Met Gly
            100                 105                 110

gga atg ccc ttg ttg gca ggc gtc gtt ctt gca gtt gtg gcg ctt caa     384
Gly Met Pro Leu Leu Ala Gly Val Val Leu Ala Val Val Ala Leu Gln
        115                 120                 125

acg gtg ctt agt ttc aga acc cgt tca att gaa aag act cct gct tca     432

Thr Val Leu Ser Phe Arg Thr Arg Ser Ile Glu Lys Thr Pro Ala Ser
    130                 135                 140

tgt ttt tagcctagga attcacgcac gac                                461
Cys Phe
145

<210> 610
<211> 146
<212> PRT
<213> Corynebacterium glutamicum

<400> 610
Ile Ser Leu His Val Ala Gly Met Tyr Ala Leu Ser Pro Val Phe Gly
  1               5                  10                  15

Leu Leu Thr Asp Lys Leu Gly Arg Asn Val Thr Ile Tyr Ser Gly Phe
             20                  25                  30

Ala Met Leu Ala Thr Ser Ala Ala Phe Leu Ile Ile Trp Pro Glu Pro
         35                  40                  45

Gln Trp Ala Met Ile Thr Ser Met Ile Leu Leu Gly Leu Gly Trp Asn
     50                  55                  60

Ser Ala Leu Val Gly Ser Ser Thr Leu Leu Val Asp Ala Thr Pro Ile
 65                  70                  75                  80

His His Arg Thr Tyr Ala Gln Gly Arg Ser Asp Leu Thr Met Asn Leu
                 85                  90                  95

Ala Gly Ala Ser Gly Gly Leu Ile Ala Gly Pro Leu Ile Ala Met Gly
            100                 105                 110

Gly Met Pro Leu Leu Ala Gly Val Val Leu Ala Val Val Ala Leu Gln
        115                 120                 125

Thr Val Leu Ser Phe Arg Thr Arg Ser Ile Glu Lys Thr Pro Ala Ser
    130                 135                 140

Cys Phe
145

<210> 611
<211> 1527
<212> DNA
<213> Corynebacterium glutamicum

<220>
<221> CDS
<222> (101)..(1504)
<223> RXA00051

<400> 611
cagtaaaagt gcaccgacac taagaatcct tatccttcag agtaaggtga tctgcgtcaa 60

aaaatgtccg ctattcaccc gaaacgaggt cagcatctcg atg aat gcc tcc cct   115
                                            Met Asn Ala Ser Pro
                                              1               5

gcc cca acc cga tct ttt aaa gga ttg cgg gct cga cac att cac ttc 163
Ala Pro Thr Arg Ser Phe Lys Gly Leu Arg Ala Arg His Ile His Phe
                10                  15                  20

atc gcg ctg ggt tcc gcg atc ggc acc ggc ttg ttc tac ggt tcc gct 211
Ile Ala Leu Gly Ser Ala Ile Gly Thr Gly Leu Phe Tyr Gly Ser Ala
            25                  30                  35

ggc gca atc caa gca gct ggt cca tct gta ctc ttg gtc tac ctt ctc 259
Gly Ala Ile Gln Ala Ala Gly Pro Ser Val Leu Leu Val Tyr Leu Leu
        40                  45                  50

ggt ggc gcc gtc gtg tac ttc atg ctg cgc gca ctc ggc gag atg gct 307
Gly Gly Ala Val Val Tyr Phe Met Leu Arg Ala Leu Gly Glu Met Ala
    55                  60                  65

gtg cat cac cca gtc cgt ggt tcc ttc gcg gtc tac acc cgc gca cac 355
Val His His Pro Val Arg Gly Ser Phe Ala Val Tyr Thr Arg Ala His
70                  75                  80                  85

ctt ggc gga tgg gca ggc tac atc acc ggc tgg atg ttc gcg ttt gag 403
Leu Gly Gly Trp Ala Gly Tyr Ile Thr Gly Trp Met Phe Ala Phe Glu
                90                  95                  100

atg ctc atc gtc tgc ctg gct gac ctc aca gcc atc ggc atc tat atg 451
Met Leu Ile Val Cys Leu Ala Asp Leu Thr Ala Ile Gly Ile Tyr Met
            105                 110                 115

aac ttc tgg ttc ccc ggc acc cca caa tgg act tgg gtg gta gcc acc 499
Asn Phe Trp Phe Pro Gly Thr Pro Gln Trp Thr Trp Val Val Ala Thr
        120                 125                 130

ctt ctt att gtc ggt ggc gca aac ctc gca tca gtg cgt tgg ttc ggt 547
Leu Leu Ile Val Gly Gly Ala Asn Leu Ala Ser Val Arg Trp Phe Gly
    135                 140                 145

gag ctc gag ttc atc ttc acc atc att aag gtc acc gca gtt gtc gcc 595
Glu Leu Glu Phe Ile Phe Thr Ile Ile Lys Val Thr Ala Val Val Ala
150                 155                 160                 165

atg atc gtc ggc ggc gca gcc atc ctc gca ttc ggt ctc ggc gcc aac 643
Met Ile Val Gly Gly Ala Ala Ile Leu Ala Phe Gly Leu Gly Ala Asn
                170                 175                 180

gct gaa gtt gcc ggc gta tcc aac ctc tgg gag cac ggc gga ttc ttc 691
Ala Glu Val Ala Gly Val Ser Asn Leu Trp Glu His Gly Gly Phe Phe
            185                 190                 195

ccc aac ggt gtt gaa ggc atg atc gca gcc ttc atc ctt gtt ctc ttc 739
Pro Asn Gly Val Glu Gly Met Ile Ala Ala Phe Ile Leu Val Leu Phe
        200                 205                 210

gca ttc ggt ggc acc gaa atc atc ggt gtt gca ggc tct gaa gct gaa 787
Ala Phe Gly Gly Thr Glu Ile Ile Gly Val Ala Gly Ser Glu Ala Glu
    215                 220                 225

gat cct gag aag tcc atc ccc aag gct gtt aat act gtc cca gta cgc 835
Asp Pro Glu Lys Ser Ile Pro Lys Ala Val Asn Thr Val Pro Val Arg
230                 235                 240                 245

atc ctc ctc ttc tat gtg ggt gcc atc ctg gtg atc ctt gcc ctt aat     883
Ile Leu Leu Phe Tyr Val Gly Ala Ile Leu Val Ile Leu Ala Leu Asn
                250                 255                 260

cct tgg cct tcc atc acc ggc gaa gaa tcc cca ttc gtc cag atc ttc     931
Pro Trp Pro Ser Ile Thr Gly Glu Glu Ser Pro Phe Val Gln Ile Phe
            265                 270                 275

gac acc ctc ggc gtc aac tgg gct gct ggt ctc ctc aac gcc gtg gtc     979
Asp Thr Leu Gly Val Asn Trp Ala Ala Gly Leu Leu Asn Ala Val Val
        280                 285                 290

atc acc gct gca ctg tct gcc atc aac gct gac ctc ttc ggc gct ggc     1027
Ile Thr Ala Ala Leu Ser Ala Ile Asn Ala Asp Leu Phe Gly Ala Gly
    295                 300                 305

cgc gtt ctc act ggt ctt gcg aag gaa aac ctc gca cca aag gcc atg     1075
Arg Val Leu Thr Gly Leu Ala Lys Glu Asn Leu Ala Pro Lys Ala Met
310                 315                 320                 325

ggc aag atc gcc aag aac ggc gtt cca gtc atg acc acc acc atc atg     1123
Gly Lys Ile Ala Lys Asn Gly Val Pro Val Met Thr Thr Thr Ile Met
                330                 335                 340

atc atc gtc ttg atc gtg gga gta atc ctc aac gca gtg ctt ccc gag     1171
Ile Ile Val Leu Ile Val Gly Val Ile Leu Asn Ala Val Leu Pro Glu
            345                 350                 355

cgc gtc ttc gag atc gtc gct tcc cta gca acc ttc gcc aca gtt tac     1219
Arg Val Phe Glu Ile Val Ala Ser Leu Ala Thr Phe Ala Thr Val Tyr
        360                 365                 370

gtc tgg ctg atg atc ctg ctc gca cag gtg gga tcc cgc cga aac atg     1267
Val Trp Leu Met Ile Leu Leu Ala Gln Val Gly Ser Arg Arg Asn Met
    375                 380                 385

cct gcc gac gag gtc aag tcc ctg aag ttc cct gtc ccc ttc tac ccc     1315
Pro Ala Asp Glu Val Lys Ser Leu Lys Phe Pro Val Pro Phe Tyr Pro
390                 395                 400                 405

ttc gga caa tac ttc gcg atc cta ttt atc gcc ttc acc ttc ggc atc     1363
Phe Gly Gln Tyr Phe Ala Ile Leu Phe Ile Ala Phe Thr Phe Gly Ile
                410                 415                 420

atg gtc tgg tac gac aac tac cac ctg cca ctc gcc gtc ggc gtt gga     1411
Met Val Trp Tyr Asp Asn Tyr His Leu Pro Leu Ala Val Gly Val Gly
            425                 430                 435

ttc ctt gtc ctg atg aca atc ctt tac tac gcc aca ggc cga cca aag     1459
Phe Leu Val Leu Met Thr Ile Leu Tyr Tyr Ala Thr Gly Arg Pro Lys
        440                 445                 450

gcg atc gct ccg atc gat tat gaa gag cta gat ccg cga cgc gat         1504
Ala Ile Ala Pro Ile Asp Tyr Glu Glu Leu Asp Pro Arg Arg Asp
    455                 460                 465

taatctagac tcgcacgaaa aag                                           1527

<210> 612
<211> 468

<212> PRT
<213> Corynebacterium glutamicum

<400> 612
Met Asn Ala Ser Pro Ala Pro Thr Arg Ser Phe Lys Gly Leu Arg Ala
1               5                   10                  15

Arg His Ile His Phe Ile Ala Leu Gly Ser Ala Ile Gly Thr Gly Leu
            20                  25                  30

Phe Tyr Gly Ser Ala Gly Ala Ile Gln Ala Ala Gly Pro Ser Val Leu
        35                  40                  45

Leu Val Tyr Leu Leu Gly Gly Ala Val Val Tyr Phe Met Leu Arg Ala
    50                  55                  60

Leu Gly Glu Met Ala Val His His Pro Val Arg Gly Ser Phe Ala Val
65                  70                  75                  80

Tyr Thr Arg Ala His Leu Gly Gly Trp Ala Gly Tyr Ile Thr Gly Trp
                85                  90                  95

Met Phe Ala Phe Glu Met Leu Ile Val Cys Leu Ala Asp Leu Thr Ala
            100                 105                 110

Ile Gly Ile Tyr Met Asn Phe Trp Phe Pro Gly Thr Pro Gln Trp Thr
        115                 120                 125

Trp Val Val Ala Thr Leu Leu Ile Val Gly Gly Ala Asn Leu Ala Ser
    130                 135                 140

Val Arg Trp Phe Gly Glu Leu Glu Phe Ile Phe Thr Ile Ile Lys Val
145                 150                 155                 160

Thr Ala Val Val Ala Met Ile Val Gly Gly Ala Ala Ile Leu Ala Phe
                165                 170                 175

Gly Leu Gly Ala Asn Ala Glu Val Ala Gly Val Ser Asn Leu Trp Glu
            180                 185                 190

His Gly Gly Phe Phe Pro Asn Gly Val Glu Gly Met Ile Ala Ala Phe
        195                 200                 205

Ile Leu Val Leu Phe Ala Phe Gly Gly Thr Glu Ile Ile Gly Val Ala
    210                 215                 220

Gly Ser Glu Ala Glu Asp Pro Glu Lys Ser Ile Pro Lys Ala Val Asn
225                 230                 235                 240

Thr Val Pro Val Arg Ile Leu Leu Phe Tyr Val Gly Ala Ile Leu Val
                245                 250                 255

Ile Leu Ala Leu Asn Pro Trp Pro Ser Ile Thr Gly Glu Glu Ser Pro
            260                 265                 270

Phe Val Gln Ile Phe Asp Thr Leu Gly Val Asn Trp Ala Ala Gly Leu
        275                 280                 285

Leu Asn Ala Val Val Ile Thr Ala Ala Leu Ser Ala Ile Asn Ala Asp
    290                 295                 300

Leu Phe Gly Ala Gly Arg Val Leu Thr Gly Leu Ala Lys Glu Asn Leu
305                 310                 315                 320

Ala Pro Lys Ala Met Gly Lys Ile Ala Lys Asn Gly Val Pro Val Met
                325                 330                 335

Thr Thr Thr Ile Met Ile Ile Val Leu Ile Val Gly Val Ile Leu Asn
            340                 345                 350

Ala Val Leu Pro Glu Arg Val Phe Glu Ile Val Ala Ser Leu Ala Thr
        355                 360                 365

Phe Ala Thr Val Tyr Val Trp Leu Met Ile Leu Leu Ala Gln Val Gly
    370                 375                 380

Ser Arg Arg Asn Met Pro Ala Asp Glu Val Lys Ser Leu Lys Phe Pro
385                 390                 395                 400

Val Pro Phe Tyr Pro Phe Gly Gln Tyr Phe Ala Ile Leu Phe Ile Ala
                405                 410                 415

Phe Thr Phe Gly Ile Met Val Trp Tyr Asp Asn Tyr His Leu Pro Leu
            420                 425                 430

Ala Val Gly Val Gly Phe Leu Val Leu Met Thr Ile Leu Tyr Tyr Ala
        435                 440                 445

Thr Gly Arg Pro Lys Ala Ile Ala Pro Ile Asp Tyr Glu Glu Leu Asp
    450                 455                 460

Pro Arg Arg Asp
465

<210> 613
<211> 1578
<212> DNA
<213> Corynebacterium glutamicum

<220>
<221> CDS
<222> (101)..(1555)
<223> RXA01172

<400> 613
taaatttttc tagactaccc agattaagtg agattctttc gtgtcttcga caacttcgac 60

aacttcggcc acccgacaag agccgttgag cccgacgggt gtg ctt gcc tcg ttt    115
                                           Val Leu Ala Ser Phe
                                           1               5

agg ttc gcg ttt agt agc ccg cgg agg ttt cgc acc gag gtt tta gct    163
Arg Phe Ala Phe Ser Ser Pro Arg Arg Phe Arg Thr Glu Val Leu Ala
                10                  15                  20

ggc ctc gtg gtg gcg ctc gcg ctg att ccg gag tcg att gcg ttt tcc    211
Gly Leu Val Val Ala Leu Ala Leu Ile Pro Glu Ser Ile Ala Phe Ser
            25                  30                  35

gta tta gct ggc gtg gat cct aag atg ggt ctt ttt gcg tcg tgc acc    259
Val Leu Ala Gly Val Asp Pro Lys Met Gly Leu Phe Ala Ser Cys Thr

        40                  45                  50

atg gca atg acc att gcg ctt aca ggt gga cgc cct gcg atg att tcc 307
Met Ala Met Thr Ile Ala Leu Thr Gly Gly Arg Pro Ala Met Ile Ser
    55                  60                  65

gcg gcg aca ggc gct gtg gcg ctg gtg att gca ccg gtg gtt cgg gat 355
Ala Ala Thr Gly Ala Val Ala Leu Val Ile Ala Pro Val Val Arg Asp
70                  75                  80                  85

cat ggc gtg gaa tat ttc ctg gcc acg gta att ttg gcg ggc att att 403
His Gly Val Glu Tyr Phe Leu Ala Thr Val Ile Leu Ala Gly Ile Ile
                90                  95                  100

cag att gcg ttg tcg ttg ctg ggt gta gcc aag ctg atg cgg ttc att 451
Gln Ile Ala Leu Ser Leu Leu Gly Val Ala Lys Leu Met Arg Phe Ile
            105                 110                 115

ccg cgt tcc gtt atg ctc gga ttt gtt aat gcg ctg gcg tgc ttg gtg 499
Pro Arg Ser Val Met Leu Gly Phe Val Asn Ala Leu Ala Cys Leu Val
        120                 125                 130

ttt ttt gcg cag ctg ccg cac ctg atc gat gtt ccg tgg atg gtg tat 547
Phe Phe Ala Gln Leu Pro His Leu Ile Asp Val Pro Trp Met Val Tyr
    135                 140                 145

ccg ctg ttc gcg ctg ggt att ggc atc atg ctg ttt tgg ccg aag ctg 595
Pro Leu Phe Ala Leu Gly Ile Gly Ile Met Leu Phe Trp Pro Lys Leu
150                 155                 160                 165

acc tcg gtg att ccg gct ccg ctg att gtt atc gtg gcc ttg act gcg 643
Thr Ser Val Ile Pro Ala Pro Leu Ile Val Ile Val Ala Leu Thr Ala
                170                 175                 180

atc gtg tgg gtt ttc gga atc aac att cca aac gtc tct gac cag ggc 691
Ile Val Trp Val Phe Gly Ile Asn Ile Pro Asn Val Ser Asp Gln Gly
            185                 190                 195

gaa ctc ccc tct tcc cta cca gaa ttc ctc att ccc aac gtt ccg ctt 739
Glu Leu Pro Ser Ser Leu Pro Glu Phe Leu Ile Pro Asn Val Pro Leu
        200                 205                 210

act ctt gag act cta aaa att att ggg ccg tat gcg ctt gga atg tcg 787
Thr Leu Glu Thr Leu Lys Ile Ile Gly Pro Tyr Ala Leu Gly Met Ser
    215                 220                 225

ctt gtt ggg ttg atg gaa tcg ctg ctg acc gcc aag ctg gtc gat gat 835
Leu Val Gly Leu Met Glu Ser Leu Leu Thr Ala Lys Leu Val Asp Asp
230                 235                 240                 245

att acg gag gtt cac tcc aat aaa tcc cgc gaa gct gca ggt cag ggc 883
Ile Thr Glu Val His Ser Asn Lys Ser Arg Glu Ala Ala Gly Gln Gly
                250                 255                 260

atc gca aac att atc acc gcg ttt ttg ggc ggc atg ggt ggc tgc gcg 931
Ile Ala Asn Ile Ile Thr Ala Phe Leu Gly Gly Met Gly Gly Cys Ala
            265                 270                 275

atg atc ggc cag acc atg atc aac gtg aaa aac tca gga gcc cgc act 979
Met Ile Gly Gln Thr Met Ile Asn Val Lys Asn Ser Gly Ala Arg Thr
        280                 285                 290

cgc cta tcc acc ttc ttg gcc ggc ggc ttc ctg ctg ctc ctg gtg gtt 1027
Arg Leu Ser Thr Phe Leu Ala Gly Gly Phe Leu Leu Leu Leu Val Val
    295                 300                 305

ttg ctg ggc gat gtc gtg ggc aaa atc ccc atg gcg gcg ctc gtg gca 1075
Leu Leu Gly Asp Val Val Gly Lys Ile Pro Met Ala Ala Leu Val Ala
310                 315                 320                 325

gtc atg att atc gtc tcc atc gac acc gcc gac tgg cat tcg ctg aac 1123
Val Met Ile Ile Val Ser Ile Asp Thr Ala Asp Trp His Ser Leu Asn
                330                 335                 340

ccg cgc acc ctc aaa ttc atg ccg ctg agt gaa acg atc gtc atg ttt 1171
Pro Arg Thr Leu Lys Phe Met Pro Leu Ser Glu Thr Ile Val Met Phe
            345                 350                 355

ata acg atc atc gcg acc ctc gtc acc gga aac ctg gcg atc ggc gtc 1219
Ile Thr Ile Ile Ala Thr Leu Val Thr Gly Asn Leu Ala Ile Gly Val
        360                 365                 370

atc ctc gga gtg ctc acc gcc atg gtc atg ttt gct cgc cgc gtg gcc 1267
Ile Leu Gly Val Leu Thr Ala Met Val Met Phe Ala Arg Arg Val Ala
    375                 380                 385

cac ctc gtg tct gtg gag cgc acc acc gac aac aac atc agc acc tat 1315
His Leu Val Ser Val Glu Arg Thr Thr Asp Asn Asn Ile Ser Thr Tyr
390                 395                 400                 405

acc gtc aag ggc cag cta ttt tgg gcg tcc tcg aac gat atg gtg tac 1363
Thr Val Lys Gly Gln Leu Phe Trp Ala Ser Ser Asn Asp Met Val Tyr
                410                 415                 420

tcc ttc gac tac tcc gac gag gcc gag cag atc atc atc gat ctc acc 1411
Ser Phe Asp Tyr Ser Asp Glu Ala Glu Gln Ile Ile Ile Asp Leu Thr
            425                 430                 435

gcc gca gaa atc tgg gac gcc tcc acg gta gcc acg ctg gac agc atc 1459
Ala Ala Glu Ile Trp Asp Ala Ser Thr Val Ala Thr Leu Asp Ser Ile
        440                 445                 450

atc cac aaa tac gcc gcg cgc ggc aag agc gtg gag atc atc ggg ctc 1507
Ile His Lys Tyr Ala Ala Arg Gly Lys Ser Val Glu Ile Ile Gly Leu
    455                 460                 465

gac ggc ccc agc cgc gat agg ctt gag cgc cta tca ggc aag ctg ggc 1555
Asp Gly Pro Ser Arg Asp Arg Leu Glu Arg Leu Ser Gly Lys Leu Gly
470                 475                 480                 485

taaaaatttg cttatcgacg cgc 1578

<210> 614
<211> 485
<212> PRT
<213> Corynebacterium glutamicum

<400> 614
Val Leu Ala Ser Phe Arg Phe Ala Phe Ser Ser Pro Arg Arg Phe Arg
1               5                   10                  15

Thr Glu Val Leu Ala Gly Leu Val Val Ala Leu Ala Leu Ile Pro Glu
            20                  25                  30

Ser Ile Ala Phe Ser Val Leu Ala Gly Val Asp Pro Lys Met Gly Leu
        35                  40                  45

Phe Ala Ser Cys Thr Met Ala Met Thr Ile Ala Leu Thr Gly Gly Arg
    50                  55                  60

Pro Ala Met Ile Ser Ala Ala Thr Gly Ala Val Ala Leu Val Ile Ala
65                  70                  75                  80

Pro Val Val Arg Asp His Gly Val Glu Tyr Phe Leu Ala Thr Val Ile
                85                  90                  95

Leu Ala Gly Ile Ile Gln Ile Ala Leu Ser Leu Leu Gly Val Ala Lys
            100                 105                 110

Leu Met Arg Phe Ile Pro Arg Ser Val Met Leu Gly Phe Val Asn Ala
        115                 120                 125

Leu Ala Cys Leu Val Phe Phe Ala Gln Leu Pro His Leu Ile Asp Val
    130                 135                 140

Pro Trp Met Val Tyr Pro Leu Phe Ala Leu Gly Ile Gly Ile Met Leu
145                 150                 155                 160

Phe Trp Pro Lys Leu Thr Ser Val Ile Pro Ala Pro Leu Ile Val Ile
                165                 170                 175

Val Ala Leu Thr Ala Ile Val Trp Val Phe Gly Ile Asn Ile Pro Asn
            180                 185                 190

Val Ser Asp Gln Gly Glu Leu Pro Ser Ser Leu Pro Glu Phe Leu Ile
        195                 200                 205

Pro Asn Val Pro Leu Thr Leu Glu Thr Leu Lys Ile Ile Gly Pro Tyr
    210                 215                 220

Ala Leu Gly Met Ser Leu Val Gly Leu Met Glu Ser Leu Leu Thr Ala
225                 230                 235                 240

Lys Leu Val Asp Asp Ile Thr Glu Val His Ser Asn Lys Ser Arg Glu
                245                 250                 255

Ala Ala Gly Gln Gly Ile Ala Asn Ile Ile Thr Ala Phe Leu Gly Gly
            260                 265                 270

Met Gly Gly Cys Ala Met Ile Gly Gln Thr Met Ile Asn Val Lys Asn
        275                 280                 285

Ser Gly Ala Arg Thr Arg Leu Ser Thr Phe Leu Ala Gly Gly Phe Leu
    290                 295                 300

Leu Leu Leu Val Val Leu Leu Gly Asp Val Val Gly Lys Ile Pro Met
305                 310                 315                 320

Ala Ala Leu Val Ala Val Met Ile Ile Val Ser Ile Asp Thr Ala Asp
                325                 330                 335

Trp His Ser Leu Asn Pro Arg Thr Leu Lys Phe Met Pro Leu Ser Glu

340                 345                 350

Thr Ile Val Met Phe Ile Thr Ile Ile Ala Thr Leu Val Thr Gly Asn
        355                 360                 365

Leu Ala Ile Gly Val Ile Leu Gly Val Leu Thr Ala Met Val Met Phe
    370                 375                 380

Ala Arg Arg Val Ala His Leu Val Ser Val Glu Arg Thr Thr Asp Asn
385                 390                 395                 400

Asn Ile Ser Thr Tyr Thr Val Lys Gly Gln Leu Phe Trp Ala Ser Ser
                405                 410                 415

Asn Asp Met Val Tyr Ser Phe Asp Tyr Ser Asp Glu Ala Glu Gln Ile
            420                 425                 430

Ile Ile Asp Leu Thr Ala Ala Glu Ile Trp Asp Ala Ser Thr Val Ala
        435                 440                 445

Thr Leu Asp Ser Ile Ile His Lys Tyr Ala Ala Arg Gly Lys Ser Val
    450                 455                 460

Glu Ile Ile Gly Leu Asp Gly Pro Ser Arg Asp Arg Leu Glu Arg Leu
465                 470                 475                 480

Ser Gly Lys Leu Gly
                485

<210> 615
<211> 1818
<212> DNA
<213> Corynebacterium glutamicum

<220>
<221> CDS
<222> (101)..(1795)
<223> RXA02128

<400> 615
tattagcaaa acttcttaaa gagccttttt gtgcctttat ccgagtatct ttaaaagcat 60

gagtattaag tgggcgcgct ttgtaccggg gataaccgcg atg cgg ggc tat caa    115
                                           Met Arg Gly Tyr Gln
                                           1               5

cga tcc tgg ttg aag ggt gat gtc atc gcg ggt ata acc gtg gcc gcg    163
Arg Ser Trp Leu Lys Gly Asp Val Ile Ala Gly Ile Thr Val Ala Ala
                10                  15                  20

tac ttg gtt cca caa gtc atg gct tat gcc gtc att gcg ggg ctg cca    211
Tyr Leu Val Pro Gln Val Met Ala Tyr Ala Val Ile Ala Gly Leu Pro
            25                  30                  35

gct gtc gtt ggt ctg tgg gga gtt ctg gct ccc atg gcg ctg tac ttt    259
Ala Val Val Gly Leu Trp Gly Val Leu Ala Pro Met Ala Leu Tyr Phe
        40                  45                  50

ttc ttg ggc acg tct cga aat ctc tcg gtt ggt cct gaa tca acc acc    307
Phe Leu Gly Thr Ser Arg Asn Leu Ser Val Gly Pro Glu Ser Thr Thr

55                  60                  65

gct ctg atg acg gct gca ggt gtg gga gct tta gtc ggg gca gct ggc    355
Ala Leu Met Thr Ala Ala Gly Val Gly Ala Leu Val Gly Ala Ala Gly
 70                  75                  80                  85

ggg cct gaa cga tac gca gaa gta gcg gca cta ttg gct att gca gtg    403
Gly Pro Glu Arg Tyr Ala Glu Val Ala Ala Leu Leu Ala Ile Ala Val
                 90                  95                 100

ggc att gta tgc gct gtt ggt ttt att ggc cga ttg gga ttt ctt acc    451
Gly Ile Val Cys Ala Val Gly Phe Ile Gly Arg Leu Gly Phe Leu Thr
            105                 110                 115

agg ctg ttg tct cga ccg gtg ctc gtt gga tat ttg atc ggt att gca    499
Arg Leu Leu Ser Arg Pro Val Leu Val Gly Tyr Leu Ile Gly Ile Ala
        120                 125                 130

gtc ttg atg atc gtc agt cag ctg tcc aaa gtc acc cag gtg aat gtg    547
Val Leu Met Ile Val Ser Gln Leu Ser Lys Val Thr Gln Val Asn Val
    135                 140                 145

gag agc ggt cag acg tgg cag gaa ata ata tcg ttt atc aaa gta gct    595
Glu Ser Gly Gln Thr Trp Gln Glu Ile Ile Ser Phe Ile Lys Val Ala
150                 155                 160                 165

ggc cag gca cat att cct aca gtg att ttg gca gtc gtg gtg ttg agc    643
Gly Gln Ala His Ile Pro Thr Val Ile Leu Ala Val Val Val Leu Ser
                170                 175                 180

ttg ctg tat ctg gca aat tgg ttg acg cct aaa ttt ccc agc aca ctc    691
Leu Leu Tyr Leu Ala Asn Trp Leu Thr Pro Lys Phe Pro Ser Thr Leu
            185                 190                 195

atg gtt ctt ctg ctt tcg gca gcc gcg gtg ggg ttt ttt cat ctg gat    739
Met Val Leu Leu Leu Ser Ala Ala Ala Val Gly Phe Phe His Leu Asp
        200                 205                 210

agg ttt ggt ctt gag gtc att ggt gag gtg ccc cgt ggc ctg cct caa    787
Arg Phe Gly Leu Glu Val Ile Gly Glu Val Pro Arg Gly Leu Pro Gln
    215                 220                 225

cca agt att ccc tcg att ggc gat cta gag atc tgg tcg ttg ttg ccc    835
Pro Ser Ile Pro Ser Ile Gly Asp Leu Glu Ile Trp Ser Leu Leu Pro
230                 235                 240                 245

tat gcc gtg ggt att gcc atc gtt ggt ttt tca gac aat gtg ttg act    883
Tyr Ala Val Gly Ile Ala Ile Val Gly Phe Ser Asp Asn Val Leu Thr
                250                 255                 260

gct cgt gca ttc gcg tcg gga aaa gat gag gtg att gat tcc aac cag    931
Ala Arg Ala Phe Ala Ser Gly Lys Asp Glu Val Ile Asp Ser Asn Gln
            265                 270                 275

gag ctg ctc gca ctg gga acc gca aac ctg gcg aat ggg ttc ttc cag    979
Glu Leu Leu Ala Leu Gly Thr Ala Asn Leu Ala Asn Gly Phe Phe Gln
        280                 285                 290

gga ttt cct gtg tca tcg agt ggc tcc cga act gtt ctt gga gac acg    1027
Gly Phe Pro Val Ser Ser Ser Gly Ser Arg Thr Val Leu Gly Asp Thr
    295                 300                 305

gca ggt gct cgc act cag gtg cat tca ctt gtc gtg gtg gcg ctg gtg 1075
Ala Gly Ala Arg Thr Gln Val His Ser Leu Val Val Val Ala Leu Val
310                 315                 320                 325

atc atg gtg ctg ttg ttt gct ggt cct gtg ctc gag tct ttc cca gat 1123
Ile Met Val Leu Leu Phe Ala Gly Pro Val Leu Glu Ser Phe Pro Asp
                330                 335                 340

gcg gca ctt ggc gcc tta gtt att tat gca gca acg cag ttg att gat 1171
Ala Ala Leu Gly Ala Leu Val Ile Tyr Ala Ala Thr Gln Leu Ile Asp
            345                 350                 355

atc gca gag atc aaa agg atc gca cgt ttc cgc aag agc gag ttg gtc 1219
Ile Ala Glu Ile Lys Arg Ile Ala Arg Phe Arg Lys Ser Glu Leu Val
        360                 365                 370

atc aca gcg gct act gct gca tcc gtt gtg gct tct ggc gtg ctc gcg 1267
Ile Thr Ala Ala Thr Ala Ala Ser Val Val Ala Ser Gly Val Leu Ala
    375                 380                 385

ggg atc ggc gtt gcg gtt acg ttg tcc atc ttg gat ctc atc aga cgt 1315
Gly Ile Gly Val Ala Val Thr Leu Ser Ile Leu Asp Leu Ile Arg Arg
390                 395                 400                 405

att acc cga cct tat gcc gat gtc cta gga tat acg ccg ggc atg gct 1363
Ile Thr Arg Pro Tyr Ala Asp Val Leu Gly Tyr Thr Pro Gly Met Ala
                410                 415                 420

gga atg cac agc ttg gag gat tat cct gag tcg aca gca gtc gaa ggg 1411
Gly Met His Ser Leu Glu Asp Tyr Pro Glu Ser Thr Ala Val Glu Gly
            425                 430                 435

ctc gtg gtt ttt aga tac gat tcc cca ctg ttt ttc gcc aac gct gat 1459
Leu Val Val Phe Arg Tyr Asp Ser Pro Leu Phe Phe Ala Asn Ala Asp
        440                 445                 450

gat ttt tcc aaa cgt gcc atc gaa gcc gtt gat gaa gca act caa ccc 1507
Asp Phe Ser Lys Arg Ala Ile Glu Ala Val Asp Glu Ala Thr Gln Pro
    455                 460                 465

gtg cat tgg ttt tta ctg aat gct gaa gcg aat acg gaa gtt gat ctc 1555
Val His Trp Phe Leu Leu Asn Ala Glu Ala Asn Thr Glu Val Asp Leu
470                 475                 480                 485

acg gcc gtc gat gcc atg gaa gca ctt cgc aaa acc ctg gag gaa cgg 1603
Thr Ala Val Asp Ala Met Glu Ala Leu Arg Lys Thr Leu Glu Glu Arg
                490                 495                 500

ggt atc cga ttt gcg atg gcc cgg gtg aag caa gat cta cgc cga agc 1651
Gly Ile Arg Phe Ala Met Ala Arg Val Lys Gln Asp Leu Arg Arg Ser
            505                 510                 515

ctc gag cct gca ggt ttc att gaa tcc gtg ggg gag gag tac att ttc 1699
Leu Glu Pro Ala Gly Phe Ile Glu Ser Val Gly Glu Glu Tyr Ile Phe
        520                 525                 530

gcc aca ctc ccc act gca gtc aag ggg tat tcc gtg gag ttt cgc gat 1747
Ala Thr Leu Pro Thr Ala Val Lys Gly Tyr Ser Val Glu Phe Arg Asp
    535                 540                 545

cgt ttt gga aac tat cca gaa ggc gtt ccg aaa gaa att ttg gaa ctt    1795
Arg Phe Gly Asn Tyr Pro Glu Gly Val Pro Lys Glu Ile Leu Glu Leu
550                 555                 560                 565

taagctgcct ggttggcgga ctt                                          1818

<210> 616
<211> 565
<212> PRT
<213> Corynebacterium glutamicum

<400> 616
Met Arg Gly Tyr Gln Arg Ser Trp Leu Lys Gly Asp Val Ile Ala Gly
1               5                   10                  15

Ile Thr Val Ala Ala Tyr Leu Val Pro Gln Val Met Ala Tyr Ala Val
            20                  25                  30

Ile Ala Gly Leu Pro Ala Val Val Gly Leu Trp Gly Val Leu Ala Pro
        35                  40                  45

Met Ala Leu Tyr Phe Phe Leu Gly Thr Ser Arg Asn Leu Ser Val Gly
    50                  55                  60

Pro Glu Ser Thr Thr Ala Leu Met Thr Ala Ala Gly Val Gly Ala Leu
65                  70                  75                  80

Val Gly Ala Ala Gly Gly Pro Glu Arg Tyr Ala Glu Val Ala Ala Leu
                85                  90                  95

Leu Ala Ile Ala Val Gly Ile Val Cys Ala Val Gly Phe Ile Gly Arg
            100                 105                 110

Leu Gly Phe Leu Thr Arg Leu Leu Ser Arg Pro Val Leu Val Gly Tyr
        115                 120                 125

Leu Ile Gly Ile Ala Val Leu Met Ile Val Ser Gln Leu Ser Lys Val
    130                 135                 140

Thr Gln Val Asn Val Glu Ser Gly Gln Thr Trp Gln Glu Ile Ile Ser
145                 150                 155                 160

Phe Ile Lys Val Ala Gly Gln Ala His Ile Pro Thr Val Ile Leu Ala
                165                 170                 175

Val Val Val Leu Ser Leu Leu Tyr Leu Ala Asn Trp Leu Thr Pro Lys
            180                 185                 190

Phe Pro Ser Thr Leu Met Val Leu Leu Leu Ser Ala Ala Ala Val Gly
        195                 200                 205

Phe Phe His Leu Asp Arg Phe Gly Leu Glu Val Ile Gly Glu Val Pro
    210                 215                 220

Arg Gly Leu Pro Gln Pro Ser Ile Pro Ser Ile Gly Asp Leu Glu Ile
225                 230                 235                 240

Trp Ser Leu Leu Pro Tyr Ala Val Gly Ile Ala Ile Val Gly Phe Ser
                245                 250                 255

Asp Asn Val Leu Thr Ala Arg Ala Phe Ala Ser Gly Lys Asp Glu Val
            260                 265                 270

Ile Asp Ser Asn Gln Glu Leu Leu Ala Leu Gly Thr Ala Asn Leu Ala
        275                 280                 285

Asn Gly Phe Phe Gln Gly Phe Pro Val Ser Ser Ser Gly Ser Arg Thr
    290                 295                 300

Val Leu Gly Asp Thr Ala Gly Ala Arg Thr Gln Val His Ser Leu Val
305                 310                 315                 320

Val Val Ala Leu Val Ile Met Val Leu Leu Phe Ala Gly Pro Val Leu
                325                 330                 335

Glu Ser Phe Pro Asp Ala Ala Leu Gly Ala Leu Val Ile Tyr Ala Ala
            340                 345                 350

Thr Gln Leu Ile Asp Ile Ala Glu Ile Lys Arg Ile Ala Arg Phe Arg
        355                 360                 365

Lys Ser Glu Leu Val Ile Thr Ala Ala Thr Ala Ala Ser Val Val Ala
    370                 375                 380

Ser Gly Val Leu Ala Gly Ile Gly Val Ala Val Thr Leu Ser Ile Leu
385                 390                 395                 400

Asp Leu Ile Arg Arg Ile Thr Arg Pro Tyr Ala Asp Val Leu Gly Tyr
                405                 410                 415

Thr Pro Gly Met Ala Gly Met His Ser Leu Glu Asp Tyr Pro Glu Ser
            420                 425                 430

Thr Ala Val Glu Gly Leu Val Val Phe Arg Tyr Asp Ser Pro Leu Phe
        435                 440                 445

Phe Ala Asn Ala Asp Asp Phe Ser Lys Arg Ala Ile Glu Ala Val Asp
    450                 455                 460

Glu Ala Thr Gln Pro Val His Trp Phe Leu Leu Asn Ala Glu Ala Asn
465                 470                 475                 480

Thr Glu Val Asp Leu Thr Ala Val Asp Ala Met Glu Ala Leu Arg Lys
                485                 490                 495

Thr Leu Glu Glu Arg Gly Ile Arg Phe Ala Met Ala Arg Val Lys Gln
            500                 505                 510

Asp Leu Arg Arg Ser Leu Glu Pro Ala Gly Phe Ile Glu Ser Val Gly
        515                 520                 525

Glu Glu Tyr Ile Phe Ala Thr Leu Pro Thr Ala Val Lys Gly Tyr Ser
    530                 535                 540

Val Glu Phe Arg Asp Arg Phe Gly Asn Tyr Pro Glu Gly Val Pro Lys
545                 550                 555                 560

Glu Ile Leu Glu Leu
                565

<210> 617
<211> 1734
<212> DNA
<213> Corynebacterium glutamicum

<220>
<221> CDS
<222> (101)..(1711)
<223> RXA02634

<400> 617
caacgcgaag gcttagttgt ttattaccgg ctcacctacc cggaggtcgc agactttctt 60

aaggtgagcc gctcactatt aaagaggatg gctggcgaag atg cgt gac ctt tta 115
Met Arg Asp Leu Leu
1               5

ccc tct aga gac gac tat cag cta ctc cgc ttc tcc tgg aag atg gac 163
Pro Ser Arg Asp Asp Tyr Gln Leu Leu Arg Phe Ser Trp Lys Met Asp
                10                  15                  20

att gct gct ggc gtc acc gtg ggc att gtt gcc ctc ccc ctc gct ctc 211
Ile Ala Ala Gly Val Thr Val Gly Ile Val Ala Leu Pro Leu Ala Leu
            25                  30                  35

gcc ttt ggt gtg agt tct gga gtc gga gcc gag gca gga tta gtg act 259
Ala Phe Gly Val Ser Ser Gly Val Gly Ala Glu Ala Gly Leu Val Thr
        40                  45                  50

gcg att att gct ggc ctg gtt gca gca atc ttc ggc ggt tcc aat gtg 307
Ala Ile Ile Ala Gly Leu Val Ala Ala Ile Phe Gly Gly Ser Asn Val
    55                  60                  65

cag gtt tca gga cca aca gga gcg atg gtg gtt gtt ctc gct cca atc 355
Gln Val Ser Gly Pro Thr Gly Ala Met Val Val Val Leu Ala Pro Ile
70                  75                  80                  85

gtc gcc caa tat ggt gtc ggt gcg gtt gca tta ctc agc ctg atg gct 403
Val Ala Gln Tyr Gly Val Gly Ala Val Ala Leu Leu Ser Leu Met Ala
                90                  95                  100

gga gtt atc gtc ctt gtc gcc gga gtg ctt cga tta ggc cga acg gtg 451
Gly Val Ile Val Leu Val Ala Gly Val Leu Arg Leu Gly Arg Thr Val
            105                 110                 115

agt ttc att ccg tgg ccg gtc atc gaa gga ttt acg gca ggc atc ggt 499
Ser Phe Ile Pro Trp Pro Val Ile Glu Gly Phe Thr Ala Gly Ile Gly
        120                 125                 130

gtg att att ttc ctg caa cag gtg cca gct gcg ttt ggt tat tcg ggc 547
Val Ile Ile Phe Leu Gln Gln Val Pro Ala Ala Phe Gly Tyr Ser Gly
    135                 140                 145

cat ttg ccc acc aat gcg ctc ctc gct gcg att cac act gtt tca cac 595
His Leu Pro Thr Asn Ala Leu Leu Ala Ala Ile His Thr Val Ser His
150                 155                 160                 165

gcc acg aag gac gca att cta cct ctc tta att att atc gtc acc gct 643
Ala Thr Lys Asp Ala Ile Leu Pro Leu Leu Ile Ile Ile Val Thr Ala
                170                 175                 180

gcg atc atg att gtg ttg gga aag atc gcc cca aag ctc cct gcc agc     691
Ala Ile Met Ile Val Leu Gly Lys Ile Ala Pro Lys Leu Pro Ala Ser
            185                 190                 195

ttc att gcc att ttg gtg gtg tcc att ggc gtg gct ctg ctc aag ctt     739
Phe Ile Ala Ile Leu Val Val Ser Ile Gly Val Ala Leu Leu Lys Leu
        200                 205                 210

ccc gtt gag ttg atc ggt gag ctc ccc aat tcc ctt cct gcc cct cac     787
Pro Val Glu Leu Ile Gly Glu Leu Pro Asn Ser Leu Pro Ala Pro His
    215                 220                 225

ctg cct gat gtg aac ctg gag atg ttc acg agt ttg ctg ggg cca gct     835
Leu Pro Asp Val Asn Leu Glu Met Phe Thr Ser Leu Leu Gly Pro Ala
230                 235                 240                 245

ttt gct gtc gcc gcg ctc gct gct att gag tct ctc ctt tcc gct cgt     883
Phe Ala Val Ala Ala Leu Ala Ala Ile Glu Ser Leu Leu Ser Ala Arg
                250                 255                 260

gtg gca gcc tcc atg gcc gat acg ggt cct tat aat gct gac cgc gaa     931
Val Ala Ala Ser Met Ala Asp Thr Gly Pro Tyr Asn Ala Asp Arg Glu
            265                 270                 275

ttg gtc ggt cag ggg ttg gcg tcg ata agc gct ggc ttt ttt ggc ggc     979
Leu Val Gly Gln Gly Leu Ala Ser Ile Ser Ala Gly Phe Phe Gly Gly
        280                 285                 290

atg ccg gca act ggc gca atc gcg cgt acg gcg gtc aat gtg cgc tcc     1027
Met Pro Ala Thr Gly Ala Ile Ala Arg Thr Ala Val Asn Val Arg Ser
    295                 300                 305

ggc ggg cgt acc cgt ata gcg tcg att att cac gcg ctt gtg ctg ctc     1075
Gly Gly Arg Thr Arg Ile Ala Ser Ile Ile His Ala Leu Val Leu Leu
310                 315                 320                 325

ggc gtt gtg tat gtg gcg gcg aat atc gtt gcg gtc att ccg ctc gcc     1123
Gly Val Val Tyr Val Ala Ala Asn Ile Val Ala Val Ile Pro Leu Ala
                330                 335                 340

gca ctg tcg ggc gtg ctc atg gtc acc gcg agc cgc atg gtg tcc att     1171
Ala Leu Ser Gly Val Leu Met Val Thr Ala Ser Arg Met Val Ser Ile
            345                 350                 355

gaa gtg atc tcg cgc gtc atg cgc tcc act cgc tct gat gcg atc gtt     1219
Glu Val Ile Ser Arg Val Met Arg Ser Thr Arg Ser Asp Ala Ile Val
        360                 365                 370

ttt gtc atc acc gcg atc gtc acc atc agc gtc gac ctc gtc att gcg     1267
Phe Val Ile Thr Ala Ile Val Thr Ile Ser Val Asp Leu Val Ile Ala
    375                 380                 385

gtc ggc atc ggc att gcc gtc gcg acg ttc ttc atg ctg cga cgc atg     1315
Val Gly Ile Gly Ile Ala Val Ala Thr Phe Phe Met Leu Arg Arg Met
390                 395                 400                 405

agc atg aac gcc ggc gtc ttc cgg gaa acc ttg ccc gaa ccg gcc acg     1363
Ser Met Asn Ala Gly Val Phe Arg Glu Thr Leu Pro Glu Pro Ala Thr
                410                 415                 420

ctt aac gac gaa aag atc ggc ctt ttc cgt atc gag ggc gca ttg ttc     1411

Leu Asn Asp Glu Lys Ile Gly Leu Phe Arg Ile Glu Gly Ala Leu Phe
            425                 430                 435

ttt ggc gca gct gaa cgt ctt tcg cag caa atc ctt gat tat gag gac    1459
Phe Gly Ala Ala Glu Arg Leu Ser Gln Gln Ile Leu Asp Tyr Glu Asp
        440                 445                 450

ctc gaa gtg gtg atc ctg cgc ctg tca cac atc caa atg atc gac gcc    1507
Leu Glu Val Val Ile Leu Arg Leu Ser His Ile Gln Met Ile Asp Ala
    455                 460                 465

acc ggc gcc cac cag ctc acc gag ctt gtc aat gcg ctg gaa aga aaa    1555
Thr Gly Ala His Gln Leu Thr Glu Leu Val Asn Ala Leu Glu Arg Lys
470                 475                 480                 485

aat gtc act gta tta att aag ggc gtc cgg aaa gaa cac att cac gtt    1603
Asn Val Thr Val Leu Ile Lys Gly Val Arg Lys Glu His Ile His Val
                490                 495                 500

ctc ggc gtc ctc gga gcg atc aga tca ctg cgg cat gag aac cac ctc    1651
Leu Gly Val Leu Gly Ala Ile Arg Ser Leu Arg His Glu Asn His Leu
            505                 510                 515

ttc gat gac ctt gcc cca gcg gtt gaa cat gcc cga aaa cac gtt aag    1699
Phe Asp Asp Leu Ala Pro Ala Val Glu His Ala Arg Lys His Val Lys
        520                 525                 530

atc gac aac agt taaggcttcc ggggcgatct aaa                          1734
Ile Asp Asn Ser
    535


<210> 618
<211> 537
<212> PRT
<213> Corynebacterium glutamicum

<400> 618
Met Arg Asp Leu Leu Pro Ser Arg Asp Asp Tyr Gln Leu Leu Arg Phe
1               5                   10                  15

Ser Trp Lys Met Asp Ile Ala Ala Gly Val Thr Val Gly Ile Val Ala
            20                  25                  30

Leu Pro Leu Ala Leu Ala Phe Gly Val Ser Ser Gly Val Gly Ala Glu
        35                  40                  45

Ala Gly Leu Val Thr Ala Ile Ile Ala Gly Leu Val Ala Ala Ile Phe
    50                  55                  60

Gly Gly Ser Asn Val Gln Val Ser Gly Pro Thr Gly Ala Met Val Val
65                  70                  75                  80

Val Leu Ala Pro Ile Val Ala Gln Tyr Gly Val Gly Ala Val Ala Leu
                85                  90                  95

Leu Ser Leu Met Ala Gly Val Ile Val Leu Val Ala Gly Val Leu Arg
            100                 105                 110

Leu Gly Arg Thr Val Ser Phe Ile Pro Trp Pro Val Ile Glu Gly Phe
        115                 120                 125

Thr Ala Gly Ile Gly Val Ile Ile Phe Leu Gln Gln Val Pro Ala Ala
    130                 135                 140

Phe Gly Tyr Ser Gly His Leu Pro Thr Asn Ala Leu Leu Ala Ala Ile
145                 150                 155                 160

His Thr Val Ser His Ala Thr Lys Asp Ala Ile Leu Pro Leu Leu Ile
                165                 170                 175

Ile Ile Val Thr Ala Ala Ile Met Ile Val Leu Gly Lys Ile Ala Pro
            180                 185                 190

Lys Leu Pro Ala Ser Phe Ile Ala Ile Leu Val Val Ser Ile Gly Val
        195                 200                 205

Ala Leu Leu Lys Leu Pro Val Glu Leu Ile Gly Glu Leu Pro Asn Ser
    210                 215                 220

Leu Pro Ala Pro His Leu Pro Asp Val Asn Leu Glu Met Phe Thr Ser
225                 230                 235                 240

Leu Leu Gly Pro Ala Phe Ala Val Ala Ala Leu Ala Ala Ile Glu Ser
                245                 250                 255

Leu Leu Ser Ala Arg Val Ala Ala Ser Met Ala Asp Thr Gly Pro Tyr
            260                 265                 270

Asn Ala Asp Arg Glu Leu Val Gly Gln Gly Leu Ala Ser Ile Ser Ala
        275                 280                 285

Gly Phe Phe Gly Gly Met Pro Ala Thr Gly Ala Ile Ala Arg Thr Ala
    290                 295                 300

Val Asn Val Arg Ser Gly Gly Arg Thr Arg Ile Ala Ser Ile Ile His
305                 310                 315                 320

Ala Leu Val Leu Leu Gly Val Val Tyr Val Ala Ala Asn Ile Val Ala
                325                 330                 335

Val Ile Pro Leu Ala Ala Leu Ser Gly Val Leu Met Val Thr Ala Ser
            340                 345                 350

Arg Met Val Ser Ile Glu Val Ile Ser Arg Val Met Arg Ser Thr Arg
        355                 360                 365

Ser Asp Ala Ile Val Phe Val Ile Thr Ala Ile Val Thr Ile Ser Val
    370                 375                 380

Asp Leu Val Ile Ala Val Gly Ile Gly Ile Ala Val Ala Thr Phe Phe
385                 390                 395                 400

Met Leu Arg Arg Met Ser Met Asn Ala Gly Val Phe Arg Glu Thr Leu
                405                 410                 415

Pro Glu Pro Ala Thr Leu Asn Asp Glu Lys Ile Gly Leu Phe Arg Ile
            420                 425                 430

Glu Gly Ala Leu Phe Phe Gly Ala Ala Glu Arg Leu Ser Gln Gln Ile
        435                 440                 445

Leu Asp Tyr Glu Asp Leu Glu Val Val Ile Leu Arg Leu Ser His Ile
    450                 455                 460

Gln Met Ile Asp Ala Thr Gly Ala His Gln Leu Thr Glu Leu Val Asn
465                 470                 475                 480

Ala Leu Glu Arg Lys Asn Val Thr Val Leu Ile Lys Gly Val Arg Lys
                485                 490                 495

Glu His Ile His Val Leu Gly Val Leu Gly Ala Ile Arg Ser Leu Arg
            500                 505                 510

His Glu Asn His Leu Phe Asp Asp Leu Ala Pro Ala Val Glu His Ala
        515                 520                 525

Arg Lys His Val Lys Ile Asp Asn Ser
    530                 535


<210> 619
<211> 1410
<212> DNA
<213> Corynebacterium glutamicum

<220>
<221> CDS
<222> (101)..(1387)
<223> RXN02233

<400> 619
atccccaccc aattggccca agagctgcag agttacgttg tagaacccac ctctgcctaa 60

ctgtcgattt cccaagagcc cccttgggag tcgataatta gtg ctc gtg act tca    115
                                           Val Leu Val Thr Ser
                                           1               5

aca tgg gga tgg acc gtc cac gga gac ggc aaa aag atc gaa ccc ggc    163
Thr Trp Gly Trp Thr Val His Gly Asp Gly Lys Lys Ile Glu Pro Gly
                10                  15                  20

gca gtt gtc gct cct aaa gag cgc ctg agc tgg ggg cgc aca att gga    211
Ala Val Val Ala Pro Lys Glu Arg Leu Ser Trp Gly Arg Thr Ile Gly
            25                  30                  35

atc ggt atg cag cac gtg atc gcc atg ttc ggc gcc acg ctc ctg gtt    259
Ile Gly Met Gln His Val Ile Ala Met Phe Gly Ala Thr Leu Leu Val
        40                  45                  50

ccc aca ctc acc gga ttt ccg gtc aac acc aca ctt tta ttc tct ggt    307
Pro Thr Leu Thr Gly Phe Pro Val Asn Thr Thr Leu Leu Phe Ser Gly
    55                  60                  65

ctg gga aca atc ctg ttc ctg ttg atc acc aga aac cga cta ccc tcg    355
Leu Gly Thr Ile Leu Phe Leu Leu Ile Thr Arg Asn Arg Leu Pro Ser
70                  75                  80                  85

tac ctg ggt agt tct ttt gct ttc att gca cct tta acc gca acc caa    403
Tyr Leu Gly Ser Ser Phe Ala Phe Ile Ala Pro Leu Thr Ala Thr Gln
                90                  95                  100

gtc cat ggc att ggc gtg cag atc ggt ggc att ctt gtc gca ggt ctc    451

Val His Gly Ile Gly Val Gln Ile Gly Gly Ile Leu Val Ala Gly Leu
            105                 110                 115

gtg ctc gtc gcc att gga ttt gtg gtg aaa gca gcg ggc aaa cgc gtt 499
Val Leu Val Ala Ile Gly Phe Val Val Lys Ala Ala Gly Lys Arg Val
        120                 125                 130

att gat gct gtc atg cca ccc gct gtc acc ggt gcg atc gtg gca ctc 547
Ile Asp Ala Val Met Pro Pro Ala Val Thr Gly Ala Ile Val Ala Leu
    135                 140                 145

atc ggc ctg aac ctg gca cca acc gcg gca gga aac ttc tcc agc caa 595
Ile Gly Leu Asn Leu Ala Pro Thr Ala Ala Gly Asn Phe Ser Ser Gln
150                 155                 160                 165

cca ctg gtt gcc acg gcg acc ctc ttt gcc att ttg atc gct acc gtt 643
Pro Leu Val Ala Thr Ala Thr Leu Phe Ala Ile Leu Ile Ala Thr Val
                170                 175                 180

gca ggc cgc gga atg att gct cgc ctg ggc att ttg atc ggt gtg gtg 691
Ala Gly Arg Gly Met Ile Ala Arg Leu Gly Ile Leu Ile Gly Val Val
            185                 190                 195

atc ggc tgg gtt ttc gca gct atc acc ggc aac ctc tca gaa ggc gca 739
Ile Gly Trp Val Phe Ala Ala Ile Thr Gly Asn Leu Ser Glu Gly Ala
        200                 205                 210

gca gac acc atc cgt gaa gca gca tgg ttc gga ctg cca cag ttc cac 787
Ala Asp Thr Ile Arg Glu Ala Ala Trp Phe Gly Leu Pro Gln Phe His
    215                 220                 225

aag ccg gaa ttc cag ctc tct gcc att ttg gtg aca ctg cca gtc atc 835
Lys Pro Glu Phe Gln Leu Ser Ala Ile Leu Val Thr Leu Pro Val Ile
230                 235                 240                 245

atc gtg ctc atc gct gaa aac gtc ggc cac gtc aaa gca gtc tca gag 883
Ile Val Leu Ile Ala Glu Asn Val Gly His Val Lys Ala Val Ser Glu
                250                 255                 260

atg aca ggg gag gac ctc gac gac ctc gcc ggc gac gca ctt atc gca 931
Met Thr Gly Glu Asp Leu Asp Asp Leu Ala Gly Asp Ala Leu Ile Ala
            265                 270                 275

gac gga ttc ggc acc acc ctc gca ggt gcc ttc ggt gga tcc ggc acc 979
Asp Gly Phe Gly Thr Thr Leu Ala Gly Ala Phe Gly Gly Ser Gly Thr
        280                 285                 290

acc acc tac gca gaa aac atc ggc gtc atg gcg gcc acc cgc gta tat 1027
Thr Thr Tyr Ala Glu Asn Ile Gly Val Met Ala Ala Thr Arg Val Tyr
    295                 300                 305

tcc acc gct gcg tac tgg gtt gcc gcg tgc act gcc atc gcc ctt gcc 1075
Ser Thr Ala Ala Tyr Trp Val Ala Ala Cys Thr Ala Ile Ala Leu Ala
310                 315                 320                 325

ttc atc ccc aaa ttc ggt gca ctg atc ttc acc atc ccc gcc ggc gtg 1123
Phe Ile Pro Lys Phe Gly Ala Leu Ile Phe Thr Ile Pro Ala Gly Val
                330                 335                 340

ctg ggt ggg gca tgt ttg gtt ctt tac ggc cta atc ggt atg ctc ggc 1171
Leu Gly Gly Ala Cys Leu Val Leu Tyr Gly Leu Ile Gly Met Leu Gly

345                    350                    355

att cgt atc tgg caa gac aac aag gtc aac ttc aac aat cca gtg aat    1219
Ile Arg Ile Trp Gln Asp Asn Lys Val Asn Phe Asn Asn Pro Val Asn
        360                 365                 370

ctg acc atg gct gcc gtt gct ttg gtt gca ggc att ggt aac ctc acc    1267
Leu Thr Met Ala Ala Val Ala Leu Val Ala Gly Ile Gly Asn Leu Thr
    375                 380                 385

ctc acc gtt ttc gga gtc act ctt gaa ggc atc gca tgg ggc tct gta    1315
Leu Thr Val Phe Gly Val Thr Leu Glu Gly Ile Ala Trp Gly Ser Val
390                 395                 400                 405

ggc atc att gtg ctg tac cca atc atg aag cgc ctg tac ctt tcc att    1363
Gly Ile Ile Val Leu Tyr Pro Ile Met Lys Arg Leu Tyr Leu Ser Ile
                410                 415                 420

ggg gaa ggc aag aac gca aag ttc tagtacgctg ctaaagtatg cag          1410
Gly Glu Gly Lys Asn Ala Lys Phe
            425

<210> 620
<211> 429
<212> PRT
<213> Corynebacterium glutamicum

<400> 620
Val Leu Val Thr Ser Thr Trp Gly Trp Thr Val His Gly Asp Gly Lys
1               5                   10                  15

Lys Ile Glu Pro Gly Ala Val Val Ala Pro Lys Glu Arg Leu Ser Trp
            20                  25                  30

Gly Arg Thr Ile Gly Ile Gly Met Gln His Val Ile Ala Met Phe Gly
        35                  40                  45

Ala Thr Leu Leu Val Pro Thr Leu Thr Gly Phe Pro Val Asn Thr Thr
    50                  55                  60

Leu Leu Phe Ser Gly Leu Gly Thr Ile Leu Phe Leu Leu Ile Thr Arg
65                  70                  75                  80

Asn Arg Leu Pro Ser Tyr Leu Gly Ser Ser Phe Ala Phe Ile Ala Pro
                85                  90                  95

Leu Thr Ala Thr Gln Val His Gly Ile Gly Val Gln Ile Gly Gly Ile
            100                 105                 110

Leu Val Ala Gly Leu Val Leu Val Ala Ile Gly Phe Val Val Lys Ala
        115                 120                 125

Ala Gly Lys Arg Val Ile Asp Ala Val Met Pro Pro Ala Val Thr Gly
    130                 135                 140

Ala Ile Val Ala Leu Ile Gly Leu Asn Leu Ala Pro Thr Ala Ala Gly
145                 150                 155                 160

Asn Phe Ser Ser Gln Pro Leu Val Ala Thr Ala Thr Leu Phe Ala Ile
                165                 170                 175

Leu Ile Ala Thr Val Ala Gly Arg Gly Met Ile Ala Arg Leu Gly Ile
            180                 185                 190

Leu Ile Gly Val Val Ile Gly Trp Val Phe Ala Ala Ile Thr Gly Asn
        195                 200                 205

Leu Ser Glu Gly Ala Ala Asp Thr Ile Arg Glu Ala Ala Trp Phe Gly
    210                 215                 220

Leu Pro Gln Phe His Lys Pro Glu Phe Gln Leu Ser Ala Ile Leu Val
225                 230                 235                 240

Thr Leu Pro Val Ile Ile Val Leu Ile Ala Glu Asn Val Gly His Val
                245                 250                 255

Lys Ala Val Ser Glu Met Thr Gly Glu Asp Leu Asp Asp Leu Ala Gly
            260                 265                 270

Asp Ala Leu Ile Ala Asp Gly Phe Gly Thr Thr Leu Ala Gly Ala Phe
        275                 280                 285

Gly Gly Ser Gly Thr Thr Thr Tyr Ala Glu Asn Ile Gly Val Met Ala
    290                 295                 300

Ala Thr Arg Val Tyr Ser Thr Ala Ala Tyr Trp Val Ala Ala Cys Thr
305                 310                 315                 320

Ala Ile Ala Leu Ala Phe Ile Pro Lys Phe Gly Ala Leu Ile Phe Thr
                325                 330                 335

Ile Pro Ala Gly Val Leu Gly Gly Ala Cys Leu Val Leu Tyr Gly Leu
            340                 345                 350

Ile Gly Met Leu Gly Ile Arg Ile Trp Gln Asp Asn Lys Val Asn Phe
        355                 360                 365

Asn Asn Pro Val Asn Leu Thr Met Ala Ala Val Ala Leu Val Ala Gly
    370                 375                 380

Ile Gly Asn Leu Thr Leu Thr Val Phe Gly Val Thr Leu Glu Gly Ile
385                 390                 395                 400

Ala Trp Gly Ser Val Gly Ile Ile Val Leu Tyr Pro Ile Met Lys Arg
                405                 410                 415

Leu Tyr Leu Ser Ile Gly Glu Gly Lys Asn Ala Lys Phe
            420                 425

<210> 621
<211> 1335
<212> DNA
<213> Corynebacterium glutamicum

<220>
<221> CDS
<222> (101)..(1312)
<223> FRXA02233

<400> 621

atccccaccc aattggccca agagctgcag agttacgttg tagaacccac ctctgcctaa 60

ctgtcgattt cccaagagcc cccttgggag tcgataatta gtg ctc gtg act tca 115
Val Leu Val Thr Ser
1               5

aca tgg gga tgg acc gtc cac gga gac ggc aaa aag atc gaa ccc ggc 163
Thr Trp Gly Trp Thr Val His Gly Asp Gly Lys Lys Ile Glu Pro Gly
                10                  15                  20

gca gtt gtc gct cct aaa gag cgc ctg agc tgg ggg cgc aca att gga 211
Ala Val Val Ala Pro Lys Glu Arg Leu Ser Trp Gly Arg Thr Ile Gly
            25                  30                  35

atc ggt atg cag cac gtg atc gcc atg ttc ggc gcc acg ctc ctg gtt 259
Ile Gly Met Gln His Val Ile Ala Met Phe Gly Ala Thr Leu Leu Val
        40                  45                  50

ccc aca ctc acc gga ttt ccg gtc aac acc aca ctt tta ttc tct ggt 307
Pro Thr Leu Thr Gly Phe Pro Val Asn Thr Thr Leu Leu Phe Ser Gly
    55                  60                  65

ctg gga aca atc ctg ttc ctg ttg atc acc aga aac cga cta ccc tcg 355
Leu Gly Thr Ile Leu Phe Leu Leu Ile Thr Arg Asn Arg Leu Pro Ser
70                  75                  80                  85

tac ctg ggt agt tct ttt gct ttc att gca cct tta acc gca acc caa 403
Tyr Leu Gly Ser Ser Phe Ala Phe Ile Ala Pro Leu Thr Ala Thr Gln
                90                  95                  100

gtc cat ggc att ggc gtg cag atc ggt ggc att ctt gtc gca ggt ctc 451
Val His Gly Ile Gly Val Gln Ile Gly Gly Ile Leu Val Ala Gly Leu
            105                 110                 115

gtg ctc gtc gcc att gga ttt gtg gtg aaa gca gcg ggc aaa cgc gtt 499
Val Leu Val Ala Ile Gly Phe Val Val Lys Ala Ala Gly Lys Arg Val
        120                 125                 130

att gat gct gtc atg cca ccc gct gtc acc ggt gcg atc gtg gca ctc 547
Ile Asp Ala Val Met Pro Pro Ala Val Thr Gly Ala Ile Val Ala Leu
    135                 140                 145

atc ggc ctg aac ctg gca cca acc gcg gca gga aac ttc tcc agc caa 595
Ile Gly Leu Asn Leu Ala Pro Thr Ala Ala Gly Asn Phe Ser Ser Gln
150                 155                 160                 165

cca ctg gtt gcc acg gcg acc ctc ttt gcc att ttg atc gct acc gtt 643
Pro Leu Val Ala Thr Ala Thr Leu Phe Ala Ile Leu Ile Ala Thr Val
                170                 175                 180

gca ggc cgc gga atg att gct cgc ctg ggc att ttg atc ggt gtg gtg 691
Ala Gly Arg Gly Met Ile Ala Arg Leu Gly Ile Leu Ile Gly Val Val
            185                 190                 195

atc ggc tgg gtt ttc gca gct atc acc ggc aac ctc tca gaa ggc gca 739
Ile Gly Trp Val Phe Ala Ala Ile Thr Gly Asn Leu Ser Glu Gly Ala
        200                 205                 210

gca gac acc atc cgt gaa gca gca tgg ttc gga ctg cca cag ttc cac 787
Ala Asp Thr Ile Arg Glu Ala Ala Trp Phe Gly Leu Pro Gln Phe His
    215                 220                 225

aag ccg gaa ttc cag ctc tct gcc att ttg gtg aca ctg cca gtc atc 835
Lys Pro Glu Phe Gln Leu Ser Ala Ile Leu Val Thr Leu Pro Val Ile
230                 235                 240                 245

atc gtg ctc atc gct gaa aac gtc ggc cac gtc aaa gca gtc tca gag 883
Ile Val Leu Ile Ala Glu Asn Val Gly His Val Lys Ala Val Ser Glu
                250                 255                 260

atg aca ggg gag gac ctc gac gac ctc gcc ggc gac gca ctt atc gca 931
Met Thr Gly Glu Asp Leu Asp Asp Leu Ala Gly Asp Ala Leu Ile Ala
            265                 270                 275

gac gga ttc ggc acc acc ctc gca ggt gcc ttc ggt gga tcc ggc acc 979
Asp Gly Phe Gly Thr Thr Leu Ala Gly Ala Phe Gly Gly Ser Gly Thr
        280                 285                 290

acc acc tac gca gaa aac atc ggc gtc atg gcg gcc acc cgc gta tat 1027
Thr Thr Tyr Ala Glu Asn Ile Gly Val Met Ala Ala Thr Arg Val Tyr
    295                 300                 305

tcc acc gct gcg tac tgg gtt gcc gcg tgc act gcc atc gcc ctt gcc 1075
Ser Thr Ala Ala Tyr Trp Val Ala Ala Cys Thr Ala Ile Ala Leu Ala
310                 315                 320                 325

ttc atc ccc aaa ttc ggt gca ctg atc ttc acc atc ccc gcc ggc gtg 1123
Phe Ile Pro Lys Phe Gly Ala Leu Ile Phe Thr Ile Pro Ala Gly Val
                330                 335                 340

ctg ggt ggg gca tgt ttg gtt ctt tac ggc cta atc ggt atg ctc ggc 1171
Leu Gly Gly Ala Cys Leu Val Leu Tyr Gly Leu Ile Gly Met Leu Gly
            345                 350                 355

att cgt atc tgg caa gac aac aag gtc aac ttc aac aat cca gtg aat 1219
Ile Arg Ile Trp Gln Asp Asn Lys Val Asn Phe Asn Asn Pro Val Asn
        360                 365                 370

ctg acc atg gct gcc gtt gct ttg gtt gca ggc att ggt aac ctc acc 1267
Leu Thr Met Ala Ala Val Ala Leu Val Ala Gly Ile Gly Asn Leu Thr
    375                 380                 385

ctc acc gtt ttc gga gtc act ctt gaa ggc atc gca tgg gct ctg 1312
Leu Thr Val Phe Gly Val Thr Leu Glu Gly Ile Ala Trp Ala Leu
390                 395                 400

taggcatcat tgtgctgtac cca 1335

<210> 622
<211> 404
<212> PRT
<213> Corynebacterium glutamicum

<400> 622
Val Leu Val Thr Ser Thr Trp Gly Trp Thr Val His Gly Asp Gly Lys
1               5                   10                  15

Lys Ile Glu Pro Gly Ala Val Val Ala Pro Lys Glu Arg Leu Ser Trp
            20                  25                  30

Gly Arg Thr Ile Gly Ile Gly Met Gln His Val Ile Ala Met Phe Gly

35                  40                  45

Ala Thr Leu Leu Val Pro Thr Leu Thr Gly Phe Pro Val Asn Thr Thr
    50                  55                  60

Leu Leu Phe Ser Gly Leu Gly Thr Ile Leu Phe Leu Leu Ile Thr Arg
65                  70                  75                  80

Asn Arg Leu Pro Ser Tyr Leu Gly Ser Ser Phe Ala Phe Ile Ala Pro
                85                  90                  95

Leu Thr Ala Thr Gln Val His Gly Ile Gly Val Gln Ile Gly Gly Ile
            100                 105                 110

Leu Val Ala Gly Leu Val Leu Val Ala Ile Gly Phe Val Val Lys Ala
        115                 120                 125

Ala Gly Lys Arg Val Ile Asp Ala Val Met Pro Pro Ala Val Thr Gly
    130                 135                 140

Ala Ile Val Ala Leu Ile Gly Leu Asn Leu Ala Pro Thr Ala Ala Gly
145                 150                 155                 160

Asn Phe Ser Ser Gln Pro Leu Val Ala Thr Ala Thr Leu Phe Ala Ile
                165                 170                 175

Leu Ile Ala Thr Val Ala Gly Arg Gly Met Ile Ala Arg Leu Gly Ile
            180                 185                 190

Leu Ile Gly Val Val Ile Gly Trp Val Phe Ala Ala Ile Thr Gly Asn
        195                 200                 205

Leu Ser Glu Gly Ala Ala Asp Thr Ile Arg Glu Ala Ala Trp Phe Gly
    210                 215                 220

Leu Pro Gln Phe His Lys Pro Glu Phe Gln Leu Ser Ala Ile Leu Val
225                 230                 235                 240

Thr Leu Pro Val Ile Ile Val Leu Ile Ala Glu Asn Val Gly His Val
                245                 250                 255

Lys Ala Val Ser Glu Met Thr Gly Glu Asp Leu Asp Asp Leu Ala Gly
            260                 265                 270

Asp Ala Leu Ile Ala Asp Gly Phe Gly Thr Thr Leu Ala Gly Ala Phe
        275                 280                 285

Gly Gly Ser Gly Thr Thr Thr Tyr Ala Glu Asn Ile Gly Val Met Ala
    290                 295                 300

Ala Thr Arg Val Tyr Ser Thr Ala Ala Tyr Trp Val Ala Ala Cys Thr
305                 310                 315                 320

Ala Ile Ala Leu Ala Phe Ile Pro Lys Phe Gly Ala Leu Ile Phe Thr
                325                 330                 335

Ile Pro Ala Gly Val Leu Gly Gly Ala Cys Leu Val Leu Tyr Gly Leu
            340                 345                 350

Ile Gly Met Leu Gly Ile Arg Ile Trp Gln Asp Asn Lys Val Asn Phe
        355                 360                 365

Asn Asn Pro Val Asn Leu Thr Met Ala Ala Val Ala Leu Val Ala Gly
    370                 375                 380

Ile Gly Asn Leu Thr Leu Thr Val Phe Gly Val Thr Leu Glu Gly Ile
385                 390                 395                 400

Ala Trp Ala Leu

<210> 623
<211> 2010
<212> DNA
<213> Corynebacterium glutamicum

<220>
<221> CDS
<222> (101)..(1987)
<223> RXN02372

<400> 623
gacaatactg atggataaat ttcatatcga ggacgaaggg acaaccccga acgccgtgac 60

aacatcaacc acaacccggg tgaaacatcc ggtagaccag gtg cca ccc gca ccc    115
                                          Val Pro Pro Ala Pro
                                            1               5

aaa ctt gca gcc cta ggg ctc caa cac gtt ctt gct ttc tac gca gga    163
Lys Leu Ala Ala Leu Gly Leu Gln His Val Leu Ala Phe Tyr Ala Gly
                10                  15                  20

gcc gtc att gtt ccg ctg ctg att gca cag tcg ctg aac ttg gac act    211
Ala Val Ile Val Pro Leu Leu Ile Ala Gln Ser Leu Asn Leu Asp Thr
            25                  30                  35

gcg acc acc att cac ctg att aac gct gac ttg ttg aca tgt ggc atc    259
Ala Thr Thr Ile His Leu Ile Asn Ala Asp Leu Leu Thr Cys Gly Ile
        40                  45                  50

gcc acg ttg att cag tct gtg ggc att ggt cgc cac att ggt gtg cgc    307
Ala Thr Leu Ile Gln Ser Val Gly Ile Gly Arg His Ile Gly Val Arg
    55                  60                  65

cta ccg atc gtt caa ggt gtc acc act act gct gtt gct ccc atc atc    355
Leu Pro Ile Val Gln Gly Val Thr Thr Thr Ala Val Ala Pro Ile Ile
70                  75                  80                  85

gcc att ggt ttg ggc gtt act gat ggt caa ggt ggc gtt gcg tcg ctg    403
Ala Ile Gly Leu Gly Val Thr Asp Gly Gln Gly Gly Val Ala Ser Leu
                90                  95                  100

cct gcc att tac ggt gca gtc att gtc tcc ggc att ttc acg ttc ttt    451
Pro Ala Ile Tyr Gly Ala Val Ile Val Ser Gly Ile Phe Thr Phe Phe
            105                 110                 115

gca gcg ccg gtg ttt gcg cgt ttc ctc aag ttc ttc cca cca gtt gtc    499
Ala Ala Pro Val Phe Ala Arg Phe Leu Lys Phe Phe Pro Pro Val Val
        120                 125                 130

acc ggt act gtg ctg ttg gtt atg ggt gct tcc ctg ctg tcg gta tct    547

Thr Gly Thr Val Leu Leu Val Met Gly Ala Ser Leu Leu Ser Val Ser
    135                 140                 145

gca aat gac ttt gtg aac tac gcc gat ggg gtg cct gct gcc cgc gat     595
Ala Asn Asp Phe Val Asn Tyr Ala Asp Gly Val Pro Ala Ala Arg Asp
150                 155                 160                 165

ctt gct tac ggt ttt ggc acc ttg gcg gtg atc att ttg gcg cag cgc     643
Leu Ala Tyr Gly Phe Gly Thr Leu Ala Val Ile Ile Leu Ala Gln Arg
                170                 175                 180

ttc ttc cgt gga ttc atg ggc acc ttg gct gtg ttg atc ggc ctg gtt     691
Phe Phe Arg Gly Phe Met Gly Thr Leu Ala Val Leu Ile Gly Leu Val
            185                 190                 195

ggt ggc acc gca gtt gct ctg atc ttg ggc gat gcc aac ttg gat gag     739
Gly Gly Thr Ala Val Ala Leu Ile Leu Gly Asp Ala Asn Leu Asp Glu
        200                 205                 210

gtg gga aat gct gaa gcg ttc gac atc acc act ccg ttt tat ttt ggt     787
Val Gly Asn Ala Glu Ala Phe Asp Ile Thr Thr Pro Phe Tyr Phe Gly
    215                 220                 225

gtt cca gaa ttt aac gct gtt gcc att ttc tcc atg att atc gtc atg     835
Val Pro Glu Phe Asn Ala Val Ala Ile Phe Ser Met Ile Ile Val Met
230                 235                 240                 245

atc atc acc atg gtg gag acc acc ggt gat gtg ttt gca acg ggg gaa     883
Ile Ile Thr Met Val Glu Thr Thr Gly Asp Val Phe Ala Thr Gly Glu
                250                 255                 260

atc gtc ggc aag cga act cgc cgc agt gat gtc acc cgc gca ctg cgc     931
Ile Val Gly Lys Arg Thr Arg Arg Ser Asp Val Thr Arg Ala Leu Arg
            265                 270                 275

gct gac ggc ctg tcc acc ctg atg ggt ggc gtc atg aac tcc ttc ccg     979
Ala Asp Gly Leu Ser Thr Leu Met Gly Gly Val Met Asn Ser Phe Pro
        280                 285                 290

tac acg tgc ttc gcg caa aac gtt ggc ctg gtg cgc atc acg ggc gtg     1027
Tyr Thr Cys Phe Ala Gln Asn Val Gly Leu Val Arg Ile Thr Gly Val
    295                 300                 305

aaa tct cgc tgg gtt gcg gca gct gct gcc ggc ttc atg atc atc ctc     1075
Lys Ser Arg Trp Val Ala Ala Ala Ala Ala Gly Phe Met Ile Ile Leu
310                 315                 320                 325

ggt gtg ctg ccc aag gct ggc gcg atc gtc gct tcc atc cct tcc cca     1123
Gly Val Leu Pro Lys Ala Gly Ala Ile Val Ala Ser Ile Pro Ser Pro
                330                 335                 340

gtc ctc ggt ggc gca tcc ttg gca ctg ttc gcc aac gtt gca tgg gtg     1171
Val Leu Gly Gly Ala Ser Leu Ala Leu Phe Ala Asn Val Ala Trp Val
            345                 350                 355

ggc atc cag acc atc gcc aag tct gac ctc gct gat agc cgc aac tcc     1219
Gly Ile Gln Thr Ile Ala Lys Ser Asp Leu Ala Asp Ser Arg Asn Ser
        360                 365                 370

gtc atc gtg acc tcc gca ctt ggc cta gcc atg ctg gtg tcc ttc cgc     1267
Val Ile Val Thr Ser Ala Leu Gly Leu Ala Met Leu Val Ser Phe Arg

    375                 380                 385

ccc gat gtt gct cag gcg ttc cct gag tgg gcg cgt atc ttc gtc tcc 1315
Pro Asp Val Ala Gln Ala Phe Pro Glu Trp Ala Arg Ile Phe Val Ser
390                 395                 400                 405

tcc ggc atg tcc gtc ggc gcg atc acc gcg atc ctg ctt aac ctg ctg 1363
Ser Gly Met Ser Val Gly Ala Ile Thr Ala Ile Leu Leu Asn Leu Leu
                410                 415                 420

ttc ttc cac gtc gga cgc caa tcc ggt gga caa gtc gct acc tca aaa 1411
Phe Phe His Val Gly Arg Gln Ser Gly Gly Gln Val Ala Thr Ser Lys
            425                 430                 435

tcc ggt gag cgc atc aat ttg gat gcg gtc aac aag atg gat cgc acc 1459
Ser Gly Glu Arg Ile Asn Leu Asp Ala Val Asn Lys Met Asp Arg Thr
        440                 445                 450

gac ttc gta gaa acc ttc gca ccg ctg ttc aac agc aag acc tgg cct 1507
Asp Phe Val Glu Thr Phe Ala Pro Leu Phe Asn Ser Lys Thr Trp Pro
    455                 460                 465

ttg gaa acc gca tgg gaa tcc cag cca ttc gcc aac gtc acg gaa ctg 1555
Leu Glu Thr Ala Trp Glu Ser Gln Pro Phe Ala Asn Val Thr Glu Leu
470                 475                 480                 485

cgc gaa gcc atc caa gtc gct gtg ctc acc gca ccg ttg tcc gac cgc 1603
Arg Glu Ala Ile Gln Val Ala Val Leu Thr Ala Pro Leu Ser Asp Arg
                490                 495                 500

gaa gag ctc atc cac gac tac ccc gac atg gca cag ctc att ttg gcc 1651
Glu Glu Leu Ile His Asp Tyr Pro Asp Met Ala Gln Leu Ile Leu Ala
            505                 510                 515

acc gaa gag gaa gcc gcc acc atc tct caa gac cgt ggt tcg atc ggt 1699
Thr Glu Glu Glu Ala Ala Thr Ile Ser Gln Asp Arg Gly Ser Ile Gly
        520                 525                 530

ctt gat gat ctc gat gac gtg gat caa gaa aag ctc atc acc gtc acc 1747
Leu Asp Asp Leu Asp Asp Val Asp Gln Glu Lys Leu Ile Thr Val Thr
    535                 540                 545

gag cag tac cgc gaa cgg ttc aac atg ccg tat gtt gcg tac ttc gac 1795
Glu Gln Tyr Arg Glu Arg Phe Asn Met Pro Tyr Val Ala Tyr Phe Asp
550                 555                 560                 565

acc atg gat tct gtg gat acc gtc gta gcc gcc ggc ttg cgc cgc ctc 1843
Thr Met Asp Ser Val Asp Thr Val Val Ala Ala Gly Leu Arg Arg Leu
                570                 575                 580

gac aac tcc gac gag cag gag cac cgc caa gcg cta tcg gaa atc att 1891
Asp Asn Ser Asp Glu Gln Glu His Arg Gln Ala Leu Ser Glu Ile Ile
            585                 590                 595

gag att gcc aat gac cgc ttc gat ata ttg ctt gcc gac gct aac cca 1939
Glu Ile Ala Asn Asp Arg Phe Asp Ile Leu Leu Ala Asp Ala Asn Pro
        600                 605                 610

gcc cgt tca gct ttc gat cgc aag ttt acc gag act gac ttc ctc ggc 1987
Ala Arg Ser Ala Phe Asp Arg Lys Phe Thr Glu Thr Asp Phe Leu Gly
    615                 620                 625

taaaacacca aaaacaaatt aag                                          2010

<210> 624
<211> 629
<212> PRT
<213> Corynebacterium glutamicum

<400> 624
Val Pro Pro Ala Pro Lys Leu Ala Ala Leu Gly Leu Gln His Val Leu
1               5                   10                  15

Ala Phe Tyr Ala Gly Ala Val Ile Val Pro Leu Leu Ile Ala Gln Ser
            20                  25                  30

Leu Asn Leu Asp Thr Ala Thr Thr Ile His Leu Ile Asn Ala Asp Leu
        35                  40                  45

Leu Thr Cys Gly Ile Ala Thr Leu Ile Gln Ser Val Gly Ile Gly Arg
    50                  55                  60

His Ile Gly Val Arg Leu Pro Ile Val Gln Gly Val Thr Thr Thr Ala
65                  70                  75                  80

Val Ala Pro Ile Ile Ala Ile Gly Leu Gly Val Thr Asp Gly Gln Gly
                85                  90                  95

Gly Val Ala Ser Leu Pro Ala Ile Tyr Gly Ala Val Ile Val Ser Gly
            100                 105                 110

Ile Phe Thr Phe Phe Ala Ala Pro Val Phe Ala Arg Phe Leu Lys Phe
        115                 120                 125

Phe Pro Pro Val Val Thr Gly Thr Val Leu Leu Val Met Gly Ala Ser
    130                 135                 140

Leu Leu Ser Val Ser Ala Asn Asp Phe Val Asn Tyr Ala Asp Gly Val
145                 150                 155                 160

Pro Ala Ala Arg Asp Leu Ala Tyr Gly Phe Gly Thr Leu Ala Val Ile
                165                 170                 175

Ile Leu Ala Gln Arg Phe Phe Arg Gly Phe Met Gly Thr Leu Ala Val
            180                 185                 190

Leu Ile Gly Leu Val Gly Gly Thr Ala Val Ala Leu Ile Leu Gly Asp
        195                 200                 205

Ala Asn Leu Asp Glu Val Gly Asn Ala Glu Ala Phe Asp Ile Thr Thr
    210                 215                 220

Pro Phe Tyr Phe Gly Val Pro Glu Phe Asn Ala Val Ala Ile Phe Ser
225                 230                 235                 240

Met Ile Ile Val Met Ile Ile Thr Met Val Glu Thr Thr Gly Asp Val
                245                 250                 255

Phe Ala Thr Gly Glu Ile Val Gly Lys Arg Thr Arg Arg Ser Asp Val
            260                 265                 270

Thr Arg Ala Leu Arg Ala Asp Gly Leu Ser Thr Leu Met Gly Gly Val
        275                 280                 285

Met Asn Ser Phe Pro Tyr Thr Cys Phe Ala Gln Asn Val Gly Leu Val
    290                 295                 300

Arg Ile Thr Gly Val Lys Ser Arg Trp Val Ala Ala Ala Ala Ala Gly
305                 310                 315                 320

Phe Met Ile Ile Leu Gly Val Leu Pro Lys Ala Gly Ala Ile Val Ala
                325                 330                 335

Ser Ile Pro Ser Pro Val Leu Gly Gly Ala Ser Leu Ala Leu Phe Ala
            340                 345                 350

Asn Val Ala Trp Val Gly Ile Gln Thr Ile Ala Lys Ser Asp Leu Ala
        355                 360                 365

Asp Ser Arg Asn Ser Val Ile Val Thr Ser Ala Leu Gly Leu Ala Met
    370                 375                 380

Leu Val Ser Phe Arg Pro Asp Val Ala Gln Ala Phe Pro Glu Trp Ala
385                 390                 395                 400

Arg Ile Phe Val Ser Ser Gly Met Ser Val Gly Ala Ile Thr Ala Ile
                405                 410                 415

Leu Leu Asn Leu Leu Phe Phe His Val Gly Arg Gln Ser Gly Gly Gln
            420                 425                 430

Val Ala Thr Ser Lys Ser Gly Glu Arg Ile Asn Leu Asp Ala Val Asn
        435                 440                 445

Lys Met Asp Arg Thr Asp Phe Val Glu Thr Phe Ala Pro Leu Phe Asn
    450                 455                 460

Ser Lys Thr Trp Pro Leu Glu Thr Ala Trp Glu Ser Gln Pro Phe Ala
465                 470                 475                 480

Asn Val Thr Glu Leu Arg Glu Ala Ile Gln Val Ala Val Leu Thr Ala
                485                 490                 495

Pro Leu Ser Asp Arg Glu Glu Leu Ile His Asp Tyr Pro Asp Met Ala
            500                 505                 510

Gln Leu Ile Leu Ala Thr Glu Glu Glu Ala Ala Thr Ile Ser Gln Asp
        515                 520                 525

Arg Gly Ser Ile Gly Leu Asp Asp Leu Asp Asp Val Asp Gln Glu Lys
    530                 535                 540

Leu Ile Thr Val Thr Glu Gln Tyr Arg Glu Arg Phe Asn Met Pro Tyr
545                 550                 555                 560

Val Ala Tyr Phe Asp Thr Met Asp Ser Val Asp Thr Val Val Ala Ala
                565                 570                 575

Gly Leu Arg Arg Leu Asp Asn Ser Asp Glu Gln Glu His Arg Gln Ala
            580                 585                 590

Leu Ser Glu Ile Ile Glu Ile Ala Asn Asp Arg Phe Asp Ile Leu Leu

595                 600                 605

Ala Asp Ala Asn Pro Ala Arg Ser Ala Phe Asp Arg Lys Phe Thr Glu
    610                 615                 620

Thr Asp Phe Leu Gly
625


<210> 625
<211> 578
<212> DNA
<213> Corynebacterium glutamicum

<220>
<221> CDS
<222> (1)..(555)
<223> FRXA02372

<400> 625
gat gcg gtc aac aag atg gat cgc acc gac ttc gta gaa acc ttc gca      48
Asp Ala Val Asn Lys Met Asp Arg Thr Asp Phe Val Glu Thr Phe Ala
  1               5                  10                  15

ccg ctg ttc aac agc aag acc tgg cct ttg gaa acc gca tgg gaa tcc      96
Pro Leu Phe Asn Ser Lys Thr Trp Pro Leu Glu Thr Ala Trp Glu Ser
             20                  25                  30

cag cca ttc gcc aac gtc acg gaa ctg cgc gaa gcc atc caa gtc gct     144
Gln Pro Phe Ala Asn Val Thr Glu Leu Arg Glu Ala Ile Gln Val Ala
         35                  40                  45

gtg ctc acc gca ccg ttg tcc gac cgc gaa gag ctc atc cac gac tac     192
Val Leu Thr Ala Pro Leu Ser Asp Arg Glu Glu Leu Ile His Asp Tyr
     50                  55                  60

ccc gac atg gca cag ctc att ttg gcc acc gaa gag gaa gcc gcc acc     240
Pro Asp Met Ala Gln Leu Ile Leu Ala Thr Glu Glu Glu Ala Ala Thr
 65                  70                  75                  80

atc tct caa gac cgt ggt tcg atc ggt ctt gat gat ctc gat gac gtg     288
Ile Ser Gln Asp Arg Gly Ser Ile Gly Leu Asp Asp Leu Asp Asp Val
                 85                  90                  95

gat caa gaa aag ctc atc acc gtc acc gag cag tac cgc gaa cgg ttc     336
Asp Gln Glu Lys Leu Ile Thr Val Thr Glu Gln Tyr Arg Glu Arg Phe
            100                 105                 110

aac atg ccg tat gtt gcg tac ttc gac acc atg gat tct gtg gat acc     384
Asn Met Pro Tyr Val Ala Tyr Phe Asp Thr Met Asp Ser Val Asp Thr
        115                 120                 125

gtc gta gcc gcc ggc ttg cgc cgc ctc gac aac tcc gac gag cag gag     432
Val Val Ala Ala Gly Leu Arg Arg Leu Asp Asn Ser Asp Glu Gln Glu
    130                 135                 140

cac cgc caa gcg cta tcg gaa atc att gag att gcc aat gac cgc ttc     480
His Arg Gln Ala Leu Ser Glu Ile Ile Glu Ile Ala Asn Asp Arg Phe
145                 150                 155                 160

gat ata ttg ctt gcc gac gct aac cca gcc cgt tca gct ttc gat cgc     528

Asp Ile Leu Leu Ala Asp Ala Asn Pro Ala Arg Ser Ala Phe Asp Arg
                165                 170                 175

aag ttt acc gag act gac ttc ctc ggc taaaacacca aaaacaaatt          575
Lys Phe Thr Glu Thr Asp Phe Leu Gly
            180                 185

aag                                                                578

<210> 626
<211> 185
<212> PRT
<213> Corynebacterium glutamicum

<400> 626
Asp Ala Val Asn Lys Met Asp Arg Thr Asp Phe Val Glu Thr Phe Ala
  1               5                  10                  15

Pro Leu Phe Asn Ser Lys Thr Trp Pro Leu Glu Thr Ala Trp Glu Ser
             20                  25                  30

Gln Pro Phe Ala Asn Val Thr Glu Leu Arg Glu Ala Ile Gln Val Ala
         35                  40                  45

Val Leu Thr Ala Pro Leu Ser Asp Arg Glu Glu Leu Ile His Asp Tyr
     50                  55                  60

Pro Asp Met Ala Gln Leu Ile Leu Ala Thr Glu Glu Glu Ala Ala Thr
 65                  70                  75                  80

Ile Ser Gln Asp Arg Gly Ser Ile Gly Leu Asp Asp Leu Asp Asp Val
                 85                  90                  95

Asp Gln Glu Lys Leu Ile Thr Val Thr Glu Gln Tyr Arg Glu Arg Phe
            100                 105                 110

Asn Met Pro Tyr Val Ala Tyr Phe Asp Thr Met Asp Ser Val Asp Thr
        115                 120                 125

Val Val Ala Ala Gly Leu Arg Arg Leu Asp Asn Ser Asp Glu Gln Glu
    130                 135                 140

His Arg Gln Ala Leu Ser Glu Ile Ile Glu Ile Ala Asn Asp Arg Phe
145                 150                 155                 160

Asp Ile Leu Leu Ala Asp Ala Asn Pro Ala Arg Ser Ala Phe Asp Arg
                165                 170                 175

Lys Phe Thr Glu Thr Asp Phe Leu Gly
            180                 185

<210> 627
<211> 1314
<212> DNA
<213> Corynebacterium glutamicum

<220>
<221> CDS
<222> (101)..(1291)

<223> FRXA02377

<400> 627
gacaatactg atggataaat ttcatatcga ggacgaaggg acaaccccga acgccgtgac 60

aacatcaacc acaacccggg tgaaacatcc ggtagaccag gtg cca ccc gca ccc 115
Val Pro Pro Ala Pro
1               5

aaa ctt gca gcc cta ggg ctc caa cac gtt ctt gct ttc tac gca gga 163
Lys Leu Ala Ala Leu Gly Leu Gln His Val Leu Ala Phe Tyr Ala Gly
                10                  15                  20

gcc gtc att gtt ccg ctg ctg att gca cag tcg ctg aac ttg gac act 211
Ala Val Ile Val Pro Leu Leu Ile Ala Gln Ser Leu Asn Leu Asp Thr
            25                  30                  35

gcg acc acc att cac ctg att aac gct gac ttg ttg aca tgt ggc atc 259
Ala Thr Thr Ile His Leu Ile Asn Ala Asp Leu Leu Thr Cys Gly Ile
        40                  45                  50

gcc acg ttg att cag tct gtg ggc att ggt cgc cac att ggt gtg cgc 307
Ala Thr Leu Ile Gln Ser Val Gly Ile Gly Arg His Ile Gly Val Arg
    55                  60                  65

cta ccg atc gtt caa ggt gtc acc act act gct gtt gct ccc atc atc 355
Leu Pro Ile Val Gln Gly Val Thr Thr Thr Ala Val Ala Pro Ile Ile
70                  75                  80                  85

gcc att ggt ttg ggc gtt act gat ggt caa ggt ggc gtt gcg tcg ctg 403
Ala Ile Gly Leu Gly Val Thr Asp Gly Gln Gly Gly Val Ala Ser Leu
                90                  95                  100

cct gcc att tac ggt gca gtc att gtc tcc ggc att ttc acg ttc ttt 451
Pro Ala Ile Tyr Gly Ala Val Ile Val Ser Gly Ile Phe Thr Phe Phe
            105                 110                 115

gca gcg ccg gtg ttt gcg cgt ttc ctc aag ttc ttc cca cca gtt gtc 499
Ala Ala Pro Val Phe Ala Arg Phe Leu Lys Phe Phe Pro Pro Val Val
        120                 125                 130

acc ggt act gtg ctg ttg gtt atg ggt gct tcc ctg ctg tcg gta tct 547
Thr Gly Thr Val Leu Leu Val Met Gly Ala Ser Leu Leu Ser Val Ser
    135                 140                 145

gca aat gac ttt gtg aac tac gcc gat ggg gtg cct gct gcc cgc gat 595
Ala Asn Asp Phe Val Asn Tyr Ala Asp Gly Val Pro Ala Ala Arg Asp
150                 155                 160                 165

ctt gct tac ggt ttt ggc acc ttg gcg gtg atc att ttg gcg cag cgc 643
Leu Ala Tyr Gly Phe Gly Thr Leu Ala Val Ile Ile Leu Ala Gln Arg
                170                 175                 180

ttc ttc cgt gga ttc atg ggc acc ttg gct gtg ttg atc ggc ctg gtt 691
Phe Phe Arg Gly Phe Met Gly Thr Leu Ala Val Leu Ile Gly Leu Val
            185                 190                 195

ggt ggc acc gca gtt gct ctg atc ttg ggc gat gcc aac ttg gat gag 739
Gly Gly Thr Ala Val Ala Leu Ile Leu Gly Asp Ala Asn Leu Asp Glu
        200                 205                 210

gtg gga aat gct gaa gcg ttc gac atc acc act ccg ttt tat ttt ggt 787
Val Gly Asn Ala Glu Ala Phe Asp Ile Thr Thr Pro Phe Tyr Phe Gly
    215                 220                 225

gtt cca gaa ttt aac gct gtt gcc att ttc tcc atg att atc gtc atg 835
Val Pro Glu Phe Asn Ala Val Ala Ile Phe Ser Met Ile Ile Val Met
230                 235                 240                 245

atc atc acc atg gtg gag acc acc ggt gat gtg ttt gca acg ggg gaa 883
Ile Ile Thr Met Val Glu Thr Thr Gly Asp Val Phe Ala Thr Gly Glu
                250                 255                 260

atc gtc ggc aag cga act cgc cgc agt gat gtc acc cgc gca ctg cgc 931
Ile Val Gly Lys Arg Thr Arg Arg Ser Asp Val Thr Arg Ala Leu Arg
            265                 270                 275

gct gac ggc ctg tcc acc ctg atg ggt ggc gtc atg aac tcc ttc ccg 979
Ala Asp Gly Leu Ser Thr Leu Met Gly Gly Val Met Asn Ser Phe Pro
        280                 285                 290

tac acg tgc ttc gcg caa aac gtt ggc ctg gtg cgc atc acg ggc gtg 1027
Tyr Thr Cys Phe Ala Gln Asn Val Gly Leu Val Arg Ile Thr Gly Val
    295                 300                 305

aaa tct cgc tgg gtt gcg gca gct gct gcc ggc ttc atg atc atc ctc 1075
Lys Ser Arg Trp Val Ala Ala Ala Ala Ala Gly Phe Met Ile Ile Leu
310                 315                 320                 325

ggt gtg ctg ccc aag gct ggc gcg atc gtc gct tcc atc cct tcc cca 1123
Gly Val Leu Pro Lys Ala Gly Ala Ile Val Ala Ser Ile Pro Ser Pro
                330                 335                 340

gtc ctc ggt ggc gca tcc ttg gca ctg ttc gcc aac gtt gca tgg gtg 1171
Val Leu Gly Gly Ala Ser Leu Ala Leu Phe Ala Asn Val Ala Trp Val
            345                 350                 355

ggc atc cag acc atc gcc aag tct gac ctc gct gat agc cgc aac tcc 1219
Gly Ile Gln Thr Ile Ala Lys Ser Asp Leu Ala Asp Ser Arg Asn Ser
        360                 365                 370

gtc atc gtg acc tcc gca ctt ggc cta gcc atg ctg gtg tcc ttc cgc 1267
Val Ile Val Thr Ser Ala Leu Gly Leu Ala Met Leu Val Ser Phe Arg
    375                 380                 385

ccc gat gtt gct caa ggc gtt ccc tgagtgggcg cgtatcttcg tct 1314
Pro Asp Val Ala Gln Gly Val Pro
390                 395

<210> 628
<211> 397
<212> PRT
<213> Corynebacterium glutamicum

<400> 628
Val Pro Pro Ala Pro Lys Leu Ala Ala Leu Gly Leu Gln His Val Leu
1               5                   10                  15

Ala Phe Tyr Ala Gly Ala Val Ile Val Pro Leu Leu Ile Ala Gln Ser
            20                  25                  30

Leu Asn Leu Asp Thr Ala Thr Thr Ile His Leu Ile Asn Ala Asp Leu
        35                  40                  45

Leu Thr Cys Gly Ile Ala Thr Leu Ile Gln Ser Val Gly Ile Gly Arg
    50                  55                  60

His Ile Gly Val Arg Leu Pro Ile Val Gln Gly Val Thr Thr Thr Ala
65                  70                  75                  80

Val Ala Pro Ile Ile Ala Ile Gly Leu Gly Val Thr Asp Gly Gln Gly
                85                  90                  95

Gly Val Ala Ser Leu Pro Ala Ile Tyr Gly Ala Val Ile Val Ser Gly
            100                 105                 110

Ile Phe Thr Phe Phe Ala Ala Pro Val Phe Ala Arg Phe Leu Lys Phe
        115                 120                 125

Phe Pro Pro Val Val Thr Gly Thr Val Leu Leu Val Met Gly Ala Ser
    130                 135                 140

Leu Leu Ser Val Ser Ala Asn Asp Phe Val Asn Tyr Ala Asp Gly Val
145                 150                 155                 160

Pro Ala Ala Arg Asp Leu Ala Tyr Gly Phe Gly Thr Leu Ala Val Ile
                165                 170                 175

Ile Leu Ala Gln Arg Phe Phe Arg Gly Phe Met Gly Thr Leu Ala Val
            180                 185                 190

Leu Ile Gly Leu Val Gly Gly Thr Ala Val Ala Leu Ile Leu Gly Asp
        195                 200                 205

Ala Asn Leu Asp Glu Val Gly Asn Ala Glu Ala Phe Asp Ile Thr Thr
    210                 215                 220

Pro Phe Tyr Phe Gly Val Pro Glu Phe Asn Ala Val Ala Ile Phe Ser
225                 230                 235                 240

Met Ile Ile Val Met Ile Ile Thr Met Val Glu Thr Thr Gly Asp Val
                245                 250                 255

Phe Ala Thr Gly Glu Ile Val Gly Lys Arg Thr Arg Arg Ser Asp Val
            260                 265                 270

Thr Arg Ala Leu Arg Ala Asp Gly Leu Ser Thr Leu Met Gly Gly Val
        275                 280                 285

Met Asn Ser Phe Pro Tyr Thr Cys Phe Ala Gln Asn Val Gly Leu Val
    290                 295                 300

Arg Ile Thr Gly Val Lys Ser Arg Trp Val Ala Ala Ala Ala Ala Gly
305                 310                 315                 320

Phe Met Ile Ile Leu Gly Val Leu Pro Lys Ala Gly Ala Ile Val Ala
                325                 330                 335

Ser Ile Pro Ser Pro Val Leu Gly Gly Ala Ser Leu Ala Leu Phe Ala
            340                 345                 350

Asn Val Ala Trp Val Gly Ile Gln Thr Ile Ala Lys Ser Asp Leu Ala

355                 360                 365

Asp Ser Arg Asn Ser Val Ile Val Thr Ser Ala Leu Gly Leu Ala Met
    370                 375                 380

Leu Val Ser Phe Arg Pro Asp Val Ala Gln Gly Val Pro
385                 390                 395


<210> 629
<211> 1512
<212> DNA
<213> Corynebacterium glutamicum

<220>
<221> CDS
<222> (101)..(1489)
<223> RXA02676

<400> 629
aaccattggg tattaagaaa tttgtggctt agatctcaat ttctgtatag tttgatcata 60

ctaattcatc cacttccaaa ttttcacgaa ggatcacccc atg gac acc tgg gaa   115
                                           Met Asp Thr Trp Glu
                                           1               5

caa acc ctt gga aca ggg cca ctg cta ggc att gca gcc ggc gcc att   163
Gln Thr Leu Gly Thr Gly Pro Leu Leu Gly Ile Ala Ala Gly Ala Ile
                10                  15                  20

gcc ctc atc ttg gtt ctc gtc atc gtt ttt aaa ctc cat gct ttt ctc   211
Ala Leu Ile Leu Val Leu Val Ile Val Phe Lys Leu His Ala Phe Leu
            25                  30                  35

acc cta ata ctg gtt tca att gtt acc gca ctt gct gcc ggc att ccc   259
Thr Leu Ile Leu Val Ser Ile Val Thr Ala Leu Ala Ala Gly Ile Pro
        40                  45                  50

gtc acc gca gta gtg gac act ctc ctt gac ggt ttt ggt aaa aca ctc   307
Val Thr Ala Val Val Asp Thr Leu Leu Asp Gly Phe Gly Lys Thr Leu
    55                  60                  65

gcc tcg gtc gcc cta ttg gta ggc ctg ggt gcc atg ctt ggt cga ttg   355
Ala Ser Val Ala Leu Leu Val Gly Leu Gly Ala Met Leu Gly Arg Leu
70                  75                  80                  85

gtt gaa aca tcc ggt ggc gca aaa tct cta gcc gac act atg gtg cga   403
Val Glu Thr Ser Gly Gly Ala Lys Ser Leu Ala Asp Thr Met Val Arg
                90                  95                  100

atc ttc ggt gaa aaa cga gca gct ttc gca ctc ggt gtc gca tcg ctg   451
Ile Phe Gly Glu Lys Arg Ala Ala Phe Ala Leu Gly Val Ala Ser Leu
            105                 110                 115

atc atg gga ttc cct atc ttc ttc gat gct ggc ctc gtg gtc atg ctc   499
Ile Met Gly Phe Pro Ile Phe Phe Asp Ala Gly Leu Val Val Met Leu
        120                 125                 130

cca gtg atc ttc gca gta gct cga cgc ctc aac ggc tcc gtc ctt act   547
Pro Val Ile Phe Ala Val Ala Arg Arg Leu Asn Gly Ser Val Leu Thr
    135                 140                 145

ttt ggt atc cct gca gct ggc gcc ttc tct gtc atg cac gtg ttc gtc 595
Phe Gly Ile Pro Ala Ala Gly Ala Phe Ser Val Met His Val Phe Val
150                 155                 160                 165

cca cct cac cca ggc cca att gca gcc tct gaa ttc ttc ggc gca caa 643
Pro Pro His Pro Gly Pro Ile Ala Ala Ser Glu Phe Phe Gly Ala Gln
                170                 175                 180

gtt gga tac gta cta atc gct ggc atc atc gtt gca cta ccc acc tgg 691
Val Gly Tyr Val Leu Ile Ala Gly Ile Ile Val Ala Leu Pro Thr Trp
            185                 190                 195

tat tta acc ggt tac ctg cta ggt aag ttc tta ggc cga aag ttc ccc 739
Tyr Leu Thr Gly Tyr Leu Leu Gly Lys Phe Leu Gly Arg Lys Phe Pro
        200                 205                 210

ctt ccc gta ccc gat cta ctc agt ggt gga gca cag gaa gat gat cag 787
Leu Pro Val Pro Asp Leu Leu Ser Gly Gly Ala Gln Glu Asp Asp Gln
    215                 220                 225

cct cag aac cca gct aac gca gtg tcg atc att gtc att ttg ctc att 835
Pro Gln Asn Pro Ala Asn Ala Val Ser Ile Ile Val Ile Leu Leu Ile
230                 235                 240                 245

cct atg ctc ctt att ttt ggc aat acc gga aca tca atg gca gtt tcc 883
Pro Met Leu Leu Ile Phe Gly Asn Thr Gly Thr Ser Met Ala Val Ser
                250                 255                 260

gcc ggc ctc cta gat gca gaa tcc acc atg gtg aaa att cta gga ttc 931
Ala Gly Leu Leu Asp Ala Glu Ser Thr Met Val Lys Ile Leu Gly Phe
            265                 270                 275

ctc ggc gaa aca cca gtg gca ctg ctc att acc ttg atc att gcc ctg 979
Leu Gly Glu Thr Pro Val Ala Leu Leu Ile Thr Leu Ile Ile Ala Leu
        280                 285                 290

ttc ttc cta ggc aac cga cgt ggc att aat ggt tct gct cta gag aaa 1027
Phe Phe Leu Gly Asn Arg Arg Gly Ile Asn Gly Ser Ala Leu Glu Lys
    295                 300                 305

acc atc gaa ggc gca ctc ggc cca atc tgt tca gtc gta tta atc act 1075
Thr Ile Glu Gly Ala Leu Gly Pro Ile Cys Ser Val Val Leu Ile Thr
310                 315                 320                 325

ggc gcc ggt ggc atg ttc ggt gga gtg cta cgc acg tct gga att gga 1123
Gly Ala Gly Gly Met Phe Gly Gly Val Leu Arg Thr Ser Gly Ile Gly
                330                 335                 340

gga gcg ctt gca gac tcc atg gca gat cta gga ctt cca gtt atc gca 1171
Gly Ala Leu Ala Asp Ser Met Ala Asp Leu Gly Leu Pro Val Ile Ala
            345                 350                 355

ggt tgt ttc atc gtg gca gcc gtc ctt cgt gtc gcg cag ggt tct gcc 1219
Gly Cys Phe Ile Val Ala Ala Val Leu Arg Val Ala Gln Gly Ser Ala
        360                 365                 370

acc gtt gcg cta acc acc gcc gca gca ctc atg gca cct gct gtt gcc 1267
Thr Val Ala Leu Thr Thr Ala Ala Ala Leu Met Ala Pro Ala Val Ala
    375                 380                 385

gct gct gac ttt aac gaa ttc cag ctt gct gcc atc gtt att tcc act 1315
Ala Ala Asp Phe Asn Glu Phe Gln Leu Ala Ala Ile Val Ile Ser Thr
390                 395                 400                 405

gcc gct ggt tct gtt att gcc agc cac gtc aac gac tcc gga ttc tgg 1363
Ala Ala Gly Ser Val Ile Ala Ser His Val Asn Asp Ser Gly Phe Trp
                410                 415                 420

ctc gtt ggt cga ctc atg aac gcc gac gta ccc acc acg cta aaa act 1411
Leu Val Gly Arg Leu Met Asn Ala Asp Val Pro Thr Thr Leu Lys Thr
            425                 430                 435

tgg acc gta aac caa acc tgc att gcg att gtg gga ttt gtg atg gcc 1459
Trp Thr Val Asn Gln Thr Cys Ile Ala Ile Val Gly Phe Val Met Ala
        440                 445                 450

tat gca atg ttc gga ttg gca tcg ctt gca tagtccgctg accccattga 1509
Tyr Ala Met Phe Gly Leu Ala Ser Leu Ala
    455                 460

ttc 1512

<210> 630
<211> 463
<212> PRT
<213> Corynebacterium glutamicum

<400> 630
Met Asp Thr Trp Glu Gln Thr Leu Gly Thr Gly Pro Leu Leu Gly Ile
1               5                   10                  15

Ala Ala Gly Ala Ile Ala Leu Ile Leu Val Leu Val Ile Val Phe Lys
            20                  25                  30

Leu His Ala Phe Leu Thr Leu Ile Leu Val Ser Ile Val Thr Ala Leu
        35                  40                  45

Ala Ala Gly Ile Pro Val Thr Ala Val Val Asp Thr Leu Leu Asp Gly
    50                  55                  60

Phe Gly Lys Thr Leu Ala Ser Val Ala Leu Leu Val Gly Leu Gly Ala
65                  70                  75                  80

Met Leu Gly Arg Leu Val Glu Thr Ser Gly Gly Ala Lys Ser Leu Ala
                85                  90                  95

Asp Thr Met Val Arg Ile Phe Gly Glu Lys Arg Ala Ala Phe Ala Leu
            100                 105                 110

Gly Val Ala Ser Leu Ile Met Gly Phe Pro Ile Phe Phe Asp Ala Gly
        115                 120                 125

Leu Val Val Met Leu Pro Val Ile Phe Ala Val Ala Arg Arg Leu Asn
    130                 135                 140

Gly Ser Val Leu Thr Phe Gly Ile Pro Ala Ala Gly Ala Phe Ser Val
145                 150                 155                 160

Met His Val Phe Val Pro Pro His Pro Gly Pro Ile Ala Ala Ser Glu
                165                 170                 175

Phe Phe Gly Ala Gln Val Gly Tyr Val Leu Ile Ala Gly Ile Ile Val
            180                 185                 190

Ala Leu Pro Thr Trp Tyr Leu Thr Gly Tyr Leu Leu Gly Lys Phe Leu
        195                 200                 205

Gly Arg Lys Phe Pro Leu Pro Val Pro Asp Leu Leu Ser Gly Gly Ala
    210                 215                 220

Gln Glu Asp Asp Gln Pro Gln Asn Pro Ala Asn Ala Val Ser Ile Ile
225                 230                 235                 240

Val Ile Leu Leu Ile Pro Met Leu Leu Ile Phe Gly Asn Thr Gly Thr
                245                 250                 255

Ser Met Ala Val Ser Ala Gly Leu Leu Asp Ala Glu Ser Thr Met Val
            260                 265                 270

Lys Ile Leu Gly Phe Leu Gly Glu Thr Pro Val Ala Leu Leu Ile Thr
        275                 280                 285

Leu Ile Ile Ala Leu Phe Phe Leu Gly Asn Arg Arg Gly Ile Asn Gly
    290                 295                 300

Ser Ala Leu Glu Lys Thr Ile Glu Gly Ala Leu Gly Pro Ile Cys Ser
305                 310                 315                 320

Val Val Leu Ile Thr Gly Ala Gly Gly Met Phe Gly Gly Val Leu Arg
                325                 330                 335

Thr Ser Gly Ile Gly Gly Ala Leu Ala Asp Ser Met Ala Asp Leu Gly
            340                 345                 350

Leu Pro Val Ile Ala Gly Cys Phe Ile Val Ala Ala Val Leu Arg Val
        355                 360                 365

Ala Gln Gly Ser Ala Thr Val Ala Leu Thr Thr Ala Ala Ala Leu Met
    370                 375                 380

Ala Pro Ala Val Ala Ala Ala Asp Phe Asn Glu Phe Gln Leu Ala Ala
385                 390                 395                 400

Ile Val Ile Ser Thr Ala Ala Gly Ser Val Ile Ala Ser His Val Asn
                405                 410                 415

Asp Ser Gly Phe Trp Leu Val Gly Arg Leu Met Asn Ala Asp Val Pro
            420                 425                 430

Thr Thr Leu Lys Thr Trp Thr Val Asn Gln Thr Cys Ile Ala Ile Val
        435                 440                 445

Gly Phe Val Met Ala Tyr Ala Met Phe Gly Leu Ala Ser Leu Ala
    450                 455                 460


<210> 631
<211> 1608
<212> DNA
<213> Corynebacterium glutamicum

<220>
<221> CDS
<222> (101)..(1585)
<223> RXN00432

<400> 631
agctaatctg ccttgcttta cacctccggg ctataacaat ccagtgccag tggctaggga 60

tttactaaaa atcgggtaac acgcgcgtag tatttttcgc atg gaa tta ttg gag 115
Met Glu Leu Leu Glu
1               5

acc ttc atc act gat gtc att aat gac aat ttg tgg atg atc ttg ccc 163
Thr Phe Ile Thr Asp Val Ile Asn Asp Asn Leu Trp Met Ile Leu Pro
                10                  15                  20

ttc ttg ctc gtt gct gct ggc ctc tat ttc ggt ggg cgt acg ttg ctg 211
Phe Leu Leu Val Ala Ala Gly Leu Tyr Phe Gly Gly Arg Thr Leu Leu
            25                  30                  35

gtt cag att cgg atg att ccg gag atg ttc aaa gcg gtc gtc gag aag 259
Val Gln Ile Arg Met Ile Pro Glu Met Phe Lys Ala Val Val Glu Lys
        40                  45                  50

cct gcg aag gat ggg gag ttc gcg gac aag cag gac att tcg gct ttt 307
Pro Ala Lys Asp Gly Glu Phe Ala Asp Lys Gln Asp Ile Ser Ala Phe
    55                  60                  65

aag gcg ttc acg att tct gcg gcg tcg cga gtt ggt acg gcg aat gtt 355
Lys Ala Phe Thr Ile Ser Ala Ala Ser Arg Val Gly Thr Ala Asn Val
70                  75                  80                  85

gcg ggt gtt gcg ctg gcg atc act ctg ggt gga ccg ggt gca gtg ttc 403
Ala Gly Val Ala Leu Ala Ile Thr Leu Gly Gly Pro Gly Ala Val Phe
                90                  95                  100

tgg atg tgg atc att gcg ctg gtt ggc ggt gcg aca tcg ttc att gag 451
Trp Met Trp Ile Ile Ala Leu Val Gly Gly Ala Thr Ser Phe Ile Glu
            105                 110                 115

tcg act ctt gga cag ttg tgg aag gtg aag gac ggc gac agc tat cgc 499
Ser Thr Leu Gly Gln Leu Trp Lys Val Lys Asp Gly Asp Ser Tyr Arg
        120                 125                 130

ggt ggc cct gcg tac tac atg acg ctt ggt ttg aat gct cgg tgg ctt 547
Gly Gly Pro Ala Tyr Tyr Met Thr Leu Gly Leu Asn Ala Arg Trp Leu
    135                 140                 145

gcg gtt gtt ttc ggt gtc gcc atc acg ttg acc ttt ggt ttt gtg tac 595
Ala Val Val Phe Gly Val Ala Ile Thr Leu Thr Phe Gly Phe Val Tyr
150                 155                 160                 165

aac gct ttg cag tcc aac gcg gtt gtt gag gcg att acg gta tca ctc 643
Asn Ala Leu Gln Ser Asn Ala Val Val Glu Ala Ile Thr Val Ser Leu
                170                 175                 180

ggc acc cct tct acc act gca aag gct ttt gtt ggc ttg ggc atg gct 691
Gly Thr Pro Ser Thr Thr Ala Lys Ala Phe Val Gly Leu Gly Met Ala
            185                 190                 195

gga ttg tct gcg ctg gtt att ttt ggc ggc gtg cag cgt atc gca aac 739

Gly Leu Ser Ala Leu Val Ile Phe Gly Gly Val Gln Arg Ile Ala Asn
        200                 205                 210

gtg acg cag tgg atg gtt ccg ttc atg gcg ggt gcg tac atc att gtg     787
Val Thr Gln Trp Met Val Pro Phe Met Ala Gly Ala Tyr Ile Ile Val
    215                 220                 225

ggt gtg gtg gtg att gtg att aac att cag cag gtt ccg acc atg atc     835
Gly Val Val Val Ile Val Ile Asn Ile Gln Gln Val Pro Thr Met Ile
230                 235                 240                 245

aac gac atc att gct ggt gct ttt ggt ttc cgt ccg gtt gct act gcg     883
Asn Asp Ile Ile Ala Gly Ala Phe Gly Phe Arg Pro Val Ala Thr Ala
                250                 255                 260

tcg gtg tgg ggc gcg ttc tgg ttg gcg ttt atg aac ggt atg cgc cgt     931
Ser Val Trp Gly Ala Phe Trp Leu Ala Phe Met Asn Gly Met Arg Arg
            265                 270                 275

gga ctg ttc tcc aat gag gct ggt gag ggt tct gtc ccg aac gct gct     979
Gly Leu Phe Ser Asn Glu Ala Gly Glu Gly Ser Val Pro Asn Ala Ala
        280                 285                 290

gct acc gcg act gtg tct cac cct gtg aag cag ggt ttg gtt cag act     1027
Ala Thr Ala Thr Val Ser His Pro Val Lys Gln Gly Leu Val Gln Thr
    295                 300                 305

ctg ggc gta tat ttc gac acc ctg ctc gtt tgt agc att acc gct ttt     1075
Leu Gly Val Tyr Phe Asp Thr Leu Leu Val Cys Ser Ile Thr Ala Phe
310                 315                 320                 325

gtc atc ctg ctg tct gga gtg gag tac gcg acc ggc gat att cag tct     1123
Val Ile Leu Leu Ser Gly Val Glu Tyr Ala Thr Gly Asp Ile Gln Ser
                330                 335                 340

tct tct ttg act cag tcc gcg ctg gct agc gtt gtt ggt ggt tgg gga     1171
Ser Ser Leu Thr Gln Ser Ala Leu Ala Ser Val Val Gly Gly Trp Gly
            345                 350                 355

acc cac ttc att acc gta gtg atg ttc ttc ctg gcg ttt tct tcc gtg     1219
Thr His Phe Ile Thr Val Val Met Phe Phe Leu Ala Phe Ser Ser Val
        360                 365                 370

ctg ggt aac tac tac ttg gca cag gcg aat att cag tac ttc acc gat     1267
Leu Gly Asn Tyr Tyr Leu Ala Gln Ala Asn Ile Gln Tyr Phe Thr Asp
    375                 380                 385

tcg aag act gtc atg act gtt ttc cga ctc ttg gtg ctg ctc agc gtg     1315
Ser Lys Thr Val Met Thr Val Phe Arg Leu Leu Val Leu Leu Ser Val
390                 395                 400                 405

ttc tct ggc gcg gtt gct tcg gtg ccg ttg atc tgg gct ttg ggt gat     1363
Phe Ser Gly Ala Val Ala Ser Val Pro Leu Ile Trp Ala Leu Gly Asp
                410                 415                 420

act ttc gct ggc atc atg gtg ctc att aac ctg gcg gcg atc att ccg     1411
Thr Phe Ala Gly Ile Met Val Leu Ile Asn Leu Ala Ala Ile Ile Pro
            425                 430                 435

ctg ggt ggc gtt gca gtg aag ttg ctt aag aat tac acc att cag aag     1459
Leu Gly Gly Val Ala Val Lys Leu Leu Lys Asn Tyr Thr Ile Gln Lys

440                 445                 450

aag gct ggt ctg gat cct gtg ttc cac cgc gac atg atg cca gag gtt     1507
Lys Ala Gly Leu Asp Pro Val Phe His Arg Asp Met Met Pro Glu Val
    455                 460                 465

cgt aat att gcg tgc tgg aac ggc aaa gat gca gct aca tcc aac tat     1555
Arg Asn Ile Ala Cys Trp Asn Gly Lys Asp Ala Ala Thr Ser Asn Tyr
470                 475                 480                 485

cac gaa gcg atg gaa gtg atc aag aag agc tagtcatcga aggaacagtg       1605
His Glu Ala Met Glu Val Ile Lys Lys Ser
                490                 495

gta                                                                 1608

<210> 632
<211> 495
<212> PRT
<213> Corynebacterium glutamicum

<400> 632
Met Glu Leu Leu Glu Thr Phe Ile Thr Asp Val Ile Asn Asp Asn Leu
1               5                   10                  15

Trp Met Ile Leu Pro Phe Leu Leu Val Ala Ala Gly Leu Tyr Phe Gly
            20                  25                  30

Gly Arg Thr Leu Leu Val Gln Ile Arg Met Ile Pro Glu Met Phe Lys
        35                  40                  45

Ala Val Val Glu Lys Pro Ala Lys Asp Gly Glu Phe Ala Asp Lys Gln
    50                  55                  60

Asp Ile Ser Ala Phe Lys Ala Phe Thr Ile Ser Ala Ala Ser Arg Val
65                  70                  75                  80

Gly Thr Ala Asn Val Ala Gly Val Ala Leu Ala Ile Thr Leu Gly Gly
                85                  90                  95

Pro Gly Ala Val Phe Trp Met Trp Ile Ile Ala Leu Val Gly Gly Ala
            100                 105                 110

Thr Ser Phe Ile Glu Ser Thr Leu Gly Gln Leu Trp Lys Val Lys Asp
        115                 120                 125

Gly Asp Ser Tyr Arg Gly Gly Pro Ala Tyr Tyr Met Thr Leu Gly Leu
    130                 135                 140

Asn Ala Arg Trp Leu Ala Val Val Phe Gly Val Ala Ile Thr Leu Thr
145                 150                 155                 160

Phe Gly Phe Val Tyr Asn Ala Leu Gln Ser Asn Ala Val Val Glu Ala
                165                 170                 175

Ile Thr Val Ser Leu Gly Thr Pro Ser Thr Thr Ala Lys Ala Phe Val
            180                 185                 190

Gly Leu Gly Met Ala Gly Leu Ser Ala Leu Val Ile Phe Gly Gly Val
        195                 200                 205

Gln Arg Ile Ala Asn Val Thr Gln Trp Met Val Pro Phe Met Ala Gly
    210                 215                 220

Ala Tyr Ile Ile Val Gly Val Val Val Ile Val Ile Asn Ile Gln Gln
225                 230                 235                 240

Val Pro Thr Met Ile Asn Asp Ile Ile Ala Gly Ala Phe Gly Phe Arg
                245                 250                 255

Pro Val Ala Thr Ala Ser Val Trp Gly Ala Phe Trp Leu Ala Phe Met
            260                 265                 270

Asn Gly Met Arg Arg Gly Leu Phe Ser Asn Glu Ala Gly Glu Gly Ser
        275                 280                 285

Val Pro Asn Ala Ala Ala Thr Ala Thr Val Ser His Pro Val Lys Gln
    290                 295                 300

Gly Leu Val Gln Thr Leu Gly Val Tyr Phe Asp Thr Leu Leu Val Cys
305                 310                 315                 320

Ser Ile Thr Ala Phe Val Ile Leu Leu Ser Gly Val Glu Tyr Ala Thr
                325                 330                 335

Gly Asp Ile Gln Ser Ser Ser Leu Thr Gln Ser Ala Leu Ala Ser Val
            340                 345                 350

Val Gly Gly Trp Gly Thr His Phe Ile Thr Val Val Met Phe Phe Leu
        355                 360                 365

Ala Phe Ser Ser Val Leu Gly Asn Tyr Tyr Leu Ala Gln Ala Asn Ile
    370                 375                 380

Gln Tyr Phe Thr Asp Ser Lys Thr Val Met Thr Val Phe Arg Leu Leu
385                 390                 395                 400

Val Leu Leu Ser Val Phe Ser Gly Ala Val Ala Ser Val Pro Leu Ile
                405                 410                 415

Trp Ala Leu Gly Asp Thr Phe Ala Gly Ile Met Val Leu Ile Asn Leu
            420                 425                 430

Ala Ala Ile Ile Pro Leu Gly Gly Val Ala Val Lys Leu Leu Lys Asn
        435                 440                 445

Tyr Thr Ile Gln Lys Lys Ala Gly Leu Asp Pro Val Phe His Arg Asp
    450                 455                 460

Met Met Pro Glu Val Arg Asn Ile Ala Cys Trp Asn Gly Lys Asp Ala
465                 470                 475                 480

Ala Thr Ser Asn Tyr His Glu Ala Met Glu Val Ile Lys Lys Ser
                485                 490                 495

<210> 633
<211> 914
<212> DNA
<213> Corynebacterium glutamicum

<220>
<221> CDS
<222> (1)..(891)
<223> FRXA00432

<400> 633
ttg tct gcg ctg gtt att ttt ggc ggc gtg cag cgt atc gca aac gtg     48
Leu Ser Ala Leu Val Ile Phe Gly Gly Val Gln Arg Ile Ala Asn Val
1               5                   10                  15

acg cag tgg atg gtt ccg ttc atg gcg ggt gcg tac atc att gtg ggt     96
Thr Gln Trp Met Val Pro Phe Met Ala Gly Ala Tyr Ile Ile Val Gly
            20                  25                  30

gtg gtg gtg att gtg att aac att cag cag gtt ccg acc atg atc aac     144
Val Val Val Ile Val Ile Asn Ile Gln Gln Val Pro Thr Met Ile Asn
        35                  40                  45

gac atc att gct ggt gct ttt ggt ttc cgt ccg gtt gct act gcg tcg     192
Asp Ile Ile Ala Gly Ala Phe Gly Phe Arg Pro Val Ala Thr Ala Ser
    50                  55                  60

gtg tgg ggc gcg ttc tgg ttg gcg ttt atg aac ggt atg cgc cgt gga     240
Val Trp Gly Ala Phe Trp Leu Ala Phe Met Asn Gly Met Arg Arg Gly
65                  70                  75                  80

ctg ttc tcc aat gag gct ggt gag ggt tct gtc ccg aac gct gct gct     288
Leu Phe Ser Asn Glu Ala Gly Glu Gly Ser Val Pro Asn Ala Ala Ala
                85                  90                  95

acc gcg act gtg tct cac cct gtg aag cag ggt ttg gtt cag act ctg     336
Thr Ala Thr Val Ser His Pro Val Lys Gln Gly Leu Val Gln Thr Leu
            100                 105                 110

ggc gta tat ttc gac acc ctg ctc gtt tgt agc att acc gct ttt gtc     384
Gly Val Tyr Phe Asp Thr Leu Leu Val Cys Ser Ile Thr Ala Phe Val
        115                 120                 125

atc ctg ctg tct gga gtg gag tac gcg acc ggc gat att cag tct tct     432
Ile Leu Leu Ser Gly Val Glu Tyr Ala Thr Gly Asp Ile Gln Ser Ser
    130                 135                 140

tct ttg act cag tcc gcg ctg gct agc gtt gtt ggt ggt tgg gga acc     480
Ser Leu Thr Gln Ser Ala Leu Ala Ser Val Val Gly Gly Trp Gly Thr
145                 150                 155                 160

cac ttc att acc gta gtg atg ttc ttc ctg gcg ttt tct tcc gtg ctg     528
His Phe Ile Thr Val Val Met Phe Phe Leu Ala Phe Ser Ser Val Leu
                165                 170                 175

ggt aac tac tac ttg gca cag gcg aat att cag tac ttc acc gat tcg     576
Gly Asn Tyr Tyr Leu Ala Gln Ala Asn Ile Gln Tyr Phe Thr Asp Ser
            180                 185                 190

aag act gtc atg act gtt ttc cga ctc ttg gtg ctg ctc agc gtg ttc     624
Lys Thr Val Met Thr Val Phe Arg Leu Leu Val Leu Leu Ser Val Phe
        195                 200                 205

tct ggc gcg gtt gct tcg gtg ccg ttg atc tgg gct ttg ggt gat act     672
Ser Gly Ala Val Ala Ser Val Pro Leu Ile Trp Ala Leu Gly Asp Thr
    210                 215                 220

ttc gct ggc atc atg gtg ctc att aac ctg gcg gcg atc att ccg ctg 720
Phe Ala Gly Ile Met Val Leu Ile Asn Leu Ala Ala Ile Ile Pro Leu
225                 230                 235                 240

ggt ggc gtt gca gtg aag ttg ctt aag aat tac acc att cag aag aag 768
Gly Gly Val Ala Val Lys Leu Leu Lys Asn Tyr Thr Ile Gln Lys Lys
                245                 250                 255

gct ggt ctg gat cct gtg ttc cac cgc gac atg atg cca gag gtt cgt 816
Ala Gly Leu Asp Pro Val Phe His Arg Asp Met Met Pro Glu Val Arg
            260                 265                 270

aat att gcg tgc tgg aac ggc aaa gat gca gct aca tcc aac tat cac 864
Asn Ile Ala Cys Trp Asn Gly Lys Asp Ala Ala Thr Ser Asn Tyr His
        275                 280                 285

gaa gcg atg gaa gtg atc aag aag agc tagtcatcga aggaacagtg 911
Glu Ala Met Glu Val Ile Lys Lys Ser
    290                 295

gta 914

<210> 634
<211> 297
<212> PRT
<213> Corynebacterium glutamicum

<400> 634
Leu Ser Ala Leu Val Ile Phe Gly Gly Val Gln Arg Ile Ala Asn Val
1               5                   10                  15

Thr Gln Trp Met Val Pro Phe Met Ala Gly Ala Tyr Ile Ile Val Gly
            20                  25                  30

Val Val Val Ile Val Ile Asn Ile Gln Gln Val Pro Thr Met Ile Asn
        35                  40                  45

Asp Ile Ile Ala Gly Ala Phe Gly Phe Arg Pro Val Ala Thr Ala Ser
    50                  55                  60

Val Trp Gly Ala Phe Trp Leu Ala Phe Met Asn Gly Met Arg Arg Gly
65                  70                  75                  80

Leu Phe Ser Asn Glu Ala Gly Glu Gly Ser Val Pro Asn Ala Ala Ala
                85                  90                  95

Thr Ala Thr Val Ser His Pro Val Lys Gln Gly Leu Val Gln Thr Leu
            100                 105                 110

Gly Val Tyr Phe Asp Thr Leu Leu Val Cys Ser Ile Thr Ala Phe Val
        115                 120                 125

Ile Leu Leu Ser Gly Val Glu Tyr Ala Thr Gly Asp Ile Gln Ser Ser
    130                 135                 140

Ser Leu Thr Gln Ser Ala Leu Ala Ser Val Val Gly Gly Trp Gly Thr
145                 150                 155                 160

His Phe Ile Thr Val Val Met Phe Phe Leu Ala Phe Ser Ser Val Leu

                165                 170                 175

Gly Asn Tyr Tyr Leu Ala Gln Ala Asn Ile Gln Tyr Phe Thr Asp Ser
            180                 185                 190

Lys Thr Val Met Thr Val Phe Arg Leu Leu Val Leu Leu Ser Val Phe
        195                 200                 205

Ser Gly Ala Val Ala Ser Val Pro Leu Ile Trp Ala Leu Gly Asp Thr
    210                 215                 220

Phe Ala Gly Ile Met Val Leu Ile Asn Leu Ala Ala Ile Ile Pro Leu
225                 230                 235                 240

Gly Gly Val Ala Val Lys Leu Leu Lys Asn Tyr Thr Ile Gln Lys Lys
                245                 250                 255

Ala Gly Leu Asp Pro Val Phe His Arg Asp Met Met Pro Glu Val Arg
            260                 265                 270

Asn Ile Ala Cys Trp Asn Gly Lys Asp Ala Ala Thr Ser Asn Tyr His
        275                 280                 285

Glu Ala Met Glu Val Ile Lys Lys Ser
    290                 295

<210> 635
<211> 569
<212> DNA
<213> Corynebacterium glutamicum

<220>
<221> CDS
<222> (45)..(569)
<223> FRXA00436

<400> 635
gggatttact aaaaatcggg taacacgcgc gtagtatttt tcgcatg gaa tta ttg 56
Met Glu Leu Leu
1

gag acc ttc atc act gat gtc att aat gac aat ttg tgg atg atc ttg 104
Glu Thr Phe Ile Thr Asp Val Ile Asn Asp Asn Leu Trp Met Ile Leu
5                   10                  15                  20

ccc ttc ttg ctc gtt gct gct ggc ctc tat ttc ggt ggg cgt acg ttg 152
Pro Phe Leu Leu Val Ala Ala Gly Leu Tyr Phe Gly Gly Arg Thr Leu
                25                  30                  35

ctg gtt cag att cgg atg att ccg gag atg ttc aaa gcg gtc gtc gag 200
Leu Val Gln Ile Arg Met Ile Pro Glu Met Phe Lys Ala Val Val Glu
            40                  45                  50

aag cct gcg aag gat ggg gag ttc gcg gac aag cag gac att tcg gct 248
Lys Pro Ala Lys Asp Gly Glu Phe Ala Asp Lys Gln Asp Ile Ser Ala
        55                  60                  65

ttt aag gcg ttc acg att tct gcg gcg tcg cga gtt ggt acg gcg aat 296
Phe Lys Ala Phe Thr Ile Ser Ala Ala Ser Arg Val Gly Thr Ala Asn
    70                  75                  80

gtt gcg ggt gtt gcg ctg gcg atc act ctg ggt gga ccg ggt gca gtg    344
Val Ala Gly Val Ala Leu Ala Ile Thr Leu Gly Gly Pro Gly Ala Val
 85                 90                  95                 100

ttc tgg atg tgg atc att gcg ctg gtt ggc ggt gcg aca tcg ttc att    392
Phe Trp Met Trp Ile Ile Ala Leu Val Gly Gly Ala Thr Ser Phe Ile
                105                 110                 115

gag tcg act ctt gga cag ttg tgg aag gtg aag gac ggc gac agc tat    440
Glu Ser Thr Leu Gly Gln Leu Trp Lys Val Lys Asp Gly Asp Ser Tyr
            120                 125                 130

cgc ggt ggc cct gcg tac tac atg acg ctt ggt ttg aat gct cgg tgg    488
Arg Gly Gly Pro Ala Tyr Tyr Met Thr Leu Gly Leu Asn Ala Arg Trp
        135                 140                 145

ctt gcg gtt gtt ttc ggt gtc gcc atc acg ttg acc ttt ggt ttt gtg    536
Leu Ala Val Val Phe Gly Val Ala Ile Thr Leu Thr Phe Gly Phe Val
    150                 155                 160

tac aac gct ttg cag tcc aac gcg gtt gtt gag                        569
Tyr Asn Ala Leu Gln Ser Asn Ala Val Val Glu
165                 170                 175

<210> 636
<211> 175
<212> PRT
<213> Corynebacterium glutamicum

<400> 636
Met Glu Leu Leu Glu Thr Phe Ile Thr Asp Val Ile Asn Asp Asn Leu
 1               5                  10                  15

Trp Met Ile Leu Pro Phe Leu Leu Val Ala Ala Gly Leu Tyr Phe Gly
            20                  25                  30

Gly Arg Thr Leu Leu Val Gln Ile Arg Met Ile Pro Glu Met Phe Lys
        35                  40                  45

Ala Val Val Glu Lys Pro Ala Lys Asp Gly Glu Phe Ala Asp Lys Gln
    50                  55                  60

Asp Ile Ser Ala Phe Lys Ala Phe Thr Ile Ser Ala Ala Ser Arg Val
65                  70                  75                  80

Gly Thr Ala Asn Val Ala Gly Val Ala Leu Ala Ile Thr Leu Gly Gly
                85                  90                  95

Pro Gly Ala Val Phe Trp Met Trp Ile Ile Ala Leu Val Gly Gly Ala
            100                 105                 110

Thr Ser Phe Ile Glu Ser Thr Leu Gly Gln Leu Trp Lys Val Lys Asp
        115                 120                 125

Gly Asp Ser Tyr Arg Gly Gly Pro Ala Tyr Tyr Met Thr Leu Gly Leu
    130                 135                 140

Asn Ala Arg Trp Leu Ala Val Val Phe Gly Val Ala Ile Thr Leu Thr
145                 150                 155                 160

Phe Gly Phe Val Tyr Asn Ala Leu Gln Ser Asn Ala Val Val Glu
                165                 170                 175

<210> 637
<211> 1572
<212> DNA
<213> Corynebacterium glutamicum

<220>
<221> CDS
<222> (101)..(1549)
<223> RXA00847

<400> 637
tctggcccgc tgtgtcctct ggacgcagcg ggccagtagt attttggggg tgagaataag 60

gtttggaatt ctaagtgttg tcgcctgcac ggtttcgttg gtg gca tgt cag gct 115
Val Ala Cys Gln Ala
1               5

aat ccg ggt cct gcg ccc gtt gag gag cca act aca gcc act gcg acg 163
Asn Pro Gly Pro Ala Pro Val Glu Glu Pro Thr Thr Ala Thr Ala Thr
                10                  15                  20

acc acg gca act gag acg acc acc gta gag act gaa gcc ccc aaa caa 211
Thr Thr Ala Thr Glu Thr Thr Thr Val Glu Thr Glu Ala Pro Lys Gln
            25                  30                  35

gat cgg gaa acc atc agt att ggt atc gat ccg atc cgc aac ggt ttc 259
Asp Arg Glu Thr Ile Ser Ile Gly Ile Asp Pro Ile Arg Asn Gly Phe
        40                  45                  50

aat cca cat ttg ttg tct gat gat tct cca ctg gtg cgc gat act gca 307
Asn Pro His Leu Leu Ser Asp Asp Ser Pro Leu Val Arg Asp Thr Ala
    55                  60                  65

tcg ttg gtg ttg ccg agt gct ttt gag ggc aat cag ctg aac act gac 355
Ser Leu Val Leu Pro Ser Ala Phe Glu Gly Asn Gln Leu Asn Thr Asp
70                  75                  80                  85

ttg ttg gac aat gtt gag cag gtg gat gaa acc acg gtg agg tac acg 403
Leu Leu Asp Asn Val Glu Gln Val Asp Glu Thr Thr Val Arg Tyr Thr
                90                  95                  100

att gct cag gaa gcg cag tgg agt gat ggc act ccg att act ggg tct 451
Ile Ala Gln Glu Ala Gln Trp Ser Asp Gly Thr Pro Ile Thr Gly Ser
            105                 110                 115

gat ttt gag tac ttg cgc agg tcc att gtg gcg ggg aca gga acg ctg 499
Asp Phe Glu Tyr Leu Arg Arg Ser Ile Val Ala Gly Thr Gly Thr Leu
        120                 125                 130

aac gat tct gca tat tcg gcg att tca gag att agg acc tca ggc ggt 547
Asn Asp Ser Ala Tyr Ser Ala Ile Ser Glu Ile Arg Thr Ser Gly Gly
    135                 140                 145

gga aaa act gtc gac gtt att ttt gaa cat cct gtc gct gat tgg cat 595
Gly Lys Thr Val Asp Val Ile Phe Glu His Pro Val Ala Asp Trp His
150                 155                 160                 165

ttg ctg ttt aac aat ttg ttg ccg agt cat ttg atc act ggc aat tcc 643
Leu Leu Phe Asn Asn Leu Leu Pro Ser His Leu Ile Thr Gly Asn Ser
                170                 175                 180

aca ttc cag aca gcg ttt tat gat tcc atc ccg gcc tct gcg ggg cgc 691
Thr Phe Gln Thr Ala Phe Tyr Asp Ser Ile Pro Ala Ser Ala Gly Arg
            185                 190                 195

tac atg gtg cgt tcc att gat cgt cag cgc ggc gtg att acg ttg tcg 739
Tyr Met Val Arg Ser Ile Asp Arg Gln Arg Gly Val Ile Thr Leu Ser
        200                 205                 210

cgc aat gat cgt ttc tgg ggt gca aat ccg gca cac gtg gaa gta ctc 787
Arg Asn Asp Arg Phe Trp Gly Ala Asn Pro Ala His Val Glu Val Leu
    215                 220                 225

caa ttc aac acc gtt gcc tct gca tcc cgg gcg ggg gag tac ctg cgc 835
Gln Phe Asn Thr Val Ala Ser Ala Ser Arg Ala Gly Glu Tyr Leu Arg
230                 235                 240                 245

acc ggg cag agt tcg ttt atg aat ctg tca ccg cag gaa acc ctg gtg 883
Thr Gly Gln Ser Ser Phe Met Asn Leu Ser Pro Gln Glu Thr Leu Val
                250                 255                 260

gac acg ttg aat ttg gtg ccg gac acg gaa gtg cgg gtg tct gac acg 931
Asp Thr Leu Asn Leu Val Pro Asp Thr Glu Val Arg Val Ser Asp Thr
            265                 270                 275

acg cgc acg ctg gag ctg gta ttc aac gcc gaa gcg ctg gca ccg gcg 979
Thr Arg Thr Leu Glu Leu Val Phe Asn Ala Glu Ala Leu Ala Pro Ala
        280                 285                 290

cag cgc gcc tac ctg aca tcg ctt atc gac gtc ccc ctc acc gcc aag 1027
Gln Arg Ala Tyr Leu Thr Ser Leu Ile Asp Val Pro Leu Thr Ala Lys
    295                 300                 305

tta gct ggc ggt cgc agt gca aac ctg ggt gtg ccc caa acg gtg gag 1075
Leu Ala Gly Gly Arg Ser Ala Asn Leu Gly Val Pro Gln Thr Val Glu
310                 315                 320                 325

gcg agc gtc gat aag caa gaa att cct gcg ttg cgc ctg gcg gcg gac 1123
Ala Ser Val Asp Lys Gln Glu Ile Pro Ala Leu Arg Leu Ala Ala Asp
                330                 335                 340

ccc gcc gac gat gct ggt ttg gca gct gcg cgg ggc atc gtc gat atg 1171
Pro Ala Asp Asp Ala Gly Leu Ala Ala Ala Arg Gly Ile Val Asp Met
            345                 350                 355

ctg gct gcg gat gga atc aag gct cag gtc gtg aca acc gat ttg aat 1219
Leu Ala Ala Asp Gly Ile Lys Ala Gln Val Val Thr Thr Asp Leu Asn
        360                 365                 370

tcg gcg att gcg ggt aat ttt gat gcg atc gtg gcg tgg acc aga act 1267
Ser Ala Ile Ala Gly Asn Phe Asp Ala Ile Val Ala Trp Thr Arg Thr
    375                 380                 385

gcc acg gat tcg att gcg ttg gcg gac cga gtg ggt tgt ggc gtg aac 1315
Ala Thr Asp Ser Ile Ala Leu Ala Asp Arg Val Gly Cys Gly Val Asn
390                 395                 400                 405

ttg gca aag tgg tgc gct gag gga acc acg gaa tat atc aac ggt gtt 1363
Leu Ala Lys Trp Cys Ala Glu Gly Thr Thr Glu Tyr Ile Asn Gly Val
                410                 415                 420

ttg gcg ggt gag att gat ttc gat cct gcg tgg gag cag cag ttt aac 1411
Leu Ala Gly Glu Ile Asp Phe Asp Pro Ala Trp Glu Gln Gln Phe Asn
            425                 430                 435

acc gag aat cac ttg cgg gtt ccg atc ctg cgg gag act cgg gta gaa 1459
Thr Glu Asn His Leu Arg Val Pro Ile Leu Arg Glu Thr Arg Val Glu
        440                 445                 450

gcg aaa aat aac ggt atc ctc ggg gct gcg gat ggc tgg cct ggg gga 1507
Ala Lys Asn Asn Gly Ile Leu Gly Ala Ala Asp Gly Trp Pro Gly Gly
    455                 460                 465

att tca agt gct gca agt tgg agg aaa aac gat gtt gaa gaa 1549
Ile Ser Ser Ala Ala Ser Trp Arg Lys Asn Asp Val Glu Glu
470                 475                 480

tgatctgtct ggtgctcgag ttg 1572

<210> 638
<211> 483
<212> PRT
<213> Corynebacterium glutamicum

<400> 638
Val Ala Cys Gln Ala Asn Pro Gly Pro Ala Pro Val Glu Glu Pro Thr
1               5                   10                  15

Thr Ala Thr Ala Thr Thr Thr Ala Thr Glu Thr Thr Thr Val Glu Thr
            20                  25                  30

Glu Ala Pro Lys Gln Asp Arg Glu Thr Ile Ser Ile Gly Ile Asp Pro
        35                  40                  45

Ile Arg Asn Gly Phe Asn Pro His Leu Leu Ser Asp Asp Ser Pro Leu
    50                  55                  60

Val Arg Asp Thr Ala Ser Leu Val Leu Pro Ser Ala Phe Glu Gly Asn
65                  70                  75                  80

Gln Leu Asn Thr Asp Leu Leu Asp Asn Val Glu Gln Val Asp Glu Thr
                85                  90                  95

Thr Val Arg Tyr Thr Ile Ala Gln Glu Ala Gln Trp Ser Asp Gly Thr
            100                 105                 110

Pro Ile Thr Gly Ser Asp Phe Glu Tyr Leu Arg Arg Ser Ile Val Ala
        115                 120                 125

Gly Thr Gly Thr Leu Asn Asp Ser Ala Tyr Ser Ala Ile Ser Glu Ile
    130                 135                 140

Arg Thr Ser Gly Gly Gly Lys Thr Val Asp Val Ile Phe Glu His Pro
145                 150                 155                 160

Val Ala Asp Trp His Leu Leu Phe Asn Asn Leu Leu Pro Ser His Leu
                165                 170                 175

Ile Thr Gly Asn Ser Thr Phe Gln Thr Ala Phe Tyr Asp Ser Ile Pro
            180                 185                 190

Ala Ser Ala Gly Arg Tyr Met Val Arg Ser Ile Asp Arg Gln Arg Gly
        195                 200                 205

Val Ile Thr Leu Ser Arg Asn Asp Arg Phe Trp Gly Ala Asn Pro Ala
    210                 215                 220

His Val Glu Val Leu Gln Phe Asn Thr Val Ala Ser Ala Ser Arg Ala
225                 230                 235                 240

Gly Glu Tyr Leu Arg Thr Gly Gln Ser Ser Phe Met Asn Leu Ser Pro
                245                 250                 255

Gln Glu Thr Leu Val Asp Thr Leu Asn Leu Val Pro Asp Thr Glu Val
            260                 265                 270

Arg Val Ser Asp Thr Thr Arg Thr Leu Glu Leu Val Phe Asn Ala Glu
        275                 280                 285

Ala Leu Ala Pro Ala Gln Arg Ala Tyr Leu Thr Ser Leu Ile Asp Val
    290                 295                 300

Pro Leu Thr Ala Lys Leu Ala Gly Gly Arg Ser Ala Asn Leu Gly Val
305                 310                 315                 320

Pro Gln Thr Val Glu Ala Ser Val Asp Lys Gln Glu Ile Pro Ala Leu
                325                 330                 335

Arg Leu Ala Ala Asp Pro Ala Asp Asp Ala Gly Leu Ala Ala Ala Arg
            340                 345                 350

Gly Ile Val Asp Met Leu Ala Ala Asp Gly Ile Lys Ala Gln Val Val
        355                 360                 365

Thr Thr Asp Leu Asn Ser Ala Ile Ala Gly Asn Phe Asp Ala Ile Val
    370                 375                 380

Ala Trp Thr Arg Thr Ala Thr Asp Ser Ile Ala Leu Ala Asp Arg Val
385                 390                 395                 400

Gly Cys Gly Val Asn Leu Ala Lys Trp Cys Ala Glu Gly Thr Thr Glu
                405                 410                 415

Tyr Ile Asn Gly Val Leu Ala Gly Glu Ile Asp Phe Asp Pro Ala Trp
            420                 425                 430

Glu Gln Gln Phe Asn Thr Glu Asn His Leu Arg Val Pro Ile Leu Arg
        435                 440                 445

Glu Thr Arg Val Glu Ala Lys Asn Asn Gly Ile Leu Gly Ala Ala Asp
    450                 455                 460

Gly Trp Pro Gly Gly Ile Ser Ser Ala Ala Ser Trp Arg Lys Asn Asp
465                 470                 475                 480

Val Glu Glu

<210> 639
<211> 1192
<212> DNA
<213> Corynebacterium glutamicum

<220>
<221> CDS
<222> (101)..(1192)
<223> RXN01382

<400> 639
aaaggatgta cctcatgagc accacgatta ctcgccgcaa tttcctccga gcaaccggaa 60

tcctcggtgt cgcagctggc atcggcgcaa cacttgctgc atg tgc acc aac aac 115
Met Cys Thr Asn Asn
1               5

acc ggc acc agc gga tcc acc tcc acc gca gct ggc acc gga act gca 163
Thr Gly Thr Ser Gly Ser Thr Ser Thr Ala Ala Gly Thr Gly Thr Ala
                10                  15                  20

aat gaa gaa ggc acc atc acc gca gcc atc tcc tac gaa ctg gga acc 211
Asn Glu Glu Gly Thr Ile Thr Ala Ala Ile Ser Tyr Glu Leu Gly Thr
            25                  30                  35

aac ggc tac gac cca atg acc acc acc tcg gct ctc act gtg gca gcc 259
Asn Gly Tyr Asp Pro Met Thr Thr Thr Ser Ala Leu Thr Val Ala Ala
        40                  45                  50

aac tgg cac acc ctt gaa ggc ctc acc gaa atc gac cca gcc acc ggc 307
Asn Trp His Thr Leu Glu Gly Leu Thr Glu Ile Asp Pro Ala Thr Gly
    55                  60                  65

gag gtc tac gca gca ctc gcc agc gca ctg cct tca gcc gat gcg acc 355
Glu Val Tyr Ala Ala Leu Ala Ser Ala Leu Pro Ser Ala Asp Ala Thr
70                  75                  80                  85

tcc cta gac atc aag ctt cgc gac ggc gcc acc ttc cac aac ggc gac 403
Ser Leu Asp Ile Lys Leu Arg Asp Gly Ala Thr Phe His Asn Gly Asp
                90                  95                  100

gca gtc acc gca gac gac gta gtc ttc tct ttt gag cga gtc ctc gat 451
Ala Val Thr Ala Asp Asp Val Val Phe Ser Phe Glu Arg Val Leu Asp
            105                 110                 115

cca gcc aac aac tcc ctt tac gca tcc ttc atc cca ttc atc aag tca 499
Pro Ala Asn Asn Ser Leu Tyr Ala Ser Phe Ile Pro Phe Ile Lys Ser
        120                 125                 130

gtc acc aag aag gat gac acc acc gtc acc atc gac ctc gac tac gca 547
Val Thr Lys Lys Asp Asp Thr Thr Val Thr Ile Asp Leu Asp Tyr Ala
    135                 140                 145

acc ggc atc atc agc gaa cgc ctc gca gta gta aag atc gtg cca aag 595
Thr Gly Ile Ile Ser Glu Arg Leu Ala Val Val Lys Ile Val Pro Lys
150                 155                 160                 165

tcc gtc gtg gaa gca gac gca tcc gga ttc gac gcc aac cca atc ggc 643
Ser Val Val Glu Ala Asp Ala Ser Gly Phe Asp Ala Asn Pro Ile Gly
                170                 175                 180

tcc ggc cca tac aag atg acc gac aac ggt gca tcc aag gtg gtc aag     691
Ser Gly Pro Tyr Lys Met Thr Asp Asn Gly Ala Ser Lys Val Val Lys
            185                 190                 195

ttc gag cgc aac gac gac tac aac ggc cca cgc cca gca cgt gcc gcc     739
Phe Glu Arg Asn Asp Asp Tyr Asn Gly Pro Arg Pro Ala Arg Ala Ala
        200                 205                 210

aag atg gaa tgg cag atc atc ccc gac gcc tcc acc cgc acc aac tcc     787
Lys Met Glu Trp Gln Ile Ile Pro Asp Ala Ser Thr Arg Thr Asn Ser
    215                 220                 225

ctt cag tcc ggc agc acc atg gcc atc gac tcc gtt cca tac ctg tcg     835
Leu Gln Ser Gly Ser Thr Met Ala Ile Asp Ser Val Pro Tyr Leu Ser
230                 235                 240                 245

atc cca cag ctt gaa gcc acc agc acc gta gaa tcc gtc caa gga ttc     883
Ile Pro Gln Leu Glu Ala Thr Ser Thr Val Glu Ser Val Gln Gly Phe
                250                 255                 260

ggc ctc ctg ttc gcc atg ttc agc tgc tcc gaa ggc aac ccc ttc aac     931
Gly Leu Leu Phe Ala Met Phe Ser Cys Ser Glu Gly Asn Pro Phe Asn
            265                 270                 275

gac gtc cgc aac cgc cag gca ttc ctc tac gca ctg gac atg gac aag     979
Asp Val Arg Asn Arg Gln Ala Phe Leu Tyr Ala Leu Asp Met Asp Lys
        280                 285                 290

atc gtt aag acc ggc atg tcc gac cag gca acc cca gcc acc tcc ttc     1027
Ile Val Lys Thr Gly Met Ser Asp Gln Ala Thr Pro Ala Thr Ser Phe
    295                 300                 305

gtg cag aag gaa cac ccc aac tac aac cag gca tcc acg gtc tac tcc     1075
Val Gln Lys Glu His Pro Asn Tyr Asn Gln Ala Ser Thr Val Tyr Ser
310                 315                 320                 325

ctc gac gcc gac aaa gcc aag gcg ctg ttc gct gaa acc ggc ctt acc     1123
Leu Asp Ala Asp Lys Ala Lys Ala Leu Phe Ala Glu Thr Gly Leu Thr
                330                 335                 340

agc ctc aac ctc ctg tgc acc gac cac gac tgg gtc aag aac tgc acc     1171
Ser Leu Asn Leu Leu Cys Thr Asp His Asp Trp Val Lys Asn Cys Thr
            345                 350                 355

cca ctg atc cag gaa tcc ctc                                         1192
Pro Leu Ile Gln Glu Ser Leu
        360

<210> 640
<211> 364
<212> PRT
<213> Corynebacterium glutamicum

<400> 640
Met Cys Thr Asn Asn Thr Gly Thr Ser Gly Ser Thr Ser Thr Ala Ala
  1               5                  10                  15

Gly Thr Gly Thr Ala Asn Glu Glu Gly Thr Ile Thr Ala Ala Ile Ser
             20                  25                  30

Tyr Glu Leu Gly Thr Asn Gly Tyr Asp Pro Met Thr Thr Thr Ser Ala
        35                  40                  45

Leu Thr Val Ala Ala Asn Trp His Thr Leu Glu Gly Leu Thr Glu Ile
    50                  55                  60

Asp Pro Ala Thr Gly Glu Val Tyr Ala Ala Leu Ala Ser Ala Leu Pro
65                  70                  75                  80

Ser Ala Asp Ala Thr Ser Leu Asp Ile Lys Leu Arg Asp Gly Ala Thr
                85                  90                  95

Phe His Asn Gly Asp Ala Val Thr Ala Asp Asp Val Val Phe Ser Phe
            100                 105                 110

Glu Arg Val Leu Asp Pro Ala Asn Asn Ser Leu Tyr Ala Ser Phe Ile
        115                 120                 125

Pro Phe Ile Lys Ser Val Thr Lys Lys Asp Asp Thr Thr Val Thr Ile
    130                 135                 140

Asp Leu Asp Tyr Ala Thr Gly Ile Ile Ser Glu Arg Leu Ala Val Val
145                 150                 155                 160

Lys Ile Val Pro Lys Ser Val Val Glu Ala Asp Ala Ser Gly Phe Asp
                165                 170                 175

Ala Asn Pro Ile Gly Ser Gly Pro Tyr Lys Met Thr Asp Asn Gly Ala
            180                 185                 190

Ser Lys Val Val Lys Phe Glu Arg Asn Asp Asp Tyr Asn Gly Pro Arg
        195                 200                 205

Pro Ala Arg Ala Ala Lys Met Glu Trp Gln Ile Ile Pro Asp Ala Ser
    210                 215                 220

Thr Arg Thr Asn Ser Leu Gln Ser Gly Ser Thr Met Ala Ile Asp Ser
225                 230                 235                 240

Val Pro Tyr Leu Ser Ile Pro Gln Leu Glu Ala Thr Ser Thr Val Glu
                245                 250                 255

Ser Val Gln Gly Phe Gly Leu Leu Phe Ala Met Phe Ser Cys Ser Glu
            260                 265                 270

Gly Asn Pro Phe Asn Asp Val Arg Asn Arg Gln Ala Phe Leu Tyr Ala
        275                 280                 285

Leu Asp Met Asp Lys Ile Val Lys Thr Gly Met Ser Asp Gln Ala Thr
    290                 295                 300

Pro Ala Thr Ser Phe Val Gln Lys Glu His Pro Asn Tyr Asn Gln Ala
305                 310                 315                 320

Ser Thr Val Tyr Ser Leu Asp Ala Asp Lys Ala Lys Ala Leu Phe Ala
                325                 330                 335

Glu Thr Gly Leu Thr Ser Leu Asn Leu Leu Cys Thr Asp His Asp Trp
            340                 345                 350

Val Lys Asn Cys Thr Pro Leu Ile Gln Glu Ser Leu
        355                 360

<210> 641
<211> 1062
<212> DNA
<213> Corynebacterium glutamicum

<220>
<221> CDS
<222> (1)..(1062)
<223> FRXA01382

<400> 641
tcc acc tcc acc gca gct ggc acc gga act gca aat gaa gaa ggc acc      48
Ser Thr Ser Thr Ala Ala Gly Thr Gly Thr Ala Asn Glu Glu Gly Thr
  1               5                  10                  15

atc acc gca gcc atc tcc tac gaa ctg gga acc aac ggc tac gac cca      96
Ile Thr Ala Ala Ile Ser Tyr Glu Leu Gly Thr Asn Gly Tyr Asp Pro
             20                  25                  30

atg acc acc acc tcg gct ctc act gtg gca gcc aac tgg cac acc ctt     144
Met Thr Thr Thr Ser Ala Leu Thr Val Ala Ala Asn Trp His Thr Leu
         35                  40                  45

gaa ggc ctc acc gaa atc gac cca gcc acc ggc gag gtc tac gca gca     192
Glu Gly Leu Thr Glu Ile Asp Pro Ala Thr Gly Glu Val Tyr Ala Ala
     50                  55                  60

ctc gcc agc gca ctg cct tca gcc gat gcg acc tcc cta gac atc aag     240
Leu Ala Ser Ala Leu Pro Ser Ala Asp Ala Thr Ser Leu Asp Ile Lys
 65                  70                  75                  80

ctt cgc gac ggc gcc acc ttc cac aac ggc gac gca gtc acc gca gac     288
Leu Arg Asp Gly Ala Thr Phe His Asn Gly Asp Ala Val Thr Ala Asp
                 85                  90                  95

gac gta gtc ttc tct ttt gag cga gtc ctc gat cca gcc aac aac tcc     336
Asp Val Val Phe Ser Phe Glu Arg Val Leu Asp Pro Ala Asn Asn Ser
            100                 105                 110

ctt tac gca tcc ttc atc cca ttc atc aag tca gtc acc aag aag gat     384
Leu Tyr Ala Ser Phe Ile Pro Phe Ile Lys Ser Val Thr Lys Lys Asp
        115                 120                 125

gac acc acc gtc acc atc gac ctc gac tac gca acc ggc atc atc agc     432
Asp Thr Thr Val Thr Ile Asp Leu Asp Tyr Ala Thr Gly Ile Ile Ser
    130                 135                 140

gaa cgc ctc gca gta gta aag atc gtg cca aag tcc gtc gtg gaa gca     480
Glu Arg Leu Ala Val Val Lys Ile Val Pro Lys Ser Val Val Glu Ala
145                 150                 155                 160

gac gca tcc gga ttc gac gcc aac cca atc ggc tcc ggc cca tac aag     528
Asp Ala Ser Gly Phe Asp Ala Asn Pro Ile Gly Ser Gly Pro Tyr Lys
                165                 170                 175

atg acc gac aac ggt gca tcc aag gtg gtc aag ttc gag cgc aac gac     576
Met Thr Asp Asn Gly Ala Ser Lys Val Val Lys Phe Glu Arg Asn Asp

180                 185                 190

gac tac aac ggc cca cgc cca gca cgt gcc gcc aag atg gaa tgg cag     624
Asp Tyr Asn Gly Pro Arg Pro Ala Arg Ala Ala Lys Met Glu Trp Gln
        195                 200                 205

atc atc ccc gac gcc tcc acc cgc acc aac tcc ctt cag tcc ggc agc     672
Ile Ile Pro Asp Ala Ser Thr Arg Thr Asn Ser Leu Gln Ser Gly Ser
    210                 215                 220

acc atg gcc atc gac tcc gtt cca tac ctg tcg atc cca cag ctt gaa     720
Thr Met Ala Ile Asp Ser Val Pro Tyr Leu Ser Ile Pro Gln Leu Glu
225                 230                 235                 240

gcc acc agc acc gta gaa tcc gtc caa gga ttc ggc ctc ctg ttc gcc     768
Ala Thr Ser Thr Val Glu Ser Val Gln Gly Phe Gly Leu Leu Phe Ala
                245                 250                 255

atg ttc agc tgc tcc gaa ggc aac ccc ttc aac gac gtc cgc aac cgc     816
Met Phe Ser Cys Ser Glu Gly Asn Pro Phe Asn Asp Val Arg Asn Arg
            260                 265                 270

cag gca ttc ctc tac gca ctg gac atg gac aag atc gtt aag acc ggc     864
Gln Ala Phe Leu Tyr Ala Leu Asp Met Asp Lys Ile Val Lys Thr Gly
        275                 280                 285

atg tcc gac cag gca acc cca gcc acc tcc ttc gtg cag aag gaa cac     912
Met Ser Asp Gln Ala Thr Pro Ala Thr Ser Phe Val Gln Lys Glu His
    290                 295                 300

ccc aac tac aac cag gca tcc acg gtc tac tcc ctc gac gcc gac aaa     960
Pro Asn Tyr Asn Gln Ala Ser Thr Val Tyr Ser Leu Asp Ala Asp Lys
305                 310                 315                 320

gcc aag gcg ctg ttc gct gaa acc ggc ctt acc agc ctc aac ctc ctg     1008
Ala Lys Ala Leu Phe Ala Glu Thr Gly Leu Thr Ser Leu Asn Leu Leu
                325                 330                 335

tgc acc gac cac gac tgg gtc aag aac tgc acc cca ctg atc cag gaa     1056
Cys Thr Asp His Asp Trp Val Lys Asn Cys Thr Pro Leu Ile Gln Glu
            340                 345                 350

tcc ctc                                                             1062
Ser Leu

<210> 642
<211> 354
<212> PRT
<213> Corynebacterium glutamicum

<400> 642
Ser Thr Ser Thr Ala Ala Gly Thr Gly Thr Ala Asn Glu Glu Gly Thr
1               5                   10                  15

Ile Thr Ala Ala Ile Ser Tyr Glu Leu Gly Thr Asn Gly Tyr Asp Pro
            20                  25                  30

Met Thr Thr Thr Ser Ala Leu Thr Val Ala Ala Asn Trp His Thr Leu
        35                  40                  45

Glu Gly Leu Thr Glu Ile Asp Pro Ala Thr Gly Glu Val Tyr Ala Ala
    50                  55                  60

Leu Ala Ser Ala Leu Pro Ser Ala Asp Ala Thr Ser Leu Asp Ile Lys
65                  70                  75                  80

Leu Arg Asp Gly Ala Thr Phe His Asn Gly Asp Ala Val Thr Ala Asp
                85                  90                  95

Asp Val Val Phe Ser Phe Glu Arg Val Leu Asp Pro Ala Asn Asn Ser
            100                 105                 110

Leu Tyr Ala Ser Phe Ile Pro Phe Ile Lys Ser Val Thr Lys Lys Asp
        115                 120                 125

Asp Thr Thr Val Thr Ile Asp Leu Asp Tyr Ala Thr Gly Ile Ile Ser
    130                 135                 140

Glu Arg Leu Ala Val Val Lys Ile Val Pro Lys Ser Val Val Glu Ala
145                 150                 155                 160

Asp Ala Ser Gly Phe Asp Ala Asn Pro Ile Gly Ser Gly Pro Tyr Lys
                165                 170                 175

Met Thr Asp Asn Gly Ala Ser Lys Val Val Lys Phe Glu Arg Asn Asp
            180                 185                 190

Asp Tyr Asn Gly Pro Arg Pro Ala Arg Ala Ala Lys Met Glu Trp Gln
        195                 200                 205

Ile Ile Pro Asp Ala Ser Thr Arg Thr Asn Ser Leu Gln Ser Gly Ser
    210                 215                 220

Thr Met Ala Ile Asp Ser Val Pro Tyr Leu Ser Ile Pro Gln Leu Glu
225                 230                 235                 240

Ala Thr Ser Thr Val Glu Ser Val Gln Gly Phe Gly Leu Leu Phe Ala
                245                 250                 255

Met Phe Ser Cys Ser Glu Gly Asn Pro Phe Asn Asp Val Arg Asn Arg
            260                 265                 270

Gln Ala Phe Leu Tyr Ala Leu Asp Met Asp Lys Ile Val Lys Thr Gly
        275                 280                 285

Met Ser Asp Gln Ala Thr Pro Ala Thr Ser Phe Val Gln Lys Glu His
    290                 295                 300

Pro Asn Tyr Asn Gln Ala Ser Thr Val Tyr Ser Leu Asp Ala Asp Lys
305                 310                 315                 320

Ala Lys Ala Leu Phe Ala Glu Thr Gly Leu Thr Ser Leu Asn Leu Leu
                325                 330                 335

Cys Thr Asp His Asp Trp Val Lys Asn Cys Thr Pro Leu Ile Gln Glu
            340                 345                 350

Ser Leu

<210> 643
<211> 335
<212> DNA
<213> Corynebacterium glutamicum

<220>
<221> CDS
<222> (1)..(312)
<223> RXA02659

<400> 643
ttc ggc aac gac cca gac ctc ctc atg cgc tgg tgg tac gcc ggc gac 48
Phe Gly Asn Asp Pro Asp Leu Leu Met Arg Trp Trp Tyr Ala Gly Asp
1               5                   10                  15

gtc tgg acc gac tcc cgc atg cac tgg aag ggc agc gaa tcc tac gac 96
Val Trp Thr Asp Ser Arg Met His Trp Lys Gly Ser Glu Ser Tyr Asp
            20                  25                  30

cag gta caa aac ctc ctg gaa gaa ggc atc cgc gct acc gac aag gca 144
Gln Val Gln Asn Leu Leu Glu Glu Gly Ile Arg Ala Thr Asp Lys Ala
        35                  40                  45

gaa cag caa gac atc tgg aac cgc acc ttc gat gtc atc tcc gac aat 192
Glu Gln Gln Asp Ile Trp Asn Arg Thr Phe Asp Val Ile Ser Asp Asn
    50                  55                  60

gtt ccc ctc tac ccg ctg ttc cac cgc aag gtc cca acc gca tgg aac 240
Val Pro Leu Tyr Pro Leu Phe His Arg Lys Val Pro Thr Ala Trp Asn
65                  70                  75                  80

tcc aac gcc ctc gtc gac ttc aag cca atc tca ctc acc ggc ttg aac 288
Ser Asn Ala Leu Val Asp Phe Lys Pro Ile Ser Leu Thr Gly Leu Asn
                85                  90                  95

ttc tca ggt gtt gca act act gaa taacaacccc agtggctttc aaa 335
Phe Ser Gly Val Ala Thr Thr Glu
            100

<210> 644
<211> 104
<212> PRT
<213> Corynebacterium glutamicum

<400> 644
Phe Gly Asn Asp Pro Asp Leu Leu Met Arg Trp Trp Tyr Ala Gly Asp
1               5                   10                  15

Val Trp Thr Asp Ser Arg Met His Trp Lys Gly Ser Glu Ser Tyr Asp
            20                  25                  30

Gln Val Gln Asn Leu Leu Glu Glu Gly Ile Arg Ala Thr Asp Lys Ala
        35                  40                  45

Glu Gln Gln Asp Ile Trp Asn Arg Thr Phe Asp Val Ile Ser Asp Asn
    50                  55                  60

Val Pro Leu Tyr Pro Leu Phe His Arg Lys Val Pro Thr Ala Trp Asn
65                  70                  75                  80

Ser Asn Ala Leu Val Asp Phe Lys Pro Ile Ser Leu Thr Gly Leu Asn
                85                  90                  95

Phe Ser Gly Val Ala Thr Thr Glu
            100

<210> 645
<211> 933
<212> DNA
<213> Corynebacterium glutamicum

<220>
<221> CDS
<222> (101)..(910)
<223> RXN02933

<400> 645
tgatctgctg tatcaggtgg ttgatccaag agtcggtgct gttggggttg ctagcactaa 60

ggttccaggg agcgtggctt aagtgacaac gatcaaaaac atg ccc ctt tca ggg   115
                                           Met Pro Leu Ser Gly
                                           1               5

aaa atc ggc ggc ttc atc gtt gcc gtt gta ttt gtt ctt gct gcg ctg   163
Lys Ile Gly Gly Phe Ile Val Ala Val Val Phe Val Leu Ala Ala Leu
                10                  15                  20

tct ttc att tgg act ccg ttt gat cca gtt caa gct ttc cca cag gag   211
Ser Phe Ile Trp Thr Pro Phe Asp Pro Val Gln Ala Phe Pro Gln Glu
            25                  30                  35

cgc ctt gag gga agt tct ttg agg cac ctg ttg gga acg gat cgt tat   259
Arg Leu Glu Gly Ser Ser Leu Arg His Leu Leu Gly Thr Asp Arg Tyr
        40                  45                  50

ggt cgc gat gtt tta tcc cag atc atg gtt ggt tcc cgc gtc acg ttg   307
Gly Arg Asp Val Leu Ser Gln Ile Met Val Gly Ser Arg Val Thr Leu
    55                  60                  65

ttg gtg ggc atc att gcg gtg gcg atc gca gca tta atc ggc acg cca   355
Leu Val Gly Ile Ile Ala Val Ala Ile Ala Ala Leu Ile Gly Thr Pro
70                  75                  80                  85

ctg ggt att gct gcg gga atg cgc cgt ggc atg gtg gaa acc ttt gtc   403
Leu Gly Ile Ala Ala Gly Met Arg Arg Gly Met Val Glu Thr Phe Val
                90                  95                  100

atg cgt ggt gcc gat tta atg ttg gcg ttc cca gca ctg ttg ttg gcg   451
Met Arg Gly Ala Asp Leu Met Leu Ala Phe Pro Ala Leu Leu Leu Ala
            105                 110                 115

att att tcc ggc gcc gtt ttc ggc gcc tcc acg tgg tcc gcg atg gtc   499
Ile Ile Ser Gly Ala Val Phe Gly Ala Ser Thr Trp Ser Ala Met Val
        120                 125                 130

gcg atc ggc atc gca ggc atc cct agt ttt gcc cgc gtg gct cgt gca   547
Ala Ile Gly Ile Ala Gly Ile Pro Ser Phe Ala Arg Val Ala Arg Ala
    135                 140                 145

ggc aca ttg cag gtg acc agt cag gat ttc atc gca gct gct cgg cta 595
Gly Thr Leu Gln Val Thr Ser Gln Asp Phe Ile Ala Ala Ala Arg Leu
150                 155                 160                 165

tca aaa gta agt tcc gcc cgg atc gcg ctt cgc cat att ttg ccc aac 643
Ser Lys Val Ser Ser Ala Arg Ile Ala Leu Arg His Ile Leu Pro Asn
                170                 175                 180

atc acc agc atg ttg atc gtt cag gca tca gta gct ttt gcc ctg gcg 691
Ile Thr Ser Met Leu Ile Val Gln Ala Ser Val Ala Phe Ala Leu Ala
            185                 190                 195

atc ctg gcg gaa gcc gca ttg agt ttc ctc ggt ttg ggc acc act ccc 739
Ile Leu Ala Glu Ala Ala Leu Ser Phe Leu Gly Leu Gly Thr Thr Pro
        200                 205                 210

ccg gat ccc agc tgg ggt cgc atg ttg caa acc gct caa gca tcc atc 787
Pro Asp Pro Ser Trp Gly Arg Met Leu Gln Thr Ala Gln Ala Ser Ile
    215                 220                 225

ggc gtc acc ccc atg ttg gcg gtg tgg ccc ggt gct gcg atc gct ttg 835
Gly Val Thr Pro Met Leu Ala Val Trp Pro Gly Ala Ala Ile Ala Leu
230                 235                 240                 245

acg gtc ctt ggt ttt aat ctt ttc ggt gat ggt tta cgc gat gcc atc 883
Thr Val Leu Gly Phe Asn Leu Phe Gly Asp Gly Leu Arg Asp Ala Ile
                250                 255                 260

gat cca aag cgg gag gtc ggc cgt gct taaagtttct gatttaacgg 930
Asp Pro Lys Arg Glu Val Gly Arg Ala
            265                 270

ttg 933

<210> 646
<211> 270
<212> PRT
<213> Corynebacterium glutamicum

<400> 646
Met Pro Leu Ser Gly Lys Ile Gly Gly Phe Ile Val Ala Val Val Phe
1               5                   10                  15

Val Leu Ala Ala Leu Ser Phe Ile Trp Thr Pro Phe Asp Pro Val Gln
            20                  25                  30

Ala Phe Pro Gln Glu Arg Leu Glu Gly Ser Ser Leu Arg His Leu Leu
        35                  40                  45

Gly Thr Asp Arg Tyr Gly Arg Asp Val Leu Ser Gln Ile Met Val Gly
    50                  55                  60

Ser Arg Val Thr Leu Leu Val Gly Ile Ile Ala Val Ala Ile Ala Ala
65                  70                  75                  80

Leu Ile Gly Thr Pro Leu Gly Ile Ala Ala Gly Met Arg Arg Gly Met
                85                  90                  95

Val Glu Thr Phe Val Met Arg Gly Ala Asp Leu Met Leu Ala Phe Pro
            100                 105                 110

Ala Leu Leu Leu Ala Ile Ile Ser Gly Ala Val Phe Gly Ala Ser Thr
        115                 120                 125

Trp Ser Ala Met Val Ala Ile Gly Ile Ala Gly Ile Pro Ser Phe Ala
    130                 135                 140

Arg Val Ala Arg Ala Gly Thr Leu Gln Val Thr Ser Gln Asp Phe Ile
145                 150                 155                 160

Ala Ala Ala Arg Leu Ser Lys Val Ser Ser Ala Arg Ile Ala Leu Arg
                165                 170                 175

His Ile Leu Pro Asn Ile Thr Ser Met Leu Ile Val Gln Ala Ser Val
            180                 185                 190

Ala Phe Ala Leu Ala Ile Leu Ala Glu Ala Ala Leu Ser Phe Leu Gly
        195                 200                 205

Leu Gly Thr Thr Pro Pro Asp Pro Ser Trp Gly Arg Met Leu Gln Thr
    210                 215                 220

Ala Gln Ala Ser Ile Gly Val Thr Pro Met Leu Ala Val Trp Pro Gly
225                 230                 235                 240

Ala Ala Ile Ala Leu Thr Val Leu Gly Phe Asn Leu Phe Gly Asp Gly
                245                 250                 255

Leu Arg Asp Ala Ile Asp Pro Lys Arg Glu Val Gly Arg Ala
            260                 265                 270

<210> 647
<211> 715
<212> DNA
<213> Corynebacterium glutamicum

<220>
<221> CDS
<222> (101)..(715)
<223> RXN02991

<400> 647
ttgtcgtgtt cgcgatcttc gccgttggct tcatgattct gaccctgcct atgggccttg 60

gcttgggcaa actctctgag cgtttggcgg tgaagaagta atg gct aat aca gtt   115
                                           Met Ala Asn Thr Val
                                           1               5

cgc gca aca gtc ctc tac gat gct cct gga cca aag ggc cgt cga ttc   163
Arg Ala Thr Val Leu Tyr Asp Ala Pro Gly Pro Lys Gly Arg Arg Phe
                10                  15                  20

aac ctc ata atc acc att ctc acg gtg gtt ctg gga ttg gcg ctc ctc   211
Asn Leu Ile Ile Thr Ile Leu Thr Val Val Leu Gly Leu Ala Leu Leu
            25                  30                  35

ttc tgg att ggc tcc atg ctt tca ggc aac ggc caa ctc gat gcc aac   259
Phe Trp Ile Gly Ser Met Leu Ser Gly Asn Gly Gln Leu Asp Ala Asn
        40                  45                  50

aaa tgg act ccg ttc atc aat tcc caa acc tgg acc acc tac att ctt     307
Lys Trp Thr Pro Phe Ile Asn Ser Gln Thr Trp Thr Thr Tyr Ile Leu
    55                  60                  65

cct ggt ttg tgg ggc acg ctg aaa tct gcc gtg ttc tcg gtg atc ttg     355
Pro Gly Leu Trp Gly Thr Leu Lys Ser Ala Val Phe Ser Val Ile Leu
70                  75                  80                  85

gct ctg gtc atg ggt acc gca ctg ggt ctt ggc cgt atc tct gaa atc     403
Ala Leu Val Met Gly Thr Ala Leu Gly Leu Gly Arg Ile Ser Glu Ile
                90                  95                  100

agg att ctc cgc tgg ttc tgc gcc gtc atc atc gag act ttc cga gcc     451
Arg Ile Leu Arg Trp Phe Cys Ala Val Ile Ile Glu Thr Phe Arg Ala
            105                 110                 115

att ccg gtt ctg atc ctc atg att ttc gcc tac cag atg ttc gcc cag     499
Ile Pro Val Leu Ile Leu Met Ile Phe Ala Tyr Gln Met Phe Ala Gln
        120                 125                 130

tac aac atc gtg ccg tcg agc cag ctc gcg ttc gcc gcc gtg gta ttc     547
Tyr Asn Ile Val Pro Ser Ser Gln Leu Ala Phe Ala Ala Val Val Phe
    135                 140                 145

ggt ctg acc atg tac aac ggt tct gtg atc gca gag att ctg cgt tct     595
Gly Leu Thr Met Tyr Asn Gly Ser Val Ile Ala Glu Ile Leu Arg Ser
150                 155                 160                 165

ggt atc gct tcc ctg cct aag ggg cag aag gaa gca gcg att gcg ttg     643
Gly Ile Ala Ser Leu Pro Lys Gly Gln Lys Glu Ala Ala Ile Ala Leu
                170                 175                 180

ggt atg tct tct agg caa acc acc tgg tca atc ttg ttg cct cag gcc     691
Gly Met Ser Ser Arg Gln Thr Thr Trp Ser Ile Leu Leu Pro Gln Ala
            185                 190                 195

gtg gct gca atg ctc cca gcg ttg                                     715
Val Ala Ala Met Leu Pro Ala Leu
        200                 205


<210> 648
<211> 205
<212> PRT
<213> Corynebacterium glutamicum

<400> 648
Met Ala Asn Thr Val Arg Ala Thr Val Leu Tyr Asp Ala Pro Gly Pro
  1               5                  10                  15

Lys Gly Arg Arg Phe Asn Leu Ile Ile Thr Ile Leu Thr Val Val Leu
            20                  25                  30

Gly Leu Ala Leu Leu Phe Trp Ile Gly Ser Met Leu Ser Gly Asn Gly
        35                  40                  45

Gln Leu Asp Ala Asn Lys Trp Thr Pro Phe Ile Asn Ser Gln Thr Trp
    50                  55                  60

Thr Thr Tyr Ile Leu Pro Gly Leu Trp Gly Thr Leu Lys Ser Ala Val
65                  70                  75                  80

Phe Ser Val Ile Leu Ala Leu Val Met Gly Thr Ala Leu Gly Leu Gly
                85                  90                  95

Arg Ile Ser Glu Ile Arg Ile Leu Arg Trp Phe Cys Ala Val Ile Ile
            100                 105                 110

Glu Thr Phe Arg Ala Ile Pro Val Leu Ile Leu Met Ile Phe Ala Tyr
        115                 120                 125

Gln Met Phe Ala Gln Tyr Asn Ile Val Pro Ser Ser Gln Leu Ala Phe
    130                 135                 140

Ala Ala Val Val Phe Gly Leu Thr Met Tyr Asn Gly Ser Val Ile Ala
145                 150                 155                 160

Glu Ile Leu Arg Ser Gly Ile Ala Ser Leu Pro Lys Gly Gln Lys Glu
                165                 170                 175

Ala Ala Ile Ala Leu Gly Met Ser Ser Arg Gln Thr Thr Trp Ser Ile
            180                 185                 190

Leu Leu Pro Gln Ala Val Ala Ala Met Leu Pro Ala Leu
        195                 200                 205


<210> 649
<211> 245
<212> DNA
<213> Corynebacterium glutamicum

<220>
<221> CDS
<222> (1)..(222)
<223> RXN02992

<400> 649
atc gtc cct ttg ggc aac aca ctg atc gca ctg act aag aac acc acc      48
Ile Val Pro Leu Gly Asn Thr Leu Ile Ala Leu Thr Lys Asn Thr Thr
  1               5                  10                  15

atc gcc tct gtc att gga gtt ggc gaa gcc tcc ctg ctg atg aaa gcc      96
Ile Ala Ser Val Ile Gly Val Gly Glu Ala Ser Leu Leu Met Lys Ala
             20                  25                  30

acc atc gaa aat cac gcc aac atg cta ttt gtc gtg ttc gcg atc ttc     144
Thr Ile Glu Asn His Ala Asn Met Leu Phe Val Val Phe Ala Ile Phe
         35                  40                  45

gcc gtt ggc ttc atg att ctg acc ctg cct atg ggc ctt ggc ttg ggc     192
Ala Val Gly Phe Met Ile Leu Thr Leu Pro Met Gly Leu Gly Leu Gly
     50                  55                  60

aaa ctc tct gag cgt ttg gcg gtg aag aag taatggctaa tacagttcgc     242
Lys Leu Ser Glu Arg Leu Ala Val Lys Lys
 65                  70

gca                                                                245


<210> 650

<211> 74
<212> PRT
<213> Corynebacterium glutamicum

<400> 650
Ile Val Pro Leu Gly Asn Thr Leu Ile Ala Leu Thr Lys Asn Thr Thr
1               5                   10                  15

Ile Ala Ser Val Ile Gly Val Gly Glu Ala Ser Leu Leu Met Lys Ala
            20                  25                  30

Thr Ile Glu Asn His Ala Asn Met Leu Phe Val Val Phe Ala Ile Phe
.       35                  40                  45

Ala Val Gly Phe Met Ile Leu Thr Leu Pro Met Gly Leu Gly Leu Gly
    50                  55                  60

Lys Leu Ser Glu Arg Leu Ala Val Lys Lys
65                  70


<210> 651
<211> 792
<212> DNA
<213> Corynebacterium glutamicum

<220>
<221> CDS
<222> (101)..(769)
<223> RXN02996

<400> 651
tcctgccaca gaatctgcct gctgctccat taactaaaaa ttgcaggcta gaatagaggg 60

tcgattagga gtcgatgaaa aagtcggcgc caacgaggag atg aac aac aac gtg   115
                                            Met Asn Asn Asn Val
                                            1               5

agt gat caa aag cta agt ggt aaa gag ctt gcg gca cta gag aaa caa   163
Ser Asp Gln Lys Leu Ser Gly Lys Glu Leu Ala Ala Leu Glu Lys Gln
                10                  15                  20

gcc gca aaa act ctc gaa ctt ggt gat aag aag tgg tat ctc atc gcg   211
Ala Ala Lys Thr Leu Glu Leu Gly Asp Lys Lys Trp Tyr Leu Ile Ala
            25                  30                  35

gga gtc gtg ctt ttc gcc atc gca ctc gtc ctc cca cat atc cgt gga   259
Gly Val Val Leu Phe Ala Ile Ala Leu Val Leu Pro His Ile Arg Gly
        40                  45                  50

gtg atg ggc tgg cag gtt ctg acg ctg tcg aat gtc gcg gag gat gcc   307
Val Met Gly Trp Gln Val Leu Thr Leu Ser Asn Val Ala Glu Asp Ala
    55                  60                  65

ggc att acc ctt ggt gag tac ggt ttc tac tgg ttg ggc acc atc ggt   355
Gly Ile Thr Leu Gly Glu Tyr Gly Phe Tyr Trp Leu Gly Thr Ile Gly
70                  75                  80                  85

gta ttc ctg ctt tct ttg ggc act gtc gtg ttt aag cgc acg tgg atg   403
Val Phe Leu Leu Ser Leu Gly Thr Val Val Phe Lys Arg Thr Trp Met
                90                  95                  100

gcg tgg att tca tgg att ttc tcc tgc gtc act ctt gtg ttc gct gtg     451
Ala Trp Ile Ser Trp Ile Phe Ser Cys Val Thr Leu Val Phe Ala Val
            105                 110                 115

ttt gcc atc tgg atg cgc caa aca acc acc agc acc caa gtg aat ttc     499
Phe Ala Ile Trp Met Arg Gln Thr Thr Thr Ser Thr Gln Val Asn Phe
        120                 125                 130

gtc aac att ggc atg atg ctt gct gtg att gca gcg atc ctt gct gtg     547
Val Asn Ile Gly Met Met Leu Ala Val Ile Ala Ala Ile Leu Ala Val
    135                 140                 145

tgg ggt cta tct tcg gtg att ttg gcc cgc agt gat cgc caa atg gag     595
Trp Gly Leu Ser Ser Val Ile Leu Ala Arg Ser Asp Arg Gln Met Glu
150                 155                 160                 165

atc gct gaa atg cgc gcc gag aac cca gac ctt gat ggt gtt gca gct     643
Ile Ala Glu Met Arg Ala Glu Asn Pro Asp Leu Asp Gly Val Ala Ala
                170                 175                 180

acc cag cgc gca ctt ctt gag cag cag caa agc aac cca gaa gat aat     691
Thr Gln Arg Ala Leu Leu Glu Gln Gln Gln Ser Asn Pro Glu Asp Asn
            185                 190                 195

cct ttg ctt gtc gac gat cgt cgc gcc cgc atc gcc cgc cgc cgc gag     739
Pro Leu Leu Val Asp Asp Arg Arg Ala Arg Ile Ala Arg Arg Arg Glu
        200                 205                 210

cgt gaa cag gat gca caa ggg gag cag gct taagttctag ttcaagcggt     789
Arg Glu Gln Asp Ala Gln Gly Glu Gln Ala
    215                 220

tga                                                                 792


<210> 652
<211> 223
<212> PRT
<213> Corynebacterium glutamicum

<400> 652
Met Asn Asn Asn Val Ser Asp Gln Lys Leu Ser Gly Lys Glu Leu Ala
  1               5                  10                  15

Ala Leu Glu Lys Gln Ala Ala Lys Thr Leu Glu Leu Gly Asp Lys Lys
             20                  25                  30

Trp Tyr Leu Ile Ala Gly Val Val Leu Phe Ala Ile Ala Leu Val Leu
         35                  40                  45

Pro His Ile Arg Gly Val Met Gly Trp Gln Val Leu Thr Leu Ser Asn
     50                  55                  60

Val Ala Glu Asp Ala Gly Ile Thr Leu Gly Glu Tyr Gly Phe Tyr Trp
 65                  70                  75                  80

Leu Gly Thr Ile Gly Val Phe Leu Leu Ser Leu Gly Thr Val Val Phe
                 85                  90                  95

Lys Arg Thr Trp Met Ala Trp Ile Ser Trp Ile Phe Ser Cys Val Thr

100                 105                 110

Leu Val Phe Ala Val Phe Ala Ile Trp Met Arg Gln Thr Thr Thr Ser
        115                 120                 125

Thr Gln Val Asn Phe Val Asn Ile Gly Met Met Leu Ala Val Ile Ala
    130                 135                 140

Ala Ile Leu Ala Val Trp Gly Leu Ser Ser Val Ile Leu Ala Arg Ser
145                 150                 155                 160

Asp Arg Gln Met Glu Ile Ala Glu Met Arg Ala Glu Asn Pro Asp Leu
                165                 170                 175

Asp Gly Val Ala Ala Thr Gln Arg Ala Leu Leu Glu Gln Gln Gln Ser
            180                 185                 190

Asn Pro Glu Asp Asn Pro Leu Leu Val Asp Asp Arg Arg Ala Arg Ile
        195                 200                 205

Ala Arg Arg Arg Glu Arg Glu Gln Asp Ala Gln Gly Glu Gln Ala
    210                 215                 220

<210> 653
<211> 1017
<212> DNA
<213> Corynebacterium glutamicum

<220>
<221> CDS
<222> (101)..(994)
<223> RXN03126

<400> 653
tcttggacaa cattacccca cacacgggac actcaaacgt gcatgaattc acctgtcgtc 60

gatacccaaa aatcactcca ccaccactag ggtgtcttat gtg cag gaa caa tcg   115
                                            Val Gln Glu Gln Ser
                                            1               5

aag cag aaa gac ctc cag gct gat atc gcc aga atc act gcc gtg aaa   163
Lys Gln Lys Asp Leu Gln Ala Asp Ile Ala Arg Ile Thr Ala Val Lys
                10                  15                  20

tcg gac aca gtt ccg aat tcc cag tca atg acc ttt ggc gct gcc tgg   211
Ser Asp Thr Val Pro Asn Ser Gln Ser Met Thr Phe Gly Ala Ala Trp
            25                  30                  35

aac gat atc gtc cgc gga ttc aag caa cat gag ttg tgg ctg caa ctc   259
Asn Asp Ile Val Arg Gly Phe Lys Gln His Glu Leu Trp Leu Gln Leu
        40                  45                  50

ggc tgg caa gac att aag caa cgc tac cgg cga tcc gtc ctt ggg ccc   307
Gly Trp Gln Asp Ile Lys Gln Arg Tyr Arg Arg Ser Val Leu Gly Pro
    55                  60                  65

ctg tgg atc acc atc gcc acc ggt gtc atg gcg ctc gcc ctc ggc ttg   355
Leu Trp Ile Thr Ile Ala Thr Gly Val Met Ala Leu Ala Leu Gly Leu
70                  75                  80                  85

ctg tac tcc gtg ctg ttc aaa atc ccc atc gcg gaa ttt ctg ccc cac 403
Leu Tyr Ser Val Leu Phe Lys Ile Pro Ile Ala Glu Phe Leu Pro His
                90                  95                  100

gtc acc gtc ggc ctg atc att tgg aac ttc atc tcc gga tgc atc aaa 451
Val Thr Val Gly Leu Ile Ile Trp Asn Phe Ile Ser Gly Cys Ile Lys
            105                 110                 115

gaa ggc tcc gac atc ttc ata gat aac gag gga ctc atc aaa cag ctc 499
Glu Gly Ser Asp Ile Phe Ile Asp Asn Glu Gly Leu Ile Lys Gln Leu
        120                 125                 130

ccc tcg gcg ctg tcc gtc cac gtg tac aga ctc gtg tgg aaa caa gcc 547
Pro Ser Ala Leu Ser Val His Val Tyr Arg Leu Val Trp Lys Gln Ala
    135                 140                 145

ctc ttc ctc gcc cac aac ctg gtc atc tgg gtc att ctc atg atg atc 595
Leu Phe Leu Ala His Asn Leu Val Ile Trp Val Ile Leu Met Met Ile
150                 155                 160                 165

ttc ccc cga ccc ctc ggc tgg gac gtc ctc ctg atc atc ccc gca atg 643
Phe Pro Arg Pro Leu Gly Trp Asp Val Leu Leu Ile Ile Pro Ala Met
                170                 175                 180

ttc ctc ctg gtg atc aac ggc gtg tgg gta gtc atg ttc ttc ggc atc 691
Phe Leu Leu Val Ile Asn Gly Val Trp Val Val Met Phe Phe Gly Ile
            185                 190                 195

atc gcc acc cgc tac cgc gac gtc tcc ccg ttg cta gaa gcc gga acc 739
Ile Ala Thr Arg Tyr Arg Asp Val Ser Pro Leu Leu Glu Ala Gly Thr
        200                 205                 210

caa ctc ctc ttc tac gtc acc ccc atc gtc tgg atg act tcc acc ctg 787
Gln Leu Leu Phe Tyr Val Thr Pro Ile Val Trp Met Thr Ser Thr Leu
    215                 220                 225

caa tcc cag agc gca gaa atc ggc aac cgc gca cgc ctc gcc gaa ctc 835
Gln Ser Gln Ser Ala Glu Ile Gly Asn Arg Ala Arg Leu Ala Glu Leu
230                 235                 240                 245

aac ccg ctc tac cac tac ctc gaa att gtc cgc gcc ccc atg gtc ggc 883
Asn Pro Leu Tyr His Tyr Leu Glu Ile Val Arg Ala Pro Met Val Gly
                250                 255                 260

gcc gac ctc ccc gcc tac cac tgg tgg atc gtc ctc gcc ttc aca ttc 931
Ala Asp Leu Pro Ala Tyr His Trp Trp Ile Val Leu Ala Phe Thr Phe
            265                 270                 275

gtc ggc ctt ggc ctt gct ctc ctc gcg atg aag caa tgg cga ttc cgc 979
Val Gly Leu Gly Leu Ala Leu Leu Ala Met Lys Gln Trp Arg Phe Arg
        280                 285                 290

gtc agc tac tgg gta taaggagcac cacatggtat cca 1017
Val Ser Tyr Trp Val
    295

<210> 654
<211> 298
<212> PRT
<213> Corynebacterium glutamicum

<400> 654
Val Gln Glu Gln Ser Lys Gln Lys Asp Leu Gln Ala Asp Ile Ala Arg
1               5                   10                  15

Ile Thr Ala Val Lys Ser Asp Thr Val Pro Asn Ser Gln Ser Met Thr
            20                  25                  30

Phe Gly Ala Ala Trp Asn Asp Ile Val Arg Gly Phe Lys Gln His Glu
        35                  40                  45

Leu Trp Leu Gln Leu Gly Trp Gln Asp Ile Lys Gln Arg Tyr Arg Arg
    50                  55                  60

Ser Val Leu Gly Pro Leu Trp Ile Thr Ile Ala Thr Gly Val Met Ala
65                  70                  75                  80

Leu Ala Leu Gly Leu Leu Tyr Ser Val Leu Phe Lys Ile Pro Ile Ala
                85                  90                  95

Glu Phe Leu Pro His Val Thr Val Gly Leu Ile Ile Trp Asn Phe Ile
            100                 105                 110

Ser Gly Cys Ile Lys Glu Gly Ser Asp Ile Phe Ile Asp Asn Glu Gly
        115                 120                 125

Leu Ile Lys Gln Leu Pro Ser Ala Leu Ser Val His Val Tyr Arg Leu
    130                 135                 140

Val Trp Lys Gln Ala Leu Phe Leu Ala His Asn Leu Val Ile Trp Val
145                 150                 155                 160

Ile Leu Met Met Ile Phe Pro Arg Pro Leu Gly Trp Asp Val Leu Leu
                165                 170                 175

Ile Ile Pro Ala Met Phe Leu Leu Val Ile Asn Gly Val Trp Val Val
            180                 185                 190

Met Phe Phe Gly Ile Ile Ala Thr Arg Tyr Arg Asp Val Ser Pro Leu
        195                 200                 205

Leu Glu Ala Gly Thr Gln Leu Leu Phe Tyr Val Thr Pro Ile Val Trp
    210                 215                 220

Met Thr Ser Thr Leu Gln Ser Gln Ser Ala Glu Ile Gly Asn Arg Ala
225                 230                 235                 240

Arg Leu Ala Glu Leu Asn Pro Leu Tyr His Tyr Leu Glu Ile Val Arg
                245                 250                 255

Ala Pro Met Val Gly Ala Asp Leu Pro Ala Tyr His Trp Trp Ile Val
            260                 265                 270

Leu Ala Phe Thr Phe Val Gly Leu Gly Leu Ala Leu Leu Ala Met Lys
        275                 280                 285

Gln Trp Arg Phe Arg Val Ser Tyr Trp Val
    290                 295

<210> 655

<211> 927
<212> DNA
<213> Corynebacterium glutamicum

<220>
<221> CDS
<222> (101)..(904)
<223> RXN00443

<400> 655
taaacaagga gcgtttaatt tccacccaat cccactgaaa gtttcccacc ccacagttat 60

gctgattaag tactgaactt accgtaggaa aggcactcag gtg aac aaa agt atc 115
Val Asn Lys Ser Ile
1               5

cgt cga gct ctc tac agt ttc atc act att tcg gcg ggt atc tca ctc 163
Arg Arg Ala Leu Tyr Ser Phe Ile Thr Ile Ser Ala Gly Ile Ser Leu
                10                  15                  20

gta gcg tgc tct tct tca gac acc gca agc act acc acc cag aat gcc 211
Val Ala Cys Ser Ser Ser Asp Thr Ala Ser Thr Thr Thr Gln Asn Ala
            25                  30                  35

tct gct acc gaa gca gct ggg gtt tcc gga acc gct agc gtt ttc gct 259
Ser Ala Thr Glu Ala Ala Gly Val Ser Gly Thr Ala Ser Val Phe Ala
        40                  45                  50

gcg gcg tct ttg acc aac gtt gct gag gat ctc gcg gct gcg ttc aac 307
Ala Ala Ser Leu Thr Asn Val Ala Glu Asp Leu Ala Ala Ala Phe Asn
    55                  60                  65

gaa gac aat cct gat gca aag ttg gag ttt aac ttc gct ggt tct tcc 355
Glu Asp Asn Pro Asp Ala Lys Leu Glu Phe Asn Phe Ala Gly Ser Ser
70                  75                  80                  85

gcg ctg gtt cgc cag atc agc gaa ggc gca cca tct gat ctg ttc att 403
Ala Leu Val Arg Gln Ile Ser Glu Gly Ala Pro Ser Asp Leu Phe Ile
                90                  95                  100

tct gcg gac atc gcc aac atg gac gat gcg ctg gca ctt cca gaa ttt 451
Ser Ala Asp Ile Ala Asn Met Asp Asp Ala Leu Ala Leu Pro Glu Phe
            105                 110                 115

gcc ggc gca acc tcg aag gtc atc gct acc aac aag ctg gtg ctg gtc 499
Ala Gly Ala Thr Ser Lys Val Ile Ala Thr Asn Lys Leu Val Leu Val
        120                 125                 130

acc gca gac ggc aac ccc ggc gaa att tca gag ctt gcc gac gtc aag 547
Thr Ala Asp Gly Asn Pro Gly Glu Ile Ser Glu Leu Ala Asp Val Lys
    135                 140                 145

gac tcc ctc gtt gct atc tgc gcc cct gaa gtt cca tgt gga acc atc 595
Asp Ser Leu Val Ala Ile Cys Ala Pro Glu Val Pro Cys Gly Thr Ile
150                 155                 160                 165

acc cac gag gct ttg gac tac gca gac atc gag ttg aac acc agc tct 643
Thr His Glu Ala Leu Asp Tyr Ala Asp Ile Glu Leu Asn Thr Ser Ser
                170                 175                 180

gag gaa gcc aat gtt gct gat gtc gcc acg aag att tcc acc ggt gca 691

Glu Glu Ala Asn Val Ala Asp Val Ala Thr Lys Ile Ser Thr Gly Ala
            185                 190                 195

gtt gat gcg ggc ttt gtt tac caa acc gac gct cag tct ttg gct aaa     739
Val Asp Ala Gly Phe Val Tyr Gln Thr Asp Ala Gln Ser Leu Ala Lys
        200                 205                 210

act cag gac aac act gtc att gaa ctc gaa ggt atc gac gcc aac gaa     787
Thr Gln Asp Asn Thr Val Ile Glu Leu Glu Gly Ile Asp Ala Asn Glu
    215                 220                 225

tac cca atg gca ttg acc acc acc ggt gaa gac aac gag gta gcg aag     835
Tyr Pro Met Ala Leu Thr Thr Thr Gly Glu Asp Asn Glu Val Ala Lys
230                 235                 240                 245

gct ttc gca gag ttc ctc agc agc gat cgt gcc aag gag atc ctt gcc     883
Ala Phe Ala Glu Phe Leu Ser Ser Asp Arg Ala Lys Glu Ile Leu Ala
                250                 255                 260

agc tat ggt ttt ggc aca aac taaaaggct cgtcgaagcg aga                927
Ser Tyr Gly Phe Gly Thr Asn
            265


<210> 656
<211> 268
<212> PRT
<213> Corynebacterium glutamicum

<400> 656
Val Asn Lys Ser Ile Arg Arg Ala Leu Tyr Ser Phe Ile Thr Ile Ser
  1               5                  10                  15

Ala Gly Ile Ser Leu Val Ala Cys Ser Ser Ser Asp Thr Ala Ser Thr
             20                  25                  30

Thr Thr Gln Asn Ala Ser Ala Thr Glu Ala Ala Gly Val Ser Gly Thr
         35                  40                  45

Ala Ser Val Phe Ala Ala Ala Ser Leu Thr Asn Val Ala Glu Asp Leu
     50                  55                  60

Ala Ala Ala Phe Asn Glu Asp Asn Pro Asp Ala Lys Leu Glu Phe Asn
 65                  70                  75                  80

Phe Ala Gly Ser Ser Ala Leu Val Arg Gln Ile Ser Glu Gly Ala Pro
                 85                  90                  95

Ser Asp Leu Phe Ile Ser Ala Asp Ile Ala Asn Met Asp Asp Ala Leu
            100                 105                 110

Ala Leu Pro Glu Phe Ala Gly Ala Thr Ser Lys Val Ile Ala Thr Asn
        115                 120                 125

Lys Leu Val Leu Val Thr Ala Asp Gly Asn Pro Gly Glu Ile Ser Glu
    130                 135                 140

Leu Ala Asp Val Lys Asp Ser Leu Val Ala Ile Cys Ala Pro Glu Val
145                 150                 155                 160

Pro Cys Gly Thr Ile Thr His Glu Ala Leu Asp Tyr Ala Asp Ile Glu

165                 170                 175

Leu Asn Thr Ser Ser Glu Glu Ala Asn Val Ala Asp Val Ala Thr Lys
            180                 185                 190

Ile Ser Thr Gly Ala Val Asp Ala Gly Phe Val Tyr Gln Thr Asp Ala
        195                 200                 205

Gln Ser Leu Ala Lys Thr Gln Asp Asn Thr Val Ile Glu Leu Glu Gly
    210                 215                 220

Ile Asp Ala Asn Glu Tyr Pro Met Ala Leu Thr Thr Thr Gly Glu Asp
225                 230                 235                 240

Asn Glu Val Ala Lys Ala Phe Ala Glu Phe Leu Ser Ser Asp Arg Ala
                245                 250                 255

Lys Glu Ile Leu Ala Ser Tyr Gly Phe Gly Thr Asn
            260                 265


<210> 657
<211> 960
<212> DNA
<213> Corynebacterium glutamicum

<220>
<221> CDS
<222> (101)..(937)
<223> RXN00444

<400> 657
tacccaatgg cattgaccac caccggtgaa gacaacgagg tagcgaaggc tttcgcagag 60

ttcctcagca gcgatcgtgc caaggagatc cttgccagct atg gtt ttg gca caa   115
                                           Met Val Leu Ala Gln
                                           1               5

act aaa aag gct cgt cga agc gag aat cat atc ctc cca ggg tgg ttg   163
Thr Lys Lys Ala Arg Arg Ser Glu Asn His Ile Leu Pro Gly Trp Leu
                10                  15                  20

ctc atc cca gcc acc ctg gcc atg ctg ctg atc att gga cct att ttt   211
Leu Ile Pro Ala Thr Leu Ala Met Leu Leu Ile Ile Gly Pro Ile Phe
            25                  30                  35

gct ttg ctg ttg cag atc ccc tgg gat cgg tct tgg gag ttg ctt acc   259
Ala Leu Leu Leu Gln Ile Pro Trp Asp Arg Ser Trp Glu Leu Leu Thr
        40                  45                  50

gcg ccg gaa tct tta gga acc gca cgg tta tct atc gga act gct ctg   307
Ala Pro Glu Ser Leu Gly Thr Ala Arg Leu Ser Ile Gly Thr Ala Leu
    55                  60                  65

ttt tct acc gcg cta tgc gca att gtg ggt ttc ccg cta gcg ttg gcg   355
Phe Ser Thr Ala Leu Cys Ala Ile Val Gly Phe Pro Leu Ala Leu Ala
70                  75                  80                  85

ctg cat tta tat gag cgt tcg cac ccc agg gtg aca tca gtt ttg acg   403
Leu His Leu Tyr Glu Arg Ser His Pro Arg Val Thr Ser Val Leu Thr
                90                  95                  100

gtg ctg gtt tat gcg cct ttg gtg ttg tcg ccg gtg gtg tct ggt ttg     451
Val Leu Val Tyr Ala Pro Leu Val Leu Ser Pro Val Val Ser Gly Leu
            105                 110                 115

gcg ctg act ttt ctg tgg ggc agg cgt ggt ttt tta ggt tct tgg ctt     499
Ala Leu Thr Phe Leu Trp Gly Arg Arg Gly Phe Leu Gly Ser Trp Leu
        120                 125                 130

gat cag gtt gga ttg ccg att gca ttt acc acc acg gct gtg gtg ttt     547
Asp Gln Val Gly Leu Pro Ile Ala Phe Thr Thr Thr Ala Val Val Phe
    135                 140                 145

gcc cag gtg ttt gta gcg ttg cca ttt ttc att tcc act gtg act act     595
Ala Gln Val Phe Val Ala Leu Pro Phe Phe Ile Ser Thr Val Thr Thr
150                 155                 160                 165

gca ctg cgt ggc att cca aaa cag ttt gag gaa atc gca gct act gaa     643
Ala Leu Arg Gly Ile Pro Lys Gln Phe Glu Glu Ile Ala Ala Thr Glu
                170                 175                 180

ggc gca acc cgc tgg gag atc atg cac aag atg atc att ccg ctg gcg     691
Gly Ala Thr Arg Trp Glu Ile Met His Lys Met Ile Ile Pro Leu Ala
            185                 190                 195

atg cct gga att ttc acc ggt atg att ttg gga ttc gcc agg gcc ttg     739
Met Pro Gly Ile Phe Thr Gly Met Ile Leu Gly Phe Ala Arg Ala Leu
        200                 205                 210

ggc gag tat ggt gcg aca ctg act ttt gct gga aat att gca ggt gtt     787
Gly Glu Tyr Gly Ala Thr Leu Thr Phe Ala Gly Asn Ile Ala Gly Val
    215                 220                 225

acc cgc acc att ccg ttg cat att gag ctt ggt ttg agt tcc aat gac     835
Thr Arg Thr Ile Pro Leu His Ile Glu Leu Gly Leu Ser Ser Asn Asp
230                 235                 240                 245

atg gat aaa gcc ttg gga gcg gtg att atg ctt ttg gct gtc tat gtc     883
Met Asp Lys Ala Leu Gly Ala Val Ile Met Leu Leu Ala Val Tyr Val
                250                 255                 260

ctc atc att gga gcc atc gga gcg tta cga ttg ttt tcc aag gtg aga     931
Leu Ile Ile Gly Ala Ile Gly Ala Leu Arg Leu Phe Ser Lys Val Arg
            265                 270                 275

aag gtt taattgatgt ctcgttcgcc gga                                   960
Lys Val

<210> 658
<211> 279
<212> PRT
<213> Corynebacterium glutamicum

<400> 658
Met Val Leu Ala Gln Thr Lys Lys Ala Arg Arg Ser Glu Asn His Ile
  1               5                  10                  15

Leu Pro Gly Trp Leu Leu Ile Pro Ala Thr Leu Ala Met Leu Leu Ile
             20                  25                  30

Ile Gly Pro Ile Phe Ala Leu Leu Leu Gln Ile Pro Trp Asp Arg Ser
        35                  40                  45

Trp Glu Leu Leu Thr Ala Pro Glu Ser Leu Gly Thr Ala Arg Leu Ser
    50                  55                  60

Ile Gly Thr Ala Leu Phe Ser Thr Ala Leu Cys Ala Ile Val Gly Phe
65                  70                  75                  80

Pro Leu Ala Leu Ala Leu His Leu Tyr Glu Arg Ser His Pro Arg Val
                85                  90                  95

Thr Ser Val Leu Thr Val Leu Val Tyr Ala Pro Leu Val Leu Ser Pro
            100                 105                 110

Val Val Ser Gly Leu Ala Leu Thr Phe Leu Trp Gly Arg Arg Gly Phe
        115                 120                 125

Leu Gly Ser Trp Leu Asp Gln Val Gly Leu Pro Ile Ala Phe Thr Thr
    130                 135                 140

Thr Ala Val Val Phe Ala Gln Val Phe Val Ala Leu Pro Phe Phe Ile
145                 150                 155                 160

Ser Thr Val Thr Thr Ala Leu Arg Gly Ile Pro Lys Gln Phe Glu Glu
                165                 170                 175

Ile Ala Ala Thr Glu Gly Ala Thr Arg Trp Glu Ile Met His Lys Met
            180                 185                 190

Ile Ile Pro Leu Ala Met Pro Gly Ile Phe Thr Gly Met Ile Leu Gly
        195                 200                 205

Phe Ala Arg Ala Leu Gly Glu Tyr Gly Ala Thr Leu Thr Phe Ala Gly
    210                 215                 220

Asn Ile Ala Gly Val Thr Arg Thr Ile Pro Leu His Ile Glu Leu Gly
225                 230                 235                 240

Leu Ser Ser Asn Asp Met Asp Lys Ala Leu Gly Ala Val Ile Met Leu
                245                 250                 255

Leu Ala Val Tyr Val Leu Ile Ile Gly Ala Ile Gly Ala Leu Arg Leu
            260                 265                 270

Phe Ser Lys Val Arg Lys Val
        275

<210> 659
<211> 594
<212> DNA
<213> Corynebacterium glutamicum

<220>
<221> CDS
<222> (1)..(594)
<223> RXN00193

<400> 659

aaa gct ttc tnc caa cgc gaa ggt ttc atc tca gcc ttc ggt ttc acc     48
Lys Ala Phe Xaa Gln Arg Glu Gly Phe Ile Ser Ala Phe Gly Phe Thr
  1               5                  10                  15

gtc ctc gtg gtc atc gtc tcc gtg atc aca gtc aac atc ttc gcc ttc     96
Val Leu Val Val Ile Val Ser Val Ile Thr Val Asn Ile Phe Ala Phe
             20                  25                  30

ctc ttg gcg tgg ttg ctg acc cgc aaa ctc cgc ggt acc aac ttt ttc    144
Leu Leu Ala Trp Leu Leu Thr Arg Lys Leu Arg Gly Thr Asn Phe Phe
         35                  40                  45

cgc aca gtc ttc ttt atg ccg aac ctt atc ggc ggc att gtg ctg ggt    192
Arg Thr Val Phe Phe Met Pro Asn Leu Ile Gly Gly Ile Val Leu Gly
     50                  55                  60

tat acc tgg cag acc atg atc aac gcc gtg ctt tcg cac tat gcc acg    240
Tyr Thr Trp Gln Thr Met Ile Asn Ala Val Leu Ser His Tyr Ala Thr
 65                  70                  75                  80

act att agc gcg gac tgg aaa ttc ggc tac gcc ggc ctc atc atg cta    288
Thr Ile Ser Ala Asp Trp Lys Phe Gly Tyr Ala Gly Leu Ile Met Leu
                 85                  90                  95

ctt aac tgg cag ctc atc ggc tac atg atg atc att tac atc gcc ggc    336
Leu Asn Trp Gln Leu Ile Gly Tyr Met Met Ile Ile Tyr Ile Ala Gly
            100                 105                 110

ctg caa aac gtc cca cca gag ctc att gag gct gcc gaa ctc gac ggc    384
Leu Gln Asn Val Pro Pro Glu Leu Ile Glu Ala Ala Glu Leu Asp Gly
        115                 120                 125

gtc aac aag tgg gag atg ctg cgg cac gtc act att ccg atg gtc atg    432
Val Asn Lys Trp Glu Met Leu Arg His Val Thr Ile Pro Met Val Met
    130                 135                 140

cca tcc atc acc atc tgc ctc ttt ttg act ttg tcg aac tcc ttt aag    480
Pro Ser Ile Thr Ile Cys Leu Phe Leu Thr Leu Ser Asn Ser Phe Lys
145                 150                 155                 160

ctc ttc gac cag aac ctg gcg ctg acc aac ggc gct cct ggc ggg caa    528
Leu Phe Asp Gln Asn Leu Ala Leu Thr Asn Gly Ala Pro Gly Gly Gln
                165                 170                 175

act gag atg gtg gcg ctc aac atc atc aac acg ctg ttt aac cgt atg    576
Thr Glu Met Val Ala Leu Asn Ile Ile Asn Thr Leu Phe Asn Arg Met
            180                 185                 190

aat gtc gag ggc gtc ggt                                            594
Asn Val Glu Gly Val Gly
        195


<210> 660
<211> 198
<212> PRT
<213> Corynebacterium glutamicum

<400> 660
Lys Ala Phe Xaa Gln Arg Glu Gly Phe Ile Ser Ala Phe Gly Phe Thr
  1               5                  10                  15

Val Leu Val Val Ile Val Ser Val Ile Thr Val Asn Ile Phe Ala Phe
            20                  25                  30

Leu Leu Ala Trp Leu Leu Thr Arg Lys Leu Arg Gly Thr Asn Phe Phe
        35                  40                  45

Arg Thr Val Phe Phe Met Pro Asn Leu Ile Gly Gly Ile Val Leu Gly
    50                  55                  60

Tyr Thr Trp Gln Thr Met Ile Asn Ala Val Leu Ser His Tyr Ala Thr
65                  70                  75                  80

Thr Ile Ser Ala Asp Trp Lys Phe Gly Tyr Ala Gly Leu Ile Met Leu
                85                  90                  95

Leu Asn Trp Gln Leu Ile Gly Tyr Met Met Ile Ile Tyr Ile Ala Gly
            100                 105                 110

Leu Gln Asn Val Pro Pro Glu Leu Ile Glu Ala Ala Glu Leu Asp Gly
        115                 120                 125

Val Asn Lys Trp Glu Met Leu Arg His Val Thr Ile Pro Met Val Met
    130                 135                 140

Pro Ser Ile Thr Ile Cys Leu Phe Leu Thr Leu Ser Asn Ser Phe Lys
145                 150                 155                 160

Leu Phe Asp Gln Asn Leu Ala Leu Thr Asn Gly Ala Pro Gly Gly Gln
                165                 170                 175

Thr Glu Met Val Ala Leu Asn Ile Ile Asn Thr Leu Phe Asn Arg Met
            180                 185                 190

Asn Val Glu Gly Val Gly
        195


<210> 661
<211> 1053
<212> DNA
<213> Corynebacterium glutamicum

<220>
<221> CDS
<222> (101)..(1030)
<223> RXN01298

<400> 661
cttaaacgtc accttattta tgcattatgt tggtttcaga ctcgaacaat tcaattagaa 60

aacactaatc ggacatttag gtcacataac atttccgctc gtg tcc aca tta att   115
                                            Val Ser Thr Leu Ile
                                            1               5

tct gaa ccc gag gtg gat aag cta cgt aaa cgt gcc aag aga tca agg   163
Ser Glu Pro Glu Val Asp Lys Leu Arg Lys Arg Ala Lys Arg Ser Arg
                10                  15                  20

cgg aca gaa tgg tgg ctt gcc gcc gca ctt ctt gcc cca aac ttg ctt   211
Arg Thr Glu Trp Trp Leu Ala Ala Ala Leu Leu Ala Pro Asn Leu Leu

            25                  30                  35

ctc ttg gcc atc ttt acg tat cgg cca ctg tta gat aac ttc cgg ttg 259
Leu Leu Ala Ile Phe Thr Tyr Arg Pro Leu Leu Asp Asn Phe Arg Leu
        40                  45                  50

tcc ttt ttc aac tgg aac att tcc tcg ccc aca tca acc ttc att ggg 307
Ser Phe Phe Asn Trp Asn Ile Ser Ser Pro Thr Ser Thr Phe Ile Gly
    55                  60                  65

ttt gat aac tac gtt gag ttc ttc act cgt agt gac act ctc caa gtt 355
Phe Asp Asn Tyr Val Glu Phe Phe Thr Arg Ser Asp Thr Leu Gln Val
70                  75                  80                  85

gtt tta aac acc gtc atc ttc acg gca tgt gct gtg atc gga tcg atg 403
Val Leu Asn Thr Val Ile Phe Thr Ala Cys Ala Val Ile Gly Ser Met
                90                  95                  100

gtg ctc ggt ttg ctc ctg gcc atg ttg ttg gat cag aag ctt ttc ggc 451
Val Leu Gly Leu Leu Leu Ala Met Leu Leu Asp Gln Lys Leu Phe Gly
            105                 110                 115

cgt aac ttt gtg cgt tcc atg gtg ttt gcc ccg ttt gtg att tcc ggt 499
Arg Asn Phe Val Arg Ser Met Val Phe Ala Pro Phe Val Ile Ser Gly
        120                 125                 130

gct gcc att ggt gtt gct ttc cag ttc gtt ttt gac cct aat ttt ggt 547
Ala Ala Ile Gly Val Ala Phe Gln Phe Val Phe Asp Pro Asn Phe Gly
    135                 140                 145

ttg gtt cag gac ttg ctg gga cgc atc ggc gtt gat tcg cca cag ttc 595
Leu Val Gln Asp Leu Leu Gly Arg Ile Gly Val Asp Ser Pro Gln Phe
150                 155                 160                 165

tac caa aac cct aac tgg gca ttg ttc atg gtg acg ttc act ttc gtg 643
Tyr Gln Asn Pro Asn Trp Ala Leu Phe Met Val Thr Phe Thr Phe Val
                170                 175                 180

tgg aag aac ttg ggc tac tcc ttt gtt atc tac ctg gct gca ttg cag 691
Trp Lys Asn Leu Gly Tyr Ser Phe Val Ile Tyr Leu Ala Ala Leu Gln
            185                 190                 195

ggg cta aac aag gat ttg tct gag gcc gca ccg gtg gat ggc gcg agc 739
Gly Leu Asn Lys Asp Leu Ser Glu Ala Ala Pro Val Asp Gly Ala Ser
        200                 205                 210

gcg tgg aca cgt ttt tgg aag gtt act ctt ccg cag ctt cgc cca acc 787
Ala Trp Thr Arg Phe Trp Lys Val Thr Leu Pro Gln Leu Arg Pro Thr
    215                 220                 225

acg ttc ttc ctt tct att act gtc acg ctg aac tcg gtt cag gtc ttc 835
Thr Phe Phe Leu Ser Ile Thr Val Thr Leu Asn Ser Val Gln Val Phe
230                 235                 240                 245

gac atc att cac acc atg act cgt ggt ggc ccc ttg ggt aac ggt acg 883
Asp Ile Ile His Thr Met Thr Arg Gly Gly Pro Leu Gly Asn Gly Thr
                250                 255                 260

acc acc ttg gtt tac cag gtg tac acc gag act ttc acc aac tat cgc 931
Thr Thr Leu Val Tyr Gln Val Tyr Thr Glu Thr Phe Thr Asn Tyr Arg
            265                 270                 275

gcg gga tat ggt gca aca atc gca acg att ttg ttc ctg ttg ctg ctg 979
Ala Gly Tyr Gly Ala Thr Ile Ala Thr Ile Leu Phe Leu Leu Leu Leu
        280                 285                 290

att atc act gtt atc cag gtt cga tac atg gat aag gag aac aag cag 1027
Ile Ile Thr Val Ile Gln Val Arg Tyr Met Asp Lys Glu Asn Lys Gln
    295                 300                 305

aaa tgatctcgac tgatagaaac gtt 1053
Lys
310

<210> 662
<211> 310
<212> PRT
<213> Corynebacterium glutamicum

<400> 662
Val Ser Thr Leu Ile Ser Glu Pro Glu Val Asp Lys Leu Arg Lys Arg
1               5                   10                  15

Ala Lys Arg Ser Arg Arg Thr Glu Trp Trp Leu Ala Ala Ala Leu Leu
            20                  25                  30

Ala Pro Asn Leu Leu Leu Leu Ala Ile Phe Thr Tyr Arg Pro Leu Leu
        35                  40                  45

Asp Asn Phe Arg Leu Ser Phe Phe Asn Trp Asn Ile Ser Ser Pro Thr
    50                  55                  60

Ser Thr Phe Ile Gly Phe Asp Asn Tyr Val Glu Phe Phe Thr Arg Ser
65                  70                  75                  80

Asp Thr Leu Gln Val Val Leu Asn Thr Val Ile Phe Thr Ala Cys Ala
                85                  90                  95

Val Ile Gly Ser Met Val Leu Gly Leu Leu Leu Ala Met Leu Leu Asp
            100                 105                 110

Gln Lys Leu Phe Gly Arg Asn Phe Val Arg Ser Met Val Phe Ala Pro
        115                 120                 125

Phe Val Ile Ser Gly Ala Ala Ile Gly Val Ala Phe Gln Phe Val Phe
    130                 135                 140

Asp Pro Asn Phe Gly Leu Val Gln Asp Leu Leu Gly Arg Ile Gly Val
145                 150                 155                 160

Asp Ser Pro Gln Phe Tyr Gln Asn Pro Asn Trp Ala Leu Phe Met Val
                165                 170                 175

Thr Phe Thr Phe Val Trp Lys Asn Leu Gly Tyr Ser Phe Val Ile Tyr
            180                 185                 190

Leu Ala Ala Leu Gln Gly Leu Asn Lys Asp Leu Ser Glu Ala Ala Pro
        195                 200                 205

Val Asp Gly Ala Ser Ala Trp Thr Arg Phe Trp Lys Val Thr Leu Pro
    210                 215                 220

Gln Leu Arg Pro Thr Thr Phe Phe Leu Ser Ile Thr Val Thr Leu Asn
225                 230                 235                 240

Ser Val Gln Val Phe Asp Ile Ile His Thr Met Thr Arg Gly Gly Pro
                245                 250                 255

Leu Gly Asn Gly Thr Thr Thr Leu Val Tyr Gln Val Tyr Thr Glu Thr
            260                 265                 270

Phe Thr Asn Tyr Arg Ala Gly Tyr Gly Ala Thr Ile Ala Thr Ile Leu
        275                 280                 285

Phe Leu Leu Leu Leu Ile Ile Thr Val Ile Gln Val Arg Tyr Met Asp
    290                 295                 300

Lys Glu Asn Lys Gln Lys
305                 310

<210> 663
<211> 1182
<212> DNA
<213> Corynebacterium glutamicum

<220>
<221> CDS
<222> (101)..(1159)
<223> RXA01737

<400> 663
ctttgagccg aacgatctgc cgactcaaga agaactagcc cggctgctca gtcaggaatg 60

atgcgcctaa gatcaaacaa gaatgaaagg acggtgaaca atg ggc cga atg aaa   115
                                          Met Gly Arg Met Lys
                                          1               5

aac gat ggt gaa ctc gcg gat ctg ccg gat cat gca ctt ttg agc att   163
Asn Asp Gly Glu Leu Ala Asp Leu Pro Asp His Ala Leu Leu Ser Ile
                10                  15                  20

att cga atc ccg cag gcg gcg aaa aga agc ccc tgg gcg ctg atc tta   211
Ile Arg Ile Pro Gln Ala Ala Lys Arg Ser Pro Trp Ala Leu Ile Leu
            25                  30                  35

acg cgc atc gga tac gcg atg gtg ctg ctg gtt atc gtc acc atg gtt   259
Thr Arg Ile Gly Tyr Ala Met Val Leu Leu Val Ile Val Thr Met Val
        40                  45                  50

gtc tat ttt gac cgc aac gga tac tcc gaa gac ctc acg ttc atc gac   307
Val Tyr Phe Asp Arg Asn Gly Tyr Ser Glu Asp Leu Thr Phe Ile Asp
    55                  60                  65

gcg ttg tac tat tcc aca gtc tcg ttg acc acc gtg ggc tac ggc gat   355
Ala Leu Tyr Tyr Ser Thr Val Ser Leu Thr Thr Val Gly Tyr Gly Asp
70                  75                  80                  85

atc acc ccg gtg acg caa tcg gca cgc ctg atc aac atc atc gtc ctc   403
Ile Thr Pro Val Thr Gln Ser Ala Arg Leu Ile Asn Ile Ile Val Leu
                90                  95                  100

acc cca gca cgc atc ggc ttc ctg atc ctc ctg gtc ggc acc acc ttg 451
Thr Pro Ala Arg Ile Gly Phe Leu Ile Leu Leu Val Gly Thr Thr Leu
            105                 110                 115

tca gtg ctc acc gaa gaa tcg cgc cgg gcc ctg caa atc caa cgt tgg 499
Ser Val Leu Thr Glu Glu Ser Arg Arg Ala Leu Gln Ile Gln Arg Trp
        120                 125                 130

aga aaa cgc atg cgc aac cac acc gtc gtt gtc gga tac gga acc aaa 547
Arg Lys Arg Met Arg Asn His Thr Val Val Val Gly Tyr Gly Thr Lys
    135                 140                 145

ggt cgc tcc gcg gtc gct gca ctg ctt ggc gac ggc gtc ccc gcc aac 595
Gly Arg Ser Ala Val Ala Ala Leu Leu Gly Asp Gly Val Pro Ala Asn
150                 155                 160                 165

cag atc gtt gtt atc gac acc gat caa gtc tcc ctc gac gcc gcc aac 643
Gln Ile Val Val Ile Asp Thr Asp Gln Val Ser Leu Asp Ala Ala Asn
                170                 175                 180

aac agc gga ctc gtc acc gtc aaa ggc tcc gcc acc aaa gca gat gtg 691
Asn Ser Gly Leu Val Thr Val Lys Gly Ser Ala Thr Lys Ala Asp Val
            185                 190                 195

ttg cgt cta gct ggc gtg tca cga gcg cgc gcc gtc gtc gtg gca ccg 739
Leu Arg Leu Ala Gly Val Ser Arg Ala Arg Ala Val Val Val Ala Pro
        200                 205                 210

aac ctg gac gat act gca gtt ctg gtg act cta tcg gtg cga gaa atc 787
Asn Leu Asp Asp Thr Ala Val Leu Val Thr Leu Ser Val Arg Glu Ile
    215                 220                 225

gcg ccg cag gca atg att gtg gcc agt gtc cga gaa tct gaa aac caa 835
Ala Pro Gln Ala Met Ile Val Ala Ser Val Arg Glu Ser Glu Asn Gln
230                 235                 240                 245

cac ctc ctc gaa caa tcc ggt gcg gac tcg gtt gtg atc tcc tca gaa 883
His Leu Leu Glu Gln Ser Gly Ala Asp Ser Val Val Ile Ser Ser Glu
                250                 255                 260

acc gct ggc cga atg ctc ggt ctg gca aca gtt acc cca tcg gtt gtg 931
Thr Ala Gly Arg Met Leu Gly Leu Ala Thr Val Thr Pro Ser Val Val
            265                 270                 275

gag atg atg gaa gac ctc ctc tca ccc gac gaa gga ttc tcc gtt gcc 979
Glu Met Met Glu Asp Leu Leu Ser Pro Asp Glu Gly Phe Ser Val Ala
        280                 285                 290

gaa cga cta gtc ggt gag gat gaa atc ggc tcc aac cca cga cac ctc 1027
Glu Arg Leu Val Gly Glu Asp Glu Ile Gly Ser Asn Pro Arg His Leu
    295                 300                 305

gct gac atc gtc ctc ggt gtt gtt cga tcc ggt gag ctc tac cgc atc 1075
Ala Asp Ile Val Leu Gly Val Val Arg Ser Gly Glu Leu Tyr Arg Ile
310                 315                 320                 325

gac tcc cca gaa gca gaa act gta gag ccc ggc gat cgt ctc ctt tac 1123
Asp Ser Pro Glu Ala Glu Thr Val Glu Pro Gly Asp Arg Leu Leu Tyr
                330                 335                 340

gtt cgc cga gta ttt agc gag gag gta aat gac aaa tgagaattct 1169

Val Arg Arg Val Phe Ser Glu Glu Val Asn Asp Lys
            345                 350

tcccatcggc ccc                                                          1182


<210> 664
<211> 353
<212> PRT
<213> Corynebacterium glutamicum

<400> 664
Met Gly Arg Met Lys Asn Asp Gly Glu Leu Ala Asp Leu Pro Asp His
1               5                   10                  15

Ala Leu Leu Ser Ile Ile Arg Ile Pro Gln Ala Ala Lys Arg Ser Pro
            20                  25                  30

Trp Ala Leu Ile Leu Thr Arg Ile Gly Tyr Ala Met Val Leu Leu Val
        35                  40                  45

Ile Val Thr Met Val Val Tyr Phe Asp Arg Asn Gly Tyr Ser Glu Asp
    50                  55                  60

Leu Thr Phe Ile Asp Ala Leu Tyr Tyr Ser Thr Val Ser Leu Thr Thr
65                  70                  75                  80

Val Gly Tyr Gly Asp Ile Thr Pro Val Thr Gln Ser Ala Arg Leu Ile
                85                  90                  95

Asn Ile Ile Val Leu Thr Pro Ala Arg Ile Gly Phe Leu Ile Leu Leu
            100                 105                 110

Val Gly Thr Thr Leu Ser Val Leu Thr Glu Glu Ser Arg Arg Ala Leu
        115                 120                 125

Gln Ile Gln Arg Trp Arg Lys Arg Met Arg Asn His Thr Val Val Val
    130                 135                 140

Gly Tyr Gly Thr Lys Gly Arg Ser Ala Val Ala Ala Leu Leu Gly Asp
145                 150                 155                 160

Gly Val Pro Ala Asn Gln Ile Val Val Ile Asp Thr Asp Gln Val Ser
                165                 170                 175

Leu Asp Ala Ala Asn Asn Ser Gly Leu Val Thr Val Lys Gly Ser Ala
            180                 185                 190

Thr Lys Ala Asp Val Leu Arg Leu Ala Gly Val Ser Arg Ala Arg Ala
        195                 200                 205

Val Val Val Ala Pro Asn Leu Asp Asp Thr Ala Val Leu Val Thr Leu
    210                 215                 220

Ser Val Arg Glu Ile Ala Pro Gln Ala Met Ile Val Ala Ser Val Arg
225                 230                 235                 240

Glu Ser Glu Asn Gln His Leu Leu Glu Gln Ser Gly Ala Asp Ser Val
                245                 250                 255

Val Ile Ser Ser Glu Thr Ala Gly Arg Met Leu Gly Leu Ala Thr Val

260                 265                 270

Thr Pro Ser Val Val Glu Met Met Glu Asp Leu Leu Ser Pro Asp Glu
        275                 280                 285

Gly Phe Ser Val Ala Glu Arg Leu Val Gly Glu Asp Glu Ile Gly Ser
    290                 295                 300

Asn Pro Arg His Leu Ala Asp Ile Val Leu Gly Val Val Arg Ser Gly
305                 310                 315                 320

Glu Leu Tyr Arg Ile Asp Ser Pro Glu Ala Glu Thr Val Glu Pro Gly
                325                 330                 335

Asp Arg Leu Leu Tyr Val Arg Arg Val Phe Ser Glu Glu Val Asn Asp
            340                 345                 350

Lys

<210> 665
<211> 2007
<212> DNA
<213> Corynebacterium glutamicum

<220>
<221> CDS
<222> (101)..(1984)
<223> RXN02348

<400> 665
aaagacccga gccgaagccc tggcctgcgc atacttcctt gtcaacgctc gctgggatta 60

ggtcttttct gagcgctagc atttctccac tcaaaggagc atg ctt aac cgc atg    115
                                            Met Leu Asn Arg Met
                                            1               5

aaa agt gcg cgg cca aaa tca gtc gct cca aaa tcc gga caa gct tta    163
Lys Ser Ala Arg Pro Lys Ser Val Ala Pro Lys Ser Gly Gln Ala Leu
                10                  15                  20

ctc act ctc ggt gcc cta ggt gtt gtg ttc ggc gac atc ggc acc agc    211
Leu Thr Leu Gly Ala Leu Gly Val Val Phe Gly Asp Ile Gly Thr Ser
            25                  30                  35

ccc ctg tac tca ctt cac act gca ttc agc atg cag cac aac aaa gtc    259
Pro Leu Tyr Ser Leu His Thr Ala Phe Ser Met Gln His Asn Lys Val
        40                  45                  50

gaa gtc act cag gaa aat gtg tac ggc atc atc tcc atg gtg ttg tgg    307
Glu Val Thr Gln Glu Asn Val Tyr Gly Ile Ile Ser Met Val Leu Trp
    55                  60                  65

acc atc act ttg atc gtc acc gtc aaa tac gtc atg ctg gtc acc cga    355
Thr Ile Thr Leu Ile Val Thr Val Lys Tyr Val Met Leu Val Thr Arg
70                  75                  80                  85

gct gac aac caa gga caa ggt ggc atc ctg gcg ctc gtt gct ttg ctg    403
Ala Asp Asn Gln Gly Gln Gly Gly Ile Leu Ala Leu Val Ala Leu Leu
                90                  95                  100

aaa aac cgt ggg cac tgg gga aaa ttc gtg gca gta gcc ggc atg ttg 451
Lys Asn Arg Gly His Trp Gly Lys Phe Val Ala Val Ala Gly Met Leu
            105                 110                 115

ggc gcc gca ttg ttt tat ggc gat gtg gtg atc acc ccg gcg atc tct 499
Gly Ala Ala Leu Phe Tyr Gly Asp Val Val Ile Thr Pro Ala Ile Ser
        120                 125                 130

gtt ctc agc gca aca gaa ggc ttg acg gtt atc tcc cca agc ttt gag 547
Val Leu Ser Ala Thr Glu Gly Leu Thr Val Ile Ser Pro Ser Phe Glu
    135                 140                 145

cgc ttc att ctg ccc gta tct ctc gca gtt ctg atc gct att ttt gca 595
Arg Phe Ile Leu Pro Val Ser Leu Ala Val Leu Ile Ala Ile Phe Ala
150                 155                 160                 165

atc caa ccg ctc ggt aca gaa aaa gtc ggc aaa gcc ttc ggc ccc atc 643
Ile Gln Pro Leu Gly Thr Glu Lys Val Gly Lys Ala Phe Gly Pro Ile
                170                 175                 180

atg ttg ctg tgg ttt gtc acc ctt gca gga ttg gga att ccg caa atc 691
Met Leu Leu Trp Phe Val Thr Leu Ala Gly Leu Gly Ile Pro Gln Ile
            185                 190                 195

atc ggg cac cca gaa atc ttg cag agc ttg tct cca cat tgg gcc ctg 739
Ile Gly His Pro Glu Ile Leu Gln Ser Leu Ser Pro His Trp Ala Leu
        200                 205                 210

cgc ttg att gtg gct gag cct ttc caa gca ttt gtg ctg ctt ggt gcc 787
Arg Leu Ile Val Ala Glu Pro Phe Gln Ala Phe Val Leu Leu Gly Ala
    215                 220                 225

gtt gtc ctg aca gta acg ggt gcg gaa gcg ctc tac gct gat atg ggc 835
Val Val Leu Thr Val Thr Gly Ala Glu Ala Leu Tyr Ala Asp Met Gly
230                 235                 240                 245

cat ttt ggg gcg agg cca atc aga gtg gcg tgg ttt tgc gtc gtc atg 883
His Phe Gly Ala Arg Pro Ile Arg Val Ala Trp Phe Cys Val Val Met
                250                 255                 260

cct gct tta atc ttg acg tat ttg ggg cag ggc gcc ttg gtg atc aac 931
Pro Ala Leu Ile Leu Thr Tyr Leu Gly Gln Gly Ala Leu Val Ile Asn
            265                 270                 275

cag cct gaa gcg gtg cgc aac ccc atg ttt tat ctc gcg ccg gaa ggt 979
Gln Pro Glu Ala Val Arg Asn Pro Met Phe Tyr Leu Ala Pro Glu Gly
        280                 285                 290

ctg cgg att ccg ttg gtt att ttg gcg acc atc gct acg gtg atc gca 1027
Leu Arg Ile Pro Leu Val Ile Leu Ala Thr Ile Ala Thr Val Ile Ala
    295                 300                 305

tcg cag gcc gtg att tct ggt gcg tat tca ttg acc aag cag gcc gtg 1075
Ser Gln Ala Val Ile Ser Gly Ala Tyr Ser Leu Thr Lys Gln Ala Val
310                 315                 320                 325

aat ttg aaa ctg ctg cca cgc atg gtg atc cgg cat acc tcc cgc aaa 1123
Asn Leu Lys Leu Leu Pro Arg Met Val Ile Arg His Thr Ser Arg Lys
                330                 335                 340

gag gaa ggc cag atc tat atg cca ctg gtt aat gga ttg ctg ttt gta 1171
Glu Glu Gly Gln Ile Tyr Met Pro Leu Val Asn Gly Leu Leu Phe Val
            345                 350                 355

tcc gtg atg gtt gtg gtg ctg gta ttc cga tcc tct gaa agc ctc gcc 1219
Ser Val Met Val Val Val Leu Val Phe Arg Ser Ser Glu Ser Leu Ala
        360                 365                 370

agc gcg tac gga ctt gca gtg acc gga acc ttg gtg ctg gtc agc gtc 1267
Ser Ala Tyr Gly Leu Ala Val Thr Gly Thr Leu Val Leu Val Ser Val
    375                 380                 385

ctg tat ctg atc tat gtt cac acc aca tgg tgg aaa aca gcg ctg ttc 1315
Leu Tyr Leu Ile Tyr Val His Thr Thr Trp Trp Lys Thr Ala Leu Phe
390                 395                 400                 405

att gtg ctc atc ggt att cca gaa gta ctt cta ttc gcc tcg aac acc 1363
Ile Val Leu Ile Gly Ile Pro Glu Val Leu Leu Phe Ala Ser Asn Thr
                410                 415                 420

acg aaa att cac gac ggt ggc tgg ctt cca cta ctt att gcg gcc gtg 1411
Thr Lys Ile His Asp Gly Gly Trp Leu Pro Leu Leu Ile Ala Ala Val
            425                 430                 435

ctc atc gtg gtg atg cgg acc tgg gag tgg gga agt gac cgc gtc aat 1459
Leu Ile Val Val Met Arg Thr Trp Glu Trp Gly Ser Asp Arg Val Asn
        440                 445                 450

cag gaa cgc gca gag ctg gaa ctt ccc atg gat aag ttc ttg gag aaa 1507
Gln Glu Arg Ala Glu Leu Glu Leu Pro Met Asp Lys Phe Leu Glu Lys
    455                 460                 465

ctc gat cag cca cac aat att ggt ctg cgt aaa gtt gcc gaa gtg gca 1555
Leu Asp Gln Pro His Asn Ile Gly Leu Arg Lys Val Ala Glu Val Ala
470                 475                 480                 485

gta ttt cca cat ggc acc agc gat act gtc ccg ttg tca ttg gtt cgc 1603
Val Phe Pro His Gly Thr Ser Asp Thr Val Pro Leu Ser Leu Val Arg
                490                 495                 500

tgc gtg aaa gac ctc aag ctt tta tac cga gag atc gtg atc gtt cga 1651
Cys Val Lys Asp Leu Lys Leu Leu Tyr Arg Glu Ile Val Ile Val Arg
            505                 510                 515

atc gtc caa gaa cac gtt ccg cac gtg cca cca gag gaa cgc gcg gaa 1699
Ile Val Gln Glu His Val Pro His Val Pro Pro Glu Glu Arg Ala Glu
        520                 525                 530

atg gaa gtg ctc cat cac gcc ccg atc aga gtc gtg cga gtt gat ctg 1747
Met Glu Val Leu His His Ala Pro Ile Arg Val Val Arg Val Asp Leu
    535                 540                 545

cac ctt ggt tat ttt gat gag cag aac ctg cct gag cat ctc cat gcc 1795
His Leu Gly Tyr Phe Asp Glu Gln Asn Leu Pro Glu His Leu His Ala
550                 555                 560                 565

att gac cca aca tgg gat aac gcc acc tac ttc ctg tct gcc ctg act 1843
Ile Asp Pro Thr Trp Asp Asn Ala Thr Tyr Phe Leu Ser Ala Leu Thr
                570                 575                 580

ctt cgg agc agg ttg cct gga aag att gct ggc tgg cgt gat cgt ttg 1891

Leu Arg Ser Arg Leu Pro Gly Lys Ile Ala Gly Trp Arg Asp Arg Leu
            585                 590                 595

tat ctt tcg atg gaa cgt aat cag gca tct cga act gag tct ttc aaa      1939
Tyr Leu Ser Met Glu Arg Asn Gln Ala Ser Arg Thr Glu Ser Phe Lys
        600                 605                 610

ttg caa cca agc aaa acc atc acg gtt gga aca gag ctg cac ctt          1984
Leu Gln Pro Ser Lys Thr Ile Thr Val Gly Thr Glu Leu His Leu
    615                 620                 625

taatcaggca gttgctggcc aac                                            2007

<210> 666
<211> 628
<212> PRT
<213> Corynebacterium glutamicum

<400> 666
Met Leu Asn Arg Met Lys Ser Ala Arg Pro Lys Ser Val Ala Pro Lys
  1               5                  10                  15

Ser Gly Gln Ala Leu Leu Thr Leu Gly Ala Leu Gly Val Val Phe Gly
             20                  25                  30

Asp Ile Gly Thr Ser Pro Leu Tyr Ser Leu His Thr Ala Phe Ser Met
         35                  40                  45

Gln His Asn Lys Val Glu Val Thr Gln Glu Asn Val Tyr Gly Ile Ile
     50                  55                  60

Ser Met Val Leu Trp Thr Ile Thr Leu Ile Val Thr Val Lys Tyr Val
 65                  70                  75                  80

Met Leu Val Thr Arg Ala Asp Asn Gln Gly Gln Gly Gly Ile Leu Ala
                 85                  90                  95

Leu Val Ala Leu Leu Lys Asn Arg Gly His Trp Gly Lys Phe Val Ala
            100                 105                 110

Val Ala Gly Met Leu Gly Ala Ala Leu Phe Tyr Gly Asp Val Val Ile
        115                 120                 125

Thr Pro Ala Ile Ser Val Leu Ser Ala Thr Glu Gly Leu Thr Val Ile
    130                 135                 140

Ser Pro Ser Phe Glu Arg Phe Ile Leu Pro Val Ser Leu Ala Val Leu
145                 150                 155                 160

Ile Ala Ile Phe Ala Ile Gln Pro Leu Gly Thr Glu Lys Val Gly Lys
                165                 170                 175

Ala Phe Gly Pro Ile Met Leu Leu Trp Phe Val Thr Leu Ala Gly Leu
            180                 185                 190

Gly Ile Pro Gln Ile Ile Gly His Pro Glu Ile Leu Gln Ser Leu Ser
        195                 200                 205

Pro His Trp Ala Leu Arg Leu Ile Val Ala Glu Pro Phe Gln Ala Phe
    210                 215                 220

Val Leu Leu Gly Ala Val Val Leu Thr Val Thr Gly Ala Glu Ala Leu
225                 230                 235                 240

Tyr Ala Asp Met Gly His Phe Gly Ala Arg Pro Ile Arg Val Ala Trp
                245                 250                 255

Phe Cys Val Val Met Pro Ala Leu Ile Leu Thr Tyr Leu Gly Gln Gly
            260                 265                 270

Ala Leu Val Ile Asn Gln Pro Glu Ala Val Arg Asn Pro Met Phe Tyr
        275                 280                 285

Leu Ala Pro Glu Gly Leu Arg Ile Pro Leu Val Ile Leu Ala Thr Ile
    290                 295                 300

Ala Thr Val Ile Ala Ser Gln Ala Val Ile Ser Gly Ala Tyr Ser Leu
305                 310                 315                 320

Thr Lys Gln Ala Val Asn Leu Lys Leu Leu Pro Arg Met Val Ile Arg
                325                 330                 335

His Thr Ser Arg Lys Glu Glu Gly Gln Ile Tyr Met Pro Leu Val Asn
            340                 345                 350

Gly Leu Leu Phe Val Ser Val Met Val Val Val Leu Val Phe Arg Ser
        355                 360                 365

Ser Glu Ser Leu Ala Ser Ala Tyr Gly Leu Ala Val Thr Gly Thr Leu
    370                 375                 380

Val Leu Val Ser Val Leu Tyr Leu Ile Tyr Val His Thr Thr Trp Trp
385                 390                 395                 400

Lys Thr Ala Leu Phe Ile Val Leu Ile Gly Ile Pro Glu Val Leu Leu
                405                 410                 415

Phe Ala Ser Asn Thr Thr Lys Ile His Asp Gly Gly Trp Leu Pro Leu
            420                 425                 430

Leu Ile Ala Ala Val Leu Ile Val Val Met Arg Thr Trp Glu Trp Gly
        435                 440                 445

Ser Asp Arg Val Asn Gln Glu Arg Ala Glu Leu Glu Leu Pro Met Asp
    450                 455                 460

Lys Phe Leu Glu Lys Leu Asp Gln Pro His Asn Ile Gly Leu Arg Lys
465                 470                 475                 480

Val Ala Glu Val Ala Val Phe Pro His Gly Thr Ser Asp Thr Val Pro
                485                 490                 495

Leu Ser Leu Val Arg Cys Val Lys Asp Leu Lys Leu Leu Tyr Arg Glu
            500                 505                 510

Ile Val Ile Val Arg Ile Val Gln Glu His Val Pro His Val Pro Pro
        515                 520                 525

Glu Glu Arg Ala Glu Met Glu Val Leu His His Ala Pro Ile Arg Val
    530                 535                 540

Val Arg Val Asp Leu His Leu Gly Tyr Phe Asp Glu Gln Asn Leu Pro
545                 550                 555                 560

Glu His Leu His Ala Ile Asp Pro Thr Trp Asp Asn Ala Thr Tyr Phe
                565                 570                 575

Leu Ser Ala Leu Thr Leu Arg Ser Arg Leu Pro Gly Lys Ile Ala Gly
            580                 585                 590

Trp Arg Asp Arg Leu Tyr Leu Ser Met Glu Arg Asn Gln Ala Ser Arg
        595                 600                 605

Thr Glu Ser Phe Lys Leu Gln Pro Ser Lys Thr Ile Thr Val Gly Thr
    610                 615                 620

Glu Leu His Leu
625

<210> 667
<211> 1656
<212> DNA
<213> Corynebacterium glutamicum

<220>
<221> CDS
<222> (101)..(1633)
<223> RXA02426

<400> 667
tgcagcagca taatcaggga aaaataggaa ctgtttactg aaaaactgac aaacctgaca 60

gacctgaccc gaagagacct tattcatgac gatattgttc atg ctc att aca ttg   115
                                            Met Leu Ile Thr Leu
                                            1               5

ctg ctc gcc acc gtg att gtg gtg gcc atc ggc gat aaa acc gga ctc   163
Leu Leu Ala Thr Val Ile Val Val Ala Ile Gly Asp Lys Thr Gly Leu
                10                  15                  20

ccc tgg cct gct ttg atg act att gtc gcc gca ggt gga gcg ttg ttg   211
Pro Trp Pro Ala Leu Met Thr Ile Val Ala Ala Gly Gly Ala Leu Leu
            25                  30                  35

cca ttt ttg ccg gaa ttt act atc ccg gcc gat ctg atg ttg cct att   259
Pro Phe Leu Pro Glu Phe Thr Ile Pro Ala Asp Leu Met Leu Pro Ile
        40                  45                  50

ttc att ccg ccg ctg ctg tgg gcg ctg gcg agg aag tca tcg tgg gcg   307
Phe Ile Pro Pro Leu Leu Trp Ala Leu Ala Arg Lys Ser Ser Trp Ala
    55                  60                  65

gtg att agg tcg cag atg tct acc atc atc acg atg tcg gtg ttg ttg   355
Val Ile Arg Ser Gln Met Ser Thr Ile Ile Thr Met Ser Val Leu Leu
70                  75                  80                  85

gtg ttc gtg acc atc gcg gca ctc acg ggt gca tcc atg ctg ttg ctt   403
Val Phe Val Thr Ile Ala Ala Leu Thr Gly Ala Ser Met Leu Leu Leu
                90                  95                  100

cct ggc att ggt ctt gct ggt gcg atc atg ttg gct gcg gct att gct   451

Pro Gly Ile Gly Leu Ala Gly Ala Ile Met Leu Ala Ala Ala Ile Ala
            105                 110                 115

cca ccg gat cct gtt gct gtt gat gca gtg gcg gaa cct gcg gga atc 499
Pro Pro Asp Pro Val Ala Val Asp Ala Val Ala Glu Pro Ala Gly Ile
        120                 125                 130

ccg aag cgc atc acc acc act ttg cag acg gag ggt ctg ttt aat gat 547
Pro Lys Arg Ile Thr Thr Thr Leu Gln Thr Glu Gly Leu Phe Asn Asp
    135                 140                 145

gca gcc agc atc gtg gcg ttc cat gtg gct tta gct gcc ctg gtc gct 595
Ala Ala Ser Ile Val Ala Phe His Val Ala Leu Ala Ala Leu Val Ala
150                 155                 160                 165

ggt gag gat ttg tcc tgg tca acg ggt gtt ttg gaa ttc ttg tgg tcg 643
Gly Glu Asp Leu Ser Trp Ser Thr Gly Val Leu Glu Phe Leu Trp Ser
                170                 175                 180

tgt ctt gct gcc gtt att ttg ggc ttg gtt att ggc cgg gcg gct gcg 691
Cys Leu Ala Ala Val Ile Leu Gly Leu Val Ile Gly Arg Ala Ala Ala
            185                 190                 195

tgg ttt acc gat cac gtg agt tcc gtt gag gct cga aat gcg ttt acg 739
Trp Phe Thr Asp His Val Ser Ser Val Glu Ala Arg Asn Ala Phe Thr
        200                 205                 210

tgg gtg ctg ccg ttt gcc att tat gtg gtg gcg gaa gaa atc ggc gga 787
Trp Val Leu Pro Phe Ala Ile Tyr Val Val Ala Glu Glu Ile Gly Gly
    215                 220                 225

tcg ggc gtt atc gcc att gtg atc gct gca gtg gag atg aat tcg agg 835
Ser Gly Val Ile Ala Ile Val Ile Ala Ala Val Glu Met Asn Ser Arg
230                 235                 240                 245

gcg tcg att ggt gcg gag gat cgt cta acg ggt tct gcg ttc tgg gga 883
Ala Ser Ile Gly Ala Glu Asp Arg Leu Thr Gly Ser Ala Phe Trp Gly
                250                 255                 260

acc att gag gtg ctg ttt act ggc gtt gcc ttt ggt ttg atc ggc ctg 931
Thr Ile Glu Val Leu Phe Thr Gly Val Ala Phe Gly Leu Ile Gly Leu
            265                 270                 275

aat gtg cgc gct gcg att gat gaa gtt gga tct gag ctg tgg cat gcc 979
Asn Val Arg Ala Ala Ile Asp Glu Val Gly Ser Glu Leu Trp His Ala
        280                 285                 290

gtg gtc gtg ggc att gtg ctc tcg gtg gtg gcg atc gtt gtc cgt ggt 1027
Val Val Val Gly Ile Val Leu Ser Val Val Ala Ile Val Val Arg Gly
    295                 300                 305

gtg tgg atg ttc gcg gcg tat aag cgc aat cgt ttc aag atc gat aag 1075
Val Trp Met Phe Ala Ala Tyr Lys Arg Asn Arg Phe Lys Ile Asp Lys
310                 315                 320                 325

aag ggt gcg acc aat agt tca ttg cgg gcg cct ctt cga ctg cag gaa 1123
Lys Gly Ala Thr Asn Ser Ser Leu Arg Ala Pro Leu Arg Leu Gln Glu
                330                 335                 340

tcg ttg ctg atg acg tgg gcg ggc atg cgc ggt ttg gtg acg ttg gcg 1171
Ser Leu Leu Met Thr Trp Ala Gly Met Arg Gly Leu Val Thr Leu Ala

345                 350                 355

ctg gtg ctg tct att ccg gag gat att ttc ccg tat cac cac gag ttg     1219
Leu Val Leu Ser Ile Pro Glu Asp Ile Phe Pro Tyr His His Glu Leu
        360                 365                 370

cag gtc att gcg ctg gtc gtt ctc tta atc acc atg gtg ggc cct ggt     1267
Gln Val Ile Ala Leu Val Val Leu Leu Ile Thr Met Val Gly Pro Gly
    375                 380                 385

ttg acg ttg ccg tgg ctg atg cgg aag ctc agc ttg gat aag ggt ccc     1315
Leu Thr Leu Pro Trp Leu Met Arg Lys Leu Ser Leu Asp Lys Gly Pro
390                 395                 400                 405

gat gct gcg ggc gat gag agt atc gcc gcg ctg aca gag agg gct cac     1363
Asp Ala Ala Gly Asp Glu Ser Ile Ala Ala Leu Thr Glu Arg Ala His
                410                 415                 420

aag gcc gca aca acg tat ttg gtg gat acc acg gag ttg ccg atg gag     1411
Lys Ala Ala Thr Thr Tyr Leu Val Asp Thr Thr Glu Leu Pro Met Glu
            425                 430                 435

cag atg gtg gcg atc aag aat tgg ttc tcg caa gaa att gac gct gat     1459
Gln Met Val Ala Ile Lys Asn Trp Phe Ser Gln Glu Ile Asp Ala Asp
        440                 445                 450

gaa ctg cag gag aac gtc gat aag ctg cat cag cgc gcg cat cat gcg     1507
Glu Leu Gln Glu Asn Val Asp Lys Leu His Gln Arg Ala His His Ala
    455                 460                 465

cgt gtc ggg gca att aag gcg gcc cag gag gag ctg ttg aag gcg cgg     1555
Arg Val Gly Ala Ile Lys Ala Ala Gln Glu Glu Leu Leu Lys Ala Arg
470                 475                 480                 485

cgg gag cgc ggc gtt aat ccg gcc tat gtt gat gag gtg ttg acc aac     1603
Arg Glu Arg Gly Val Asn Pro Ala Tyr Val Asp Glu Val Leu Thr Asn
                490                 495                 500

att gac cgg atg ctt gtt gcg gct gaa cgc tagataaaca gcagcaggct     1653
Ile Asp Arg Met Leu Val Ala Ala Glu Arg
            505                 510

gat                                                                 1656

<210> 668
<211> 511
<212> PRT
<213> Corynebacterium glutamicum

<400> 668
Met Leu Ile Thr Leu Leu Leu Ala Thr Val Ile Val Val Ala Ile Gly
1               5                   10                  15

Asp Lys Thr Gly Leu Pro Trp Pro Ala Leu Met Thr Ile Val Ala Ala
            20                  25                  30

Gly Gly Ala Leu Leu Pro Phe Leu Pro Glu Phe Thr Ile Pro Ala Asp
        35                  40                  45

Leu Met Leu Pro Ile Phe Ile Pro Pro Leu Leu Trp Ala Leu Ala Arg

    50                  55                  60

Lys Ser Ser Trp Ala Val Ile Arg Ser Gln Met Ser Thr Ile Ile Thr
65                  70                  75                  80

Met Ser Val Leu Leu Val Phe Val Thr Ile Ala Ala Leu Thr Gly Ala
                85                  90                  95

Ser Met Leu Leu Leu Pro Gly Ile Gly Leu Ala Gly Ala Ile Met Leu
            100                 105                 110

Ala Ala Ala Ile Ala Pro Pro Asp Pro Val Ala Val Asp Ala Val Ala
        115                 120                 125

Glu Pro Ala Gly Ile Pro Lys Arg Ile Thr Thr Thr Leu Gln Thr Glu
    130                 135                 140

Gly Leu Phe Asn Asp Ala Ala Ser Ile Val Ala Phe His Val Ala Leu
145                 150                 155                 160

Ala Ala Leu Val Ala Gly Glu Asp Leu Ser Trp Ser Thr Gly Val Leu
                165                 170                 175

Glu Phe Leu Trp Ser Cys Leu Ala Ala Val Ile Leu Gly Leu Val Ile
            180                 185                 190

Gly Arg Ala Ala Ala Trp Phe Thr Asp His Val Ser Ser Val Glu Ala
        195                 200                 205

Arg Asn Ala Phe Thr Trp Val Leu Pro Phe Ala Ile Tyr Val Val Ala
    210                 215                 220

Glu Glu Ile Gly Gly Ser Gly Val Ile Ala Ile Val Ile Ala Ala Val
225                 230                 235                 240

Glu Met Asn Ser Arg Ala Ser Ile Gly Ala Glu Asp Arg Leu Thr Gly
                245                 250                 255

Ser Ala Phe Trp Gly Thr Ile Glu Val Leu Phe Thr Gly Val Ala Phe
            260                 265                 270

Gly Leu Ile Gly Leu Asn Val Arg Ala Ala Ile Asp Glu Val Gly Ser
        275                 280                 285

Glu Leu Trp His Ala Val Val Val Gly Ile Val Leu Ser Val Val Ala
    290                 295                 300

Ile Val Val Arg Gly Val Trp Met Phe Ala Ala Tyr Lys Arg Asn Arg
305                 310                 315                 320

Phe Lys Ile Asp Lys Lys Gly Ala Thr Asn Ser Ser Leu Arg Ala Pro
                325                 330                 335

Leu Arg Leu Gln Glu Ser Leu Leu Met Thr Trp Ala Gly Met Arg Gly
            340                 345                 350

Leu Val Thr Leu Ala Leu Val Leu Ser Ile Pro Glu Asp Ile Phe Pro
        355                 360                 365

Tyr His His Glu Leu Gln Val Ile Ala Leu Val Val Leu Leu Ile Thr
    370                 375                 380

Met Val Gly Pro Gly Leu Thr Leu Pro Trp Leu Met Arg Lys Leu Ser
385                 390                 395                 400

Leu Asp Lys Gly Pro Asp Ala Ala Gly Asp Glu Ser Ile Ala Ala Leu
                405                 410                 415

Thr Glu Arg Ala His Lys Ala Ala Thr Thr Tyr Leu Val Asp Thr Thr
            420                 425                 430

Glu Leu Pro Met Glu Gln Met Val Ala Ile Lys Asn Trp Phe Ser Gln
        435                 440                 445

Glu Ile Asp Ala Asp Glu Leu Gln Glu Asn Val Asp Lys Leu His Gln
    450                 455                 460

Arg Ala His His Ala Arg Val Gly Ala Ile Lys Ala Ala Gln Glu Glu
465                 470                 475                 480

Leu Leu Lys Ala Arg Arg Glu Arg Gly Val Asn Pro Ala Tyr Val Asp
                485                 490                 495

Glu Val Leu Thr Asn Ile Asp Arg Met Leu Val Ala Ala Glu Arg
            500                 505                 510

<210> 669
<211> 993
<212> DNA
<213> Corynebacterium glutamicum

<220>
<221> CDS
<222> (101)..(970)
<223> RXN03164

<400> 669
cttttttgca tccagatgca caaagccgtg gcacaaacga gacaaactga gcacaatggc 60

tgtcatggca tatcaaccag cagacaatcg ctatgacgac atg atc tac cgc agg   115
                                           Met Ile Tyr Arg Arg
                                           1               5

gtg gga aat tct ggg ctg aag ctt ccg gca att tcg ctt ggg ctg tgg   163
Val Gly Asn Ser Gly Leu Lys Leu Pro Ala Ile Ser Leu Gly Leu Trp
                10                  15                  20

cac aac ttc ggt gat gac aag ccg ctt tca acg cag cgc agc att att   211
His Asn Phe Gly Asp Asp Lys Pro Leu Ser Thr Gln Arg Ser Ile Ile
            25                  30                  35

cac cgc gcg ttt gat agg gga gtc act cac ttc gat ttg gct aat aac   259
His Arg Ala Phe Asp Arg Gly Val Thr His Phe Asp Leu Ala Asn Asn
        40                  45                  50

tat gga cct cca gca ggt tcc gca gag acc aac ttt ggc agg att ttg   307
Tyr Gly Pro Pro Ala Gly Ser Ala Glu Thr Asn Phe Gly Arg Ile Leu
    55                  60                  65

cgt gag gat ctc aaa agc cac cgc gat gag ttg atc att tct tcc aag   355
Arg Glu Asp Leu Lys Ser His Arg Asp Glu Leu Ile Ile Ser Ser Lys

70                    75                    80                    85

gcg ggt tgg gat atg tgg cct gga cct tat ggt ttt ggt ggt tcc cga     403
Ala Gly Trp Asp Met Trp Pro Gly Pro Tyr Gly Phe Gly Gly Ser Arg
                90                    95                    100

aag tat cta gtg agt tcc ctt gat cag tcc ctg act cgc ctc ggc ttg     451
Lys Tyr Leu Val Ser Ser Leu Asp Gln Ser Leu Thr Arg Leu Gly Leu
            105                   110                   115

gat tac gtg gat att ttc tat cat cac cgc ccg gat cca gat act cct     499
Asp Tyr Val Asp Ile Phe Tyr His His Arg Pro Asp Pro Asp Thr Pro
        120                   125                   130

ttg gaa gaa acc atg tac gca ttg cgt gac att gtt gcg tct gga aag     547
Leu Glu Glu Thr Met Tyr Ala Leu Arg Asp Ile Val Ala Ser Gly Lys
    135                   140                   145

gct ctt tac gtg ggt att tct tcc tac ggt cca gag ctc aca gcg gag     595
Ala Leu Tyr Val Gly Ile Ser Ser Tyr Gly Pro Glu Leu Thr Ala Glu
150                   155                   160                   165

gcg gct gag ttc atg gcg gag gag ggc tgc ccg ctt ctg att cat cag     643
Ala Ala Glu Phe Met Ala Glu Glu Gly Cys Pro Leu Leu Ile His Gln
                170                   175                   180

cca agc tat tcc atc att aat cgt tgg gtg gag gaa ccg ggc gat gac     691
Pro Ser Tyr Ser Ile Ile Asn Arg Trp Val Glu Glu Pro Gly Asp Asp
            185                   190                   195

ggt gag aac ttg ttg cag tca gct gcc aac aat ggt ctt ggc gtc att     739
Gly Glu Asn Leu Leu Gln Ser Ala Ala Asn Asn Gly Leu Gly Val Ile
        200                   205                   210

gct ttc tca cca ctt gcg cag ggc ctg ctc acg gac aaa tat ctc gat     787
Ala Phe Ser Pro Leu Ala Gln Gly Leu Leu Thr Asp Lys Tyr Leu Asp
    215                   220                   225

gga att cca gag ggt tcc cgc gcc agc cag ggt aag tcc ctg tct gag     835
Gly Ile Pro Glu Gly Ser Arg Ala Ser Gln Gly Lys Ser Leu Ser Glu
230                   235                   240                   245

ggc atg ttg aac gtg aac aat att gat atg gtc cgc aag ctc aat gac     883
Gly Met Leu Asn Val Asn Asn Ile Asp Met Val Arg Lys Leu Asn Asp
                250                   255                   260

atc gcc cag gaa cgc ggg cag tca ctt gcg cag atg gcg ctt gca tgg     931
Ile Ala Gln Glu Arg Gly Gln Ser Leu Ala Gln Met Ala Leu Ala Trp
            265                   270                   275

gtg ctg cgc gag caa aga gag tac ggc gcc gga tta ccg tgaccagtgc     980
Val Leu Arg Glu Gln Arg Glu Tyr Gly Ala Gly Leu Pro
        280                   285                   290

attgattggt gct                                                      993

<210> 670
<211> 290
<212> PRT
<213> Corynebacterium glutamicum

<400> 670
Met Ile Tyr Arg Arg Val Gly Asn Ser Gly Leu Lys Leu Pro Ala Ile
1               5                   10                  15

Ser Leu Gly Leu Trp His Asn Phe Gly Asp Asp Lys Pro Leu Ser Thr
            20                  25                  30

Gln Arg Ser Ile Ile His Arg Ala Phe Asp Arg Gly Val Thr His Phe
        35                  40                  45

Asp Leu Ala Asn Asn Tyr Gly Pro Pro Ala Gly Ser Ala Glu Thr Asn
    50                  55                  60

Phe Gly Arg Ile Leu Arg Glu Asp Leu Lys Ser His Arg Asp Glu Leu
65                  70                  75                  80

Ile Ile Ser Ser Lys Ala Gly Trp Asp Met Trp Pro Gly Pro Tyr Gly
                85                  90                  95

Phe Gly Gly Ser Arg Lys Tyr Leu Val Ser Ser Leu Asp Gln Ser Leu
            100                 105                 110

Thr Arg Leu Gly Leu Asp Tyr Val Asp Ile Phe Tyr His His Arg Pro
        115                 120                 125

Asp Pro Asp Thr Pro Leu Glu Glu Thr Met Tyr Ala Leu Arg Asp Ile
    130                 135                 140

Val Ala Ser Gly Lys Ala Leu Tyr Val Gly Ile Ser Ser Tyr Gly Pro
145                 150                 155                 160

Glu Leu Thr Ala Glu Ala Ala Glu Phe Met Ala Glu Glu Gly Cys Pro
                165                 170                 175

Leu Leu Ile His Gln Pro Ser Tyr Ser Ile Ile Asn Arg Trp Val Glu
            180                 185                 190

Glu Pro Gly Asp Asp Gly Glu Asn Leu Leu Gln Ser Ala Ala Asn Asn
        195                 200                 205

Gly Leu Gly Val Ile Ala Phe Ser Pro Leu Ala Gln Gly Leu Leu Thr
    210                 215                 220

Asp Lys Tyr Leu Asp Gly Ile Pro Glu Gly Ser Arg Ala Ser Gln Gly
225                 230                 235                 240

Lys Ser Leu Ser Glu Gly Met Leu Asn Val Asn Asn Ile Asp Met Val
                245                 250                 255

Arg Lys Leu Asn Asp Ile Ala Gln Glu Arg Gly Gln Ser Leu Ala Gln
            260                 265                 270

Met Ala Leu Ala Trp Val Leu Arg Glu Gln Arg Glu Tyr Gly Ala Gly
        275                 280                 285

Leu Pro
    290

<210> 671

<211> 1068
<212> DNA
<213> Corynebacterium glutamicum

<220>
<221> CDS
<222> (101)..(1045)
<223> RXN00024

<400> 671
ttaagacatc aacatatggc ttgtgctact gaaagatttt tcttctgaaa ttctgtagaa 60

acgctcctat gctcggggca gtaagttgtg agcataggaa atg gag cac ggc gtg 115
Met Glu His Gly Val
1               5

acc gtt att aaa ggc act gaa ttt gat gtt ttc cca cta aac ctc ggt 163
Thr Val Ile Lys Gly Thr Glu Phe Asp Val Phe Pro Leu Asn Leu Gly
                10                  15                  20

gga aat acc ttt ggc tgg acc tcg aat agg gaa cag acc ttc gcg gtt 211
Gly Asn Thr Phe Gly Trp Thr Ser Asn Arg Glu Gln Thr Phe Ala Val
            25                  30                  35

ttg gat gca ttc gtg gca gcg gga gga aac ttt gtt gac acc gcc gat 259
Leu Asp Ala Phe Val Ala Ala Gly Gly Asn Phe Val Asp Thr Ala Asp
        40                  45                  50

tct tat tct gca tgg gtt gaa ggc aat gag ggt ggc gag tcg gag cgg 307
Ser Tyr Ser Ala Trp Val Glu Gly Asn Glu Gly Gly Glu Ser Glu Arg
    55                  60                  65

gag ctc ggc gcg tgg att aag gaa cgt ggc gca gac aag ctg atc att 355
Glu Leu Gly Ala Trp Ile Lys Glu Arg Gly Ala Asp Lys Leu Ile Ile
70                  75                  80                  85

gct acc aag tct ggt gcg ttg gag cct gtt gct ggt cgt tcc cgt gag 403
Ala Thr Lys Ser Gly Ala Leu Glu Pro Val Ala Gly Arg Ser Arg Glu
                90                  95                  100

gca act ttc aag gct gtc gag ggt tcc ctg gag cgt ttg ggc gtg gaa 451
Ala Thr Phe Lys Ala Val Glu Gly Ser Leu Glu Arg Leu Gly Val Glu
            105                 110                 115

tcg atc gat att ttt tac tac cac tac gac gat gag gca gtc agc att 499
Ser Ile Asp Ile Phe Tyr Tyr His Tyr Asp Asp Glu Ala Val Ser Ile
        120                 125                 130

gat gag cag gtt gct atc gct aat gat ctg att gca cag ggc aag att 547
Asp Glu Gln Val Ala Ile Ala Asn Asp Leu Ile Ala Gln Gly Lys Ile
    135                 140                 145

aag cac ctc gca ttg tct aac tac agc gcg gag cgt tta gct gag ttc 595
Lys His Leu Ala Leu Ser Asn Tyr Ser Ala Glu Arg Leu Ala Glu Phe
150                 155                 160                 165

ttt gag aag tct gta ggc act cca gcg cag ccg gtt gct ctg caa ccg 643
Phe Glu Lys Ser Val Gly Thr Pro Ala Gln Pro Val Ala Leu Gln Pro
                170                 175                 180

cac tac aac ctg gtg tcg agg aag gat tat gag gag aac gtg cag cca 691

His Tyr Asn Leu Val Ser Arg Lys Asp Tyr Glu Glu Asn Val Gln Pro
            185                 190                 195

ctc gcc gag aag cat ggc gtt gca gtc ttc cct tat ttc gcg ctt gcc 739
Leu Ala Glu Lys His Gly Val Ala Val Phe Pro Tyr Phe Ala Leu Ala
        200                 205                 210

gcg ggt ctt ttg acc gga aag tac acc tcc aag gag gat att tcg ggt 787
Ala Gly Leu Leu Thr Gly Lys Tyr Thr Ser Lys Glu Asp Ile Ser Gly
    215                 220                 225

aaa gcg cgt gcg ggg cag ttg gat cgt tac gcc agc gat gag gcg ttt 835
Lys Ala Arg Ala Gly Gln Leu Asp Arg Tyr Ala Ser Asp Glu Ala Phe
230                 235                 240                 245

gcc gtg gtg aca gag ttg cgt gct gtt gcc gat gag ttg ggt gtt gcg 883
Ala Val Val Thr Glu Leu Arg Ala Val Ala Asp Glu Leu Gly Val Ala
                250                 255                 260

cca acg act gtg gcg ctt gcg tgg ttg gtt gcg cat ggt gtg acc gca 931
Pro Thr Thr Val Ala Leu Ala Trp Leu Val Ala His Gly Val Thr Ala
            265                 270                 275

ccg att gcg tcc gtg tcc aag gta gag cag ttg aag gat ttg atg gct 979
Pro Ile Ala Ser Val Ser Lys Val Glu Gln Leu Lys Asp Leu Met Ala
        280                 285                 290

gtg aag gat gtg gag ctg agc gct gag cag ctt gca cgt ttg gat aag 1027
Val Lys Asp Val Glu Leu Ser Ala Glu Gln Leu Ala Arg Leu Asp Lys
    295                 300                 305

gtt tcg gag cct ttc gct taagctctcc tcaaaagtaa gtg 1068
Val Ser Glu Pro Phe Ala
310                 315


<210> 672
<211> 315
<212> PRT
<213> Corynebacterium glutamicum

<400> 672
Met Glu His Gly Val Thr Val Ile Lys Gly Thr Glu Phe Asp Val Phe
1               5                   10                  15

Pro Leu Asn Leu Gly Gly Asn Thr Phe Gly Trp Thr Ser Asn Arg Glu
            20                  25                  30

Gln Thr Phe Ala Val Leu Asp Ala Phe Val Ala Ala Gly Gly Asn Phe
        35                  40                  45

Val Asp Thr Ala Asp Ser Tyr Ser Ala Trp Val Glu Gly Asn Glu Gly
    50                  55                  60

Gly Glu Ser Glu Arg Glu Leu Gly Ala Trp Ile Lys Glu Arg Gly Ala
65                  70                  75                  80

Asp Lys Leu Ile Ile Ala Thr Lys Ser Gly Ala Leu Glu Pro Val Ala
                85                  90                  95

Gly Arg Ser Arg Glu Ala Thr Phe Lys Ala Val Glu Gly Ser Leu Glu

            100                 105                 110

Arg Leu Gly Val Glu Ser Ile Asp Ile Phe Tyr Tyr His Tyr Asp Asp
        115                 120                 125

Glu Ala Val Ser Ile Asp Glu Gln Val Ala Ile Ala Asn Asp Leu Ile
    130                 135                 140

Ala Gln Gly Lys Ile Lys His Leu Ala Leu Ser Asn Tyr Ser Ala Glu
145                 150                 155                 160

Arg Leu Ala Glu Phe Phe Glu Lys Ser Val Gly Thr Pro Ala Gln Pro
                165                 170                 175

Val Ala Leu Gln Pro His Tyr Asn Leu Val Ser Arg Lys Asp Tyr Glu
            180                 185                 190

Glu Asn Val Gln Pro Leu Ala Glu Lys His Gly Val Ala Val Phe Pro
        195                 200                 205

Tyr Phe Ala Leu Ala Ala Gly Leu Leu Thr Gly Lys Tyr Thr Ser Lys
    210                 215                 220

Glu Asp Ile Ser Gly Lys Ala Arg Ala Gly Gln Leu Asp Arg Tyr Ala
225                 230                 235                 240

Ser Asp Glu Ala Phe Ala Val Val Thr Glu Leu Arg Ala Val Ala Asp
                245                 250                 255

Glu Leu Gly Val Ala Pro Thr Thr Val Ala Leu Ala Trp Leu Val Ala
            260                 265                 270

His Gly Val Thr Ala Pro Ile Ala Ser Val Ser Lys Val Glu Gln Leu
        275                 280                 285

Lys Asp Leu Met Ala Val Lys Asp Val Glu Leu Ser Ala Glu Gln Leu
    290                 295                 300

Ala Arg Leu Asp Lys Val Ser Glu Pro Phe Ala
305                 310                 315

<210> 673
<211> 1758
<212> DNA
<213> Corynebacterium glutamicum

<220>
<221> CDS
<222> (101)..(1735)
<223> RXN01164

<400> 673
gccgatcgtg attgatgaag acgagatcca agcctggact tctgatctca aacctgaaga 60

tttcaccaaa ggtaaagatg aatccgacgg tgagaaataa gtg aca ctg ttt gtt 115
Val Thr Leu Phe Val
1               5

cgg ctc gcc ctt gct gct gtg ggc ggg ctt ttt gtc ttt gct tcc aat 163
Arg Leu Ala Leu Ala Ala Val Gly Gly Leu Phe Val Phe Ala Ser Asn

10                  15                  20

gaa ccg atc ggc tgg ttt gtc gcg gga att gtt ggc act gca tta ttt    211
Glu Pro Ile Gly Trp Phe Val Ala Gly Ile Val Gly Thr Ala Leu Phe
            25                  30                  35

ttt atc tcc ctt gcg ccg tgg gat ctg gga gtt ccc caa aag cgg cgg    259
Phe Ile Ser Leu Ala Pro Trp Asp Leu Gly Val Pro Gln Lys Arg Arg
        40                  45                  50

aag aag aat gag cca gtc cca ttt ttg caa cag atg tcc acg ggc cca    307
Lys Lys Asn Glu Pro Val Pro Phe Leu Gln Gln Met Ser Thr Gly Pro
    55                  60                  65

act gtt gta cag ggc atg ctt tta ggt ttt gtc cat ggc ctg gtg aca    355
Thr Val Val Gln Gly Met Leu Leu Gly Phe Val His Gly Leu Val Thr
70                  75                  80                  85

tat ttg cag ctg ttg ccg tgg atc ggt gag ttt gtt ggc tca ctg cct    403
Tyr Leu Gln Leu Leu Pro Trp Ile Gly Glu Phe Val Gly Ser Leu Pro
                90                  95                  100

tat gtc gcg ttg tca gtt gtc gag gcg ctt tat tcc att gct ctt ggt    451
Tyr Val Ala Leu Ser Val Val Glu Ala Leu Tyr Ser Ile Ala Leu Gly
            105                 110                 115

gct ttc ggc gtg ctc att gcg cgt tgg agg gac tgg aag gtt ctc ctg    499
Ala Phe Gly Val Leu Ile Ala Arg Trp Arg Asp Trp Lys Val Leu Leu
        120                 125                 130

ttt ccg gcg atg tat gtg gct gtg gag tat cta aga agc tcg tgg cca    547
Phe Pro Ala Met Tyr Val Ala Val Glu Tyr Leu Arg Ser Ser Trp Pro
    135                 140                 145

ttt gat gga ttc gcg tgg gtt cgc ctg gca tgg ggt caa att aac ggt    595
Phe Asp Gly Phe Ala Trp Val Arg Leu Ala Trp Gly Gln Ile Asn Gly
150                 155                 160                 165

ccg ttg gct aat ctc gca gcg ctt ggt ggg gta gcg ttt gtc act ttt    643
Pro Leu Ala Asn Leu Ala Ala Leu Gly Gly Val Ala Phe Val Thr Phe
                170                 175                 180

tcc acg gtg ctg gct gcc gtg ggt gtg gcc atg gtg att att tcc aag    691
Ser Thr Val Leu Ala Ala Val Gly Val Ala Met Val Ile Ile Ser Lys
            185                 190                 195

aag cga ctg gcc ggc gca atc atc acc gcg agt gtg att gct atc ggc    739
Lys Arg Leu Ala Gly Ala Ile Ile Thr Ala Ser Val Ile Ala Ile Gly
        200                 205                 210

gcg gtg tca tcc ctg tac gtt gac cgc aat ggc acg agc gat gaa agc    787
Ala Val Ser Ser Leu Tyr Val Asp Arg Asn Gly Thr Ser Asp Glu Ser
    215                 220                 225

atc gaa gta gcc gca att cag ggc aat gtg cct cgg atg gga ttg gac    835
Ile Glu Val Ala Ala Ile Gln Gly Asn Val Pro Arg Met Gly Leu Asp
230                 235                 240                 245

ttc aat gca cag cgc cgc gcg gtg ctg gcg aat cac gca cgg gaa acc    883
Phe Asn Ala Gln Arg Arg Ala Val Leu Ala Asn His Ala Arg Glu Thr
                250                 255                 260

ctc aag ctg gat gaa caa gtg gat ttg gtg atc tgg ccg gag aat tcc     931
Leu Lys Leu Asp Glu Gln Val Asp Leu Val Ile Trp Pro Glu Asn Ser
            265                 270                 275

tca gac gtc aac cca ttt tcc gat gca caa gca aga gcc att atc gat     979
Ser Asp Val Asn Pro Phe Ser Asp Ala Gln Ala Arg Ala Ile Ile Asp
        280                 285                 290

gga gca gtg gaa cat gtt cag gca cct att ttg gtg ggc acg atc acc     1027
Gly Ala Val Glu His Val Gln Ala Pro Ile Leu Val Gly Thr Ile Thr
    295                 300                 305

gtc gat gag gtt ggt cca cgc aac acc atg cag gta ttt gat cct gtt     1075
Val Asp Glu Val Gly Pro Arg Asn Thr Met Gln Val Phe Asp Pro Val
310                 315                 320                 325

gaa ggt gcc gcg gag tac cac aat aag aag ttc ttg cag ccg ttt ggt     1123
Glu Gly Ala Ala Glu Tyr His Asn Lys Lys Phe Leu Gln Pro Phe Gly
                330                 335                 340

gaa tac atg ccg ttt cgc gaa ttc ctg aga att ttc tcg ccc tac gtt     1171
Glu Tyr Met Pro Phe Arg Glu Phe Leu Arg Ile Phe Ser Pro Tyr Val
            345                 350                 355

gat tcc gct gga aac ttc cag ccc ggt gat ggc acc ggc gta gtg gag     1219
Asp Ser Ala Gly Asn Phe Gln Pro Gly Asp Gly Thr Gly Val Val Glu
        360                 365                 370

atg aat gct gcg aac tta ggc cgc gct gtg aca gtg ggc gtg atg acg     1267
Met Asn Ala Ala Asn Leu Gly Arg Ala Val Thr Val Gly Val Met Thr
    375                 380                 385

tgt tac gag gtc atc ttc gac cgt gct ggc cgc gac gcc atc gcc aat     1315
Cys Tyr Glu Val Ile Phe Asp Arg Ala Gly Arg Asp Ala Ile Ala Asn
390                 395                 400                 405

ggg gct gaa ttt ttg acc acg ccc acc aac aac gcc acc ttc gga ttc     1363
Gly Ala Glu Phe Leu Thr Thr Pro Thr Asn Asn Ala Thr Phe Gly Phe
                410                 415                 420

acg gac atg acg tat cag caa tta gca atg agc agg atg cgt gcc atc     1411
Thr Asp Met Thr Tyr Gln Gln Leu Ala Met Ser Arg Met Arg Ala Ile
            425                 430                 435

gaa ttt gat agg gcg gtg gtt gtt gca gct aca tcg ggt gtt tcg gct     1459
Glu Phe Asp Arg Ala Val Val Val Ala Ala Thr Ser Gly Val Ser Ala
        440                 445                 450

atc gtc aac cct gat gga agc att tcc caa aac acc cga att ttt gag     1507
Ile Val Asn Pro Asp Gly Ser Ile Ser Gln Asn Thr Arg Ile Phe Glu
    455                 460                 465

gcc gcc acc ttg acg gaa tcc att cca ctc aag gac act gtc acc atc     1555
Ala Ala Thr Leu Thr Glu Ser Ile Pro Leu Lys Asp Thr Val Thr Ile
470                 475                 480                 485

gca gcg cgg gtt ggt ttc tat gtt gaa tta ctg ttg gtt atc att ggt     1603
Ala Ala Arg Val Gly Phe Tyr Val Glu Leu Leu Leu Val Ile Ile Gly
                490                 495                 500

gta tta gct gga cta ttc gcc att cga atg aat agc cgt tca aag tct    1651
Val Leu Ala Gly Leu Phe Ala Ile Arg Met Asn Ser Arg Ser Lys Ser
            505                 510                 515

gcg aaa ggt tcc gct cgg ccc gca caa gtt cgg gtt aag aag gtg cct    1699
Ala Lys Gly Ser Ala Arg Pro Ala Gln Val Arg Val Lys Lys Val Pro
        520                 525                 530

gcg aaa aag gca gca act aat cgt cga aaa gta aaa taaaaacgtc         1745
Ala Lys Lys Ala Ala Thr Asn Arg Arg Lys Val Lys
    535                 540                 545

ccgaagggac gag                                                     1758

<210> 674
<211> 545
<212> PRT
<213> Corynebacterium glutamicum

<400> 674
Val Thr Leu Phe Val Arg Leu Ala Leu Ala Ala Val Gly Gly Leu Phe
  1               5                  10                  15

Val Phe Ala Ser Asn Glu Pro Ile Gly Trp Phe Val Ala Gly Ile Val
             20                  25                  30

Gly Thr Ala Leu Phe Phe Ile Ser Leu Ala Pro Trp Asp Leu Gly Val
         35                  40                  45

Pro Gln Lys Arg Arg Lys Lys Asn Glu Pro Val Pro Phe Leu Gln Gln
     50                  55                  60

Met Ser Thr Gly Pro Thr Val Val Gln Gly Met Leu Leu Gly Phe Val
 65                  70                  75                  80

His Gly Leu Val Thr Tyr Leu Gln Leu Leu Pro Trp Ile Gly Glu Phe
                 85                  90                  95

Val Gly Ser Leu Pro Tyr Val Ala Leu Ser Val Val Glu Ala Leu Tyr
            100                 105                 110

Ser Ile Ala Leu Gly Ala Phe Gly Val Leu Ile Ala Arg Trp Arg Asp
        115                 120                 125

Trp Lys Val Leu Leu Phe Pro Ala Met Tyr Val Ala Val Glu Tyr Leu
    130                 135                 140

Arg Ser Ser Trp Pro Phe Asp Gly Phe Ala Trp Val Arg Leu Ala Trp
145                 150                 155                 160

Gly Gln Ile Asn Gly Pro Leu Ala Asn Leu Ala Ala Leu Gly Gly Val
                165                 170                 175

Ala Phe Val Thr Phe Ser Thr Val Leu Ala Ala Val Gly Val Ala Met
            180                 185                 190

Val Ile Ile Ser Lys Lys Arg Leu Ala Gly Ala Ile Ile Thr Ala Ser
        195                 200                 205

Val Ile Ala Ile Gly Ala Val Ser Ser Leu Tyr Val Asp Arg Asn Gly

210                 215                 220

Thr Ser Asp Glu Ser Ile Glu Val Ala Ala Ile Gln Gly Asn Val Pro
225                 230                 235                 240

Arg Met Gly Leu Asp Phe Asn Ala Gln Arg Arg Ala Val Leu Ala Asn
                245                 250                 255

His Ala Arg Glu Thr Leu Lys Leu Asp Glu Gln Val Asp Leu Val Ile
            260                 265                 270

Trp Pro Glu Asn Ser Ser Asp Val Asn Pro Phe Ser Asp Ala Gln Ala
        275                 280                 285

Arg Ala Ile Ile Asp Gly Ala Val Glu His Val Gln Ala Pro Ile Leu
    290                 295                 300

Val Gly Thr Ile Thr Val Asp Glu Val Gly Pro Arg Asn Thr Met Gln
305                 310                 315                 320

Val Phe Asp Pro Val Glu Gly Ala Ala Glu Tyr His Asn Lys Lys Phe
                325                 330                 335

Leu Gln Pro Phe Gly Glu Tyr Met Pro Phe Arg Glu Phe Leu Arg Ile
            340                 345                 350

Phe Ser Pro Tyr Val Asp Ser Ala Gly Asn Phe Gln Pro Gly Asp Gly
        355                 360                 365

Thr Gly Val Val Glu Met Asn Ala Ala Asn Leu Gly Arg Ala Val Thr
    370                 375                 380

Val Gly Val Met Thr Cys Tyr Glu Val Ile Phe Asp Arg Ala Gly Arg
385                 390                 395                 400

Asp Ala Ile Ala Asn Gly Ala Glu Phe Leu Thr Thr Pro Thr Asn Asn
                405                 410                 415

Ala Thr Phe Gly Phe Thr Asp Met Thr Tyr Gln Gln Leu Ala Met Ser
            420                 425                 430

Arg Met Arg Ala Ile Glu Phe Asp Arg Ala Val Val Val Ala Ala Thr
        435                 440                 445

Ser Gly Val Ser Ala Ile Val Asn Pro Asp Gly Ser Ile Ser Gln Asn
    450                 455                 460

Thr Arg Ile Phe Glu Ala Ala Thr Leu Thr Glu Ser Ile Pro Leu Lys
465                 470                 475                 480

Asp Thr Val Thr Ile Ala Ala Arg Val Gly Phe Tyr Val Glu Leu Leu
                485                 490                 495

Leu Val Ile Ile Gly Val Leu Ala Gly Leu Phe Ala Ile Arg Met Asn
            500                 505                 510

Ser Arg Ser Lys Ser Ala Lys Gly Ser Ala Arg Pro Ala Gln Val Arg
        515                 520                 525

Val Lys Lys Val Pro Ala Lys Lys Ala Ala Thr Asn Arg Arg Lys Val
    530                 535                 540

Lys
545

<210> 675
<211> 933
<212> DNA
<213> Corynebacterium glutamicum

<220>
<221> CDS
<222> (101)..(910)
<223> RXN01168

<400> 675
ccgcacaagt tcgggttaag aaggtgcctg cgaaaaaggc agcaactaat cgtcgaaaag 60

taaaataaaa acgtcccgaa gggacgagga ggacaacacc atg agc agt gag gca 115
Met Ser Ser Glu Ala
1               5

gta gat gct acg acg ctg gtg att att cca acg tac aac gag ctg gaa 163
Val Asp Ala Thr Thr Leu Val Ile Ile Pro Thr Tyr Asn Glu Leu Glu
                10                  15                  20

aac ctt cca ctc atc gtg gat cgc gtg cgc acc gca acc cct gac gtt 211
Asn Leu Pro Leu Ile Val Asp Arg Val Arg Thr Ala Thr Pro Asp Val
            25                  30                  35

cac gta ctc atc gtg gac gac aac agc cca gac ggc acc ggc gag cgc 259
His Val Leu Ile Val Asp Asp Asn Ser Pro Asp Gly Thr Gly Glu Arg
        40                  45                  50

gca gac aag ctt gct gct gac gac gac cac att ttt gtc ctc cac cgc 307
Ala Asp Lys Leu Ala Ala Asp Asp Asp His Ile Phe Val Leu His Arg
    55                  60                  65

gaa ggc aaa ggc ggc ctg tgc gca gag tac atg gct ggc ttc cag tgg 355
Glu Gly Lys Gly Gly Leu Cys Ala Glu Tyr Met Ala Gly Phe Gln Trp
70                  75                  80                  85

ggc ctg gag cgc gac tac cag gtc ctg tgc gaa atg gac gcc gac ggc 403
Gly Leu Glu Arg Asp Tyr Gln Val Leu Cys Glu Met Asp Ala Asp Gly
                90                  95                  100

tcc cac gca cca gaa cag ctg cac ctg ctg ctc gct gag atc acc aat 451
Ser His Ala Pro Glu Gln Leu His Leu Leu Leu Ala Glu Ile Thr Asn
            105                 110                 115

ggc gct gac ctg gtc atc ggc tcg cgc tac gtg cca ggc ggc cgc gta 499
Gly Ala Asp Leu Val Ile Gly Ser Arg Tyr Val Pro Gly Gly Arg Val
        120                 125                 130

gtc aac tgg ccc aag aac cgt tgg ctc ttg tcc aag ggc ggc aac gtc 547
Val Asn Trp Pro Lys Asn Arg Trp Leu Leu Ser Lys Gly Gly Asn Val
    135                 140                 145

tac atc agc gtc gcg ctc ggc gcc ggc ttg acc gat atg acc gca ggg 595
Tyr Ile Ser Val Ala Leu Gly Ala Gly Leu Thr Asp Met Thr Ala Gly
150                 155                 160                 165

tac cgc gct ttt cga cgt gaa gtg cta gaa gca ctg ccg ctt gat gag     643
Tyr Arg Ala Phe Arg Arg Glu Val Leu Glu Ala Leu Pro Leu Asp Glu
            170                 175                 180

ctc tcc aac gct ggg tac att ttc caa gtt gag att gcc tac cgt gca     691
Leu Ser Asn Ala Gly Tyr Ile Phe Gln Val Glu Ile Ala Tyr Arg Ala
        185                 190                 195

gtt gaa gcc gga ttc gat gtt cgt gaa gtt ccc atc act ttc acc gag     739
Val Glu Ala Gly Phe Asp Val Arg Glu Val Pro Ile Thr Phe Thr Glu
    200                 205                 210

cgt gag atc ggc gaa tcc aag ctg gac ggc agc ttt gtc aag gat tcc     787
Arg Glu Ile Gly Glu Ser Lys Leu Asp Gly Ser Phe Val Lys Asp Ser
    215                 220                 225

ctg ctc gag gta acc aag tgg ggc ctc aag cac cgc ggt ggc cag gcc     835
Leu Leu Glu Val Thr Lys Trp Gly Leu Lys His Arg Gly Gly Gln Ala
230                 235                 240                 245

aag gaa ctg tcc aag gaa atg gtc ggc ctg ctg aac tat gag tgg aag     883
Lys Glu Leu Ser Lys Glu Met Val Gly Leu Leu Asn Tyr Glu Trp Lys
            250                 255                 260

cac ttc aaa aag cgc aac acc tgg ctc taaactgctt gccggttagt           930
His Phe Lys Lys Arg Asn Thr Trp Leu
            265                 270

gaa                                                                 933


<210> 676
<211> 270
<212> PRT
<213> Corynebacterium glutamicum

<400> 676
Met Ser Ser Glu Ala Val Asp Ala Thr Thr Leu Val Ile Ile Pro Thr
1               5                   10                  15

Tyr Asn Glu Leu Glu Asn Leu Pro Leu Ile Val Asp Arg Val Arg Thr
            20                  25                  30

Ala Thr Pro Asp Val His Val Leu Ile Val Asp Asp Asn Ser Pro Asp
        35                  40                  45

Gly Thr Gly Glu Arg Ala Asp Lys Leu Ala Ala Asp Asp Asp His Ile
    50                  55                  60

Phe Val Leu His Arg Glu Gly Lys Gly Gly Leu Cys Ala Glu Tyr Met
65                  70                  75                  80

Ala Gly Phe Gln Trp Gly Leu Glu Arg Asp Tyr Gln Val Leu Cys Glu
                85                  90                  95

Met Asp Ala Asp Gly Ser His Ala Pro Glu Gln Leu His Leu Leu Leu
            100                 105                 110

Ala Glu Ile Thr Asn Gly Ala Asp Leu Val Ile Gly Ser Arg Tyr Val
        115                 120                 125

Pro Gly Gly Arg Val Val Asn Trp Pro Lys Asn Arg Trp Leu Leu Ser
    130                 135                 140

Lys Gly Gly Asn Val Tyr Ile Ser Val Ala Leu Gly Ala Gly Leu Thr
145                 150                 155                 160

Asp Met Thr Ala Gly Tyr Arg Ala Phe Arg Arg Glu Val Leu Glu Ala
                165                 170                 175

Leu Pro Leu Asp Glu Leu Ser Asn Ala Gly Tyr Ile Phe Gln Val Glu
            180                 185                 190

Ile Ala Tyr Arg Ala Val Glu Ala Gly Phe Asp Val Arg Glu Val Pro
        195                 200                 205

Ile Thr Phe Thr Glu Arg Glu Ile Gly Glu Ser Lys Leu Asp Gly Ser
    210                 215                 220

Phe Val Lys Asp Ser Leu Leu Glu Val Thr Lys Trp Gly Leu Lys His
225                 230                 235                 240

Arg Gly Gly Gln Ala Lys Glu Leu Ser Lys Glu Met Val Gly Leu Leu
                245                 250                 255

Asn Tyr Glu Trp Lys His Phe Lys Lys Arg Asn Thr Trp Leu
            260                 265                 270